# EUROPEAN PATENT APPLICATION

(11) **EP 4 803 615 A1**
(43) Date of publication of application: **09.09.2026**
(21) Application number: 24884693.3
(22) Date of filing: 29.10.2024
(51) Int. Cl.: C12N 5/10, C12N 15/11, A61K 35/17, A61P 35/00

(54) **MODIFIED CELL AND USE THEREOF**

(30) Priority: 30.10.2023 WO PCT/CN2023/127550; 30.10.2023 WO PCT/CN2023/127552; 30.10.2023 WO PCT/CN2023/127549
(71) Applicant: Beijing Grit Biotherapeutics Co., Ltd., Shanghai 200120 (CN); Shanghai Grit Biotechnology Co., Ltd., Shanghai 200120 (CN); Suzhou Grit Biotechnology Co., Ltd., Shanghai 200120 (CN)
(72) Inventor: LIU, Yarong, Shanghai 200120 (CN); SUN, Jingwei, Shanghai 200120 (CN); SHENG, Yao, Shanghai 200120 (CN)
(74) Representative: Fish & Richardson P.C.
(86) International application number: PCT/CN2024/127935
(87) International publication number: WO 2025/092690

(57) **Abstract**

The present invention belongs to the field of biomedicine, and a modified cell and uses thereof are provided. In particular, a method for culturing a cell is provided, which comprises causing expression of a target gene of the cell to be reduced and/or activity to be attenuated. The present invention further relates to a method for preventing and/or treating a tumor using the cultured cell.

## Description

### TECHNICAL FIELD

The present invention relates to the biomedical field, and in particular relates to a modified cell and the use thereof.

### BACKGROUND

At present, immunotherapy is an effective method for treating patients with poor prognosis. However, immune cells used in immunotherapy have problems in that, after being reinfused in vivo, cell functions are not strong, or proliferation and persistence capabilities are weak. Therefore, a modified immune cell and a robust and reliable method for culturing immune cells need to be provided, which is an urgent problem to be solved.

### SUMMARY

The present invention provides a method for culturing cells, wherein the method has one or more of the following advantages: enhanced target cell killing ability, enhanced cell proliferation ability, enhanced cytokine release ability, an increased proportion of activated cells, a reduced proportion of regulatory cells, a reduced proportion of exhausted cells, an increased proportion of central memory cells and/or naïve cells, a reduced proportion of apoptotic cells, and an increased proportion of stem cell-like cells.

In one aspect, the present invention provides a method for culturing cells, the method comprising: causing expression of a family member selected from the Bcl-2 family or the protein tyrosine phosphatase family and/or a functionally active fragment thereof in the cells to be reduced and/or causing activity thereof to be weakened.

In another aspect, the present invention provides a cell, wherein the cell is obtained by the method of the present invention.

In another aspect, the present invention provides a pharmaceutical composition, comprising the cell of the present invention and an optional pharmaceutically acceptable carrier.

In another aspect, the present invention provides a method for affecting cell growth, comprising administering the cell of the present invention and/or the pharmaceutical composition of the present invention.

In another aspect, the present invention provides use of the cell of the present invention and/or the pharmaceutical composition of the present invention in the preparation of a medicament, wherein the medicament is used for preventing and/or treating a disease and/or symptom.

Other aspects and advantages of the present invention are readily apparent to those skilled in the art from the detailed description below. Only exemplary embodiments of the present invention are shown and described in the detailed description below. As will be appreciated by those skilled in the art, the disclosure of the present invention enables those skilled in the art to modify the disclosed specific embodiments without departing from the spirit and scope of the present invention. Accordingly, the drawings and the description in the specification of the present invention are merely exemplary and are not limiting.

### BRIEF DESCRIPTION OF THE DRAWINGS

The features and advantages of the present invention are better understood with reference to the exemplary embodiments described in detail below and the accompanying drawings. The drawings are briefly described as follows:
FIG. 1 shows, relative to the start codon, a gene-editing targeting segment of the human BCL2L11 gene provided by the present invention; for example, the segment may be a continuous region having about 3 or more transcription factor binding sites; and the segment may be an exon region of the gene or an intron region about 100 bp away from the exon.
FIG. 2 shows, relative to the start codon, a gene-editing targeting segment of the human PTPN2 gene provided by the present invention; for example, the segment may be a continuous region having about 3 or more transcription factor binding sites; and the segment may be an exon region of the gene or an intron region about 100 bp away from the exon.
FIG. 3 shows the fold expansion of TIL subjected to single-target gene editing of PTPN2 or BCL2L11 in an unstimulated medium group.
FIG. 4 shows the fold expansion of TIL subjected to single-target gene editing of PTPN2 or BCL2L11 in a TransACT-stimulated group.
FIG. 5 shows the fold expansion of TIL subjected to gene editing of a TNFAIP3 and PTPN2 combination, a TNFAIP3 and BCL2L11 combination, or an IKZF1 and PTPN2 combination in an unstimulated medium group.
FIG. 6 shows the fold expansion of TIL subjected to gene editing of a TNFAIP3 and PTPN2 combination or a TNFAIP3 and BCL2L11 combination in a TransACT-stimulated group.
FIG. 7 shows the target cell killing capability of PTPN2-edited TIL cells derived from donor 812.
FIG. 8 shows the target cell killing capability of PTPN2-edited TIL cells derived from donor 107.
FIG. 9 shows the target cell killing capability of TIL cells subjected to combination editing of TNFAIP3 and PTPN2 and derived from donor 309.
FIG. 10 shows the target cell killing capability of TIL cells subjected to combination editing of IKZF1 and PTPN2 and derived from donor 309.
FIG. 11 shows the target cell killing capability of TIL cells subjected to combination editing of TNFAIP3 and PTPN2 and derived from donor 812.
FIG. 12 shows the target cell killing capability of TIL cells subjected to combination editing of IKZF1 and PTPN2 and derived from donor 812.
FIG. 13 shows results of multiple-round killing by PTPN2-edited cells in TCR-T cells.
FIG. 14 shows results of multiple-round killing by PTPN2-edited or BCL2L11-edited cells in TCR-T cells.
FIG. 15 shows PDO model killing results of TIL cells subjected to combination editing of IKZF1 and PTPN2.
FIG. 16 shows the proportion of central memory T cells in TIL cells after PTPN2 or BCL2L11 editing. For example, the central memory T cells may be CD45RO-positive CD62L-positive cells.
FIG. 17 shows the proportion of naïve T cells in TIL cells after BCL2L11 editing. For example, the naïve T cells may be CD45RO-negative CD62L-positive cells.
FIGs. 18, 19, and 20 show the proportion of exhausted cells in TIL cells after PTPN2 or BCL2L11 editing. For example, the exhausted T cells may be PD-1-positive, LAG-3-positive, TIM-3-positive, CD38-positive, and/or CD101-positive cells.
FIG. 21 shows the proportion of stem-like T cells in TIL cells after PTPN2 or BCL2L11 editing. For example, the stem-like T cells may have a CD39-negative CD69-negative phenotype.
FIG. 22 shows the proportion of stem-like T cells in TIL cells after PTPN2 editing. For example, the stem-like T cells may have a TCF1-positive phenotype.
FIG. 23 shows that, in the unstimulated medium group, PTPN2-edited TIL cells have a higher proportion of cytokine expression.
FIG. 24 shows that, in the unstimulated medium group, PTPN2-edited or BCL2L11-edited TIL cells have a higher proportion of cytokine expression.
FIG. 25 shows that, in the unstimulated medium group, BCL2L11-edited TIL cells have a higher proportion of cytokine expression.
FIGs. 26 and 27 show that, in the TransACT-stimulated group, PTPN2-edited TIL cells have a higher proportion of cytokine expression.
FIGs. 28 and 29 show that, in the TransACT-stimulated group, BCL2L11-edited TIL cells have a higher proportion of cytokine expression.
FIG. 30 shows that, in the unstimulated medium group, TIL cells after combination editing of TNFAIP3 and PTPN2 or after combination editing of TNFAIP3 and BCL2L11 have a higher proportion of cytokine expression.
FIG. 31 shows that, in the unstimulated medium group, TIL cells after combination editing of TNFAIP3 and PTPN2 have a higher proportion of cytokine expression.
FIG. 32 shows that, in the unstimulated medium group, TIL cells after combination editing of IKZF1 and PTPN2 have a higher proportion of cytokine expression.
FIG. 33 shows that, in the TransACT-stimulated group, TIL cells after combination editing of TNFAIP3 and PTPN2 or after combination editing of TNFAIP3 and BCL2L11 have a higher proportion of cytokine expression.
FIG. 34 shows that, in the TransACT-stimulated group, TIL cells after combination editing of TNFAIP3 and PTPN2 have a higher proportion of cytokine expression.
FIG. 35 shows that, in the TransACT-stimulated group, TIL cells after combination editing of IKZF1 and PTPN2 have a higher proportion of cytokine expression.
FIG. 36 shows that, in TCR-T cells, cells after BCL2L11 editing have a higher level of cytokine release.
FIG. 37 shows that, in TIL cells, cells after combination editing of IKZF1 and PTPN2 have a higher level of cytokine release in the TransACT-stimulated group.
FIG. 38 shows that, in TIL cells, cells after combination editing of IKZF1 and PTPN2 have a higher level of cytokine release after co-culture with A375 tumor cells.
FIG. 39 shows that, in TIL cells, cells after combination editing of IKZF1 and PTPN2 have a higher level of cytokine release after co-culture with an autologous PDO.
FIG. 40A shows that, in the unstimulated group, TCR-T cells subjected to BCL2L11 gene editing may have significant expansion capability.
FIG. 40B shows that, in the unstimulated group, TCR-T cells subjected to PTPN2 gene editing may have significant expansion capability.
FIG. 40C shows that, in the TransACT-stimulated group, TCR-T cells subjected to BCL2L11 gene editing may have significant expansion capability.
FIG. 40D shows that, in the TransACT-stimulated group, TCR-T cells subjected to PTPN2 gene editing may have significant expansion capability.
FIG. 40E shows the target cell killing capability of TCR-T cells subjected to BCL2L11 gene editing.
FIG. 40F shows the target cell killing capability of TCR-T cells subjected to PTPN2 gene editing.
FIG. 40G shows the target cell killing capability of TCR-T cells subjected to BCL2L11 gene editing.
FIG. 40H shows the target cell killing capability of TCR-T cells subjected to PTPN2 gene editing.
FIGs. 40I-40N show cytokine release of unedited TCR T cells or TCR T cells in which the BCL2L11 target site or the PTPN2 target site is knocked out, as detected using a CBA kit.
FIGs. 41A-41E show, relative to the start codon, gene-editing targeting segments of the human AFF3, AXL, NFE2L1, RARG, and UBFD1 genes provided by the present invention; for example, the segments may be continuous regions having about 3 or more transcription factor binding sites; and the segments may be exon regions of the genes or intron regions about 100 bp away from the exons.
FIG. 42 shows the expansion capability of TCRT subjected to single-target gene editing of UBFD1.
FIG. 43 shows results of multiple-round killing by cells subjected to single-target gene editing of AFF3, AXL, NFE2L1, RARG, or UBFD1 in TCR-T cells.
FIG. 44 shows that, in TCR-T cells, cells after single-target editing of AFF3, AXL, NFE2L1, RARG, or UBFD1 have a higher level of cytokine release.
FIG. 45 shows the target cell killing capability of UBFD1-edited TIL cells derived from donor 309.
FIG. 46 shows the target cell killing capability of AFF3-edited, NFE2L1-edited, RARG-edited, or UBFD1-edited TIL cells derived from donor 812.
FIG. 47 shows the fold expansion of TIL subjected to single-target gene editing of AFF3, AXL, NFE2L1, RARG, or UBFD1 in the unstimulated medium group.
FIG. 48 shows the fold expansion of TIL subjected to single-target gene editing of AFF3, AXL, NFE2L1, RARG, or UBFD1 in the TransACT-stimulated group.
FIG. 49 shows the proportion of central memory T cells in TIL cells after AXL, NFE2L1, RARG, or UBFD1 editing. For example, the central memory T cells may be CD45RO-positive CD62L-positive cells.
FIG. 50 shows the proportion of naïve T cells in TIL cells after AXL editing. For example, the naïve T cells may be CD45RO-negative CD62L-positive cells.
FIGs. 51, 52, and 53 show the proportion of exhausted cells in TIL cells after AFF3, AXL, NFE2L1, RARG, or UBFD1 editing. For example, the exhausted T cells may be PD-1-positive, LAG-3-positive, TIM-3-positive, CD38-positive, and/or CD101-positive cells.
FIGs. 54 and 55 show the proportion of stem-like T cells in TIL cells after AFF3, AXL, NFE2L1, RARG, or UBFD1 editing. For example, the stem-like T cells may have a CD39-negative CD69-negative or TCF1-positive phenotype.
FIGs. 56 and 57 show that, in the unstimulated medium group, TIL cells after AFF3, AXL, or NFE2L1 editing have a higher proportion of cytokine expression.
FIGs. 58, 59, and 60 show that, in the TransACT-stimulated group, TIL cells after AFF3, AXL, NFE2L1, or RARG editing have a higher proportion of cytokine expression.

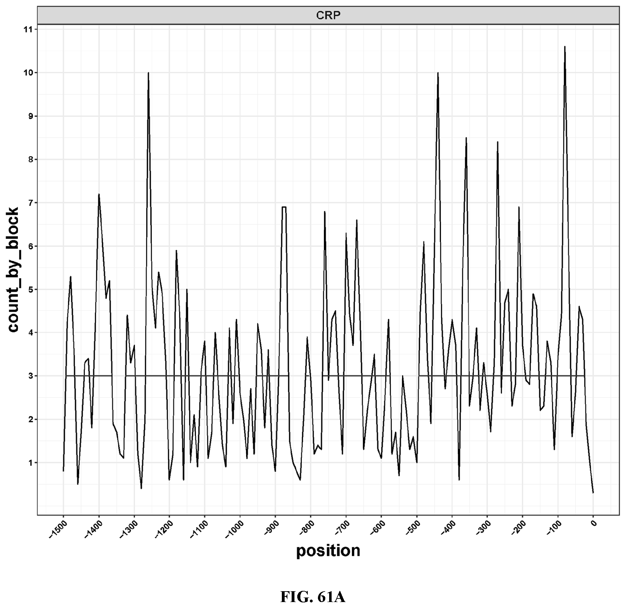

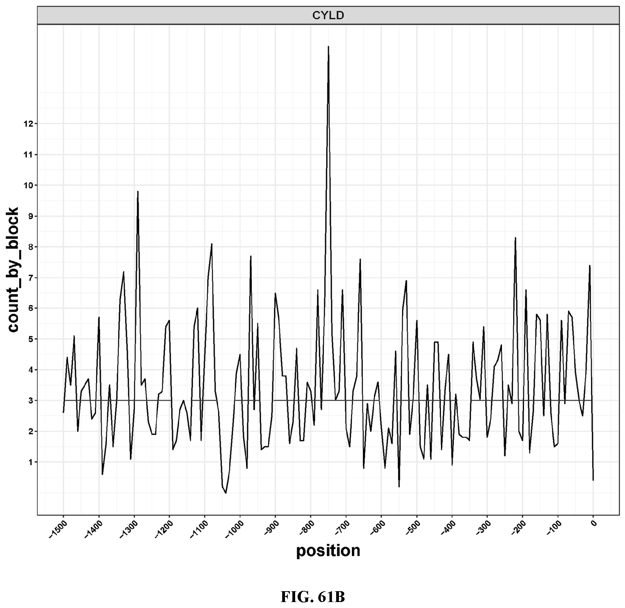

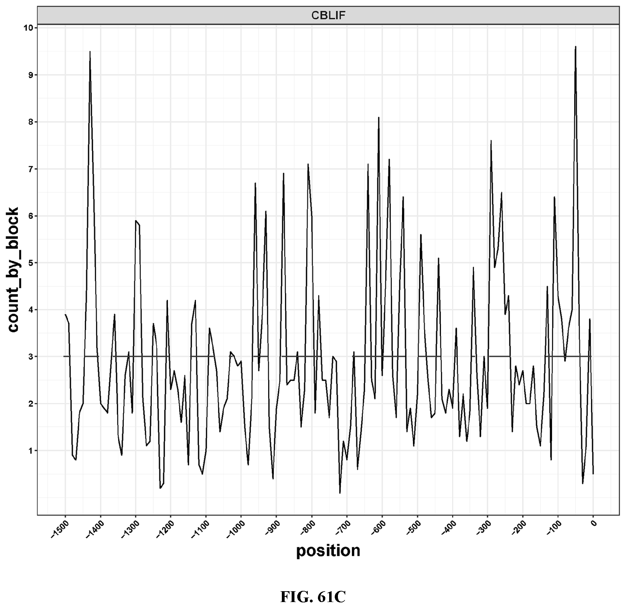

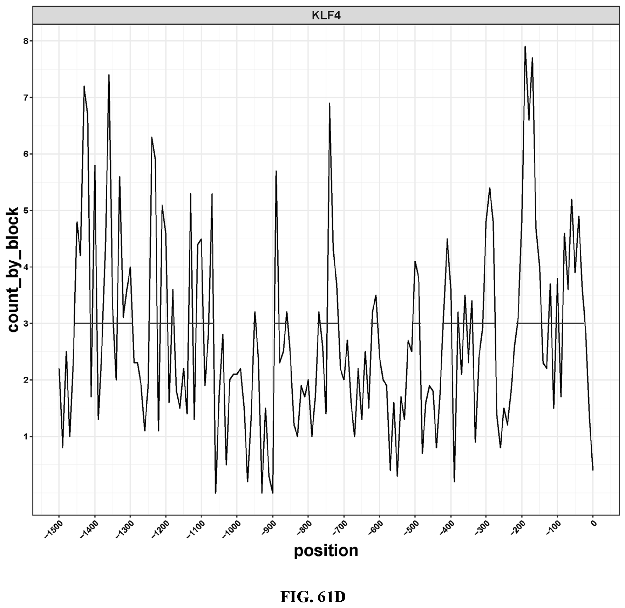

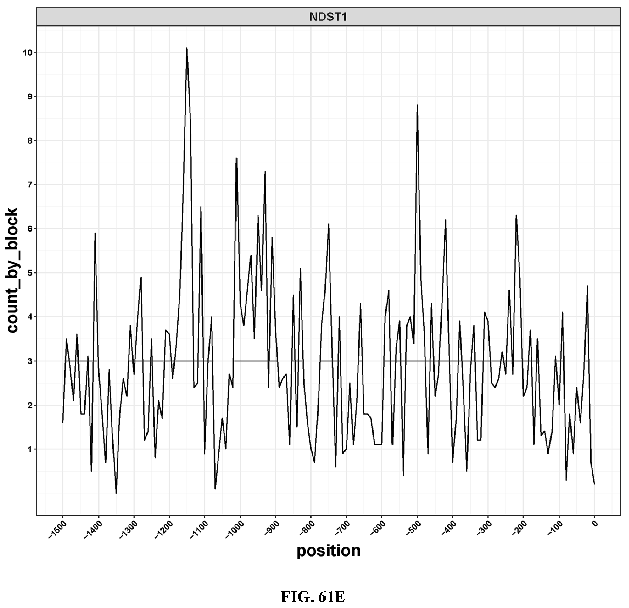

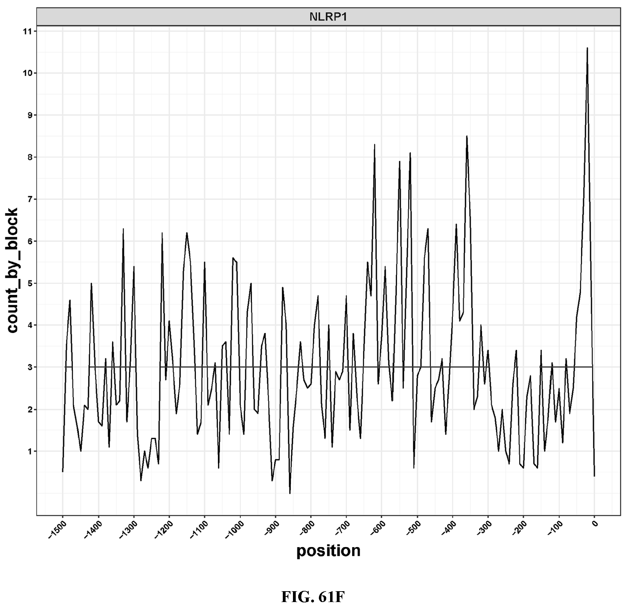

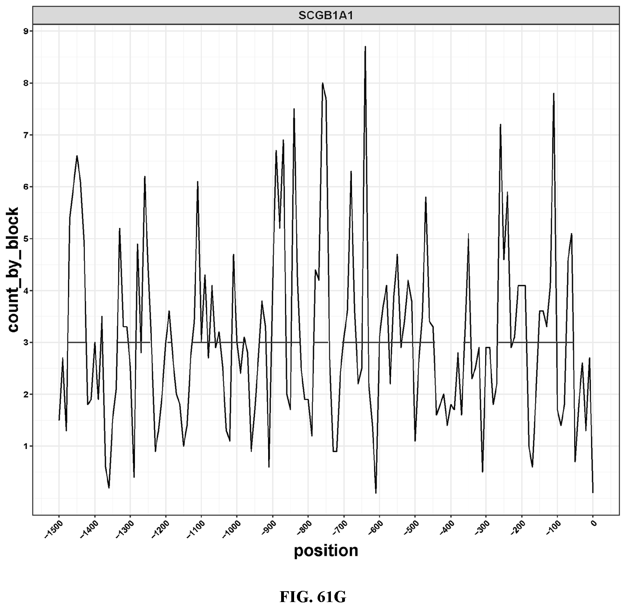

FIGs. 61A-61G show, relative to the start codon, gene-editing targeting segments of the human CRP, CYLD, CBLIF, KLF4, NDST1, NLRP1, and SCGB1A1 genes provided by the present invention; for example, the segments may be continuous regions having about 3 or more transcription factor binding sites; and the segments may be exon regions of the genes or intron regions about 100 bp away from the exons.
FIG. 62 shows the expansion capability of TCRT subjected to single-target gene editing of KLF4, NDST1, NLRP1, or SCGB1A1.
FIG. 63 shows results of multiple-round killing by cells subjected to single-target gene editing of CRP, CYLD, CBLIF, KLF4, NDST1, or SCGB1A1 in TCR-T cells.
FIGs. 64, 65, and 66 show that, in TCR-T cells, cells subjected to single-target editing of CRP, CYLD, CBLIF, KLF4, NDST1, NLRP1, or SCGB1A1 have higher cytokine release levels.
FIG. 67 shows the fold expansion of TILs subjected to single-target gene editing of CRP, CYLD, KLF4, NDST1, or SCGB1A1 in the unstimulated medium group.
FIG. 68 shows the fold expansion of TILs subjected to single-target gene editing of CRP, CYLD, KLF4, or SCGB1A1 in the TransACT-stimulated group.
FIGs. 69, 70, and 71 show the tumor cell killing ability of cells subjected to single-target editing of CYLD, NDST1, or SCGB1A1 in TIL cells.
FIG. 72 shows the proportion of central memory T cells in TIL cells subjected to editing of CBLIF, KLF4, NDST1, or SCGB1A1. For example, the central memory T cells may be CD45RO-positive CD62L-positive cells.
FIG. 73 shows the proportion of naïve T cells in TIL cells subjected to editing of CRP, CYLD, CBLIF, KLF4, NDST1, or SCGB1A1. For example, the naïve T cells may be CD45RO-negative CD62L-positive cells.
FIGs. 74, 75, and 76 show the proportion of exhausted cells in TIL cells subjected to editing of CRP, CYLD, CBLIF, KLF4, NDST1, or SCGB1A1. For example, the exhausted T cells may be PD-1-positive, LAG-3-positive, TIM-3-positive, CD38-positive, and/or CD101-positive cells.
FIGs. 77 and 78 show the proportion of stem-like T cells in TIL cells subjected to editing of CRP, CYLD, CBLIF, KLF4, NDST1, NLRP1, or SCGB1A1. For example, the stem-like T cells may have a CD39-negative CD69-negative or TCF1-positive phenotype.
FIGs. 79, 80, and 81 show that, in the unstimulated medium group, TIL cells subjected to editing of CRP, CYLD, CBLIF, KLF4, NDST1, NLRP1, or SCGB1A1 have a higher proportion of cytokine expression.
FIGs. 82 and 83 show that, in the TransACT-stimulated group, TIL cells subjected to editing of CRP, CYLD, CBLIF, KLF4, NDST1, NLRP1, or SCGB1A1 have a higher proportion of cytokine expression.

### DETAILED DESCRIPTION

The embodiments of the present invention are described below by specific embodiments, and other advantages and effects of the present invention may be readily understood by those skilled in the art from the content disclosed in this specification.

### Definition of Terms

In the present application, the term "Bcl-2 family member" generally refers to a family member protein having a Bcl-2 homology domain 3 (BH3) domain or a functionally active fragment thereof. For example, a Bcl-2 family member comprised may comprise BCL2L11. For example, the UniProt accession number of the Bcl-2 family member may be O43521. The Bcl-2 family member of the present application may further encompass a functionally active fragment thereof, without being limited to human and mammalian forms, conservative amino acid substitutions, glycoform modifications or variants, active fragments thereof, or substances comprising the functionally active fragment produced by processing and/or modification occurring in cells. For example, the Bcl-2 family member of the present application may comprise a functionally active fragment thereof and any other domain.

In the present application, the term "protein tyrosine phosphatase family member" generally refers to a family member protein having a tyrosine phosphatase domain or a functionally active fragment thereof. For example, a protein tyrosine phosphatase family member comprised may comprise PTPN2. For example, the UniProt accession number of the protein tyrosine phosphatase family member may be P17706. The protein tyrosine phosphatase family member of the present application may further encompass a functionally active fragment thereof, without being limited to human and mammalian forms, conservative amino acid substitutions, glycoform modifications or variants, active fragments thereof, or substances comprising the functionally active fragment produced by processing and/or modification occurring in cells. For example, the protein tyrosine phosphatase family member of the present application may comprise a functionally active fragment thereof and any other domain.

In the present application, the term "AF4 family member" generally refers to a family member protein having a transcription activation domain or a functionally active fragment thereof. For example, an AF4 family member comprised may comprise AFF3. For example, the UniProt accession number of the AF4 family member may be P51826. The AF4 family member of the present application may further encompass a functionally active fragment thereof, without being limited to human and mammalian forms, conservative amino acid substitutions, glycoform modifications or variants, active fragments thereof, or substances comprising the functionally active fragment produced by processing and/or modification occurring in cells. For example, the AF4 family member of the present application may comprise a functionally active fragment thereof and any other domain.

In the present application, the term "tyrosine protein kinase family member" generally refers to a family member protein having a phosphotransferase domain or a functionally active fragment thereof. For example, a tyrosine protein kinase family member comprised may comprise AXL. For example, the UniProt accession number of the tyrosine protein kinase family member may be P30530. The tyrosine protein kinase family member of the present application may further encompass a functionally active fragment thereof, without being limited to human and mammalian forms, conservative amino acid substitutions, glycoform modifications or variants, active fragments thereof, or substances comprising the functionally active fragment produced by processing and/or modification occurring in cells. For example, the tyrosine protein kinase family member of the present application may comprise a functionally active fragment thereof and any other domain.

In the present application, the term "bZIP family member" generally refers to a family member protein having a bZIP-related DNA-binding domain or a functionally active fragment thereof. For example, a bZIP family member comprised may comprise NFE2L1. For example, the UniProt accession number of the bZIP family member may be Q14494. The bZIP family member of the present application may further encompass a functionally active fragment thereof, without being limited to human and mammalian forms, conservative amino acid substitutions, glycoform modifications or variants, active fragments thereof, or substances comprising the functionally active fragment produced by processing and/or modification occurring in cells. For example, the bZIP family member of the present application may comprise a functionally active fragment thereof and any other domain.

In the present application, the term "nuclear receptor family member" generally refers to a family member protein having a nuclear receptor-related DNA-binding domain or a functionally active fragment thereof. For example, a nuclear receptor family member comprised may comprise RARG. For example, the UniProt accession number of the nuclear receptor family member may be P13631. The nuclear receptor family member of the present application may further encompass a functionally active fragment thereof, without being limited to human and mammalian forms, conservative amino acid substitutions, glycoform modifications or variants, active fragments thereof, or substances comprising the functionally active fragment produced by processing and/or modification occurring in cells. For example, the nuclear receptor family member of the present application may comprise a functionally active fragment thereof and any other domain.

In the present application, the term "ubiquitin family member" generally refers to a family member protein having a ubiquitin-like domain or a functionally active fragment thereof. For example, a ubiquitin family member comprised may comprise UBFD1. For example, the UniProt accession number of the ubiquitin family member may be O14562. The ubiquitin family member of the present application may further encompass a functionally active fragment thereof, without being limited to human and mammalian forms, conservative amino acid substitutions, glycoform modifications or variants, active fragments thereof, or substances comprising the functionally active fragment produced by processing and/or modification occurring in cells. For example, the ubiquitin family member of the present application may comprise a functionally active fragment thereof and any other domain.

In the present application, the term "pentraxin family member" generally refers to a family member protein having a pentraxin domain or a functionally active fragment thereof. For example, a pentraxin family member comprised may comprise CRP. For example, the UniProt accession number of the pentraxin family member may be P02741. The pentraxin family member of the present application may further encompass a functionally active fragment thereof, without being limited to human and mammalian forms, conservative amino acid substitutions, glycoform modifications or variants, active fragments thereof, or substances comprising the functionally active fragment produced by processing and/or modification occurring in cells. For example, the pentraxin family member of the present application may comprise a functionally active fragment thereof and any other domain.

In the present application, the term "peptidase C19 family member" generally refers to a family member protein having a cytoskeleton-associated protein glycine-conserved (CAP-GLY) domain or a functionally active fragment thereof. For example, a peptidase C19 family member comprised may comprise CYLD. For example, the UniProt accession number of the peptidase C19 family member may be Q9NQC7. The peptidase C19 family member of the present application may further encompass a functionally active fragment thereof, without being limited to human and mammalian forms, conservative amino acid substitutions, glycoform modifications or variants, active fragments thereof, or substances comprising the functionally active fragment produced by processing and/or modification occurring in cells. For example, the peptidase C19 family member of the present application may comprise a functionally active fragment thereof and any other domain.

In the present application, the term "cobalamin transporter family member" generally refers to a family member protein having a cobalamin-binding domain or a functionally active fragment thereof. For example, a cobalamin transporter family member comprised may comprise CBLIF. For example, the UniProt accession number of the cobalamin transporter family member may be P27352. The cobalamin transporter family member of the present application may further encompass a functionally active fragment thereof, without being limited to human and mammalian forms, conservative amino acid substitutions, glycoform modifications or variants, active fragments thereof, or substances comprising the functionally active fragment produced by processing and/or modification occurring in cells. For example, the cobalamin transporter family member of the present application may comprise a functionally active fragment thereof and any other domain.

In the present application, the term "Krueppel C2H2-type zinc finger protein family member" generally refers to a family member protein having a C2H2-type zinc finger domain or a functionally active fragment thereof. For example, a Krueppel C2H2-type zinc finger protein family member comprised may comprise KLF4. For example, the UniProt accession number of the Krueppel C2H2-type zinc finger protein family member may be O43474. The Krueppel C2H2-type zinc finger protein family member of the present application may further encompass a functionally active fragment thereof, without being limited to human and mammalian forms, conservative amino acid substitutions, glycoform modifications or variants, active fragments thereof, or substances comprising the functionally active fragment produced by processing and/or modification occurring in cells. For example, the Krueppel C2H2-type zinc finger protein family member of the present application may comprise a functionally active fragment thereof and any other domain.

In the present application, the term "sulfotransferase 1 family member" generally refers to a family member protein having a sulfotransferase domain or a functionally active fragment thereof. For example, a sulfotransferase 1 family member comprised may comprise NDST1. For example, the UniProt accession number of the sulfotransferase 1 family member may be P52848. The sulfotransferase 1 family member of the present application may further encompass a functionally active fragment thereof, without being limited to human and mammalian forms, conservative amino acid substitutions, glycoform modifications or variants, active fragments thereof, or substances comprising the functionally active fragment produced by processing and/or modification occurring in cells. For example, the sulfotransferase 1 family member of the present application may comprise a functionally active fragment thereof and any other domain.

In the present application, the term "Nod-like receptor (NLR) protein family member" generally refers to a family member protein having a caspase recruitment domain (CARD) or a functionally active fragment thereof. For example, a Nod-like receptor (NLR) protein family member comprised may comprise NLRP1. For example, the UniProt accession number of the Nod-like receptor (NLR) protein family member may be Q9C000. The Nod-like receptor (NLR) protein family member of the present application may further encompass a functionally active fragment thereof, without being limited to human and mammalian forms, conservative amino acid substitutions, glycoform modifications or variants, active fragments thereof, or substances comprising the functionally active fragment produced by processing and/or modification occurring in cells. For example, the Nod-like receptor (NLR) protein family member of the present application may comprise a functionally active fragment thereof and any other domain.

In the present application, the term "secretoglobin family member" generally refers to a family member protein having a uteroglobin domain or a functionally active fragment thereof. For example, a secretoglobin family member comprised may comprise SCGB1A1. For example, the UniProt accession number of the secretoglobin family member may be P11684. The secretoglobin family member of the present application may further encompass a functionally active fragment thereof, without being limited to human and mammalian forms, conservative amino acid substitutions, glycoform modifications or variants, active fragments thereof, or substances comprising the functionally active fragment produced by processing and/or modification occurring in cells. For example, the secretoglobin family member of the present application may comprise a functionally active fragment thereof and any other domain.

In the present application, the term "CBL family member" generally refers to a family member protein having an SH3 domain or a functionally active fragment thereof. For example, the CBL family member may comprise CBLB. For example, the UniProt accession number of the CBL family member may be Q13191. The CBL family member of the present application may further encompass a functionally active fragment thereof, without being limited to human and mammalian forms, conservative amino acid substitutions, glycoform modifications or variants, active fragments thereof, or substances comprising the functionally active fragment produced after processing and/or modification occurring in cells. For example, the CBL family member of the present application may comprise a functionally active fragment thereof and any other domains.

In the present application, the term "signal transducer and activator of transcription (STAT)-induced STAT inhibitor (SSI) family member" generally refers to a family member protein having an SH2 domain or a functionally active fragment thereof. For example, the STAT-induced STAT inhibitor (SSI) family member may comprise SOCS1. For example, the UniProt accession number of the STAT-induced STAT inhibitor (SSI) family member may be O15524. The STAT-induced STAT inhibitor (SSI) family member of the present application may further encompass a functionally active fragment thereof, without being limited to human and mammalian forms, conservative amino acid substitutions, glycoform modifications or variants, active fragments thereof, or substances comprising the functionally active fragment produced after processing and/or modification occurring in cells. For example, the STAT-induced STAT inhibitor (SSI) family member of the present application may comprise a functionally active fragment thereof and any other domains.

In the present application, the term "peptidase C64 family member" generally refers to a family member protein having a ubiquitin-binding domain or a functionally active fragment thereof. For example, the peptidase C64 family member may comprise TNFAIP3. For example, the UniProt accession number of the peptidase C64 family member may be P21580. The peptidase C64 family member of the present application may further encompass a functionally active fragment thereof, without being limited to human and mammalian forms, conservative amino acid substitutions, glycoform modifications or variants, active fragments thereof, or substances comprising the functionally active fragment produced after processing and/or modification occurring in cells. For example, the peptidase C64 family member of the present application may comprise a functionally active fragment thereof and any other domains.

In the present application, the term "ZC3H12 family member" generally refers to a family member protein having a C3H1-type zinc finger domain or a functionally active fragment thereof. For example, the ZC3H12 family member may comprise ZC3H12A. For example, the UniProt accession number of the ZC3H12 family member may be Q5D1E8. The ZC3H12 family member of the present application may further encompass a functionally active fragment thereof, without being limited to human and mammalian forms, conservative amino acid substitutions, glycoform modifications or variants, active fragments thereof, or substances comprising the functionally active fragment produced after processing and/or modification occurring in cells. For example, the ZC3H12 family member of the present application may comprise a functionally active fragment thereof and any other domains.

In the present invention, the term "IKAROS zinc finger protein family member" generally refers to a family member protein having a zinc finger domain or a functionally active fragment thereof. For example, the IKAROS zinc finger protein family member may comprise IKZF1. For example, the UniProt accession number of the IKAROS zinc finger protein family member may be Q13422. The IKAROS zinc finger protein family member of the present invention may further encompass a functionally active fragment thereof, without being limited to human and mammalian forms, conservative amino acid substitutions, glycoform modifications or variants, active fragments thereof, or substances comprising the functionally active fragment produced after processing and/or modification occurring in cells. For example, the IKAROS zinc finger protein family member of the present invention may comprise a functionally active fragment thereof and any other domains.

In the present application, the term "tumor necrosis factor α-induced protein 3 (TNFAIP3)" generally refers to an inhibitory molecule of a signaling pathway. For example, ubiquitination of a signal transduction substance of the NF-κB pathway may be caused by TNFAIP3. For example, the UniProt accession number of TNFAIP3 may be P21580. In the present application, TNFAIP3 may encompass unprocessed TNFAIP3, TNFAIP3 processed in any form, a variant of TNFAIP3, or a substance comprising a functionally active fragment of TNFAIP3.

In the present invention, the term "GTPase-activating protein 1 family member" generally refers to a family member protein having a GTPase-activating domain or a functionally active fragment thereof. For example, the GTPase-activating protein 1 family member may comprise RASA2. For example, the UniProt accession number of the GTPase-activating protein 1 family member may be Q15283. The GTPase-activating protein 1 family member of the present invention may further encompass a functionally active fragment thereof, without being limited to human and mammalian forms, conservative amino acid substitutions, glycoform modifications or variants, active fragments thereof, or substances comprising the functionally active fragment produced after processing and/or modification occurring in cells. For example, the GTPase-activating protein 1 family member of the present invention may comprise a functionally active fragment thereof and any other domains.

In the present invention, the term "FGF-binding protein family member" generally refers to a family member protein having an FGF-binding domain or a functionally active fragment thereof. For example, the FGF-binding protein family member may comprise FIBP. For example, the UniProt accession number of the FGF-binding protein family member may be O43427. The FGF-binding protein family member of the present invention may further encompass a functionally active fragment thereof, without being limited to human and mammalian forms, conservative amino acid substitutions, glycoform modifications or variants, active fragments thereof, or substances comprising the functionally active fragment produced after processing and/or modification occurring in cells. For example, the FGF-binding protein family member of the present invention may comprise a functionally active fragment thereof and any other domains.

In the present invention, the term "Mediator (MED) family member" generally refers to a family member protein having a CDK8-binding domain or a functionally active fragment thereof. For example, the Mediator (MED) family member may comprise MED12. For example, the UniProt accession number of the Mediator (MED) family member may be Q93074. The Mediator (MED) family member of the present invention may further encompass a functionally active fragment thereof, without being limited to human and mammalian forms, conservative amino acid substitutions, glycoform modifications or variants, active fragments thereof, or substances comprising the functionally active fragment produced after processing and/or modification occurring in cells. For example, the Mediator (MED) family member of the present invention may comprise a functionally active fragment thereof and any other domains.

In the present invention, the term "immune cell" generally refers to a cell participating in innate and adaptive immune responses. For example, immune cells may include, but are not limited to, lymphocytes (such as T cells (including thymocytes) and B cells), natural killer (NK) cells, NKT cells, macrophages, monocytes, eosinophils, basophils, neutrophils, dendritic cells, and mast cells. In some embodiments, the modified immune effector cell is a T cell, such as a CD4+ T cell, a CD8+ T cell (also referred to as a cytotoxic T cell or CTL), a regulatory T cell (Treg), a Th1 cell, a Th2 cell, a Th17 cell, an αβ T cell, and/or a γδ T cell. For example, the immune cells of the present invention further comprise immune cells differentiated from stem cells. For example, the immune cells of the present invention further comprise immune cells differentiated from pluripotent stem cells. For example, the stem cells of the present invention may be obtained by induction. For example, the above stem cells of the present invention may comprise induced pluripotent stem cells (iPSC).

In the present invention, the term "chimeric antigen receptor" generally refers to an engineered antigen receptor. For example, a CAR may comprise an extracellular antigen-binding domain fused, via a hinge and a transmembrane domain, to a cytoplasmic domain comprising a signal transduction domain. In some embodiments, the extracellular domain of the CAR is capable of binding, in an MHC-independent manner, to an antigen expressed by a target cell, thereby causing activation and proliferation of the cell. In some embodiments, the extracellular domain of the CAR is capable of recognizing a tag fused to an antibody or an antigen-binding fragment thereof. For example, a single CAR construct is enabled to target a plurality of different antigens by replacing one antibody with another antibody. In some embodiments, the extracellular domain of the CAR may comprise an antigen-binding fragment derived from an antibody. The antigen-binding domain for use in the present invention may include, for example, an scFv, an antibody, an antigen-binding region of an antibody, a variable region of a heavy chain/light chain, and/or a single-chain antibody.

In the present invention, the term "T cell receptor" generally refers to an engineered antigen receptor. For example, a TCR may comprise a TCRα and/or TCRβ chain isolated and cloned from a T cell population recognizing a specific target antigen. For example, the TCRα and/or TCRβ genes (i.e., TRAC and TRBC) may be cloned from a T cell population isolated from an individual suffering from a specific malignancy, or may be cloned from a T cell population isolated from a humanized mouse immunized with a specific tumor antigen or tumor cell. The engineered TCR is capable of recognizing an antigen via the same mechanism as its endogenous counterpart (e.g., by recognizing its cognate antigen presented in the context of a major histocompatibility complex (MHC) protein expressed on the surface of a target cell), thereby causing activation and proliferation of the TCR-engineered cell.

In the present invention, the term "gene regulation system" generally refers to a system that regulates expression or activity of a target gene. For example, the gene regulation system may comprise a gene regulatory molecule. For example, expression or activity of a gene may be regulated by the gene regulation system, such as placing the gene in an inactivated or activated state, increasing or decreasing the copy number of the gene, placing the gene in a state of increased or decreased transcription level, and/or placing a transcription product of the gene in an inactivated or activated state; for example, expression or activity of a gene may be regulated by the gene regulation system, such as increasing or decreasing an amount of an expression product of the gene in a single cell and/or increasing or decreasing a number of cells expressing the expression product of the gene.

In the present invention, the term "guide nucleic acid molecule" generally refers to a nucleic acid molecule that is usable for gene editing. For example, information for nucleotide insertion or deletion may be provided by the guide nucleic acid molecule to guide an editing process. For example, the guide nucleic acid molecule may be a guide RNA or a guide RNA (guide RNA, gRNA). For example, "gRNA" may refer to an RNA molecule that binds to a Cas protein and targets the Cas protein to a specific position within a target DNA. For example, formation of a CRISPR complex is promoted by hybridization between the gRNA and a DNA targeting sequence, and complete complementarity is not necessarily required, for example, as long as sufficient complementarity exists to cause hybridization and promote formation of the CRISPR complex.

In the present invention, the term "enzyme protein" generally refers to a protein having enzymatic activity. For example, the enzyme protein may refer to a Cas protein. For example, the Cas protein may comprise at least one RNA recognition or binding domain, and the domain may interact with gRNA. The Cas protein may further comprise a nuclease domain (e.g., a DNase or RNase domain), a DNA-binding domain, a helicase domain, a protein-protein interaction domain, a dimerization domain, and/or other domains. The nuclease domain may have catalytic activity for nucleic acid cleavage. The cleavage may comprise breakage of a covalent bond of a nucleic acid molecule. The Cas protein may be a wild-type protein (i.e., a naturally occurring protein), a modified Cas protein (i.e., a Cas protein variant), or a fragment of a wild-type or modified Cas protein. The Cas protein may further be an active variant or fragment of a wild-type or modified Cas protein. In the present invention, the Cas protein may encompass an unprocessed Cas protein, a Cas protein processed in any form, a variant of a Cas protein, or a substance comprising a functionally active fragment of a Cas protein.

In the present invention, the term "ribonucleoprotein complex" generally refers to a complex formed by a protein and a nucleic acid. For example, the protein in the ribonucleoprotein complex may have nuclease activity. For example, the ribonucleoprotein complex may, under the guidance of the nucleic acid therein, cleave a target sequence. For example, the ribonucleoprotein complex may be a complex formed by a Cas protein and gRNA.

In the present invention, the term "lipid nanoparticle (LNP)" generally refers to a lipid-nucleic acid particle or a nucleic acid-lipid particle. For example, an LNP denotes a particle made of lipids (e.g., cationic lipids, non-cationic lipids, and conjugated lipids that prevent particle aggregation) and nucleic acids, wherein the nucleic acids (e.g., mRNA, gRNA, siRNA, aiRNA, miRNA, ssDNA, dsDNA, ssRNA, short hairpin RNA (shRNA), dsRNA, self-amplifying RNA, or plasmids, including plasmids from which interfering RNA or mRNA is transcribed) are encapsulated in the lipids. For example, a protein may be encapsulated in an LNP; for example, a Cas protein known in the art may be encapsulated in an LNP. For example, the lipids in the LNP comprise (1) "simple lipids", which comprise fats and oils and waxes; (2) "compound lipids", which comprise phospholipids and glycolipids; and (3) "derived lipids" such as steroids. For example, the lipids in the LNP may also comprise lipid derivatives, such as lipids covalently or non-covalently bound to a protein or polypeptide. For example, the components in the LNP may further comprise a polypeptide component, wherein the polypeptide component may replace one or more lipid components in a conventional LNP while maintaining or improving the delivery capability of the LNP.

In the present invention, the term "exon" generally refers to a portion of a gene that is capable of being expressed as a protein. For example, an exon may refer to a portion that has the capability of being expressed as a protein during protein biosynthesis. For example, cleavage of an exon sequence of a target gene may reduce the activity or function of the target gene.

In the present invention, the term "intron" generally refers to a segment in DNA that does not encode all or part of an expressed protein. Typically, under endogenous conditions, an intron is transcribed into an RNA molecule, but is spliced out from endogenous RNA before being translated into a protein. For example, editing by targeting a position in an intron may reduce the activity or function of a target gene. For example, editing by targeting an intron region at an intron-exon junction, such as an intron region about 0 bp to about 100 bp, preferably about 0 bp to about 20 bp, upstream or downstream of an exon, may reduce the activity or function of the target gene.

In the present invention, the term "start codon" generally refers to a unit ("codon") of adjacent nucleotides on a gene that defines the initiation of protein synthesis (mRNA translation). For example, editing by targeting a region 0 bp to 1500 bp upstream of a start codon, preferably a region 0 bp to 100 bp upstream of a start codon, may reduce the activity or function of a target gene.

In the present invention, the term "protospacer adjacent motif (PAM)" generally refers to a short sequence downstream of a target sequence. For example, when site-specific cleavage of target DNA is performed by Cas9, the PAM sequence may be used to determine the cleavage position. For example, once a PAM region is determined, a person skilled in the art may readily determine a suitable target sequence position, and may readily design a gRNA sequence for cleaving the target sequence.

In the present invention, the term "reduced expression" generally refers to a reduction in the expression level of a product or its gene and/or a decrease in the proportion of cells capable of expressing the product (e.g., at least about 5-100%). For example, reduced expression may be a reduction in the amount of the product expressed by the gene in cells, or a reduction in the proportion of cells comprising the product expressed by the gene, or a reduction in the proportion of cells secreting the product expressed by the gene. For example, reduced expression of the gene may be indirectly indicated by detecting the knockout amount of the gene in the genome of cells. For example, reduced expression of the gene may be indirectly indicated by detecting the proportion of cells in which the gene is knocked out in a cell population.

In the present invention, the term "activity" generally refers to the biological function of a substance. For example, the activity of a gene may refer to the transcription and/or translation status of the gene. For example, reduced activity of a gene (e.g., at least about 5-100%) may refer to reduced transcriptional function of the gene, inability of the gene to be normally transcribed, or inhibition of the function of a transcription product of the gene.

In the present invention, the term "CD80" generally refers to a costimulatory molecule. For example, CD80 may be a ligand of CD28. For example, CD80 may be found under GenBank accession number P33681. The CD80 protein of the present invention may further encompass a functionally active fragment thereof, without limitation to a substance comprising a functionally active fragment of CD80 produced after processing and/or modification occurring in cells. For example, the CD80 of the present invention may comprise a functionally active fragment of CD80 and any other domain.

In the present invention, the term "CD86" generally refers to a costimulatory molecule. For example, CD86 may be a ligand of CD28. For example, CD86 may be found under GenBank accession number P42081. The CD86 protein of the present invention may further encompass a functionally active fragment thereof, without limitation to a substance comprising a functionally active fragment of CD86 produced after processing and/or modification occurring in cells. For example, the CD86 of the present invention may comprise a functionally active fragment of CD86 and any other domain.

In the present invention, the term "secretion" generally refers to a substance being capable of being localized extracellularly of a cell. For example, a secreted substance may, after being synthesized intracellularly, be transported to the extracellular space of the cell. For example, whether a substance is a secreted substance may be detected by enzyme-linked immunosorbent assay or other detection methods.

In the present invention, the term "T cell receptor" or "TCR" generally refers to a complex of membrane proteins that participates in activation of T cells in response to antigen presentation. An antigen bound to a major histocompatibility complex molecule may be recognized by the TCR. The TCR may be composed of a heterodimer of alpha (α) and beta (β) chains, or may be composed of gamma and delta (γ/δ) chains. The TCR may exist in α/β and γ/δ forms, which are structurally similar but have distinct anatomical locations and functions. For example, the TCR may be a TCR modified on any cell expressing the TCR. For example, the types of TCR may be analyzed using a TCR subtype analysis reagent.

In the present invention, the term "clonal diversity" generally refers to a substance having multiple clonotypes. For example, the clonal diversity of a TCR may refer to the TCR having different sequence structures and/or antigen recognition capabilities. For example, the diversity of a TCR is commonly distinguished by β-chain subtypes, which may include Vβ 23, Vβ 7.2, Vβ 5.2, Vβ 11, Vβ 16, Vβ 3, and the like; when a T cell population has more β-chain subtypes, the T cell population may be considered to have higher clonal diversity.

In the present invention, "CD4⁺ cells" generally refer to CD4-positive cells, for example, T cells. The terms "CD4⁺ cells" and "CD4-positive cells" are used interchangeably. Such cells may be identified by methods known in the art, for example, by staining cells with a fluorescently labeled anti-CD4 antibody and using fluorescence-activated cell sorting. For example, existing data may demonstrate that an increased proportion of CD4⁺ cells may increase the ability of a cell population to secrete IFN and/or TNF, and may improve the tumor suppression-promoting effect of a T cell population. For example, see Tay, R. E., Richardson, E. K. et al. (2020). Cancer Gene Therapy, 1-13. However, a method for increasing the proportion of CD4⁺ cells is lacking in the art, and the present invention may provide a method for affecting the proportion of CD4⁺ cells.

In the present invention, "CD8⁺ cells" generally refer to CD8-positive cells, for example, T cells. The terms "CD8⁺ cells" and "CD8-positive cells" are used interchangeably. Such cells may be identified by methods known in the art, for example, by staining cells with a fluorescently labeled anti-CD8 antibody and using fluorescence-activated cell sorting.

In the present invention, the term "IC₅₀ value" or "IC50 value" generally refers to the concentration required for a target to achieve 50% inhibition of a biological process. The IC50 value may be converted into an absolute inhibition constant (Ki) using the Cheng-Prusoff equation (Biochem.Pharmacol.(1973)22:3099).

In the present invention, the term "K_{D} value" or "KD value" generally refers to a dissociation constant, which may be determined by surface plasmon resonance. Typically, surface plasmon resonance analysis is performed using a BIAcore system (Pharmacia Biosensor, Piscataway, NJ), and real-time binding interactions between a ligand (a substance immobilized on a biosensor matrix) and an analyte (a substance in solution) are measured by surface plasmon resonance (SPR). Surface plasmon analysis may also be performed by immobilizing an analyte (a substance on a biosensor matrix) and presenting a ligand.

In the present invention, the term "encode" generally refers to being able, according to substantially defined rules, to directly or indirectly infer structural or compositional information of another type of molecule related thereto from structural or compositional information of one type of molecule. For example, a nucleotide sequence may be inferred from an amino acid sequence; for example, such inference may be based on the properties of deoxyribonucleic acid in transcribing complementary nucleic acid, including nucleic acids that can be translated into polypeptides. For example, deoxyribonucleic acid may encode RNA transcribed from deoxyribonucleic acid. Deoxyribonucleic acid may similarly encode a polypeptide translated from RNA transcribed from deoxyribonucleic acid.

In the present invention, the term "small molecule compound" generally refers to peptides, peptidomimetics, amino acids, amino acid analogs, polynucleotides, polynucleotide analogs, nucleotides, nucleotide analogs, organic or inorganic substances having a molecular weight of less than about 10,000 g/mol (i.e., including heteroorganic substances and organometallic compounds), organic or inorganic substances having a molecular weight of less than about 5,000 g/mol, organic or inorganic substances having a molecular weight of less than about 1,000 g/mol, organic or inorganic substances having a molecular weight of less than about 500 g/mol, and salts, esters, and other pharmaceutically acceptable forms of such drugs.

In the present invention, the term "NK cell", also referred to as "natural killer cell", generally refers to a cell having large granules in the cytoplasm. NK cells are developed from bone marrow lymphoid stem cells, and differentiation and development are dependent on a bone marrow or thymic microenvironment. In the present invention, the proportion of NK cells in TIL cells is altered by the method of the present invention.

In the present invention, the term "antibody" generally refers to an immunoglobulin, or a fragment or derivative thereof, and encompasses any polypeptide comprising an antigen-binding site, whether produced in vitro or in vivo. The term includes, but is not limited to, polyclonal, monoclonal, monospecific, multispecific, nonspecific, humanized, single-chain, chimeric, synthetic, recombinant, hybrid, mutated, and grafted antibodies. Unless otherwise modified by the term "intact", as in "intact antibody", for purposes of the present invention, the term "antibody" also includes antibody fragments, such as Fab, F(ab')₂, Fv, scFv, Fd, dAb, and other antibody fragments that retain antigen-binding function (e.g., specifically binding CD3). Generally, such fragments should comprise an antigen-binding domain. The basic 4-chain antibody unit is a heterotetrameric glycoprotein composed of two identical light (L) chains and two identical heavy (H) chains. IgM antibodies are composed of five basic heterotetrameric units and an additional polypeptide referred to as a J chain, and contain 10 antigen-binding sites, whereas IgA antibodies comprise 2-5 basic 4-chain units that can bind to a J chain and polymerize to form multivalent combinations. For IgG, the 4-chain unit is generally about 150,000 daltons. Each L chain is linked to an H chain by a covalent disulfide bond, and the two H chains are linked to each other by one or more disulfide bonds depending on the H chain isotype. Each H chain and each L chain also has regularly spaced intrachain disulfide bridges. Each H chain has a variable domain (VH) at the N-terminus, which is followed by three constant domains (CH) for α and γ chains, respectively, and is followed by four CH domains for µ and ε isotypes. Each L chain has a variable domain (VL) at the N-terminus and has a constant domain at the other end. VL corresponds to VH, and CL corresponds to the first constant domain (CH1) of the heavy chain. Certain amino acid residues are considered to form an interface between the light-chain and heavy-chain variable domains. VH and VL are paired together to form a single antigen-binding site. L chains from any vertebrate species are classified into one of two clearly distinct types, referred to as κ and λ, based on the amino acid sequence of their constant domains. Based on the amino acid sequence of the heavy chain constant domains (CH), immunoglobulins are divided into different classes or isotypes. There are currently five classes of immunoglobulins: IgA, IgD, IgE, IgG, and IgM, having heavy chains named α, δ, ε, γ, and µ, respectively.

In the present invention, the term "antigen-binding fragment" generally refers to one or more polypeptide fragments having the capability of specifically binding an antigen. In the present invention, the antigen-binding fragment can comprise Fab, Fab', F(ab)₂, Fv fragments, F(ab')₂, scFv, di-scFv, and/or dAb.

In the present invention, the term "expression" generally refers to a transcription and/or translation process of a gene encoding a target polypeptide in a cell. A transcription level of the gene encoding the target polypeptide in a host cell is determined by measuring an amount of the corresponding mRNA present in the cell. For example, quantitative measurement of the mRNA transcribed from the gene encoding the target polypeptide is performed by PCR or by RNA hybridization. A translation level of the gene encoding the target polypeptide is measured by a variety of methods, for example, by ELISA, by a polypeptide bioactivity assay, or by protein blotting or radioimmunoassay. In the present invention, the term "expression" also generally refers to a transcription and/or translation process of a product. For example, expression of a cytokine is a process by which a cell transcribes and/or translates the cytokine. For example, expression of a cytokine is determined by detecting an amount of the corresponding mRNA present in the cell or detecting an amount of the cytokine produced by the cell, or both.

In the present invention, the "stage" in the terms "one stage in vitro expansion", "single stage in vitro expansion", or "first stage in vitro expansion", and the like, generally refers to a period of expansion of TIL in vitro. In one embodiment, each stage is divided based on a change in the number of TIL cells. In one embodiment, when the number of TIL cells increases by at least about 1-fold, the TIL cells are considered to enter a next stage of in vitro expansion. In some embodiments, when the number of TIL cells increases by at least about 1-50 fold, for example, at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold, the TIL cells are considered to enter a next stage of in vitro expansion. In one embodiment, each stage is also divided based on culture conditions of the TIL cells. In one embodiment, when a T cell activator and/or a T cell growth factor is added or supplemented in a cell culture medium, the TIL cells are considered to enter a next stage of in vitro expansion. In one embodiment, when the TIL cells are subjected to centrifugation and/or cell washing, the TIL cells are considered to enter a next stage of in vitro expansion. In one embodiment, each stage is also divided based on the number of days of culture of the TIL cells. In one embodiment, when the TIL cells are cultured in vitro for about 1-100 days, for example, about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 30 days, about 40 days, about 50 days, or about 100 days, the TIL cells are considered to enter a next stage of in vitro expansion.

In the present invention, the term "first stage in vitro expansion" generally refers to a stage in which primary TIL obtained from tissue are expanded using a T cell growth factor. In one embodiment, the tissue of the present invention is selected from the group consisting of tumor tissue, tumor-associated lymph nodes with or without tumor metastasis, tumor metastatic lesions, fragments of paracancerous tissue, pleural effusion and/or ascites, and the pleural effusion of the present invention is pleural effusion from a patient with metastatic cancer. In one embodiment, the expansion of the present invention is autologous or allogeneic in vivo expansion, or is in vitro expansion. The first stage in vitro expansion of the present invention is also referred to as a preREP (pre-rapid expansion) stage. For example, TIL derived from tumor tissue and not subjected to in vitro expansion are referred to as a first TIL population. For example, in a culture mode of the present invention divided by a two-step method, TIL obtained after the first stage in vitro expansion are referred to as a second TIL population.

In the present invention, the term "second stage in vitro expansion" generally refers to a stage in which tissue taken from a subject and expanded is expanded again. In one embodiment, compared with TIL subjected to the first stage in vitro expansion, the number of TIL cells subjected to the second stage in vitro expansion of the present invention is increased; for example, the number is increased by at least about 10-fold (or at least about 20-, 30-, 40-, 50-, 60-, 70-, 80- or 90-fold), or, in one embodiment, the number of cells is increased by at least about 100-fold. In one embodiment, the second stage in vitro expansion has culture conditions different from those of the first stage in vitro expansion; for example, added culture substances are different. For example, in a culture mode of the present invention divided by a two-step method, the second stage in vitro expansion is also referred to as a REP (rapid expansion) stage. For example, in a culture mode of the present invention divided by a two-step method, TIL obtained after the second stage in vitro expansion are referred to as a third TIL population.

In the present invention, the term "in vivo" generally refers to an event occurring in a subject.

In the present invention, the term "in vitro" generally refers to an event occurring outside a subject.

In the present invention, the term "ex vivo" generally refers to an event involving treatment of, or a procedure performed on, cells, tissues, and/or organs that are removed from a subject. In one embodiment, the cells, tissues, and/or organs are returned to the body of the subject by surgery or a therapeutic method.

In the present invention, the term "secretion capability" generally refers to a capability of a cell to express a polypeptide or protein and to transfer the polypeptide or protein of the present invention to an extracellular environment.

In the present invention, the term "irradiation" generally refers to treatment of a substance by radiation. For example, in one embodiment, irradiation refers to irradiating a substance with X-rays, α-rays, β-rays, or γ-rays.

In the present invention, the term "engineered cell" generally refers to a genetically modified cell in which additional genetic material in the form of DNA or RNA is added to the total genetic material of the cell. In one embodiment, the engineered cell is a TIL genetically modified to express the T cell activator and/or the T cell growth factor of the present invention.

In the present invention, the term "co-culture" generally refers to culturing two or more different populations of cells under conditions in which there is a certain degree of contact between them. In the present invention, the "contact" between two or more different populations of cells is, in one embodiment, direct contact, i.e., cells of one population are in direct physical contact with cells of another population. Alternatively, in one embodiment, the contact is indirect contact mediated by sharing a culture medium. The shared culture medium of the present invention contains metabolites produced and released by at least one population of the co-cultured cells, and is used to culture another population of cells.

In the present invention, the term "contact" generally refers to two or more different types of substances being brought into contact with each other in any order, in any manner, and for any duration. In one embodiment, contact is achieved by direct contact; for example, one or more feeder cells, a T cell activator, and/or a T cell growth factor are added to a culture medium of TIL cells; for example, a culture medium comprising one or more feeder cells, a T cell activator, and/or a T cell growth factor is added to and/or used to replace the culture medium of TIL cells; for example, a culture medium comprising one or more feeder cells, a T cell activator, and/or a T cell growth factor is used for culturing TIL cells. In one embodiment, contact is achieved by indirect contact; for example, metabolites produced and released by feeder cells are used for culturing TIL cells.

In the present invention, the terms "simultaneous contact", "co-contact", "contact with ... simultaneously", "simultaneously", and "co-" generally refer to administering two or more substances to a subject and/or cells such that the substances are simultaneously present in the environment of the subject and/or cell culture. Simultaneous contact includes simultaneous administration in different compositions, administration in different compositions at different times, or administration in a composition in which two or more active pharmaceutical ingredients are present. For example, "simultaneous contact" in the present invention generally refers to substantially simultaneous contact.

In the present invention, the term "expansion" generally refers to an increase in the number of cells by several fold over a period of time. In one embodiment, the number of cells is increased by at least about 3-fold (or 4-, 5-, 6-, 7-, 8-, or 9-fold); in one embodiment, the number of cells is increased by at least about 10-fold (or 20-, 30-, 40-, 50-, 60-, 70-, 80-, or 90-fold); or in one embodiment, the number of cells is increased by at least about 100-fold. In the present invention, the term "expanded" generally means that the cells of the present invention have undergone one or more of the expansions described above.

In the present invention, the term "polymer" generally refers to a molecule composed of individual chemical moieties linked together, wherein the polymer moieties of the present invention are the same or different. In one embodiment, the term "polymer" refers to individual chemical moieties linked end-to-end to form a linear molecule, and individual chemical moieties linked together in a branched (e.g., "multi-arm" or "star") structure. In one embodiment, the polymer includes, for example, a polysaccharide, dextran, a hydrogel, polyethylene glycol, or poloxamer. Poloxamer is a nonionic triblock copolymer having a central hydrophobic polyoxypropylene (poly(propylene oxide)) chain with two polyoxyethylene (poly(ethylene oxide)) hydrophilic chains attached thereto. The substances comprised in the present invention are formulated with, or administered together with, any polymer described herein or known in the art.

In the present invention, the term "chimeric antibody" generally refers to an antibody formed by fusing a variable region of a murine antibody with a constant region of a human antibody, thereby reducing an immune response induced by the murine antibody. To establish a chimeric antibody, a hybridoma secreting a murine-specific monoclonal antibody is established, and then a variable region gene is cloned from murine hybridoma cells; as needed, a constant region gene of a human antibody is cloned; after the murine variable region gene and the human constant region gene are linked to form a chimeric gene, the chimeric gene is inserted into an expression vector, and the chimeric antibody molecule is expressed in a eukaryotic system or a prokaryotic system.

In the present invention, the term "humanized antibody", also referred to as a CDR-grafted antibody, generally refers to an antibody produced by grafting murine CDR sequences into a human antibody variable region framework, i.e., into framework sequences of different types of human germline antibodies. A heterologous reaction induced by a chimeric antibody due to carrying a large amount of murine protein components is overcome. Such framework sequences are obtained from public DNA databases comprising germline antibody gene sequences or from published references. For example, germline DNA sequences of human heavy chain and light chain variable region genes are found in the "VBase" human germline sequence database.

In the present invention, the terms "fully human antibody", "human antibody", or "completely human antibody", also referred to as a "fully human monoclonal antibody", generally mean that both the variable region and the constant region of the antibody are of human origin, thereby removing immunogenicity and toxic side effects. The development of monoclonal antibodies has undergone four stages, namely: murine monoclonal antibodies, chimeric monoclonal antibodies, humanized monoclonal antibodies, and fully human monoclonal antibodies. The antibody or ligand described in the present invention is a fully human monoclonal antibody. Related techniques for preparing fully human antibodies include human hybridoma technology, EBV-transformed B lymphocyte technology, phage display technology, transgenic mouse antibody preparation technology, and single B cell antibody preparation technology, among others.

In the present invention, the term "CDR" generally refers to one of six hypervariable regions within the variable domains of an antibody that primarily contribute to antigen binding. One of the most commonly used definitions of the six CDRs is provided by Kabat E.A. et al., Chothia et al., and MacCallum et al. As used in the present invention, the Kabat definition of CDRs is applied to CDR1, CDR2, and CDR3 of the light chain variable domain (CDR L1, CDR L2, CDR L3, or L1, L2, L3), and CDR1, CDR2, and CDR3 of the heavy chain variable domain (CDR H1, CDR H2, CDR H3, or H1, H2, H3).

In the present invention, the term "IL-2" or "IL2" generally refers to a T cell growth factor called interleukin 2, and includes all forms of IL-2, which include, in one embodiment, human and mammalian forms, conservative amino acid substitutions, glycoform modifications or variants, or active fragments thereof. The GeneID encoding the IL-2 gene is 3558.

In the present invention, the terms "antigen-presenting cell", "antigen presenting cell", or "APC" generally refer to an immune system cell that displays, on its surface, an exogenous antigen complexed with a major histocompatibility complex (MHC), such as a helper cell (e.g., a B cell, a dendritic cell, etc.). T cells recognize these complexes using their T cell receptor (TCR). APCs process antigens and present them to T cells. In one embodiment, the antigen-presenting cell includes those selected from the group consisting of peripheral blood mononuclear cells, dendritic cells, and artificial antigen-presenting cells.

In the present invention, the term "TIL characteristics" generally refers to characteristics obtained by TIL cells through the culture method of the present invention. Changes in TIL characteristics include an increased number of TIL cells, an increased proportion of viable cells, increased persistence, an improved proportion of T cell subsets, enhanced cytokine secretion capability, enhanced in vitro tumor cell killing capability, enhanced in vivo tumor killing capability, increased T cell receptor (TCR) clonal diversity, and an increased number of TIL cells in tissues, or any combination thereof. The changes of the present invention are an increase or a decrease.

In the present invention, the term "persistence" generally refers to the presence of cells in vitro and/or in a subject. For example, an increase in persistence of TIL cells refers to an increase in the time during which TIL cells are present in vivo. For example, increased persistence refers to an increase in the time during which cells are present in subject tissues, such as tumors, spleen, bone marrow, lung tissue, and blood. For example, increased persistence is an increase in persistence of TIL cells after IL-2 is withdrawn from the culture medium.

In the present invention, the term "artificial antigen-presenting cell" generally refers to an artificially constructed immune cell for presenting an exogenous antigen; for example, the manner of presenting an exogenous antigen is that the surface of the artificial antigen-presenting cell comprises a complex of the exogenous antigen and a major histocompatibility complex (MHC). In one embodiment, isolated artificial antigen-presenting cells (aAPCs) are included, which comprise cells expressing HLA-A/B/C (the GeneID encoding the same is 3105, 3106, or 3107), CD64 (the GeneID encoding the same is 2209), CD80 (the GeneID encoding the same is 941), ICOS-L (the GeneID encoding the same is 23308), and CD58 (the GeneID encoding the same is 965), and are modified to express one or more T cell activators.

In the present invention, the term "fusion protein" generally refers to a polypeptide or protein comprising an amino acid sequence of a first polypeptide or protein or a fragment, analog, or derivative thereof, and an amino acid sequence of a heterologous polypeptide or protein (i.e., a second polypeptide or protein or a fragment, analog, or derivative thereof that is different from the first polypeptide or protein or the fragment, analog, or derivative thereof, or that is generally not a part of the first polypeptide or protein or the fragment, analog, or derivative thereof). In certain cases, the fusion protein comprises a prophylactic or therapeutic agent fused to a heterologous protein, polypeptide, or peptide. Therein, the heterologous protein, polypeptide, or peptide of the present invention is or is not a different type of prophylactic or therapeutic agent. For example, two different proteins, polypeptides, or peptides having immunomodulatory activity are fused together to form a fusion protein. In certain cases, compared with the activity of the initial polypeptide or protein prior to fusion with the heterologous protein, polypeptide, or protein, the fusion protein retains or enhances activity.

In the present invention, the term "killing capability" generally refers to killing target cells by bringing the cells of the present invention into contact with an effective amount of a substance, thereby achieving killing of the target cells. In one embodiment, the substance of the present invention is TIL cells. The killing of the present invention includes killing cells by itself or by promoting CDC, apoptosis, ADCC, and/or phagocytosis of other cells or substances, or by a combination of two or more of these mechanisms.

In the present invention, the term "administration" or "administering" generally refers to delivering a substance to a subject in need thereof via any route known in the art. Pharmaceutical carriers and formulations or compositions are also well known in the art. Routes of administration include: intravenous, intramuscular, intradermal, subcutaneous, transdermal, mucosal, intratumoral, and/or mucosal.

In the present invention, the term "kit" generally refers to two or more components packaged together in a container, receptacle, or other container, wherein one corresponds to a substance of the present invention. For example, the kit comprises the TIL cells of the present invention.

In the present invention, the term "subject" generally refers to a cell or an animal, which is a mammal, such as a human, a non-human primate (ape, gibbon, gorilla, chimpanzee, orangutan and macaque), a domestic animal (dog and cat), a farm animal (poultry such as chicken, duck, horse, cattle, goat, sheep and pig), and an experimental animal (mouse, rat, rabbit, and guinea pig). Human subjects include fetal, neonatal, infant, juvenile, and adult subjects. Subjects include animal disease models, such as tumor animal models, and other animal models known to those skilled in the art.

In the present invention, the term "feeder cell" generally refers to a cultured cell that is used to support the growth of another target cell in culture. For example, at least one factor is grown in vitro and secreted into a culture medium. In one embodiment, the feeder cell comprises an antigen-presenting cell.

In the present invention, the term "specific binding" generally refers to recognition of a specific target substance, but substantially no recognition of, or binding to, other molecules in a sample. For example, if a binding substance specifically binds to the specific target substance of the present invention from one species, the binding substance of the present invention is also capable of specifically binding to the target substance or a homologous target substance of the present invention from one or more other species. Such interspecies reactivity per se does not change the classification of the binding substance as specific. In certain cases, a binding substance that specifically binds to a target substance is also capable of binding to different allelic forms of the target substance.

In the present invention, the term "complete culture process" generally refers to a complete process that starts from isolation of cells from tumor tissue isolated from a patient, proceeds through one or more rounds of expansion, and finally obtains cells that are capable of being administered to a subject.

In the present invention, the term "cell culture medium" generally refers to a nutrient solution in which cells, such as mammalian cells, are grown. The preparation of cell culture media is well known in the art. Typically, a cell culture medium comprises a buffer, salts, carbohydrates, amino acids, vitamins, and necessary trace elements. The cell culture medium may contain, or may not contain, serum, peptone, and/or protein. The cell culture medium may be supplemented with additional components or components at increased concentrations, such as amino acids, salts, sugars, vitamins, hormones, growth factors, buffers, antibiotics, lipids, trace elements, and the like, depending on the requirements of the cells to be cultured and/or the desired cell culture parameters.

In the present invention, the term "pharmaceutical composition" or "pharmaceutical formulation" generally refers to a preparation, wherein the preparation of the present invention allows the bioactivity of an active ingredient to be effective and is free of additional components that are unacceptably toxic to a subject to whom the formulation is to be administered. Such formulations are sterile. "Pharmaceutically acceptable" excipients (carriers, additives) are those excipients that are reasonably administered to a test mammal to provide an effective dose of the active ingredient used.

In the present invention, the term "tumor-infiltrating lymphocyte" or "TIL" generally refers to a cell population that is initially obtained as leukocytes, wherein the cells of the present invention have left the bloodstream of a subject and migrated into a tumor. TIL can include, but are not limited to, CD8⁺ cytotoxic T cells (lymphocytes), Th1 and Th17 CD4⁺ T cells, natural killer cells, dendritic cells, and M1 macrophages. TIL can include primary TIL and secondary TIL. "Primary TIL" can be those TIL cells obtained from a subject tissue sample, and "secondary TIL" can be any TIL population that has been expanded or is expanded in the present invention. In some embodiments, the tumor-infiltrating lymphocytes of the present invention are not isolated and purified, or are mutually infiltrated with tumor cells. In one embodiment, the TIL of the present invention refers to a TIL population.

In the present invention, the term "central memory T cell" generally refers to a T cell that has long-term memory and is capable of receiving antigen restimulation. Central memory T cells can have a CD45RO⁺CD62L⁺ phenotype; for example, central memory T cells are identified by CD45RO⁺ and CD62L⁺. Central memory T cells can have a stronger ability to resist tumor growth as compared with conventional T cells.

In the present invention, the term "regulatory T cell" generally refers to a class of T-cell subsets that control autoimmune reactivity in vivo. Regulatory T cells can have a CD4⁺CD25⁺Foxp3⁺ phenotype; for example, regulatory T cells are identified by CD4⁺, CD25⁺, and Foxp3⁺. Regulatory T cells can have an ability to suppress the anti-tumor growth ability of T cells.

In the present invention, the term "activated T cell" generally refers to a T cell that has been activated and has an ability to resist tumor growth. Activated T cells can have a PD-1⁺ (PD1⁺), LAG-3⁺ (LAG3⁺), or CD28⁺ phenotype; for example, activated T cells are identified by PD-1⁺, LAG-3⁺, or CD28⁺. Activated T cells can have an ability to resist tumor growth.

In the present invention, the term "tumor-specific T cell" generally refers to a T cell that is capable of specifically resisting tumor growth. Tumor-specific T cells can have a CD103⁺CD39⁺ phenotype; for example, tumor-specific T cells are identified by CD103⁺ and CD39⁺. Tumor-specific T cells can have a more specific ability to resist tumor growth as compared with conventional T cells.

In the present invention, the term "stem cell-like T cell" generally refers to a class of T cells that has a potential for self-proliferation and/or differentiation. For example, in the present invention, cells having differentiation potential and/or sustained proliferative capacity are considered stem cell-like cells. For example, naive T cells (CD45RO⁻CD62L⁺) are considered stem cell-like cells. For example, naive T cells can have a CD45RO⁻CD62L⁺ phenotype. For example, stem cell-like T cells are identified by CD45RO⁻ and CD62L⁺. For example, stem cell-like T cells are identified by CD39⁻ and CD69⁻. For example, stem cell-like T cells can have a TCF1⁺ phenotype; for example, stem cell-like T cells are identified by TCF1⁺. Stem cell-like T cells can have a stronger and/or longer-term ability to resist tumor growth as compared with conventional T cells.

In the present invention, the term tumor "fragment" generally refers to a tumor fragment that is formed after tumor tissue is removed from a subject by mechanical disruption, enzymatic digestion, and/or other disruption methods.

In the present invention, the term "composition" or "pharmaceutical composition" generally refers to a mixture of at least one cell and at least one, and optionally more than one, other pharmaceutically acceptable chemical component, such as a carrier, stabilizer, diluent, dispersant, suspending agent, thickening agent, and/or excipient.

In the present invention, the term "pharmaceutically acceptable carrier" generally refers to one or more non-toxic materials that do not interfere with an active ingredient. For example, a pharmaceutically acceptable carrier does not interfere with the bioactivity of an active ingredient; for example, a pharmaceutically acceptable carrier does not interfere with the effectiveness of the bioactivity possessed by an active ingredient. Such formulations conventionally contain salts, buffers, preservatives, compatible carriers, and optionally other therapeutic agents. Such pharmaceutically acceptable carriers further contain compatible solid or liquid fillers, diluents, or encapsulating substances suitable for administration to humans. Other contemplated carriers, excipients, and/or additives that are used in the formulations described herein include, for example, flavoring agents, antimicrobial agents, sweetening agents, antioxidants, antistatic agents, lipids, protein excipients (such as serum albumin, gelatin, casein), salt-forming balance ions (such as sodium), and the like. These and other known pharmaceutical carriers, excipients, and/or additives suitable for use in the formulations described herein are known in the art. In the present invention, "pharmaceutically acceptable carrier" is understood to exclude a carrier in the form of a nucleic acid vector used in genetic engineering.

In the present invention, the term "functionally active fragment" generally refers to a fragment that has a partial region of a full-length protein or nucleic acid, but retains or partially retains the biological activity or function of the full-length protein or nucleic acid. For example, a functionally active fragment can retain or partially retain the ability of a full-length protein to bind to another molecule.

In the present invention, the term "T cell activator" generally refers to a substance that binds to a corresponding binding receptor on a T cell and mediates a T cell co-stimulatory response. A T cell activator may be a substance, other than an antigen receptor, that is required for a T cell to generate an effective immune response. A T cell activator may refer to a T cell co-stimulatory molecule. For example, the T cell activator of the present invention may comprise any substance that is a variant or homolog thereof, or that comprises a functionally active fragment thereof. The T cell activator may include, but is not limited to, MHC class I molecules, TNF receptor proteins, immunoglobulin-like proteins, cytokine receptors, integrins, signaling lymphocytic activation molecules (SLAM proteins), NK cell activating receptors, BTLA (the GeneID of the gene encoding the same may be 151888), Toll ligand receptors, OX40 (the GeneID of the gene encoding the same may be 7293), CD2 (the GeneID of the gene encoding the same may be 914), CD7 (the GeneID of the gene encoding the same may be 924), CD27 (the GeneID of the gene encoding the same may be 939), CD28 (the GeneID of the gene encoding the same may be 940), CD30 (the GeneID of the gene encoding the same may be 943), CD40 (the GeneID of the gene encoding the same may be 958), CDS, ICAM-1 (the GeneID of the gene encoding the same may be 3383), LFA-1 (CD11a/CD18) (the GeneID of the gene encoding the same may be 3689), 4-1BB (CD137) (the GeneID of the gene encoding the same may be 3604), B7-H3 (the GeneID of the gene encoding the same may be 80381), ICOS (CD278) (the GeneID of the gene encoding the same may be 29851), GITR (the GeneID of the gene encoding the same may be 8784), BAFFR (the GeneID of the gene encoding the same may be 115650), LIGHT (the GeneID of the gene encoding the same may be 8740), HVEM (LIGHTR) (the GeneID of the gene encoding the same may be 8764), KIRDS2 (the GeneID of the gene encoding the same may be 100132285), SLAMF7 (the GeneID of the gene encoding the same may be 57823), NKp80 (KLRF1) (the GeneID of the gene encoding the same may be 51348), NKp44 (the GeneID of the gene encoding the same may be 9436), NKp30 (the GeneID of the gene encoding the same may be 259197), NKp46 (the GeneID of the gene encoding the same may be 9437), CD19 (the GeneID of the gene encoding the same may be 930), CD4 (the GeneID of the gene encoding the same may be 920), CD8α (the GeneID of the gene encoding the same may be 925), CD8β (the GeneID of the gene encoding the same may be 926), IL-2Rβ, IL-2Rγ, IL7Rα (the GeneID of the gene encoding the same may be 3575), ITGA4 (the GeneID of the gene encoding the same may be 3676), VLA1 (the GeneID of the gene encoding the same may be 3672), CD49a (the GeneID of the gene encoding the same may be 3672), IA4 (the GeneID of the gene encoding the same may be 3732), CD49D (the GeneID of the gene encoding the same may be 3676), ITGA6 (the GeneID of the gene encoding the same may be 3655), VLA-6 (the GeneID of the gene encoding the same may be 3655), CD49f (the GeneID of the gene encoding the same may be 3655), ITGAD (the GeneID of the gene encoding the same may be 3681), CD11d (the GeneID of the gene encoding the same may be 3681), ITGAE (the GeneID of the gene encoding the same may be 3682), CD103 (the GeneID of the gene encoding the same may be 3682), ITGAL (the GeneID of the gene encoding the same may be 3683), CD11a (the GeneID of the gene encoding the same may be 3683), LFA-1 (the GeneID of the gene encoding the same may be 3683), ITGAM (the GeneID of the gene encoding the same may be 3684), CD11b (the GeneID of the gene encoding the same may be 3684), ITGAX (the GeneID of the gene encoding the same may be 3687), CD11c (the GeneID of the gene encoding the same may be 3687), ITGB1 (the GeneID of the gene encoding the same may be 3688), CD29 (the GeneID of the gene encoding the same may be 3688), ITGB2 (the GeneID of the gene encoding the same may be 3689), CD18 (the GeneID of the gene encoding the same may be 3689), LFA-1 (the GeneID of the gene encoding the same may be 3689), ITGB7 (the GeneID of the gene encoding the same may be 3695), NKG2D (the GeneID of the gene encoding the same may be 22914), NKG2C (the GeneID of the gene encoding the same may be 3822), TNFR2 (the GeneID of the gene encoding the same may be 7133), TRANCE/RANKL (the GeneID of the gene encoding the same may be 8600), DNAM1 (CD226) (the GeneID of the gene encoding the same may be 10666), SLAMF4 (CD244, 2B4) (the GeneID of the gene encoding the same may be 51744), CD84 (the GeneID of the gene encoding the same may be 8832), CD96 (Tactile) (the GeneID of the gene encoding the same may be 10225), CEACAM1 (the GeneID of the gene encoding the same may be 634), CRTAM (the GeneID of the gene encoding the same may be 56253), Ly9 (CD229) (the GeneID of the gene encoding the same may be 4063), CD160 (BY55) (the GeneID of the gene encoding the same may be 11126), PSGL1 (the GeneID of the gene encoding the same may be 6404), CD100 (SEMA4D) (the GeneID of the gene encoding the same may be 10507), CD69 (the GeneID of the gene encoding the same may be 969), SLAMF6 (NTB-A, Ly108) (the GeneID of the gene encoding the same may be 114836), SLAM (SLAMF1, CD150, IPO-3) (the GeneID of the gene encoding the same may be 6504), BLAME (SLAMF8) (the GeneID of the gene encoding the same may be 56833), SELPLG (CD162) (the GeneID of the gene encoding the same may be 6404), LTBR (the GeneID of the gene encoding the same may be 4055), LAT (the GeneID of the gene encoding the same may be 27040), GADS (the GeneID of the gene encoding the same may be 9402), SLP-76 (the GeneID of the gene encoding the same may be 3937), PAG/Cbp (the GeneID of the gene encoding the same may be 55824), CD19a, a ligand that specifically binds CD3, a ligand that specifically binds CD28, a ligand that specifically binds HVEM, a ligand that specifically binds CD40L, a ligand that specifically binds OX40, and a ligand that specifically binds 4-1BB. The co-stimulatory intracellular signal transduction domain may refer to an intracellular portion of the T cell activator. The intracellular signal transduction domain may comprise a complete intracellular portion of a molecule derived therefrom, a complete native intracellular signal transduction domain, or a functional fragment thereof.

In the present invention, the term "T cell growth factor" generally refers to a bioactive polypeptide or small-molecule compound that causes cell proliferation. For example, the T cell growth factor of the present invention may comprise any substance that is a variant or homolog thereof, or that comprises a functionally active fragment thereof. In one embodiment, the T cell growth factor may be selected from one or more of the group consisting of: IL-2 (the GeneID of the gene encoding the same may be 3558), IL-4 (the GeneID of the gene encoding the same may be 3565), IL-6 (the GeneID of the gene encoding the same may be 3569), IL-7 (the GeneID of the gene encoding the same may be 3574), IL-10 (the GeneID of the gene encoding the same may be 3586), IL-12 (the GeneID of the gene encoding the same may be 3592 or 3593), IL-15 (the GeneID of the gene encoding the same may be 3600), IL-21 (the GeneID of the gene encoding the same may be 59067), TNF-α (the GeneID of the gene encoding the same may be 100137091), interferon-γ (the GeneID of the gene encoding the same may be 3458), GZMB (the GeneID of the gene encoding the same may be 3002), CD107a (the GeneID of the gene encoding the same may be 6499), and the like.

In the present invention, the term "substantially simultaneously" generally means that, during a period of a contact process, TIL is capable of being in contact with two or more substances simultaneously, but it is not limited to that TIL is always in contact with two or more substances simultaneously throughout the entire contact process. In one embodiment, "substantially simultaneously" may mean that, during a period of time, TIL is capable of being in contact simultaneously with each substance of two or more substances for at least 10-95%, such as at least 10%, 20%, 30%, 40%, 50%, 60%, 70%, 75%, 80%, 85%, 90%, or 95%.

In the present invention, the term "dendritic cell" generally refers to an antigen-presenting cell that is present in vivo, in vitro, ex vivo, or within a host or subject, or that is capable of being derived from a hematopoietic stem cell or a monocyte. Dendritic cells and precursors thereof may be isolated from various lymphoid organs such as the spleen and lymph nodes, and from bone marrow and peripheral blood. The dendritic cell of the present invention may have a characteristic morphology, such as lamellae (lamellipodia) extending in multiple directions from the dendritic cell body. Generally, high levels of MHC and co-stimulatory (e.g., B7-1 and B7-2) molecules are capable of being expressed by dendritic cells. Antigen-specific differentiation of T cells is capable of being induced by dendritic cells in vitro, and primary T cell responses are capable of being elicited by dendritic cells in vitro and in vivo.

In the present invention, the term "in vitro expansion" generally refers to a change in the number of cells produced through culturing, and the expanded cells may also exhibit a change in cell number and/or proportion, a change in secretion capacity, a change in cytotoxic capacity, or a change in expression capacity, or any combination thereof. The change of the present invention may be an increase or a decrease. In the present invention, in vitro expansion may be for the purpose of expansion; for the purpose of detecting the function of TIL cells, for example, detecting the ability of TIL cells to release cytokines, an operational step performed on TIL cells (for example, adding one or more substances to a culture medium of TIL cells to detect the ability of TIL cells to release cytokines) may not belong to the in vitro expansion of the present invention.

In the present invention, the term "peripheral mononuclear cell" or "peripheral blood mononuclear cell" generally refers to a cell having a single nucleus in peripheral blood. For example, in the present invention, the peripheral blood mononuclear cell of the present invention may comprise lymphocytes, monocytes, and/or dendritic cells.

In the present invention, the term "cytokine" generally refers to a protein released by one group of cells that acts as an intercellular regulator on another cell. The cytokine of the present invention may be lymphokines, monokines, and polypeptide hormones. The cytokine of the present invention may comprise interleukins (ILs) such as IL-1, IL-1α, IL-2, IL-3, IL-4, IL-5, IL-6, IL-7, IL-8, IL-9, IL-10, IL-11, IL-15, IL-21, and/or IL-12. In the present invention, the term "cytokine" may comprise proteins from natural sources or from recombinant cell cultures, bioactive equivalents of naturally sequenced cytokines, and functionally active fragments thereof.

In the present invention, the term "diameter" generally refers to the diameter of a cross section of a substance of the present invention. For example, when the substance of the present invention is not spherical, the term "diameter" generally refers to the maximum diameter and/or the average diameter of the maximum cross section of the substance of the present invention. A method for determining the diameter of a substance may be a method commonly used in the art, such as transmission electron microscopy.

In the present invention, the term "tumor" generally refers to any new pathological tissue hyperplasia. The tumor of the present invention may be benign or malignant. The tumor of the present invention may be solid or hematological. The term "tumor" may be selected from one or more of the group consisting of melanoma, ovarian cancer, cervical cancer, lung cancer, bladder cancer, breast cancer, head and neck cancer, pancreatic cancer, liver cancer, gastric cancer, colorectal cancer, and renal cancer.

In the present invention, the term "tumor tissue" generally refers to a tumor derived from a subject, including a sample of any tissue of any solid tumor and/or non-solid tumor in the subject.

In the present invention, the term "T cell subset proportion" generally refers to a proportion of different T cell subsets in TIL cells or in a TIL population. For example, different T cell subsets of the present invention may have different immune activity and/or differentiation capability. For example, the T cell subsets of the present invention may be distinguished according to T cell surface markers. For example, central memory T cells may have a phenotype of CD45RO⁺CD62L⁺. For example, naïve T cells may have a phenotype of CD45RO⁻CD62L⁺. For example, regulatory T cells may have a phenotype of CD4⁺CD25⁺Foxp3⁺. For example, activated T cells may have a phenotype of CD25⁺, CD28⁺, PD-1⁺, or 41BB⁺. For example, tumor-specific T cells may have a phenotype of CD103⁺CD39⁺. For example, stem cell-like T cells may have a phenotype of TCF1⁺.

In the present invention, the term "TIL cell number" generally refers to the number of cells in the TIL cells of the present invention. In the present invention, the TIL cell number may refer to the number of cells in a TIL population obtained at any stage of the present invention. For example, the TIL cell number may refer to the number of cells in a first TIL population derived from tumor tissue and not subjected to in vitro expansion. For example, the TIL cell number may refer to the number of cells in a second TIL population subjected to first stage in vitro expansion. For example, the TIL cell number may refer to the number of cells in a third TIL population subjected to second stage in vitro expansion. For example, the TIL cell number may refer to the number of TIL cells finally obtained by any culture method of the present invention. In the present invention, the TIL cell number may be measured by methods commonly used in the art, for example, including but not limited to manual cell counting using a hemocytometer and/or counting using an automated cell counter.

In the present invention, the terms "about" and "approximately" generally refer to a statistically meaningful numerical range. Such a range may be within one order of magnitude of a given value or range, may be within 50%, preferably within 20%, more preferably within 10%, and most preferably within 5%. The allowable variation encompassed by the term "about" or "approximately" may depend on the particular system under study and may be readily understood by a person of ordinary skill in the art.

In the present invention, the terms "above", "below", "at most", and "at least" include the number itself.

### Embodiments of the Invention

### BCL2L11 and/or PTPN2

In one aspect, a method for culturing cells is provided in the present invention, such that a member selected from the Bcl-2 family or the protein tyrosine phosphatase family and/or a functionally active fragment thereof in the cells is reduced and/or attenuated.

For example, the cells may further comprise reduced expression and/or attenuated activity of, optionally, a gene selected from BRD4, FAS, TNFAIP3, ZC3H12A, SOCS1, CBLB, FIBP, IKZF1, LAG3, MED12, PD1, RASA2, TIGIT, TIM3, ADNP, NFKBIA, PTPN6, BCL2L11, PTPN2, AFF3, AXL, NFE2L1, RARG, UBFD1, CRP, CYLD, CBLIF, KLF4, NDST1, NLRP1, SCGB1A1, or TNIP1.

For example, the Bcl-2 family member may comprise a Bcl-2 homology domain 3 . For example, the Bcl-2 family member may comprise BCL2L11.

For example, the protein tyrosine phosphatase family member may comprise a tyrosine phosphatase domain. For example, the protein tyrosine phosphatase family member may comprise PTPN2.

For example, in the cells of the present invention, a Bcl-2 family member and a protein tyrosine phosphatase family member and/or a functionally active fragment thereof is reduced and/or attenuated. For example, BCL2L11 and PTPN2 are reduced and/or attenuated.

For example, the target gene of the present invention may be a gene encoding a member selected from the Bcl-2 family or the protein tyrosine phosphatase family and/or a functionally active fragment thereof. For example, as compared with cells in which expression and/or activity of the target gene is not altered, cells obtained by reducing expression and/or attenuating activity of the target gene in the cells may exhibit improvement. In one embodiment, the cells in which expression and/or activity of the target gene is not altered may refer to cells derived from the same donor and in which expression and/or activity of the target gene of the cells has not been reduced and/or attenuated. In one embodiment, the cells in which expression and/or activity of the target gene is not altered may refer to cells derived from the same donor and in which expression and/or activity of other genes than the target gene of the cells (e.g., knocking out such other gene, which has substantially no effect on cell function) has not been reduced and/or attenuated.

In one embodiment, corresponding cells in which expression and/or activity of the target gene of the cells has not been reduced and/or attenuated may refer to cells derived from the same donor, isolated in the same manner, and in which expression and/or activity of the target gene of the cells has not been reduced and/or attenuated. In one embodiment, corresponding cells in which expression and/or activity of the target gene of the cells has not been reduced and/or attenuated may refer to cells derived from the same donor and from the same tumor source, and in which expression and/or activity of the target gene of the cells has not been reduced and/or attenuated. In one embodiment, corresponding cells in which expression and/or activity of the target gene of the cells has not been reduced and/or attenuated may refer to cells obtained by dividing cells derived from the same donor and from the same tumor source into two groups, wherein one group of cells in which expression and/or activity of the target gene of the cells has not been reduced and/or attenuated serves as the corresponding cells in which expression and/or activity of the target gene of the cells has not been reduced and/or attenuated. For example, reduced expression and/or attenuated activity of the target gene may refer to that, when the target gene of a native cell is in an expression state to a certain extent, after treatment of the present invention, the expression level of the target gene in the cell is reduced, i.e., the reduction in the expression level of the target gene causes the native cell to change from expressing the target gene to substantially not expressing the target gene, or to express the target gene at a reduced level.

For example, the cells comprise immune cells. For example, the cells comprise phagocytes, lymphocytes, neutrophils, eosinophils, and/or basophils.

For example, the cells comprise monocytes, macrophages, and/or dendritic cells.

For example, the cells of the present invention further comprise cells differentiated from stem cells. For example, the cells of the present invention further comprise cells differentiated from pluripotent stem cells. For example, the stem cells of the present invention may be obtained by induction. For example, the stem cells described above of the present invention may comprise induced pluripotent stem cells (iPSC), embryonic stem cells, bone marrow stem cells, cord blood stem cells, and/or peripheral blood stem cells.

For example, the "stem cells" of the present invention further comprise pluripotent cells, multipotent cells, precursor cells, and progenitor cells. For example, stem cells may be obtained from hematopoietic or mesenchymal stem cells obtained from bone marrow tissue, placental stem cells obtained from placental tissue, embryonic stem cells obtained from embryonic tissue, or embryonic germ cells obtained from fetal germ tissue. Exemplary pluripotent stem cells may also be generated from somatic cells by reprogramming the somatic cells to a pluripotent state through expression of certain transcription factors associated with pluripotency; such cells are referred to as "induced pluripotent stem cells" or "iPSC".

For example, the cells comprise B cells, T cells, natural killer cells, and/or natural killer-like T cells (NKT). For example, "unmodified cells" or "unengineered cells" may refer to cells or a cell population in which the genome is not modified and that does not comprise a gene regulation system, or that comprises a control gene regulation system (e.g., an empty vector control, a non-targeting gRNA, an interfering siRNA, etc.). For example, the cells comprise αβ T cells and/or γδ T cells. For example, the cells comprise tumor-infiltrating lymphocytes (TIL). For example, the TIL are TIL derived from tumor tissue, tumor-associated lymph nodes with or without tumor metastasis, tumor metastatic lesions, fragments of paracancerous tissue, pleural effusion and/or ascites, and/or TIL derived from cryopreserved and resuscitated TIL.

For example, the TIL of the present invention may be TIL derived from tumor tissue, tumor-associated lymph nodes with or without tumor metastasis, tumor metastatic lesions, fragments of paracancerous tissue, pleural effusion and/or ascites, and/or TIL derived from cryopreserved and resuscitated TIL. For example, the TIL of the present invention may be obtained by processing tumor tissue into tumor fragments. For example, the volume of the tumor fragments of the present invention is about 1-27 cubic millimeters. For example, the volume of the tumor fragments of the present invention is about 1 cubic millimeter, about 2 cubic millimeters, about 3 cubic millimeters, about 4 cubic millimeters, about 5 cubic millimeters, about 6 cubic millimeters, about 7 cubic millimeters, about 8 cubic millimeters, about 9 cubic millimeters, about 10 cubic millimeters, about 11 cubic millimeters, about 12 cubic millimeters, about 13 cubic millimeters, about 14 cubic millimeters, about 15 cubic millimeters, about 16 cubic millimeters, about 17 cubic millimeters, about 18 cubic millimeters, about 19 cubic millimeters, about 20 cubic millimeters, about 21 cubic millimeters, about 23 cubic millimeters, about 24 cubic millimeters, about 25 cubic millimeters, about 26 cubic millimeters, or about 27 cubic millimeters.

For example, the cells comprise an engineered immune receptor displayed on the cell surface. For example, the engineered immune receptor specifically binds to an antigen expressed on a target cell. For example, the cells comprise a chimeric antigen receptor and/or a T cell receptor.

In one aspect, a method for culturing tumor-infiltrating lymphocytes (TIL) is provided in the present invention, which may comprise reducing expression and/or activity of a member selected from the Bcl-2 family or the protein tyrosine phosphatase family and/or a functionally active fragment thereof in the TIL.

For example, TIL derived from tumor tissue, tumor-associated lymph nodes with or without tumor metastasis, tumor metastatic lesions, fragments of paracancerous tissue, pleural effusion and/or ascites, and not subjected to in vitro expansion are subjected to in vitro expansion of at least one stage, wherein, in at least one stage of the in vitro expansion, expression and/or activity of a member selected from the Bcl-2 family or the protein tyrosine phosphatase family and/or a functionally active fragment thereof in the TIL is reduced.

For example, the TIL of the present invention derived from tumor tissue, tumor-associated lymph nodes with or without tumor metastasis, tumor metastatic lesions, fragments of paracancerous tissue, pleural effusion and/or ascites, and not subjected to in vitro expansion are subjected to first stage in vitro expansion and second stage in vitro expansion, and, in the second stage in vitro expansion of the present invention, expression and/or activity of a member selected from the Bcl-2 family or the protein tyrosine phosphatase family and/or a functionally active fragment thereof in the TIL is reduced. For example, the TIL of the present invention derived from tumor tissue, tumor-associated lymph nodes with or without tumor metastasis, tumor metastatic lesions, fragments of paracancerous tissue, pleural effusion and/or ascites, and not subjected to in vitro expansion are subjected to first stage in vitro expansion and second stage in vitro expansion, and, in the first stage in vitro expansion of the present invention, expression and/or activity of a member selected from the Bcl-2 family or the protein tyrosine phosphatase family and/or a functionally active fragment thereof in the TIL is reduced.

For example, the TIL of the present invention derived from tumor tissue, tumor-associated lymph nodes with or without tumor metastasis, tumor metastatic lesions, fragments of paracancerous tissue, pleural effusion and/or ascites, and not subjected to in vitro expansion are subjected to first stage in vitro expansion and second stage in vitro expansion, and, in the first stage in vitro expansion of the present invention, expression and/or activity of a member selected from the Bcl-2 family or the protein tyrosine phosphatase family and/or a functionally active fragment thereof in the TIL is reduced, and, in the second stage in vitro expansion of the present invention, expression and/or activity of a member selected from the Bcl-2 family or the protein tyrosine phosphatase family and/or a functionally active fragment thereof in the TIL is reduced.

For example, the TIL of the present invention derived from tumor tissue, tumor-associated lymph nodes with or without tumor metastasis, tumor metastatic lesions, fragments of paracancerous tissue, pleural effusion and/or ascites, and not subjected to in vitro expansion are subjected to first stage in vitro expansion, second stage in vitro expansion, and third stage in vitro expansion, and, in the first stage in vitro expansion of the present invention, expression and/or activity of a member selected from the Bcl-2 family or the protein tyrosine phosphatase family and/or a functionally active fragment thereof in the TIL is reduced.

For example, TILs of the present invention, which are derived from tumor tissue, tumor-associated lymph nodes with or without tumor metastasis, tumor metastatic lesions, fragments of paracancerous tissue, pleural effusion and/or ascites and are not subjected to in vitro expansion, are subjected to a first stage in vitro expansion, a second stage in vitro expansion, and a third stage in vitro expansion, and, in the second stage in vitro expansion of the present invention, the expression and/or activity of a member selected from the Bcl-2 family or the protein tyrosine phosphatase family and/or a functionally active fragment thereof in the TILs is reduced and/or attenuated.

For example, TILs of the present invention, which are derived from tumor tissue, tumor-associated lymph nodes with or without tumor metastasis, tumor metastatic lesions, fragments of paracancerous tissue, pleural effusion and/or ascites and are not subjected to in vitro expansion, are subjected to a first stage in vitro expansion, a second stage in vitro expansion, and a third stage in vitro expansion, and, in the third stage in vitro expansion of the present invention, the expression and/or activity of a member selected from the Bcl-2 family or the protein tyrosine phosphatase family and/or a functionally active fragment thereof in the TILs is reduced and/or attenuated.

For example, TILs of the present invention, which are derived from tumor tissue, tumor-associated lymph nodes with or without tumor metastasis, tumor metastatic lesions, fragments of paracancerous tissue, pleural effusion and/or ascites and are not subjected to in vitro expansion, are subjected to a first stage in vitro expansion, a second stage in vitro expansion, and a third stage in vitro expansion, and, in the first stage in vitro expansion of the present invention, the expression and/or activity of a member selected from the Bcl-2 family or the protein tyrosine phosphatase family and/or a functionally active fragment thereof in the TILs is reduced and/or attenuated, and, in the second stage in vitro expansion of the present invention, the expression and/or activity of a member selected from the Bcl-2 family or the protein tyrosine phosphatase family and/or a functionally active fragment thereof in the TILs is reduced and/or attenuated.

For example, TILs of the present invention, which are derived from tumor tissue, tumor-associated lymph nodes with or without tumor metastasis, tumor metastatic lesions, fragments of paracancerous tissue, pleural effusion and/or ascites and are not subjected to in vitro expansion, are subjected to a first stage in vitro expansion, a second stage in vitro expansion, and a third stage in vitro expansion, and, in the first stage in vitro expansion of the present invention, the expression and/or activity of a member selected from the Bcl-2 family or the protein tyrosine phosphatase family and/or a functionally active fragment thereof in the TILs is reduced and/or attenuated, and, in the third stage in vitro expansion of the present invention, the expression and/or activity of a member selected from the Bcl-2 family or the protein tyrosine phosphatase family and/or a functionally active fragment thereof in the TILs is reduced and/or attenuated.

For example, TILs of the present invention, which are derived from tumor tissue, tumor-associated lymph nodes with or without tumor metastasis, tumor metastatic lesions, fragments of paracancerous tissue, pleural effusion and/or ascites and are not subjected to in vitro expansion, are subjected to a first stage in vitro expansion, a second stage in vitro expansion, and a third stage in vitro expansion, and, in the second stage in vitro expansion of the present invention, the expression and/or activity of a member selected from the Bcl-2 family or the protein tyrosine phosphatase family and/or a functionally active fragment thereof in the TILs is reduced and/or attenuated, and, in the third stage in vitro expansion of the present invention, the expression and/or activity of a member selected from the Bcl-2 family or the protein tyrosine phosphatase family and/or a functionally active fragment thereof in the TILs is reduced and/or attenuated.

For example, TILs of the present invention, which are derived from tumor tissue, tumor-associated lymph nodes with or without tumor metastasis, tumor metastatic lesions, fragments of paracancerous tissue, pleural effusion and/or ascites and are not subjected to in vitro expansion, are subjected to a first stage in vitro expansion, a second stage in vitro expansion, and a third stage in vitro expansion, and, in the first stage in vitro expansion of the present invention, the expression and/or activity of a member selected from the Bcl-2 family or the protein tyrosine phosphatase family and/or a functionally active fragment thereof in the TILs is reduced and/or attenuated, and, in the second stage in vitro expansion of the present invention, the expression and/or activity of a member selected from the Bcl-2 family or the protein tyrosine phosphatase family and/or a functionally active fragment thereof in the TILs is reduced and/or attenuated, and, in the third stage in vitro expansion of the present invention, the expression and/or activity of a member selected from the Bcl-2 family or the protein tyrosine phosphatase family and/or a functionally active fragment thereof in the TILs is reduced and/or attenuated.

For example, each stage of in vitro expansion may be delineated by a change in the number of TIL cells; for example, when the number of TIL cells increases by at least about 1-fold, the TIL cells may be considered to have entered a next stage of in vitro expansion. In some embodiments, when the number of TIL cells increases by at least about 1 to 1000-fold, for example, at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, at least about 50-fold, at least about 100-fold, at least about 200-fold, at least about 500-fold, or at least about 1000-fold, the TIL cells may be considered to have entered a next stage of in vitro expansion. For example, each stage of in vitro expansion may also be delineated by a change in culture conditions of the TIL cells. For example, when a cell activator and/or a cell growth factor is added or additionally supplemented in the cell culture medium, the TIL cells may be considered to have entered a next stage of in vitro expansion. For example, when IL-2 is added or additionally supplemented in the cell culture medium, the TIL cells may be considered to have entered a next stage of in vitro expansion. For example, when one or more gene regulation systems is added or additionally supplemented in the cell culture medium, the TIL cells may be considered to have entered a next stage of in vitro expansion. For example, when feeder cells are added or additionally supplemented in the cell culture medium, the TIL cells may be considered to have entered a next stage of in vitro expansion. For example, when the TIL cells are subjected to centrifugation and/or cell washing, the TIL cells may be considered to have entered a next stage of in vitro expansion. For example, each stage may also be delineated by the number of days of TIL cell culture. For example, when the TIL cells are cultured in vitro for about 1-100 days, for example, about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 30 days, about 40 days, about 50 days, or about 100 days, the TIL cells may be considered to have entered a next stage of in vitro expansion.

For example, the reduction and/or attenuation of a Bcl-2 family member of the cells comprises inhibition of an apoptosis-initiating function.

For example, the reduction and/or attenuation of a protein tyrosine phosphatase family member of the cells comprises inhibition of a tyrosine phosphatase function.

For example, as compared with cells in which the expression and/or activity of a member selected from the Bcl-2 family or the protein tyrosine phosphatase family is not changed, cells obtained by reducing the expression and/or attenuating the activity of the member selected from the Bcl-2 family or the protein tyrosine phosphatase family exhibit improved cellular characteristics.

For example, the improved cellular characteristics comprise one or more selected from the group consisting of: an improved and increased proportion of viable cells, an improved and enhanced secretion capacity, an enhanced in vitro tumor cell killing capacity, and an enhanced in vivo tumor killing capacity.

For example, the improved proportion of cell subpopulations comprises one or more selected from the group consisting of: an increased proportion of activated cells, a decreased proportion of regulatory cells, a decreased proportion of exhausted cells, an increased proportion of central memory cells and/or naïve cells, a decreased proportion of apoptotic cells, and an increased proportion of stem-like cells.

For example, the improved cell number of the present invention means that, as compared with cells in which the expression and/or activity of a member selected from the Bcl-2 family or the protein tyrosine phosphatase family is not changed, the number of cells of the present invention, in which the expression of the member selected from the Bcl-2 family or the protein tyrosine phosphatase family is reduced and/or the activity thereof is attenuated in at least one in vitro expansion stage, is increased by at least about 1 to 50-fold, for example, at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold.

For example, the increased proportion of viable cells may be manifested as an increase in cell viability. For example, the increased proportion of viable cells of the present invention may mean that, as compared with cells in which the expression and/or activity of a member selected from the Bcl-2 family or the protein tyrosine phosphatase family is not changed, the proportion of viable cells of the cells of the present invention, in which the expression of the member selected from the Bcl-2 family or the protein tyrosine phosphatase family is reduced and/or the activity thereof is attenuated in at least one in vitro expansion stage, is increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the enhanced cytokine secretion capacity of the present invention may refer to an increased cytokine secretion capacity of the cells for cytokines selected from the group consisting of IL-2, IL-6, CD107a, GZMB, TNF-α, and IFN-γ. For example, the enhanced cytokine secretion capacity of the present invention may refer to that, as compared with cells in which the expression and/or activity of a member selected from the Bcl-2 family or the protein tyrosine phosphatase family is not altered, in the cells of the present invention in which, in at least one in vitro expansion stage, the expression of said member selected from the Bcl-2 family or the protein tyrosine phosphatase family is reduced and/or the activity is weakened, the proportion of cytokine-secreting cells is increased by at least about 1-50 fold, for example, at least about 1 fold, at least about 2 fold, at least about 3 fold, at least about 4 fold, at least about 5 fold, at least about 6 fold, at least about 7 fold, at least about 8 fold, at least about 9 fold, at least about 10 fold, at least about 11 fold, at least about 12 fold, at least about 13 fold, at least about 14 fold, at least about 15 fold, at least about 20 fold, at least about 30 fold, at least about 40 fold, or at least about 50 fold. For example, the enhanced cytokine secretion capacity of the present invention may refer to that, as compared with cells in which the expression and/or activity of a member selected from the Bcl-2 family or the protein tyrosine phosphatase family is not altered, in the cells of the present invention in which, in at least one in vitro expansion stage, the expression of said member selected from the Bcl-2 family or the protein tyrosine phosphatase family is reduced and/or the activity is weakened, the proportion of cytokine-secreting cells is increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%. For example, the cytokine secretion capacity of the cells of the present invention is determined by flow cytometry or by Cytometric Bead Array (CBA).

For example, the enhanced in vitro tumor cell killing capacity and/or the enhanced in vivo tumor killing capacity of the present invention may refer to that, as compared with cells in which the expression and/or activity of a member selected from the Bcl-2 family or the protein tyrosine phosphatase family is not altered, in the cells of the present invention in which, in at least one in vitro expansion stage, the expression of said member selected from the Bcl-2 family or the protein tyrosine phosphatase family is reduced and/or the activity is weakened, the tumor cell killing rate is increased by at least about 1 to 50 fold, for example, at least about 1 fold, at least about 2 fold, at least about 3 fold, at least about 4 fold, at least about 5 fold, at least about 6 fold, at least about 7 fold, at least about 8 fold, at least about 9 fold, at least about 10 fold, at least about 11 fold, at least about 12 fold, at least about 13 fold, at least about 14 fold, at least about 15 fold, at least about 20 fold, at least about 30 fold, at least about 40 fold, or at least about 50 fold. For example, the enhanced in vitro tumor cell killing capacity and/or the enhanced in vivo tumor killing capacity of the present invention may refer to that, as compared with cells in which the expression and/or activity of a member selected from the Bcl-2 family or the protein tyrosine phosphatase family is not altered, in the cells of the present invention in which, in at least one in vitro expansion stage, the expression of said member selected from the Bcl-2 family or the protein tyrosine phosphatase family is reduced and/or the activity is weakened, the tumor cell killing rate is increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%. For example, the tumor cell killing rate of the cells of the present invention is measured by an IncuCyte system or by a CFSE and DAPI staining method. For example, the tumor cell killing of the cells of the present invention may refer to the ability of the cells to kill solid tumor cells.

For example, the improved cell subset proportion of the present invention may comprise one or more selected from the group consisting of: an increased proportion of CD8⁺ cells, an increased proportion of central memory cells and/or naïve cells, a decreased proportion of regulatory cells, an increased proportion of activated cells, an increased proportion of tumor-specific cells (having a CD103⁺CD39⁺ phenotype), an increased proportion of stem cell-like cells, a decreased proportion of exhausted cells, and a decreased proportion of apoptotic cells.

For example, the increased proportion of CD8⁺ cells of the present invention may be an increase in the proportion of CD8-positive cells in the cells. For example, in the CD8⁺ cells, the proportion is increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the increased proportion of activated cells of the present invention may be an increase in the proportion of CD28⁺, CD25⁺ and/or 41BB⁺ cells in the cells. For example, in the cells, the proportion of activated cells is increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%, or is increased by at least about 1-50 fold, for example, at least about 1 fold, at least about 2 fold, at least about 3 fold, at least about 4 fold, at least about 5 fold, at least about 6 fold, at least about 7 fold, at least about 8 fold, at least about 9 fold, at least about 10 fold, at least about 11 fold, at least about 12 fold, at least about 13 fold, at least about 14 fold, at least about 15 fold, at least about 20 fold, at least about 30 fold, at least about 40 fold, or at least about 50 fold.

For example, the reduced proportion of exhausted cells of the present invention may be an increase in the proportion of PD-1⁺, LAG-3⁺, TIM-3⁺, CD39⁺, CD38⁺ and/or CD101⁺ cells in the cells. For example, in the cells, the proportion of exhausted cells is reduced by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%, or is reduced by at least about 1-50 fold, for example, at least about 1 fold, at least about 2 fold, at least about 3 fold, at least about 4 fold, at least about 5 fold, at least about 6 fold, at least about 7 fold, at least about 8 fold, at least about 9 fold, at least about 10 fold, at least about 11 fold, at least about 12 fold, at least about 13 fold, at least about 14 fold, at least about 15 fold, at least about 20 fold, at least about 30 fold, at least about 40 fold, or at least about 50 fold.

For example, the reduced proportion of regulatory cells of the present invention may be a reduction in the proportion of CD4⁺CD25⁺Foxp3⁺ cells in the cells. For example, in the cells, the proportion of regulatory cells is reduced by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the reduced proportion of apoptotic cells of the present invention may be a reduction in the proportion of Annexin V⁺7-AAD⁺ cells and/or Annexin V⁺7-AAD⁻ cells in the cells. For example, in the cells, the proportion of apoptotic cells is reduced by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the increased proportion of stem-like cells in the present invention may be an increase in the proportion of CD69⁻CD39⁻ cells and/or TCF1⁺ cells among the cells. For example, the proportion of stem-like cells among the cells may be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the increased proportion of central memory cells in the present invention may be an increase in the proportion of CD45RA⁻CCR7⁺ cells or CD45RO⁺CD62L⁺ cells among the cells. For example, the proportion of central memory cells among the cells may be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the increased proportion of naïve T cells in the present invention may be an increase in the proportion of CD45RO⁻CD62L⁺ cells among the cells. For example, the proportion among the cells may be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the method of the present invention may comprise editing a target gene of cells in vivo, ex vivo, and/or in vitro. For example, a reduction in an in vivo expression level of a target gene in cells in vivo may be achieved by delivery and editing in vivo via an in vivo gene regulation system. For example, in vivo editing of a target gene may be performed by delivering LNPs comprising a gene regulation system or mRNA encoding a gene regulation system by targeting immune cells or precursor cells thereof, such as bone marrow stem cells and the like. By adjusting the composition and/or proportion of LNP components, or by introducing components having targeting capability, the in vivo editing efficiency of the present invention may be improved.

For example, the culture method of the present invention may comprise a gene editing step for the cells. For example, the culture method comprises subjecting the cells to at least one stage, wherein, in in vitro expansion of at least one stage, the gene regulation system is introduced into the cells.

For example, the gene regulation system may disrupt the target gene at the DNA level. For example, the gene regulation system may disrupt a region of the target gene in the genome of the cell or a fragment thereof. For example, after the gene regulation system is used, the DNA region in which the target gene is located in the cell or a fragment thereof is cleaved, such that the expression capability of the target gene is reduced or the activity of the target gene is inhibited. For example, the editing effect of the gene regulation system on the target gene may be long-term and sustained. For example, in the cells of the present invention, the activity of a member selected from the Bcl-2 family or the protein tyrosine phosphatase family is inhibited.

Wherein the genomic region described in the present invention is determined according to the human reference genome hg38 version.

For example, the gene regulation system may comprise a guide nucleic acid molecule and an enzyme protein. For example, the enzyme protein may have nuclease activity, and the guide nucleic acid molecule may guide the enzyme protein to specifically cleave a region in which the target gene is located or a fragment thereof. For example, the guide nucleic acid molecule and the enzyme protein may exist in the form of a ribonucleoprotein complex (RNP), or may each independently exist alone. For example, the enzyme protein may comprise a Cas protein. For example, a polynucleotide encoding gRNA and a Cas protein may be introduced into a target cell, or may each independently be introduced alone into the target cell.

For example, in the present invention, reducing expression and/or weakening activity of a target gene of a cell may comprise introducing a ribonucleoprotein complex (RNP) comprising the guide nucleic acid molecule and the enzyme protein into the cell. For example, the enzyme protein may comprise a Cas protein, a Cas protein homolog, or a functionally active fragment thereof. For example, the guide nucleic acid molecule may comprise a guide RNA (gRNA). For example, a complex comprising a polynucleotide encoding gRNA and a Cas protein may be introduced into the cell. For example, a complex comprising gRNA and a Cas protein may be introduced into the cell.

For example, the gRNA may be used to bind to a sequence of the target gene. For example, binding of the gRNA to the sequence of the target gene may be fully complementary, may be partially complementary, or may hybridize to the sequence of the target gene under moderately stringent or stringent conditions. For example, binding of the gRNA to the sequence of the target gene may enable a CRISPR system to specifically cleave the target gene.

For example, an editing target region of the present invention may be a region upstream of a start codon. For example, the editing target region of the present invention may be a region having high transcription factor binding affinity. For example, the editing target region of the present invention may be a region having a specific number of transcription factor binding sites. For example, the editing target region of the present invention may be a continuous region having about 3 or more transcription factor binding sites. For example, genomic coordinates of the editing target region of the present invention may be selected from preferred targeted sub-regions shown in Tables 1A to 1B.

For example, the guide nucleic acid molecule targeting BCL2L11 in the present invention may bind to a region or a fragment thereof selected from the group consisting of: SEQ ID NO: 24789-26713. For example, the guide nucleic acid molecule targeting PTPN2 in the present invention may bind to a region or a fragment thereof selected from the group consisting of: SEQ ID NO: 26714-28115.

For example, when the gene editing system comprises CRISPR/Cas9, a protospacer adjacent motif (PAM) may be present downstream of the region targeted by the guide nucleic acid molecule of the present invention, and the protospacer adjacent motif (PAM) may be AGG, TGG, GGG, or CGG. For example, when a PAM region of a target gene is determined, a target sequence composed of about 15 to about 25 (for example, about 15, about 16, about 17, about 18, about 19, about 20, about 21, about 22, about 23, about 24, about 25) nucleotides upstream of the 5' end of the PAM of the target gene may be readily determined by a person skilled in the art, and an appropriate gRNA may be designed for the target sequence. For example, the guide nucleic acid molecule is capable of binding to a sequence composed of about 15 to about 25 nucleotides upstream of the 5' end of a protospacer adjacent motif (PAM) selected from the group consisting of: AGG, TGG, GGG, and CGG.

For example, when the gene editing system comprises CRISPR/Cas12, a protospacer adjacent motif (PAM) may be present upstream of the region targeted by the guide nucleic acid molecule of the present invention, and the protospacer adjacent motif (PAM) may be NTTN, TTYN, VTTV, TRTV, TTTV, TATV, TYCV, TNN, or NTN, or may be TTTN, ATTN, GTTN, CTTN, TTC, TTG, TTA, TTT, TAN, TGN, or TCN, wherein N is A, T, C or G, Y is T or C, V is A, C or G, and R is A or G. For example, the protospacer adjacent motif (PAM) may be TTTN. For example, the protospacer adjacent motif (PAM) may be TTN. For example, when a PAM region of a target gene is determined, a target sequence composed of about 15 to about 25 (for example, about 15, about 16, about 17, about 18, about 19, about 20, about 21, about 22, about 23, about 24, about 25) nucleotides downstream of the 3' end of the PAM of the target gene may be readily determined by a person skilled in the art, and an appropriate gRNA may be designed for the target sequence. For example, the guide nucleic acid molecule is capable of binding to a sequence composed of about 15 to about 25 nucleotides downstream of the 3' end of a protospacer adjacent motif (PAM) selected from the group consisting of: NTTN, TTYN, VTTV, TRTV, TTTV, TATV, TYCV, TNN, or NTN, or TTTN, ATTN, GTTN, CTTN, TTC, TTG, TTA, TTT, TAN, TGN, or TCN, wherein N is A, T, C or G, Y is T or C, V is A, C or G, and R is A or G. For example, the protospacer adjacent motif (PAM) may be TTTN. For example, the protospacer adjacent motif (PAM) may be TTN.

For example, when the gene editing system of the present invention comprises wild-type Cas12a (which may also be referred to as Cpf1, such as AsCas12a, FnCas12a, LbCas12a, BbCas12a, CMaCas12a and OsCas12a), a PAM sequence selected from the following may be present upstream of the region targeted by the guide nucleic acid molecule of the present invention: NTTN, wherein N may be A, T, C or G. For example, when a PAM region of a target gene is determined, a target sequence composed of about 17 to about 25 (for example, about 17, about 18, about 19, about 20, about 21, about 22, about 23, about 24, or about 25) nucleotides downstream of the 3' end of the PAM of the target gene may be readily determined by a person skilled in the art, and an appropriate gRNA may be designed for the target sequence.

For example, when the gene editing system of the present invention comprises a mutant Cas12a, such as enAsCas12a (mutation sites E174R, S542R, and K548R), a PAM sequence selected from the following is present upstream of the region targeted by the guide nucleic acid molecule of the present invention: TTYN (TTTN/TTCN), VTTV (ATTV/CTTV/GTTV), or TRTV (TATV/TGTV), wherein N is A, T, C, or G, Y is T or C, V is A, C, or G, and R is A or G. For example, when the PAM region of a target gene is determined, a target sequence consisting of about 17 to about 25 (e.g., about 17, about 18, about 19, about 20, about 21, about 22, about 23, about 24, or about 25) nucleotides downstream of the 3' end of the PAM of the target gene is readily determined by a person skilled in the art, and an appropriate gRNA is designed for the target sequence.

For example, when the gene editing system of the present invention comprises a mutant Cas12a, such as opAsCas12a (mutation sites: E174R and S542R), a PAM sequence selected from the following is present upstream of the region targeted by the guide nucleic acid molecule of the present invention: TTTV (TTTA, TTTC, or TTTG), wherein V is A, C, or G. For example, when the PAM region of a target gene is determined, a target sequence consisting of about 17 to about 25 (e.g., about 17, about 18, about 19, about 20, about 21, about 22, about 23, about 24, or about 25) nucleotides downstream of the 3' end of the PAM of the target gene is readily determined by a person skilled in the art, and an appropriate gRNA is designed for the target sequence.

For example, when the gene editing system of the present invention comprises a mutant Cas12a, such as AsCas12a Ultra (mutation sites: M537R and F870L), a PAM sequence selected from the following is present upstream of the region targeted by the guide nucleic acid molecule of the present invention: TTTV, TATV, or TYCV, wherein V is A, C, or G, and Y is T or C. For example, when the PAM region of a target gene is determined, a target sequence consisting of about 17 to about 25 (e.g., about 17, about 18, about 19, about 20, about 21, about 22, about 23, about 24, or about 25) nucleotides downstream of the 3' end of the PAM of the target gene is readily determined by a person skilled in the art, and an appropriate gRNA is designed for the target sequence.

For example, when the gene editing system of the present invention comprises a mutant Cas12a, such as hfCas12Max (mutation sites: N243R/E336R/D892R) and Cas12Max (mutation site: N243R), a PAM sequence selected from the following is present upstream of the region targeted by the guide nucleic acid molecule of the present invention: TNN or NTN, wherein N is A, T, C, or G. For example, when the PAM region of a target gene is determined, a target sequence consisting of about 17 to about 25 (e.g., about 17, about 18, about 19, about 20, about 21, about 22, about 23, about 24, or about 25) nucleotides downstream of the 3' end of the PAM of the target gene is readily determined by a person skilled in the art, and an appropriate gRNA is designed for the target sequence.

For example, when the gene editing system of the present invention comprises wild-type Cas12b or mutant Cas12b (such as an AaCas12b protein from Alicyclobacillus acidiphilus), a PAM sequence selected from the following is present upstream of the region targeted by the guide nucleic acid molecule of the present invention: TTN, wherein N is A, T, C, or G. For example, when the PAM region of a target gene is determined, a target sequence consisting of about 17 to about 25 (e.g., about 17, about 18, about 19, about 20, about 21, about 22, about 23, about 24, or about 25) nucleotides downstream of the 3' end of the PAM of the target gene is readily determined by a person skilled in the art, and an appropriate gRNA is designed for the target sequence.

For example, when the gene editing system of the present invention comprises wild-type Cas12i or mutant Cas12i (a Cas protein having a smaller size), a PAM sequence selected from the following is present upstream of the region targeted by the guide nucleic acid molecule of the present invention: TTN or TTTN, wherein N is A, T, C, or G. For example, when the PAM region of a target gene is determined, a target sequence consisting of about 17 to about 25 (e.g., about 17, about 18, about 19, about 20, about 21, about 22, about 23, about 24, or about 25) nucleotides downstream of the 3' end of the PAM of the target gene is readily determined by a person skilled in the art, and an appropriate gRNA is designed for the target sequence.

For example, the guide nucleic acid molecule comprises a target sequence consisting of about 15 to about 25 nucleotides upstream of a PAM region indicated by AGG, TGG, GGG and/or CGG in DNA in which a gene encoding a member selected from the Bcl-2 family or the protein tyrosine phosphatase family and/or a functionally active fragment thereof is located, and is capable of binding thereto. For example, the guide nucleic acid molecule comprises a target sequence consisting of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 22 to about 23, about 15 to about 21, about 17 to about 21, about 19 to about 21, about 20 to about 21, about 15 to about 20, about 17 to about 20, about 19 to about 20, about 15 to about 19, about 17 to about 19, or about 15 to about 17 nucleotides upstream of a PAM region indicated by AGG, TGG, GGG and/or CGG in DNA in which a gene encoding a member selected from the Bcl-2 family or the protein tyrosine phosphatase family and/or a functionally active fragment thereof is located, and is capable of binding thereto. For example, the target sequence is selected from chr2:111120146-111120960, chr2:111120963-111121094, chr2:111121097-111123471, chr2:111123902-111124004, chr2:111124127-111124207, chr2:111150166-111150221, chr2:111164187-111164245, chr2:111164248-111164454, chr2:111164460-111164518, chr2:111164532-111164578, chr2:111164590-111164738, chr2:111164755-111164850, chr2:111165197-111165712, chr2:111166057-111166502, chr2:111166514-111166695, chr2:111166720-111166783, chr2:111167145-111167298, chr2:111167303-111167360, chr2:111167523-111167697, chr2:111167772-111168023, chr2:111168246-111168347, or a fragment thereof. For example, the target sequence is selected from a region defined by the genomic coordinates shown in chr18:12785604-12785781, chr18:12793371-12793435, chr18:12793447-12793535, chr18:12794308-12794410, chr18:12794504-12794607, chr18:12817358-12817388, chr18:12831040-12831154, chr18:12859082-12859177, chr18:12884154-12884234, and/or chr18:12884248-12884853, or a fragment thereof. For example, the target sequence is selected from a region defined by the genomic coordinates shown in Tables 2A to 2B, or a fragment thereof.

For example, the guide nucleic acid molecule comprises an sgRNA targeting BCL2L11 as shown in any one of SEQ ID NO: 1-1925 and 49607-49627, or an sgRNA targeting PTPN2 as shown in any one of SEQ ID NO: 1926-3327 and 49628-49637.

For example, as compared with cells in which the expression and/or activity of the target gene is not altered, in cells obtained by reducing the expression and/or weakening the activity of the target gene of the cells, the proportion of cells expressing a product of the target gene is reduced and/or the expression level of the target gene in a single cell is decreased.

For example, in the method of the present invention, as compared with cells in which the expression and/or activity of the target gene is not altered, the proportion of cells expressing a product of the target gene in cells obtained by reducing the expression and/or weakening the activity of the target gene of the cells is reduced by at least about 5%. For example, the proportion of cells expressing a product of a gene encoding a member selected from the Bcl-2 family or the protein tyrosine phosphatase family and/or a functionally active fragment thereof is reduced by at least about 100-5%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the proportion of cells expressing a product of a gene encoding a member selected from the Bcl-2 family or the protein tyrosine phosphatase family and/or a functionally active fragment thereof is from an observable proportion of cells to 1%. For example, the proportion of cells expressing a product of a gene encoding a member selected from the Bcl-2 family or the protein tyrosine phosphatase family and/or a functionally active fragment thereof is reduced to at least about 100-1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, or at least about 1%. For example, the proportion of cells expressing a product of a gene encoding a member selected from the Bcl-2 family or the protein tyrosine phosphatase family and/or a functionally active fragment thereof is detected by a flow cytometer.

For example, in the method of the present invention, in cells obtained by reducing the expression and/or weakening the activity of the target gene of the cells, the proportion of cells expressing a product of a gene encoding a member selected from the Bcl-2 family or the protein tyrosine phosphatase family and/or a functionally active fragment thereof is at most about 95%. For example, the proportion of cells expressing a product of a gene encoding a member selected from the Bcl-2 family or the protein tyrosine phosphatase family and/or a functionally active fragment thereof is at most about 95-5%, for example, at most about 95%, at most about 90%, at most about 80%, at most about 70%, at most about 60%, at most about 50%, at most about 40%, at most about 30%, at most about 20%, at most about 19%, at most about 18%, at most about 17%, at most about 16%, at most about 15%, at most about 14%, at most about 13%, at most about 12%, at most about 11%, at most about 10%, at most about 9%, at most about 8%, at most about 7%, at most about 6%, or at most about 5%. For example, the proportion of cells expressing a product of a gene encoding a member selected from the Bcl-2 family or the protein tyrosine phosphatase family and/or a functionally active fragment thereof is detected by a flow cytometer.

For example, in the method of the present invention, as compared with cells in which the expression and/or activity of the target gene is not altered, in cells obtained by reducing the expression and/or weakening the activity of the target gene in said cells, the expression level of the target gene in a single cell can be reduced by at least about 5%. For example, the expression level of the target gene in a single cell can be reduced by at least about 100-5%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the expression level of the target gene in a single cell can be from an observable expression level to 1%. For example, the expression level of the target gene in a single cell can be reduced to at least about 100-1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, or at least about 1%.

For example, in cells obtained by reducing the expression and/or weakening the activity of the target gene in said cells by the method of the present invention, the expression level of the target gene in a single cell can be at most about 95% of that in cells in which the expression and/or activity of the target gene is not altered. For example, the expression level, in a single cell, of a gene encoding a member selected from the Bcl-2 family or the protein tyrosine phosphatase family and/or a functionally active fragment thereof (for example, a gene encoding BCL2L11 or PTPN2) can be at most about 95-5% of that in cells in which the expression and/or activity of the member selected from the Bcl-2 family or the protein tyrosine phosphatase family and/or the functionally active fragment thereof is not altered, for example, at most about 95%, at most about 90%, at most about 80%, at most about 70%, at most about 60%, at most about 50%, at most about 40%, at most about 30%, at most about 20%, at most about 19%, at most about 18%, at most about 17%, at most about 16%, at most about 15%, at most about 14%, at most about 13%, at most about 12%, at most about 11%, at most about 10%, at most about 9%, at most about 8%, at most about 7%, at most about 6%, or at most about 5%.

For example, the method of the present invention comprises subjecting said cells to at least one stage, wherein, in the in vitro expansion of at least one stage, the expression of a member selected from the Bcl-2 family or the protein tyrosine phosphatase family in said cells is reduced and/or the activity thereof is weakened.

For example, TIL derived from tumor tissue, tumor-associated lymph nodes with or without tumor metastasis, tumor metastatic lesions, fragments of paracancerous tissue, pleural effusion and/or ascites and not subjected to in vitro expansion is subjected to a first stage in vitro expansion and a second stage in vitro expansion, wherein during the second stage in vitro expansion, the expression of a member selected from the Bcl-2 family or the protein tyrosine phosphatase family in the TIL subjected to the first stage in vitro expansion is reduced and/or the activity thereof is weakened.

For example, the first stage in vitro expansion is performed for at least 7 days. For example, the second stage in vitro expansion is performed for at least about 7 days.

For example, in the in vitro expansion of the present invention in a single stage, said cells can be contacted with said one or more cell activators, and the expression and/or activity of a member selected from the Bcl-2 family or the protein tyrosine phosphatase family and/or a functionally active fragment thereof in said cells can be reduced. For example, the cell activator can comprise an agonist of one or more targets selected from the group consisting of CD3, CD28, HVEM, CD40L, OX40, and 4-1BB. For example, in the in vitro expansion in a single stage, a member selected from the Bcl-2 family or the protein tyrosine phosphatase family in the cells of the present invention is reduced and/or weakened, and the cells are contacted with one or more cell activators of the present invention. For example, in the first stage in vitro expansion of the present invention, a member selected from the Bcl-2 family or the protein tyrosine phosphatase family in the TIL of the present invention can be reduced and/or weakened, and the TIL can be contacted with one or more cell activators of the present invention. For example, in the second stage in vitro expansion of the present invention, a member selected from the Bcl-2 family or the protein tyrosine phosphatase family in the TIL of the present invention can be reduced and/or weakened, and the TIL can be contacted with one or more cell activators of the present invention. For example, in the third stage in vitro expansion of the present invention, a member selected from the Bcl-2 family or the protein tyrosine phosphatase family in the TIL of the present invention can be reduced and/or weakened, and the TIL can be contacted with one or more cell activators of the present invention.

For example, in the in vitro expansion in a single stage, the cells of the present invention are substantially simultaneously subjected to reducing the expression and/or weakening the activity of a member selected from the Bcl-2 family or the protein tyrosine phosphatase family and to being contacted with one or more cell activators of the present invention. For example, in the in vitro expansion in a single stage, the cells of the present invention are first subjected to reducing the expression and/or weakening the activity of a member selected from the Bcl-2 family or the protein tyrosine phosphatase family, for example, 2-48 hours in advance, for example, 2 hours in advance, 4 hours in advance, 8 hours in advance, 12 hours in advance, 24 hours in advance, or 48 hours in advance, and then are contacted with one or more cell activators of the present invention. For example, in the in vitro expansion in a single stage, the cells of the present invention are first contacted with one or more cell activators of the present invention, for example, 2-48 hours in advance, for example, 2 hours in advance, 4 hours in advance, 8 hours in advance, 12 hours in advance, 24 hours in advance, or 48 hours in advance, and then are subjected to reducing the expression and/or weakening the activity of a member selected from the Bcl-2 family or the protein tyrosine phosphatase family.

For example, in the first stage in vitro expansion of the present invention, the TIL of the present invention are substantially simultaneously subjected to reducing the expression and/or weakening the activity of a member selected from the Bcl-2 family or the protein tyrosine phosphatase family and to being contacted with one or more cell activators of the present invention. For example, in the second stage in vitro expansion of the present invention, the TIL of the present invention are substantially simultaneously subjected to reducing the expression and/or weakening the activity of a member selected from the Bcl-2 family or the protein tyrosine phosphatase family and to being contacted with one or more cell activators of the present invention. For example, in the third stage in vitro expansion of the present invention, the TIL of the present invention are substantially simultaneously subjected to reducing the expression and/or weakening the activity of a member selected from the Bcl-2 family or the protein tyrosine phosphatase family and to being contacted with one or more cell activators of the present invention.

For example, the second stage in vitro expansion of the present invention is performed for at least about 9 days. For example, the second stage in vitro expansion of the present invention can be performed for at least about 9 days, at least about 10 days, at least about 11 days, at least about 12 days, at least about 13 days, or at least about 14 days. For example, the second stage in vitro expansion of the present invention can be performed for about 9 days to about 14 days, about 10 days to about 14 days, about 11 days to about 14 days, about 12 days to about 14 days, about 13 days to about 14 days, about 9 days to about 13 days, about 10 days to about 13 days, about 11 days to about 13 days, about 12 days to about 13 days, about 9 days to about 12 days, about 10 days to about 12 days, about 11 days to about 12 days, or about 10 days to about 11 days. For example, the second stage in vitro expansion of the present invention can be considered a REP (rapid expansion protocol) stage. For example, the first stage in vitro expansion of the present invention can be considered a preREP stage.

For example, the number of days for which the second stage in vitro expansion of the present invention is performed can be calculated from the start time of the second stage in vitro expansion. For example, at the time when the second stage in vitro expansion starts, it can be considered that the second stage in vitro expansion has been performed for about 0 hours. For example, after about 24 hours have elapsed after the start of the second stage in vitro expansion, it can be considered that the second stage in vitro expansion has been performed for about 1 day. For example, on the day when the second stage in vitro expansion starts, it can be considered that the second stage in vitro expansion has been performed for about 0 days. For example, the number of days for which the second stage in vitro expansion of the present invention is performed can be calculated based on the number of days of the second stage in vitro expansion. For example, on the second day after the start of the second stage in vitro expansion, it can be considered that the second stage in vitro expansion has been performed for about 1 day.

For example, the cell activator of the present invention can comprise one or more selected from the group consisting of CD80, CD86, B7-H3, 4-1BBL, CD27, CD30, CD134, B7h, CD40, LIGHT, and functionally active fragments thereof. For example, the cell activator of the present invention can comprise an agonist of one or more targets selected from the group consisting of CD3, CD28, HVEM, CD40L, OX40, and 4-1BB. For example, the cell activator of the present invention can comprise an antibody selected from the group consisting of CD3, CD28, HVEM, CD40L, OX40, and 4-1BB, and antigen-binding fragments thereof. For example, the cell activator of the present invention can comprise a CD3 agonist. For example, the cell activator of the present invention can comprise an anti-CD3 antibody and/or an antigen-binding fragment thereof, for example, Miltenyi Biotech OKT3, or BD SP34. For example, the cell activator of the present invention can comprise a CD28 agonist. For example, the cell activator of the present invention can comprise an anti-CD28 antibody and/or an antigen-binding fragment thereof, for example, Merck 15E8.

For example, the cell activator of the present invention may comprise an anti-CD3 antibody and/or an antigen-binding fragment thereof; for example, the light chain VL and heavy chain VH of OKT3 from Miltenyi Biotech may be comprised, and the light chain VL and heavy chain VH of SP34 from BD may be comprised. For example, the cell activator of the present invention may comprise a CD28 agonist. For example, the cell activator of the present invention may comprise an anti-CD28 antibody and/or an antigen-binding fragment thereof; for example, the light chain VL and heavy chain VH of 15E8 from Merck may be comprised. For example, the cell activator of the present invention may comprise an anti-CD3 antibody and/or an antigen-binding fragment thereof; for example, the light chain LCDR1-3 and heavy chain HCDR1-3 of OKT3 from Miltenyi Biotech may be comprised, and the light chain LCDR1-3 and heavy chain HCDR1-3 of SP34 from BD may be comprised, and the anti-CD3 antibody and/or antigen-binding fragment thereof of the present invention may have CD3 binding capability. For example, the cell activator of the present invention may comprise a CD28 agonist. For example, the cell activator of the present invention may comprise an anti-CD28 antibody and/or an antigen-binding fragment thereof; for example, the light chain LCDR1-3 and heavy chain HCDR1-3 of 15E8 from Merck may be comprised, and the anti-CD28 antibody and/or antigen-binding fragment thereof of the present invention may have CD28 binding capability. In the present invention, the antibody or antigen-binding protein of the present invention comprises at least one CDR in an antibody heavy chain variable region VH and/or at least one CDR in an antibody light chain variable region VL. The CDRs of the present invention may be defined according to IMGT nomenclature, the CDRs of the present invention may be defined according to Chothia, or the CDRs of the present invention may be defined according to Kabat.

For example, contacting the cells of the present invention with one or more cell activators of the present invention may comprise one or more modes selected from the group consisting of: (1) the cell activator of the present invention is added to a cell culture medium of the cells of the present invention; (2) the cell activator of the present invention is added to a cell culture medium of the cells of the present invention; and (3) a composition comprising the cell activator of the present invention is added to a cell culture medium of the cells of the present invention. For example, contacting the cells of the present invention with one or more cell activators of the present invention may comprise adding a composition comprising the cell activator of the present invention to a cell culture medium of the cells of the present invention. For example, contacting the cells of the present invention with one or more cell activators of the present invention may comprise adding a solid-phase medium comprising the CD28 antibody and the CD3 antibody of the present invention to the cell culture medium of the cells of the present invention.

For example, an initial concentration of the cell activator in a cell culture medium of the cells of the present invention may be at least about 30 ng/mL. For example, an initial concentration of the CD28 antibody of the present invention in a cell culture medium of the cells of the present invention may be at least about 30 ng/mL; for example, an initial concentration of the CD3 antibody of the present invention in a cell culture medium of the cells of the present invention may be at least about 30 ng/mL. For example, selection of the initial concentration of the CD28 antibody of the present invention may be independent of selection of the initial concentration of the CD3 antibody of the present invention; for example, the initial concentrations of the CD28 antibody of the present invention and the CD3 antibody of the present invention in a cell culture medium of the cells of the present invention may be combined in any manner. For example, the initial concentration of the CD28 antibody of the present invention in a cell culture medium of the cells of the present invention may be arbitrarily selected from about 30 ng/mL to about 300 ng/mL. For example, the initial concentration of the CD3 antibody of the present invention in a cell culture medium of the cells of the present invention may be arbitrarily selected from about 30 ng/mL to about 300 ng/mL. For example, the initial concentration of the CD28 antibody of the present invention in a cell culture medium of the cells of the present invention may be arbitrarily selected from about 30 ng/mL to about 300 ng/mL, and the initial concentration of the CD3 antibody of the present invention in a cell culture medium of the cells of the present invention may be arbitrarily selected from about 30 ng/mL to about 300 ng/mL, and selection of the initial concentration of the CD28 antibody of the present invention may be independent of selection of the initial concentration of the CD3 antibody of the present invention. For example, the diameter of the solid-phase medium of the present invention may be about 500 nm to about 10 µm. For example, the diameter of the solid-phase medium of the present invention may be measured by transmission electron microscopy. For example, the diameter of the solid-phase medium of the present invention may be about 1 nm to about 500 nm. For example, the diameter of the solid-phase medium of the present invention may be about 100 nm to about 500 nm. For example, the diameter of the solid-phase medium of the present invention may be about 200 nm to about 500 nm. For example, the diameter of the solid-phase medium of the present invention may be measured by transmission electron microscopy.

For example, the solid-phase medium of the present invention may comprise a polymer. For example, the solid-phase medium of the present invention may comprise dextran.

For example, each mg of the solid-phase medium of the present invention comprises at least about 25 µg of the cell activator of the present invention.

For example, at a ratio of the solid-phase medium of the present invention to the cells of the present invention of about 100:1 to about 1:2000, preferably about 1:100 to about 1:2000, a solid-phase medium comprising the cell activator of the present invention is added to a cell culture medium of the cells of the present invention. For example, at a ratio of the solid-phase medium of the present invention to the cells of the present invention of about 2:1 to about 1:2, a solid-phase medium comprising the cell activator of the present invention is added to a cell culture medium of the cells of the present invention.

For example, when a diameter of the solid-phase medium of the present invention is about 500 nm to about 10 µm, a solid-phase medium comprising the cell activator of the present invention may be added to a cell culture medium of the cells of the present invention at a ratio of the solid-phase medium of the present invention to the cells of the present invention of about 2:1 to about 1:2. For example, when a diameter of the solid-phase medium of the present invention is about 500 nm to about 10 µm, a solid-phase medium comprising the cell activator of the present invention, for example, a CD3 agonist and/or a CD28 agonist, may be added to a cell culture medium of the cells of the present invention at a ratio of the solid-phase medium of the present invention to the cells of the present invention of about 2:1 to about 1:2, about 2:1 to about 1:1, or about 1:1 to about 1:2.

For example, when a diameter of the solid-phase medium of the present invention is about 100 nm to about 500 nm, a solid-phase medium comprising the cell activator of the present invention may be added to a cell culture medium of the cells of the present invention at a ratio of the solid-phase medium of the present invention to the cells of the present invention of about 1:100 to about 1:2000. For example, when a diameter of the solid-phase medium of the present invention is about 100 nm to about 500 nm, at a ratio of the solid-phase medium of the present invention to the cells of the present invention of about 1:100 to about 1:2000, about 1:200 to about 1:2000, about 1:300 to about 1:2000, about 1:400 to about 1:2000, about 1:500 to about 1:2000, about 1:600 to about 1:2000, about 1:700 to about 1:2000, about 1:800 to about 1:2000, about 1:900 to about 1:2000, about 1:1000 to about 1:2000, about 1:1200 to about 1:2000, about 1:1400 to about 1:2000, about 1:1600 to about 1:2000, or about 1:1800 to about 1:2000, a solid-phase medium comprising the CD28 agonist and the CD3 agonist of the present invention may be added to a cell culture medium of the cells of the present invention.

For example, the method of the present invention may further comprise: during in vitro expansion of the present invention at at least one stage, the cells of the present invention are contacted with one or more cell growth factors.

For example, in a single stage of in vitro expansion of the present invention, the cells of the present invention may be contacted with the cell activator of the present invention and contacted with one or more cell growth factors of the present invention. For example, in a first stage of in vitro expansion of the present invention, the TIL of the present invention may be contacted with the cell activator of the present invention and contacted with one or more cell growth factors of the present invention. For example, in a second stage of in vitro expansion of the present invention, the TIL of the present invention may be contacted with the cell activator of the present invention and contacted with one or more cell growth factors of the present invention. For example, in a third stage of in vitro expansion of the present invention, the TIL of the present invention may be contacted with the cell activator of the present invention and contacted with one or more cell growth factors of the present invention.

For example, in a single stage of in vitro expansion of the present invention, the cells of the present invention are contacted with the cell activator of the present invention and one or more cell growth factors of the present invention. For example, in a single stage of in vitro expansion of the present invention, the cells of the present invention may be contacted substantially simultaneously with one or more cell growth factors of the present invention and one or more cell activators of the present invention. For example, in a single stage of in vitro expansion of the present invention, the cells of the present invention may be first contacted with one or more cell growth factors of the present invention; for example, 2-48 hours in advance, for example, 2 hours, 4 hours, 8 hours, 12 hours, 24 hours, or 48 hours in advance, and then contacted with one or more cell activators of the present invention. For example, in a single stage of in vitro expansion of the present invention, the cells of the present invention may be first contacted with one or more cell activators of the present invention; for example, 2-48 hours in advance, for example, 2 hours, 4 hours, 8 hours, 12 hours, 24 hours, or 48 hours in advance, and then contacted with one or more cell growth factors of the present invention.

For example, in a first stage of in vitro expansion of the present invention, the cells of the present invention may be contacted with the cell activator of the present invention and one or more cell growth factors of the present invention. For example, in a second stage of in vitro expansion of the present invention, the TIL of the present invention may be contacted with the cell activator of the present invention and one or more cell growth factors of the present invention. For example, in a third stage of in vitro expansion of the present invention, the TIL of the present invention may be contacted with the cell activator of the present invention and one or more cell growth factors of the present invention.

For example, the cell growth factor of the present invention may be one or more selected from the group consisting of: IL-2, IL-7, IL-12, IL-15, IL-21, interferon-γ, and functional active fragments thereof. For example, the cell growth factor of the present invention may comprise IL-2 and/or a functionally active fragment thereof. For example, the functionally active fragment of IL-2 may comprise a fragment of IL-2 known in the art that is capable of binding to an IL-2 receptor of a cell. For example, the cell growth factor of the present invention may comprise IL-2 and/or a functionally active fragment thereof, IL-7 and/or a functionally active fragment thereof, and IL-15 and/or a functionally active fragment thereof.

For example, contact between the cells of the present invention and one or more cell growth factors of the present invention may comprise adding the cell growth factor of the present invention to a cell culture medium of the cells of the present invention. For example, an initial concentration of the cell growth factor of the present invention in the cell culture medium of the cells of the present invention may be at least about 300 IU/mL. For example, an initial concentration of IL-2 of the present invention in the cell culture medium of the cells of the present invention may be at least about 300-9000 IU/mL, for example, at least about 300 IU/mL, at least about 350 IU/mL, at least about 400 IU/mL, at least about 500 IU/mL, at least about 600 IU/mL, at least about 700 IU/mL, at least about 800 IU/mL, at least about 900 IU/mL, at least about 1000 IU/mL, at least about 1100 IU/mL, at least about 1200 IU/mL, at least about 1300 IU/mL, at least about 1400 IU/mL, at least about 1500 IU/mL, at least about 2000 IU/mL, at least about 2500 IU/mL, at least about 2600 IU/mL, at least about 2700 IU/mL, at least about 2800 IU/mL, at least about 2900 IU/mL, at least about 3000 IU/mL, at least about 3100 IU/mL, at least about 3200 IU/mL, at least about 3300 IU/mL, at least about 3400 IU/mL, at least about 3500 IU/mL, at least about 4000 IU/mL, at least about 4500 IU/mL, at least about 5000 IU/mL, at least about 5500 IU/mL, at least about 6000 IU/mL, at least about 6500 IU/mL, at least about 7000 IU/mL, at least about 7500 IU/mL, at least about 8000 IU/mL, at least about 8500 IU/mL, or at least about 9000 IU/mL.

For example, contact between the cells of the present invention and IL-2, IL-7, and IL-15, as compared with contact with IL-2 alone, may reduce the amount of cytokines used. For example, the amount of IL-2 added may be reduced under conditions in which IL-7 and IL-15 are added. For example, the concentration of IL-7 may be about 1 to 1000 ng/mL, preferably about 1-100 ng/mL. For example, the concentration of IL-15 may be about 1 to 1000 ng/mL, preferably about 1-100 ng/mL. For example, with respect to the amount of IL-2 added, the amount may be reduced to a range commonly used in the art for various immune cells, for example, reduced to 50-10% of the range commonly used in the art, for example, 50%, 20%, or 10%. For example, for TCR-T, the range commonly used in the art for IL-2 addition may be 30-300 IU/mL. For example, for TIL, the range commonly used in the art for IL-2 addition may be 300-9000 IU/mL (for example, 1000-9000 IU/mL).

For example, the method of the present invention may further comprise: in at least one stage of the in vitro expansion of the present invention, the cells of the present invention may be co-cultured with.

For example, in a single stage of the in vitro expansion of the present invention, the cells of the present invention may be contacted with one or more cell activators and/or one or more cell growth factors and may be co-cultured with the feeder cells of the present invention. For example, the single stage of the in vitro expansion of the present invention may refer to the in vitro expansion of the present invention in the same stage; for example, the single stage may be simultaneously in the first stage in vitro expansion of the present invention, simultaneously in the second stage in vitro expansion of the present invention, or simultaneously in the third stage in vitro expansion of the present invention, and the like.

For example, in the first stage in vitro expansion of the present invention, the TIL of the present invention may be contacted with one or more cell activators and/or one or more cell growth factors and may be co-cultured with the feeder cells of the present invention. For example, in the second stage in vitro expansion of the present invention, the TIL of the present invention may be contacted with one or more cell activators of the present invention and/or one or more cell growth factors of the present invention and may be co-cultured with the feeder cells of the present invention. For example, in the third stage in vitro expansion of the present invention, the TIL of the present invention may be contacted with one or more cell activators of the present invention and/or one or more cell growth factors of the present invention and may be co-cultured with the feeder cells of the present invention.

For example, in a single stage of the in vitro expansion of the present invention, after the cells of the present invention are contacted with one or more cell activators of the present invention and/or one or more cell growth factors of the present invention for a certain period of time, the cells may then be co-cultured with the feeder cells of the present invention. For example, in the first stage in vitro expansion of the present invention, after the TIL of the present invention are contacted with one or more cell activators of the present invention and/or one or more cell growth factors of the present invention for a certain period of time, the TIL may then be co-cultured with the feeder cells of the present invention. For example, in the second stage in vitro expansion of the present invention, after the TIL of the present invention are contacted with one or more cell activators of the present invention and/or one or more cell growth factors of the present invention for a certain period of time, the TIL may then be co-cultured with the feeder cells of the present invention. For example, in the third stage in vitro expansion of the present invention, after the TIL of the present invention are contacted with one or more cell activators of the present invention and/or one or more cell growth factors of the present invention for a certain period of time, the TIL may then be co-cultured with the feeder cells of the present invention.

For example, in a single stage of the in vitro expansion of the present invention, after the cells of the present invention are contacted with one or more cell activators of the present invention and/or one or more cell growth factors of the present invention for a certain period of time, the cells may then be co-cultured with the feeder cells of the present invention. For example, the certain period of time of the present invention may be at least about 1 hour. For example, the certain period of time of the present invention may be at least about 1-72 hours, for example, at least about 1 hour, at least about 2 hours, at least about 3 hours, at least about 4 hours, at least about 5 hours, at least about 6 hours, at least about 7 hours, at least about 8 hours, at least about 9 hours, at least about 10 hours, at least about 11 hours, at least about 12 hours, at least about 13 hours, at least about 14 hours, at least about 15 hours, at least about 16 hours, at least about 17 hours, at least about 18 hours, at least about 19 hours, at least about 20 hours, at least about 21 hours, at least about 22 hours, at least about 23 hours, at least about 24 hours, at least about 36 hours, at least about 48 hours, at least about 60 hours, or at least about 72 hours. For example, the certain period of time of the present invention may be about 2 hours to about 72 hours. For example, the certain period of time of the present invention may be about 6 hours to about 7 hours, about 6 hours to about 8 hours, about 6 hours to about 9 hours, about 6 hours to about 10 hours, about 6 hours to about 11 hours, about 6 hours to about 12 hours, about 6 hours to about 13 hours, about 6 hours to about 14 hours, about 6 hours to about 15 hours, about 6 hours to about 16 hours, about 6 hours to about 17 hours, about 6 hours to about 18 hours, about 6 hours to about 19 hours, about 6 hours to about 20 hours, about 6 hours to about 21 hours, about 6 hours to about 22 hours, about 6 hours to about 23 hours, about 6 hours to about 24 hours, about 6 hours to about 36 hours, about 6 hours to about 48 hours, about 6 hours to about 60 hours, or about 6 hours to about 72 hours. For example, the certain period of time of the present invention may be about 12 hours to about 13 hours, about 12 hours to about 14 hours, about 12 hours to about 15 hours, about 12 hours to about 16 hours, about 12 hours to about 17 hours, about 12 hours to about 18 hours, about 12 hours to about 19 hours, about 12 hours to about 20 hours, about 12 hours to about 21 hours, about 12 hours to about 22 hours, about 12 hours to about 23 hours, about 12 hours to about 24 hours, about 12 hours to about 36 hours, about 12 hours to about 48 hours, about 12 hours to about 60 hours, or about 12 hours to about 72 hours. For example, the certain period of time of the present invention may be about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 36 hours, about 48 hours, about 60 hours, or about 72 hours.

For example, the feeder cells of the present invention may comprise antigen-presenting cells. For example, the feeder cells of the present invention may comprise one or more selected from the group consisting of: peripheral blood mononuclear cells, dendritic cells, and artificial antigen-presenting cells. For example, the feeder cells of the present invention may be peripheral blood mononuclear cells. For example, the feeder cells of the present invention may be irradiated feeder cells. For example, the feeder cells of the present invention may be isolated artificial antigen-presenting cells (aAPC); the artificial antigen-presenting cells of the present invention may comprise cells expressing HLA-A/B/C, CD64, CD80, ICOS-L, and/or CD58, and may be modified to express more than one cell activator of the present invention. For example, the feeder cells of the present invention may be irradiated, for example, may be irradiated with gamma rays, or may be irradiated with X-rays.

For example, co-culturing the cells of the present invention with the feeder cells of the present invention may comprise bringing a surface of the feeder cells of the present invention into contact with a surface of the cells of the present invention. For example, co-culturing the cells of the present invention with the feeder cells of the present invention may comprise adding the feeder cells of the present invention to a cell culture medium of the cells of the present invention.

For example, the feeder cells of the present invention may be added to the cell culture medium of the cells of the present invention at a ratio of the feeder cells of the present invention to the cells of the present invention of about 40:1 to about 400:1. For example, the feeder cells of the present invention may be added to the cell culture medium of the cells of the present invention at a ratio of the feeder cells of the present invention to the cells of the present invention of about 40:1 to about 400:1, about 40:1 to about 300:1, about 40:1 to about 200:1, about 40:1 to about 100:1, about 40:1 to about 90:1, about 40:1 to about 80:1, about 40:1 to about 70:1, about 40:1 to about 60:1, about 40:1 to about 50:1, about 50:1 to about 400:1, about 60:1 to about 400:1, about 70:1 to about 400:1, about 80:1 to about 400:1, about 90:1 to about 400:1, about 100:1 to about 400:1, about 200:1 to about 400:1, or about 300:1 to about 400:1.

In another aspect, the present invention provides a method for culturing tumor-infiltrating lymphocytes (TIL), which may comprise: (A) contacting a first TIL population, which is derived from tumor tissue, a tumor-associated lymph node with or without tumor metastasis, a tumor metastatic lesion, fragments of paracancerous tissue, pleural effusion and/or ascites and has not been subjected to in vitro expansion, with one or more cell growth factors; wherein a second TIL population is obtained through step (A); (B) reducing expression and/or weakening activity of a member selected from the Bcl-2 family or the protein tyrosine phosphatase family in the second TIL population; wherein a third TIL population is obtained through step (B).

In another aspect, the present invention provides a method for culturing tumor-infiltrating lymphocytes (TIL), comprising: (A) contacting a first TIL population, which is derived from tumor tissue, a tumor-associated lymph node with or without tumor metastasis, a tumor metastatic lesion, fragments of paracancerous tissue, pleural effusion and/or ascites and has not been subjected to in vitro expansion, with one or more T cell growth factors, wherein a second TIL population is obtained through step (A); (B) reducing expression and/or weakening activity of a member selected from the Bcl-2 family or the protein tyrosine phosphatase family in the second TIL population, and contacting the second TIL population with a T cell activator and/or a T cell growth factor, wherein a third TIL population is obtained through step (B); and (C) co-culturing the third TIL population with feeder cells, wherein a fourth TIL population is obtained through step (C).

In one embodiment, the first stage in vitro expansion of the present invention may be interchangeably used with step (A) in the method of the above aspect. In one embodiment, the second stage in vitro expansion of the present invention may be interchangeably used with step (B) in the method of the above aspect. In one embodiment, the TIL subjected to the first stage in vitro expansion of the present invention may be interchangeably used with the second TIL population obtained by step (A) in the method of the above aspect. In one embodiment, the TIL subjected to the second stage in vitro expansion of the present invention may be interchangeably used with the third TIL population obtained by step (B) in the method of the above aspect. In one embodiment, if needed, the third stage in vitro expansion of the present invention may be interchangeably used with any additionally added step (C) in the method of the above aspect. In one embodiment, if needed, the TIL subjected to the third stage in vitro expansion of the present invention may be interchangeably used with the fourth TIL population obtained by any additionally added step (C) in the method of the above aspect.

In another aspect, the present invention provides a method for culturing tumor-infiltrating lymphocytes (TIL), which may comprise: (A) allowing a first TIL population, which is derived from tumor tissue, tumor-associated lymph nodes with or without tumor metastasis, tumor metastatic lesions, fragments of paracancerous tissue, pleural effusion and/or ascites and has not been subjected to in vitro expansion, to be brought into contact with a plurality of cell growth factors, wherein a second TIL population is obtained by said step (A); and (B) allowing said second TIL population to be brought into contact with a plurality of cell growth factors, to be brought into contact with a plurality of cell activators, to have expression of a member selected from the Bcl-2 family or the protein tyrosine phosphatase family reduced and/or activity thereof weakened, and to be co-cultured with feeder cells, wherein a third TIL population is obtained by said step (B).

In another aspect, the present invention provides a method for culturing tumor-infiltrating lymphocytes (TIL), which may comprise: (A) allowing a first TIL population, which is derived from tumor tissue, tumor-associated lymph nodes with or without tumor metastasis, tumor metastatic lesions, fragments of paracancerous tissue, pleural effusion and/or ascites and has not been subjected to in vitro expansion, to be brought into contact with a cell growth factor, wherein a second TIL population is obtained by said step (A); and (B) allowing said second TIL population to be brought into contact with a cell growth factor, to be brought into contact with a cell activator, to have expression of a member selected from the Bcl-2 family or the protein tyrosine phosphatase family reduced and/or activity thereof weakened, and to be co-cultured with feeder cells, wherein the member selected from the Bcl-2 family or the protein tyrosine phosphatase family may respectively comprise BCL2L11 and PTPN2, wherein a third TIL population is obtained by said step (B).

In another aspect, the present invention provides a method for culturing tumor-infiltrating lymphocytes (TIL). A method for obtaining TIL cells from a subject tissue sample may comprise obtaining, by patient surgery, an in situ tumor sample or a metastatic tumor sample, wherein the weight may be at least about 1 g, and a plurality of tissue pieces may also be combined. Tumor tissue, tumor-associated lymph nodes with or without tumor metastasis, tumor metastatic lesions, fragments of paracancerous tissue, pleural effusion and/or ascites are transported in a sample transport solution, for example, a commercially commonly used tumor tissue transport solution, tumor tissue preservation solution, or tumor tissue transfer solution, at about 2-8°C, and are processed within 48 hours. Tissue blocks may be mechanically disrupted to a size of about 1-27 cubic millimeters per block, transferred into a gas-permeable culture bag or Grex, and cultured for about 3-14 days by adding a serum-free cell culture medium and IL-2 at a concentration of 300-9000 IU/mL (e.g., 1000-9000 IU/mL, e.g., 6000 IU/mL). Cells in the culture medium may be collected and transferred into a gas-permeable culture bag, or Grex, or a Xuri device, and the serum-free cell culture medium may be added with the CD28 antibody of the present invention, a CD3 antibody, magnetic beads (e.g., Dynabeads) comprising a CD3 antibody and a CD28 antibody and/or a nanomatrix (e.g., transACT) comprising a CD3 antibody and a CD28 antibody, IL-2 at a concentration of 300-9000 IU/mL (e.g., 1000-9000 IU/mL, e.g., 6000 IU/mL), and reducing expression and/or weakening activity of a member selected from the Bcl-2 family or the protein tyrosine phosphatase family (wherein the Bcl-2 family member may comprise BCL2L11, and the protein tyrosine phosphatase family member may comprise PTPN2, for example, by transducing with a ribonucleoprotein complex (RNP) carrying gRNA of the present invention and a Cas protein, or an LNP comprising gRNA and a Cas protein, or an LNP comprising a nucleic acid encoding gRNA and a Cas protein, such that the proportion of cells in said TIL in which a gene encoding a member selected from the Bcl-2 family or the protein tyrosine phosphatase family is less than about 95%). After the TIL of the present invention are activated for a certain period of time, irradiated PBMC are added (TIL and PBMC at a ratio of about 1:40 to about 1:400), and expansion culture is performed for about 3-14 days. A cell processing system may be used to collect cells in the culture medium, wash and cryopreserve the cells, and perform testing. In the final product, the CD3 proportion may be greater than 80%, the cell viability may be greater than 50%, and greater than 80% of the cells may be memory effector cells and effector cells. After stimulation, IFN-γ may be secreted, and/or a characteristic of an upregulated proportion of activated cells may be present.

### AFF3, AXL, NFE2L1, RARG, and UBFD1

1. A method for culturing cells, said method comprising reducing and/or weakening a family member selected from the AF4 family, the tyrosine protein kinase family, the bZIP family, the nuclear receptor family, or the ubiquitin family, and/or a functionally active fragment thereof, in said cells.
2. The method according to technical solution 1, wherein said cells comprise immune cells.
3. The method according to technical solution 2, wherein said immune cells comprise phagocytes, lymphocytes, neutrophils, eosinophils, and/or basophils.
4. The method according to any one of technical solutions 2 to 3, wherein said immune cells comprise monocytes, macrophages, and/or dendritic cells.
5. The method according to any one of technical solutions 2-4, wherein said immune cells are immune cells derived from stem cell differentiation.
6. The method according to technical solution 5, wherein said stem cells comprise induced pluripotent stem cells (iPSC), embryonic stem cells, bone marrow stem cells, cord blood stem cells, and/or peripheral blood stem cells.
7. The method according to any one of technical solutions 2-6, wherein said immune cells comprise B cells, T cells, natural killer cells, and/or natural killer-like T cells (NKT).
8. The method according to any one of technical solutions 2-7, wherein said immune cells comprise αβ T cells and/or γδ T cells.
9. The method according to any one of technical solutions 2-8, wherein said immune cells comprise tumor-infiltrating lymphocytes (TIL).
10. The method according to technical solution 9, wherein said TIL are TIL derived from fragments, pleural effusion and/or ascites, and/or TIL derived from cryopreserved TIL after resuscitation.
11. The method according to technical solution 10, wherein said volume is about 1 cubic millimeter to about 27 cubic millimeters.
12. The method according to any one of technical solutions 2-11, wherein said immune cells comprise an engineered immune receptor displayed on the cell surface.
13. The method according to technical solution 12, wherein said engineered immune receptor specifically binds to an antigen expressed on a target cell.
14. The method according to any one of technical solutions 2-13, wherein said immune cells comprise a chimeric antigen receptor and/or a T cell receptor.
15. The method according to any one of technical solutions 1-14, wherein said reducing and/or weakening a family member selected from the AF4 family, the tyrosine protein kinase family, the bZIP family, the nuclear receptor family, or the ubiquitin family, and/or a functionally active fragment thereof, in the cells comprises an effect selected from the group consisting of inhibiting a function of nuclear transcription activation, inhibiting a function of phosphotransferase, inhibiting a function of DNA binding, or inhibiting a function of RNA binding.
16. The method according to any one of technical solutions 1-15, wherein, as compared with cells in which the expression and/or activity of a member selected from the AF4 family, the tyrosine protein kinase family, the bZIP family, the nuclear receptor family, or the ubiquitin family and/or a functionally active fragment thereof is unaltered, a cell obtained by reducing the expression and/or attenuating the activity of the member selected from the AF4 family, the tyrosine protein kinase family, the bZIP family, the nuclear receptor family, or the ubiquitin family and/or the functionally active fragment thereof exhibits improved cellular characteristics.
17. The method according to technical solution 16, wherein said improved cellular characteristics comprise one or more selected from the group consisting of improved cell proliferation capacity, an increased proportion of viable cells, an improved cell subset proportion, enhanced cytokine secretion capacity, enhanced in vitro tumor cell killing capacity, and enhanced in vivo tumor killing capacity.
18. The method according to technical solution 17, wherein said improved cell subset proportion comprises one or more selected from the group consisting of an increased proportion of activated cells, a reduced proportion of regulatory cells, a reduced proportion of exhausted cells, an increased proportion of central memory cells and/or naïve cells, a reduced proportion of apoptotic cells, and an increased proportion of stem cell-like cells.
19. The method according to any one of technical solutions 1-18, wherein the family members of the AF4 family, the tyrosine protein kinase family, the bZIP family, the nuclear receptor family, and the ubiquitin family respectively comprise a transcription activation domain, a phosphotransferase domain, a DNA binding domain, a DNA binding domain, and a ubiquitin-like domain.
20. The method according to any one of technical solutions 1-19, wherein the family members of the AF4 family, the tyrosine protein kinase family, the bZIP family, the nuclear receptor family, and the ubiquitin family respectively comprise AFF3, AXL, NFE2L1, RARG, and UBFD1.
21. The method according to any one of technical solutions 1-20, wherein reducing and/or weakening a family member of the AF4 family, the tyrosine protein kinase family, the bZIP family, the nuclear receptor family, or the ubiquitin family in said cells comprises introducing the family member into said cells.
22. The method according to technical solution 21, wherein the gene regulatory system disrupts, at the DNA level, a family member of the AF4 family, the tyrosine protein kinase family, the bZIP family, the nuclear receptor family, or the ubiquitin family; and optionally, the cell is selected from cells in which the expression and/or activity of BRD4, FAS, TNFAIP3, ZC3H12A, SOCS1, CBLB, FIBP, IKZF1, LAG3, MED12, PD1, RASA2, TIGIT, TIM3, ADNP, NFKBIA, PTPN6, BCL2L11, PTPN2, CRP, CYLD, CBLIF, KLF4, NDST1, NLRP1, SCGB1A1, or TNIP1 is reduced and/or attenuated.
23. The method according to any one of technical solutions 21 to 22, wherein the gene regulatory system comprises a guide nucleic acid molecule and an enzyme protein.
24. The method according to technical solution 23, wherein reducing the expression and/or attenuating the activity of a family member of the AF4 family, the tyrosine protein kinase family, the bZIP family, the nuclear receptor family, or the ubiquitin family comprises introducing, into the cell, a complex comprising the guide nucleic acid molecule and the enzyme protein, or a complex comprising the guide nucleic acid molecule and a nucleic acid encoding the enzyme protein.
25. The method according to any one of technical solutions 23 to 24, wherein the enzyme protein comprises a Cas protein, a Cas protein homolog, or a functionally active fragment thereof, preferably selected from Cas 9 and Cas 12.
26. The method according to any one of technical solutions 23-25, wherein the guide nucleic acid molecule comprises (gRNA).
27. The method according to any one of technical solutions 23-26, wherein the guide nucleic acid molecule binds to a sequence of a family member selected from the AF4 family, the tyrosine protein kinase family, the bZIP family, the nuclear receptor family, or the ubiquitin family.
28. The method according to any one of technical solutions 23-27, wherein the guide nucleic acid molecule binds to a sequence consisting of about 15 to about 25 nucleotides upstream of the 5' end of a protospacer adjacent motif (PAM) selected from the group consisting of AGG, TGG, CGG, and GGG, or binds to a sequence consisting of about 15 to about 25 nucleotides downstream of the 3' end of a protospacer adjacent motif (PAM) selected from the group consisting of NTTN, TTYN, VTTV, TRTV, TTTV, TATV, TYCV, TNN, and NTN, wherein N is A, T, C, or G, Y is T or C, V is A, C, or G, and R is A or G, or binds to a sequence consisting of about 15 to about 25 nucleotides downstream of the 3' end of a protospacer adjacent motif (PAM) selected from the group consisting of TTTN, ATTN, GTTN, CTTN, TTC, TTG, TTA, TTT, TAN, TGN, and TCN, wherein N is A, T, C, or G.
29. The method according to any one of technical solutions 23-28, wherein the guide nucleic acid molecule binds to a region defined by genomic coordinates shown in Tables 2C-2G, or a fragment thereof.
30. The method according to any one of technical solutions 23-29, wherein the guide nucleic acid molecule binds to a region or a fragment thereof selected from the group consisting of SEQ ID NO: 28116-34100, SEQ ID NO: 34101-36390, SEQ ID NO: 36391-37812, SEQ ID NO: 37813-39627, and SEQ ID NO: 39628-41119.
31. The method according to any one of technical solutions 23-30, wherein the guide nucleic acid molecule comprises a sequence as shown in SEQ ID NO: 3328-9312, SEQ ID NO: 9313-11602, SEQ ID NO: 11603-13024, SEQ ID NO: 13025-14839, and SEQ ID NO: 14840-16331.
32. The method according to any one of technical solutions 1-31, wherein, compared with a cell in which the expression and/or activity of a family member of the AF4 family, the tyrosine protein kinase family, the bZIP family, the nuclear receptor family, or the ubiquitin family is not changed, in a cell obtained by reducing the expression and/or attenuating the activity of the family member of the AF4 family, the tyrosine protein kinase family, the bZIP family, the nuclear receptor family, or the ubiquitin family, the proportion of cells expressing a target gene is reduced and/or the expression level of the target gene in a single cell is decreased.
33. The method according to any one of technical solutions 1-32, wherein, in a cell obtained by reducing the expression and/or attenuating the activity of a family member of the AF4 family, the tyrosine protein kinase family, the bZIP family, the nuclear receptor family, or the ubiquitin family, the proportion of cells expressing a target gene is about 95% or less.
34. A cell, wherein the cell is obtained by the method according to any one of technical solutions 1-33.
35. A pharmaceutical composition, comprising the cell according to technical solution 34, and optionally a pharmaceutically acceptable carrier.
36. A method for affecting cell growth, comprising the cell according to technical solution 34 and/or the pharmaceutical composition according to technical solution 35.
37. Use of the cell according to technical solution 34 and/or the pharmaceutical composition according to technical solution 35 in the manufacture of a medicament, wherein the medicament is used for preventing and/or treating a disease and/or symptom.
38. A medicament for preventing and/or treating a disease and/or symptom, comprising the cell according to technical solution 34 and/or the pharmaceutical composition according to technical solution 35 as an active ingredient.
39. A method for preventing and/or treating a disease and/or symptom, comprising administering, to a subject in need thereof, the cell according to technical solution 34 and/or the pharmaceutical composition according to technical solution 35.
40. The cell according to technical solution 34 and/or the pharmaceutical composition according to technical solution 35, for use in preventing and/or treating a disease and/or symptom.
41. In the use according to technical solution 37, the medicament according to technical solution 38, the method according to technical solution 39, and/or the cell and/or pharmaceutical composition for use according to technical solution 40, wherein the disease and/or symptom comprises a tumor.
42. In the use according to technical solution 37, the medicament according to technical solution 38, the method according to technical solution 39, and/or the cell and/or pharmaceutical composition for use according to technical solution 40, wherein the disease and/or symptom comprises a solid tumor.
43. In the use according to technical solution 37, the medicament according to technical solution 38, the method according to technical solution 39, and/or the cell and/or pharmaceutical composition for use according to technical solution 40, wherein the disease and/or symptom comprises one or more selected from the group consisting of melanoma, ovarian cancer, cervical cancer, lung cancer, bladder cancer, breast cancer, head and neck cancer, pancreatic cancer, liver cancer, gastric cancer, colorectal cancer, and renal cancer.

In one aspect, the present invention provides a method for culturing cells, such that a member selected from the AF4 family, the tyrosine protein kinase family, the bZIP family, the nuclear receptor family, or the ubiquitin family, and/or a functionally active fragment thereof, in the cells is reduced and/or attenuated.

For example, the cells may further comprise, optionally, cells in which the expression and/or activity of BRD4, FAS, TNFAIP3, ZC3H12A, SOCS1, CBLB, FIBP, IKZF1, LAG3, MED12, PD1, RASA2, TIGIT, TIM3, ADNP, NFKBIA, PTPN6, BCL2L11, PTPN2, AFF3, AXL, NFE2L1, RARG, UBFD1, CRP, CYLD, CBLIF, KLF4, NDST1, NLRP1, SCGB1A1, or TNIP1 is reduced and/or attenuated.

For example, the AF4 family member may comprise a transcriptional activation domain. For example, the AF4 family member may comprise AFF3.

For example, the tyrosine protein kinase family member may comprise a phosphotransferase domain. For example, the tyrosine protein kinase family member may comprise AXL.

For example, the bZIP family member may comprise a DNA-binding domain. For example, the bZIP family member may comprise NFE2L1.

For example, the nuclear receptor family member may comprise a DNA-binding domain. For example, the nuclear receptor family member may comprise RARG.

For example, the ubiquitin family member may comprise a ubiquitin-like domain. For example, the ubiquitin family member may comprise UBFD1.

For example, in the cells of the present invention, members of the AF4 family, the tyrosine protein kinase family, the bZIP family, the nuclear receptor family, and the ubiquitin family, and/or functionally active fragments thereof, are reduced and/or attenuated. For example, AFF3, AXL, NFE2L1, RARG, and UBFD1 are reduced and/or attenuated.

For example, the target gene of the present invention may be a gene encoding a member selected from the AF4 family, the tyrosine protein kinase family, the bZIP family, the nuclear receptor family, or the ubiquitin family, and/or a functionally active fragment thereof. For example, compared with a cell in which the expression and/or activity of the target gene is not changed, a cell obtained by reducing the expression and/or attenuating the activity of the target gene of the cell may exhibit improvement. In one embodiment, the cell in which the expression and/or activity of the target gene is not changed may refer to a cell derived from the same donor and in which the expression and/or activity of the target gene of the cell has not been reduced and/or attenuated. In one embodiment, the cell in which the expression and/or activity of the target gene is not changed may refer to a cell derived from the same donor and in which the expression and/or activity of other genes other than the target gene of the cell (e.g., knocking out the other gene, which has substantially no effect on cell function) has not been reduced and/or attenuated.

In one embodiment, a corresponding cell in which the expression and/or activity of the target gene of the cell has not been reduced and/or attenuated may refer to a cell derived from the same donor, isolated in the same manner, and in which the expression and/or activity of the target gene of the cell has not been reduced and/or attenuated. In one embodiment, a corresponding cell in which the expression and/or activity of the target gene of the cell has not been reduced and/or attenuated may refer to a cell derived from the same donor and from the same tumor source, and in which the expression and/or activity of the target gene of the cell has not been reduced and/or attenuated. In one embodiment, a corresponding cell in which the expression and/or activity of the target gene of the cell has not been reduced and/or attenuated may refer to dividing cells derived from the same donor and from the same tumor source into two groups, wherein one group of cells in which the expression and/or activity of the target gene of the cell has not been reduced and/or attenuated is used as the corresponding cell in which the expression and/or activity of the target gene of the cell has not been reduced and/or attenuated. For example, reducing the expression and/or attenuating the activity of the target gene may refer to a state in which the target gene of a native cell is expressed to a certain extent, and, after the treatment of the present invention, the expression level of the target gene in the cell is reduced; that is, the reduction in the expression level of the target gene may be such that the native cell is changed from expressing the target gene to substantially not expressing the target gene, or the amount of expression of the target gene is reduced.

For example, the cell comprises an immune cell. For example, the cell comprises a phagocyte, a lymphocyte, a neutrophil, an eosinophil, and/or a basophil.

For example, the cell comprises a monocyte, a macrophage, and/or a dendritic cell.

For example, the cell of the present invention further comprises a cell derived from differentiation of a stem cell. For example, the cell of the present invention further comprises a cell derived from differentiation of a pluripotent stem cell. For example, the stem cell of the present invention is obtained by induction. For example, the above stem cell of the present invention comprises an induced pluripotent stem cell (iPSC), an embryonic stem cell, a bone marrow stem cell, a cord blood stem cell, and/or a peripheral blood stem cell.

For example, the "stem cell" of the present invention further comprises a pluripotent cell, a multipotent cell, a precursor cell, and a progenitor cell. For example, the stem cell is obtained from a hematopoietic or mesenchymal stem cell obtained from bone marrow tissue, a placental stem cell obtained from placental tissue, an embryonic stem cell obtained from embryonic tissue, or an embryonic germ cell obtained from fetal germ tissue. Exemplary pluripotent stem cells are also produced from somatic cells by reprogramming the somatic cells to a pluripotent state through expression of certain transcription factors associated with pluripotency; such cells are referred to as "induced pluripotent stem cells" or "iPSC".

For example, the cell comprises a B cell, a T cell, a natural killer cell, and/or a natural killer-like T cell (NKT). For example, an "unmodified cell" or an "unengineered cell" refers to a cell or cell population in which the genome is not modified and that does not comprise a gene regulation system, or that comprises a control gene regulation system (e.g., an empty vector control, a non-targeting gRNA, an interfering siRNA, etc.). For example, the cell comprises an αβ T cell and/or a γδ T cell. For example, the cell comprises a tumor-infiltrating lymphocyte (TIL). For example, the TIL is a TIL derived from tumor tissue, a tumor-associated lymph node with or without tumor metastasis, a tumor metastatic lesion, a fragment of paracancerous tissue, pleural effusion, and/or ascites, and/or is a TIL derived from recovery after cryopreservation.

For example, the TIL of the present invention is a TIL derived from tumor tissue, a tumor-associated lymph node with or without tumor metastasis, a tumor metastatic lesion, a fragment of paracancerous tissue, pleural effusion, and/or ascites, and/or is a TIL derived from recovery after cryopreservation. For example, the TIL of the present invention is obtained by processing tumor tissue into tumor fragments. For example, the volume of the tumor fragments of the present invention is about 1-27 cubic millimeters. For example, the volume of the tumor fragments of the present invention is about 1 cubic millimeter, about 2 cubic millimeters, about 3 cubic millimeters, about 4 cubic millimeters, about 5 cubic millimeters, about 6 cubic millimeters, about 7 cubic millimeters, about 8 cubic millimeters, about 9 cubic millimeters, about 10 cubic millimeters, about 11 cubic millimeters, about 12 cubic millimeters, about 13 cubic millimeters, about 14 cubic millimeters, about 15 cubic millimeters, about 16 cubic millimeters, about 17 cubic millimeters, about 18 cubic millimeters, about 19 cubic millimeters, about 20 cubic millimeters, about 21 cubic millimeters, about 23 cubic millimeters, about 24 cubic millimeters, about 25 cubic millimeters, about 26 cubic millimeters, or about 27 cubic millimeters.

For example, the cell comprises an engineered immune receptor displayed on the cell surface. For example, the engineered immune receptor specifically binds to an antigen expressed on a target cell. For example, the cell comprises a chimeric antigen receptor and/or a T cell receptor.

In one aspect, the present invention provides a method for culturing tumor-infiltrating lymphocytes (TIL), which comprises reducing expression and/or activity, in the TIL, of a member selected from the AF4 family, the tyrosine protein kinase family, the bZIP family, the nuclear receptor family, or the ubiquitin family, and/or a functionally active fragment thereof.

For example, a TIL derived from tumor tissue, a tumor-associated lymph node with or without tumor metastasis, a tumor metastatic lesion, a fragment of paracancerous tissue, pleural effusion, and/or ascites and not subjected to in vitro expansion is subjected to in vitro expansion of at least one stage, wherein, in at least one stage of the in vitro expansion, expression and/or activity, in the TIL, of a member selected from the AF4 family, the tyrosine protein kinase family, the bZIP family, the nuclear receptor family, or the ubiquitin family, and/or a functionally active fragment thereof is reduced.

For example, a TIL of the present invention derived from tumor tissue, a tumor-associated lymph node with or without tumor metastasis, a tumor metastatic lesion, a fragment of paracancerous tissue, pleural effusion, and/or ascites and not subjected to in vitro expansion is subjected to first stage in vitro expansion and second stage in vitro expansion, and, in the second stage in vitro expansion of the present invention, expression and/or activity, in the TIL, of a member selected from the AF4 family, the tyrosine protein kinase family, the bZIP family, the nuclear receptor family, or the ubiquitin family, and/or a functionally active fragment thereof is reduced. For example, a TIL of the present invention derived from tumor tissue, a tumor-associated lymph node with or without tumor metastasis, a tumor metastatic lesion, a fragment of paracancerous tissue, pleural effusion, and/or ascites and not subjected to in vitro expansion is subjected to first stage in vitro expansion and second stage in vitro expansion, and, in the first stage in vitro expansion of the present invention, expression and/or activity, in the TIL, of a member selected from the AF4 family, the tyrosine protein kinase family, the bZIP family, the nuclear receptor family, or the ubiquitin family, and/or a functionally active fragment thereof is reduced.

For example, a TIL of the present invention derived from tumor tissue, a tumor-associated lymph node with or without tumor metastasis, a tumor metastatic lesion, a fragment of paracancerous tissue, pleural effusion, and/or ascites and not subjected to in vitro expansion is subjected to first stage in vitro expansion and second stage in vitro expansion, and, in the first stage in vitro expansion of the present invention, expression and/or activity, in the TIL, of a member selected from the AF4 family, the tyrosine protein kinase family, the bZIP family, the nuclear receptor family, or the ubiquitin family, and/or a functionally active fragment thereof is reduced, and, in the second stage in vitro expansion of the present invention, expression and/or activity, in the TIL, of a member selected from the AF4 family, the tyrosine protein kinase family, the bZIP family, the nuclear receptor family, or the ubiquitin family, and/or a functionally active fragment thereof is reduced.

For example, a TIL of the present invention derived from tumor tissue, a tumor-associated lymph node with or without tumor metastasis, a tumor metastatic lesion, a fragment of paracancerous tissue, pleural effusion, and/or ascites and not subjected to in vitro expansion is subjected to first stage in vitro expansion, second stage in vitro expansion, and third stage in vitro expansion, and, in the first stage in vitro expansion of the present invention, expression and/or activity, in the TIL, of a member selected from the AF4 family, the tyrosine protein kinase family, the bZIP family, the nuclear receptor family, or the ubiquitin family, and/or a functionally active fragment thereof is reduced.

For example, a TIL of the present invention derived from tumor tissue, a tumor-associated lymph node with or without tumor metastasis, a tumor metastatic lesion, a fragment of paracancerous tissue, pleural effusion, and/or ascites and not subjected to in vitro expansion is subjected to first stage in vitro expansion, second stage in vitro expansion, and third stage in vitro expansion, and, in the second stage in vitro expansion of the present invention, expression and/or activity, in the TIL, of a member selected from the AF4 family, the tyrosine protein kinase family, the bZIP family, the nuclear receptor family, or the ubiquitin family, and/or a functionally active fragment thereof is reduced.

For example, a TIL of the present invention derived from tumor tissue, a tumor-associated lymph node with or without tumor metastasis, a tumor metastatic lesion, a fragment of paracancerous tissue, pleural effusion, and/or ascites and not subjected to in vitro expansion is subjected to first stage in vitro expansion, second stage in vitro expansion, and third stage in vitro expansion, and, in the third stage in vitro expansion of the present invention, expression and/or activity, in the TIL, of a member selected from the AF4 family, the tyrosine protein kinase family, the bZIP family, the nuclear receptor family, or the ubiquitin family, and/or a functionally active fragment thereof is reduced.

For example, a TIL of the present invention derived from tumor tissue, a tumor-associated lymph node with or without tumor metastasis, a tumor metastatic lesion, a fragment of paracancerous tissue, pleural effusion, and/or ascites and not subjected to in vitro expansion is subjected to first stage in vitro expansion, second stage in vitro expansion, and third stage in vitro expansion, and, in the first stage in vitro expansion of the present invention, expression and/or activity, in the TIL, of a member selected from the AF4 family, the tyrosine protein kinase family, the bZIP family, the nuclear receptor family, or the ubiquitin family, and/or a functionally active fragment thereof is reduced, and, in the second stage in vitro expansion of the present invention, expression and/or activity, in the TIL, of a member selected from the AF4 family, the tyrosine protein kinase family, the bZIP family, the nuclear receptor family, or the ubiquitin family, and/or a functionally active fragment thereof is reduced.

For example, a TIL of the present invention derived from tumor tissue, a tumor-associated lymph node with or without tumor metastasis, a tumor metastatic lesion, a fragment of paracancerous tissue, pleural effusion, and/or ascites and not subjected to in vitro expansion is subjected to first stage in vitro expansion, second stage in vitro expansion, and third stage in vitro expansion, and, in the first stage in vitro expansion of the present invention, expression and/or activity, in the TIL, of a member selected from the AF4 family, the tyrosine protein kinase family, the bZIP family, the nuclear receptor family, or the ubiquitin family, and/or a functionally active fragment thereof is reduced, and, in the third stage in vitro expansion of the present invention, expression and/or activity, in the TIL, of a member selected from the AF4 family, the tyrosine protein kinase family, the bZIP family, the nuclear receptor family, or the ubiquitin family, and/or a functionally active fragment thereof is reduced.

For example, tumor-infiltrating lymphocytes (TILs) of the present invention, which are derived from tumor tissue, tumor-associated lymph nodes with or without tumor metastasis, tumor metastatic lesions, fragments of paracancerous tissue, pleural effusion and/or ascites and are not subjected to in vitro expansion, may be subjected to a first stage in vitro expansion, a second stage in vitro expansion, and a third stage in vitro expansion, and, in the second stage in vitro expansion of the present invention, the expression and/or activity, in the TILs, of a member selected from the AF4 family, the tyrosine protein kinase family, the bZIP family, the nuclear receptor family, or the ubiquitin family and/or a functionally active fragment thereof may be reduced and/or attenuated, and, in the third stage in vitro expansion of the present invention, the expression and/or activity, in the TILs, of a member selected from the AF4 family, the tyrosine protein kinase family, the bZIP family, the nuclear receptor family, or the ubiquitin family and/or a functionally active fragment thereof may be reduced and/or attenuated.

For example, tumor-infiltrating lymphocytes (TILs) of the present invention, which are derived from tumor tissue, tumor-associated lymph nodes with or without tumor metastasis, tumor metastatic lesions, fragments of paracancerous tissue, pleural effusion and/or ascites and are not subjected to in vitro expansion, may be subjected to a first stage in vitro expansion, a second stage in vitro expansion, and a third stage in vitro expansion, and, in the first stage in vitro expansion of the present invention, the expression and/or activity, in the TILs, of a member selected from the AF4 family, the tyrosine protein kinase family, the bZIP family, the nuclear receptor family, or the ubiquitin family and/or a functionally active fragment thereof may be reduced and/or attenuated, and, in the second stage in vitro expansion of the present invention, the expression and/or activity, in the TILs, of a member selected from the AF4 family, the tyrosine protein kinase family, the bZIP family, the nuclear receptor family, or the ubiquitin family and/or a functionally active fragment thereof may be reduced and/or attenuated, and, in the third stage in vitro expansion of the present invention, the expression and/or activity, in the TILs, of a member selected from the AF4 family, the tyrosine protein kinase family, the bZIP family, the nuclear receptor family, or the ubiquitin family and/or a functionally active fragment thereof may be reduced and/or attenuated.

For example, each stage of in vitro expansion may be delineated by a change in the number of TIL cells; for example, when the number of TIL cells increases by at least about 1-fold, the TIL cells may be considered to have entered the next stage of in vitro expansion. In some embodiments, when the number of TIL cells increases by at least about 1-1000-fold, for example, at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, at least about 50-fold, at least about 100-fold, at least about 200-fold, at least about 500-fold, or at least about 1000-fold, the TIL cells may be considered to have entered the next stage of in vitro expansion. For example, each stage of in vitro expansion may also be delineated by a change in the conditions of TIL cell culture. For example, when a cell activator and/or a cell growth factor is added or supplemented in the cell culture medium, the TIL cells may be considered to have entered the next stage of in vitro expansion. For example, when IL-2 is added or supplemented in the cell culture medium, the TIL cells may be considered to have entered the next stage of in vitro expansion. For example, when one or more gene regulation systems are added or supplemented in the cell culture medium, the TIL cells may be considered to have entered the next stage of in vitro expansion. For example, when feeder cells are added or supplemented in the cell culture medium, the TIL cells may be considered to have entered the next stage of in vitro expansion. For example, when the TIL cells are subjected to centrifugation and/or cell washing, the TIL cells may be considered to have entered the next stage of in vitro expansion. For example, each stage may also be delineated by the number of days of TIL cell culture. For example, when the TIL cells are cultured in vitro for about 1-100 days, for example, about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 30 days, about 40 days, about 50 days, or about 100 days, the TIL cells may be considered to have entered the next stage of in vitro expansion.

For example, the reduction and/or attenuation of an AF4 family member of the cells comprises inhibition of the function of nuclear transcription activation.

For example, the reduction and/or attenuation of a tyrosine protein kinase family member of the cells comprises inhibition of the function of phosphotransferase.

For example, the reduction and/or attenuation of a bZIP family member of the cells comprises inhibition of the function of DNA binding.

For example, the reduction and/or attenuation of a nuclear receptor family member of the cells comprises inhibition of the function of DNA binding.

For example, the reduction and/or attenuation of a ubiquitin family member of the cells comprises inhibition of the function of RNA binding.

For example, as compared with cells in which the expression and/or activity of a member selected from the AF4 family, the tyrosine protein kinase family, the bZIP family, the nuclear receptor family, or the ubiquitin family is not changed, cells obtained by reducing the expression and/or attenuating the activity of the member selected from the AF4 family, the tyrosine protein kinase family, the bZIP family, the nuclear receptor family, or the ubiquitin family exhibit improved cellular characteristics.

For example, the improved cellular characteristics comprise one or more selected from the group consisting of an improved and increased proportion of viable cells, an improved and enhanced secretion capacity, an enhanced in vitro tumor cell killing capacity, and an enhanced in vivo tumor killing capacity.

For example, the improved cell subpopulation proportion comprises one or more selected from the group consisting of an increased proportion of activated cells, a reduced proportion of regulatory cells, a reduced proportion of exhausted cells, an increased proportion of central memory cells and/or naïve cells, a reduced proportion of apoptotic cells, and an increased proportion of stem cell-like cells.

For example, the improved cell number of the present invention means that, as compared with cells in which the expression and/or activity of a member selected from the AF4 family, the tyrosine protein kinase family, the bZIP family, the nuclear receptor family, or the ubiquitin family is not changed, the cell number of the cells of the present invention, in which the expression of the member selected from the AF4 family, the tyrosine protein kinase family, the bZIP family, the nuclear receptor family, or the ubiquitin family is reduced and/or the activity is attenuated in at least one in vitro expansion stage, may be increased by at least about 1 to 50-fold, for example, at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold.

For example, the increased proportion of viable cells may be manifested as an increase in cell viability. For example, the increased proportion of viable cells of the present invention may mean that, as compared with cells in which the expression and/or activity of a member selected from the AF4 family, the tyrosine protein kinase family, the bZIP family, the nuclear receptor family, or the ubiquitin family is not changed, the proportion of viable cells of the cells of the present invention, in which the expression of the member selected from the AF4 family, the tyrosine protein kinase family, the bZIP family, the nuclear receptor family, or the ubiquitin family is reduced and/or the activity is attenuated in at least one in vitro expansion stage, may be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the enhanced cytokine secretion capacity of the present invention may mean that the cytokine secretion capacity of the cells for cytokines selected from the group consisting of IL-2, IL-6, CD107a, GZMB, TNF-α, and IFN-γ is enhanced. For example, the enhanced cytokine secretion capacity of the present invention may mean that, as compared with cells in which the expression and/or activity of a member selected from the AF4 family, the tyrosine protein kinase family, the bZIP family, the nuclear receptor family, or the ubiquitin family is not changed, the proportion of cells secreting cytokines in the cells of the present invention, in which the expression of the member selected from the AF4 family, the tyrosine protein kinase family, the bZIP family, the nuclear receptor family, or the ubiquitin family is reduced and/or the activity is attenuated in at least one in vitro expansion stage, may be increased by at least about 1 to 50-fold, for example, at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the enhanced cytokine secretion capacity of the present invention may mean that, as compared with cells in which the expression and/or activity of a member selected from the AF4 family, the tyrosine protein kinase family, the bZIP family, the nuclear receptor family, or the ubiquitin family is not changed, the proportion of cells secreting cytokines in the cells of the present invention, in which the expression of the member selected from the AF4 family, the tyrosine protein kinase family, the bZIP family, the nuclear receptor family, or the ubiquitin family is reduced and/or the activity is attenuated in at least one in vitro expansion stage, may be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%. For example, the cytokine secretion capacity of the cells of the present invention is determined by flow cytometry or by a Cytometric Bead Array (CBA).

For example, the enhanced in vitro tumor cell killing capability and/or enhanced in vivo tumor killing capability of the present invention may refer to that, as compared with cells in which the expression and/or activity of a member selected from the AF4 family, the tyrosine protein kinase family, the bZIP family, the nuclear receptor family, or the ubiquitin family is not changed, the tumor cell killing rate of the cells of the present invention, in which, in at least one in vitro expansion stage, the expression of said member selected from the AF4 family, the tyrosine protein kinase family, the bZIP family, the nuclear receptor family, or the ubiquitin family is reduced and/or the activity is attenuated, is increased by at least about 1-50 fold, for example, at least about 1 fold, at least about 2 fold, at least about 3 fold, at least about 4 fold, at least about 5 fold, at least about 6 fold, at least about 7 fold, at least about 8 fold, at least about 9 fold, at least about 10 fold, at least about 11 fold, at least about 12 fold, at least about 13 fold, at least about 14 fold, at least about 15 fold, at least about 20 fold, at least about 30 fold, at least about 40 fold, or at least about 50 fold. For example, the enhanced in vitro tumor cell killing capability and/or enhanced in vivo tumor killing capability of the present invention may refer to that, as compared with cells in which the expression and/or activity of a member selected from the AF4 family, the tyrosine protein kinase family, the bZIP family, the nuclear receptor family, or the ubiquitin family is not changed, the tumor cell killing rate of the cells of the present invention, in which, in at least one in vitro expansion stage, the expression of said member selected from the AF4 family, the tyrosine protein kinase family, the bZIP family, the nuclear receptor family, or the ubiquitin family is reduced and/or the activity is attenuated, is increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%. For example, the tumor cell killing rate of the cells of the present invention may be measured by an IncuCyte system or by a CFSE and DAPI staining method. For example, the tumor cell killing by the cells of the present invention may refer to the ability of the cells to kill solid tumor cells.

For example, the improved cell subpopulation proportions of the present invention may comprise one or more selected from the following group: an increased proportion of CD8⁺ cells, an increased proportion of central memory cells and/or naïve cells, a decreased proportion of regulatory cells, an increased proportion of activated cells, an increased proportion of tumor-specific cells (having a CD103⁺CD39⁺ phenotype), an increased proportion of stem cell-like cells, a decreased proportion of exhausted cells, and a decreased proportion of apoptotic cells.

For example, the increased proportion of CD8⁺ cells of the present invention may be an increase in the proportion of CD8-positive cells in the cells. For example, in the CD8⁺ cells, the proportion is increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the increased proportion of activated cells of the present invention may be an increase in the proportion of CD28⁺, CD25⁺ and/or 41BB⁺ cells in the cells. For example, in the cells, the proportion of activated cells is increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%, or is increased by at least about 1-50 fold, for example, at least about 1 fold, at least about 2 fold, at least about 3 fold, at least about 4 fold, at least about 5 fold, at least about 6 fold, at least about 7 fold, at least about 8 fold, at least about 9 fold, at least about 10 fold, at least about 11 fold, at least about 12 fold, at least about 13 fold, at least about 14 fold, at least about 15 fold, at least about 20 fold, at least about 30 fold, at least about 40 fold, or at least about 50 fold.

For example, the reduced proportion of exhausted cells of the present invention may be a reduction in the proportion of PD-1⁺, LAG-3⁺, TIM-3⁺, CD39⁺, CD38⁺ and/or CD101⁺ cells in the cells. For example, in the cells, the proportion of exhausted cells is reduced by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%, or is reduced by at least about 1-50 fold, for example, at least about 1 fold, at least about 2 fold, at least about 3 fold, at least about 4 fold, at least about 5 fold, at least about 6 fold, at least about 7 fold, at least about 8 fold, at least about 9 fold, at least about 10 fold, at least about 11 fold, at least about 12 fold, at least about 13 fold, at least about 14 fold, at least about 15 fold, at least about 20 fold, at least about 30 fold, at least about 40 fold, or at least about 50 fold.

For example, the reduced proportion of regulatory cells of the present invention may be a reduction in the proportion of CD4⁺CD25⁺Foxp3⁺ cells in the cells. For example, in the cells, the proportion of regulatory cells is reduced by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the reduced proportion of apoptotic cells of the present invention may be a reduction in the proportion of Annexin V⁺7-AAD⁺ cells and/or Annexin V⁺7-AAD⁻ cells in the cells. For example, in the cells, the proportion of apoptotic cells is reduced by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the increased proportion of stem-like cells of the present invention may be an increase in the proportion of CD69⁻CD39⁻ cells and/or TCF1⁺ cells in the cells. For example, in the cells, the proportion of stem-like cells is increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the increased proportion of central memory cells of the present invention may be an increase in the proportion of CD45RA⁻CCR7⁺ or CD45RO⁺CD62L⁺ cells in the cells. For example, in the cells, the proportion of central memory cells is increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the increased proportion of naïve cells in the present invention may be an increase in the proportion of CD45RO⁻CD62L⁺ cells in the cells. For example, the proportion of naïve cells in the cells may be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the method of the present invention may comprise, in vivo, ex vivo, and/or in vitro, editing a target gene of cells. For example, a reduction in an in vivo expression level of the target gene in cells in vivo may be achieved by delivery and editing via an in vivo gene regulation system. For example, in vivo editing of the target gene may be performed by delivering LNPs comprising a gene regulation system or mRNA encoding a gene regulation system by targeting immune cells or precursor cells thereof, such as bone marrow stem cells and the like. By adjusting the composition and/or proportion of LNP components, or by introducing components having targeting capability, the in vivo editing efficiency of the present invention may be improved.

For example, the culture method of the present invention may comprise a gene editing step for cells. For example, it comprises: subjecting the cells to at least one stage, wherein, in in vitro expansion of at least one stage, the gene regulation system is introduced into the cells.

For example, the gene regulation system may disrupt the target gene at the DNA level. For example, the gene regulation system may disrupt a region of the target gene in the genome of the cell or a fragment thereof. For example, after the gene regulation system is used, the DNA region in which the target gene is located in the cell or a fragment thereof is cleaved, such that the expression capability of the target gene is reduced or the activity of the target gene is inhibited. For example, the editing effect of the gene regulation system on the target gene may be long-term and sustained. For example, in the cells of the present invention, the activity of a member selected from the group consisting of the AF4 family, the tyrosine protein kinase family, the bZIP family, the nuclear receptor family, or the ubiquitin family is inhibited.

Wherein the genomic region of the present invention is determined according to the human reference genome hg38 version.

For example, the gene regulation system may comprise a guide nucleic acid molecule and an enzyme protein. For example, the enzyme protein may have nuclease activity, and the guide nucleic acid molecule may guide the enzyme protein to specifically cleave a region in which the target gene is located or a fragment thereof. For example, the guide nucleic acid molecule and the enzyme protein may exist in the form of a ribonucleoprotein complex (RNP), or may each independently exist alone. For example, the enzyme protein may comprise a Cas protein. For example, a polynucleotide encoding gRNA and a Cas protein may be introduced into a target cell, or may each independently be introduced alone into the target cell.

For example, reduction and/or weakening of expression and/or activity of the target gene of the cell in the present invention may comprise: introducing a ribonucleoprotein complex (RNP) comprising the guide nucleic acid molecule and the enzyme protein into the cell. For example, the enzyme protein may comprise a Cas protein, a Cas protein homolog, or a functionally active fragment thereof. For example, the guide nucleic acid molecule may comprise a guide RNA (gRNA). For example, a complex comprising a polynucleotide encoding gRNA and a Cas protein may be introduced into the cell. For example, a complex comprising gRNA and a Cas protein may be introduced into the cell.

For example, the gRNA may be used to bind to a sequence of the target gene. For example, binding of the gRNA to the sequence of the target gene may be fully complementary, may be partially complementary, or may hybridize to the sequence of the target gene under moderately stringent or stringent conditions. For example, binding of the gRNA to the sequence of the target gene may enable the CRISPR system of the gRNA to specifically cleave the target gene.

For example, the editing target region of the present invention may be a region upstream of a start codon. For example, the editing target region of the present invention may be a region having high transcription factor binding affinity. For example, the editing target region of the present invention may be a region having a specific number of transcription factor binding sites. For example, the editing target region of the present invention may be a continuous region having about 3 or more transcription factor binding sites. For example, the genomic coordinates of the editing target region of the present invention may be selected from the preferred targeted sub-regions shown in Tables 1C to 1G.

For example, the guide nucleic acid molecule targeting AFF3 of the present invention may bind to a region or a fragment thereof selected from the group consisting of: SEQ ID NO: 28116-34100.

For example, the guide nucleic acid molecule targeting AXL of the present invention may bind to a region or a fragment thereof selected from the group consisting of: SEQ ID NO: 34101-36390.

For example, the guide nucleic acid molecule targeting NFE2L1 of the present invention may bind to a region or a fragment thereof selected from the group consisting of: SEQ ID NO: 36391-37812.

For example, the guide nucleic acid molecule targeting RARG of the present invention may bind to a region or a fragment thereof selected from the group consisting of: SEQ ID NO: 37813-39627.

For example, the guide nucleic acid molecule targeting UBFD1 of the present invention may bind to a region or a fragment thereof selected from the group consisting of: SEQ ID NO: 39628-41119.

For example, when the gene editing system comprises CRISPR/Cas9, a protospacer adjacent motif (PAM) may be present downstream of the region targeted by the guide nucleic acid molecule of the present invention, and the protospacer adjacent motif (PAM) may be AGG, TGG, GGG, or CGG. For example, when the PAM region of the target gene is determined, a person skilled in the art can readily determine a target sequence consisting of about 15 to about 25 (e.g., about 15, about 16, about 17, about 18, about 19, about 20, about 21, about 22, about 23, about 24, about 25) nucleotides upstream of the 5' end of the PAM of the target gene, and an appropriate gRNA may be designed for the target sequence. For example, the guide nucleic acid molecule is capable of binding to a sequence consisting of about 15 to about 25 nucleotides upstream of the 5' end of a protospacer adjacent motif (PAM) selected from the group consisting of: AGG, TGG, GGG, and CGG.

For example, when the gene editing system comprises CRISPR/Cas12, a protospacer adjacent motif (PAM) may be present upstream of the region targeted by the guide nucleic acid molecule of the present invention, and the protospacer adjacent motif (PAM) may be NTTN, TTYN, VTTV, TRTV, TTTV, TATV, TYCV, TNN, or NTN, and may also be TTTN, ATTN, GTTN, CTTN, TTC, TTG, TTA, TTT, TAN, TGN, or TCN, wherein N is A, T, C, or G, Y is T or C, V is A, C, or G, and R is A or G. For example, the protospacer adjacent motif (PAM) may be TTTN. For example, the protospacer adjacent motif (PAM) may be TTN. For example, when the PAM region of the target gene is determined, a person skilled in the art can readily determine a target sequence consisting of about 15 to about 25 (e.g., about 15, about 16, about 17, about 18, about 19, about 20, about 21, about 22, about 23, about 24, about 25) nucleotides downstream of the 3' end of the PAM of the target gene, and an appropriate gRNA may be designed for the target sequence. For example, the guide nucleic acid molecule is capable of binding to a sequence consisting of about 15 to about 25 nucleotides downstream of the 3' end of a protospacer adjacent motif (PAM) selected from the group consisting of: NTTN, TTYN, VTTV, TRTV, TTTV, TATV, TYCV, TNN, or NTN, and may also be TTTN, ATTN, GTTN, CTTN, TTC, TTG, TTA, TTT, TAN, TGN, or TCN, wherein N is A, T, C, or G, Y is T or C, V is A, C, or G, and R is A or G. For example, the protospacer adjacent motif (PAM) may be TTTN. For example, the protospacer adjacent motif (PAM) may be TTN.

For example, when the gene editing system of the present invention comprises wild-type Cas12a (which may also be referred to as Cpf1, such as AsCas12a, FnCas12a, LbCas12a, BbCas12a, CMaCas12a, and OsCas12a), a PAM sequence selected from the following may be present upstream of the region targeted by the guide nucleic acid molecule of the present invention: NTTN, wherein N may be A, T, C, or G. For example, when the PAM region of the target gene is determined, a person skilled in the art can readily determine a target sequence consisting of about 17 to about 25 (e.g., about 17, about 18, about 19, about 20, about 21, about 22, about 23, about 24, or about 25) nucleotides downstream of the 3' end of the PAM of the target gene, and an appropriate gRNA may be designed for the target sequence.

For example, when the gene editing system of the present invention comprises a mutant Cas12a, such as enAsCas12a (mutation sites E174R, S542R, and K548R), a PAM sequence selected from the following may be present upstream of the region targeted by the guide nucleic acid molecule of the present invention: TTYN (TTTN/TTCN), VTTV (ATTV/CTTV/GTTV), or TRTV (TATV/TGTV), wherein N may be A, T, C, or G, Y may be T or C, V may be A, C, or G, and R may be A or G. For example, when the PAM region of the target gene is determined, a person skilled in the art can readily determine a target sequence consisting of about 17 to about 25 (e.g., about 17, about 18, about 19, about 20, about 21, about 22, about 23, about 24, or about 25) nucleotides downstream of the 3' end of the PAM of the target gene, and an appropriate gRNA may be designed for the target sequence.

For example, when the gene editing system of the present invention comprises a mutant Cas12a, such as opAsCas12a (mutation sites: E174R and S542R), a PAM sequence selected from the following may be present upstream of the region targeted by the guide nucleic acid molecule of the present invention: TTTV (TTTA, TTTC, or TTTG), wherein V may be A, C, or G. For example, when the PAM region of the target gene is determined, a person skilled in the art can readily determine a target sequence consisting of about 17 to about 25 (e.g., about 17, about 18, about 19, about 20, about 21, about 22, about 23, about 24, or about 25) nucleotides downstream of the 3' end of the PAM of the target gene, and an appropriate gRNA may be designed for the target sequence.

For example, when the gene editing system of the present invention comprises a mutant Cas12a, such as AsCas12a Ultra (mutation sites: M537R and F870L), a PAM sequence selected from the following is present upstream of the region targeted by the guide nucleic acid molecule of the present invention: TTTV, TATV, or TYCV, wherein V is A, C, or G, and Y is T or C. For example, when the PAM region of a target gene is determined, a target sequence composed of about 17 to about 25 (e.g., about 17, about 18, about 19, about 20, about 21, about 22, about 23, about 24, or about 25) nucleotides downstream of the 3' end of the PAM of the target gene is readily determined by a person skilled in the art, and an appropriate gRNA is designed for the target sequence.

For example, when the gene editing system of the present invention comprises a mutant Cas12a, such as hfCas12Max (mutation sites: N243R/E336R/D892R) and Cas12Max (mutation site: N243R), a PAM sequence selected from the following is present upstream of the region targeted by the guide nucleic acid molecule of the present invention: TNN or NTN, wherein N is A, T, C, or G. For example, when the PAM region of a target gene is determined, a target sequence composed of about 17 to about 25 (e.g., about 17, about 18, about 19, about 20, about 21, about 22, about 23, about 24, or about 25) nucleotides downstream of the 3' end of the PAM of the target gene is readily determined by a person skilled in the art, and an appropriate gRNA is designed for the target sequence.

For example, when the gene editing system of the present invention comprises a wild-type Cas12b or a mutant Cas12b (such as an AaCas12b protein from Alicyclobacillus acidiphilus), a PAM sequence selected from the following is present upstream of the region targeted by the guide nucleic acid molecule of the present invention: TTN, wherein N is A, T, C, or G. For example, when the PAM region of a target gene is determined, a target sequence composed of about 17 to about 25 (e.g., about 17, about 18, about 19, about 20, about 21, about 22, about 23, about 24, or about 25) nucleotides downstream of the 3' end of the PAM of the target gene is readily determined by a person skilled in the art, and an appropriate gRNA is designed for the target sequence.

For example, when the gene editing system of the present invention comprises a wild-type Cas12i or a mutant Cas12i (a Cas protein having a smaller size), a PAM sequence selected from the following is present upstream of the region targeted by the guide nucleic acid molecule of the present invention: TTN or TTTN, wherein N is A, T, C, or G. For example, when the PAM region of a target gene is determined, a target sequence composed of about 17 to about 25 (e.g., about 17, about 18, about 19, about 20, about 21, about 22, about 23, about 24, or about 25) nucleotides downstream of the 3' end of the PAM of the target gene is readily determined by a person skilled in the art, and an appropriate gRNA is designed for the target sequence.

For example, the guide nucleic acid molecule comprises a target sequence composed of about 15 to about 25 nucleotides upstream of a PAM region indicated by AGG, TGG, GGG and/or CGG in DNA in which a gene encoding a member selected from the AF4 family, the tyrosine protein kinase family, the bZIP family, the nuclear receptor family, or the ubiquitin family and/or a functionally active fragment thereof is located. For example, the guide nucleic acid molecule comprises a target sequence composed of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 22 to about 23, about 15 to about 21, about 17 to about 21, about 19 to about 21, about 20 to about 21, about 15 to about 20, about 17 to about 20, about 19 to about 20, about 15 to about 19, about 17 to about 19, or about 15 to about 17 nucleotides upstream of a PAM region indicated by AGG, TGG, GGG and/or CGG in DNA in which a gene encoding a member selected from the AF4 family, the tyrosine protein kinase family, the bZIP family, the nuclear receptor family, or the ubiquitin family and/or a functionally active fragment thereof is located. For example, the target sequence is a region defined by genomic coordinates shown in Tables 2C-2G or a fragment thereof.

For example, the guide nucleic acid molecule comprises an sgRNA targeting AFF3 as shown in any one of SEQ ID NO: 3328-9312, an sgRNA targeting AXL as shown in any one of SEQ ID NO: 9313-11602, an sgRNA targeting NFE2L1 as shown in any one of SEQ ID NO: 11603-13024, an sgRNA targeting RARG as shown in any one of SEQ ID NO: 13025-14839, or an sgRNA targeting UBFD1 as shown in any one of SEQ ID NO: 14840-16331.

For example, as compared with cells in which the expression and/or activity of the target gene is not changed, in cells obtained by reducing the expression and/or weakening the activity of the target gene of the cells, the proportion of cells expressing a product of the target gene is reduced and/or the expression level of the target gene in a single cell is decreased.

For example, in the method of the present invention, as compared with cells in which the expression and/or activity of the target gene is not changed, the proportion of cells expressing a product of the target gene in cells obtained by reducing the expression and/or weakening the activity of the target gene of the cells is reduced by at least about 5%. For example, the proportion of cells expressing a product of a gene encoding a member selected from the AF4 family, the tyrosine protein kinase family, the bZIP family, the nuclear receptor family, or the ubiquitin family and/or a functionally active fragment thereof is reduced by at least about 100-5%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the proportion of cells expressing a product of a gene encoding a member selected from the AF4 family, the tyrosine protein kinase family, the bZIP family, the nuclear receptor family, or the ubiquitin family and/or a functionally active fragment thereof is from an observable proportion of cells to 1%. For example, the proportion of cells expressing a product of a gene encoding a member selected from the AF4 family, the tyrosine protein kinase family, the bZIP family, the nuclear receptor family, or the ubiquitin family and/or a functionally active fragment thereof is reduced to at least about 100-1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, or at least about 1%. For example, the proportion of cells expressing a product of a gene encoding a member selected from the AF4 family, the tyrosine protein kinase family, the bZIP family, the nuclear receptor family, or the ubiquitin family and/or a functionally active fragment thereof is detected by a flow cytometer.

For example, in the method of the present invention, the proportion of cells expressing a product of a gene encoding a member selected from the AF4 family, the tyrosine protein kinase family, the bZIP family, the nuclear receptor family, or the ubiquitin family and/or a functionally active fragment thereof in cells obtained by reducing the expression and/or weakening the activity of the target gene of the cells is at most about 95%. For example, the proportion of cells expressing a product of a gene encoding a member selected from the AF4 family, the tyrosine protein kinase family, the bZIP family, the nuclear receptor family, or the ubiquitin family and/or a functionally active fragment thereof is at most about 95-5%, for example, at most about 95%, at most about 90%, at most about 80%, at most about 70%, at most about 60%, at most about 50%, at most about 40%, at most about 30%, at most about 20%, at most about 19%, at most about 18%, at most about 17%, at most about 16%, at most about 15%, at most about 14%, at most about 13%, at most about 12%, at most about 11%, at most about 10%, at most about 9%, at most about 8%, at most about 7%, at most about 6%, or at most about 5%. For example, the proportion of cells expressing a product of a gene encoding a member selected from the AF4 family, the tyrosine protein kinase family, the bZIP family, the nuclear receptor family, or the ubiquitin family and/or a functionally active fragment thereof is detected by a flow cytometer.

For example, in the method of the present invention, as compared with cells in which the expression and/or activity of the target gene is not changed, the expression level of the target gene in a single cell in cells obtained by reducing the expression and/or weakening the activity of the target gene of the cells is decreased by at least about 5%. For example, the expression level of the target gene in a single cell is decreased by at least about 100-5%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the expression level of the target gene in a single cell is from an observable expression level to 1%. For example, the expression level of the target gene in a single cell is decreased to at least about 100-1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, or at least about 1%.

For example, in cells obtained by reducing the expression and/or weakening the activity of the target gene of the cells by the method of the present invention, the expression level of the target gene in a single cell is up to about 95% of that in cells in which the expression and/or activity of the target gene is not altered. For example, the expression level, in a single cell, of a gene encoding a member selected from the AF4 family, the tyrosine protein kinase family, the bZIP family, the nuclear receptor family, or the ubiquitin family and/or a functionally active fragment thereof (e.g., a gene encoding AFF3, AXL, NFE2L1, RARG, or UBFD1) is up to about 95-5% of that in cells in which the expression and/or activity of the member selected from the AF4 family, the tyrosine protein kinase family, the bZIP family, the nuclear receptor family, or the ubiquitin family and/or the functionally active fragment thereof is not altered, for example, up to about 95%, up to about 90%, up to about 80%, up to about 70%, up to about 60%, up to about 50%, up to about 40%, up to about 30%, up to about 20%, up to about 19%, up to about 18%, up to about 17%, up to about 16%, up to about 15%, up to about 14%, up to about 13%, up to about 12%, up to about 11%, up to about 10%, up to about 9%, up to about 8%, up to about 7%, up to about 6%, or up to about 5%.

For example, the method of the present invention comprises subjecting the cells to at least one stage, wherein, in the in vitro expansion of the at least one stage, the expression of a member selected from the AF4 family, the tyrosine protein kinase family, the bZIP family, the nuclear receptor family, or the ubiquitin family in the cells is reduced and/or the activity thereof is weakened.

For example, TIL derived from tumor tissue, tumor-associated lymph nodes with or without tumor metastasis, tumor metastatic lesions, fragments of paracancerous tissue, pleural effusion and/or ascites and not subjected to in vitro expansion is subjected to a first stage in vitro expansion and a second stage in vitro expansion, wherein during the second stage in vitro expansion, the expression of a member selected from the AF4 family, the tyrosine protein kinase family, the bZIP family, the nuclear receptor family, or the ubiquitin family in the TIL subjected to the first stage in vitro expansion is reduced and/or the activity thereof is weakened.

For example, the first stage in vitro expansion is performed for at least 7 days. For example, the second stage in vitro expansion is performed for at least about 7 days.

For example, in the in vitro expansion of the present invention in a single stage, the cells are contacted with the one or more cell activators, and the expression and/or activity, in the cells, of a member selected from the AF4 family, the tyrosine protein kinase family, the bZIP family, the nuclear receptor family, or the ubiquitin family and/or a functionally active fragment thereof is reduced. For example, the cell activator comprises an agonist of one or more targets selected from the group consisting of: CD3, CD28, HVEM, CD40L, OX40, and 4-1BB. For example, in the in vitro expansion in the single stage, a member selected from the AF4 family, the tyrosine protein kinase family, the bZIP family, the nuclear receptor family, or the ubiquitin family in the cells of the present invention is reduced and/or weakened, and the cells are contacted with one or more cell activators of the present invention. For example, in the first stage in vitro expansion of the present invention, a member selected from the AF4 family, the tyrosine protein kinase family, the bZIP family, the nuclear receptor family, or the ubiquitin family in the TIL of the present invention is reduced and/or weakened, and the TIL is contacted with one or more cell activators of the present invention. For example, in the second stage in vitro expansion of the present invention, a member selected from the AF4 family, the tyrosine protein kinase family, the bZIP family, the nuclear receptor family, or the ubiquitin family in the TIL of the present invention is reduced and/or weakened, and the TIL is contacted with one or more cell activators of the present invention. For example, in the third stage in vitro expansion of the present invention, a member selected from the AF4 family, the tyrosine protein kinase family, the bZIP family, the nuclear receptor family, or the ubiquitin family in the TIL of the present invention is reduced and/or weakened, and the TIL is contacted with one or more cell activators of the present invention.

For example, in the in vitro expansion in a single stage, the cells of the present invention substantially simultaneously reduce the expression and/or weaken the activity of a member selected from the AF4 family, the tyrosine protein kinase family, the bZIP family, the nuclear receptor family, or the ubiquitin family and are contacted with one or more cell activators of the present invention. For example, in the in vitro expansion in a single stage, the cells of the present invention first reduce the expression and/or weaken the activity of a member selected from the AF4 family, the tyrosine protein kinase family, the bZIP family, the nuclear receptor family, or the ubiquitin family, for example, 2-48 hours in advance, for example, 2 hours in advance, 4 hours in advance, 8 hours in advance, 12 hours in advance, 24 hours in advance, or 48 hours in advance, and then are contacted with one or more cell activators of the present invention. For example, in the in vitro expansion in a single stage, the cells of the present invention are first contacted with one or more cell activators of the present invention, for example, 2-48 hours in advance, for example, 2 hours in advance, 4 hours in advance, 8 hours in advance, 12 hours in advance, 24 hours in advance, or 48 hours in advance, and then the expression and/or activity of a member selected from the AF4 family, the tyrosine protein kinase family, the bZIP family, the nuclear receptor family, or the ubiquitin family is reduced and/or weakened.

For example, in the first stage in vitro expansion of the present invention, the TIL of the present invention substantially simultaneously reduce the expression and/or weaken the activity of a member selected from the AF4 family, the tyrosine protein kinase family, the bZIP family, the nuclear receptor family, or the ubiquitin family and are contacted with one or more cell activators of the present invention. For example, in the second stage in vitro expansion of the present invention, the TIL of the present invention substantially simultaneously reduce the expression and/or weaken the activity of a member selected from the AF4 family, the tyrosine protein kinase family, the bZIP family, the nuclear receptor family, or the ubiquitin family and are contacted with one or more cell activators of the present invention. For example, in the third stage in vitro expansion of the present invention, the TIL of the present invention substantially simultaneously reduce the expression and/or weaken the activity of a member selected from the AF4 family, the tyrosine protein kinase family, the bZIP family, the nuclear receptor family, or the ubiquitin family and are contacted with one or more cell activators of the present invention.

For example, the second stage in vitro expansion of the present invention is performed for at least about 9 days. For example, the second stage in vitro expansion of the present invention is performed for at least about 9 days, at least about 10 days, at least about 11 days, at least about 12 days, at least about 13 days, or at least about 14 days. For example, the second stage in vitro expansion of the present invention is performed for about 9 days to about 14 days, about 10 days to about 14 days, about 11 days to about 14 days, about 12 days to about 14 days, about 13 days to about 14 days, about 9 days to about 13 days, about 10 days to about 13 days, about 11 days to about 13 days, about 12 days to about 13 days, about 9 days to about 12 days, about 10 days to about 12 days, about 11 days to about 12 days, or about 10 days to about 11 days. For example, the second stage in vitro expansion of the present invention is considered a REP (rapid expansion protocol) stage. For example, the first stage in vitro expansion of the present invention is considered a preREP stage.

For example, the number of days for which the second stage in vitro expansion of the present invention is performed is calculated from the start time of the second stage in vitro expansion. For example, at the time when the second stage in vitro expansion starts, it is considered that the second stage in vitro expansion has been performed for about 0 hours. For example, after about 24 hours have elapsed after the start of the second stage in vitro expansion, it is considered that the second stage in vitro expansion has been performed for about 1 day. For example, on the day when the second stage in vitro expansion starts, it is considered that the second stage in vitro expansion has been performed for about 0 days. For example, the number of days for which the second stage in vitro expansion of the present invention is performed is calculated by the number of days for which the second stage in vitro expansion is performed. For example, on the second day after the start of the second stage in vitro expansion, it is considered that the second stage in vitro expansion has been performed for about 1 day.

For example, the cell activator of the present invention comprises one or more selected from the group consisting of: CD80, CD86, B7-H3, 4-1BBL, CD27, CD30, CD134, B7h, CD40, LIGHT, and functionally active fragments thereof. For example, the cell activator of the present invention comprises an agonist of one or more targets selected from the group consisting of: CD3, CD28, HVEM, CD40L, OX40, and 4-1BB. For example, the cell activator of the present invention comprises an antibody against CD3, CD28, HVEM, CD40L, OX40, or 4-1BB and an antigen-binding fragment thereof. For example, the cell activator of the present invention comprises a CD3 agonist. For example, the cell activator of the present invention comprises an anti-CD3 antibody and/or an antigen-binding fragment thereof, for example, Miltenyi Biotech OKT3, or BD SP34. For example, the cell activator of the present invention comprises a CD28 agonist. For example, the cell activator of the present invention comprises an anti-CD28 antibody and/or an antigen-binding fragment thereof, for example, Merck 15E8.

For example, the cell activator of the present invention comprises an anti-CD3 antibody and/or an antigen-binding fragment thereof, for example, comprises the light chain VL and heavy chain VH of OKT3 from Miltenyi Biotech, and comprises the light chain VL and heavy chain VH of SP34 from BD. For example, the cell activator of the present invention comprises a CD28 agonist. For example, the cell activator of the present invention comprises an anti-CD28 antibody and/or an antigen-binding fragment thereof, for example, comprises the light chain VL and heavy chain VH of Merck 15E8. For example, the cell activator of the present invention comprises an anti-CD3 antibody and/or an antigen-binding fragment thereof, for example, comprises the light chain LCDR1-3 and heavy chain HCDR1-3 of OKT3 from Miltenyi Biotech, and comprises the light chain LCDR1-3 and heavy chain HCDR1-3 of SP34 from BD, and the anti-CD3 antibody and/or antigen-binding fragment thereof of the present invention has CD3 binding ability. For example, the cell activator of the present invention comprises a CD28 agonist. For example, the cell activator of the present invention comprises an anti-CD28 antibody and/or an antigen-binding fragment thereof, for example, comprises the light chain LCDR1-3 and heavy chain HCDR1-3 of Merck 15E8, and the anti-CD28 antibody and/or antigen-binding fragment thereof of the present invention has CD28 binding ability. In the present invention, the antibody or antigen-binding protein of the present invention comprises at least one CDR in the antibody heavy chain variable region VH and/or at least one CDR in the antibody light chain variable region VL. The CDR of the present invention is defined according to the IMGT nomenclature, the CDR of the present invention is defined according to Chothia, or the CDR of the present invention is defined according to Kabat.

For example, contacting the cells of the present invention with one or more cell activators of the present invention may comprise one or more modes selected from the group consisting of: (1) a cell activator of the present invention is added to a cell culture medium of the cells of the present invention; (2) a cell activator of the present invention is added to a cell culture medium of the cells of the present invention; and (3) an addition comprising a cell activator of the present invention is added to a cell culture medium of the cells of the present invention. For example, contacting the cells of the present invention with one or more cell activators of the present invention may comprise adding an addition comprising a cell activator of the present invention to a cell culture medium of the cells of the present invention. For example, contacting the cells of the present invention with one or more cell activators of the present invention may comprise adding an addition comprising a CD28 antibody and a CD3 antibody of the present invention to a cell culture medium of the cells of the present invention.

For example, an initial concentration of the cell activator in a cell culture medium of the cells of the present invention may be at least about 30 ng/mL. For example, an initial concentration of the CD28 antibody of the present invention in a cell culture medium of the cells of the present invention may be at least about 30 ng/mL; for example, an initial concentration of the CD3 antibody of the present invention in a cell culture medium of the cells of the present invention may be at least about 30 ng/mL. For example, selection of the initial concentration of the CD28 antibody of the present invention may be independent of selection of the initial concentration of the CD3 antibody of the present invention; for example, the initial concentrations of the CD28 antibody of the present invention and the CD3 antibody of the present invention in a cell culture medium of the cells of the present invention may be combined in any manner. For example, the initial concentration of the CD28 antibody of the present invention in a cell culture medium of the cells of the present invention may be optionally selected from about 30 ng/mL to about 300 ng/mL. For example, the initial concentration of the CD3 antibody of the present invention in a cell culture medium of the cells of the present invention may be optionally selected from about 30 ng/mL to about 300 ng/mL. For example, the initial concentration of the CD28 antibody of the present invention in a cell culture medium of the cells of the present invention may be optionally selected from about 30 ng/mL to about 300 ng/mL, and the initial concentration of the CD3 antibody of the present invention in a cell culture medium of the cells of the present invention may be optionally selected from about 30 ng/mL to about 300 ng/mL, and selection of the initial concentration of the CD28 antibody of the present invention may be independent of selection of the initial concentration of the CD3 antibody of the present invention. For example, the solid-phase medium of the present invention may be about 500 nm to about 10 µm. For example, the solid-phase medium of the present invention may be measured by transmission electron microscopy. For example, the solid-phase medium of the present invention may be about 1 nm to about 500 nm. For example, the solid-phase medium of the present invention may be about 100 nm to about 500 nm. For example, the solid-phase medium of the present invention may be about 200 nm to about 500 nm. For example, the solid-phase medium of the present invention may be measured by transmission electron microscopy.

For example, the solid-phase medium of the present invention may comprise a polymer. For example, the solid-phase medium of the present invention may comprise dextran.

For example, each mg of the solid-phase medium of the present invention comprises at least about 25 µg of the cell activator of the present invention.

For example, at a ratio of the solid-phase medium of the present invention to the cells of the present invention of about 100:1 to about 1:2000, preferably about 1:100 to about 1:2000, a solid-phase medium comprising the cell activator of the present invention is added to a cell culture medium of the cells of the present invention. For example, at a ratio of the solid-phase medium of the present invention to the cells of the present invention of about 2:1 to about 1:2, a solid-phase medium comprising the cell activator of the present invention is added to a cell culture medium of the cells of the present invention.

For example, when a diameter of the solid-phase medium of the present invention is about 500 nm to about 10 µm, a solid-phase medium comprising the cell activator of the present invention may be added to a cell culture medium of the cells of the present invention at a ratio of the solid-phase medium of the present invention to the cells of the present invention of about 2:1 to about 1:2. For example, when a diameter of the solid-phase medium of the present invention is about 500 nm to about 10 µm, a solid-phase medium comprising the cell activator of the present invention, for example, a CD3 agonist and/or a CD28 agonist, may be added to a cell culture medium of the cells of the present invention at a ratio of the solid-phase medium of the present invention to the cells of the present invention of about 2:1 to about 1:2, about 2:1 to about 1:1, or about 1:1 to about 1:2.

For example, when a diameter of the solid-phase medium of the present invention is about 100 nm to about 500 nm, a solid-phase medium comprising the cell activator of the present invention may be added to a cell culture medium of the cells of the present invention at a ratio of the solid-phase medium of the present invention to the cells of the present invention of about 1:100 to about 1:2000. For example, when a diameter of the solid-phase medium of the present invention is about 100 nm to about 500 nm, at a ratio of the solid-phase medium of the present invention to the cells of the present invention of about 1:100 to about 1:2000, about 1:200 to about 1:2000, about 1:300 to about 1:2000, about 1:400 to about 1:2000, about 1:500 to about 1:2000, about 1:600 to about 1:2000, about 1:700 to about 1:2000, about 1:800 to about 1:2000, about 1:900 to about 1:2000, about 1:1000 to about 1:2000, about 1:1200 to about 1:2000, about 1:1400 to about 1:2000, about 1:1600 to about 1:2000, or about 1:1800 to about 1:2000, a solid-phase medium comprising a CD28 agonist and a CD3 agonist of the present invention may, for example, be added to a cell culture medium of the cells of the present invention.

For example, the method of the present invention may further comprise: during in vitro expansion of the present invention at at least one stage, the cells of the present invention are contacted with one or more cell growth factors.

For example, in in vitro expansion of the present invention in a single stage, the cells of the present invention may be contacted with the cell activator of the present invention and may be contacted with one or more cell growth factors of the present invention. For example, in first stage in vitro expansion of the present invention, the TIL of the present invention may be contacted with the cell activator of the present invention and may be contacted with one or more cell growth factors of the present invention. For example, in second stage in vitro expansion of the present invention, the TIL of the present invention may be contacted with the cell activator of the present invention and may be contacted with one or more cell growth factors of the present invention. For example, in third stage in vitro expansion of the present invention, the TIL of the present invention may be contacted with the cell activator of the present invention and may be contacted with one or more cell growth factors of the present invention.

For example, in in vitro expansion of the present invention in a single stage, the cells of the present invention may be contacted with the cell activator of the present invention and one or more cell growth factors of the present invention. For example, in in vitro expansion of the present invention in a single stage, the cells of the present invention may be contacted substantially simultaneously with one or more cell growth factors of the present invention and one or more cell activators of the present invention. For example, in in vitro expansion of the present invention in a single stage, the cells of the present invention may first be contacted with one or more cell growth factors of the present invention, for example, 2-48 hours in advance, for example, 2 hours, 4 hours, 8 hours, 12 hours, 24 hours, or 48 hours in advance, and then may be contacted with one or more cell activators of the present invention. For example, in in vitro expansion of the present invention in a single stage, the cells of the present invention may first be contacted with one or more cell activators of the present invention, for example, 2-48 hours in advance, for example, 2 hours, 4 hours, 8 hours, 12 hours, 24 hours, or 48 hours in advance, and then may be contacted with one or more cell growth factors of the present invention.

For example, in first stage in vitro expansion of the present invention, the cells of the present invention may be contacted with the cell activator of the present invention and one or more cell growth factors of the present invention. For example, in second stage in vitro expansion of the present invention, the TIL of the present invention may be contacted with the cell activator of the present invention and one or more cell growth factors of the present invention. For example, in third stage in vitro expansion of the present invention, the TIL of the present invention may be contacted with the cell activator of the present invention and one or more cell growth factors of the present invention.

For example, the cell growth factor of the present invention may be one or more selected from the group consisting of: IL-2, IL-7, IL-12, IL-15, IL-21, interferon-γ, and functionally active fragments thereof. For example, the cell growth factor of the present invention may comprise IL-2 and/or a functionally active fragment thereof. For example, the functionally active fragment of IL-2 may comprise a fragment of IL-2 known in the art that is capable of binding to an IL-2 receptor of a cell. For example, the cell growth factor of the present invention may comprise IL-2 and/or a functionally active fragment thereof, IL-7 and/or a functionally active fragment thereof, and IL-15 and/or a functionally active fragment thereof.

For example, contacting the cells of the present invention with one or more cell growth factors of the present invention may comprise adding the cell growth factor of the present invention to a cell culture medium of the cells of the present invention. For example, an initial concentration of the cell growth factor in a cell culture medium of the cells of the present invention may be at least about 300 IU/mL. For example, an initial concentration of IL-2 of the present invention in a cell culture medium of the cells of the present invention may be at least about 300-9000 IU/mL, for example, at least about 300 IU/mL, at least about 350 IU/mL, at least about 400 IU/mL, at least about 500 IU/mL, at least about 600 IU/mL, at least about 700 IU/mL, at least about 800 IU/mL, at least about 900 IU/mL, at least about 1000 IU/mL, at least about 1100 IU/mL, at least about 1200 IU/mL, at least about 1300 IU/mL, at least about 1400 IU/mL, at least about 1500 IU/mL, at least about 2000 IU/mL, at least about 2500 IU/mL, at least about 2600 IU/mL, at least about 2700 IU/mL, at least about 2800 IU/mL, at least about 2900 IU/mL, at least about 3000 IU/mL, at least about 3100 IU/mL, at least about 3200 IU/mL, at least about 3300 IU/mL, at least about 3400 IU/mL, at least about 3500 IU/mL, at least about 4000 IU/mL, at least about 4500 IU/mL, at least about 5000 IU/mL, at least about 5500 IU/mL, at least about 6000 IU/mL, at least about 6500 IU/mL, at least about 7000 IU/mL, at least about 7500 IU/mL, at least about 8000 IU/mL, at least about 8500 IU/mL, or at least about 9000 IU/mL.

For example, contacting the cells of the present invention with IL-2, IL-7, and IL-15, as compared with contacting only with IL-2, may reduce an amount of cytokines used. For example, an amount of IL-2 added may be reduced under conditions in which IL-7 and IL-15 are added. For example, a concentration of IL-7 may be about 1 to 1000 ng/mL, preferably about 1-100 ng/mL. For example, a concentration of IL-15 may be about 1 to 1000 ng/mL, preferably about 1-100 ng/mL. For example, for an amount of IL-2 added, it may be reduced to a range commonly used in the art for various immune cells, for example, reduced to 50-10% of the commonly used range in the art, for example, 50%, 20%, or 10%. For example, for TCR-T, the commonly used range in the art for an amount of IL-2 added may be 30-300 IU/mL. For example, for TIL, the commonly used range in the art for an amount of IL-2 added may be 300-9000 IU/mL (for example, 1000-9000 IU/mL).

For example, the method of the present invention can further comprise: in the in vitro expansion of the present invention at at least one stage, the cells of the present invention can be co-cultured with feeder cells of the present invention.

For example, in the in vitro expansion of the present invention at a single stage, the cells of the present invention can be brought into contact with one or more cell activators and/or one or more cell growth factors and can be co-cultured with feeder cells of the present invention; for example, the in vitro expansion of the present invention at a single stage can refer to the in vitro expansion of the present invention in the same stage, for example, in the first stage in vitro expansion of the present invention, in the second stage in vitro expansion of the present invention, or in the third stage in vitro expansion of the present invention, and the like.

For example, in the first stage in vitro expansion of the present invention, the TIL of the present invention can be brought into contact with one or more cell activators and/or one or more cell growth factors and can be co-cultured with feeder cells of the present invention. For example, in the second stage in vitro expansion of the present invention, the TIL of the present invention can be brought into contact with one or more cell activators of the present invention and/or one or more cell growth factors of the present invention and can be co-cultured with feeder cells of the present invention. For example, in the third stage in vitro expansion of the present invention, the TIL of the present invention can be brought into contact with one or more cell activators of the present invention and/or one or more cell growth factors of the present invention and can be co-cultured with feeder cells of the present invention.

For example, in the in vitro expansion of the present invention at a single stage, after the cells of the present invention are brought into contact with one or more cell activators of the present invention and/or one or more cell growth factors for a certain period of time, the cells can then be co-cultured with feeder cells of the present invention. For example, in the first stage in vitro expansion of the present invention, after the TIL of the present invention are brought into contact with one or more cell activators of the present invention and/or one or more cell growth factors for a certain period of time, the TIL can then be co-cultured with feeder cells of the present invention. For example, in the second stage in vitro expansion of the present invention, after the TIL of the present invention are brought into contact with one or more cell activators of the present invention and/or one or more cell growth factors for a certain period of time, the TIL can then be co-cultured with feeder cells of the present invention. For example, in the third stage in vitro expansion of the present invention, after the TIL of the present invention are brought into contact with one or more cell activators of the present invention and/or one or more cell growth factors for a certain period of time, the TIL can then be co-cultured with feeder cells of the present invention.

For example, in the in vitro expansion of the present invention at a single stage, after the cells of the present invention are brought into contact with one or more cell activators of the present invention and/or one or more cell growth factors for a certain period of time, the cells can then be co-cultured with feeder cells of the present invention. For example, the certain period of time of the present invention can be at least about 1 hour. For example, the certain period of time of the present invention can be at least about 1-72 hours, for example, at least about 1 hour, at least about 2 hours, at least about 3 hours, at least about 4 hours, at least about 5 hours, at least about 6 hours, at least about 7 hours, at least about 8 hours, at least about 9 hours, at least about 10 hours, at least about 11 hours, at least about 12 hours, at least about 13 hours, at least about 14 hours, at least about 15 hours, at least about 16 hours, at least about 17 hours, at least about 18 hours, at least about 19 hours, at least about 20 hours, at least about 21 hours, at least about 22 hours, at least about 23 hours, at least about 24 hours, at least about 36 hours, at least about 48 hours, at least about 60 hours, or at least about 72 hours. For example, the certain period of time of the present invention can be about 2 hours to about 72 hours. For example, the certain period of time of the present invention can be about 6 hours to about 7 hours, about 6 hours to about 8 hours, about 6 hours to about 9 hours, about 6 hours to about 10 hours, about 6 hours to about 11 hours, about 6 hours to about 12 hours, about 6 hours to about 13 hours, about 6 hours to about 14 hours, about 6 hours to about 15 hours, about 6 hours to about 16 hours, about 6 hours to about 17 hours, about 6 hours to about 18 hours, about 6 hours to about 19 hours, about 6 hours to about 20 hours, about 6 hours to about 21 hours, about 6 hours to about 22 hours, about 6 hours to about 23 hours, about 6 hours to about 24 hours, about 6 hours to about 36 hours, about 6 hours to about 48 hours, about 6 hours to about 60 hours, or about 6 hours to about 72 hours. For example, the certain period of time of the present invention can be about 12 hours to about 13 hours, about 12 hours to about 14 hours, about 12 hours to about 15 hours, about 12 hours to about 16 hours, about 12 hours to about 17 hours, about 12 hours to about 18 hours, about 12 hours to about 19 hours, about 12 hours to about 20 hours, about 12 hours to about 21 hours, about 12 hours to about 22 hours, about 12 hours to about 23 hours, about 12 hours to about 24 hours, about 12 hours to about 36 hours, about 12 hours to about 48 hours, about 12 hours to about 60 hours, or about 12 hours to about 72 hours. For example, the certain period of time of the present invention can be about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 36 hours, about 48 hours, about 60 hours, or about 72 hours.

For example, the feeder cells of the present invention can comprise antigen-presenting cells. For example, the feeder cells of the present invention can comprise one or more selected from the group consisting of: peripheral blood mononuclear cells, dendritic cells, and artificial antigen-presenting cells. For example, the feeder cells of the present invention can be peripheral blood mononuclear cells. For example, the feeder cells of the present invention may be irradiated feeder cells. For example, the feeder cells of the present invention can be isolated artificial antigen-presenting cells (aAPC), and the artificial antigen-presenting cells of the present invention can comprise cells expressing HLA-A/B/C, CD64, CD80, ICOS-L, and/or CD58, and can be modified to express more than one cell activator of the present invention. For example, the feeder cells of the present invention can be irradiated; for example, gamma irradiation can be performed, or X-ray irradiation can be performed.

For example, co-culturing the cells of the present invention with the feeder cells of the present invention can comprise bringing a surface of the feeder cells of the present invention into contact with a surface of the cells of the present invention. For example, co-culturing the cells of the present invention with the feeder cells of the present invention comprises adding the feeder cells of the present invention to a cell culture medium of the cells of the present invention.

For example, the feeder cells of the present invention can be added to the cell culture medium of the cells of the present invention at a ratio of the feeder cells of the present invention to the cells of the present invention of about 40:1 to about 400:1. For example, the feeder cells of the present invention can be added to the cell culture medium of the cells of the present invention at a ratio of the feeder cells of the present invention to the cells of the present invention of about 40:1 to about 400:1, about 40:1 to about 300:1, about 40:1 to about 200:1, about 40:1 to about 100:1, about 40:1 to about 90:1, about 40:1 to about 80:1, about 40:1 to about 70:1, about 40:1 to about 60:1, about 40:1 to about 50:1, about 50:1 to about 400:1, about 60:1 to about 400:1, about 70:1 to about 400:1, about 80:1 to about 400:1, about 90:1 to about 400:1, about 100:1 to about 400:1, about 200:1 to about 400:1, or about 300:1 to about 400:1.

In another aspect, the present invention provides a method for culturing tumor-infiltrating lymphocytes (TIL), which can comprise: (A) bringing a first TIL population, which is derived from tumor tissue, tumor-associated lymph nodes with or without tumor metastasis, tumor metastatic lesions, fragments of paracancerous tissue, pleural effusion and/or ascites and is not subjected to in vitro expansion, into contact with one or more cell growth factors, wherein a second TIL population is obtained through step (A); and (B) reducing the expression and/or weakening the activity of a member selected from the group consisting of the AF4 family, the tyrosine protein kinase family, the bZIP family, the nuclear receptor family, and the ubiquitin family in the second TIL population, wherein a third TIL population is obtained through step (B).

In another aspect, the present invention provides a method for culturing tumor-infiltrating lymphocytes (TIL), comprising: (A) bringing a first TIL population, which is derived from tumor tissue, tumor-associated lymph nodes with or without tumor metastasis, tumor metastatic lesions, fragments of paracancerous tissue, pleural effusion and/or ascites and is not subjected to in vitro expansion, into contact with one or more T cell growth factors, wherein a second TIL population is obtained through step (A); (B) reducing the expression and/or weakening the activity of a member selected from the group consisting of the AF4 family, the tyrosine protein kinase family, the bZIP family, the nuclear receptor family, and the ubiquitin family in the second TIL population, and bringing the second TIL population into contact with a T cell activator and/or a T cell growth factor, wherein a third TIL population is obtained through step (B); and (C) co-culturing the third TIL population with feeder cells, wherein a fourth TIL population is obtained through step (C).

In one embodiment, the first stage in vitro expansion of the present invention can be interchangeably used with step (A) in the method of the above aspect. In one embodiment, the second stage in vitro expansion of the present invention can be interchangeably used with step (B) in the method of the above aspect. In one embodiment, the TIL subjected to the first stage in vitro expansion of the present invention can be interchangeably used with the second TIL population obtained through step (A) in the method of the above aspect. In one embodiment, the TIL subjected to the second stage in vitro expansion of the present invention can be interchangeably used with the third TIL population obtained through step (B) in the method of the above aspect. In one embodiment, if needed, the third stage in vitro expansion of the present invention can be interchangeably used with an optionally added step (C) in the method of the above aspect. In one embodiment, if needed, the TIL subjected to the third stage in vitro expansion of the present invention can be interchangeably used with the fourth TIL population obtained through the optionally added step (C) in the method of the above aspect.

In another aspect, the present invention provides a method for culturing tumor-infiltrating lymphocytes (TIL), which can comprise: (A) bringing a first TIL population, which is derived from tumor tissue, tumor-associated lymph nodes with or without tumor metastasis, tumor metastatic lesions, fragments of paracancerous tissue, pleural effusion and/or ascites and is not subjected to in vitro expansion, into contact with a plurality of cell growth factors, wherein a second TIL population is obtained through step (A); and (B) bringing the second TIL population into contact with a plurality of cell growth factors, bringing the second TIL population into contact with a plurality of cell activators, reducing the expression and/or weakening the activity of a member selected from the group consisting of the AF4 family, the tyrosine protein kinase family, the bZIP family, the nuclear receptor family, and the ubiquitin family, and co-culturing the TIL with feeder cells, wherein a third TIL population is obtained through step (B).

In another aspect, the present invention provides a method for culturing tumor-infiltrating lymphocytes (TIL), which may comprise: (A) allowing a first TIL population, which is derived from tumor tissue, tumor-associated lymph nodes with or without tumor metastasis, tumor metastatic lesions, fragments of paracancerous tissue, pleural effusion and/or ascites and is not subjected to in vitro expansion, to be brought into contact with a cell growth factor; wherein a second TIL population is obtained through said step (A); and (B) allowing said second TIL population to be brought into contact with a cell growth factor, to be brought into contact with a cell activator, to have reduced expression and/or weakened activity of a member selected from the AF4 family, the tyrosine protein kinase family, the bZIP family, the nuclear receptor family, or the ubiquitin family, and allowing said TIL to be co-cultured with feeder cells, wherein the member selected from the AF4 family, the tyrosine protein kinase family, the bZIP family, the nuclear receptor family, or the ubiquitin family respectively comprises AFF3, AXL, NFE2L1, RARG, and UBFD1; wherein a third TIL population is obtained through said step (B).

In another aspect, the present invention provides a method for culturing tumor-infiltrating lymphocytes (TIL). A method for obtaining TIL cells from a subject tissue sample may comprise obtaining, by surgery from a patient, an in situ tumor sample or a metastatic tumor sample, wherein the weight is at least about 1 g, and multiple tissue pieces may also be combined. Tumor tissue, tumor-associated lymph nodes with or without tumor metastasis, tumor metastatic lesions, fragments of paracancerous tissue, pleural effusion and/or ascites are transported in a sample transport solution, for example, a commercially commonly used tumor tissue transport solution, tumor tissue preservation solution, or tumor tissue transfer solution, at about 2-8°C , and are processed within 48 hours. Tissue blocks may be mechanically disrupted to a size of about 1-27 cubic millimeters per block, transferred into a gas-permeable culture bag or Grex, and cultured for about 3-14 days by adding a serum-free cell culture medium and IL-2 at a concentration of 300-9000 IU/mL (e.g., 1000-9000 IU/mL, e.g., 6000 IU/mL). Cells in the culture medium are collected and transferred into a gas-permeable culture bag, or Grex, or Xuri equipment; the serum-free cell culture medium may be added with the CD28 antibody of the present invention, magnetic beads (e.g., Dynabeads) comprising a CD3 antibody and a CD28 antibody and/or a nanomatrix (e.g., transACT) comprising a CD3 antibody and a CD28 antibody, IL-2 at a concentration of 300-9000 IU/mL (e.g., 1000-9000 IU/mL, e.g., 6000 IU/mL), and reducing expression and/or weakening activity of a member selected from the AF4 family, the tyrosine protein kinase family, the bZIP family, the nuclear receptor family, or the ubiquitin family (wherein an AF4 family member comprises AFF3, a tyrosine protein kinase family member comprises AXL, a bZIP family member comprises NFE2L1, a nuclear receptor family member comprises RARG, and a ubiquitin family member comprises UBFD1), for example, by transducing with a ribonucleoprotein complex (RNP) carrying gRNA of the present invention and a Cas protein, or an LNP comprising gRNA and a Cas protein, or an LNP comprising a nucleic acid encoding gRNA and a Cas protein, such that the proportion of cells in said TIL in which the gene encoding a member selected from the AF4 family, the tyrosine protein kinase family, the bZIP family, the nuclear receptor family, or the ubiquitin family is about 95% or less. After the TIL of the present invention are activated for a certain period of time, irradiated PBMC are added (TIL and PBMC at a ratio of about 1:40 to about 1:400), and expansion culture is performed for about 3-14 days. A cell processing system may be used to collect cells in the culture medium, wash and cryopreserve the cells, and perform testing. The final product may have a CD3 proportion of greater than 80%, a cell viability of greater than 50%, and more than 80% of the cells may be memory effector cells and effector cells. IFN-γ may be secreted after stimulation, and/or a characteristic of an upregulated proportion of activated cells may be present.

### CRP, CYLD, CBLIF, KLF4, NDST1, NLRP1, and SCGB1A1

1. A method for culturing cells, said method comprising: allowing a family member selected from the pentraxin family, the peptidase C19 family, the cobalamin transport protein family, the krueppel C2H2-type zinc finger protein family, the sulfotransferase 1 family, the Nod-like receptor (NLR) protein family, and the secretoglobin family, and/or a functionally active fragment thereof, in said cells to be reduced and/or weakened.
2. The method according to technical solution 1, wherein said cells comprise immune cells.
3. The method according to technical solution 2, wherein said immune cells comprise phagocytes, lymphocytes, neutrophils, eosinophils, and/or basophils.
4. The method according to any one of technical solutions 2 to 3, wherein said immune cells comprise monocytes, macrophages, and/or dendritic cells.
5. The method according to any one of technical solutions 2-4, wherein said immune cells are immune cells derived from stem cell differentiation.
6. The method according to technical solution 5, wherein said stem cells comprise induced pluripotent stem cells (iPSC), embryonic stem cells, bone marrow stem cells, cord blood stem cells, and/or peripheral blood stem cells.
7. The method according to any one of technical solutions 2-6, wherein said immune cells comprise B cells, T cells, natural killer cells, and/or natural killer-like T cells (NKT).
8. The method according to any one of technical solutions 2-7, wherein said immune cells comprise αβ T cells and/or γδ T cells.
9. The method according to any one of technical solutions 2-8, wherein said immune cells comprise tumor-infiltrating lymphocytes (TIL).
10. The method according to technical solution 9, wherein said TIL are TIL derived from fragments, pleural effusion and/or ascites, and/or TIL derived from cryopreserved TIL after resuscitation.
11. The method according to technical solution 10, wherein said volume is about 1 cubic millimeter to about 27 cubic millimeters.
12. The method according to any one of technical solutions 2-11, wherein said immune cells comprise an engineered immune receptor displayed on the cell surface.
13. The method according to technical solution 12, wherein said engineered immune receptor specifically binds to an antigen expressed on a target cell.
14. The method according to any one of technical solutions 2-13, wherein said immune cells comprise a chimeric antigen receptor and/or a T cell receptor.
15. The method according to any one of technical solutions 1-14, wherein said reducing and/or weakening of a family member selected from the pentraxin family, the peptidase C19 family, the cobalamin transport protein family, the krueppel C2H2-type zinc finger protein family, the sulfotransferase 1 family, the Nod-like receptor (NLR) protein family, and the secretoglobin family, and/or a functionally active fragment thereof, in the cells comprises an effect selected from the group consisting of: inhibiting a calcium ion binding function, inhibiting a deubiquitinase function, inhibiting a cobalamin endocytosis function, inhibiting a transcriptional regulation function, inhibiting a sulfotransferase function, inhibiting an apoptosis-inducing function, or inhibiting an inhibitory function on phospholipase A2.
16. The method according to any one of technical solutions 1-15, wherein, compared with cells having a family member selected from the pentraxin family, the peptidase C19 family, the cobalamin transport protein family, the krueppel C2H2-type zinc finger protein family, the sulfotransferase 1 family, the Nod-like receptor (NLR) protein family, and the secretoglobin family, and/or a functionally active fragment thereof, and/or compared with unchanged cells, cells obtained by reducing and/or weakening said family member selected from the pentraxin family, the peptidase C19 family, the cobalamin transport protein family, the krueppel C2H2-type zinc finger protein family, the sulfotransferase 1 family, the Nod-like receptor (NLR) protein family, and the secretoglobin family, and/or a functionally active fragment thereof, exhibit improved cellular characteristics.
17. The method according to technical solution 16, wherein said improved cellular characteristics comprise one or more selected from the group consisting of: improved cell proliferation capacity, an increased proportion of viable cells, an improved cell subpopulation proportion, enhanced cytokine secretion capacity, enhanced in vitro tumor cell killing capacity, and enhanced in vivo tumor killing capacity.
18. The method according to technical solution 17, wherein said improved cell subpopulation proportion comprises one or more selected from the group consisting of: an increased proportion of activated cells, a reduced proportion of regulatory cells, a reduced proportion of exhausted cells, an increased proportion of central memory cells and/or naïve cells, a reduced proportion of apoptotic cells, and an increased proportion of stem cell-like cells.
19. The method according to any one of technical solutions 1-18, wherein the family members of said pentraxin family, peptidase C19 family, cobalamin transport protein family, krueppel C2H2-type zinc finger protein family, sulfotransferase 1 family, Nod-like receptor (NLR) protein family, and secretoglobin family respectively comprise a pentraxin domain, a cytoskeleton-associated protein glycine-conserved (CAP-GLY) domain, a cobalamin-binding domain, a C2H2-type zinc finger domain, a sulfotransferase domain, a caspase recruitment domain (CARD), and a uteroglobin domain.
20. The method according to any one of technical solutions 1-19, wherein the family members of said pentraxin family, peptidase C19 family, cobalamin transport protein family, krueppel C2H2-type zinc finger protein family, sulfotransferase 1 family, Nod-like receptor (NLR) protein family, and secretoglobin family respectively comprise CRP, CYLD, CBLIF, KLF4, NDST1, NLRP1, and SCGB1A1.
21. The method according to any one of technical solutions 1-20, wherein said reducing and/or weakening of a family member of the pentraxin family, the peptidase C19 family, the cobalamin transport protein family, the krueppel C2H2-type zinc finger protein family, the sulfotransferase 1 family, the Nod-like receptor (NLR) protein family, or the secretoglobin family comprises being introduced into said cells.
22. The method according to technical solution 21, wherein the gene regulatory system is used to disrupt, at the DNA level, a family member of the pentraxin family, the peptidase C19 family, the cobalamin transport protein family, the krueppel C2H2-type zinc finger protein family, the sulfotransferase 1 family, the Nod-like receptor (NLR) protein family, or the secretoglobin family; and optionally, the cell is selected such that the expression of and/or the activity of BRD4, FAS, TNFAIP3, ZC3H12A, SOCS1, CBLB, FIBP, IKZF1, LAG3, MED12, PD1, RASA2, TIGIT, TIM3, ADNP, NFKBIA, PTPN6, TNIP1, BCL2L11, PTPN2, AFF3, AXL, NFE2L1, RARG, UBFD1, CRP, CYLD, CBLIF, KLF4, NDST1, NLRP1 or SCGB1A1 is reduced and/or attenuated.
23. The method according to any one of technical solutions 21 to 22, wherein the gene regulatory system comprises a guide nucleic acid molecule and an enzyme protein.
24. The method according to technical solution 23, wherein reducing the expression of and/or attenuating the activity of a family member of the pentraxin family, the peptidase C19 family, the cobalamin transport protein family, the krueppel C2H2-type zinc finger protein family, the sulfotransferase 1 family, the Nod-like receptor (NLR) protein family, or the secretoglobin family comprises introducing, into the cell, a complex comprising the guide nucleic acid molecule and the enzyme protein, or a complex comprising the guide nucleic acid molecule and a nucleic acid encoding the enzyme protein.
25. The method according to any one of technical solutions 23 to 24, wherein the enzyme protein comprises a Cas protein, a Cas protein homolog, or a functionally active fragment thereof, preferably selected from Cas 9 and Cas 12.
26. The method according to any one of technical solutions 23-25, wherein the guide nucleic acid molecule comprises (gRNA).
27. The method according to any one of technical solutions 23-26, wherein the guide nucleic acid molecule binds to a sequence of a family member selected from the pentraxin family, the peptidase C19 family, the cobalamin transport protein family, the krueppel C2H2-type zinc finger protein family, the sulfotransferase 1 family, the Nod-like receptor (NLR) protein family, or the secretoglobin family.
28. The method according to any one of technical solutions 23-27, wherein the guide nucleic acid molecule binds to a sequence consisting of about 15 to about 25 nucleotides upstream of the 5' end of a protospacer adjacent motif (PAM) selected from the group consisting of: AGG, TGG, CGG, and GGG, or binds to a sequence consisting of about 15 to about 25 nucleotides downstream of the 3' end of a protospacer adjacent motif (PAM) selected from the group consisting of: NTTN, TTYN, VTTV, TRTV, TTTV, TATV, TYCV, TNN, and NTN, wherein N is A, T, C, or G, Y is T or C, V is A, C, or G, and R is A or G, or binds to a sequence consisting of about 15 to about 25 nucleotides downstream of the 3' end of a protospacer adjacent motif (PAM) selected from the group consisting of: TTTN, ATTN, GTTN, CTTN, TTC, TTG, TTA, TTT, TAN, TGN, and TCN, wherein N is A, T, C, or G.
29. The method according to any one of technical solutions 23-28, wherein the guide nucleic acid molecule binds to a region defined by genomic coordinates shown in Tables 2H-2N, or a fragment thereof.
30. The method according to any one of technical solutions 23-29, wherein the guide nucleic acid molecule binds to a region or a fragment thereof selected from the group consisting of: SEQ ID NO: 41120-41593, SEQ ID NO: 41594-43088, SEQ ID NO: 43089-43638, SEQ ID NO: 43639-44560, SEQ ID NO: 44561-47251, SEQ ID NO: 47252-49203, and SEQ ID NO: 49204-49576.
31. The method according to any one of technical solutions 23-30, wherein the guide nucleic acid molecule comprises a sequence as shown in SEQ ID NO: 16332-16805, SEQ ID NO: 16806-18300, SEQ ID NO: 18301-18850, SEQ ID NO: 18851-19772, SEQ ID NO: 19773-22463, SEQ ID NO: 22464-24415, and SEQ ID NO: 24416-24788.
32. The method according to any one of technical solutions 1-31, wherein, as compared with a cell in which the expression of and/or the activity of a family member of the pentraxin family, the peptidase C19 family, the cobalamin transport protein family, the krueppel C2H2-type zinc finger protein family, the sulfotransferase 1 family, the Nod-like receptor (NLR) protein family, or the secretoglobin family is not changed, in a cell obtained by reducing the expression of and/or attenuating the activity of the family member of the pentraxin family, the peptidase C19 family, the cobalamin transport protein family, the krueppel C2H2-type zinc finger protein family, the sulfotransferase 1 family, the Nod-like receptor (NLR) protein family, or the secretoglobin family, the proportion of cells expressing a target gene is reduced and/or the expression level of the target gene in a single cell is decreased.
33. The method according to any one of technical solutions 1-32, wherein, in a cell obtained by reducing the expression of and/or attenuating the activity of a family member of the pentraxin family, the peptidase C19 family, the cobalamin transport protein family, the krueppel C2H2-type zinc finger protein family, the sulfotransferase 1 family, the Nod-like receptor (NLR) protein family, or the secretoglobin family, the proportion of cells expressing a target gene is about 95% or less.
34. A cell, wherein the cell is obtained by the method according to any one of technical solutions 1-33.
35. A composition, comprising the cell according to technical solution 34, and optionally a pharmaceutically acceptable carrier.
36. A method for affecting cell growth, comprising the cell according to technical solution 34 and/or the pharmaceutical composition according to technical solution 35.
37. Use of the cell according to technical solution 34 and/or the pharmaceutical composition according to technical solution 35 in the manufacture of a medicament, wherein the medicament is used for preventing and/or treating a disease and/or symptom.
38. A medicament, which is used for preventing and/or treating a disease and/or symptom, comprising the cell according to technical solution 34 and/or the pharmaceutical composition according to technical solution 35 as an active ingredient.
39. A method for preventing and/or treating a disease and/or symptom, comprising administering, to a subject in need thereof, the cell according to technical solution 34 and/or the pharmaceutical composition according to technical solution 35.
40. The cell according to technical solution 34 and/or the pharmaceutical composition according to technical solution 35, for use in preventing and/or treating a disease and/or symptom.
41. The use according to technical solution 37, the medicament according to technical solution 38, the method according to technical solution 39, and/or the cell and/or pharmaceutical composition for use according to technical solution 40, wherein the disease and/or symptom comprises a tumor.
42. The use according to technical solution 37, the medicament according to technical solution 38, the method according to technical solution 39, and/or the cell and/or pharmaceutical composition for use according to technical solution 40, wherein the disease and/or symptom comprises a solid tumor.
43. The use according to technical solution 37, the medicament according to technical solution 38, the method according to technical solution 39, and/or the cell and/or pharmaceutical composition for use according to technical solution 40, wherein the disease and/or symptom comprises one or more selected from the group consisting of: melanoma, ovarian cancer, cervical cancer, lung cancer, bladder cancer, breast cancer, head and neck cancer, pancreatic cancer, liver cancer, gastric cancer, colorectal cancer, and renal cancer.

In one aspect, the present invention provides a method for culturing a cell, such that a member selected from the pentraxin family, the peptidase C19 family, the cobalamin transport protein family, the krueppel C2H2-type zinc finger protein family, the sulfotransferase 1 family, the Nod-like receptor (NLR) protein family, or the secretoglobin family, and/or a functionally active fragment thereof, in the cell is reduced and/or attenuated.

For example, the cell may further comprise, optionally, reduced expression and/or attenuated activity of BRD4, FAS, TNFAIP3, ZC3H12A, SOCS1, CBLB, FIBP, IKZF1, LAG3, MED12, PD1, RASA2, TIGIT, TIM3, ADNP, NFKBIA, PTPN6, BCL2L11, PTPN2, AFF3, AXL, NFE2L1, RARG, UBFD1, CRP, CYLD, CBLIF, KLF4, NDST1, NLRP1, SCGB1A1, or TNIP1.

For example, the pentraxin family member may comprise a pentraxin domain. For example, the pentraxin family member may comprise CRP.

For example, the peptidase C19 family member may comprise a cytoskeleton-associated protein glycine-conserved (CAP-GLY) domain. For example, the peptidase C19 family member may comprise CYLD.

For example, the cobalamin transport protein family member may comprise a cobalamin-binding domain. For example, the cobalamin transport protein family member may comprise CBLIF.

For example, the krueppel C2H2-type zinc finger protein family member may comprise a C2H2-type zinc finger domain. For example, the krueppel C2H2-type zinc finger protein family member may comprise KLF4.

For example, the sulfotransferase 1 family member may comprise a sulfotransferase domain. For example, the sulfotransferase 1 family member may comprise NDST1.

For example, the Nod-like receptor (NLR) protein family member may comprise a caspase recruitment domain (CARD). For example, the Nod-like receptor (NLR) protein family member may comprise NLRP1.

For example, the secretoglobin family member may comprise a uteroglobin domain. For example, the secretoglobin family member may comprise SCGB1A1.

For example, in the cell of the present invention, a member selected from the pentraxin family, the peptidase C19 family, the cobalamin transport protein family, the krueppel C2H2-type zinc finger protein family, the sulfotransferase 1 family, the Nod-like receptor (NLR) protein family, and the secretoglobin family, and/or a functionally active fragment thereof, is reduced and/or attenuated. For example, CRP, CYLD, CBLIF, KLF4, NDST1, NLRP1, and/or SCGB1A1 is reduced and/or attenuated.

For example, the target gene of the present invention may be a gene encoding a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a gene encoding a functionally active fragment thereof. For example, as compared with cells in which the expression and/or activity of the target gene is not altered, cells obtained by reducing the expression of the target gene in the cells and/or weakening the activity are capable of exhibiting improvement. In one embodiment, the cells in which the expression and/or activity of the target gene is not altered refer to cells derived from the same donor, in which the expression of the target gene in the cells has not been reduced and/or the activity has not been weakened. In one embodiment, the cells in which the expression and/or activity of the target gene is not altered refer to cells derived from the same donor, in which the expression of other genes other than the target gene in the cells (e.g., by knocking out such other gene, which has substantially no effect on cell function) has not been reduced and/or the activity has not been weakened.

In one embodiment, the corresponding cells in which the expression of the target gene in the cells has not been reduced and/or the activity has not been weakened refer to cells derived from the same donor, isolated in the same manner, and in which the expression of the target gene in the cells has not been reduced and/or the activity has not been weakened. In one embodiment, the corresponding cells in which the expression of the target gene in the cells has not been reduced and/or the activity has not been weakened refer to cells derived from the same donor and from the same tumor source, and in which the expression of the target gene in the cells has not been reduced and/or the activity has not been weakened. In one embodiment, the corresponding cells in which the expression of the target gene in the cells has not been reduced and/or the activity has not been weakened refer to cells obtained by dividing cells derived from the same donor and from the same tumor source into two groups, wherein one group of cells in which the expression of the target gene in the cells has not been reduced and/or the activity has not been weakened serves as the corresponding cells in which the expression of the target gene in the cells has not been reduced and/or the activity has not been weakened. For example, reduced expression and/or weakened activity of the target gene means that the target gene in a native cell is in an expressed state to a certain extent, and after the treatment of the present invention, the expression level of the target gene in the cell is reduced; that is, the reduction in the expression level of the target gene causes the native cell to change from expressing the target gene to substantially not expressing the target gene, or causes the amount of expression of the target gene to be reduced.

For example, the cells comprise immune cells. For example, the cells comprise phagocytes, lymphocytes, neutrophils, eosinophils, and/or basophils.

For example, the cells comprise monocytes, macrophages, and/or dendritic cells.

For example, the cells of the present invention further comprise cells differentiated from stem cells. For example, the cells of the present invention further comprise cells differentiated from pluripotent stem cells. For example, the stem cells of the present invention are obtained by induction. For example, the above stem cells of the present invention comprise induced pluripotent stem cells (iPSC), embryonic stem cells, bone marrow stem cells, cord blood stem cells, and/or peripheral blood stem cells.

For example, the "stem cells" of the present invention further comprise pluripotent cells, multipotent cells, precursor cells, and progenitor cells. For example, stem cells are obtained from hematopoietic or mesenchymal stem cells obtained from bone marrow tissue, placental stem cells obtained from placental tissue, embryonic stem cells obtained from embryonic tissue, or embryonic germ cells obtained from fetal germ tissue. Exemplary pluripotent stem cells are also generated from somatic cells by reprogramming the somatic cells to a pluripotent state through expression of certain transcription factors associated with pluripotency; such cells are referred to as "induced pluripotent stem cells" or "iPSC".

For example, the cells comprise B cells, T cells, natural killer cells, and/or natural killer-like T cells (NKT). For example, "unmodified cells" or "unengineered cells" refer to cells or a cell population in which the genome is not modified and that does not comprise a gene regulatory system, or that comprises a control gene regulatory system (e.g., an empty vector control, a non-targeting gRNA, an interfering siRNA, etc.). For example, the cells comprise αβ T cells and/or γδ T cells. For example, the cells comprise tumor-infiltrating lymphocytes (TIL). For example, the TIL are TIL derived from tumor tissue, tumor-associated lymph nodes with or without tumor metastasis, tumor metastatic lesions, fragments of paracancerous tissue, pleural effusion and/or ascites, and/or TIL derived from cryopreserved and resuscitated TIL.

For example, the TIL of the present invention are TIL derived from tumor tissue, tumor-associated lymph nodes with or without tumor metastasis, tumor metastatic lesions, fragments of paracancerous tissue, pleural effusion and/or ascites, and/or TIL derived from cryopreserved and resuscitated TIL. For example, the TIL of the present invention are obtained by processing tumor tissue into tumor fragments. For example, the volume of the tumor fragments of the present invention is about 1-27 cubic millimeters. For example, the volume of the tumor fragments of the present invention is about 1 cubic millimeter, about 2 cubic millimeters, about 3 cubic millimeters, about 4 cubic millimeters, about 5 cubic millimeters, about 6 cubic millimeters, about 7 cubic millimeters, about 8 cubic millimeters, about 9 cubic millimeters, about 10 cubic millimeters, about 11 cubic millimeters, about 12 cubic millimeters, about 13 cubic millimeters, about 14 cubic millimeters, about 15 cubic millimeters, about 16 cubic millimeters, about 17 cubic millimeters, about 18 cubic millimeters, about 19 cubic millimeters, about 20 cubic millimeters, about 21 cubic millimeters, about 23 cubic millimeters, about 24 cubic millimeters, about 25 cubic millimeters, about 26 cubic millimeters, or about 27 cubic millimeters.

For example, the cells comprise an engineered immune receptor displayed on the cell surface. For example, the engineered immune receptor specifically binds to an antigen expressed on a target cell. For example, the cells comprise a chimeric antigen receptor and/or a T cell receptor.

In one aspect, the present invention provides a method for culturing tumor-infiltrating lymphocytes (TIL), which comprises causing the expression and/or activity, in the TIL, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, to be reduced and/or weakened.

For example, TIL derived from tumor tissue, tumor-associated lymph nodes with or without tumor metastasis, tumor metastatic lesions, fragments of paracancerous tissue, pleural effusion and/or ascites, and not subjected to in vitro expansion are subjected to at least one stage of in vitro expansion, wherein, in at least one stage of the in vitro expansion, the expression and/or activity, in the TIL, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced and/or weakened.

For example, the TIL of the present invention, derived from tumor tissue, tumor-associated lymph nodes with or without tumor metastasis, tumor metastatic lesions, fragments of paracancerous tissue, pleural effusion and/or ascites, and not subjected to in vitro expansion, are subjected to a first stage of in vitro expansion and a second stage of in vitro expansion, and in the second stage of in vitro expansion of the present invention, the expression and/or activity, in the TIL, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced and/or weakened. For example, the TIL of the present invention, derived from tumor tissue, tumor-associated lymph nodes with or without tumor metastasis, tumor metastatic lesions, fragments of paracancerous tissue, pleural effusion and/or ascites, and not subjected to in vitro expansion, are subjected to a first stage of in vitro expansion and a second stage of in vitro expansion, and in the first stage of in vitro expansion of the present invention, the expression and/or activity, in the TIL, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced and/or weakened.

For example, the TIL of the present invention, derived from tumor tissue, tumor-associated lymph nodes with or without tumor metastasis, tumor metastatic lesions, fragments of paracancerous tissue, pleural effusion and/or ascites, and not subjected to in vitro expansion, are subjected to a first stage of in vitro expansion and a second stage of in vitro expansion, and in the first stage of in vitro expansion of the present invention, the expression and/or activity, in the TIL, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced and/or weakened, and in the second stage of in vitro expansion of the present invention, the expression and/or activity, in the TIL, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced and/or weakened.

For example, tumor-infiltrating lymphocytes (TILs) derived from tumor tissue, tumor-associated lymph nodes with or without tumor metastasis, tumor metastatic lesions, fragments of paracancerous tissue, pleural effusion and/or ascites, and not subjected to in vitro expansion, are subjected to a first stage in vitro expansion, a second stage in vitro expansion, and a third stage in vitro expansion according to the present invention, and, in the first stage in vitro expansion of the present invention, the expression and/or activity, in the TILs, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced.

For example, tumor-infiltrating lymphocytes (TILs) derived from tumor tissue, tumor-associated lymph nodes with or without tumor metastasis, tumor metastatic lesions, fragments of paracancerous tissue, pleural effusion and/or ascites, and not subjected to in vitro expansion, are subjected to a first stage in vitro expansion, a second stage in vitro expansion, and a third stage in vitro expansion according to the present invention, and, in the second stage in vitro expansion of the present invention, the expression and/or activity, in the TILs, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced.

For example, tumor-infiltrating lymphocytes (TILs) derived from tumor tissue, tumor-associated lymph nodes with or without tumor metastasis, tumor metastatic lesions, fragments of paracancerous tissue, pleural effusion and/or ascites, and not subjected to in vitro expansion, are subjected to a first stage in vitro expansion, a second stage in vitro expansion, and a third stage in vitro expansion according to the present invention, and, in the third stage in vitro expansion of the present invention, the expression and/or activity, in the TILs, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced.

For example, tumor-infiltrating lymphocytes (TILs) derived from tumor tissue, tumor-associated lymph nodes with or without tumor metastasis, tumor metastatic lesions, fragments of paracancerous tissue, pleural effusion and/or ascites, and not subjected to in vitro expansion, are subjected to a first stage in vitro expansion, a second stage in vitro expansion, and a third stage in vitro expansion according to the present invention, and, in the first stage in vitro expansion of the present invention, the expression and/or activity, in the TILs, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced, and, in the second stage in vitro expansion of the present invention, the expression and/or activity, in the TILs, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced.

For example, tumor-infiltrating lymphocytes (TILs) derived from tumor tissue, tumor-associated lymph nodes with or without tumor metastasis, tumor metastatic lesions, fragments of paracancerous tissue, pleural effusion and/or ascites, and not subjected to in vitro expansion, are subjected to a first stage in vitro expansion, a second stage in vitro expansion, and a third stage in vitro expansion according to the present invention, and, in the first stage in vitro expansion of the present invention, the expression and/or activity, in the TILs, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced, and, in the third stage in vitro expansion of the present invention, the expression and/or activity, in the TILs, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced.

For example, tumor-infiltrating lymphocytes (TILs) derived from tumor tissue, tumor-associated lymph nodes with or without tumor metastasis, tumor metastatic lesions, fragments of paracancerous tissue, pleural effusion and/or ascites, and not subjected to in vitro expansion, are subjected to a first stage in vitro expansion, a second stage in vitro expansion, and a third stage in vitro expansion according to the present invention, and, in the second stage in vitro expansion of the present invention, the expression and/or activity, in the TILs, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced, and, in the third stage in vitro expansion of the present invention, the expression and/or activity, in the TILs, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced.

For example, tumor-infiltrating lymphocytes (TILs) derived from tumor tissue, tumor-associated lymph nodes with or without tumor metastasis, tumor metastatic lesions, fragments of paracancerous tissue, pleural effusion and/or ascites, and not subjected to in vitro expansion, are subjected to a first stage in vitro expansion, a second stage in vitro expansion, and a third stage in vitro expansion according to the present invention, and, in the first stage in vitro expansion of the present invention, the expression and/or activity, in the TILs, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced, and, in the second stage in vitro expansion of the present invention, the expression and/or activity, in the TILs, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced, and, in the third stage in vitro expansion of the present invention, the expression and/or activity, in the TILs, of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced.

For example, each stage of in vitro expansion is delineated by a change in the number of TIL cells; for example, when the number of TIL cells increases by at least about 1-fold, it is considered that the TIL cells enter a next stage of in vitro expansion. In some embodiments, when the number of TIL cells increases by at least about 1 to 1000-fold, for example, at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, at least about 50-fold, at least about 100-fold, at least about 200-fold, at least about 500-fold, or at least about 1000-fold, it is considered that the TIL cells enter a next stage of in vitro expansion. For example, each stage of in vitro expansion is also delineated by a change in culture conditions of the TIL cells. For example, when a cell activator and/or a cell growth factor is added or additionally supplemented in the cell culture medium, it is considered that the TIL cells enter a next stage of in vitro expansion. For example, when IL-2 is added or additionally supplemented in the cell culture medium, it is considered that the TIL cells enter a next stage of in vitro expansion. For example, when one or more gene regulation systems is added or additionally supplemented in the cell culture medium, it is considered that the TIL cells enter a next stage of in vitro expansion. For example, when feeder cells are added or additionally supplemented in the cell culture medium, it is considered that the TIL cells enter a next stage of in vitro expansion. For example, when the TIL cells are subjected to centrifugation and/or cell washing, it is considered that the TIL cells enter a next stage of in vitro expansion. For example, each stage is also delineated by the number of days of TIL cell culture. For example, when the TIL cells are cultured in vitro for about 1-100 days, for example, about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 30 days, about 40 days, about 50 days, or about 100 days, it is considered that the TIL cells enter a next stage of in vitro expansion.

For example, the reduction and/or attenuation of a pentraxin family member of the cells comprises inhibition of a calcium ion-binding function.

For example, the reduction and/or attenuation of a peptidase C19 family member of the cells comprises inhibition of a deubiquitinating enzyme function.

For example, the reduction and/or attenuation of a cobalamin transporter family member of the cells comprises inhibition of a cobalamin endocytosis function.

For example, the reduction and/or attenuation of a krueppel C2H2-type zinc finger protein family member of the cells comprises inhibition of a transcriptional regulation function.

For example, the reduction and/or attenuation of a sulfotransferase 1 family member of the cell as described herein comprises inhibition of the function of a sulfotransferase.

For example, the reduction and/or attenuation of a Nod-like receptor (NLR) protein family member of the cell as described herein comprises inhibition of the function of inducing apoptosis.

For example, the reduction and/or attenuation of a secretoglobin family member of the cell as described herein comprises inhibition of the inhibitory function on phospholipase A2.

For example, compared with a cell in which the expression and/or activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family is not altered, a cell obtained by reducing the expression and/or attenuating the activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family is shown to exhibit improved cellular characteristics.

For example, the improved cellular characteristics comprise one or more selected from the group consisting of an improved, increased proportion of viable cells, an improved, enhanced secretion capacity, an enhanced in vitro tumor cell killing capacity, and an enhanced in vivo tumor killing capacity.

For example, the improved proportion of cell subpopulations comprises one or more selected from the group consisting of an increased proportion of activated cells, a reduced proportion of regulatory cells, a reduced proportion of exhausted cells, an increased proportion of central memory cells and/or naïve cells, a reduced proportion of apoptotic cells, and an increased proportion of stem cell-like cells.

For example, the improved cell number of the present invention means that, compared with a cell in which the expression and/or activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family is not altered, the cell number of a cell of the present invention, in which, in at least one in vitro expansion stage, the expression of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family is reduced and/or the activity is attenuated, is able to be increased by at least about 1-50 fold, for example, at least about 1 fold, at least about 2 fold, at least about 3 fold, at least about 4 fold, at least about 5 fold, at least about 6 fold, at least about 7 fold, at least about 8 fold, at least about 9 fold, at least about 10 fold, at least about 11 fold, at least about 12 fold, at least about 13 fold, at least about 14 fold, at least about 15 fold, at least about 20 fold, at least about 30 fold, at least about 40 fold, or at least about 50 fold.

For example, the increased proportion of viable cells is able to be manifested as an increase in cell viability. For example, the increased proportion of viable cells of the present invention is able to mean that, compared with a cell in which the expression and/or activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family is not altered, the proportion of viable cells of a cell of the present invention, in which, in at least one in vitro expansion stage, the expression of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family is reduced and/or the activity is attenuated, is able to be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the enhanced cytokine secretion capacity of the present invention may mean that the cytokine secretion capacity of the cell for cytokines selected from the group consisting of IL-2, IL-6, CD107a, GZMB, TNF-α, and IFN-γ is enhanced. For example, the enhanced cytokine secretion capacity of the present invention is able to mean that, compared with a cell in which the expression and/or activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family is not altered, the proportion of cells secreting cytokines in a cell of the present invention, in which, in at least one in vitro expansion stage, the expression of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family is reduced and/or the activity is attenuated, is able to be increased by at least about 1 to 50 fold, for example, at least about 1 fold, at least about 2 fold, at least about 3 fold, at least about 4 fold, at least about 5 fold, at least about 6 fold, at least about 7 fold, at least about 8 fold, at least about 9 fold, at least about 10 fold, at least about 11 fold, at least about 12 fold, at least about 13 fold, at least about 14 fold, at least about 15 fold, at least about 20 fold, at least about 30 fold, at least about 40 fold, or at least about 50 fold. For example, the enhanced cytokine secretion capacity of the present invention may mean that, compared with a cell in which the expression and/or activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family is not altered, the proportion of cells secreting cytokines in a cell of the present invention, in which, in at least one in vitro expansion stage, the expression of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family is reduced and/or the activity is attenuated, is able to be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%. For example, the cytokine secretion capacity of the cells of the present invention is determined by flow cytometry or by Cytometric Bead Array (CBA).

For example, the enhanced in vitro tumor cell killing capability and/or the enhanced in vivo tumor killing capability of the present invention may refer to that, as compared with cells in which the expression and/or activity of a member selected from the pentraxin family, the peptidase C19 family, the cobalamin transport protein family, the krueppel C2H2-type zinc finger protein family, the sulfotransferase 1 family, the Nod-like receptor (NLR) protein family, or the secretoglobin family is not altered, the tumor cell killing rate of the cells of the present invention, in which the expression of the member selected from the pentraxin family, the peptidase C19 family, the cobalamin transport protein family, the krueppel C2H2-type zinc finger protein family, the sulfotransferase 1 family, the Nod-like receptor (NLR) protein family, or the secretoglobin family is reduced and/or the activity thereof is weakened during at least one in vitro expansion stage, is increased by at least about 1 to 50 fold, for example, at least about 1 fold, at least about 2 fold, at least about 3 fold, at least about 4 fold, at least about 5 fold, at least about 6 fold, at least about 7 fold, at least about 8 fold, at least about 9 fold, at least about 10 fold, at least about 11 fold, at least about 12 fold, at least about 13 fold, at least about 14 fold, at least about 15 fold, at least about 20 fold, at least about 30 fold, at least about 40 fold, or at least about 50 fold. For example, the enhanced in vitro tumor cell killing capability and/or the enhanced in vivo tumor killing capability of the present invention may refer to that, as compared with cells in which the expression and/or activity of a member selected from the pentraxin family, the peptidase C19 family, the cobalamin transport protein family, the krueppel C2H2-type zinc finger protein family, the sulfotransferase 1 family, the Nod-like receptor (NLR) protein family, or the secretoglobin family is not altered, the tumor cell killing rate of the cells of the present invention, in which the expression of the member selected from the pentraxin family, the peptidase C19 family, the cobalamin transport protein family, the krueppel C2H2-type zinc finger protein family, the sulfotransferase 1 family, the Nod-like receptor (NLR) protein family, or the secretoglobin family is reduced and/or the activity thereof is weakened during at least one in vitro expansion stage, is increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%. For example, the tumor cell killing rate of the cells of the present invention is measured by an IncuCyte system or by a CFSE and DAPI staining method. For example, the tumor cell killing of the cells of the present invention refers to the ability of the cells to kill solid tumor cells.

For example, the improved cell subpopulation proportions of the present invention comprise one or more selected from the group consisting of: an increased proportion of CD8⁺ cells, an increased proportion of central memory cells and/or naïve cells, a decreased proportion of regulatory cells, an increased proportion of activated cells, an increased proportion of tumor-specific cells (having a CD103⁺CD39⁺ phenotype), an increased proportion of stem cell-like cells, a decreased proportion of exhausted cells, and a decreased proportion of apoptotic cells.

For example, the increased proportion of CD8⁺ cells of the present invention may be an increase in the proportion of CD8-positive cells in the cells. For example, the proportion of CD8⁺ cells in the cells is increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the increased proportion of activated cells of the present invention may be an increase in the proportion of CD28⁺, CD25⁺ and/or 41BB⁺ cells in the cells. For example, the proportion of activated cells in the cells is increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%, or is increased by at least about 1 to 50 fold, for example, at least about 1 fold, at least about 2 fold, at least about 3 fold, at least about 4 fold, at least about 5 fold, at least about 6 fold, at least about 7 fold, at least about 8 fold, at least about 9 fold, at least about 10 fold, at least about 11 fold, at least about 12 fold, at least about 13 fold, at least about 14 fold, at least about 15 fold, at least about 20 fold, at least about 30 fold, at least about 40 fold, or at least about 50 fold.

For example, the reduced proportion of exhausted cells of the present invention is an increase in the proportion of PD-1⁺, LAG-3⁺, TIM-3⁺, CD39⁺, CD38⁺ and/or CD101⁺ cells in the cells. For example, the proportion of exhausted cells in the cells is reduced by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%, or is reduced by at least about 1-50 fold, for example, at least about 1 fold, at least about 2 fold, at least about 3 fold, at least about 4 fold, at least about 5 fold, at least about 6 fold, at least about 7 fold, at least about 8 fold, at least about 9 fold, at least about 10 fold, at least about 11 fold, at least about 12 fold, at least about 13 fold, at least about 14 fold, at least about 15 fold, at least about 20 fold, at least about 30 fold, at least about 40 fold, or at least about 50 fold.

For example, the reduced proportion of regulatory cells of the present invention is a reduction in the proportion of CD4⁺CD25⁺Foxp3⁺ cells in the cells. For example, the proportion of regulatory cells in the cells is reduced by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the reduced proportion of apoptotic cells of the present invention is a reduction in the proportion of Annexin V⁺7-AAD⁺ cells and/or Annexin V⁺7-AAD⁻ cells in the cells. For example, the proportion of apoptotic cells in the cells is reduced by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the increased proportion of stem-like cells of the present invention is an increase in the proportion of CD69⁻CD39⁻ cells and/or TCF1⁺ cells in the cells. For example, the proportion of stem-like cells in the cells is increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the increased proportion of central memory cells in the present invention may be an increase in the proportion of CD45RA⁻CCR7⁺ or CD45RO⁺CD62L⁺ cells in the cells. For example, the proportion in the cells may be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the increased proportion of naïve cells in the present invention may be an increase in the proportion of CD45RO⁻CD62L⁺ cells in the cells. For example, the proportion in the cells may be increased by at least about 100-0.1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.9%, at least about 0.8%, at least about 0.7%, at least about 0.6%, at least about 0.5%, at least about 0.4%, at least about 0.3%, at least about 0.2%, or at least about 0.1%.

For example, the method of the present invention may comprise editing a target gene of cells in vivo, ex vivo, and/or in vitro. For example, a reduction in an in vivo expression level of the target gene in cells in vivo may be achieved by delivery and editing via an in vivo gene regulation system. For example, in vivo editing of the target gene may be performed by targeting immune cells or precursor cells thereof, such as bone marrow stem cells and the like, and delivering LNPs comprising a gene regulation system or mRNA encoding a gene regulation system. By adjusting the composition and/or proportion of LNP components, or by introducing components having targeting capability, the in vivo editing efficiency of the present invention may be improved.

For example, the culture method of the present invention may comprise a gene editing step for cells. For example, the culture method comprises: subjecting the cells to at least one stage, wherein, in in vitro expansion of the at least one stage, the gene regulation system is introduced into the cells.

For example, the gene regulation system may disrupt the target gene at the DNA level. For example, the gene regulation system may disrupt a region of the target gene in the genome of the cell or a fragment thereof. For example, after the gene regulation system is used, the DNA region in which the target gene is located in the cell or a fragment thereof is cleaved, such that the expression capability of the target gene is reduced or the activity of the target gene is inhibited. For example, the editing effect of the gene regulation system on the target gene may be long-term and sustained. For example, in the cells of the present invention, the activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family is inhibited.

Wherein the genomic region described in the present invention is determined according to the human reference genome hg38 version.

For example, the gene regulation system may comprise a guide nucleic acid molecule and an enzyme protein. For example, the enzyme protein may have nuclease activity, and the guide nucleic acid molecule may guide the enzyme protein to specifically cleave a region in which the target gene is located or a fragment thereof. For example, the guide nucleic acid molecule and the enzyme protein may exist in the form of a ribonucleoprotein complex (RNP), or may each independently exist alone. For example, the enzyme protein may comprise a Cas protein. For example, a polynucleotide encoding gRNA and a Cas protein may be introduced into a target cell, or each may independently be introduced into the target cell.

For example, reduction in expression and/or weakening of activity of the target gene of the cell in the present invention may comprise introducing a ribonucleoprotein complex (RNP) comprising the guide nucleic acid molecule and the enzyme protein into the cell. For example, the enzyme protein may comprise a Cas protein, a Cas protein homolog, or a functionally active fragment thereof. For example, the guide nucleic acid molecule may comprise a guide RNA (gRNA). For example, a complex comprising a polynucleotide encoding gRNA and a Cas protein may be introduced into the cell. For example, a complex comprising gRNA and a Cas protein may be introduced into the cell.

For example, the gRNA may be used to bind to a sequence of the target gene. For example, binding of the gRNA to the sequence of the target gene may be fully complementary, may be partially complementary, or may hybridize to the sequence of the target gene under moderately stringent or stringent conditions. For example, binding of the gRNA to the sequence of the target gene may enable the CRISPR system of the gRNA to specifically cleave the target gene.

For example, the editing target region of the present invention may be a region upstream of a start codon. For example, the editing target region of the present invention may be a region having high transcription factor binding affinity. For example, the editing target region of the present invention may be a region having a specific number of transcription factor binding sites. For example, the editing target region of the present invention may be a continuous region having about 3 or more transcription factor binding sites. For example, the genomic coordinates of the editing target region of the present invention may be selected from preferred targeted sub-regions shown in Tables 1H to 1N.

For example, the guide nucleic acid molecule targeting CRP in the present invention may bind to a region or a fragment thereof selected from the group shown below: SEQ ID NO: 41120-41593.

For example, the guide nucleic acid molecule targeting CYLD in the present invention may bind to a region or a fragment thereof selected from the group shown below: SEQ ID NO: 41594-43088.

For example, the guide nucleic acid molecule targeting CBLIF in the present invention may bind to a region or a fragment thereof selected from the group shown below: SEQ ID NO: 43089-43638.

For example, the guide nucleic acid molecule targeting KLF4 in the present invention may bind to a region or a fragment thereof selected from the group shown below: SEQ ID NO: 43639-44560.

For example, the guide nucleic acid molecule targeting NDST1 in the present invention may bind to a region or a fragment thereof selected from the group shown below: SEQ ID NO: 44561-47251.

For example, the guide nucleic acid molecule targeting NLRP1 in the present invention may bind to a region or a fragment thereof selected from the group shown below: SEQ ID NO: 47252-49203.

For example, the guide nucleic acid molecule targeting SCGB1A1 in the present invention may bind to a region or a fragment thereof selected from the group shown below: SEQ ID NO: 49204-49576.

For example, when the gene editing system comprises CRISPR/Cas9, a protospacer adjacent motif (PAM) may be present downstream of the region targeted by the guide nucleic acid molecule of the present invention, and the protospacer adjacent motif (PAM) may be AGG, TGG, GGG, or CGG. For example, when the PAM region of the target gene is determined, a person skilled in the

art may readily determine a target sequence consisting of about 15 to about 25 (for example, about 15, about 16, about 17, about 18, about 19, about 20, about 21, about 22, about 23, about 24, about 25) nucleotides upstream of the 5' end of the PAM of the target gene, and an appropriate gRNA may be designed for the target sequence. For example, the guide nucleic acid molecule is capable of binding to a sequence consisting of about 15 to about 25 nucleotides upstream of the 5' end of a protospacer adjacent motif (PAM) selected from the group shown below: AGG, TGG, GGG, and CGG.

For example, when the gene editing system comprises CRISPR/Cas12, a protospacer adjacent motif (PAM) may be present upstream of the region targeted by the guide nucleic acid molecule of the present invention, and the protospacer adjacent motif (PAM) may be NTTN, TTYN, VTTV, TRTV, TTTV, TATV, TYCV, TNN, or NTN, and may also be TTTN, ATTN, GTTN, CTTN, TTC, TTG, TTA, TTT, TAN, TGN, or TCN, wherein N is A, T, C or G, Y is T or C, V is A, C or G, and R is A or G. For example, the protospacer adjacent motif (PAM) may be TTTN. For example, the protospacer adjacent motif (PAM) may be TTN. For example, when the PAM region of the target gene is determined, a person skilled in the art may readily determine a target sequence consisting of about 15 to about 25 (for example, about 15, about 16, about 17, about 18, about 19, about 20, about 21, about 22, about 23, about 24, about 25) nucleotides downstream of the 3' end of the PAM of the target gene, and an appropriate gRNA may be designed for the target sequence. For example, the guide nucleic acid molecule is capable of binding to a sequence consisting of about 15 to about 25 nucleotides downstream of the 3' end of a protospacer adjacent motif (PAM) selected from the group shown below: NTTN, TTYN, VTTV, TRTV, TTTV, TATV, TYCV, TNN, or NTN, and may also be TTTN, ATTN, GTTN, CTTN, TTC, TTG, TTA, TTT, TAN, TGN, or TCN, wherein N is A, T, C or G, Y is T or C, V is A, C or G, and R is A or G. For example, the protospacer adjacent motif (PAM) may be TTTN. For example, the protospacer adjacent motif (PAM) may be TTN.

For example, when the gene editing system of the present invention comprises wild-type Cas12a (which may also be referred to as Cpf1, such as AsCas12a, FnCas12a, LbCas12a, BbCas12a, CMaCas12a, and OsCas12a), a PAM sequence selected from the following may be present upstream of the region targeted by the guide nucleic acid molecule of the present invention: NTTN, wherein N may be A, T, C or G. For example, when the PAM region of the target gene is determined, a person skilled in the art may readily determine a target sequence consisting of about 17 to about 25 (for example, about 17, about 18, about 19, about 20, about 21, about 22, about 23, about 24, or about 25) nucleotides downstream of the 3' end of the PAM of the target gene, and an appropriate gRNA may be designed for the target sequence.

For example, when the gene editing system of the present invention comprises a mutant Cas12a, such as enAsCas12a (mutation sites E174R, S542R, and K548R), a PAM sequence selected from the following can be present upstream of the region targeted by the guide nucleic acid molecule of the present invention: TTYN (TTTN/TTCN), VTTV (ATTV/CTTV/GTTV), or TRTV (TATV/TGTV), wherein N can be A, T, C, or G, Y can be T or C, V can be A, C, or G, and R can be A or G. For example, when the PAM region of a target gene is determined, a target sequence consisting of about 17 to about 25 (e.g., about 17, about 18, about 19, about 20, about 21, about 22, about 23, about 24, or about 25) nucleotides downstream of the 3' end of the PAM of the target gene can be readily determined by a person skilled in the art, and an appropriate gRNA can be designed for the target sequence.

For example, when the gene editing system of the present invention comprises a mutant Cas12a, such as opAsCas12a (mutation sites: E174R and S542R), a PAM sequence selected from the following can be present upstream of the region targeted by the guide nucleic acid molecule of the present invention: TTTV (TTTA, TTTC, or TTTG), wherein V can be A, C, or G. For example, when the PAM region of a target gene is determined, a target sequence consisting of about 17 to about 25 (e.g., about 17, about 18, about 19, about 20, about 21, about 22, about 23, about 24, or about 25) nucleotides downstream of the 3' end of the PAM of the target gene can be readily determined by a person skilled in the art, and an appropriate gRNA can be designed for the target sequence.

For example, when the gene editing system of the present invention comprises a mutant Cas12a, such as AsCas12a Ultra (mutation sites: M537R and F870L), a PAM sequence selected from the following can be present upstream of the region targeted by the guide nucleic acid molecule of the present invention: TTTV, TATV, or TYCV, wherein V can be A, C, or G, and Y can be T or C. For example, when the PAM region of a target gene is determined, a target sequence consisting of about 17 to about 25 (e.g., about 17, about 18, about 19, about 20, about 21, about 22, about 23, about 24, or about 25) nucleotides downstream of the 3' end of the PAM of the target gene can be readily determined by a person skilled in the art, and an appropriate gRNA can be designed for the target sequence.

For example, when the gene editing system of the present invention comprises a mutant Cas12a, such as hfCas12Max (mutation sites: N243R/E336R/D892R) and Cas12Max (mutation site: N243R), a PAM sequence selected from the following can be present upstream of the region targeted by the guide nucleic acid molecule of the present invention: TNN or NTN, wherein N can be A, T, C, or G. For example, when the PAM region of a target gene is determined, a target sequence consisting of about 17 to about 25 (e.g., about 17, about 18, about 19, about 20, about 21, about 22, about 23, about 24, or about 25) nucleotides downstream of the 3' end of the PAM of the target gene can be readily determined by a person skilled in the art, and an appropriate gRNA can be designed for the target sequence.

For example, when the gene editing system of the present invention comprises wild-type Cas12b or mutant Cas12b (such as an AaCas12b protein from Alicyclobacillus acidiphilus), a PAM sequence selected from the following can be present upstream of the region targeted by the guide nucleic acid molecule of the present invention: TTN, wherein N can be A, T, C, or G. For example, when the PAM region of a target gene is determined, a target sequence consisting of about 17 to about 25 (e.g., about 17, about 18, about 19, about 20, about 21, about 22, about 23, about 24, or about 25) nucleotides downstream of the 3' end of the PAM of the target gene can be readily determined by a person skilled in the art, and an appropriate gRNA can be designed for the target sequence.

For example, when the gene editing system of the present invention comprises wild-type Cas12i or mutant Cas12i (a Cas protein having a smaller size), a PAM sequence selected from the following can be present upstream of the region targeted by the guide nucleic acid molecule of the present invention: TTN or TTTN, wherein N can be A, T, C, or G. For example, when the PAM region of a target gene is determined, a target sequence consisting of about 17 to about 25 (e.g., about 17, about 18, about 19, about 20, about 21, about 22, about 23, about 24, or about 25) nucleotides downstream of the 3' end of the PAM of the target gene can be readily determined by a person skilled in the art, and an appropriate gRNA can be designed for the target sequence.

For example, the guide nucleic acid molecule can comprise a target sequence consisting of about 15 to about 25 nucleotides upstream of a PAM region indicated by AGG, TGG, GGG, and/or CGG in DNA in which a gene encoding a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is located. For example, the guide nucleic acid molecule can comprise a target sequence consisting of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 22 to about 23, about 15 to about 21, about 17 to about 21, about 19 to about 21, about 20 to about 21, about 15 to about 20, about 17 to about 20, about 19 to about 20, about 15 to about 19, about 17 to about 19, or about 15 to about 17 nucleotides upstream of a PAM region indicated by AGG, TGG, GGG, and/or CGG in DNA in which a gene encoding a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is located. For example, the target sequence can be a region defined by genomic coordinates shown in Tables 2H-2N, or a fragment thereof.

For example, the guide nucleic acid molecule can comprise an sgRNA targeting CRP as shown in any one of SEQ ID NO: 16332-16805.

For example, the guide nucleic acid molecule can comprise an sgRNA targeting CYLD as shown in any one of SEQ ID NO: 16806-18300.

For example, the guide nucleic acid molecule can comprise an sgRNA targeting CBLIF as shown in any one of SEQ ID NO: 18301-18850.

For example, the guide nucleic acid molecule can comprise an sgRNA targeting KLF4 as shown in any one of SEQ ID NO: 18851-19772.

For example, the guide nucleic acid molecule can comprise an sgRNA targeting NDST1 as shown in any one of SEQ ID NO: 19773-22463.

For example, the guide nucleic acid molecule can comprise an sgRNA targeting NLRP1 as shown in any one of SEQ ID NO: 22464-24415.

For example, the guide nucleic acid molecule can comprise an sgRNA targeting SCGB1A1 as shown in any one of SEQ ID NO: 24416-24788.

For example, as compared with cells in which the expression and/or activity of the target gene is not changed, in cells obtained by reducing the expression and/or weakening the activity of the target gene in the cells, the proportion of cells expressing a product of the target gene can be reduced and/or the expression level of the target gene in a single cell can be decreased.

For example, in the method of the present invention, as compared with cells in which the expression and/or activity of the target gene is not changed, the proportion of cells expressing a product of the target gene in cells obtained by reducing the expression and/or weakening the activity of the target gene in the cells is reduced by at least about 5%. For example, the proportion of cells expressing a product of a gene encoding a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is reduced by at least about 100-5%, e.g., at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the proportion of cells expressing a product of a gene encoding a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, can be from an observable proportion of cells to 1%. For example, the proportion of cells expressing a product of a gene encoding a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, can be reduced to at least about 100-1%, e.g., at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, or at least about 1%. For example, the proportion of cells expressing a product of a gene encoding a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, can be detected by a flow cytometer.

For example, in cells obtained by reducing the expression and/or weakening the activity of the target gene of the cells as described in the method of the present invention, the proportion of cells expressing a product of a gene encoding a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, is up to about 95%. For example, the proportion of cells expressing the product of the gene encoding the member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or the functionally active fragment thereof, is up to about 95-5%, for example, up to about 95%, up to about 90%, up to about 80%, up to about 70%, up to about 60%, up to about 50%, up to about 40%, up to about 30%, up to about 20%, up to about 19%, up to about 18%, up to about 17%, up to about 16%, up to about 15%, up to about 14%, up to about 13%, up to about 12%, up to about 11%, up to about 10%, up to about 9%, up to about 8%, up to about 7%, up to about 6%, or up to about 5%. For example, the proportion of cells expressing the product of the gene encoding the member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or the functionally active fragment thereof, is detected by flow cytometry.

For example, compared with cells in which the expression and/or activity of the target gene is not changed in the method of the present invention, in cells obtained by reducing the expression and/or weakening the activity of the target gene of the cells, the expression level of the target gene in a single cell is decreased by at least about 5%. For example, the expression level of the target gene in a single cell is decreased by at least about 100-5%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the expression level of the target gene in a single cell is from an observable expression level to 1%. For example, the expression level of the target gene in a single cell is decreased to at least about 100-1%, for example, at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, or at least about 1%.

For example, in cells obtained by reducing the expression and/or weakening the activity of the target gene of the cells by the method of the present invention, the expression level of the target gene in a single cell is up to about 95% of that of cells in which the expression and/or activity of the target gene is not changed. For example, the expression level, in a single cell, of a gene encoding a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof (for example, a gene encoding CRP, CYLD, CBLIF, KLF4, NDST1, NLRP1, or SCGB1A1) is up to about 95-5% of that of cells in which the expression and/or activity of the member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or the functionally active fragment thereof, is not changed, for example, up to about 95%, up to about 90%, up to about 80%, up to about 70%, up to about 60%, up to about 50%, up to about 40%, up to about 30%, up to about 20%, up to about 19%, up to about 18%, up to about 17%, up to about 16%, up to about 15%, up to about 14%, up to about 13%, up to about 12%, up to about 11%, up to about 10%, up to about 9%, up to about 8%, up to about 7%, up to about 6%, or up to about 5%.

For example, the method of the present invention comprises subjecting the cells to at least one stage, wherein, in the in vitro expansion of the at least one stage, the expression of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family in the cells is reduced and/or the activity thereof is weakened.

For example, TIL derived from tumor tissue, tumor-associated lymph nodes with or without tumor metastasis, tumor metastatic lesions, fragments of paracancerous tissue, pleural effusion and/or ascites and not subjected to in vitro expansion is subjected to a first stage in vitro expansion and a second stage in vitro expansion, wherein during the second stage in vitro expansion, the expression of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family in the TIL subjected to the first stage in vitro expansion is reduced and/or the activity thereof is weakened.

For example, the first stage in vitro expansion is performed for at least 7 days. For example, the second stage in vitro expansion is performed for at least about 7 days.

For example, in the in vitro expansion of the present invention in a single stage, the cells are brought into contact with the one or more cell activators, and the expression and/or activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and/or a functionally active fragment thereof, in the cells is reduced. For example, the cell activator comprises an agonist of one or more targets selected from the group consisting of CD3, CD28, HVEM, CD40L, OX40, and 4-1BB. For example, in the in vitro expansion in the single stage, the expression of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family in the cells of the present invention is reduced and/or the activity thereof is weakened, and the cell is brought into contact with the one or more cell activators of the present invention. For example, in the first stage in vitro expansion of the present invention, the expression of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family in the TIL of the present invention is reduced and/or the activity thereof is weakened, and the TIL is brought into contact with the one or more cell activators of the present invention. For example, in the second stage in vitro expansion of the present invention, the expression of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family in the TIL of the present invention is reduced and/or the activity thereof is weakened, and the TIL is brought into contact with the one or more cell activators of the present invention. For example, in the third stage in vitro expansion of the present invention, the expression of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family in the TIL of the present invention is reduced and/or the activity thereof is weakened, and the TIL is brought into contact with the one or more cell activators of the present invention.

For example, in a single stage in vitro expansion, the cells of the present invention are caused to, substantially simultaneously, reduce the expression and/or attenuate the activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and to be contacted with one or more cell activators of the present invention. For example, in a single-stage in vitro expansion, the cells of the present invention are first caused to reduce the expression and/or attenuate the activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, for example, 2-48 hours in advance, for example, 2 hours in advance, 4 hours in advance, 8 hours in advance, 12 hours in advance, 24 hours in advance, or 48 hours in advance, and then are contacted with one or more cell activators of the present invention. For example, in a single-stage in vitro expansion, the cells of the present invention are first contacted with one or more cell activators of the present invention, for example, 2-48 hours in advance, for example, 2 hours in advance, 4 hours in advance, 8 hours in advance, 12 hours in advance, 24 hours in advance, or 48 hours in advance, and then are caused to reduce the expression and/or attenuate the activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family.

For example, in the first stage in vitro expansion of the present invention, the TIL of the present invention are caused to, substantially simultaneously, reduce the expression and/or attenuate the activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and to be contacted with one or more cell activators of the present invention. For example, in the second stage in vitro expansion of the present invention, the TIL of the present invention are caused to, substantially simultaneously, reduce the expression and/or attenuate the activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and to be contacted with one or more cell activators of the present invention. For example, in the third stage in vitro expansion of the present invention, the TIL of the present invention are caused to, substantially simultaneously, reduce the expression and/or attenuate the activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and to be contacted with one or more cell activators of the present invention.

For example, the second stage in vitro expansion of the present invention is carried out for at least about 9 days. For example, the second stage in vitro expansion of the present invention is carried out for at least about 9 days, at least about 10 days, at least about 11 days, at least about 12 days, at least about 13 days, or at least about 14 days. For example, the second stage in vitro expansion of the present invention is carried out for about 9 days to about 14 days, about 10 days to about 14 days, about 11 days to about 14 days, about 12 days to about 14 days, about 13 days to about 14 days, about 9 days to about 13 days, about 10 days to about 13 days, about 11 days to about 13 days, about 12 days to about 13 days, about 9 days to about 12 days, about 10 days to about 12 days, about 11 days to about 12 days, or about 10 days to about 11 days. For example, the second stage in vitro expansion of the present invention is considered a REP (rapid expansion protocol) stage. For example, the first stage in vitro expansion of the present invention is considered a preREP stage.

For example, the number of days for which the second stage in vitro expansion of the present invention is carried out is calculated from the start time point of the second stage in vitro expansion. For example, at the time when the second stage in vitro expansion starts, it is considered that the second stage in vitro expansion has been carried out for about 0 hours. For example, after about 24 hours have elapsed after the start of the second stage in vitro expansion, it is considered that the second stage in vitro expansion has been carried out for about 1 day. For example, on the day on which the second stage in vitro expansion starts, it is considered that the second stage in vitro expansion has been carried out for about 0 days. For example, the number of days for which the second stage in vitro expansion of the present invention is carried out is calculated by the number of days during which the second stage in vitro expansion is carried out. For example, on the second day after the start of the second stage in vitro expansion, it is considered that the second stage in vitro expansion has been carried out for about 1 day.

For example, the cell activator of the present invention comprises one or more selected from the group consisting of: CD80, CD86, B7-H3, 4-1BBL, CD27, CD30, CD134, B7h, CD40, LIGHT, and functionally active fragments thereof. For example, the cell activator of the present invention comprises an agonist of one or more targets selected from the group consisting of: CD3, CD28, HVEM, CD40L, OX40, and 4-1BB. For example, the cell activator of the present invention comprises an antibody against CD3, CD28, HVEM, CD40L, OX40, and 4-1BB, and antigen-binding fragments thereof. For example, the cell activator of the present invention comprises a CD3 agonist. For example, the cell activator of the present invention comprises an anti-CD3 antibody and/or an antigen-binding fragment thereof, for example OKT3 from Miltenyi Biotech, or SP34 from BD. For example, the cell activator of the present invention comprises a CD28 agonist. For example, the cell activator of the present invention comprises an anti-CD28 antibody and/or an antigen-binding fragment thereof, for example, Merck 15E8.

For example, the cell activator of the present invention comprises an anti-CD3 antibody and/or an antigen-binding fragment thereof, for example, comprises the light chain VL and heavy chain VH of OKT3 from Miltenyi Biotech, and comprises the light chain VL and heavy chain VH of SP34 from BD. For example, the cell activator of the present invention comprises a CD28 agonist. For example, the cell activator of the present invention comprises an anti-CD28 antibody and/or an antigen-binding fragment thereof, for example, comprises the light chain VL and heavy chain VH of Merck 15E8. For example, the cell activator of the present invention comprises an anti-CD3 antibody and/or an antigen-binding fragment thereof, for example, comprises the light chain LCDR1-3 and heavy chain HCDR1-3 of OKT3 from Miltenyi Biotech, and comprises the light chain LCDR1-3 and heavy chain HCDR1-3 of SP34 from BD, and the anti-CD3 antibody and/or antigen-binding fragment thereof of the present invention has CD3 binding capability. For example, the cell activator of the present invention comprises a CD28 agonist. For example, the cell activator of the present invention comprises an anti-CD28 antibody and/or an antigen-binding fragment thereof, for example, comprises the light chain LCDR1-3 and heavy chain HCDR1-3 of Merck 15E8, and the anti-CD28 antibody and/or antigen-binding fragment thereof of the present invention has CD28 binding capability. In the present invention, the antibody or antigen-binding protein of the present invention comprises at least one CDR in an antibody heavy chain variable region VH and/or at least one CDR in an antibody light chain variable region VL. The CDR of the present invention is defined according to the IMGT nomenclature, the CDR of the present invention is defined according to Chothia, or the CDR of the present invention is defined according to Kabat.

For example, contacting the cells of the present invention with one or more cell activators of the present invention comprises one or more modes selected from the group consisting of: (1) the cell activator of the present invention is added to a cell culture medium of the cells of the present invention; (2) the cell activator of the present invention is added to a cell culture medium of the cells of the present invention; (3) a composition comprising the cell activator of the present invention is added to a cell culture medium of the cells of the present invention. For example, contacting the cells of the present invention with one or more cell activators of the present invention comprises adding a composition comprising the cell activator of the present invention to a cell culture medium of the cells of the present invention. For example, contacting the cells of the present invention with one or more cell activators of the present invention comprises adding a CD28 antibody and a CD3 antibody of the present invention to a cell culture medium of the cells of the present invention.

For example, an initial concentration of the cell activator in a cell culture medium of the cells of the present invention is at least about 30 ng/mL. For example, an initial concentration of the CD28 antibody of the present invention in a cell culture medium of the cells of the present invention is at least about 30 ng/mL; for example, an initial concentration of the CD3 antibody of the present invention in a cell culture medium of the cells of the present invention is at least about 30 ng/mL. For example, selection of the initial concentration of the CD28 antibody of the present invention is independent of selection of the initial concentration of the CD3 antibody of the present invention; for example, the initial concentrations of the CD28 antibody and the CD3 antibody of the present invention in a cell culture medium of the cells of the present invention are combined in any manner. For example, the initial concentration of the CD28 antibody of the present invention in a cell culture medium of the cells of the present invention is arbitrarily selected from about 30 ng/mL- about 300 ng/mL. For example, the initial concentration of the CD3 antibody of the present invention in a cell culture medium of the cells of the present invention is arbitrarily selected from about 30 ng/mL- about 300 ng/mL. For example, the initial concentration of the CD28 antibody of the present invention in a cell culture medium of the cells of the present invention is arbitrarily selected from about 30 ng/mL-about 300 ng/mL, and the initial concentration of the CD3 antibody of the present invention in a cell culture medium of the cells of the present invention is arbitrarily selected from about 30 ng/mL- about 300 ng/mL, and selection of the initial concentration of the CD28 antibody of the present invention is independent of selection of the initial concentration of the CD3 antibody of the present invention. For example, the solid-phase medium of the present invention is about 500 nm to about 10 µm. For example, the solid-phase medium of the present invention is measured by transmission electron microscopy. For example, the solid-phase medium of the present invention is about 1 nm to about 500 nm. For example, the solid-phase medium of the present invention is about 100 nm to about 500 nm. For example, the solid-phase medium of the present invention is about 200 nm to about 500 nm. For example, the solid-phase medium of the present invention is measured by transmission electron microscopy.

For example, the solid-phase medium of the present invention may comprise a polymer. For example, the solid-phase medium of the present invention comprises dextran.

For example, each mg of the solid-phase medium of the present invention comprises at least about 25 µg of the cell activator of the present invention.

For example, a solid-phase medium comprising the cell activator of the present invention is added to a cell culture medium of the cells of the present invention at a ratio of the solid-phase medium of the present invention to the cells of the present invention of about 100:1- about 1:2000, preferably about 1:100- about 1:2000. For example, a solid-phase medium comprising the cell activator of the present invention is added to a cell culture medium of the cells of the present invention at a ratio of the solid-phase medium of the present invention to the cells of the present invention of about 2:1- about 1:2.

For example, when the diameter of the solid-phase medium of the present invention is from about 500 nm to about 10 µm, a solid-phase medium comprising a cell activator of the present invention may be added to a cell culture medium of the cells of the present invention at a ratio of the solid-phase medium of the present invention to the cells of the present invention of about 2:1 to about 1:2. For example, when the diameter of the solid-phase medium of the present invention is from about 500 nm to about 10 µm, a solid-phase medium comprising a cell activator of the present invention, for example, a CD3 agonist and/or a CD28 agonist, may be added to a cell culture medium of the cells of the present invention at a ratio of the solid-phase medium of the present invention to the cells of the present invention of about 2:1 to about 1:2, about 2:1 to about 1:1, or about 1:1 to about 1:2.

For example, when the diameter of the solid-phase medium of the present invention is from about 100 nm to about 500 nm, a solid-phase medium comprising a cell activator of the present invention may be added to a cell culture medium of the cells of the present invention at a ratio of the solid-phase medium of the present invention to the cells of the present invention of about 1:100 to about 1:2000. For example, when the diameter of the solid-phase medium of the present invention is from about 100 nm to about 500 nm, a solid-phase medium comprising a CD28 agonist and a CD3 agonist of the present invention may be added to a cell culture medium of the cells of the present invention at a ratio of the solid-phase medium of the present invention to the cells of the present invention of about 1:100 to about 1:2000, about 1:200 to about 1:2000, about 1:300 to about 1:2000, about 1:400 to about 1:2000, about 1:500 to about 1:2000, about 1:600 to about 1:2000, about 1:700 to about 1:2000, about 1:800 to about 1:2000, about 1:900 to about 1:2000, about 1:1000 to about 1:2000, about 1:1200 to about 1:2000, about 1:1400 to about 1:2000, about 1:1600 to about 1:2000, or about 1:1800 to about 1:2000.

For example, the method of the present invention may further comprise: during in vitro expansion of the present invention at at least one stage, the cells of the present invention are brought into contact with one or more cell growth factors.

For example, in a single stage of the in vitro expansion of the present invention, the cells of the present invention may be brought into contact with the cell activator of the present invention and brought into contact with one or more cell growth factors of the present invention. For example, in the first stage in vitro expansion of the present invention, the TIL of the present invention may be brought into contact with the cell activator of the present invention and brought into contact with one or more cell growth factors of the present invention. For example, in the second stage in vitro expansion of the present invention, the TIL of the present invention may be brought into contact with the cell activator of the present invention and brought into contact with one or more cell growth factors of the present invention. For example, in the third stage in vitro expansion of the present invention, the TIL of the present invention may be brought into contact with the cell activator of the present invention and brought into contact with one or more cell growth factors of the present invention.

For example, in a single stage of the in vitro expansion of the present invention, the cells of the present invention may be brought into contact with the cell activator of the present invention and one or more cell growth factors of the present invention. For example, in a single stage of the in vitro expansion of the present invention, the cells of the present invention may be brought into contact substantially simultaneously with one or more cell growth factors of the present invention and one or more cell activators of the present invention. For example, in a single stage of the in vitro expansion of the present invention, the cells of the present invention may first be brought into contact with one or more cell growth factors of the present invention, for example, 2-48 hours in advance, for example, 2 hours in advance, 4 hours in advance, 8 hours in advance, 12 hours in advance, 24 hours in advance, or 48 hours in advance, and then be brought into contact with one or more cell activators of the present invention. For example, in a single stage of the in vitro expansion of the present invention, the cells of the present invention may first be brought into contact with one or more cell activators of the present invention, for example, 2-48 hours in advance, for example, 2 hours in advance, 4 hours in advance, 8 hours in advance, 12 hours in advance, 24 hours in advance, or 48 hours in advance, and then be brought into contact with one or more cell growth factors of the present invention.

For example, in the first stage in vitro expansion of the present invention, the cells of the present invention may be brought into contact with the cell activator of the present invention and one or more cell growth factors of the present invention. For example, in the second stage in vitro expansion of the present invention, the TIL of the present invention may be brought into contact with the cell activator of the present invention and one or more cell growth factors of the present invention. For example, in the third stage in vitro expansion of the present invention, the TIL of the present invention may be brought into contact with the cell activator of the present invention and one or more cell growth factors of the present invention.

For example, the cell growth factor of the present invention may be selected from one or more of the group consisting of: IL-2, IL-7, IL-12, IL-15, IL-21, interferon-γ, and functional active fragments thereof. For example, the cell growth factor of the present invention may comprise IL-2 and/or a functionally active fragment thereof. For example, the functionally active fragment of IL-2 may comprise an IL-2 fragment known in the art that is capable of binding to an IL-2 receptor of a cell. For example, the cell growth factor of the present invention may comprise IL-2 and/or a functionally active fragment thereof, IL-7 and/or a functionally active fragment thereof, and IL-15 and/or a functionally active fragment thereof.

For example, bringing the cells of the present invention into contact with one or more cell growth factors of the present invention may comprise adding the cell growth factor of the present invention to a cell culture medium of the cells of the present invention. For example, the initial concentration of the cell growth factor of the present invention in the cell culture medium of the cells of the present invention may be at least about 300 IU/mL. For example, the initial concentration of IL-2 of the present invention in the cell culture medium of the cells of the present invention may be at least about 300-9000 IU/mL, for example, at least about 300 IU/mL, at least about 350 IU/mL, at least about 400 IU/mL, at least about 500 IU/mL, at least about 600 IU/mL, at least about 700 IU/mL, at least about 800 IU/mL, at least about 900 IU/mL, at least about 1000 IU/mL, at least about 1100 IU/mL, at least about 1200 IU/mL, at least about 1300 IU/mL, at least about 1400 IU/mL, at least about 1500 IU/mL, at least about 2000 IU/mL, at least about 2500 IU/mL, at least about 2600 IU/mL, at least about 2700 IU/mL, at least about 2800 IU/mL, at least about 2900 IU/mL, at least about 3000 IU/mL, at least about 3100 IU/mL, at least about 3200 IU/mL, at least about 3300 IU/mL, at least about 3400 IU/mL, at least about 3500 IU/mL, at least about 4000 IU/mL, at least about 4500 IU/mL, at least about 5000 IU/mL, at least about 5500 IU/mL, at least about 6000 IU/mL, at least about 6500 IU/mL, at least about 7000 IU/mL, at least about 7500 IU/mL, at least about 8000 IU/mL, at least about 8500 IU/mL, or at least about 9000 IU/mL.

For example, bringing the cells of the present invention into contact with IL-2, IL-7, and IL-15, as compared with bringing the cells into contact with IL-2 alone, may reduce the amount of cytokines used. For example, under conditions in which IL-7 and IL-15 are added, the amount of IL-2 added may be reduced. For example, the concentration of IL-7 may be about 1 to 1000 ng/mL, preferably about 1-100 ng/mL. For example, the concentration of IL-15 may be about 1 to 1000 ng/mL, preferably about 1-100 ng/mL. For example, with respect to the amount of IL-2 added, the amount may be reduced to a range commonly used in the art for various immune cells, for example, reduced to 50-10% of the range commonly used in the art, for example, 50%, 20%, or 10%. For example, for the amount of IL-2 added for TCR-T, the range commonly used in the art may be 30-300 IU/mL. For example, for the amount of IL-2 added for TIL, the range commonly used in the art may be 300-9000 IU/mL (for example, 1000-9000 IU/mL).

For example, the method of the present invention may further comprise: during in vitro expansion of the present invention at at least one stage, the cells of the present invention may be co-cultured with feeder cells.

For example, in a single stage of the in vitro expansion of the present invention, the cells of the present invention may be brought into contact with one or more cell activators and/or one or more cell growth factors and co-cultured with feeder cells of the present invention; for example, the single stage of the in vitro expansion of the present invention may refer to in vitro expansion of the present invention in the same stage, for example, in the first stage in vitro expansion of the present invention, in the second stage in vitro expansion of the present invention, or in the third stage in vitro expansion of the present invention, and the like.

For example, in the first stage in vitro expansion of the present invention, the TIL of the present invention may be brought into contact with one or more cell activators and/or one or more cell growth factors and co-cultured with feeder cells of the present invention. For example, in the second stage in vitro expansion of the present invention, the TIL of the present invention may be brought into contact with one or more cell activators and/or one or more cell growth factors of the present invention and co-cultured with feeder cells of the present invention. For example, in the third stage in vitro expansion of the present invention, the TIL of the present invention may be brought into contact with one or more cell activators and/or one or more cell growth factors of the present invention and co-cultured with feeder cells of the present invention.

For example, in a single stage of the in vitro expansion of the present invention, after the cells of the present invention are brought into contact with one or more cell activators of the present invention and/or one or more cell growth factors for a certain period of time, the cells may then be co-cultured with feeder cells of the present invention. For example, in the first stage in vitro expansion of the present invention, after the TIL of the present invention is brought into contact with one or more cell activators of the present invention and/or one or more cell growth factors for a certain period of time, the TIL may then be co-cultured with feeder cells of the present invention. For example, in the second stage in vitro expansion of the present invention, after the TIL of the present invention is brought into contact with one or more cell activators of the present invention and/or one or more cell growth factors for a certain period of time, the TIL may then be co-cultured with feeder cells of the present invention. For example, in the third stage in vitro expansion of the present invention, after the TIL of the present invention is brought into contact with one or more cell activators of the present invention and/or one or more cell growth factors for a certain period of time, the TIL may then be co-cultured with feeder cells of the present invention.

For example, in the single stage in vitro expansion of the present invention, the cells of the present invention may be brought into contact with one or more cell activators of the present invention and/or one or more cell growth factors for a certain period of time, and then with feeder cells of the present invention. For example, the certain period of time of the present invention may be at least about 1 hour. For example, the certain period of time of the present invention may be at least about 1-72 hours, for example, at least about 1 hour, at least about 2 hours, at least about 3 hours, at least about 4 hours, at least about 5 hours, at least about 6 hours, at least about 7 hours, at least about 8 hours, at least about 9 hours, at least about 10 hours, at least about 11 hours, at least about 12 hours, at least about 13 hours, at least about 14 hours, at least about 15 hours, at least about 16 hours, at least about 17 hours, at least about 18 hours, at least about 19 hours, at least about 20 hours, at least about 21 hours, at least about 22 hours, at least about 23 hours, at least about 24 hours, at least about 36 hours, at least about 48 hours, at least about 60 hours, or at least about 72 hours. For example, the certain period of time of the present invention may be about 2 hours to about 72 hours. For example, the certain period of time of the present invention may be about 6 hours to about 7 hours, about 6 hours to about 8 hours, about 6 hours to about 9 hours, about 6 hours to about 10 hours, about 6 hours to about 11 hours, about 6 hours to about 12 hours, about 6 hours to about 13 hours, about 6 hours to about 14 hours, about 6 hours to about 15 hours, about 6 hours to about 16 hours, about 6 hours to about 17 hours, about 6 hours to about 18 hours, about 6 hours to about 19 hours, about 6 hours to about 20 hours, about 6 hours to about 21 hours, about 6 hours to about 22 hours, about 6 hours to about 23 hours, about 6 hours to about 24 hours, about 6 hours to about 36 hours, about 6 hours to about 48 hours, about 6 hours to about 60 hours, or about 6 hours to about 72 hours. For example, the certain period of time of the present invention may be about 12 hours to about 13 hours, about 12 hours to about 14 hours, about 12 hours to about 15 hours, about 12 hours to about 16 hours, about 12 hours to about 17 hours, about 12 hours to about 18 hours, about 12 hours to about 19 hours, about 12 hours to about 20 hours, about 12 hours to about 21 hours, about 12 hours to about 22 hours, about 12 hours to about 23 hours, about 12 hours to about 24 hours, about 12 hours to about 36 hours, about 12 hours to about 48 hours, about 12 hours to about 60 hours, or about 12 hours to about 72 hours. For example, the certain period of time of the present invention may be about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 36 hours, about 48 hours, about 60 hours, or about 72 hours.

For example, the feeder cells of the present invention may comprise antigen-presenting cells. For example, the feeder cells of the present invention may comprise one or more selected from the group consisting of: peripheral blood mononuclear cells, dendritic cells, and artificial antigen-presenting cells. For example, the feeder cells of the present invention may be peripheral blood mononuclear cells. For example, the feeder cells of the present invention may be feeder cells that have been irradiated. For example, the feeder cells of the present invention may be isolated artificial antigen-presenting cells (aAPCs), and the artificial antigen-presenting cells of the present invention may comprise cells expressing HLA-A/B/C, CD64, CD80, ICOS-L, and/or CD58, and may be modified to express more than one cell activator of the present invention. For example, the feeder cells of the present invention may be irradiated; for example, the feeder cells may be irradiated with gamma rays, or may be irradiated with X-rays.

For example, co-culturing the cells of the present invention with the feeder cells of the present invention may comprise bringing a surface of the feeder cells of the present invention into contact with a surface of the cells of the present invention. For example, co-culturing the cells of the present invention with the feeder cells of the present invention comprises adding the feeder cells of the present invention to a cell culture medium of the cells of the present invention.

For example, the feeder cells of the present invention may be added to a cell culture medium of the cells of the present invention at a ratio of the feeder cells of the present invention to the cells of the present invention of about 40:1 to about 400:1. For example, the feeder cells of the present invention may be added to a cell culture medium of the cells of the present invention at a ratio of the feeder cells of the present invention to the cells of the present invention of about 40:1 to about 400:1, about 40:1 to about 300:1, about 40:1 to about 200:1, about 40:1 to about 100:1, about 40:1 to about 90:1, about 40:1 to about 80:1, about 40:1 to about 70:1, about 40:1 to about 60:1, about 40:1 to about 50:1, about 50:1 to about 400:1, about 60:1 to about 400:1, about 70:1 to about 400:1, about 80:1 to about 400:1, about 90:1 to about 400:1, about 100:1 to about 400:1, about 200:1 to about 400:1, or about 300:1 to about 400:1.

In another aspect, a method for culturing tumor-infiltrating lymphocytes (TIL) is provided in the present invention, which may comprise: (A) bringing a first TIL population, which is derived from tumor tissue, tumor-associated lymph nodes with or without tumor metastasis, tumor metastatic lesions, fragments of paracancerous tissue, pleural effusion and/or ascites and has not been subjected to in vitro expansion, into contact with one or more cell growth factors; wherein a second TIL population is obtained through step (A); and (B) reducing expression and/or weakening activity of members selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family in the second TIL population; wherein a third TIL population is obtained through step (B).

In another aspect, a method for culturing tumor-infiltrating lymphocytes (TIL) is provided in the present invention, comprising: (A) bringing a first TIL population, which is derived from tumor tissue, tumor-associated lymph nodes with or without tumor metastasis, tumor metastatic lesions, fragments of paracancerous tissue, pleural effusion and/or ascites and has not been subjected to in vitro expansion, into contact with one or more T cell growth factors, wherein a second TIL population is obtained through step (A); (B) reducing expression and/or weakening activity of members selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family in the second TIL population, and bringing the second TIL population into contact with a T cell activator and/or a T cell growth factor, wherein a third TIL population is obtained through step (B); and (C) co-culturing the third TIL population with feeder cells, wherein a fourth TIL population is obtained through step (C).

In one embodiment, the first stage in vitro expansion of the present invention may be used interchangeably with step (A) in the method of the above aspect. In one embodiment, the second stage in vitro expansion of the present invention may be used interchangeably with step (B) in the method of the above aspect. In one embodiment, the TIL subjected to the first stage in vitro expansion of the present invention may be used interchangeably with the second TIL population obtained through step (A) in the method of the above aspect. In one embodiment, the TIL subjected to the second stage in vitro expansion of the present invention may be used interchangeably with the third TIL population obtained through step (B) in the method of the above aspect. In one embodiment, if needed, the third stage in vitro expansion of the present invention may be used interchangeably with an optionally added step (C) in the method of the above aspect. In one embodiment, if needed, the TIL subjected to the third stage in vitro expansion of the present invention may be used interchangeably with the fourth TIL population obtained through an optionally added step (C) in the method of the above aspect.

In another aspect, a method for culturing tumor-infiltrating lymphocytes (TIL) is provided in the present invention, which may comprise: (A) bringing a first TIL population, which is derived from tumor tissue, tumor-associated lymph nodes with or without tumor metastasis, tumor metastatic lesions, fragments of paracancerous tissue, pleural effusion and/or ascites and has not been subjected to in vitro expansion, into contact with a plurality of cell growth factors; wherein a second TIL population is obtained through step (A); and (B) bringing the second TIL population into contact with a plurality of cell growth factors, bringing the second TIL population into contact with a plurality of cell activators, reducing expression and/or weakening activity of members selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and co-culturing the TIL with feeder cells; wherein a third TIL population is obtained through step (B).

In another aspect, a method for culturing tumor-infiltrating lymphocytes (TIL) is provided in the present invention, which may comprise: (A) bringing a first TIL population, which is derived from tumor tissue, tumor-associated lymph nodes with or without tumor metastasis, tumor metastatic lesions, fragments of paracancerous tissue, pleural effusion and/or ascites and has not been subjected to in vitro expansion, into contact with a cell growth factor; wherein a second TIL population is obtained through step (A); and (B) bringing the second TIL population into contact with a cell growth factor, bringing the second TIL population into contact with a cell activator, reducing expression and/or weakening activity of members selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family, and co-culturing the TIL with feeder cells, wherein the members selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family may respectively comprise CRP, CYLD, CBLIF, KLF4, NDST1, NLRP1, and SCGB1A1; wherein a third TIL population is obtained through step (B).

In another aspect, the present invention provides a method for culturing tumor-infiltrating lymphocytes (TIL). A method for obtaining TIL cells from a subject tissue sample may comprise surgically obtaining an in situ tumor sample or a metastatic tumor sample from a patient, wherein the weight is at least about 1 g, or multiple tissue pieces are combined. Tumor tissue, tumor-associated lymph nodes with or without tumor metastasis, tumor metastatic lesions, fragments of paracancerous tissue, pleural effusion and/or ascites are transported in a sample transport solution, for example, a commercially commonly used tumor tissue transport solution, tumor tissue preservation solution, or tumor tissue transfer solution, at about 2-8°C, and are processed within 48 hours. Tissue blocks may be mechanically disrupted to a size of about 1-27 cubic millimeters per piece, transferred into a gas-permeable culture bag or Grex, and cultured by adding a serum-free cell culture medium and IL-2 at a concentration of 300-9000 IU/mL (e.g., 1000-9000 IU/mL, e.g., 6000 IU/mL) for about 3-14 days. Cells in the culture medium are collected and transferred into a gas-permeable culture bag, or Grex, or a Xuri device; the serum-free cell culture medium may be added with a CD28 antibody of the present invention, a CD3 antibody, magnetic beads (e.g., Dynabeads) comprising a CD3 antibody and a CD28 antibody and/or a nanomatrix (e.g., transACT) comprising a CD3 antibody and a CD28 antibody, IL-2 at a concentration of 300-9000 IU/mL (e.g., 1000-9000 IU/mL, e.g., 6000 IU/mL), and an agent that reduces expression and/or weakens activity of a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family (wherein pentraxin family members may comprise CRP, peptidase C19 family members may comprise CYLD, cobalamin transporter family members may comprise CBLIF, Krueppel C2H2-type zinc finger protein family members may comprise KLF4, sulfotransferase 1 family members may comprise NDST1, Nod-like receptor (NLR) protein family members may comprise NLRP1, and secretoglobin family members may comprise SCGB1A1); for example, transduction may be performed with a ribonucleoprotein complex (RNP) formed by gRNA of the present invention and a Cas protein, or an LNP comprising gRNA and a Cas protein, or an LNP comprising a nucleic acid encoding gRNA and a Cas protein, such that the proportion of cells in the TIL in which a gene encoding a member selected from the pentraxin family, the peptidase C19 family, the cobalamin transporter family, the krueppel C2H2-type zinc finger protein family, the sulfotransferase 1 family, the Nod-like receptor (NLR) protein family, or the secretoglobin family is about 95% or less. After the TIL of the present invention is activated for a certain period of time, irradiated PBMC is added (TIL and PBMC at a ratio of about 1:40 to about 1:400), and expansion culture is performed for about 3-14 days. Cells in the culture medium may be collected using a cell processing system, washed, cryopreserved, and tested. The final product may have a CD3 proportion greater than 80%, a cell viability greater than 50%, and greater than 80% of the cells may be memory effector cells and effector cells. IFN-γ may be secreted after stimulation, and/or a characteristic of an upregulated proportion of activated cells may be present.

### Cells, compositions, kits, pharmaceutical uses

In one aspect, the present invention provides a cell, and the cell of the present invention may be obtained by culturing according to the culture method of the present invention. In one embodiment, the cell provided by the present invention may comprise cells obtained by culturing using one type or one batch of the culture method of the present invention. In one embodiment, the cell provided by the present invention may comprise cells obtained by culturing using multiple types or multiple batches of the culture method of the present invention and combined in any proportion.

In some embodiments, cells expanded using the method of the present invention may be administered to a patient as a pharmaceutical composition. In some embodiments, the pharmaceutical composition may be a suspension of cells in a sterile buffer. Cells expanded using PBMC of the present invention may be administered by any suitable route known in the art. In some embodiments, the cells may be administered as a single intra-arterial or intravenous infusion, and the infusion lasts about 30 to 60 minutes. Other suitable routes of administration may include intraperitoneal, intrathecal, and intralymphatic administration.

In some embodiments, any suitable dose of cells may be administered. In some embodiments, for example, when the tumor is melanoma, about 1×10⁹ to about 13.7×10¹⁰ cells may be administered, preferably about 2.3×10⁹ to about 13.7×10¹⁰ cells. In some embodiments, about 1×10⁹ to about 12×10¹⁰ cells may be administered. In some embodiments, about 1.2×10¹⁰ to about 4.3×10¹⁰ cells may be administered. In some embodiments, about 3×10¹⁰ to about 12×10¹⁰ cells may be administered. In some embodiments, about 4×10¹⁰ to about 10×10¹⁰ cells may be administered. In some embodiments, about 5×10¹⁰ to about 8×10¹⁰ cells may be administered. In some embodiments, about 6×10¹⁰ to about 8×10¹⁰ cells may be administered. In some embodiments, about 7×10¹⁰ to about 8×10¹⁰ cells may be administered. In some embodiments, a therapeutically effective dose may be about 1×10⁹ to about 13.7×10¹⁰, preferably about 2.3×10⁹ to about 13.7×10¹⁰. In some embodiments, a therapeutically effective dose may be about 1×10⁹ to about 12×10¹⁰ cells. In some embodiments, a therapeutically effective dose may be about 1.2×10¹⁰ to about 4.3×10¹⁰ cells. In some embodiments, a therapeutically effective dose may be about 3×10¹⁰ to about 12×10¹⁰ cells. In some embodiments, a therapeutically effective dose may be about 4×10¹⁰ to about 10×10¹⁰ cells. In some embodiments, a therapeutically effective dose may be about 5×10¹⁰ to about 8×10¹⁰ cells. In some embodiments, a therapeutically effective dose may be about 6×10¹⁰ to about 8×10¹⁰ cells. In some embodiments, a therapeutically effective dose may be about 7×10¹⁰ to about 8×10¹⁰ cells.

In some embodiments, the number of cells provided in the composition of the present invention may be about 1×10⁶-9×10¹³, for example, about 1×10⁶, about 2×10⁶, about 3×10⁶, about 4×10⁶, about 5×10⁶, about 6×10⁶, about 7×10⁶, about 8×10⁶, about 9×10⁶, about 1×10⁷, about 2×10⁷, about 3×10⁷, about 4×10⁷, about 5×10⁷, about 6×10⁷, about 7×10⁷, about 8×10⁷, about 9×10⁷, about 1×10⁸, about 2×10⁸, about 3×10⁸, about 4×10⁸, about 5×10⁸, about 6×10⁸, about 7×10⁸, about 8×10⁸, about 9×10⁸, about 1×10⁹, about 2×10⁹, about 3×10⁹, about 4×10⁹, about 5×10⁹, about 6×10⁹, about 7×10⁹, about 8×10⁹, about 9×10⁹, about 1×10¹⁰, about 2×10¹⁰, about 3×10¹⁰, about 4×10¹⁰, about 5×10¹⁰, about 6×10¹⁰, about 7×10¹⁰, about 8×10¹⁰, about 9×10¹⁰, about 1×10¹¹, about 2×10¹¹, about 3×10¹¹, about 4×10¹¹, about 5×10¹¹, about 6×10¹¹, about 7×10¹¹, about 8×10¹¹, about 9×10¹¹, about 1×10¹², about 2×10¹², about 3×10¹², about 4×10¹², about 5×10¹², about 6×10¹², about 7×10¹², about 8×10¹², about 9×10¹², about 1×10¹³, about 2×10¹³, about 3×10¹³, about 4×10¹³, about 5×10¹³, about 6×10¹³, about 7×10¹³, about 8×10¹³, or about 9×10¹³. In some embodiments, the range of the number of cells provided in the composition of the present invention may be about 1×10⁶ to 5×10⁶, about 5×10⁶ to 1×10⁷, about 1×10⁷ to 5×10⁷, about 5×10⁷ to 1×10⁸, about 1×10⁸ to 5×10⁸, about 5×10⁸ to 1×10⁹, about 1×10⁹ to 5×10⁹, about 5×10⁹ to 1×10¹⁰, about 1×10¹⁰ to 5×10¹⁰, about 5×10¹⁰ to 1×10¹¹, about 5×10¹¹ to 1×10¹², about 1×10¹² to 5×10¹², about 5×10¹² to 1×10¹³, about 1×10¹³ to 5×10¹³, or about 5×10¹³ to 9×10¹³.

In some embodiments, the cell concentration provided in the composition of the present invention may be less than about 100-0.0001%w/w, w/v or v/v of the composition, for example, about 100%, about 90%, about 80%, about 70%, about 60%, about 50%, about 40%, about 30%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1%, about 0.9%, about 0.8%, about 0.7%, about 0.6%, about 0.5%, about 0.4%, about 0.3%, about 0.2%, about 0.1%, about 0.09%, about 0.08%, about 0.07%, about 0.06%, about 0.05%, about 0.04%, about 0.03%, about 0.02%, about 0.01%, about 0.009%, about 0.008%, about 0.007%, about 0.006%, about 0.005%, about 0.004%, about 0.003%, about 0.002%, about 0.001%, about 0.0009%, about 0.0008%, about 0.0007%, about 0.0006%, about 0.0005%, about 0.0004%, about 0.0003%, about 0.0002%, or about 0.0001%w/w, w/v or v/v.

In some embodiments, the cell concentration provided in the composition of the present invention is greater than about 90-0.0001% w/w, w/v, or v/v, for example, about 90%, about 80%, about 70%, about 60%, about 50%, about 40%, about 30%, about 20%, about 19.75%, about 19.50%, about 19.25%, about 19%, about 18.75%, about 18.50%, about 18.25%, about 18%, about 17.75%, about 17.50%, about 17.25%, about 17%, about 16.75%, about 16.50%, about 16.25%, about 16%, about 15.75%, about 15.50%, about 15.25%, about 15%, about 14.75%, about 14.50%, about 14.25%, about 14%, about 13.75%, about 13.50%, about 13.25%, about 13%, about 12.75%, about 12.50%, about 12.25%, about 12%, about 11.75%, about 11.50%, about 11.25%, about 11%, about 10.75%, about 10.50%, about 10.25%, about 10%, about 9.75%, about 9.50%, about 9.25%, about 9%, about 8.75%, about 8.50%, about 8.25%, about 8%, about 7.75%, about 7.50%, about 7.25%, about 7%, about 6.75%, about 6.50%, about 6.25%, about 6%, about 5.75%, about 5.50%, about 5.25%, about 5%, about 4.75%, about 4.50%, about 4.25%, about 4%, about 3.75%, about 3.50%, about 3.25%, about 3%, about 2.75%, about 2.50%, about 2.25%, about 2%, about 1.75%, about 1.50%, about 1.25%, about 1%, about 0.9%, about 0.8%, about 0.7%, about 0.6%, about 0.5%, about 0.4%, about 0.3%, about 0.2%, about 0.1%, about 0.09%, about 0.08%, about 0.07%, about 0.06%, about 0.05%, about 0.04%, about 0.03%, about 0.02%, about 0.01%, about 0.009%, about 0.008%, about 0.007%, about 0.006%, about 0.005%, about 0.004%, about 0.003%, about 0.002%, about 0.001%, about 0.0009%, about 0.0008%, about 0.0007%, about 0.0006%, about 0.0005%, about 0.0004%, about 0.0003%, about or 0.0002%, or about 0.0001% w/w, w/v, or v/v.

In some embodiments, the cell concentration range provided in the composition of the present invention is about 0.0001% to about 50%, about 0.001% to about 40%, about 0.01% to about 30%, about 0.02% to about 29%, about 0.03% to about 28%, about 0.04% to about 27%, about 0.05% to about 26%, about 0.06% to about 25%, about 0.07% to about 24%, about 0.08% to about 23%, about 0.09% to about 22%, about 0.1% to about 21%, about 0.2% to about 20%, about 0.3% to about 19%, about 0.4% to about 18%, about 0.5% to about 17%, about 0.6% to about 16%, about 0.7% to about 15%, about 0.8% to about 14%, about 0.9% to about 12%, or about 1% to about 10% w/w, w/v, or v/v.

In some embodiments, the cell concentration range provided in the composition of the present invention is about 0.001% to about 10%, about 0.01% to about 5%, about 0.02% to about 4.5%, about 0.03% to about 4%, about 0.04% to about 3.5%, about 0.05% to about 3%, about 0.06% to about 2.5%, about 0.07% to about 2%, about 0.08% to about 1.5%, about 0.09% to about 1%, or about 0.1% to about 0.9% w/w, w/v, or v/v.

In some embodiments, the amount of cells provided in the composition of the present invention is equal to or less than about 10-0.0001g, for example, about 10g, about 9.5g, about 9.0g, about 8.5g, about 8.0g, about 7.5g, about 7.0g, about 6.5g, about 6.0g, about 5.5g, about 5.0g, about 4.5g, about 4.0g, about 3.5g, about 3.0g, about 2.5g, about 2.0g, about 1.5g, about 1.0g, about 0.95g, about 0.9g, about 0.85g, about 0.8g, about 0.75g, about 0.7g, about 0.65g, about 0.6g, about 0.55g, about 0.5g, about 0.45g, about 0.4g, about 0.35g, about 0.3g, about 0.25g, about 0.2g, about 0.15g, about 0.1g, about 0.09g, about 0.08g, about 0.07g, about 0.06g, about 0.05g, about 0.04g, about 0.03g, about 0.02g, about 0.01g, about 0.009g, about 0.008g, about 0.007g, about 0.006g, about 0.005g, about 0.004g, about 0.003g, about 0.002g, about 0.001g, about 0.0009g, about 0.0008g, about 0.0007g, about 0.0006g, about 0.0005g, about 0.0004g, about 0.0003g, about 0.0002g, or about 0.0001g.

In some embodiments, the amount of cells provided in the composition of the present invention is greater than about 0.0001-10g, for example, about 0.0001g, about 0.0002g, about 0.0003g, about 0.0004g, about 0.0005g, about 0.0006g, about 0.0007g, about 0.0008g, about 0.0009g, about 0.001g, about 0.0015g, about 0.002g, about 0.0025g, about 0.003g, about 0.0035g, about 0.004g, about 0.0045g, about 0.005g, about 0.0055g, about 0.006g, about 0.0065g, about 0.007g, about 0.0075g, about 0.008g, about 0.0085g, about 0.009g, about 0.0095g, about 0.01g, about 0.015g, about 0.02g, about 0.025g, about 0.03g, about 0.035g, about 0.04g, about 0.045g, about 0.05g, about 0.055g, about 0.06g, about 0.065g, about 0.07g, about 0.075g, about 0.08g, about 0.085g, about 0.09g, about 0.095g, about 0.1g, about 0.15g, about 0.2g, about 0.25g, about 0.3g, about 0.35g, about 0.4g, about 0.45g, about 0.5g, about 0.55g, about 0.6g, about 0.65g, about 0.7g, about 0.75g, about 0.8g, about 0.85g, about 0.9g, about 0.95g, about 1g, about 1.5g, about 2g, about 2.5g, about 3g, about 3.5g, about 4g, about 4.5g, about 5g, about 5.5g, about 6g, about 6.5g, about 7g, about 7.5g, about 8g, about 8.5g, about 9g, about 9.5g, or about 10g.

In some embodiments, the cells are administered as a single dose. Such administration is performed by injection, for example, by intravenous injection. In some embodiments, the cells are administered in multiple doses. The dose is administered once, twice, three times, four times, five times, six times, or more than six times per year. The dose is administered once per month, once every two weeks, once per week, or once every 2 days. In some embodiments, the administration of the cells is performed continuously.

In one aspect, a pharmaceutical composition is provided by the present invention. In some embodiments, the pharmaceutical composition comprises the cells of the present invention and a pharmaceutically acceptable carrier.

In one aspect, a kit is provided by the present invention, and the kit of the present invention comprises a cell activator, a cell growth factor and/or feeder cells of the cell culture method of the present invention, and an instruction manual describing the steps of the cell culture method of the present invention. In one aspect, a kit is provided by the present invention, and the kit of the present invention comprises the cells of the present invention and/or the pharmaceutical composition of the present invention.

In one aspect, a method for affecting the growth of cells, such as tumor cells, is provided by the present invention, and the method comprises administering the cells of the present invention and/or the pharmaceutical composition of the present invention to a subject. In some embodiments, affecting tumor growth comprises reducing the tumor volume to about 99-0.1% of that before administration, for example, about 99%, about 95%, about 90%, about 80%, about 70%, about 60%, about 50%, about 40%, about 30%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1%, about 0.9%, about 0.8%, about 0.7%, about 0.6%, about 0.5%, about 0.4%, about 0.3%, about 0.2%, or about 0.1%.

In one aspect, use of the cells of the present invention and/or the pharmaceutical composition of the present invention in the preparation of a medicament is provided, wherein the medicament of the present invention is used for preventing and/or treating a disease and/or symptom. For example, the disease and/or symptom of the present invention comprises a tumor. In some embodiments, the tumor of the present invention is selected from solid tumors. In some embodiments, the tumor of the present invention is selected from one or more of the group consisting of melanoma, ovarian cancer, cervical cancer, lung cancer, bladder cancer, breast cancer, head and neck cancer, pancreatic cancer, liver cancer, gastric cancer, colorectal cancer, and renal cancer.

In one aspect, a method for preventing and/or treating a disease and/or symptom is provided by the present invention, and the method comprises administering the cells of the present invention and/or the pharmaceutical composition of the present invention to a subject. For example, the disease and/or symptom of the present invention comprises a tumor. In some embodiments, the tumor of the present invention is selected from solid tumors. In some embodiments, the tumor of the present invention is selected from one or more of the group consisting of melanoma, ovarian cancer, cervical cancer, lung cancer, bladder cancer, breast cancer, head and neck cancer, pancreatic cancer, liver cancer, gastric cancer, colorectal cancer, and renal cancer.

In one aspect, a TIL of the present invention and/or a pharmaceutical composition of the present invention is provided, which can be used for preventing and/or treating a disease and/or symptom. For example, the disease and/or symptom of the present invention can comprise a tumor. In some embodiments, the tumor of the present invention is selected from solid tumors. In some embodiments, the tumor of the present invention can be selected from one or more of the following group: melanoma, ovarian cancer, cervical cancer, lung cancer, bladder cancer, breast cancer, head and neck cancer, pancreatic cancer, liver cancer, gastric cancer, colorectal cancer, and renal cancer.

Without wishing to be bound by any theory, the embodiments below are merely for illustrating the methods and uses, etc. of the present invention, and are not used to limit the scope of the present invention.

### EXAMPLES

### Example 1

### 1.1 Receipt and processing of tumor tissue

### 1.1.1 Tissue receipt

Tumor tissue and blood samples from a donor were received, sample information was checked and recorded, and corresponding sample labels were printed.

### 1.1.2 Tissue processing and culture

Sample tubes and blood collection tubes were disinfected with 75% alcohol and were transferred into a biosafety cabinet. PBMCs in the blood sample were isolated and cryopreserved according to the above PBMC manual separation and cryopreservation operating procedure. A culture flask or a culture bag having a gas-permeable surface was provided, for example, a culture bag (Origen), and 300 mL of rewarmed complete medium was added. The complete medium could be arbitrarily selected from X-vivo 15 medium or other commercially available T cell media, for example, T cell media of brands such as Stem Cell, Lonza, Thermo, Miltenyi, etc.; essential amino acids and antibiotics could be added; and IL-2 at a concentration of 300-9000 IU/mL (e.g., 1000-9000 IU/mL, e.g., 6000 IU/mL) was added. Several 10 cm culture dishes were provided, an appropriate amount of medium was added, and sterile ophthalmic forceps were used to take the tumor tissue out of the sample tube into a 10 cm culture dish; the tissue was washed and the culture dish was replaced. Ophthalmic scissors and ophthalmic forceps were used for preliminary cutting to remove adipose tissue and necrotic tissue, and each tissue block was further minced to a size of about 27 cubic millimeters. Non-suspended tumor tissue blocks were taken, an internal plunger was removed from a 20 mL syringe, the syringe was connected to the culture bag, and about 1 g of tissue blocks were transferred into the culture bag through the syringe using a pipette. The culture bag was placed into a carbon dioxide incubator for culture. The scissors and forceps were cleaned, preliminarily disinfected with 75% alcohol, ultrasonically cleaned, and then sterilized, thereby obtaining a first TIL population.

### 1.2 Step (A) in vitro expansion and harvest

### 1.2.1 Step (A) in vitro expansion

According to the cell growth status, medium was replenished or half of the medium was replaced every 3-7 days to ensure cell nutrition. Complete medium was used, and the complete medium could be arbitrarily selected from X-vivo 15 medium or other commercially available T cell media, for example, T cell media of brands such as Stem Cell, Lonza, Thermo, Miltenyi, etc.; essential amino acids and antibiotics could be added; and IL-2 at a concentration of 300-9000 IU/mL (e.g., 1000-9000 IU/mL, e.g., 6000 IU/mL) (SL Pharma and/or Sihuan) was added. After 3-14 days of step (A), for example, sampling and counting could be performed on day 13 or day 14, and when the cell number was between 5×10⁵ and 5×10⁸, the harvest step of step (A) was entered.

### 1.2.2 Harvest of step (A)

Cells at the end of the in vitro expansion of step (A) were collected and centrifuged, the medium was discarded, and the cells were washed once with PBS or normal saline, thereby obtaining TILs subjected to the in vitro expansion of step (A) (a second TIL population). Sampling and counting were performed, and about 5×10⁵ to 2×10⁸ cells were retained for a subsequent in vitro expansion step; about 5×10⁵ cells were taken for quality control testing; and the remaining cells were added with cryopreservation solution and cryopreserved as cryopreserved preREP TIL in vitro cells.

### 1.3 Step (B) TIL activation

TILs subjected to the in vitro expansion of step (A) (the second TIL population) were continuously cultured, or the cryopreserved preREP TIL in vitro cells were resuscitated, to perform TIL activation of step (B).

Complete medium was used, and the complete medium could be arbitrarily selected from X-vivo 15 medium or other commercially available T cell media, for example, T cell media of brands such as Stem Cell, Lonza, Thermo, Miltenyi, etc.; essential amino acids and antibiotics could be added. The cell density was adjusted to 5×10⁵ to 2×10⁶ cells/mL, and the cells were placed in a suspension 24-well culture plate at 1 mL/well, and IL-2 at a concentration of 300-9000 IU/mL (e.g., 1000-9000 IU/mL, e.g., 6000 IU/mL) was added. A T cell activator could also be added simultaneously to the medium of each TIL cell population, for example, a CD3 agonist and/or a CD28 agonist was added, for example, about 30 ng/mL of a CD3 antibody (Miltenyi Biotech, OKT3), about 30 ng/mL of a CD28 antibody (Merck, 15E8), beads were added at a bead-to-TIL ratio of about 1:2-2:1 (Dynabeads having a diameter of about 1 to 10 µm, Thermo Fisher) and/or transACT was added at a transACT-to-TIL ratio of about 1:100-1:2000 (having a diameter of about 100 to 500 nm, Miltenyi). Culture was performed for about 0-4 days to obtain a third TIL population.

### 1.4 Step (C) TIL cell gene editing

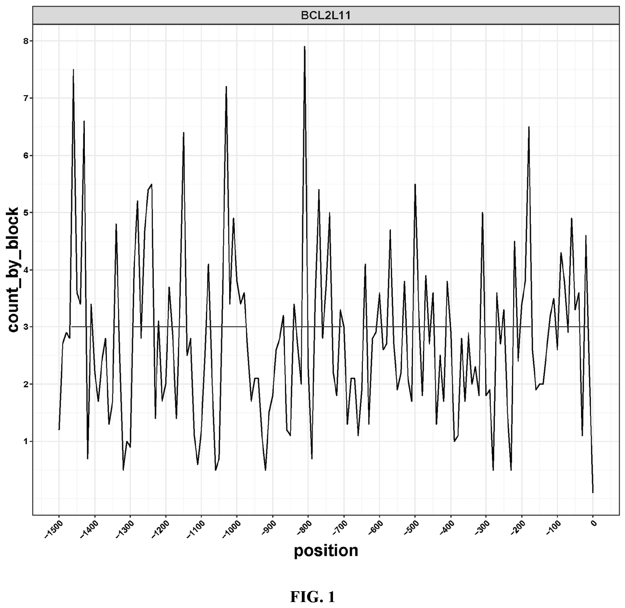

A targeting guide sequence selected from preferred regions shown in FIGs.1 to 2 of the present invention, for example, an sgRNA sequence as shown in SEQ ID NO: 1-1925, 1926-3327, 49607-49627, or 49628-49637, was thawed, and nuclease-free water was added to prepare a concentration of about 100 µM. About 2 µL of gRNA (50 µM) was annealed by incubation at 95°C for 2 minutes and then added into P3 buffer, and 0.3-1 µL of Cas9 (e.g., Kaika, Kairui, Acro, 10 mg/mL) was added, followed by incubation at 25°C for 10 minutes to form a ribonucleoprotein complex (RNP). In P3 buffer (Lonza), the above RNP and about 1×10⁶ cells of the third TIL population were electroporated using a Lonza electroporator. For example, the electroporation program could be human T cell stim (EO115). After gene editing by electroporation, culture was performed for about 0-4 days to obtain a fourth TIL population.

### 1.5 Step (D) Post-gene-editing culture of TIL cells

Feeder cells (irradiated healthy donor PBMC T cells) were added to the fourth TIL cell population for culture. The contact time between TILs and feeder cells was required to be after a time Tₙ following contact between the TILs and IL-2 and a T cell activator (e.g., a CD3 antibody or a nanomatrix comprising a CD3 antibody and a CD28 antibody, such as transACT) in step (B) (Tₙ for each experimental group could be 0 hours to 12 days, e.g., 24 hours or 48 hours). First, feeder cells mixed from 1-5 donors were resuscitated. Activated TIL cells and feeder cells were mixed at a ratio of TIL cells:feeder cells of about 1:200, transferred into a G-Rex100 culture flask or a gas-permeable bag, and supplemented with complete medium. Samples were taken for counting every 1-3 days, and medium was replenished or half of the medium was replaced according to cell status until the total cell number was greater than 1×10⁹, or step (D) in vitro expansion culture was performed for about 5 days to about 14 days, and the culture of step (D) in vitro expansion was terminated.

### 1.6 Harvest of tumor-infiltrating lymphocytes

Cells expanded in step (D) were collected, the supernatant medium was discarded after centrifugation, and the cells were washed three times with PBS or normal saline or a compound electrolyte solution, thereby obtaining TILs expanded in step (D) (a fifth TIL population). During the third wash, sampling and counting were performed, and according to the counting result, after the last centrifugation the supernatant was discarded, and 3×10⁶ cells were taken for quality control testing; all remaining cells were added with cryopreservation solution, and the cell density was adjusted to 1-3×10⁸ cells/mL for cryopreservation.

### Example 2. TCR transduction and gene editing

T cell activation: T cells cryopreserved in liquid nitrogen were resuscitated and cultured, centrifuged, and resuspended to 5E5/ml using T cell medium RPMI 1640 (Gibco) + 10% FBS (Bovogen); T cell TransAct (Miltenyi) was added at 1:100; recombinant human IL-2 at a concentration of 30 IU/ml was added; and culture was performed for about 72 hr.

TCR transduction: One day before transduction, a 24-well suspension culture plate was coated with a recombinant human fibronectin fragment (Retronectin, Takara) at a final concentration of 15 µg/mL, at 250 µL per well of the 24-well plate. The plate was protected from light and stored at 4°C overnight for later use. The coated 24-well plate was taken out, the coating solution was aspirated and discarded, and 500 µL of a blocking solution containing 2% BSA was added for blocking at room temperature for 30 minutes. The blocking solution was aspirated and discarded, and the plate was washed twice with 500 µL/well of a plate-washing solution containing 2.5% HEPES, and the plate-washing solution was aspirated and discarded. The experimental group was transduced with a retrovirus carrying a specific TCR nucleic acid fragment of an NY-ESO-1 antigen peptide. 0.1-1 mL of retrovirus solution was added per well, followed by centrifugation at 32°C and 2000g for 2 hours. The supernatant in the 24-well plate was discarded, and resuscitated and activated T cells were added to each well of the 24-well plate in a volume of 500-1000 µL, with a cell concentration of about 5×10⁵ cells/mL. Centrifugation was performed at 30-32°C and 1000g for 10 minutes. After centrifugation, the culture plate was placed in a 37°C, 5%CO₂ incubator for culture to obtain transduced cells. After transduction, culture was performed for about 0-4 days to obtain a TCR-T cell population. After transduction, according to cell counting density and viability every 1-3 days, T cell medium was added according to the counting result, recombinant human IL-2 at a concentration of 30-100 IU/ml was added, the initial culture cell density was adjusted to 0.5-2×10⁶/ml, and culture was continued.

TCR-T gene editing: The gRNA of the present invention (an sgRNA sequence as shown in SEQ ID NO: 1-1925, 1926-3327, 49607-49627, or 49628-49637) was thawed, and nuclease-free water was added to prepare a concentration of about 100 µM. About 2 µL of gRNA (50 µM) was annealed by incubation at 95°C for 2 minutes and then added into P3 buffer, and 0.3-1 µL of Cas9 (e.g., Kaika, Kairui, Acro, 10 mg/mL) was added, followed by incubation at 25°C for 10 minutes to form a ribonucleoprotein complex (RNP). In P3 buffer (Lonza), the above RNP and about 1×10⁶ TCR-T cells were electroporated using a Lonza electroporator. For example, the electroporation program could be human T cell stim (EO115). Recombinant human IL-2 at a concentration of 100-300 IU/ml was added to the electroporated T cells, and culture was continued to obtain the target gene-edited TCR-T cells of the present invention.

### Example 3. PDO model culture

Tumor tissue was obtained from surgery, transported and stored at 2~8°C after excision, mechanically dissociated within 24 hours, and cut into pieces of about 0.5 mm³ in size, and then digested at 37°C using a dedicated tissue digestion solution (bioGenous), and the reaction was terminated with 10% FBS. The tissue suspension was filtered through a 100 µm filter and washed, placed on ice and thoroughly mixed with Martrigel, and then the mixture was spotted onto the bottom of a cell culture plate. The culture plate was placed in a 37°C incubator, and after Martrigel was fully solidified, complete medium was carefully added for culture. When the organoids grew to a sufficient size and density, passaging or cryopreservation was performed to obtain a PDO (tumor organoid, Patient-Derived Organoid) model.

The Matrigel and organoid mixture was scraped off with the tip of a pipette tip and transferred into a centrifuge tube containing basal medium, and the organoids and Matrigel were separated by pipetting up and down; after centrifugation, a passaging tissue digestion solution was added, the mixture was mixed well, and the mixture was placed in a 37 °C incubator for digestion; basal medium was added to dilute the digestion solution, followed by centrifugation and washing; a small amount of the cell suspension was taken and added into an 8-fold volume of digestion solution for digestion to obtain single cells, the reaction was terminated using FBS and counting was performed, and, according to the counting results, the required volume of organoids was aspirated and centrifuged and then resuspended using T cell medium; the cells to be tested (e.g., TIL cells or TCR-T cells) were counted, and the cells to be tested were resuspended to the required density according to different effector-to-target ratios; 100 µl of PDO target cells and 100 µl of the cells to be tested were added into a flat-bottom 96-well plate, and three replicate wells were set for each group for PDO co-culture detection. Several different effector-to-target ratios were set; meanwhile, a group containing only PDO target cells was set, and a control group (NT) was set for co-culturing unedited cells to be tested only with PDO target cells.

### Example 4. Detection of cell knockout efficiency

Reagents and materials: DNA extraction solution (QuickExtract DNA extraction solution, Lucigen, QE09050), nuclease-free water (RNase/DNase free water, Tiangen), EDTA (Sangon, 0.5M), Recombinant DNase I (RNase-free, TAKARA).

Genomic DNA extraction: With reference to the gene editing method of the examples of the present invention, at about 2-7 days after knockout in T cells, about 1×10⁵ to about 2×10⁵ cells were taken and washed once with PBS, and then the gene-edited cells were resuspended in 44 µL PBS, and 6 µL of a prepared nuclease mixture (containing 1 µL DNase I and 5 µL 10× DNase I Buffer) was added, followed by incubation at 37°C for 5 minutes. Then, 2.5 µL of 0.5 M EDTA was added into the sample, followed by incubation at 80°C for 10 minutes. After centrifugation, the supernatant was discarded, 50 µL DNA extraction solution was added to the cell pellet, and, after brief centrifugation, the following program was run: 75°C - 10 minutes; 95°C - 5 minutes; 4°C - hold. The concentration of the DNA sample could be measured using a spectrophotometer (NanoDrop^{™}).

Sequencing: PCR primers could be designed in a region of about 100 to about 200 nucleotides upstream and downstream of the PAM site. A PCR reaction system was designed according to the following:

| Reagent | Volume |
|---|---|
| 2×PCR Premix Buffer | 25 µL |
| DNA template | about 100-500 ng |
| Forward primer (10 µM) | 1 µL |
| Reverse primer (10 µM) | 1 µL |
| Double-distilled water | added to make up to 50 µL |
| DMSO | 1.5 µ L |

Amplification was performed according to the following PCR program:

| Temperature | Time | |
|---|---|---|
| 95°C | 4 minutes | |
| 95°C | 15 seconds | These 3 steps were repeated for 35 cycles |
| 58°C | 30 seconds | |
| 72°C | 30 seconds | |
| 72°C | 7 minutes | |
| 4°C | Maintained | |

The PCR product was subjected to Sanger sequencing analysis.

### Analysis of Crispr Cas9 knockout efficiency

The Crispr Cas9 knockout efficiency was analyzed by using the Tracking of Indels by DEcomposition (Tide) method based on the Sanger sequencing data, and the specific method could be referred to (Brinkman et al., Nucl. Acids Res. (2014) or shinyapps.datacurators.nl/tide/). By inputting the corresponding sgRNA sequence of the present invention, the pre-knockout control sequence, and the test sequence after Crispr Cas9 knockout, with the P-value threshold set to 0.001, the knockout efficiency analysis was performed. Among them, donors 005 and 031 were healthy donors and peripheral blood were derived from healthy donors; donors 906 and 309 were cervical cancer patients; donors 107 and 707 were lung cancer patients; and donor 812 was a melanoma patient.

| Target | gRNA | Donor knockout efficiency (%) |
|---|---|---|
| BCL2L11 | BCL2L11-3 | 90.3, 92.1, 91.1, 92.4 |
| BCL2L11 | BCL2L11-4 | 71.3, 59.8, 78.0, 80.3 |
| PTPN2 | PTPN2-3 | 83.0, 87.1, 75.9 |
| PTPN2 | PTPN2-4 | 83.8, 86.6, 73.1 |
| TNFAIP3+BCL2L11 | TP2 | 90.8, 91.8 |
| | BCL2L11-3 | 92.3, 91.7 |
| TNFAIP3+BCL2L11 | TP2 | 90.8, 84.5 |
| | BCL2L11-4 | 70.0, 75.5 |
| TNFAIP3+ PTPN2 | TP2 | 94.1, 93.2 |
| | PTPN2-3 | 94.3, 94.0 |
| TNFAIP3+ PTPN2 | TP2 | 95.3, 93.2 |
| | PTPN2-4 | 15.2, 20.9 |
| IKZF1+PTPN2 | IKZF1-2 | 72.1, 62.5 |
| | PTPN2-3 | 83 |

The knockout efficiencies of the guide of the same sequence in different donor cells were separated by commas.

| gRNA | Sequence | SEQ ID NO: |
|---|---|---|
| BCL2L11-3 | GTTCTGATGCAGCTTCCATG | 49577 |
| BCL2L11-4 | TATGGATCGCCCAAGAGTTG | 49578 |
| PTPN2-3 | TGTCATGCTGAACCGCATTG | 49579 |
| PTPN2-4 | TTGACATAGAAGAGGCACAA | 49580 |
| TP2 | CTTGTGGCGCTGAAAACGAA | 49581 |
| IKZF1-2 | GATGGCTTGGTCCATCACGT | 49582 |

The results showed that the editing modes of various genes of the present invention or combinations thereof could achieve a certain proportion of knockout efficiency.

### Example 5. Detection of Cell Expansion

### Experimental Preparation

The proliferation of TIL cells was detected on day 7-10 after gene editing (with IL-2 withdrawn).

TIL cells of each group were harvested, washed once with PBS, and then resuspended in T cell culture medium (without IL-2). After counting, the cell density was adjusted to 1e5 to 2e6/mL, and 100 µL/well was added to a flat-bottom 96-well plate. In the CD3 antibody stimulation group, a CD3 antibody (OKT3) was added at 30 ng/ml for stimulation. In the TransACT stimulation group, transACT (with a diameter of about 100 to 500 nm, Miltenyi) was added such that the working solution concentration of transACT was 1:1000 (v/v). In the unstimulated medium group, only an equal volume of cell culture medium was added. A CTG kit (CellTiter-Glo Luminescent Cell Viability Assay, Promega) was used to analyze the fluorescence intensity at the time of T cell plating, and after 3 days, the CTG kit was used to analyze the fluorescence intensity of T cells. The expansion efficiency of T cells was characterized by the fluorescence intensity on day 3/the fluorescence intensity at the time of plating.

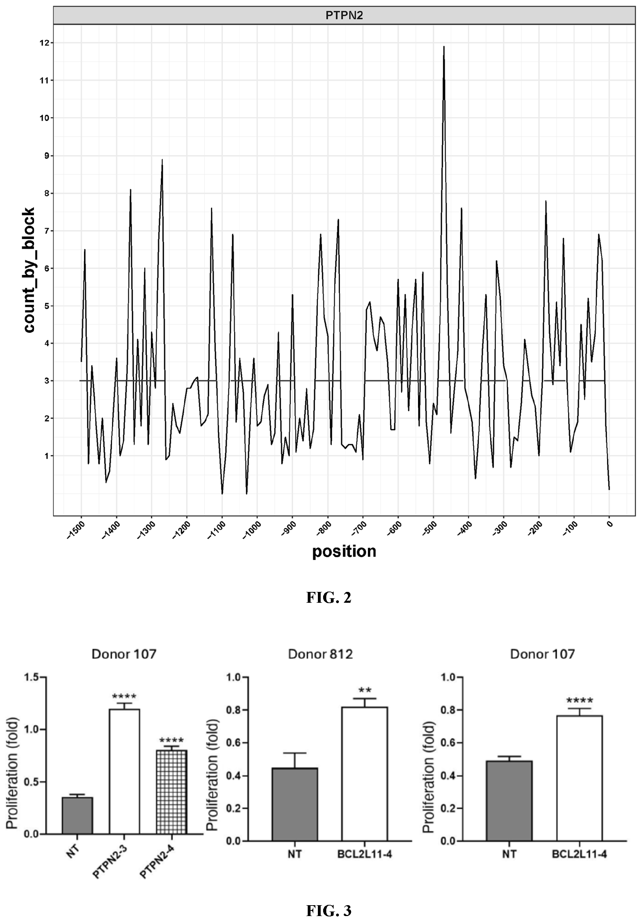

FIG. 3 showed the fold expansion of TILs with single-target gene editing of PTPN2 or BCL2L11 in the unstimulated medium group.

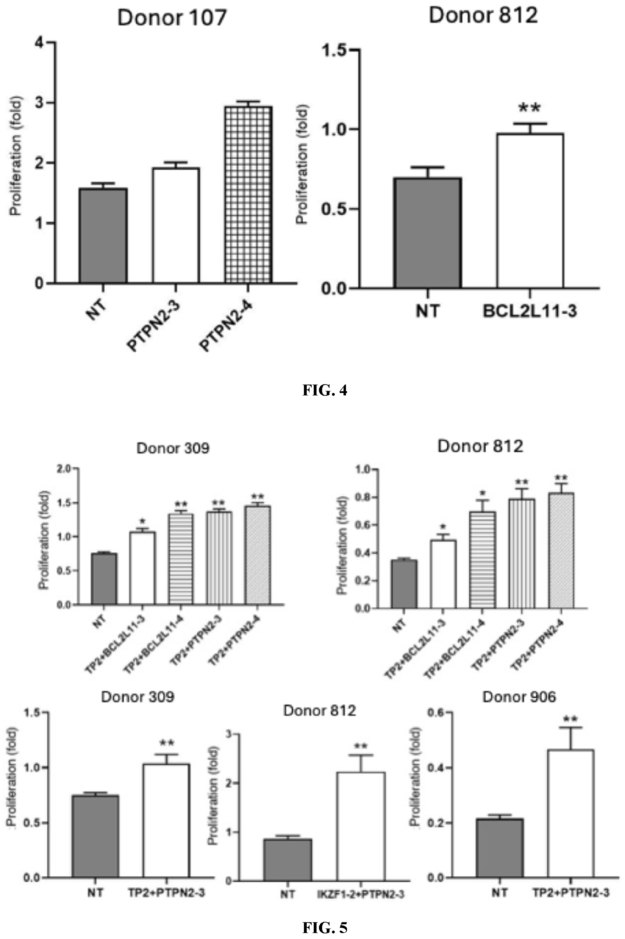

FIG. 4 showed the fold expansion of TILs with single-target gene editing of PTPN2 or BCL2L11 in the TransACT stimulation group.

FIG. 5 showed the fold expansion of TILs with gene editing of the combination of TNFAIP3 and PTPN2, the combination of TNFAIP3 and BCL2L11, or the combination of IKZF1 and PTPN2 in the unstimulated medium group.

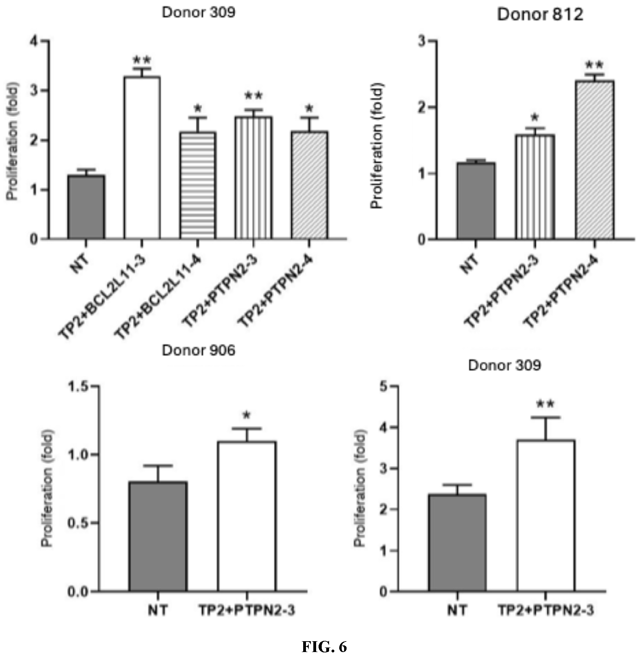

FIG. 6 showed the fold expansion of TILs with gene editing of the combination of TNFAIP3 and PTPN2 or the combination of TNFAIP3 and BCL2L11 in the TransACT stimulation group.

The significant differences were relative to the unedited NT group, wherein * indicated P<0.05, ** indicated P<0.01, *** indicated P<0.001, and **** indicated P<0.0001.

The results indicated that, relative to the control group without gene editing (NT), the cells subjected to target gene editing of the present invention could have significantly improved expansion capability.

### Example 6. Detection of Cell Killing Capability

Starting from day 6 after gene editing, tumor target cells were plated in a flat-bottom 96-well plate. On the next day, TIL cells of each group were co-cultured with the target cells at different effector-to-target ratios (T cells:target cells, E:T). The target cells and T cells were each 100 µL. Three replicate wells were set for each group, and a control group containing only target cells was also set. The target cells could be selected from Hey-T30 ovarian cancer cells and A375 melanoma cells.

According to the instructions for the apoptosis detection reagent (Incucyte Caspase-3/7 Green Dye for Apoptosis, Sartorius), the apoptosis detection reagent was added at 0.2 µL/well, and medium was added at 25 µL/well to dilute Caspase 3/7 Green Dye. An Incucyte recorder (Sartorius) was used to record Caspase 3/7 activity to analyze the killing capability of TIL cells against target cells, once every 3 hours, with a total recording duration of about 5 days.

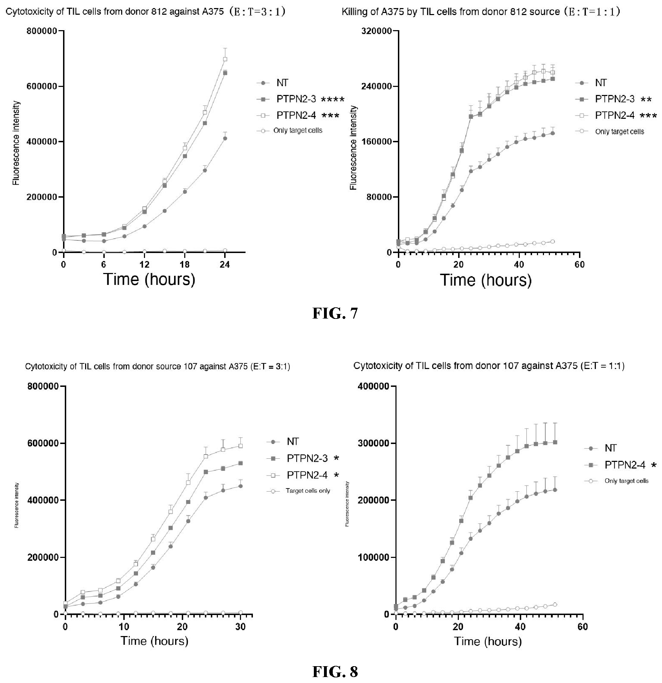

FIG. 7 showed the target cell killing capability of PTPN2-edited TIL cells derived from donor 812.

FIG. 8 showed the target cell killing capability of PTPN2-edited TIL cells derived from donor 107.

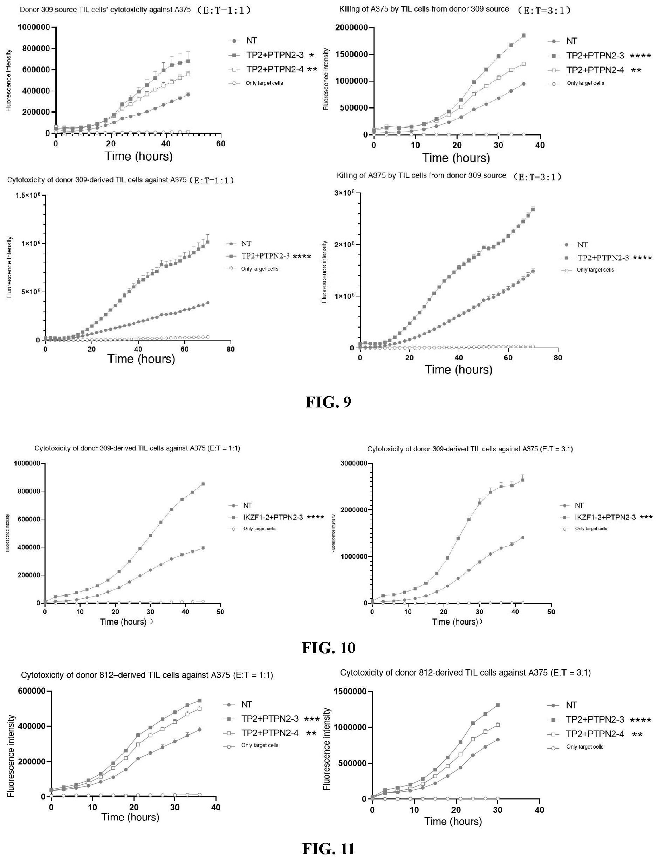

FIG. 9 showed the target cell killing capability of TIL cells derived from donor 309 and edited with the combination of TNFAIP3 and PTPN2.

FIG. 10 showed the target cell killing capability of TIL cells derived from donor 309 and edited with the combination of IKZF1 and PTPN2.

FIG. 11 showed the target cell killing capability of TIL cells derived from donor 812 and edited with the combination of TNFAIP3 and PTPN2.

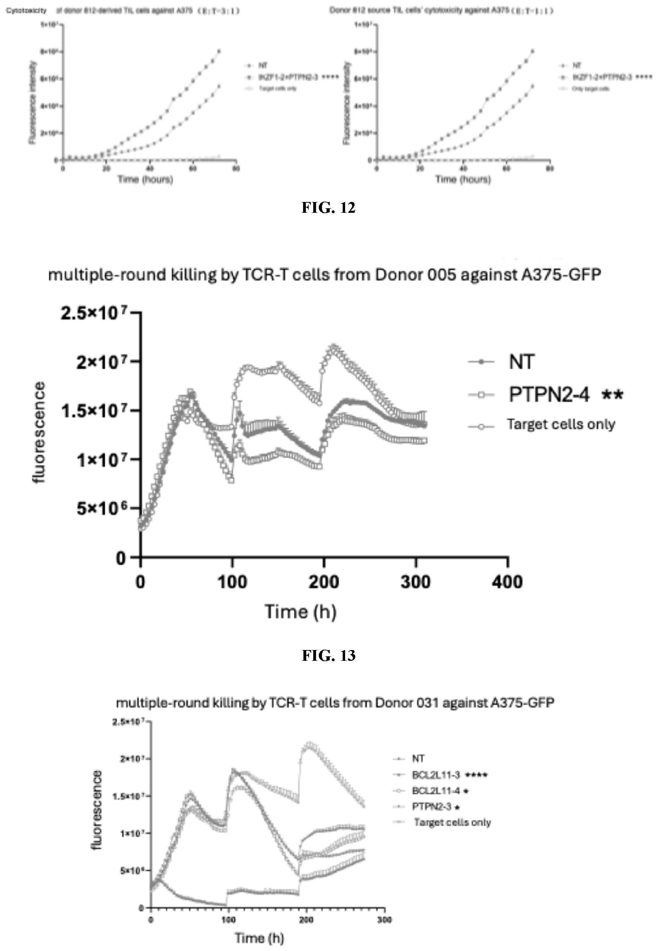

FIG. 12 showed the target cell killing capability of TIL cells derived from donor 812 and edited with the combination of IKZF1 and PTPN2.

The significant differences were relative to the unedited NT group, wherein * indicated P<0.05, ** indicated P<0.01, *** indicated P<0.001, and **** indicated P<0.0001.

The results indicated that, relative to the control group (NT), the cells subjected to target gene editing of the present invention could have significantly improved target cell killing capability.

### Multi-round Killing Assay

IncuCyte was used to monitor changes in fluorescence intensity of target cells A375-GFP during multi-round killing. A375-GFP cells were uniformly plated in a multi-well plate, and after incubation at 37°C for 4 hours, unedited cells or cells with knockout of the targets of the present invention were taken and added to the corresponding wells, and IncuCyte recording of the GFP fluorescence signal was started, with 3 replicate wells for each group. After 24 hours of killing, the co-culture supernatant (20 µL/well) was collected for CBA detection. After 96 hours of killing, a new multi-well plate uniformly plated with A375-GFP was incubated at 37°C for 4 hours, and the mixture of unedited cells or cells with knockout of the targets of the present invention and A375-GFP cells from the previous round of killing was transferred to a new 96-well plate, and IncuCyte was used to record the change curve of the GFP fluorescence signal in a new round of killing.

FIG. 13 showed the multi-round killing results of PTPN2-edited cells in TCR-T cells.

FIG. 14 showed the multi-round killing results of PTPN2- or BCL2L11-edited cells in TCR-T cells.

The significant differences were relative to the unedited NT group, wherein * indicated P<0.05, ** indicated P<0.01, *** indicated P<0.001, and **** indicated P<0.0001.

The results indicated that, relative to the control group (NT), the gene-edited cells of the present invention could have significantly improved multi-round killing capability.

### Detection of Killing Capability in a PDO Model

A PDO model was prepared with reference to the examples of the present invention and was co-cultured with TIL cells. After a Caspase3/7 substrate was added for apoptosis signal labeling, the experimental plate was placed in Incucyte for observation and recording.

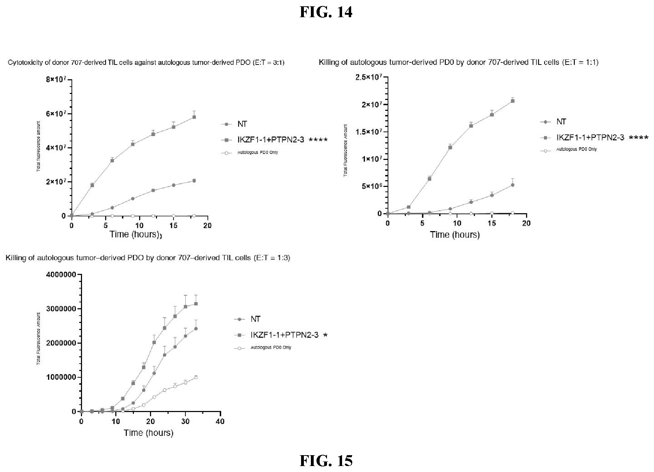

FIG. 15 showed the PDO model killing results of TIL cells edited with the combination of IKZF1 and PTPN2.

The results indicated that, relative to the control group (NT), the gene-edited cells of the present invention could have significantly improved cell killing capability in the PDO model.

### Example 7. Flow Cytometry Detection of Cells

A flow cytometer was used to detect the expression of molecules related to T cell exhaustion, stemness, and the like in TIL cells obtained on day 8 after gene editing.

Sources of main reagents and materials for the TIL flow cytometry test:

V-bottom 96-well plates, manufacturer Corning, catalog number 3894; flow cytometry tubes, manufacturer Corning, catalog number 352052; flow cytometry antibodies were purchased from BD or Biolegend.

Cell surface molecule detection: 1×10⁵ to 5×10⁵ cell samples from each group were added into flow cytometry tubes or V-bottom 96-well plates. The samples were centrifuged at 600 g for 3 minutes, and the supernatant was discarded. The samples were washed once with PBS, with 1 mL/tube for

flow cytometry tubes and 200 µL/well for 96-well plates, and the supernatant was discarded. A prepared antibody working solution was added for cell surface staining, wherein the antibody (BD or Biolegend) concentration was 1:100 to 1:200, and a viability detection dye was included at 1:10000. Staining was performed with 100 µL/tube for flow cytometry tubes and 50 µL/well for 96-well plates, followed by incubation at 2-8°C for 30 minutes in the dark. After completion of surface staining, the cells were washed once with PBS (200 µL/time for 96-well plates and 1 mL/time for flow cytometry tubes), centrifuged at 600 g for 3 minutes at room temperature, and the supernatant was discarded after centrifugation. The cells were resuspended in 100-500 µL PBS and were subjected to flow cytometry analysis.

Intracellular molecule detection: an antibody mixed working solution was prepared for cell surface staining of CD3/CD4/CD8, wherein the antibody concentration was 1:100, and the cell viability detection dye concentration was 1:10000. Staining was performed with 50 µL/well for 96-well plates and 100 µL/tube for flow cytometry tubes, followed by incubation at 2-8°C for 30 minutes in the dark. The cells were washed once with PBS (200 µL/time for 96-well plates and 1 mL/time for flow cytometry tubes), centrifuged at 600 g for 3 minutes at room temperature, and the supernatant was discarded after centrifugation. 100 µL of fixation/permeabilization solution (BD, Fixation/Permeabilization) was added to each well, followed by incubation at 2-8°C for 20-40 minutes in the dark. After fixation and permeabilization, washing was performed twice with 1×Perm/Wash Buffer (200 µL/time for 96-well plates and 1 mL/time for flow cytometry tubes), centrifugation was performed at 600 g for 3 minutes, and the supernatant was discarded after centrifugation. Intracellular molecule antibodies (e.g., TCF1) were prepared using 1×Perm/Wash Buffer, and TIL cells were resuspended (staining with 50 µL/well for 96-well plates and 100 µL/tube for flow cytometry tubes), followed by incubation at 2-8°C for 30 minutes in the dark. After intracellular molecule staining, washing was performed 1 to 2 times with 1×Perm/Wash Buffer (200 µL/time for 96-well plates and 1 mL/time for flow cytometry tubes), centrifugation was performed at 600 g for 3 minutes, and the supernatant was discarded after centrifugation. The cells were resuspended in 100-500 µL PBS and were subjected to flow cytometry analysis.

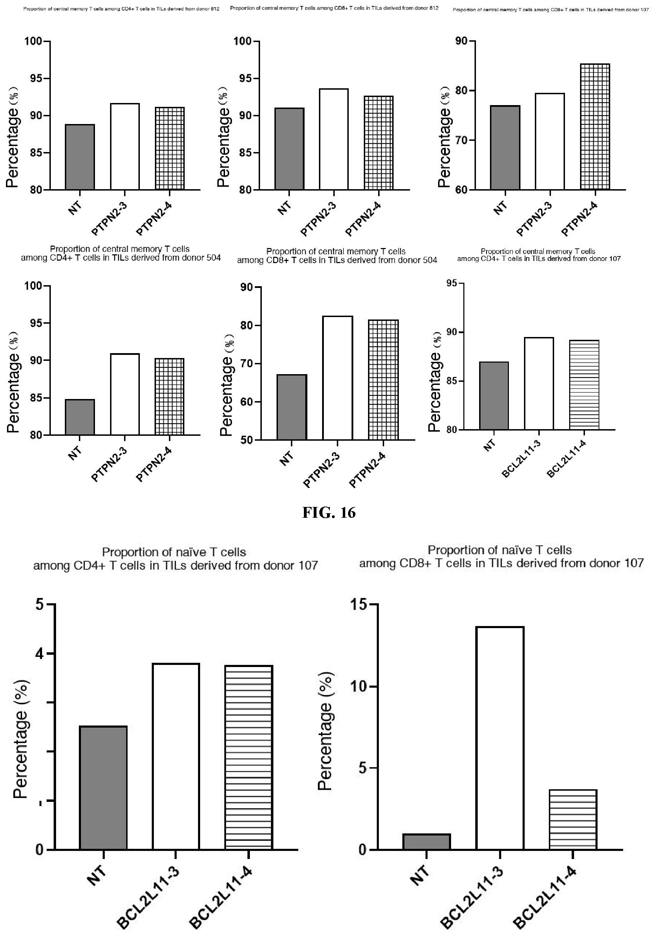

FIG. 16 showed the proportion of central memory T cells in TIL cells after PTPN2 or BCL2L11 editing. For example, central memory T cells could be CD45RO-positive CD62L-positive cells.

FIG. 17 showed the proportion of naive T cells in TIL cells after BCL2L11 editing. For example, naive T cells could be CD45RO-negative CD62L-positive cells.

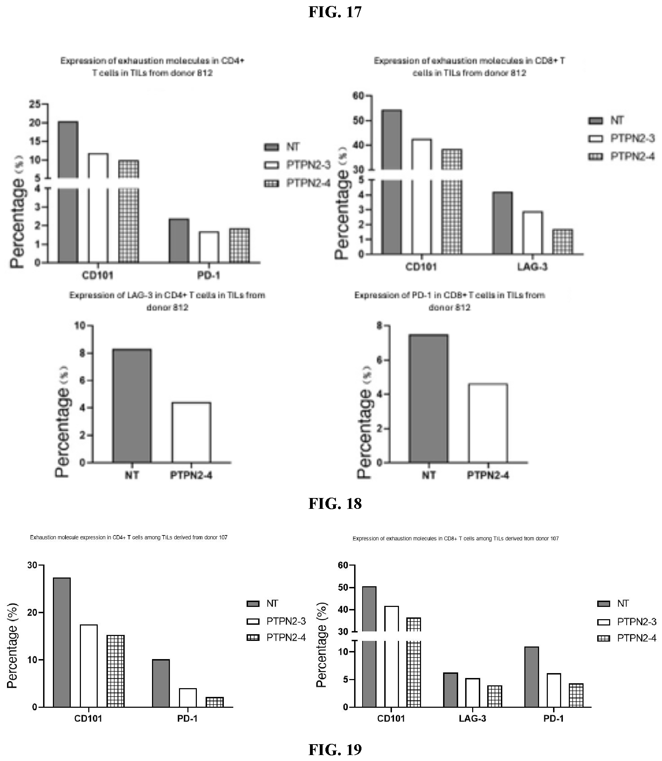

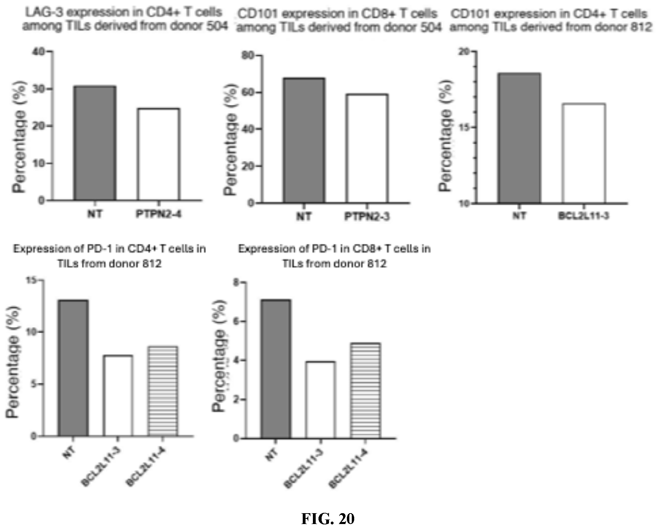

FIGs. 18, 19, and 20 showed the proportion of exhausted cells in TIL cells after PTPN2 or BCL2L11 editing. For example, exhausted T cells could be PD-1-positive, LAG-3-positive, TIM-3-positive, CD38-positive, and/or CD101-positive cells.

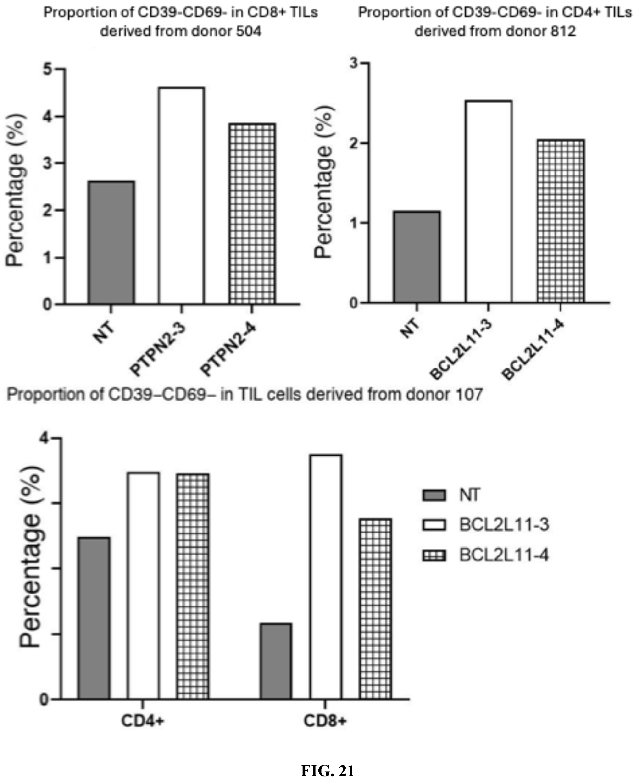

FIG. 21 showed the proportion of stem cell-like T cells in TIL cells after PTPN2 or BCL2L11 editing. For example, stem cell-like T cells could have a CD39-negative CD69-negative phenotype.

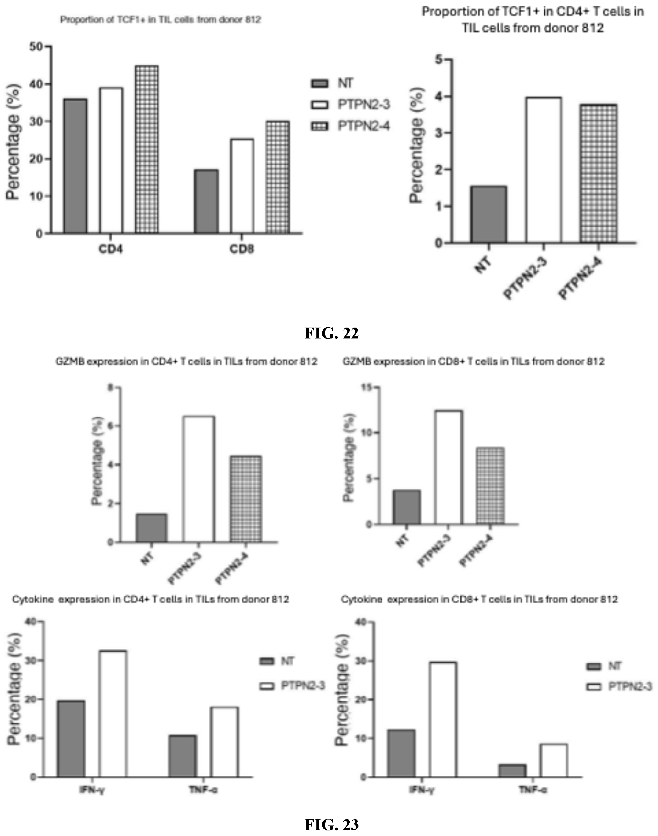

FIG. 22 showed the proportion of stem cell-like T cells in TIL cells after PTPN2 editing. For example, stem cell-like T cells could have a TCF1-positive phenotype.

The results indicated that, relative to the control group without gene editing (NT), the cells subjected to target gene editing of the present invention could have more favorable cellular phenotypic characteristics.

### Example 8. Flow Cytometry Detection of Cytokine Expression

For TIL cell populations obtained on day 7 or day 8 after gene editing in each test group, a flow cytometer was used to detect cytokine expression.

### Experimental Preparation

A medium required for intracellular cytokine expression detection was prepared: TIL cell culture medium was taken, and the following were added according to volume ratio: Golgistop 0.7:1000, Golgiplug 1:1000, and CD107a antibody 1:500, i.e., 2 µL/mL. No interleukin was added.

### Detection Steps

After TIL cells from each experimental group were centrifuged, the TIL cells of each group were resuspended using the medium required for the above intracellular factor expression detection. After counting, the cell density was adjusted to 1×10⁶ cells/mL, and the cells were added into a 96-well plate at 200 µL/well. In the CD3 antibody stimulation group, a CD3 antibody (OKT3) was added at 30 ng/ml for stimulation; in the TransACT stimulation group, transACT (with a diameter of about 100 to 500 nm, Miltenyi) was added such that the concentration of the transACT working solution was 1:1000 (v/v); and in the unstimulated medium group, only an equal volume of cell culture medium was added. The cells were placed in a 37°C incubator and incubated overnight.

The sources of the main reagents and materials for the TIL cell cytokine flow cytometry detection experiment were as follows:

V-bottom 96-well plates, manufacturer Corning, catalog No. 3894; flow cytometry tubes, manufacturer Corning, catalog No. 352052; and flow cytometry antibodies were purchased from BD or Biolegend.

After incubation was completed, the cells were washed once with PBS at 200 µL/well, centrifuged at 600 g for 3 minutes, and the supernatant was discarded. An antibody mixed working solution was prepared for cell surface staining of CD3/CD4/CD8, wherein the antibody concentration was 1:100 and the cell viability detection dye concentration was 1:10000. Staining was performed in a 96-well plate at 50 µL/well and in flow cytometry tubes at 100 µL/tube, followed by incubation at 2-8°C in the dark for 30 minutes. The cells were washed once with PBS (200 µL/time for the 96-well plate and 1 mL/time for the flow cytometry tubes), centrifuged at 600 g for 3 minutes at room temperature, and the supernatant was discarded after centrifugation. A fixation/permeabilization solution (BD, Fixation/Permeabilization) was added at 100 µL per well, followed by incubation at 2-8°C in the dark for 20-40 minutes. After fixation and permeabilization, the cells were washed twice with 1×Perm/Wash Buffer (200 µL/time for the 96-well plate and 1 mL/time for the flow cytometry tubes), centrifuged at 600 g for 3 minutes, and the supernatant was discarded after centrifugation. Cytokine detection antibodies (e.g., GZMB, TNF-α, IFN-γ) were prepared using 1×Perm/Wash Buffer, and the TIL cells were resuspended (50 µL/well for the 96-well plate and 100 µL/tube for the flow cytometry tubes) and incubated at 2-8°C in the dark for 30 minutes. After cytokine staining, the cells were washed 1-2 times with 1×Perm/Wash Buffer (200 µL/time for the 96-well plate and 1 mL/time for the flow cytometry tubes), centrifuged at 600 g for 3 minutes, and the supernatant was discarded after centrifugation. The cells were resuspended in 100-500 µL PBS and subjected to flow cytometric analysis.

FIG. 23 showed that PTPN2-edited TIL cells in the unstimulated medium group had a higher proportion of cytokine expression.

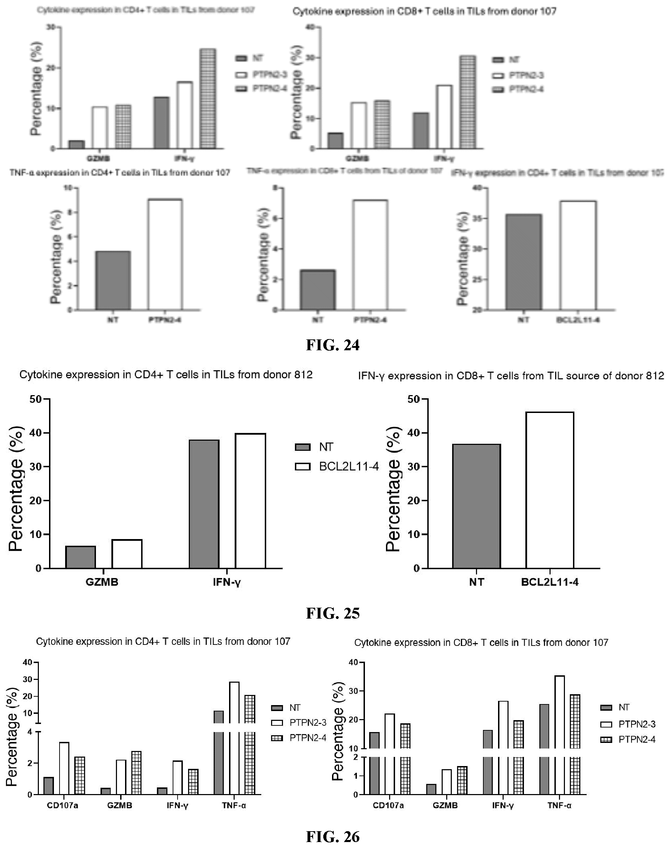

FIG. 24 showed that PTPN2- or BCL2L11-edited TIL cells in the unstimulated medium group had a higher proportion of cytokine expression.

FIG. 25 showed that BCL2L11-edited TIL cells in the unstimulated medium group had a higher proportion of cytokine expression.

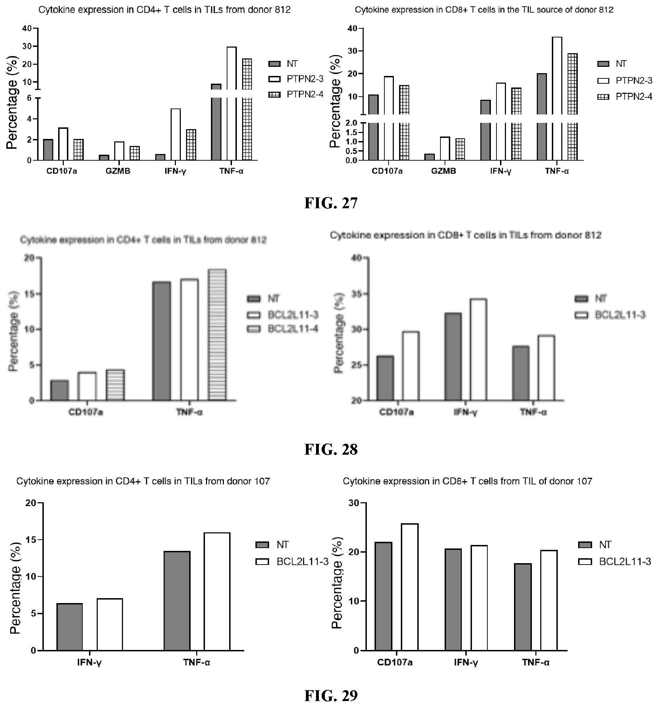

FIGs. 26 and 27 showed that PTPN2-edited TIL cells in the TransACT stimulation group had a higher proportion of cytokine expression.

FIGs. 28 and 29 showed that BCL2L11-edited TIL cells in the TransACT stimulation group had a higher proportion of cytokine expression.

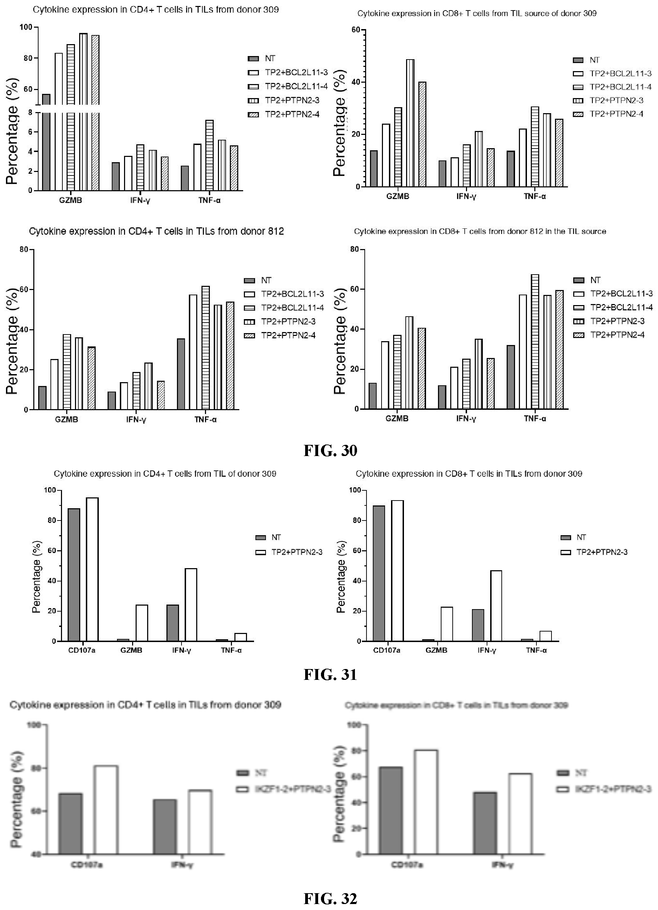

FIG. 30 showed that TIL cells edited with a TNFAIP3 and PTPN2 combination or a TNFAIP3 and BCL2L11 combination in the unstimulated medium group had a higher proportion of cytokine expression.

FIG. 31 showed that TIL cells edited with a TNFAIP3 and PTPN2 combination in the unstimulated medium group had a higher proportion of cytokine expression.

FIG. 32 showed that TIL cells edited with an IKZF1 and PTPN2 combination in the unstimulated medium group had a higher proportion of cytokine expression.

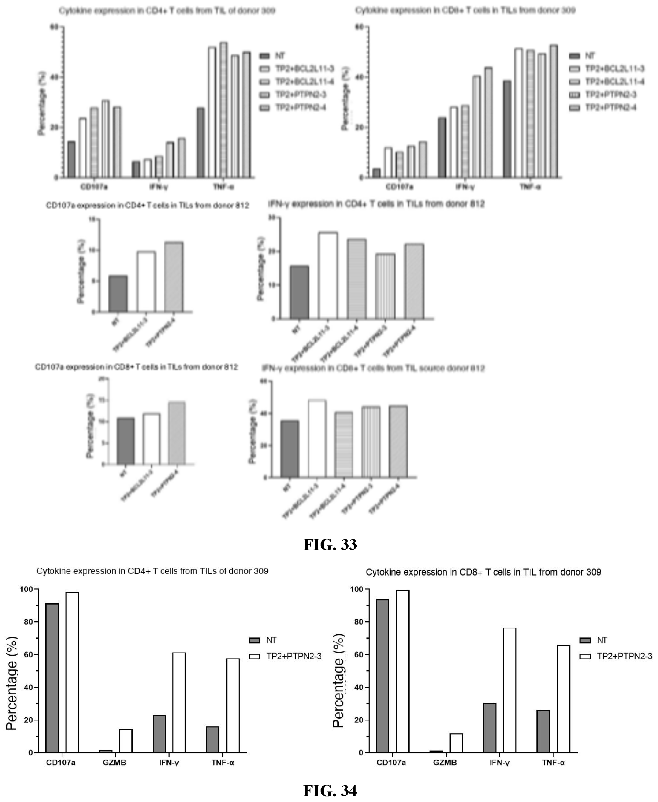

FIG. 33 showed that TIL cells edited with a TNFAIP3 and PTPN2 combination or a TNFAIP3 and BCL2L11 combination in the TransACT stimulation group had a higher proportion of cytokine expression.

FIG. 34 showed that TIL cells edited with a TNFAIP3 and PTPN2 combination in the TransACT stimulation group had a higher proportion of cytokine expression.

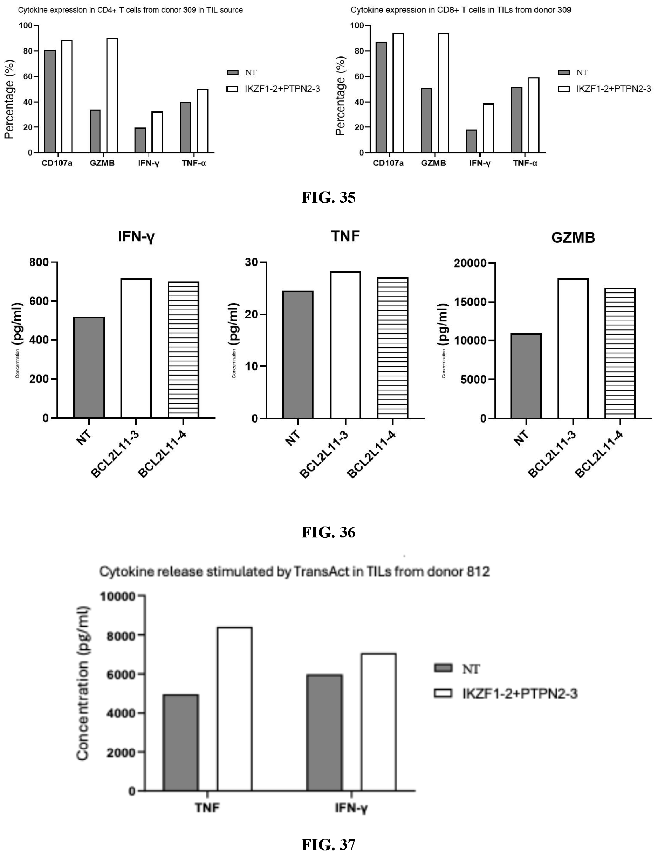

FIG. 35 showed that TIL cells edited with an IKZF1 and PTPN2 combination in the TransACT stimulation group had a higher proportion of cytokine expression.

For example, the cytokine expression capability comprised a higher CD107a expression capability, a higher IFN-γ expression capability, a higher TNF-α expression capability, or a higher GZMB expression capability.

The results indicated that the target gene-edited cells of the present invention had a higher functional cytokine expression capability.

### Example 9. Detection of cytokine release by CBA method

For co-culture of gene-edited cells with autologous tumor cells, co-culture supernatants were collected, and a CBA kit (BD) was used to detect cytokine release of unedited cells or cells in which the targets of the present invention were knocked out.

FIG. 36 showed that BCL2L11-edited cells in TCR-T cells had a higher level of cytokine release.

FIG. 37 showed that cells edited with an IKZF1 and PTPN2 combination in TIL cells had a higher level of cytokine release in the TransACT stimulation group. In the TransACT stimulation group, transACT (with a diameter of about 100 to 500 nm, Miltenyi) was added such that the concentration of the transACT working solution was 1:1000 (v/v).

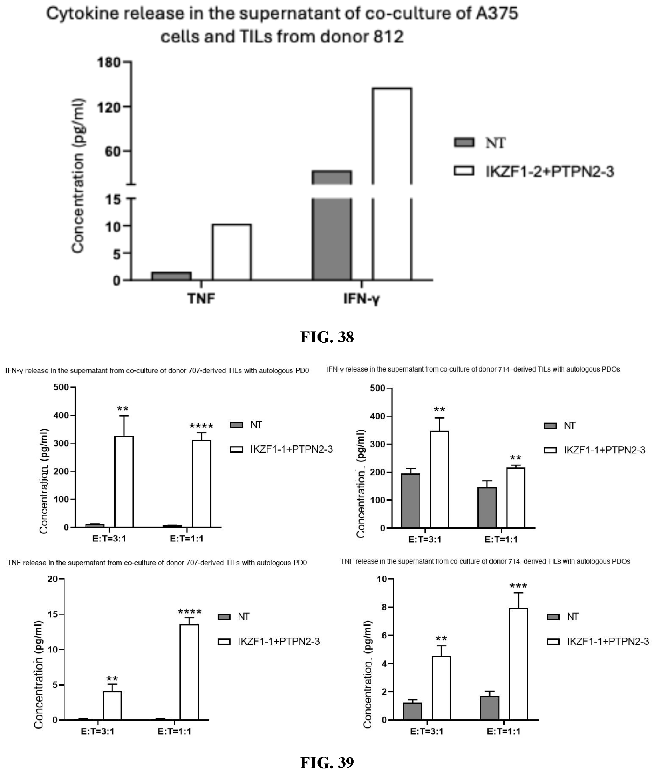

FIG. 38 showed that cells edited with an IKZF1 and PTPN2 combination in TIL cells had a higher level of cytokine release after co-culture with A375 tumor cells.

FIG. 39 showed that cells edited with an IKZF1 and PTPN2 combination in TIL cells had a higher level of cytokine release after co-culture with autologous PDO. The PDO model was prepared with reference to the method provided in the present invention.

For example, the cytokine expression capability comprised a higher IFN-γ release level, a higher TNF-α release level, or a higher GZMB release level.

The results indicated that the target gene-edited cells of the present invention had a higher level of functional cytokine release.

### Example 10. Effect detection of gene-edited cells

According to the experimental methods in the Examples, the knockout effects of sgRNAs targeting BCL2L11 and PTPN2 were detected.

| sgRNA No. | Sequence | SEQ ID NO: |
|---|---|---|
| BC-N1 | GCTCCTACGCCCAATCACTG | 49607 |
| BC-N2 | ACACTGCGATCGCATCATCG | 49608 |
| BC-N3 | GTAAATACCTCCAAATCCCG | 49609 |
| BC-N4 | TCTCATAAAGATGAAAAGCG | 49610 |
| BC-N5 | GGTAAGAGGCAGTTGACGTG | 49611 |
| BC-N6 | GAGGGTGTGAGCAGAAAAGC | 49612 |
| BC-N7 | AATGCATTCTCCACACCAGG | 49613 |
| BC-N8 | ACCAACAAGACCCAGCACCG | 49614 |
| BC-N9 | AATCCTGCTCCAAAAAACCA | 49615 |
| BC-N10 | AAACCTTATTGAAGGTGAGG | 49616 |
| BC-N11 | AACCTACGGCCTATTCTCAG | 49617 |
| BC-N12 | ATAATTTAAAAAGGTCAGAG | 49618 |
| BC-N13 | GGACTCTGCTGTAATGGACA | 49619 |
| BC-N14 | AATGGGCTCGAGACACATCA | 49620 |
| BC-N15 | GAGGCATACTTTCTGCACAG | 49621 |
| BC-N16 | TAGTTGATCAAGAATTTTGG | 49622 |
| BC-N17 | TAACTCCTGAGAGTTGTCGG | 49623 |
| BC-N18 | CACACACTTCATCATAAGGG | 49624 |
| BC-N19 | GCTACTCACAGTTGTCCACA | 49625 |
| BC-N20 | GCTTTAAGGAGACTTCATGG | 49626 |
| BC-N21 | TGCACTGATGAATTTTGACA | 49627 |
| PT-N1 | AAAGTAAACTTTACATCCAG | 49628 |
| PT-N2 | TGAAAATTTCAATGTAGTTG | 49629 |
| PT-N3 | GGGATAGTCAGGTAAACAGT | 49630 |
| PT-N4 | AACCTATTCTCACTAAGATG | 49631 |
| PT-N5 | ACTTGAGTACTCTAACACAG | 49632 |
| PT-N6 | ACTCTAAAAAGTGAAAATCA | 49633 |
| PT-N7 | GGATGTTACAAGACAGACAG | 49634 |
| PT-N8 | AAACCCACAAGTACTTACAT | 49635 |
| PT-N9 | CTCAGGCCCCGCACGATCCG | 49636 |
| PT-N10 | GCCAAGCCCTGTCGGCCGTG | 49637 |

### (A) Knockout efficiency

| sgRNA No. | Knockout Efficiency (%) | sgRNA No. | Knockout Efficiency (%) | sgRNA No. | Knockout Efficiency (%) |
|---|---|---|---|---|---|
| BC-N1 | C | BC-N12 | B | PT-N2 | B |
| BC-N2 | A | BC-N13 | A | PT-N3 | A |
| BC-N3 | B | BC-N14 | A | PT-N4 | A |
| BC-N4 | A | BC-N15 | A | PT-N5 | A |
| BC-N5 | A | BC-N16 | B | PT-N6 | A |
| BC-N6 | A | BC-N17 | A | PT-N7 | A |
| BC-N7 | A | BC-N18 | B | PT-N8 | A |
| BC-N8 | A | BC-N19 | A | PT-N9 | A |
| BC-N9 | A | BC-N20 | A | PT-N10 | A |
| BC-N10 | A | BC-N21 | B | | |
| BC-N11 | A | PT-N1 | A | | |

Wherein, A indicated a knockout efficiency greater than or equal to 70%, B indicated a knockout efficiency greater than or equal to 50% and less than 70%, C indicated a knockout efficiency greater than or equal to 10% and less than 50%, D indicated a knockout efficiency of less than 10%, and N/A indicated not detected.

Wherein, donor 045 was a healthy donor, and donor 026 was a healthy donor; PBMC cells were isolated and then transduced with a TCR to serve as TCR-T cells.

The results showed that the editing modes of various genes or combinations thereof of the present invention each achieved a certain proportion of knockout efficiency.

### (B) Proliferation capability

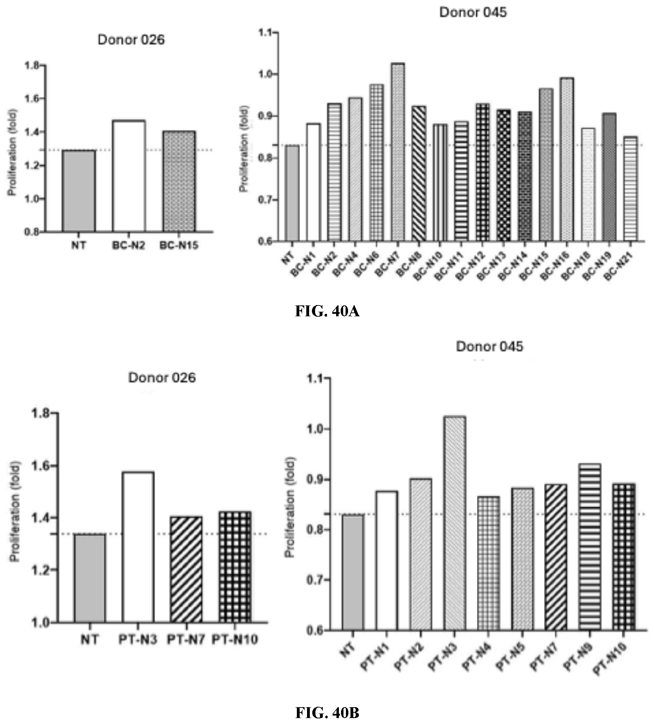

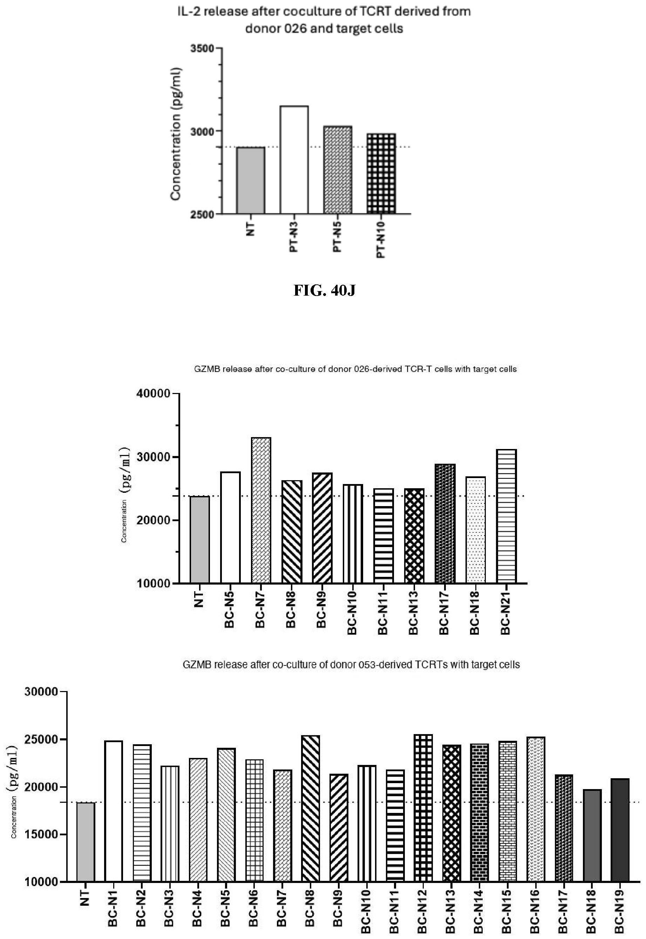

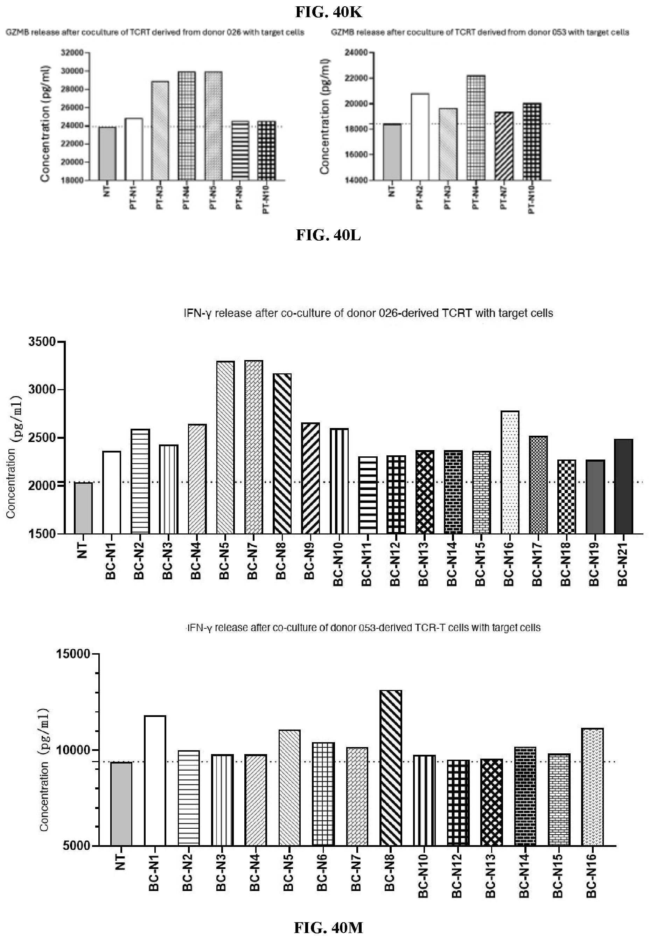

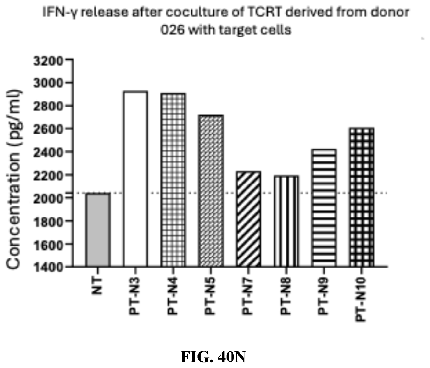

FIG. 40A showed that, in the unstimulated group, BCL2L11 gene-edited TCR-T cells had significant expansion capability.

FIG. 40B showed that, in the unstimulated group, PTPN2 gene-edited TCR-T cells had significant expansion capability.

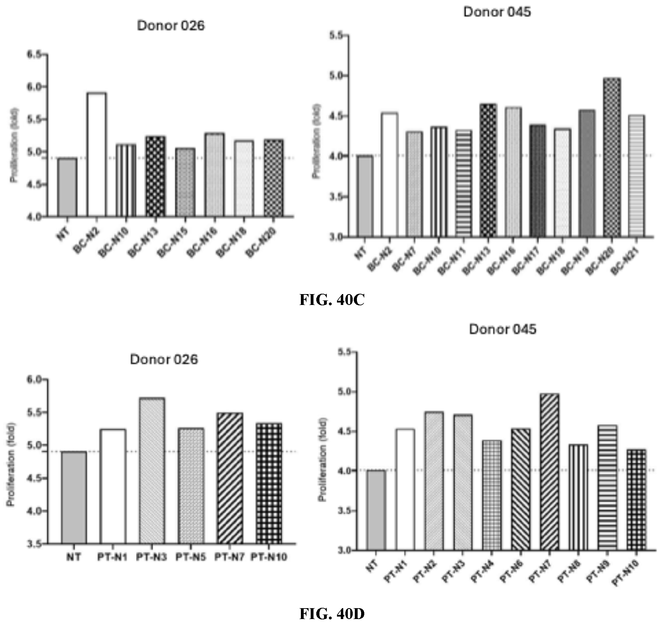

FIG. 40C showed that, in the TransACT-stimulated group, BCL2L11 gene-edited TCR-T cells had significant expansion capability.

FIG. 40D showed that, in the TransACT-stimulated group, PTPN2 gene-edited TCR-T cells had significant expansion capability.

The results indicated that, as compared with the non-gene-edited control group (NT), the target gene-edited T cells of the present invention had significantly improved expansion capability.

### (C) Cytotoxic capability

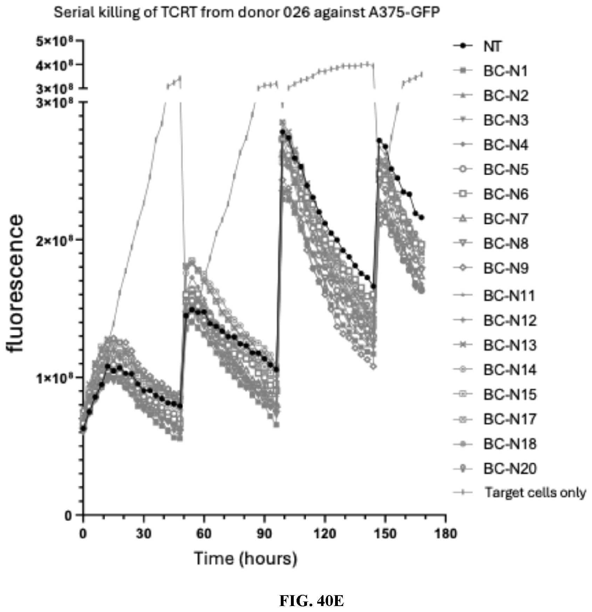

FIG. 40E showed the target cell killing capability of BCL2L11 gene-edited TCR-T cells.

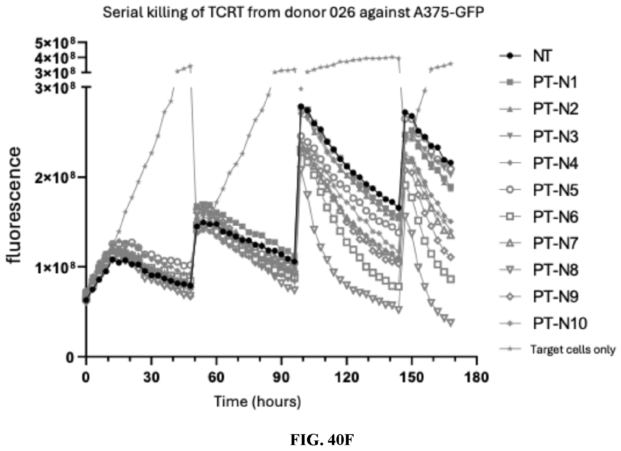

FIG. 40F showed the target cell killing capability of PTPN2 gene-edited TCR-T cells.

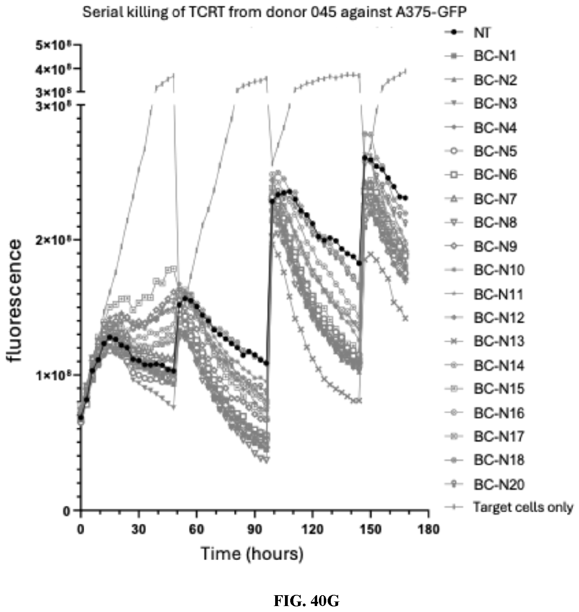

FIG. 40G showed the target cell killing capability of BCL2L11 gene-edited TCR-T cells.

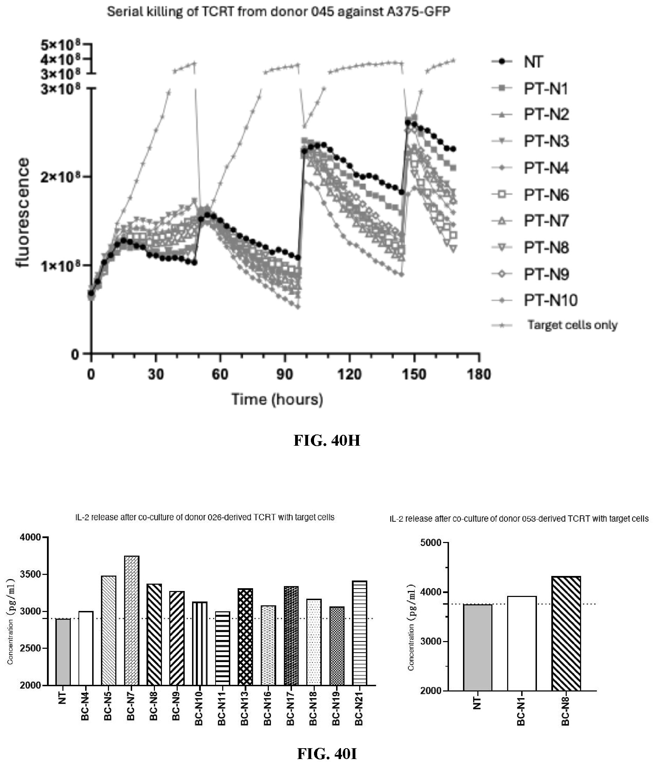

FIG. 40H showed the target cell killing capability of PTPN2 gene-edited TCR-T cells.

The results indicated that, as compared with the control group (NT), the target gene-edited T cells of the present invention had significantly improved target cell killing capability and/or serial killing capability.

### (D) Cytokine release capability

A CBA kit was used to detect cytokine release of unedited cells or cells in which the targets of the present invention were knocked out.

FIGs. 40I-40N showed cytokine release of TCR T cells that were unedited or in which the BCL2L11 target or the PTPN2 target was knocked out, as detected using a CBA kit.

The results indicated that, as compared with the control group (NT), the target gene-edited T cells of the present invention had significantly improved cytokine release capability.

### Example 11. Gene editing of the targets of the present invention

### 1.1 Receipt and processing of tumor tissue

### 1.1.1 Tissue receipt

Tumor tissue and blood samples from a donor were received, sample information was checked and recorded, and corresponding sample labels were printed.

### 1.1.2 Tissue processing and culture

Sample tubes and blood collection tubes were disinfected with 75% alcohol and transferred into a biosafety cabinet. PBMC cells in the blood samples were separated and cryopreserved according to the above PBMC manual separation and cryopreservation operating procedure. A culture flask or culture bag having a gas-permeable surface was selected, for example, a culture bag (Origen), and 300 mL of rewarmed complete medium was added; the complete medium could be arbitrarily selected from X-vivo 15 medium or other commercially available T cell culture media, for example, T cell culture media of brands such as Stem Cell, Lonza, Thermo, Miltenyi, etc., and essential amino acids and antibiotics could be added, and IL-2 at a concentration of 300-9000 IU/mL (for example, 1000-9000 IU/mL, for example, 6000 IU/mL) was added. Several 10 cm culture dishes were taken, an appropriate amount of medium was added, sterile ophthalmic forceps were used to take out tumor tissue from the sample tube into the 10 cm culture dish, and the tissue was washed and the culture dish was replaced. Ophthalmic scissors and ophthalmic forceps were used for preliminary cutting to remove adipose tissue and necrotic tissue, and each tissue block was further cut into pieces of about 27 cubic millimeters. Non-suspended tumor tissue blocks were taken, an internal plunger was removed from a 20 mL syringe, the syringe was connected to the culture bag, and a pipette was used to transfer about 1 g of tissue blocks into the culture bag through the syringe. The culture bag was placed into a carbon dioxide incubator for culture. The scissors and forceps were cleaned, preliminarily disinfected with 75% alcohol, ultrasonically cleaned, and sterilized, thereby obtaining a first TIL population.

### 1.2 Step (A) in vitro expansion and harvest

### 1.2.1 Step (A) in vitro expansion

According to the cell growth state, medium was replenished or half of the medium was replaced every 3-7 days to ensure cell nutrition. Complete medium was used; the complete medium could be arbitrarily selected from X-vivo 15 medium or other commercially available T cell culture media, for example, T cell culture media of brands such as Stem Cell, Lonza, Thermo, Miltenyi, etc., and essential amino acids and antibiotics could be added, and IL-2 at a concentration of 300-9000 IU/mL (for example, 1000-9000 IU/mL, for example, 6000 IU/mL) (Shuanglu and/or Sihuan) was added. On day 3-14 of step (A), for example, sampling and counting could be performed on day 13 or 14, and when the cell number was between 5×10⁵ and 5×10⁸, the harvest step of step (A) was entered.

### 1.2.2 Harvest of step (A)

Cells at the end of in vitro expansion in step (A) were collected and centrifuged, the medium was discarded, and the cells were washed once with PBS or normal saline, thereby obtaining TILs (a second TIL population) subjected to in vitro expansion in step (A); sampling and counting were performed, and about 5×10⁵ to 2×10⁸ cells were retained for subsequent in vitro expansion steps; about 5×10⁵ cells could be taken for quality control testing; and the remaining cells were added with cryopreservation solution and cryopreserved as cryopreserved preREP TIL in vitro cells.

### 1.3 Step (B) TIL activation

TILs (the second TIL population) subjected to in vitro expansion in step (A) were continuously cultured, or cryopreserved preREP TIL in vitro cells were resuscitated, to perform TIL activation of step (B).

Complete medium was used; the complete medium could be arbitrarily selected from X-vivo 15 medium or other commercially available T cell culture media, for example, T cell culture media of brands such as Stem Cell, Lonza, Thermo, Miltenyi, etc., and essential amino acids and antibiotics could be added; the cell density was adjusted to 5×10⁵ to 2×10⁶ cells/mL, in a suspension 24-well culture plate, at 1 mL/well, and IL-2 at a concentration of 300-9000 IU/mL (for example, 1000-9000 IU/mL, for example, 6000 IU/mL) was added. A T cell activator could also be added simultaneously into the medium of each TIL cell population, for example, a CD3 agonist and/or a CD28 agonist was added, for example, about 30 ng/mL CD3 antibody (Miltenyi Biotech, OKT3), about 30 ng/mL CD28 antibody (Merck, 15E8), magnetic beads were added at a ratio of magnetic beads to TIL of about 1:2-2:1 (Dynabeads with a diameter of about 1 to 10 µm, Thermo Fisher) and/or transACT was added at a ratio of transACT to TIL of about 1:100-1:2000 (with a diameter of about 100 to 500 nm, Miltenyi). The cells were cultured for about 0-4 days to obtain a third TIL population.

### 1.4 Step (C) TIL cell gene editing

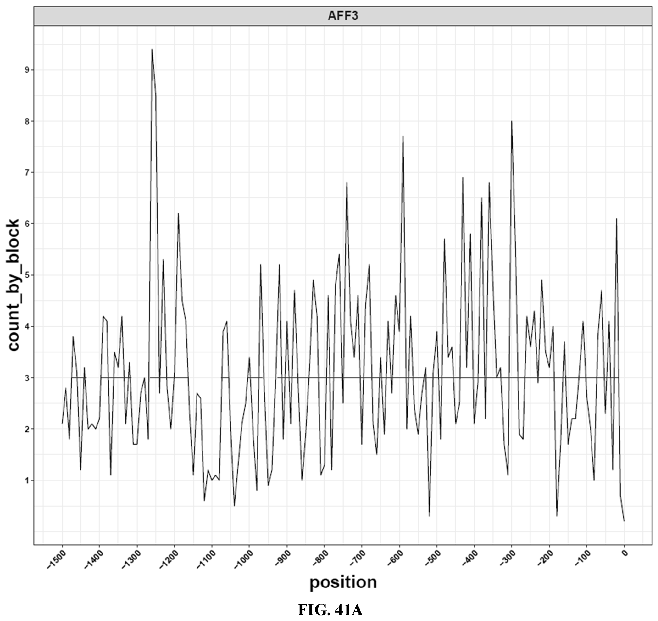

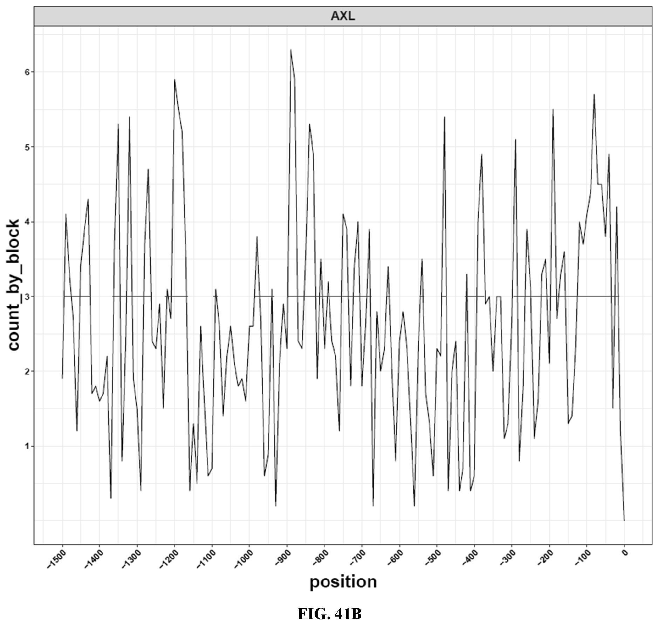

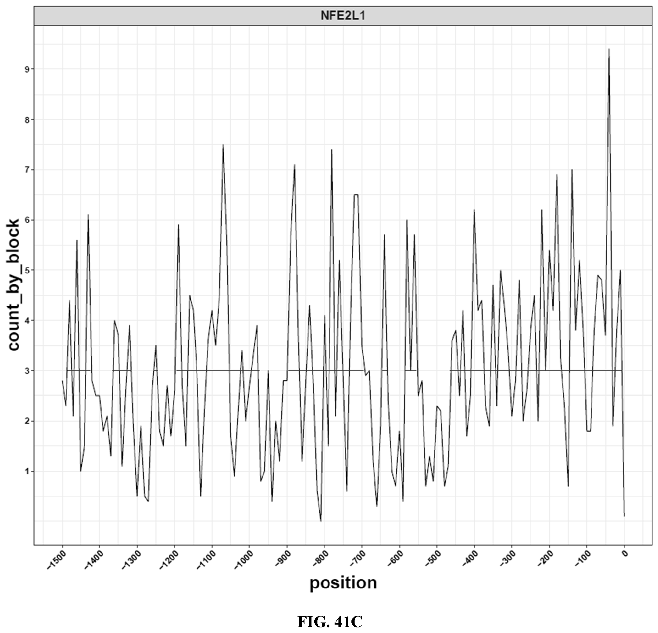

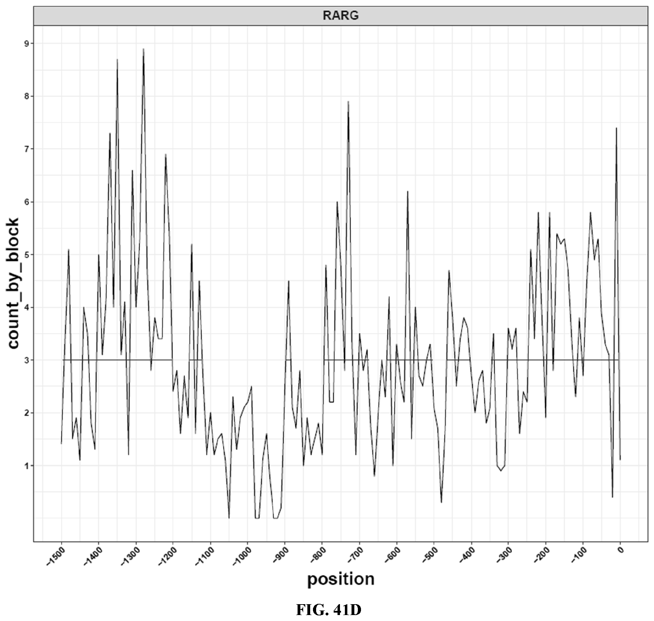

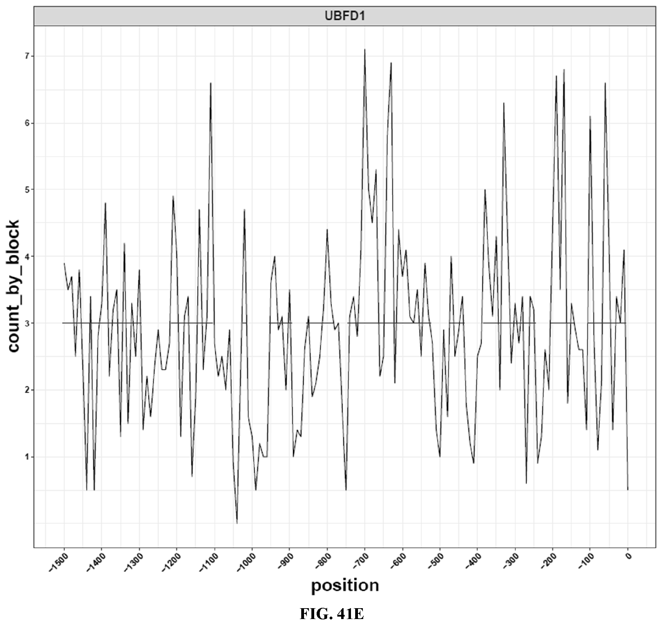

A targeting guide sequence selected from preferred regions shown in FIGS. 41A-41E of the present invention, for example, an sgRNA sequence as shown in SEQ ID NO:3328-9312, SEQ ID NO:9313-11602, SEQ ID NO:11603-13024, SEQ ID NO:13025-14839, and SEQ ID NO:14840-16331, or SEQ ID NO:16332-16805, SEQ ID NO:16806-18300, SEQ ID NO:18301-18850, SEQ ID NO:18851-19772, SEQ ID NO:19773-22463, SEQ ID NO:22464-24415, and SEQ ID NO:24416-24788, was thawed and added with nuclease-free water and prepared to a concentration of about 100 µM. About 2 µL of gRNA (50 µM) was annealed by incubation at 95°C for 2 minutes and then added into P3 buffer, and 0.3-1 µL of Cas9 (for example, Kaika, Kerui, Acro, 10 mg/mL) was added, followed by incubation at 25°C for 10 minutes to form a ribonucleoprotein complex (RNP). In P3 buffer (Lonza), the above RNP and about 1×10⁶ cells of the third TIL population were electroporated using a Lonza electroporator. For example, the electroporation program could be human T cell stim (EO115). After electroporation, the gene-edited cells were cultured for about 0-4 days to obtain a fourth TIL population.

### 1.5 Step (D) Post-gene-editing culture of TIL cells

Feeder cells (irradiated healthy donor PBMC T cells) were added into the fourth TIL cell population for culture. The contact time between TIL and feeder cells was required to be after a certain time Tₙ after the TIL in step (B) had contacted IL-2 and a T cell activator (for example, a CD3 antibody or a nanomatrix comprising a CD3 antibody and a CD28 antibody, such as transACT) (Tₙ of each experimental group could be 0 hours to 12 days, for example, 24 hours or 48 hours). First, feeder cells mixed from 1-5 donors were resuscitated; activated TIL cells and feeder cells were mixed at a ratio of TIL cells to feeder cells of about 1:200, transferred into a G-Rex100 culture flask or a gas-permeable bag, supplemented with complete medium, sampled and counted every 1-3 days, and replenished with medium or subjected to half-medium replacement according to the cell state until the total cell number was greater than 1×10⁹ or step (D) in vitro expansion was cultured for about 5 days to about 14 days, and the culture of step (D) in vitro expansion was terminated.

### 1.6 Harvest of tumor-infiltrating lymphocytes

Cells expanded in step (D) were collected, the medium supernatant was discarded after centrifugation, and the cells were washed three times with PBS or normal saline or a compound electrolyte solution, thereby obtaining TILs (a fifth TIL population) expanded in step (D); during the third wash, sampling and counting were performed, and according to the counting result, after the last centrifugation the supernatant was discarded, and 3×10⁶ cells were taken for quality control testing; all remaining cells were added with cryopreservation solution, and the cell density was adjusted to 1-3×10⁸ cells/mL for cryopreservation.

### Example 12. TCR transduction and gene editing

T cell activation: T cells cryopreserved in liquid nitrogen were resuscitated and cultured, centrifuged and resuspended to 5E5/ml using T cell culture medium RPMI 1640 (Gibco) + 10% FBS (Bovogen), T cell TransAct (Miltenyi) was added at 1:100, recombinant human IL-2 at a concentration of 30 IU/ml was added, and the cells were cultured for about 72 hr.

TCR transduction: One day before transduction, a 24-well suspension culture plate was coated with a recombinant human fibronectin fragment (Retronectin, Takara) at a final concentration of 15 µg/mL, with 250 µL added per well of the 24-well plate. The plate was protected from light and stored at 4°C overnight for later use. The coated 24-well plate was taken out, the coating solution was aspirated and discarded, and 500 µL of a blocking solution containing 2% BSA was added, followed by blocking at room temperature for 30 minutes. The blocking solution was aspirated and discarded, the plate was washed twice with 500 µL/well of a plate-washing solution containing 2.5% HEPES, and the washing solution was aspirated and discarded. The experimental group was transduced with a retrovirus carrying a specific TCR nucleic acid fragment of an NY-ESO-1 antigen peptide. 0.1-1 mL of retroviral solution was added per well, followed by centrifugation at 32°C and 2000g for 2 hours. The supernatant in the 24-well plate was discarded, and resuscitated and activated T cells were added to each well of the 24-well plate in a volume of 500-1000 µL, with a cell concentration of about 5×10⁵ cells/mL. Centrifugation was performed at 30-32°C and 1000g for 10 minutes. After centrifugation, the culture plate was placed in a 37°C, 5%CO₂ incubator for culture to obtain transduced cells. The transduced cells were cultured for about 0-4 days to obtain a TCR-T cell population. The transduced cells were counted for density and viability every 1-3 days, and according to the counting results, T cell culture medium was added, recombinant human IL-2 at a concentration of 30-100 IU/ml was added, the initial culture cell density was adjusted to 0.5-2×10⁶ cells/ml, and culture was continued.

TCR-T gene editing: The gRNAs of the present invention (sgRNA sequences shown in SEQ ID NO: 3328-9312, SEQ ID NO: 9313-11602, SEQ ID NO: 11603-13024, SEQ ID NO: 13025-14839, and SEQ ID NO: 14840-16331) were thawed and nuclease-free water was added to prepare a concentration of about 100 µM. About 2 µL of gRNA (50 µM) was incubated at 95°C for 2 minutes for annealing and then added to P3 buffer, and 0.3-1 µL of Cas9 (e.g., Kaika, Kerui, Acro, 10 mg/mL) was added, followed by incubation at 25°C for 10 minutes to form a ribonucleoprotein complex (RNP). In P3 buffer (Lonza), the above RNP and about 1×10⁶ TCR-T cells were electroporated using a Lonza electroporator. For example, the electroporation program could be human T cell stim (EO115). Recombinant human IL-2 at a concentration of 100-300 IU/ml was added to the electroporated T cells, and culture was continued to obtain the target gene-edited TCR-T cells of the present invention.

### Example 13. PDO model culture

Tumor tissue was obtained from surgery, transported and stored at 2-8°C after ex vivo collection, and mechanically dissociated within 24 hours. The tissue was cut into pieces of about 0.5 mm³ in size, and then digested at 37°C using a dedicated tissue digestion solution (bioGenous), and the reaction was terminated with 10% FBS. The tissue suspension was filtered through a 100 µm filter, washed, placed on ice, and thoroughly mixed with Matrigel, and then the mixture was spotted onto the bottom of a cell culture plate. The culture plate was placed in a 37°C incubator, and after Matrigel was fully solidified, complete culture medium was carefully added for culture. When the organoids grew to a sufficient size and density, passaging or cryopreservation was performed to obtain a PDO (tumor organoid, Patient-Derived Organoid) model.

The Matrigel and organoid mixture was scraped off with the tip of a pipette tip and transferred to a centrifuge tube containing basal medium, and pipetting was performed to separate the organoids from Matrigel. After centrifugation, a tissue digestion solution for passaging was added, mixed well, and placed in a 37°C incubator for digestion. Basal medium was added to dilute the digestion solution, followed by centrifugation and washing. A small amount of cell suspension was taken and 8 volumes of digestion solution were added for digestion to obtain single cells, the reaction was terminated with FBS, and counting was performed. According to the counting results, the required volume of organoids was aspirated, centrifuged, and resuspended in T cell culture medium; the cells to be tested (e.g., TIL cells or TCR-T cells) were counted, and the cells to be tested were resuspended to the required density according to different effector-to-target ratios; in a flat-bottom 96-well plate, 100 µl each of PDO target cells and the cells to be tested were added, three replicate wells were set for each group, and the plate was used for PDO co-culture detection. Several different effector-to-target ratios were set; meanwhile, one group containing only PDO target cells was set, and one control group (NT) was set for co-culturing unedited cells to be tested with only PDO target cells.

### Example 14. Detection of knockout efficiency of cells

Reagents and materials: DNA extraction solution (QuickExtract DNA extraction solution, Lucigen, QE09050), nuclease-free water (RNase/DNase free water, Tiangen), EDTA (Sangon, 0.5M), Recombinant DNase I (RNase-free, TAKARA).

Genomic DNA extraction: With reference to the gene editing method of the examples of the present invention, about 2-7 days after T-cell knockout, about 1×10⁵ to about 2×10⁵ cells were taken, washed once with PBS, and then the gene-edited cells were resuspended in 44 µL PBS, and 6 µL of a prepared nuclease mixture (containing 1 µL DNase I and 5 µL 10× DNase I Buffer) was added, followed by incubation at 37°C for 5 minutes. 2.5 µL of 0.5 M EDTA was added to the sample, followed by incubation at 80°C for 10 minutes. After centrifugation, the supernatant was discarded, 50 µL of DNA extraction solution was added to the cell pellet, and after brief centrifugation, the following program was run: 75°C - 10 minutes; 95°C - 5 minutes; 4°C - hold. The concentration of the DNA sample could be measured using a spectrophotometer (NanoDrop^{™}).

Sequencing: PCR primers could be designed in a region of about 100 to about 200 nucleotides upstream and downstream of the PAM site. A PCR reaction system was designed according to the following:

| Reagent | Volume |
|---|---|
| 2×PCR Premix Buffer | 25 µL |
| DNA template | about 100-500 ng |
| Forward primer (10 µM) | 1 µL |
| Reverse primer (10 µM) | 1 µL |
| Double-distilled water | added to make up to 50 µL |
| DMSO | 1.5 µL |

Amplification was performed according to the following PCR program:

| Temperature | Time | |
|---|---|---|
| 95°C | 4 minutes | |
| 95°C | 15 seconds | These 3 steps were repeated for 35 cycles. |
| 58°C | 30 seconds | |
| 72°C | 30 seconds | |
| 72°C | 7 minutes | |
| 4°C | Maintained | |

The PCR products were subjected to Sanger sequencing analysis.

### Analysis of the Crispr Cas9 knockout efficiency

The Crispr Cas9 knockout efficiency was analyzed using the Tracking of Indels by DEcomposition (Tide) method based on Sanger sequencing data; the specific method could be referred to Brinkman et al., Nucl. Acids Res. (2014) or shinyapps.datacurators.nl/tide/. The corresponding sgRNA sequence of the present invention, the pre-knockout control sequence, and the test sequence after Crispr Cas9 knockout were input, the P-value threshold was set to 0.001, and the knockout efficiency analysis was performed.

The knockout efficiency detection was performed in TCRT cells.

| Target gene | gRNA | Donor knockout efficiency (%) |
|---|---|---|
| AFF3 | AFF3-1 | 53.4, 79.7 |
| AFF3 | AFF3-2 | 74.1, 81.1 |
| AXL | AXL-2 | 90.5 |
| AXL | AXL-4 | 5.2 |
| NFE2L1 | NFE2L1-3 | 71.5, 79.9 |
| NFE2L1 | NFE2L1-4 | 91.8, 65 |
| RARG | RARG-2 | 16.5, 72.2 |
| RARG | RARG-4 | 77.3 |
| UBFD1 | UBFD1-1 | 85.6, 68.1 |
| UBFD1 | UBFD1-2 | 66.9, 56.6 |

The knockout efficiencies of the guide of the same sequence in different donor cells were separated by commas.

Knockout efficiency detection was performed in TIL cells.

Wherein donors 005 and 031 were healthy donors and were derived from peripheral blood of healthy donors, donor 309 was a cervical cancer patient, donor 107 was a lung cancer patient, and donor 812 was a melanoma patient.

| Target | gRNA | Donor knockout efficiency (%) |
|---|---|---|
| AFF3 | AFF3-1 | 71, 85.1 |
| AXL | AXL-2 | 82.8, 87.5 |
| NFE2L1 | NFE2L1-3 | 77.5, 82.4 |
| RARG | RARG-4 | 69.4, 93.5 |
| UBFD1 | UBFD1-2 | 85, 85.3 |

The knockout efficiencies of the guide of the same sequence in different donor cells were separated by commas.

| Target | gRNA | Sequence | SEQ ID NO: |
|---|---|---|---|
| AFF3 | AFF3-1 | CAGCCTCTGATCATTCCCGG | 49583 |
| AFF3 | AFF3-2 | CAGCTTAGGAGACTCATCAG | 49584 |
| AXL | AXL-2 | CCTAGCAGTACATACCACCA | 49585 |
| AXL | AXL-4 | CTGAGAACATTAGTGCTACG | 49586 |
| NFE2L1 | NFE2L1-3 | GTGGATACCATAGCCATCCA | 49587 |
| NFE2L1 | NFE2L1-4 | TATGGCTCGAGTCTAAGGAA | 49588 |
| RARG | RARG-2 | GCTACAGAAGTGCTTCGAAG | 49589 |
| RARG | RARG-4 | TGGGCAAGTATACCACGGTG | 49590 |
| UBFD1 | UBFD1-1 | AGCAACGGCGAAGACGCGGG | 49591 |
| UBFD1 | UBFD1-2 | AGGAATTACCTGTAATCGAG | 49592 |

The results showed that the editing modes of various genes of the present invention or combinations thereof each achieved a certain proportion of knockout efficiency.

### Example 15. Detection of the expansion of TCRT cells

### Experimental preparation

The proliferation of the gene-edited TCRT cells was detected.

TCRT cells of each group were harvested, washed once with PBS, and then resuspended in T cell medium (without IL-2). After counting, the cell density was adjusted to 1e5 to 2e6/mL, and 100 µL/well was added into a flat-bottom 96-well plate. For the CD3 antibody stimulation group, a CD3 antibody (OKT3) was added at 30 ng/ml for stimulation. For the TransACT stimulation group, transACT (with a diameter of about 100 to 500 nm, Miltenyi) was added such that the concentration of the transACT working solution was 1:1000 (v/v). For the unstimulated medium group, only an equal volume of cell culture medium was added. After 3 days, a CTG kit (CellTiter-Glo Luminescent Cell Viability Assay, Promega) was used to analyze the fluorescence intensity of T cells, and the expansion efficiency of T cells was characterized by the fluorescence intensity on day 3.

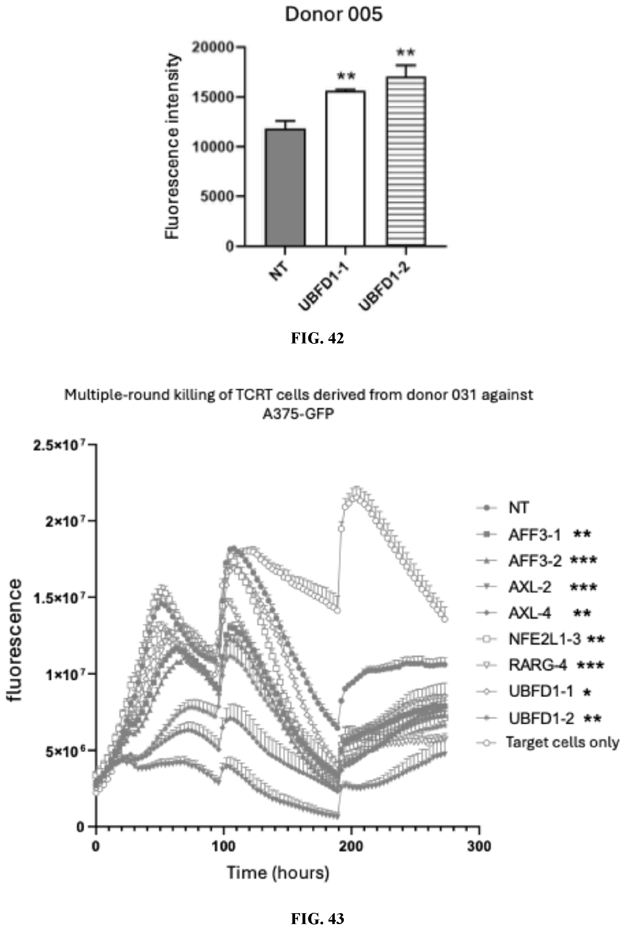

FIG. 42 showed the TCRT expansion capability of UBFD1 single-target gene-edited cells.

The significant difference was relative to the unedited NT group, wherein ** indicated P<0.01.

The results showed that, relative to the unedited control group (NT), the target gene-edited cells of the present invention had significantly improved expansion capability.

### Example 16. Detection of multi-round killing capability of cells

IncuCyte was used to monitor changes in fluorescence intensity of target cells A375-GFP during multi-round killing. A375-GFP cells were uniformly plated in a multiwell plate and cultured at 37°C for 4 hours. Then, unedited cells or cells with knockout of the targets of the present invention were taken and added into the corresponding wells, after which IncuCyte was used to start recording GFP fluorescence signals, with 3 replicate wells for each group. After 24 hours of killing, co-culture supernatants (20 µL/well) were collected for CBA detection. After 96 hours of killing, a new multiwell plate uniformly plated with A375-GFP was cultured at 37°C for 4 hours, and then the mixture of unedited cells or cells with knockout of the targets of the present invention from the previous round of killing and A375-GFP cells was transferred into a new 96-well plate, and IncuCyte was used to record the change curve of GFP fluorescence signals in a new round of killing.

FIG. 43 showed the multi-round killing results of AFF3, AXL, NFE2L1, RARG, and UBFD1 single-target gene-edited cells in TCR-T cells.

The significant difference was relative to the unedited NT group, wherein * indicated P<0.05, ** indicated P<0.01, *** indicated P<0.001, and **** indicated P<0.0001.

The results showed that, relative to the control group (NT), the gene-edited cells of the present invention had significantly improved multi-round killing capability.

### Example 17. Detection of cytokine release by CBA method

For co-culture of gene-edited cells with autologous tumor cells, co-culture supernatants were collected, and a CBA kit (BD) was used to detect cytokine release of unedited cells or cells with knockout of the targets of the present invention.

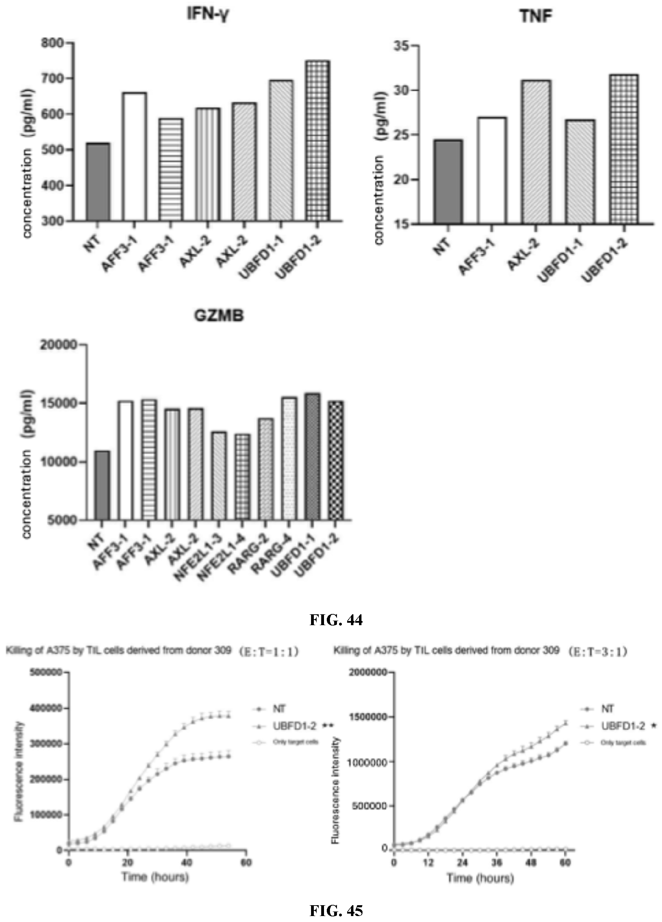

FIG. 44 showed that, in TCR-T cells, AFF3-, AXL-, NFE2L1-, RARG-, and UBFD1-single-target-edited cells had higher cytokine release levels.

For example, the cytokine expression capability included a higher IFN-γ release level, a higher TNF-α release level, or a higher GZMB release level.

The results showed that the target gene-edited cells of the present invention had higher functional cytokine release levels.

### Example 18. Detection of cell killing capability

Starting from day 6 after gene editing, tumor target cells were plated in a 96-well flat-bottom plate. On the next day, TIL cells of each group were co-cultured with target cells at different effector-to-target ratios (T cells: target cells, E:T). Target cells and T cells were each 100 µL, three replicate wells were set for each group, and a control group containing only target cells was set at the same time. The target cells could be selected from Hey-T30 ovarian cancer cells and A375 melanoma cells.

According to the instructions of an apoptosis detection reagent (Incucyte Caspase-3/7 Green Dye for Apoptosis, Sartorius), the apoptosis detection reagent was added at 0.2 µL/well, and medium was added at 25 µL/well to dilute Caspase 3/7 Green Dye. An Incucyte recorder (Sartorius) was used to record Caspase 3/7 activity to analyze the killing capability of TIL cells against target cells. Recording was performed once every 3 hours, with a total recording duration of about 5 days.

FIG. 45 showed the target cell killing capability of UBFD1-edited TIL cells derived from donor 309.

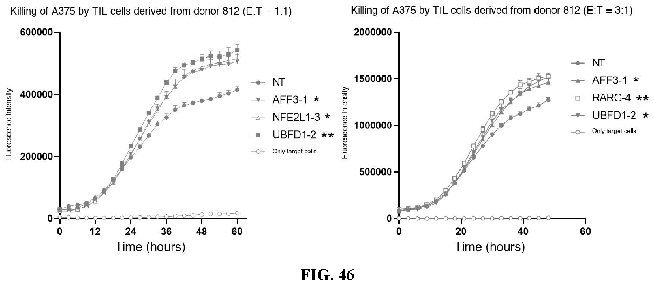

FIG. 46 showed the target cell killing capability of AFF3-, NFE2L1-, RARG-, and UBFD1-edited TIL cells derived from donor 812.

The significant difference was relative to the unedited NT group, wherein * indicated P<0.05 and ** indicated P<0.01.

The results showed that, relative to the control group (NT), the target gene-edited cells of the present invention had significantly improved target cell killing capability.

### Detection of killing capability in a PDO model

A PDO model was prepared with reference to the examples of the present invention and was co-cultured with TIL cells. After a Caspase3/7 substrate was added for apoptosis signal labeling, the experimental plate was placed in Incucyte for observation and recording.

The results showed that, relative to the control group (NT), the gene-edited cells of the present invention had significantly improved cell killing capability in the PDO model.

### Example 19. Detection of the expansion of TIL cells

### Experimental preparation

The proliferation of TIL cells was detected on days 7-10 after gene editing (with IL-2 withdrawn).

TIL cells of each group were harvested, washed once with PBS, and then resuspended in T cell medium (without IL-2). After counting, the cell density was adjusted to 1e5 to 2e6/mL, and 100 µL/well was added into a flat-bottom 96-well plate. For the CD3 antibody stimulation group, a CD3 antibody (OKT3) was added at 30 ng/ml for stimulation. For the TransACT stimulation group, transACT (with a diameter of about 100 to 500 nm, Miltenyi) was added such that the concentration of the transACT working solution was 1:1000 (v/v). For the unstimulated medium group, only an equal volume of cell culture medium was added. A CTG kit (CellTiter-Glo Luminescent Cell Viability Assay, Promega) was used to analyze the fluorescence intensity at the time of T-cell plating, and after 3 days, the CTG kit was used to analyze the fluorescence intensity of T cells. The expansion efficiency of T cells was characterized by the fluorescence intensity on day 3/the fluorescence intensity at the time of plating.

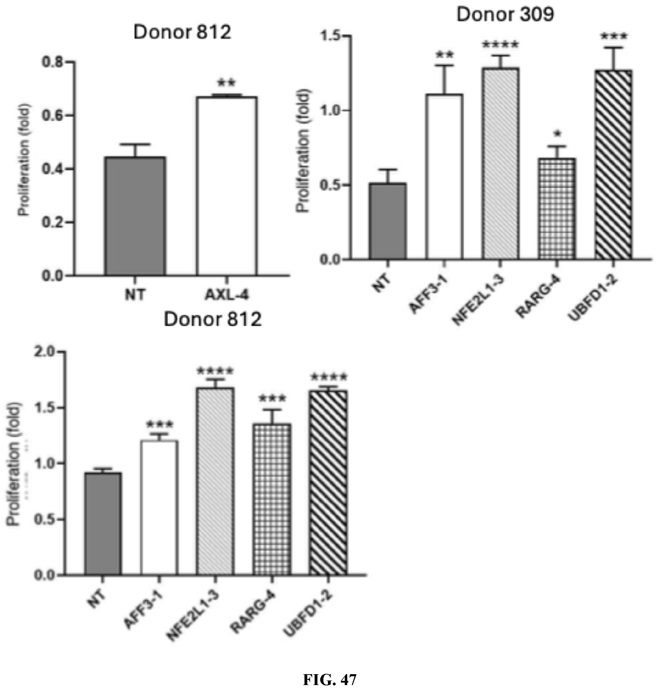

FIG. 47 showed the fold expansion of AFF3-, AXL-, NFE2L1-, RARG-, and UBFD1-single-target gene-edited TILs in the unstimulated medium group.

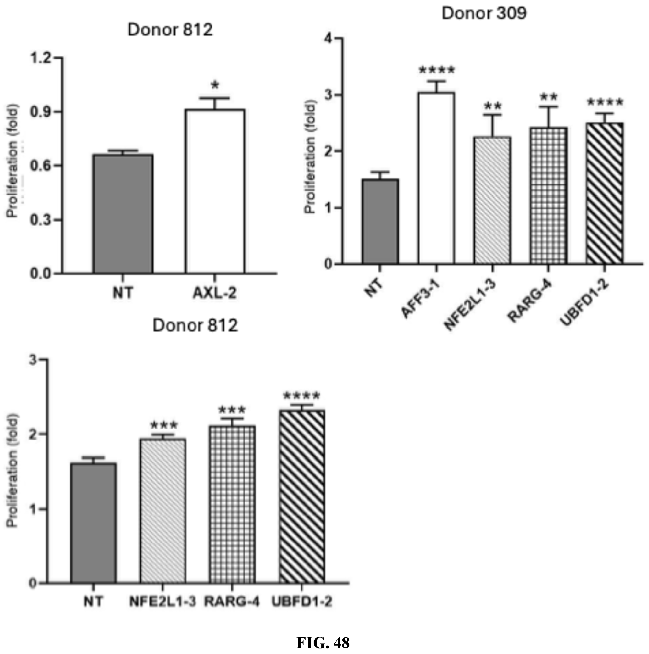

FIG. 48 showed the fold expansion of AFF3-, AXL-, NFE2L1-, RARG-, and UBFD1-single-target gene-edited TILs in the TransACT stimulation group.

The significant difference was relative to the unedited NT group, wherein * indicated P<0.05, ** indicated P<0.01, *** indicated P<0.001, and **** indicated P<0.0001.

The results showed that, relative to the unedited control group (NT), the target gene-edited cells of the present invention had significantly improved expansion capability.

### Example 20. Flow cytometric detection of cells

A flow cytometer was used to detect expression of molecules related to T-cell exhaustion, stemness, and the like in TIL cells obtained on day 8 after gene editing.

### Sources of main reagents and materials for the TIL flow cytometry test:

V-bottom 96-well plates, manufacturer Corning, catalog No. 3894; flow cytometry tubes, manufacturer Corning, catalog No. 352052; flow cytometry antibodies were purchased from BD or Biolegend.

Detection of cell surface molecules: 1×10⁵ to 5×10⁵ cell samples from each group were added into flow cytometry tubes or V-bottom 96-well plates. Centrifugation was performed at 600 g for 3 minutes, and the supernatant was discarded. PBS washing was performed once, with 1 mL/tube for flow cytometry tubes and 200 µL/well for 96-well plates, and the supernatant was discarded. A prepared antibody working solution was added for cell surface staining, wherein the antibody (BD or Biolegend) concentration was 1:100 to 1:200, and an activity detection dye was included at 1:10000. Staining was performed with 100 µL/tube for flow cytometry tubes and 50 µL/well for 96-well plates, followed by incubation at 2-8°C for 30 minutes in the dark. After completion of surface staining, the cells were washed once with PBS (200 µL/time for 96-well plates and 1 mL/time for flow cytometry tubes), centrifuged at 600 g for 3 minutes at room temperature, and the supernatant was discarded after centrifugation. The cells were resuspended in 100-500 µL PBS and subjected to flow cytometric analysis.

Detection of intracellular molecules: an antibody mixed working solution was prepared for cell surface staining of CD3/CD4/CD8, wherein the antibody concentration was 1:100, and the cell viability detection dye concentration was (1:10000). Staining was performed with 50 µL/well for 96-well plates and 100 µL/tube for flow cytometry tubes, followed by incubation at 2-8°C for 30 minutes in the dark. The cells were washed once with PBS (200 µL/time for 96-well plates and 1 mL/time for flow cytometry tubes), centrifuged at 600 g for 3 minutes at room temperature, and the supernatant was discarded after centrifugation. 100 µL of fixation/permeabilization solution (BD, Fixation/Permeabilization) was added to each well, followed by incubation at 2-8°C for 20-40 minutes in the dark. After fixation and permeabilization, washing was performed twice with 1×Perm/Wash Buffer (200 µL/time for 96-well plates and 1 mL/time for flow cytometry tubes), centrifugation was performed at 600 g for 3 minutes, and the supernatant was discarded after centrifugation. Intracellular molecule antibodies (e.g., TCF1) were prepared with 1×Perm/Wash Buffer, and TIL cells were resuspended (staining with 50 µL/well for 96-well plates and 100 µL/tube for flow cytometry tubes), followed by incubation at 2-8°C for 30 minutes in the dark. After intracellular molecule staining, washing was performed 1-2 times with 1×Perm/Wash Buffer (200 µL/time for 96-well plates and 1 mL/time for flow cytometry tubes), centrifugation was performed at 600 g for 3 minutes, and the supernatant was discarded after centrifugation. The cells were resuspended in 100-500 µL PBS and subjected to flow cytometric analysis.

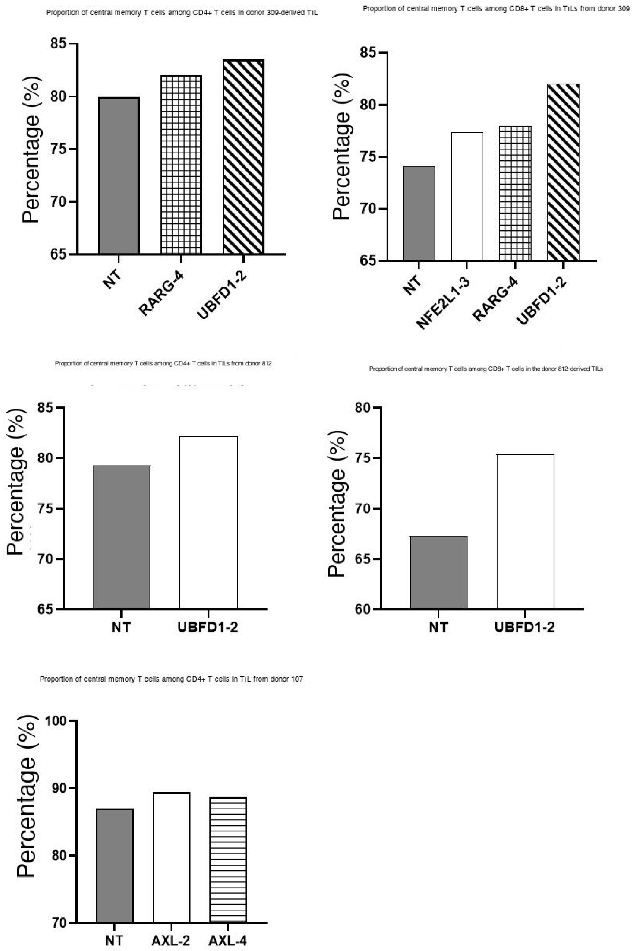

FIG. 49 showed the proportion of central memory T cells in AXL-, NFE2L1-, RARG-, and UBFD1-edited TIL cells. For example, central memory T cells could be CD45RO-positive CD62L-positive cells.

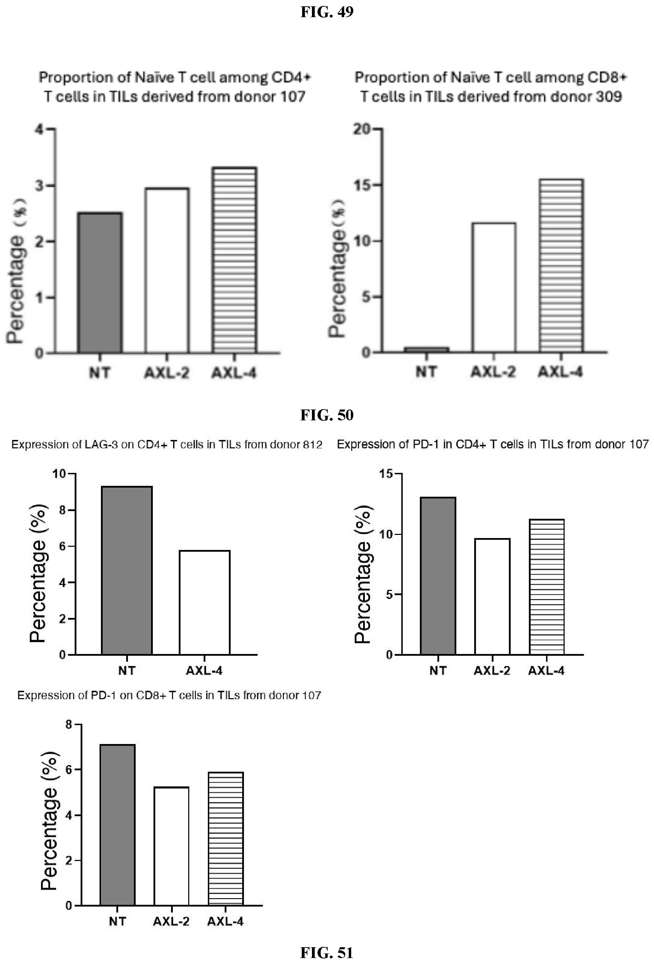

FIG. 50 showed the proportion of naive T cells in AXL-edited TIL cells. For example, naive T cells could be CD45RO-negative CD62L-positive cells.

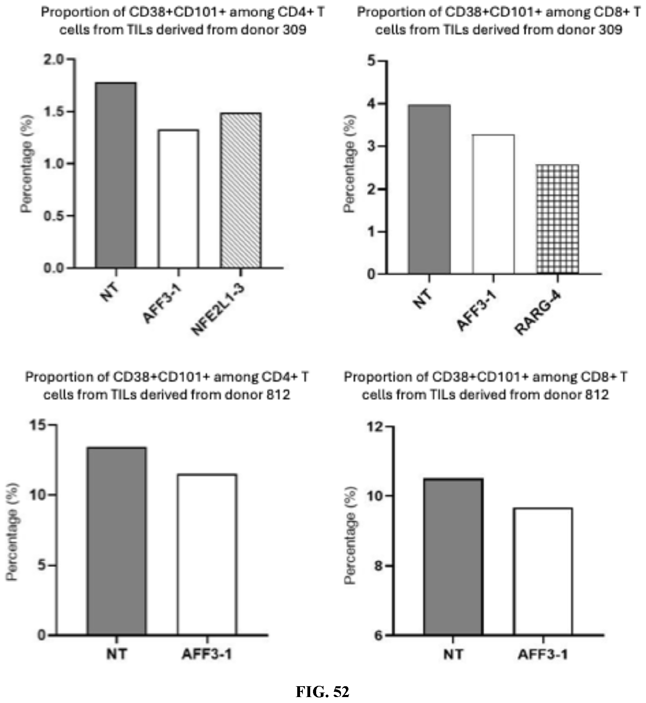

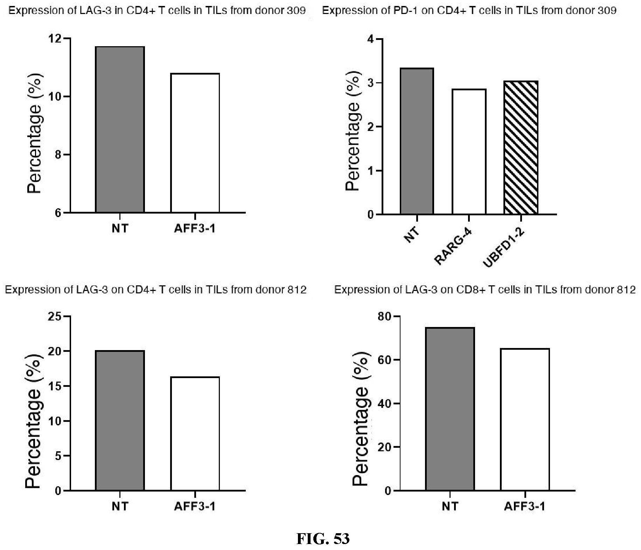

FIGs. 51, 52, and 53 showed the proportion of exhausted cells in AFF3-, AXL-, NFE2L1-, RARG-, and UBFD1-edited TIL cells. For example, exhausted T cells could be PD-1-positive, LAG-3-positive, TIM-3-positive, CD38-positive, and/or CD101-positive cells.

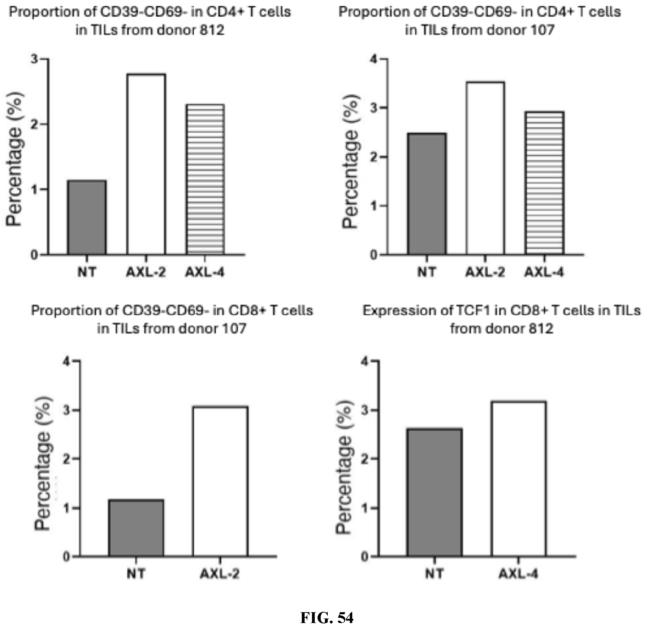

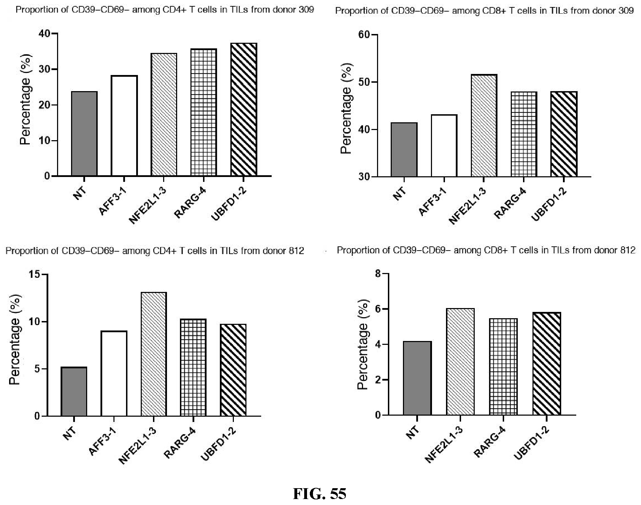

FIGs. 54 and 55 showed the proportion of stem cell-like T cells in TIL cells after editing of AFF3, AXL, NFE2L1, RARG, and UBFD1. For example, the stem cell-like T cells could have a CD39-negative CD69-negative or TCF1-positive phenotype.

The results showed that, relative to the non-gene-edited control group (NT), the target gene-edited cells of the present invention could have more favorable cellular phenotypic characteristics.

### Example 21. Flow cytometric detection of cytokine expression

For the TIL cell populations obtained on day 7 or day 8 after gene editing in each experimental group, cytokine expression was detected by using a flow cytometer.

### Experimental preparation

A medium required for intracellular factor expression detection was prepared as follows: a TIL cell culture medium was taken, and the following were added according to a volume ratio: Golgistop 0.7:1000, Golgiplug 1:1000, and CD107a antibody 1:500, i.e., 2 µL/mL. No interleukin was added.

### Detection procedure

After the TIL cells of each experimental group were centrifuged, each group of TIL cells was resuspended by using the above medium required for intracellular factor expression detection. After counting, the cell density was adjusted to 1×10⁶ cells/mL, and the cells were added into a 96-well plate at 200 µL/well. For the CD3 antibody stimulation group, a CD3 antibody (OKT3) was added at 30 ng/ml for stimulation. For the TransACT stimulation group, transACT (with a diameter of about 100 to 500 nm, Miltenyi) was added such that the working concentration of transACT was 1:1000 (v/v). For the unstimulated medium group, only an equal volume of cell culture medium was added. The cells were placed in a 37°C incubator and incubated overnight.

### Sources of main reagents and materials for the TIL cell cytokine flow cytometry detection experiment:

V-bottom 96-well plates, manufacturer Corning, catalog No. 3894; flow cytometry tubes, manufacturer Corning, catalog No. 352052; and flow cytometry antibodies purchased from BD or Biolegend.

After incubation, the cells were washed once with PBS at 200 µL/well, centrifuged at 600 g for 3 minutes, and the supernatant was discarded. An antibody mixed working solution was prepared for cell surface staining of CD3/CD4/CD8, wherein the antibody concentration was 1:100 and the viability dye concentration was 1:10000. Staining was performed at 50 µL/well for the 96-well plate and 100 µL/tube for the flow cytometry tubes, followed by incubation at 2-8°C for 30 minutes in the dark. The cells were washed once with PBS (200 µL/time for the 96-well plate, 1 mL/time for the flow cytometry tubes), centrifuged at 600 g for 3 minutes at room temperature, and the supernatant was discarded after centrifugation. 100 µL of fixation/permeabilization solution (BD, Fixation/Permeabilization) was added to each well, followed by incubation at 2-8°C for 20-40 minutes in the dark. After fixation and permeabilization, the cells were washed twice with 1×Perm/Wash Buffer (200 µL/time for the 96-well plate, 1 mL/time for the flow cytometry tubes), centrifuged at 600 g for 3 minutes, and the supernatant was discarded after centrifugation. Cytokine detection antibodies (e.g., GZMB, TNF-α, IFN-γ) were prepared by using 1×Perm/Wash Buffer, and the TIL cells were resuspended (50 µL/well for the 96-well plate, 100 µL/tube for the flow cytometry tubes) and stained, followed by incubation at 2-8°C for 30 minutes in the dark. After cytokine staining, the cells were washed 1-2 times with 1×Perm/Wash Buffer (200 µL/time for the 96-well plate, 1 mL/time for the flow cytometry tubes), centrifuged at 600 g for 3 minutes, and the supernatant was discarded after centrifugation. The cells were resuspended in 100-500 µL PBS and subjected to flow cytometric analysis.

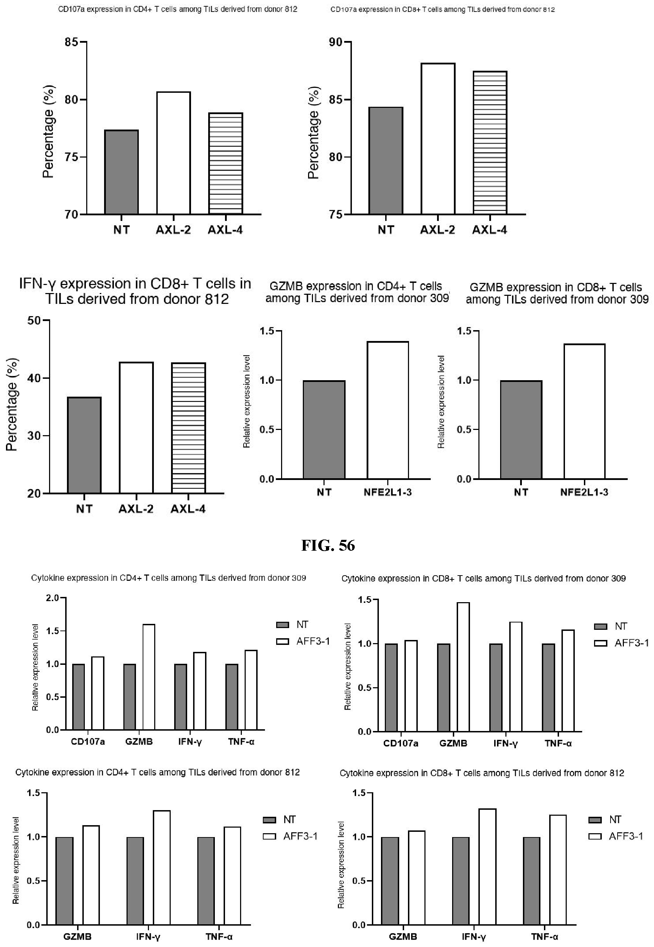

FIGs. 56 and 57 showed that, in the unstimulated medium group, TIL cells edited for AFF3, AXL, and NFE2L1 had a higher proportion of cytokine expression.

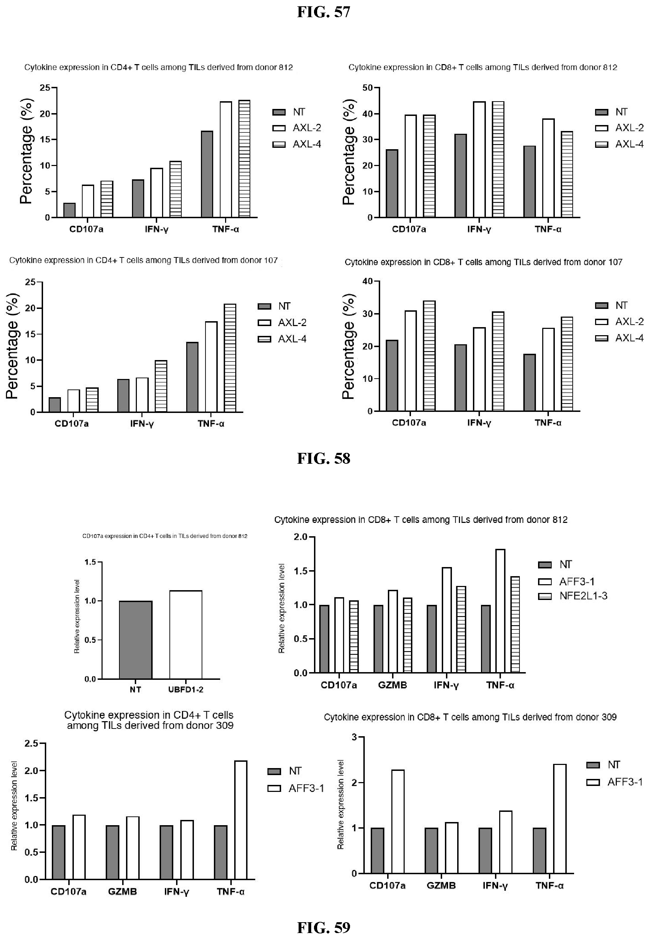

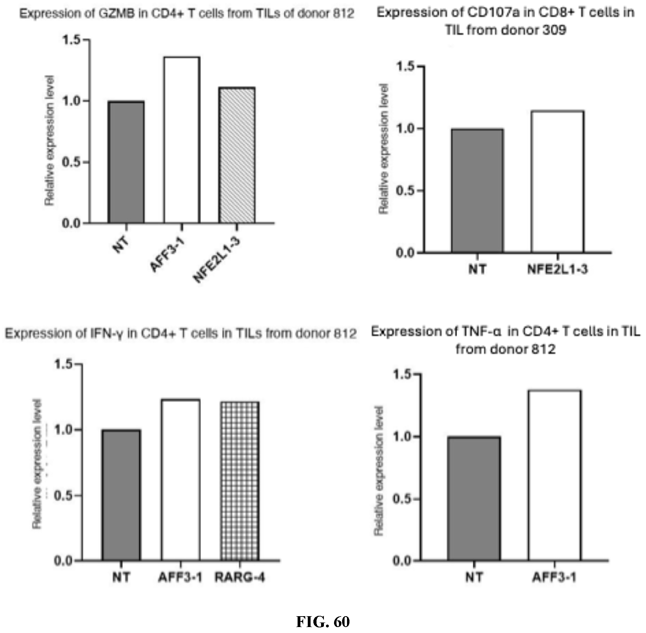

FIGs. 58, 59, and 60 showed that, in the TransACT stimulation group, TIL cells edited for AFF3, AXL, NFE2L1, and RARG had a higher proportion of cytokine expression.

For example, the cytokine expression capability comprised a higher CD107a expression capability, a higher IFN-γ expression capability, a higher TNF-α expression capability, or a higher GZMB expression capability.

The results showed that the target gene-edited cells of the present invention had a higher functional cytokine expression capability.

### Example 22. TCR transduction and gene editing

T cell activation: T cells cryopreserved in liquid nitrogen were revived and cultured, centrifuged, and resuspended in a T cell culture medium RPMI 1640 (Gibco) + 10% FBS (Bovogen) to 5E5/ml. T cell TransAct (Miltenyi) was added at 1:100, recombinant human IL-2 was added at a concentration of 30 IU/ml, and the cells were cultured for about 72 hr.

TCR transduction: One day before transduction, a 24-well suspension culture plate was coated with a recombinant human fibronectin fragment (Retronectin, Takara) at a final concentration of 15 µg/mL, at 250 µL per well of the 24-well plate. The plate was protected from light and stored at 4°C overnight for later use. The coated 24-well plate was taken out, the coating solution was aspirated and discarded, and 500 µL of a blocking solution containing 2% BSA was added for blocking at room temperature for 30 minutes. The blocking solution was aspirated and discarded, and the plate was washed twice with 500 µL/well of a plate-washing solution containing 2.5% HEPES, and the plate-washing solution was aspirated and discarded. The experimental group was transduced with a retrovirus carrying a specific TCR nucleic acid fragment of an NY-ESO-1 antigen peptide. 0.1-1 mL of retrovirus solution was added to each well, followed by centrifugation at 32°C and 2000g for 2 hours. The supernatant in the 24-well plate was discarded, and the revived and activated T cells were added to each well of the 24-well plate at a volume of 500-1000 µL, with a cell concentration of about 5×10⁵ cells/mL. Centrifugation was performed at 30-32°C and 1000g for 10 minutes. After centrifugation, the culture plate was placed in a 37°C, 5% CO₂ incubator for culture to obtain transduced cells. After transduction, the cells were cultured for about 0-4 days to obtain a TCR-T cell population. After transduction, according to the cell counting density and viability of the cells every 1-3 days, a T cell culture medium was added according to the counting results, recombinant human IL-2 was added at a concentration of 30-100 IU/ml, the initial culture cell density was adjusted to 0.5-2×10⁶ cells/ml, and culture was continued.

TCR-T gene editing: The gRNA of the present invention (sgRNA sequences shown in SEQ ID NO: 16332-16805, SEQ ID NO: 16806-18300, SEQ ID NO: 18301-18850, SEQ ID NO: 18851-19772, SEQ ID NO: 19773-22463, SEQ ID NO: 22464-24415, and SEQ ID NO: 24416-24788) was thawed and added with nuclease-free water to prepare a concentration of about 100 µM. About 2 µL of gRNA (50 µM) was annealed by incubation at 95°C for 2 minutes and then added into P3 buffer, and 0.3-1 µL of Cas9 (e.g., Kaika, Kerui, Acro, 10 mg/mL) was added, followed by incubation at 25°C for 10 minutes to form a ribonucleoprotein complex (RNP). In P3 buffer (Lonza), the above RNP and about 1×10⁶ TCR-T cells were electroporated by using a Lonza electroporator. For example, the electroporation program could be human T cell stim (EO115). Recombinant human IL-2 was added to the electroporated T cells at a concentration of 100-300 IU/ml, and culture was continued to obtain the target gene-edited TCR-T cells of the present invention.

### Example 23. PDO model culture

Tumor tissue was obtained from surgery, transported and stored at 2~8°C after ex vivo collection, mechanically dissociated within 24 hours, and cut into pieces of about 0.5 mm³ in size, and then digested at 37°C by using a dedicated tissue digestion solution (bioGenous). The reaction was terminated with 10% FBS. The tissue suspension was filtered through a 100 µm filter and washed, placed on ice and thoroughly mixed with Martrigel, and then the mixture was spotted onto the bottom of a cell culture plate. The culture plate was placed in a 37°C incubator, and after Martrigel was fully solidified, a complete medium was carefully added for culture. When organoids grew to a sufficient size and density, passaging or cryopreservation was performed to obtain a PDO (tumor organoid, Patient-Derived Organoid) model.

The Matrigel and organoid mixture was scraped off with the tip of a pipette tip and transferred into a centrifuge tube containing a basal medium, and pipetting was performed to separate the organoids and Matrigel. After centrifugation, a tissue digestion solution for passaging was added. After mixing, digestion was performed in a 37 °C incubator. A basal medium was added to dilute the digestion solution, followed by centrifugation and washing. A small amount of cell suspension was taken and added with 8 times the volume of digestion solution for digestion to make single cells. The reaction was terminated with FBS, and counting was performed. According to the counting results, a required volume of organoids was aspirated and centrifuged and then resuspended with a T cell culture medium. Cells to be tested (e.g., TIL cells or TCR-T cells) were counted, and the cells to be tested were resuspended to a required density according to different effector-to-target ratios. In a flat-bottom 96-well plate, 100 µl each of PDO target cells and the cells to be tested were added, and three replicate wells were set for each group for PDO co-culture detection. Several different effector-to-target ratios were set. Meanwhile, a group containing only PDO target cells was set, and a control group (NT) was set for co-culturing unedited cells to be tested with only PDO target cells.

### Example 24. Detection of knockout efficiency of cells

Reagents and materials: DNA extraction solution (QuickExtract DNA extraction solution, Lucigen, QE09050), nuclease-free water (RNase/DNase free water, Tiangen), EDTA (Sangon, 0.5M), and Recombinant DNase I (RNase-free, TAKARA).

Genomic DNA extraction: With reference to the gene editing method of the examples of the present invention, at about 2-7 days after T cell knockout, about 1×10⁵ to about 2×10⁵ cells were taken and washed once with PBS, and then the gene-edited cells were resuspended in 44 µL PBS, and 6 µL of a prepared nuclease mixture (containing 1 µL DNase I and 5 µL 10× DNase I Buffer) was added, followed by incubation at 37°C for 5 minutes. 2.5 µL of 0.5 M EDTA was added into the sample, followed by incubation at 80°C for 10 minutes. After centrifugation, the supernatant was discarded, 50 µL DNA extraction solution was added to the cell pellet, and after brief centrifugation, the following program was run: 75°C - 10 minutes; 95°C - 5 minutes; 4°C - hold. The concentration of the DNA sample could be measured by using a spectrophotometer (NanoDrop^{™}).

Sequencing: PCR primers could be designed in a region of about 100 to about 200 nucleotides upstream and downstream of the PAM site. A PCR reaction system was designed according to the following:

| Reagent | Volume |
|---|---|
| 2 × PCR Premix Buffer | 25 µL |
| DNA template | about 100-500 ng |
| Forward primer (10 µM) | 1 µL |
| Reverse primer (10 µM) | 1 µL |
| Double-distilled water | added to 50 µL |
| DMSO | 1.5 µL |

Amplification was performed according to the following PCR program:

| Temperature | Time | |
|---|---|---|
| 95°C | 4 min | |
| 95°C | 15 s | These three steps were |
| 58°C | 30 s | |
| 72°C | 30 s | repeated for 35 cycles. |
| 72°C | 7 min | |
| 4°C | Hold | |

The PCR product was subjected to Sanger sequencing analysis.

### Analysis of the Crispr Cas9 knockout efficiency

The Crispr Cas9 knockout efficiency was analyzed based on the Sanger sequencing data by using the Tracking of Indels by DEcomposition (Tide) method, and the specific method could be referred to (Brinkman et al, Nucl. Acids Res. (2014) or shinyapps.datacurators.nl/tide/). By inputting the corresponding sgRNA sequence of the present invention, the pre-knockout control sequence, and the test sequence after Crispr Cas9 knockout, with the P-value threshold set to 0.001, the knockout efficiency analysis was performed.

For PBMC-derived cells, the knockout efficiency was as follows:

| Target | gRNA | Knockout efficiency of Donor 1 (%) | Knockout efficiency of Donor 2 (%) |
|---|---|---|---|
| CRP | CRP-1 | 50.4 | 78.30% |
| CRP | CRP-4 | 43 | 58.10% |
| CYLD | CYLD-2 | Not tested (No test) | 91.90% |
| CYLD | CYLD-3 | 53.3 | 79% |
| CBLIF | CBLIF-2 | 15.5 | 76.50% |
| CBLIF | CBLIF-4 | 13.1 | 29.20% |
| KLF4 | KLF4-3 | 64.5 | 54.70% |
| KLF4 | KLF4-4 | 68.8 | 85.10% |
| NDST1 | NDST1-1 | 20 | 14.20% |
| NDST1 | NDST1-3 | 82.1 | 79.90% |
| NLRP1 | NLRP1-2 | 65.1 | 69.90% |
| NLRP1 | NLRP1-3 | Not tested (No test) | 77.50% |
| SCGB1A1 | SCGB1A1-1 | 93.6 | 83.50% |
| SCGB1A1 | SCGB1A1-4 | 60.4 | 79.70% |

Among them, donors 005 and 031 were healthy donors and peripheral blood were derived from healthy donors; donor 309 was a cervical cancer patient, donor 107 was a lung cancer patient, and donor 812 was a melanoma patient.

| Target | gRNA | Donor knockout efficiency (%) |
|---|---|---|
| CRP | CRP-1 | 52.60, 80.80 |
| CRP | CRP-4 | Not tested (No test) |
| CYLD | CYLD-2 | 94.90, 92.60 |
| CYLD | CYLD-3 | Not tested (No test) |
| CBLIF | CBLIF-2 | 78.80, 82.40 |
| CBLIF | CBLIF-4 | 27.10, 26.30 |
| KLF4 | KLF4-3 | Not tested (No test) |
| KLF4 | KLF4-4 | 72.10, 88.90 |
| NDST1 | NDST1-1 | 25.10, 34.70 |
| NDST1 | NDST1-3 | 22.60, 68 |
| NLRP1 | NLRP1-2 | Not tested (No test) |
| NLRP1 | NLRP1-3 | Not tested (No test) |
| SCGB1A1 | SCGB1A1-1 | Not tested (No test) |
| SCGB1A1 | SCGB1A1-4 | 77.10, 82.20 |

The knockout efficiencies of the guide of the same sequence in different donor cells were separated by commas.

| Target | gRNA | Guide RNA Sequence | SEQ ID NO: |
|---|---|---|---|
| CRP | CRP-1 | AGTACACATTTGTACAAGCT | 49593 |
| CRP | CRP-4 | TCTGGCCAAAAGCATGAGAG | 49594 |
| CYLD | CYLD-2 | ATATTCAAGATCGTTCTGTG | 49595 |
| CYLD | CYLD-3 | TATGGGGTAATCCGTTGGAT | 49596 |
| CBLIF | CBLIF-2 | ATGTACAACAAGATCCCTGT | 49597 |
| CBLIF | CBLIF-4 | CTGATTGCCATGAATCTGGC | 49598 |
| KLF4 | KLF4-3 | GCCAGGTTGAAGGGAGCCGT | 49599 |
| KLF4 | KLF4-4 | TCGGTCATCAGCGTCAGCAA | 49600 |
| NDST1 | NDST1-1 | CGTGACGCGACCTAGCGAGG | 49601 |
| NDST1 | NDST1-3 | TCTCTGTGCGGTATTTGAAG | 49602 |
| NLRP1 | NLRP1-2 | CAGGCCCAATAGGAAACGTG | 49603 |
| NLRP1 | NLRP1-3 | GATTATGTGGAGGAGAATCG | 49604 |
| SCGB1A1 | SCGB1A1-1 | ATGGCAGCCTCATAACTGGA | 49605 |
| SCGB1A1 | SCGB1A1-4 | TGTCATCGAAACCCTCCTCA | 49606 |

The results showed that the editing modes of various genes of the present invention or combinations thereof each achieved a certain proportion of knockout efficiency.

### Example 25. Detection of the expansion of TCRT cells

### Experimental preparation

The proliferation of the gene-edited TCRT cells was detected.

TCRT cells of each group were harvested, washed once with PBS, and then resuspended in T cell medium (without IL-2). After counting, the cell density was adjusted to 1e5 to 2e6/mL, and 100 µL/well was added to a flat-bottom 96-well plate. For the CD3 antibody stimulation group, a CD3 antibody (OKT3) was added at 30 ng/ml for stimulation. For the TransACT stimulation group, transACT (with a diameter of about 100 to 500 nm, Miltenyi) was added such that the concentration of the transACT working solution was 1:1000 (v/v). For the unstimulated medium group, only an equal volume of cell culture medium was added. A CTG kit (CellTiter-Glo Luminescent Cell Viability Assay, Promega) was used to analyze the fluorescence intensity of T cells after 3 days, and the expansion efficiency of T cells was characterized by the fluorescence intensity on day 3.

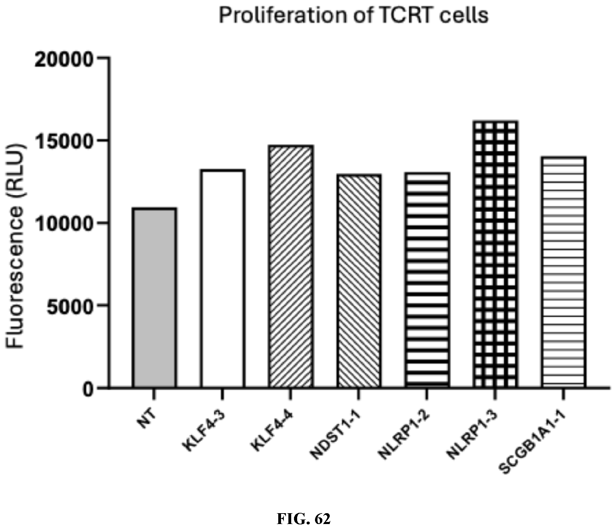

FIG. 62 showed the TCRT expansion capacity of cells subjected to single-target gene editing of KLF4, NDST1, NLRP1, and SCGB1A1.

The results indicated that, compared with the non-gene-edited control group (NT), the target gene-edited cells of the present invention could have significantly improved expansion capacity.

### Example 26. Detection of multi-round cell killing capacity

IncuCyte was used to monitor changes in fluorescence intensity of target cells A375-GFP during multi-round killing. A375-GFP cells were uniformly plated in a multiwell plate and cultured at 37°C for 4 hours, and then unedited cells or cells with knockout of the targets of the present invention were taken and added to the corresponding wells, after which IncuCyte was used to record GFP fluorescence signals, with 3 replicate wells for each group. After 24 hours of killing, co-culture supernatants (20 µL/well) were collected for CBA detection. After 96 hours of killing, A375-GFP cells were uniformly plated in a new multiwell plate and cultured at 37°C for 4 hours, and then the mixture of unedited cells or cells with knockout of the targets of the present invention and A375-GFP cells from the previous round of killing was transferred to a new 96-well plate, and IncuCyte was used to record the change curve of GFP fluorescence signals in a new round of killing.

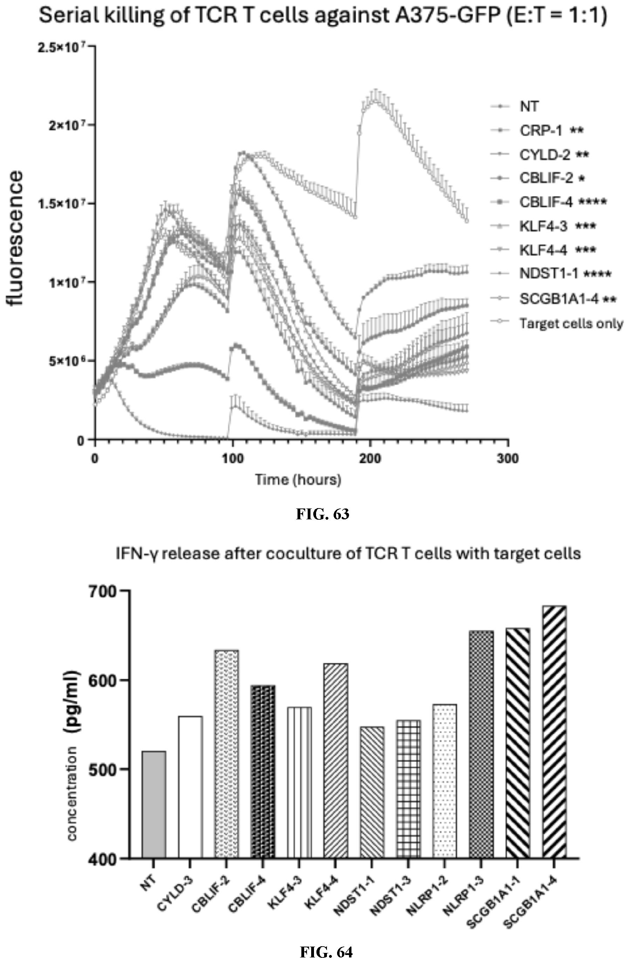

FIG. 63 showed the multi-round killing results of cells subjected to single-target gene editing of CRP, CYLD, CBLIF, KLF4, NDST1, and SCGB1A1 in TCR-T cells.

The significance differences were relative to the unedited NT group, wherein * indicated P<0.05, ** indicated P<0.01, *** indicated P<0.001, and **** indicated P<0.0001.

The results indicated that, compared with the control group (NT), the gene-edited cells of the present invention could have significantly improved multi-round killing capacity.

### Example 27. Detection of cytokine release by CBA method

For co-culture of gene-edited cells with autologous tumor cells, co-culture supernatants were collected, and a CBA kit (BD) was used to detect cytokine release of unedited cells or cells with knockout of the targets of the present invention.

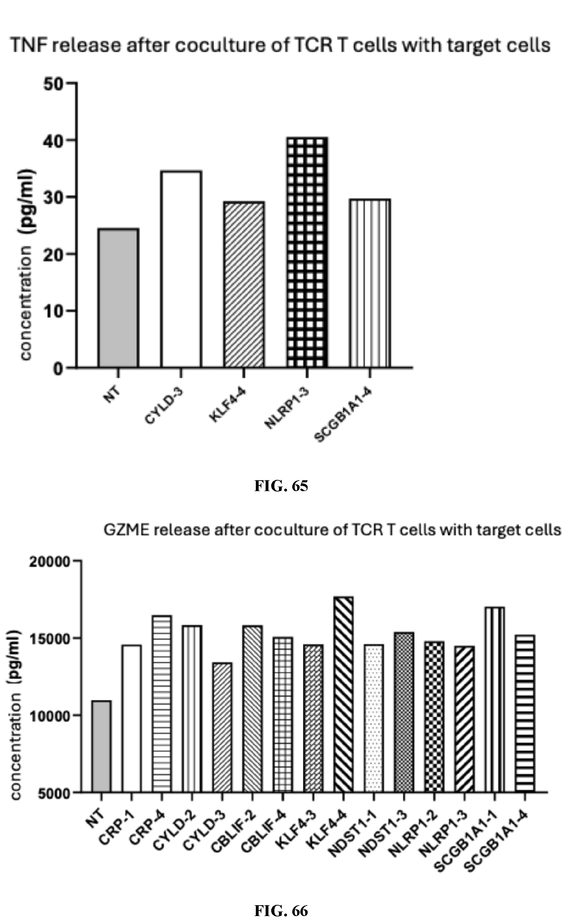

FIGs. 64, 65, and 66 showed that, in TCR-T cells, cells after single-target editing of CRP, CYLD, CBLIF, KLF4, NDST1, NLRP1, and SCGB1A1 had higher cytokine release levels.

For example, cytokine expression capacity comprised a higher IFN-γ release level, a higher TNF-α release level, or a higher GZMB release level.

The results indicated that the target gene-edited cells of the present invention had higher functional cytokine release levels.

### Example 28. Detection of the expansion of TIL cells

### Experimental preparation

The proliferation of TIL cells was detected on day 7-10 after gene editing (with IL-2 withdrawn).

TIL cells of each group were harvested, washed once with PBS, and then resuspended in T cell medium (without IL-2). After counting, the cell density was adjusted to 1e5 to 2e6/mL, and 100 µL/well was added to a flat-bottom 96-well plate. For the CD3 antibody stimulation group, a CD3 antibody (OKT3) was added at 30ng/ml for stimulation. For the TransACT stimulation group, transACT (with a diameter of about 100 to 500 nm, Miltenyi) was added such that the concentration of the transACT working solution was 1:1000 (v/v). For the unstimulated medium group, only an equal volume of cell culture medium was added. A CTG kit (CellTiter-Glo Luminescent Cell Viability Assay, Promega) was used to analyze the fluorescence intensity at the time of T-cell plating, and after 3 days the CTG kit was used to analyze the fluorescence intensity of T cells, and the expansion efficiency of T cells was characterized by the fluorescence intensity on day 3/the fluorescence intensity at the time of plating.

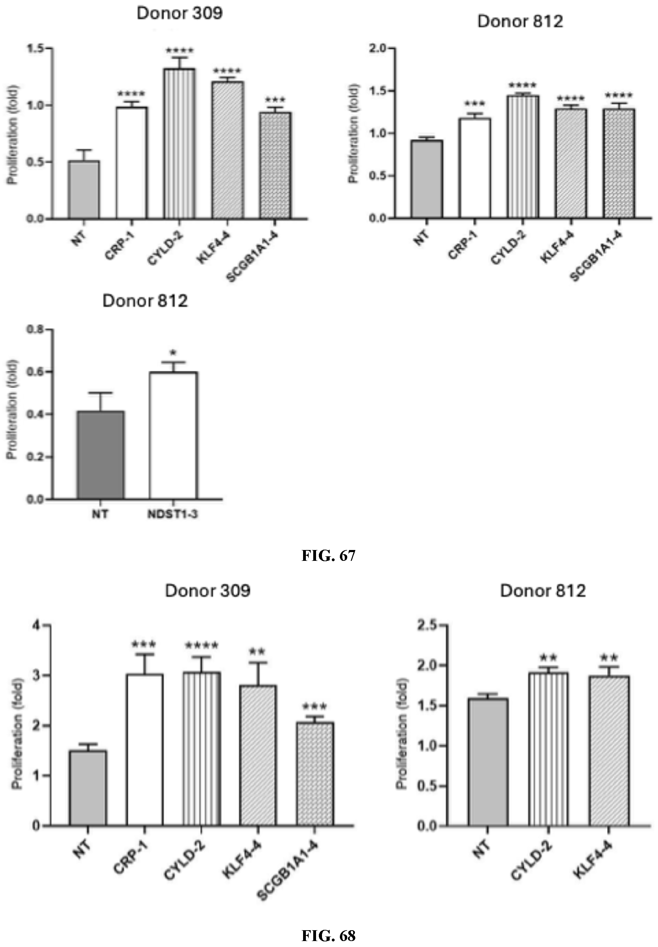

FIG. 67 showed the fold expansion of TIL subjected to single-target gene editing of CRP, CYLD, KLF4, NDST1, and SCGB1A1 in the unstimulated medium group.

FIG. 68 showed the fold expansion of TIL subjected to single-target gene editing of CRP, CYLD, KLF4, and SCGB1A1 in the TransACT stimulation group.

The significance differences were relative to the unedited NT group, wherein * indicated P<0.05, ** indicated P<0.01, *** indicated P<0.001, and **** indicated P<0.0001.

The results indicated that, compared with the non-gene-edited control group (NT), the target gene-edited cells of the present invention could have significantly improved expansion capacity.

### Example 29. Detection of cell killing capacity

Starting from day 6 after gene editing, tumor target cells were plated in a 96-well flat-bottom plate. On the next day, TIL cells of each group were co-cultured with the target cells at different effector-to-target ratios (T cells: target cells, E:T). Target cells and T cells were each 100 µL, three replicate wells were set for each group, and meanwhile a control group containing only target cells was set. The target cells could be selected from Hey-T30 ovarian cancer cells and A375 melanoma cells.

According to the instructions of an apoptosis detection reagent (Incucyte Caspase-3/7 Green Dye for Apoptosis, Sartorius), the apoptosis detection reagent was added at 0.2 µL/well, and medium was added at 25 µL/well to dilute Caspase 3/7 Green Dye. An Incucyte recorder (Sartorius) was used to record Caspase 3/7 activity to analyze the killing capacity of TIL cells against target cells, once every 3 hours, with a total recording duration of about 5 days.

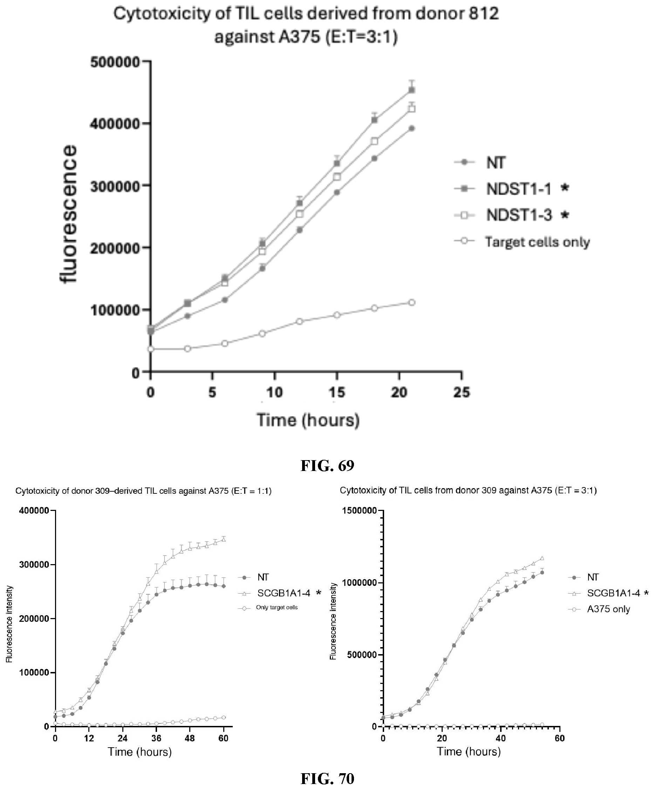

FIGs. 69, 70, and 71 showed that, in TIL cells, cells after single-target editing of CYLD, NDST1, and SCGB1A1 had higher tumor cell killing capacity.

The results indicated that, compared with the control group (NT), the target gene-edited cells of the present invention could have significantly improved target cell killing capacity.

### Detection of killing capacity in a PDO model

A PDO model was prepared with reference to the examples of the present invention and co-cultured with TIL cells. After a Caspase3/7 substrate was added for apoptosis signal labeling, the experimental plate was placed in Incucyte for observation and recording.

The results indicated that, compared with the control group (NT), the gene-edited cells of the present invention could have significantly improved cell killing capacity in the PDO model.

### Example 30. Flow cytometry detection

A flow cytometer was used to detect expression of molecules related to T-cell exhaustion, stemness, and the like in TIL cells obtained on day 8 after gene editing.

### Sources of main reagents and materials for the TIL flow cytometry test:

V-bottom 96-well plates, manufacturer Corning, catalog No. 3894; flow cytometry tubes, manufacturer Corning, catalog No. 352052; flow cytometry antibodies were purchased from BD or Biolegend.

Cell surface molecule detection: 1×10⁵ to 5×10⁵ cell samples from each group were added into flow cytometry tubes or V-bottom 96-well plates. The samples were centrifuged at 600 g for 3 minutes, and the supernatant was discarded. The samples were washed once with PBS (1 mL/tube for flow cytometry tubes, 200 µL/well for 96-well plates), and the supernatant was discarded. A prepared antibody working solution was added for cell surface staining, wherein the antibody (BD or Biolegend) concentration was 1:100 to 1:200 and a viability detection dye was included at 1:10000. Staining was performed with 100 µL/tube for flow cytometry tubes and 50 µL/well for 96-well plates, and incubation was carried out at 2-8°C for 30 minutes in the dark. After completion of surface staining, the cells were washed once with PBS (200 µL/time for 96-well plates, 1 mL/time for flow cytometry tubes), centrifuged at 600 g for 3 minutes at room temperature, and the supernatant was discarded after centrifugation. The cells were resuspended in 100-500 µL PBS and subjected to flow cytometry analysis.

Intracellular molecule detection: an antibody mixed working solution was prepared for cell surface staining of CD3/CD4/CD8, wherein the antibody concentration was 1:100 and the cell viability detection dye concentration was (1:10000). Staining was performed with 50 µL/well for 96-well plates and 100 µL/tube for flow cytometry tubes, and incubation was carried out at 2-8°C for 30 minutes in the dark. The cells were washed once with PBS (200 µL/time for 96-well plates, 1 mL/time for flow cytometry tubes), centrifuged at 600 g for 3 minutes at room temperature, and the supernatant was discarded after centrifugation. 100 µL of fixation/permeabilization solution (BD, Fixation/Permeabilization) was added to each well, and incubation was carried out at 2-8°C for 20-40 minutes in the dark. After fixation and permeabilization, washing was performed twice with 1×Perm/Wash Buffer (200 µL/time for 96-well plates, 1 mL/time for flow cytometry tubes), centrifuged at 600 g for 3 minutes, and the supernatant was discarded after centrifugation. Intracellular molecule antibodies (e.g., TCF1) were prepared with 1×Perm/Wash Buffer, and TIL cells were resuspended (50 µL/well for 96-well plates, 100 µL/tube for flow cytometry tubes for staining), followed by incubation at 2-8°C for 30 minutes in the dark. After intracellular molecule staining, washing was performed 1-2 times with 1×Perm/Wash Buffer (200 µL/time for 96-well plates, 1 mL/time for flow cytometry tubes), centrifuged at 600 g for 3 minutes, and the supernatant was discarded after centrifugation. The cells were resuspended in 100-500 µL PBS and subjected to flow cytometry analysis.

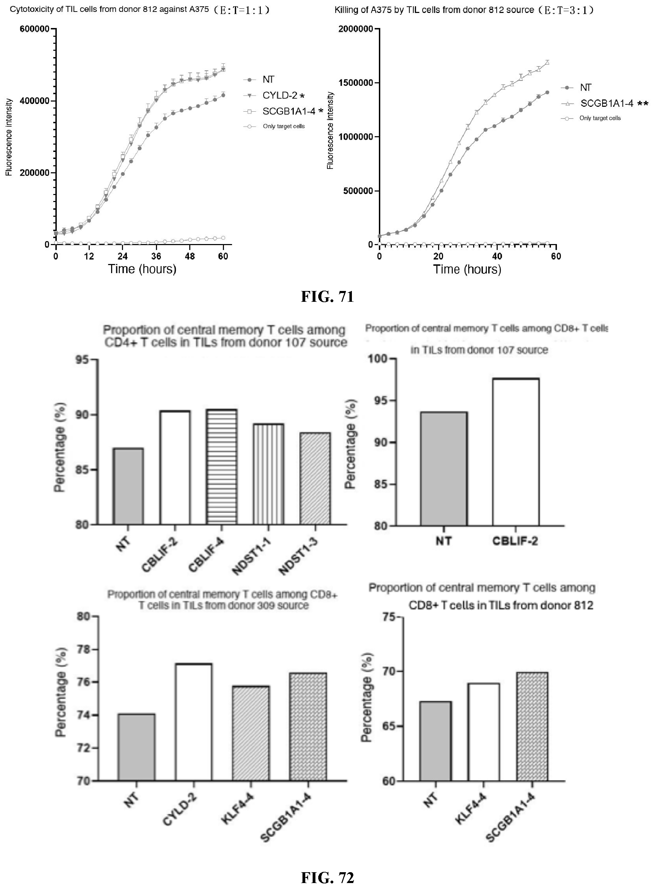

FIG. 72 showed the proportion of central memory T cells in TIL cells after editing of CBLIF, KLF4, NDST1, and SCGB1A1. For example, central memory T cells could be CD45RO-positive CD62L-positive cells.

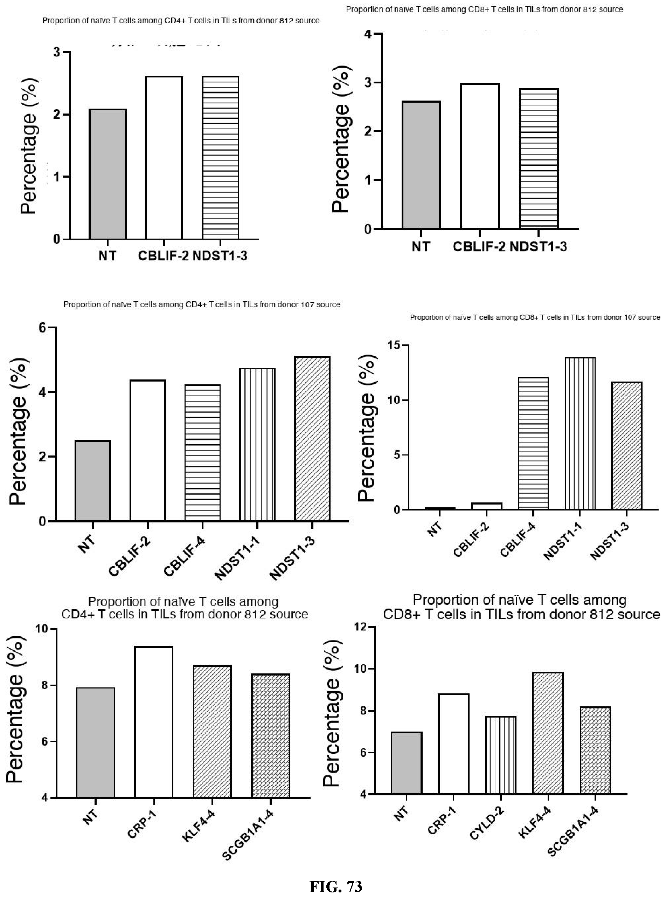

FIG. 73 showed the proportion of naive T cells in TIL cells after editing of CRP, CYLD, CBLIF, KLF4, NDST1, and SCGB1A1. For example, naive T cells could be CD45RO-negative CD62L-positive cells.

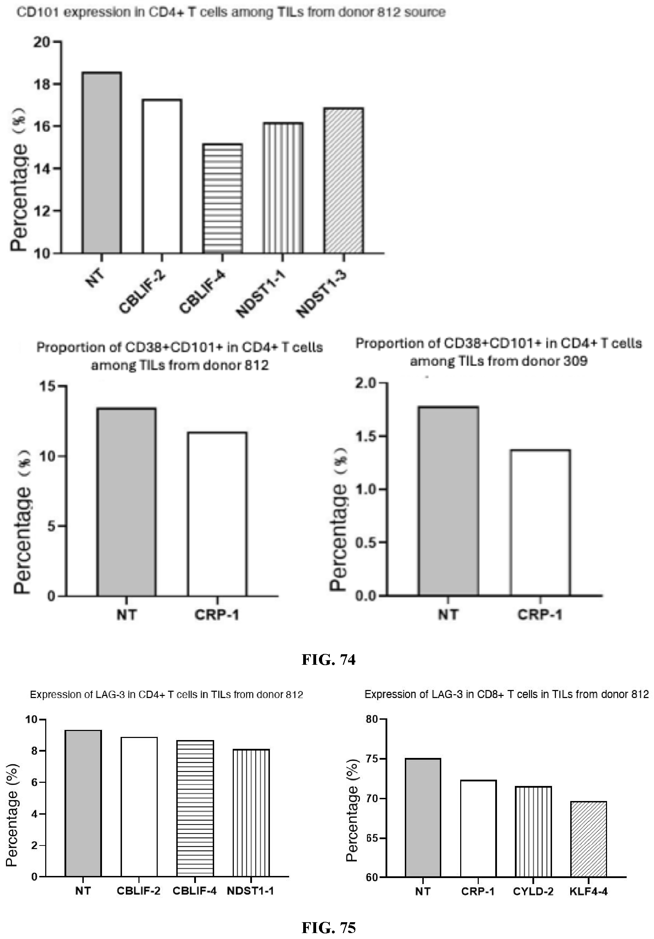

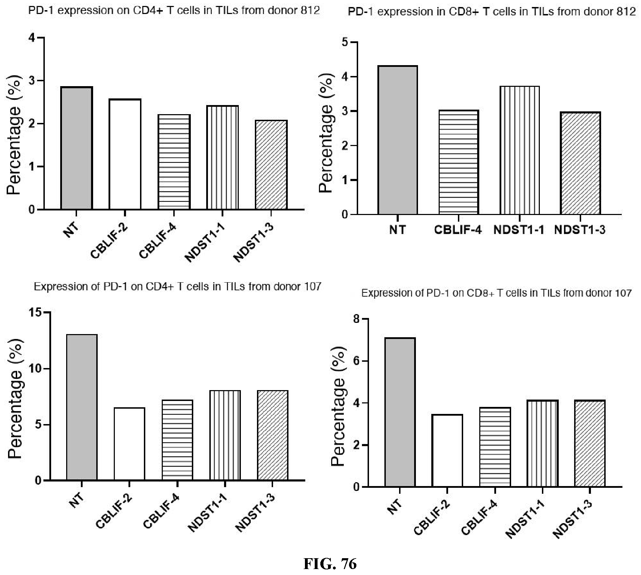

FIGs. 74, 75, and 76 showed the proportion of exhausted cells in TIL cells after editing of CRP, CYLD, CBLIF, KLF4, NDST1, and SCGB1A1. For example, exhausted T cells could be PD-1-positive, LAG-3-positive, TIM-3-positive, CD38-positive, and/or CD101-positive cells.

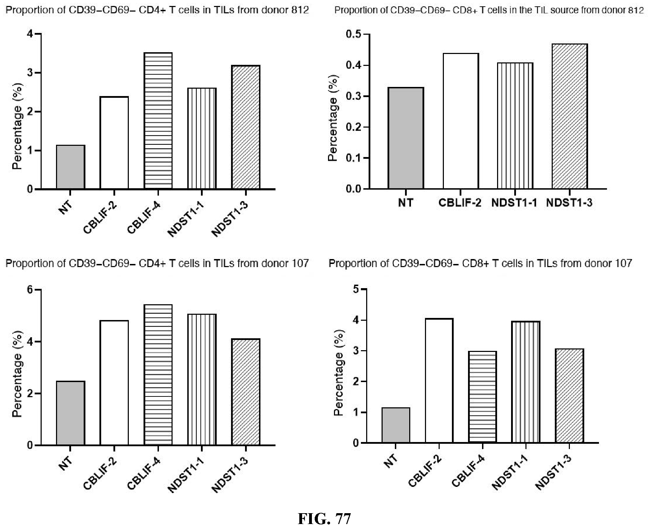

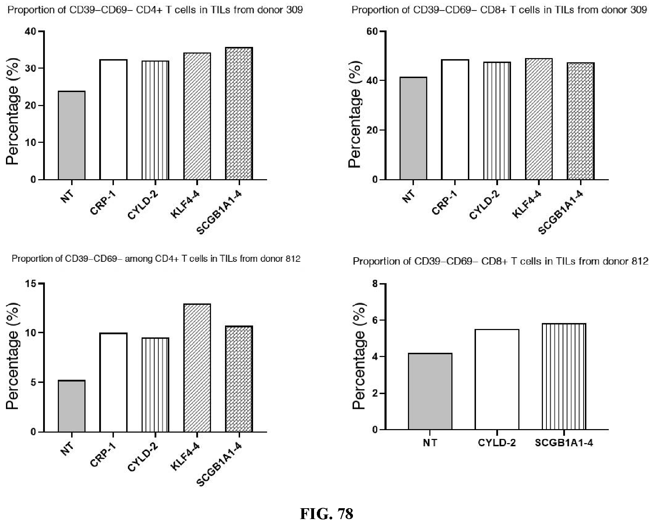

FIGs. 77 and 78 showed the proportion of stem-like T cells in TIL cells after editing of CRP, CYLD, CBLIF, KLF4, NDST1, NLRP1, and SCGB1A1. For example, stem-like T cells could have a CD39-negative CD69-negative or TCF1-positive phenotype.

The results indicated that, relative to the non-gene-edited control group (NT), the target gene-edited cells of the present invention could have more favorable cellular phenotypic characteristics.

### Example 31. Flow Cytometric Detection of Cytokine Expression

For the TIL cell populations obtained on day 7 or day 8 after gene editing in each experimental group, cytokine expression was detected using a flow cytometer.

### Experimental Preparation

A medium required for intracellular factor expression detection was prepared as follows: TIL cell culture medium was taken, and the following were added according to a volume ratio: Golgistop 0.7:1000, Golgiplug 1:1000, and CD107a antibody 1:500, i.e., 2 µL/mL. No interleukin was added.

### Detection Procedure

After the TIL cells from each experimental group were centrifuged, the TIL cells of each group were resuspended using the above medium required for intracellular factor expression detection; after counting, the cell density was adjusted to 1×10⁶ cells/mL, and the cells were added into a 96-well plate at 200 µL/well. In the CD3 antibody stimulation group, CD3 antibody (OKT3) was added at 30 ng/ml for stimulation. In the TransACT stimulation group, transACT (with a diameter of about 100 to 500 nm, Miltenyi) was added such that the working solution concentration of transACT was 1:1000 (v/v). In the unstimulated medium group, only an equal volume of cell culture medium was added. The cells were placed in a 37°C incubator and incubated overnight.

### Sources of main reagents and materials for the TIL cell cytokine flow cytometry detection experiment:

V-bottom 96-well plates were obtained from Corning, catalog number 3894; flow cytometry tubes were obtained from Corning, catalog number 352052; and flow cytometry antibodies were purchased from BD or Biolegend.

After incubation was completed, the cells were washed once with PBS at 200 µL/well, centrifuged at 600 g for 3 minutes, and the supernatant was discarded. An antibody mixed working solution was prepared for cell surface staining of CD3/CD4/CD8, wherein the antibody concentration was 1:100 and the cell viability detection dye concentration was 1:10000. Staining was performed at 50 µL/well for the 96-well plate and 100 µL/tube for the flow cytometry tubes, followed by incubation at 2-8°C in the dark for 30 minutes. The cells were washed once with PBS (200 µL/time for the 96-well plate and 1 mL/time for the flow cytometry tubes), centrifuged at 600 g for 3 minutes at room temperature, and the supernatant was discarded after centrifugation. Fixation/permeabilization solution (BD, Fixation/Permeabilization) was added at 100 µL per well, followed by incubation at 2-8°C in the dark for 20-40 minutes. After fixation and permeabilization, the cells were washed twice with 1×Perm/Wash Buffer (200 µL/time for the 96-well plate and 1 mL/time for the flow cytometry tubes), centrifuged at 600 g for 3 minutes, and the supernatant was discarded after centrifugation. Cytokine detection antibodies (e.g., GZMB, TNF-α, IFN-γ) were prepared using 1×Perm/Wash Buffer, and the TIL cells were resuspended (50 µL/well for the 96-well plate and 100 µL/tube for the flow cytometry tubes for staining), followed by incubation at 2-8°C in the dark for 30 minutes. After cytokine staining, the cells were washed 1-2 times with 1×Perm/Wash Buffer (200 µL/time for the 96-well plate and 1 mL/time for the flow cytometry tubes), centrifuged at 600 g for 3 minutes, and the supernatant was discarded after centrifugation. The cells were resuspended in 100-500 µL PBS and subjected to flow cytometric analysis.

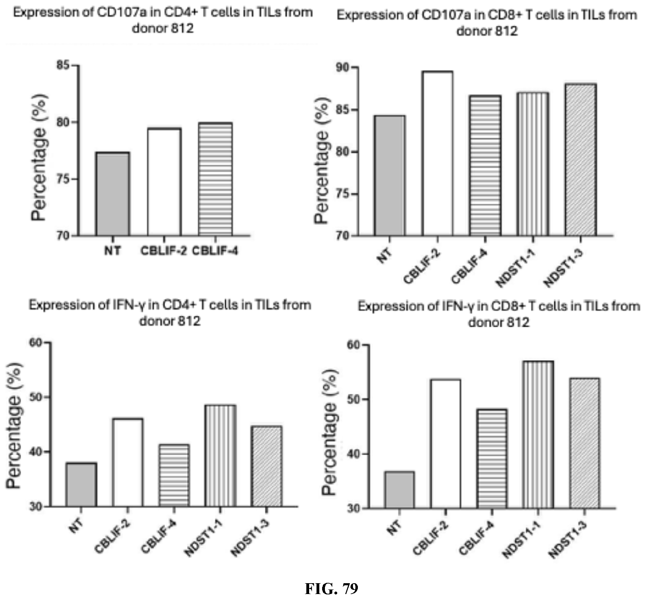

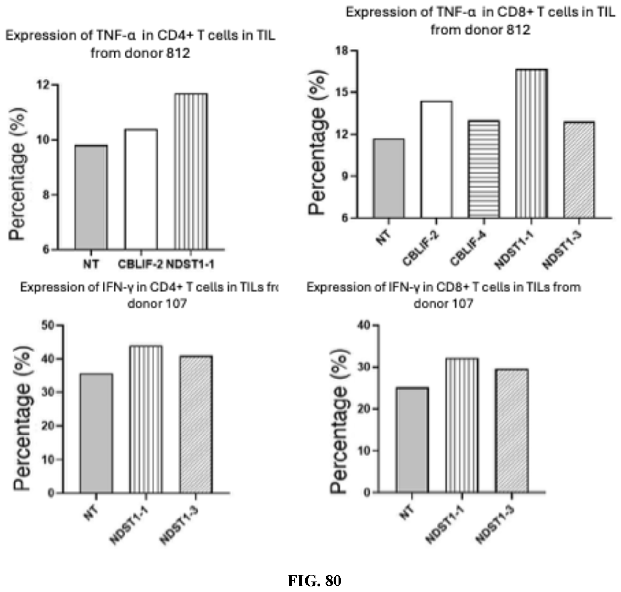

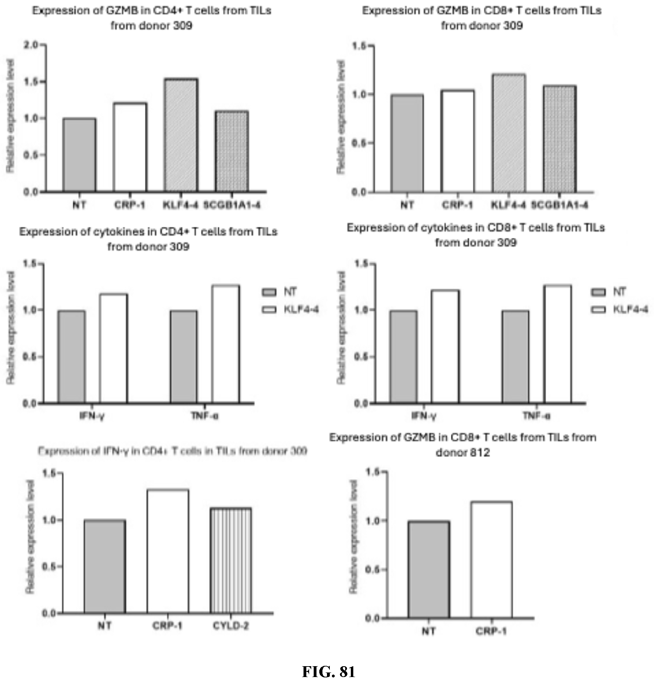

FIGs. 79, 80, and 81 show that, in the unstimulated medium group, the TIL cells edited for CRP, CYLD, CBLIF, KLF4, NDST1, NLRP1, and SCGB1A1 had a higher proportion of cytokine expression.

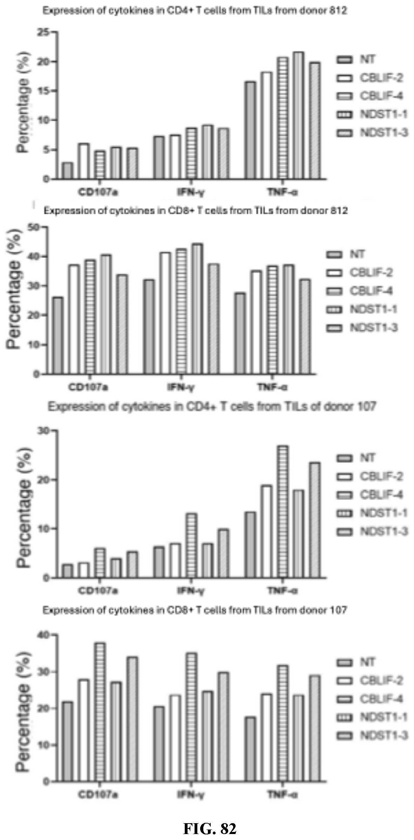

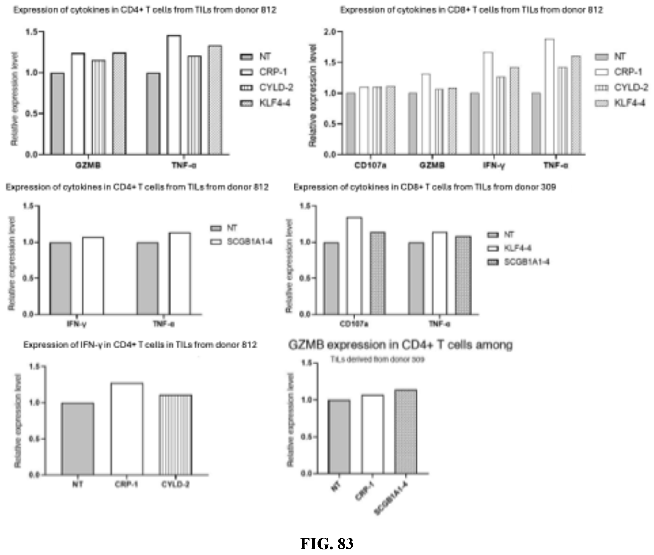

FIGs. 82 and 83 show that, in the TransACT stimulation group, the TIL cells edited for CRP, CYLD, CBLIF, KLF4, NDST1, NLRP1, and SCGB1A1 had a higher proportion of cytokine expression.

For example, the cytokine expression capability included higher CD107a expression capability, higher IFN-γ expression capability, higher TNF-α expression capability, or higher GZMB expression capability.

The results indicated that the target gene-edited cells of the present invention had higher functional cytokine expression capability.

### Example 32. In Vivo Efficacy Detection of Gene-Edited Cells

Tumor tissue samples were obtained by surgery and transferred to the laboratory under sterile conditions. Tumor necrotic tissue was excised in a sterile clean bench, and the tumor tissue was cut into a size of 1-2 mm³. The cut tumor tissue was dropwise added with a small amount of Matrigel and subcutaneously inoculated into NOG mice (Vital River, strain code 408) in an SPF-grade animal facility, with 4-5 pieces of tissue inoculated per mouse; this batch of tumor tissue was the F0 generation. When the F0 tumor tissue grew subcutaneously in the mice to 800 mm³, the F0 tumor tissue was surgically excised, and the tumor tissue was again divided into 1-2 mm³; a portion was cryopreserved for later use, and a portion was subcutaneously inoculated into a second batch of NOG mice and continuously passaged and expanded in the mice; this batch of tumor tissue was the F1 generation. By analogy, until the tumor tissue was passaged to the F3 generation in the mice, the tumor tissue size was measured periodically; when the tumor volume grew to about 100 mm³, the mice were randomly grouped. The mice in the NT group were intravenously injected with non-gene-edited cells, and the mice in the experimental group were intravenously injected with the gene-edited cells of the present invention.

The results showed that, compared with the NT group, the mice in the experimental group produced better tumor control effects and/or in vivo functional cytokine release, thereby demonstrating that the tumor-inhibiting effect of the combinatorial knockout cells of the present invention was significantly stronger than that of the NT group.

The foregoing detailed description was provided by way of explanation and examples, and was not intended to limit the scope of the appended claims. Various modifications of the embodiments of the present invention set forth herein would be apparent to those of ordinary skill in the art, and were intended to be retained within the scope of the appended claims and equivalents thereof.

**Table 1A Genomic coordinates of preferred targeted subregions of human BCL2L11**

| Region No. | Subregion coordinate range | Region No. | Subregion coordinate range |
|---|---|---|---|
| BCL2L11 R_1 | chr2:111119416-111119468 | BCL2L11 R_51 | chr2:111151745-111151790 |
| BCL2L11 R_2 | chr2:111119480-111120135 | BCL2L11 R_52 | chr2:111151798-111151918 |
| BCL2L11 R_3 | chr2:111120146-111120960 | BCL2L11 R_53 | chr2:111151942-111152394 |
| BCL2L11 R_4 | chr2:111120963-111121094 | BCL2L11 R_54 | chr2:111152398-111152441 |
| BCL2L11 R_5 | chr2:111121097-111123471 | BCL2L11 R_55 | chr2:111152465-111152644 |
| BCL2L11 R_6 | chr2:111123491-111123536 | BCL2L11 R_56 | chr2:111152649-111152812 |
| BCL2L11 R_7 | chr2:111123539-111123596 | BCL2L11 R_57 | chr2:111152827-111152897 |
| BCL2L11 R_8 | chr2:111123633-111123665 | BCL2L11 R_58 | chr2:111153660-111153687 |
| BCL2L11 R_9 | chr2:111123722-111123749 | BCL2L11 R_59 | chr2:111153706-111153768 |
| BCL2L11 R_10 | chr2:111123902-111124004 | BCL2L11 R_60 | chr2:111153807-111153905 |
| BCL2L11 R_11 | chr2:111124127-111124207 | BCL2L11 R_61 | chr2:111153918-111154026 |
| BCL2L11 R_12 | chr2:111124218-111124262 | BCL2L11 R_62 | chr2:111161298-111161347 |
| BCL2L11 R_13 | chr2:111128509-111128624 | BCL2L11 R_63 | chr2:111161356-111161402 |
| BCL2L11 R_14 | chr2:111128657-111128816 | BCL2L11 R_64 | chr2:111161422-111161671 |
| BCL2L11 R_15 | chr2:111130011-111130077 | BCL2L11 R_65 | chr2:111164017-111164069 |
| BCL2L11 R_16 | chr2:111130148-111130175 | BCL2L11 R_66 | chr2:111164071-111164144 |
| BCL2L11 R_17 | chr2:111130182-111130374 | BCL2L11 R_67 | chr2:111164187-111164245 |
| BCL2L11 R_18 | chr2:111142189-111142216 | BCL2L11 R_68 | chr2:111164248-111164454 |
| BCL2L11 R_19 | chr2:111142222-111142276 | BCL2L11 R_69 | chr2:111164460-111164518 |
| BCL2L11 R_20 | chr2:111142366-111142422 | BCL2L11 R_70 | chr2:111164532-111164578 |
| BCL2L11 R_21 | chr2:111142440-111142467 | BCL2L11 R_71 | chr2:111164590-111164738 |
| BCL2L11 R_22 | chr2:111142482-111142510 | BCL2L11 R_72 | chr2:111164755-111164850 |
| BCL2L11 R_23 | chr2:111144392-111144432 | BCL2L11 R_73 | chr2:111164858-111164930 |
| BCL2L11 R_24 | chr2:111144436-111144552 | BCL2L11 R_74 | chr2:111164958-111165011 |
| BCL2L11 R_25 | chr2:111144556-111144594 | BCL2L11 R_75 | chr2:111165013-111165040 |
| BCL2L11 R_26 | chr2:111144602-111144629 | BCL2L11 R_76 | chr2:111165067-111165123 |
| BCL2L11 R_27 | chr2:111145825-111145864 | BCL2L11 R_77 | chr2:111165128-111165172 |
| BCL2L11 R_28 | chr2:111145888-111145915 | BCL2L11 R_78 | chr2:111165197-111165712 |
| BCL2L11 R_29 | chr2:111145941-111146019 | BCL2L11 R_79 | chr2:111165758-111165785 |
| BCL2L11 R_30 | chr2:111146055-111146158 | BCL2L11 R_80 | chr2:111165814-111165875 |
| BCL2L11 R_31 | chr2:111146183-111146212 | BCL2L11 R_81 | chr2:111165924-111166046 |
| BCL2L11 R_32 | chr2:111146219-111146307 | BCL2L11 R_82 | chr2:111166057-111166502 |
| BCL2L11 R_33 | chr2:111149928-111150018 | BCL2L11 R_83 | chr2:111166514-111166695 |
| BCL2L11 R_34 | chr2:111150166-111150221 | BCL2L11 R_84 | chr2:111166720-111166783 |
| BCL2L11 R_35 | chr2:111150223-111150250 | BCL2L11 R_85 | chr2:111166800-111166830 |
| BCL2L11 R_36 | chr2:111150266-111150367 | BCL2L11 R_86 | chr2:111166842-111166892 |
| BCL2L11 R_37 | chr2:111150380-111150408 | BCL2L11 R_87 | chr2:111166914-111166973 |
| BCL2L11 R_38 | chr2:111150427-111150454 | BCL2L11 R_88 | chr2:111166989-111167016 |
| BCL2L11 R_39 | chr2:111150462-111150514 | BCL2L11 R_89 | chr2:111167055-111167082 |
| BCL2L11 R_40 | chr2:111150525-111150553 | BCL2L11 R_90 | chr2:111167145-111167298 |
| BCL2L11 R_41 | chr2:111150561-111150724 | BCL2L11 R_91 | chr2:111167303-111167360 |
| BCL2L11 R_42 | chr2:111150739-111150766 | BCL2L11 R_92 | chr2:111167373-111167502 |
| BCL2L11 R_43 | chr2:111150782-111150890 | BCL2L11 R_93 | chr2:111167523-111167697 |
| BCL2L11 R_44 | chr2:111150903-111151303 | BCL2L11 R_94 | chr2:111167701-111167756 |
| BCL2L11 R_45 | chr2:111151326-111151405 | BCL2L11 R_95 | chr2:111167772-111168023 |
| BCL2L11 R_46 | chr2:111151440-111151469 | BCL2L11 R_96 | chr2:111168027-111168056 |
| BCL2L11 R_47 | chr2:111151492-111151563 | BCL2L11 R_97 | chr2:111168090-111168131 |
| BCL2L11 R_48 | chr2:111151570-111151618 | BCL2L11 R_98 | chr2:111168168-111168241 |
| BCL2L11 R_49 | chr2:111151623-111151673 | BCL2L11 R_99 | chr2:111168246-111168347 |
| BCL2L11 R_50 | chr2:111151679-111151743 | BCL2L11 R_100 | chr2:111168354-111168381 |

**Table 1B Genomic coordinates of preferred targeted subregions of human PTPN2**

| Region No. | Subregion coordinate range | Region No. | Subregion coordinate range |
|---|---|---|---|
| PTPN2 R_1 | chr18:12785503-12785535 | PTPN2 R_54 | chr18:12842295-12842379 |
| PTPN2 R_2 | chr18:12785557-12785586 | PTPN2 R_55 | chr18:12842408-12842489 |
| PTPN2 R_3 | chr18:12785604-12785781 | PTPN2 R_56 | chr18:12859082-12859177 |
| PTPN2 R_4 | chr18:12785800-12785871 | PTPN2 R_57 | chr18:12859254-12859281 |
| PTPN2 R_5 | chr18:12785887-12785949 | PTPN2 R_58 | chr18:12859284-12859383 |
| PTPN2 R_6 | chr18:12792182-12792231 | PTPN2 R_59 | chr18:12859392-12859470 |
| PTPN2 R_7 | chr18:12792246-12792294 | PTPN2 R_60 | chr18:12859479-12859583 |
| PTPN2 R_8 | chr18:12792301-12793116 | PTPN2 R_61 | chr18:12859596-12859655 |
| PTPN2 R_9 | chr18:12793124-12793168 | PTPN2 R_62 | chr18:12859660-12859748 |
| PTPN2 R_10 | chr18:12793225-12793252 | PTPN2 R_63 | chr18:12859777-12859815 |
| PTPN2 R_11 | chr18:12793371-12793435 | PTPN2 R_64 | chr18:12859826-12860284 |
| PTPN2 R_12 | chr18:12793447-12793535 | PTPN2 R_65 | chr18:12860291-12860596 |
| PTPN2 R_13 | chr18:12793541-12793570 | PTPN2 R_66 | chr18:12860599-12860757 |
| PTPN2 R_14 | chr18:12793621-12793664 | PTPN2 R_67 | chr18:12862516-12862794 |
| PTPN2 R_15 | chr18:12793674-12793701 | PTPN2 R_68 | chr18:12862798-12862861 |
| PTPN2 R_16 | chr18:12793791-12793819 | PTPN2 R_69 | chr18:12863080-12863140 |
| PTPN2 R_17 | chr18:12793823-12793851 | PTPN2 R_70 | chr18:12863142-12863386 |
| PTPN2 R_18 | chr18:12793878-12793927 | PTPN2 R_71 | chr18:12863408-12863794 |
| PTPN2 R_19 | chr18:12793935-12793990 | PTPN2 R_72 | chr18:12863816-12863871 |
| PTPN2 R_20 | chr18:12794035-12794157 | PTPN2 R_73 | chr18:12863880-12863942 |
| PTPN2 R_21 | chr18:12794160-12794205 | PTPN2 R_74 | chr18:12863949-12863976 |
| PTPN2 R_22 | chr18:12794214-12794285 | PTPN2 R_75 | chr18:12863990-12864032 |
| PTPN2 R_23 | chr18:12794308-12794410 | PTPN2 R_76 | chr18:12864038-12864163 |
| PTPN2 R_24 | chr18:12794504-12794607 | PTPN2 R_77 | chr18:12864167-12864194 |
| PTPN2 R_25 | chr18:12801862-12801933 | PTPN2 R_78 | chr18:12864216-12864253 |
| PTPN2 R_26 | chr18:12801935-12801962 | PTPN2 R_79 | chr18:12864265-12864773 |
| PTPN2 R_27 | chr18:12802204-12802231 | PTPN2 R_80 | chr18:12864775-12864822 |
| PTPN2 R_28 | chr18:12802234-12802261 | PTPN2 R_81 | chr18:12864832-12864876 |
| PTPN2 R_29 | chr18:12814086-12814135 | PTPN2 R_82 | chr18:12864925-12864988 |
| PTPN2 R_30 | chr18:12814368-12814426 | PTPN2 R_83 | chr18:12865007-12865034 |
| PTPN2 R_31 | chr18:12814454-12814481 | PTPN2 R_84 | chr18:12865063-12865092 |
| PTPN2 R_32 | chr18:12817038-12817119 | PTPN2 R_85 | chr18:12865108-12865365 |
| PTPN2 R_33 | chr18:12817358-12817388 | PTPN2 R_86 | chr18:12865382-12865851 |
| PTPN2 R_34 | chr18:12817442-12817488 | PTPN2 R_87 | chr18:12865860-12865887 |
| PTPN2 R_35 | chr18:12819088-12819173 | PTPN2 R_88 | chr18:12865896-12865985 |
| PTPN2 R_36 | chr18:12819302-12819332 | PTPN2 R_89 | chr18:12866004-12866069 |
| PTPN2 R_37 | chr18:12819342-12819394 | PTPN2 R_90 | chr18:12866135-12866458 |
| PTPN2 R_38 | chr18:12825712-12825739 | PTPN2 R_91 | chr18:12866480-12866585 |
| PTPN2 R_39 | chr18:12825766-12825809 | PTPN2 R_92 | chr18:12866590-12866639 |
| PTPN2 R_40 | chr18:12825998-12826051 | PTPN2 R_93 | chr18:12866652-12866979 |
| PTPN2 R_41 | chr18:12830817-12830898 | PTPN2 R_94 | chr18:12883946-12884060 |
| PTPN2 R_42 | chr18:12831040-12831154 | PTPN2 R_95 | chr18:12884154-12884234 |
| PTPN2 R_43 | chr18:12836684-12836775 | PTPN2 R_96 | chr18:12884248-12884853 |
| PTPN2 R_44 | chr18:12836913-12836965 | PTPN2 R_97 | chr18:12884886-12884922 |
| PTPN2 R_45 | chr18:12840587-12840646 | PTPN2 R_98 | chr18:12884969-12885129 |
| PTPN2 R_46 | chr18:12840686-12840713 | PTPN2 R_99 | chr18:12885150-12885215 |
| PTPN2 R_47 | chr18:12840806-12840977 | PTPN2 R_100 | chr18:12885223-12885284 |
| PTPN2 R_48 | chr18:12840988-12841058 | PTPN2 R_101 | chr18:12885333-12885360 |
| PTPN2 R_49 | chr18:12841079-12841379 | PTPN2 R_102 | chr18:12885368-12885450 |
| PTPN2 R_50 | chr18:12841401-12841593 | PTPN2 R_103 | chr18:12885456-12885493 |
| PTPN2 R_51 | chr18:12841603-12841699 | PTPN2 R_104 | chr18:12885506-12885538 |
| PTPN2 R_52 | chr18:12841713-12841865 | PTPN2 R_105 | chr18:12885567-12885679 |
| PTPN2 R_53 | chr18:12841903-12842292 | PTPN2 R_106 | chr18:12885688-12885738 |

**Table 1C Genomic coordinates of preferred targeted subregions of human AFF3**

| Region No. | Subregion coordinate range | Region No. | Subregion coordinate range |
|---|---|---|---|
| AFF3 R_1 | chr2:99545444-99545550 | AFF3 R_188 | chr2:100006618-100006856 |
| AFF3 R_2 | chr2:99545563-99545705 | AFF3 R_189 | chr2:100006867-100007102 |
| AFF3 R_3 | chr2:99545735-99545878 | AFF3 R_190 | chr2:100007131-100007282 |
| AFF3 R_4 | chr2:99545892-99545954 | AFF3 R_191 | chr2:100007298-100007381 |
| AFF3 R_5 | chr2:99545971-99546096 | AFF3 R_192 | chr2:100007401-100007478 |
| AFF3 R_6 | chr2:99546105-99546216 | AFF3 R_193 | chr2:100007481-100007575 |
| AFF3 R_7 | chr2:99546227-99546255 | AFF3 R_194 | chr2:100008687-100008783 |
| AFF3 R_8 | chr2:99546272-99546305 | AFF3 R_195 | chr2:100008792-100008838 |
| AFF3 R_9 | chr2:99546315-99546553 | AFF3 R_196 | chr2:100008855-100008889 |
| AFF3 R_10 | chr2:99546563-99546615 | AFF3 R_197 | chr2:100008895-100008942 |
| AFF3 R_11 | chr2:99546634-99546723 | AFF3 R_198 | chr2:100008961-100009056 |
| AFF3 R_12 | chr2:99546734-99546813 | AFF3 R_199 | chr2:100011370-100011698 |
| AFF3 R_13 | chr2:99546818-99546943 | AFF3 R_200 | chr2:100053031-100053058 |
| AFF3 R_14 | chr2:99546968-99546995 | AFF3 R_201 | chr2:100053064-100053108 |
| AFF3 R_15 | chr2:99547053-99547091 | AFF3 R_202 | chr2:100053133-100053198 |
| AFF3 R_16 | chr2:99547105-99547149 | AFF3 R_203 | chr2:100053209-100053279 |
| AFF3 R_17 | chr2:99547152-99547179 | AFF3 R_204 | chr2:100053284-100053334 |
| AFF3 R_18 | chr2:99547191-99547221 | AFF3 R_205 | chr2:100053337-100053486 |
| AFF3 R_19 | chr2:99547271-99547359 | AFF3 R_206 | chr2:100053504-100053580 |
| AFF3 R_20 | chr2:99547372-99547460 | AFF3 R_207 | chr2:100053588-100053873 |
| AFF3 R_21 | chr2:99547484-99547538 | AFF3 R_208 | chr2:100053877-100054140 |
| AFF3 R_22 | chr2:99547594-99547621 | AFF3 R_209 | chr2:100054148-100054336 |
| AFF3 R_23 | chr2:99547657-99547739 | AFF3 R_210 | chr2:100054342-100054469 |
| AFF3 R_24 | chr2:99547776-99547805 | AFF3 R_211 | chr2:100054478-100054630 |
| AFF3 R_25 | chr2:99547837-99547864 | AFF3 R_212 | chr2:100054641-100055096 |
| AFF3 R_26 | chr2:99547902-99547929 | AFF3 R_213 | chr2:100055117-100055375 |
| AFF3 R_27 | chr2:99547949-99548069 | AFF3 R_214 | chr2:100055378-100055478 |
| AFF3 R_28 | chr2:99548086-99548225 | AFF3 R_215 | chr2:100055483-100055719 |
| AFF3 R_29 | chr2:99548229-99548256 | AFF3 R_216 | chr2:100055745-100055773 |
| AFF3 R_30 | chr2:99548266-99548372 | AFF3 R_217 | chr2:100055786-100055858 |
| AFF3 R_31 | chr2:99548413-99548440 | AFF3 R_218 | chr2:100055863-100056050 |
| AFF3 R_32 | chr2:99548442-99548794 | AFF3 R_219 | chr2:100056082-100056115 |
| AFF3 R_33 | chr2:99548803-99548837 | AFF3 R_220 | chr2:100056165-100056295 |
| AFF3 R_34 | chr2:99548845-99548932 | AFF3 R_221 | chr2:100056298-100056353 |
| AFF3 R_35 | chr2:99548935-99549056 | AFF3 R_222 | chr2:100056373-100056473 |
| AFF3 R_36 | chr2:99549070-99549195 | AFF3 R_223 | chr2:100056477-100056514 |
| AFF3 R_37 | chr2:99549206-99549272 | AFF3 R_224 | chr2:100056517-100056602 |
| AFF3 R_38 | chr2:99549276-99549449 | AFF3 R_225 | chr2:100056614-100056686 |
| AFF3 R_39 | chr2:99549452-99549725 | AFF3 R_226 | chr2:100056709-100057257 |
| AFF3 R_40 | chr2:99549751-99549778 | AFF3 R_227 | chr2:100057270-100057320 |
| AFF3 R_41 | chr2:99549787-99549829 | AFF3 R_228 | chr2:100057332-100057445 |
| AFF3 R_42 | chr2:99549840-99549872 | AFF3 R_229 | chr2:100057456-100057483 |
| AFF3 R_43 | chr2:99549895-99550061 | AFF3 R_230 | chr2:100057525-100057610 |
| AFF3 R_44 | chr2:99550077-99550110 | AFF3 R_231 | chr2:100057621-100057683 |
| AFF3 R_45 | chr2:99550197-99550305 | AFF3 R_232 | chr2:100057690-100057722 |
| AFF3 R_46 | chr2:99550314-99550634 | AFF3 R_233 | chr2:100057730-100057791 |
| AFF3 R_47 | chr2:99550648-99550967 | AFF3 R_234 | chr2:100057796-100057834 |
| AFF3 R_48 | chr2:99550977-99551114 | AFF3 R_235 | chr2:100057856-100057945 |
| AFF3 R_49 | chr2:99551127-99551154 | AFF3 R_236 | chr2:100057979-100058091 |
| AFF3 R_50 | chr2:99551188-99551251 | AFF3 R_237 | chr2:100058123-100058308 |
| AFF3 R_51 | chr2:99551264-99551294 | AFF3 R_238 | chr2:100058324-100058352 |
| AFF3 R_52 | chr2:99551306-99551358 | AFF3 R_239 | chr2:100058357-100058391 |
| AFF3 R_53 | chr2:99551363-99551395 | AFF3 R_240 | chr2:100058432-100058467 |
| AFF3 R_54 | chr2:99551411-99551605 | AFF3 R_241 | chr2:100058476-100058586 |
| AFF3 R_55 | chr2:99551612-99551702 | AFF3 R_242 | chr2:100058590-100058686 |
| AFF3 R_56 | chr2:99554212-99554724 | AFF3 R_243 | chr2:100058701-100058788 |
| AFF3 R_57 | chr2:99554751-99554850 | AFF3 R_244 | chr2:100058819-100058856 |
| AFF3 R_58 | chr2:99558753-99558845 | AFF3 R_245 | chr2:100058862-100058890 |
| AFF3 R_59 | chr2:99558874-99558917 | AFF3 R_246 | chr2:100058898-100058925 |
| AFF3 R_60 | chr2:99558919-99559020 | AFF3 R_247 | chr2:100058944-100059286 |
| AFF3 R_61 | chr2:99559028-99559055 | AFF3 R_248 | chr2:100059298-100059370 |
| AFF3 R_62 | chr2:99559060-99559087 | AFF3 R_249 | chr2:100059386-100059548 |
| AFF3 R_63 | chr2:99560251-99560357 | AFF3 R_250 | chr2:100059576-100059613 |
| AFF3 R_64 | chr2:99560364-99560463 | AFF3 R_251 | chr2:100059619-100059948 |
| AFF3 R_65 | chr2:99560503-99560530 | AFF3 R_252 | chr2:100059984-100060152 |
| AFF3 R_66 | chr2:99565360-99565410 | AFF3 R_253 | chr2:100060170-100060524 |
| AFF3 R_67 | chr2:99565425-99565589 | AFF3 R_254 | chr2:100060618-100060647 |
| AFF3 R_68 | chr2:99565605-99565679 | AFF3 R_255 | chr2:100060676-100060782 |
| AFF3 R_69 | chr2:99565699-99565741 | AFF3 R_256 | chr2:100060791-100060818 |
| AFF3 R_70 | chr2:99568727-99568845 | AFF3 R_257 | chr2:100060835-100061066 |
| AFF3 R_71 | chr2:99568851-99568950 | AFF3 R_258 | chr2:100061073-100061400 |
| AFF3 R_72 | chr2:99568975-99569030 | AFF3 R_259 | chr2:100061404-100061617 |
| AFF3 R_73 | chr2:99578203-99578328 | AFF3 R_260 | chr2:100061635-100061708 |
| AFF3 R_74 | chr2:99578341-99578431 | AFF3 R_261 | chr2:100061735-100061796 |
| AFF3 R_75 | chr2:99578460-99578542 | AFF3 R_262 | chr2:100061817-100061911 |
| AFF3 R_76 | chr2:99582699-99582956 | AFF3 R_263 | chr2:100061919-100062014 |
| AFF3 R_77 | chr2:99582960-99583052 | AFF3 R_264 | chr2:100062016-100062340 |
| AFF3 R_78 | chr2:99583060-99583117 | AFF3 R_265 | chr2:100062356-100062447 |
| AFF3 R_79 | chr2:99587044-99587404 | AFF3 R_266 | chr2:100062449-100062693 |
| AFF3 R_80 | chr2:99593077-99593357 | AFF3 R_267 | chr2:100062697-100062811 |
| AFF3 R_81 | chr2:99593359-99594232 | AFF3 R_268 | chr2:100062817-100062899 |
| AFF3 R_82 | chr2:99594248-99594411 | AFF3 R_269 | chr2:100062938-100063322 |
| AFF3 R_83 | chr2:99601308-99601339 | AFF3 R_270 | chr2:100063361-100063388 |
| AFF3 R_84 | chr2:99601342-99601535 | AFF3 R_271 | chr2:100063409-100063441 |
| AFF3 R_85 | chr2:99601547-99601733 | AFF3 R_272 | chr2:100063527-100063603 |
| AFF3 R_86 | chr2:99649507-99649541 | AFF3 R_273 | chr2:100063610-100064110 |
| AFF3 R_87 | chr2:99649543-99649588 | AFF3 R_274 | chr2:100064117-100064144 |
| AFF3 R_88 | chr2:99649616-99649690 | AFF3 R_275 | chr2:100064179-100064342 |
| AFF3 R_89 | chr2:99649748-99649778 | AFF3 R_276 | chr2:100064361-100064430 |
| AFF3 R_90 | chr2:99672283-99672368 | AFF3 R_277 | chr2:100064491-100064518 |
| AFF3 R_91 | chr2:99672390-99672533 | AFF3 R_278 | chr2:100064526-100064571 |
| AFF3 R_92 | chr2:99672537-99672636 | AFF3 R_279 | chr2:100064578-100064734 |
| AFF3 R_93 | chr2:99672655-99672696 | AFF3 R_280 | chr2:100064754-100064827 |
| AFF3 R_94 | chr2:99672716-99672776 | AFF3 R_281 | chr2:100064874-100064992 |
| AFF3 R_95 | chr2:99672779-99672825 | AFF3 R_282 | chr2:100064996-100065023 |
| AFF3 R_96 | chr2:99672827-99672855 | AFF3 R_283 | chr2:100065032-100065070 |
| AFF3 R_97 | chr2:99672877-99672951 | AFF3 R_284 | chr2:100065073-100065126 |
| AFF3 R_98 | chr2:99672962-99673048 | AFF3 R_285 | chr2:100065130-100065209 |
| AFF3 R_99 | chr2:99673051-99673194 | AFF3 R_286 | chr2:100065241-100065268 |
| AFF3 R_100 | chr2:99673205-99673407 | AFF3 R_287 | chr2:100065282-100065331 |
| AFF3 R_101 | chr2:99673455-99673482 | AFF3 R_288 | chr2:100065450-100065478 |
| AFF3 R_102 | chr2:99673561-99673880 | AFF3 R_289 | chr2:100065480-100065507 |
| AFF3 R_103 | chr2:99673888-99673977 | AFF3 R_290 | chr2:100065514-100065542 |
| AFF3 R_104 | chr2:99682345-99682663 | AFF3 R_291 | chr2:100065592-100065660 |
| AFF3 R_105 | chr2:99682672-99682945 | AFF3 R_292 | chr2:100065664-100065750 |
| AFF3 R_106 | chr2:99682978-99683139 | AFF3 R_293 | chr2:100065808-100065835 |
| AFF3 R_107 | chr2:99683148-99683343 | AFF3 R_294 | chr2:100065881-100065908 |
| AFF3 R_108 | chr2:99683353-99683380 | AFF3 R_295 | chr2:100065927-100065963 |
| AFF3 R_109 | chr2:99683391-99683424 | AFF3 R_296 | chr2:100065994-100066035 |
| AFF3 R_110 | chr2:99683432-99683467 | AFF3 R_297 | chr2:100066043-100066093 |
| AFF3 R_111 | chr2:99683470-99683516 | AFF3 R_298 | chr2:100066105-100066151 |
| AFF3 R_112 | chr2:99683518-99683746 | AFF3 R_299 | chr2:100066200-100066252 |
| AFF3 R_113 | chr2:99683762-99683847 | AFF3 R_300 | chr2:100066271-100066365 |
| AFF3 R_114 | chr2:99683849-99683895 | AFF3 R_301 | chr2:100066372-100066417 |
| AFF3 R_115 | chr2:99683904-99683974 | AFF3 R_302 | chr2:100066421-100066545 |
| AFF3 R_116 | chr2:99683990-99684257 | AFF3 R_303 | chr2:100066590-100066667 |
| AFF3 R_117 | chr2:99684261-99684440 | AFF3 R_304 | chr2:100066681-100066708 |
| AFF3 R_118 | chr2:99684454-99684508 | AFF3 R_305 | chr2:100066738-100066765 |
| AFF3 R_119 | chr2:99684528-99684826 | AFF3 R_306 | chr2:100066767-100066854 |
| AFF3 R_120 | chr2:99684837-99684890 | AFF3 R_307 | chr2:100066863-100066910 |
| AFF3 R_121 | chr2:99684898-99685009 | AFF3 R_308 | chr2:100066918-100066962 |
| AFF3 R_122 | chr2:99685014-99685323 | AFF3 R_309 | chr2:100066967-100066994 |
| AFF3 R_123 | chr2:99685336-99685404 | AFF3 R_310 | chr2:100067001-100067092 |
| AFF3 R_124 | chr2:99685428-99685560 | AFF3 R_311 | chr2:100067112-100067168 |
| AFF3 R_125 | chr2:99685608-99685662 | AFF3 R_312 | chr2:100067171-100067210 |
| AFF3 R_126 | chr2:99685689-99685738 | AFF3 R_313 | chr2:100067231-100067325 |
| AFF3 R_127 | chr2:99685764-99686294 | AFF3 R_314 | chr2:100067332-100067393 |
| AFF3 R_128 | chr2:99686308-99686344 | AFF3 R_315 | chr2:100067409-100067438 |
| AFF3 R_129 | chr2:99686346-99686462 | AFF3 R_316 | chr2:100067446-100067497 |
| AFF3 R_130 | chr2:99686466-99686627 | AFF3 R_317 | chr2:100067511-100067542 |
| AFF3 R_131 | chr2:99686631-99686929 | AFF3 R_318 | chr2:100067576-100067621 |
| AFF3 R_132 | chr2:99686943-99687240 | AFF3 R_319 | chr2:100067662-100067689 |
| AFF3 R_133 | chr2:99687244-99687700 | AFF3 R_320 | chr2:100067693-100067742 |
| AFF3 R_134 | chr2:99687725-99687763 | AFF3 R_321 | chr2:100067744-100067831 |
| AFF3 R_135 | chr2:99687765-99688192 | AFF3 R_322 | chr2:100067834-100067890 |
| AFF3 R_136 | chr2:99688201-99688547 | AFF3 R_323 | chr2:100067898-100068003 |
| AFF3 R_137 | chr2:99688566-99688608 | AFF3 R_324 | chr2:100068007-100068697 |
| AFF3 R_138 | chr2:99688620-99688710 | AFF3 R_325 | chr2:100104275-100104702 |
| AFF3 R_139 | chr2:99688712-99689092 | AFF3 R_326 | chr2:100104718-100105612 |
| AFF3 R_140 | chr2:99689096-99689152 | AFF3 R_327 | chr2:100105615-100106239 |
| AFF3 R_141 | chr2:99689160-99689338 | AFF3 R_328 | chr2:100106295-100106399 |
| AFF3 R_142 | chr2:99689343-99689468 | AFF3 R_329 | chr2:100106403-100106438 |
| AFF3 R_143 | chr2:99689472-99689551 | AFF3 R_330 | chr2:100106467-100106513 |
| AFF3 R_144 | chr2:99689563-99689590 | AFF3 R_331 | chr2:100106520-100106986 |
| AFF3 R_145 | chr2:99689601-99689654 | AFF3 R_332 | chr2:100106989-100107114 |
| AFF3 R_146 | chr2:99689680-99689785 | AFF3 R_333 | chr2:100107120-100107171 |
| AFF3 R_147 | chr2:99689823-99690086 | AFF3 R_334 | chr2:100107175-100107222 |
| AFF3 R_148 | chr2:99690101-99690174 | AFF3 R_335 | chr2:100107228-100107302 |
| AFF3 R_149 | chr2:99690199-99690633 | AFF3 R_336 | chr2:100107311-100107375 |
| AFF3 R_150 | chr2:99690673-99690793 | AFF3 R_337 | chr2:100107382-100107449 |
| AFF3 R_151 | chr2:99690808-99691011 | AFF3 R_338 | chr2:100107522-100107956 |
| AFF3 R_152 | chr2:99691013-99691180 | AFF3 R_339 | chr2:100107960-100108032 |
| AFF3 R_153 | chr2:99707002-99707148 | AFF3 R_340 | chr2:100108048-100108717 |
| AFF3 R_154 | chr2:99707159-99707298 | AFF3 R_341 | chr2:100108727-100108810 |
| AFF3 R_155 | chr2:99707353-99707407 | AFF3 R_342 | chr2:100108816-100108843 |
| AFF3 R_156 | chr2:99707414-99707476 | AFF3 R_343 | chr2:100108847-100108897 |
| AFF3 R_157 | chr2:99707481-99707525 | AFF3 R_344 | chr2:100108907-100109023 |
| AFF3 R_158 | chr2:99707529-99707605 | AFF3 R_345 | chr2:100109025-100109145 |
| AFF3 R_159 | chr2:99707619-99707690 | AFF3 R_346 | chr2:100109156-100109197 |
| AFF3 R_160 | chr2:99707694-99707751 | AFF3 R_347 | chr2:100109202-100109229 |
| AFF3 R_161 | chr2:99707759-99707821 | AFF3 R_348 | chr2:100109239-100109427 |
| AFF3 R_162 | chr2:99707829-99707991 | AFF3 R_349 | chr2:100109443-100109504 |
| AFF3 R_163 | chr2:99707993-99708051 | AFF3 R_350 | chr2:100109506-100109656 |
| AFF3 R_164 | chr2:99708080-99708136 | AFF3 R_351 | chr2:100109679-100109707 |
| AFF3 R_165 | chr2:99708160-99708328 | AFF3 R_352 | chr2:100109729-100109858 |
| AFF3 R_166 | chr2:99708342-99708526 | AFF3 R_353 | chr2:100109870-100109937 |
| AFF3 R_167 | chr2:99708531-99708591 | AFF3 R_354 | chr2:100109945-100110121 |
| AFF3 R_168 | chr2:99708595-99708632 | AFF3 R_355 | chr2:100110146-100110173 |
| AFF3 R_169 | chr2:99708662-99708727 | AFF3 R_356 | chr2:100110188-100110320 |
| AFF3 R_170 | chr2:99708776-99708803 | AFF3 R_357 | chr2:100110327-100110764 |
| AFF3 R_171 | chr2:99708806-99708899 | AFF3 R_358 | chr2:100110768-100110943 |
| AFF3 R_172 | chr2:99708902-99708956 | AFF3 R_359 | chr2:100110955-100111089 |
| AFF3 R_173 | chr2:99708972-99709069 | AFF3 R_360 | chr2:100111091-100111211 |
| AFF3 R_174 | chr2:99726965-99727046 | AFF3 R_361 | chr2:100111217-100111389 |
| AFF3 R_175 | chr2:99727075-99727149 | AFF3 R_362 | chr2:100111397-100111424 |
| AFF3 R_176 | chr2:99727154-99727181 | AFF3 R_363 | chr2:100111434-100111461 |
| AFF3 R_177 | chr2:99727206-99727247 | AFF3 R_364 | chr2:100111464-100111526 |
| AFF3 R_178 | chr2:99743979-99744167 | AFF3 R_365 | chr2:100112265-100112642 |
| AFF3 R_179 | chr2:99744191-99744223 | AFF3 R_366 | chr2:100129102-100129143 |
| AFF3 R_180 | chr2:99744234-99744261 | AFF3 R_367 | chr2:100129153-100129196 |
| AFF3 R_181 | chr2:99752109-99752149 | AFF3 R_368 | chr2:100129216-100129282 |
| AFF3 R_182 | chr2:99752155-99752328 | AFF3 R_369 | chr2:100129300-100129336 |
| AFF3 R_183 | chr2:99752333-99752424 | AFF3 R_370 | chr2:100129338-100129400 |
| AFF3 R_184 | chr2:99837362-99837505 | AFF3 R_371 | chr2:100142360-100142900 |
| AFF3 R_185 | chr2:99837511-99837645 | AFF3 R_372 | chr2:100142903-100143958 |
| AFF3 R_186 | chr2:100006511-100006574 | AFF3 R_373 | chr2:100143963-100144108 |
| AFF3 R_187 | chr2:100006587-100006614 | | |

**Table 1D Genomic coordinates of preferred targeting subregions of human AXL**

| Region No. | Subregion coordinate range | Region No. | Subregion coordinate range |
|---|---|---|---|
| AXL R_1 | chr19:41217698-41218559 | AXL R_26 | chr19:41243664-41243821 |
| AXL R_2 | chr19:41218563-41218758 | AXL R_27 | chr19:41248406-41248844 |
| AXL R_3 | chr19:41218789-41219595 | AXL R_28 | chr19:41248846-41248936 |
| AXL R_4 | chr19:41220511-41220981 | AXL R_29 | chr19:41252256-41252298 |
| AXL R_5 | chr19:41221034-41221358 | AXL R_30 | chr19:41252304-41252566 |
| AXL R_6 | chr19:41221754-41222122 | AXL R_31 | chr19:41252722-41252989 |
| AXL R_7 | chr19:41222133-41222183 | AXL R_32 | chr19:41252993-41253086 |
| AXL R_8 | chr19:41225230-41225553 | AXL R_33 | chr19:41253480-41253558 |
| AXL R_9 | chr19:41225557-41225587 | AXL R_34 | chr19:41253563-41253832 |
| AXL R_10 | chr19:41225615-41225661 | AXL R_35 | chr19:41256332-41256733 |
| AXL R_11 | chr19:41225685-41225715 | AXL R_36 | chr19:41257378-41257460 |
| AXL R_12 | chr19:41225744-41225800 | AXL R_37 | chr19:41257468-41257749 |
| AXL R_13 | chr19:41225804-41225866 | AXL R_38 | chr19:41259434-41260243 |
| AXL R_14 | chr19:41225875-41226641 | AXL R_39 | chr19:41260245-41260321 |
| AXL R_15 | chr19:41226685-41226723 | AXL R_40 | chr19:41260330-41260380 |
| AXL R_16 | chr19:41226725-41226752 | AXL R_41 | chr19:41260382-41260735 |
| AXL R_17 | chr19:41226778-41227034 | AXL R_42 | chr19:41260776-41260913 |
| AXL R_18 | chr19:41230846-41231424 | AXL R_43 | chr19:41260948-41260975 |
| AXL R_19 | chr19:41237837-41238275 | AXL R_44 | chr19:41260993-41261041 |
| AXL R_20 | chr19:41238355-41238439 | AXL R_45 | chr19:41261044-41261111 |
| AXL R_21 | chr19:41238442-41238729 | AXL R_46 | chr19:41261164-41261276 |
| AXL R_22 | chr19:41239037-41239438 | AXL R_47 | chr19:41261301-41261418 |
| AXL R_23 | chr 19:41239568-41239833 | AXL R_48 | chr19:41261440-41261502 |
| AXL R_24 | chr19:41242760-41243135 | AXL R_49 | chr19:41261504-41261753 |
| AXL R_25 | chr19:41243490-41243661 | | |

**Table 1E Genomic coordinates of preferred targeted sub-regions of human NFE2L1**

| Region No. | Subregion coordinate range | Region No. | Subregion coordinate range |
|---|---|---|---|
| NFE2L1 R_1 | chr17:48046868-48046906 | NFE2L1 R_15 | chr17:48058190-48059511 |
| NFE2L1 R_2 | chr17:48046909-48047015 | NFE2L1 R_16 | chr17:48059516-48060034 |
| NFE2L1 R_3 | chr17:48047028-48047073 | NFE2L1 R_17 | chr17:48060037-48060226 |
| NFE2L1 R_4 | chr17:48047105-48047235 | NFE2L1 R_18 | chr17:48060241-48060380 |
| NFE2L1 R_5 | chr17:48047245-48047521 | NFE2L1 R_19 | chr17:48060382-48060760 |
| NFE2L1 R_6 | chr17:48047531-48048986 | NFE2L1 R_20 | chr17:48060778-48060890 |
| NFE2L1 R_7 | chr17:48050502-48050573 | NFE2L1 R_21 | chr17:48060896-48060924 |
| NFE2L1 R_8 | chr17:48050579-48051150 | NFE2L1 R_22 | chr17:48060929-48061010 |
| NFE2L1 R_9 | chr17:48051157-48051203 | NFE2L1 R_23 | chr17:48061019-48061046 |
| NFE2L1 R_10 | chr17:48051211-48051749 | NFE2L1 R_24 | chr17:48061056-48061204 |
| NFE2L1 R_11 | chr17:48056263-48056712 | NFE2L1 R_25 | chr17:48061209-48061439 |
| NFE2L1 R_12 | chr17:48056906-48057130 | NFE2L1 R_26 | chr17:48061445-48061472 |
| NFE2L1 R_13 | chr17:48057133-48057301 | NFE2L1 R_27 | chr17:48061499-48061547 |
| NFE2L1 R_14 | chr17:48057307-48057626 | | |

**Table 1F Genomic coordinates of preferred targeted subregions of human RARG**

| Region No. | Subregion coordinate range | Region No. | Subregion coordinate range |
|---|---|---|---|
| RARG R_1 | chr12:53210576-53210659 | RARG R_14 | chr12:53220615-53220739 |
| RARG R_2 | chr12:53210663-53211623 | RARG R_15 | chr12:53220742-53221338 |
| RARG R_3 | chr12:53211630-53211985 | RARG R_16 | chr12:53221351-53221887 |
| RARG R_4 | chr12:53212967-53213822 | RARG R_17 | chr12:53227237-53227595 |
| RARG R_5 | chr12:53213935-53214098 | RARG R_18 | chr12:53227602-53227814 |
| RARG R_6 | chr12:53214100-53214553 | RARG R_19 | chr12:53231052-53231360 |
| RARG R_7 | chr12:53214557-53214721 | RARG R_20 | chr12:53231847-53232210 |
| RARG R_8 | chr12:53215175-53215611 | RARG R_21 | chr12:53232233-53233130 |
| RARG R_9 | chr12:53215614-53215920 | RARG R_22 | chr12:53233162-53233197 |
| RARG R_10 | chr12:53219824-53220308 | RARG R_23 | chr12:53233226-53233405 |
| RARG R_11 | chr12:53220312-53220462 | RARG R_24 | chr12:53233415-53233652 |
| RARG R_12 | chr12:53220468-53220575 | RARG R_25 | chr12:53233654-53233704 |
| RARG R_13 | chr12:53220581-53220608 | RARG R_26 | chr12:53233708-53233735 |

**Table 1G Genomic coordinates of preferred target subregions of human UBFD1**

| Region No. | Subregion coordinate range | Region No. | Subregion coordinate range |
|---|---|---|---|
| UBFD1 R_1 | chr16:23556209-23556425 | UBFD1 R_28 | chr16:23566358-23567087 |
| UBFD1 R_2 | chr16:23556430-23556579 | UBFD1 R_29 | chr16:23567094-23567156 |
| UBFD1 R_3 | chr16:23556581-23556848 | UBFD1 R_30 | chr16:23570377-23570578 |
| UBFD1 R_4 | chr16:23556852-23556962 | UBFD1 R_31 | chr16:23570581-23570840 |
| UBFD1 R_5 | chr16:23556965-23558200 | UBFD1 R_32 | chr16:23570845-23570883 |
| UBFD1 R_6 | chr16:23558207-23558406 | UBFD1 R_33 | chr16:23570895-23570932 |
| UBFD1 R_7 | chr16:23559357-23559384 | UBFD1 R_34 | chr16:23570969-23571032 |
| UBFD1 R_8 | chr16:23559392-23559869 | UBFD1 R_35 | chr16:23571045-23571082 |
| UBFD1 R_9 | chr16:23559872-23559956 | UBFD1 R_36 | chr16:23571091-23571322 |
| UBFD1 R_10 | chr16:23562093-23562158 | UBFD1 R_37 | chr16:23571347-23571776 |
| UBFD1 R_11 | chr16:23562187-23562231 | UBFD1 R_38 | chr16:23571784-23571898 |
| UBFD1 R_12 | chr16:23562236-23562390 | UBFD1 R_39 | chr16:23571936-23571989 |
| UBFD1 R_13 | chr16:23562498-23562677 | UBFD1 R_40 | chr16:23571991-23572028 |
| UBFD1 R_14 | chr16:23562689-23562806 | UBFD1 R_41 | chr16:23572033-23572062 |
| UBFD1 R_15 | chr16:23562812-23562854 | UBFD1 R_42 | chr16:23572068-23572238 |
| UBFD1 R_16 | chr16:23563922-23563989 | UBFD1 R_43 | chr16:23572267-23572411 |
| UBFD1 R_17 | chr16:23563994-23564167 | UBFD1 R_44 | chr16:23572417-23572445 |
| UBFD1 R_18 | chr16:23564173-23564234 | UBFD1 R_45 | chr16:23572456-23572719 |
| UBFD1 R_19 | chr16:23564427-23564492 | UBFD1 R_46 | chr16:23572721-23572764 |
| UBFD1 R_20 | chr16:23564494-23564559 | UBFD1 R_47 | chr16:23572769-23572839 |
| UBFD1 R_21 | chr16:23564591-23564806 | UBFD1 R_48 | chr16:23572860-23573016 |
| UBFD1 R_22 | chr16:23564815-23564926 | UBFD1 R_49 | chr16:23573022-23573058 |
| UBFD1 R_23 | chr16:23564935-23565014 | UBFD1 R_50 | chr16:23573082-23574133 |
| UBFD1 R_24 | chr16:23565026-23565305 | UBFD1 R_51 | chr16:23574146-23574173 |
| UBFD1 R_25 | chr16:23565318-23565536 | UBFD1 R_52 | chr16:23574188-23574304 |
| UBFD1 R_26 | chr16:23565544-23566099 | UBFD1 R_53 | chr16:23574325-23574378 |
| UBFD1 R_27 | chr16:23566123-23566349 | | |

**Table 1H Genomic coordinates of preferred target subregions of human CRP**

| Region No. | Subregion coordinate range | Region No. | Subregion coordinate range |
|---|---|---|---|
| CRP R_1 | chr1:159712274-159712760 | CRP R_14 | chr1:159714988-159715026 |
| CRP R_2 | chr1:159712766-159712839 | CRP R_15 | chr1:159715029-159715116 |
| CRP R_3 | chr1:159712846-159713120 | CRP R_16 | chr1:159715121-159715186 |
| CRP R_4 | chr1:159713143-159713221 | CRP R_17 | chr1:159715203-159715329 |
| CRP R_5 | chr1:159713235-159713644 | CRP R_18 | chr1:159715344-159715456 |
| CRP R_6 | chr1:159713659-159713887 | CRP R_19 | chr1:159715465-159715509 |
| CRP R_7 | chr1:159713890-159714173 | CRP R_20 | chr1:159715530-159715586 |
| CRP R_8 | chr1:159714195-159714224 | CRP R_21 | chr1:159715588-159715616 |
| CRP R_9 | chr1:159714238-159714265 | CRP R_22 | chr1:159715625-159715675 |
| CRP R_10 | chr1:159714310-159714337 | CRP R_23 | chr1:159715688-159715715 |
| CRP R_11 | chr1:159714371-159714564 | CRP R_24 | chr1:159715724-159715820 |
| CRP R_12 | chr1:159714573-159714707 | CRP R_25 | chr1:159715825-159715945 |
| CRP R_13 | chr1:159714761-159714974 | CRP R_26 | chr1:159715956-159716113 |

**Table 1I Genomic coordinates of preferred target subregions of human CYLD**

| Region No. | Subregion coordinate range | Region No. | Subregion coordinate range |
|---|---|---|---|
| CYLD R_1 | chr16:50740584-50740626 | CYLD R_69 | chr16:50792712-50792747 |
| CYLD R_2 | chr16:50740631-50740934 | CYLD R_70 | chr16:50792750-50792832 |
| CYLD R_3 | chr16:50740936-50741027 | CYLD R_71 | chr16:50793437-50793488 |
| CYLD R_4 | chr16:50741064-50741178 | CYLD R_72 | chr16:50793512-50793638 |
| CYLD R_5 | chr16:50741189-50741219 | CYLD R_73 | chr16:50793651-50793701 |
| CYLD R_6 | chr16:50741227-50742962 | CYLD R_74 | chr16:50793717-50793771 |
| CYLD R_7 | chr16:50744607-50744663 | CYLD R_75 | chr16:50794086-50794337 |
| CYLD R_8 | chr16:50744673-50744700 | CYLD R_76 | chr16:50794351-50794497 |
| CYLD R_9 | chr16:50744709-50744749 | CYLD R_77 | chr16:50794502-50794550 |
| CYLD R_10 | chr16:50744775-50744854 | CYLD R_78 | chr16:50796216-50796277 |
| CYLD R_11 | chr16:50744874-50744982 | CYLD R_79 | chr16:50796293-50796345 |
| CYLD R_12 | chr16:50749437-50749464 | CYLD R_80 | chr16:50796347-50796457 |
| CYLD R_13 | chr16:50749522-50749549 | CYLD R_81 | chr16:50796478-50796542 |
| CYLD R_14 | chr16:50749553-50749641 | CYLD R_82 | chr16:50796548-50796669 |
| CYLD R_15 | chr16:50749662-50749716 | CYLD R_83 | chr16:50796672-50796726 |
| CYLD R_16 | chr16:50749719-50749766 | CYLD R_84 | chr16:50796759-50796824 |
| CYLD R_17 | chr16:50749801-50749910 | CYLD R_85 | chr16:50796835-50796951 |
| CYLD R_18 | chr16:50749930-50749960 | CYLD R_86 | chr16:50796970-50796997 |
| CYLD R_19 | chr16:50749975-50750203 | CYLD R_87 | chr16:50797007-50797034 |
| CYLD R_20 | chr16:50750217-50750244 | CYLD R_88 | chr16:50797048-50797075 |
| CYLD R_21 | chr16:50750286-50750313 | CYLD R_89 | chr16:50797077-50797126 |
| CYLD R_22 | chr16:50751510-50751697 | CYLD R_90 | chr16:50797152-50797208 |
| CYLD R_23 | chr16:50751709-50751839 | CYLD R_91 | chr16:50797219-50797312 |
| CYLD R_24 | chr16:50751842-50751921 | CYLD R_92 | chr16:50797325-50797434 |
| CYLD R_25 | chr16:50754211-50754252 | CYLD R_93 | chr16:50797457-50797493 |
| CYLD R_26 | chr16:50754260-50754356 | CYLD R_94 | chr16:50797535-50797565 |
| CYLD R_27 | chr16:50754398-50754456 | CYLD R_95 | chr16:50797584-50797655 |
| CYLD R_28 | chr16:50754477-50754547 | CYLD R_96 | chr16:50797658-50797685 |
| CYLD R_29 | chr16:50775053-50775232 | CYLD R_97 | chr16:50797699-50797728 |
| CYLD R_30 | chr16:50775250-50775291 | CYLD R_98 | chr16:50797765-50797792 |
| CYLD R_31 | chr16:50776059-50776103 | CYLD R_99 | chr16:50797804-50797847 |
| CYLD R_32 | chr16:50776119-50776292 | CYLD R_100 | chr16:50797858-50797899 |
| CYLD R_33 | chr16:50776299-50776356 | CYLD R_101 | chr16:50797937-50798176 |
| CYLD R_34 | chr16:50777798-50777942 | CYLD R_102 | chr16:50798192-50798330 |
| CYLD R_35 | chr16:50777975-50778005 | CYLD R_103 | chr16:50798337-50798364 |
| CYLD R_36 | chr16:50778007-50778034 | CYLD R_104 | chr16:50798395-50798422 |
| CYLD R_37 | chr16:50779541-50779579 | CYLD R_105 | chr16:50798437-50798467 |
| CYLD R_38 | chr16:50779624-50779654 | CYLD R_106 | chr16:50798484-50798632 |
| CYLD R_39 | chr16:50779675-50779703 | CYLD R_107 | chr16:50798648-50799157 |
| CYLD R_40 | chr16:50779714-50780045 | CYLD R_108 | chr16:50799167-50799220 |
| CYLD R_41 | chr16:50780059-50780089 | CYLD R_109 | chr16:50799267-50799371 |
| CYLD R_42 | chr16:50780106-50780134 | CYLD R_110 | chr16:50799382-50799415 |
| CYLD R_43 | chr16:50781126-50781169 | CYLD R_111 | chr16:50799420-50799471 |
| CYLD R_44 | chr16:50781179-50781214 | CYLD R_112 | chr16:50799492-50799554 |
| CYLD R_45 | chr16:50781219-50781491 | CYLD R_113 | chr16:50799558-50799663 |
| CYLD R_46 | chr16:50782232-50782260 | CYLD R_114 | chr16:50799676-50799841 |
| CYLD R_47 | chr16:50782304-50782334 | CYLD R_115 | chr16:50799866-50799935 |
| CYLD R_48 | chr16:50782351-50782550 | CYLD R_116 | chr16:50799942-50800068 |
| CYLD R_49 | chr16:50784231-50784276 | CYLD R_117 | chr16:50800077-50800117 |
| CYLD R_50 | chr16:50784313-50784354 | CYLD R_118 | chr16:50800133-50800329 |
| CYLD R_51 | chr16:50784372-50784437 | CYLD R_119 | chr16:50800337-50800368 |
| CYLD R_52 | chr16:50784441-50784511 | CYLD R_120 | chr16:50800374-50800409 |
| CYLD R_53 | chr16:50784528-50784575 | CYLD R_121 | chr16:50800478-50800611 |
| CYLD R_54 | chr16:50786718-50786761 | CYLD R_122 | chr16:50800614-50800641 |
| CYLD R_55 | chr16:50786822-50786860 | CYLD R_123 | chr16:50800675-50800731 |
| CYLD R_56 | chr16:50786867-50786978 | CYLD R_124 | chr16:50800733-50800891 |
| CYLD R_57 | chr16:50787009-50787040 | CYLD R_125 | chr16:50800901-50800944 |
| CYLD R_58 | chr16:50787751-50787884 | CYLD R_126 | chr16:50800948-50801127 |
| CYLD R_59 | chr16:50787910-50787978 | CYLD R_127 | chr16:50801132-50801252 |
| CYLD R_60 | chr16:50791434-50791487 | CYLD R_128 | chr16:50801254-50801362 |
| CYLD R_61 | chr16:50791498-50791525 | CYLD R_129 | chr16:50801372-50801471 |
| CYLD R_62 | chr16:50791539-50791574 | CYLD R_130 | chr16:50801481-50801583 |
| CYLD R_63 | chr16:50791580-50791626 | CYLD R_131 | chr16:50801621-50801683 |
| CYLD R_64 | chr16:50791630-50791659 | CYLD R_132 | chr16:50801686-50801731 |
| CYLD R_65 | chr16:50791664-50791751 | CYLD R_133 | chr16:50801760-50801787 |
| CYLD R_66 | chr16:50792471-50792547 | CYLD R_134 | chr16:50801791-50801881 |
| CYLD R_67 | chr16:50792570-50792597 | CYLD R_135 | chr16:50801905-50801932 |
| CYLD R_68 | chr16:50792600-50792693 | CYLD R_136 | chr16:50801935-50801962 |

**Table 1J Genomic coordinates of preferred target subregions of human CBLIF**

| Region No. | Subregion coordinate range | Region No. | Subregion coordinate range |
|---|---|---|---|
| CBLIF R_1 | chr11:59829320-59829396 | CBLIF R_19 | chr11:59842987-59843230 |
| CBLIF R_2 | chr11:59829400-59829664 | CBLIF R_20 | chr11:59843757-59843864 |
| CBLIF R_3 | chr11:59831619-59831799 | CBLIF R_21 | chr11:59843866-59843983 |
| CBLIF R_4 | chr11:59831809-59831863 | CBLIF R_22 | chr11:59843996-59844066 |
| CBLIF R_5 | chr11:59831886-59831913 | CBLIF R_23 | chr11:59844071-59844145 |
| CBLIF R_6 | chr11:59835694-59835889 | CBLIF R_24 | chr11:59845260-59845395 |
| CBLIF R_7 | chr11:59835894-59836084 | CBLIF R_25 | chr11:59845397-59845516 |
| CBLIF R_8 | chr11:59836092-59836121 | CBLIF R_26 | chr11:59845518-59845630 |
| CBLIF R_9 | chr11:59837052-59837259 | CBLIF R_27 | chr11:59845639-59845790 |
| CBLIF R_10 | chr11:59837265-59837407 | CBLIF R_28 | chr11:59845812-59845911 |
| CBLIF R_11 | chr11:59837422-59837449 | CBLIF R_29 | chr11:59845914-59846162 |
| CBLIF R_12 | chr11:59841019-59841046 | CBLIF R_30 | chr11:59846187-59846214 |
| CBLIF R_13 | chr11:59841049-59841210 | CBLIF R_31 | chr11:59846224-59846289 |
| CBLIF R_14 | chr11:59841214-59841345 | CBLIF R_32 | chr11:59846308-59846735 |
| CBLIF R_15 | chr11:59841352-59841413 | CBLIF R_33 | chr11:59846751-59846798 |
| CBLIF R_16 | chr11:59841416-59841443 | CBLIF R_34 | chr11:59846812-59846859 |
| CBLIF R_17 | chr11:59842323-59842688 | CBLIF R_35 | chr11:59846870-59847025 |
| CBLIF R_18 | chr11:59842922-59842984 | | |

**Table 1K Genomic coordinates of preferred target subregions of human KLF4**

| Region No. | Subregion coordinate range | Region No. | Subregion coordinate range |
|---|---|---|---|
| KLF4 R_1 | chr9:107484844-107484871 | KLF4 R_16 | chr9:107487215-107487405 |
| KLF4 R_2 | chr9:107484894-107484945 | KLF4 R_17 | chr9:107487409-107488389 |
| KLF4 R_3 | chr9:107484973-107485005 | KLF4 R_18 | chr9:107488809-107488941 |
| KLF4 R_4 | chr9:107485013-107485050 | KLF4 R_19 | chr9:107488943-107489006 |
| KLF4 R_5 | chr9:107485057-107485084 | KLF4 R_20 | chr9:107489016-107489316 |
| KLF4 R_6 | chr9:107485086-107485172 | KLF4 R_21 | chr9:107489338-107489365 |
| KLF4 R_7 | chr9:107485191-107485294 | KLF4 R_22 | chr9:107489369-107490181 |
| KLF4 R_8 | chr9:107485301-107485366 | KLF4 R_23 | chr9:107490190-107490244 |
| KLF4 R_9 | chr9:107485380-107485527 | KLF4 R_24 | chr9: 107490253-107490426 |
| KLF4 R_10 | chr9:107485541-107485582 | KLF4 R_25 | chr9:107490429-107490618 |
| KLF4 R_11 | chr9:107485586-107485883 | KLF4 R_26 | chr9:107490632-107490680 |
| KLF4 R_12 | chr9:107485886-107485953 | KLF4 R_27 | chr9: 107490689-107490735 |
| KLF4 R_13 | chr9:107485972-107486050 | KLF4 R_28 | chr9:107490748-107490984 |
| KLF4 R_14 | chr9:107486901-107487076 | KLF4 R_29 | chr9:107491013-107491041 |
| KLF4 R_15 | chr9:107487080-107487213 | KLF4 R_30 | chr9:107491054-107491292 |

**Table 1L Genomic coordinates of preferred targeted subregions of human NDST1**

| Region No. | Subregion coordinate range | Region No. | Subregion coordinate range |
|---|---|---|---|
| NDST1 R_1 | chr5:150496256-150496397 | NDST1 R_29 | chr5:150545581-150545609 |
| NDST1 R_2 | chr5:150496413-150496886 | NDST1 R_30 | chr5:150548098-150548449 |
| NDST1 R_3 | chr5:150496892-150496976 | NDST1 R_31 | chr5:150548453-150548502 |
| NDST1 R_4 | chr5:150496979-150497007 | NDST1 R_32 | chr5:150549551-150549904 |
| NDST1 R_5 | chr5:150497042-150497372 | NDST1 R_33 | chr5:150551628-150551712 |
| NDST1 R_6 | chr5:150497379-150498001 | NDST1 R_34 | chr5:150551718-150551932 |
| NDST1 R_7 | chr5:150498009-150498102 | NDST1 R_35 | chr5:150551934-150551978 |
| NDST1 R_8 | chr5:150506604-150507478 | NDST1 R_36 | chr5:150553089-150553188 |
| NDST1 R_9 | chr5:150507488-150508257 | NDST1 R_37 | chr5:150553199-150553375 |
| NDST1 R_10 | chr5:150508259-150508342 | NDST1 R_38 | chr5:150553379-150554338 |
| NDST1 R_11 | chr5:150520750-150521784 | NDST1 R_39 | chr5:150554364-150554396 |
| NDST1 R_12 | chr5:150521786-150521894 | NDST1 R_40 | chr5:150554398-150554703 |
| NDST1 R_13 | chr5:150527684-150527773 | NDST1 R_41 | chr5:150554708-150554842 |
| NDST1 R_14 | chr5:150527777-150528422 | NDST1 R_42 | chr5:150554849-150555450 |
| NDST1 R_15 | chr5:150532820-150532855 | NDST1 R_43 | chr5:150555453-150555553 |
| NDST1 R_16 | chr5:150532858-150532974 | NDST1 R_44 | chr5:150555568-150556061 |
| NDST1 R_17 | chr5:150532978-150533152 | NDST1 R_45 | chr5:150556065-150556502 |
| NDST1 R_18 | chr5:150534741-150535148 | NDST1 R_46 | chr5:150556518-150556574 |
| NDST1 R_19 | chr5:150535585-150535933 | NDST1 R_47 | chr5:150556580-150556607 |
| NDST1 R_20 | chr5:150535936-150535994 | NDST1 R_48 | chr5:150556624-150556651 |
| NDST1 R_21 | chr5:150539109-150539325 | NDST1 R_49 | chr5:150556664-150556865 |
| NDST1 R_22 | chr5:150539335-150539481 | NDST1 R_50 | chr5:150556875-150557011 |
| NDST1 R_23 | chr5:150539966-150540390 | NDST1 R_51 | chr5:150557022-150557064 |
| NDST1 R_24 | chr5:150541455-150541683 | NDST1 R_52 | chr5:150557077-150557104 |
| NDST1 R_25 | chr5:150541687-150541790 | NDST1 R_53 | chr5:150557109-150557907 |
| NDST1 R_26 | chr5:150542722-150543068 | NDST1 R_54 | chr5:150557918-150557946 |
| NDST1 R_27 | chr5:150543070-150543098 | NDST1 R_55 | chr5:150557949-150558200 |
| NDST1 R_28 | chr5:150545190-150545559 | | |

**Table 1M Genomic coordinates of preferred targeted subregions of human NLRP1**

| Region No. | Subregion coordinate range | Region No. | Subregion coordinate range |
|---|---|---|---|
| NLRP1 R_1 | chr17:5501371-5501398 | NLRP1 R_28 | chr17:5534042-5534102 |
| NLRP1 R_2 | chr17:5501455-5501482 | NLRP1 R_29 | chr17:5536731-5537061 |
| NLRP1 R_3 | chr17:5501495-5501551 | NLRP1 R_30 | chr17:5539293-5539325 |
| NLRP1 R_4 | chr17:5501563-5501686 | NLRP1 R_31 | chr17:5539342-5539706 |
| NLRP1 R_5 | chr17:5501694-5501990 | NLRP1 R_32 | chr17:5541737-5541911 |
| NLRP1 R_6 | chr17:5514093-5514166 | NLRP1 R_33 | chr17:5541914-5542149 |
| NLRP1 R_7 | chr17:5514184-5514231 | NLRP1 R_34 | chr17:5553260-5553302 |
| NLRP1 R_8 | chr17:5514236-5514263 | NLRP1 R_35 | chr17:5553309-5553676 |
| NLRP1 R_9 | chr17:5514270-5514323 | NLRP1 R_36 | chr17:5558218-5558413 |
| NLRP1 R_10 | chr17:5514330-5514772 | NLRP1 R_37 | chr17:5558448-5558763 |
| NLRP1 R_11 | chr17:5514776-5515094 | NLRP1 R_38 | chr17:5558765-5558857 |
| NLRP1 R_12 | chr17:5515097-5515200 | NLRP1 R_39 | chr17:5558861-5558947 |
| NLRP1 R_13 | chr17:5515346-5515379 | NLRP1 R_40 | chr17:5558956-5559065 |
| NLRP1 R_14 | chr17:5515384-5515544 | NLRP1 R_41 | chr17:5559082-5559837 |
| NLRP1 R_15 | chr17:5515550-5515577 | NLRP1 R_42 | chr17:5559844-5560076 |
| NLRP1 R_16 | chr17:5515582-5515620 | NLRP1 R_43 | chr17:5560094-5560 121 |
| NLRP1 R_17 | chr17:5517628-5518003 | NLRP1 R_44 | chr17:5560124-5560163 |
| NLRP1 R_18 | chr17:5520754-5521116 | NLRP1 R_45 | chr17:5581754-5582070 |
| NLRP1 R_19 | chr17:5521403-5521816 | NLRP1 R_46 | chr17:5582088-5582117 |
| NLRP1 R_20 | chr17:5521843-5521895 | NLRP1 R_47 | chr17:5582130-5582173 |
| NLRP1 R_21 | chr17:5530356-5530632 | NLRP1 R_48 | chr17:5582545-5582959 |
| NLRP1 R_22 | chr17:5530634-5530724 | NLRP1 R_49 | chr17:5583561-5584282 |
| NLRP1 R_23 | chr17:5530735-5530823 | NLRP1 R_50 | chr17:5584299-5584924 |
| NLRP1 R_24 | chr17:5532701-5533110 | NLRP1 R_51 | chr17:5584927-5585151 |
| NLRP1 R_25 | chr17:5533169-5533492 | NLRP1 R_52 | chr17:5585154-5585543 |
| NLRP1 R_26 | chr17:5533773-5533828 | NLRP1 R_53 | chr17:5585546-5585691 |
| NLRP1 R_27 | chr17:5533833-5534040 | NLRP1 R_54 | chr17:5585694-5586032 |

**Table 1N Genomic coordinates of preferred targeted subregions of human SCGB1A1**

| Region No. | Subregion coordinate range | Region No. | Subregion coordinate range |
|---|---|---|---|
| SCGB1A1 R_1 | chr11:62417509-62417610 | SCGB1A1 R_9 | chr11:62422237-62422364 |
| SCGB1A1 R_2 | chr11:62417624-62417723 | SCGB1A1 R_10 | chr11:62422368-62422533 |
| SCGB1A1 R_3 | chr11:62417737-62417844 | SCGB1A1 R_11 | chr11:62422933-62422981 |
| SCGB1A1 R_4 | chr11:62417849-62418493 | SCGB1A1 R_12 | chr11:62422995-62423028 |
| SCGB1A1 R_5 | chr11:62418495-62418900 | SCGB1A1 R_13 | chr11:62423032-62423059 |
| SCGB1A1 R_6 | chr11:62418909-62418956 | SCGB1A1 R_14 | chr11:62423069-62423097 |
| SCGB1A1 R_7 | chr11:62418958-62419271 | SCGB1A1 R_15 | chr11:62423110-62423209 |
| SCGB1A1 R_8 | chr11:62422099-62422234 | | |

**Table 2A Genomic coordinates of the human BCL2L11 gene**

| Target ID | Target Coordinate Range | Target ID | Target Coordinate Range |
|---|---|---|---|
| BCL2L11-1 | chr2:111119416-111119443 | BCL2L11-964 | chr2:111130221-111130248 |
| BCL2L11-2 | chr2:111119422-111119449 | BCL2L11-965 | chr2:111130224-111130251 |
| BCL2L11-3 | chr2:111119434-111119461 | BCL2L11-966 | chr2:111130227-111130254 |
| BCL2L11-4 | chr2:111119438-111119465 | BCL2L11-967 | chr2:111130228-111130255 |
| BCL2L11-5 | chr2:111119439-111119466 | BCL2L11-968 | chr2:111130255-111130282 |
| BCL2L11-6 | chr2:111119440-111119467 | BCL2L11-969 | chr2:111130258-111130285 |
| BCL2L11-7 | chr2:111119441-111119468 | BCL2L11-970 | chr2:111130263-111130290 |
| BCL2L11-8 | chr2:111119480-111119507 | BCL2L11-971 | chr2:111130264-111130291 |
| BCL2L11-9 | chr2:111119481-111119508 | BCL2L11-972 | chr2:111130268-111130295 |
| BCL2L11-10 | chr2:111119499-111119526 | BCL2L11-973 | chr2:111130269-111130296 |
| BCL2L11-11 | chr2:111119502-111119529 | BCL2L11-974 | chr2:111130283-111130310 |
| BCL2L11-12 | chr2:111119506-111119533 | BCL2L11-975 | chr2:111130288-111130315 |
| BCL2L11-13 | chr2:111119510-111119537 | BCL2L11-976 | chr2:111130292-111130319 |
| BCL2L11-14 | chr2:111119513-111119540 | BCL2L11-977 | chr2:111130302-111130329 |
| BCL2L11-15 | chr2:111119514-111119541 | BCL2L11-978 | chr2:111130311-111130338 |
| BCL2L11-16 | chr2:111119522-111119549 | BCL2L11-979 | chr2:111130313-111130340 |
| BCL2L11-17 | chr2:111119530-111119557 | BCL2L11-980 | chr2:111130329-111130356 |
| BCL2L11-18 | chr2:111119543-111119570 | BCL2L11-981 | chr2:111130330-111130357 |
| BCL2L11-19 | chr2:111119548-111119575 | BCL2L11-982 | chr2:111130334-111130361 |
| BCL2L11-20 | chr2:111119551-111119578 | BCL2L11-983 | chr2:111130346-111130373 |
| BCL2L11-21 | chr2:111119562-111119589 | BCL2L11-984 | chr2:111130347-111130374 |
| BCL2L11-22 | chr2:111119569-111119596 | BCL2L11-985 | chr2:111142189-111142216 |
| BCL2L11-23 | chr2:111119575-111119602 | BCL2L11-986 | chr2:111142222-111142249 |
| BCL2L11-24 | chr2:111119576-111119603 | BCL2L11-987 | chr2:111142225-111142252 |
| BCL2L11-25 | chr2:111119577-111119604 | BCL2L11-988 | chr2:111142236-111142263 |
| BCL2L11-26 | chr2:111119578-111119605 | BCL2L11-989 | chr2:111142239-111142266 |
| BCL2L11-27 | chr2:111119582-111119609 | BCL2L11-990 | chr2:111142240-111142267 |
| BCL2L11-28 | chr2:111119586-111119613 | BCL2L11-991 | chr2:111142249-111142276 |
| BCL2L11-29 | chr2:111119587-111119614 | BCL2L11-992 | chr2:111142366-111142393 |
| BCL2L11-30 | chr2:111119588-111119615 | BCL2L11-993 | chr2:111142394-111142421 |
| BCL2L11-31 | chr2:111119589-111119616 | BCL2L11-994 | chr2:111142395-111142422 |
| BCL2L11-32 | chr2:111119590-111119617 | BCL2L11-995 | chr2:111142440-111142467 |
| BCL2L11-33 | chr2:111119593-111119620 | BCL2L11-996 | chr2:111142482-111142509 |
| BCL2L11-34 | chr2:111119619-111119646 | BCL2L11-997 | chr2:111142483-111142510 |
| BCL2L11-35 | chr2:111119620-111119647 | BCL2L11-998 | chr2:111144392-111144419 |
| BCL2L11-36 | chr2:111119634-111119661 | BCL2L11-999 | chr2:111144405-111144432 |
| BCL2L11-37 | chr2:111119638-111119665 | BCL2L11-1000 | chr2:111144436-111144463 |
| BCL2L11-38 | chr2:111119639-111119666 | BCL2L11-1001 | chr2:111144448-111144475 |
| BCL2L11-39 | chr2:111119641-111119668 | BCL2L11-1002 | chr2:111144468-111144495 |
| BCL2L11-40 | chr2:111119649-111119676 | BCL2L11-1003 | chr2:111144469-111144496 |
| BCL2L11-41 | chr2:111119654-111119681 | BCL2L11-1004 | chr2:111144474-111144501 |
| BCL2L11-42 | chr2:111119659-111119686 | BCL2L11-1005 | chr2:111144480-111144507 |
| BCL2L11-43 | chr2:111119672-111119699 | BCL2L11-1006 | chr2:111144494-111144521 |
| BCL2L11-44 | chr2:111119678-111119705 | BCL2L11-1007 | chr2:111144508-111144535 |
| BCL2L11-45 | chr2:111119679-111119706 | BCL2L11-1008 | chr2:111144510-111144537 |
| BCL2L11-46 | chr2:111119684-111119711 | BCL2L11-1009 | chr2:111144516-111144543 |
| BCL2L11-47 | chr2:111119687-111119714 | BCL2L11-1010 | chr2:111144517-111144544 |
| BCL2L11-48 | chr2:111119690-111119717 | BCL2L11-1011 | chr2:111144518-111144545 |
| BCL2L11-49 | chr2:111119691-111119718 | BCL2L11-1012 | chr2:111144519-111144546 |
| BCL2L11-50 | chr2:111119695-111119722 | BCL2L11-1013 | chr2:111144520-111144547 |
| BCL2L11-51 | chr2:111119696-111119723 | BCL2L11-1014 | chr2:111144521-111144548 |
| BCL2L11-52 | chr2:111119697-111119724 | BCL2L11-1015 | chr2:111144525-111144552 |
| BCL2L11-53 | chr2:111119698-111119725 | BCL2L11-1016 | chr2:111144556-111144583 |
| BCL2L11-54 | chr2:111119699-111119726 | BCL2L11-1017 | chr2:111144563-111144590 |
| BCL2L11-55 | chr2:111119701-111119728 | BCL2L11-1018 | chr2:111144564-111144591 |
| BCL2L11-56 | chr2:111119702-111119729 | BCL2L11-1019 | chr2:111144567-111144594 |
| BCL2L11-57 | chr2:111119703-111119730 | BCL2L11-1020 | chr2:111144602-111144629 |
| BCL2L11-58 | chr2:111119711-111119738 | BCL2L11-1021 | chr2:111145825-111145852 |
| BCL2L11-59 | chr2:111119716-111119743 | BCL2L11-1022 | chr2:111145837-111145864 |
| BCL2L11-60 | chr2:111119717-111119744 | BCL2L11-1023 | chr2:111145888-111145915 |
| BCL2L11-61 | chr2:111119720-111119747 | BCL2L11-1024 | chr2:111145941-111145968 |
| BCL2L11-62 | chr2:111119730-111119757 | BCL2L11-1025 | chr2:111145952-111145979 |
| BCL2L11-63 | chr2:111119733-111119760 | BCL2L11-1026 | chr2:111145974-111146001 |
| BCL2L11-64 | chr2:111119734-111119761 | BCL2L11-1027 | chr2:111145985-111146012 |
| BCL2L11-65 | chr2:111119738-111119765 | BCL2L11-1028 | chr2:111145986-111146013 |
| BCL2L11-66 | chr2:111119739-111119766 | BCL2L11-1029 | chr2:111145990-111146017 |
| BCL2L11-67 | chr2:111119740-111119767 | BCL2L11-1030 | chr2:111145992-111146019 |
| BCL2L11-68 | chr2:111119744-111119771 | BCL2L11-1031 | chr2:111146055-111146082 |
| BCL2L11-69 | chr2:111119745-111119772 | BCL2L11-1032 | chr2:111146081-111146108 |
| BCL2L11-70 | chr2:111119746-111119773 | BCL2L11-1033 | chr2:111146088-111146115 |
| BCL2L11-71 | chr2:111119762-111119789 | BCL2L11-1034 | chr2:111146104-111146131 |
| BCL2L11-72 | chr2:111119763-111119790 | BCL2L11-1035 | chr2:111146130-111146157 |
| BCL2L11-73 | chr2:111119764-111119791 | BCL2L11-1036 | chr2:111146131-111146158 |
| BCL2L11-74 | chr2:111119776-111119803 | BCL2L11-1037 | chr2:111146183-111146210 |
| BCL2L11-75 | chr2:111119777-111119804 | BCL2L11-1038 | chr2:111146184-111146211 |
| BCL2L11-76 | chr2:111119778-111119805 | BCL2L11-1039 | chr2:111146185-111146212 |
| BCL2L11-77 | chr2:111119780-111119807 | BCL2L11-1040 | chr2:111146219-111146246 |
| BCL2L11-78 | chr2:111119792-111119819 | BCL2L11-1041 | chr2:111146239-111146266 |
| BCL2L11-79 | chr2:111119797-111119824 | BCL2L11-1042 | chr2:111146240-111146267 |
| BCL2L11-80 | chr2:111119800-111119827 | BCL2L11-1043 | chr2:111146262-111146289 |
| BCL2L11-81 | chr2:111119801-111119828 | BCL2L11-1044 | chr2:111146273-111146300 |
| BCL2L11-82 | chr2:111119802-111119829 | BCL2L11-1045 | chr2:111146277-111146304 |
| BCL2L11-83 | chr2:111119805-111119832 | BCL2L11-1046 | chr2:111146280-111146307 |
| BCL2L11-84 | chr2:111119810-111119837 | BCL2L11-1047 | chr2:111149928-111149955 |
| BCL2L11-85 | chr2:111119817-111119844 | BCL2L11-1048 | chr2:111149929-111149956 |
| BCL2L11-86 | chr2:111119825-111119852 | BCL2L11-1049 | chr2:111149930-111149957 |
| BCL2L11-87 | chr2:111119826-111119853 | BCL2L11-1050 | chr2:111149931-111149958 |
| BCL2L11-88 | chr2:111119829-111119856 | BCL2L11-1051 | chr2:111149956-111149983 |
| BCL2L11-89 | chr2:111119839-111119866 | BCL2L11-1052 | chr2:111149963-111149990 |
| BCL2L11-90 | chr2:111119847-111119874 | BCL2L11-1053 | chr2:111149977-111150004 |
| BCL2L11-91 | chr2:111119848-111119875 | BCL2L11-1054 | chr2:111149990-111150017 |
| BCL2L11-92 | chr2:111119855-111119882 | BCL2L11-1055 | chr2:111149991-111150018 |
| BCL2L11-93 | chr2:111119856-111119883 | BCL2L11-1056 | chr2:111150166-111150193 |
| BCL2L11-94 | chr2:111119859-111119886 | BCL2L11-1057 | chr2:111150167-111150194 |
| BCL2L11-95 | chr2:111119864-111119891 | BCL2L11-1058 | chr2:111150184-111150211 |
| BCL2L11-96 | chr2:111119865-111119892 | BCL2L11-1059 | chr2:111150185-111150212 |
| BCL2L11-97 | chr2:111119869-111119896 | BCL2L11-1060 | chr2:111150188-111150215 |
| BCL2L11-98 | chr2:111119870-111119897 | BCL2L11-1061 | chr2:111150189-111150216 |
| BCL2L11-99 | chr2:111119873-111119900 | BCL2L11-1062 | chr2:111150190-111150217 |
| BCL2L11-100 | chr2:111119874-111119901 | BCL2L11-1063 | chr2:111150194-111150221 |
| BCL2L11-101 | chr2:111119875-111119902 | BCL2L11-1064 | chr2:111150223-111150250 |
| BCL2L11-102 | chr2:111119878-111119905 | BCL2L11-1065 | chr2:111150266-111150293 |
| BCL2L11-103 | chr2:111119881-111119908 | BCL2L11-1066 | chr2:111150277-111150304 |
| BCL2L11-104 | chr2:111119882-111119909 | BCL2L11-1067 | chr2:111150292-111150319 |
| BCL2L11-105 | chr2:111119885-111119912 | BCL2L11-1068 | chr2:111150297-111150324 |
| BCL2L11-106 | chr2:111119886-111119913 | BCL2L11-1069 | chr2:111150298-111150325 |
| BCL2L11-107 | chr2:111119887-111119914 | BCL2L11-1070 | chr2:111150303-111150330 |
| BCL2L11-108 | chr2:111119890-111119917 | BCL2L11-1071 | chr2:111150328-111150355 |
| BCL2L11-109 | chr2:111119893-111119920 | BCL2L11-1072 | chr2:111150331-111150358 |
| BCL2L11-110 | chr2:111119894-111119921 | BCL2L11-1073 | chr2:111150340-111150367 |
| BCL2L11-111 | chr2:111119898-111119925 | BCL2L11-1074 | chr2:111150380-111150407 |
| BCL2L11-112 | chr2:111119899-111119926 | BCL2L11-1075 | chr2:111150381-111150408 |
| BCL2L11-113 | chr2:111119900-111119927 | BCL2L11-1076 | chr2:111150427-111150454 |
| BCL2L11-114 | chr2:111119923-111119950 | BCL2L11-1077 | chr2:111150462-111150489 |
| BCL2L11-115 | chr2:111119939-111119966 | BCL2L11-1078 | chr2:111150474-111150501 |
| BCL2L11-116 | chr2:111119940-111119967 | BCL2L11-1079 | chr2:111150484-111150511 |
| BCL2L11-117 | chr2:111119945-111119972 | BCL2L11-1080 | chr2:111150485-111150512 |
| BCL2L11-118 | chr2:111119951-111119978 | BCL2L11-1081 | chr2:111150486-111150513 |
| BCL2L11-119 | chr2:111119952-111119979 | BCL2L11-1082 | chr2:111150487-111150514 |
| BCL2L11-120 | chr2:111119968-111119995 | BCL2L11-1083 | chr2:111150525-111150552 |
| BCL2L11-121 | chr2:111119969-111119996 | BCL2L11-1084 | chr2:111150526-111150553 |
| BCL2L11-122 | chr2:111119970-111119997 | BCL2L11-1085 | chr2:111150561-111150588 |
| BCL2L11-123 | chr2:111119980-111120007 | BCL2L11-1086 | chr2:111150571-111150598 |
| BCL2L11-124 | chr2:111119982-111120009 | BCL2L11-1087 | chr2:111150572-111150599 |
| BCL2L11-125 | chr2:111119986-111120013 | BCL2L11-1088 | chr2:111150575-111150602 |
| BCL2L11-126 | chr2:111120001-111120028 | BCL2L11-1089 | chr2:111150582-111150609 |
| BCL2L11-127 | chr2:111120008-111120035 | BCL2L11-1090 | chr2:111150584-111150611 |
| BCL2L11-128 | chr2:111120009-111120036 | BCL2L11-1091 | chr2:111150589-111150616 |
| BCL2L11-129 | chr2:111120010-111120037 | BCL2L11-1092 | chr2:111150590-111150617 |
| BCL2L11-130 | chr2:111120011-111120038 | BCL2L11-1093 | chr2:111150603-111150630 |
| BCL2L11-131 | chr2:111120021-111120048 | BCL2L11-1094 | chr2:111150604-111150631 |
| BCL2L11-132 | chr2:111120022-111120049 | BCL2L11-1095 | chr2:111150620-111150647 |
| BCL2L11-133 | chr2:111120040-111120067 | BCL2L11-1096 | chr2:111150626-111150653 |
| BCL2L11-134 | chr2:111120045-111120072 | BCL2L11-1097 | chr2:111150627-111150654 |
| BCL2L11-135 | chr2:111120051-111120078 | BCL2L11-1098 | chr2:111150637-111150664 |
| BCL2L11-136 | chr2:111120054-111120081 | BCL2L11-1099 | chr2:111150638-111150665 |
| BCL2L11-137 | chr2:111120082-111120109 | BCL2L11-1100 | chr2:111150652-111150679 |
| BCL2L11-138 | chr2:111120085-111120112 | BCL2L11-1101 | chr2:111150655-111150682 |
| BCL2L11-139 | chr2:111120089-111120116 | BCL2L11-1102 | chr2:111150681-111150708 |
| BCL2L11-140 | chr2:111120092-111120119 | BCL2L11-1103 | chr2:111150682-111150709 |
| BCL2L11-141 | chr2:111120108-111120135 | BCL2L11-1104 | chr2:111150683-111150710 |
| BCL2L11-142 | chr2:111120146-111120173 | BCL2L11-1105 | chr2:111150697-111150724 |
| BCL2L11-143 | chr2:111120147-111120174 | BCL2L11-1106 | chr2:111150739-111150766 |
| BCL2L11-144 | chr2:111120156-111120183 | BCL2L11-1107 | chr2:111150782-111150809 |
| BCL2L11-145 | chr2:111120161-111120188 | BCL2L11-1108 | chr2:111150786-111150813 |
| BCL2L11-146 | chr2:111120162-111120189 | BCL2L11-1109 | chr2:111150809-111150836 |
| BCL2L11-147 | chr2:111120165-111120192 | BCL2L11-1110 | chr2:111150810-111150837 |
| BCL2L11-148 | chr2:111120168-111120195 | BCL2L11-1111 | chr2:111150811-111150838 |
| BCL2L11-149 | chr2:111120171-111120198 | BCL2L11-1112 | chr2:111150812-111150839 |
| BCL2L11-150 | chr2:111120174-111120201 | BCL2L11-1113 | chr2:111150821-111150848 |
| BCL2L11-151 | chr2:111120175-111120202 | BCL2L11-1114 | chr2:111150832-111150859 |
| BCL2L11-152 | chr2:111120178-111120205 | BCL2L11-1115 | chr2:111150849-111150876 |
| BCL2L11-153 | chr2:111120179-111120206 | BCL2L11-1116 | chr2:111150850-111150877 |
| BCL2L11-154 | chr2:111120184-111120211 | BCL2L11-1117 | chr2:111150852-111150879 |
| BCL2L11-155 | chr2:111120185-111120212 | BCL2L11-1118 | chr2:111150863-111150890 |
| BCL2L11-156 | chr2:111120196-111120223 | BCL2L11-1119 | chr2:111150903-111150930 |
| BCL2L11-157 | chr2:111120204-111120231 | BCL2L11-1120 | chr2:111150923-111150950 |
| BCL2L11-158 | chr2:111120205-111120232 | BCL2L11-1121 | chr2:111150937-111150964 |
| BCL2L11-159 | chr2:111120207-111120234 | BCL2L11-1122 | chr2:111150946-111150973 |
| BCL2L11-160 | chr2:111120208-111120235 | BCL2L11-1123 | chr2:111150971-111150998 |
| BCL2L11-161 | chr2:111120214-111120241 | BCL2L11-1124 | chr2:111150972-111150999 |
| BCL2L11-162 | chr2:111120219-111120246 | BCL2L11-1125 | chr2:111150973-111151000 |
| BCL2L11-163 | chr2:111120224-111120251 | BCL2L11-1126 | chr2:111150986-111151013 |
| BCL2L11-164 | chr2:111120226-111120253 | BCL2L11-1127 | chr2:111150989-111151016 |
| BCL2L11-165 | chr2:111120241-111120268 | BCL2L11-1128 | chr2:111150992-111151019 |
| BCL2L11-166 | chr2:111120242-111120269 | BCL2L11-1129 | chr2:111150995-111151022 |
| BCL2L11-167 | chr2:111120245-111120272 | BCL2L11-1130 | chr2:111150996-111151023 |
| BCL2L11-168 | chr2:111120246-111120273 | BCL2L11-1131 | chr2:111151012-111151039 |
| BCL2L11-169 | chr2:111120247-111120274 | BCL2L11-1132 | chr2:111151013-111151040 |
| BCL2L11-170 | chr2:111120248-111120275 | BCL2L11-1133 | chr2:111151014-111151041 |
| BCL2L11-171 | chr2:111120249-111120276 | BCL2L11-1134 | chr2:111151018-111151045 |
| BCL2L11-172 | chr2:111120250-111120277 | BCL2L11-1135 | chr2:111151021-111151048 |
| BCL2L11-173 | chr2:111120254-111120281 | BCL2L11-1136 | chr2:111151024-111151051 |
| BCL2L11-174 | chr2:111120255-111120282 | BCL2L11-1137 | chr2:111151051-111151078 |
| BCL2L11-175 | chr2:111120267-111120294 | BCL2L11-1138 | chr2:111151058-111151085 |
| BCL2L11-176 | chr2:111120280-111120307 | BCL2L11-1139 | chr2:111151063-111151090 |
| BCL2L11-177 | chr2:111120281-111120308 | BCL2L11-1140 | chr2:111151064-111151091 |
| BCL2L11-178 | chr2:111120282-111120309 | BCL2L11-1141 | chr2:111151067-111151094 |
| BCL2L11-179 | chr2:111120285-111120312 | BCL2L11-1142 | chr2:111151068-111151095 |
| BCL2L11-180 | chr2:111120289-111120316 | BCL2L11-1143 | chr2:111151069-111151096 |
| BCL2L11-181 | chr2:111120292-111120319 | BCL2L11-1144 | chr2:111151088-111151115 |
| BCL2L11-182 | chr2:111120295-111120322 | BCL2L11-1145 | chr2:111151102-111151129 |
| BCL2L11-183 | chr2:111120296-111120323 | BCL2L11-1146 | chr2:111151111-111151138 |
| BCL2L11-184 | chr2:111120299-111120326 | BCL2L11-1147 | chr2:111151128-111151155 |
| BCL2L11-185 | chr2:111120300-111120327 | BCL2L11-1148 | chr2:111151129-111151156 |
| BCL2L11-186 | chr2:111120301-111120328 | BCL2L11-1149 | chr2:111151144-111151171 |
| BCL2L11-187 | chr2:111120316-111120343 | BCL2L11-1150 | chr2:111151145-111151172 |
| BCL2L11-188 | chr2:111120321-111120348 | BCL2L11-1151 | chr2:111151146-111151173 |
| BCL2L11-189 | chr2:111120322-111120349 | BCL2L11-1152 | chr2:111151150-111151177 |
| BCL2L11-190 | chr2:111120326-111120353 | BCL2L11-1153 | chr2:111151153-111151180 |
| BCL2L11-191 | chr2:111120327-111120354 | BCL2L11-1154 | chr2:111151156-111151183 |
| BCL2L11-192 | chr2:111120333-111120360 | BCL2L11-1155 | chr2:111151159-111151186 |
| BCL2L11-193 | chr2:111120334-111120361 | BCL2L11-1156 | chr2:111151160-111151187 |
| BCL2L11-194 | chr2:111120344-111120371 | BCL2L11-1157 | chr2:111151172-111151199 |
| BCL2L11-195 | chr2:111120345-111120372 | BCL2L11-1158 | chr2:111151180-111151207 |
| BCL2L11-196 | chr2:111120362-111120389 | BCL2L11-1159 | chr2:111151181-111151208 |
| BCL2L11-197 | chr2:111120366-111120393 | BCL2L11-1160 | chr2:111151187-111151214 |
| BCL2L11-198 | chr2:111120369-111120396 | BCL2L11-1161 | chr2:111151195-111151222 |
| BCL2L11-199 | chr2:111120370-111120397 | BCL2L11-1162 | chr2:111151200-111151227 |
| BCL2L11-200 | chr2:111120375-111120402 | BCL2L11-1163 | chr2:111151215-111151242 |
| BCL2L11-201 | chr2:111120381-111120408 | BCL2L11-1164 | chr2:111151230-111151257 |
| BCL2L11-202 | chr2:111120382-111120409 | BCL2L11-1165 | chr2:111151231-111151258 |
| BCL2L11-203 | chr2:111120385-111120412 | BCL2L11-1166 | chr2:111151238-111151265 |
| BCL2L11-204 | chr2:111120386-111120413 | BCL2L11-1167 | chr2:111151251-111151278 |
| BCL2L11-205 | chr2:111120392-111120419 | BCL2L11-1168 | chr2:111151258-111151285 |
| BCL2L11-206 | chr2:111120399-111120426 | BCL2L11-1169 | chr2:111151259-111151286 |
| BCL2L11-207 | chr2:111120400-111120427 | BCL2L11-1170 | chr2:111151263-111151290 |
| BCL2L11-208 | chr2:111120420-111120447 | BCL2L11-1171 | chr2:111151276-111151303 |
| BCL2L11-209 | chr2:111120425-111120452 | BCL2L11-1172 | chr2:111151326-111151353 |
| BCL2L11-210 | chr2:111120430-111120457 | BCL2L11-1173 | chr2:111151341-111151368 |
| BCL2L11-211 | chr2:111120431-111120458 | BCL2L11-1174 | chr2:111151354-111151381 |
| BCL2L11-212 | chr2:111120432-111120459 | BCL2L11-1175 | chr2:111151374-111151401 |
| BCL2L11-213 | chr2:111120437-111120464 | BCL2L11-1176 | chr2:111151378-111151405 |
| BCL2L11-214 | chr2:111120449-111120476 | BCL2L11-1177 | chr2:111151440-111151467 |
| BCL2L11-215 | chr2:111120453-111120480 | BCL2L11-1178 | chr2:111151441-111151468 |
| BCL2L11-216 | chr2:111120454-111120481 | BCL2L11-1179 | chr2:111151442-111151469 |
| BCL2L11-217 | chr2:111120459-111120486 | BCL2L11-1180 | chr2:111151492-111151519 |
| BCL2L11-218 | chr2:111120460-111120487 | BCL2L11-1181 | chr2:111151499-111151526 |
| BCL2L11-219 | chr2:111120461-111120488 | BCL2L11-1182 | chr2:111151500-111151527 |
| BCL2L11-220 | chr2:111120466-111120493 | BCL2L11-1183 | chr2:111151501-111151528 |
| BCL2L11-221 | chr2:111120467-111120494 | BCL2L11-1184 | chr2:111151504-111151531 |
| BCL2L11-222 | chr2:111120468-111120495 | BCL2L11-1185 | chr2:111151514-111151541 |
| BCL2L11-223 | chr2:111120470-111120497 | BCL2L11-1186 | chr2:111151536-111151563 |
| BCL2L11-224 | chr2:111120471-111120498 | BCL2L11-1187 | chr2:111151570-111151597 |
| BCL2L11-225 | chr2:111120472-111120499 | BCL2L11-1188 | chr2:111151585-111151612 |
| BCL2L11-226 | chr2:111120485-111120512 | BCL2L11-1189 | chr2:111151591-111151618 |
| BCL2L11-227 | chr2:111120486-111120513 | BCL2L11-1190 | chr2:111151623-111151650 |
| BCL2L11-228 | chr2:111120498-111120525 | BCL2L11-1191 | chr2:111151638-111151665 |
| BCL2L11-229 | chr2:111120512-111120539 | BCL2L11-1192 | chr2:111151639-111151666 |
| BCL2L11-230 | chr2:111120516-111120543 | BCL2L11-1193 | chr2:111151646-111151673 |
| BCL2L11-231 | chr2:111120517-111120544 | BCL2L11-1194 | chr2:111151679-111151706 |
| BCL2L11-232 | chr2:111120519-111120546 | BCL2L11-1195 | chr2:111151699-111151726 |
| BCL2L11-233 | chr2:111120520-111120547 | BCL2L11-1196 | chr2:111151700-111151727 |
| BCL2L11-234 | chr2:111120524-111120551 | BCL2L11-1197 | chr2:111151701-111151728 |
| BCL2L11-235 | chr2:111120527-111120554 | BCL2L11-1198 | chr2:111151702-111151729 |
| BCL2L11-236 | chr2:111120533-111120560 | BCL2L11-1199 | chr2:111151711-111151738 |
| BCL2L11-237 | chr2:111120536-111120563 | BCL2L11-1200 | chr2:111151712-111151739 |
| BCL2L11-238 | chr2:111120537-111120564 | BCL2L11-1201 | chr2:111151716-111151743 |
| BCL2L11-239 | chr2:111120540-111120567 | BCL2L11-1202 | chr2:111151745-111151772 |
| BCL2L11-240 | chr2:111120545-111120572 | BCL2L11-1203 | chr2:111151746-111151773 |
| BCL2L11-241 | chr2:111120561-111120588 | BCL2L11-1204 | chr2:111151763-111151790 |
| BCL2L11-242 | chr2:111120567-111120594 | BCL2L11-1205 | chr2:111151798-111151825 |
| BCL2L11-243 | chr2:111120569-111120596 | BCL2L11-1206 | chr2:111151810-111151837 |
| BCL2L11-244 | chr2:111120570-111120597 | BCL2L11-1207 | chr2:111151833-111151860 |
| BCL2L11-245 | chr2:111120571-111120598 | BCL2L11-1208 | chr2:111151841-111151868 |
| BCL2L11-246 | chr2:111120572-111120599 | BCL2L11-1209 | chr2:111151844-111151871 |
| BCL2L11-247 | chr2:111120577-111120604 | BCL2L11-1210 | chr2:111151847-111151874 |
| BCL2L11-248 | chr2:111120578-111120605 | BCL2L11-1211 | chr2:111151858-111151885 |
| BCL2L11-249 | chr2:111120580-111120607 | BCL2L11-1212 | chr2:111151867-111151894 |
| BCL2L11-250 | chr2:111120582-111120609 | BCL2L11-1213 | chr2:111151875-111151902 |
| BCL2L11-251 | chr2:111120583-111120610 | BCL2L11-1214 | chr2:111151879-111151906 |
| BCL2L11-252 | chr2:111120584-111120611 | BCL2L11-1215 | chr2:111151887-111151914 |
| BCL2L11-253 | chr2:111120588-111120615 | BCL2L11-1216 | chr2:111151891-111151918 |
| BCL2L11-254 | chr2:111120589-111120616 | BCL2L11-1217 | chr2:111151942-111151969 |
| BCL2L11-255 | chr2:111120595-111120622 | BCL2L11-1218 | chr2:111151943-111151970 |
| BCL2L11-256 | chr2:111120596-111120623 | BCL2L11-1219 | chr2:111151948-111151975 |
| BCL2L11-257 | chr2:111120598-111120625 | BCL2L11-1220 | chr2:111151949-111151976 |
| BCL2L11-258 | chr2:111120599-111120626 | BCL2L11-1221 | chr2:111151952-111151979 |
| BCL2L11-259 | chr2:111120601-111120628 | BCL2L11-1222 | chr2:111151954-111151981 |
| BCL2L11-260 | chr2:111120604-111120631 | BCL2L11-1223 | chr2:111151955-111151982 |
| BCL2L11-261 | chr2:111120605-111120632 | BCL2L11-1224 | chr2:111151958-111151985 |
| BCL2L11-262 | chr2:111120615-111120642 | BCL2L11-1225 | chr2:111151962-111151989 |
| BCL2L11-263 | chr2:111120616-111120643 | BCL2L11-1226 | chr2:111151964-111151991 |
| BCL2L11-264 | chr2:111120617-111120644 | BCL2L11-1227 | chr2:111151974-111152001 |
| BCL2L11-265 | chr2:111120620-111120647 | BCL2L11-1228 | chr2:111151981-111152008 |
| BCL2L11-266 | chr2:111120621-111120648 | BCL2L11-1229 | chr2:111151987-111152014 |
| BCL2L11-267 | chr2:111120622-111120649 | BCL2L11-1230 | chr2:111151994-111152021 |
| BCL2L11-268 | chr2:111120623-111120650 | BCL2L11-1231 | chr2:111152005-111152032 |
| BCL2L11-269 | chr2:111120627-111120654 | BCL2L11-1232 | chr2:111152014-111152041 |
| BCL2L11-270 | chr2:111120628-111120655 | BCL2L11-1233 | chr2:111152024-111152051 |
| BCL2L11-271 | chr2:111120639-111120666 | BCL2L11-1234 | chr2:111152025-111152052 |
| BCL2L11-272 | chr2:111120650-111120677 | BCL2L11-1235 | chr2:111152042-111152069 |
| BCL2L11-273 | chr2:111120664-111120691 | BCL2L11-1236 | chr2:111152052-111152079 |
| BCL2L11-274 | chr2:111120667-111120694 | BCL2L11-1237 | chr2:111152053-111152080 |
| BCL2L11-275 | chr2:111120670-111120697 | BCL2L11-1238 | chr2:111152054-111152081 |
| BCL2L11-276 | chr2:111120677-111120704 | BCL2L11-1239 | chr2:111152057-111152084 |
| BCL2L11-277 | chr2:111120678-111120705 | BCL2L11-1240 | chr2:111152077-111152104 |
| BCL2L11-278 | chr2:111120682-111120709 | BCL2L11-1241 | chr2:111152078-111152105 |
| BCL2L11-279 | chr2:111120685-111120712 | BCL2L11-1242 | chr2:111152082-111152109 |
| BCL2L11-280 | chr2:111120688-111120715 | BCL2L11-1243 | chr2:111152093-111152120 |
| BCL2L11-281 | chr2:111120694-111120721 | BCL2L11-1244 | chr2:111152094-111152121 |
| BCL2L11-282 | chr2:111120695-111120722 | BCL2L11-1245 | chr2:111152108-111152135 |
| BCL2L11-283 | chr2:111120698-111120725 | BCL2L11-1246 | chr2:111152125-111152152 |
| BCL2L11-284 | chr2:111120700-111120727 | BCL2L11-1247 | chr2:111152126-111152153 |
| BCL2L11-285 | chr2:111120709-111120736 | BCL2L11-1248 | chr2:111152131-111152158 |
| BCL2L11-286 | chr2:111120710-111120737 | BCL2L11-1249 | chr2:111152144-111152171 |
| BCL2L11-287 | chr2:111120711-111120738 | BCL2L11-1250 | chr2:111152149-111152176 |
| BCL2L11-288 | chr2:111120729-111120756 | BCL2L11-1251 | chr2:111152150-111152177 |
| BCL2L11-289 | chr2:111120730-111120757 | BCL2L11-1252 | chr2:111152151-111152178 |
| BCL2L11-290 | chr2:111120742-111120769 | BCL2L11-1253 | chr2:111152155-111152182 |
| BCL2L11-291 | chr2:111120743-111120770 | BCL2L11-1254 | chr2:111152174-111152201 |
| BCL2L11-292 | chr2:111120744-111120771 | BCL2L11-1255 | chr2:111152196-111152223 |
| BCL2L11-293 | chr2:111120745-111120772 | BCL2L11-1256 | chr2:111152197-111152224 |
| BCL2L11-294 | chr2:111120748-111120775 | BCL2L11-1257 | chr2:111152213-111152240 |
| BCL2L11-295 | chr2:111120750-111120777 | BCL2L11-1258 | chr2:111152217-111152244 |
| BCL2L11-296 | chr2:111120753-111120780 | BCL2L11-1259 | chr2:111152218-111152245 |
| BCL2L11-297 | chr2:111120754-111120781 | BCL2L11-1260 | chr2:111152219-111152246 |
| BCL2L11-298 | chr2:111120759-111120786 | BCL2L11-1261 | chr2:111152221-111152248 |
| BCL2L11-299 | chr2:111120764-111120791 | BCL2L11-1262 | chr2:111152222-111152249 |
| BCL2L11-300 | chr2:111120767-111120794 | BCL2L11-1263 | chr2:111152223-111152250 |
| BCL2L11-301 | chr2:111120768-111120795 | BCL2L11-1264 | chr2:111152225-111152252 |
| BCL2L11-302 | chr2:111120769-111120796 | BCL2L11-1265 | chr2:111152232-111152259 |
| BCL2L11-303 | chr2:111120770-111120797 | BCL2L11-1266 | chr2:111152236-111152263 |
| BCL2L11-304 | chr2:111120771-111120798 | BCL2L11-1267 | chr2:111152237-111152264 |
| BCL2L11-305 | chr2:111120774-111120801 | BCL2L11-1268 | chr2:111152241-111152268 |
| BCL2L11-306 | chr2:111120781-111120808 | BCL2L11-1269 | chr2:111152242-111152269 |
| BCL2L11-307 | chr2:111120789-111120816 | BCL2L11-1270 | chr2:111152243-111152270 |
| BCL2L11-308 | chr2:111120796-111120823 | BCL2L11-1271 | chr2:111152262-111152289 |
| BCL2L11-309 | chr2:111120797-111120824 | BCL2L11-1272 | chr2:111152263-111152290 |
| BCL2L11-310 | chr2:111120798-111120825 | BCL2L11-1273 | chr2:111152264-111152291 |
| BCL2L11-311 | chr2:111120801-111120828 | BCL2L11-1274 | chr2:111152280-111152307 |
| BCL2L11-312 | chr2:111120802-111120829 | BCL2L11-1275 | chr2:111152282-111152309 |
| BCL2L11-313 | chr2:111120808-111120835 | BCL2L11-1276 | chr2:111152285-111152312 |
| BCL2L11-314 | chr2:111120811-111120838 | BCL2L11-1277 | chr2:111152292-111152319 |
| BCL2L11-315 | chr2:111120812-111120839 | BCL2L11-1278 | chr2:111152295-111152322 |
| BCL2L11-316 | chr2:111120813-111120840 | BCL2L11-1279 | chr2:111152302-111152329 |
| BCL2L11-317 | chr2:111120814-111120841 | BCL2L11-1280 | chr2:111152320-111152347 |
| BCL2L11-318 | chr2:111120817-111120844 | BCL2L11-1281 | chr2:111152325-111152352 |
| BCL2L11-319 | chr2:111120818-111120845 | BCL2L11-1282 | chr2:111152332-111152359 |
| BCL2L11-320 | chr2:111120819-111120846 | BCL2L11-1283 | chr2:111152333-111152360 |
| BCL2L11-321 | chr2:111120820-111120847 | BCL2L11-1284 | chr2:111152337-111152364 |
| BCL2L11-322 | chr2:111120831-111120858 | BCL2L11-1285 | chr2:111152338-111152365 |
| BCL2L11-323 | chr2:111120833-111120860 | BCL2L11-1286 | chr2:111152344-111152371 |
| BCL2L11-324 | chr2:111120834-111120861 | BCL2L11-1287 | chr2:111152345-111152372 |
| BCL2L11-325 | chr2:111120840-111120867 | BCL2L11-1288 | chr2:111152352-111152379 |
| BCL2L11-326 | chr2:111120845-111120872 | BCL2L11-1289 | chr2:111152367-111152394 |
| BCL2L11-327 | chr2:111120846-111120873 | BCL2L11-1290 | chr2:111152398-111152425 |
| BCL2L11-328 | chr2:111120847-111120874 | BCL2L11-1291 | chr2:111152410-111152437 |
| BCL2L11-329 | chr2:111120848-111120875 | BCL2L11-1292 | chr2:111152414-111152441 |
| BCL2L11-330 | chr2:111120864-111120891 | BCL2L11-1293 | chr2:111152465-111152492 |
| BCL2L11-331 | chr2:111120865-111120892 | BCL2L11-1294 | chr2:111152476-111152503 |
| BCL2L11-332 | chr2:111120872-111120899 | BCL2L11-1295 | chr2:111152477-111152504 |
| BCL2L11-333 | chr2:111120873-111120900 | BCL2L11-1296 | chr2:111152485-111152512 |
| BCL2L11-334 | chr2:111120875-111120902 | BCL2L11-1297 | chr2:111152490-111152517 |
| BCL2L11-335 | chr2:111120878-111120905 | BCL2L11-1298 | chr2:111152496-111152523 |
| BCL2L11-336 | chr2:111120882-111120909 | BCL2L11-1299 | chr2:111152497-111152524 |
| BCL2L11-337 | chr2:111120901-111120928 | BCL2L11-1300 | chr2:111152499-111152526 |
| BCL2L11-338 | chr2:111120910-111120937 | BCL2L11-1301 | chr2:111152505-111152532 |
| BCL2L11-339 | chr2:111120922-111120949 | BCL2L11-1302 | chr2:111152509-111152536 |
| BCL2L11-340 | chr2:111120925-111120952 | BCL2L11-1303 | chr2:111152510-111152537 |
| BCL2L11-341 | chr2:111120930-111120957 | BCL2L11-1304 | chr2:111152513-111152540 |
| BCL2L11-342 | chr2:111120933-111120960 | BCL2L11-1305 | chr2:111152519-111152546 |
| BCL2L11-343 | chr2:111120963-111120990 | BCL2L11-1306 | chr2:111152526-111152553 |
| BCL2L11-344 | chr2:111120969-111120996 | BCL2L11-1307 | chr2:111152532-111152559 |
| BCL2L11-345 | chr2:111120975-111121002 | BCL2L11-1308 | chr2:111152535-111152562 |
| BCL2L11-346 | chr2:111120981-111121008 | BCL2L11-1309 | chr2:111152544-111152571 |
| BCL2L11-347 | chr2:111120984-111121011 | BCL2L11-1310 | chr2:111152550-111152577 |
| BCL2L11-348 | chr2:111120987-111121014 | BCL2L11-1311 | chr2:111152554-111152581 |
| BCL2L11-349 | chr2:111120990-111121017 | BCL2L11-1312 | chr2:111152559-111152586 |
| BCL2L11-350 | chr2:111120993-111121020 | BCL2L11-1313 | chr2:111152560-111152587 |
| BCL2L11-351 | chr2:111120996-111121023 | BCL2L11-1314 | chr2:111152561-111152588 |
| BCL2L11-352 | chr2:111120999-111121026 | BCL2L11-1315 | chr2:111152566-111152593 |
| BCL2L11-353 | chr2:111121002-111121029 | BCL2L11-1316 | chr2:111152585-111152612 |
| BCL2L11-354 | chr2:111121005-111121032 | BCL2L11-1317 | chr2:111152601-111152628 |
| BCL2L11-355 | chr2:111121008-111121035 | BCL2L11-1318 | chr2:111152602-111152629 |
| BCL2L11-356 | chr2:111121011-111121038 | BCL2L11-1319 | chr2:111152607-111152634 |
| BCL2L11-357 | chr2:111121014-111121041 | BCL2L11-1320 | chr2:111152608-111152635 |
| BCL2L11-358 | chr2:111121017-111121044 | BCL2L11-1321 | chr2:111152609-111152636 |
| BCL2L11-359 | chr2:111121020-111121047 | BCL2L11-1322 | chr2:111152611-111152638 |
| BCL2L11-360 | chr2:111121026-111121053 | BCL2L11-1323 | chr2:111152613-111152640 |
| BCL2L11-361 | chr2:111121029-111121056 | BCL2L11-1324 | chr2:111152614-111152641 |
| BCL2L11-362 | chr2:111121046-111121073 | BCL2L11-1325 | chr2:111152615-111152642 |
| BCL2L11-363 | chr2:111121049-111121076 | BCL2L11-1326 | chr2:111152616-111152643 |
| BCL2L11-364 | chr2:111121050-111121077 | BCL2L11-1327 | chr2:111152617-111152644 |
| BCL2L11-365 | chr2:111121058-111121085 | BCL2L11-1328 | chr2:111152649-111152676 |
| BCL2L11-366 | chr2:111121059-111121086 | BCL2L11-1329 | chr2:111152656-111152683 |
| BCL2L11-367 | chr2:111121060-111121087 | BCL2L11-1330 | chr2:111152657-111152684 |
| BCL2L11-368 | chr2:111121063-111121090 | BCL2L11-1331 | chr2:111152671-111152698 |
| BCL2L11-369 | chr2:111121067-111121094 | BCL2L11-1332 | chr2:111152688-111152715 |
| BCL2L11-370 | chr2:111121097-111121124 | BCL2L11-1333 | chr2:111152697-111152724 |
| BCL2L11-371 | chr2:111121106-111121133 | BCL2L11-1334 | chr2:111152702-111152729 |
| BCL2L11-372 | chr2:111121107-111121134 | BCL2L11-1335 | chr2:111152711-111152738 |
| BCL2L11-373 | chr2:111121110-111121137 | BCL2L11-1336 | chr2:111152730-111152757 |
| BCL2L11-374 | chr2:111121113-111121140 | BCL2L11-1337 | chr2:111152746-111152773 |
| BCL2L11-375 | chr2:111121116-111121143 | BCL2L11-1338 | chr2:111152750-111152777 |
| BCL2L11-376 | chr2:111121119-111121146 | BCL2L11-1339 | chr2:111152753-111152780 |
| BCL2L11-377 | chr2:111121127-111121154 | BCL2L11-1340 | chr2:111152754-111152781 |
| BCL2L11-378 | chr2:111121128-111121155 | BCL2L11-1341 | chr2:111152755-111152782 |
| BCL2L11-379 | chr2:111121138-111121165 | BCL2L11-1342 | chr2:111152758-111152785 |
| BCL2L11-380 | chr2:111121139-111121166 | BCL2L11-1343 | chr2:111152766-111152793 |
| BCL2L11-381 | chr2:111121146-111121173 | BCL2L11-1344 | chr2:111152767-111152794 |
| BCL2L11-382 | chr2:111121147-111121174 | BCL2L11-1345 | chr2:111152785-111152812 |
| BCL2L11-383 | chr2:111121148-111121175 | BCL2L11-1346 | chr2:111152827-111152854 |
| BCL2L11-384 | chr2:111121149-111121176 | BCL2L11-1347 | chr2:111152835-111152862 |
| BCL2L11-385 | chr2:111121162-111121189 | BCL2L11-1348 | chr2:111152841-111152868 |
| BCL2L11-386 | chr2:111121182-111121209 | BCL2L11-1349 | chr2:111152850-111152877 |
| BCL2L11-387 | chr2:111121183-111121210 | BCL2L11-1350 | chr2:111152851-111152878 |
| BCL2L11-388 | chr2:111121184-111121211 | BCL2L11-1351 | chr2:111152870-111152897 |
| BCL2L11-389 | chr2:111121189-111121216 | BCL2L11-1352 | chr2:111153660-111153687 |
| BCL2L11-390 | chr2:111121190-111121217 | BCL2L11-1353 | chr2:111153706-111153733 |
| BCL2L11-391 | chr2:111121195-111121222 | BCL2L11-1354 | chr2:111153732-111153759 |
| BCL2L11-392 | chr2:111121196-111121223 | BCL2L11-1355 | chr2:111153734-111153761 |
| BCL2L11-393 | chr2:111121199-111121226 | BCL2L11-1356 | chr2:111153741-111153768 |
| BCL2L11-394 | chr2:111121204-111121231 | BCL2L11-1357 | chr2:111153807-111153834 |
| BCL2L11-395 | chr2:111121205-111121232 | BCL2L11-1358 | chr2:111153808-111153835 |
| BCL2L11-396 | chr2:111121206-111121233 | BCL2L11-1359 | chr2:111153814-111153841 |
| BCL2L11-397 | chr2:111121218-111121245 | BCL2L11-1360 | chr2:111153815-111153842 |
| BCL2L11-398 | chr2:111121225-111121252 | BCL2L11-1361 | chr2:111153816-111153843 |
| BCL2L11-399 | chr2:111121229-111121256 | BCL2L11-1362 | chr2:111153825-111153852 |
| BCL2L11-400 | chr2:111121248-111121275 | BCL2L11-1363 | chr2:111153831-111153858 |
| BCL2L11-401 | chr2:111121249-111121276 | BCL2L11-1364 | chr2:111153839-111153866 |
| BCL2L11-402 | chr2:111121259-111121286 | BCL2L11-1365 | chr2:111153842-111153869 |
| BCL2L11-403 | chr2:111121262-111121289 | BCL2L11-1366 | chr2:111153857-111153884 |
| BCL2L11-404 | chr2:111121267-111121294 | BCL2L11-1367 | chr2:111153860-111153887 |
| BCL2L11-405 | chr2:111121269-111121296 | BCL2L11-1368 | chr2:111153863-111153890 |
| BCL2L11-406 | chr2:111121270-111121297 | BCL2L11-1369 | chr2:111153872-111153899 |
| BCL2L11-407 | chr2:111121276-111121303 | BCL2L11-1370 | chr2:111153876-111153903 |
| BCL2L11-408 | chr2:111121277-111121304 | BCL2L11-1371 | chr2:111153878-111153905 |
| BCL2L11-409 | chr2:111121282-111121309 | BCL2L11-1372 | chr2:111153918-111153945 |
| BCL2L11-410 | chr2:111121298-111121325 | BCL2L11-1373 | chr2:111153919-111153946 |
| BCL2L11-411 | chr2:111121301-111121328 | BCL2L11-1374 | chr2:111153930-111153957 |
| BCL2L11-412 | chr2:111121302-111121329 | BCL2L11-1375 | chr2:111153931-111153958 |
| BCL2L11-413 | chr2:111121305-111121332 | BCL2L11-1376 | chr2:111153932-111153959 |
| BCL2L11-414 | chr2:111121308-111121335 | BCL2L11-1377 | chr2:111153937-111153964 |
| BCL2L11-415 | chr2:111121309-111121336 | BCL2L11-1378 | chr2:111153942-111153969 |
| BCL2L11-416 | chr2:111121310-111121337 | BCL2L11-1379 | chr2:111153943-111153970 |
| BCL2L11-417 | chr2:111121312-111121339 | BCL2L11-1380 | chr2:111153949-111153976 |
| BCL2L11-418 | chr2:111121320-111121347 | BCL2L11-1381 | chr2:111153950-111153977 |
| BCL2L11-419 | chr2:111121322-111121349 | BCL2L11-1382 | chr2:111153958-111153985 |
| BCL2L11-420 | chr2:111121325-111121352 | BCL2L11-1383 | chr2:111153959-111153986 |
| BCL2L11-421 | chr2:111121326-111121353 | BCL2L11-1384 | chr2:111153960-111153987 |
| BCL2L11-422 | chr2:111121337-111121364 | BCL2L11-1385 | chr2:111153963-111153990 |
| BCL2L11-423 | chr2:111121338-111121365 | BCL2L11-1386 | chr2:111153969-111153996 |
| BCL2L11-424 | chr2:111121340-111121367 | BCL2L11-1387 | chr2:111153973-111154000 |
| BCL2L11-425 | chr2:111121342-111121369 | BCL2L11-1388 | chr2:111153980-111154007 |
| BCL2L11-426 | chr2:111121356-111121383 | BCL2L11-1389 | chr2:111153986-111154013 |
| BCL2L11-427 | chr2:111121357-111121384 | BCL2L11-1390 | chr2:111153991-111154018 |
| BCL2L11-428 | chr2:111121371-111121398 | BCL2L11-1391 | chr2:111153992-111154019 |
| BCL2L11-429 | chr2:111121381-111121408 | BCL2L11-1392 | chr2:111153993-111154020 |
| BCL2L11-430 | chr2:111121388-111121415 | BCL2L11-1393 | chr2:111153999-111154026 |
| BCL2L11-431 | chr2:111121399-111121426 | BCL2L11-1394 | chr2:111161298-111161325 |
| BCL2L11-432 | chr2:111121401-111121428 | BCL2L11-1395 | chr2:111161299-111161326 |
| BCL2L11-433 | chr2:111121404-111121431 | BCL2L11-1396 | chr2:111161320-111161347 |
| BCL2L11-434 | chr2:111121405-111121432 | BCL2L11-1397 | chr2:111161356-111161383 |
| BCL2L11-435 | chr2:111121409-111121436 | BCL2L11-1398 | chr2:111161357-111161384 |
| BCL2L11-436 | chr2:111121410-111121437 | BCL2L11-1399 | chr2:111161362-111161389 |
| BCL2L11-437 | chr2:111121416-111121443 | BCL2L11-1400 | chr2:111161374-111161401 |
| BCL2L11-438 | chr2:111121423-111121450 | BCL2L11-1401 | chr2:111161375-111161402 |
| BCL2L11-439 | chr2:111121424-111121451 | BCL2L11-1402 | chr2:111161422-111161449 |
| BCL2L11-440 | chr2:111121429-111121456 | BCL2L11-1403 | chr2:111161428-111161455 |
| BCL2L11-441 | chr2:111121435-111121462 | BCL2L11-1404 | chr2:111161431-111161458 |
| BCL2L11-442 | chr2:111121436-111121463 | BCL2L11-1405 | chr2:111161442-111161469 |
| BCL2L11-443 | chr2:111121443-111121470 | BCL2L11-1406 | chr2:111161443-111161470 |
| BCL2L11-444 | chr2:111121448-111121475 | BCL2L11-1407 | chr2:111161450-111161477 |
| BCL2L11-445 | chr2:111121449-111121476 | BCL2L11-1408 | chr2:111161453-111161480 |
| BCL2L11-446 | chr2:111121452-111121479 | BCL2L11-1409 | chr2:111161454-111161481 |
| BCL2L11-447 | chr2:111121453-111121480 | BCL2L11-1410 | chr2:111161457-111161484 |
| BCL2L11-448 | chr2:111121456-111121483 | BCL2L11-1411 | chr2:111161477-111161504 |
| BCL2L11-449 | chr2:111121461-111121488 | BCL2L11-1412 | chr2:111161484-111161511 |
| BCL2L11-450 | chr2:111121472-111121499 | BCL2L11-1413 | chr2:111161485-111161512 |
| BCL2L11-451 | chr2:111121478-111121505 | BCL2L11-1414 | chr2:111161491-111161518 |
| BCL2L11-452 | chr2:111121484-111121511 | BCL2L11-1415 | chr2:111161497-111161524 |
| BCL2L11-453 | chr2:111121489-111121516 | BCL2L11-1416 | chr2:111161502-111161529 |
| BCL2L11-454 | chr2:111121490-111121517 | BCL2L11-1417 | chr2:111161523-111161550 |
| BCL2L11-455 | chr2:111121498-111121525 | BCL2L11-1418 | chr2:111161524-111161551 |
| BCL2L11-456 | chr2:111121508-111121535 | BCL2L11-1419 | chr2:111161541-111161568 |
| BCL2L11-457 | chr2:111121512-111121539 | BCL2L11-1420 | chr2:111161559-111161586 |
| BCL2L11-458 | chr2:111121513-111121540 | BCL2L11-1421 | chr2:111161569-111161596 |
| BCL2L11-459 | chr2:111121516-111121543 | BCL2L11-1422 | chr2:111161570-111161597 |
| BCL2L11-460 | chr2:111121517-111121544 | BCL2L11-1423 | chr2:111161589-111161616 |
| BCL2L11-461 | chr2:111121527-111121554 | BCL2L11-1424 | chr2:111161590-111161617 |
| BCL2L11-462 | chr2:111121533-111121560 | BCL2L11-1425 | chr2:111161613-111161640 |
| BCL2L11-463 | chr2:111121549-111121576 | BCL2L11-1426 | chr2:111161623-111161650 |
| BCL2L11-464 | chr2:111121552-111121579 | BCL2L11-1427 | chr2:111161640-111161667 |
| BCL2L11-465 | chr2:111121553-111121580 | BCL2L11-1428 | chr2:111161643-111161670 |
| BCL2L11-466 | chr2:111121555-111121582 | BCL2L11-1429 | chr2:111161644-111161671 |
| BCL2L11-467 | chr2:111121570-111121597 | BCL2L11-1430 | chr2:111164017-111164044 |
| BCL2L11-468 | chr2:111121572-111121599 | BCL2L11-1431 | chr2:111164018-111164045 |
| BCL2L11-469 | chr2:111121573-111121600 | BCL2L11-1432 | chr2:111164023-111164050 |
| BCL2L11-470 | chr2:111121579-111121606 | BCL2L11-1433 | chr2:111164041-111164068 |
| BCL2L11-471 | chr2:111121585-111121612 | BCL2L11-1434 | chr2:111164042-111164069 |
| BCL2L11-472 | chr2:111121586-111121613 | BCL2L11-1435 | chr2:111164071-111164098 |
| BCL2L11-473 | chr2:111121587-111121614 | BCL2L11-1436 | chr2:111164072-111164099 |
| BCL2L11-474 | chr2:111121588-111121615 | BCL2L11-1437 | chr2:111164073-111164100 |
| BCL2L11-475 | chr2:111121600-111121627 | BCL2L11-1438 | chr2:111164076-111164103 |
| BCL2L11-476 | chr2:111121603-111121630 | BCL2L11-1439 | chr2:111164077-111164104 |
| BCL2L11-477 | chr2:111121611-111121638 | BCL2L11-1440 | chr2:111164078-111164105 |
| BCL2L11-478 | chr2:111121612-111121639 | BCL2L11-1441 | chr2:111164081-111164108 |
| BCL2L11-479 | chr2:111121613-111121640 | BCL2L11-1442 | chr2:111164082-111164109 |
| BCL2L11-480 | chr2:111121620-111121647 | BCL2L11-1443 | chr2:111164083-111164110 |
| BCL2L11-481 | chr2:111121621-111121648 | BCL2L11-1444 | chr2:111164086-111164113 |
| BCL2L11-482 | chr2:111121631-111121658 | BCL2L11-1445 | chr2:111164087-111164114 |
| BCL2L11-483 | chr2:111121634-111121661 | BCL2L11-1446 | chr2:111164088-111164115 |
| BCL2L11-484 | chr2:111121642-111121669 | BCL2L11-1447 | chr2:111164089-111164116 |
| BCL2L11-485 | chr2:111121649-111121676 | BCL2L11-1448 | chr2:111164106-111164133 |
| BCL2L11-486 | chr2:111121650-111121677 | BCL2L11-1449 | chr2:111164117-111164144 |
| BCL2L11-487 | chr2:111121653-111121680 | BCL2L11-1450 | chr2:111164187-111164214 |
| BCL2L11-488 | chr2:111121675-111121702 | BCL2L11-1451 | chr2:111164192-111164219 |
| BCL2L11-489 | chr2:111121697-111121724 | BCL2L11-1452 | chr2:111164207-111164234 |
| BCL2L11-490 | chr2:111121703-111121730 | BCL2L11-1453 | chr2:111164208-111164235 |
| BCL2L11-491 | chr2:111121708-111121735 | BCL2L11-1454 | chr2:111164210-111164237 |
| BCL2L11-492 | chr2:111121709-111121736 | BCL2L11-1455 | chr2:111164218-111164245 |
| BCL2L11-493 | chr2:111121710-111121737 | BCL2L11-1456 | chr2:111164248-111164275 |
| BCL2L11-494 | chr2:111121711-111121738 | BCL2L11-1457 | chr2:111164256-111164283 |
| BCL2L11-495 | chr2:111121725-111121752 | BCL2L11-1458 | chr2:111164276-111164303 |
| BCL2L11-496 | chr2:111121739-111121766 | BCL2L11-1459 | chr2:111164282-111164309 |
| BCL2L11-497 | chr2:111121745-111121772 | BCL2L11-1460 | chr2:111164284-111164311 |
| BCL2L11-498 | chr2:111121746-111121773 | BCL2L11-1461 | chr2:111164291-111164318 |
| BCL2L11-499 | chr2:111121747-111121774 | BCL2L11-1462 | chr2:111164292-111164319 |
| BCL2L11-500 | chr2:111121766-111121793 | BCL2L11-1463 | chr2:111164298-111164325 |
| BCL2L11-501 | chr2:111121768-111121795 | BCL2L11-1464 | chr2:111164307-111164334 |
| BCL2L11-502 | chr2:111121775-111121802 | BCL2L11-1465 | chr2:111164314-111164341 |
| BCL2L11-503 | chr2:111121776-111121803 | BCL2L11-1466 | chr2:111164318-111164345 |
| BCL2L11-504 | chr2:111121777-111121804 | BCL2L11-1467 | chr2:111164321-111164348 |
| BCL2L11-505 | chr2:111121797-111121824 | BCL2L11-1468 | chr2:111164322-111164349 |
| BCL2L11-506 | chr2:111121802-111121829 | BCL2L11-1469 | chr2:111164323-111164350 |
| BCL2L11-507 | chr2:111121804-111121831 | BCL2L11-1470 | chr2:111164324-111164351 |
| BCL2L11-508 | chr2:111121810-111121837 | BCL2L11-1471 | chr2:111164328-111164355 |
| BCL2L11-509 | chr2:111121814-111121841 | BCL2L11-1472 | chr2:111164354-111164381 |
| BCL2L11-510 | chr2:111121818-111121845 | BCL2L11-1473 | chr2:111164358-111164385 |
| BCL2L11-511 | chr2:111121824-111121851 | BCL2L11-1474 | chr2:111164359-111164386 |
| BCL2L11-512 | chr2:111121825-111121852 | BCL2L11-1475 | chr2:111164373-111164400 |
| BCL2L11-513 | chr2:111121837-111121864 | BCL2L11-1476 | chr2:111164391-111164418 |
| BCL2L11-514 | chr2:111121847-111121874 | BCL2L11-1477 | chr2:111164401-111164428 |
| BCL2L11-515 | chr2:111121848-111121875 | BCL2L11-1478 | chr2:111164406-111164433 |
| BCL2L11-516 | chr2:111121849-111121876 | BCL2L11-1479 | chr2:111164423-111164450 |
| BCL2L11-517 | chr2:111121850-111121877 | BCL2L11-1480 | chr2:111164426-111164453 |
| BCL2L11-518 | chr2:111121851-111121878 | BCL2L11-1481 | chr2:111164427-111164454 |
| BCL2L11-519 | chr2:111121852-111121879 | BCL2L11-1482 | chr2:111164460-111164487 |
| BCL2L11-520 | chr2:111121857-111121884 | BCL2L11-1483 | chr2:111164468-111164495 |
| BCL2L11-521 | chr2:111121860-111121887 | BCL2L11-1484 | chr2:111164474-111164501 |
| BCL2L11-522 | chr2:111121864-111121891 | BCL2L11-1485 | chr2:111164482-111164509 |
| BCL2L11-523 | chr2:111121866-111121893 | BCL2L11-1486 | chr2:111164491-111164518 |
| BCL2L11-524 | chr2:111121878-111121905 | BCL2L11-1487 | chr2:111164532-111164559 |
| BCL2L11-525 | chr2:111121885-111121912 | BCL2L11-1488 | chr2:111164540-111164567 |
| BCL2L11-526 | chr2:111121886-111121913 | BCL2L11-1489 | chr2:111164541-111164568 |
| BCL2L11-527 | chr2:111121888-111121915 | BCL2L11-1490 | chr2:111164542-111164569 |
| BCL2L11-528 | chr2:111121890-111121917 | BCL2L11-1491 | chr2:111164543-111164570 |
| BCL2L11-529 | chr2:111121891-111121918 | BCL2L11-1492 | chr2:111164545-111164572 |
| BCL2L11-530 | chr2:111121897-111121924 | BCL2L11-1493 | chr2:111164548-111164575 |
| BCL2L11-531 | chr2:111121903-111121930 | BCL2L11-1494 | chr2:111164551-111164578 |
| BCL2L11-532 | chr2:111121907-111121934 | BCL2L11-1495 | chr2:111164590-111164617 |
| BCL2L11-533 | chr2:111121908-111121935 | BCL2L11-1496 | chr2:111164607-111164634 |
| BCL2L11-534 | chr2:111121914-111121941 | BCL2L11-1497 | chr2:111164621-111164648 |
| BCL2L11-535 | chr2:111121933-111121960 | BCL2L11-1498 | chr2:111164628-111164655 |
| BCL2L11-536 | chr2:111121940-111121967 | BCL2L11-1499 | chr2:111164635-111164662 |
| BCL2L11-537 | chr2:111121941-111121968 | BCL2L11-1500 | chr2:111164642-111164669 |
| BCL2L11-538 | chr2:111121942-111121969 | BCL2L11-1501 | chr2:111164658-111164685 |
| BCL2L11-539 | chr2:111121946-111121973 | BCL2L11-1502 | chr2:111164659-111164686 |
| BCL2L11-540 | chr2:111121950-111121977 | BCL2L11-1503 | chr2:111164660-111164687 |
| BCL2L11-541 | chr2:111121951-111121978 | BCL2L11-1504 | chr2:111164678-111164705 |
| BCL2L11-542 | chr2:111121952-111121979 | BCL2L11-1505 | chr2:111164698-111164725 |
| BCL2L11-543 | chr2:111121953-111121980 | BCL2L11-1506 | chr2:111164710-111164737 |
| BCL2L11-544 | chr2:111121956-111121983 | BCL2L11-1507 | chr2:111164711-111164738 |
| BCL2L11-545 | chr2:111121957-111121984 | BCL2L11-1508 | chr2:111164755-111164782 |
| BCL2L11-546 | chr2:111121968-111121995 | BCL2L11-1509 | chr2:111164756-111164783 |
| BCL2L11-547 | chr2:111121969-111121996 | BCL2L11-1510 | chr2:111164759-111164786 |
| BCL2L11-548 | chr2:111121983-111122010 | BCL2L11-1511 | chr2:111164760-111164787 |
| BCL2L11-549 | chr2:111121984-111122011 | BCL2L11-1512 | chr2:111164764-111164791 |
| BCL2L11-550 | chr2:111121985-111122012 | BCL2L11-1513 | chr2:111164765-111164792 |
| BCL2L11-551 | chr2:111121988-111122015 | BCL2L11-1514 | chr2:111164775-111164802 |
| BCL2L11-552 | chr2:111121989-111122016 | BCL2L11-1515 | chr2:111164784-111164811 |
| BCL2L11-553 | chr2:111121998-111122025 | BCL2L11-1516 | chr2:111164801-111164828 |
| BCL2L11-554 | chr2:111122001-111122028 | BCL2L11-1517 | chr2:111164806-111164833 |
| BCL2L11-555 | chr2:111122004-111122031 | BCL2L11-1518 | chr2:111164815-111164842 |
| BCL2L11-556 | chr2:111122005-111122032 | BCL2L11-1519 | chr2:111164816-111164843 |
| BCL2L11-557 | chr2:111122006-111122033 | BCL2L11-1520 | chr2:111164822-111164849 |
| BCL2L11-558 | chr2:111122007-111122034 | BCL2L11-1521 | chr2:111164823-111164850 |
| BCL2L11-559 | chr2:111122013-111122040 | BCL2L11-1522 | chr2:111164858-111164885 |
| BCL2L11-560 | chr2:111122021-111122048 | BCL2L11-1523 | chr2:111164862-111164889 |
| BCL2L11-561 | chr2:111122022-111122049 | BCL2L11-1524 | chr2:111164876-111164903 |
| BCL2L11-562 | chr2:111122025-111122052 | BCL2L11-1525 | chr2:111164881-111164908 |
| BCL2L11-563 | chr2:111122028-111122055 | BCL2L11-1526 | chr2:111164882-111164909 |
| BCL2L11-564 | chr2:111122029-111122056 | BCL2L11-1527 | chr2:111164902-111164929 |
| BCL2L11-565 | chr2:111122033-111122060 | BCL2L11-1528 | chr2:111164903-111164930 |
| BCL2L11-566 | chr2:111122034-111122061 | BCL2L11-1529 | chr2:111164958-111164985 |
| BCL2L11-567 | chr2:111122044-111122071 | BCL2L11-1530 | chr2:111164964-111164991 |
| BCL2L11-568 | chr2:111122047-111122074 | BCL2L11-1531 | chr2:111164983-111165010 |
| BCL2L11-569 | chr2:111122053-111122080 | BCL2L11-1532 | chr2:111164984-111165011 |
| BCL2L11-570 | chr2:111122069-111122096 | BCL2L11-1533 | chr2:111165013-111165040 |
| BCL2L11-571 | chr2:111122074-111122101 | BCL2L11-1534 | chr2:111165067-111165094 |
| BCL2L11-572 | chr2:111122075-111122102 | BCL2L11-1535 | chr2:111165079-111165106 |
| BCL2L11-573 | chr2:111122076-111122103 | BCL2L11-1536 | chr2:111165080-111165107 |
| BCL2L11-574 | chr2:111122079-111122106 | BCL2L11-1537 | chr2:111165090-111165117 |
| BCL2L11-575 | chr2:111122087-111122114 | BCL2L11-1538 | chr2:111165096-111165123 |
| BCL2L11-576 | chr2:111122091-111122118 | BCL2L11-1539 | chr2:111165128-111165155 |
| BCL2L11-577 | chr2:111122097-111122124 | BCL2L11-1540 | chr2:111165129-111165156 |
| BCL2L11-578 | chr2:111122106-111122133 | BCL2L11-1541 | chr2:111165132-111165159 |
| BCL2L11-579 | chr2:111122115-111122142 | BCL2L11-1542 | chr2:111165145-111165172 |
| BCL2L11-580 | chr2:111122124-111122151 | BCL2L11-1543 | chr2:111165197-111165224 |
| BCL2L11-581 | chr2:111122134-111122161 | BCL2L11-1544 | chr2:111165206-111165233 |
| BCL2L11-582 | chr2:111122137-111122164 | BCL2L11-1545 | chr2:111165208-111165235 |
| BCL2L11-583 | chr2:111122138-111122165 | BCL2L11-1546 | chr2:111165209-111165236 |
| BCL2L11-584 | chr2:111122158-111122185 | BCL2L11-1547 | chr2:111165210-111165237 |
| BCL2L11-585 | chr2:111122159-111122186 | BCL2L11-1548 | chr2:111165213-111165240 |
| BCL2L11-586 | chr2:111122162-111122189 | BCL2L11-1549 | chr2:111165224-111165251 |
| BCL2L11-587 | chr2:111122168-111122195 | BCL2L11-1550 | chr2:111165231-111165258 |
| BCL2L11-588 | chr2:111122175-111122202 | BCL2L11-1551 | chr2:111165245-111165272 |
| BCL2L11-589 | chr2:111122176-111122203 | BCL2L11-1552 | chr2:111165246-111165273 |
| BCL2L11-590 | chr2:111122177-111122204 | BCL2L11-1553 | chr2:111165250-111165277 |
| BCL2L11-591 | chr2:111122181-111122208 | BCL2L11-1554 | chr2:111165259-111165286 |
| BCL2L11-592 | chr2:111122191-111122218 | BCL2L11-1555 | chr2:111165260-111165287 |
| BCL2L11-593 | chr2:111122192-111122219 | BCL2L11-1556 | chr2:111165279-111165306 |
| BCL2L11-594 | chr2:111122196-111122223 | BCL2L11-1557 | chr2:111165280-111165307 |
| BCL2L11-595 | chr2:111122203-111122230 | BCL2L11-1558 | chr2:111165288-111165315 |
| BCL2L11-596 | chr2:111122209-111122236 | BCL2L11-1559 | chr2:111165291-111165318 |
| BCL2L11-597 | chr2:111122210-111122237 | BCL2L11-1560 | chr2:111165297-111165324 |
| BCL2L11-598 | chr2:111122211-111122238 | BCL2L11-1561 | chr2:111165312-111165339 |
| BCL2L11-599 | chr2:111122212-111122239 | BCL2L11-1562 | chr2:111165316-111165343 |
| BCL2L11-600 | chr2:111122216-111122243 | BCL2L11-1563 | chr2:111165317-111165344 |
| BCL2L11-601 | chr2:111122220-111122247 | BCL2L11-1564 | chr2:111165323-111165350 |
| BCL2L11-602 | chr2:111122221-111122248 | BCL2L11-1565 | chr2:111165325-111165352 |
| BCL2L11-603 | chr2:111122222-111122249 | BCL2L11-1566 | chr2:111165329-111165356 |
| BCL2L11-604 | chr2:111122227-111122254 | BCL2L11-1567 | chr2:111165334-111165361 |
| BCL2L11-605 | chr2:111122228-111122255 | BCL2L11-1568 | chr2:111165335-111165362 |
| BCL2L11-606 | chr2:111122229-111122256 | BCL2L11-1569 | chr2:111165336-111165363 |
| BCL2L11-607 | chr2:111122233-111122260 | BCL2L11-1570 | chr2:111165340-111165367 |
| BCL2L11-608 | chr2:111122241-111122268 | BCL2L11-1571 | chr2:111165341-111165368 |
| BCL2L11-609 | chr2:111122242-111122269 | BCL2L11-1572 | chr2:111165351-111165378 |
| BCL2L11-610 | chr2:111122247-111122274 | BCL2L11-1573 | chr2:111165357-111165384 |
| BCL2L11-611 | chr2:111122248-111122275 | BCL2L11-1574 | chr2:111165363-111165390 |
| BCL2L11-612 | chr2:111122250-111122277 | BCL2L11-1575 | chr2:111165379-111165406 |
| BCL2L11-613 | chr2:111122253-111122280 | BCL2L11-1576 | chr2:111165391-111165418 |
| BCL2L11-614 | chr2:111122261-111122288 | BCL2L11-1577 | chr2:111165394-111165421 |
| BCL2L11-615 | chr2:111122262-111122289 | BCL2L11-1578 | chr2:111165407-111165434 |
| BCL2L11-616 | chr2:111122271-111122298 | BCL2L11-1579 | chr2:111165419-111165446 |
| BCL2L11-617 | chr2:111122277-111122304 | BCL2L11-1580 | chr2:111165429-111165456 |
| BCL2L11-618 | chr2:111122284-111122311 | BCL2L11-1581 | chr2:111165433-111165460 |
| BCL2L11-619 | chr2:111122288-111122315 | BCL2L11-1582 | chr2:111165457-111165484 |
| BCL2L11-620 | chr2:111122293-111122320 | BCL2L11-1583 | chr2:111165463-111165490 |
| BCL2L11-621 | chr2:111122297-111122324 | BCL2L11-1584 | chr2:111165464-111165491 |
| BCL2L11-622 | chr2:111122300-111122327 | BCL2L11-1585 | chr2:111165470-111165497 |
| BCL2L11-623 | chr2:111122301-111122328 | BCL2L11-1586 | chr2:111165471-111165498 |
| BCL2L11-624 | chr2:111122312-111122339 | BCL2L11-1587 | chr2:111165472-111165499 |
| BCL2L11-625 | chr2:111122316-111122343 | BCL2L11-1588 | chr2:111165473-111165500 |
| BCL2L11-626 | chr2:111122323-111122350 | BCL2L11-1589 | chr2:111165479-111165506 |
| BCL2L11-627 | chr2:111122327-111122354 | BCL2L11-1590 | chr2:111165502-111165529 |
| BCL2L11-628 | chr2:111122335-111122362 | BCL2L11-1591 | chr2:111165510-111165537 |
| BCL2L11-629 | chr2:111122336-111122363 | BCL2L11-1592 | chr2:111165521-111165548 |
| BCL2L11-630 | chr2:111122340-111122367 | BCL2L11-1593 | chr2:111165525-111165552 |
| BCL2L11-631 | chr2:111122341-111122368 | BCL2L11-1594 | chr2:111165549-111165576 |
| BCL2L11-632 | chr2:111122345-111122372 | BCL2L11-1595 | chr2:111165550-111165577 |
| BCL2L11-633 | chr2:111122346-111122373 | BCL2L11-1596 | chr2:111165551-111165578 |
| BCL2L11-634 | chr2:111122347-111122374 | BCL2L11-1597 | chr2:111165559-111165586 |
| BCL2L11-635 | chr2:111122349-111122376 | BCL2L11-1598 | chr2:111165560-111165587 |
| BCL2L11-636 | chr2:111122353-111122380 | BCL2L11-1599 | chr2:111165561-111165588 |
| BCL2L11-637 | chr2:111122354-111122381 | BCL2L11-1600 | chr2:111165565-111165592 |
| BCL2L11-638 | chr2:111122362-111122389 | BCL2L11-1601 | chr2:111165589-111165616 |
| BCL2L11-639 | chr2:111122377-111122404 | BCL2L11-1602 | chr2:111165593-111165620 |
| BCL2L11-640 | chr2:111122382-111122409 | BCL2L11-1603 | chr2:111165594-111165621 |
| BCL2L11-641 | chr2:111122386-111122413 | BCL2L11-1604 | chr2:111165600-111165627 |
| BCL2L11-642 | chr2:111122390-111122417 | BCL2L11-1605 | chr2:111165607-111165634 |
| BCL2L11-643 | chr2:111122391-111122418 | BCL2L11-1606 | chr2:111165623-111165650 |
| BCL2L11-644 | chr2:111122399-111122426 | BCL2L11-1607 | chr2:111165624-111165651 |
| BCL2L11-645 | chr2:111122402-111122429 | BCL2L11-1608 | chr2:111165638-111165665 |
| BCL2L11-646 | chr2:111122404-111122431 | BCL2L11-1609 | chr2:111165639-111165666 |
| BCL2L11-647 | chr2:111122417-111122444 | BCL2L11-1610 | chr2:111165659-111165686 |
| BCL2L11-648 | chr2:111122420-111122447 | BCL2L11-1611 | chr2:111165660-111165687 |
| BCL2L11-649 | chr2:111122423-111122450 | BCL2L11-1612 | chr2:111165661-111165688 |
| BCL2L11-650 | chr2:111122426-111122453 | BCL2L11-1613 | chr2:111165664-111165691 |
| BCL2L11-651 | chr2:111122432-111122459 | BCL2L11-1614 | chr2:111165665-111165692 |
| BCL2L11-652 | chr2:111122453-111122480 | BCL2L11-1615 | chr2:111165670-111165697 |
| BCL2L11-653 | chr2:111122456-111122483 | BCL2L11-1616 | chr2:111165671-111165698 |
| BCL2L11-654 | chr2:111122469-111122496 | BCL2L11-1617 | chr2:111165681-111165708 |
| BCL2L11-655 | chr2:111122474-111122501 | BCL2L11-1618 | chr2:111165684-111165711 |
| BCL2L11-656 | chr2:111122484-111122511 | BCL2L11-1619 | chr2:111165685-111165712 |
| BCL2L11-657 | chr2:111122486-111122513 | BCL2L11-1620 | chr2:111165758-111165785 |
| BCL2L11-658 | chr2:111122489-111122516 | BCL2L11-1621 | chr2:111165814-111165841 |
| BCL2L11-659 | chr2:111122490-111122517 | BCL2L11-1622 | chr2:111165817-111165844 |
| BCL2L11-660 | chr2:111122497-111122524 | BCL2L11-1623 | chr2:111165832-111165859 |
| BCL2L11-661 | chr2:111122499-111122526 | BCL2L11-1624 | chr2:111165833-111165860 |
| BCL2L11-662 | chr2:111122502-111122529 | BCL2L11-1625 | chr2:111165845-111165872 |
| BCL2L11-663 | chr2:111122503-111122530 | BCL2L11-1626 | chr2:111165848-111165875 |
| BCL2L11-664 | chr2:111122504-111122531 | BCL2L11-1627 | chr2:111165924-111165951 |
| BCL2L11-665 | chr2:111122505-111122532 | BCL2L11-1628 | chr2:111165944-111165971 |
| BCL2L11-666 | chr2:111122508-111122535 | BCL2L11-1629 | chr2:111165968-111165995 |
| BCL2L11-667 | chr2:111122517-111122544 | BCL2L11-1630 | chr2:111165989-111166016 |
| BCL2L11-668 | chr2:111122523-111122550 | BCL2L11-1631 | chr2:111165990-111166017 |
| BCL2L11-669 | chr2:111122532-111122559 | BCL2L11-1632 | chr2:111165994-111166021 |
| BCL2L11-670 | chr2:111122533-111122560 | BCL2L11-1633 | chr2:111165999-111166026 |
| BCL2L11-671 | chr2:111122540-111122567 | BCL2L11-1634 | chr2:111166000-111166027 |
| BCL2L11-672 | chr2:111122541-111122568 | BCL2L11-1635 | chr2:111166001-111166028 |
| BCL2L11-673 | chr2:111122548-111122575 | BCL2L11-1636 | chr2:111166011-111166038 |
| BCL2L11-674 | chr2:111122552-111122579 | BCL2L11-1637 | chr2:111166019-111166046 |
| BCL2L11-675 | chr2:111122557-111122584 | BCL2L11-1638 | chr2:111166057-111166084 |
| BCL2L11-676 | chr2:111122562-111122589 | BCL2L11-1639 | chr2:111166060-111166087 |
| BCL2L11-677 | chr2:111122563-111122590 | BCL2L11-1640 | chr2:111166061-111166088 |
| BCL2L11-678 | chr2:111122564-111122591 | BCL2L11-1641 | chr2:111166062-111166089 |
| BCL2L11-679 | chr2:111122567-111122594 | BCL2L11-1642 | chr2:111166077-111166104 |
| BCL2L11-680 | chr2:111122571-111122598 | BCL2L11-1643 | chr2:111166078-111166105 |
| BCL2L11-681 | chr2:111122572-111122599 | BCL2L11-1644 | chr2:111166079-111166106 |
| BCL2L11-682 | chr2:111122574-111122601 | BCL2L11-1645 | chr2:111166092-111166119 |
| BCL2L11-683 | chr2:111122578-111122605 | BCL2L11-1646 | chr2:111166096-111166123 |
| BCL2L11-684 | chr2:111122580-111122607 | BCL2L11-1647 | chr2:111166100-111166127 |
| BCL2L11-685 | chr2:111122583-111122610 | BCL2L11-1648 | chr2:111166101-111166128 |
| BCL2L11-686 | chr2:111122586-111122613 | BCL2L11-1649 | chr2:111166109-111166136 |
| BCL2L11-687 | chr2:111122589-111122616 | BCL2L11-1650 | chr2:111166110-111166137 |
| BCL2L11-688 | chr2:111122590-111122617 | BCL2L11-1651 | chr2:111166118-111166145 |
| BCL2L11-689 | chr2:111122604-111122631 | BCL2L11-1652 | chr2:111166131-111166158 |
| BCL2L11-690 | chr2:111122605-111122632 | BCL2L11-1653 | chr2:111166138-111166165 |
| BCL2L11-691 | chr2:111122606-111122633 | BCL2L11-1654 | chr2:111166147-111166174 |
| BCL2L11-692 | chr2:111122609-111122636 | BCL2L11-1655 | chr2:111166153-111166180 |
| BCL2L11-693 | chr2:111122614-111122641 | BCL2L11-1656 | chr2:111166154-111166181 |
| BCL2L11-694 | chr2:111122615-111122642 | BCL2L11-1657 | chr2:111166159-111166186 |
| BCL2L11-695 | chr2:111122618-111122645 | BCL2L11-1658 | chr2:111166168-111166195 |
| BCL2L11-696 | chr2:111122621-111122648 | BCL2L11-1659 | chr2:111166184-111166211 |
| BCL2L11-697 | chr2:111122624-111122651 | BCL2L11-1660 | chr2:111166185-111166212 |
| BCL2L11-698 | chr2:111122627-111122654 | BCL2L11-1661 | chr2:111166186-111166213 |
| BCL2L11-699 | chr2:111122637-111122664 | BCL2L11-1662 | chr2:111166187-111166214 |
| BCL2L11-700 | chr2:111122640-111122667 | BCL2L11-1663 | chr2:111166190-111166217 |
| BCL2L11-701 | chr2:111122646-111122673 | BCL2L11-1664 | chr2:111166191-111166218 |
| BCL2L11-702 | chr2:111122650-111122677 | BCL2L11-1665 | chr2:111166198-111166225 |
| BCL2L11-703 | chr2:111122651-111122678 | BCL2L11-1666 | chr2:111166199-111166226 |
| BCL2L11-704 | chr2:111122652-111122679 | BCL2L11-1667 | chr2:111166213-111166240 |
| BCL2L11-705 | chr2:111122660-111122687 | BCL2L11-1668 | chr2:111166220-111166247 |
| BCL2L11-706 | chr2:111122661-111122688 | BCL2L11-1669 | chr2:111166239-111166266 |
| BCL2L11-707 | chr2:111122668-111122695 | BCL2L11-1670 | chr2:111166240-111166267 |
| BCL2L11-708 | chr2:111122673-111122700 | BCL2L11-1671 | chr2:111166246-111166273 |
| BCL2L11-709 | chr2:111122674-111122701 | BCL2L11-1672 | chr2:111166252-111166279 |
| BCL2L11-710 | chr2:111122680-111122707 | BCL2L11-1673 | chr2:111166253-111166280 |
| BCL2L11-711 | chr2:111122689-111122716 | BCL2L11-1674 | chr2:111166260-111166287 |
| BCL2L11-712 | chr2:111122697-111122724 | BCL2L11-1675 | chr2:111166261-111166288 |
| BCL2L11-713 | chr2:111122700-111122727 | BCL2L11-1676 | chr2:111166262-111166289 |
| BCL2L11-714 | chr2:111122707-111122734 | BCL2L11-1677 | chr2:111166264-111166291 |
| BCL2L11-715 | chr2:111122710-111122737 | BCL2L11-1678 | chr2:111166265-111166292 |
| BCL2L11-716 | chr2:111122711-111122738 | BCL2L11-1679 | chr2:111166270-111166297 |
| BCL2L11-717 | chr2:111122713-111122740 | BCL2L11-1680 | chr2:111166275-111166302 |
| BCL2L11-718 | chr2:111122714-111122741 | BCL2L11-1681 | chr2:111166276-111166303 |
| BCL2L11-719 | chr2:111122718-111122745 | BCL2L11-1682 | chr2:111166279-111166306 |
| BCL2L11-720 | chr2:111122720-111122747 | BCL2L11-1683 | chr2:111166286-111166313 |
| BCL2L11-721 | chr2:111122721-111122748 | BCL2L11-1684 | chr2:111166287-111166314 |
| BCL2L11-722 | chr2:111122722-111122749 | BCL2L11-1685 | chr2:111166293-111166320 |
| BCL2L11-723 | chr2:111122726-111122753 | BCL2L11-1686 | chr2:111166300-111166327 |
| BCL2L11-724 | chr2:111122731-111122758 | BCL2L11-1687 | chr2:111166301-111166328 |
| BCL2L11-725 | chr2:111122732-111122759 | BCL2L11-1688 | chr2:111166302-111166329 |
| BCL2L11-726 | chr2:111122748-111122775 | BCL2L11-1689 | chr2:111166307-111166334 |
| BCL2L11-727 | chr2:111122749-111122776 | BCL2L11-1690 | chr2:111166308-111166335 |
| BCL2L11-728 | chr2:111122750-111122777 | BCL2L11-1691 | chr2:111166312-111166339 |
| BCL2L11-729 | chr2:111122770-111122797 | BCL2L11-1692 | chr2:111166313-111166340 |
| BCL2L11-730 | chr2:111122771-111122798 | BCL2L11-1693 | chr2:111166322-111166349 |
| BCL2L11-731 | chr2:111122772-111122799 | BCL2L11-1694 | chr2:111166323-111166350 |
| BCL2L11-732 | chr2:111122786-111122813 | BCL2L11-1695 | chr2:111166335-111166362 |
| BCL2L11-733 | chr2:111122787-111122814 | BCL2L11-1696 | chr2:111166339-111166366 |
| BCL2L11-734 | chr2:111122793-111122820 | BCL2L11-1697 | chr2:111166340-111166367 |
| BCL2L11-735 | chr2:111122794-111122821 | BCL2L11-1698 | chr2:111166343-111166370 |
| BCL2L11-736 | chr2:111122797-111122824 | BCL2L11-1699 | chr2:111166351-111166378 |
| BCL2L11-737 | chr2:111122803-111122830 | BCL2L11-1700 | chr2:111166355-111166382 |
| BCL2L11-738 | chr2:111122807-111122834 | BCL2L11-1701 | chr2:111166356-111166383 |
| BCL2L11-739 | chr2:111122808-111122835 | BCL2L11-1702 | chr2:111166361-111166388 |
| BCL2L11-740 | chr2:111122809-111122836 | BCL2L11-1703 | chr2:111166363-111166390 |
| BCL2L11-741 | chr2:111122810-111122837 | BCL2L11-1704 | chr2:111166371-111166398 |
| BCL2L11-742 | chr2:111122811-111122838 | BCL2L11-1705 | chr2:111166374-111166401 |
| BCL2L11-743 | chr2:111122818-111122845 | BCL2L11-1706 | chr2:111166376-111166403 |
| BCL2L11-744 | chr2:111122822-111122849 | BCL2L11-1707 | chr2:111166397-111166424 |
| BCL2L11-745 | chr2:111122828-111122855 | BCL2L11-1708 | chr2:111166402-111166429 |
| BCL2L11-746 | chr2:111122829-111122856 | BCL2L11-1709 | chr2:111166418-111166445 |
| BCL2L11-747 | chr2:111122834-111122861 | BCL2L11-1710 | chr2:111166425-111166452 |
| BCL2L11-748 | chr2:111122835-111122862 | BCL2L11-1711 | chr2:111166441-111166468 |
| BCL2L11-749 | chr2:111122839-111122866 | BCL2L11-1712 | chr2:111166450-111166477 |
| BCL2L11-750 | chr2:111122841-111122868 | BCL2L11-1713 | chr2:111166455-111166482 |
| BCL2L11-751 | chr2:111122842-111122869 | BCL2L11-1714 | chr2:111166475-111166502 |
| BCL2L11-752 | chr2:111122848-111122875 | BCL2L11-1715 | chr2:111166514-111166541 |
| BCL2L11-753 | chr2:111122849-111122876 | BCL2L11-1716 | chr2:111166515-111166542 |
| BCL2L11-754 | chr2:111122850-111122877 | BCL2L11-1717 | chr2:111166516-111166543 |
| BCL2L11-755 | chr2:111122855-111122882 | BCL2L11-1718 | chr2:111166533-111166560 |
| BCL2L11-756 | chr2:111122862-111122889 | BCL2L11-1719 | chr2:111166536-111166563 |
| BCL2L11-757 | chr2:111122863-111122890 | BCL2L11-1720 | chr2:111166537-111166564 |
| BCL2L11-758 | chr2:111122867-111122894 | BCL2L11-1721 | chr2:111166556-111166583 |
| BCL2L11-759 | chr2:111122870-111122897 | BCL2L11-1722 | chr2:111166579-111166606 |
| BCL2L11-760 | chr2:111122884-111122911 | BCL2L11-1723 | chr2:111166593-111166620 |
| BCL2L11-761 | chr2:111122885-111122912 | BCL2L11-1724 | chr2:111166596-111166623 |
| BCL2L11-762 | chr2:111122889-111122916 | BCL2L11-1725 | chr2:111166597-111166624 |
| BCL2L11-763 | chr2:111122890-111122917 | BCL2L11-1726 | chr2:111166598-111166625 |
| BCL2L11-764 | chr2:111122891-111122918 | BCL2L11-1727 | chr2:111166626-111166653 |
| BCL2L11-765 | chr2:111122894-111122921 | BCL2L11-1728 | chr2:111166628-111166655 |
| BCL2L11-766 | chr2:111122907-111122934 | BCL2L11-1729 | chr2:111166637-111166664 |
| BCL2L11-767 | chr2:111122925-111122952 | BCL2L11-1730 | chr2:111166642-111166669 |
| BCL2L11-768 | chr2:111122926-111122953 | BCL2L11-1731 | chr2:111166643-111166670 |
| BCL2L11-769 | chr2:111122927-111122954 | BCL2L11-1732 | chr2:111166668-111166695 |
| BCL2L11-770 | chr2:111122930-111122957 | BCL2L11-1733 | chr2:111166720-111166747 |
| BCL2L11-771 | chr2:111122931-111122958 | BCL2L11-1734 | chr2:111166734-111166761 |
| BCL2L11-772 | chr2:111122938-111122965 | BCL2L11-1735 | chr2:111166735-111166762 |
| BCL2L11-773 | chr2:111122939-111122966 | BCL2L11-1736 | chr2:111166739-111166766 |
| BCL2L11-774 | chr2:111122943-111122970 | BCL2L11-1737 | chr2:111166740-111166767 |
| BCL2L11-775 | chr2:111122946-111122973 | BCL2L11-1738 | chr2:111166741-111166768 |
| BCL2L11-776 | chr2:111122949-111122976 | BCL2L11-1739 | chr2:111166742-111166769 |
| BCL2L11-777 | chr2:111122954-111122981 | BCL2L11-1740 | chr2:111166743-111166770 |
| BCL2L11-778 | chr2:111122964-111122991 | BCL2L11-1741 | chr2:111166744-111166771 |
| BCL2L11-779 | chr2:111122981-111123008 | BCL2L11-1742 | chr2:111166747-111166774 |
| BCL2L11-780 | chr2:111122988-111123015 | BCL2L11-1743 | chr2:111166748-111166775 |
| BCL2L11-781 | chr2:111122997-111123024 | BCL2L11-1744 | chr2:111166749-111166776 |
| BCL2L11-782 | chr2:111123002-111123029 | BCL2L11-1745 | chr2:111166750-111166777 |
| BCL2L11-783 | chr2:111123003-111123030 | BCL2L11-1746 | chr2:111166756-111166783 |
| BCL2L11-784 | chr2:111123010-111123037 | BCL2L11-1747 | chr2:111166800-111166827 |
| BCL2L11-785 | chr2:111123022-111123049 | BCL2L11-1748 | chr2:111166803-111166830 |
| BCL2L11-786 | chr2:111123045-111123072 | BCL2L11-1749 | chr2:111166842-111166869 |
| BCL2L11-787 | chr2:111123046-111123073 | BCL2L11-1750 | chr2:111166848-111166875 |
| BCL2L11-788 | chr2:111123049-111123076 | BCL2L11-1751 | chr2:111166849-111166876 |
| BCL2L11-789 | chr2:111123052-111123079 | BCL2L11-1752 | chr2:111166850-111166877 |
| BCL2L11-790 | chr2:111123055-111123082 | BCL2L11-1753 | chr2:111166865-111166892 |
| BCL2L11-791 | chr2:111123056-111123083 | BCL2L11-1754 | chr2:111166914-111166941 |
| BCL2L11-792 | chr2:111123057-111123084 | BCL2L11-1755 | chr2:111166940-111166967 |
| BCL2L11-793 | chr2:111123058-111123085 | BCL2L11-1756 | chr2:111166946-111166973 |
| BCL2L11-794 | chr2:111123060-111123087 | BCL2L11-1757 | chr2:111166989-111167016 |
| BCL2L11-795 | chr2:111123061-111123088 | BCL2L11-1758 | chr2:111167055-111167082 |
| BCL2L11-796 | chr2:111123062-111123089 | BCL2L11-1759 | chr2:111167145-111167172 |
| BCL2L11-797 | chr2:111123063-111123090 | BCL2L11-1760 | chr2:111167146-111167173 |
| BCL2L11-798 | chr2:111123080-111123107 | BCL2L11-1761 | chr2:111167153-111167180 |
| BCL2L11-799 | chr2:111123092-111123119 | BCL2L11-1762 | chr2:111167154-111167181 |
| BCL2L11-800 | chr2:111123094-111123121 | BCL2L11-1763 | chr2:111167159-111167186 |
| BCL2L11-801 | chr2:111123097-111123124 | BCL2L11-1764 | chr2:111167160-111167187 |
| BCL2L11-802 | chr2:111123104-111123131 | BCL2L11-1765 | chr2:111167161-111167188 |
| BCL2L11-803 | chr2:111123109-111123136 | BCL2L11-1766 | chr2:111167165-111167192 |
| BCL2L11-804 | chr2:111123110-111123137 | BCL2L11-1767 | chr2:111167169-111167196 |
| BCL2L11-805 | chr2:111123115-111123142 | BCL2L11-1768 | chr2:111167170-111167197 |
| BCL2L11-806 | chr2:111123116-111123143 | BCL2L11-1769 | chr2:111167171-111167198 |
| BCL2L11-807 | chr2:111123119-111123146 | BCL2L11-1770 | chr2:111167172-111167199 |
| BCL2L11-808 | chr2:111123120-111123147 | BCL2L11-1771 | chr2:111167173-111167200 |
| BCL2L11-809 | chr2:111123123-111123150 | BCL2L11-1772 | chr2:111167174-111167201 |
| BCL2L11-810 | chr2:111123124-111123151 | BCL2L11-1773 | chr2:111167181-111167208 |
| BCL2L11-811 | chr2:111123131-111123158 | BCL2L11-1774 | chr2:111167184-111167211 |
| BCL2L11-812 | chr2:111123132-111123159 | BCL2L11-1775 | chr2:111167204-111167231 |
| BCL2L11-813 | chr2:111123135-111123162 | BCL2L11-1776 | chr2:111167213-111167240 |
| BCL2L11-814 | chr2:111123153-111123180 | BCL2L11-1777 | chr2:111167214-111167241 |
| BCL2L11-815 | chr2:111123154-111123181 | BCL2L11-1778 | chr2:111167217-111167244 |
| BCL2L11-816 | chr2:111123168-111123195 | BCL2L11-1779 | chr2:111167224-111167251 |
| BCL2L11-817 | chr2:111123171-111123198 | BCL2L11-1780 | chr2:111167231-111167258 |
| BCL2L11-818 | chr2:111123177-111123204 | BCL2L11-1781 | chr2:111167232-111167259 |
| BCL2L11-819 | chr2:111123182-111123209 | BCL2L11-1782 | chr2:111167233-111167260 |
| BCL2L11-820 | chr2:111123187-111123214 | BCL2L11-1783 | chr2:111167236-111167263 |
| BCL2L11-821 | chr2:111123209-111123236 | BCL2L11-1784 | chr2:111167238-111167265 |
| BCL2L11-822 | chr2:111123237-111123264 | BCL2L11-1785 | chr2:111167249-111167276 |
| BCL2L11-823 | chr2:111123243-111123270 | BCL2L11-1786 | chr2:111167252-111167279 |
| BCL2L11-824 | chr2:111123248-111123275 | BCL2L11-1787 | chr2:111167255-111167282 |
| BCL2L11-825 | chr2:111123252-111123279 | BCL2L11-1788 | chr2:111167256-111167283 |
| BCL2L11-826 | chr2:111123253-111123280 | BCL2L11-1789 | chr2:111167261-111167288 |
| BCL2L11-827 | chr2:111123254-111123281 | BCL2L11-1790 | chr2:111167269-111167296 |
| BCL2L11-828 | chr2:111123264-111123291 | BCL2L11-1791 | chr2:111167270-111167297 |
| BCL2L11-829 | chr2:111123265-111123292 | BCL2L11-1792 | chr2:111167271-111167298 |
| BCL2L11-830 | chr2:111123266-111123293 | BCL2L11-1793 | chr2:111167303-111167330 |
| BCL2L11-831 | chr2:111123267-111123294 | BCL2L11-1794 | chr2:111167306-111167333 |
| BCL2L11-832 | chr2:111123270-111123297 | BCL2L11-1795 | chr2:111167307-111167334 |
| BCL2L11-833 | chr2:111123276-111123303 | BCL2L11-1796 | chr2:111167326-111167353 |
| BCL2L11-834 | chr2:111123277-111123304 | BCL2L11-1797 | chr2:111167330-111167357 |
| BCL2L11-835 | chr2:111123284-111123311 | BCL2L11-1798 | chr2:111167333-111167360 |
| BCL2L11-836 | chr2:111123294-111123321 | BCL2L11-1799 | chr2:111167373-111167400 |
| BCL2L11-837 | chr2:111123312-111123339 | BCL2L11-1800 | chr2:111167383-111167410 |
| BCL2L11-838 | chr2:111123316-111123343 | BCL2L11-1801 | chr2:111167384-111167411 |
| BCL2L11-839 | chr2:111123319-111123346 | BCL2L11-1802 | chr2:111167391-111167418 |
| BCL2L11-840 | chr2:111123330-111123357 | BCL2L11-1803 | chr2:111167404-111167431 |
| BCL2L11-841 | chr2:111123340-111123367 | BCL2L11-1804 | chr2:111167422-111167449 |
| BCL2L11-842 | chr2:111123341-111123368 | BCL2L11-1805 | chr2:111167435-111167462 |
| BCL2L11-843 | chr2:111123346-111123373 | BCL2L11-1806 | chr2:111167445-111167472 |
| BCL2L11-844 | chr2:111123348-111123375 | BCL2L11-1807 | chr2:111167466-111167493 |
| BCL2L11-845 | chr2:111123349-111123376 | BCL2L11-1808 | chr2:111167469-111167496 |
| BCL2L11-846 | chr2:111123354-111123381 | BCL2L11-1809 | chr2:111167470-111167497 |
| BCL2L11-847 | chr2:111123361-111123388 | BCL2L11-1810 | chr2:111167475-111167502 |
| BCL2L11-848 | chr2:111123365-111123392 | BCL2L11-1811 | chr2:111167523-111167550 |
| BCL2L11-849 | chr2:111123366-111123393 | BCL2L11-1812 | chr2:111167524-111167551 |
| BCL2L11-850 | chr2:111123370-111123397 | BCL2L11-1813 | chr2:111167546-111167573 |
| BCL2L11-851 | chr2:111123371-111123398 | BCL2L11-1814 | chr2:111167561-111167588 |
| BCL2L11-852 | chr2:111123372-111123399 | BCL2L11-1815 | chr2:111167567-111167594 |
| BCL2L11-853 | chr2:111123386-111123413 | BCL2L11-1816 | chr2:111167568-111167595 |
| BCL2L11-854 | chr2:111123399-111123426 | BCL2L11-1817 | chr2:111167569-111167596 |
| BCL2L11-855 | chr2:111123400-111123427 | BCL2L11-1818 | chr2:111167574-111167601 |
| BCL2L11-856 | chr2:111123422-111123449 | BCL2L11-1819 | chr2:111167575-111167602 |
| BCL2L11-857 | chr2:111123444-111123471 | BCL2L11-1820 | chr2:111167580-111167607 |
| BCL2L11-858 | chr2:111123491-111123518 | BCL2L11-1821 | chr2:111167584-111167611 |
| BCL2L11-859 | chr2:111123497-111123524 | BCL2L11-1822 | chr2:111167596-111167623 |
| BCL2L11-860 | chr2:111123506-111123533 | BCL2L11-1823 | chr2:111167597-111167624 |
| BCL2L11-861 | chr2:111123507-111123534 | BCL2L11-1824 | chr2:111167600-111167627 |
| BCL2L11-862 | chr2:111123508-111123535 | BCL2L11-1825 | chr2:111167601-111167628 |
| BCL2L11-863 | chr2:111123509-111123536 | BCL2L11-1826 | chr2:111167603-111167630 |
| BCL2L11-864 | chr2:111123539-111123566 | BCL2L11-1827 | chr2:111167606-111167633 |
| BCL2L11-865 | chr2:111123540-111123567 | BCL2L11-1828 | chr2:111167609-111167636 |
| BCL2L11-866 | chr2:111123546-111123573 | BCL2L11-1829 | chr2:111167622-111167649 |
| BCL2L11-867 | chr2:111123550-111123577 | BCL2L11-1830 | chr2:111167633-111167660 |
| BCL2L11-868 | chr2:111123562-111123589 | BCL2L11-1831 | chr2:111167639-111167666 |
| BCL2L11-869 | chr2:111123568-111123595 | BCL2L11-1832 | chr2:111167643-111167670 |
| BCL2L11-870 | chr2:111123569-111123596 | BCL2L11-1833 | chr2:111167650-111167677 |
| BCL2L11-871 | chr2:111123633-111123660 | BCL2L11-1834 | chr2:111167651-111167678 |
| BCL2L11-872 | chr2:111123637-111123664 | BCL2L11-1835 | chr2:111167654-111167681 |
| BCL2L11-873 | chr2:111123638-111123665 | BCL2L11-1836 | chr2:111167661-111167688 |
| BCL2L11-874 | chr2:111123722-111123749 | BCL2L11-1837 | chr2:111167668-111167695 |
| BCL2L11-875 | chr2:111123902-111123929 | BCL2L11-1838 | chr2:111167669-111167696 |
| BCL2L11-876 | chr2:111123903-111123930 | BCL2L11-1839 | chr2:111167670-111167697 |
| BCL2L11-877 | chr2:111123910-111123937 | BCL2L11-1840 | chr2:111167701-111167728 |
| BCL2L11-878 | chr2:111123911-111123938 | BCL2L11-1841 | chr2:111167702-111167729 |
| BCL2L11-879 | chr2:111123912-111123939 | BCL2L11-1842 | chr2:111167703-111167730 |
| BCL2L11-880 | chr2:111123913-111123940 | BCL2L11-1843 | chr2:111167704-111167731 |
| BCL2L11-881 | chr2:111123922-111123949 | BCL2L11-1844 | chr2:111167707-111167734 |
| BCL2L11-882 | chr2:111123923-111123950 | BCL2L11-1845 | chr2:111167708-111167735 |
| BCL2L11-883 | chr2:111123930-111123957 | BCL2L11-1846 | chr2:111167712-111167739 |
| BCL2L11-884 | chr2:111123931-111123958 | BCL2L11-1847 | chr2:111167722-111167749 |
| BCL2L11-885 | chr2:111123932-111123959 | BCL2L11-1848 | chr2:111167729-111167756 |
| BCL2L11-886 | chr2:111123939-111123966 | BCL2L11-1849 | chr2:111167772-111167799 |
| BCL2L11-887 | chr2:111123940-111123967 | BCL2L11-1850 | chr2:111167775-111167802 |
| BCL2L11-888 | chr2:111123941-111123968 | BCL2L11-1851 | chr2:111167776-111167803 |
| BCL2L11-889 | chr2:111123944-111123971 | BCL2L11-1852 | chr2:111167779-111167806 |
| BCL2L11-890 | chr2:111123948-111123975 | BCL2L11-1853 | chr2:111167780-111167807 |
| BCL2L11-891 | chr2:111123952-111123979 | BCL2L11-1854 | chr2:111167781-111167808 |
| BCL2L11-892 | chr2:111123953-111123980 | BCL2L11-1855 | chr2:111167785-111167812 |
| BCL2L11-893 | chr2:111123958-111123985 | BCL2L11-1856 | chr2:111167786-111167813 |
| BCL2L11-894 | chr2:111123959-111123986 | BCL2L11-1857 | chr2:111167787-111167814 |
| BCL2L11-895 | chr2:111123969-111123996 | BCL2L11-1858 | chr2:111167788-111167815 |
| BCL2L11-896 | chr2:111123975-111124002 | BCL2L11-1859 | chr2:111167791-111167818 |
| BCL2L11-897 | chr2:111123976-111124003 | BCL2L11-1860 | chr2:111167792-111167819 |
| BCL2L11-898 | chr2:111123977-111124004 | BCL2L11-1861 | chr2:111167803-111167830 |
| BCL2L11-899 | chr2:111124127-111124154 | BCL2L11-1862 | chr2:111167817-111167844 |
| BCL2L11-900 | chr2:111124128-111124155 | BCL2L11-1863 | chr2:111167837-111167864 |
| BCL2L11-901 | chr2:111124135-111124162 | BCL2L11-1864 | chr2:111167838-111167865 |
| BCL2L11-902 | chr2:111124149-111124176 | BCL2L11-1865 | chr2:111167839-111167866 |
| BCL2L11-903 | chr2:111124150-111124177 | BCL2L11-1866 | chr2:111167840-111167867 |
| BCL2L11-904 | chr2:111124153-111124180 | BCL2L11-1867 | chr2:111167841-111167868 |
| BCL2L11-905 | chr2:111124170-111124197 | BCL2L11-1868 | chr2:111167844-111167871 |
| BCL2L11-906 | chr2:111124180-111124207 | BCL2L11-1869 | chr2:111167848-111167875 |
| BCL2L11-907 | chr2:111124218-111124245 | BCL2L11-1870 | chr2:111167849-111167876 |
| BCL2L11-908 | chr2:111124219-111124246 | BCL2L11-1871 | chr2:111167850-111167877 |
| BCL2L11-909 | chr2:111124220-111124247 | BCL2L11-1872 | chr2:111167853-111167880 |
| BCL2L11-910 | chr2:111124230-111124257 | BCL2L11-1873 | chr2:111167859-111167886 |
| BCL2L11-911 | chr2:111124231-111124258 | BCL2L11-1874 | chr2:111167864-111167891 |
| BCL2L11-912 | chr2:111124232-111124259 | BCL2L11-1875 | chr2:111167865-111167892 |
| BCL2L11-913 | chr2:111124234-111124261 | BCL2L11-1876 | chr2:111167878-111167905 |
| BCL2L11-914 | chr2:111124235-111124262 | BCL2L11-1877 | chr2:111167879-111167906 |
| BCL2L11-915 | chr2:111128509-111128536 | BCL2L11-1878 | chr2:111167880-111167907 |
| BCL2L11-916 | chr2:111128513-111128540 | BCL2L11-1879 | chr2:111167888-111167915 |
| BCL2L11-917 | chr2:111128528-111128555 | BCL2L11-1880 | chr2:111167889-111167916 |
| BCL2L11-918 | chr2:111128538-111128565 | BCL2L11-1881 | chr2:111167899-111167926 |
| BCL2L11-919 | chr2:111128539-111128566 | BCL2L11-1882 | chr2:111167910-111167937 |
| BCL2L11-920 | chr2:111128542-111128569 | BCL2L11-1883 | chr2:111167913-111167940 |
| BCL2L11-921 | chr2:111128546-111128573 | BCL2L11-1884 | chr2:111167924-111167951 |
| BCL2L11-922 | chr2:111128547-111128574 | BCL2L11-1885 | chr2:111167927-111167954 |
| BCL2L11-923 | chr2:111128554-111128581 | BCL2L11-1886 | chr2:111167943-111167970 |
| BCL2L11-924 | chr2:111128555-111128582 | BCL2L11-1887 | chr2:111167955-111167982 |
| BCL2L11-925 | chr2:111128558-111128585 | BCL2L11-1888 | chr2:111167960-111167987 |
| BCL2L11-926 | chr2:111128577-111128604 | BCL2L11-1889 | chr2:111167964-111167991 |
| BCL2L11-927 | chr2:111128586-111128613 | BCL2L11-1890 | chr2:111167965-111167992 |
| BCL2L11-928 | chr2:111128597-111128624 | BCL2L11-1891 | chr2:111167978-111168005 |
| BCL2L11-929 | chr2:111128657-111128684 | BCL2L11-1892 | chr2:111167985-111168012 |
| BCL2L11-930 | chr2:111128672-111128699 | BCL2L11-1893 | chr2:111167986-111168013 |
| BCL2L11-931 | chr2:111128700-111128727 | BCL2L11-1894 | chr2:111167987-111168014 |
| BCL2L11-932 | chr2:111128703-111128730 | BCL2L11-1895 | chr2:111167988-111168015 |
| BCL2L11-933 | chr2:111128706-111128733 | BCL2L11-1896 | chr2:111167991-111168018 |
| BCL2L11-934 | chr2:111128713-111128740 | BCL2L11-1897 | chr2:111167992-111168019 |
| BCL2L11-935 | chr2:111128733-111128760 | BCL2L11-1898 | chr2:111167993-111168020 |
| BCL2L11-936 | chr2:111128754-111128781 | BCL2L11-1899 | chr2:111167994-111168021 |
| BCL2L11-937 | chr2:111128765-111128792 | BCL2L11-1900 | chr2:111167996-111168023 |
| BCL2L11-938 | chr2:111128771-111128798 | BCL2L11-1901 | chr2:111168027-111168054 |
| BCL2L11-939 | chr2:111128774-111128801 | BCL2L11-1902 | chr2:111168028-111168055 |
| BCL2L11-940 | chr2:111128781-111128808 | BCL2L11-1903 | chr2:111168029-111168056 |
| BCL2L11-941 | chr2:111128782-111128809 | BCL2L11-1904 | chr2:111168090-111168117 |
| BCL2L11-942 | chr2:111128788-111128815 | BCL2L11-1905 | chr2:111168099-111168126 |
| BCL2L11-943 | chr2:111128789-111128816 | BCL2L11-1906 | chr2:111168100-111168127 |
| BCL2L11-944 | chr2:111130011-111130038 | BCL2L11-1907 | chr2:111168103-111168130 |
| BCL2L11-945 | chr2:111130016-111130043 | BCL2L11-1908 | chr2:111168104-111168131 |
| BCL2L11-946 | chr2:111130021-111130048 | BCL2L11-1909 | chr2:111168168-111168195 |
| BCL2L11-947 | chr2:111130027-111130054 | BCL2L11-1910 | chr2:111168181-111168208 |
| BCL2L11-948 | chr2:111130036-111130063 | BCL2L11-1911 | chr2:111168183-111168210 |
| BCL2L11-949 | chr2:111130044-111130071 | BCL2L11-1912 | chr2:111168211-111168238 |
| BCL2L11-950 | chr2:111130045-111130072 | BCL2L11-1913 | chr2:111168213-111168240 |
| BCL2L11-951 | chr2:111130048-111130075 | BCL2L11-1914 | chr2:111168214-111168241 |
| BCL2L11-952 | chr2:111130049-111130076 | BCL2L11-1915 | chr2:111168246-111168273 |
| BCL2L11-953 | chr2:111130050-111130077 | BCL2L11-1916 | chr2:111168251-111168278 |
| BCL2L11-954 | chr2:111130148-111130175 | BCL2L11-1917 | chr2:111168261-111168288 |
| BCL2L11-955 | chr2:111130182-111130209 | BCL2L11-1918 | chr2:111168275-111168302 |
| BCL2L11-956 | chr2:111130186-111130213 | BCL2L11-1919 | chr2:111168276-111168303 |
| BCL2L11-957 | chr2:111130189-111130216 | BCL2L11-1920 | chr2:111168284-111168311 |
| BCL2L11-958 | chr2:111130195-111130222 | BCL2L11-1921 | chr2:111168306-111168333 |
| BCL2L11-959 | chr2:111130196-111130223 | BCL2L11-1922 | chr2:111168307-111168334 |
| BCL2L11-960 | chr2:111130212-111130239 | BCL2L11-1923 | chr2:111168319-111168346 |
| BCL2L11-961 | chr2:111130213-111130240 | BCL2L11-1924 | chr2:111168320-111168347 |
| BCL2L11-962 | chr2:111130214-111130241 | BCL2L11-1925 | chr2:111168354-111168381 |
| BCL2L11-963 | chr2:111130218-111130245 | | |

**Table 2B Genomic coordinates of the human PTPN2 gene**

| Target ID | Target Coordinate Range | Target ID | Target Coordinate Range |
|---|---|---|---|
| PTPN2-1 | chr18:12785503-12785530 | PTPN2-702 | chr18:12862638-12862665 |
| PTPN2-2 | chr18:12785506-12785533 | PTPN2-703 | chr18:12862639-12862666 |
| PTPN2-3 | chr18:12785508-12785535 | PTPN2-704 | chr18:12862646-12862673 |
| PTPN2-4 | chr18:12785557-12785584 | PTPN2-705 | chr18:12862657-12862684 |
| PTPN2-5 | chr18:12785558-12785585 | PTPN2-706 | chr18:12862658-12862685 |
| PTPN2-6 | chr18:12785559-12785586 | PTPN2-707 | chr18:12862659-12862686 |
| PTPN2-7 | chr18:12785604-12785631 | PTPN2-708 | chr18:12862660-12862687 |
| PTPN2-8 | chr18:12785621-12785648 | PTPN2-709 | chr18:12862661-12862688 |
| PTPN2-9 | chr18:12785625-12785652 | PTPN2-710 | chr18:12862666-12862693 |
| PTPN2-10 | chr18:12785628-12785655 | PTPN2-711 | chr18:12862667-12862694 |
| PTPN2-11 | chr18:12785629-12785656 | PTPN2-712 | chr18:12862668-12862695 |
| PTPN2-12 | chr18:12785646-12785673 | PTPN2-713 | chr18:12862671-12862698 |
| PTPN2-13 | chr18:12785667-12785694 | PTPN2-714 | chr18:12862672-12862699 |
| PTPN2-14 | chr18:12785668-12785695 | PTPN2-715 | chr18:12862678-12862705 |
| PTPN2-15 | chr18:12785669-12785696 | PTPN2-716 | chr18:12862682-12862709 |
| PTPN2-16 | chr18:12785682-12785709 | PTPN2-717 | chr18:12862703-12862730 |
| PTPN2-17 | chr18:12785693-12785720 | PTPN2-718 | chr18:12862712-12862739 |
| PTPN2-18 | chr18:12785694-12785721 | PTPN2-719 | chr18:12862716-12862743 |
| PTPN2-19 | chr18:12785720-12785747 | PTPN2-720 | chr18:12862727-12862754 |
| PTPN2-20 | chr18:12785725-12785752 | PTPN2-721 | chr18:12862744-12862771 |
| PTPN2-21 | chr18:12785726-12785753 | PTPN2-722 | chr18:12862756-12862783 |
| PTPN2-22 | chr18:12785730-12785757 | PTPN2-723 | chr18:12862757-12862784 |
| PTPN2-23 | chr18:12785733-12785760 | PTPN2-724 | chr18:12862767-12862794 |
| PTPN2-24 | chr18:12785744-12785771 | PTPN2-725 | chr18:12862798-12862825 |
| PTPN2-25 | chr18:12785754-12785781 | PTPN2-726 | chr18:12862810-12862837 |
| PTPN2-26 | chr18:12785800-12785827 | PTPN2-727 | chr18:12862811-12862838 |
| PTPN2-27 | chr18:12785816-12785843 | PTPN2-728 | chr18:12862825-12862852 |
| PTPN2-28 | chr18:12785844-12785871 | PTPN2-729 | chr18:12862834-12862861 |
| PTPN2-29 | chr18:12785887-12785914 | PTPN2-730 | chr18:12863080-12863107 |
| PTPN2-30 | chr18:12785901-12785928 | PTPN2-731 | chr18:12863106-12863133 |
| PTPN2-31 | chr18:12785922-12785949 | PTPN2-732 | chr18:12863113-12863140 |
| PTPN2-32 | chr18:12792182-12792209 | PTPN2-733 | chr18:12863142-12863169 |
| PTPN2-33 | chr18:12792185-12792212 | PTPN2-734 | chr18:12863146-12863173 |
| PTPN2-34 | chr18:12792192-12792219 | PTPN2-735 | chr18:12863147-12863174 |
| PTPN2-35 | chr18:12792204-12792231 | PTPN2-736 | chr18:12863148-12863175 |
| PTPN2-36 | chr18:12792246-12792273 | PTPN2-737 | chr18:12863153-12863180 |
| PTPN2-37 | chr18:12792267-12792294 | PTPN2-738 | chr18:12863154-12863181 |
| PTPN2-38 | chr18:12792301-12792328 | PTPN2-739 | chr18:12863162-12863189 |
| PTPN2-39 | chr18:12792305-12792332 | PTPN2-740 | chr18:12863164-12863191 |
| PTPN2-40 | chr18:12792315-12792342 | PTPN2-741 | chr18:12863174-12863201 |
| PTPN2-41 | chr18:12792324-12792351 | PTPN2-742 | chr18:12863181-12863208 |
| PTPN2-42 | chr18:12792326-12792353 | PTPN2-743 | chr18:12863189-12863216 |
| PTPN2-43 | chr18:12792350-12792377 | PTPN2-744 | chr18:12863190-12863217 |
| PTPN2-44 | chr18:12792351-12792378 | PTPN2-745 | chr18:12863193-12863220 |
| PTPN2-45 | chr18:12792364-12792391 | PTPN2-746 | chr18:12863196-12863223 |
| PTPN2-46 | chr18:12792370-12792397 | PTPN2-747 | chr18:12863202-12863229 |
| PTPN2-47 | chr18:12792373-12792400 | PTPN2-748 | chr18:12863204-12863231 |
| PTPN2-48 | chr18:12792374-12792401 | PTPN2-749 | chr18:12863206-12863233 |
| PTPN2-49 | chr18:12792384-12792411 | PTPN2-750 | chr18:12863217-12863244 |
| PTPN2-50 | chr18:12792390-12792417 | PTPN2-751 | chr18:12863218-12863245 |
| PTPN2-51 | chr18:12792391-12792418 | PTPN2-752 | chr18:12863221-12863248 |
| PTPN2-52 | chr18:12792392-12792419 | PTPN2-753 | chr18:12863222-12863249 |
| PTPN2-53 | chr18:12792396-12792423 | PTPN2-754 | chr18:12863240-12863267 |
| PTPN2-54 | chr18:12792399-12792426 | PTPN2-755 | chr18:12863241-12863268 |
| PTPN2-55 | chr18:12792402-12792429 | PTPN2-756 | chr18:12863259-12863286 |
| PTPN2-56 | chr18:12792405-12792432 | PTPN2-757 | chr18:12863263-12863290 |
| PTPN2-57 | chr18:12792406-12792433 | PTPN2-758 | chr18:12863284-12863311 |
| PTPN2-58 | chr18:12792433-12792460 | PTPN2-759 | chr18:12863290-12863317 |
| PTPN2-59 | chr18:12792436-12792463 | PTPN2-760 | chr18:12863291-12863318 |
| PTPN2-60 | chr18:12792441-12792468 | PTPN2-761 | chr18:12863292-12863319 |
| PTPN2-61 | chr18:12792442-12792469 | PTPN2-762 | chr18:12863293-12863320 |
| PTPN2-62 | chr18:12792446-12792473 | PTPN2-763 | chr18:12863296-12863323 |
| PTPN2-63 | chr18:12792447-12792474 | PTPN2-764 | chr18:12863299-12863326 |
| PTPN2-64 | chr18:12792462-12792489 | PTPN2-765 | chr18:12863326-12863353 |
| PTPN2-65 | chr18:12792467-12792494 | PTPN2-766 | chr18:12863327-12863354 |
| PTPN2-66 | chr18:12792481-12792508 | PTPN2-767 | chr18:12863330-12863357 |
| PTPN2-67 | chr18:12792490-12792517 | PTPN2-768 | chr18:12863340-12863367 |
| PTPN2-68 | chr18:12792502-12792529 | PTPN2-769 | chr18:12863342-12863369 |
| PTPN2-69 | chr18:12792507-12792534 | PTPN2-770 | chr18:12863343-12863370 |
| PTPN2-70 | chr18:12792513-12792540 | PTPN2-771 | chr18:12863359-12863386 |
| PTPN2-71 | chr18:12792522-12792549 | PTPN2-772 | chr18:12863408-12863435 |
| PTPN2-72 | chr18:12792523-12792550 | PTPN2-773 | chr18:12863409-12863436 |
| PTPN2-73 | chr18:12792524-12792551 | PTPN2-774 | chr18:12863413-12863440 |
| PTPN2-74 | chr18:12792525-12792552 | PTPN2-775 | chr18:12863422-12863449 |
| PTPN2-75 | chr18:12792526-12792553 | PTPN2-776 | chr18:12863431-12863458 |
| PTPN2-76 | chr18:12792529-12792556 | PTPN2-777 | chr18:12863450-12863477 |
| PTPN2-77 | chr18:12792535-12792562 | PTPN2-778 | chr18:12863451-12863478 |
| PTPN2-78 | chr18:12792539-12792566 | PTPN2-779 | chr18:12863473-12863500 |
| PTPN2-79 | chr18:12792551-12792578 | PTPN2-780 | chr18:12863474-12863501 |
| PTPN2-80 | chr18:12792566-12792593 | PTPN2-781 | chr18:12863476-12863503 |
| PTPN2-81 | chr18:12792569-12792596 | PTPN2-782 | chr18:12863489-12863516 |
| PTPN2-82 | chr18:12792574-12792601 | PTPN2-783 | chr18:12863498-12863525 |
| PTPN2-83 | chr18:12792575-12792602 | PTPN2-784 | chr18:12863508-12863535 |
| PTPN2-84 | chr18:12792579-12792606 | PTPN2-785 | chr18:12863512-12863539 |
| PTPN2-85 | chr18:12792580-12792607 | PTPN2-786 | chr18:12863526-12863553 |
| PTPN2-86 | chr18:12792588-12792615 | PTPN2-787 | chr18:12863527-12863554 |
| PTPN2-87 | chr18:12792609-12792636 | PTPN2-788 | chr18:12863528-12863555 |
| PTPN2-88 | chr18:12792613-12792640 | PTPN2-789 | chr18:12863529-12863556 |
| PTPN2-89 | chr18:12792631-12792658 | PTPN2-790 | chr18:12863530-12863557 |
| PTPN2-90 | chr18:12792632-12792659 | PTPN2-791 | chr18:12863531-12863558 |
| PTPN2-91 | chr18:12792658-12792685 | PTPN2-792 | chr18:12863532-12863559 |
| PTPN2-92 | chr18:12792659-12792686 | PTPN2-793 | chr18:12863533-12863560 |
| PTPN2-93 | chr18:12792672-12792699 | PTPN2-794 | chr18:12863534-12863561 |
| PTPN2-94 | chr18:12792675-12792702 | PTPN2-795 | chr18:12863535-12863562 |
| PTPN2-95 | chr18:12792681-12792708 | PTPN2-796 | chr18:12863536-12863563 |
| PTPN2-96 | chr18:12792700-12792727 | PTPN2-797 | chr18:12863537-12863564 |
| PTPN2-97 | chr18:12792704-12792731 | PTPN2-798 | chr18:12863544-12863571 |
| PTPN2-98 | chr18:12792707-12792734 | PTPN2-799 | chr18:12863545-12863572 |
| PTPN2-99 | chr18:12792710-12792737 | PTPN2-800 | chr18:12863546-12863573 |
| PTPN2-100 | chr18:12792713-12792740 | PTPN2-801 | chr18:12863566-12863593 |
| PTPN2-101 | chr18:12792737-12792764 | PTPN2-802 | chr18:12863589-12863616 |
| PTPN2-102 | chr18:12792738-12792765 | PTPN2-803 | chr18:12863611-12863638 |
| PTPN2-103 | chr18:12792741-12792768 | PTPN2-804 | chr18:12863612-12863639 |
| PTPN2-104 | chr18:12792744-12792771 | PTPN2-805 | chr18:12863613-12863640 |
| PTPN2-105 | chr18:12792749-12792776 | PTPN2-806 | chr18:12863617-12863644 |
| PTPN2-106 | chr18:12792750-12792777 | PTPN2-807 | chr18:12863623-12863650 |
| PTPN2-107 | chr18:12792754-12792781 | PTPN2-808 | chr18:12863624-12863651 |
| PTPN2-108 | chr18:12792770-12792797 | PTPN2-809 | chr18:12863643-12863670 |
| PTPN2-109 | chr18:12792775-12792802 | PTPN2-810 | chr18:12863644-12863671 |
| PTPN2-110 | chr18:12792779-12792806 | PTPN2-811 | chr18:12863645-12863672 |
| PTPN2-111 | chr18:12792789-12792816 | PTPN2-812 | chr18:12863648-12863675 |
| PTPN2-112 | chr18:12792798-12792825 | PTPN2-813 | chr18:12863649-12863676 |
| PTPN2-113 | chr18:12792800-12792827 | PTPN2-814 | chr18:12863654-12863681 |
| PTPN2-114 | chr18:12792816-12792843 | PTPN2-815 | chr18:12863665-12863692 |
| PTPN2-115 | chr18:12792821-12792848 | PTPN2-816 | chr18:12863677-12863704 |
| PTPN2-116 | chr18:12792833-12792860 | PTPN2-817 | chr18:12863687-12863714 |
| PTPN2-117 | chr18:12792834-12792861 | PTPN2-818 | chr18:12863695-12863722 |
| PTPN2-118 | chr18:12792835-12792862 | PTPN2-819 | chr18:12863699-12863726 |
| PTPN2-119 | chr18:12792836-12792863 | PTPN2-820 | chr18:12863711-12863738 |
| PTPN2-120 | chr18:12792842-12792869 | PTPN2-821 | chr18:12863726-12863753 |
| PTPN2-121 | chr18:12792845-12792872 | PTPN2-822 | chr18:12863727-12863754 |
| PTPN2-122 | chr18:12792848-12792875 | PTPN2-823 | chr18:12863728-12863755 |
| PTPN2-123 | chr18:12792849-12792876 | PTPN2-824 | chr18:12863729-12863756 |
| PTPN2-124 | chr18:12792876-12792903 | PTPN2-825 | chr18:12863732-12863759 |
| PTPN2-125 | chr18:12792879-12792906 | PTPN2-826 | chr18:12863748-12863775 |
| PTPN2-126 | chr18:12792884-12792911 | PTPN2-827 | chr18:12863751-12863778 |
| PTPN2-127 | chr18:12792885-12792912 | PTPN2-828 | chr18:12863758-12863785 |
| PTPN2-128 | chr18:12792889-12792916 | PTPN2-829 | chr18:12863763-12863790 |
| PTPN2-129 | chr18:12792914-12792941 | PTPN2-830 | chr18:12863767-12863794 |
| PTPN2-130 | chr18:12792915-12792942 | PTPN2-831 | chr18:12863816-12863843 |
| PTPN2-131 | chr18:12792916-12792943 | PTPN2-832 | chr18:12863844-12863871 |
| PTPN2-132 | chr18:12792917-12792944 | PTPN2-833 | chr18:12863880-12863907 |
| PTPN2-133 | chr18:12792929-12792956 | PTPN2-834 | chr18:12863899-12863926 |
| PTPN2-134 | chr18:12792934-12792961 | PTPN2-835 | chr18:12863915-12863942 |
| PTPN2-135 | chr18:12792946-12792973 | PTPN2-836 | chr18:12863949-12863976 |
| PTPN2-136 | chr18:12792960-12792987 | PTPN2-837 | chr18:12863990-12864017 |
| PTPN2-137 | chr18:12792987-12793014 | PTPN2-838 | chr18:12864005-12864032 |
| PTPN2-138 | chr18:12792998-12793025 | PTPN2-839 | chr18:12864038-12864065 |
| PTPN2-139 | chr18:12793006-12793033 | PTPN2-840 | chr18:12864045-12864072 |
| PTPN2-140 | chr18:12793007-12793034 | PTPN2-841 | chr18:12864062-12864089 |
| PTPN2-141 | chr18:12793010-12793037 | PTPN2-842 | chr18:12864073-12864100 |
| PTPN2-142 | chr18:12793019-12793046 | PTPN2-843 | chr18:12864074-12864101 |
| PTPN2-143 | chr18:12793026-12793053 | PTPN2-844 | chr18:12864081-12864108 |
| PTPN2-144 | chr18:12793040-12793067 | PTPN2-845 | chr18:12864089-12864116 |
| PTPN2-145 | chr18:12793062-12793089 | PTPN2-846 | chr18:12864116-12864143 |
| PTPN2-146 | chr18:12793081-12793108 | PTPN2-847 | chr18:12864136-12864163 |
| PTPN2-147 | chr18:12793086-12793113 | PTPN2-848 | chr18:12864167-12864194 |
| PTPN2-148 | chr18:12793089-12793116 | PTPN2-849 | chr18:12864216-12864243 |
| PTPN2-149 | chr18:12793124-12793151 | PTPN2-850 | chr18:12864226-12864253 |
| PTPN2-150 | chr18:12793141-12793168 | PTPN2-851 | chr18:12864265-12864292 |
| PTPN2-151 | chr18:12793225-12793252 | PTPN2-852 | chr18:12864277-12864304 |
| PTPN2-152 | chr18:12793371-12793398 | PTPN2-853 | chr18:12864284-12864311 |
| PTPN2-153 | chr18:12793387-12793414 | PTPN2-854 | chr18:12864287-12864314 |
| PTPN2-154 | chr18:12793388-12793415 | PTPN2-855 | chr18:12864294-12864321 |
| PTPN2-155 | chr18:12793389-12793416 | PTPN2-856 | chr18:12864302-12864329 |
| PTPN2-156 | chr18:12793407-12793434 | PTPN2-857 | chr18:12864319-12864346 |
| PTPN2-157 | chr18:12793408-12793435 | PTPN2-858 | chr18:12864331-12864358 |
| PTPN2-158 | chr18:12793447-12793474 | PTPN2-859 | chr18:12864347-12864374 |
| PTPN2-159 | chr18:12793470-12793497 | PTPN2-860 | chr18:12864362-12864389 |
| PTPN2-160 | chr18:12793472-12793499 | PTPN2-861 | chr18:12864367-12864394 |
| PTPN2-161 | chr18:12793481-12793508 | PTPN2-862 | chr18:12864368-12864395 |
| PTPN2-162 | chr18:12793490-12793517 | PTPN2-863 | chr18:12864393-12864420 |
| PTPN2-163 | chr18:12793491-12793518 | PTPN2-864 | chr18:12864401-12864428 |
| PTPN2-164 | chr18:12793500-12793527 | PTPN2-865 | chr18:12864414-12864441 |
| PTPN2-165 | chr18:12793508-12793535 | PTPN2-866 | chr18:12864419-12864446 |
| PTPN2-166 | chr18:12793541-12793568 | PTPN2-867 | chr18:12864420-12864447 |
| PTPN2-167 | chr18:12793542-12793569 | PTPN2-868 | chr18:12864428-12864455 |
| PTPN2-168 | chr18:12793543-12793570 | PTPN2-869 | chr18:12864437-12864464 |
| PTPN2-169 | chr18:12793621-12793648 | PTPN2-870 | chr18:12864439-12864466 |
| PTPN2-170 | chr18:12793637-12793664 | PTPN2-871 | chr18:12864463-12864490 |
| PTPN2-171 | chr18:12793674-12793701 | PTPN2-872 | chr18:12864464-12864491 |
| PTPN2-172 | chr18:12793791-12793818 | PTPN2-873 | chr18:12864477-12864504 |
| PTPN2-173 | chr18:12793792-12793819 | PTPN2-874 | chr18:12864483-12864510 |
| PTPN2-174 | chr18:12793823-12793850 | PTPN2-875 | chr18:12864486-12864513 |
| PTPN2-175 | chr18:12793824-12793851 | PTPN2-876 | chr18:12864503-12864530 |
| PTPN2-176 | chr18:12793878-12793905 | PTPN2-877 | chr18:12864504-12864531 |
| PTPN2-177 | chr18:12793896-12793923 | PTPN2-878 | chr18:12864505-12864532 |
| PTPN2-178 | chr18:12793900-12793927 | PTPN2-879 | chr18:12864509-12864536 |
| PTPN2-179 | chr18:12793935-12793962 | PTPN2-880 | chr18:12864515-12864542 |
| PTPN2-180 | chr18:12793943-12793970 | PTPN2-881 | chr18:12864518-12864545 |
| PTPN2-181 | chr18:12793946-12793973 | PTPN2-882 | chr18:12864519-12864546 |
| PTPN2-182 | chr18:12793958-12793985 | PTPN2-883 | chr18:12864531-12864558 |
| PTPN2-183 | chr18:12793963-12793990 | PTPN2-884 | chr18:12864533-12864560 |
| PTPN2-184 | chr18:12794035-12794062 | PTPN2-885 | chr18:12864542-12864569 |
| PTPN2-185 | chr18:12794061-12794088 | PTPN2-886 | chr18:12864543-12864570 |
| PTPN2-186 | chr18:12794062-12794089 | PTPN2-887 | chr18:12864546-12864573 |
| PTPN2-187 | chr18:12794063-12794090 | PTPN2-888 | chr18:12864549-12864576 |
| PTPN2-188 | chr18:12794071-12794098 | PTPN2-889 | chr18:12864554-12864581 |
| PTPN2-189 | chr18:12794074-12794101 | PTPN2-890 | chr18:12864555-12864582 |
| PTPN2-190 | chr18:12794101-12794128 | PTPN2-891 | chr18:12864563-12864590 |
| PTPN2-191 | chr18:12794102-12794129 | PTPN2-892 | chr18:12864564-12864591 |
| PTPN2-192 | chr18:12794116-12794143 | PTPN2-893 | chr18:12864565-12864592 |
| PTPN2-193 | chr18:12794130-12794157 | PTPN2-894 | chr18:12864584-12864611 |
| PTPN2-194 | chr18:12794160-12794187 | PTPN2-895 | chr18:12864588-12864615 |
| PTPN2-195 | chr18:12794161-12794188 | PTPN2-896 | chr18:12864598-12864625 |
| PTPN2-196 | chr18:12794169-12794196 | PTPN2-897 | chr18:12864607-12864634 |
| PTPN2-197 | chr18:12794178-12794205 | PTPN2-898 | chr18:12864625-12864652 |
| PTPN2-198 | chr18:12794214-12794241 | PTPN2-899 | chr18:12864630-12864657 |
| PTPN2-199 | chr18:12794215-12794242 | PTPN2-900 | chr18:12864640-12864667 |
| PTPN2-200 | chr18:12794216-12794243 | PTPN2-901 | chr18:12864641-12864668 |
| PTPN2-201 | chr18:12794236-12794263 | PTPN2-902 | chr18:12864642-12864669 |
| PTPN2-202 | chr18:12794237-12794264 | PTPN2-903 | chr18:12864643-12864670 |
| PTPN2-203 | chr18:12794257-12794284 | PTPN2-904 | chr18:12864646-12864673 |
| PTPN2-204 | chr18:12794258-12794285 | PTPN2-905 | chr18:12864649-12864676 |
| PTPN2-205 | chr18:12794308-12794335 | PTPN2-906 | chr18:12864652-12864679 |
| PTPN2-206 | chr18:12794312-12794339 | PTPN2-907 | chr18:12864655-12864682 |
| PTPN2-207 | chr18:12794324-12794351 | PTPN2-908 | chr18:12864656-12864683 |
| PTPN2-208 | chr18:12794328-12794355 | PTPN2-909 | chr18:12864668-12864695 |
| PTPN2-209 | chr18:12794340-12794367 | PTPN2-910 | chr18:12864683-12864710 |
| PTPN2-210 | chr18:12794347-12794374 | PTPN2-911 | chr18:12864686-12864713 |
| PTPN2-211 | chr18:12794348-12794375 | PTPN2-912 | chr18:12864691-12864718 |
| PTPN2-212 | chr18:12794349-12794376 | PTPN2-913 | chr18:12864696-12864723 |
| PTPN2-213 | chr18:12794371-12794398 | PTPN2-914 | chr18:12864706-12864733 |
| PTPN2-214 | chr18:12794380-12794407 | PTPN2-915 | chr18:12864731-12864758 |
| PTPN2-215 | chr18:12794383-12794410 | PTPN2-916 | chr18:12864743-12864770 |
| PTPN2-216 | chr18:12794504-12794531 | PTPN2-917 | chr18:12864746-12864773 |
| PTPN2-217 | chr18:12794527-12794554 | PTPN2-918 | chr18:12864775-12864802 |
| PTPN2-218 | chr18:12794534-12794561 | PTPN2-919 | chr18:12864776-12864803 |
| PTPN2-219 | chr18:12794556-12794583 | PTPN2-920 | chr18:12864783-12864810 |
| PTPN2-220 | chr18:12794573-12794600 | PTPN2-921 | chr18:12864795-12864822 |
| PTPN2-221 | chr18:12794577-12794604 | PTPN2-922 | chr18:12864832-12864859 |
| PTPN2-222 | chr18:12794580-12794607 | PTPN2-923 | chr18:12864833-12864860 |
| PTPN2-223 | chr18:12801862-12801889 | PTPN2-924 | chr18:12864848-12864875 |
| PTPN2-224 | chr18:12801869-12801896 | PTPN2-925 | chr18:12864849-12864876 |
| PTPN2-225 | chr18:12801876-12801903 | PTPN2-926 | chr18:12864925-12864952 |
| PTPN2-226 | chr18:12801882-12801909 | PTPN2-927 | chr18:12864942-12864969 |
| PTPN2-227 | chr18:12801890-12801917 | PTPN2-928 | chr18:12864943-12864970 |
| PTPN2-228 | chr18:12801899-12801926 | PTPN2-929 | chr18:12864944-12864971 |
| PTPN2-229 | chr18:12801905-12801932 | PTPN2-930 | chr18:12864961-12864988 |
| PTPN2-230 | chr18:12801906-12801933 | PTPN2-931 | chr18:12865007-12865034 |
| PTPN2-231 | chr18:12801935-12801962 | PTPN2-932 | chr18:12865063-12865090 |
| PTPN2-232 | chr18:12802204-12802231 | PTPN2-933 | chr18:12865065-12865092 |
| PTPN2-233 | chr18:12802234-12802261 | PTPN2-934 | chr18:12865108-12865135 |
| PTPN2-234 | chr18:12814086-12814113 | PTPN2-935 | chr18:12865117-12865144 |
| PTPN2-235 | chr18:12814098-12814125 | PTPN2-936 | chr18:12865120-12865147 |
| PTPN2-236 | chr18:12814099-12814126 | PTPN2-937 | chr18:12865121-12865148 |
| PTPN2-237 | chr18:12814108-12814135 | PTPN2-938 | chr18:12865139-12865166 |
| PTPN2-238 | chr18:12814368-12814395 | PTPN2-939 | chr18:12865144-12865171 |
| PTPN2-239 | chr18:12814376-12814403 | PTPN2-940 | chr18:12865167-12865194 |
| PTPN2-240 | chr18:12814398-12814425 | PTPN2-941 | chr18:12865179-12865206 |
| PTPN2-241 | chr18:12814399-12814426 | PTPN2-942 | chr18:12865180-12865207 |
| PTPN2-242 | chr18:12814454-12814481 | PTPN2-943 | chr18:12865183-12865210 |
| PTPN2-243 | chr18:12817038-12817065 | PTPN2-944 | chr18:12865186-12865213 |
| PTPN2-244 | chr18:12817039-12817066 | PTPN2-945 | chr18:12865189-12865216 |
| PTPN2-245 | chr18:12817054-12817081 | PTPN2-946 | chr18:12865192-12865219 |
| PTPN2-246 | chr18:12817071-12817098 | PTPN2-947 | chr18:12865193-12865220 |
| PTPN2-247 | chr18:12817092-12817119 | PTPN2-948 | chr18:12865194-12865221 |
| PTPN2-248 | chr18:12817358-12817385 | PTPN2-949 | chr18:12865195-12865222 |
| PTPN2-249 | chr18:12817359-12817386 | PTPN2-950 | chr18:12865216-12865243 |
| PTPN2-250 | chr18:12817361-12817388 | PTPN2-951 | chr18:12865217-12865244 |
| PTPN2-251 | chr18:12817442-12817469 | PTPN2-952 | chr18:12865235-12865262 |
| PTPN2-252 | chr18:12817461-12817488 | PTPN2-953 | chr18:12865258-12865285 |
| PTPN2-253 | chr18:12819088-12819115 | PTPN2-954 | chr18:12865259-12865286 |
| PTPN2-254 | chr18:12819113-12819140 | PTPN2-955 | chr18:12865260-12865287 |
| PTPN2-255 | chr18:12819137-12819164 | PTPN2-956 | chr18:12865261-12865288 |
| PTPN2-256 | chr18:12819145-12819172 | PTPN2-957 | chr18:12865267-12865294 |
| PTPN2-257 | chr18:12819146-12819173 | PTPN2-958 | chr18:12865272-12865299 |
| PTPN2-258 | chr18:12819302-12819329 | PTPN2-959 | chr18:12865275-12865302 |
| PTPN2-259 | chr18:12819305-12819332 | PTPN2-960 | chr18:12865288-12865315 |
| PTPN2-260 | chr18:12819342-12819369 | PTPN2-961 | chr18:12865302-12865329 |
| PTPN2-261 | chr18:12819367-12819394 | PTPN2-962 | chr18:12865303-12865330 |
| PTPN2-262 | chr18:12825712-12825739 | PTPN2-963 | chr18:12865306-12865333 |
| PTPN2-263 | chr18:12825766-12825793 | PTPN2-964 | chr18:12865315-12865342 |
| PTPN2-264 | chr18:12825767-12825794 | PTPN2-965 | chr18:12865316-12865343 |
| PTPN2-265 | chr18:12825768-12825795 | PTPN2-966 | chr18:12865317-12865344 |
| PTPN2-266 | chr18:12825776-12825803 | PTPN2-967 | chr18:12865318-12865345 |
| PTPN2-267 | chr18:12825782-12825809 | PTPN2-968 | chr18:12865319-12865346 |
| PTPN2-268 | chr18:12825998-12826025 | PTPN2-969 | chr18:12865335-12865362 |
| PTPN2-269 | chr18:12826024-12826051 | PTPN2-970 | chr18:12865338-12865365 |
| PTPN2-270 | chr18:12830817-12830844 | PTPN2-971 | chr18:12865382-12865409 |
| PTPN2-271 | chr18:12830821-12830848 | PTPN2-972 | chr18:12865384-12865411 |
| PTPN2-272 | chr18:12830828-12830855 | PTPN2-973 | chr18:12865393-12865420 |
| PTPN2-273 | chr18:12830829-12830856 | PTPN2-974 | chr18:12865403-12865430 |
| PTPN2-274 | chr18:12830841-12830868 | PTPN2-975 | chr18:12865407-12865434 |
| PTPN2-275 | chr18:12830845-12830872 | PTPN2-976 | chr18:12865435-12865462 |
| PTPN2-276 | chr18:12830869-12830896 | PTPN2-977 | chr18:12865438-12865465 |
| PTPN2-277 | chr18:12830870-12830897 | PTPN2-978 | chr18:12865439-12865466 |
| PTPN2-278 | chr18:12830871-12830898 | PTPN2-979 | chr18:12865440-12865467 |
| PTPN2-279 | chr18:12831040-12831067 | PTPN2-980 | chr18:12865448-12865475 |
| PTPN2-280 | chr18:12831041-12831068 | PTPN2-981 | chr18:12865475-12865502 |
| PTPN2-281 | chr18:12831046-12831073 | PTPN2-982 | chr18:12865479-12865506 |
| PTPN2-282 | chr18:12831047-12831074 | PTPN2-983 | chr18:12865482-12865509 |
| PTPN2-283 | chr18:12831068-12831095 | PTPN2-984 | chr18:12865485-12865512 |
| PTPN2-284 | chr18:12831075-12831102 | PTPN2-985 | chr18:12865488-12865515 |
| PTPN2-285 | chr18:12831079-12831106 | PTPN2-986 | chr18:12865489-12865516 |
| PTPN2-286 | chr18:12831098-12831125 | PTPN2-987 | chr18:12865490-12865517 |
| PTPN2-287 | chr18:12831100-12831127 | PTPN2-988 | chr18:12865492-12865519 |
| PTPN2-288 | chr18:12831101-12831128 | PTPN2-989 | chr18:12865503-12865530 |
| PTPN2-289 | chr18:12831107-12831134 | PTPN2-990 | chr18:12865524-12865551 |
| PTPN2-290 | chr18:12831122-12831149 | PTPN2-991 | chr18:12865526-12865553 |
| PTPN2-291 | chr18:12831123-12831150 | PTPN2-992 | chr18:12865535-12865562 |
| PTPN2-292 | chr18:12831126-12831153 | PTPN2-993 | chr18:12865545-12865572 |
| PTPN2-293 | chr18:12831127-12831154 | PTPN2-994 | chr18:12865549-12865576 |
| PTPN2-294 | chr18:12836684-12836711 | PTPN2-995 | chr18:12865551-12865578 |
| PTPN2-295 | chr18:12836695-12836722 | PTPN2-996 | chr18:12865554-12865581 |
| PTPN2-296 | chr18:12836721-12836748 | PTPN2-997 | chr18:12865555-12865582 |
| PTPN2-297 | chr18:12836748-12836775 | PTPN2-998 | chr18:12865568-12865595 |
| PTPN2-298 | chr18:12836913-12836940 | PTPN2-999 | chr18:12865569-12865596 |
| PTPN2-299 | chr18:12836938-12836965 | PTPN2-1000 | chr18:12865574-12865601 |
| PTPN2-300 | chr18:12840587-12840614 | PTPN2-1001 | chr18:12865590-12865617 |
| PTPN2-301 | chr18:12840588-12840615 | PTPN2-1002 | chr18:12865592-12865619 |
| PTPN2-302 | chr18:12840611-12840638 | PTPN2-1003 | chr18:12865593-12865620 |
| PTPN2-303 | chr18:12840612-12840639 | PTPN2-1004 | chr18:12865601-12865628 |
| PTPN2-304 | chr18:12840618-12840645 | PTPN2-1005 | chr18:12865611-12865638 |
| PTPN2-305 | chr18:12840619-12840646 | PTPN2-1006 | chr18:12865615-12865642 |
| PTPN2-306 | chr18:12840686-12840713 | PTPN2-1007 | chr18:12865634-12865661 |
| PTPN2-307 | chr18:12840806-12840833 | PTPN2-1008 | chr18:12865635-12865662 |
| PTPN2-308 | chr18:12840808-12840835 | PTPN2-1009 | chr18:12865640-12865667 |
| PTPN2-309 | chr18:12840825-12840852 | PTPN2-1010 | chr18:12865641-12865668 |
| PTPN2-310 | chr18:12840842-12840869 | PTPN2-1011 | chr18:12865658-12865685 |
| PTPN2-311 | chr18:12840848-12840875 | PTPN2-1012 | chr18:12865659-12865686 |
| PTPN2-312 | chr18:12840863-12840890 | PTPN2-1013 | chr18:12865667-12865694 |
| PTPN2-313 | chr18:12840864-12840891 | PTPN2-1014 | chr18:12865694-12865721 |
| PTPN2-314 | chr18:12840865-12840892 | PTPN2-1015 | chr18:12865698-12865725 |
| PTPN2-315 | chr18:12840876-12840903 | PTPN2-1016 | chr18:12865704-12865731 |
| PTPN2-316 | chr18:12840886-12840913 | PTPN2-1017 | chr18:12865709-12865736 |
| PTPN2-317 | chr18:12840894-12840921 | PTPN2-1018 | chr18:12865710-12865737 |
| PTPN2-318 | chr18:12840915-12840942 | PTPN2-1019 | chr18:12865727-12865754 |
| PTPN2-319 | chr18:12840926-12840953 | PTPN2-1020 | chr18:12865730-12865757 |
| PTPN2-320 | chr18:12840929-12840956 | PTPN2-1021 | chr18:12865733-12865760 |
| PTPN2-321 | chr18:12840949-12840976 | PTPN2-1022 | chr18:12865736-12865763 |
| PTPN2-322 | chr18:12840950-12840977 | PTPN2-1023 | chr18:12865749-12865776 |
| PTPN2-323 | chr18:12840988-12841015 | PTPN2-1024 | chr18:12865750-12865777 |
| PTPN2-324 | chr18:12840989-12841016 | PTPN2-1025 | chr18:12865774-12865801 |
| PTPN2-325 | chr18:12840998-12841025 | PTPN2-1026 | chr18:12865785-12865812 |
| PTPN2-326 | chr18:12841008-12841035 | PTPN2-1027 | chr18:12865795-12865822 |
| PTPN2-327 | chr18:12841031-12841058 | PTPN2-1028 | chr18:12865799-12865826 |
| PTPN2-328 | chr18:12841079-12841106 | PTPN2-1029 | chr18:12865800-12865827 |
| PTPN2-329 | chr18:12841095-12841122 | PTPN2-1030 | chr18:12865801-12865828 |
| PTPN2-330 | chr18:12841096-12841123 | PTPN2-1031 | chr18:12865804-12865831 |
| PTPN2-331 | chr18:12841100-12841127 | PTPN2-1032 | chr18:12865805-12865832 |
| PTPN2-332 | chr18:12841127-12841154 | PTPN2-1033 | chr18:12865806-12865833 |
| PTPN2-333 | chr18:12841145-12841172 | PTPN2-1034 | chr18:12865811-12865838 |
| PTPN2-334 | chr18:12841149-12841176 | PTPN2-1035 | chr18:12865812-12865839 |
| PTPN2-335 | chr18:12841150-12841177 | PTPN2-1036 | chr18:12865818-12865845 |
| PTPN2-336 | chr18:12841151-12841178 | PTPN2-1037 | chr18:12865824-12865851 |
| PTPN2-337 | chr18:12841155-12841182 | PTPN2-1038 | chr18:12865860-12865887 |
| PTPN2-338 | chr18:12841166-12841193 | PTPN2-1039 | chr18:12865896-12865923 |
| PTPN2-339 | chr18:12841167-12841194 | PTPN2-1040 | chr18:12865918-12865945 |
| PTPN2-340 | chr18:12841182-12841209 | PTPN2-1041 | chr18:12865930-12865957 |
| PTPN2-341 | chr18:12841184-12841211 | PTPN2-1042 | chr18:12865931-12865958 |
| PTPN2-342 | chr18:12841191-12841218 | PTPN2-1043 | chr18:12865942-12865969 |
| PTPN2-343 | chr18:12841192-12841219 | PTPN2-1044 | chr18:12865958-12865985 |
| PTPN2-344 | chr18:12841197-12841224 | PTPN2-1045 | chr18:12866004-12866031 |
| PTPN2-345 | chr18:12841205-12841232 | PTPN2-1046 | chr18:12866011-12866038 |
| PTPN2-346 | chr18:12841209-12841236 | PTPN2-1047 | chr18:12866021-12866048 |
| PTPN2-347 | chr18:12841214-12841241 | PTPN2-1048 | chr18:12866024-12866051 |
| PTPN2-348 | chr18:12841228-12841255 | PTPN2-1049 | chr18:12866042-12866069 |
| PTPN2-349 | chr18:12841232-12841259 | PTPN2-1050 | chr18:12866135-12866162 |
| PTPN2-350 | chr18:12841240-12841267 | PTPN2-1051 | chr18:12866136-12866163 |
| PTPN2-351 | chr18:12841249-12841276 | PTPN2-1052 | chr18:12866141-12866168 |
| PTPN2-352 | chr18:12841250-12841277 | PTPN2-1053 | chr18:12866144-12866171 |
| PTPN2-353 | chr18:12841269-12841296 | PTPN2-1054 | chr18:12866155-12866182 |
| PTPN2-354 | chr18:12841270-12841297 | PTPN2-1055 | chr18:12866159-12866186 |
| PTPN2-355 | chr18:12841273-12841300 | PTPN2-1056 | chr18:12866171-12866198 |
| PTPN2-356 | chr18:12841280-12841307 | PTPN2-1057 | chr18:12866172-12866199 |
| PTPN2-357 | chr18:12841281-12841308 | PTPN2-1058 | chr18:12866175-12866202 |
| PTPN2-358 | chr18:12841282-12841309 | PTPN2-1059 | chr18:12866178-12866205 |
| PTPN2-359 | chr18:12841283-12841310 | PTPN2-1060 | chr18:12866181-12866208 |
| PTPN2-360 | chr18:12841284-12841311 | PTPN2-1061 | chr18:12866185-12866212 |
| PTPN2-361 | chr18:12841291-12841318 | PTPN2-1062 | chr18:12866186-12866213 |
| PTPN2-362 | chr18:12841292-12841319 | PTPN2-1063 | chr18:12866188-12866215 |
| PTPN2-363 | chr18:12841302-12841329 | PTPN2-1064 | chr18:12866197-12866224 |
| PTPN2-364 | chr18:12841303-12841330 | PTPN2-1065 | chr18:12866202-12866229 |
| PTPN2-365 | chr18:12841304-12841331 | PTPN2-1066 | chr18:12866203-12866230 |
| PTPN2-366 | chr18:12841310-12841337 | PTPN2-1067 | chr18:12866220-12866247 |
| PTPN2-367 | chr18:12841316-12841343 | PTPN2-1068 | chr18:12866239-12866266 |
| PTPN2-368 | chr18:12841320-12841347 | PTPN2-1069 | chr18:12866243-12866270 |
| PTPN2-369 | chr18:12841321-12841348 | PTPN2-1070 | chr18:12866262-12866289 |
| PTPN2-370 | chr18:12841322-12841349 | PTPN2-1071 | chr18:12866263-12866290 |
| PTPN2-371 | chr18:12841344-12841371 | PTPN2-1072 | chr18:12866264-12866291 |
| PTPN2-372 | chr18:12841349-12841376 | PTPN2-1073 | chr18:12866269-12866296 |
| PTPN2-373 | chr18:12841350-12841377 | PTPN2-1074 | chr18:12866270-12866297 |
| PTPN2-374 | chr18:12841351-12841378 | PTPN2-1075 | chr18:12866275-12866302 |
| PTPN2-375 | chr18:12841352-12841379 | PTPN2-1076 | chr18:12866278-12866305 |
| PTPN2-376 | chr18:12841401-12841428 | PTPN2-1077 | chr18:12866281-12866308 |
| PTPN2-377 | chr18:12841419-12841446 | PTPN2-1078 | chr18:12866282-12866309 |
| PTPN2-378 | chr18:12841435-12841462 | PTPN2-1079 | chr18:12866293-12866320 |
| PTPN2-379 | chr18:12841459-12841486 | PTPN2-1080 | chr18:12866304-12866331 |
| PTPN2-380 | chr18:12841467-12841494 | PTPN2-1081 | chr18:12866305-12866332 |
| PTPN2-381 | chr18:12841484-12841511 | PTPN2-1082 | chr18:12866306-12866333 |
| PTPN2-382 | chr18:12841487-12841514 | PTPN2-1083 | chr18:12866309-12866336 |
| PTPN2-383 | chr18:12841490-12841517 | PTPN2-1084 | chr18:12866312-12866339 |
| PTPN2-384 | chr18:12841491-12841518 | PTPN2-1085 | chr18:12866315-12866342 |
| PTPN2-385 | chr18:12841495-12841522 | PTPN2-1086 | chr18:12866319-12866346 |
| PTPN2-386 | chr18:12841513-12841540 | PTPN2-1087 | chr18:12866320-12866347 |
| PTPN2-387 | chr18:12841535-12841562 | PTPN2-1088 | chr18:12866321-12866348 |
| PTPN2-388 | chr18:12841546-12841573 | PTPN2-1089 | chr18:12866326-12866353 |
| PTPN2-389 | chr18:12841556-12841583 | PTPN2-1090 | chr18:12866338-12866365 |
| PTPN2-390 | chr18:12841566-12841593 | PTPN2-1091 | chr18:12866341-12866368 |
| PTPN2-391 | chr18:12841603-12841630 | PTPN2-1092 | chr18:12866344-12866371 |
| PTPN2-392 | chr18:12841609-12841636 | PTPN2-1093 | chr18:12866345-12866372 |
| PTPN2-393 | chr18:12841628-12841655 | PTPN2-1094 | chr18:12866346-12866373 |
| PTPN2-394 | chr18:12841653-12841680 | PTPN2-1095 | chr18:12866360-12866387 |
| PTPN2-395 | chr18:12841654-12841681 | PTPN2-1096 | chr18:12866361-12866388 |
| PTPN2-396 | chr18:12841659-12841686 | PTPN2-1097 | chr18:12866385-12866412 |
| PTPN2-397 | chr18:12841672-12841699 | PTPN2-1098 | chr18:12866388-12866415 |
| PTPN2-398 | chr18:12841713-12841740 | PTPN2-1099 | chr18:12866396-12866423 |
| PTPN2-399 | chr18:12841723-12841750 | PTPN2-1100 | chr18:12866406-12866433 |
| PTPN2-400 | chr18:12841738-12841765 | PTPN2-1101 | chr18:12866410-12866437 |
| PTPN2-401 | chr18:12841752-12841779 | PTPN2-1102 | chr18:12866424-12866451 |
| PTPN2-402 | chr18:12841753-12841780 | PTPN2-1103 | chr18:12866431-12866458 |
| PTPN2-403 | chr18:12841754-12841781 | PTPN2-1104 | chr18:12866480-12866507 |
| PTPN2-404 | chr18:12841777-12841804 | PTPN2-1105 | chr18:12866492-12866519 |
| PTPN2-405 | chr18:12841779-12841806 | PTPN2-1106 | chr18:12866497-12866524 |
| PTPN2-406 | chr18:12841795-12841822 | PTPN2-1107 | chr18:12866498-12866525 |
| PTPN2-407 | chr18:12841819-12841846 | PTPN2-1108 | chr18:12866506-12866533 |
| PTPN2-408 | chr18:12841838-12841865 | PTPN2-1109 | chr18:12866515-12866542 |
| PTPN2-409 | chr18:12841903-12841930 | PTPN2-1110 | chr18:12866527-12866554 |
| PTPN2-410 | chr18:12841906-12841933 | PTPN2-1111 | chr18:12866535-12866562 |
| PTPN2-411 | chr18:12841925-12841952 | PTPN2-1112 | chr18:12866536-12866563 |
| PTPN2-412 | chr18:12841929-12841956 | PTPN2-1113 | chr18:12866537-12866564 |
| PTPN2-413 | chr18:12841939-12841966 | PTPN2-1114 | chr18:12866541-12866568 |
| PTPN2-414 | chr18:12841947-12841974 | PTPN2-1115 | chr18:12866542-12866569 |
| PTPN2-415 | chr18:12841948-12841975 | PTPN2-1116 | chr18:12866548-12866575 |
| PTPN2-416 | chr18:12841974-12842001 | PTPN2-1117 | chr18:12866549-12866576 |
| PTPN2-417 | chr18:12841975-12842002 | PTPN2-1118 | chr18:12866558-12866585 |
| PTPN2-418 | chr18:12841982-12842009 | PTPN2-1119 | chr18:12866590-12866617 |
| PTPN2-419 | chr18:12841988-12842015 | PTPN2-1120 | chr18:12866591-12866618 |
| PTPN2-420 | chr18:12841991-12842018 | PTPN2-1121 | chr18:12866592-12866619 |
| PTPN2-421 | chr18:12841994-12842021 | PTPN2-1122 | chr18:12866602-12866629 |
| PTPN2-422 | chr18:12841997-12842024 | PTPN2-1123 | chr18:12866603-12866630 |
| PTPN2-423 | chr18:12842014-12842041 | PTPN2-1124 | chr18:12866612-12866639 |
| PTPN2-424 | chr18:12842015-12842042 | PTPN2-1125 | chr18:12866652-12866679 |
| PTPN2-425 | chr18:12842016-12842043 | PTPN2-1126 | chr18:12866670-12866697 |
| PTPN2-426 | chr18:12842020-12842047 | PTPN2-1127 | chr18:12866676-12866703 |
| PTPN2-427 | chr18:12842023-12842050 | PTPN2-1128 | chr18:12866682-12866709 |
| PTPN2-428 | chr18:12842026-12842053 | PTPN2-1129 | chr18:12866689-12866716 |
| PTPN2-429 | chr18:12842029-12842056 | PTPN2-1130 | chr18:12866701-12866728 |
| PTPN2-430 | chr18:12842057-12842084 | PTPN2-1131 | chr18:12866705-12866732 |
| PTPN2-431 | chr18:12842060-12842087 | PTPN2-1132 | chr18:12866706-12866733 |
| PTPN2-432 | chr18:12842065-12842092 | PTPN2-1133 | chr18:12866711-12866738 |
| PTPN2-433 | chr18:12842066-12842093 | PTPN2-1134 | chr18:12866714-12866741 |
| PTPN2-434 | chr18:12842069-12842096 | PTPN2-1135 | chr18:12866729-12866756 |
| PTPN2-435 | chr18:12842070-12842097 | PTPN2-1136 | chr18:12866741-12866768 |
| PTPN2-436 | chr18:12842071-12842098 | PTPN2-1137 | chr18:12866742-12866769 |
| PTPN2-437 | chr18:12842085-12842112 | PTPN2-1138 | chr18:12866745-12866772 |
| PTPN2-438 | chr18:12842090-12842117 | PTPN2-1139 | chr18:12866748-12866775 |
| PTPN2-439 | chr18:12842094-12842121 | PTPN2-1140 | chr18:12866751-12866778 |
| PTPN2-440 | chr18:12842104-12842131 | PTPN2-1141 | chr18:12866756-12866783 |
| PTPN2-441 | chr18:12842115-12842142 | PTPN2-1142 | chr18:12866757-12866784 |
| PTPN2-442 | chr18:12842132-12842159 | PTPN2-1143 | chr18:12866763-12866790 |
| PTPN2-443 | chr18:12842136-12842163 | PTPN2-1144 | chr18:12866768-12866795 |
| PTPN2-444 | chr18:12842147-12842174 | PTPN2-1145 | chr18:12866773-12866800 |
| PTPN2-445 | chr18:12842148-12842175 | PTPN2-1146 | chr18:12866786-12866813 |
| PTPN2-446 | chr18:12842149-12842176 | PTPN2-1147 | chr18:12866795-12866822 |
| PTPN2-447 | chr18:12842150-12842177 | PTPN2-1148 | chr18:12866805-12866832 |
| PTPN2-448 | chr18:12842154-12842181 | PTPN2-1149 | chr18:12866809-12866836 |
| PTPN2-449 | chr18:12842157-12842184 | PTPN2-1150 | chr18:12866830-12866857 |
| PTPN2-450 | chr18:12842160-12842187 | PTPN2-1151 | chr18:12866836-12866863 |
| PTPN2-451 | chr18:12842163-12842190 | PTPN2-1152 | chr18:12866842-12866869 |
| PTPN2-452 | chr18:12842164-12842191 | PTPN2-1153 | chr18:12866845-12866872 |
| PTPN2-453 | chr18:12842169-12842196 | PTPN2-1154 | chr18:12866848-12866875 |
| PTPN2-454 | chr18:12842191-12842218 | PTPN2-1155 | chr18:12866849-12866876 |
| PTPN2-455 | chr18:12842214-12842241 | PTPN2-1156 | chr18:12866864-12866891 |
| PTPN2-456 | chr18:12842222-12842249 | PTPN2-1157 | chr18:12866872-12866899 |
| PTPN2-457 | chr18:12842239-12842266 | PTPN2-1158 | chr18:12866873-12866900 |
| PTPN2-458 | chr18:12842249-12842276 | PTPN2-1159 | chr18:12866879-12866906 |
| PTPN2-459 | chr18:12842252-12842279 | PTPN2-1160 | chr18:12866882-12866909 |
| PTPN2-460 | chr18:12842255-12842282 | PTPN2-1161 | chr18:12866886-12866913 |
| PTPN2-461 | chr18:12842256-12842283 | PTPN2-1162 | chr18:12866887-12866914 |
| PTPN2-462 | chr18:12842260-12842287 | PTPN2-1163 | chr18:12866888-12866915 |
| PTPN2-463 | chr18:12842263-12842290 | PTPN2-1164 | chr18:12866908-12866935 |
| PTPN2-464 | chr18:12842264-12842291 | PTPN2-1165 | chr18:12866911-12866938 |
| PTPN2-465 | chr18:12842265-12842292 | PTPN2-1166 | chr18:12866914-12866941 |
| PTPN2-466 | chr18:12842295-12842322 | PTPN2-1167 | chr18:12866927-12866954 |
| PTPN2-467 | chr18:12842316-12842343 | PTPN2-1168 | chr18:12866928-12866955 |
| PTPN2-468 | chr18:12842323-12842350 | PTPN2-1169 | chr18:12866952-12866979 |
| PTPN2-469 | chr18:12842324-12842351 | PTPN2-1170 | chr18:12883946-12883973 |
| PTPN2-470 | chr18:12842334-12842361 | PTPN2-1171 | chr18:12883949-12883976 |
| PTPN2-471 | chr18:12842341-12842368 | PTPN2-1172 | chr18:12883951-12883978 |
| PTPN2-472 | chr18:12842352-12842379 | PTPN2-1173 | chr18:12883955-12883982 |
| PTPN2-473 | chr18:12842408-12842435 | PTPN2-1174 | chr18:12883958-12883985 |
| PTPN2-474 | chr18:12842409-12842436 | PTPN2-1175 | chr18:12883959-12883986 |
| PTPN2-475 | chr18:12842423-12842450 | PTPN2-1176 | chr18:12883960-12883987 |
| PTPN2-476 | chr18:12842424-12842451 | PTPN2-1177 | chr18:12883961-12883988 |
| PTPN2-477 | chr18:12842425-12842452 | PTPN2-1178 | chr18:12883962-12883989 |
| PTPN2-478 | chr18:12842430-12842457 | PTPN2-1179 | chr18:12883963-12883990 |
| PTPN2-479 | chr18:12842431-12842458 | PTPN2-1180 | chr18:12883964-12883991 |
| PTPN2-480 | chr18:12842432-12842459 | PTPN2-1181 | chr18:12883967-12883994 |
| PTPN2-481 | chr18:12842436-12842463 | PTPN2-1182 | chr18:12883970-12883997 |
| PTPN2-482 | chr18:12842453-12842480 | PTPN2-1183 | chr18:12883971-12883998 |
| PTPN2-483 | chr18:12842454-12842481 | PTPN2-1184 | chr18:12883974-12884001 |
| PTPN2-484 | chr18:12842457-12842484 | PTPN2-1185 | chr18:12883975-12884002 |
| PTPN2-485 | chr18:12842462-12842489 | PTPN2-1186 | chr18:12883984-12884011 |
| PTPN2-486 | chr18:12859082-12859109 | PTPN2-1187 | chr18:12883989-12884016 |
| PTPN2-487 | chr18:12859083-12859110 | PTPN2-1188 | chr18:12883990-12884017 |
| PTPN2-488 | chr18:12859084-12859111 | PTPN2-1189 | chr18:12884001-12884028 |
| PTPN2-489 | chr18:12859089-12859116 | PTPN2-1190 | chr18:12884002-12884029 |
| PTPN2-490 | chr18:12859090-12859117 | PTPN2-1191 | chr18:12884004-12884031 |
| PTPN2-491 | chr18:12859093-12859120 | PTPN2-1192 | chr18:12884005-12884032 |
| PTPN2-492 | chr18:12859107-12859134 | PTPN2-1193 | chr18:12884008-12884035 |
| PTPN2-493 | chr18:12859111-12859138 | PTPN2-1194 | chr18:12884011-12884038 |
| PTPN2-494 | chr18:12859114-12859141 | PTPN2-1195 | chr18:12884014-12884041 |
| PTPN2-495 | chr18:12859125-12859152 | PTPN2-1196 | chr18:12884018-12884045 |
| PTPN2-496 | chr18:12859140-12859167 | PTPN2-1197 | chr18:12884025-12884052 |
| PTPN2-497 | chr18:12859141-12859168 | PTPN2-1198 | chr18:12884032-12884059 |
| PTPN2-498 | chr18:12859149-12859176 | PTPN2-1199 | chr18:12884033-12884060 |
| PTPN2-499 | chr18:12859150-12859177 | PTPN2-1200 | chr18:12884154-12884181 |
| PTPN2-500 | chr18:12859254-12859281 | PTPN2-1201 | chr18:12884157-12884184 |
| PTPN2-501 | chr18:12859284-12859311 | PTPN2-1202 | chr18:12884162-12884189 |
| PTPN2-502 | chr18:12859285-12859312 | PTPN2-1203 | chr18:12884168-12884195 |
| PTPN2-503 | chr18:12859298-12859325 | PTPN2-1204 | chr18:12884172-12884199 |
| PTPN2-504 | chr18:12859317-12859344 | PTPN2-1205 | chr18:12884178-12884205 |
| PTPN2-505 | chr18:12859318-12859345 | PTPN2-1206 | chr18:12884183-12884210 |
| PTPN2-506 | chr18:12859322-12859349 | PTPN2-1207 | chr18:12884184-12884211 |
| PTPN2-507 | chr18:12859341-12859368 | PTPN2-1208 | chr18:12884185-12884212 |
| PTPN2-508 | chr18:12859356-12859383 | PTPN2-1209 | chr18:12884195-12884222 |
| PTPN2-509 | chr18:12859392-12859419 | PTPN2-1210 | chr18:12884196-12884223 |
| PTPN2-510 | chr18:12859403-12859430 | PTPN2-1211 | chr18:12884197-12884224 |
| PTPN2-511 | chr18:12859424-12859451 | PTPN2-1212 | chr18:12884198-12884225 |
| PTPN2-512 | chr18:12859425-12859452 | PTPN2-1213 | chr18:12884203-12884230 |
| PTPN2-513 | chr18:12859433-12859460 | PTPN2-1214 | chr18:12884207-12884234 |
| PTPN2-514 | chr18:12859434-12859461 | PTPN2-1215 | chr18:12884248-12884275 |
| PTPN2-515 | chr18:12859435-12859462 | PTPN2-1216 | chr18:12884252-12884279 |
| PTPN2-516 | chr18:12859443-12859470 | PTPN2-1217 | chr18:12884253-12884280 |
| PTPN2-517 | chr18:12859479-12859506 | PTPN2-1218 | chr18:12884254-12884281 |
| PTPN2-518 | chr18:12859480-12859507 | PTPN2-1219 | chr18:12884255-12884282 |
| PTPN2-519 | chr18:12859482-12859509 | PTPN2-1220 | chr18:12884258-12884285 |
| PTPN2-520 | chr18:12859490-12859517 | PTPN2-1221 | chr18:12884259-12884286 |
| PTPN2-521 | chr18:12859491-12859518 | PTPN2-1222 | chr18:12884260-12884287 |
| PTPN2-522 | chr18:12859502-12859529 | PTPN2-1223 | chr18:12884261-12884288 |
| PTPN2-523 | chr18:12859512-12859539 | PTPN2-1224 | chr18:12884266-12884293 |
| PTPN2-524 | chr18:12859513-12859540 | PTPN2-1225 | chr18:12884271-12884298 |
| PTPN2-525 | chr18:12859524-12859551 | PTPN2-1226 | chr18:12884272-12884299 |
| PTPN2-526 | chr18:12859531-12859558 | PTPN2-1227 | chr18:12884279-12884306 |
| PTPN2-527 | chr18:12859541-12859568 | PTPN2-1228 | chr18:12884280-12884307 |
| PTPN2-528 | chr18:12859556-12859583 | PTPN2-1229 | chr18:12884294-12884321 |
| PTPN2-529 | chr18:12859596-12859623 | PTPN2-1230 | chr18:12884299-12884326 |
| PTPN2-530 | chr18:12859616-12859643 | PTPN2-1231 | chr18:12884312-12884339 |
| PTPN2-531 | chr18:12859619-12859646 | PTPN2-1232 | chr18:12884323-12884350 |
| PTPN2-532 | chr18:12859628-12859655 | PTPN2-1233 | chr18:12884326-12884353 |
| PTPN2-533 | chr18:12859660-12859687 | PTPN2-1234 | chr18:12884329-12884356 |
| PTPN2-534 | chr18:12859685-12859712 | PTPN2-1235 | chr18:12884330-12884357 |
| PTPN2-535 | chr18:12859701-12859728 | PTPN2-1236 | chr18:12884331-12884358 |
| PTPN2-536 | chr18:12859721-12859748 | PTPN2-1237 | chr18:12884334-12884361 |
| PTPN2-537 | chr18:12859777-12859804 | PTPN2-1238 | chr18:12884335-12884362 |
| PTPN2-538 | chr18:12859778-12859805 | PTPN2-1239 | chr18:12884336-12884363 |
| PTPN2-539 | chr18:12859788-12859815 | PTPN2-1240 | chr18:12884339-12884366 |
| PTPN2-540 | chr18:12859826-12859853 | PTPN2-1241 | chr18:12884340-12884367 |
| PTPN2-541 | chr18:12859850-12859877 | PTPN2-1242 | chr18:12884341-12884368 |
| PTPN2-542 | chr18:12859852-12859879 | PTPN2-1243 | chr18:12884348-12884375 |
| PTPN2-543 | chr18:12859858-12859885 | PTPN2-1244 | chr18:12884349-12884376 |
| PTPN2-544 | chr18:12859873-12859900 | PTPN2-1245 | chr18:12884350-12884377 |
| PTPN2-545 | chr18:12859885-12859912 | PTPN2-1246 | chr18:12884351-12884378 |
| PTPN2-546 | chr18:12859886-12859913 | PTPN2-1247 | chr18:12884352-12884379 |
| PTPN2-547 | chr18:12859889-12859916 | PTPN2-1248 | chr18:12884355-12884382 |
| PTPN2-548 | chr18:12859892-12859919 | PTPN2-1249 | chr18:12884356-12884383 |
| PTPN2-549 | chr18:12859895-12859922 | PTPN2-1250 | chr18:12884357-12884384 |
| PTPN2-550 | chr18:12859899-12859926 | PTPN2-1251 | chr18:12884362-12884389 |
| PTPN2-551 | chr18:12859900-12859927 | PTPN2-1252 | chr18:12884363-12884390 |
| PTPN2-552 | chr18:12859901-12859928 | PTPN2-1253 | chr18:12884364-12884391 |
| PTPN2-553 | chr18:12859902-12859929 | PTPN2-1254 | chr18:12884372-12884399 |
| PTPN2-554 | chr18:12859913-12859940 | PTPN2-1255 | chr18:12884375-12884402 |
| PTPN2-555 | chr18:12859917-12859944 | PTPN2-1256 | chr18:12884376-12884403 |
| PTPN2-556 | chr18:12859918-12859945 | PTPN2-1257 | chr18:12884379-12884406 |
| PTPN2-557 | chr18:12859933-12859960 | PTPN2-1258 | chr18:12884380-12884407 |
| PTPN2-558 | chr18:12859934-12859961 | PTPN2-1259 | chr18:12884381-12884408 |
| PTPN2-559 | chr18:12859936-12859963 | PTPN2-1260 | chr18:12884382-12884409 |
| PTPN2-560 | chr18:12859959-12859986 | PTPN2-1261 | chr18:12884385-12884412 |
| PTPN2-561 | chr18:12859964-12859991 | PTPN2-1262 | chr18:12884386-12884413 |
| PTPN2-562 | chr18:12859979-12860006 | PTPN2-1263 | chr18:12884387-12884414 |
| PTPN2-563 | chr18:12859980-12860007 | PTPN2-1264 | chr18:12884392-12884419 |
| PTPN2-564 | chr18:12859981-12860008 | PTPN2-1265 | chr18:12884393-12884420 |
| PTPN2-565 | chr18:12859986-12860013 | PTPN2-1266 | chr18:12884394-12884421 |
| PTPN2-566 | chr18:12859991-12860018 | PTPN2-1267 | chr18:12884395-12884422 |
| PTPN2-567 | chr18:12859994-12860021 | PTPN2-1268 | chr18:12884403-12884430 |
| PTPN2-568 | chr18:12860009-12860036 | PTPN2-1269 | chr18:12884406-12884433 |
| PTPN2-569 | chr18:12860021-12860048 | PTPN2-1270 | chr18:12884412-12884439 |
| PTPN2-570 | chr18:12860022-12860049 | PTPN2-1271 | chr18:12884415-12884442 |
| PTPN2-571 | chr18:12860025-12860052 | PTPN2-1272 | chr18:12884423-12884450 |
| PTPN2-572 | chr18:12860028-12860055 | PTPN2-1273 | chr18:12884425-12884452 |
| PTPN2-573 | chr18:12860031-12860058 | PTPN2-1274 | chr18:12884426-12884453 |
| PTPN2-574 | chr18:12860034-12860061 | PTPN2-1275 | chr18:12884427-12884454 |
| PTPN2-575 | chr18:12860035-12860062 | PTPN2-1276 | chr18:12884432-12884459 |
| PTPN2-576 | chr18:12860036-12860063 | PTPN2-1277 | chr18:12884434-12884461 |
| PTPN2-577 | chr18:12860037-12860064 | PTPN2-1278 | chr18:12884441-12884468 |
| PTPN2-578 | chr18:12860048-12860075 | PTPN2-1279 | chr18:12884442-12884469 |
| PTPN2-579 | chr18:12860053-12860080 | PTPN2-1280 | chr18:12884447-12884474 |
| PTPN2-580 | chr18:12860069-12860096 | PTPN2-1281 | chr18:12884448-12884475 |
| PTPN2-581 | chr18:12860071-12860098 | PTPN2-1282 | chr18:12884464-12884491 |
| PTPN2-582 | chr18:12860090-12860117 | PTPN2-1283 | chr18:12884467-12884494 |
| PTPN2-583 | chr18:12860094-12860121 | PTPN2-1284 | chr18:12884468-12884495 |
| PTPN2-584 | chr18:12860099-12860126 | PTPN2-1285 | chr18:12884469-12884496 |
| PTPN2-585 | chr18:12860113-12860140 | PTPN2-1286 | chr18:12884473-12884500 |
| PTPN2-586 | chr18:12860114-12860141 | PTPN2-1287 | chr18:12884476-12884503 |
| PTPN2-587 | chr18:12860115-12860142 | PTPN2-1288 | chr18:12884479-12884506 |
| PTPN2-588 | chr18:12860121-12860148 | PTPN2-1289 | chr18:12884486-12884513 |
| PTPN2-589 | chr18:12860126-12860153 | PTPN2-1290 | chr18:12884487-12884514 |
| PTPN2-590 | chr18:12860132-12860159 | PTPN2-1291 | chr18:12884495-12884522 |
| PTPN2-591 | chr18:12860133-12860160 | PTPN2-1292 | chr18:12884496-12884523 |
| PTPN2-592 | chr18:12860156-12860183 | PTPN2-1293 | chr18:12884497-12884524 |
| PTPN2-593 | chr18:12860157-12860184 | PTPN2-1294 | chr18:12884504-12884531 |
| PTPN2-594 | chr18:12860160-12860187 | PTPN2-1295 | chr18:12884508-12884535 |
| PTPN2-595 | chr18:12860163-12860190 | PTPN2-1296 | chr18:12884509-12884536 |
| PTPN2-596 | chr18:12860166-12860193 | PTPN2-1297 | chr18:12884510-12884537 |
| PTPN2-597 | chr18:12860171-12860198 | PTPN2-1298 | chr18:12884521-12884548 |
| PTPN2-598 | chr18:12860172-12860199 | PTPN2-1299 | chr18:12884541-12884568 |
| PTPN2-599 | chr18:12860188-12860215 | PTPN2-1300 | chr18:12884542-12884569 |
| PTPN2-600 | chr18:12860189-12860216 | PTPN2-1301 | chr18:12884543-12884570 |
| PTPN2-601 | chr18:12860192-12860219 | PTPN2-1302 | chr18:12884553-12884580 |
| PTPN2-602 | chr18:12860195-12860222 | PTPN2-1303 | chr18:12884554-12884581 |
| PTPN2-603 | chr18:12860198-12860225 | PTPN2-1304 | chr18:12884555-12884582 |
| PTPN2-604 | chr18:12860211-12860238 | PTPN2-1305 | chr18:12884567-12884594 |
| PTPN2-605 | chr18:12860212-12860239 | PTPN2-1306 | chr18:12884577-12884604 |
| PTPN2-606 | chr18:12860236-12860263 | PTPN2-1307 | chr18:12884580-12884607 |
| PTPN2-607 | chr18:12860238-12860265 | PTPN2-1308 | chr18:12884584-12884611 |
| PTPN2-608 | chr18:12860239-12860266 | PTPN2-1309 | chr18:12884595-12884622 |
| PTPN2-609 | chr18:12860247-12860274 | PTPN2-1310 | chr18:12884596-12884623 |
| PTPN2-610 | chr18:12860257-12860284 | PTPN2-1311 | chr18:12884618-12884645 |
| PTPN2-611 | chr18:12860291-12860318 | PTPN2-1312 | chr18:12884645-12884672 |
| PTPN2-612 | chr18:12860295-12860322 | PTPN2-1313 | chr18:12884657-12884684 |
| PTPN2-613 | chr18:12860296-12860323 | PTPN2-1314 | chr18:12884673-12884700 |
| PTPN2-614 | chr18:12860297-12860324 | PTPN2-1315 | chr18:12884685-12884712 |
| PTPN2-615 | chr18:12860301-12860328 | PTPN2-1316 | chr18:12884692-12884719 |
| PTPN2-616 | chr18:12860304-12860331 | PTPN2-1317 | chr18:12884703-12884730 |
| PTPN2-617 | chr18:12860331-12860358 | PTPN2-1318 | chr18:12884710-12884737 |
| PTPN2-618 | chr18:12860332-12860359 | PTPN2-1319 | chr18:12884725-12884752 |
| PTPN2-619 | chr18:12860335-12860362 | PTPN2-1320 | chr18:12884726-12884753 |
| PTPN2-620 | chr18:12860338-12860365 | PTPN2-1321 | chr18:12884739-12884766 |
| PTPN2-621 | chr18:12860341-12860368 | PTPN2-1322 | chr18:12884747-12884774 |
| PTPN2-622 | chr18:12860344-12860371 | PTPN2-1323 | chr18:12884750-12884777 |
| PTPN2-623 | chr18:12860345-12860372 | PTPN2-1324 | chr18:12884759-12884786 |
| PTPN2-624 | chr18:12860346-12860373 | PTPN2-1325 | chr18:12884762-12884789 |
| PTPN2-625 | chr18:12860347-12860374 | PTPN2-1326 | chr18:12884764-12884791 |
| PTPN2-626 | chr18:12860348-12860375 | PTPN2-1327 | chr18:12884771-12884798 |
| PTPN2-627 | chr18:12860357-12860384 | PTPN2-1328 | chr18:12884781-12884808 |
| PTPN2-628 | chr18:12860362-12860389 | PTPN2-1329 | chr18:12884788-12884815 |
| PTPN2-629 | chr18:12860363-12860390 | PTPN2-1330 | chr18:12884789-12884816 |
| PTPN2-630 | chr18:12860380-12860407 | PTPN2-1331 | chr18:12884794-12884821 |
| PTPN2-631 | chr18:12860385-12860412 | PTPN2-1332 | chr18:12884795-12884822 |
| PTPN2-632 | chr18:12860389-12860416 | PTPN2-1333 | chr18:12884799-12884826 |
| PTPN2-633 | chr18:12860399-12860426 | PTPN2-1334 | chr18:12884804-12884831 |
| PTPN2-634 | chr18:12860403-12860430 | PTPN2-1335 | chr18:12884805-12884832 |
| PTPN2-635 | chr18:12860408-12860435 | PTPN2-1336 | chr18:12884826-12884853 |
| PTPN2-636 | chr18:12860422-12860449 | PTPN2-1337 | chr18:12884886-12884913 |
| PTPN2-637 | chr18:12860429-12860456 | PTPN2-1338 | chr18:12884889-12884916 |
| PTPN2-638 | chr18:12860437-12860464 | PTPN2-1339 | chr18:12884890-12884917 |
| PTPN2-639 | chr18:12860441-12860468 | PTPN2-1340 | chr18:12884895-12884922 |
| PTPN2-640 | chr18:12860452-12860479 | PTPN2-1341 | chr18:12884969-12884996 |
| PTPN2-641 | chr18:12860464-12860491 | PTPN2-1342 | chr18:12884974-12885001 |
| PTPN2-642 | chr18:12860465-12860492 | PTPN2-1343 | chr18:12884975-12885002 |
| PTPN2-643 | chr18:12860468-12860495 | PTPN2-1344 | chr18:12884980-12885007 |
| PTPN2-644 | chr18:12860471-12860498 | PTPN2-1345 | chr18:12884985-12885012 |
| PTPN2-645 | chr18:12860474-12860501 | PTPN2-1346 | chr18:12885009-12885036 |
| PTPN2-646 | chr18:12860478-12860505 | PTPN2-1347 | chr18:12885010-12885037 |
| PTPN2-647 | chr18:12860479-12860506 | PTPN2-1348 | chr18:12885018-12885045 |
| PTPN2-648 | chr18:12860480-12860507 | PTPN2-1349 | chr18:12885027-12885054 |
| PTPN2-649 | chr18:12860496-12860523 | PTPN2-1350 | chr18:12885046-12885073 |
| PTPN2-650 | chr18:12860497-12860524 | PTPN2-1351 | chr18:12885064-12885091 |
| PTPN2-651 | chr18:12860500-12860527 | PTPN2-1352 | chr18:12885065-12885092 |
| PTPN2-652 | chr18:12860506-12860533 | PTPN2-1353 | chr18:12885076-12885103 |
| PTPN2-653 | chr18:12860520-12860547 | PTPN2-1354 | chr18:12885080-12885107 |
| PTPN2-654 | chr18:12860522-12860549 | PTPN2-1355 | chr18:12885084-12885111 |
| PTPN2-655 | chr18:12860544-12860571 | PTPN2-1356 | chr18:12885091-12885118 |
| PTPN2-656 | chr18:12860546-12860573 | PTPN2-1357 | chr18:12885096-12885123 |
| PTPN2-657 | chr18:12860547-12860574 | PTPN2-1358 | chr18:12885102-12885129 |
| PTPN2-658 | chr18:12860555-12860582 | PTPN2-1359 | chr18:12885150-12885177 |
| PTPN2-659 | chr18:12860560-12860587 | PTPN2-1360 | chr18:12885160-12885187 |
| PTPN2-660 | chr18:12860563-12860590 | PTPN2-1361 | chr18:12885168-12885195 |
| PTPN2-661 | chr18:12860569-12860596 | PTPN2-1362 | chr18:12885182-12885209 |
| PTPN2-662 | chr18:12860599-12860626 | PTPN2-1363 | chr18:12885183-12885210 |
| PTPN2-663 | chr18:12860617-12860644 | PTPN2-1364 | chr18:12885184-12885211 |
| PTPN2-664 | chr18:12860622-12860649 | PTPN2-1365 | chr18:12885188-12885215 |
| PTPN2-665 | chr18:12860630-12860657 | PTPN2-1366 | chr18:12885223-12885250 |
| PTPN2-666 | chr18:12860633-12860660 | PTPN2-1367 | chr18:12885248-12885275 |
| PTPN2-667 | chr18:12860636-12860663 | PTPN2-1368 | chr18:12885249-12885276 |
| PTPN2-668 | chr18:12860639-12860666 | PTPN2-1369 | chr18:12885251-12885278 |
| PTPN2-669 | chr18:12860644-12860671 | PTPN2-1370 | chr18:12885254-12885281 |
| PTPN2-670 | chr18:12860645-12860672 | PTPN2-1371 | chr18:12885257-12885284 |
| PTPN2-671 | chr18:12860662-12860689 | PTPN2-1372 | chr18:12885333-12885360 |
| PTPN2-672 | chr18:12860665-12860692 | PTPN2-1373 | chr18:12885368-12885395 |
| PTPN2-673 | chr18:12860668-12860695 | PTPN2-1374 | chr18:12885369-12885396 |
| PTPN2-674 | chr18:12860671-12860698 | PTPN2-1375 | chr18:12885370-12885397 |
| PTPN2-675 | chr18:12860684-12860711 | PTPN2-1376 | chr18:12885371-12885398 |
| PTPN2-676 | chr18:12860685-12860712 | PTPN2-1377 | chr18:12885376-12885403 |
| PTPN2-677 | chr18:12860712-12860739 | PTPN2-1378 | chr18:12885379-12885406 |
| PTPN2-678 | chr18:12860719-12860746 | PTPN2-1379 | chr18:12885380-12885407 |
| PTPN2-679 | chr18:12860720-12860747 | PTPN2-1380 | chr18:12885381-12885408 |
| PTPN2-680 | chr18:12860730-12860757 | PTPN2-1381 | chr18:12885398-12885425 |
| PTPN2-681 | chr18:12862516-12862543 | PTPN2-1382 | chr18:12885418-12885445 |
| PTPN2-682 | chr18:12862517-12862544 | PTPN2-1383 | chr18:12885423-12885450 |
| PTPN2-683 | chr18:12862522-12862549 | PTPN2-1384 | chr18:12885456-12885483 |
| PTPN2-684 | chr18:12862536-12862563 | PTPN2-1385 | chr18:12885457-12885484 |
| PTPN2-685 | chr18:12862537-12862564 | PTPN2-1386 | chr18:12885466-12885493 |
| PTPN2-686 | chr18:12862538-12862565 | PTPN2-1387 | chr18:12885506-12885533 |
| PTPN2-687 | chr18:12862541-12862568 | PTPN2-1388 | chr18:12885508-12885535 |
| PTPN2-688 | chr18:12862545-12862572 | PTPN2-1389 | chr18:12885510-12885537 |
| PTPN2-689 | chr18:12862546-12862573 | PTPN2-1390 | chr18:12885511-12885538 |
| PTPN2-690 | chr18:12862551-12862578 | PTPN2-1391 | chr18:12885567-12885594 |
| PTPN2-691 | chr18:12862565-12862592 | PTPN2-1392 | chr18:12885572-12885599 |
| PTPN2-692 | chr18:12862566-12862593 | PTPN2-1393 | chr18:12885590-12885617 |
| PTPN2-693 | chr18:12862570-12862597 | PTPN2-1394 | chr18:12885596-12885623 |
| PTPN2-694 | chr18:12862571-12862598 | PTPN2-1395 | chr18:12885597-12885624 |
| PTPN2-695 | chr18:12862572-12862599 | PTPN2-1396 | chr18:12885609-12885636 |
| PTPN2-696 | chr18:12862579-12862606 | PTPN2-1397 | chr18:12885620-12885647 |
| PTPN2-697 | chr18:12862586-12862613 | PTPN2-1398 | chr18:12885636-12885663 |
| PTPN2-698 | chr18:12862597-12862624 | PTPN2-1399 | chr18:12885652-12885679 |
| PTPN2-699 | chr18:12862602-12862629 | PTPN2-1400 | chr18:12885688-12885715 |
| PTPN2-700 | chr18:12862609-12862636 | PTPN2-1401 | chr18:12885704-12885731 |
| PTPN2-701 | chr18:12862632-12862659 | PTPN2-1402 | chr18:12885711-12885738 |
| PTPN2-702 | chr2:111122650-111122677 | PTPN2-1665 | chr2:111166198-111166225 |
| PTPN2-703 | chr2:111122651-111122678 | PTPN2-1666 | chr2:111166199-111166226 |
| PTPN2-704 | chr2:111122652-111122679 | PTPN2-1667 | chr2:111166213-111166240 |
| PTPN2-705 | chr2:111122660-111122687 | PTPN2-1668 | chr2:111166220-111166247 |
| PTPN2-706 | chr2:111122661-111122688 | PTPN2-1669 | chr2:111166239-111166266 |
| PTPN2-707 | chr2:111122668-111122695 | PTPN2-1670 | chr2:111166240-111166267 |
| PTPN2-708 | chr2:111122673-111122700 | PTPN2-1671 | chr2:111166246-111166273 |
| PTPN2-709 | chr2:111122674-111122701 | PTPN2-1672 | chr2:111166252-111166279 |
| PTPN2-710 | chr2:111122680-111122707 | PTPN2-1673 | chr2:111166253-111166280 |
| PTPN2-711 | chr2:111122689-111122716 | PTPN2-1674 | chr2:111166260-111166287 |
| PTPN2-712 | chr2:111122697-111122724 | PTPN2-1675 | chr2:111166261-111166288 |
| PTPN2-713 | chr2:111122700-111122727 | PTPN2-1676 | chr2:111166262-111166289 |
| PTPN2-714 | chr2:111122707-111122734 | PTPN2-1677 | chr2:111166264-111166291 |
| PTPN2-715 | chr2:111122710-111122737 | PTPN2-1678 | chr2:111166265-111166292 |
| PTPN2-716 | chr2:111122711-111122738 | PTPN2-1679 | chr2:111166270-111166297 |
| PTPN2-717 | chr2:111122713-111122740 | PTPN2-1680 | chr2:111166275-111166302 |
| PTPN2-718 | chr2:111122714-111122741 | PTPN2-1681 | chr2:111166276-111166303 |
| PTPN2-719 | chr2:111122718-111122745 | PTPN2-1682 | chr2:111166279-111166306 |
| PTPN2-720 | chr2:111122720-111122747 | PTPN2-1683 | chr2:111166286-111166313 |
| PTPN2-721 | chr2:111122721-111122748 | PTPN2-1684 | chr2:111166287-111166314 |
| PTPN2-722 | chr2:111122722-111122749 | PTPN2-1685 | chr2:111166293-111166320 |
| **PTPN2-**723 | chr2:111122726-111122753 | PTPN2-1686 | chr2:111166300-111166327 |
| PTPN2-724 | chr2:111122731-111122758 | PTPN2-1687 | chr2:111166301-111166328 |
| PTPN2-725 | chr2:111122732-111122759 | PTPN2-1688 | chr2:111166302-111166329 |
| PTPN2-726 | chr2:111122748-111122775 | PTPN2-1689 | chr2:111166307-111166334 |
| PTPN2-727 | chr2:111122749-111122776 | PTPN2-1690 | chr2:111166308-111166335 |
| PTPN2-728 | chr2:111122750-111122777 | PTPN2-1691 | chr2:111166312-111166339 |
| PTPN2-729 | chr2:111122770-111122797 | PTPN2-1692 | chr2:111166313-111166340 |
| PTPN2-730 | chr2:111122771-111122798 | PTPN2-1693 | chr2:111166322-111166349 |
| PTPN2-731 | chr2:111122772-111122799 | PTPN2-1694 | chr2:111166323-111166350 |
| PTPN2-732 | chr2:111122786-111122813 | PTPN2-1695 | chr2:111166335-111166362 |
| PTPN2-733 | chr2:111122787-111122814 | PTPN2-1696 | chr2:111166339-111166366 |
| PTPN2-734 | chr2:111122793-111122820 | PTPN2-1697 | chr2:111166340-111166367 |
| PTPN2-735 | chr2:111122794-111122821 | PTPN2-1698 | chr2:111166343-111166370 |
| PTPN2-736 | chr2:111122797-111122824 | PTPN2-1699 | chr2:111166351-111166378 |
| PTPN2-737 | chr2:111122803-111122830 | PTPN2-1700 | chr2:111166355-111166382 |
| PTPN2-738 | chr2:111122807-111122834 | PTPN2-1701 | chr2:111166356-111166383 |
| PTPN2-739 | chr2:111122808-111122835 | PTPN2-1702 | chr2:111166361-111166388 |
| PTPN2-740 | chr2:111122809-111122836 | PTPN2-1703 | chr2:111166363-111166390 |
| PTPN2-741 | chr2:111122810-111122837 | PTPN2-1704 | chr2:111166371-111166398 |
| PTPN2-742 | chr2:111122811-111122838 | PTPN2-1705 | chr2:111166374-111166401 |
| PTPN2-743 | chr2:111122818-111122845 | PTPN2-1706 | chr2:111166376-111166403 |
| PTPN2-744 | chr2:111122822-111122849 | PTPN2-1707 | chr2:111166397-111166424 |
| PTPN2-745 | chr2:111122828-111122855 | PTPN2-1708 | chr2:111166402-111166429 |
| PTPN2-746 | chr2:111122829-111122856 | PTPN2-1709 | chr2:111166418-111166445 |
| PTPN2-747 | chr2:111122834-111122861 | PTPN2-1710 | chr2:111166425-111166452 |
| PTPN2-748 | chr2:111122835-111122862 | PTPN2-1711 | chr2:111166441-111166468 |
| PTPN2-749 | chr2:111122839-111122866 | PTPN2-1712 | chr2:111166450-111166477 |
| PTPN2-750 | chr2:111122841-111122868 | PTPN2-1713 | chr2:111166455-111166482 |
| PTPN2-751 | chr2:111122842-111122869 | PTPN2-1714 | chr2:111166475-111166502 |
| PTPN2-752 | chr2:111122848-111122875 | PTPN2-1715 | chr2:111166514-111166541 |
| PTPN2-753 | chr2:111122849-111122876 | PTPN2-1716 | chr2:111166515-111166542 |
| PTPN2-754 | chr2:111122850-111122877 | PTPN2-1717 | chr2:111166516-111166543 |
| PTPN2-755 | chr2:111122855-111122882 | PTPN2-1718 | chr2:111166533-111166560 |
| PTPN2-756 | chr2:111122862-111122889 | PTPN2-1719 | chr2:111166536-111166563 |
| PTPN2-757 | chr2:111122863-111122890 | PTPN2-1720 | chr2:111166537-111166564 |
| PTPN2-758 | chr2:111122867-111122894 | PTPN2-1721 | chr2:111166556-111166583 |
| PTPN2-759 | chr2:111122870-111122897 | PTPN2-1722 | chr2:111166579-111166606 |
| PTPN2-760 | chr2:111122884-111122911 | PTPN2-1723 | chr2:111166593-111166620 |
| PTPN2-761 | chr2:111122885-111122912 | PTPN2-1724 | chr2:111166596-111166623 |
| PTPN2-762 | chr2:111122889-111122916 | PTPN2-1725 | chr2:111166597-111166624 |
| PTPN2-763 | chr2:111122890-111122917 | PTPN2-1726 | chr2:111166598-111166625 |
| PTPN2-764 | chr2:111122891-111122918 | PTPN2-1727 | chr2:111166626-111166653 |
| PTPN2-765 | chr2:111122894-111122921 | PTPN2-1728 | chr2:111166628-111166655 |
| PTPN2-766 | chr2:111122907-111122934 | PTPN2-1729 | chr2:111166637-111166664 |
| PTPN2-767 | chr2:111122925-111122952 | PTPN2-1730 | chr2:111166642-111166669 |
| PTPN2-768 | chr2:111122926-111122953 | PTPN2-1731 | chr2:111166643-111166670 |
| PTPN2-769 | chr2:111122927-111122954 | PTPN2-1732 | chr2:111166668-111166695 |
| PTPN2-770 | chr2:111122930-111122957 | PTPN2-1733 | chr2:111166720-111166747 |
| PTPN2-771 | chr2:111122931-111122958 | PTPN2-1734 | chr2:111166734-111166761 |
| PTPN2-772 | chr2:111122938-111122965 | PTPN2-1735 | chr2:111166735-111166762 |
| PTPN2-773 | chr2:111122939-111122966 | PTPN2-1736 | chr2:111166739-111166766 |
| PTPN2-774 | chr2:111122943-111122970 | PTPN2-1737 | chr2:111166740-111166767 |
| PTPN2-775 | chr2:111122946-111122973 | PTPN2-1738 | chr2:111166741-111166768 |
| PTPN2-776 | chr2:111122949-111122976 | PTPN2-1739 | chr2:111166742-111166769 |
| PTPN2-777 | chr2:111122954-111122981 | PTPN2-1740 | chr2:111166743-111166770 |
| PTPN2-778 | chr2:111122964-111122991 | PTPN2-1741 | chr2:111166744-111166771 |
| PTPN2-779 | chr2:111122981-111123008 | PTPN2-1742 | chr2:111166747-111166774 |
| PTPN2-780 | chr2:111122988-111123015 | PTPN2-1743 | chr2:111166748-111166775 |
| PTPN2-781 | chr2:111122997-111123024 | PTPN2-1744 | chr2:111166749-111166776 |
| PTPN2-782 | chr2:111123002-111123029 | PTPN2-1745 | chr2:111166750-111166777 |
| PTPN2-783 | chr2:111123003-111123030 | PTPN2-1746 | chr2:111166756-111166783 |
| PTPN2-784 | chr2:111123010-111123037 | PTPN2-1747 | chr2:111166800-111166827 |
| PTPN2-785 | chr2:111123022-111123049 | PTPN2-1748 | chr2:111166803-111166830 |
| PTPN2-786 | chr2:111123045-111123072 | PTPN2-1749 | chr2:111166842-111166869 |
| PTPN2-787 | chr2:111123046-111123073 | PTPN2-1750 | chr2:111166848-111166875 |
| PTPN2-788 | chr2:111123049-111123076 | PTPN2-1751 | chr2:111166849-111166876 |
| PTPN2-789 | chr2:111123052-111123079 | PTPN2-1752 | chr2:111166850-111166877 |
| PTPN2-790 | chr2:111123055-111123082 | PTPN2-1753 | chr2:111166865-111166892 |
| PTPN2-791 | chr2:111123056-111123083 | PTPN2-1754 | chr2:111166914-111166941 |
| PTPN2-792 | chr2:111123057-111123084 | PTPN2-1755 | chr2:111166940-111166967 |
| PTPN2-793 | chr2:111123058-111123085 | PTPN2-1756 | chr2:111166946-111166973 |
| PTPN2-794 | chr2:111123060-111123087 | PTPN2-1757 | chr2:111166989-111167016 |
| PTPN2-795 | chr2:111123061-111123088 | PTPN2-1758 | chr2:111167055-111167082 |
| PTPN2-796 | chr2:111123062-111123089 | PTPN2-1759 | chr2:111167145-111167172 |
| PTPN2-797 | chr2:111123063-111123090 | PTPN2-1760 | chr2:111167146-111167173 |
| PTPN2-798 | chr2:111123080-111123107 | PTPN2-1761 | chr2:111167153-111167180 |
| PTPN2-799 | chr2:111123092-111123119 | PTPN2-1762 | chr2:111167154-111167181 |
| PTPN2-800 | chr2:111123094-111123121 | PTPN2-1763 | chr2:111167159-111167186 |
| PTPN2-801 | chr2:111123097-111123124 | PTPN2-1764 | chr2:111167160-111167187 |
| PTPN2-802 | chr2:111123104-111123131 | PTPN2-1765 | chr2:111167161-111167188 |
| PTPN2-803 | chr2:111123109-111123136 | PTPN2-1766 | chr2:111167165-111167192 |
| PTPN2-804 | chr2:111123110-111123137 | PTPN2-1767 | chr2:111167169-111167196 |
| PTPN2-805 | chr2:111123115-111123142 | PTPN2-1768 | chr2:111167170-111167197 |
| PTPN2-806 | chr2:111123116-111123143 | PTPN2-1769 | chr2:111167171-111167198 |
| PTPN2-807 | chr2:111123119-111123146 | PTPN2-1770 | chr2:111167172-111167199 |
| PTPN2-808 | chr2:111123120-111123147 | PTPN2-1771 | chr2:111167173-111167200 |
| PTPN2-809 | chr2:111123123-111123150 | PTPN2-1772 | chr2:111167174-111167201 |
| PTPN2-810 | chr2:111123124-111123151 | PTPN2-1773 | chr2:111167181-111167208 |
| PTPN2-811 | chr2:111123131-111123158 | PTPN2-1774 | chr2:111167184-111167211 |
| PTPN2-812 | chr2:111123132-111123159 | PTPN2-1775 | chr2:111167204-111167231 |
| PTPN2-813 | chr2:111123135-111123162 | PTPN2-1776 | chr2:111167213-111167240 |
| PTPN2-814 | chr2:111123153-111123180 | PTPN2-1777 | chr2:111167214-111167241 |
| PTPN2-815 | chr2:111123154-111123181 | PTPN2-1778 | chr2:111167217-111167244 |
| PTPN2-816 | chr2:111123168-111123195 | PTPN2-1779 | chr2:111167224-111167251 |
| PTPN2-817 | chr2:111123171-111123198 | PTPN2-1780 | chr2:111167231-111167258 |
| PTPN2-818 | chr2:111123177-111123204 | PTPN2-1781 | chr2:111167232-111167259 |
| PTPN2-819 | chr2:111123182-111123209 | PTPN2-1782 | chr2:111167233-111167260 |
| PTPN2-820 | chr2:111123187-111123214 | PTPN2-1783 | chr2:111167236-111167263 |
| PTPN2-821 | chr2:111123209-111123236 | PTPN2-1784 | chr2:111167238-111167265 |
| PTPN2-822 | chr2:111123237-111123264 | PTPN2-1785 | chr2:111167249-111167276 |
| PTPN2-823 | chr2:111123243-111123270 | PTPN2-1786 | chr2:111167252-111167279 |
| PTPN2-824 | chr2:111123248-111123275 | PTPN2-1787 | chr2:111167255-111167282 |
| PTPN2-825 | chr2:111123252-111123279 | PTPN2-1788 | chr2:111167256-111167283 |
| PTPN2-826 | chr2:111123253-111123280 | PTPN2-1789 | chr2:111167261-111167288 |
| PTPN2-827 | chr2:111123254-111123281 | PTPN2-1790 | chr2:111167269-111167296 |
| PTPN2-828 | chr2:111123264-111123291 | PTPN2-1791 | chr2:111167270-111167297 |
| PTPN2-829 | chr2:111123265-111123292 | PTPN2-1792 | chr2:111167271-111167298 |
| PTPN2-830 | chr2:111123266-111123293 | PTPN2-1793 | chr2:111167303-111167330 |
| PTPN2-831 | chr2:111123267-111123294 | PTPN2-1794 | chr2:111167306-111167333 |
| PTPN2-832 | chr2:111123270-111123297 | PTPN2-1795 | chr2:111167307-111167334 |
| PTPN2-833 | chr2:111123276-111123303 | PTPN2-1796 | chr2:111167326-111167353 |
| PTPN2-834 | chr2:111123277-111123304 | PTPN2-1797 | chr2:111167330-111167357 |
| PTPN2-835 | chr2:111123284-111123311 | PTPN2-1798 | chr2:111167333-111167360 |
| PTPN2-836 | chr2:111123294-111123321 | PTPN2-1799 | chr2:111167373-111167400 |
| PTPN2-837 | chr2:111123312-111123339 | PTPN2-1800 | chr2:111167383-111167410 |
| PTPN2-838 | chr2:111123316-111123343 | PTPN2-1801 | chr2:111167384-111167411 |
| PTPN2-839 | chr2:111123319-111123346 | PTPN2-1802 | chr2:111167391-111167418 |
| PTPN2-840 | chr2:111123330-111123357 | PTPN2-1803 | chr2:111167404-111167431 |
| PTPN2-841 | chr2:111123340-111123367 | PTPN2-1804 | chr2:111167422-111167449 |
| PTPN2-842 | chr2:111123341-111123368 | PTPN2-1805 | chr2:111167435-111167462 |
| PTPN2-843 | chr2:111123346-111123373 | PTPN2-1806 | chr2:111167445-111167472 |
| PTPN2-844 | chr2:111123348-111123375 | PTPN2-1807 | chr2:111167466-111167493 |
| PTPN2-845 | chr2:111123349-111123376 | PTPN2-1808 | chr2:111167469-111167496 |
| PTPN2-846 | chr2:111123354-111123381 | PTPN2-1809 | chr2:111167470-111167497 |
| PTPN2-847 | chr2:111123361-111123388 | PTPN2-1810 | chr2:111167475-111167502 |
| PTPN2-848 | chr2:111123365-111123392 | PTPN2-1811 | chr2:111167523-111167550 |
| PTPN2-849 | chr2:111123366-111123393 | PTPN2-1812 | chr2:111167524-111167551 |
| PTPN2-850 | chr2:111123370-111123397 | PTPN2-1813 | chr2:111167546-111167573 |
| PTPN2-851 | chr2:111123371-111123398 | PTPN2-1814 | chr2:111167561-111167588 |
| PTPN2-852 | chr2:111123372-111123399 | PTPN2-1815 | chr2:111167567-111167594 |
| PTPN2-853 | chr2:111123386-111123413 | PTPN2-1816 | chr2:111167568-111167595 |
| PTPN2-854 | chr2:111123399-111123426 | PTPN2-1817 | chr2:111167569-111167596 |
| PTPN2-855 | chr2:111123400-111123427 | PTPN2-1818 | chr2:111167574-111167601 |
| PTPN2-856 | chr2:111123422-111123449 | PTPN2-1819 | chr2:111167575-111167602 |
| PTPN2-857 | chr2:111123444-111123471 | PTPN2-1820 | chr2:111167580-111167607 |
| PTPN2-858 | chr2:111123491-111123518 | PTPN2-1821 | chr2:111167584-111167611 |
| PTPN2-859 | chr2:111123497-111123524 | PTPN2-1822 | chr2:111167596-111167623 |
| PTPN2-860 | chr2:111123506-111123533 | PTPN2-1823 | chr2:111167597-111167624 |
| PTPN2-861 | chr2:111123507-111123534 | PTPN2-1824 | chr2:111167600-111167627 |
| PTPN2-862 | chr2:111123508-111123535 | PTPN2-1825 | chr2:111167601-111167628 |
| PTPN2-863 | chr2:111123509-111123536 | PTPN2-1826 | chr2:111167603-111167630 |
| PTPN2-864 | chr2:111123539-111123566 | PTPN2-1827 | chr2:111167606-111167633 |
| PTPN2-865 | chr2:111123540-111123567 | PTPN2-1828 | chr2:111167609-111167636 |
| PTPN2-866 | chr2:111123546-111123573 | PTPN2-1829 | chr2:111167622-111167649 |
| PTPN2-867 | chr2:111123550-111123577 | PTPN2-1830 | chr2:111167633-111167660 |
| PTPN2-868 | chr2:111123562-111123589 | PTPN2-1831 | chr2:111167639-111167666 |
| PTPN2-869 | chr2:111123568-111123595 | PTPN2-1832 | chr2:111167643-111167670 |
| PTPN2-870 | chr2:111123569-111123596 | PTPN2-1833 | chr2:111167650-111167677 |
| PTPN2-871 | chr2:111123633-111123660 | PTPN2-1834 | chr2:111167651-111167678 |
| PTPN2-872 | chr2:111123637-111123664 | PTPN2-1835 | chr2:111167654-111167681 |
| PTPN2-873 | chr2:111123638-111123665 | PTPN2-1836 | chr2:111167661-111167688 |
| PTPN2-874 | chr2:111123722-111123749 | PTPN2-1837 | chr2:111167668-111167695 |
| PTPN2-875 | chr2:111123902-111123929 | PTPN2-1838 | chr2:111167669-111167696 |
| PTPN2-876 | chr2:111123903-111123930 | PTPN2-1839 | chr2:111167670-111167697 |
| PTPN2-877 | chr2:111123910-111123937 | PTPN2-1840 | chr2:111167701-111167728 |
| PTPN2-878 | chr2:111123911-111123938 | PTPN2-1841 | chr2:111167702-111167729 |
| PTPN2-879 | chr2:111123912-111123939 | PTPN2-1842 | chr2:111167703-111167730 |
| PTPN2-880 | chr2:111123913-111123940 | PTPN2-1843 | chr2:111167704-111167731 |
| PTPN2-881 | chr2:111123922-111123949 | PTPN2-1844 | chr2:111167707-111167734 |
| PTPN2-882 | chr2:111123923-111123950 | PTPN2-1845 | chr2:111167708-111167735 |
| PTPN2-883 | chr2:111123930-111123957 | PTPN2-1846 | chr2:111167712-111167739 |
| PTPN2-884 | chr2:111123931-111123958 | PTPN2-1847 | chr2:111167722-111167749 |
| PTPN2-885 | chr2:111123932-111123959 | PTPN2-1848 | chr2:111167729-111167756 |
| PTPN2-886 | chr2:111123939-111123966 | PTPN2-1849 | chr2:111167772-111167799 |
| PTPN2-887 | chr2:111123940-111123967 | PTPN2-1850 | chr2:111167775-111167802 |
| PTPN2-888 | chr2:111123941-111123968 | PTPN2-1851 | chr2:111167776-111167803 |
| PTPN2-889 | chr2:111123944-111123971 | PTPN2-1852 | chr2:111167779-111167806 |
| PTPN2-890 | chr2:111123948-111123975 | PTPN2-1853 | chr2:111167780-111167807 |
| PTPN2-891 | chr2:111123952-111123979 | PTPN2-1854 | chr2:111167781-111167808 |
| PTPN2-892 | chr2:111123953-111123980 | PTPN2-1855 | chr2:111167785-111167812 |
| PTPN2-893 | chr2:111123958-111123985 | PTPN2-1856 | chr2:111167786-111167813 |
| PTPN2-894 | chr2:111123959-111123986 | PTPN2-1857 | chr2:111167787-111167814 |
| PTPN2-895 | chr2:111123969-111123996 | PTPN2-1858 | chr2:111167788-111167815 |
| PTPN2-896 | chr2:111123975-111124002 | PTPN2-1859 | chr2:111167791-111167818 |
| PTPN2-897 | chr2:111123976-111124003 | PTPN2-1860 | chr2:111167792-111167819 |
| PTPN2-898 | chr2:111123977-111124004 | PTPN2-1861 | chr2:111167803-111167830 |
| PTPN2-899 | chr2:111124127-111124154 | PTPN2-1862 | chr2:111167817-111167844 |
| PTPN2-900 | chr2:111124128-111124155 | PTPN2-1863 | chr2:111167837-111167864 |
| PTPN2-901 | chr2:111124135-111124162 | PTPN2-1864 | chr2:111167838-111167865 |
| PTPN2-902 | chr2:111124149-111124176 | PTPN2-1865 | chr2:111167839-111167866 |
| PTPN2-903 | chr2:111124150-111124177 | PTPN2-1866 | chr2:111167840-111167867 |
| PTPN2-904 | chr2:111124153-111124180 | PTPN2-1867 | chr2:111167841-111167868 |
| PTPN2-905 | chr2:111124170-111124197 | PTPN2-1868 | chr2:111167844-111167871 |
| PTPN2-906 | chr2:111124180-111124207 | PTPN2-1869 | chr2:111167848-111167875 |
| PTPN2-907 | chr2:111124218-111124245 | PTPN2-1870 | chr2:111167849-111167876 |
| PTPN2-908 | chr2:111124219-111124246 | PTPN2-1871 | chr2:111167850-111167877 |
| PTPN2-909 | chr2:111124220-111124247 | PTPN2-1872 | chr2:111167853-111167880 |
| PTPN2-910 | chr2:111124230-111124257 | PTPN2-1873 | chr2:111167859-111167886 |
| PTPN2-911 | chr2:111124231-111124258 | PTPN2-1874 | chr2:111167864-111167891 |
| PTPN2-912 | chr2:111124232-111124259 | PTPN2-1875 | chr2:111167865-111167892 |
| PTPN2-913 | chr2:111124234-111124261 | PTPN2-1876 | chr2:111167878-111167905 |
| PTPN2-914 | chr2:111124235-111124262 | PTPN2-1877 | chr2:111167879-111167906 |
| PTPN2-915 | chr2:111128509-111128536 | PTPN2-1878 | chr2:111167880-111167907 |
| PTPN2-916 | chr2:111128513-111128540 | PTPN2-1879 | chr2:111167888-111167915 |
| PTPN2-917 | chr2:111128528-111128555 | PTPN2-1880 | chr2:111167889-111167916 |
| PTPN2-918 | chr2:111128538-111128565 | PTPN2-1881 | chr2:111167899-111167926 |
| PTPN2-919 | chr2:111128539-111128566 | PTPN2-1882 | chr2:111167910-111167937 |
| PTPN2-920 | chr2:111128542-111128569 | PTPN2-1883 | chr2:111167913-111167940 |
| PTPN2-921 | chr2:111128546-111128573 | PTPN2-1884 | chr2:111167924-111167951 |
| PTPN2-922 | chr2:111128547-111128574 | PTPN2-1885 | chr2:111167927-111167954 |
| PTPN2-923 | chr2:111128554-111128581 | PTPN2-1886 | chr2:111167943-111167970 |
| PTPN2-924 | chr2:111128555-111128582 | PTPN2-1887 | chr2:111167955-111167982 |
| PTPN2-925 | chr2:111128558-111128585 | PTPN2-1888 | chr2:111167960-111167987 |
| PTPN2-926 | chr2:111128577-111128604 | PTPN2-1889 | chr2:111167964-111167991 |
| PTPN2-927 | chr2:111128586-111128613 | PTPN2-1890 | chr2:111167965-111167992 |
| PTPN2-928 | chr2:111128597-111128624 | PTPN2-1891 | chr2:111167978-111168005 |
| PTPN2-929 | chr2:111128657-111128684 | PTPN2-1892 | chr2:111167985-111168012 |
| PTPN2-930 | chr2:111128672-111128699 | PTPN2-1893 | chr2:111167986-111168013 |
| PTPN2-931 | chr2:111128700-111128727 | PTPN2-1894 | chr2:111167987-111168014 |
| PTPN2-932 | chr2:111128703-111128730 | PTPN2-1895 | chr2:111167988-111168015 |
| PTPN2-933 | chr2:111128706-111128733 | PTPN2-1896 | chr2:111167991-111168018 |
| PTPN2-934 | chr2:111128713-111128740 | PTPN2-1897 | chr2:111167992-111168019 |
| PTPN2-935 | chr2:111128733-111128760 | PTPN2-1898 | chr2:111167993-111168020 |
| PTPN2-936 | chr2:111128754-111128781 | PTPN2-1899 | chr2:111167994-111168021 |
| PTPN2-937 | chr2:111128765-111128792 | PTPN2-1900 | chr2:111167996-111168023 |
| PTPN2-938 | chr2:111128771-111128798 | PTPN2-1901 | chr2:111168027-111168054 |
| PTPN2-939 | chr2:111128774-111128801 | PTPN2-1902 | chr2:111168028-111168055 |
| PTPN2-940 | chr2:111128781-111128808 | PTPN2-1903 | chr2:111168029-111168056 |
| PTPN2-941 | chr2:111128782-111128809 | PTPN2-1904 | chr2:111168090-111168117 |
| PTPN2-942 | chr2:111128788-111128815 | PTPN2-1905 | chr2:111168099-111168126 |
| PTPN2-943 | chr2:111128789-111128816 | PTPN2-1906 | chr2:111168100-111168127 |
| PTPN2-944 | chr2:111130011-111130038 | PTPN2-1907 | chr2:111168103-111168130 |
| PTPN2-945 | chr2:111130016-111130043 | PTPN2-1908 | chr2:111168104-111168131 |
| PTPN2-946 | chr2:111130021-111130048 | PTPN2-1909 | chr2:111168168-111168195 |
| PTPN2-947 | chr2:111130027-111130054 | PTPN2-1910 | chr2:111168181-111168208 |
| PTPN2-948 | chr2:111130036-111130063 | PTPN2-1911 | chr2:111168183-111168210 |
| PTPN2-949 | chr2:111130044-111130071 | PTPN2-1912 | chr2:111168211-111168238 |
| PTPN2-950 | chr2:111130045-111130072 | PTPN2-1913 | chr2:111168213-111168240 |
| PTPN2-951 | chr2:111130048-111130075 | PTPN2-1914 | chr2:111168214-111168241 |
| PTPN2-952 | chr2:111130049-111130076 | PTPN2-1915 | chr2:111168246-111168273 |
| PTPN2-953 | chr2:111130050-111130077 | PTPN2-1916 | chr2:111168251-111168278 |
| PTPN2-954 | chr2:111130148-111130175 | PTPN2-1917 | chr2:111168261-111168288 |
| PTPN2-955 | chr2:111130182-111130209 | PTPN2-1918 | chr2:111168275-111168302 |
| PTPN2-956 | chr2:111130186-111130213 | PTPN2-1919 | chr2:111168276-111168303 |
| PTPN2-957 | chr2:111130189-111130216 | PTPN2-1920 | chr2:111168284-111168311 |
| PTPN2-958 | chr2:111130195-111130222 | PTPN2-1921 | chr2:111168306-111168333 |
| PTPN2-959 | chr2:111130196-111130223 | PTPN2-1922 | chr2:111168307-111168334 |
| PTPN2-960 | chr2:111130212-111130239 | PTPN2-1923 | chr2:111168319-111168346 |
| PTPN2-961 | chr2:111130213-111130240 | PTPN2-1924 | chr2:111168320-111168347 |
| PTPN2-962 | chr2:111130214-111130241 | PTPN2-1925 | chr2:111168354-111168381 |
| PTPN2-963 | chr2:111130218-111130245 | | |

**Table 2C Genomic coordinates of the human AFF3 gene**

| Target ID | Target Coordinate Range | Target ID | Target Coordinate Range |
|---|---|---|---|
| AFF3-1 | chr2:100006511-100006538 | AFF3-2994 | chr2:100143270-100143297 |
| AFF3-2 | chr2:100006515-100006542 | AFF3-2995 | chr2:100143281-100143308 |
| AFF3-3 | chr2:100006529-100006556 | AFF3-2996 | chr2:100143301-100143328 |
| AFF3-4 | chr2:100006546-100006573 | AFF3-2997 | chr2:100143302-100143329 |
| AFF3-5 | chr2:100006547-100006574 | AFF3-2998 | chr2:100143315-100143342 |
| AFF3-6 | chr2:100006587-100006614 | AFF3-2999 | chr2:100143318-100143345 |
| AFF3-7 | chr2:100006618-100006645 | AFF3-3000 | chr2:100143319-100143346 |
| AFF3-8 | chr2:100006619-100006646 | AFF3-3001 | chr2:100143325-100143352 |
| AFF3-9 | chr2:100006620-100006647 | AFF3-3002 | chr2:100143328-100143355 |
| AFF3-10 | chr2:100006631-100006658 | AFF3-3003 | chr2:100143329-100143356 |
| AFF3-11 | chr2:100006633-100006660 | AFF3-3004 | chr2:100143341-100143368 |
| AFF3-12 | chr2:100006634-100006661 | AFF3-3005 | chr2:100143344-100143371 |
| AFF3-13 | chr2:100006635-100006662 | AFF3-3006 | chr2:100143347-100143374 |
| AFF3-14 | chr2:100006636-100006663 | AFF3-3007 | chr2:100143350-100143377 |
| AFF3-15 | chr2:100006637-100006664 | AFF3-3008 | chr2:100143355-100143382 |
| AFF3-16 | chr2:100006642-100006669 | AFF3-3009 | chr2:100143358-100143385 |
| AFF3-17 | chr2:100006648-100006675 | AFF3-3010 | chr2:100143367-100143394 |
| AFF3-18 | chr2:100006654-100006681 | AFF3-3011 | chr2:100143371-100143398 |
| AFF3-19 | chr2:100006658-100006685 | AFF3-3012 | chr2:100143375-100143402 |
| AFF3-20 | chr2:100006664-100006691 | AFF3-3013 | chr2:100143389-100143416 |
| AFF3-21 | chr2:100006670-100006697 | AFF3-3014 | chr2:100143406-100143433 |
| AFF3-22 | chr2:100006672-100006699 | AFF3-3015 | chr2:100143429-100143456 |
| AFF3-23 | chr2:100006676-100006703 | AFF3-3016 | chr2:100143430-100143457 |
| AFF3-24 | chr2:100006677-100006704 | AFF3-3017 | chr2:100143433-100143460 |
| AFF3-25 | chr2:100006688-100006715 | AFF3-3018 | chr2:100143443-100143470 |
| AFF3-26 | chr2:100006708-100006735 | AFF3-3019 | chr2:100143449-100143476 |
| AFF3-27 | chr2:100006709-100006736 | AFF3-3020 | chr2:100143450-100143477 |
| AFF3-28 | chr2:100006710-100006737 | AFF3-3021 | chr2:100143453-100143480 |
| AFF3-29 | chr2:100006733-100006760 | AFF3-3022 | chr2:100143463-100143490 |
| AFF3-30 | chr2:100006739-100006766 | AFF3-3023 | chr2:100143471-100143498 |
| AFF3-31 | chr2:100006745-100006772 | AFF3-3024 | chr2:100143474-100143501 |
| AFF3-32 | chr2:100006746-100006773 | AFF3-3025 | chr2:100143481-100143508 |
| AFF3-33 | chr2:100006747-100006774 | AFF3-3026 | chr2:100143487-100143514 |
| AFF3-34 | chr2:100006758-100006785 | AFF3-3027 | chr2:100143488-100143515 |
| AFF3-35 | chr2:100006769-100006796 | AFF3-3028 | chr2:100143494-100143521 |
| AFF3-36 | chr2:100006775-100006802 | AFF3-3029 | chr2:100143508-100143535 |
| AFF3-37 | chr2:100006783-100006810 | AFF3-3030 | chr2:100143513-100143540 |
| AFF3-38 | chr2:100006786-100006813 | AFF3-3031 | chr2:100143514-100143541 |
| AFF3-39 | chr2:100006787-100006814 | AFF3-3032 | chr2:100143520-100143547 |
| AFF3-40 | chr2:100006790-100006817 | AFF3-3033 | chr2:100143525-100143552 |
| AFF3-41 | chr2:100006797-100006824 | AFF3-3034 | chr2:100143548-100143575 |
| AFF3-42 | chr2:100006803-100006830 | AFF3-3035 | chr2:100143558-100143585 |
| AFF3-43 | chr2:100006807-100006834 | AFF3-3036 | chr2:100143559-100143586 |
| AFF3-44 | chr2:100006808-100006835 | AFF3-3037 | chr2:100143560-100143587 |
| AFF3-45 | chr2:100006821-100006848 | AFF3-3038 | chr2:100143561-100143588 |
| AFF3-46 | chr2:100006822-100006849 | AFF3-3039 | chr2:100143567-100143594 |
| AFF3-47 | chr2:100006826-100006853 | AFF3-3040 | chr2:100143570-100143597 |
| AFF3-48 | chr2:100006829-100006856 | AFF3-3041 | chr2:100143571-100143598 |
| AFF3-49 | chr2:100006867-100006894 | AFF3-3042 | chr2:100143583-100143610 |
| AFF3-50 | chr2:100006876-100006903 | AFF3-3043 | chr2:100143594-100143621 |
| AFF3-51 | chr2:100006880-100006907 | AFF3-3044 | chr2:100143595-100143622 |
| AFF3-52 | chr2:100006883-100006910 | AFF3-3045 | chr2:100143605-100143632 |
| AFF3-53 | chr2:100006893-100006920 | AFF3-3046 | chr2:100143607-100143634 |
| AFF3-54 | chr2:100006894-100006921 | AFF3-3047 | chr2:100143611-100143638 |
| AFF3-55 | chr2:100006895-100006922 | AFF3-3048 | chr2:100143612-100143639 |
| AFF3-56 | chr2:100006898-100006925 | AFF3-3049 | chr2:100143613-100143640 |
| AFF3-57 | chr2:100006902-100006929 | AFF3-3050 | chr2:100143627-100143654 |
| AFF3-58 | chr2:100006911-100006938 | AFF3-3051 | chr2:100143635-100143662 |
| AFF3-59 | chr2:100006916-100006943 | AFF3-3052 | chr2:100143645-100143672 |
| AFF3-60 | chr2:100006930-100006957 | AFF3-3053 | chr2:100143646-100143673 |
| AFF3-61 | chr2:100006931-100006958 | AFF3-3054 | chr2:100143649-100143676 |
| AFF3-62 | chr2:100006934-100006961 | AFF3-3055 | chr2:100143653-100143680 |
| AFF3-63 | chr2:100006937-100006964 | AFF3-3056 | chr2:100143659-100143686 |
| AFF3-64 | chr2:100006958-100006985 | AFF3-3057 | chr2:100143660-100143687 |
| AFF3-65 | chr2:100006959-100006986 | AFF3-3058 | chr2:100143670-100143697 |
| AFF3-66 | chr2:100006969-100006996 | AFF3-3059 | chr2:100143683-100143710 |
| AFF3-67 | chr2:100006975-100007002 | AFF3-3060 | chr2:100143684-100143711 |
| AFF3-68 | chr2:100006981-100007008 | AFF3-3061 | chr2:100143685-100143712 |
| AFF3-69 | chr2:100006987-100007014 | AFF3-3062 | chr2:100143686-100143713 |
| AFF3-70 | chr2:100006998-100007025 | AFF3-3063 | chr2:100143699-100143726 |
| AFF3-71 | chr2:100006999-100007026 | AFF3-3064 | chr2:100143702-100143729 |
| AFF3-72 | chr2:100007008-100007035 | AFF3-3065 | chr2:100143709-100143736 |
| AFF3-73 | chr2:100007009-100007036 | AFF3-3066 | chr2:100143719-100143746 |
| AFF3-74 | chr2:100007014-100007041 | AFF3-3067 | chr2:100143722-100143749 |
| AFF3-75 | chr2:100007023-100007050 | AFF3-3068 | chr2:100143750-100143777 |
| AFF3-76 | chr2:100007029-100007056 | AFF3-3069 | chr2:100143752-100143779 |
| AFF3-77 | chr2:100007030-100007057 | AFF3-3070 | chr2:100143753-100143780 |
| AFF3-78 | chr2:100007031-100007058 | AFF3-3071 | chr2:100143756-100143783 |
| AFF3-79 | chr2:100007032-100007059 | AFF3-3072 | chr2:100143763-100143790 |
| AFF3-80 | chr2:100007033-100007060 | AFF3-3073 | chr2:100143769-100143796 |
| AFF3-81 | chr2:100007045-100007072 | AFF3-3074 | chr2:100143772-100143799 |
| AFF3-82 | chr2:100007046-100007073 | AFF3-3075 | chr2:100143778-100143805 |
| AFF3-83 | chr2:100007074-100007101 | AFF3-3076 | chr2:100143779-100143806 |
| AFF3-84 | chr2:100007075-100007102 | AFF3-3077 | chr2:100143783-100143810 |
| AFF3-85 | chr2:100007131-100007158 | AFF3-3078 | chr2:100143789-100143816 |
| AFF3-86 | chr2:100007134-100007161 | AFF3-3079 | chr2:100143803-100143830 |
| AFF3-87 | chr2:100007137-100007164 | AFF3-3080 | chr2:100143814-100143841 |
| AFF3-88 | chr2:100007144-100007171 | AFF3-3081 | chr2:100143815-100143842 |
| AFF3-89 | chr2:100007145-100007172 | AFF3-3082 | chr2:100143816-100143843 |
| AFF3-90 | chr2:100007148-100007175 | AFF3-3083 | chr2:100143819-100143846 |
| AFF3-91 | chr2:100007149-100007176 | AFF3-3084 | chr2:100143820-100143847 |
| AFF3-92 | chr2:100007162-100007189 | AFF3-3085 | chr2:100143825-100143852 |
| AFF3-93 | chr2:100007179-100007206 | AFF3-3086 | chr2:100143830-100143857 |
| AFF3-94 | chr2:100007180-100007207 | AFF3-3087 | chr2:100143831-100143858 |
| AFF3-95 | chr2:100007184-100007211 | AFF3-3088 | chr2:100143836-100143863 |
| AFF3-96 | chr2:100007191-100007218 | AFF3-3089 | chr2:100143837-100143864 |
| AFF3-97 | chr2:100007195-100007222 | AFF3-3090 | chr2:100143850-100143877 |
| AFF3-98 | chr2:100007196-100007223 | AFF3-3091 | chr2:100143851-100143878 |
| AFF3-99 | chr2:100007198-100007225 | AFF3-3092 | chr2:100143855-100143882 |
| AFF3-100 | chr2:100007199-100007226 | AFF3-3093 | chr2:100143864-100143891 |
| AFF3-101 | chr2:100007200-100007227 | AFF3-3094 | chr2:100143872-100143899 |
| AFF3-102 | chr2:100007204-100007231 | AFF3-3095 | chr2:100143873-100143900 |
| AFF3-103 | chr2:100007211-100007238 | AFF3-3096 | chr2:100143894-100143921 |
| AFF3-104 | chr2:100007216-100007243 | AFF3-3097 | chr2:100143895-100143922 |
| AFF3-105 | chr2:100007243-100007270 | AFF3-3098 | chr2:100143896-100143923 |
| AFF3-106 | chr2:100007244-100007271 | AFF3-3099 | chr2:100143899-100143926 |
| AFF3-107 | chr2:100007248-100007275 | AFF3-3100 | chr2:100143906-100143933 |
| AFF3-108 | chr2:100007254-100007281 | AFF3-3101 | chr2:100143907-100143934 |
| AFF3-109 | chr2:100007255-100007282 | AFF3-3102 | chr2:100143912-100143939 |
| AFF3-110 | chr2:100007298-100007325 | AFF3-3103 | chr2:100143930-100143957 |
| AFF3-111 | chr2:100007307-100007334 | AFF3-3104 | chr2:100143931-100143958 |
| AFF3-112 | chr2:100007316-100007343 | AFF3-3105 | chr2:100143963-100143990 |
| AFF3-113 | chr2:100007321-100007348 | AFF3-3106 | chr2:100143967-100143994 |
| AFF3-114 | chr2:100007322-100007349 | AFF3-3107 | chr2:100143973-100144000 |
| AFF3-115 | chr2:100007337-100007364 | AFF3-3108 | chr2:100143978-100144005 |
| AFF3-116 | chr2:100007338-100007365 | AFF3-3109 | chr2:100143979-100144006 |
| AFF3-117 | chr2:100007339-100007366 | AFF3-3110 | chr2:100143983-100144010 |
| AFF3-118 | chr2:100007348-100007375 | AFF3-3111 | chr2:100143984-100144011 |
| AFF3-119 | chr2:100007354-100007381 | AFF3-3112 | chr2:100143992-100144019 |
| AFF3-120 | chr2:100007401-100007428 | AFF3-3113 | chr2:100144006-100144033 |
| AFF3-121 | chr2:100007407-100007434 | AFF3-3114 | chr2:100144014-100144041 |
| AFF3-122 | chr2:100007411-100007438 | AFF3-3115 | chr2:100144021-100144048 |
| AFF3-123 | chr2:100007414-100007441 | AFF3-3116 | chr2:100144022-100144049 |
| AFF3-124 | chr2:100007431-100007458 | AFF3-3117 | chr2:100144026-100144053 |
| AFF3-125 | chr2:100007437-100007464 | AFF3-3118 | chr2:100144034-100144061 |
| AFF3-126 | chr2:100007448-100007475 | AFF3-3119 | chr2:100144040-100144067 |
| AFF3-127 | chr2:100007449-100007476 | AFF3-3120 | chr2:100144045-100144072 |
| AFF3-128 | chr2:100007450-100007477 | AFF3-3121 | chr2:100144046-100144073 |
| AFF3-129 | chr2:100007451-100007478 | AFF3-3122 | chr2:100144049-100144076 |
| AFF3-130 | chr2:100007481-100007508 | AFF3-3123 | chr2:100144050-100144077 |
| AFF3-131 | chr2:100007486-100007513 | AFF3-3124 | chr2:100144053-100144080 |
| AFF3-132 | chr2:100007500-100007527 | AFF3-3125 | chr2:100144059-100144086 |
| AFF3-133 | chr2:100007510-100007537 | AFF3-3126 | chr2:100144060-100144087 |
| AFF3-134 | chr2:100007525-100007552 | AFF3-3127 | chr2:100144061-100144088 |
| AFF3-135 | chr2:100007526-100007553 | AFF3-3128 | chr2:100144072-100144099 |
| AFF3-136 | chr2:100007530-100007557 | AFF3-3129 | chr2:100144073-100144100 |
| AFF3-137 | chr2:100007531-100007558 | AFF3-3130 | chr2:100144079-100144106 |
| AFF3-138 | chr2:100007548-100007575 | AFF3-3131 | chr2:100144080-100144107 |
| AFF3-139 | chr2:100008687-100008714 | AFF3-3132 | chr2:100144081-100144108 |
| AFF3-140 | chr2:100008688-100008715 | AFF3-3133 | chr2:99545444-99545471 |
| AFF3-141 | chr2:100008697-100008724 | AFF3-3134 | chr2:99545445-99545472 |
| AFF3-142 | chr2:100008701-100008728 | AFF3-3135 | chr2:99545448-99545475 |
| AFF3-143 | chr2:100008720-100008747 | AFF3-3136 | chr2:99545464-99545491 |
| AFF3-144 | chr2:100008729-100008756 | AFF3-3137 | chr2:99545486-99545513 |
| AFF3-145 | chr2:100008733-100008760 | AFF3-3138 | chr2:99545500-99545527 |
| AFF3-146 | chr2:100008734-100008761 | AFF3-3139 | chr2:99545511-99545538 |
| AFF3-147 | chr2:100008748-100008775 | AFF3-3140 | chr2:99545512-99545539 |
| AFF3-148 | chr2:100008751-100008778 | AFF3-3141 | chr2:99545523-99545550 |
| AFF3-149 | chr2:100008756-100008783 | AFF3-3142 | chr2:99545563-99545590 |
| AFF3-150 | chr2:100008792-100008819 | AFF3-3143 | chr2:99545564-99545591 |
| AFF3-151 | chr2:100008793-100008820 | AFF3-3144 | chr2:99545565-99545592 |
| AFF3-152 | chr2:100008804-100008831 | AFF3-3145 | chr2:99545571-99545598 |
| AFF3-153 | chr2:100008811-100008838 | AFF3-3146 | chr2:99545572-99545599 |
| AFF3-154 | chr2:100008855-100008882 | AFF3-3147 | chr2:99545581-99545608 |
| AFF3-155 | chr2:100008862-100008889 | AFF3-3148 | chr2:99545602-99545629 |
| AFF3-156 | chr2:100008895-100008922 | AFF3-3149 | chr2:99545603-99545630 |
| AFF3-157 | chr2:100008899-100008926 | AFF3-3150 | chr2:99545604-99545631 |
| AFF3-158 | chr2:100008902-100008929 | AFF3-3151 | chr2:99545617-99545644 |
| AFF3-159 | chr2:100008915-100008942 | AFF3-3152 | chr2:99545629-99545656 |
| AFF3-160 | chr2:100008961-100008988 | AFF3-3153 | chr2:99545630-99545657 |
| AFF3-161 | chr2:100008987-100009014 | AFF3-3154 | chr2:99545654-99545681 |
| AFF3-162 | chr2:100008988-100009015 | AFF3-3155 | chr2:99545655-99545682 |
| AFF3-163 | chr2:100008994-100009021 | AFF3-3156 | chr2:99545665-99545692 |
| AFF3-164 | chr2:100009001-100009028 | AFF3-3157 | chr2:99545666-99545693 |
| AFF3-165 | chr2:100009009-100009036 | AFF3-3158 | chr2:99545667-99545694 |
| AFF3-166 | chr2:100009029-100009056 | AFF3-3159 | chr2:99545675-99545702 |
| AFF3-167 | chr2:100011370-100011397 | AFF3-3160 | chr2:99545678-99545705 |
| AFF3-168 | chr2:100011375-100011402 | AFF3-3161 | chr2:99545735-99545762 |
| AFF3-169 | chr2:100011376-100011403 | AFF3-3162 | chr2:99545736-99545763 |
| AFF3-170 | chr2:100011380-100011407 | AFF3-3163 | chr2:99545744-99545771 |
| AFF3-171 | chr2:100011387-100011414 | AFF3-3164 | chr2:99545745-99545772 |
| AFF3-172 | chr2:100011402-100011429 | AFF3-3165 | chr2:99545771-99545798 |
| AFF3-173 | chr2:100011408-100011435 | AFF3-3166 | chr2:99545778-99545805 |
| AFF3-174 | chr2:100011412-100011439 | AFF3-3167 | chr2:99545779-99545806 |
| AFF3-175 | chr2:100011415-100011442 | AFF3-3168 | chr2:99545780-99545807 |
| AFF3-176 | chr2:100011435-100011462 | AFF3-3169 | chr2:99545788-99545815 |
| AFF3-177 | chr2:100011436-100011463 | AFF3-3170 | chr2:99545791-99545818 |
| AFF3-178 | chr2:100011448-100011475 | AFF3-3171 | chr2:99545803-99545830 |
| AFF3-179 | chr2:100011476-100011503 | AFF3-3172 | chr2:99545804-99545831 |
| AFF3-180 | chr2:100011477-100011504 | AFF3-3173 | chr2:99545805-99545832 |
| AFF3-181 | chr2:100011478-100011505 | AFF3-3174 | chr2:99545806-99545833 |
| AFF3-182 | chr2:100011479-100011506 | AFF3-3175 | chr2:99545813-99545840 |
| AFF3-183 | chr2:100011484-100011511 | AFF3-3176 | chr2:99545823-99545850 |
| AFF3-184 | chr2:100011485-100011512 | AFF3-3177 | chr2:99545826-99545853 |
| AFF3-185 | chr2:100011496-100011523 | AFF3-3178 | chr2:99545827-99545854 |
| AFF3-186 | chr2:100011499-100011526 | AFF3-3179 | chr2:99545842-99545869 |
| AFF3-187 | chr2:100011526-100011553 | AFF3-3180 | chr2:99545848-99545875 |
| AFF3-188 | chr2:100011528-100011555 | AFF3-3181 | chr2:99545851-99545878 |
| AFF3-189 | chr2:100011547-100011574 | AFF3-3182 | chr2:99545892-99545919 |
| AFF3-190 | chr2:100011569-100011596 | AFF3-3183 | chr2:99545897-99545924 |
| AFF3-191 | chr2:100011570-100011597 | AFF3-3184 | chr2:99545899-99545926 |
| AFF3-192 | chr2:100011571-100011598 | AFF3-3185 | chr2:99545916-99545943 |
| AFF3-193 | chr2:100011590-100011617 | AFF3-3186 | chr2:99545917-99545944 |
| AFF3-194 | chr2:100011615-100011642 | AFF3-3187 | chr2:99545918-99545945 |
| AFF3-195 | chr2:100011624-100011651 | AFF3-3188 | chr2:99545919-99545946 |
| AFF3-196 | chr2:100011650-100011677 | AFF3-3189 | chr2:99545926-99545953 |
| AFF3-197 | chr2:100011651-100011678 | AFF3-3190 | chr2:99545927-99545954 |
| AFF3-198 | chr2:100011652-100011679 | AFF3-3191 | chr2:99545971-99545998 |
| AFF3-199 | chr2:100011665-100011692 | AFF3-3192 | chr2:99545994-99546021 |
| AFF3-200 | chr2:100011671-100011698 | AFF3-3193 | chr2:99546010-99546037 |
| AFF3-201 | chr2:100053031-100053058 | AFF3-3194 | chr2:99546011-99546038 |
| AFF3-202 | chr2:100053064-100053091 | AFF3-3195 | chr2:99546020-99546047 |
| AFF3-203 | chr2:100053081-100053108 | AFF3-3196 | chr2:99546033-99546060 |
| AFF3-204 | chr2:100053133-100053160 | AFF3-3197 | chr2:99546043-99546070 |
| AFF3-205 | chr2:100053134-100053161 | AFF3-3198 | chr2:99546047-99546074 |
| AFF3-206 | chr2:100053154-100053181 | AFF3-3199 | chr2:99546063-99546090 |
| AFF3-207 | chr2:100053171-100053198 | AFF3-3200 | chr2:99546064-99546091 |
| AFF3-208 | chr2:100053209-100053236 | AFF3-3201 | chr2:99546069-99546096 |
| AFF3-209 | chr2:100053210-100053237 | AFF3-3202 | chr2:99546105-99546132 |
| AFF3-210 | chr2:100053233-100053260 | AFF3-3203 | chr2:99546106-99546133 |
| AFF3-211 | chr2:100053252-100053279 | AFF3-3204 | chr2:99546107-99546134 |
| AFF3-212 | chr2:100053284-100053311 | AFF3-3205 | chr2:99546119-99546146 |
| AFF3-213 | chr2:100053288-100053315 | AFF3-3206 | chr2:99546120-99546147 |
| AFF3-214 | chr2:100053289-100053316 | AFF3-3207 | chr2:99546134-99546161 |
| AFF3-215 | chr2:100053307-100053334 | AFF3-3208 | chr2:99546143-99546170 |
| AFF3-216 | chr2:100053337-100053364 | AFF3-3209 | chr2:99546165-99546192 |
| AFF3-217 | chr2:100053358-100053385 | AFF3-3210 | chr2:99546166-99546193 |
| AFF3-218 | chr2:100053359-100053386 | AFF3-3211 | chr2:99546189-99546216 |
| AFF3-219 | chr2:100053360-100053387 | AFF3-3212 | chr2:99546227-99546254 |
| AFF3-220 | chr2:100053361-100053388 | AFF3-3213 | chr2:99546228-99546255 |
| AFF3-221 | chr2:100053364-100053391 | AFF3-3214 | chr2:99546272-99546299 |
| AFF3-222 | chr2:100053380-100053407 | AFF3-3215 | chr2:99546274-99546301 |
| AFF3-223 | chr2:100053381-100053408 | AFF3-3216 | chr2:99546275-99546302 |
| AFF3-224 | chr2:100053403-100053430 | AFF3-3217 | chr2:99546278-99546305 |
| AFF3-225 | chr2:100053410-100053437 | AFF3-3218 | chr2:99546315-99546342 |
| AFF3-226 | chr2:100053431-100053458 | AFF3-3219 | chr2:99546316-99546343 |
| AFF3-227 | chr2:100053434-100053461 | AFF3-3220 | chr2:99546322-99546349 |
| AFF3-228 | chr2:100053452-100053479 | AFF3-3221 | chr2:99546323-99546350 |
| AFF3-229 | chr2:100053454-100053481 | AFF3-3222 | chr2:99546328-99546355 |
| AFF3-230 | chr2:100053457-100053484 | AFF3-3223 | chr2:99546329-99546356 |
| AFF3-231 | chr2:100053458-100053485 | AFF3-3224 | chr2:99546332-99546359 |
| AFF3-232 | chr2:100053459-100053486 | AFF3-3225 | chr2:99546333-99546360 |
| AFF3-233 | chr2:100053504-100053531 | AFF3-3226 | chr2:99546334-99546361 |
| AFF3-234 | chr2:100053505-100053532 | AFF3-3227 | chr2:99546349-99546376 |
| AFF3-235 | chr2:100053527-100053554 | AFF3-3228 | chr2:99546356-99546383 |
| AFF3-236 | chr2:100053547-100053574 | AFF3-3229 | chr2:99546365-99546392 |
| AFF3-237 | chr2:100053548-100053575 | AFF3-3230 | chr2:99546366-99546393 |
| AFF3-238 | chr2:100053549-100053576 | AFF3-3231 | chr2:99546373-99546400 |
| AFF3-239 | chr2:100053552-100053579 | AFF3-3232 | chr2:99546382-99546409 |
| AFF3-240 | chr2:100053553-100053580 | AFF3-3233 | chr2:99546388-99546415 |
| AFF3-241 | chr2:100053588-100053615 | AFF3-3234 | chr2:99546389-99546416 |
| AFF3-242 | chr2:100053608-100053635 | AFF3-3235 | chr2:99546390-99546417 |
| AFF3-243 | chr2:100053609-100053636 | AFF3-3236 | chr2:99546396-99546423 |
| AFF3-244 | chr2:100053620-100053647 | AFF3-3237 | chr2:99546424-99546451 |
| AFF3-245 | chr2:100053622-100053649 | AFF3-3238 | chr2:99546425-99546452 |
| AFF3-246 | chr2:100053623-100053650 | AFF3-3239 | chr2:99546440-99546467 |
| AFF3-247 | chr2:100053644-100053671 | AFF3-3240 | chr2:99546441-99546468 |
| AFF3-248 | chr2:100053648-100053675 | AFF3-3241 | chr2:99546460-99546487 |
| AFF3-249 | chr2:100053651-100053678 | AFF3-3242 | chr2:99546468-99546495 |
| AFF3-250 | chr2:100053673-100053700 | AFF3-3243 | chr2:99546469-99546496 |
| AFF3-251 | chr2:100053674-100053701 | AFF3-3244 | chr2:99546482-99546509 |
| AFF3-252 | chr2:100053679-100053706 | AFF3-3245 | chr2:99546498-99546525 |
| AFF3-253 | chr2:100053687-100053714 | AFF3-3246 | chr2:99546504-99546531 |
| AFF3-254 | chr2:100053699-100053726 | AFF3-3247 | chr2:99546505-99546532 |
| AFF3-255 | chr2:100053706-100053733 | AFF3-3248 | chr2:99546509-99546536 |
| AFF3-256 | chr2:100053707-100053734 | AFF3-3249 | chr2:99546515-99546542 |
| AFF3-257 | chr2:100053708-100053735 | AFF3-3250 | chr2:99546521-99546548 |
| AFF3-258 | chr2:100053709-100053736 | AFF3-3251 | chr2:99546522-99546549 |
| AFF3-259 | chr2:100053730-100053757 | AFF3-3252 | chr2:99546526-99546553 |
| AFF3-260 | chr2:100053731-100053758 | AFF3-3253 | chr2:99546563-99546590 |
| AFF3-261 | chr2:100053755-100053782 | AFF3-3254 | chr2:99546564-99546591 |
| AFF3-262 | chr2:100053770-100053797 | AFF3-3255 | chr2:99546565-99546592 |
| AFF3-263 | chr2:100053778-100053805 | AFF3-3256 | chr2:99546568-99546595 |
| AFF3-264 | chr2:100053779-100053806 | AFF3-3257 | chr2:99546571-99546598 |
| AFF3-265 | chr2:100053785-100053812 | AFF3-3258 | chr2:99546577-99546604 |
| AFF3-266 | chr2:100053789-100053816 | AFF3-3259 | chr2:99546583-99546610 |
| AFF3-267 | chr2:100053791-100053818 | AFF3-3260 | chr2:99546584-99546611 |
| AFF3-268 | chr2:100053805-100053832 | AFF3-3261 | chr2:99546588-99546615 |
| AFF3-269 | chr2:100053806-100053833 | AFF3-3262 | chr2:99546634-99546661 |
| AFF3-270 | chr2:100053807-100053834 | AFF3-3263 | chr2:99546635-99546662 |
| AFF3-271 | chr2:100053816-100053843 | AFF3-3264 | chr2:99546636-99546663 |
| AFF3-272 | chr2:100053817-100053844 | AFF3-3265 | chr2:99546648-99546675 |
| AFF3-273 | chr2:100053818-100053845 | AFF3-3266 | chr2:99546649-99546676 |
| AFF3-274 | chr2:100053844-100053871 | AFF3-3267 | chr2:99546652-99546679 |
| AFF3-275 | chr2:100053846-100053873 | AFF3-3268 | chr2:99546653-99546680 |
| AFF3-276 | chr2:100053877-100053904 | AFF3-3269 | chr2:99546658-99546685 |
| AFF3-277 | chr2:100053885-100053912 | AFF3-3270 | chr2:99546679-99546706 |
| AFF3-278 | chr2:100053888-100053915 | AFF3-3271 | chr2:99546693-99546720 |
| AFF3-279 | chr2:100053889-100053916 | AFF3-3272 | chr2:99546696-99546723 |
| AFF3-280 | chr2:100053916-100053943 | AFF3-3273 | chr2:99546734-99546761 |
| AFF3-281 | chr2:100053922-100053949 | AFF3-3274 | chr2:99546739-99546766 |
| AFF3-282 | chr2:100053927-100053954 | AFF3-3275 | chr2:99546755-99546782 |
| AFF3-283 | chr2:100053930-100053957 | AFF3-3276 | chr2:99546758-99546785 |
| AFF3-284 | chr2:100053955-100053982 | AFF3-3277 | chr2:99546779-99546806 |
| AFF3-285 | chr2:100053968-100053995 | AFF3-3278 | chr2:99546780-99546807 |
| AFF3-286 | chr2:100053973-100054000 | AFF3-3279 | chr2:99546785-99546812 |
| AFF3-287 | chr2:100053974-100054001 | AFF3-3280 | chr2:99546786-99546813 |
| AFF3-288 | chr2:100053975-100054002 | AFF3-3281 | chr2:99546818-99546845 |
| AFF3-289 | chr2:100053976-100054003 | AFF3-3282 | chr2:99546827-99546854 |
| AFF3-290 | chr2:100053980-100054007 | AFF3-3283 | chr2:99546828-99546855 |
| AFF3-291 | chr2:100053993-100054020 | AFF3-3284 | chr2:99546835-99546862 |
| AFF3-292 | chr2:100053995-100054022 | AFF3-3285 | chr2:99546844-99546871 |
| AFF3-293 | chr2:100053997-100054024 | AFF3-3286 | chr2:99546849-99546876 |
| AFF3-294 | chr2:100054008-100054035 | AFF3-3287 | chr2:99546850-99546877 |
| AFF3-295 | chr2:100054013-100054040 | AFF3-3288 | chr2:99546854-99546881 |
| AFF3-296 | chr2:100054034-100054061 | AFF3-3289 | chr2:99546874-99546901 |
| AFF3-297 | chr2:100054035-100054062 | AFF3-3290 | chr2:99546875-99546902 |
| AFF3-298 | chr2:100054039-100054066 | AFF3-3291 | chr2:99546882-99546909 |
| AFF3-299 | chr2:100054044-100054071 | AFF3-3292 | chr2:99546894-99546921 |
| AFF3-300 | chr2:100054045-100054072 | AFF3-3293 | chr2:99546898-99546925 |
| AFF3-301 | chr2:100054054-100054081 | AFF3-3294 | chr2:99546899-99546926 |
| AFF3-302 | chr2:100054055-100054082 | AFF3-3295 | chr2:99546906-99546933 |
| AFF3-303 | chr2:100054073-100054100 | AFF3-3296 | chr2:99546907-99546934 |
| AFF3-304 | chr2:100054091-100054118 | AFF3-3297 | chr2:99546916-99546943 |
| AFF3-305 | chr2:100054094-100054121 | AFF3-3298 | chr2:99546968-99546995 |
| AFF3-306 | chr2:100054095-100054122 | AFF3-3299 | chr2:99547053-99547080 |
| AFF3-307 | chr2:100054102-100054129 | AFF3-3300 | chr2:99547063-99547090 |
| AFF3-308 | chr2:100054106-100054133 | AFF3-3301 | chr2:99547064-99547091 |
| AFF3-309 | chr2:100054107-100054134 | AFF3-3302 | chr2:99547105-99547132 |
| AFF3-310 | chr2:100054113-100054140 | AFF3-3303 | chr2:99547121-99547148 |
| AFF3-311 | chr2:100054148-100054175 | AFF3-3304 | chr2:99547122-99547149 |
| AFF3-312 | chr2:100054151-100054178 | AFF3-3305 | chr2:99547152-99547179 |
| AFF3-313 | chr2:100054159-100054186 | AFF3-3306 | chr2:99547191-99547218 |
| AFF3-314 | chr2:100054160-100054187 | AFF3-3307 | chr2:99547192-99547219 |
| AFF3-315 | chr2:100054163-100054190 | AFF3-3308 | chr2:99547194-99547221 |
| AFF3-316 | chr2:100054164-100054191 | AFF3-3309 | chr2:99547271-99547298 |
| AFF3-317 | chr2:100054165-100054192 | AFF3-3310 | chr2:99547288-99547315 |
| AFF3-318 | chr2:100054166-100054193 | AFF3-3311 | chr2:99547313-99547340 |
| AFF3-319 | chr2:100054170-100054197 | AFF3-3312 | chr2:99547318-99547345 |
| AFF3-320 | chr2:100054174-100054201 | AFF3-3313 | chr2:99547319-99547346 |
| AFF3-321 | chr2:100054175-100054202 | AFF3-3314 | chr2:99547332-99547359 |
| AFF3-322 | chr2:100054176-100054203 | AFF3-3315 | chr2:99547372-99547399 |
| AFF3-323 | chr2:100054183-100054210 | AFF3-3316 | chr2:99547376-99547403 |
| AFF3-324 | chr2:100054196-100054223 | AFF3-3317 | chr2:99547382-99547409 |
| AFF3-325 | chr2:100054213-100054240 | AFF3-3318 | chr2:99547396-99547423 |
| AFF3-326 | chr2:100054215-100054242 | AFF3-3319 | chr2:99547397-99547424 |
| AFF3-327 | chr2:100054221-100054248 | AFF3-3320 | chr2:99547411-99547438 |
| AFF3-328 | chr2:100054236-100054263 | AFF3-3321 | chr2:99547432-99547459 |
| AFF3-329 | chr2:100054237-100054264 | AFF3-3322 | chr2:99547433-99547460 |
| AFF3-330 | chr2:100054260-100054287 | AFF3-3323 | chr2:99547484-99547511 |
| AFF3-331 | chr2:100054261-100054288 | AFF3-3324 | chr2:99547511-99547538 |
| AFF3-332 | chr2:100054267-100054294 | AFF3-3325 | chr2:99547594-99547621 |
| AFF3-333 | chr2:100054268-100054295 | AFF3-3326 | chr2:99547657-99547684 |
| AFF3-334 | chr2:100054269-100054296 | AFF3-3327 | chr2:99547672-99547699 |
| AFF3-335 | chr2:100054289-100054316 | AFF3-3328 | chr2:99547692-99547719 |
| AFF3-336 | chr2:100054299-100054326 | AFF3-3329 | chr2:99547712-99547739 |
| AFF3-337 | chr2:100054309-100054336 | AFF3-3330 | chr2:99547776-99547803 |
| AFF3-338 | chr2:100054342-100054369 | AFF3-3331 | chr2:99547777-99547804 |
| AFF3-339 | chr2:100054360-100054387 | AFF3-3332 | chr2:99547778-99547805 |
| AFF3-340 | chr2:100054361-100054388 | AFF3-3333 | chr2:99547837-99547864 |
| AFF3-341 | chr2:100054365-100054392 | AFF3-3334 | chr2:99547902-99547929 |
| AFF3-342 | chr2:100054373-100054400 | AFF3-3335 | chr2:99547949-99547976 |
| AFF3-343 | chr2:100054374-100054401 | AFF3-3336 | chr2:99547950-99547977 |
| AFF3-344 | chr2:100054377-100054404 | AFF3-3337 | chr2:99547951-99547978 |
| AFF3-345 | chr2:100054378-100054405 | AFF3-3338 | chr2:99547961-99547988 |
| AFF3-346 | chr2:100054379-100054406 | AFF3-3339 | chr2:99547988-99548015 |
| AFF3-347 | chr2:100054393-100054420 | AFF3-3340 | chr2:99548009-99548036 |
| AFF3-348 | chr2:100054394-100054421 | AFF3-3341 | chr2:99548019-99548046 |
| AFF3-349 | chr2:100054395-100054422 | AFF3-3342 | chr2:99548028-99548055 |
| AFF3-350 | chr2:100054405-100054432 | AFF3-3343 | chr2:99548033-99548060 |
| AFF3-351 | chr2:100054414-100054441 | AFF3-3344 | chr2:99548042-99548069 |
| AFF3-352 | chr2:100054427-100054454 | AFF3-3345 | chr2:99548086-99548113 |
| AFF3-353 | chr2:100054428-100054455 | AFF3-3346 | chr2:99548097-99548124 |
| AFF3-354 | chr2:100054429-100054456 | AFF3-3347 | chr2:99548098-99548125 |
| AFF3-355 | chr2:100054430-100054457 | AFF3-3348 | chr2:99548115-99548142 |
| AFF3-356 | chr2:100054437-100054464 | AFF3-3349 | chr2:99548120-99548147 |
| AFF3-357 | chr2:100054438-100054465 | AFF3-3350 | chr2:99548137-99548164 |
| AFF3-358 | chr2:100054439-100054466 | AFF3-3351 | chr2:99548153-99548180 |
| AFF3-359 | chr2:100054442-100054469 | AFF3-3352 | chr2:99548159-99548186 |
| AFF3-360 | chr2:100054478-100054505 | AFF3-3353 | chr2:99548163-99548190 |
| AFF3-361 | chr2:100054485-100054512 | AFF3-3354 | chr2:99548169-99548196 |
| AFF3-362 | chr2:100054487-100054514 | AFF3-3355 | chr2:99548195-99548222 |
| AFF3-363 | chr2:100054503-100054530 | AFF3-3356 | chr2:99548198-99548225 |
| AFF3-364 | chr2:100054504-100054531 | AFF3-3357 | chr2:99548229-99548256 |
| AFF3-365 | chr2:100054506-100054533 | AFF3-3358 | chr2:99548266-99548293 |
| AFF3-366 | chr2:100054507-100054534 | AFF3-3359 | chr2:99548270-99548297 |
| AFF3-367 | chr2:100054508-100054535 | AFF3-3360 | chr2:99548295-99548322 |
| AFF3-368 | chr2:100054517-100054544 | AFF3-3361 | chr2:99548305-99548332 |
| AFF3-369 | chr2:100054539-100054566 | AFF3-3362 | chr2:99548312-99548339 |
| AFF3-370 | chr2:100054544-100054571 | AFF3-3363 | chr2:99548313-99548340 |
| AFF3-371 | chr2:100054556-100054583 | AFF3-3364 | chr2:99548322-99548349 |
| AFF3-372 | chr2:100054559-100054586 | AFF3-3365 | chr2:99548323-99548350 |
| AFF3-373 | chr2:100054560-100054587 | AFF3-3366 | chr2:99548345-99548372 |
| AFF3-374 | chr2:100054580-100054607 | AFF3-3367 | chr2:99548413-99548440 |
| AFF3-375 | chr2:100054591-100054618 | AFF3-3368 | chr2:99548442-99548469 |
| AFF3-376 | chr2:100054600-100054627 | AFF3-3369 | chr2:99548447-99548474 |
| AFF3-377 | chr2:100054603-100054630 | AFF3-3370 | chr2:99548452-99548479 |
| AFF3-378 | chr2:100054641-100054668 | AFF3-3371 | chr2:99548455-99548482 |
| AFF3-379 | chr2:100054644-100054671 | AFF3-3372 | chr2:99548469-99548496 |
| AFF3-380 | chr2:100054650-100054677 | AFF3-3373 | chr2:99548470-99548497 |
| AFF3-381 | chr2:100054659-100054686 | AFF3-3374 | chr2:99548473-99548500 |
| AFF3-382 | chr2:100054665-100054692 | AFF3-3375 | chr2:99548479-99548506 |
| AFF3-383 | chr2:100054671-100054698 | AFF3-3376 | chr2:99548486-99548513 |
| AFF3-384 | chr2:100054684-100054711 | AFF3-3377 | chr2:99548501-99548528 |
| AFF3-385 | chr2:100054696-100054723 | AFF3-3378 | chr2:99548502-99548529 |
| AFF3-386 | chr2:100054698-100054725 | AFF3-3379 | chr2:99548507-99548534 |
| AFF3-387 | chr2:100054700-100054727 | AFF3-3380 | chr2:99548520-99548547 |
| AFF3-388 | chr2:100054704-100054731 | AFF3-3381 | chr2:99548521-99548548 |
| AFF3-389 | chr2:100054720-100054747 | AFF3-3382 | chr2:99548524-99548551 |
| AFF3-390 | chr2:100054721-100054748 | AFF3-3383 | chr2:99548525-99548552 |
| AFF3-391 | chr2:100054731-100054758 | AFF3-3384 | chr2:99548539-99548566 |
| AFF3-392 | chr2:100054742-100054769 | AFF3-3385 | chr2:99548543-99548570 |
| AFF3-393 | chr2:100054743-100054770 | AFF3-3386 | chr2:99548562-99548589 |
| AFF3-394 | chr2:100054744-100054771 | AFF3-3387 | chr2:99548563-99548590 |
| AFF3-395 | chr2:100054754-100054781 | AFF3-3388 | chr2:99548564-99548591 |
| AFF3-396 | chr2:100054768-100054795 | AFF3-3389 | chr2:99548565-99548592 |
| AFF3-397 | chr2:100054769-100054796 | AFF3-3390 | chr2:99548574-99548601 |
| AFF3-398 | chr2:100054770-100054797 | AFF3-3391 | chr2:99548575-99548602 |
| AFF3-399 | chr2:100054774-100054801 | AFF3-3392 | chr2:99548580-99548607 |
| AFF3-400 | chr2:100054782-100054809 | AFF3-3393 | chr2:99548583-99548610 |
| AFF3-401 | chr2:100054791-100054818 | AFF3-3394 | chr2:99548598-99548625 |
| AFF3-402 | chr2:100054802-100054829 | AFF3-3395 | chr2:99548610-99548637 |
| AFF3-403 | chr2:100054808-100054835 | AFF3-3396 | chr2:99548611-99548638 |
| AFF3-404 | chr2:100054828-100054855 | AFF3-3397 | chr2:99548614-99548641 |
| AFF3-405 | chr2:100054829-100054856 | AFF3-3398 | chr2:99548617-99548644 |
| AFF3-406 | chr2:100054835-100054862 | AFF3-3399 | chr2:99548620-99548647 |
| AFF3-407 | chr2:100054838-100054865 | AFF3-3400 | chr2:99548624-99548651 |
| AFF3-408 | chr2:100054843-100054870 | AFF3-3401 | chr2:99548625-99548652 |
| AFF3-409 | chr2:100054844-100054871 | AFF3-3402 | chr2:99548626-99548653 |
| AFF3-410 | chr2:100054850-100054877 | AFF3-3403 | chr2:99548627-99548654 |
| AFF3-411 | chr2:100054851-100054878 | AFF3-3404 | chr2:99548643-99548670 |
| AFF3-412 | chr2:100054866-100054893 | AFF3-3405 | chr2:99548652-99548679 |
| AFF3-413 | chr2:100054870-100054897 | AFF3-3406 | chr2:99548659-99548686 |
| AFF3-414 | chr2:100054887-100054914 | AFF3-3407 | chr2:99548660-99548687 |
| AFF3-415 | chr2:100054888-100054915 | AFF3-3408 | chr2:99548665-99548692 |
| AFF3-416 | chr2:100054889-100054916 | AFF3-3409 | chr2:99548666-99548693 |
| AFF3-417 | chr2:100054899-100054926 | AFF3-3410 | chr2:99548690-99548717 |
| AFF3-418 | chr2:100054910-100054937 | AFF3-3411 | chr2:99548692-99548719 |
| AFF3-419 | chr2:100054914-100054941 | AFF3-3412 | chr2:99548693-99548720 |
| AFF3-420 | chr2:100054921-100054948 | AFF3-3413 | chr2:99548701-99548728 |
| AFF3-421 | chr2:100054922-100054949 | AFF3-3414 | chr2:99548704-99548731 |
| AFF3-422 | chr2:100054923-100054950 | AFF3-3415 | chr2:99548711-99548738 |
| AFF3-423 | chr2:100054925-100054952 | AFF3-3416 | chr2:99548729-99548756 |
| AFF3-424 | chr2:100054926-100054953 | AFF3-3417 | chr2:99548734-99548761 |
| AFF3-425 | chr2:100054943-100054970 | AFF3-3418 | chr2:99548735-99548762 |
| AFF3-426 | chr2:100054961-100054988 | AFF3-3419 | chr2:99548747-99548774 |
| AFF3-427 | chr2:100054988-100055015 | AFF3-3420 | chr2:99548751-99548778 |
| AFF3-428 | chr2:100054989-100055016 | AFF3-3421 | chr2:99548756-99548783 |
| AFF3-429 | chr2:100054992-100055019 | AFF3-3422 | chr2:99548767-99548794 |
| AFF3-430 | chr2:100055007-100055034 | AFF3-3423 | chr2:99548803-99548830 |
| AFF3-431 | chr2:100055020-100055047 | AFF3-3424 | chr2:99548806-99548833 |
| AFF3-432 | chr2:100055021-100055048 | AFF3-3425 | chr2:99548810-99548837 |
| AFF3-433 | chr2:100055029-100055056 | AFF3-3426 | chr2:99548845-99548872 |
| AFF3-434 | chr2:100055037-100055064 | AFF3-3427 | chr2:99548854-99548881 |
| AFF3-435 | chr2:100055038-100055065 | AFF3-3428 | chr2:99548855-99548882 |
| AFF3-436 | chr2:100055046-100055073 | AFF3-3429 | chr2:99548860-99548887 |
| AFF3-437 | chr2:100055067-100055094 | AFF3-3430 | chr2:99548865-99548892 |
| AFF3-438 | chr2:100055069-100055096 | AFF3-3431 | chr2:99548866-99548893 |
| AFF3-439 | chr2:100055117-100055144 | AFF3-3432 | chr2:99548867-99548894 |
| AFF3-440 | chr2:100055118-100055145 | AFF3-3433 | chr2:99548884-99548911 |
| AFF3-441 | chr2:100055121-100055148 | AFF3-3434 | chr2:99548894-99548921 |
| AFF3-442 | chr2:100055122-100055149 | AFF3-3435 | chr2:99548905-99548932 |
| AFF3-443 | chr2:100055127-100055154 | AFF3-3436 | chr2:99548935-99548962 |
| AFF3-444 | chr2:100055137-100055164 | AFF3-3437 | chr2:99548947-99548974 |
| AFF3-445 | chr2:100055155-100055182 | AFF3-3438 | chr2:99548953-99548980 |
| AFF3-446 | chr2:100055163-100055190 | AFF3-3439 | chr2:99548954-99548981 |
| AFF3-447 | chr2:100055166-100055193 | AFF3-3440 | chr2:99548958-99548985 |
| AFF3-448 | chr2:100055173-100055200 | AFF3-3441 | chr2:99548959-99548986 |
| AFF3-449 | chr2:100055174-100055201 | AFF3-3442 | chr2:99548967-99548994 |
| AFF3-450 | chr2:100055178-100055205 | AFF3-3443 | chr2:99548968-99548995 |
| AFF3-451 | chr2:100055206-100055233 | AFF3-3444 | chr2:99548981-99549008 |
| AFF3-452 | chr2:100055222-100055249 | AFF3-3445 | chr2:99548996-99549023 |
| AFF3-453 | chr2:100055223-100055250 | AFF3-3446 | chr2:99548997-99549024 |
| AFF3-454 | chr2:100055226-100055253 | AFF3-3447 | chr2:99549013-99549040 |
| AFF3-455 | chr2:100055243-100055270 | AFF3-3448 | chr2:99549014-99549041 |
| AFF3-456 | chr2:100055251-100055278 | AFF3-3449 | chr2:99549015-99549042 |
| AFF3-457 | chr2:100055272-100055299 | AFF3-3450 | chr2:99549024-99549051 |
| AFF3-458 | chr2:100055273-100055300 | AFF3-3451 | chr2:99549025-99549052 |
| AFF3-459 | chr2:100055289-100055316 | AFF3-3452 | chr2:99549026-99549053 |
| AFF3-460 | chr2:100055295-100055322 | AFF3-3453 | chr2:99549029-99549056 |
| AFF3-461 | chr2:100055298-100055325 | AFF3-3454 | chr2:99549070-99549097 |
| AFF3-462 | chr2:100055299-100055326 | AFF3-3455 | chr2:99549073-99549100 |
| AFF3-463 | chr2:100055303-100055330 | AFF3-3456 | chr2:99549074-99549101 |
| AFF3-464 | chr2:100055304-100055331 | AFF3-3457 | chr2:99549078-99549105 |
| AFF3-465 | chr2:100055316-100055343 | AFF3-3458 | chr2:99549097-99549124 |
| AFF3-466 | chr2:100055329-100055356 | AFF3-3459 | chr2:99549105-99549132 |
| AFF3-467 | chr2:100055336-100055363 | AFF3-3460 | chr2:99549107-99549134 |
| AFF3-468 | chr2:100055342-100055369 | AFF3-3461 | chr2:99549118-99549145 |
| AFF3-469 | chr2:100055347-100055374 | AFF3-3462 | chr2:99549119-99549146 |
| AFF3-470 | chr2:100055348-100055375 | AFF3-3463 | chr2:99549120-99549147 |
| AFF3-471 | chr2:100055378-100055405 | AFF3-3464 | chr2:99549138-99549165 |
| AFF3-472 | chr2:100055379-100055406 | AFF3-3465 | chr2:99549141-99549168 |
| AFF3-473 | chr2:100055387-100055414 | AFF3-3466 | chr2:99549147-99549174 |
| AFF3-474 | chr2:100055391-100055418 | AFF3-3467 | chr2:99549151-99549178 |
| AFF3-475 | chr2:100055392-100055419 | AFF3-3468 | chr2:99549152-99549179 |
| AFF3-476 | chr2:100055393-100055420 | AFF3-3469 | chr2:99549168-99549195 |
| AFF3-477 | chr2:100055394-100055421 | AFF3-3470 | chr2:99549206-99549233 |
| AFF3-478 | chr2:100055402-100055429 | AFF3-3471 | chr2:99549213-99549240 |
| AFF3-479 | chr2:100055403-100055430 | AFF3-3472 | chr2:99549216-99549243 |
| AFF3-480 | chr2:100055406-100055433 | AFF3-3473 | chr2:99549221-99549248 |
| AFF3-481 | chr2:100055407-100055434 | AFF3-3474 | chr2:99549240-99549267 |
| AFF3-482 | chr2:100055410-100055437 | AFF3-3475 | chr2:99549244-99549271 |
| AFF3-483 | chr2:100055415-100055442 | AFF3-3476 | chr2:99549245-99549272 |
| AFF3-484 | chr2:100055425-100055452 | AFF3-3477 | chr2:99549276-99549303 |
| AFF3-485 | chr2:100055451-100055478 | AFF3-3478 | chr2:99549277-99549304 |
| AFF3-486 | chr2:100055483-100055510 | AFF3-3479 | chr2:99549281-99549308 |
| AFF3-487 | chr2:100055506-100055533 | AFF3-3480 | chr2:99549282-99549309 |
| AFF3-488 | chr2:100055510-100055537 | AFF3-3481 | chr2:99549286-99549313 |
| AFF3-489 | chr2:100055513-100055540 | AFF3-3482 | chr2:99549287-99549314 |
| AFF3-490 | chr2:100055514-100055541 | AFF3-3483 | chr2:99549290-99549317 |
| AFF3-491 | chr2:100055515-100055542 | AFF3-3484 | chr2:99549317-99549344 |
| AFF3-492 | chr2:100055516-100055543 | AFF3-3485 | chr2:99549318-99549345 |
| AFF3-493 | chr2:100055521-100055548 | AFF3-3486 | chr2:99549321-99549348 |
| AFF3-494 | chr2:100055524-100055551 | AFF3-3487 | chr2:99549324-99549351 |
| AFF3-495 | chr2:100055536-100055563 | AFF3-3488 | chr2:99549327-99549354 |
| AFF3-496 | chr2:100055537-100055564 | AFF3-3489 | chr2:99549330-99549357 |
| AFF3-497 | chr2:100055558-100055585 | AFF3-3490 | chr2:99549331-99549358 |
| AFF3-498 | chr2:100055578-100055605 | AFF3-3491 | chr2:99549345-99549372 |
| AFF3-499 | chr2:100055602-100055629 | AFF3-3492 | chr2:99549350-99549377 |
| AFF3-500 | chr2:100055613-100055640 | AFF3-3493 | chr2:99549366-99549393 |
| AFF3-501 | chr2:100055630-100055657 | AFF3-3494 | chr2:99549368-99549395 |
| AFF3-502 | chr2:100055655-100055682 | AFF3-3495 | chr2:99549377-99549404 |
| AFF3-503 | chr2:100055662-100055689 | AFF3-3496 | chr2:99549391-99549418 |
| AFF3-504 | chr2:100055663-100055690 | AFF3-3497 | chr2:99549396-99549423 |
| AFF3-505 | chr2:100055664-100055691 | AFF3-3498 | chr2:99549410-99549437 |
| AFF3-506 | chr2:100055672-100055699 | AFF3-3499 | chr2:99549411-99549438 |
| AFF3-507 | chr2:100055692-100055719 | AFF3-3500 | chr2:99549416-99549443 |
| AFF3-508 | chr2:100055745-100055772 | AFF3-3501 | chr2:99549417-99549444 |
| AFF3-509 | chr2:100055746-100055773 | AFF3-3502 | chr2:99549422-99549449 |
| AFF3-510 | chr2:100055786-100055813 | AFF3-3503 | chr2:99549452-99549479 |
| AFF3-511 | chr2:100055793-100055820 | AFF3-3504 | chr2:99549453-99549480 |
| AFF3-512 | chr2:100055821-100055848 | AFF3-3505 | chr2:99549456-99549483 |
| AFF3-513 | chr2:100055829-100055856 | AFF3-3506 | chr2:99549459-99549486 |
| AFF3-514 | chr2:100055830-100055857 | AFF3-3507 | chr2:99549462-99549489 |
| AFF3-515 | chr2:100055831-100055858 | AFF3-3508 | chr2:99549465-99549492 |
| AFF3-516 | chr2:100055863-100055890 | AFF3-3509 | chr2:99549466-99549493 |
| AFF3-517 | chr2:100055864-100055891 | AFF3-3510 | chr2:99549467-99549494 |
| AFF3-518 | chr2:100055869-100055896 | AFF3-3511 | chr2:99549468-99549495 |
| AFF3-519 | chr2:100055870-100055897 | AFF3-3512 | chr2:99549485-99549512 |
| AFF3-520 | chr2:100055877-100055904 | AFF3-3513 | chr2:99549488-99549515 |
| AFF3-521 | chr2:100055892-100055919 | AFF3-3514 | chr2:99549494-99549521 |
| AFF3-522 | chr2:100055893-100055920 | AFF3-3515 | chr2:99549507-99549534 |
| AFF3-523 | chr2:100055911-100055938 | AFF3-3516 | chr2:99549508-99549535 |
| AFF3-524 | chr2:100055917-100055944 | AFF3-3517 | chr2:99549532-99549559 |
| AFF3-525 | chr2:100055919-100055946 | AFF3-3518 | chr2:99549534-99549561 |
| AFF3-526 | chr2:100055920-100055947 | AFF3-3519 | chr2:99549535-99549562 |
| AFF3-527 | chr2:100055921-100055948 | AFF3-3520 | chr2:99549543-99549570 |
| AFF3-528 | chr2:100055932-100055959 | AFF3-3521 | chr2:99549553-99549580 |
| AFF3-529 | chr2:100055937-100055964 | AFF3-3522 | chr2:99549557-99549584 |
| AFF3-530 | chr2:100055946-100055973 | AFF3-3523 | chr2:99549573-99549600 |
| AFF3-531 | chr2:100055960-100055987 | AFF3-3524 | chr2:99549574-99549601 |
| AFF3-532 | chr2:100055980-100056007 | AFF3-3525 | chr2:99549596-99549623 |
| AFF3-533 | chr2:100055981-100056008 | AFF3-3526 | chr2:99549611-99549638 |
| AFF3-534 | chr2:100055999-100056026 | AFF3-3527 | chr2:99549614-99549641 |
| AFF3-535 | chr2:100056003-100056030 | AFF3-3528 | chr2:99549615-99549642 |
| AFF3-536 | chr2:100056022-100056049 | AFF3-3529 | chr2:99549616-99549643 |
| AFF3-537 | chr2:100056023-100056050 | AFF3-3530 | chr2:99549643-99549670 |
| AFF3-538 | chr2:100056082-100056109 | AFF3-3531 | chr2:99549656-99549683 |
| AFF3-539 | chr2:100056088-100056115 | AFF3-3532 | chr2:99549671-99549698 |
| AFF3-540 | chr2:100056165-100056192 | AFF3-3533 | chr2:99549677-99549704 |
| AFF3-541 | chr2:100056173-100056200 | AFF3-3534 | chr2:99549690-99549717 |
| AFF3-542 | chr2:100056177-100056204 | AFF3-3535 | chr2:99549697-99549724 |
| AFF3-543 | chr2:100056182-100056209 | AFF3-3536 | chr2:99549698-99549725 |
| AFF3-544 | chr2:100056205-100056232 | AFF3-3537 | chr2:99549751-99549778 |
| AFF3-545 | chr2:100056229-100056256 | AFF3-3538 | chr2:99549787-99549814 |
| AFF3-546 | chr2:100056237-100056264 | AFF3-3539 | chr2:99549798-99549825 |
| AFF3-547 | chr2:100056238-100056265 | AFF3-3540 | chr2:99549799-99549826 |
| AFF3-548 | chr2:100056261-100056288 | AFF3-3541 | chr2:99549802-99549829 |
| AFF3-549 | chr2:100056262-100056289 | AFF3-3542 | chr2:99549840-99549867 |
| AFF3-550 | chr2:100056268-100056295 | AFF3-3543 | chr2:99549844-99549871 |
| AFF3-551 | chr2:100056298-100056325 | AFF3-3544 | chr2:99549845-99549872 |
| AFF3-552 | chr2:100056309-100056336 | AFF3-3545 | chr2:99549895-99549922 |
| AFF3-553 | chr2:100056310-100056337 | AFF3-3546 | chr2:99549900-99549927 |
| AFF3-554 | chr2:100056326-100056353 | AFF3-3547 | chr2:99549909-99549936 |
| AFF3-555 | chr2:100056373-100056400 | AFF3-3548 | chr2:99549929-99549956 |
| AFF3-556 | chr2:100056396-100056423 | AFF3-3549 | chr2:99549940-99549967 |
| AFF3-557 | chr2:100056397-100056424 | AFF3-3550 | chr2:99549941-99549968 |
| AFF3-558 | chr2:100056410-100056437 | AFF3-3551 | chr2:99549944-99549971 |
| AFF3-559 | chr2:100056412-100056439 | AFF3-3552 | chr2:99549948-99549975 |
| AFF3-560 | chr2:100056413-100056440 | AFF3-3553 | chr2:99549959-99549986 |
| AFF3-561 | chr2:100056415-100056442 | AFF3-3554 | chr2:99549962-99549989 |
| AFF3-562 | chr2:100056424-100056451 | AFF3-3555 | chr2:99549963-99549990 |
| AFF3-563 | chr2:100056425-100056452 | AFF3-3556 | chr2:99549970-99549997 |
| AFF3-564 | chr2:100056426-100056453 | AFF3-3557 | chr2:99549990-99550017 |
| AFF3-565 | chr2:100056427-100056454 | AFF3-3558 | chr2:99549996-99550023 |
| AFF3-566 | chr2:100056446-100056473 | AFF3-3559 | chr2:99550019-99550046 |
| AFF3-567 | chr2:100056477-100056504 | AFF3-3560 | chr2:99550033-99550060 |
| AFF3-568 | chr2:100056485-100056512 | AFF3-3561 | chr2:99550034-99550061 |
| AFF3-569 | chr2:100056487-100056514 | AFF3-3562 | chr2:99550077-99550104 |
| AFF3-570 | chr2:100056517-100056544 | AFF3-3563 | chr2:99550082-99550109 |
| AFF3-571 | chr2:100056525-100056552 | AFF3-3564 | chr2:99550083-99550110 |
| AFF3-572 | chr2:100056528-100056555 | AFF3-3565 | chr2:99550197-99550224 |
| AFF3-573 | chr2:100056536-100056563 | AFF3-3566 | chr2:99550220-99550247 |
| AFF3-574 | chr2:100056538-100056565 | AFF3-3567 | chr2:99550227-99550254 |
| AFF3-575 | chr2:100056542-100056569 | AFF3-3568 | chr2:99550233-99550260 |
| AFF3-576 | chr2:100056547-100056574 | AFF3-3569 | chr2:99550235-99550262 |
| AFF3-577 | chr2:100056548-100056575 | AFF3-3570 | chr2:99550239-99550266 |
| AFF3-578 | chr2:100056574-100056601 | AFF3-3571 | chr2:99550259-99550286 |
| AFF3-579 | chr2:100056575-100056602 | AFF3-3572 | chr2:99550276-99550303 |
| AFF3-580 | chr2:100056614-100056641 | AFF3-3573 | chr2:99550277-99550304 |
| AFF3-581 | chr2:100056619-100056646 | AFF3-3574 | chr2:99550278-99550305 |
| AFF3-582 | chr2:100056633-100056660 | AFF3-3575 | chr2:99550314-99550341 |
| AFF3-583 | chr2:100056634-100056661 | AFF3-3576 | chr2:99550328-99550355 |
| AFF3-584 | chr2:100056639-100056666 | AFF3-3577 | chr2:99550337-99550364 |
| AFF3-585 | chr2:100056659-100056686 | AFF3-3578 | chr2:99550338-99550365 |
| AFF3-586 | chr2:100056709-100056736 | AFF3-3579 | chr2:99550340-99550367 |
| AFF3-587 | chr2:100056723-100056750 | AFF3-3580 | chr2:99550341-99550368 |
| AFF3-588 | chr2:100056732-100056759 | AFF3-3581 | chr2:99550342-99550369 |
| AFF3-589 | chr2:100056741-100056768 | AFF3-3582 | chr2:99550343-99550370 |
| AFF3-590 | chr2:100056742-100056769 | AFF3-3583 | chr2:99550345-99550372 |
| AFF3-591 | chr2:100056743-100056770 | AFF3-3584 | chr2:99550363-99550390 |
| AFF3-592 | chr2:100056757-100056784 | AFF3-3585 | chr2:99550388-99550415 |
| AFF3-593 | chr2:100056777-100056804 | AFF3-3586 | chr2:99550407-99550434 |
| AFF3-594 | chr2:100056784-100056811 | AFF3-3587 | chr2:99550417-99550444 |
| AFF3-595 | chr2:100056800-100056827 | AFF3-3588 | chr2:99550424-99550451 |
| AFF3-596 | chr2:100056811-100056838 | AFF3-3589 | chr2:99550430-99550457 |
| AFF3-597 | chr2:100056815-100056842 | AFF3-3590 | chr2:99550437-99550464 |
| AFF3-598 | chr2:100056819-100056846 | AFF3-3591 | chr2:99550438-99550465 |
| AFF3-599 | chr2:100056832-100056859 | AFF3-3592 | chr2:99550448-99550475 |
| AFF3-600 | chr2:100056838-100056865 | AFF3-3593 | chr2:99550454-99550481 |
| AFF3-601 | chr2:100056839-100056866 | AFF3-3594 | chr2:99550482-99550509 |
| AFF3-602 | chr2:100056840-100056867 | AFF3-3595 | chr2:99550492-99550519 |
| AFF3-603 | chr2:100056856-100056883 | AFF3-3596 | chr2:99550495-99550522 |
| AFF3-604 | chr2:100056860-100056887 | AFF3-3597 | chr2:99550520-99550547 |
| AFF3-605 | chr2:100056861-100056888 | AFF3-3598 | chr2:99550538-99550565 |
| AFF3-606 | chr2:100056881-100056908 | AFF3-3599 | chr2:99550558-99550585 |
| AFF3-607 | chr2:100056884-100056911 | AFF3-3600 | chr2:99550563-99550590 |
| AFF3-608 | chr2:100056897-100056924 | AFF3-3601 | chr2:99550567-99550594 |
| AFF3-609 | chr2:100056923-100056950 | AFF3-3602 | chr2:99550574-99550601 |
| AFF3-610 | chr2:100056939-100056966 | AFF3-3603 | chr2:99550577-99550604 |
| AFF3-611 | chr2:100056940-100056967 | AFF3-3604 | chr2:99550585-99550612 |
| AFF3-612 | chr2:100056947-100056974 | AFF3-3605 | chr2:99550589-99550616 |
| AFF3-613 | chr2:100056948-100056975 | AFF3-3606 | chr2:99550592-99550619 |
| AFF3-614 | chr2:100056956-100056983 | AFF3-3607 | chr2:99550593-99550620 |
| AFF3-615 | chr2:100056960-100056987 | AFF3-3608 | chr2:99550607-99550634 |
| AFF3-616 | chr2:100056988-100057015 | AFF3-3609 | chr2:99550648-99550675 |
| AFF3-617 | chr2:100057007-100057034 | AFF3-3610 | chr2:99550652-99550679 |
| AFF3-618 | chr2:100057016-100057043 | AFF3-3611 | chr2:99550664-99550691 |
| AFF3-619 | chr2:100057017-100057044 | AFF3-3612 | chr2:99550684-99550711 |
| AFF3-620 | chr2:100057022-100057049 | AFF3-3613 | chr2:99550712-99550739 |
| AFF3-621 | chr2:100057027-100057054 | AFF3-3614 | chr2:99550727-99550754 |
| AFF3-622 | chr2:100057035-100057062 | AFF3-3615 | chr2:99550730-99550757 |
| AFF3-623 | chr2:100057045-100057072 | AFF3-3616 | chr2:99550733-99550760 |
| AFF3-624 | chr2:100057053-100057080 | AFF3-3617 | chr2:99550734-99550761 |
| AFF3-625 | chr2:100057054-100057081 | AFF3-3618 | chr2:99550735-99550762 |
| AFF3-626 | chr2:100057060-100057087 | AFF3-3619 | chr2:99550736-99550763 |
| AFF3-627 | chr2:100057066-100057093 | AFF3-3620 | chr2:99550739-99550766 |
| AFF3-628 | chr2:100057067-100057094 | AFF3-3621 | chr2:99550740-99550767 |
| AFF3-629 | chr2:100057068-100057095 | AFF3-3622 | chr2:99550744-99550771 |
| AFF3-630 | chr2:100057069-100057096 | AFF3-3623 | chr2:99550745-99550772 |
| AFF3-631 | chr2:100057070-100057097 | AFF3-3624 | chr2:99550771-99550798 |
| AFF3-632 | chr2:100057085-100057112 | AFF3-3625 | chr2:99550783-99550810 |
| AFF3-633 | chr2:100057086-100057113 | AFF3-3626 | chr2:99550794-99550821 |
| AFF3-634 | chr2:100057102-100057129 | AFF3-3627 | chr2:99550802-99550829 |
| AFF3-635 | chr2:100057104-100057131 | AFF3-3628 | chr2:99550803-99550830 |
| AFF3-636 | chr2:100057113-100057140 | AFF3-3629 | chr2:99550804-99550831 |
| AFF3-637 | chr2:100057123-100057150 | AFF3-3630 | chr2:99550805-99550832 |
| AFF3-638 | chr2:100057132-100057159 | AFF3-3631 | chr2:99550809-99550836 |
| AFF3-639 | chr2:100057153-100057180 | AFF3-3632 | chr2:99550810-99550837 |
| AFF3-640 | chr2:100057157-100057184 | AFF3-3633 | chr2:99550819-99550846 |
| AFF3-641 | chr2:100057158-100057185 | AFF3-3634 | chr2:99550828-99550855 |
| AFF3-642 | chr2:100057161-100057188 | AFF3-3635 | chr2:99550829-99550856 |
| AFF3-643 | chr2:100057164-100057191 | AFF3-3636 | chr2:99550836-99550863 |
| AFF3-644 | chr2:100057165-100057192 | AFF3-3637 | chr2:99550862-99550889 |
| AFF3-645 | chr2:100057176-100057203 | AFF3-3638 | chr2:99550877-99550904 |
| AFF3-646 | chr2:100057189-100057216 | AFF3-3639 | chr2:99550886-99550913 |
| AFF3-647 | chr2:100057192-100057219 | AFF3-3640 | chr2:99550900-99550927 |
| AFF3-648 | chr2:100057195-100057222 | AFF3-3641 | chr2:99550926-99550953 |
| AFF3-649 | chr2:100057198-100057225 | AFF3-3642 | chr2:99550927-99550954 |
| AFF3-650 | chr2:100057202-100057229 | AFF3-3643 | chr2:99550931-99550958 |
| AFF3-651 | chr2:100057203-100057230 | AFF3-3644 | chr2:99550932-99550959 |
| AFF3-652 | chr2:100057204-100057231 | AFF3-3645 | chr2:99550933-99550960 |
| AFF3-653 | chr2:100057213-100057240 | AFF3-3646 | chr2:99550940-99550967 |
| AFF3-654 | chr2:100057220-100057247 | AFF3-3647 | chr2:99550977-99551004 |
| AFF3-655 | chr2:100057221-100057248 | AFF3-3648 | chr2:99550991-99551018 |
| AFF3-656 | chr2:100057224-100057251 | AFF3-3649 | chr2:99551006-99551033 |
| AFF3-657 | chr2:100057230-100057257 | AFF3-3650 | chr2:99551025-99551052 |
| AFF3-658 | chr2:100057270-100057297 | AFF3-3651 | chr2:99551048-99551075 |
| AFF3-659 | chr2:100057271-100057298 | AFF3-3652 | chr2:99551061-99551088 |
| AFF3-660 | chr2:100057279-100057306 | AFF3-3653 | chr2:99551062-99551089 |
| AFF3-661 | chr2:100057289-100057316 | AFF3-3654 | chr2:99551071-99551098 |
| AFF3-662 | chr2:100057293-100057320 | AFF3-3655 | chr2:99551082-99551109 |
| AFF3-663 | chr2:100057332-100057359 | AFF3-3656 | chr2:99551087-99551114 |
| AFF3-664 | chr2:100057346-100057373 | AFF3-3657 | chr2:99551127-99551154 |
| AFF3-665 | chr2:100057351-100057378 | AFF3-3658 | chr2:99551188-99551215 |
| AFF3-666 | chr2:100057368-100057395 | AFF3-3659 | chr2:99551199-99551226 |
| AFF3-667 | chr2:100057374-100057401 | AFF3-3660 | chr2:99551200-99551227 |
| AFF3-668 | chr2:100057376-100057403 | AFF3-3661 | chr2:99551202-99551229 |
| AFF3-669 | chr2:100057378-100057405 | AFF3-3662 | chr2:99551207-99551234 |
| AFF3-670 | chr2:100057397-100057424 | AFF3-3663 | chr2:99551222-99551249 |
| AFF3-671 | chr2:100057412-100057439 | AFF3-3664 | chr2:99551224-99551251 |
| AFF3-672 | chr2:100057413-100057440 | AFF3-3665 | chr2:99551264-99551291 |
| AFF3-673 | chr2:100057418-100057445 | AFF3-3666 | chr2:99551267-99551294 |
| AFF3-674 | chr2:100057456-100057483 | AFF3-3667 | chr2:99551306-99551333 |
| AFF3-675 | chr2:100057525-100057552 | AFF3-3668 | chr2:99551307-99551334 |
| AFF3-676 | chr2:100057538-100057565 | AFF3-3669 | chr2:99551328-99551355 |
| AFF3-677 | chr2:100057563-100057590 | AFF3-3670 | chr2:99551329-99551356 |
| AFF3-678 | chr2:100057565-100057592 | AFF3-3671 | chr2:99551331-99551358 |
| AFF3-679 | chr2:100057566-100057593 | AFF3-3672 | chr2:99551363-99551390 |
| AFF3-680 | chr2:100057572-100057599 | AFF3-3673 | chr2:99551367-99551394 |
| AFF3-681 | chr2:100057573-100057600 | AFF3-3674 | chr2:99551368-99551395 |
| AFF3-682 | chr2:100057574-100057601 | AFF3-3675 | chr2:99551411-99551438 |
| AFF3-683 | chr2:100057577-100057604 | AFF3-3676 | chr2:99551415-99551442 |
| AFF3-684 | chr2:100057583-100057610 | AFF3-3677 | chr2:99551429-99551456 |
| AFF3-685 | chr2:100057621-100057648 | AFF3-3678 | chr2:99551430-99551457 |
| AFF3-686 | chr2:100057625-100057652 | AFF3-3679 | chr2:99551437-99551464 |
| AFF3-687 | chr2:100057646-100057673 | AFF3-3680 | chr2:99551438-99551465 |
| AFF3-688 | chr2:100057656-100057683 | AFF3-3681 | chr2:99551442-99551469 |
| AFF3-689 | chr2:100057690-100057717 | AFF3-3682 | chr2:99551443-99551470 |
| AFF3-690 | chr2:100057693-100057720 | AFF3-3683 | chr2:99551448-99551475 |
| AFF3-691 | chr2:100057694-100057721 | AFF3-3684 | chr2:99551449-99551476 |
| AFF3-692 | chr2:100057695-100057722 | AFF3-3685 | chr2:99551456-99551483 |
| AFF3-693 | chr2:100057730-100057757 | AFF3-3686 | chr2:99551458-99551485 |
| AFF3-694 | chr2:100057742-100057769 | AFF3-3687 | chr2:99551459-99551486 |
| AFF3-695 | chr2:100057757-100057784 | AFF3-3688 | chr2:99551460-99551487 |
| AFF3-696 | chr2:100057764-100057791 | AFF3-3689 | chr2:99551472-99551499 |
| AFF3-697 | chr2:100057796-100057823 | AFF3-3690 | chr2:99551473-99551500 |
| AFF3-698 | chr2:100057807-100057834 | AFF3-3691 | chr2:99551483-99551510 |
| AFF3-699 | chr2:100057856-100057883 | AFF3-3692 | chr2:99551492-99551519 |
| AFF3-700 | chr2:100057859-100057886 | AFF3-3693 | chr2:99551493-99551520 |
| AFF3-701 | chr2:100057864-100057891 | AFF3-3694 | chr2:99551495-99551522 |
| AFF3-702 | chr2:100057865-100057892 | AFF3-3695 | chr2:99551501-99551528 |
| AFF3-703 | chr2:100057882-100057909 | AFF3-3696 | chr2:99551507-99551534 |
| AFF3-704 | chr2:100057887-100057914 | AFF3-3697 | chr2:99551511-99551538 |
| AFF3-705 | chr2:100057896-100057923 | AFF3-3698 | chr2:99551512-99551539 |
| AFF3-706 | chr2:100057897-100057924 | AFF3-3699 | chr2:99551528-99551555 |
| AFF3-707 | chr2:100057917-100057944 | AFF3-3700 | chr2:99551529-99551556 |
| AFF3-708 | chr2:100057918-100057945 | AFF3-3701 | chr2:99551530-99551557 |
| AFF3-709 | chr2:100057979-100058006 | AFF3-3702 | chr2:99551536-99551563 |
| AFF3-710 | chr2:100057980-100058007 | AFF3-3703 | chr2:99551539-99551566 |
| AFF3-711 | chr2:100057994-100058021 | AFF3-3704 | chr2:99551555-99551582 |
| AFF3-712 | chr2:100058006-100058033 | AFF3-3705 | chr2:99551560-99551587 |
| AFF3-713 | chr2:100058020-100058047 | AFF3-3706 | chr2:99551564-99551591 |
| AFF3-714 | chr2:100058021-100058048 | AFF3-3707 | chr2:99551565-99551592 |
| AFF3-715 | chr2:100058022-100058049 | AFF3-3708 | chr2:99551566-99551593 |
| AFF3-716 | chr2:100058027-100058054 | AFF3-3709 | chr2:99551573-99551600 |
| AFF3-717 | chr2:100058028-100058055 | AFF3-3710 | chr2:99551574-99551601 |
| AFF3-718 | chr2:100058034-100058061 | AFF3-3711 | chr2:99551575-99551602 |
| AFF3-719 | chr2:100058035-100058062 | AFF3-3712 | chr2:99551576-99551603 |
| AFF3-720 | chr2:100058045-100058072 | AFF3-3713 | chr2:99551578-99551605 |
| AFF3-721 | chr2:100058064-100058091 | AFF3-3714 | chr2:99551612-99551639 |
| AFF3-722 | chr2:100058123-100058150 | AFF3-3715 | chr2:99551626-99551653 |
| AFF3-723 | chr2:100058131-100058158 | AFF3-3716 | chr2:99551640-99551667 |
| AFF3-724 | chr2:100058140-100058167 | AFF3-3717 | chr2:99551648-99551675 |
| AFF3-725 | chr2:100058148-100058175 | AFF3-3718 | chr2:99551657-99551684 |
| AFF3-726 | chr2:100058163-100058190 | AFF3-3719 | chr2:99551675-99551702 |
| AFF3-727 | chr2:100058164-100058191 | AFF3-3720 | chr2:99554212-99554239 |
| AFF3-728 | chr2:100058187-100058214 | AFF3-3721 | chr2:99554215-99554242 |
| AFF3-729 | chr2:100058196-100058223 | AFF3-3722 | chr2:99554221-99554248 |
| AFF3-730 | chr2:100058204-100058231 | AFF3-3723 | chr2:99554226-99554253 |
| AFF3-731 | chr2:100058220-100058247 | AFF3-3724 | chr2:99554227-99554254 |
| AFF3-732 | chr2:100058221-100058248 | AFF3-3725 | chr2:99554243-99554270 |
| AFF3-733 | chr2:100058238-100058265 | AFF3-3726 | chr2:99554244-99554271 |
| AFF3-734 | chr2:100058241-100058268 | AFF3-3727 | chr2:99554247-99554274 |
| AFF3-735 | chr2:100058242-100058269 | AFF3-3728 | chr2:99554250-99554277 |
| AFF3-736 | chr2:100058259-100058286 | AFF3-3729 | chr2:99554260-99554287 |
| AFF3-737 | chr2:100058260-100058287 | AFF3-3730 | chr2:99554266-99554293 |
| AFF3-738 | chr2:100058281-100058308 | AFF3-3731 | chr2:99554267-99554294 |
| AFF3-739 | chr2:100058324-100058351 | AFF3-3732 | chr2:99554270-99554297 |
| AFF3-740 | chr2:100058325-100058352 | AFF3-3733 | chr2:99554271-99554298 |
| AFF3-741 | chr2:100058357-100058384 | AFF3-3734 | chr2:99554272-99554299 |
| AFF3-742 | chr2:100058364-100058391 | AFF3-3735 | chr2:99554281-99554308 |
| AFF3-743 | chr2:100058432-100058459 | AFF3-3736 | chr2:99554282-99554309 |
| AFF3-744 | chr2:100058440-100058467 | AFF3-3737 | chr2:99554283-99554310 |
| AFF3-745 | chr2:100058476-100058503 | AFF3-3738 | chr2:99554288-99554315 |
| AFF3-746 | chr2:100058489-100058516 | AFF3-3739 | chr2:99554290-99554317 |
| AFF3-747 | chr2:100058503-100058530 | AFF3-3740 | chr2:99554291-99554318 |
| AFF3-748 | chr2:100058508-100058535 | AFF3-3741 | chr2:99554292-99554319 |
| AFF3-749 | chr2:100058509-100058536 | AFF3-3742 | chr2:99554298-99554325 |
| AFF3-750 | chr2:100058521-100058548 | AFF3-3743 | chr2:99554303-99554330 |
| AFF3-751 | chr2:100058522-100058549 | AFF3-3744 | chr2:99554310-99554337 |
| AFF3-752 | chr2:100058531-100058558 | AFF3-3745 | chr2:99554320-99554347 |
| AFF3-753 | chr2:100058554-100058581 | AFF3-3746 | chr2:99554326-99554353 |
| AFF3-754 | chr2:100058558-100058585 | AFF3-3747 | chr2:99554338-99554365 |
| AFF3-755 | chr2:100058559-100058586 | AFF3-3748 | chr2:99554339-99554366 |
| AFF3-756 | chr2:100058590-100058617 | AFF3-3749 | chr2:99554344-99554371 |
| AFF3-757 | chr2:100058611-100058638 | AFF3-3750 | chr2:99554345-99554372 |
| AFF3-758 | chr2:100058617-100058644 | AFF3-3751 | chr2:99554349-99554376 |
| AFF3-759 | chr2:100058630-100058657 | AFF3-3752 | chr2:99554363-99554390 |
| AFF3-760 | chr2:100058654-100058681 | AFF3-3753 | chr2:99554367-99554394 |
| AFF3-761 | chr2:100058655-100058682 | AFF3-3754 | chr2:99554378-99554405 |
| AFF3-762 | chr2:100058659-100058686 | AFF3-3755 | chr2:99554381-99554408 |
| AFF3-763 | chr2:100058701-100058728 | AFF3-3756 | chr2:99554392-99554419 |
| AFF3-764 | chr2:100058718-100058745 | AFF3-3757 | chr2:99554393-99554420 |
| AFF3-765 | chr2:100058746-100058773 | AFF3-3758 | chr2:99554395-99554422 |
| AFF3-766 | chr2:100058761-100058788 | AFF3-3759 | chr2:99554398-99554425 |
| AFF3-767 | chr2:100058819-100058846 | AFF3-3760 | chr2:99554406-99554433 |
| AFF3-768 | chr2:100058828-100058855 | AFF3-3761 | chr2:99554413-99554440 |
| AFF3-769 | chr2:100058829-100058856 | AFF3-3762 | chr2:99554414-99554441 |
| AFF3-770 | chr2:100058862-100058889 | AFF3-3763 | chr2:99554415-99554442 |
| AFF3-771 | chr2:100058863-100058890 | AFF3-3764 | chr2:99554420-99554447 |
| AFF3-772 | chr2:100058898-100058925 | AFF3-3765 | chr2:99554421-99554448 |
| AFF3-773 | chr2:100058944-100058971 | AFF3-3766 | chr2:99554427-99554454 |
| AFF3-774 | chr2:100058945-100058972 | AFF3-3767 | chr2:99554433-99554460 |
| AFF3-775 | chr2:100058950-100058977 | AFF3-3768 | chr2:99554438-99554465 |
| AFF3-776 | chr2:100058958-100058985 | AFF3-3769 | chr2:99554457-99554484 |
| AFF3-777 | chr2:100058973-100059000 | AFF3-3770 | chr2:99554463-99554490 |
| AFF3-778 | chr2:100058985-100059012 | AFF3-3771 | chr2:99554466-99554493 |
| AFF3-779 | chr2:100058986-100059013 | AFF3-3772 | chr2:99554467-99554494 |
| AFF3-780 | chr2:100058989-100059016 | AFF3-3773 | chr2:99554469-99554496 |
| AFF3-781 | chr2:100058995-100059022 | AFF3-3774 | chr2:99554470-99554497 |
| AFF3-782 | chr2:100058996-100059023 | AFF3-3775 | chr2:99554472-99554499 |
| AFF3-783 | chr2:100058999-100059026 | AFF3-3776 | chr2:99554473-99554500 |
| AFF3-784 | chr2:100059000-100059027 | AFF3-3777 | chr2:99554474-99554501 |
| AFF3-785 | chr2:100059001-100059028 | AFF3-3778 | chr2:99554477-99554504 |
| AFF3-786 | chr2:100059002-100059029 | AFF3-3779 | chr2:99554478-99554505 |
| AFF3-787 | chr2:100059013-100059040 | AFF3-3780 | chr2:99554479-99554506 |
| AFF3-788 | chr2:100059017-100059044 | AFF3-3781 | chr2:99554487-99554514 |
| AFF3-789 | chr2:100059018-100059045 | AFF3-3782 | chr2:99554488-99554515 |
| AFF3-790 | chr2:100059036-100059063 | AFF3-3783 | chr2:99554489-99554516 |
| AFF3-791 | chr2:100059045-100059072 | AFF3-3784 | chr2:99554490-99554517 |
| AFF3-792 | chr2:100059055-100059082 | AFF3-3785 | chr2:99554494-99554521 |
| AFF3-793 | chr2:100059059-100059086 | AFF3-3786 | chr2:99554495-99554522 |
| AFF3-794 | chr2:100059064-100059091 | AFF3-3787 | chr2:99554496-99554523 |
| AFF3-795 | chr2:100059078-100059105 | AFF3-3788 | chr2:99554511-99554538 |
| AFF3-796 | chr2:100059079-100059106 | AFF3-3789 | chr2:99554515-99554542 |
| AFF3-797 | chr2:100059080-100059107 | AFF3-3790 | chr2:99554516-99554543 |
| AFF3-798 | chr2:100059085-100059112 | AFF3-3791 | chr2:99554517-99554544 |
| AFF3-799 | chr2:100059090-100059117 | AFF3-3792 | chr2:99554522-99554549 |
| AFF3-800 | chr2:100059093-100059120 | AFF3-3793 | chr2:99554526-99554553 |
| AFF3-801 | chr2:100059096-100059123 | AFF3-3794 | chr2:99554534-99554561 |
| AFF3-802 | chr2:100059097-100059124 | AFF3-3795 | chr2:99554539-99554566 |
| AFF3-803 | chr2:100059108-100059135 | AFF3-3796 | chr2:99554544-99554571 |
| AFF3-804 | chr2:100059120-100059147 | AFF3-3797 | chr2:99554548-99554575 |
| AFF3-805 | chr2:100059121-100059148 | AFF3-3798 | chr2:99554549-99554576 |
| AFF3-806 | chr2:100059124-100059151 | AFF3-3799 | chr2:99554558-99554585 |
| AFF3-807 | chr2:100059127-100059154 | AFF3-3800 | chr2:99554560-99554587 |
| AFF3-808 | chr2:100059130-100059157 | AFF3-3801 | chr2:99554564-99554591 |
| AFF3-809 | chr2:100059134-100059161 | AFF3-3802 | chr2:99554565-99554592 |
| AFF3-810 | chr2:100059152-100059179 | AFF3-3803 | chr2:99554576-99554603 |
| AFF3-811 | chr2:100059153-100059180 | AFF3-3804 | chr2:99554577-99554604 |
| AFF3-812 | chr2:100059156-100059183 | AFF3-3805 | chr2:99554578-99554605 |
| AFF3-813 | chr2:100059175-100059202 | AFF3-3806 | chr2:99554596-99554623 |
| AFF3-814 | chr2:100059189-100059216 | AFF3-3807 | chr2:99554597-99554624 |
| AFF3-815 | chr2:100059200-100059227 | AFF3-3808 | chr2:99554598-99554625 |
| AFF3-816 | chr2:100059202-100059229 | AFF3-3809 | chr2:99554600-99554627 |
| AFF3-817 | chr2:100059203-100059230 | AFF3-3810 | chr2:99554622-99554649 |
| AFF3-818 | chr2:100059211-100059238 | AFF3-3811 | chr2:99554628-99554655 |
| AFF3-819 | chr2:100059221-100059248 | AFF3-3812 | chr2:99554629-99554656 |
| AFF3-820 | chr2:100059225-100059252 | AFF3-3813 | chr2:99554635-99554662 |
| AFF3-821 | chr2:100059253-100059280 | AFF3-3814 | chr2:99554641-99554668 |
| AFF3-822 | chr2:100059259-100059286 | AFF3-3815 | chr2:99554665-99554692 |
| AFF3-823 | chr2:100059298-100059325 | AFF3-3816 | chr2:99554675-99554702 |
| AFF3-824 | chr2:100059312-100059339 | AFF3-3817 | chr2:99554681-99554708 |
| AFF3-825 | chr2:100059318-100059345 | AFF3-3818 | chr2:99554682-99554709 |
| AFF3-826 | chr2:100059343-100059370 | AFF3-3819 | chr2:99554683-99554710 |
| AFF3-827 | chr2:100059386-100059413 | AFF3-3820 | chr2:99554686-99554713 |
| AFF3-828 | chr2:100059392-100059419 | AFF3-3821 | chr2:99554688-99554715 |
| AFF3-829 | chr2:100059393-100059420 | AFF3-3822 | chr2:99554689-99554716 |
| AFF3-830 | chr2:100059399-100059426 | AFF3-3823 | chr2:99554692-99554719 |
| AFF3-831 | chr2:100059402-100059429 | AFF3-3824 | chr2:99554693-99554720 |
| AFF3-832 | chr2:100059424-100059451 | AFF3-3825 | chr2:99554694-99554721 |
| AFF3-833 | chr2:100059437-100059464 | AFF3-3826 | chr2:99554695-99554722 |
| AFF3-834 | chr2:100059443-100059470 | AFF3-3827 | chr2:99554696-99554723 |
| AFF3-835 | chr2:100059452-100059479 | AFF3-3828 | chr2:99554697-99554724 |
| AFF3-836 | chr2:100059474-100059501 | AFF3-3829 | chr2:99554751-99554778 |
| AFF3-837 | chr2:100059475-100059502 | AFF3-3830 | chr2:99554760-99554787 |
| AFF3-838 | chr2:100059482-100059509 | AFF3-3831 | chr2:99554765-99554792 |
| AFF3-839 | chr2:100059485-100059512 | AFF3-3832 | chr2:99554781-99554808 |
| AFF3-840 | chr2:100059489-100059516 | AFF3-3833 | chr2:99554803-99554830 |
| AFF3-841 | chr2:100059490-100059517 | AFF3-3834 | chr2:99554816-99554843 |
| AFF3-842 | chr2:100059492-100059519 | AFF3-3835 | chr2:99554823-99554850 |
| AFF3-843 | chr2:100059515-100059542 | AFF3-3836 | chr2:99558753-99558780 |
| AFF3-844 | chr2:100059521-100059548 | AFF3-3837 | chr2:99558762-99558789 |
| AFF3-845 | chr2:100059576-100059603 | AFF3-3838 | chr2:99558768-99558795 |
| AFF3-846 | chr2:100059579-100059606 | AFF3-3839 | chr2:99558772-99558799 |
| AFF3-847 | chr2:100059586-100059613 | AFF3-3840 | chr2:99558779-99558806 |
| AFF3-848 | chr2:100059619-100059646 | AFF3-3841 | chr2:99558782-99558809 |
| AFF3-849 | chr2:100059638-100059665 | AFF3-3842 | chr2:99558783-99558810 |
| AFF3-850 | chr2:100059640-100059667 | AFF3-3843 | chr2:99558784-99558811 |
| AFF3-851 | chr2:100059668-100059695 | AFF3-3844 | chr2:99558799-99558826 |
| AFF3-852 | chr2:100059673-100059700 | AFF3-3845 | chr2:99558800-99558827 |
| AFF3-853 | chr2:100059682-100059709 | AFF3-3846 | chr2:99558802-99558829 |
| AFF3-854 | chr2:100059684-100059711 | AFF3-3847 | chr2:99558818-99558845 |
| AFF3-855 | chr2:100059690-100059717 | AFF3-3848 | chr2:99558874-99558901 |
| AFF3-856 | chr2:100059700-100059727 | AFF3-3849 | chr2:99558890-99558917 |
| AFF3-857 | chr2:100059719-100059746 | AFF3-3850 | chr2:99558919-99558946 |
| AFF3-858 | chr2:100059740-100059767 | AFF3-3851 | chr2:99558920-99558947 |
| AFF3-859 | chr2:100059747-100059774 | AFF3-3852 | chr2:99558923-99558950 |
| AFF3-860 | chr2:100059760-100059787 | AFF3-3853 | chr2:99558926-99558953 |
| AFF3-861 | chr2:100059772-100059799 | AFF3-3854 | chr2:99558927-99558954 |
| AFF3-862 | chr2:100059777-100059804 | AFF3-3855 | chr2:99558932-99558959 |
| AFF3-863 | chr2:100059780-100059807 | AFF3-3856 | chr2:99558938-99558965 |
| AFF3-864 | chr2:100059788-100059815 | AFF3-3857 | chr2:99558941-99558968 |
| AFF3-865 | chr2:100059795-100059822 | AFF3-3858 | chr2:99558948-99558975 |
| AFF3-866 | chr2:100059801-100059828 | AFF3-3859 | chr2:99558955-99558982 |
| AFF3-867 | chr2:100059812-100059839 | AFF3-3860 | chr2:99558964-99558991 |
| AFF3-868 | chr2:100059818-100059845 | AFF3-3861 | chr2:99558975-99559002 |
| AFF3-869 | chr2:100059833-100059860 | AFF3-3862 | chr2:99558992-99559019 |
| AFF3-870 | chr2:100059848-100059875 | AFF3-3863 | chr2:99558993-99559020 |
| AFF3-871 | chr2:100059852-100059879 | AFF3-3864 | chr2:99559028-99559055 |
| AFF3-872 | chr2:100059868-100059895 | AFF3-3865 | chr2:99559060-99559087 |
| AFF3-873 | chr2:100059882-100059909 | AFF3-3866 | chr2:99560251-99560278 |
| AFF3-874 | chr2:100059898-100059925 | AFF3-3867 | chr2:99560252-99560279 |
| AFF3-875 | chr2:100059921-100059948 | AFF3-3868 | chr2:99560278-99560305 |
| AFF3-876 | chr2:100059984-100060011 | AFF3-3869 | chr2:99560291-99560318 |
| AFF3-877 | chr2:100060001-100060028 | AFF3-3870 | chr2:99560292-99560319 |
| AFF3-878 | chr2:100060010-100060037 | AFF3-3871 | chr2:99560301-99560328 |
| AFF3-879 | chr2:100060013-100060040 | AFF3-3872 | chr2:99560305-99560332 |
| AFF3-880 | chr2:100060014-100060041 | AFF3-3873 | chr2:99560306-99560333 |
| AFF3-881 | chr2:100060015-100060042 | AFF3-3874 | chr2:99560307-99560334 |
| AFF3-882 | chr2:100060034-100060061 | AFF3-3875 | chr2:99560310-99560337 |
| AFF3-883 | chr2:100060035-100060062 | AFF3-3876 | chr2:99560323-99560350 |
| AFF3-884 | chr2:100060036-100060063 | AFF3-3877 | chr2:99560329-99560356 |
| AFF3-885 | chr2:100060037-100060064 | AFF3-3878 | chr2:99560330-99560357 |
| AFF3-886 | chr2:100060038-100060065 | AFF3-3879 | chr2:99560364-99560391 |
| AFF3-887 | chr2:100060044-100060071 | AFF3-3880 | chr2:99560366-99560393 |
| AFF3-888 | chr2:100060045-100060072 | AFF3-3881 | chr2:99560371-99560398 |
| AFF3-889 | chr2:100060057-100060084 | AFF3-3882 | chr2:99560375-99560402 |
| AFF3-890 | chr2:100060058-100060085 | AFF3-3883 | chr2:99560377-99560404 |
| AFF3-891 | chr2:100060071-100060098 | AFF3-3884 | chr2:99560378-99560405 |
| AFF3-892 | chr2:100060072-100060099 | AFF3-3885 | chr2:99560379-99560406 |
| AFF3-893 | chr2:100060076-100060103 | AFF3-3886 | chr2:99560388-99560415 |
| AFF3-894 | chr2:100060077-100060104 | AFF3-3887 | chr2:99560389-99560416 |
| AFF3-895 | chr2:100060083-100060110 | AFF3-3888 | chr2:99560407-99560434 |
| AFF3-896 | chr2:100060093-100060120 | AFF3-3889 | chr2:99560423-99560450 |
| AFF3-897 | chr2:100060094-100060121 | AFF3-3890 | chr2:99560424-99560451 |
| AFF3-898 | chr2:100060097-100060124 | AFF3-3891 | chr2:99560425-99560452 |
| AFF3-899 | chr2:100060101-100060128 | AFF3-3892 | chr2:99560428-99560455 |
| AFF3-900 | chr2:100060104-100060131 | AFF3-3893 | chr2:99560431-99560458 |
| AFF3-901 | chr2:100060108-100060135 | AFF3-3894 | chr2:99560436-99560463 |
| AFF3-902 | chr2:100060116-100060143 | AFF3-3895 | chr2:99560503-99560530 |
| AFF3-903 | chr2:100060117-100060144 | AFF3-3896 | chr2:99565360-99565387 |
| AFF3-904 | chr2:100060118-100060145 | AFF3-3897 | chr2:99565363-99565390 |
| AFF3-905 | chr2:100060120-100060147 | AFF3-3898 | chr2:99565364-99565391 |
| AFF3-906 | chr2:100060122-100060149 | AFF3-3899 | chr2:99565365-99565392 |
| AFF3-907 | chr2:100060125-100060152 | AFF3-3900 | chr2:99565366-99565393 |
| AFF3-908 | chr2:100060170-100060197 | AFF3-3901 | chr2:99565370-99565397 |
| AFF3-909 | chr2:100060182-100060209 | AFF3-3902 | chr2:99565376-99565403 |
| AFF3-910 | chr2:100060184-100060211 | AFF3-3903 | chr2:99565383-99565410 |
| AFF3-911 | chr2:100060204-100060231 | AFF3-3904 | chr2:99565425-99565452 |
| AFF3-912 | chr2:100060227-100060254 | AFF3-3905 | chr2:99565436-99565463 |
| AFF3-913 | chr2:100060251-100060278 | AFF3-3906 | chr2:99565445-99565472 |
| AFF3-914 | chr2:100060257-100060284 | AFF3-3907 | chr2:99565452-99565479 |
| AFF3-915 | chr2:100060275-100060302 | AFF3-3908 | chr2:99565455-99565482 |
| AFF3-916 | chr2:100060276-100060303 | AFF3-3909 | chr2:99565456-99565483 |
| AFF3-917 | chr2:100060280-100060307 | AFF3-3910 | chr2:99565457-99565484 |
| AFF3-918 | chr2:100060291-100060318 | AFF3-3911 | chr2:99565463-99565490 |
| AFF3-919 | chr2:100060305-100060332 | AFF3-3912 | chr2:99565486-99565513 |
| AFF3-920 | chr2:100060311-100060338 | AFF3-3913 | chr2:99565497-99565524 |
| AFF3-921 | chr2:100060330-100060357 | AFF3-3914 | chr2:99565505-99565532 |
| AFF3-922 | chr2:100060346-100060373 | AFF3-3915 | chr2:99565514-99565541 |
| AFF3-923 | chr2:100060351-100060378 | AFF3-3916 | chr2:99565515-99565542 |
| AFF3-924 | chr2:100060357-100060384 | AFF3-3917 | chr2:99565516-99565543 |
| AFF3-925 | chr2:100060358-100060385 | AFF3-3918 | chr2:99565536-99565563 |
| AFF3-926 | chr2:100060379-100060406 | AFF3-3919 | chr2:99565544-99565571 |
| AFF3-927 | chr2:100060388-100060415 | AFF3-3920 | chr2:99565551-99565578 |
| AFF3-928 | chr2:100060393-100060420 | AFF3-3921 | chr2:99565562-99565589 |
| AFF3-929 | chr2:100060394-100060421 | AFF3-3922 | chr2:99565605-99565632 |
| AFF3-930 | chr2:100060409-100060436 | AFF3-3923 | chr2:99565610-99565637 |
| AFF3-931 | chr2:100060421-100060448 | AFF3-3924 | chr2:99565620-99565647 |
| AFF3-932 | chr2:100060427-100060454 | AFF3-3925 | chr2:99565623-99565650 |
| AFF3-933 | chr2:100060430-100060457 | AFF3-3926 | chr2:99565650-99565677 |
| AFF3-934 | chr2:100060431-100060458 | AFF3-3927 | chr2:99565652-99565679 |
| AFF3-935 | chr2:100060442-100060469 | AFF3-3928 | chr2:99565699-99565726 |
| AFF3-936 | chr2:100060445-100060472 | AFF3-3929 | chr2:99565713-99565740 |
| AFF3-937 | chr2:100060454-100060481 | AFF3-3930 | chr2:99565714-99565741 |
| AFF3-938 | chr2:100060462-100060489 | AFF3-3931 | chr2:99568727-99568754 |
| AFF3-939 | chr2:100060468-100060495 | AFF3-3932 | chr2:99568739-99568766 |
| AFF3-940 | chr2:100060471-100060498 | AFF3-3933 | chr2:99568740-99568767 |
| AFF3-941 | chr2:100060473-100060500 | AFF3-3934 | chr2:99568744-99568771 |
| AFF3-942 | chr2:100060480-100060507 | AFF3-3935 | chr2:99568748-99568775 |
| AFF3-943 | chr2:100060485-100060512 | AFF3-3936 | chr2:99568771-99568798 |
| AFF3-944 | chr2:100060486-100060513 | AFF3-3937 | chr2:99568772-99568799 |
| AFF3-945 | chr2:100060497-100060524 | AFF3-3938 | chr2:99568773-99568800 |
| AFF3-946 | chr2:100060618-100060645 | AFF3-3939 | chr2:99568786-99568813 |
| AFF3-947 | chr2:100060619-100060646 | AFF3-3940 | chr2:99568787-99568814 |
| AFF3-948 | chr2:100060620-100060647 | AFF3-3941 | chr2:99568793-99568820 |
| AFF3-949 | chr2:100060676-100060703 | AFF3-3942 | chr2:99568807-99568834 |
| AFF3-950 | chr2:100060679-100060706 | AFF3-3943 | chr2:99568817-99568844 |
| AFF3-951 | chr2:100060680-100060707 | AFF3-3944 | chr2:99568818-99568845 |
| AFF3-952 | chr2:100060689-100060716 | AFF3-3945 | chr2:99568851-99568878 |
| AFF3-953 | chr2:100060690-100060717 | AFF3-3946 | chr2:99568877-99568904 |
| AFF3-954 | chr2:100060691-100060718 | AFF3-3947 | chr2:99568887-99568914 |
| AFF3-955 | chr2:100060696-100060723 | AFF3-3948 | chr2:99568896-99568923 |
| AFF3-956 | chr2:100060697-100060724 | AFF3-3949 | chr2:99568915-99568942 |
| AFF3-957 | chr2:100060698-100060725 | AFF3-3950 | chr2:99568923-99568950 |
| AFF3-958 | chr2:100060699-100060726 | AFF3-3951 | chr2:99568975-99569002 |
| AFF3-959 | chr2:100060707-100060734 | AFF3-3952 | chr2:99568979-99569006 |
| AFF3-960 | chr2:100060708-100060735 | AFF3-3953 | chr2:99568983-99569010 |
| AFF3-961 | chr2:100060709-100060736 | AFF3-3954 | chr2:99569003-99569030 |
| AFF3-962 | chr2:100060734-100060761 | AFF3-3955 | chr2:99578203-99578230 |
| AFF3-963 | chr2:100060742-100060769 | AFF3-3956 | chr2:99578204-99578231 |
| AFF3-964 | chr2:100060743-100060770 | AFF3-3957 | chr2:99578210-99578237 |
| AFF3-965 | chr2:100060746-100060773 | AFF3-3958 | chr2:99578213-99578240 |
| AFF3-966 | chr2:100060755-100060782 | AFF3-3959 | chr2:99578220-99578247 |
| AFF3-967 | chr2:100060791-100060818 | AFF3-3960 | chr2:99578224-99578251 |
| AFF3-968 | chr2:100060835-100060862 | AFF3-3961 | chr2:99578241-99578268 |
| AFF3-969 | chr2:100060836-100060863 | AFF3-3962 | chr2:99578246-99578273 |
| AFF3-970 | chr2:100060837-100060864 | AFF3-3963 | chr2:99578254-99578281 |
| AFF3-971 | chr2:100060846-100060873 | AFF3-3964 | chr2:99578255-99578282 |
| AFF3-972 | chr2:100060856-100060883 | AFF3-3965 | chr2:99578260-99578287 |
| AFF3-973 | chr2:100060857-100060884 | AFF3-3966 | chr2:99578261-99578288 |
| AFF3-974 | chr2:100060864-100060891 | AFF3-3967 | chr2:99578262-99578289 |
| AFF3-975 | chr2:100060865-100060892 | AFF3-3968 | chr2:99578266-99578293 |
| AFF3-976 | chr2:100060869-100060896 | AFF3-3969 | chr2:99578269-99578296 |
| AFF3-977 | chr2:100060870-100060897 | AFF3-3970 | chr2:99578294-99578321 |
| AFF3-978 | chr2:100060877-100060904 | AFF3-3971 | chr2:99578295-99578322 |
| AFF3-979 | chr2:100060882-100060909 | AFF3-3972 | chr2:99578296-99578323 |
| AFF3-980 | chr2:100060894-100060921 | AFF3-3973 | chr2:99578301-99578328 |
| AFF3-981 | chr2:100060900-100060927 | AFF3-3974 | chr2:99578341-99578368 |
| AFF3-982 | chr2:100060905-100060932 | AFF3-3975 | chr2:99578348-99578375 |
| AFF3-983 | chr2:100060930-100060957 | AFF3-3976 | chr2:99578350-99578377 |
| AFF3-984 | chr2:100060933-100060960 | AFF3-3977 | chr2:99578355-99578382 |
| AFF3-985 | chr2:100060934-100060961 | AFF3-3978 | chr2:99578361-99578388 |
| AFF3-986 | chr2:100060938-100060965 | AFF3-3979 | chr2:99578362-99578389 |
| AFF3-987 | chr2:100060950-100060977 | AFF3-3980 | chr2:99578364-99578391 |
| AFF3-988 | chr2:100060957-100060984 | AFF3-3981 | chr2:99578369-99578396 |
| AFF3-989 | chr2:100060973-100061000 | AFF3-3982 | chr2:99578376-99578403 |
| AFF3-990 | chr2:100060978-100061005 | AFF3-3983 | chr2:99578378-99578405 |
| AFF3-991 | chr2:100060993-100061020 | AFF3-3984 | chr2:99578386-99578413 |
| AFF3-992 | chr2:100060994-100061021 | AFF3-3985 | chr2:99578389-99578416 |
| AFF3-993 | chr2:100061022-100061049 | AFF3-3986 | chr2:99578392-99578419 |
| AFF3-994 | chr2:100061023-100061050 | AFF3-3987 | chr2:99578393-99578420 |
| AFF3-995 | chr2:100061032-100061059 | AFF3-3988 | chr2:99578394-99578421 |
| AFF3-996 | chr2:100061038-100061065 | AFF3-3989 | chr2:99578404-99578431 |
| AFF3-997 | chr2:100061039-100061066 | AFF3-3990 | chr2:99578460-99578487 |
| AFF3-998 | chr2:100061073-100061100 | AFF3-3991 | chr2:99578468-99578495 |
| AFF3-999 | chr2:100061077-100061104 | AFF3-3992 | chr2:99578488-99578515 |
| AFF3-1000 | chr2:100061085-100061112 | AFF3-3993 | chr2:99578491-99578518 |
| AFF3-1001 | chr2:100061091-100061118 | AFF3-3994 | chr2:99578508-99578535 |
| AFF3-1002 | chr2:100061104-100061131 | AFF3-3995 | chr2:99578509-99578536 |
| AFF3-1003 | chr2:100061105-100061132 | AFF3-3996 | chr2:99578515-99578542 |
| AFF3-1004 | chr2:100061125-100061152 | AFF3-3997 | chr2:99582699-99582726 |
| AFF3-1005 | chr2:100061148-100061175 | AFF3-3998 | chr2:99582700-99582727 |
| AFF3-1006 | chr2:100061158-100061185 | AFF3-3999 | chr2:99582717-99582744 |
| AFF3-1007 | chr2:100061161-100061188 | AFF3-4000 | chr2:99582718-99582745 |
| AFF3-1008 | chr2:100061180-100061207 | AFF3-4001 | chr2:99582719-99582746 |
| AFF3-1009 | chr2:100061185-100061212 | AFF3-4002 | chr2:99582720-99582747 |
| AFF3-1010 | chr2:100061186-100061213 | AFF3-4003 | chr2:99582738-99582765 |
| AFF3-1011 | chr2:100061208-100061235 | AFF3-4004 | chr2:99582748-99582775 |
| AFF3-1012 | chr2:100061211-100061238 | AFF3-4005 | chr2:99582749-99582776 |
| AFF3-1013 | chr2:100061212-100061239 | AFF3-4006 | chr2:99582754-99582781 |
| AFF3-1014 | chr2:100061215-100061242 | AFF3-4007 | chr2:99582777-99582804 |
| AFF3-1015 | chr2:100061223-100061250 | AFF3-4008 | chr2:99582791-99582818 |
| AFF3-1016 | chr2:100061224-100061251 | AFF3-4009 | chr2:99582797-99582824 |
| AFF3-1017 | chr2:100061239-100061266 | AFF3-4010 | chr2:99582801-99582828 |
| AFF3-1018 | chr2:100061240-100061267 | AFF3-4011 | chr2:99582808-99582835 |
| AFF3-1019 | chr2:100061241-100061268 | AFF3-4012 | chr2:99582811-99582838 |
| AFF3-1020 | chr2:100061252-100061279 | AFF3-4013 | chr2:99582815-99582842 |
| AFF3-1021 | chr2:100061258-100061285 | AFF3-4014 | chr2:99582826-99582853 |
| AFF3-1022 | chr2:100061278-100061305 | AFF3-4015 | chr2:99582832-99582859 |
| AFF3-1023 | chr2:100061288-100061315 | AFF3-4016 | chr2:99582836-99582863 |
| AFF3-1024 | chr2:100061291-100061318 | AFF3-4017 | chr2:99582860-99582887 |
| AFF3-1025 | chr2:100061311-100061338 | AFF3-4018 | chr2:99582864-99582891 |
| AFF3-1026 | chr2:100061333-100061360 | AFF3-4019 | chr2:99582868-99582895 |
| AFF3-1027 | chr2:100061343-100061370 | AFF3-4020 | chr2:99582880-99582907 |
| AFF3-1028 | chr2:100061361-100061388 | AFF3-4021 | chr2:99582889-99582916 |
| AFF3-1029 | chr2:100061362-100061389 | AFF3-4022 | chr2:99582908-99582935 |
| AFF3-1030 | chr2:100061364-100061391 | AFF3-4023 | chr2:99582918-99582945 |
| AFF3-1031 | chr2:100061366-100061393 | AFF3-4024 | chr2:99582919-99582946 |
| AFF3-1032 | chr2:100061367-100061394 | AFF3-4025 | chr2:99582920-99582947 |
| AFF3-1033 | chr2:100061373-100061400 | AFF3-4026 | chr2:99582928-99582955 |
| AFF3-1034 | chr2:100061404-100061431 | AFF3-4027 | chr2:99582929-99582956 |
| AFF3-1035 | chr2:100061407-100061434 | AFF3-4028 | chr2:99582960-99582987 |
| AFF3-1036 | chr2:100061410-100061437 | AFF3-4029 | chr2:99582962-99582989 |
| AFF3-1037 | chr2:100061431-100061458 | AFF3-4030 | chr2:99582982-99583009 |
| AFF3-1038 | chr2:100061453-100061480 | AFF3-4031 | chr2:99582991-99583018 |
| AFF3-1039 | chr2:100061454-100061481 | AFF3-4032 | chr2:99582995-99583022 |
| AFF3-1040 | chr2:100061463-100061490 | AFF3-4033 | chr2:99582997-99583024 |
| AFF3-1041 | chr2:100061470-100061497 | AFF3-4034 | chr2:99583019-99583046 |
| AFF3-1042 | chr2:100061471-100061498 | AFF3-4035 | chr2:99583020-99583047 |
| AFF3-1043 | chr2:100061482-100061509 | AFF3-4036 | chr2:99583025-99583052 |
| AFF3-1044 | chr2:100061503-100061530 | AFF3-4037 | chr2:99583060-99583087 |
| AFF3-1045 | chr2:100061508-100061535 | AFF3-4038 | chr2:99583061-99583088 |
| AFF3-1046 | chr2:100061511-100061538 | AFF3-4039 | chr2:99583070-99583097 |
| AFF3-1047 | chr2:100061524-100061551 | AFF3-4040 | chr2:99583071-99583098 |
| AFF3-1048 | chr2:100061546-100061573 | AFF3-4041 | chr2:99583074-99583101 |
| AFF3-1049 | chr2:100061549-100061576 | AFF3-4042 | chr2:99583076-99583103 |
| AFF3-1050 | chr2:100061564-100061591 | AFF3-4043 | chr2:99583079-99583106 |
| AFF3-1051 | chr2:100061565-100061592 | AFF3-4044 | chr2:99583080-99583107 |
| AFF3-1052 | chr2:100061574-100061601 | AFF3-4045 | chr2:99583087-99583114 |
| AFF3-1053 | chr2:100061575-100061602 | AFF3-4046 | chr2:99583088-99583115 |
| AFF3-1054 | chr2:100061579-100061606 | AFF3-4047 | chr2:99583090-99583117 |
| AFF3-1055 | chr2:100061590-100061617 | AFF3-4048 | chr2:99587044-99587071 |
| AFF3-1056 | chr2:100061635-100061662 | AFF3-4049 | chr2:99587050-99587077 |
| AFF3-1057 | chr2:100061642-100061669 | AFF3-4050 | chr2:99587060-99587087 |
| AFF3-1058 | chr2:100061649-100061676 | AFF3-4051 | chr2:99587067-99587094 |
| AFF3-1059 | chr2:100061659-100061686 | AFF3-4052 | chr2:99587070-99587097 |
| AFF3-1060 | chr2:100061660-100061687 | AFF3-4053 | chr2:99587075-99587102 |
| AFF3-1061 | chr2:100061672-100061699 | AFF3-4054 | chr2:99587076-99587103 |
| AFF3-1062 | chr2:100061681-100061708 | AFF3-4055 | chr2:99587079-99587106 |
| AFF3-1063 | chr2:100061735-100061762 | AFF3-4056 | chr2:99587082-99587109 |
| AFF3-1064 | chr2:100061736-100061763 | AFF3-4057 | chr2:99587106-99587133 |
| AFF3-1065 | chr2:100061737-100061764 | AFF3-4058 | chr2:99587107-99587134 |
| AFF3-1066 | chr2:100061741-100061768 | AFF3-4059 | chr2:99587111-99587138 |
| AFF3-1067 | chr2:100061766-100061793 | AFF3-4060 | chr2:99587114-99587141 |
| AFF3-1068 | chr2:100061767-100061794 | AFF3-4061 | chr2:99587115-99587142 |
| AFF3-1069 | chr2:100061768-100061795 | AFF3-4062 | chr2:99587119-99587146 |
| AFF3-1070 | chr2:100061769-100061796 | AFF3-4063 | chr2:99587130-99587157 |
| AFF3-1071 | chr2:100061817-100061844 | AFF3-4064 | chr2:99587150-99587177 |
| AFF3-1072 | chr2:100061818-100061845 | AFF3-4065 | chr2:99587153-99587180 |
| AFF3-1073 | chr2:100061819-100061846 | AFF3-4066 | chr2:99587172-99587199 |
| AFF3-1074 | chr2:100061820-100061847 | AFF3-4067 | chr2:99587175-99587202 |
| AFF3-1075 | chr2:100061821-100061848 | AFF3-4068 | chr2:99587178-99587205 |
| AFF3-1076 | chr2:100061824-100061851 | AFF3-4069 | chr2:99587181-99587208 |
| AFF3-1077 | chr2:100061827-100061854 | AFF3-4070 | chr2:99587209-99587236 |
| AFF3-1078 | chr2:100061829-100061856 | AFF3-4071 | chr2:99587213-99587240 |
| AFF3-1079 | chr2:100061832-100061859 | AFF3-4072 | chr2:99587214-99587241 |
| AFF3-1080 | chr2:100061833-100061860 | AFF3-4073 | chr2:99587235-99587262 |
| AFF3-1081 | chr2:100061834-100061861 | AFF3-4074 | chr2:99587236-99587263 |
| AFF3-1082 | chr2:100061835-100061862 | AFF3-4075 | chr2:99587254-99587281 |
| AFF3-1083 | chr2:100061836-100061863 | AFF3-4076 | chr2:99587255-99587282 |
| AFF3-1084 | chr2:100061837-100061864 | AFF3-4077 | chr2:99587259-99587286 |
| AFF3-1085 | chr2:100061838-100061865 | AFF3-4078 | chr2:99587260-99587287 |
| AFF3-1086 | chr2:100061839-100061866 | AFF3-4079 | chr2:99587286-99587313 |
| AFF3-1087 | chr2:100061840-100061867 | AFF3-4080 | chr2:99587297-99587324 |
| AFF3-1088 | chr2:100061841-100061868 | AFF3-4081 | chr2:99587314-99587341 |
| AFF3-1089 | chr2:100061869-100061896 | AFF3-4082 | chr2:99587336-99587363 |
| AFF3-1090 | chr2:100061871-100061898 | AFF3-4083 | chr2:99587337-99587364 |
| AFF3-1091 | chr2:100061884-100061911 | AFF3-4084 | chr2:99587344-99587371 |
| AFF3-1092 | chr2:100061919-100061946 | AFF3-4085 | chr2:99587345-99587372 |
| AFF3-1093 | chr2:100061922-100061949 | AFF3-4086 | chr2:99587351-99587378 |
| AFF3-1094 | chr2:100061930-100061957 | AFF3-4087 | chr2:99587361-99587388 |
| AFF3-1095 | chr2:100061933-100061960 | AFF3-4088 | chr2:99587364-99587391 |
| AFF3-1096 | chr2:100061935-100061962 | AFF3-4089 | chr2:99587367-99587394 |
| AFF3-1097 | chr2:100061942-100061969 | AFF3-4090 | chr2:99587375-99587402 |
| AFF3-1098 | chr2:100061950-100061977 | AFF3-4091 | chr2:99587376-99587403 |
| AFF3-1099 | chr2:100061963-100061990 | AFF3-4092 | chr2:99587377-99587404 |
| AFF3-1100 | chr2:100061982-100062009 | AFF3-4093 | chr2:99593077-99593104 |
| AFF3-1101 | chr2:100061983-100062010 | AFF3-4094 | chr2:99593086-99593113 |
| AFF3-1102 | chr2:100061987-100062014 | AFF3-4095 | chr2:99593087-99593114 |
| AFF3-1103 | chr2:100062016-100062043 | AFF3-4096 | chr2:99593091-99593118 |
| AFF3-1104 | chr2:100062019-100062046 | AFF3-4097 | chr2:99593095-99593122 |
| AFF3-1105 | chr2:100062024-100062051 | AFF3-4098 | chr2:99593096-99593123 |
| AFF3-1106 | chr2:100062031-100062058 | AFF3-4099 | chr2:99593107-99593134 |
| AFF3-1107 | chr2:100062032-100062059 | AFF3-4100 | chr2:99593123-99593150 |
| AFF3-1108 | chr2:100062037-100062064 | AFF3-4101 | chr2:99593130-99593157 |
| AFF3-1109 | chr2:100062038-100062065 | AFF3-4102 | chr2:99593131-99593158 |
| AFF3-1110 | chr2:100062054-100062081 | AFF3-4103 | chr2:99593149-99593176 |
| AFF3-1111 | chr2:100062063-100062090 | AFF3-4104 | chr2:99593150-99593177 |
| AFF3-1112 | chr2:100062064-100062091 | AFF3-4105 | chr2:99593151-99593178 |
| AFF3-1113 | chr2:100062065-100062092 | AFF3-4106 | chr2:99593155-99593182 |
| AFF3-1114 | chr2:100062069-100062096 | AFF3-4107 | chr2:99593156-99593183 |
| AFF3-1115 | chr2:100062078-100062105 | AFF3-4108 | chr2:99593157-99593184 |
| AFF3-1116 | chr2:100062100-100062127 | AFF3-4109 | chr2:99593160-99593187 |
| AFF3-1117 | chr2:100062106-100062133 | AFF3-4110 | chr2:99593162-99593189 |
| AFF3-1118 | chr2:100062107-100062134 | AFF3-4111 | chr2:99593165-99593192 |
| AFF3-1119 | chr2:100062108-100062135 | AFF3-4112 | chr2:99593166-99593193 |
| AFF3-1120 | chr2:100062109-100062136 | AFF3-4113 | chr2:99593167-99593194 |
| AFF3-1121 | chr2:100062110-100062137 | AFF3-4114 | chr2:99593168-99593195 |
| AFF3-1122 | chr2:100062114-100062141 | AFF3-4115 | chr2:99593173-99593200 |
| AFF3-1123 | chr2:100062115-100062142 | AFF3-4116 | chr2:99593174-99593201 |
| AFF3-1124 | chr2:100062117-100062144 | AFF3-4117 | chr2:99593175-99593202 |
| AFF3-1125 | chr2:100062127-100062154 | AFF3-4118 | chr2:99593183-99593210 |
| AFF3-1126 | chr2:100062128-100062155 | AFF3-4119 | chr2:99593184-99593211 |
| AFF3-1127 | chr2:100062129-100062156 | AFF3-4120 | chr2:99593185-99593212 |
| AFF3-1128 | chr2:100062133-100062160 | AFF3-4121 | chr2:99593190-99593217 |
| AFF3-1129 | chr2:100062139-100062166 | AFF3-4122 | chr2:99593191-99593218 |
| AFF3-1130 | chr2:100062146-100062173 | AFF3-4123 | chr2:99593194-99593221 |
| AFF3-1131 | chr2:100062147-100062174 | AFF3-4124 | chr2:99593204-99593231 |
| AFF3-1132 | chr2:100062175-100062202 | AFF3-4125 | chr2:99593209-99593236 |
| AFF3-1133 | chr2:100062176-100062203 | AFF3-4126 | chr2:99593220-99593247 |
| AFF3-1134 | chr2:100062183-100062210 | AFF3-4127 | chr2:99593224-99593251 |
| AFF3-1135 | chr2:100062184-100062211 | AFF3-4128 | chr2:99593225-99593252 |
| AFF3-1136 | chr2:100062188-100062215 | AFF3-4129 | chr2:99593229-99593256 |
| AFF3-1137 | chr2:100062193-100062220 | AFF3-4130 | chr2:99593230-99593257 |
| AFF3-1138 | chr2:100062207-100062234 | AFF3-4131 | chr2:99593233-99593260 |
| AFF3-1139 | chr2:100062210-100062237 | AFF3-4132 | chr2:99593242-99593269 |
| AFF3-1140 | chr2:100062220-100062247 | AFF3-4133 | chr2:99593253-99593280 |
| AFF3-1141 | chr2:100062223-100062250 | AFF3-4134 | chr2:99593256-99593283 |
| AFF3-1142 | chr2:100062224-100062251 | AFF3-4135 | chr2:99593260-99593287 |
| AFF3-1143 | chr2:100062225-100062252 | AFF3-4136 | chr2:99593261-99593288 |
| AFF3-1144 | chr2:100062236-100062263 | AFF3-4137 | chr2:99593262-99593289 |
| AFF3-1145 | chr2:100062245-100062272 | AFF3-4138 | chr2:99593275-99593302 |
| AFF3-1146 | chr2:100062253-100062280 | AFF3-4139 | chr2:99593278-99593305 |
| AFF3-1147 | chr2:100062254-100062281 | AFF3-4140 | chr2:99593285-99593312 |
| AFF3-1148 | chr2:100062258-100062285 | AFF3-4141 | chr2:99593286-99593313 |
| AFF3-1149 | chr2:100062262-100062289 | AFF3-4142 | chr2:99593287-99593314 |
| AFF3-1150 | chr2:100062277-100062304 | AFF3-4143 | chr2:99593291-99593318 |
| AFF3-1151 | chr2:100062278-100062305 | AFF3-4144 | chr2:99593299-99593326 |
| AFF3-1152 | chr2:100062282-100062309 | AFF3-4145 | chr2:99593300-99593327 |
| AFF3-1153 | chr2:100062290-100062317 | AFF3-4146 | chr2:99593301-99593328 |
| AFF3-1154 | chr2:100062291-100062318 | AFF3-4147 | chr2:99593307-99593334 |
| AFF3-1155 | chr2:100062297-100062324 | AFF3-4148 | chr2:99593308-99593335 |
| AFF3-1156 | chr2:100062300-100062327 | AFF3-4149 | chr2:99593312-99593339 |
| AFF3-1157 | chr2:100062313-100062340 | AFF3-4150 | chr2:99593315-99593342 |
| AFF3-1158 | chr2:100062356-100062383 | AFF3-4151 | chr2:99593316-99593343 |
| AFF3-1159 | chr2:100062371-100062398 | AFF3-4152 | chr2:99593321-99593348 |
| AFF3-1160 | chr2:100062372-100062399 | AFF3-4153 | chr2:99593330-99593357 |
| AFF3-1161 | chr2:100062377-100062404 | AFF3-4154 | chr2:99593359-99593386 |
| AFF3-1162 | chr2:100062380-100062407 | AFF3-4155 | chr2:99593360-99593387 |
| AFF3-1163 | chr2:100062404-100062431 | AFF3-4156 | chr2:99593368-99593395 |
| AFF3-1164 | chr2:100062405-100062432 | AFF3-4157 | chr2:99593369-99593396 |
| AFF3-1165 | chr2:100062406-100062433 | AFF3-4158 | chr2:99593370-99593397 |
| AFF3-1166 | chr2:100062414-100062441 | AFF3-4159 | chr2:99593386-99593413 |
| AFF3-1167 | chr2:100062420-100062447 | AFF3-4160 | chr2:99593387-99593414 |
| AFF3-1168 | chr2:100062449-100062476 | AFF3-4161 | chr2:99593394-99593421 |
| AFF3-1169 | chr2:100062450-100062477 | AFF3-4162 | chr2:99593395-99593422 |
| AFF3-1170 | chr2:100062463-100062490 | AFF3-4163 | chr2:99593396-99593423 |
| AFF3-1171 | chr2:100062469-100062496 | AFF3-4164 | chr2:99593411-99593438 |
| AFF3-1172 | chr2:100062470-100062497 | AFF3-4165 | chr2:99593415-99593442 |
| AFF3-1173 | chr2:100062471-100062498 | AFF3-4166 | chr2:99593425-99593452 |
| AFF3-1174 | chr2:100062472-100062499 | AFF3-4167 | chr2:99593430-99593457 |
| AFF3-1175 | chr2:100062476-100062503 | AFF3-4168 | chr2:99593442-99593469 |
| AFF3-1176 | chr2:100062485-100062512 | AFF3-4169 | chr2:99593443-99593470 |
| AFF3-1177 | chr2:100062486-100062513 | AFF3-4170 | chr2:99593445-99593472 |
| AFF3-1178 | chr2:100062494-100062521 | AFF3-4171 | chr2:99593446-99593473 |
| AFF3-1179 | chr2:100062505-100062532 | AFF3-4172 | chr2:99593451-99593478 |
| AFF3-1180 | chr2:100062514-100062541 | AFF3-4173 | chr2:99593452-99593479 |
| AFF3-1181 | chr2:100062515-100062542 | AFF3-4174 | chr2:99593460-99593487 |
| AFF3-1182 | chr2:100062518-100062545 | AFF3-4175 | chr2:99593470-99593497 |
| AFF3-1183 | chr2:100062522-100062549 | AFF3-4176 | chr2:99593471-99593498 |
| AFF3-1184 | chr2:100062523-100062550 | AFF3-4177 | chr2:99593472-99593499 |
| AFF3-1185 | chr2:100062537-100062564 | AFF3-4178 | chr2:99593474-99593501 |
| AFF3-1186 | chr2:100062541-100062568 | AFF3-4179 | chr2:99593479-99593506 |
| AFF3-1187 | chr2:100062542-100062569 | AFF3-4180 | chr2:99593490-99593517 |
| AFF3-1188 | chr2:100062545-100062572 | AFF3-4181 | chr2:99593496-99593523 |
| AFF3-1189 | chr2:100062546-100062573 | AFF3-4182 | chr2:99593500-99593527 |
| AFF3-1190 | chr2:100062547-100062574 | AFF3-4183 | chr2:99593501-99593528 |
| AFF3-1191 | chr2:100062548-100062575 | AFF3-4184 | chr2:99593502-99593529 |
| AFF3-1192 | chr2:100062551-100062578 | AFF3-4185 | chr2:99593508-99593535 |
| AFF3-1193 | chr2:100062567-100062594 | AFF3-4186 | chr2:99593514-99593541 |
| AFF3-1194 | chr2:100062568-100062595 | AFF3-4187 | chr2:99593515-99593542 |
| AFF3-1195 | chr2:100062569-100062596 | AFF3-4188 | chr2:99593516-99593543 |
| AFF3-1196 | chr2:100062577-100062604 | AFF3-4189 | chr2:99593517-99593544 |
| AFF3-1197 | chr2:100062587-100062614 | AFF3-4190 | chr2:99593529-99593556 |
| AFF3-1198 | chr2:100062591-100062618 | AFF3-4191 | chr2:99593530-99593557 |
| AFF3-1199 | chr2:100062597-100062624 | AFF3-4192 | chr2:99593532-99593559 |
| AFF3-1200 | chr2:100062609-100062636 | AFF3-4193 | chr2:99593533-99593560 |
| AFF3-1201 | chr2:100062628-100062655 | AFF3-4194 | chr2:99593535-99593562 |
| AFF3-1202 | chr2:100062638-100062665 | AFF3-4195 | chr2:99593540-99593567 |
| AFF3-1203 | chr2:100062646-100062673 | AFF3-4196 | chr2:99593541-99593568 |
| AFF3-1204 | chr2:100062647-100062674 | AFF3-4197 | chr2:99593550-99593577 |
| AFF3-1205 | chr2:100062648-100062675 | AFF3-4198 | chr2:99593552-99593579 |
| AFF3-1206 | chr2:100062649-100062676 | AFF3-4199 | chr2:99593553-99593580 |
| AFF3-1207 | chr2:100062652-100062679 | AFF3-4200 | chr2:99593562-99593589 |
| AFF3-1208 | chr2:100062665-100062692 | AFF3-4201 | chr2:99593569-99593596 |
| AFF3-1209 | chr2:100062666-100062693 | AFF3-4202 | chr2:99593570-99593597 |
| AFF3-1210 | chr2:100062697-100062724 | AFF3-4203 | chr2:99593572-99593599 |
| AFF3-1211 | chr2:100062713-100062740 | AFF3-4204 | chr2:99593586-99593613 |
| AFF3-1212 | chr2:100062722-100062749 | AFF3-4205 | chr2:99593594-99593621 |
| AFF3-1213 | chr2:100062725-100062752 | AFF3-4206 | chr2:99593598-99593625 |
| AFF3-1214 | chr2:100062726-100062753 | AFF3-4207 | chr2:99593602-99593629 |
| AFF3-1215 | chr2:100062727-100062754 | AFF3-4208 | chr2:99593605-99593632 |
| AFF3-1216 | chr2:100062751-100062778 | AFF3-4209 | chr2:99593607-99593634 |
| AFF3-1217 | chr2:100062755-100062782 | AFF3-4210 | chr2:99593610-99593637 |
| AFF3-1218 | chr2:100062768-100062795 | AFF3-4211 | chr2:99593613-99593640 |
| AFF3-1219 | chr2:100062769-100062796 | AFF3-4212 | chr2:99593617-99593644 |
| AFF3-1220 | chr2:100062784-100062811 | AFF3-4213 | chr2:99593629-99593656 |
| AFF3-1221 | chr2:100062817-100062844 | AFF3-4214 | chr2:99593649-99593676 |
| AFF3-1222 | chr2:100062839-100062866 | AFF3-4215 | chr2:99593655-99593682 |
| AFF3-1223 | chr2:100062864-100062891 | AFF3-4216 | chr2:99593656-99593683 |
| AFF3-1224 | chr2:100062872-100062899 | AFF3-4217 | chr2:99593657-99593684 |
| AFF3-1225 | chr2:100062938-100062965 | AFF3-4218 | chr2:99593671-99593698 |
| AFF3-1226 | chr2:100062942-100062969 | AFF3-4219 | chr2:99593684-99593711 |
| AFF3-1227 | chr2:100062943-100062970 | AFF3-4220 | chr2:99593690-99593717 |
| AFF3-1228 | chr2:100062947-100062974 | AFF3-4221 | chr2:99593697-99593724 |
| AFF3-1229 | chr2:100062948-100062975 | AFF3-4222 | chr2:99593699-99593726 |
| AFF3-1230 | chr2:100062956-100062983 | AFF3-4223 | chr2:99593700-99593727 |
| AFF3-1231 | chr2:100062961-100062988 | AFF3-4224 | chr2:99593701-99593728 |
| AFF3-1232 | chr2:100062983-100063010 | AFF3-4225 | chr2:99593702-99593729 |
| AFF3-1233 | chr2:100062984-100063011 | AFF3-4226 | chr2:99593703-99593730 |
| AFF3-1234 | chr2:100062987-100063014 | AFF3-4227 | chr2:99593706-99593733 |
| AFF3-1235 | chr2:100062991-100063018 | AFF3-4228 | chr2:99593723-99593750 |
| AFF3-1236 | chr2:100062993-100063020 | AFF3-4229 | chr2:99593726-99593753 |
| AFF3-1237 | chr2:100062996-100063023 | AFF3-4230 | chr2:99593743-99593770 |
| AFF3-1238 | chr2:100062997-100063024 | AFF3-4231 | chr2:99593748-99593775 |
| AFF3-1239 | chr2:100062998-100063025 | AFF3-4232 | chr2:99593749-99593776 |
| AFF3-1240 | chr2:100062999-100063026 | AFF3-4233 | chr2:99593754-99593781 |
| AFF3-1241 | chr2:100063000-100063027 | AFF3-4234 | chr2:99593760-99593787 |
| AFF3-1242 | chr2:100063002-100063029 | AFF3-4235 | chr2:99593763-99593790 |
| AFF3-1243 | chr2:100063015-100063042 | AFF3-4236 | chr2:99593764-99593791 |
| AFF3-1244 | chr2:100063016-100063043 | AFF3-4237 | chr2:99593769-99593796 |
| AFF3-1245 | chr2:100063024-100063051 | AFF3-4238 | chr2:99593770-99593797 |
| AFF3-1246 | chr2:100063032-100063059 | AFF3-4239 | chr2:99593771-99593798 |
| AFF3-1247 | chr2:100063034-100063061 | AFF3-4240 | chr2:99593772-99593799 |
| AFF3-1248 | chr2:100063039-100063066 | AFF3-4241 | chr2:99593773-99593800 |
| AFF3-1249 | chr2:100063043-100063070 | AFF3-4242 | chr2:99593774-99593801 |
| AFF3-1250 | chr2:100063053-100063080 | AFF3-4243 | chr2:99593777-99593804 |
| AFF3-1251 | chr2:100063057-100063084 | AFF3-4244 | chr2:99593794-99593821 |
| AFF3-1252 | chr2:100063062-100063089 | AFF3-4245 | chr2:99593803-99593830 |
| AFF3-1253 | chr2:100063076-100063103 | AFF3-4246 | chr2:99593805-99593832 |
| AFF3-1254 | chr2:100063077-100063104 | AFF3-4247 | chr2:99593806-99593833 |
| AFF3-1255 | chr2:100063078-100063105 | AFF3-4248 | chr2:99593811-99593838 |
| AFF3-1256 | chr2:100063083-100063110 | AFF3-4249 | chr2:99593812-99593839 |
| AFF3-1257 | chr2:100063088-100063115 | AFF3-4250 | chr2:99593816-99593843 |
| AFF3-1258 | chr2:100063091-100063118 | AFF3-4251 | chr2:99593817-99593844 |
| AFF3-1259 | chr2:100063106-100063133 | AFF3-4252 | chr2:99593818-99593845 |
| AFF3-1260 | chr2:100063111-100063138 | AFF3-4253 | chr2:99593820-99593847 |
| AFF3-1261 | chr2:100063118-100063145 | AFF3-4254 | chr2:99593821-99593848 |
| AFF3-1262 | chr2:100063119-100063146 | AFF3-4255 | chr2:99593825-99593852 |
| AFF3-1263 | chr2:100063122-100063149 | AFF3-4256 | chr2:99593827-99593854 |
| AFF3-1264 | chr2:100063125-100063152 | AFF3-4257 | chr2:99593828-99593855 |
| AFF3-1265 | chr2:100063128-100063155 | AFF3-4258 | chr2:99593833-99593860 |
| AFF3-1266 | chr2:100063132-100063159 | AFF3-4259 | chr2:99593835-99593862 |
| AFF3-1267 | chr2:100063133-100063160 | AFF3-4260 | chr2:99593842-99593869 |
| AFF3-1268 | chr2:100063134-100063161 | AFF3-4261 | chr2:99593847-99593874 |
| AFF3-1269 | chr2:100063150-100063177 | AFF3-4262 | chr2:99593849-99593876 |
| AFF3-1270 | chr2:100063151-100063178 | AFF3-4263 | chr2:99593853-99593880 |
| AFF3-1271 | chr2:100063154-100063181 | AFF3-4264 | chr2:99593862-99593889 |
| AFF3-1272 | chr2:100063157-100063184 | AFF3-4265 | chr2:99593865-99593892 |
| AFF3-1273 | chr2:100063160-100063187 | AFF3-4266 | chr2:99593869-99593896 |
| AFF3-1274 | chr2:100063173-100063200 | AFF3-4267 | chr2:99593870-99593897 |
| AFF3-1275 | chr2:100063198-100063225 | AFF3-4268 | chr2:99593871-99593898 |
| AFF3-1276 | chr2:100063200-100063227 | AFF3-4269 | chr2:99593874-99593901 |
| AFF3-1277 | chr2:100063201-100063228 | AFF3-4270 | chr2:99593875-99593902 |
| AFF3-1278 | chr2:100063209-100063236 | AFF3-4271 | chr2:99593882-99593909 |
| AFF3-1279 | chr2:100063219-100063246 | AFF3-4272 | chr2:99593889-99593916 |
| AFF3-1280 | chr2:100063223-100063250 | AFF3-4273 | chr2:99593894-99593921 |
| AFF3-1281 | chr2:100063246-100063273 | AFF3-4274 | chr2:99593897-99593924 |
| AFF3-1282 | chr2:100063254-100063281 | AFF3-4275 | chr2:99593898-99593925 |
| AFF3-1283 | chr2:100063277-100063304 | AFF3-4276 | chr2:99593902-99593929 |
| AFF3-1284 | chr2:100063288-100063315 | AFF3-4277 | chr2:99593907-99593934 |
| AFF3-1285 | chr2:100063295-100063322 | AFF3-4278 | chr2:99593908-99593935 |
| AFF3-1286 | chr2:100063361-100063388 | AFF3-4279 | chr2:99593910-99593937 |
| AFF3-1287 | chr2:100063409-100063436 | AFF3-4280 | chr2:99593911-99593938 |
| AFF3-1288 | chr2:100063414-100063441 | AFF3-4281 | chr2:99593918-99593945 |
| AFF3-1289 | chr2:100063527-100063554 | AFF3-4282 | chr2:99593921-99593948 |
| AFF3-1290 | chr2:100063531-100063558 | AFF3-4283 | chr2:99593922-99593949 |
| AFF3-1291 | chr2:100063557-100063584 | AFF3-4284 | chr2:99593923-99593950 |
| AFF3-1292 | chr2:100063575-100063602 | AFF3-4285 | chr2:99593931-99593958 |
| AFF3-1293 | chr2:100063576-100063603 | AFF3-4286 | chr2:99593932-99593959 |
| AFF3-1294 | chr2:100063610-100063637 | AFF3-4287 | chr2:99593940-99593967 |
| AFF3-1295 | chr2:100063627-100063654 | AFF3-4288 | chr2:99593941-99593968 |
| AFF3-1296 | chr2:100063629-100063656 | AFF3-4289 | chr2:99593944-99593971 |
| AFF3-1297 | chr2:100063647-100063674 | AFF3-4290 | chr2:99593947-99593974 |
| AFF3-1298 | chr2:100063656-100063683 | AFF3-4291 | chr2:99593948-99593975 |
| AFF3-1299 | chr2:100063673-100063700 | AFF3-4292 | chr2:99593949-99593976 |
| AFF3-1300 | chr2:100063677-100063704 | AFF3-4293 | chr2:99593952-99593979 |
| AFF3-1301 | chr2:100063678-100063705 | AFF3-4294 | chr2:99593970-99593997 |
| AFF3-1302 | chr2:100063680-100063707 | AFF3-4295 | chr2:99593976-99594003 |
| AFF3-1303 | chr2:100063685-100063712 | AFF3-4296 | chr2:99593982-99594009 |
| AFF3-1304 | chr2:100063686-100063713 | AFF3-4297 | chr2:99593983-99594010 |
| AFF3-1305 | chr2:100063697-100063724 | AFF3-4298 | chr2:99593986-99594013 |
| AFF3-1306 | chr2:100063722-100063749 | AFF3-4299 | chr2:99593987-99594014 |
| AFF3-1307 | chr2:100063745-100063772 | AFF3-4300 | chr2:99593988-99594015 |
| AFF3-1308 | chr2:100063761-100063788 | AFF3-4301 | chr2:99593989-99594016 |
| AFF3-1309 | chr2:100063762-100063789 | AFF3-4302 | chr2:99593992-99594019 |
| AFF3-1310 | chr2:100063769-100063796 | AFF3-4303 | chr2:99593998-99594025 |
| AFF3-1311 | chr2:100063782-100063809 | AFF3-4304 | chr2:99594004-99594031 |
| AFF3-1312 | chr2:100063786-100063813 | AFF3-4305 | chr2:99594016-99594043 |
| AFF3-1313 | chr2:100063787-100063814 | AFF3-4306 | chr2:99594017-99594044 |
| AFF3-1314 | chr2:100063792-100063819 | AFF3-4307 | chr2:99594018-99594045 |
| AFF3-1315 | chr2:100063795-100063822 | AFF3-4308 | chr2:99594027-99594054 |
| AFF3-1316 | chr2:100063822-100063849 | AFF3-4309 | chr2:99594030-99594057 |
| AFF3-1317 | chr2:100063823-100063850 | AFF3-4310 | chr2:99594037-99594064 |
| AFF3-1318 | chr2:100063826-100063853 | AFF3-4311 | chr2:99594038-99594065 |
| AFF3-1319 | chr2:100063829-100063856 | AFF3-4312 | chr2:99594039-99594066 |
| AFF3-1320 | chr2:100063832-100063859 | AFF3-4313 | chr2:99594046-99594073 |
| AFF3-1321 | chr2:100063835-100063862 | AFF3-4314 | chr2:99594059-99594086 |
| AFF3-1322 | chr2:100063836-100063863 | AFF3-4315 | chr2:99594065-99594092 |
| AFF3-1323 | chr2:100063837-100063864 | AFF3-4316 | chr2:99594073-99594100 |
| AFF3-1324 | chr2:100063838-100063865 | AFF3-4317 | chr2:99594076-99594103 |
| AFF3-1325 | chr2:100063839-100063866 | AFF3-4318 | chr2:99594083-99594110 |
| AFF3-1326 | chr2:100063850-100063877 | AFF3-4319 | chr2:99594087-99594114 |
| AFF3-1327 | chr2:100063855-100063882 | AFF3-4320 | chr2:99594088-99594115 |
| AFF3-1328 | chr2:100063871-100063898 | AFF3-4321 | chr2:99594092-99594119 |
| AFF3-1329 | chr2:100063882-100063909 | AFF3-4322 | chr2:99594097-99594124 |
| AFF3-1330 | chr2:100063892-100063919 | AFF3-4323 | chr2:99594102-99594129 |
| AFF3-1331 | chr2:100063896-100063923 | AFF3-4324 | chr2:99594103-99594130 |
| AFF3-1332 | chr2:100063901-100063928 | AFF3-4325 | chr2:99594104-99594131 |
| AFF3-1333 | chr2:100063915-100063942 | AFF3-4326 | chr2:99594122-99594149 |
| AFF3-1334 | chr2:100063916-100063943 | AFF3-4327 | chr2:99594137-99594164 |
| AFF3-1335 | chr2:100063917-100063944 | AFF3-4328 | chr2:99594138-99594165 |
| AFF3-1336 | chr2:100063920-100063947 | AFF3-4329 | chr2:99594139-99594166 |
| AFF3-1337 | chr2:100063921-100063948 | AFF3-4330 | chr2:99594140-99594167 |
| AFF3-1338 | chr2:100063926-100063953 | AFF3-4331 | chr2:99594145-99594172 |
| AFF3-1339 | chr2:100063929-100063956 | AFF3-4332 | chr2:99594154-99594181 |
| AFF3-1340 | chr2:100063944-100063971 | AFF3-4333 | chr2:99594163-99594190 |
| AFF3-1341 | chr2:100063956-100063983 | AFF3-4334 | chr2:99594170-99594197 |
| AFF3-1342 | chr2:100063957-100063984 | AFF3-4335 | chr2:99594182-99594209 |
| AFF3-1343 | chr2:100063960-100063987 | AFF3-4336 | chr2:99594191-99594218 |
| AFF3-1344 | chr2:100063963-100063990 | AFF3-4337 | chr2:99594192-99594219 |
| AFF3-1345 | chr2:100063966-100063993 | AFF3-4338 | chr2:99594193-99594220 |
| AFF3-1346 | chr2:100063971-100063998 | AFF3-4339 | chr2:99594196-99594223 |
| AFF3-1347 | chr2:100063972-100063999 | AFF3-4340 | chr2:99594203-99594230 |
| AFF3-1348 | chr2:100063988-100064015 | AFF3-4341 | chr2:99594204-99594231 |
| AFF3-1349 | chr2:100063989-100064016 | AFF3-4342 | chr2:99594205-99594232 |
| AFF3-1350 | chr2:100063992-100064019 | AFF3-4343 | chr2:99594248-99594275 |
| AFF3-1351 | chr2:100063995-100064022 | AFF3-4344 | chr2:99594249-99594276 |
| AFF3-1352 | chr2:100063998-100064025 | AFF3-4345 | chr2:99594256-99594283 |
| AFF3-1353 | chr2:100064011-100064038 | AFF3-4346 | chr2:99594263-99594290 |
| AFF3-1354 | chr2:100064012-100064039 | AFF3-4347 | chr2:99594280-99594307 |
| AFF3-1355 | chr2:100064036-100064063 | AFF3-4348 | chr2:99594289-99594316 |
| AFF3-1356 | chr2:100064038-100064065 | AFF3-4349 | chr2:99594304-99594331 |
| AFF3-1357 | chr2:100064039-100064066 | AFF3-4350 | chr2:99594308-99594335 |
| AFF3-1358 | chr2:100064047-100064074 | AFF3-4351 | chr2:99594321-99594348 |
| AFF3-1359 | chr2:100064057-100064084 | AFF3-4352 | chr2:99594322-99594349 |
| AFF3-1360 | chr2:100064061-100064088 | AFF3-4353 | chr2:99594323-99594350 |
| AFF3-1361 | chr2:100064083-100064110 | AFF3-4354 | chr2:99594328-99594355 |
| AFF3-1362 | chr2:100064117-100064144 | AFF3-4355 | chr2:99594351-99594378 |
| AFF3-1363 | chr2:100064179-100064206 | AFF3-4356 | chr2:99594379-99594406 |
| AFF3-1364 | chr2:100064207-100064234 | AFF3-4357 | chr2:99594380-99594407 |
| AFF3-1365 | chr2:100064208-100064235 | AFF3-4358 | chr2:99594384-99594411 |
| AFF3-1366 | chr2:100064223-100064250 | AFF3-4359 | chr2:99601308-99601335 |
| AFF3-1367 | chr2:100064234-100064261 | AFF3-4360 | chr2:99601311-99601338 |
| AFF3-1368 | chr2:100064239-100064266 | AFF3-4361 | chr2:99601312-99601339 |
| AFF3-1369 | chr2:100064240-100064267 | AFF3-4362 | chr2:99601342-99601369 |
| AFF3-1370 | chr2:100064252-100064279 | AFF3-4363 | chr2:99601345-99601372 |
| AFF3-1371 | chr2:100064253-100064280 | AFF3-4364 | chr2:99601368-99601395 |
| AFF3-1372 | chr2:100064273-100064300 | AFF3-4365 | chr2:99601369-99601396 |
| AFF3-1373 | chr2:100064277-100064304 | AFF3-4366 | chr2:99601374-99601401 |
| AFF3-1374 | chr2:100064278-100064305 | AFF3-4367 | chr2:99601375-99601402 |
| AFF3-1375 | chr2:100064279-100064306 | AFF3-4368 | chr2:99601381-99601408 |
| AFF3-1376 | chr2:100064282-100064309 | AFF3-4369 | chr2:99601382-99601409 |
| AFF3-1377 | chr2:100064290-100064317 | AFF3-4370 | chr2:99601386-99601413 |
| AFF3-1378 | chr2:100064298-100064325 | AFF3-4371 | chr2:99601397-99601424 |
| AFF3-1379 | chr2:100064299-100064326 | AFF3-4372 | chr2:99601412-99601439 |
| AFF3-1380 | chr2:100064300-100064327 | AFF3-4373 | chr2:99601413-99601440 |
| AFF3-1381 | chr2:100064304-100064331 | AFF3-4374 | chr2:99601414-99601441 |
| AFF3-1382 | chr2:100064309-100064336 | AFF3-4375 | chr2:99601418-99601445 |
| AFF3-1383 | chr2:100064310-100064337 | AFF3-4376 | chr2:99601420-99601447 |
| AFF3-1384 | chr2:100064313-100064340 | AFF3-4377 | chr2:99601426-99601453 |
| AFF3-1385 | chr2:100064314-100064341 | AFF3-4378 | chr2:99601427-99601454 |
| AFF3-1386 | chr2:100064315-100064342 | AFF3-4379 | chr2:99601428-99601455 |
| AFF3-1387 | chr2:100064361-100064388 | AFF3-4380 | chr2:99601429-99601456 |
| AFF3-1388 | chr2:100064365-100064392 | AFF3-4381 | chr2:99601430-99601457 |
| AFF3-1389 | chr2:100064369-100064396 | AFF3-4382 | chr2:99601431-99601458 |
| AFF3-1390 | chr2:100064372-100064399 | AFF3-4383 | chr2:99601434-99601461 |
| AFF3-1391 | chr2:100064375-100064402 | AFF3-4384 | chr2:99601454-99601481 |
| AFF3-1392 | chr2:100064376-100064403 | AFF3-4385 | chr2:99601466-99601493 |
| AFF3-1393 | chr2:100064390-100064417 | AFF3-4386 | chr2:99601467-99601494 |
| AFF3-1394 | chr2:100064391-100064418 | AFF3-4387 | chr2:99601484-99601511 |
| AFF3-1395 | chr2:100064396-100064423 | AFF3-4388 | chr2:99601497-99601524 |
| AFF3-1396 | chr2:100064403-100064430 | AFF3-4389 | chr2:99601505-99601532 |
| AFF3-1397 | chr2:100064491-100064518 | AFF3-4390 | chr2:99601508-99601535 |
| AFF3-1398 | chr2:100064526-100064553 | AFF3-4391 | chr2:99601547-99601574 |
| AFF3-1399 | chr2:100064544-100064571 | AFF3-4392 | chr2:99601553-99601580 |
| AFF3-1400 | chr2:100064578-100064605 | AFF3-4393 | chr2:99601557-99601584 |
| AFF3-1401 | chr2:100064582-100064609 | AFF3-4394 | chr2:99601565-99601592 |
| AFF3-1402 | chr2:100064608-100064635 | AFF3-4395 | chr2:99601571-99601598 |
| AFF3-1403 | chr2:100064615-100064642 | AFF3-4396 | chr2:99601572-99601599 |
| AFF3-1404 | chr2:100064642-100064669 | AFF3-4397 | chr2:99601577-99601604 |
| AFF3-1405 | chr2:100064647-100064674 | AFF3-4398 | chr2:99601578-99601605 |
| AFF3-1406 | chr2:100064648-100064675 | AFF3-4399 | chr2:99601585-99601612 |
| AFF3-1407 | chr2:100064658-100064685 | AFF3-4400 | chr2:99601587-99601614 |
| AFF3-1408 | chr2:100064669-100064696 | AFF3-4401 | chr2:99601592-99601619 |
| AFF3-1409 | chr2:100064670-100064697 | AFF3-4402 | chr2:99601610-99601637 |
| AFF3-1410 | chr2:100064679-100064706 | AFF3-4403 | chr2:99601614-99601641 |
| AFF3-1411 | chr2:100064683-100064710 | AFF3-4404 | chr2:99601617-99601644 |
| AFF3-1412 | chr2:100064707-100064734 | AFF3-4405 | chr2:99601618-99601645 |
| AFF3-1413 | chr2:100064754-100064781 | AFF3-4406 | chr2:99601625-99601652 |
| AFF3-1414 | chr2:100064764-100064791 | AFF3-4407 | chr2:99601627-99601654 |
| AFF3-1415 | chr2:100064767-100064794 | AFF3-4408 | chr2:99601628-99601655 |
| AFF3-1416 | chr2:100064771-100064798 | AFF3-4409 | chr2:99601629-99601656 |
| AFF3-1417 | chr2:100064772-100064799 | AFF3-4410 | chr2:99601630-99601657 |
| AFF3-1418 | chr2:100064800-100064827 | AFF3-4411 | chr2:99601632-99601659 |
| AFF3-1419 | chr2:100064874-100064901 | AFF3-4412 | chr2:99601638-99601665 |
| AFF3-1420 | chr2:100064875-100064902 | AFF3-4413 | chr2:99601639-99601666 |
| AFF3-1421 | chr2:100064876-100064903 | AFF3-4414 | chr2:99601640-99601667 |
| AFF3-1422 | chr2:100064879-100064906 | AFF3-4415 | chr2:99601641-99601668 |
| AFF3-1423 | chr2:100064892-100064919 | AFF3-4416 | chr2:99601653-99601680 |
| AFF3-1424 | chr2:100064901-100064928 | AFF3-4417 | chr2:99601664-99601691 |
| AFF3-1425 | chr2:100064902-100064929 | AFF3-4418 | chr2:99601687-99601714 |
| AFF3-1426 | chr2:100064906-100064933 | AFF3-4419 | chr2:99601688-99601715 |
| AFF3-1427 | chr2:100064922-100064949 | AFF3-4420 | chr2:99601689-99601716 |
| AFF3-1428 | chr2:100064923-100064950 | AFF3-4421 | chr2:99601690-99601717 |
| AFF3-1429 | chr2:100064926-100064953 | AFF3-4422 | chr2:99601692-99601719 |
| AFF3-1430 | chr2:100064939-100064966 | AFF3-4423 | chr2:99601695-99601722 |
| AFF3-1431 | chr2:100064940-100064967 | AFF3-4424 | chr2:99601705-99601732 |
| AFF3-1432 | chr2:100064965-100064992 | AFF3-4425 | chr2:99601706-99601733 |
| AFF3-1433 | chr2:100064996-100065023 | AFF3-4426 | chr2:99649507-99649534 |
| AFF3-1434 | chr2:100065032-100065059 | AFF3-4427 | chr2:99649508-99649535 |
| AFF3-1435 | chr2:100065042-100065069 | AFF3-4428 | chr2:99649513-99649540 |
| AFF3-1436 | chr2:100065043-100065070 | AFF3-4429 | chr2:99649514-99649541 |
| AFF3-1437 | chr2:100065073-100065100 | AFF3-4430 | chr2:99649543-99649570 |
| AFF3-1438 | chr2:100065084-100065111 | AFF3-4431 | chr2:99649547-99649574 |
| AFF3-1439 | chr2:100065099-100065126 | AFF3-4432 | chr2:99649548-99649575 |
| AFF3-1440 | chr2:100065130-100065157 | AFF3-4433 | chr2:99649561-99649588 |
| AFF3-1441 | chr2:100065149-100065176 | AFF3-4434 | chr2:99649616-99649643 |
| AFF3-1442 | chr2:100065166-100065193 | AFF3-4435 | chr2:99649625-99649652 |
| AFF3-1443 | chr2:100065167-100065194 | AFF3-4436 | chr2:99649643-99649670 |
| AFF3-1444 | chr2:100065182-100065209 | AFF3-4437 | chr2:99649648-99649675 |
| AFF3-1445 | chr2:100065241-100065268 | AFF3-4438 | chr2:99649655-99649682 |
| AFF3-1446 | chr2:100065282-100065309 | AFF3-4439 | chr2:99649656-99649683 |
| AFF3-1447 | chr2:100065295-100065322 | AFF3-4440 | chr2:99649663-99649690 |
| AFF3-1448 | chr2:100065303-100065330 | AFF3-4441 | chr2:99649748-99649775 |
| AFF3-1449 | chr2:100065304-100065331 | AFF3-4442 | chr2:99649749-99649776 |
| AFF3-1450 | chr2:100065450-100065477 | AFF3-4443 | chr2:99649750-99649777 |
| AFF3-1451 | chr2:100065451-100065478 | AFF3-4444 | chr2:99649751-99649778 |
| AFF3-1452 | chr2:100065480-100065507 | AFF3-4445 | chr2:99672283-99672310 |
| AFF3-1453 | chr2:100065514-100065541 | AFF3-4446 | chr2:99672290-99672317 |
| AFF3-1454 | chr2:100065515-100065542 | AFF3-4447 | chr2:99672297-99672324 |
| AFF3-1455 | chr2:100065592-100065619 | AFF3-4448 | chr2:99672307-99672334 |
| AFF3-1456 | chr2:100065602-100065629 | AFF3-4449 | chr2:99672308-99672335 |
| AFF3-1457 | chr2:100065605-100065632 | AFF3-4450 | chr2:99672323-99672350 |
| AFF3-1458 | chr2:100065624-100065651 | AFF3-4451 | chr2:99672330-99672357 |
| AFF3-1459 | chr2:100065625-100065652 | AFF3-4452 | chr2:99672331-99672358 |
| AFF3-1460 | chr2:100065632-100065659 | AFF3-4453 | chr2:99672340-99672367 |
| AFF3-1461 | chr2:100065633-100065660 | AFF3-4454 | chr2:99672341-99672368 |
| AFF3-1462 | chr2:100065664-100065691 | AFF3-4455 | chr2:99672390-99672417 |
| AFF3-1463 | chr2:100065669-100065696 | AFF3-4456 | chr2:99672391-99672418 |
| AFF3-1464 | chr2:100065670-100065697 | AFF3-4457 | chr2:99672412-99672439 |
| AFF3-1465 | chr2:100065674-100065701 | AFF3-4458 | chr2:99672428-99672455 |
| AFF3-1466 | chr2:100065700-100065727 | AFF3-4459 | chr2:99672432-99672459 |
| AFF3-1467 | chr2:100065703-100065730 | AFF3-4460 | chr2:99672435-99672462 |
| AFF3-1468 | chr2:100065723-100065750 | AFF3-4461 | chr2:99672443-99672470 |
| AFF3-1469 | chr2:100065808-100065835 | AFF3-4462 | chr2:99672444-99672471 |
| AFF3-1470 | chr2:100065881-100065908 | AFF3-4463 | chr2:99672447-99672474 |
| AFF3-1471 | chr2:100065927-100065954 | AFF3-4464 | chr2:99672457-99672484 |
| AFF3-1472 | chr2:100065936-100065963 | AFF3-4465 | chr2:99672466-99672493 |
| AFF3-1473 | chr2:100065994-100066021 | AFF3-4466 | chr2:99672467-99672494 |
| AFF3-1474 | chr2:100066008-100066035 | AFF3-4467 | chr2:99672477-99672504 |
| AFF3-1475 | chr2:100066043-100066070 | AFF3-4468 | chr2:99672480-99672507 |
| AFF3-1476 | chr2:100066044-100066071 | AFF3-4469 | chr2:99672485-99672512 |
| AFF3-1477 | chr2:100066066-100066093 | AFF3-4470 | chr2:99672494-99672521 |
| AFF3-1478 | chr2:100066105-100066132 | AFF3-4471 | chr2:99672503-99672530 |
| AFF3-1479 | chr2:100066106-100066133 | AFF3-4472 | chr2:99672506-99672533 |
| AFF3-1480 | chr2:100066107-100066134 | AFF3-4473 | chr2:99672537-99672564 |
| AFF3-1481 | chr2:100066108-100066135 | AFF3-4474 | chr2:99672547-99672574 |
| AFF3-1482 | chr2:100066109-100066136 | AFF3-4475 | chr2:99672549-99672576 |
| AFF3-1483 | chr2:100066112-100066139 | AFF3-4476 | chr2:99672573-99672600 |
| AFF3-1484 | chr2:100066113-100066140 | AFF3-4477 | chr2:99672582-99672609 |
| AFF3-1485 | chr2:100066114-100066141 | AFF3-4478 | chr2:99672583-99672610 |
| AFF3-1486 | chr2:100066115-100066142 | AFF3-4479 | chr2:99672593-99672620 |
| AFF3-1487 | chr2:100066119-100066146 | AFF3-4480 | chr2:99672609-99672636 |
| AFF3-1488 | chr2:100066122-100066149 | AFF3-4481 | chr2:99672655-99672682 |
| AFF3-1489 | chr2:100066123-100066150 | AFF3-4482 | chr2:99672666-99672693 |
| AFF3-1490 | chr2:100066124-100066151 | AFF3-4483 | chr2:99672669-99672696 |
| AFF3-1491 | chr2:100066200-100066227 | AFF3-4484 | chr2:99672716-99672743 |
| AFF3-1492 | chr2:100066225-100066252 | AFF3-4485 | chr2:99672728-99672755 |
| AFF3-1493 | chr2:100066271-100066298 | AFF3-4486 | chr2:99672732-99672759 |
| AFF3-1494 | chr2:100066284-100066311 | AFF3-4487 | chr2:99672738-99672765 |
| AFF3-1495 | chr2:100066301-100066328 | AFF3-4488 | chr2:99672749-99672776 |
| AFF3-1496 | chr2:100066304-100066331 | AFF3-4489 | chr2:99672779-99672806 |
| AFF3-1497 | chr2:100066308-100066335 | AFF3-4490 | chr2:99672780-99672807 |
| AFF3-1498 | chr2:100066309-100066336 | AFF3-4491 | chr2:99672781-99672808 |
| AFF3-1499 | chr2:100066310-100066337 | AFF3-4492 | chr2:99672797-99672824 |
| AFF3-1500 | chr2:100066313-100066340 | AFF3-4493 | chr2:99672798-99672825 |
| AFF3-1501 | chr2:100066338-100066365 | AFF3-4494 | chr2:99672827-99672854 |
| AFF3-1502 | chr2:100066372-100066399 | AFF3-4495 | chr2:99672828-99672855 |
| AFF3-1503 | chr2:100066373-100066400 | AFF3-4496 | chr2:99672877-99672904 |
| AFF3-1504 | chr2:100066377-100066404 | AFF3-4497 | chr2:99672878-99672905 |
| AFF3-1505 | chr2:100066390-100066417 | AFF3-4498 | chr2:99672887-99672914 |
| AFF3-1506 | chr2:100066421-100066448 | AFF3-4499 | chr2:99672902-99672929 |
| AFF3-1507 | chr2:100066426-100066453 | AFF3-4500 | chr2:99672921-99672948 |
| AFF3-1508 | chr2:100066427-100066454 | AFF3-4501 | chr2:99672922-99672949 |
| AFF3-1509 | chr2:100066438-100066465 | AFF3-4502 | chr2:99672924-99672951 |
| AFF3-1510 | chr2:100066439-100066466 | AFF3-4503 | chr2:99672962-99672989 |
| AFF3-1511 | chr2:100066443-100066470 | AFF3-4504 | chr2:99672970-99672997 |
| AFF3-1512 | chr2:100066458-100066485 | AFF3-4505 | chr2:99672971-99672998 |
| AFF3-1513 | chr2:100066466-100066493 | AFF3-4506 | chr2:99672997-99673024 |
| AFF3-1514 | chr2:100066485-100066512 | AFF3-4507 | chr2:99672998-99673025 |
| AFF3-1515 | chr2:100066512-100066539 | AFF3-4508 | chr2:99673021-99673048 |
| AFF3-1516 | chr2:100066518-100066545 | AFF3-4509 | chr2:99673051-99673078 |
| AFF3-1517 | chr2:100066590-100066617 | AFF3-4510 | chr2:99673052-99673079 |
| AFF3-1518 | chr2:100066596-100066623 | AFF3-4511 | chr2:99673053-99673080 |
| AFF3-1519 | chr2:100066610-100066637 | AFF3-4512 | chr2:99673065-99673092 |
| AFF3-1520 | chr2:100066628-100066655 | AFF3-4513 | chr2:99673066-99673093 |
| AFF3-1521 | chr2:100066629-100066656 | AFF3-4514 | chr2:99673086-99673113 |
| AFF3-1522 | chr2:100066639-100066666 | AFF3-4515 | chr2:99673087-99673114 |
| AFF3-1523 | chr2:100066640-100066667 | AFF3-4516 | chr2:99673088-99673115 |
| AFF3-1524 | chr2:100066681-100066708 | AFF3-4517 | chr2:99673105-99673132 |
| AFF3-1525 | chr2:100066738-100066765 | AFF3-4518 | chr2:99673119-99673146 |
| AFF3-1526 | chr2:100066767-100066794 | AFF3-4519 | chr2:99673120-99673147 |
| AFF3-1527 | chr2:100066774-100066801 | AFF3-4520 | chr2:99673129-99673156 |
| AFF3-1528 | chr2:100066798-100066825 | AFF3-4521 | chr2:99673136-99673163 |
| AFF3-1529 | chr2:100066799-100066826 | AFF3-4522 | chr2:99673137-99673164 |
| AFF3-1530 | chr2:100066818-100066845 | AFF3-4523 | chr2:99673144-99673171 |
| AFF3-1531 | chr2:100066827-100066854 | AFF3-4524 | chr2:99673145-99673172 |
| AFF3-1532 | chr2:100066863-100066890 | AFF3-4525 | chr2:99673146-99673173 |
| AFF3-1533 | chr2:100066870-100066897 | AFF3-4526 | chr2:99673155-99673182 |
| AFF3-1534 | chr2:100066874-100066901 | AFF3-4527 | chr2:99673156-99673183 |
| AFF3-1535 | chr2:100066878-100066905 | AFF3-4528 | chr2:99673161-99673188 |
| AFF3-1536 | chr2:100066879-100066906 | AFF3-4529 | chr2:99673165-99673192 |
| AFF3-1537 | chr2:100066880-100066907 | AFF3-4530 | chr2:99673166-99673193 |
| AFF3-1538 | chr2:100066883-100066910 | AFF3-4531 | chr2:99673167-99673194 |
| AFF3-1539 | chr2:100066918-100066945 | AFF3-4532 | chr2:99673205-99673232 |
| AFF3-1540 | chr2:100066921-100066948 | AFF3-4533 | chr2:99673213-99673240 |
| AFF3-1541 | chr2:100066922-100066949 | AFF3-4534 | chr2:99673219-99673246 |
| AFF3-1542 | chr2:100066923-100066950 | AFF3-4535 | chr2:99673242-99673269 |
| AFF3-1543 | chr2:100066935-100066962 | AFF3-4536 | chr2:99673247-99673274 |
| AFF3-1544 | chr2:100066967-100066994 | AFF3-4537 | chr2:99673251-99673278 |
| AFF3-1545 | chr2:100067001-100067028 | AFF3-4538 | chr2:99673275-99673302 |
| AFF3-1546 | chr2:100067014-100067041 | AFF3-4539 | chr2:99673276-99673303 |
| AFF3-1547 | chr2:100067015-100067042 | AFF3-4540 | chr2:99673277-99673304 |
| AFF3-1548 | chr2:100067029-100067056 | AFF3-4541 | chr2:99673282-99673309 |
| AFF3-1549 | chr2:100067037-100067064 | AFF3-4542 | chr2:99673283-99673310 |
| AFF3-1550 | chr2:100067048-100067075 | AFF3-4543 | chr2:99673284-99673311 |
| AFF3-1551 | chr2:100067049-100067076 | AFF3-4544 | chr2:99673290-99673317 |
| AFF3-1552 | chr2:100067065-100067092 | AFF3-4545 | chr2:99673291-99673318 |
| AFF3-1553 | chr2:100067112-100067139 | AFF3-4546 | chr2:99673293-99673320 |
| AFF3-1554 | chr2:100067120-100067147 | AFF3-4547 | chr2:99673294-99673321 |
| AFF3-1555 | chr2:100067121-100067148 | AFF3-4548 | chr2:99673295-99673322 |
| AFF3-1556 | chr2:100067133-100067160 | AFF3-4549 | chr2:99673309-99673336 |
| AFF3-1557 | chr2:100067134-100067161 | AFF3-4550 | chr2:99673318-99673345 |
| AFF3-1558 | chr2:100067135-100067162 | AFF3-4551 | chr2:99673336-99673363 |
| AFF3-1559 | chr2:100067136-100067163 | AFF3-4552 | chr2:99673339-99673366 |
| AFF3-1560 | chr2:100067141-100067168 | AFF3-4553 | chr2:99673351-99673378 |
| AFF3-1561 | chr2:100067171-100067198 | AFF3-4554 | chr2:99673352-99673379 |
| AFF3-1562 | chr2:100067172-100067199 | AFF3-4555 | chr2:99673353-99673380 |
| AFF3-1563 | chr2:100067175-100067202 | AFF3-4556 | chr2:99673357-99673384 |
| AFF3-1564 | chr2:100067176-100067203 | AFF3-4557 | chr2:99673358-99673385 |
| AFF3-1565 | chr2:100067177-100067204 | AFF3-4558 | chr2:99673359-99673386 |
| AFF3-1566 | chr2:100067178-100067205 | AFF3-4559 | chr2:99673361-99673388 |
| AFF3-1567 | chr2:100067179-100067206 | AFF3-4560 | chr2:99673362-99673389 |
| AFF3-1568 | chr2:100067182-100067209 | AFF3-4561 | chr2:99673368-99673395 |
| AFF3-1569 | chr2:100067183-100067210 | AFF3-4562 | chr2:99673369-99673396 |
| AFF3-1570 | chr2:100067231-100067258 | AFF3-4563 | chr2:99673370-99673397 |
| AFF3-1571 | chr2:100067237-100067264 | AFF3-4564 | chr2:99673371-99673398 |
| AFF3-1572 | chr2:100067243-100067270 | AFF3-4565 | chr2:99673372-99673399 |
| AFF3-1573 | chr2:100067244-100067271 | AFF3-4566 | chr2:99673380-99673407 |
| AFF3-1574 | chr2:100067245-100067272 | AFF3-4567 | chr2:99673455-99673482 |
| AFF3-1575 | chr2:100067253-100067280 | AFF3-4568 | chr2:99673561-99673588 |
| AFF3-1576 | chr2:100067254-100067281 | AFF3-4569 | chr2:99673562-99673589 |
| AFF3-1577 | chr2:100067274-100067301 | AFF3-4570 | chr2:99673568-99673595 |
| AFF3-1578 | chr2:100067278-100067305 | AFF3-4571 | chr2:99673569-99673596 |
| AFF3-1579 | chr2:100067279-100067306 | AFF3-4572 | chr2:99673570-99673597 |
| AFF3-1580 | chr2:100067284-100067311 | AFF3-4573 | chr2:99673587-99673614 |
| AFF3-1581 | chr2:100067295-100067322 | AFF3-4574 | chr2:99673588-99673615 |
| AFF3-1582 | chr2:100067297-100067324 | AFF3-4575 | chr2:99673595-99673622 |
| AFF3-1583 | chr2:100067298-100067325 | AFF3-4576 | chr2:99673596-99673623 |
| AFF3-1584 | chr2:100067332-100067359 | AFF3-4577 | chr2:99673597-99673624 |
| AFF3-1585 | chr2:100067337-100067364 | AFF3-4578 | chr2:99673602-99673629 |
| AFF3-1586 | chr2:100067346-100067373 | AFF3-4579 | chr2:99673603-99673630 |
| AFF3-1587 | chr2:100067350-100067377 | AFF3-4580 | chr2:99673617-99673644 |
| AFF3-1588 | chr2:100067366-100067393 | AFF3-4581 | chr2:99673618-99673645 |
| AFF3-1589 | chr2:100067409-100067436 | AFF3-4582 | chr2:99673627-99673654 |
| AFF3-1590 | chr2:100067410-100067437 | AFF3-4583 | chr2:99673642-99673669 |
| AFF3-1591 | chr2:100067411-100067438 | AFF3-4584 | chr2:99673645-99673672 |
| AFF3-1592 | chr2:100067446-100067473 | AFF3-4585 | chr2:99673646-99673673 |
| AFF3-1593 | chr2:100067451-100067478 | AFF3-4586 | chr2:99673654-99673681 |
| AFF3-1594 | chr2:100067465-100067492 | AFF3-4587 | chr2:99673655-99673682 |
| AFF3-1595 | chr2:100067469-100067496 | AFF3-4588 | chr2:99673667-99673694 |
| AFF3-1596 | chr2:100067470-100067497 | AFF3-4589 | chr2:99673670-99673697 |
| AFF3-1597 | chr2:100067511-100067538 | AFF3-4590 | chr2:99673681-99673708 |
| AFF3-1598 | chr2:100067514-100067541 | AFF3-4591 | chr2:99673688-99673715 |
| AFF3-1599 | chr2:100067515-100067542 | AFF3-4592 | chr2:99673699-99673726 |
| AFF3-1600 | chr2:100067576-100067603 | AFF3-4593 | chr2:99673700-99673727 |
| AFF3-1601 | chr2:100067583-100067610 | AFF3-4594 | chr2:99673701-99673728 |
| AFF3-1602 | chr2:100067591-100067618 | AFF3-4595 | chr2:99673706-99673733 |
| AFF3-1603 | chr2:100067594-100067621 | AFF3-4596 | chr2:99673712-99673739 |
| AFF3-1604 | chr2:100067662-100067689 | AFF3-4597 | chr2:99673713-99673740 |
| AFF3-1605 | chr2:100067693-100067720 | AFF3-4598 | chr2:99673720-99673747 |
| AFF3-1606 | chr2:100067694-100067721 | AFF3-4599 | chr2:99673730-99673757 |
| AFF3-1607 | chr2:100067695-100067722 | AFF3-4600 | chr2:99673741-99673768 |
| AFF3-1608 | chr2:100067699-100067726 | AFF3-4601 | chr2:99673748-99673775 |
| AFF3-1609 | chr2:100067700-100067727 | AFF3-4602 | chr2:99673758-99673785 |
| AFF3-1610 | chr2:100067705-100067732 | AFF3-4603 | chr2:99673769-99673796 |
| AFF3-1611 | chr2:100067706-100067733 | AFF3-4604 | chr2:99673770-99673797 |
| AFF3-1612 | chr2:100067709-100067736 | AFF3-4605 | chr2:99673771-99673798 |
| AFF3-1613 | chr2:100067710-100067737 | AFF3-4606 | chr2:99673779-99673806 |
| AFF3-1614 | chr2:100067715-100067742 | AFF3-4607 | chr2:99673780-99673807 |
| AFF3-1615 | chr2:100067744-100067771 | AFF3-4608 | chr2:99673781-99673808 |
| AFF3-1616 | chr2:100067745-100067772 | AFF3-4609 | chr2:99673782-99673809 |
| AFF3-1617 | chr2:100067768-100067795 | AFF3-4610 | chr2:99673783-99673810 |
| AFF3-1618 | chr2:100067778-100067805 | AFF3-4611 | chr2:99673794-99673821 |
| AFF3-1619 | chr2:100067786-100067813 | AFF3-4612 | chr2:99673798-99673825 |
| AFF3-1620 | chr2:100067803-100067830 | AFF3-4613 | chr2:99673804-99673831 |
| AFF3-1621 | chr2:100067804-100067831 | AFF3-4614 | chr2:99673809-99673836 |
| AFF3-1622 | chr2:100067834-100067861 | AFF3-4615 | chr2:99673815-99673842 |
| AFF3-1623 | chr2:100067835-100067862 | AFF3-4616 | chr2:99673837-99673864 |
| AFF3-1624 | chr2:100067862-100067889 | AFF3-4617 | chr2:99673838-99673865 |
| AFF3-1625 | chr2:100067863-100067890 | AFF3-4618 | chr2:99673842-99673869 |
| AFF3-1626 | chr2:100067898-100067925 | AFF3-4619 | chr2:99673843-99673870 |
| AFF3-1627 | chr2:100067917-100067944 | AFF3-4620 | chr2:99673844-99673871 |
| AFF3-1628 | chr2:100067936-100067963 | AFF3-4621 | chr2:99673848-99673875 |
| AFF3-1629 | chr2:100067957-100067984 | AFF3-4622 | chr2:99673849-99673876 |
| AFF3-1630 | chr2:100067964-100067991 | AFF3-4623 | chr2:99673850-99673877 |
| AFF3-1631 | chr2:100067965-100067992 | AFF3-4624 | chr2:99673853-99673880 |
| AFF3-1632 | chr2:100067976-100068003 | AFF3-4625 | chr2:99673888-99673915 |
| AFF3-1633 | chr2:100068007-100068034 | AFF3-4626 | chr2:99673889-99673916 |
| AFF3-1634 | chr2:100068010-100068037 | AFF3-4627 | chr2:99673894-99673921 |
| AFF3-1635 | chr2:100068021-100068048 | AFF3-4628 | chr2:99673895-99673922 |
| AFF3-1636 | chr2:100068044-100068071 | AFF3-4629 | chr2:99673896-99673923 |
| AFF3-1637 | chr2:100068048-100068075 | AFF3-4630 | chr2:99673902-99673929 |
| AFF3-1638 | chr2:100068068-100068095 | AFF3-4631 | chr2:99673903-99673930 |
| AFF3-1639 | chr2:100068076-100068103 | AFF3-4632 | chr2:99673904-99673931 |
| AFF3-1640 | chr2:100068089-100068116 | AFF3-4633 | chr2:99673905-99673932 |
| AFF3-1641 | chr2:100068091-100068118 | AFF3-4634 | chr2:99673916-99673943 |
| AFF3-1642 | chr2:100068096-100068123 | AFF3-4635 | chr2:99673937-99673964 |
| AFF3-1643 | chr2:100068115-100068142 | AFF3-4636 | chr2:99673950-99673977 |
| AFF3-1644 | chr2:100068129-100068156 | AFF3-4637 | chr2:99682345-99682372 |
| AFF3-1645 | chr2:100068148-100068175 | AFF3-4638 | chr2:99682355-99682382 |
| AFF3-1646 | chr2:100068175-100068202 | AFF3-4639 | chr2:99682356-99682383 |
| AFF3-1647 | chr2:100068197-100068224 | AFF3-4640 | chr2:99682357-99682384 |
| AFF3-1648 | chr2:100068203-100068230 | AFF3-4641 | chr2:99682358-99682385 |
| AFF3-1649 | chr2:100068226-100068253 | AFF3-4642 | chr2:99682359-99682386 |
| AFF3-1650 | chr2:100068227-100068254 | AFF3-4643 | chr2:99682363-99682390 |
| AFF3-1651 | chr2:100068253-100068280 | AFF3-4644 | chr2:99682364-99682391 |
| AFF3-1652 | chr2:100068254-100068281 | AFF3-4645 | chr2:99682366-99682393 |
| AFF3-1653 | chr2:100068264-100068291 | AFF3-4646 | chr2:99682368-99682395 |
| AFF3-1654 | chr2:100068279-100068306 | AFF3-4647 | chr2:99682369-99682396 |
| AFF3-1655 | chr2:100068290-100068317 | AFF3-4648 | chr2:99682370-99682397 |
| AFF3-1656 | chr2:100068291-100068318 | AFF3-4649 | chr2:99682391-99682418 |
| AFF3-1657 | chr2:100068300-100068327 | AFF3-4650 | chr2:99682397-99682424 |
| AFF3-1658 | chr2:100068301-100068328 | AFF3-4651 | chr2:99682405-99682432 |
| AFF3-1659 | chr2:100068304-100068331 | AFF3-4652 | chr2:99682413-99682440 |
| AFF3-1660 | chr2:100068323-100068350 | AFF3-4653 | chr2:99682419-99682446 |
| AFF3-1661 | chr2:100068326-100068353 | AFF3-4654 | chr2:99682431-99682458 |
| AFF3-1662 | chr2:100068327-100068354 | AFF3-4655 | chr2:99682432-99682459 |
| AFF3-1663 | chr2:100068347-100068374 | AFF3-4656 | chr2:99682452-99682479 |
| AFF3-1664 | chr2:100068357-100068384 | AFF3-4657 | chr2:99682474-99682501 |
| AFF3-1665 | chr2:100068363-100068390 | AFF3-4658 | chr2:99682483-99682510 |
| AFF3-1666 | chr2:100068369-100068396 | AFF3-4659 | chr2:99682484-99682511 |
| AFF3-1667 | chr2:100068394-100068421 | AFF3-4660 | chr2:99682485-99682512 |
| AFF3-1668 | chr2:100068400-100068427 | AFF3-4661 | chr2:99682494-99682521 |
| AFF3-1669 | chr2:100068403-100068430 | AFF3-4662 | chr2:99682518-99682545 |
| AFF3-1670 | chr2:100068404-100068431 | AFF3-4663 | chr2:99682519-99682546 |
| AFF3-1671 | chr2:100068421-100068448 | AFF3-4664 | chr2:99682520-99682547 |
| AFF3-1672 | chr2:100068422-100068449 | AFF3-4665 | chr2:99682523-99682550 |
| AFF3-1673 | chr2:100068428-100068455 | AFF3-4666 | chr2:99682524-99682551 |
| AFF3-1674 | chr2:100068429-100068456 | AFF3-4667 | chr2:99682540-99682567 |
| AFF3-1675 | chr2:100068443-100068470 | AFF3-4668 | chr2:99682566-99682593 |
| AFF3-1676 | chr2:100068470-100068497 | AFF3-4669 | chr2:99682572-99682599 |
| AFF3-1677 | chr2:100068473-100068500 | AFF3-4670 | chr2:99682577-99682604 |
| AFF3-1678 | chr2:100068484-100068511 | AFF3-4671 | chr2:99682588-99682615 |
| AFF3-1679 | chr2:100068487-100068514 | AFF3-4672 | chr2:99682589-99682616 |
| AFF3-1680 | chr2:100068490-100068517 | AFF3-4673 | chr2:99682600-99682627 |
| AFF3-1681 | chr2:100068502-100068529 | AFF3-4674 | chr2:99682601-99682628 |
| AFF3-1682 | chr2:100068516-100068543 | AFF3-4675 | chr2:99682602-99682629 |
| AFF3-1683 | chr2:100068535-100068562 | AFF3-4676 | chr2:99682612-99682639 |
| AFF3-1684 | chr2:100068536-100068563 | AFF3-4677 | chr2:99682613-99682640 |
| AFF3-1685 | chr2:100068537-100068564 | AFF3-4678 | chr2:99682628-99682655 |
| AFF3-1686 | chr2:100068551-100068578 | AFF3-4679 | chr2:99682636-99682663 |
| AFF3-1687 | chr2:100068564-100068591 | AFF3-4680 | chr2:99682672-99682699 |
| AFF3-1688 | chr2:100068565-100068592 | AFF3-4681 | chr2:99682673-99682700 |
| AFF3-1689 | chr2:100068581-100068608 | AFF3-4682 | chr2:99682674-99682701 |
| AFF3-1690 | chr2:100068585-100068612 | AFF3-4683 | chr2:99682675-99682702 |
| AFF3-1691 | chr2:100068589-100068616 | AFF3-4684 | chr2:99682676-99682703 |
| AFF3-1692 | chr2:100068609-100068636 | AFF3-4685 | chr2:99682680-99682707 |
| AFF3-1693 | chr2:100068632-100068659 | AFF3-4686 | chr2:99682683-99682710 |
| AFF3-1694 | chr2:100068653-100068680 | AFF3-4687 | chr2:99682687-99682714 |
| AFF3-1695 | chr2:100068664-100068691 | AFF3-4688 | chr2:99682688-99682715 |
| AFF3-1696 | chr2:100068665-100068692 | AFF3-4689 | chr2:99682689-99682716 |
| AFF3-1697 | chr2:100068670-100068697 | AFF3-4690 | chr2:99682711-99682738 |
| AFF3-1698 | chr2:100104275-100104302 | AFF3-4691 | chr2:99682721-99682748 |
| AFF3-1699 | chr2:100104276-100104303 | AFF3-4692 | chr2:99682722-99682749 |
| AFF3-1700 | chr2:100104277-100104304 | AFF3-4693 | chr2:99682727-99682754 |
| AFF3-1701 | chr2:100104278-100104305 | AFF3-4694 | chr2:99682728-99682755 |
| AFF3-1702 | chr2:100104281-100104308 | AFF3-4695 | chr2:99682729-99682756 |
| AFF3-1703 | chr2:100104282-100104309 | AFF3-4696 | chr2:99682736-99682763 |
| AFF3-1704 | chr2:100104285-100104312 | AFF3-4697 | chr2:99682737-99682764 |
| AFF3-1705 | chr2:100104286-100104313 | AFF3-4698 | chr2:99682741-99682768 |
| AFF3-1706 | chr2:100104287-100104314 | AFF3-4699 | chr2:99682750-99682777 |
| AFF3-1707 | chr2:100104290-100104317 | AFF3-4700 | chr2:99682762-99682789 |
| AFF3-1708 | chr2:100104291-100104318 | AFF3-4701 | chr2:99682763-99682790 |
| AFF3-1709 | chr2:100104292-100104319 | AFF3-4702 | chr2:99682767-99682794 |
| AFF3-1710 | chr2:100104293-100104320 | AFF3-4703 | chr2:99682768-99682795 |
| AFF3-1711 | chr2:100104295-100104322 | AFF3-4704 | chr2:99682780-99682807 |
| AFF3-1712 | chr2:100104301-100104328 | AFF3-4705 | chr2:99682806-99682833 |
| AFF3-1713 | chr2:100104302-100104329 | AFF3-4706 | chr2:99682817-99682844 |
| AFF3-1714 | chr2:100104306-100104333 | AFF3-4707 | chr2:99682819-99682846 |
| AFF3-1715 | chr2:100104309-100104336 | AFF3-4708 | chr2:99682828-99682855 |
| AFF3-1716 | chr2:100104310-100104337 | AFF3-4709 | chr2:99682829-99682856 |
| AFF3-1717 | chr2:100104314-100104341 | AFF3-4710 | chr2:99682840-99682867 |
| AFF3-1718 | chr2:100104315-100104342 | AFF3-4711 | chr2:99682851-99682878 |
| AFF3-1719 | chr2:100104316-100104343 | AFF3-4712 | chr2:99682863-99682890 |
| AFF3-1720 | chr2:100104317-100104344 | AFF3-4713 | chr2:99682871-99682898 |
| AFF3-1721 | chr2:100104318-100104345 | AFF3-4714 | chr2:99682894-99682921 |
| AFF3-1722 | chr2:100104319-100104346 | AFF3-4715 | chr2:99682898-99682925 |
| AFF3-1723 | chr2:100104323-100104350 | AFF3-4716 | chr2:99682901-99682928 |
| AFF3-1724 | chr2:100104324-100104351 | AFF3-4717 | chr2:99682902-99682929 |
| AFF3-1725 | chr2:100104343-100104370 | AFF3-4718 | chr2:99682918-99682945 |
| AFF3-1726 | chr2:100104346-100104373 | AFF3-4719 | chr2:99682978-99683005 |
| AFF3-1727 | chr2:100104352-100104379 | AFF3-4720 | chr2:99682993-99683020 |
| AFF3-1728 | chr2:100104355-100104382 | AFF3-4721 | chr2:99683007-99683034 |
| AFF3-1729 | chr2:100104356-100104383 | AFF3-4722 | chr2:99683013-99683040 |
| AFF3-1730 | chr2:100104357-100104384 | AFF3-4723 | chr2:99683019-99683046 |
| AFF3-1731 | chr2:100104361-100104388 | AFF3-4724 | chr2:99683020-99683047 |
| AFF3-1732 | chr2:100104362-100104389 | AFF3-4725 | chr2:99683022-99683049 |
| AFF3-1733 | chr2:100104366-100104393 | AFF3-4726 | chr2:99683023-99683050 |
| AFF3-1734 | chr2:100104367-100104394 | AFF3-4727 | chr2:99683024-99683051 |
| AFF3-1735 | chr2:100104368-100104395 | AFF3-4728 | chr2:99683025-99683052 |
| AFF3-1736 | chr2:100104371-100104398 | AFF3-4729 | chr2:99683029-99683056 |
| AFF3-1737 | chr2:100104372-100104399 | AFF3-4730 | chr2:99683030-99683057 |
| AFF3-1738 | chr2:100104375-100104402 | AFF3-4731 | chr2:99683035-99683062 |
| AFF3-1739 | chr2:100104376-100104403 | AFF3-4732 | chr2:99683036-99683063 |
| AFF3-1740 | chr2:100104385-100104412 | AFF3-4733 | chr2:99683039-99683066 |
| AFF3-1741 | chr2:100104389-100104416 | AFF3-4734 | chr2:99683065-99683092 |
| AFF3-1742 | chr2:100104401-100104428 | AFF3-4735 | chr2:99683066-99683093 |
| AFF3-1743 | chr2:100104407-100104434 | AFF3-4736 | chr2:99683074-99683101 |
| AFF3-1744 | chr2:100104408-100104435 | AFF3-4737 | chr2:99683092-99683119 |
| AFF3-1745 | chr2:100104418-100104445 | AFF3-4738 | chr2:99683112-99683139 |
| AFF3-1746 | chr2:100104419-100104446 | AFF3-4739 | chr2:99683148-99683175 |
| AFF3-1747 | chr2:100104424-100104451 | AFF3-4740 | chr2:99683171-99683198 |
| AFF3-1748 | chr2:100104429-100104456 | AFF3-4741 | chr2:99683191-99683218 |
| AFF3-1749 | chr2:100104432-100104459 | AFF3-4742 | chr2:99683203-99683230 |
| AFF3-1750 | chr2:100104433-100104460 | AFF3-4743 | chr2:99683220-99683247 |
| AFF3-1751 | chr2:100104436-100104463 | AFF3-4744 | chr2:99683248-99683275 |
| AFF3-1752 | chr2:100104451-100104478 | AFF3-4745 | chr2:99683249-99683276 |
| AFF3-1753 | chr2:100104474-100104501 | AFF3-4746 | chr2:99683259-99683286 |
| AFF3-1754 | chr2:100104475-100104502 | AFF3-4747 | chr2:99683274-99683301 |
| AFF3-1755 | chr2:100104478-100104505 | AFF3-4748 | chr2:99683293-99683320 |
| AFF3-1756 | chr2:100104479-100104506 | AFF3-4749 | chr2:99683296-99683323 |
| AFF3-1757 | chr2:100104480-100104507 | AFF3-4750 | chr2:99683316-99683343 |
| AFF3-1758 | chr2:100104481-100104508 | AFF3-4751 | chr2:99683353-99683380 |
| AFF3-1759 | chr2:100104487-100104514 | AFF3-4752 | chr2:99683391-99683418 |
| AFF3-1760 | chr2:100104490-100104517 | AFF3-4753 | chr2:99683392-99683419 |
| AFF3-1761 | chr2:100104493-100104520 | AFF3-4754 | chr2:99683397-99683424 |
| AFF3-1762 | chr2:100104496-100104523 | AFF3-4755 | chr2:99683432-99683459 |
| AFF3-1763 | chr2:100104504-100104531 | AFF3-4756 | chr2:99683440-99683467 |
| AFF3-1764 | chr2:100104508-100104535 | AFF3-4757 | chr2:99683470-99683497 |
| AFF3-1765 | chr2:100104511-100104538 | AFF3-4758 | chr2:99683480-99683507 |
| AFF3-1766 | chr2:100104512-100104539 | AFF3-4759 | chr2:99683488-99683515 |
| AFF3-1767 | chr2:100104516-100104543 | AFF3-4760 | chr2:99683489-99683516 |
| AFF3-1768 | chr2:100104534-100104561 | AFF3-4761 | chr2:99683518-99683545 |
| AFF3-1769 | chr2:100104540-100104567 | AFF3-4762 | chr2:99683531-99683558 |
| AFF3-1770 | chr2:100104549-100104576 | AFF3-4763 | chr2:99683537-99683564 |
| AFF3-1771 | chr2:100104550-100104577 | AFF3-4764 | chr2:99683540-99683567 |
| AFF3-1772 | chr2:100104554-100104581 | AFF3-4765 | chr2:99683541-99683568 |
| AFF3-1773 | chr2:100104559-100104586 | AFF3-4766 | chr2:99683556-99683583 |
| AFF3-1774 | chr2:100104568-100104595 | AFF3-4767 | chr2:99683557-99683584 |
| AFF3-1775 | chr2:100104569-100104596 | AFF3-4768 | chr2:99683559-99683586 |
| AFF3-1776 | chr2:100104571-100104598 | AFF3-4769 | chr2:99683563-99683590 |
| AFF3-1777 | chr2:100104573-100104600 | AFF3-4770 | chr2:99683566-99683593 |
| AFF3-1778 | chr2:100104574-100104601 | AFF3-4771 | chr2:99683569-99683596 |
| AFF3-1779 | chr2:100104575-100104602 | AFF3-4772 | chr2:99683570-99683597 |
| AFF3-1780 | chr2:100104578-100104605 | AFF3-4773 | chr2:99683571-99683598 |
| AFF3-1781 | chr2:100104579-100104606 | AFF3-4774 | chr2:99683572-99683599 |
| AFF3-1782 | chr2:100104582-100104609 | AFF3-4775 | chr2:99683573-99683600 |
| AFF3-1783 | chr2:100104585-100104612 | AFF3-4776 | chr2:99683583-99683610 |
| AFF3-1784 | chr2:100104586-100104613 | AFF3-4777 | chr2:99683597-99683624 |
| AFF3-1785 | chr2:100104589-100104616 | AFF3-4778 | chr2:99683598-99683625 |
| AFF3-1786 | chr2:100104590-100104617 | AFF3-4779 | chr2:99683601-99683628 |
| AFF3-1787 | chr2:100104591-100104618 | AFF3-4780 | chr2:99683606-99683633 |
| AFF3-1788 | chr2:100104592-100104619 | AFF3-4781 | chr2:99683625-99683652 |
| AFF3-1789 | chr2:100104604-100104631 | AFF3-4782 | chr2:99683629-99683656 |
| AFF3-1790 | chr2:100104605-100104632 | AFF3-4783 | chr2:99683643-99683670 |
| AFF3-1791 | chr2:100104606-100104633 | AFF3-4784 | chr2:99683644-99683671 |
| AFF3-1792 | chr2:100104609-100104636 | AFF3-4785 | chr2:99683647-99683674 |
| AFF3-1793 | chr2:100104612-100104639 | AFF3-4786 | chr2:99683648-99683675 |
| AFF3-1794 | chr2:100104613-100104640 | AFF3-4787 | chr2:99683664-99683691 |
| AFF3-1795 | chr2:100104614-100104641 | AFF3-4788 | chr2:99683665-99683692 |
| AFF3-1796 | chr2:100104615-100104642 | AFF3-4789 | chr2:99683666-99683693 |
| AFF3-1797 | chr2:100104616-100104643 | AFF3-4790 | chr2:99683682-99683709 |
| AFF3-1798 | chr2:100104618-100104645 | AFF3-4791 | chr2:99683683-99683710 |
| AFF3-1799 | chr2:100104619-100104646 | AFF3-4792 | chr2:99683684-99683711 |
| AFF3-1800 | chr2:100104620-100104647 | AFF3-4793 | chr2:99683685-99683712 |
| AFF3-1801 | chr2:100104621-100104648 | AFF3-4794 | chr2:99683689-99683716 |
| AFF3-1802 | chr2:100104622-100104649 | AFF3-4795 | chr2:99683695-99683722 |
| AFF3-1803 | chr2:100104627-100104654 | AFF3-4796 | chr2:99683698-99683725 |
| AFF3-1804 | chr2:100104628-100104655 | AFF3-4797 | chr2:99683699-99683726 |
| AFF3-1805 | chr2:100104631-100104658 | AFF3-4798 | chr2:99683719-99683746 |
| AFF3-1806 | chr2:100104633-100104660 | AFF3-4799 | chr2:99683762-99683789 |
| AFF3-1807 | chr2:100104637-100104664 | AFF3-4800 | chr2:99683765-99683792 |
| AFF3-1808 | chr2:100104651-100104678 | AFF3-4801 | chr2:99683768-99683795 |
| AFF3-1809 | chr2:100104655-100104682 | AFF3-4802 | chr2:99683771-99683798 |
| AFF3-1810 | chr2:100104663-100104690 | AFF3-4803 | chr2:99683792-99683819 |
| AFF3-1811 | chr2:100104667-100104694 | AFF3-4804 | chr2:99683819-99683846 |
| AFF3-1812 | chr2:100104668-100104695 | AFF3-4805 | chr2:99683820-99683847 |
| AFF3-1813 | chr2:100104673-100104700 | AFF3-4806 | chr2:99683849-99683876 |
| AFF3-1814 | chr2:100104674-100104701 | AFF3-4807 | chr2:99683864-99683891 |
| AFF3-1815 | chr2:100104675-100104702 | AFF3-4808 | chr2:99683865-99683892 |
| AFF3-1816 | chr2:100104718-100104745 | AFF3-4809 | chr2:99683868-99683895 |
| AFF3-1817 | chr2:100104723-100104750 | AFF3-4810 | chr2:99683904-99683931 |
| AFF3-1818 | chr2:100104726-100104753 | AFF3-4811 | chr2:99683906-99683933 |
| AFF3-1819 | chr2:100104729-100104756 | AFF3-4812 | chr2:99683915-99683942 |
| AFF3-1820 | chr2:100104732-100104759 | AFF3-4813 | chr2:99683929-99683956 |
| AFF3-1821 | chr2:100104733-100104760 | AFF3-4814 | chr2:99683930-99683957 |
| AFF3-1822 | chr2:100104734-100104761 | AFF3-4815 | chr2:99683934-99683961 |
| AFF3-1823 | chr2:100104735-100104762 | AFF3-4816 | chr2:99683935-99683962 |
| AFF3-1824 | chr2:100104736-100104763 | AFF3-4817 | chr2:99683947-99683974 |
| AFF3-1825 | chr2:100104739-100104766 | AFF3-4818 | chr2:99683990-99684017 |
| AFF3-1826 | chr2:100104740-100104767 | AFF3-4819 | chr2:99684000-99684027 |
| AFF3-1827 | chr2:100104741-100104768 | AFF3-4820 | chr2:99684001-99684028 |
| AFF3-1828 | chr2:100104742-100104769 | AFF3-4821 | chr2:99684002-99684029 |
| AFF3-1829 | chr2:100104746-100104773 | AFF3-4822 | chr2:99684007-99684034 |
| AFF3-1830 | chr2:100104747-100104774 | AFF3-4823 | chr2:99684008-99684035 |
| AFF3-1831 | chr2:100104751-100104778 | AFF3-4824 | chr2:99684009-99684036 |
| AFF3-1832 | chr2:100104753-100104780 | AFF3-4825 | chr2:99684013-99684040 |
| AFF3-1833 | chr2:100104754-100104781 | AFF3-4826 | chr2:99684027-99684054 |
| AFF3-1834 | chr2:100104757-100104784 | AFF3-4827 | chr2:99684036-99684063 |
| AFF3-1835 | chr2:100104758-100104785 | AFF3-4828 | chr2:99684041-99684068 |
| AFF3-1836 | chr2:100104759-100104786 | AFF3-4829 | chr2:99684043-99684070 |
| AFF3-1837 | chr2:100104762-100104789 | AFF3-4830 | chr2:99684049-99684076 |
| AFF3-1838 | chr2:100104765-100104792 | AFF3-4831 | chr2:99684050-99684077 |
| AFF3-1839 | chr2:100104766-100104793 | AFF3-4832 | chr2:99684051-99684078 |
| AFF3-1840 | chr2:100104767-100104794 | AFF3-4833 | chr2:99684052-99684079 |
| AFF3-1841 | chr2:100104770-100104797 | AFF3-4834 | chr2:99684074-99684101 |
| AFF3-1842 | chr2:100104771-100104798 | AFF3-4835 | chr2:99684087-99684114 |
| AFF3-1843 | chr2:100104782-100104809 | AFF3-4836 | chr2:99684088-99684115 |
| AFF3-1844 | chr2:100104783-100104810 | AFF3-4837 | chr2:99684099-99684126 |
| AFF3-1845 | chr2:100104787-100104814 | AFF3-4838 | chr2:99684127-99684154 |
| AFF3-1846 | chr2:100104788-100104815 | AFF3-4839 | chr2:99684154-99684181 |
| AFF3-1847 | chr2:100104799-100104826 | AFF3-4840 | chr2:99684157-99684184 |
| AFF3-1848 | chr2:100104800-100104827 | AFF3-4841 | chr2:99684170-99684197 |
| AFF3-1849 | chr2:100104803-100104830 | AFF3-4842 | chr2:99684177-99684204 |
| AFF3-1850 | chr2:100104806-100104833 | AFF3-4843 | chr2:99684181-99684208 |
| AFF3-1851 | chr2:100104809-100104836 | AFF3-4844 | chr2:99684182-99684209 |
| AFF3-1852 | chr2:100104812-100104839 | AFF3-4845 | chr2:99684183-99684210 |
| AFF3-1853 | chr2:100104815-100104842 | AFF3-4846 | chr2:99684198-99684225 |
| AFF3-1854 | chr2:100104818-100104845 | AFF3-4847 | chr2:99684205-99684232 |
| AFF3-1855 | chr2:100104821-100104848 | AFF3-4848 | chr2:99684208-99684235 |
| AFF3-1856 | chr2:100104836-100104863 | AFF3-4849 | chr2:99684209-99684236 |
| AFF3-1857 | chr2:100104837-100104864 | AFF3-4850 | chr2:99684210-99684237 |
| AFF3-1858 | chr2:100104840-100104867 | AFF3-4851 | chr2:99684212-99684239 |
| AFF3-1859 | chr2:100104841-100104868 | AFF3-4852 | chr2:99684215-99684242 |
| AFF3-1860 | chr2:100104844-100104871 | AFF3-4853 | chr2:99684230-99684257 |
| AFF3-1861 | chr2:100104845-100104872 | AFF3-4854 | chr2:99684261-99684288 |
| AFF3-1862 | chr2:100104848-100104875 | AFF3-4855 | chr2:99684269-99684296 |
| AFF3-1863 | chr2:100104853-100104880 | AFF3-4856 | chr2:99684275-99684302 |
| AFF3-1864 | chr2:100104854-100104881 | AFF3-4857 | chr2:99684279-99684306 |
| AFF3-1865 | chr2:100104857-100104884 | AFF3-4858 | chr2:99684290-99684317 |
| AFF3-1866 | chr2:100104859-100104886 | AFF3-4859 | chr2:99684297-99684324 |
| AFF3-1867 | chr2:100104860-100104887 | AFF3-4860 | chr2:99684298-99684325 |
| AFF3-1868 | chr2:100104865-100104892 | AFF3-4861 | chr2:99684299-99684326 |
| AFF3-1869 | chr2:100104887-100104914 | AFF3-4862 | chr2:99684309-99684336 |
| AFF3-1870 | chr2:100104903-100104930 | AFF3-4863 | chr2:99684315-99684342 |
| AFF3-1871 | chr2:100104910-100104937 | AFF3-4864 | chr2:99684316-99684343 |
| AFF3-1872 | chr2:100104911-100104938 | AFF3-4865 | chr2:99684327-99684354 |
| AFF3-1873 | chr2:100104912-100104939 | AFF3-4866 | chr2:99684328-99684355 |
| AFF3-1874 | chr2:100104917-100104944 | AFF3-4867 | chr2:99684338-99684365 |
| AFF3-1875 | chr2:100104922-100104949 | AFF3-4868 | chr2:99684360-99684387 |
| AFF3-1876 | chr2:100104923-100104950 | AFF3-4869 | chr2:99684366-99684393 |
| AFF3-1877 | chr2:100104926-100104953 | AFF3-4870 | chr2:99684367-99684394 |
| AFF3-1878 | chr2:100104927-100104954 | AFF3-4871 | chr2:99684368-99684395 |
| AFF3-1879 | chr2:100104928-100104955 | AFF3-4872 | chr2:99684371-99684398 |
| AFF3-1880 | chr2:100104935-100104962 | AFF3-4873 | chr2:99684372-99684399 |
| AFF3-1881 | chr2:100104936-100104963 | AFF3-4874 | chr2:99684373-99684400 |
| AFF3-1882 | chr2:100104937-100104964 | AFF3-4875 | chr2:99684374-99684401 |
| AFF3-1883 | chr2:100104945-100104972 | AFF3-4876 | chr2:99684384-99684411 |
| AFF3-1884 | chr2:100104946-100104973 | AFF3-4877 | chr2:99684385-99684412 |
| AFF3-1885 | chr2:100104947-100104974 | AFF3-4878 | chr2:99684391-99684418 |
| AFF3-1886 | chr2:100104952-100104979 | AFF3-4879 | chr2:99684406-99684433 |
| AFF3-1887 | chr2:100104955-100104982 | AFF3-4880 | chr2:99684407-99684434 |
| AFF3-1888 | chr2:100104957-100104984 | AFF3-4881 | chr2:99684410-99684437 |
| AFF3-1889 | chr2:100104966-100104993 | AFF3-4882 | chr2:99684413-99684440 |
| AFF3-1890 | chr2:100104967-100104994 | AFF3-4883 | chr2:99684454-99684481 |
| AFF3-1891 | chr2:100104983-100105010 | AFF3-4884 | chr2:99684462-99684489 |
| AFF3-1892 | chr2:100104985-100105012 | AFF3-4885 | chr2:99684470-99684497 |
| AFF3-1893 | chr2:100104988-100105015 | AFF3-4886 | chr2:99684481-99684508 |
| AFF3-1894 | chr2:100104991-100105018 | AFF3-4887 | chr2:99684528-99684555 |
| AFF3-1895 | chr2:100104992-100105019 | AFF3-4888 | chr2:99684539-99684566 |
| AFF3-1896 | chr2:100104994-100105021 | AFF3-4889 | chr2:99684543-99684570 |
| AFF3-1897 | chr2:100104996-100105023 | AFF3-4890 | chr2:99684548-99684575 |
| AFF3-1898 | chr2:100104999-100105026 | AFF3-4891 | chr2:99684550-99684577 |
| AFF3-1899 | chr2:100105000-100105027 | AFF3-4892 | chr2:99684571-99684598 |
| AFF3-1900 | chr2:100105001-100105028 | AFF3-4893 | chr2:99684586-99684613 |
| AFF3-1901 | chr2:100105019-100105046 | AFF3-4894 | chr2:99684604-99684631 |
| AFF3-1902 | chr2:100105020-100105047 | AFF3-4895 | chr2:99684610-99684637 |
| AFF3-1903 | chr2:100105021-100105048 | AFF3-4896 | chr2:99684630-99684657 |
| AFF3-1904 | chr2:100105022-100105049 | AFF3-4897 | chr2:99684634-99684661 |
| AFF3-1905 | chr2:100105023-100105050 | AFF3-4898 | chr2:99684644-99684671 |
| AFF3-1906 | chr2:100105026-100105053 | AFF3-4899 | chr2:99684653-99684680 |
| AFF3-1907 | chr2:100105027-100105054 | AFF3-4900 | chr2:99684679-99684706 |
| AFF3-1908 | chr2:100105028-100105055 | AFF3-4901 | chr2:99684693-99684720 |
| AFF3-1909 | chr2:100105029-100105056 | AFF3-4902 | chr2:99684699-99684726 |
| AFF3-1910 | chr2:100105030-100105057 | AFF3-4903 | chr2:99684702-99684729 |
| AFF3-1911 | chr2:100105031-100105058 | AFF3-4904 | chr2:99684703-99684730 |
| AFF3-1912 | chr2:100105032-100105059 | AFF3-4905 | chr2:99684710-99684737 |
| AFF3-1913 | chr2:100105033-100105060 | AFF3-4906 | chr2:99684718-99684745 |
| AFF3-1914 | chr2:100105041-100105068 | AFF3-4907 | chr2:99684721-99684748 |
| AFF3-1915 | chr2:100105042-100105069 | AFF3-4908 | chr2:99684722-99684749 |
| AFF3-1916 | chr2:100105045-100105072 | AFF3-4909 | chr2:99684725-99684752 |
| AFF3-1917 | chr2:100105046-100105073 | AFF3-4910 | chr2:99684728-99684755 |
| AFF3-1918 | chr2:100105051-100105078 | AFF3-4911 | chr2:99684731-99684758 |
| AFF3-1919 | chr2:100105052-100105079 | AFF3-4912 | chr2:99684734-99684761 |
| AFF3-1920 | chr2:100105055-100105082 | AFF3-4913 | chr2:99684735-99684762 |
| AFF3-1921 | chr2:100105060-100105087 | AFF3-4914 | chr2:99684756-99684783 |
| AFF3-1922 | chr2:100105061-100105088 | AFF3-4915 | chr2:99684762-99684789 |
| AFF3-1923 | chr2:100105062-100105089 | AFF3-4916 | chr2:99684765-99684792 |
| AFF3-1924 | chr2:100105063-100105090 | AFF3-4917 | chr2:99684770-99684797 |
| AFF3-1925 | chr2:100105066-100105093 | AFF3-4918 | chr2:99684771-99684798 |
| AFF3-1926 | chr2:100105078-100105105 | AFF3-4919 | chr2:99684775-99684802 |
| AFF3-1927 | chr2:100105079-100105106 | AFF3-4920 | chr2:99684776-99684803 |
| AFF3-1928 | chr2:100105080-100105107 | AFF3-4921 | chr2:99684799-99684826 |
| AFF3-1929 | chr2:100105081-100105108 | AFF3-4922 | chr2:99684837-99684864 |
| AFF3-1930 | chr2:100105082-100105109 | AFF3-4923 | chr2:99684840-99684867 |
| AFF3-1931 | chr2:100105088-100105115 | AFF3-4924 | chr2:99684843-99684870 |
| AFF3-1932 | chr2:100105096-100105123 | AFF3-4925 | chr2:99684863-99684890 |
| AFF3-1933 | chr2:100105100-100105127 | AFF3-4926 | chr2:99684898-99684925 |
| AFF3-1934 | chr2:100105118-100105145 | AFF3-4927 | chr2:99684921-99684948 |
| AFF3-1935 | chr2:100105123-100105150 | AFF3-4928 | chr2:99684930-99684957 |
| AFF3-1936 | chr2:100105124-100105151 | AFF3-4929 | chr2:99684957-99684984 |
| AFF3-1937 | chr2:100105128-100105155 | AFF3-4930 | chr2:99684963-99684990 |
| AFF3-1938 | chr2:100105129-100105156 | AFF3-4931 | chr2:99684971-99684998 |
| AFF3-1939 | chr2:100105136-100105163 | AFF3-4932 | chr2:99684979-99685006 |
| AFF3-1940 | chr2:100105139-100105166 | AFF3-4933 | chr2:99684980-99685007 |
| AFF3-1941 | chr2:100105140-100105167 | AFF3-4934 | chr2:99684982-99685009 |
| AFF3-1942 | chr2:100105146-100105173 | AFF3-4935 | chr2:99685014-99685041 |
| AFF3-1943 | chr2:100105149-100105176 | AFF3-4936 | chr2:99685020-99685047 |
| AFF3-1944 | chr2:100105150-100105177 | AFF3-4937 | chr2:99685023-99685050 |
| AFF3-1945 | chr2:100105153-100105180 | AFF3-4938 | chr2:99685026-99685053 |
| AFF3-1946 | chr2:100105154-100105181 | AFF3-4939 | chr2:99685029-99685056 |
| AFF3-1947 | chr2:100105156-100105183 | AFF3-4940 | chr2:99685030-99685057 |
| AFF3-1948 | chr2:100105157-100105184 | AFF3-4941 | chr2:99685046-99685073 |
| AFF3-1949 | chr2:100105159-100105186 | AFF3-4942 | chr2:99685047-99685074 |
| AFF3-1950 | chr2:100105160-100105187 | AFF3-4943 | chr2:99685055-99685082 |
| AFF3-1951 | chr2:100105164-100105191 | AFF3-4944 | chr2:99685058-99685085 |
| AFF3-1952 | chr2:100105165-100105192 | AFF3-4945 | chr2:99685061-99685088 |
| AFF3-1953 | chr2:100105166-100105193 | AFF3-4946 | chr2:99685062-99685089 |
| AFF3-1954 | chr2:100105169-100105196 | AFF3-4947 | chr2:99685074-99685101 |
| AFF3-1955 | chr2:100105170-100105197 | AFF3-4948 | chr2:99685085-99685112 |
| AFF3-1956 | chr2:100105174-100105201 | AFF3-4949 | chr2:99685086-99685113 |
| AFF3-1957 | chr2:100105178-100105205 | AFF3-4950 | chr2:99685089-99685116 |
| AFF3-1958 | chr2:100105179-100105206 | AFF3-4951 | chr2:99685092-99685119 |
| AFF3-1959 | chr2:100105180-100105207 | AFF3-4952 | chr2:99685097-99685124 |
| AFF3-1960 | chr2:100105199-100105226 | AFF3-4953 | chr2:99685103-99685130 |
| AFF3-1961 | chr2:100105200-100105227 | AFF3-4954 | chr2:99685115-99685142 |
| AFF3-1962 | chr2:100105203-100105230 | AFF3-4955 | chr2:99685120-99685147 |
| AFF3-1963 | chr2:100105204-100105231 | AFF3-4956 | chr2:99685125-99685152 |
| AFF3-1964 | chr2:100105206-100105233 | AFF3-4957 | chr2:99685127-99685154 |
| AFF3-1965 | chr2:100105207-100105234 | AFF3-4958 | chr2:99685129-99685156 |
| AFF3-1966 | chr2:100105214-100105241 | AFF3-4959 | chr2:99685137-99685164 |
| AFF3-1967 | chr2:100105215-100105242 | AFF3-4960 | chr2:99685148-99685175 |
| AFF3-1968 | chr2:100105216-100105243 | AFF3-4961 | chr2:99685171-99685198 |
| AFF3-1969 | chr2:100105219-100105246 | AFF3-4962 | chr2:99685183-99685210 |
| AFF3-1970 | chr2:100105222-100105249 | AFF3-4963 | chr2:99685184-99685211 |
| AFF3-1971 | chr2:100105223-100105250 | AFF3-4964 | chr2:99685185-99685212 |
| AFF3-1972 | chr2:100105228-100105255 | AFF3-4965 | chr2:99685189-99685216 |
| AFF3-1973 | chr2:100105229-100105256 | AFF3-4966 | chr2:99685192-99685219 |
| AFF3-1974 | chr2:100105232-100105259 | AFF3-4967 | chr2:99685198-99685225 |
| AFF3-1975 | chr2:100105233-100105260 | AFF3-4968 | chr2:99685199-99685226 |
| AFF3-1976 | chr2:100105236-100105263 | AFF3-4969 | chr2:99685210-99685237 |
| AFF3-1977 | chr2:100105237-100105264 | AFF3-4970 | chr2:99685220-99685247 |
| AFF3-1978 | chr2:100105238-100105265 | AFF3-4971 | chr2:99685226-99685253 |
| AFF3-1979 | chr2:100105239-100105266 | AFF3-4972 | chr2:99685229-99685256 |
| AFF3-1980 | chr2:100105240-100105267 | AFF3-4973 | chr2:99685230-99685257 |
| AFF3-1981 | chr2:100105241-100105268 | AFF3-4974 | chr2:99685231-99685258 |
| AFF3-1982 | chr2:100105251-100105278 | AFF3-4975 | chr2:99685232-99685259 |
| AFF3-1983 | chr2:100105252-100105279 | AFF3-4976 | chr2:99685233-99685260 |
| AFF3-1984 | chr2:100105260-100105287 | AFF3-4977 | chr2:99685238-99685265 |
| AFF3-1985 | chr2:100105265-100105292 | AFF3-4978 | chr2:99685244-99685271 |
| AFF3-1986 | chr2:100105266-100105293 | AFF3-4979 | chr2:99685272-99685299 |
| AFF3-1987 | chr2:100105267-100105294 | AFF3-4980 | chr2:99685279-99685306 |
| AFF3-1988 | chr2:100105273-100105300 | AFF3-4981 | chr2:99685292-99685319 |
| AFF3-1989 | chr2:100105274-100105301 | AFF3-4982 | chr2:99685296-99685323 |
| AFF3-1990 | chr2:100105275-100105302 | AFF3-4983 | chr2:99685336-99685363 |
| AFF3-1991 | chr2:100105279-100105306 | AFF3-4984 | chr2:99685344-99685371 |
| AFF3-1992 | chr2:100105282-100105309 | AFF3-4985 | chr2:99685345-99685372 |
| AFF3-1993 | chr2:100105283-100105310 | AFF3-4986 | chr2:99685349-99685376 |
| AFF3-1994 | chr2:100105308-100105335 | AFF3-4987 | chr2:99685357-99685384 |
| AFF3-1995 | chr2:100105311-100105338 | AFF3-4988 | chr2:99685359-99685386 |
| AFF3-1996 | chr2:100105312-100105339 | AFF3-4989 | chr2:99685360-99685387 |
| AFF3-1997 | chr2:100105316-100105343 | AFF3-4990 | chr2:99685369-99685396 |
| AFF3-1998 | chr2:100105323-100105350 | AFF3-4991 | chr2:99685377-99685404 |
| AFF3-1999 | chr2:100105324-100105351 | AFF3-4992 | chr2:99685428-99685455 |
| AFF3-2000 | chr2:100105325-100105352 | AFF3-4993 | chr2:99685440-99685467 |
| AFF3-2001 | chr2:100105334-100105361 | AFF3-4994 | chr2:99685441-99685468 |
| AFF3-2002 | chr2:100105351-100105378 | AFF3-4995 | chr2:99685442-99685469 |
| AFF3-2003 | chr2:100105353-100105380 | AFF3-4996 | chr2:99685447-99685474 |
| AFF3-2004 | chr2:100105354-100105381 | AFF3-4997 | chr2:99685453-99685480 |
| AFF3-2005 | chr2:100105359-100105386 | AFF3-4998 | chr2:99685454-99685481 |
| AFF3-2006 | chr2:100105362-100105389 | AFF3-4999 | chr2:99685455-99685482 |
| AFF3-2007 | chr2:100105379-100105406 | AFF3-5000 | chr2:99685459-99685486 |
| AFF3-2008 | chr2:100105393-100105420 | AFF3-5001 | chr2:99685465-99685492 |
| AFF3-2009 | chr2:100105397-100105424 | AFF3-5002 | chr2:99685475-99685502 |
| AFF3-2010 | chr2:100105399-100105426 | AFF3-5003 | chr2:99685486-99685513 |
| AFF3-2011 | chr2:100105402-100105429 | AFF3-5004 | chr2:99685498-99685525 |
| AFF3-2012 | chr2:100105403-100105430 | AFF3-5005 | chr2:99685503-99685530 |
| AFF3-2013 | chr2:100105408-100105435 | AFF3-5006 | chr2:99685504-99685531 |
| AFF3-2014 | chr2:100105412-100105439 | AFF3-5007 | chr2:99685514-99685541 |
| AFF3-2015 | chr2:100105415-100105442 | AFF3-5008 | chr2:99685519-99685546 |
| AFF3-2016 | chr2:100105416-100105443 | AFF3-5009 | chr2:99685521-99685548 |
| AFF3-2017 | chr2:100105430-100105457 | AFF3-5010 | chr2:99685533-99685560 |
| AFF3-2018 | chr2:100105433-100105460 | AFF3-5011 | chr2:99685608-99685635 |
| AFF3-2019 | chr2:100105436-100105463 | AFF3-5012 | chr2:99685617-99685644 |
| AFF3-2020 | chr2:100105444-100105471 | AFF3-5013 | chr2:99685621-99685648 |
| AFF3-2021 | chr2:100105445-100105472 | AFF3-5014 | chr2:99685635-99685662 |
| AFF3-2022 | chr2:100105446-100105473 | AFF3-5015 | chr2:99685689-99685716 |
| AFF3-2023 | chr2:100105448-100105475 | AFF3-5016 | chr2:99685690-99685717 |
| AFF3-2024 | chr2:100105449-100105476 | AFF3-5017 | chr2:99685696-99685723 |
| AFF3-2025 | chr2:100105452-100105479 | AFF3-5018 | chr2:99685711-99685738 |
| AFF3-2026 | chr2:100105453-100105480 | AFF3-5019 | chr2:99685764-99685791 |
| AFF3-2027 | chr2:100105464-100105491 | AFF3-5020 | chr2:99685787-99685814 |
| AFF3-2028 | chr2:100105468-100105495 | AFF3-5021 | chr2:99685795-99685822 |
| AFF3-2029 | chr2:100105469-100105496 | AFF3-5022 | chr2:99685804-99685831 |
| AFF3-2030 | chr2:100105477-100105504 | AFF3-5023 | chr2:99685818-99685845 |
| AFF3-2031 | chr2:100105479-100105506 | AFF3-5024 | chr2:99685824-99685851 |
| AFF3-2032 | chr2:100105480-100105507 | AFF3-5025 | chr2:99685825-99685852 |
| AFF3-2033 | chr2:100105481-100105508 | AFF3-5026 | chr2:99685835-99685862 |
| AFF3-2034 | chr2:100105484-100105511 | AFF3-5027 | chr2:99685840-99685867 |
| AFF3-2035 | chr2:100105485-100105512 | AFF3-5028 | chr2:99685843-99685870 |
| AFF3-2036 | chr2:100105486-100105513 | AFF3-5029 | chr2:99685848-99685875 |
| AFF3-2037 | chr2:100105487-100105514 | AFF3-5030 | chr2:99685856-99685883 |
| AFF3-2038 | chr2:100105496-100105523 | AFF3-5031 | chr2:99685857-99685884 |
| AFF3-2039 | chr2:100105503-100105530 | AFF3-5032 | chr2:99685872-99685899 |
| AFF3-2040 | chr2:100105504-100105531 | AFF3-5033 | chr2:99685879-99685906 |
| AFF3-2041 | chr2:100105524-100105551 | AFF3-5034 | chr2:99685883-99685910 |
| AFF3-2042 | chr2:100105535-100105562 | AFF3-5035 | chr2:99685884-99685911 |
| AFF3-2043 | chr2:100105540-100105567 | AFF3-5036 | chr2:99685889-99685916 |
| AFF3-2044 | chr2:100105545-100105572 | AFF3-5037 | chr2:99685892-99685919 |
| AFF3-2045 | chr2:100105549-100105576 | AFF3-5038 | chr2:99685902-99685929 |
| AFF3-2046 | chr2:100105552-100105579 | AFF3-5039 | chr2:99685907-99685934 |
| AFF3-2047 | chr2:100105553-100105580 | AFF3-5040 | chr2:99685915-99685942 |
| AFF3-2048 | chr2:100105559-100105586 | AFF3-5041 | chr2:99685921-99685948 |
| AFF3-2049 | chr2:100105564-100105591 | AFF3-5042 | chr2:99685922-99685949 |
| AFF3-2050 | chr2:100105565-100105592 | AFF3-5043 | chr2:99685925-99685952 |
| AFF3-2051 | chr2:100105585-100105612 | AFF3-5044 | chr2:99685926-99685953 |
| AFF3-2052 | chr2:100105615-100105642 | AFF3-5045 | chr2:99685928-99685955 |
| AFF3-2053 | chr2:100105636-100105663 | AFF3-5046 | chr2:99685931-99685958 |
| AFF3-2054 | chr2:100105637-100105664 | AFF3-5047 | chr2:99685934-99685961 |
| AFF3-2055 | chr2:100105639-100105666 | AFF3-5048 | chr2:99685935-99685962 |
| AFF3-2056 | chr2:100105659-100105686 | AFF3-5049 | chr2:99685938-99685965 |
| AFF3-2057 | chr2:100105660-100105687 | AFF3-5050 | chr2:99685947-99685974 |
| AFF3-2058 | chr2:100105661-100105688 | AFF3-5051 | chr2:99685952-99685979 |
| AFF3-2059 | chr2:100105662-100105689 | AFF3-5052 | chr2:99685970-99685997 |
| AFF3-2060 | chr2:100105682-100105709 | AFF3-5053 | chr2:99685989-99686016 |
| AFF3-2061 | chr2:100105683-100105710 | AFF3-5054 | chr2:99685993-99686020 |
| AFF3-2062 | chr2:100105702-100105729 | AFF3-5055 | chr2:99686012-99686039 |
| AFF3-2063 | chr2:100105709-100105736 | AFF3-5056 | chr2:99686013-99686040 |
| AFF3-2064 | chr2:100105711-100105738 | AFF3-5057 | chr2:99686020-99686047 |
| AFF3-2065 | chr2:100105712-100105739 | AFF3-5058 | chr2:99686021-99686048 |
| AFF3-2066 | chr2:100105716-100105743 | AFF3-5059 | chr2:99686022-99686049 |
| AFF3-2067 | chr2:100105720-100105747 | AFF3-5060 | chr2:99686027-99686054 |
| AFF3-2068 | chr2:100105727-100105754 | AFF3-5061 | chr2:99686030-99686057 |
| AFF3-2069 | chr2:100105734-100105761 | AFF3-5062 | chr2:99686033-99686060 |
| AFF3-2070 | chr2:100105737-100105764 | AFF3-5063 | chr2:99686034-99686061 |
| AFF3-2071 | chr2:100105744-100105771 | AFF3-5064 | chr2:99686043-99686070 |
| AFF3-2072 | chr2:100105751-100105778 | AFF3-5065 | chr2:99686045-99686072 |
| AFF3-2073 | chr2:100105752-100105779 | AFF3-5066 | chr2:99686058-99686085 |
| AFF3-2074 | chr2:100105753-100105780 | AFF3-5067 | chr2:99686061-99686088 |
| AFF3-2075 | chr2:100105756-100105783 | AFF3-5068 | chr2:99686064-99686091 |
| AFF3-2076 | chr2:100105759-100105786 | AFF3-5069 | chr2:99686071-99686098 |
| AFF3-2077 | chr2:100105762-100105789 | AFF3-5070 | chr2:99686072-99686099 |
| AFF3-2078 | chr2:100105763-100105790 | AFF3-5071 | chr2:99686073-99686100 |
| AFF3-2079 | chr2:100105764-100105791 | AFF3-5072 | chr2:99686078-99686105 |
| AFF3-2080 | chr2:100105765-100105792 | AFF3-5073 | chr2:99686090-99686117 |
| AFF3-2081 | chr2:100105771-100105798 | AFF3-5074 | chr2:99686093-99686120 |
| AFF3-2082 | chr2:100105774-100105801 | AFF3-5075 | chr2:99686096-99686123 |
| AFF3-2083 | chr2:100105779-100105806 | AFF3-5076 | chr2:99686099-99686126 |
| AFF3-2084 | chr2:100105783-100105810 | AFF3-5077 | chr2:99686112-99686139 |
| AFF3-2085 | chr2:100105784-100105811 | AFF3-5078 | chr2:99686113-99686140 |
| AFF3-2086 | chr2:100105787-100105814 | AFF3-5079 | chr2:99686137-99686164 |
| AFF3-2087 | chr2:100105795-100105822 | AFF3-5080 | chr2:99686139-99686166 |
| AFF3-2088 | chr2:100105806-100105833 | AFF3-5081 | chr2:99686140-99686167 |
| AFF3-2089 | chr2:100105815-100105842 | AFF3-5082 | chr2:99686148-99686175 |
| AFF3-2090 | chr2:100105816-100105843 | AFF3-5083 | chr2:99686158-99686185 |
| AFF3-2091 | chr2:100105817-100105844 | AFF3-5084 | chr2:99686162-99686189 |
| AFF3-2092 | chr2:100105825-100105852 | AFF3-5085 | chr2:99686180-99686207 |
| AFF3-2093 | chr2:100105844-100105871 | AFF3-5086 | chr2:99686183-99686210 |
| AFF3-2094 | chr2:100105845-100105872 | AFF3-5087 | chr2:99686210-99686237 |
| AFF3-2095 | chr2:100105857-100105884 | AFF3-5088 | chr2:99686211-99686238 |
| AFF3-2096 | chr2:100105862-100105889 | AFF3-5089 | chr2:99686216-99686243 |
| AFF3-2097 | chr2:100105870-100105897 | AFF3-5090 | chr2:99686238-99686265 |
| AFF3-2098 | chr2:100105871-100105898 | AFF3-5091 | chr2:99686261-99686288 |
| AFF3-2099 | chr2:100105876-100105903 | AFF3-5092 | chr2:99686267-99686294 |
| AFF3-2100 | chr2:100105885-100105912 | AFF3-5093 | chr2:99686308-99686335 |
| AFF3-2101 | chr2:100105886-100105913 | AFF3-5094 | chr2:99686311-99686338 |
| AFF3-2102 | chr2:100105887-100105914 | AFF3-5095 | chr2:99686317-99686344 |
| AFF3-2103 | chr2:100105890-100105917 | AFF3-5096 | chr2:99686346-99686373 |
| AFF3-2104 | chr2:100105902-100105929 | AFF3-5097 | chr2:99686348-99686375 |
| AFF3-2105 | chr2:100105903-100105930 | AFF3-5098 | chr2:99686358-99686385 |
| AFF3-2106 | chr2:100105907-100105934 | AFF3-5099 | chr2:99686359-99686386 |
| AFF3-2107 | chr2:100105910-100105937 | AFF3-5100 | chr2:99686360-99686387 |
| AFF3-2108 | chr2:100105911-100105938 | AFF3-5101 | chr2:99686364-99686391 |
| AFF3-2109 | chr2:100105917-100105944 | AFF3-5102 | chr2:99686365-99686392 |
| AFF3-2110 | chr2:100105918-100105945 | AFF3-5103 | chr2:99686382-99686409 |
| AFF3-2111 | chr2:100105919-100105946 | AFF3-5104 | chr2:99686383-99686410 |
| AFF3-2112 | chr2:100105927-100105954 | AFF3-5105 | chr2:99686388-99686415 |
| AFF3-2113 | chr2:100105928-100105955 | AFF3-5106 | chr2:99686402-99686429 |
| AFF3-2114 | chr2:100105929-100105956 | AFF3-5107 | chr2:99686403-99686430 |
| AFF3-2115 | chr2:100105930-100105957 | AFF3-5108 | chr2:99686404-99686431 |
| AFF3-2116 | chr2:100105945-100105972 | AFF3-5109 | chr2:99686410-99686437 |
| AFF3-2117 | chr2:100105952-100105979 | AFF3-5110 | chr2:99686417-99686444 |
| AFF3-2118 | chr2:100105953-100105980 | AFF3-5111 | chr2:99686424-99686451 |
| AFF3-2119 | chr2:100105961-100105988 | AFF3-5112 | chr2:99686426-99686453 |
| AFF3-2120 | chr2:100105965-100105992 | AFF3-5113 | chr2:99686429-99686456 |
| AFF3-2121 | chr2:100105966-100105993 | AFF3-5114 | chr2:99686434-99686461 |
| AFF3-2122 | chr2:100105974-100106001 | AFF3-5115 | chr2:99686435-99686462 |
| AFF3-2123 | chr2:100105975-100106002 | AFF3-5116 | chr2:99686466-99686493 |
| AFF3-2124 | chr2:100105996-100106023 | AFF3-5117 | chr2:99686471-99686498 |
| AFF3-2125 | chr2:100105997-100106024 | AFF3-5118 | chr2:99686475-99686502 |
| AFF3-2126 | chr2:100106000-100106027 | AFF3-5119 | chr2:99686491-99686518 |
| AFF3-2127 | chr2:100106011-100106038 | AFF3-5120 | chr2:99686505-99686532 |
| AFF3-2128 | chr2:100106023-100106050 | AFF3-5121 | chr2:99686506-99686533 |
| AFF3-2129 | chr2:100106024-100106051 | AFF3-5122 | chr2:99686511-99686538 |
| AFF3-2130 | chr2:100106030-100106057 | AFF3-5123 | chr2:99686512-99686539 |
| AFF3-2131 | chr2:100106035-100106062 | AFF3-5124 | chr2:99686531-99686558 |
| AFF3-2132 | chr2:100106036-100106063 | AFF3-5125 | chr2:99686535-99686562 |
| AFF3-2133 | chr2:100106039-100106066 | AFF3-5126 | chr2:99686555-99686582 |
| AFF3-2134 | chr2:100106042-100106069 | AFF3-5127 | chr2:99686571-99686598 |
| AFF3-2135 | chr2:100106044-100106071 | AFF3-5128 | chr2:99686585-99686612 |
| AFF3-2136 | chr2:100106051-100106078 | AFF3-5129 | chr2:99686586-99686613 |
| AFF3-2137 | chr2:100106066-100106093 | AFF3-5130 | chr2:99686600-99686627 |
| AFF3-2138 | chr2:100106086-100106113 | AFF3-5131 | chr2:99686631-99686658 |
| AFF3-2139 | chr2:100106094-100106121 | AFF3-5132 | chr2:99686632-99686659 |
| AFF3-2140 | chr2:100106097-100106124 | AFF3-5133 | chr2:99686633-99686660 |
| AFF3-2141 | chr2:100106110-100106137 | AFF3-5134 | chr2:99686634-99686661 |
| AFF3-2142 | chr2:100106118-100106145 | AFF3-5135 | chr2:99686635-99686662 |
| AFF3-2143 | chr2:100106141-100106168 | AFF3-5136 | chr2:99686646-99686673 |
| AFF3-2144 | chr2:100106148-100106175 | AFF3-5137 | chr2:99686652-99686679 |
| AFF3-2145 | chr2:100106149-100106176 | AFF3-5138 | chr2:99686660-99686687 |
| AFF3-2146 | chr2:100106156-100106183 | AFF3-5139 | chr2:99686668-99686695 |
| AFF3-2147 | chr2:100106157-100106184 | AFF3-5140 | chr2:99686682-99686709 |
| AFF3-2148 | chr2:100106158-100106185 | AFF3-5141 | chr2:99686683-99686710 |
| AFF3-2149 | chr2:100106159-100106186 | AFF3-5142 | chr2:99686699-99686726 |
| AFF3-2150 | chr2:100106175-100106202 | AFF3-5143 | chr2:99686700-99686727 |
| AFF3-2151 | chr2:100106192-100106219 | AFF3-5144 | chr2:99686701-99686728 |
| AFF3-2152 | chr2:100106212-100106239 | AFF3-5145 | chr2:99686702-99686729 |
| AFF3-2153 | chr2:100106295-100106322 | AFF3-5146 | chr2:99686711-99686738 |
| AFF3-2154 | chr2:100106296-100106323 | AFF3-5147 | chr2:99686715-99686742 |
| AFF3-2155 | chr2:100106297-100106324 | AFF3-5148 | chr2:99686719-99686746 |
| AFF3-2156 | chr2:100106305-100106332 | AFF3-5149 | chr2:99686735-99686762 |
| AFF3-2157 | chr2:100106306-100106333 | AFF3-5150 | chr2:99686757-99686784 |
| AFF3-2158 | chr2:100106307-100106334 | AFF3-5151 | chr2:99686763-99686790 |
| AFF3-2159 | chr2:100106320-100106347 | AFF3-5152 | chr2:99686767-99686794 |
| AFF3-2160 | chr2:100106347-100106374 | AFF3-5153 | chr2:99686771-99686798 |
| AFF3-2161 | chr2:100106359-100106386 | AFF3-5154 | chr2:99686779-99686806 |
| AFF3-2162 | chr2:100106367-100106394 | AFF3-5155 | chr2:99686780-99686807 |
| AFF3-2163 | chr2:100106368-100106395 | AFF3-5156 | chr2:99686791-99686818 |
| AFF3-2164 | chr2:100106372-100106399 | AFF3-5157 | chr2:99686804-99686831 |
| AFF3-2165 | chr2:100106403-100106430 | AFF3-5158 | chr2:99686812-99686839 |
| AFF3-2166 | chr2:100106404-100106431 | AFF3-5159 | chr2:99686822-99686849 |
| AFF3-2167 | chr2:100106410-100106437 | AFF3-5160 | chr2:99686823-99686850 |
| AFF3-2168 | chr2:100106411-100106438 | AFF3-5161 | chr2:99686827-99686854 |
| AFF3-2169 | chr2:100106467-100106494 | AFF3-5162 | chr2:99686837-99686864 |
| AFF3-2170 | chr2:100106472-100106499 | AFF3-5163 | chr2:99686838-99686865 |
| AFF3-2171 | chr2:100106473-100106500 | AFF3-5164 | chr2:99686841-99686868 |
| AFF3-2172 | chr2:100106474-100106501 | AFF3-5165 | chr2:99686842-99686869 |
| AFF3-2173 | chr2:100106482-100106509 | AFF3-5166 | chr2:99686851-99686878 |
| AFF3-2174 | chr2:100106486-100106513 | AFF3-5167 | chr2:99686852-99686879 |
| AFF3-2175 | chr2:100106520-100106547 | AFF3-5168 | chr2:99686868-99686895 |
| AFF3-2176 | chr2:100106528-100106555 | AFF3-5169 | chr2:99686872-99686899 |
| AFF3-2177 | chr2:100106532-100106559 | AFF3-5170 | chr2:99686873-99686900 |
| AFF3-2178 | chr2:100106533-100106560 | AFF3-5171 | chr2:99686889-99686916 |
| AFF3-2179 | chr2:100106534-100106561 | AFF3-5172 | chr2:99686895-99686922 |
| AFF3-2180 | chr2:100106535-100106562 | AFF3-5173 | chr2:99686902-99686929 |
| AFF3-2181 | chr2:100106538-100106565 | AFF3-5174 | chr2:99686943-99686970 |
| AFF3-2182 | chr2:100106541-100106568 | AFF3-5175 | chr2:99686956-99686983 |
| AFF3-2183 | chr2:100106542-100106569 | AFF3-5176 | chr2:99686966-99686993 |
| AFF3-2184 | chr2:100106543-100106570 | AFF3-5177 | chr2:99686978-99687005 |
| AFF3-2185 | chr2:100106545-100106572 | AFF3-5178 | chr2:99686982-99687009 |
| AFF3-2186 | chr2:100106548-100106575 | AFF3-5179 | chr2:99686985-99687012 |
| AFF3-2187 | chr2:100106551-100106578 | AFF3-5180 | chr2:99686992-99687019 |
| AFF3-2188 | chr2:100106552-100106579 | AFF3-5181 | chr2:99686998-99687025 |
| AFF3-2189 | chr2:100106558-100106585 | AFF3-5182 | chr2:99686999-99687026 |
| AFF3-2190 | chr2:100106574-100106601 | AFF3-5183 | chr2:99687012-99687039 |
| AFF3-2191 | chr2:100106591-100106618 | AFF3-5184 | chr2:99687015-99687042 |
| AFF3-2192 | chr2:100106592-100106619 | AFF3-5185 | chr2:99687016-99687043 |
| AFF3-2193 | chr2:100106593-100106620 | AFF3-5186 | chr2:99687017-99687044 |
| AFF3-2194 | chr2:100106594-100106621 | AFF3-5187 | chr2:99687018-99687045 |
| AFF3-2195 | chr2:100106622-100106649 | AFF3-5188 | chr2:99687019-99687046 |
| AFF3-2196 | chr2:100106631-100106658 | AFF3-5189 | chr2:99687025-99687052 |
| AFF3-2197 | chr2:100106640-100106667 | AFF3-5190 | chr2:99687031-99687058 |
| AFF3-2198 | chr2:100106641-100106668 | AFF3-5191 | chr2:99687048-99687075 |
| AFF3-2199 | chr2:100106642-100106669 | AFF3-5192 | chr2:99687065-99687092 |
| AFF3-2200 | chr2:100106646-100106673 | AFF3-5193 | chr2:99687071-99687098 |
| AFF3-2201 | chr2:100106647-100106674 | AFF3-5194 | chr2:99687087-99687114 |
| AFF3-2202 | chr2:100106654-100106681 | AFF3-5195 | chr2:99687088-99687115 |
| AFF3-2203 | chr2:100106659-100106686 | AFF3-5196 | chr2:99687109-99687136 |
| AFF3-2204 | chr2:100106670-100106697 | AFF3-5197 | chr2:99687128-99687155 |
| AFF3-2205 | chr2:100106680-100106707 | AFF3-5198 | chr2:99687135-99687162 |
| AFF3-2206 | chr2:100106681-100106708 | AFF3-5199 | chr2:99687154-99687181 |
| AFF3-2207 | chr2:100106687-100106714 | AFF3-5200 | chr2:99687173-99687200 |
| AFF3-2208 | chr2:100106690-100106717 | AFF3-5201 | chr2:99687182-99687209 |
| AFF3-2209 | chr2:100106693-100106720 | AFF3-5202 | chr2:99687190-99687217 |
| AFF3-2210 | chr2:100106694-100106721 | AFF3-5203 | chr2:99687196-99687223 |
| AFF3-2211 | chr2:100106698-100106725 | AFF3-5204 | chr2:99687199-99687226 |
| AFF3-2212 | chr2:100106699-100106726 | AFF3-5205 | chr2:99687200-99687227 |
| AFF3-2213 | chr2:100106704-100106731 | AFF3-5206 | chr2:99687213-99687240 |
| AFF3-2214 | chr2:100106719-100106746 | AFF3-5207 | chr2:99687244-99687271 |
| AFF3-2215 | chr2:100106723-100106750 | AFF3-5208 | chr2:99687245-99687272 |
| AFF3-2216 | chr2:100106724-100106751 | AFF3-5209 | chr2:99687253-99687280 |
| AFF3-2217 | chr2:100106725-100106752 | AFF3-5210 | chr2:99687264-99687291 |
| AFF3-2218 | chr2:100106728-100106755 | AFF3-5211 | chr2:99687291-99687318 |
| AFF3-2219 | chr2:100106729-100106756 | AFF3-5212 | chr2:99687292-99687319 |
| AFF3-2220 | chr2:100106732-100106759 | AFF3-5213 | chr2:99687302-99687329 |
| AFF3-2221 | chr2:100106739-100106766 | AFF3-5214 | chr2:99687307-99687334 |
| AFF3-2222 | chr2:100106757-100106784 | AFF3-5215 | chr2:99687308-99687335 |
| AFF3-2223 | chr2:100106776-100106803 | AFF3-5216 | chr2:99687321-99687348 |
| AFF3-2224 | chr2:100106777-100106804 | AFF3-5217 | chr2:99687344-99687371 |
| AFF3-2225 | chr2:100106782-100106809 | AFF3-5218 | chr2:99687350-99687377 |
| AFF3-2226 | chr2:100106794-100106821 | AFF3-5219 | chr2:99687352-99687379 |
| AFF3-2227 | chr2:100106795-100106822 | AFF3-5220 | chr2:99687355-99687382 |
| AFF3-2228 | chr2:100106805-100106832 | AFF3-5221 | chr2:99687359-99687386 |
| AFF3-2229 | chr2:100106812-100106839 | AFF3-5222 | chr2:99687360-99687387 |
| AFF3-2230 | chr2:100106815-100106842 | AFF3-5223 | chr2:99687361-99687388 |
| AFF3-2231 | chr2:100106828-100106855 | AFF3-5224 | chr2:99687371-99687398 |
| AFF3-2232 | chr2:100106829-100106856 | AFF3-5225 | chr2:99687372-99687399 |
| AFF3-2233 | chr2:100106834-100106861 | AFF3-5226 | chr2:99687373-99687400 |
| AFF3-2234 | chr2:100106845-100106872 | AFF3-5227 | chr2:99687374-99687401 |
| AFF3-2235 | chr2:100106852-100106879 | AFF3-5228 | chr2:99687378-99687405 |
| AFF3-2236 | chr2:100106853-100106880 | AFF3-5229 | chr2:99687381-99687408 |
| AFF3-2237 | chr2:100106857-100106884 | AFF3-5230 | chr2:99687389-99687416 |
| AFF3-2238 | chr2:100106862-100106889 | AFF3-5231 | chr2:99687405-99687432 |
| AFF3-2239 | chr2:100106863-100106890 | AFF3-5232 | chr2:99687408-99687435 |
| AFF3-2240 | chr2:100106864-100106891 | AFF3-5233 | chr2:99687409-99687436 |
| AFF3-2241 | chr2:100106868-100106895 | AFF3-5234 | chr2:99687410-99687437 |
| AFF3-2242 | chr2:100106879-100106906 | AFF3-5235 | chr2:99687415-99687442 |
| AFF3-2243 | chr2:100106885-100106912 | AFF3-5236 | chr2:99687416-99687443 |
| AFF3-2244 | chr2:100106886-100106913 | AFF3-5237 | chr2:99687425-99687452 |
| AFF3-2245 | chr2:100106887-100106914 | AFF3-5238 | chr2:99687426-99687453 |
| AFF3-2246 | chr2:100106902-100106929 | AFF3-5239 | chr2:99687428-99687455 |
| AFF3-2247 | chr2:100106930-100106957 | AFF3-5240 | chr2:99687441-99687468 |
| AFF3-2248 | chr2:100106931-100106958 | AFF3-5241 | chr2:99687452-99687479 |
| AFF3-2249 | chr2:100106935-100106962 | AFF3-5242 | chr2:99687475-99687502 |
| AFF3-2250 | chr2:100106936-100106963 | AFF3-5243 | chr2:99687495-99687522 |
| AFF3-2251 | chr2:100106944-100106971 | AFF3-5244 | chr2:99687515-99687542 |
| AFF3-2252 | chr2:100106959-100106986 | AFF3-5245 | chr2:99687516-99687543 |
| AFF3-2253 | chr2:100106989-100107016 | AFF3-5246 | chr2:99687523-99687550 |
| AFF3-2254 | chr2:100107008-100107035 | AFF3-5247 | chr2:99687530-99687557 |
| AFF3-2255 | chr2:100107011-100107038 | AFF3-5248 | chr2:99687532-99687559 |
| AFF3-2256 | chr2:100107012-100107039 | AFF3-5249 | chr2:99687537-99687564 |
| AFF3-2257 | chr2:100107018-100107045 | AFF3-5250 | chr2:99687540-99687567 |
| AFF3-2258 | chr2:100107034-100107061 | AFF3-5251 | chr2:99687541-99687568 |
| AFF3-2259 | chr2:100107057-100107084 | AFF3-5252 | chr2:99687551-99687578 |
| AFF3-2260 | chr2:100107058-100107085 | AFF3-5253 | chr2:99687558-99687585 |
| AFF3-2261 | chr2:100107059-100107086 | AFF3-5254 | chr2:99687568-99687595 |
| AFF3-2262 | chr2:100107067-100107094 | AFF3-5255 | chr2:99687579-99687606 |
| AFF3-2263 | chr2:100107071-100107098 | AFF3-5256 | chr2:99687591-99687618 |
| AFF3-2264 | chr2:100107072-100107099 | AFF3-5257 | chr2:99687592-99687619 |
| AFF3-2265 | chr2:100107073-100107100 | AFF3-5258 | chr2:99687604-99687631 |
| AFF3-2266 | chr2:100107081-100107108 | AFF3-5259 | chr2:99687605-99687632 |
| AFF3-2267 | chr2:100107087-100107114 | AFF3-5260 | chr2:99687606-99687633 |
| AFF3-2268 | chr2:100107120-100107147 | AFF3-5261 | chr2:99687608-99687635 |
| AFF3-2269 | chr2:100107125-100107152 | AFF3-5262 | chr2:99687609-99687636 |
| AFF3-2270 | chr2:100107134-100107161 | AFF3-5263 | chr2:99687618-99687645 |
| AFF3-2271 | chr2:100107144-100107171 | AFF3-5264 | chr2:99687619-99687646 |
| AFF3-2272 | chr2:100107175-100107202 | AFF3-5265 | chr2:99687620-99687647 |
| AFF3-2273 | chr2:100107176-100107203 | AFF3-5266 | chr2:99687628-99687655 |
| AFF3-2274 | chr2:100107192-100107219 | AFF3-5267 | chr2:99687629-99687656 |
| AFF3-2275 | chr2:100107195-100107222 | AFF3-5268 | chr2:99687638-99687665 |
| AFF3-2276 | chr2:100107228-100107255 | AFF3-5269 | chr2:99687642-99687669 |
| AFF3-2277 | chr2:100107253-100107280 | AFF3-5270 | chr2:99687645-99687672 |
| AFF3-2278 | chr2:100107254-100107281 | AFF3-5271 | chr2:99687673-99687700 |
| AFF3-2279 | chr2:100107272-100107299 | AFF3-5272 | chr2:99687725-99687752 |
| AFF3-2280 | chr2:100107275-100107302 | AFF3-5273 | chr2:99687726-99687753 |
| AFF3-2281 | chr2:100107311-100107338 | AFF3-5274 | chr2:99687736-99687763 |
| AFF3-2282 | chr2:100107312-100107339 | AFF3-5275 | chr2:99687765-99687792 |
| AFF3-2283 | chr2:100107317-100107344 | AFF3-5276 | chr2:99687766-99687793 |
| AFF3-2284 | chr2:100107325-100107352 | AFF3-5277 | chr2:99687772-99687799 |
| AFF3-2285 | chr2:100107328-100107355 | AFF3-5278 | chr2:99687780-99687807 |
| AFF3-2286 | chr2:100107332-100107359 | AFF3-5279 | chr2:99687786-99687813 |
| AFF3-2287 | chr2:100107348-100107375 | AFF3-5280 | chr2:99687812-99687839 |
| AFF3-2288 | chr2:100107382-100107409 | AFF3-5281 | chr2:99687832-99687859 |
| AFF3-2289 | chr2:100107383-100107410 | AFF3-5282 | chr2:99687842-99687869 |
| AFF3-2290 | chr2:100107387-100107414 | AFF3-5283 | chr2:99687857-99687884 |
| AFF3-2291 | chr2:100107388-100107415 | AFF3-5284 | chr2:99687861-99687888 |
| AFF3-2292 | chr2:100107396-100107423 | AFF3-5285 | chr2:99687865-99687892 |
| AFF3-2293 | chr2:100107397-100107424 | AFF3-5286 | chr2:99687875-99687902 |
| AFF3-2294 | chr2:100107398-100107425 | AFF3-5287 | chr2:99687883-99687910 |
| AFF3-2295 | chr2:100107421-100107448 | AFF3-5288 | chr2:99687884-99687911 |
| AFF3-2296 | chr2:100107422-100107449 | AFF3-5289 | chr2:99687911-99687938 |
| AFF3-2297 | chr2:100107522-100107549 | AFF3-5290 | chr2:99687924-99687951 |
| AFF3-2298 | chr2:100107534-100107561 | AFF3-5291 | chr2:99687927-99687954 |
| AFF3-2299 | chr2:100107535-100107562 | AFF3-5292 | chr2:99687930-99687957 |
| AFF3-2300 | chr2:100107539-100107566 | AFF3-5293 | chr2:99687933-99687960 |
| AFF3-2301 | chr2:100107553-100107580 | AFF3-5294 | chr2:99687934-99687961 |
| AFF3-2302 | chr2:100107555-100107582 | AFF3-5295 | chr2:99687950-99687977 |
| AFF3-2303 | chr2:100107571-100107598 | AFF3-5296 | chr2:99687952-99687979 |
| AFF3-2304 | chr2:100107572-100107599 | AFF3-5297 | chr2:99687956-99687983 |
| AFF3-2305 | chr2:100107573-100107600 | AFF3-5298 | chr2:99687959-99687986 |
| AFF3-2306 | chr2:100107593-100107620 | AFF3-5299 | chr2:99687965-99687992 |
| AFF3-2307 | chr2:100107594-100107621 | AFF3-5300 | chr2:99687978-99688005 |
| AFF3-2308 | chr2:100107595-100107622 | AFF3-5301 | chr2:99687989-99688016 |
| AFF3-2309 | chr2:100107599-100107626 | AFF3-5302 | chr2:99687990-99688017 |
| AFF3-2310 | chr2:100107600-100107627 | AFF3-5303 | chr2:99687993-99688020 |
| AFF3-2311 | chr2:100107601-100107628 | AFF3-5304 | chr2:99687996-99688023 |
| AFF3-2312 | chr2:100107604-100107631 | AFF3-5305 | chr2:99688001-99688028 |
| AFF3-2313 | chr2:100107605-100107632 | AFF3-5306 | chr2:99688002-99688029 |
| AFF3-2314 | chr2:100107612-100107639 | AFF3-5307 | chr2:99688008-99688035 |
| AFF3-2315 | chr2:100107613-100107640 | AFF3-5308 | chr2:99688009-99688036 |
| AFF3-2316 | chr2:100107614-100107641 | AFF3-5309 | chr2:99688028-99688055 |
| AFF3-2317 | chr2:100107615-100107642 | AFF3-5310 | chr2:99688032-99688059 |
| AFF3-2318 | chr2:100107616-100107643 | AFF3-5311 | chr2:99688042-99688069 |
| AFF3-2319 | chr2:100107617-100107644 | AFF3-5312 | chr2:99688051-99688078 |
| AFF3-2320 | chr2:100107633-100107660 | AFF3-5313 | chr2:99688068-99688095 |
| AFF3-2321 | chr2:100107643-100107670 | AFF3-5314 | chr2:99688069-99688096 |
| AFF3-2322 | chr2:100107651-100107678 | AFF3-5315 | chr2:99688074-99688101 |
| AFF3-2323 | chr2:100107652-100107679 | AFF3-5316 | chr2:99688083-99688110 |
| AFF3-2324 | chr2:100107657-100107684 | AFF3-5317 | chr2:99688084-99688111 |
| AFF3-2325 | chr2:100107661-100107688 | AFF3-5318 | chr2:99688085-99688112 |
| AFF3-2326 | chr2:100107665-100107692 | AFF3-5319 | chr2:99688086-99688113 |
| AFF3-2327 | chr2:100107669-100107696 | AFF3-5320 | chr2:99688087-99688114 |
| AFF3-2328 | chr2:100107674-100107701 | AFF3-5321 | chr2:99688090-99688117 |
| AFF3-2329 | chr2:100107675-100107702 | AFF3-5322 | chr2:99688093-99688120 |
| AFF3-2330 | chr2:100107695-100107722 | AFF3-5323 | chr2:99688096-99688123 |
| AFF3-2331 | chr2:100107696-100107723 | AFF3-5324 | chr2:99688099-99688126 |
| AFF3-2332 | chr2:100107712-100107739 | AFF3-5325 | chr2:99688100-99688127 |
| AFF3-2333 | chr2:100107724-100107751 | AFF3-5326 | chr2:99688112-99688139 |
| AFF3-2334 | chr2:100107725-100107752 | AFF3-5327 | chr2:99688127-99688154 |
| AFF3-2335 | chr2:100107726-100107753 | AFF3-5328 | chr2:99688135-99688162 |
| AFF3-2336 | chr2:100107738-100107765 | AFF3-5329 | chr2:99688136-99688163 |
| AFF3-2337 | chr2:100107754-100107781 | AFF3-5330 | chr2:99688140-99688167 |
| AFF3-2338 | chr2:100107755-100107782 | AFF3-5331 | chr2:99688141-99688168 |
| AFF3-2339 | chr2:100107757-100107784 | AFF3-5332 | chr2:99688142-99688169 |
| AFF3-2340 | chr2:100107770-100107797 | AFF3-5333 | chr2:99688165-99688192 |
| AFF3-2341 | chr2:100107774-100107801 | AFF3-5334 | chr2:99688201-99688228 |
| AFF3-2342 | chr2:100107788-100107815 | AFF3-5335 | chr2:99688210-99688237 |
| AFF3-2343 | chr2:100107791-100107818 | AFF3-5336 | chr2:99688220-99688247 |
| AFF3-2344 | chr2:100107802-100107829 | AFF3-5337 | chr2:99688226-99688253 |
| AFF3-2345 | chr2:100107813-100107840 | AFF3-5338 | chr2:99688232-99688259 |
| AFF3-2346 | chr2:100107832-100107859 | AFF3-5339 | chr2:99688254-99688281 |
| AFF3-2347 | chr2:100107833-100107860 | AFF3-5340 | chr2:99688255-99688282 |
| AFF3-2348 | chr2:100107844-100107871 | AFF3-5341 | chr2:99688256-99688283 |
| AFF3-2349 | chr2:100107862-100107889 | AFF3-5342 | chr2:99688281-99688308 |
| AFF3-2350 | chr2:100107884-100107911 | AFF3-5343 | chr2:99688290-99688317 |
| AFF3-2351 | chr2:100107889-100107916 | AFF3-5344 | chr2:99688292-99688319 |
| AFF3-2352 | chr2:100107890-100107917 | AFF3-5345 | chr2:99688302-99688329 |
| AFF3-2353 | chr2:100107909-100107936 | AFF3-5346 | chr2:99688303-99688330 |
| AFF3-2354 | chr2:100107910-100107937 | AFF3-5347 | chr2:99688304-99688331 |
| AFF3-2355 | chr2:100107929-100107956 | AFF3-5348 | chr2:99688328-99688355 |
| AFF3-2356 | chr2:100107960-100107987 | AFF3-5349 | chr2:99688340-99688367 |
| AFF3-2357 | chr2:100107961-100107988 | AFF3-5350 | chr2:99688342-99688369 |
| AFF3-2358 | chr2:100107962-100107989 | AFF3-5351 | chr2:99688368-99688395 |
| AFF3-2359 | chr2:100107964-100107991 | AFF3-5352 | chr2:99688387-99688414 |
| AFF3-2360 | chr2:100107969-100107996 | AFF3-5353 | chr2:99688388-99688415 |
| AFF3-2361 | chr2:100107970-100107997 | AFF3-5354 | chr2:99688412-99688439 |
| AFF3-2362 | chr2:100107971-100107998 | AFF3-5355 | chr2:99688417-99688444 |
| AFF3-2363 | chr2:100107973-100108000 | AFF3-5356 | chr2:99688433-99688460 |
| AFF3-2364 | chr2:100107982-100108009 | AFF3-5357 | chr2:99688454-99688481 |
| AFF3-2365 | chr2:100107985-100108012 | AFF3-5358 | chr2:99688455-99688482 |
| AFF3-2366 | chr2:100107990-100108017 | AFF3-5359 | chr2:99688456-99688483 |
| AFF3-2367 | chr2:100107994-100108021 | AFF3-5360 | chr2:99688457-99688484 |
| AFF3-2368 | chr2:100107998-100108025 | AFF3-5361 | chr2:99688467-99688494 |
| AFF3-2369 | chr2:100108005-100108032 | AFF3-5362 | chr2:99688480-99688507 |
| AFF3-2370 | chr2:100108048-100108075 | AFF3-5363 | chr2:99688489-99688516 |
| AFF3-2371 | chr2:100108054-100108081 | AFF3-5364 | chr2:99688495-99688522 |
| AFF3-2372 | chr2:100108055-100108082 | AFF3-5365 | chr2:99688507-99688534 |
| AFF3-2373 | chr2:100108056-100108083 | AFF3-5366 | chr2:99688511-99688538 |
| AFF3-2374 | chr2:100108061-100108088 | AFF3-5367 | chr2:99688520-99688547 |
| AFF3-2375 | chr2:100108068-100108095 | AFF3-5368 | chr2:99688566-99688593 |
| AFF3-2376 | chr2:100108071-100108098 | AFF3-5369 | chr2:99688581-99688608 |
| AFF3-2377 | chr2:100108081-100108108 | AFF3-5370 | chr2:99688620-99688647 |
| AFF3-2378 | chr2:100108082-100108109 | AFF3-5371 | chr2:99688622-99688649 |
| AFF3-2379 | chr2:100108101-100108128 | AFF3-5372 | chr2:99688637-99688664 |
| AFF3-2380 | chr2:100108107-100108134 | AFF3-5373 | chr2:99688638-99688665 |
| AFF3-2381 | chr2:100108127-100108154 | AFF3-5374 | chr2:99688639-99688666 |
| AFF3-2382 | chr2:100108132-100108159 | AFF3-5375 | chr2:99688648-99688675 |
| AFF3-2383 | chr2:100108135-100108162 | AFF3-5376 | chr2:99688649-99688676 |
| AFF3-2384 | chr2:100108136-100108163 | AFF3-5377 | chr2:99688651-99688678 |
| AFF3-2385 | chr2:100108144-100108171 | AFF3-5378 | chr2:99688652-99688679 |
| AFF3-2386 | chr2:100108148-100108175 | AFF3-5379 | chr2:99688670-99688697 |
| AFF3-2387 | chr2:100108156-100108183 | AFF3-5380 | chr2:99688671-99688698 |
| AFF3-2388 | chr2:100108157-100108184 | AFF3-5381 | chr2:99688674-99688701 |
| AFF3-2389 | chr2:100108165-100108192 | AFF3-5382 | chr2:99688679-99688706 |
| AFF3-2390 | chr2:100108179-100108206 | AFF3-5383 | chr2:99688683-99688710 |
| AFF3-2391 | chr2:100108198-100108225 | AFF3-5384 | chr2:99688712-99688739 |
| AFF3-2392 | chr2:100108213-100108240 | AFF3-5385 | chr2:99688718-99688745 |
| AFF3-2393 | chr2:100108221-100108248 | AFF3-5386 | chr2:99688723-99688750 |
| AFF3-2394 | chr2:100108242-100108269 | AFF3-5387 | chr2:99688724-99688751 |
| AFF3-2395 | chr2:100108243-100108270 | AFF3-5388 | chr2:99688728-99688755 |
| AFF3-2396 | chr2:100108244-100108271 | AFF3-5389 | chr2:99688737-99688764 |
| AFF3-2397 | chr2:100108248-100108275 | AFF3-5390 | chr2:99688744-99688771 |
| AFF3-2398 | chr2:100108266-100108293 | AFF3-5391 | chr2:99688749-99688776 |
| AFF3-2399 | chr2:100108267-100108294 | AFF3-5392 | chr2:99688752-99688779 |
| AFF3-2400 | chr2:100108270-100108297 | AFF3-5393 | chr2:99688757-99688784 |
| AFF3-2401 | chr2:100108271-100108298 | AFF3-5394 | chr2:99688758-99688785 |
| AFF3-2402 | chr2:100108288-100108315 | AFF3-5395 | chr2:99688759-99688786 |
| AFF3-2403 | chr2:100108294-100108321 | AFF3-5396 | chr2:99688762-99688789 |
| AFF3-2404 | chr2:100108299-100108326 | AFF3-5397 | chr2:99688763-99688790 |
| AFF3-2405 | chr2:100108300-100108327 | AFF3-5398 | chr2:99688764-99688791 |
| AFF3-2406 | chr2:100108301-100108328 | AFF3-5399 | chr2:99688765-99688792 |
| AFF3-2407 | chr2:100108309-100108336 | AFF3-5400 | chr2:99688772-99688799 |
| AFF3-2408 | chr2:100108313-100108340 | AFF3-5401 | chr2:99688774-99688801 |
| AFF3-2409 | chr2:100108321-100108348 | AFF3-5402 | chr2:99688780-99688807 |
| AFF3-2410 | chr2:100108335-100108362 | AFF3-5403 | chr2:99688782-99688809 |
| AFF3-2411 | chr2:100108351-100108378 | AFF3-5404 | chr2:99688785-99688812 |
| AFF3-2412 | chr2:100108357-100108384 | AFF3-5405 | chr2:99688805-99688832 |
| AFF3-2413 | chr2:100108371-100108398 | AFF3-5406 | chr2:99688810-99688837 |
| AFF3-2414 | chr2:100108376-100108403 | AFF3-5407 | chr2:99688811-99688838 |
| AFF3-2415 | chr2:100108380-100108407 | AFF3-5408 | chr2:99688828-99688855 |
| AFF3-2416 | chr2:100108386-100108413 | AFF3-5409 | chr2:99688839-99688866 |
| AFF3-2417 | chr2:100108387-100108414 | AFF3-5410 | chr2:99688862-99688889 |
| AFF3-2418 | chr2:100108414-100108441 | AFF3-5411 | chr2:99688863-99688890 |
| AFF3-2419 | chr2:100108422-100108449 | AFF3-5412 | chr2:99688877-99688904 |
| AFF3-2420 | chr2:100108423-100108450 | AFF3-5413 | chr2:99688895-99688922 |
| AFF3-2421 | chr2:100108424-100108451 | AFF3-5414 | chr2:99688900-99688927 |
| AFF3-2422 | chr2:100108427-100108454 | AFF3-5415 | chr2:99688913-99688940 |
| AFF3-2423 | chr2:100108430-100108457 | AFF3-5416 | chr2:99688914-99688941 |
| AFF3-2424 | chr2:100108431-100108458 | AFF3-5417 | chr2:99688916-99688943 |
| AFF3-2425 | chr2:100108436-100108463 | AFF3-5418 | chr2:99688928-99688955 |
| AFF3-2426 | chr2:100108447-100108474 | AFF3-5419 | chr2:99688931-99688958 |
| AFF3-2427 | chr2:100108448-100108475 | AFF3-5420 | chr2:99688934-99688961 |
| AFF3-2428 | chr2:100108460-100108487 | AFF3-5421 | chr2:99688944-99688971 |
| AFF3-2429 | chr2:100108466-100108493 | AFF3-5422 | chr2:99688949-99688976 |
| AFF3-2430 | chr2:100108469-100108496 | AFF3-5423 | chr2:99688951-99688978 |
| AFF3-2431 | chr2:100108490-100108517 | AFF3-5424 | chr2:99688957-99688984 |
| AFF3-2432 | chr2:100108518-100108545 | AFF3-5425 | chr2:99688964-99688991 |
| AFF3-2433 | chr2:100108519-100108546 | AFF3-5426 | chr2:99688967-99688994 |
| AFF3-2434 | chr2:100108520-100108547 | AFF3-5427 | chr2:99688968-99688995 |
| AFF3-2435 | chr2:100108521-100108548 | AFF3-5428 | chr2:99688969-99688996 |
| AFF3-2436 | chr2:100108522-100108549 | AFF3-5429 | chr2:99688971-99688998 |
| AFF3-2437 | chr2:100108523-100108550 | AFF3-5430 | chr2:99688972-99688999 |
| AFF3-2438 | chr2:100108524-100108551 | AFF3-5431 | chr2:99688980-99689007 |
| AFF3-2439 | chr2:100108525-100108552 | AFF3-5432 | chr2:99688985-99689012 |
| AFF3-2440 | chr2:100108526-100108553 | AFF3-5433 | chr2:99688986-99689013 |
| AFF3-2441 | chr2:100108527-100108554 | AFF3-5434 | chr2:99688990-99689017 |
| AFF3-2442 | chr2:100108528-100108555 | AFF3-5435 | chr2:99688991-99689018 |
| AFF3-2443 | chr2:100108531-100108558 | AFF3-5436 | chr2:99688994-99689021 |
| AFF3-2444 | chr2:100108536-100108563 | AFF3-5437 | chr2:99689000-99689027 |
| AFF3-2445 | chr2:100108540-100108567 | AFF3-5438 | chr2:99689023-99689050 |
| AFF3-2446 | chr2:100108541-100108568 | AFF3-5439 | chr2:99689032-99689059 |
| AFF3-2447 | chr2:100108542-100108569 | AFF3-5440 | chr2:99689035-99689062 |
| AFF3-2448 | chr2:100108543-100108570 | AFF3-5441 | chr2:99689036-99689063 |
| AFF3-2449 | chr2:100108556-100108583 | AFF3-5442 | chr2:99689054-99689081 |
| AFF3-2450 | chr2:100108557-100108584 | AFF3-5443 | chr2:99689064-99689091 |
| AFF3-2451 | chr2:100108559-100108586 | AFF3-5444 | chr2:99689065-99689092 |
| AFF3-2452 | chr2:100108570-100108597 | AFF3-5445 | chr2:99689096-99689123 |
| AFF3-2453 | chr2:100108571-100108598 | AFF3-5446 | chr2:99689099-99689126 |
| AFF3-2454 | chr2:100108579-100108606 | AFF3-5447 | chr2:99689100-99689127 |
| AFF3-2455 | chr2:100108598-100108625 | AFF3-5448 | chr2:99689103-99689130 |
| AFF3-2456 | chr2:100108609-100108636 | AFF3-5449 | chr2:99689107-99689134 |
| AFF3-2457 | chr2:100108613-100108640 | AFF3-5450 | chr2:99689111-99689138 |
| AFF3-2458 | chr2:100108614-100108641 | AFF3-5451 | chr2:99689112-99689139 |
| AFF3-2459 | chr2:100108615-100108642 | AFF3-5452 | chr2:99689125-99689152 |
| AFF3-2460 | chr2:100108620-100108647 | AFF3-5453 | chr2:99689160-99689187 |
| AFF3-2461 | chr2:100108623-100108650 | AFF3-5454 | chr2:99689164-99689191 |
| AFF3-2462 | chr2:100108624-100108651 | AFF3-5455 | chr2:99689176-99689203 |
| AFF3-2463 | chr2:100108627-100108654 | AFF3-5456 | chr2:99689177-99689204 |
| AFF3-2464 | chr2:100108628-100108655 | AFF3-5457 | chr2:99689183-99689210 |
| AFF3-2465 | chr2:100108636-100108663 | AFF3-5458 | chr2:99689196-99689223 |
| AFF3-2466 | chr2:100108637-100108664 | AFF3-5459 | chr2:99689197-99689224 |
| AFF3-2467 | chr2:100108638-100108665 | AFF3-5460 | chr2:99689200-99689227 |
| AFF3-2468 | chr2:100108639-100108666 | AFF3-5461 | chr2:99689212-99689239 |
| AFF3-2469 | chr2:100108651-100108678 | AFF3-5462 | chr2:99689213-99689240 |
| AFF3-2470 | chr2:100108657-100108684 | AFF3-5463 | chr2:99689214-99689241 |
| AFF3-2471 | chr2:100108658-100108685 | AFF3-5464 | chr2:99689215-99689242 |
| AFF3-2472 | chr2:100108667-100108694 | AFF3-5465 | chr2:99689225-99689252 |
| AFF3-2473 | chr2:100108673-100108700 | AFF3-5466 | chr2:99689226-99689253 |
| AFF3-2474 | chr2:100108674-100108701 | AFF3-5467 | chr2:99689230-99689257 |
| AFF3-2475 | chr2:100108675-100108702 | AFF3-5468 | chr2:99689235-99689262 |
| AFF3-2476 | chr2:100108678-100108705 | AFF3-5469 | chr2:99689245-99689272 |
| AFF3-2477 | chr2:100108690-100108717 | AFF3-5470 | chr2:99689249-99689276 |
| AFF3-2478 | chr2:100108727-100108754 | AFF3-5471 | chr2:99689276-99689303 |
| AFF3-2479 | chr2:100108728-100108755 | AFF3-5472 | chr2:99689277-99689304 |
| AFF3-2480 | chr2:100108735-100108762 | AFF3-5473 | chr2:99689299-99689326 |
| AFF3-2481 | chr2:100108742-100108769 | AFF3-5474 | chr2:99689301-99689328 |
| AFF3-2482 | chr2:100108761-100108788 | AFF3-5475 | chr2:99689305-99689332 |
| AFF3-2483 | chr2:100108762-100108789 | AFF3-5476 | chr2:99689306-99689333 |
| AFF3-2484 | chr2:100108783-100108810 | AFF3-5477 | chr2:99689310-99689337 |
| AFF3-2485 | chr2:100108816-100108843 | AFF3-5478 | chr2:99689311-99689338 |
| AFF3-2486 | chr2:100108847-100108874 | AFF3-5479 | chr2:99689343-99689370 |
| AFF3-2487 | chr2:100108848-100108875 | AFF3-5480 | chr2:99689348-99689375 |
| AFF3-2488 | chr2:100108863-100108890 | AFF3-5481 | chr2:99689349-99689376 |
| AFF3-2489 | chr2:100108864-100108891 | AFF3-5482 | chr2:99689352-99689379 |
| AFF3-2490 | chr2:100108869-100108896 | AFF3-5483 | chr2:99689371-99689398 |
| AFF3-2491 | chr2:100108870-100108897 | AFF3-5484 | chr2:99689380-99689407 |
| AFF3-2492 | chr2:100108907-100108934 | AFF3-5485 | chr2:99689381-99689408 |
| AFF3-2493 | chr2:100108920-100108947 | AFF3-5486 | chr2:99689385-99689412 |
| AFF3-2494 | chr2:100108933-100108960 | AFF3-5487 | chr2:99689389-99689416 |
| AFF3-2495 | chr2:100108943-100108970 | AFF3-5488 | chr2:99689396-99689423 |
| AFF3-2496 | chr2:100108953-100108980 | AFF3-5489 | chr2:99689397-99689424 |
| AFF3-2497 | chr2:100108954-100108981 | AFF3-5490 | chr2:99689403-99689430 |
| AFF3-2498 | chr2:100108967-100108994 | AFF3-5491 | chr2:99689404-99689431 |
| AFF3-2499 | chr2:100108971-100108998 | AFF3-5492 | chr2:99689420-99689447 |
| AFF3-2500 | chr2:100108972-100108999 | AFF3-5493 | chr2:99689421-99689448 |
| AFF3-2501 | chr2:100108973-100109000 | AFF3-5494 | chr2:99689427-99689454 |
| AFF3-2502 | chr2:100108982-100109009 | AFF3-5495 | chr2:99689434-99689461 |
| AFF3-2503 | chr2:100108983-100109010 | AFF3-5496 | chr2:99689436-99689463 |
| AFF3-2504 | chr2:100108989-100109016 | AFF3-5497 | chr2:99689439-99689466 |
| AFF3-2505 | chr2:100108992-100109019 | AFF3-5498 | chr2:99689440-99689467 |
| AFF3-2506 | chr2:100108996-100109023 | AFF3-5499 | chr2:99689441-99689468 |
| AFF3-2507 | chr2:100109025-100109052 | AFF3-5500 | chr2:99689472-99689499 |
| AFF3-2508 | chr2:100109041-100109068 | AFF3-5501 | chr2:99689485-99689512 |
| AFF3-2509 | chr2:100109045-100109072 | AFF3-5502 | chr2:99689486-99689513 |
| AFF3-2510 | chr2:100109046-100109073 | AFF3-5503 | chr2:99689497-99689524 |
| AFF3-2511 | chr2:100109053-100109080 | AFF3-5504 | chr2:99689498-99689525 |
| AFF3-2512 | chr2:100109054-100109081 | AFF3-5505 | chr2:99689505-99689532 |
| AFF3-2513 | chr2:100109062-100109089 | AFF3-5506 | chr2:99689519-99689546 |
| AFF3-2514 | chr2:100109063-100109090 | AFF3-5507 | chr2:99689524-99689551 |
| AFF3-2515 | chr2:100109078-100109105 | AFF3-5508 | chr2:99689563-99689590 |
| AFF3-2516 | chr2:100109079-100109106 | AFF3-5509 | chr2:99689601-99689628 |
| AFF3-2517 | chr2:100109080-100109107 | AFF3-5510 | chr2:99689605-99689632 |
| AFF3-2518 | chr2:100109094-100109121 | AFF3-5511 | chr2:99689608-99689635 |
| AFF3-2519 | chr2:100109095-100109122 | AFF3-5512 | chr2:99689614-99689641 |
| AFF3-2520 | chr2:100109100-100109127 | AFF3-5513 | chr2:99689621-99689648 |
| AFF3-2521 | chr2:100109101-100109128 | AFF3-5514 | chr2:99689627-99689654 |
| AFF3-2522 | chr2:100109102-100109129 | AFF3-5515 | chr2:99689680-99689707 |
| AFF3-2523 | chr2:100109106-100109133 | AFF3-5516 | chr2:99689693-99689720 |
| AFF3-2524 | chr2:100109112-100109139 | AFF3-5517 | chr2:99689694-99689721 |
| AFF3-2525 | chr2:100109115-100109142 | AFF3-5518 | chr2:99689695-99689722 |
| AFF3-2526 | chr2:100109116-100109143 | AFF3-5519 | chr2:99689716-99689743 |
| AFF3-2527 | chr2:100109118-100109145 | AFF3-5520 | chr2:99689725-99689752 |
| AFF3-2528 | chr2:100109156-100109183 | AFF3-5521 | chr2:99689730-99689757 |
| AFF3-2529 | chr2:100109157-100109184 | AFF3-5522 | chr2:99689737-99689764 |
| AFF3-2530 | chr2:100109167-100109194 | AFF3-5523 | chr2:99689745-99689772 |
| AFF3-2531 | chr2:100109170-100109197 | AFF3-5524 | chr2:99689752-99689779 |
| AFF3-2532 | chr2:100109202-100109229 | AFF3-5525 | chr2:99689757-99689784 |
| AFF3-2533 | chr2:100109239-100109266 | AFF3-5526 | chr2:99689758-99689785 |
| AFF3-2534 | chr2:100109243-100109270 | AFF3-5527 | chr2:99689823-99689850 |
| AFF3-2535 | chr2:100109244-100109271 | AFF3-5528 | chr2:99689829-99689856 |
| AFF3-2536 | chr2:100109245-100109272 | AFF3-5529 | chr2:99689833-99689860 |
| AFF3-2537 | chr2:100109265-100109292 | AFF3-5530 | chr2:99689836-99689863 |
| AFF3-2538 | chr2:100109290-100109317 | AFF3-5531 | chr2:99689847-99689874 |
| AFF3-2539 | chr2:100109299-100109326 | AFF3-5532 | chr2:99689851-99689878 |
| AFF3-2540 | chr2:100109303-100109330 | AFF3-5533 | chr2:99689852-99689879 |
| AFF3-2541 | chr2:100109304-100109331 | AFF3-5534 | chr2:99689868-99689895 |
| AFF3-2542 | chr2:100109306-100109333 | AFF3-5535 | chr2:99689879-99689906 |
| AFF3-2543 | chr2:100109307-100109334 | AFF3-5536 | chr2:99689889-99689916 |
| AFF3-2544 | chr2:100109314-100109341 | AFF3-5537 | chr2:99689898-99689925 |
| AFF3-2545 | chr2:100109338-100109365 | AFF3-5538 | chr2:99689912-99689939 |
| AFF3-2546 | chr2:100109342-100109369 | AFF3-5539 | chr2:99689913-99689940 |
| AFF3-2547 | chr2:100109344-100109371 | AFF3-5540 | chr2:99689914-99689941 |
| AFF3-2548 | chr2:100109369-100109396 | AFF3-5541 | chr2:99689918-99689945 |
| AFF3-2549 | chr2:100109373-100109400 | AFF3-5542 | chr2:99689919-99689946 |
| AFF3-2550 | chr2:100109377-100109404 | AFF3-5543 | chr2:99689923-99689950 |
| AFF3-2551 | chr2:100109400-100109427 | AFF3-5544 | chr2:99689927-99689954 |
| AFF3-2552 | chr2:100109443-100109470 | AFF3-5545 | chr2:99689954-99689981 |
| AFF3-2553 | chr2:100109459-100109486 | AFF3-5546 | chr2:99689955-99689982 |
| AFF3-2554 | chr2:100109460-100109487 | AFF3-5547 | chr2:99689958-99689985 |
| AFF3-2555 | chr2:100109461-100109488 | AFF3-5548 | chr2:99689961-99689988 |
| AFF3-2556 | chr2:100109462-100109489 | AFF3-5549 | chr2:99689962-99689989 |
| AFF3-2557 | chr2:100109468-100109495 | AFF3-5550 | chr2:99689963-99689990 |
| AFF3-2558 | chr2:100109477-100109504 | AFF3-5551 | chr2:99689968-99689995 |
| AFF3-2559 | chr2:100109506-100109533 | AFF3-5552 | chr2:99689969-99689996 |
| AFF3-2560 | chr2:100109521-100109548 | AFF3-5553 | chr2:99689970-99689997 |
| AFF3-2561 | chr2:100109543-100109570 | AFF3-5554 | chr2:99689986-99690013 |
| AFF3-2562 | chr2:100109551-100109578 | AFF3-5555 | chr2:99689987-99690014 |
| AFF3-2563 | chr2:100109552-100109579 | AFF3-5556 | chr2:99689990-99690017 |
| AFF3-2564 | chr2:100109571-100109598 | AFF3-5557 | chr2:99689993-99690020 |
| AFF3-2565 | chr2:100109572-100109599 | AFF3-5558 | chr2:99689996-99690023 |
| AFF3-2566 | chr2:100109573-100109600 | AFF3-5559 | chr2:99690009-99690036 |
| AFF3-2567 | chr2:100109585-100109612 | AFF3-5560 | chr2:99690034-99690061 |
| AFF3-2568 | chr2:100109606-100109633 | AFF3-5561 | chr2:99690036-99690063 |
| AFF3-2569 | chr2:100109607-100109634 | AFF3-5562 | chr2:99690037-99690064 |
| AFF3-2570 | chr2:100109608-100109635 | AFF3-5563 | chr2:99690055-99690082 |
| AFF3-2571 | chr2:100109613-100109640 | AFF3-5564 | chr2:99690059-99690086 |
| AFF3-2572 | chr2:100109629-100109656 | AFF3-5565 | chr2:99690101-99690128 |
| AFF3-2573 | chr2:100109679-100109706 | AFF3-5566 | chr2:99690102-99690129 |
| AFF3-2574 | chr2:100109680-100109707 | AFF3-5567 | chr2:99690103-99690130 |
| AFF3-2575 | chr2:100109729-100109756 | AFF3-5568 | chr2:99690104-99690131 |
| AFF3-2576 | chr2:100109730-100109757 | AFF3-5569 | chr2:99690105-99690132 |
| AFF3-2577 | chr2:100109738-100109765 | AFF3-5570 | chr2:99690106-99690133 |
| AFF3-2578 | chr2:100109741-100109768 | AFF3-5571 | chr2:99690109-99690136 |
| AFF3-2579 | chr2:100109742-100109769 | AFF3-5572 | chr2:99690110-99690137 |
| AFF3-2580 | chr2:100109758-100109785 | AFF3-5573 | chr2:99690111-99690138 |
| AFF3-2581 | chr2:100109765-100109792 | AFF3-5574 | chr2:99690112-99690139 |
| AFF3-2582 | chr2:100109772-100109799 | AFF3-5575 | chr2:99690121-99690148 |
| AFF3-2583 | chr2:100109778-100109805 | AFF3-5576 | chr2:99690122-99690149 |
| AFF3-2584 | chr2:100109802-100109829 | AFF3-5577 | chr2:99690123-99690150 |
| AFF3-2585 | chr2:100109811-100109838 | AFF3-5578 | chr2:99690131-99690158 |
| AFF3-2586 | chr2:100109817-100109844 | AFF3-5579 | chr2:99690147-99690174 |
| AFF3-2587 | chr2:100109819-100109846 | AFF3-5580 | chr2:99690199-99690226 |
| AFF3-2588 | chr2:100109820-100109847 | AFF3-5581 | chr2:99690203-99690230 |
| AFF3-2589 | chr2:100109822-100109849 | AFF3-5582 | chr2:99690213-99690240 |
| AFF3-2590 | chr2:100109831-100109858 | AFF3-5583 | chr2:99690221-99690248 |
| AFF3-2591 | chr2:100109870-100109897 | AFF3-5584 | chr2:99690222-99690249 |
| AFF3-2592 | chr2:100109877-100109904 | AFF3-5585 | chr2:99690224-99690251 |
| AFF3-2593 | chr2:100109880-100109907 | AFF3-5586 | chr2:99690248-99690275 |
| AFF3-2594 | chr2:100109883-100109910 | AFF3-5587 | chr2:99690249-99690276 |
| AFF3-2595 | chr2:100109884-100109911 | AFF3-5588 | chr2:99690265-99690292 |
| AFF3-2596 | chr2:100109890-100109917 | AFF3-5589 | chr2:99690268-99690295 |
| AFF3-2597 | chr2:100109896-100109923 | AFF3-5590 | chr2:99690271-99690298 |
| AFF3-2598 | chr2:100109910-100109937 | AFF3-5591 | chr2:99690272-99690299 |
| AFF3-2599 | chr2:100109945-100109972 | AFF3-5592 | chr2:99690283-99690310 |
| AFF3-2600 | chr2:100109949-100109976 | AFF3-5593 | chr2:99690288-99690315 |
| AFF3-2601 | chr2:100109964-100109991 | AFF3-5594 | chr2:99690289-99690316 |
| AFF3-2602 | chr2:100109965-100109992 | AFF3-5595 | chr2:99690290-99690317 |
| AFF3-2603 | chr2:100109987-100110014 | AFF3-5596 | chr2:99690294-99690321 |
| AFF3-2604 | chr2:100109988-100110015 | AFF3-5597 | chr2:99690297-99690324 |
| AFF3-2605 | chr2:100109994-100110021 | AFF3-5598 | chr2:99690300-99690327 |
| AFF3-2606 | chr2:100109995-100110022 | AFF3-5599 | chr2:99690303-99690330 |
| AFF3-2607 | chr2:100109996-100110023 | AFF3-5600 | chr2:99690304-99690331 |
| AFF3-2608 | chr2:100110011-100110038 | AFF3-5601 | chr2:99690316-99690343 |
| AFF3-2609 | chr2:100110015-100110042 | AFF3-5602 | chr2:99690327-99690354 |
| AFF3-2610 | chr2:100110018-100110045 | AFF3-5603 | chr2:99690328-99690355 |
| AFF3-2611 | chr2:100110019-100110046 | AFF3-5604 | chr2:99690331-99690358 |
| AFF3-2612 | chr2:100110028-100110055 | AFF3-5605 | chr2:99690334-99690361 |
| AFF3-2613 | chr2:100110029-100110056 | AFF3-5606 | chr2:99690339-99690366 |
| AFF3-2614 | chr2:100110030-100110057 | AFF3-5607 | chr2:99690340-99690367 |
| AFF3-2615 | chr2:100110037-100110064 | AFF3-5608 | chr2:99690346-99690373 |
| AFF3-2616 | chr2:100110042-100110069 | AFF3-5609 | chr2:99690347-99690374 |
| AFF3-2617 | chr2:100110048-100110075 | AFF3-5610 | chr2:99690348-99690375 |
| AFF3-2618 | chr2:100110053-100110080 | AFF3-5611 | chr2:99690367-99690394 |
| AFF3-2619 | chr2:100110069-100110096 | AFF3-5612 | chr2:99690371-99690398 |
| AFF3-2620 | chr2:100110081-100110108 | AFF3-5613 | chr2:99690381-99690408 |
| AFF3-2621 | chr2:100110084-100110111 | AFF3-5614 | chr2:99690390-99690417 |
| AFF3-2622 | chr2:100110088-100110115 | AFF3-5615 | chr2:99690407-99690434 |
| AFF3-2623 | chr2:100110093-100110120 | AFF3-5616 | chr2:99690408-99690435 |
| AFF3-2624 | chr2:100110094-100110121 | AFF3-5617 | chr2:99690413-99690440 |
| AFF3-2625 | chr2:100110146-100110173 | AFF3-5618 | chr2:99690422-99690449 |
| AFF3-2626 | chr2:100110188-100110215 | AFF3-5619 | chr2:99690423-99690450 |
| AFF3-2627 | chr2:100110196-100110223 | AFF3-5620 | chr2:99690424-99690451 |
| AFF3-2628 | chr2:100110197-100110224 | AFF3-5621 | chr2:99690425-99690452 |
| AFF3-2629 | chr2:100110207-100110234 | AFF3-5622 | chr2:99690426-99690453 |
| AFF3-2630 | chr2:100110219-100110246 | AFF3-5623 | chr2:99690429-99690456 |
| AFF3-2631 | chr2:100110220-100110247 | AFF3-5624 | chr2:99690432-99690459 |
| AFF3-2632 | chr2:100110237-100110264 | AFF3-5625 | chr2:99690435-99690462 |
| AFF3-2633 | chr2:100110241-100110268 | AFF3-5626 | chr2:99690438-99690465 |
| AFF3-2634 | chr2:100110247-100110274 | AFF3-5627 | chr2:99690439-99690466 |
| AFF3-2635 | chr2:100110254-100110281 | AFF3-5628 | chr2:99690451-99690478 |
| AFF3-2636 | chr2:100110270-100110297 | AFF3-5629 | chr2:99690466-99690493 |
| AFF3-2637 | chr2:100110272-100110299 | AFF3-5630 | chr2:99690469-99690496 |
| AFF3-2638 | chr2:100110273-100110300 | AFF3-5631 | chr2:99690479-99690506 |
| AFF3-2639 | chr2:100110278-100110305 | AFF3-5632 | chr2:99690486-99690513 |
| AFF3-2640 | chr2:100110293-100110320 | AFF3-5633 | chr2:99690503-99690530 |
| AFF3-2641 | chr2:100110327-100110354 | AFF3-5634 | chr2:99690508-99690535 |
| AFF3-2642 | chr2:100110334-100110361 | AFF3-5635 | chr2:99690512-99690539 |
| AFF3-2643 | chr2:100110346-100110373 | AFF3-5636 | chr2:99690513-99690540 |
| AFF3-2644 | chr2:100110352-100110379 | AFF3-5637 | chr2:99690531-99690558 |
| AFF3-2645 | chr2:100110366-100110393 | AFF3-5638 | chr2:99690549-99690576 |
| AFF3-2646 | chr2:100110371-100110398 | AFF3-5639 | chr2:99690553-99690580 |
| AFF3-2647 | chr2:100110372-100110399 | AFF3-5640 | chr2:99690554-99690581 |
| AFF3-2648 | chr2:100110373-100110400 | AFF3-5641 | chr2:99690563-99690590 |
| AFF3-2649 | chr2:100110374-100110401 | AFF3-5642 | chr2:99690566-99690593 |
| AFF3-2650 | chr2:100110382-100110409 | AFF3-5643 | chr2:99690584-99690611 |
| AFF3-2651 | chr2:100110383-100110410 | AFF3-5644 | chr2:99690594-99690621 |
| AFF3-2652 | chr2:100110384-100110411 | AFF3-5645 | chr2:99690602-99690629 |
| AFF3-2653 | chr2:100110407-100110434 | AFF3-5646 | chr2:99690603-99690630 |
| AFF3-2654 | chr2:100110408-100110435 | AFF3-5647 | chr2:99690605-99690632 |
| AFF3-2655 | chr2:100110415-100110442 | AFF3-5648 | chr2:99690606-99690633 |
| AFF3-2656 | chr2:100110428-100110455 | AFF3-5649 | chr2:99690673-99690700 |
| AFF3-2657 | chr2:100110429-100110456 | AFF3-5650 | chr2:99690684-99690711 |
| AFF3-2658 | chr2:100110430-100110457 | AFF3-5651 | chr2:99690702-99690729 |
| AFF3-2659 | chr2:100110453-100110480 | AFF3-5652 | chr2:99690713-99690740 |
| AFF3-2660 | chr2:100110458-100110485 | AFF3-5653 | chr2:99690714-99690741 |
| AFF3-2661 | chr2:100110460-100110487 | AFF3-5654 | chr2:99690722-99690749 |
| AFF3-2662 | chr2:100110479-100110506 | AFF3-5655 | chr2:99690744-99690771 |
| AFF3-2663 | chr2:100110482-100110509 | AFF3-5656 | chr2:99690765-99690792 |
| AFF3-2664 | chr2:100110490-100110517 | AFF3-5657 | chr2:99690766-99690793 |
| AFF3-2665 | chr2:100110491-100110518 | AFF3-5658 | chr2:99690808-99690835 |
| AFF3-2666 | chr2:100110492-100110519 | AFF3-5659 | chr2:99690816-99690843 |
| AFF3-2667 | chr2:100110500-100110527 | AFF3-5660 | chr2:99690841-99690868 |
| AFF3-2668 | chr2:100110501-100110528 | AFF3-5661 | chr2:99690844-99690871 |
| AFF3-2669 | chr2:100110512-100110539 | AFF3-5662 | chr2:99690845-99690872 |
| AFF3-2670 | chr2:100110513-100110540 | AFF3-5663 | chr2:99690848-99690875 |
| AFF3-2671 | chr2:100110530-100110557 | AFF3-5664 | chr2:99690849-99690876 |
| AFF3-2672 | chr2:100110547-100110574 | AFF3-5665 | chr2:99690850-99690877 |
| AFF3-2673 | chr2:100110557-100110584 | AFF3-5666 | chr2:99690864-99690891 |
| AFF3-2674 | chr2:100110558-100110585 | AFF3-5667 | chr2:99690869-99690896 |
| AFF3-2675 | chr2:100110565-100110592 | AFF3-5668 | chr2:99690876-99690903 |
| AFF3-2676 | chr2:100110569-100110596 | AFF3-5669 | chr2:99690882-99690909 |
| AFF3-2677 | chr2:100110572-100110599 | AFF3-5670 | chr2:99690883-99690910 |
| AFF3-2678 | chr2:100110579-100110606 | AFF3-5671 | chr2:99690884-99690911 |
| AFF3-2679 | chr2:100110580-100110607 | AFF3-5672 | chr2:99690888-99690915 |
| AFF3-2680 | chr2:100110601-100110628 | AFF3-5673 | chr2:99690900-99690927 |
| AFF3-2681 | chr2:100110610-100110637 | AFF3-5674 | chr2:99690903-99690930 |
| AFF3-2682 | chr2:100110616-100110643 | AFF3-5675 | chr2:99690904-99690931 |
| AFF3-2683 | chr2:100110619-100110646 | AFF3-5676 | chr2:99690914-99690941 |
| AFF3-2684 | chr2:100110620-100110647 | AFF3-5677 | chr2:99690922-99690949 |
| AFF3-2685 | chr2:100110621-100110648 | AFF3-5678 | chr2:99690923-99690950 |
| AFF3-2686 | chr2:100110630-100110657 | AFF3-5679 | chr2:99690937-99690964 |
| AFF3-2687 | chr2:100110644-100110671 | AFF3-5680 | chr2:99690947-99690974 |
| AFF3-2688 | chr2:100110645-100110672 | AFF3-5681 | chr2:99690959-99690986 |
| AFF3-2689 | chr2:100110646-100110673 | AFF3-5682 | chr2:99690960-99690987 |
| AFF3-2690 | chr2:100110647-100110674 | AFF3-5683 | chr2:99690968-99690995 |
| AFF3-2691 | chr2:100110649-100110676 | AFF3-5684 | chr2:99690970-99690997 |
| AFF3-2692 | chr2:100110650-100110677 | AFF3-5685 | chr2:99690980-99691007 |
| AFF3-2693 | chr2:100110664-100110691 | AFF3-5686 | chr2:99690983-99691010 |
| AFF3-2694 | chr2:100110676-100110703 | AFF3-5687 | chr2:99690984-99691011 |
| AFF3-2695 | chr2:100110677-100110704 | AFF3-5688 | chr2:99691013-99691040 |
| AFF3-2696 | chr2:100110678-100110705 | AFF3-5689 | chr2:99691014-99691041 |
| AFF3-2697 | chr2:100110684-100110711 | AFF3-5690 | chr2:99691015-99691042 |
| AFF3-2698 | chr2:100110685-100110712 | AFF3-5691 | chr2:99691023-99691050 |
| AFF3-2699 | chr2:100110698-100110725 | AFF3-5692 | chr2:99691028-99691055 |
| AFF3-2700 | chr2:100110716-100110743 | AFF3-5693 | chr2:99691029-99691056 |
| AFF3-2701 | chr2:100110733-100110760 | AFF3-5694 | chr2:99691037-99691064 |
| AFF3-2702 | chr2:100110737-100110764 | AFF3-5695 | chr2:99691043-99691070 |
| AFF3-2703 | chr2:100110768-100110795 | AFF3-5696 | chr2:99691044-99691071 |
| AFF3-2704 | chr2:100110769-100110796 | AFF3-5697 | chr2:99691052-99691079 |
| AFF3-2705 | chr2:100110772-100110799 | AFF3-5698 | chr2:99691071-99691098 |
| AFF3-2706 | chr2:100110773-100110800 | AFF3-5699 | chr2:99691075-99691102 |
| AFF3-2707 | chr2:100110780-100110807 | AFF3-5700 | chr2:99691099-99691126 |
| AFF3-2708 | chr2:100110791-100110818 | AFF3-5701 | chr2:99691107-99691134 |
| AFF3-2709 | chr2:100110794-100110821 | AFF3-5702 | chr2:99691112-99691139 |
| AFF3-2710 | chr2:100110796-100110823 | AFF3-5703 | chr2:99691113-99691140 |
| AFF3-2711 | chr2:100110797-100110824 | AFF3-5704 | chr2:99691115-99691142 |
| AFF3-2712 | chr2:100110798-100110825 | AFF3-5705 | chr2:99691116-99691143 |
| AFF3-2713 | chr2:100110808-100110835 | AFF3-5706 | chr2:99691121-99691148 |
| AFF3-2714 | chr2:100110836-100110863 | AFF3-5707 | chr2:99691145-99691172 |
| AFF3-2715 | chr2:100110837-100110864 | AFF3-5708 | chr2:99691150-99691177 |
| AFF3-2716 | chr2:100110857-100110884 | AFF3-5709 | chr2:99691153-99691180 |
| AFF3-2717 | chr2:100110858-100110885 | AFF3-5710 | chr2:99707002-99707029 |
| AFF3-2718 | chr2:100110876-100110903 | AFF3-5711 | chr2:99707021-99707048 |
| AFF3-2719 | chr2:100110904-100110931 | AFF3-5712 | chr2:99707030-99707057 |
| AFF3-2720 | chr2:100110906-100110933 | AFF3-5713 | chr2:99707037-99707064 |
| AFF3-2721 | chr2:100110915-100110942 | AFF3-5714 | chr2:99707038-99707065 |
| AFF3-2722 | chr2:100110916-100110943 | AFF3-5715 | chr2:99707039-99707066 |
| AFF3-2723 | chr2:100110955-100110982 | AFF3-5716 | chr2:99707054-99707081 |
| AFF3-2724 | chr2:100110956-100110983 | AFF3-5717 | chr2:99707069-99707096 |
| AFF3-2725 | chr2:100110958-100110985 | AFF3-5718 | chr2:99707087-99707114 |
| AFF3-2726 | chr2:100110969-100110996 | AFF3-5719 | chr2:99707091-99707118 |
| AFF3-2727 | chr2:100110972-100110999 | AFF3-5720 | chr2:99707103-99707130 |
| AFF3-2728 | chr2:100110973-100111000 | AFF3-5721 | chr2:99707104-99707131 |
| AFF3-2729 | chr2:100110974-100111001 | AFF3-5722 | chr2:99707105-99707132 |
| AFF3-2730 | chr2:100110978-100111005 | AFF3-5723 | chr2:99707121-99707148 |
| AFF3-2731 | chr2:100110985-100111012 | AFF3-5724 | chr2:99707159-99707186 |
| AFF3-2732 | chr2:100110988-100111015 | AFF3-5725 | chr2:99707160-99707187 |
| AFF3-2733 | chr2:100110999-100111026 | AFF3-5726 | chr2:99707172-99707199 |
| AFF3-2734 | chr2:100111004-100111031 | AFF3-5727 | chr2:99707181-99707208 |
| AFF3-2735 | chr2:100111010-100111037 | AFF3-5728 | chr2:99707196-99707223 |
| AFF3-2736 | chr2:100111021-100111048 | AFF3-5729 | chr2:99707197-99707224 |
| AFF3-2737 | chr2:100111035-100111062 | AFF3-5730 | chr2:99707198-99707225 |
| AFF3-2738 | chr2:100111046-100111073 | AFF3-5731 | chr2:99707202-99707229 |
| AFF3-2739 | chr2:100111054-100111081 | AFF3-5732 | chr2:99707216-99707243 |
| AFF3-2740 | chr2:100111057-100111084 | AFF3-5733 | chr2:99707222-99707249 |
| AFF3-2741 | chr2:100111062-100111089 | AFF3-5734 | chr2:99707238-99707265 |
| AFF3-2742 | chr2:100111091-100111118 | AFF3-5735 | chr2:99707253-99707280 |
| AFF3-2743 | chr2:100111100-100111127 | AFF3-5736 | chr2:99707270-99707297 |
| AFF3-2744 | chr2:100111113-100111140 | AFF3-5737 | chr2:99707271-99707298 |
| AFF3-2745 | chr2:100111114-100111141 | AFF3-5738 | chr2:99707353-99707380 |
| AFF3-2746 | chr2:100111118-100111145 | AFF3-5739 | chr2:99707354-99707381 |
| AFF3-2747 | chr2:100111120-100111147 | AFF3-5740 | chr2:99707362-99707389 |
| AFF3-2748 | chr2:100111129-100111156 | AFF3-5741 | chr2:99707380-99707407 |
| AFF3-2749 | chr2:100111133-100111160 | AFF3-5742 | chr2:99707414-99707441 |
| AFF3-2750 | chr2:100111137-100111164 | AFF3-5743 | chr2:99707434-99707461 |
| AFF3-2751 | chr2:100111138-100111165 | AFF3-5744 | chr2:99707449-99707476 |
| AFF3-2752 | chr2:100111151-100111178 | AFF3-5745 | chr2:99707481-99707508 |
| AFF3-2753 | chr2:100111168-100111195 | AFF3-5746 | chr2:99707498-99707525 |
| AFF3-2754 | chr2:100111169-100111196 | AFF3-5747 | chr2:99707529-99707556 |
| AFF3-2755 | chr2:100111170-100111197 | AFF3-5748 | chr2:99707549-99707576 |
| AFF3-2756 | chr2:100111184-100111211 | AFF3-5749 | chr2:99707575-99707602 |
| AFF3-2757 | chr2:100111217-100111244 | AFF3-5750 | chr2:99707576-99707603 |
| AFF3-2758 | chr2:100111218-100111245 | AFF3-5751 | chr2:99707577-99707604 |
| AFF3-2759 | chr2:100111237-100111264 | AFF3-5752 | chr2:99707578-99707605 |
| AFF3-2760 | chr2:100111238-100111265 | AFF3-5753 | chr2:99707619-99707646 |
| AFF3-2761 | chr2:100111248-100111275 | AFF3-5754 | chr2:99707634-99707661 |
| AFF3-2762 | chr2:100111249-100111276 | AFF3-5755 | chr2:99707642-99707669 |
| AFF3-2763 | chr2:100111250-100111277 | AFF3-5756 | chr2:99707645-99707672 |
| AFF3-2764 | chr2:100111251-100111278 | AFF3-5757 | chr2:99707652-99707679 |
| AFF3-2765 | chr2:100111261-100111288 | AFF3-5758 | chr2:99707657-99707684 |
| AFF3-2766 | chr2:100111280-100111307 | AFF3-5759 | chr2:99707663-99707690 |
| AFF3-2767 | chr2:100111282-100111309 | AFF3-5760 | chr2:99707694-99707721 |
| AFF3-2768 | chr2:100111283-100111310 | AFF3-5761 | chr2:99707696-99707723 |
| AFF3-2769 | chr2:100111284-100111311 | AFF3-5762 | chr2:99707698-99707725 |
| AFF3-2770 | chr2:100111285-100111312 | AFF3-5763 | chr2:99707708-99707735 |
| AFF3-2771 | chr2:100111288-100111315 | AFF3-5764 | chr2:99707724-99707751 |
| AFF3-2772 | chr2:100111289-100111316 | AFF3-5765 | chr2:99707759-99707786 |
| AFF3-2773 | chr2:100111293-100111320 | AFF3-5766 | chr2:99707763-99707790 |
| AFF3-2774 | chr2:100111301-100111328 | AFF3-5767 | chr2:99707767-99707794 |
| AFF3-2775 | chr2:100111302-100111329 | AFF3-5768 | chr2:99707774-99707801 |
| AFF3-2776 | chr2:100111303-100111330 | AFF3-5769 | chr2:99707790-99707817 |
| AFF3-2777 | chr2:100111321-100111348 | AFF3-5770 | chr2:99707794-99707821 |
| AFF3-2778 | chr2:100111349-100111376 | AFF3-5771 | chr2:99707829-99707856 |
| AFF3-2779 | chr2:100111362-100111389 | AFF3-5772 | chr2:99707835-99707862 |
| AFF3-2780 | chr2:100111397-100111424 | AFF3-5773 | chr2:99707855-99707882 |
| AFF3-2781 | chr2:100111434-100111461 | AFF3-5774 | chr2:99707871-99707898 |
| AFF3-2782 | chr2:100111464-100111491 | AFF3-5775 | chr2:99707872-99707899 |
| AFF3-2783 | chr2:100111483-100111510 | AFF3-5776 | chr2:99707878-99707905 |
| AFF3-2784 | chr2:100111499-100111526 | AFF3-5777 | chr2:99707879-99707906 |
| AFF3-2785 | chr2:100112265-100112292 | AFF3-5778 | chr2:99707880-99707907 |
| AFF3-2786 | chr2:100112266-100112293 | AFF3-5779 | chr2:99707881-99707908 |
| AFF3-2787 | chr2:100112291-100112318 | AFF3-5780 | chr2:99707895-99707922 |
| AFF3-2788 | chr2:100112302-100112329 | AFF3-5781 | chr2:99707896-99707923 |
| AFF3-2789 | chr2:100112319-100112346 | AFF3-5782 | chr2:99707905-99707932 |
| AFF3-2790 | chr2:100112324-100112351 | AFF3-5783 | chr2:99707924-99707951 |
| AFF3-2791 | chr2:100112327-100112354 | AFF3-5784 | chr2:99707928-99707955 |
| AFF3-2792 | chr2:100112331-100112358 | AFF3-5785 | chr2:99707931-99707958 |
| AFF3-2793 | chr2:100112336-100112363 | AFF3-5786 | chr2:99707936-99707963 |
| AFF3-2794 | chr2:100112337-100112364 | AFF3-5787 | chr2:99707948-99707975 |
| AFF3-2795 | chr2:100112341-100112368 | AFF3-5788 | chr2:99707959-99707986 |
| AFF3-2796 | chr2:100112362-100112389 | AFF3-5789 | chr2:99707963-99707990 |
| AFF3-2797 | chr2:100112363-100112390 | AFF3-5790 | chr2:99707964-99707991 |
| AFF3-2798 | chr2:100112364-100112391 | AFF3-5791 | chr2:99707993-99708020 |
| AFF3-2799 | chr2:100112373-100112400 | AFF3-5792 | chr2:99708019-99708046 |
| AFF3-2800 | chr2:100112374-100112401 | AFF3-5793 | chr2:99708024-99708051 |
| AFF3-2801 | chr2:100112380-100112407 | AFF3-5794 | chr2:99708080-99708107 |
| AFF3-2802 | chr2:100112381-100112408 | AFF3-5795 | chr2:99708081-99708108 |
| AFF3-2803 | chr2:100112384-100112411 | AFF3-5796 | chr2:99708108-99708135 |
| AFF3-2804 | chr2:100112385-100112412 | AFF3-5797 | chr2:99708109-99708136 |
| AFF3-2805 | chr2:100112390-100112417 | AFF3-5798 | chr2:99708160-99708187 |
| AFF3-2806 | chr2:100112395-100112422 | AFF3-5799 | chr2:99708161-99708188 |
| AFF3-2807 | chr2:100112398-100112425 | AFF3-5800 | chr2:99708180-99708207 |
| AFF3-2808 | chr2:100112413-100112440 | AFF3-5801 | chr2:99708194-99708221 |
| AFF3-2809 | chr2:100112429-100112456 | AFF3-5802 | chr2:99708197-99708224 |
| AFF3-2810 | chr2:100112432-100112459 | AFF3-5803 | chr2:99708205-99708232 |
| AFF3-2811 | chr2:100112435-100112462 | AFF3-5804 | chr2:99708209-99708236 |
| AFF3-2812 | chr2:100112439-100112466 | AFF3-5805 | chr2:99708210-99708237 |
| AFF3-2813 | chr2:100112440-100112467 | AFF3-5806 | chr2:99708229-99708256 |
| AFF3-2814 | chr2:100112441-100112468 | AFF3-5807 | chr2:99708249-99708276 |
| AFF3-2815 | chr2:100112442-100112469 | AFF3-5808 | chr2:99708260-99708287 |
| AFF3-2816 | chr2:100112457-100112484 | AFF3-5809 | chr2:99708272-99708299 |
| AFF3-2817 | chr2:100112475-100112502 | AFF3-5810 | chr2:99708291-99708318 |
| AFF3-2818 | chr2:100112476-100112503 | AFF3-5811 | chr2:99708296-99708323 |
| AFF3-2819 | chr2:100112480-100112507 | AFF3-5812 | chr2:99708301-99708328 |
| AFF3-2820 | chr2:100112484-100112511 | AFF3-5813 | chr2:99708342-99708369 |
| AFF3-2821 | chr2:100112498-100112525 | AFF3-5814 | chr2:99708364-99708391 |
| AFF3-2822 | chr2:100112517-100112544 | AFF3-5815 | chr2:99708365-99708392 |
| AFF3-2823 | chr2:100112518-100112545 | AFF3-5816 | chr2:99708387-99708414 |
| AFF3-2824 | chr2:100112522-100112549 | AFF3-5817 | chr2:99708404-99708431 |
| AFF3-2825 | chr2:100112523-100112550 | AFF3-5818 | chr2:99708405-99708432 |
| AFF3-2826 | chr2:100112524-100112551 | AFF3-5819 | chr2:99708411-99708438 |
| AFF3-2827 | chr2:100112528-100112555 | AFF3-5820 | chr2:99708421-99708448 |
| AFF3-2828 | chr2:100112531-100112558 | AFF3-5821 | chr2:99708422-99708449 |
| AFF3-2829 | chr2:100112550-100112577 | AFF3-5822 | chr2:99708426-99708453 |
| AFF3-2830 | chr2:100112562-100112589 | AFF3-5823 | chr2:99708427-99708454 |
| AFF3-2831 | chr2:100112568-100112595 | AFF3-5824 | chr2:99708428-99708455 |
| AFF3-2832 | chr2:100112571-100112598 | AFF3-5825 | chr2:99708433-99708460 |
| AFF3-2833 | chr2:100112572-100112599 | AFF3-5826 | chr2:99708452-99708479 |
| AFF3-2834 | chr2:100112573-100112600 | AFF3-5827 | chr2:99708456-99708483 |
| AFF3-2835 | chr2:100112574-100112601 | AFF3-5828 | chr2:99708461-99708488 |
| AFF3-2836 | chr2:100112575-100112602 | AFF3-5829 | chr2:99708469-99708496 |
| AFF3-2837 | chr2:100112599-100112626 | AFF3-5830 | chr2:99708470-99708497 |
| AFF3-2838 | chr2:100112615-100112642 | AFF3-5831 | chr2:99708477-99708504 |
| AFF3-2839 | chr2:100129102-100129129 | AFF3-5832 | chr2:99708481-99708508 |
| AFF3-2840 | chr2:100129116-100129143 | AFF3-5833 | chr2:99708482-99708509 |
| AFF3-2841 | chr2:100129153-100129180 | AFF3-5834 | chr2:99708483-99708510 |
| AFF3-2842 | chr2:100129154-100129181 | AFF3-5835 | chr2:99708484-99708511 |
| AFF3-2843 | chr2:100129155-100129182 | AFF3-5836 | chr2:99708499-99708526 |
| AFF3-2844 | chr2:100129167-100129194 | AFF3-5837 | chr2:99708531-99708558 |
| AFF3-2845 | chr2:100129168-100129195 | AFF3-5838 | chr2:99708547-99708574 |
| AFF3-2846 | chr2:100129169-100129196 | AFF3-5839 | chr2:99708558-99708585 |
| AFF3-2847 | chr2:100129216-100129243 | AFF3-5840 | chr2:99708564-99708591 |
| AFF3-2848 | chr2:100129223-100129250 | AFF3-5841 | chr2:99708595-99708622 |
| AFF3-2849 | chr2:100129231-100129258 | AFF3-5842 | chr2:99708596-99708623 |
| AFF3-2850 | chr2:100129240-100129267 | AFF3-5843 | chr2:99708597-99708624 |
| AFF3-2851 | chr2:100129243-100129270 | AFF3-5844 | chr2:99708605-99708632 |
| AFF3-2852 | chr2:100129254-100129281 | AFF3-5845 | chr2:99708662-99708689 |
| AFF3-2853 | chr2:100129255-100129282 | AFF3-5846 | chr2:99708663-99708690 |
| AFF3-2854 | chr2:100129300-100129327 | AFF3-5847 | chr2:99708686-99708713 |
| AFF3-2855 | chr2:100129301-100129328 | AFF3-5848 | chr2:99708687-99708714 |
| AFF3-2856 | chr2:100129309-100129336 | AFF3-5849 | chr2:99708699-99708726 |
| AFF3-2857 | chr2:100129338-100129365 | AFF3-5850 | chr2:99708700-99708727 |
| AFF3-2858 | chr2:100129360-100129387 | AFF3-5851 | chr2:99708776-99708803 |
| AFF3-2859 | chr2:100129361-100129388 | AFF3-5852 | chr2:99708806-99708833 |
| AFF3-2860 | chr2:100129373-100129400 | AFF3-5853 | chr2:99708809-99708836 |
| AFF3-2861 | chr2:100142360-100142387 | AFF3-5854 | chr2:99708810-99708837 |
| AFF3-2862 | chr2:100142361-100142388 | AFF3-5855 | chr2:99708828-99708855 |
| AFF3-2863 | chr2:100142366-100142393 | AFF3-5856 | chr2:99708829-99708856 |
| AFF3-2864 | chr2:100142382-100142409 | AFF3-5857 | chr2:99708850-99708877 |
| AFF3-2865 | chr2:100142394-100142421 | AFF3-5858 | chr2:99708858-99708885 |
| AFF3-2866 | chr2:100142395-100142422 | AFF3-5859 | chr2:99708872-99708899 |
| AFF3-2867 | chr2:100142396-100142423 | AFF3-5860 | chr2:99708902-99708929 |
| AFF3-2868 | chr2:100142398-100142425 | AFF3-5861 | chr2:99708905-99708932 |
| AFF3-2869 | chr2:100142401-100142428 | AFF3-5862 | chr2:99708929-99708956 |
| AFF3-2870 | chr2:100142402-100142429 | AFF3-5863 | chr2:99708972-99708999 |
| AFF3-2871 | chr2:100142412-100142439 | AFF3-5864 | chr2:99708981-99709008 |
| AFF3-2872 | chr2:100142417-100142444 | AFF3-5865 | chr2:99708996-99709023 |
| AFF3-2873 | chr2:100142427-100142454 | AFF3-5866 | chr2:99708997-99709024 |
| AFF3-2874 | chr2:100142428-100142455 | AFF3-5867 | chr2:99708998-99709025 |
| AFF3-2875 | chr2:100142429-100142456 | AFF3-5868 | chr2:99708999-99709026 |
| AFF3-2876 | chr2:100142435-100142462 | AFF3-5869 | chr2:99709005-99709032 |
| AFF3-2877 | chr2:100142448-100142475 | AFF3-5870 | chr2:99709006-99709033 |
| AFF3-2878 | chr2:100142457-100142484 | AFF3-5871 | chr2:99709022-99709049 |
| AFF3-2879 | chr2:100142458-100142485 | AFF3-5872 | chr2:99709042-99709069 |
| AFF3-2880 | chr2:100142465-100142492 | AFF3-5873 | chr2:99726965-99726992 |
| AFF3-2881 | chr2:100142466-100142493 | AFF3-5874 | chr2:99726975-99727002 |
| AFF3-2882 | chr2:100142469-100142496 | AFF3-5875 | chr2:99726979-99727006 |
| AFF3-2883 | chr2:100142483-100142510 | AFF3-5876 | chr2:99727003-99727030 |
| AFF3-2884 | chr2:100142493-100142520 | AFF3-5877 | chr2:99727019-99727046 |
| AFF3-2885 | chr2:100142494-100142521 | AFF3-5878 | chr2:99727075-99727102 |
| AFF3-2886 | chr2:100142499-100142526 | AFF3-5879 | chr2:99727084-99727111 |
| AFF3-2887 | chr2:100142513-100142540 | AFF3-5880 | chr2:99727092-99727119 |
| AFF3-2888 | chr2:100142514-100142541 | AFF3-5881 | chr2:99727111-99727138 |
| AFF3-2889 | chr2:100142515-100142542 | AFF3-5882 | chr2:99727122-99727149 |
| AFF3-2890 | chr2:100142519-100142546 | AFF3-5883 | chr2:99727154-99727181 |
| AFF3-2891 | chr2:100142529-100142556 | AFF3-5884 | chr2:99727206-99727233 |
| AFF3-2892 | chr2:100142546-100142573 | AFF3-5885 | chr2:99727213-99727240 |
| AFF3-2893 | chr2:100142562-100142589 | AFF3-5886 | chr2:99727217-99727244 |
| AFF3-2894 | chr2:100142581-100142608 | AFF3-5887 | chr2:99727220-99727247 |
| AFF3-2895 | chr2:100142595-100142622 | AFF3-5888 | chr2:99743979-99744006 |
| AFF3-2896 | chr2:100142615-100142642 | AFF3-5889 | chr2:99743980-99744007 |
| AFF3-2897 | chr2:100142627-100142654 | AFF3-5890 | chr2:99743981-99744008 |
| AFF3-2898 | chr2:100142629-100142656 | AFF3-5891 | chr2:99744008-99744035 |
| AFF3-2899 | chr2:100142630-100142657 | AFF3-5892 | chr2:99744016-99744043 |
| AFF3-2900 | chr2:100142641-100142668 | AFF3-5893 | chr2:99744031-99744058 |
| AFF3-2901 | chr2:100142644-100142671 | AFF3-5894 | chr2:99744034-99744061 |
| AFF3-2902 | chr2:100142672-100142699 | AFF3-5895 | chr2:99744035-99744062 |
| AFF3-2903 | chr2:100142684-100142711 | AFF3-5896 | chr2:99744038-99744065 |
| AFF3-2904 | chr2:100142689-100142716 | AFF3-5897 | chr2:99744039-99744066 |
| AFF3-2905 | chr2:100142691-100142718 | AFF3-5898 | chr2:99744040-99744067 |
| AFF3-2906 | chr2:100142692-100142719 | AFF3-5899 | chr2:99744047-99744074 |
| AFF3-2907 | chr2:100142693-100142720 | AFF3-5900 | chr2:99744048-99744075 |
| AFF3-2908 | chr2:100142694-100142721 | AFF3-5901 | chr2:99744054-99744081 |
| AFF3-2909 | chr2:100142696-100142723 | AFF3-5902 | chr2:99744055-99744082 |
| AFF3-2910 | chr2:100142699-100142726 | AFF3-5903 | chr2:99744056-99744083 |
| AFF3-2911 | chr2:100142700-100142727 | AFF3-5904 | chr2:99744057-99744084 |
| AFF3-2912 | chr2:100142711-100142738 | AFF3-5905 | chr2:99744060-99744087 |
| AFF3-2913 | chr2:100142715-100142742 | AFF3-5906 | chr2:99744061-99744088 |
| AFF3-2914 | chr2:100142722-100142749 | AFF3-5907 | chr2:99744062-99744089 |
| AFF3-2915 | chr2:100142729-100142756 | AFF3-5908 | chr2:99744063-99744090 |
| AFF3-2916 | chr2:100142731-100142758 | AFF3-5909 | chr2:99744069-99744096 |
| AFF3-2917 | chr2:100142741-100142768 | AFF3-5910 | chr2:99744070-99744097 |
| AFF3-2918 | chr2:100142742-100142769 | AFF3-5911 | chr2:99744071-99744098 |
| AFF3-2919 | chr2:100142743-100142770 | AFF3-5912 | chr2:99744080-99744107 |
| AFF3-2920 | chr2:100142752-100142779 | AFF3-5913 | chr2:99744083-99744110 |
| AFF3-2921 | chr2:100142766-100142793 | AFF3-5914 | chr2:99744097-99744124 |
| AFF3-2922 | chr2:100142770-100142797 | AFF3-5915 | chr2:99744118-99744145 |
| AFF3-2923 | chr2:100142773-100142800 | AFF3-5916 | chr2:99744140-99744167 |
| AFF3-2924 | chr2:100142774-100142801 | AFF3-5917 | chr2:99744191-99744218 |
| AFF3-2925 | chr2:100142779-100142806 | AFF3-5918 | chr2:99744196-99744223 |
| AFF3-2926 | chr2:100142782-100142809 | AFF3-5919 | chr2:99744234-99744261 |
| AFF3-2927 | chr2:100142788-100142815 | AFF3-5920 | chr2:99752109-99752136 |
| AFF3-2928 | chr2:100142789-100142816 | AFF3-5921 | chr2:99752121-99752148 |
| AFF3-2929 | chr2:100142795-100142822 | AFF3-5922 | chr2:99752122-99752149 |
| AFF3-2930 | chr2:100142801-100142828 | AFF3-5923 | chr2:99752155-99752182 |
| AFF3-2931 | chr2:100142802-100142829 | AFF3-5924 | chr2:99752156-99752183 |
| AFF3-2932 | chr2:100142810-100142837 | AFF3-5925 | chr2:99752162-99752189 |
| AFF3-2933 | chr2:100142834-100142861 | AFF3-5926 | chr2:99752163-99752190 |
| AFF3-2934 | chr2:100142848-100142875 | AFF3-5927 | chr2:99752175-99752202 |
| AFF3-2935 | chr2:100142849-100142876 | AFF3-5928 | chr2:99752178-99752205 |
| AFF3-2936 | chr2:100142850-100142877 | AFF3-5929 | chr2:99752179-99752206 |
| AFF3-2937 | chr2:100142859-100142886 | AFF3-5930 | chr2:99752196-99752223 |
| AFF3-2938 | chr2:100142860-100142887 | AFF3-5931 | chr2:99752199-99752226 |
| AFF3-2939 | chr2:100142872-100142899 | AFF3-5932 | chr2:99752202-99752229 |
| AFF3-2940 | chr2:100142873-100142900 | AFF3-5933 | chr2:99752208-99752235 |
| AFF3-2941 | chr2:100142903-100142930 | AFF3-5934 | chr2:99752216-99752243 |
| AFF3-2942 | chr2:100142904-100142931 | AFF3-5935 | chr2:99752220-99752247 |
| AFF3-2943 | chr2:100142932-100142959 | AFF3-5936 | chr2:99752235-99752262 |
| AFF3-2944 | chr2:100142941-100142968 | AFF3-5937 | chr2:99752250-99752277 |
| AFF3-2945 | chr2:100142945-100142972 | AFF3-5938 | chr2:99752256-99752283 |
| AFF3-2946 | chr2:100142949-100142976 | AFF3-5939 | chr2:99752258-99752285 |
| AFF3-2947 | chr2:100142950-100142977 | AFF3-5940 | chr2:99752259-99752286 |
| AFF3-2948 | chr2:100142963-100142990 | AFF3-5941 | chr2:99752267-99752294 |
| AFF3-2949 | chr2:100142964-100142991 | AFF3-5942 | chr2:99752281-99752308 |
| AFF3-2950 | chr2:100142966-100142993 | AFF3-5943 | chr2:99752285-99752312 |
| AFF3-2951 | chr2:100142974-100143001 | AFF3-5944 | chr2:99752286-99752313 |
| AFF3-2952 | chr2:100142978-100143005 | AFF3-5945 | chr2:99752290-99752317 |
| AFF3-2953 | chr2:100142981-100143008 | AFF3-5946 | chr2:99752301-99752328 |
| AFF3-2954 | chr2:100142989-100143016 | AFF3-5947 | chr2:99752333-99752360 |
| AFF3-2955 | chr2:100142995-100143022 | AFF3-5948 | chr2:99752357-99752384 |
| AFF3-2956 | chr2:100143007-100143034 | AFF3-5949 | chr2:99752358-99752385 |
| AFF3-2957 | chr2:100143033-100143060 | AFF3-5950 | chr2:99752369-99752396 |
| AFF3-2958 | chr2:100143036-100143063 | AFF3-5951 | chr2:99752373-99752400 |
| AFF3-2959 | chr2:100143038-100143065 | AFF3-5952 | chr2:99752374-99752401 |
| AFF3-2960 | chr2:100143052-100143079 | AFF3-5953 | chr2:99752378-99752405 |
| AFF3-2961 | chr2:100143057-100143084 | AFF3-5954 | chr2:99752379-99752406 |
| AFF3-2962 | chr2:100143059-100143086 | AFF3-5955 | chr2:99752386-99752413 |
| AFF3-2963 | chr2:100143074-100143101 | AFF3-5956 | chr2:99752397-99752424 |
| AFF3-2964 | chr2:100143095-100143122 | AFF3-5957 | chr2:99837362-99837389 |
| AFF3-2965 | chr2:100143099-100143126 | AFF3-5958 | chr2:99837363-99837390 |
| AFF3-2966 | chr2:100143100-100143127 | AFF3-5959 | chr2:99837377-99837404 |
| AFF3-2967 | chr2:100143115-100143142 | AFF3-5960 | chr2:99837386-99837413 |
| AFF3-2968 | chr2:100143133-100143160 | AFF3-5961 | chr2:99837393-99837420 |
| AFF3-2969 | chr2:100143136-100143163 | AFF3-5962 | chr2:99837407-99837434 |
| AFF3-2970 | chr2:100143137-100143164 | AFF3-5963 | chr2:99837417-99837444 |
| AFF3-2971 | chr2:100143143-100143170 | AFF3-5964 | chr2:99837439-99837466 |
| AFF3-2972 | chr2:100143144-100143171 | AFF3-5965 | chr2:99837440-99837467 |
| AFF3-2973 | chr2:100143145-100143172 | AFF3-5966 | chr2:99837453-99837480 |
| AFF3-2974 | chr2:100143157-100143184 | AFF3-5967 | chr2:99837476-99837503 |
| AFF3-2975 | chr2:100143158-100143185 | AFF3-5968 | chr2:99837477-99837504 |
| AFF3-2976 | chr2:100143175-100143202 | AFF3-5969 | chr2:99837478-99837505 |
| AFF3-2977 | chr2:100143179-100143206 | AFF3-5970 | chr2:99837511-99837538 |
| AFF3-2978 | chr2:100143180-100143207 | AFF3-5971 | chr2:99837524-99837551 |
| AFF3-2979 | chr2:100143185-100143212 | AFF3-5972 | chr2:99837531-99837558 |
| AFF3-2980 | chr2:100143186-100143213 | AFF3-5973 | chr2:99837551-99837578 |
| AFF3-2981 | chr2:100143187-100143214 | AFF3-5974 | chr2:99837565-99837592 |
| AFF3-2982 | chr2:100143191-100143218 | AFF3-5975 | chr2:99837572-99837599 |
| AFF3-2983 | chr2:100143192-100143219 | AFF3-5976 | chr2:99837595-99837622 |
| AFF3-2984 | chr2:100143197-100143224 | AFF3-5977 | chr2:99837605-99837632 |
| AFF3-2985 | chr2:100143198-100143225 | AFF3-5978 | chr2:99837606-99837633 |
| AFF3-2986 | chr2:100143199-100143226 | AFF3-5979 | chr2:99837607-99837634 |
| AFF3-2987 | chr2:100143208-100143235 | AFF3-5980 | chr2:99837608-99837635 |
| AFF3-2988 | chr2:100143210-100143237 | AFF3-5981 | chr2:99837609-99837636 |
| AFF3-2989 | chr2:100143211-100143238 | AFF3-5982 | chr2:99837610-99837637 |
| AFF3-2990 | chr2:100143223-100143250 | AFF3-5983 | chr2:99837616-99837643 |
| AFF3-2991 | chr2:100143233-100143260 | AFF3-5984 | chr2:99837617-99837644 |
| AFF3-2992 | chr2:100143260-100143287 | AFF3-5985 | chr2:99837618-99837645 |
| AFF3-2993 | chr2:100143265-100143292 | | |

**Table 2D Genomic coordinates of the human AXL gene**

| Target ID | Target Coordinate Range | Target ID | Target Coordinate Range |
|---|---|---|---|
| AXL-1 | chr19:41217698-41217725 | AXL-1146 | chr19:41237973-41238000 |
| AXL-2 | chr19:41217712-41217739 | AXL-1147 | chr19:41237974-41238001 |
| AXL-3 | chr19:41217713-41217740 | AXL-1148 | chr19:41237986-41238013 |
| AXL-4 | chr19:41217720-41217747 | AXL-1149 | chr19:41237991-41238018 |
| AXL-5 | chr19:41217723-41217750 | AXL-1150 | chr19:41237992-41238019 |
| AXL-6 | chr19:41217729-41217756 | AXL-1151 | chr19:41237993-41238020 |
| AXL-7 | chr19:41217730-41217757 | AXL-1152 | chr19:41237994-41238021 |
| AXL-8 | chr19:41217732-41217759 | AXL-1153 | chr19:41237995-41238022 |
| AXL-9 | chr19:41217733-41217760 | AXL-1154 | chr19:41238004-41238031 |
| AXL-10 | chr19:41217743-41217770 | AXL-1155 | chr19:41238005-41238032 |
| AXL-11 | chr19:41217744-41217771 | AXL-1156 | chr19:41238006-41238033 |
| AXL-12 | chr19:41217749-41217776 | AXL-1157 | chr19:41238011-41238038 |
| AXL-13 | chr19:41217750-41217777 | AXL-1158 | chr19:41238018-41238045 |
| AXL-14 | chr19:41217751-41217778 | AXL-1159 | chr19:41238023-41238050 |
| AXL-15 | chr19:41217754-41217781 | AXL-1160 | chr19:41238028-41238055 |
| AXL-16 | chr19:41217781-41217808 | AXL-1161 | chr19:41238029-41238056 |
| AXL-17 | chr19:41217792-41217819 | AXL-1162 | chr19:41238030-41238057 |
| AXL-18 | chr19:41217793-41217820 | AXL-1163 | chr19:41238031-41238058 |
| AXL-19 | chr19:41217800-41217827 | AXL-1164 | chr19:41238032-41238059 |
| AXL-20 | chr19:41217802-41217829 | AXL-1165 | chr19:41238033-41238060 |
| AXL-21 | chr19:41217814-41217841 | AXL-1166 | chr19:41238054-41238081 |
| AXL-22 | chr19:41217818-41217845 | AXL-1167 | chr19:41238057-41238084 |
| AXL-23 | chr19:41217824-41217851 | AXL-1168 | chr19:41238061-41238088 |
| AXL-24 | chr19:41217828-41217855 | AXL-1169 | chr19:41238068-41238095 |
| AXL-25 | chr19:41217837-41217864 | AXL-1170 | chr19:41238069-41238096 |
| AXL-26 | chr19:41217838-41217865 | AXL-1171 | chr19:41238070-41238097 |
| AXL-27 | chr19:41217839-41217866 | AXL-1172 | chr19:41238079-41238106 |
| AXL-28 | chr19:41217840-41217867 | AXL-1173 | chr19:41238080-41238107 |
| AXL-29 | chr19:41217846-41217873 | AXL-1174 | chr19:41238081-41238108 |
| AXL-30 | chr19:41217847-41217874 | AXL-1175 | chr19:41238093-41238120 |
| AXL-31 | chr19:41217851-41217878 | AXL-1176 | chr19:41238095-41238122 |
| AXL-32 | chr19:41217852-41217879 | AXL-1177 | chr19:41238102-41238129 |
| AXL-33 | chr19:41217853-41217880 | AXL-1178 | chr19:41238108-41238135 |
| AXL-34 | chr19:41217857-41217884 | AXL-1179 | chr19:41238109-41238136 |
| AXL-35 | chr19:41217873-41217900 | AXL-1180 | chr19:41238110-41238137 |
| AXL-36 | chr19:41217876-41217903 | AXL-1181 | chr19:41238112-41238139 |
| AXL-37 | chr19:41217889-41217916 | AXL-1182 | chr19:41238116-41238143 |
| AXL-38 | chr19:41217904-41217931 | AXL-1183 | chr19:41238121-41238148 |
| AXL-39 | chr19:41217909-41217936 | AXL-1184 | chr19:41238122-41238149 |
| AXL-40 | chr19:41217930-41217957 | AXL-1185 | chr19:41238123-41238150 |
| AXL-41 | chr19:41217931-41217958 | AXL-1186 | chr19:41238124-41238151 |
| AXL-42 | chr19:41217934-41217961 | AXL-1187 | chr19:41238125-41238152 |
| AXL-43 | chr19:41217941-41217968 | AXL-1188 | chr19:41238128-41238155 |
| AXL-44 | chr19:41217947-41217974 | AXL-1189 | chr19:41238133-41238160 |
| AXL-45 | chr19:41217948-41217975 | AXL-1190 | chr19:41238136-41238163 |
| AXL-46 | chr19:41217962-41217989 | AXL-1191 | chr19:41238137-41238164 |
| AXL-47 | chr19:41217983-41218010 | AXL-1192 | chr19:41238141-41238168 |
| AXL-48 | chr19:41217984-41218011 | AXL-1193 | chr19:41238142-41238169 |
| AXL-49 | chr19:41217988-41218015 | AXL-1194 | chr19:41238143-41238170 |
| AXL-50 | chr19:41217991-41218018 | AXL-1195 | chr19:41238145-41238172 |
| AXL-51 | chr19:41217994-41218021 | AXL-1196 | chr19:41238146-41238173 |
| AXL-52 | chr19:41218006-41218033 | AXL-1197 | chr19:41238147-41238174 |
| AXL-53 | chr19:41218016-41218043 | AXL-1198 | chr19:41238170-41238197 |
| AXL-54 | chr19:41218017-41218044 | AXL-1199 | chr19:41238183-41238210 |
| AXL-55 | chr19:41218020-41218047 | AXL-1200 | chr19:41238189-41238216 |
| AXL-56 | chr19:41218021-41218048 | AXL-1201 | chr19:41238190-41238217 |
| AXL-57 | chr19:41218023-41218050 | AXL-1202 | chr19:41238191-41238218 |
| AXL-58 | chr19:41218024-41218051 | AXL-1203 | chr19:41238194-41238221 |
| AXL-59 | chr19:41218028-41218055 | AXL-1204 | chr19:41238199-41238226 |
| AXL-60 | chr19:41218031-41218058 | AXL-1205 | chr19:41238216-41238243 |
| AXL-61 | chr19:41218045-41218072 | AXL-1206 | chr19:41238219-41238246 |
| AXL-62 | chr19:41218053-41218080 | AXL-1207 | chr19:41238220-41238247 |
| AXL-63 | chr19:41218069-41218096 | AXL-1208 | chr19:41238221-41238248 |
| AXL-64 | chr19:41218075-41218102 | AXL-1209 | chr19:41238222-41238249 |
| AXL-65 | chr19:41218096-41218123 | AXL-1210 | chr19:41238228-41238255 |
| AXL-66 | chr19:41218098-41218125 | AXL-1211 | chr19:41238229-41238256 |
| AXL-67 | chr19:41218108-41218135 | AXL-1212 | chr19:41238230-41238257 |
| AXL-68 | chr19:41218112-41218139 | AXL-1213 | chr19:41238231-41238258 |
| AXL-69 | chr19:41218113-41218140 | AXL-1214 | chr19:41238240-41238267 |
| AXL-70 | chr19:41218116-41218143 | AXL-1215 | chr19:41238246-41238273 |
| AXL-71 | chr19:41218118-41218145 | AXL-1216 | chr19:41238247-41238274 |
| AXL-72 | chr19:41218119-41218146 | AXL-1217 | chr19:41238248-41238275 |
| AXL-73 | chr19:41218143-41218170 | AXL-1218 | chr19:41238355-41238382 |
| AXL-74 | chr19:41218157-41218184 | AXL-1219 | chr19:41238359-41238386 |
| AXL-75 | chr19:41218182-41218209 | AXL-1220 | chr19:41238368-41238395 |
| AXL-76 | chr19:41218183-41218210 | AXL-1221 | chr19:41238369-41238396 |
| AXL-77 | chr19:41218184-41218211 | AXL-1222 | chr19:41238380-41238407 |
| AXL-78 | chr19:41218191-41218218 | AXL-1223 | chr19:41238381-41238408 |
| AXL-79 | chr19:41218192-41218219 | AXL-1224 | chr19:41238387-41238414 |
| AXL-80 | chr19:41218195-41218222 | AXL-1225 | chr19:41238388-41238415 |
| AXL-81 | chr19:41218198-41218225 | AXL-1226 | chr19:41238389-41238416 |
| AXL-82 | chr19:41218199-41218226 | AXL-1227 | chr19:41238390-41238417 |
| AXL-83 | chr19:41218211-41218238 | AXL-1228 | chr19:41238392-41238419 |
| AXL-84 | chr19:41218213-41218240 | AXL-1229 | chr19:41238393-41238420 |
| AXL-85 | chr19:41218226-41218253 | AXL-1230 | chr19:41238394-41238421 |
| AXL-86 | chr19:41218234-41218261 | AXL-1231 | chr19:41238395-41238422 |
| AXL-87 | chr19:41218246-41218273 | AXL-1232 | chr19:41238396-41238423 |
| AXL-88 | chr19:41218247-41218274 | AXL-1233 | chr19:41238397-41238424 |
| AXL-89 | chr19:41218248-41218275 | AXL-1234 | chr19:41238400-41238427 |
| AXL-90 | chr19:41218249-41218276 | AXL-1235 | chr19:41238403-41238430 |
| AXL-91 | chr19:41218272-41218299 | AXL-1236 | chr19:41238406-41238433 |
| AXL-92 | chr19:41218286-41218313 | AXL-1237 | chr19:41238409-41238436 |
| AXL-93 | chr19:41218287-41218314 | AXL-1238 | chr19:41238410-41238437 |
| AXL-94 | chr19:41218291-41218318 | AXL-1239 | chr19:41238411-41238438 |
| AXL-95 | chr19:41218309-41218336 | AXL-1240 | chr19:41238412-41238439 |
| AXL-96 | chr19:41218310-41218337 | AXL-1241 | chr19:41238442-41238469 |
| AXL-97 | chr19:41218325-41218352 | AXL-1242 | chr19:41238449-41238476 |
| AXL-98 | chr19:41218326-41218353 | AXL-1243 | chr19:41238450-41238477 |
| AXL-99 | chr19:41218327-41218354 | AXL-1244 | chr19:41238451-41238478 |
| AXL-100 | chr19:41218328-41218355 | AXL-1245 | chr19:41238452-41238479 |
| AXL-101 | chr19:41218329-41218356 | AXL-1246 | chr19:41238457-41238484 |
| AXL-102 | chr19:41218332-41218359 | AXL-1247 | chr19:41238458-41238485 |
| AXL-103 | chr19:41218335-41218362 | AXL-1248 | chr19:41238463-41238490 |
| AXL-104 | chr19:41218338-41218365 | AXL-1249 | chr19:41238466-41238493 |
| AXL-105 | chr19:41218341-41218368 | AXL-1250 | chr19:41238472-41238499 |
| AXL-106 | chr19:41218342-41218369 | AXL-1251 | chr19:41238473-41238500 |
| AXL-107 | chr19:41218354-41218381 | AXL-1252 | chr19:41238474-41238501 |
| AXL-108 | chr19:41218377-41218404 | AXL-1253 | chr19:41238480-41238507 |
| AXL-109 | chr19:41218378-41218405 | AXL-1254 | chr19:41238481-41238508 |
| AXL-110 | chr19:41218382-41218409 | AXL-1255 | chr19:41238482-41238509 |
| AXL-111 | chr19:41218388-41218415 | AXL-1256 | chr19:41238483-41238510 |
| AXL-112 | chr19:41218399-41218426 | AXL-1257 | chr19:41238484-41238511 |
| AXL-113 | chr19:41218400-41218427 | AXL-1258 | chr19:41238485-41238512 |
| AXL-114 | chr19:41218401-41218428 | AXL-1259 | chr19:41238486-41238513 |
| AXL-115 | chr19:41218405-41218432 | AXL-1260 | chr19:41238493-41238520 |
| AXL-116 | chr19:41218408-41218435 | AXL-1261 | chr19:41238507-41238534 |
| AXL-117 | chr19:41218436-41218463 | AXL-1262 | chr19:41238516-41238543 |
| AXL-118 | chr19:41218440-41218467 | AXL-1263 | chr19:41238519-41238546 |
| AXL-119 | chr19:41218447-41218474 | AXL-1264 | chr19:41238520-41238547 |
| AXL-120 | chr19:41218449-41218476 | AXL-1265 | chr19:41238521-41238548 |
| AXL-121 | chr19:41218450-41218477 | AXL-1266 | chr19:41238532-41238559 |
| AXL-122 | chr19:41218454-41218481 | AXL-1267 | chr19:41238533-41238560 |
| AXL-123 | chr19:41218455-41218482 | AXL-1268 | chr19:41238541-41238568 |
| AXL-124 | chr19:41218462-41218489 | AXL-1269 | chr19:41238542-41238569 |
| AXL-125 | chr19:41218463-41218490 | AXL-1270 | chr19:41238543-41238570 |
| AXL-126 | chr19:41218481-41218508 | AXL-1271 | chr19:41238545-41238572 |
| AXL-127 | chr19:41218488-41218515 | AXL-1272 | chr19:41238546-41238573 |
| AXL-128 | chr19:41218492-41218519 | AXL-1273 | chr19:41238550-41238577 |
| AXL-129 | chr19:41218493-41218520 | AXL-1274 | chr19:41238551-41238578 |
| AXL-130 | chr19:41218494-41218521 | AXL-1275 | chr19:41238552-41238579 |
| AXL-131 | chr19:41218501-41218528 | AXL-1276 | chr19:41238556-41238583 |
| AXL-132 | chr19:41218505-41218532 | AXL-1277 | chr19:41238563-41238590 |
| AXL-133 | chr19:41218513-41218540 | AXL-1278 | chr19:41238564-41238591 |
| AXL-134 | chr19:41218519-41218546 | AXL-1279 | chr19:41238566-41238593 |
| AXL-135 | chr19:41218520-41218547 | AXL-1280 | chr19:41238571-41238598 |
| AXL-136 | chr19:41218532-41218559 | AXL-1281 | chr19:41238576-41238603 |
| AXL-137 | chr19:41218563-41218590 | AXL-1282 | chr19:41238583-41238610 |
| AXL-138 | chr19:41218568-41218595 | AXL-1283 | chr19:41238586-41238613 |
| AXL-139 | chr19:41218569-41218596 | AXL-1284 | chr19:41238587-41238614 |
| AXL-140 | chr19:41218570-41218597 | AXL-1285 | chr19:41238594-41238621 |
| AXL-141 | chr19:41218582-41218609 | AXL-1286 | chr19:41238596-41238623 |
| AXL-142 | chr19:41218583-41218610 | AXL-1287 | chr19:41238599-41238626 |
| AXL-143 | chr19:41218584-41218611 | AXL-1288 | chr19:41238600-41238627 |
| AXL-144 | chr19:41218585-41218612 | AXL-1289 | chr19:41238602-41238629 |
| AXL-145 | chr19:41218594-41218621 | AXL-1290 | chr19:41238603-41238630 |
| AXL-146 | chr19:41218595-41218622 | AXL-1291 | chr19:41238604-41238631 |
| AXL-147 | chr19:41218597-41218624 | AXL-1292 | chr19:41238605-41238632 |
| AXL-148 | chr19:41218598-41218625 | AXL-1293 | chr19:41238610-41238637 |
| AXL-149 | chr19:41218599-41218626 | AXL-1294 | chr19:41238615-41238642 |
| AXL-150 | chr19:41218603-41218630 | AXL-1295 | chr19:41238620-41238647 |
| AXL-151 | chr19:41218607-41218634 | AXL-1296 | chr19:41238621-41238648 |
| AXL-152 | chr19:41218609-41218636 | AXL-1297 | chr19:41238622-41238649 |
| AXL-153 | chr19:41218612-41218639 | AXL-1298 | chr19:41238625-41238652 |
| AXL-154 | chr19:41218621-41218648 | AXL-1299 | chr19:41238628-41238655 |
| AXL-155 | chr19:41218626-41218653 | AXL-1300 | chr19:41238629-41238656 |
| AXL-156 | chr19:41218630-41218657 | AXL-1301 | chr19:41238632-41238659 |
| AXL-157 | chr19:41218644-41218671 | AXL-1302 | chr19:41238645-41238672 |
| AXL-158 | chr19:41218647-41218674 | AXL-1303 | chr19:41238646-41238673 |
| AXL-159 | chr19:41218648-41218675 | AXL-1304 | chr19:41238659-41238686 |
| AXL-160 | chr19:41218649-41218676 | AXL-1305 | chr19:41238668-41238695 |
| AXL-161 | chr19:41218674-41218701 | AXL-1306 | chr19:41238672-41238699 |
| AXL-162 | chr19:41218675-41218702 | AXL-1307 | chr19:41238673-41238700 |
| AXL-163 | chr19:41218676-41218703 | AXL-1308 | chr19:41238699-41238726 |
| AXL-164 | chr19:41218687-41218714 | AXL-1309 | chr19:41238700-41238727 |
| AXL-165 | chr19:41218688-41218715 | AXL-1310 | chr19:41238701-41238728 |
| AXL-166 | chr19:41218689-41218716 | AXL-1311 | chr19:41238702-41238729 |
| AXL-167 | chr19:41218693-41218720 | AXL-1312 | chr19:41239037-41239064 |
| AXL-168 | chr19:41218694-41218721 | AXL-1313 | chr19:41239060-41239087 |
| AXL-169 | chr19:41218704-41218731 | AXL-1314 | chr19:41239061-41239088 |
| AXL-170 | chr19:41218705-41218732 | AXL-1315 | chr19:41239062-41239089 |
| AXL-171 | chr19:41218726-41218753 | AXL-1316 | chr19:41239083-41239110 |
| AXL-172 | chr19:41218731-41218758 | AXL-1317 | chr19:41239095-41239122 |
| AXL-173 | chr19:41218789-41218816 | AXL-1318 | chr19:41239096-41239123 |
| AXL-174 | chr19:41218805-41218832 | AXL-1319 | chr19:41239097-41239124 |
| AXL-175 | chr19:41218806-41218833 | AXL-1320 | chr19:41239098-41239125 |
| AXL-176 | chr19:41218807-41218834 | AXL-1321 | chr19:41239099-41239126 |
| AXL-177 | chr19:41218808-41218835 | AXL-1322 | chr19:41239104-41239131 |
| AXL-178 | chr19:41218811-41218838 | AXL-1323 | chr19:41239127-41239154 |
| AXL-179 | chr19:41218816-41218843 | AXL-1324 | chr19:41239137-41239164 |
| AXL-180 | chr19:41218817-41218844 | AXL-1325 | chr19:41239138-41239165 |
| AXL-181 | chr19:41218819-41218846 | AXL-1326 | chr19:41239145-41239172 |
| AXL-182 | chr19:41218823-41218850 | AXL-1327 | chr19:41239146-41239173 |
| AXL-183 | chr19:41218826-41218853 | AXL-1328 | chr19:41239153-41239180 |
| AXL-184 | chr19:41218831-41218858 | AXL-1329 | chr19:41239154-41239181 |
| AXL-185 | chr19:41218835-41218862 | AXL-1330 | chr19:41239156-41239183 |
| AXL-186 | chr19:41218836-41218863 | AXL-1331 | chr19:41239159-41239186 |
| AXL-187 | chr19:41218837-41218864 | AXL-1332 | chr19:41239160-41239187 |
| AXL-188 | chr19:41218841-41218868 | AXL-1333 | chr19:41239167-41239194 |
| AXL-189 | chr19:41218842-41218869 | AXL-1334 | chr19:41239176-41239203 |
| AXL-190 | chr19:41218845-41218872 | AXL-1335 | chr19:41239185-41239212 |
| AXL-191 | chr19:41218846-41218873 | AXL-1336 | chr19:41239186-41239213 |
| AXL-192 | chr19:41218847-41218874 | AXL-1337 | chr19:41239187-41239214 |
| AXL-193 | chr19:41218848-41218875 | AXL-1338 | chr19:41239188-41239215 |
| AXL-194 | chr19:41218849-41218876 | AXL-1339 | chr19:41239192-41239219 |
| AXL-195 | chr19:41218852-41218879 | AXL-1340 | chr19:41239193-41239220 |
| AXL-196 | chr19:41218854-41218881 | AXL-1341 | chr19:41239198-41239225 |
| AXL-197 | chr19:41218855-41218882 | AXL-1342 | chr19:41239199-41239226 |
| AXL-198 | chr19:41218858-41218885 | AXL-1343 | chr19:41239221-41239248 |
| AXL-199 | chr19:41218859-41218886 | AXL-1344 | chr19:41239228-41239255 |
| AXL-200 | chr19:41218860-41218887 | AXL-1345 | chr19:41239240-41239267 |
| AXL-201 | chr19:41218865-41218892 | AXL-1346 | chr19:41239241-41239268 |
| AXL-202 | chr19:41218866-41218893 | AXL-1347 | chr19:41239242-41239269 |
| AXL-203 | chr19:41218867-41218894 | AXL-1348 | chr19:41239246-41239273 |
| AXL-204 | chr19:41218875-41218902 | AXL-1349 | chr19:41239252-41239279 |
| AXL-205 | chr19:41218889-41218916 | AXL-1350 | chr19:41239253-41239280 |
| AXL-206 | chr19:41218890-41218917 | AXL-1351 | chr19:41239272-41239299 |
| AXL-207 | chr19:41218891-41218918 | AXL-1352 | chr19:41239275-41239302 |
| AXL-208 | chr19:41218896-41218923 | AXL-1353 | chr19:41239276-41239303 |
| AXL-209 | chr19:41218903-41218930 | AXL-1354 | chr19:41239280-41239307 |
| AXL-210 | chr19:41218904-41218931 | AXL-1355 | chr19:41239283-41239310 |
| AXL-211 | chr19:41218907-41218934 | AXL-1356 | chr19:41239286-41239313 |
| AXL-212 | chr19:41218935-41218962 | AXL-1357 | chr19:41239287-41239314 |
| AXL-213 | chr19:41218936-41218963 | AXL-1358 | chr19:41239288-41239315 |
| AXL-214 | chr19:41218943-41218970 | AXL-1359 | chr19:41239293-41239320 |
| AXL-215 | chr19:41218954-41218981 | AXL-1360 | chr19:41239294-41239321 |
| AXL-216 | chr19:41218955-41218982 | AXL-1361 | chr19:41239295-41239322 |
| AXL-217 | chr19:41218956-41218983 | AXL-1362 | chr19:41239303-41239330 |
| AXL-218 | chr19:41218968-41218995 | AXL-1363 | chr19:41239310-41239337 |
| AXL-219 | chr19:41218970-41218997 | AXL-1364 | chr19:41239311-41239338 |
| AXL-220 | chr19:41218971-41218998 | AXL-1365 | chr19:41239321-41239348 |
| AXL-221 | chr19:41218977-41219004 | AXL-1366 | chr19:41239327-41239354 |
| AXL-222 | chr19:41218978-41219005 | AXL-1367 | chr19:41239332-41239359 |
| AXL-223 | chr19:41218983-41219010 | AXL-1368 | chr19:41239333-41239360 |
| AXL-224 | chr19:41218988-41219015 | AXL-1369 | chr19:41239337-41239364 |
| AXL-225 | chr19:41218989-41219016 | AXL-1370 | chr19:41239347-41239374 |
| AXL-226 | chr19:41218990-41219017 | AXL-1371 | chr19:41239348-41239375 |
| AXL-227 | chr19:41218991-41219018 | AXL-1372 | chr19:41239357-41239384 |
| AXL-228 | chr19:41218996-41219023 | AXL-1373 | chr19:41239358-41239385 |
| AXL-229 | chr19:41219004-41219031 | AXL-1374 | chr19:41239366-41239393 |
| AXL-230 | chr19:41219005-41219032 | AXL-1375 | chr19:41239367-41239394 |
| AXL-231 | chr19:41219024-41219051 | AXL-1376 | chr19:41239378-41239405 |
| AXL-232 | chr19:41219027-41219054 | AXL-1377 | chr19:41239382-41239409 |
| AXL-233 | chr19:41219034-41219061 | AXL-1378 | chr19:41239385-41239412 |
| AXL-234 | chr19:41219035-41219062 | AXL-1379 | chr19:41239398-41239425 |
| AXL-235 | chr19:41219036-41219063 | AXL-1380 | chr19:41239410-41239437 |
| AXL-236 | chr19:41219040-41219067 | AXL-1381 | chr19:41239411-41239438 |
| AXL-237 | chr19:41219041-41219068 | AXL-1382 | chr19:41239568-41239595 |
| AXL-238 | chr19:41219042-41219069 | AXL-1383 | chr19:41239576-41239603 |
| AXL-239 | chr19:41219043-41219070 | AXL-1384 | chr19:41239577-41239604 |
| AXL-240 | chr19:41219046-41219073 | AXL-1385 | chr19:41239588-41239615 |
| AXL-241 | chr19:41219054-41219081 | AXL-1386 | chr19:41239593-41239620 |
| AXL-242 | chr19:41219055-41219082 | AXL-1387 | chr19:41239594-41239621 |
| AXL-243 | chr19:41219058-41219085 | AXL-1388 | chr19:41239601-41239628 |
| AXL-244 | chr19:41219059-41219086 | AXL-1389 | chr19:41239602-41239629 |
| AXL-245 | chr19:41219064-41219091 | AXL-1390 | chr19:41239607-41239634 |
| AXL-246 | chr19:41219065-41219092 | AXL-1391 | chr19:41239608-41239635 |
| AXL-247 | chr19:41219066-41219093 | AXL-1392 | chr19:41239613-41239640 |
| AXL-248 | chr19:41219085-41219112 | AXL-1393 | chr19:41239618-41239645 |
| AXL-249 | chr19:41219090-41219117 | AXL-1394 | chr19:41239619-41239646 |
| AXL-250 | chr19:41219093-41219120 | AXL-1395 | chr19:41239626-41239653 |
| AXL-251 | chr19:41219096-41219123 | AXL-1396 | chr19:41239627-41239654 |
| AXL-252 | chr19:41219103-41219130 | AXL-1397 | chr19:41239632-41239659 |
| AXL-253 | chr19:41219104-41219131 | AXL-1398 | chr19:41239633-41239660 |
| AXL-254 | chr19:41219105-41219132 | AXL-1399 | chr19:41239638-41239665 |
| AXL-255 | chr19:41219106-41219133 | AXL-1400 | chr19:41239643-41239670 |
| AXL-256 | chr19:41219107-41219134 | AXL-1401 | chr19:41239653-41239680 |
| AXL-257 | chr19:41219110-41219137 | AXL-1402 | chr19:41239667-41239694 |
| AXL-258 | chr19:41219116-41219143 | AXL-1403 | chr19:41239668-41239695 |
| AXL-259 | chr19:41219119-41219146 | AXL-1404 | chr19:41239670-41239697 |
| AXL-260 | chr19:41219120-41219147 | AXL-1405 | chr19:41239679-41239706 |
| AXL-261 | chr19:41219123-41219150 | AXL-1406 | chr19:41239693-41239720 |
| AXL-262 | chr19:41219124-41219151 | AXL-1407 | chr19:41239694-41239721 |
| AXL-263 | chr19:41219125-41219152 | AXL-1408 | chr19:41239699-41239726 |
| AXL-264 | chr19:41219126-41219153 | AXL-1409 | chr19:41239700-41239727 |
| AXL-265 | chr19:41219132-41219159 | AXL-1410 | chr19:41239705-41239732 |
| AXL-266 | chr19:41219133-41219160 | AXL-1411 | chr19:41239710-41239737 |
| AXL-267 | chr19:41219134-41219161 | AXL-1412 | chr19:41239713-41239740 |
| AXL-268 | chr19:41219135-41219162 | AXL-1413 | chr19:41239731-41239758 |
| AXL-269 | chr19:41219138-41219165 | AXL-1414 | chr19:41239736-41239763 |
| AXL-270 | chr19:41219139-41219166 | AXL-1415 | chr19:41239737-41239764 |
| AXL-271 | chr19:41219142-41219169 | AXL-1416 | chr19:41239738-41239765 |
| AXL-272 | chr19:41219143-41219170 | AXL-1417 | chr19:41239744-41239771 |
| AXL-273 | chr19:41219144-41219171 | AXL-1418 | chr19:41239745-41239772 |
| AXL-274 | chr19:41219147-41219174 | AXL-1419 | chr19:41239746-41239773 |
| AXL-275 | chr19:41219154-41219181 | AXL-1420 | chr19:41239747-41239774 |
| AXL-276 | chr19:41219155-41219182 | AXL-1421 | chr19:41239748-41239775 |
| AXL-277 | chr19:41219165-41219192 | AXL-1422 | chr19:41239751-41239778 |
| AXL-278 | chr19:41219169-41219196 | AXL-1423 | chr19:41239752-41239779 |
| AXL-279 | chr19:41219170-41219197 | AXL-1424 | chr19:41239756-41239783 |
| AXL-280 | chr19:41219177-41219204 | AXL-1425 | chr19:41239757-41239784 |
| AXL-281 | chr19:41219178-41219205 | AXL-1426 | chr19:41239765-41239792 |
| AXL-282 | chr19:41219182-41219209 | AXL-1427 | chr19:41239784-41239811 |
| AXL-283 | chr19:41219185-41219212 | AXL-1428 | chr19:41239805-41239832 |
| AXL-284 | chr19:41219186-41219213 | AXL-1429 | chr19:41239806-41239833 |
| AXL-285 | chr19:41219187-41219214 | AXL-1430 | chr19:41242760-41242787 |
| AXL-286 | chr19:41219189-41219216 | AXL-1431 | chr19:41242764-41242791 |
| AXL-287 | chr19:41219190-41219217 | AXL-1432 | chr19:41242786-41242813 |
| AXL-288 | chr19:41219191-41219218 | AXL-1433 | chr19:41242792-41242819 |
| AXL-289 | chr19:41219202-41219229 | AXL-1434 | chr19:41242796-41242823 |
| AXL-290 | chr19:41219205-41219232 | AXL-1435 | chr19:41242797-41242824 |
| AXL-291 | chr19:41219212-41219239 | AXL-1436 | chr19:41242799-41242826 |
| AXL-292 | chr19:41219213-41219240 | AXL-1437 | chr19:41242802-41242829 |
| AXL-293 | chr19:41219222-41219249 | AXL-1438 | chr19:41242803-41242830 |
| AXL-294 | chr19:41219225-41219252 | AXL-1439 | chr19:41242806-41242833 |
| AXL-295 | chr19:41219230-41219257 | AXL-1440 | chr19:41242807-41242834 |
| AXL-296 | chr19:41219231-41219258 | AXL-1441 | chr19:41242811-41242838 |
| AXL-297 | chr19:41219246-41219273 | AXL-1442 | chr19:41242815-41242842 |
| AXL-298 | chr19:41219253-41219280 | AXL-1443 | chr19:41242816-41242843 |
| AXL-299 | chr19:41219257-41219284 | AXL-1444 | chr19:41242817-41242844 |
| AXL-300 | chr19:41219259-41219286 | AXL-1445 | chr19:41242819-41242846 |
| AXL-301 | chr19:41219262-41219289 | AXL-1446 | chr19:41242826-41242853 |
| AXL-302 | chr19:41219263-41219290 | AXL-1447 | chr19:41242827-41242854 |
| AXL-303 | chr19:41219264-41219291 | AXL-1448 | chr19:41242841-41242868 |
| AXL-304 | chr19:41219268-41219295 | AXL-1449 | chr19:41242853-41242880 |
| AXL-305 | chr19:41219272-41219299 | AXL-1450 | chr19:41242859-41242886 |
| AXL-306 | chr19:41219273-41219300 | AXL-1451 | chr19:41242861-41242888 |
| AXL-307 | chr19:41219274-41219301 | AXL-1452 | chr19:41242865-41242892 |
| AXL-308 | chr19:41219276-41219303 | AXL-1453 | chr19:41242872-41242899 |
| AXL-309 | chr19:41219281-41219308 | AXL-1454 | chr19:41242873-41242900 |
| AXL-310 | chr19:41219287-41219314 | AXL-1455 | chr19:41242874-41242901 |
| AXL-311 | chr19:41219288-41219315 | AXL-1456 | chr19:41242875-41242902 |
| AXL-312 | chr19:41219289-41219316 | AXL-1457 | chr19:41242887-41242914 |
| AXL-313 | chr19:41219290-41219317 | AXL-1458 | chr19:41242891-41242918 |
| AXL-314 | chr19:41219291-41219318 | AXL-1459 | chr19:41242893-41242920 |
| AXL-315 | chr19:41219294-41219321 | AXL-1460 | chr19:41242896-41242923 |
| AXL-316 | chr19:41219295-41219322 | AXL-1461 | chr19:41242900-41242927 |
| AXL-317 | chr19:41219296-41219323 | AXL-1462 | chr19:41242904-41242931 |
| AXL-318 | chr19:41219299-41219326 | AXL-1463 | chr19:41242910-41242937 |
| AXL-319 | chr19:41219300-41219327 | AXL-1464 | chr19:41242915-41242942 |
| AXL-320 | chr19:41219301-41219328 | AXL-1465 | chr19:41242916-41242943 |
| AXL-321 | chr19:41219304-41219331 | AXL-1466 | chr19:41242921-41242948 |
| AXL-322 | chr19:41219310-41219337 | AXL-1467 | chr19:41242945-41242972 |
| AXL-323 | chr19:41219323-41219350 | AXL-1468 | chr19:41242949-41242976 |
| AXL-324 | chr19:41219331-41219358 | AXL-1469 | chr19:41242955-41242982 |
| AXL-325 | chr19:41219332-41219359 | AXL-1470 | chr19:41242962-41242989 |
| AXL-326 | chr19:41219333-41219360 | AXL-1471 | chr19:41242965-41242992 |
| AXL-327 | chr19:41219334-41219361 | AXL-1472 | chr19:41242975-41243002 |
| AXL-328 | chr19:41219338-41219365 | AXL-1473 | chr19:41242980-41243007 |
| AXL-329 | chr19:41219339-41219366 | AXL-1474 | chr19:41242986-41243013 |
| AXL-330 | chr19:41219343-41219370 | AXL-1475 | chr19:41242988-41243015 |
| AXL-331 | chr19:41219344-41219371 | AXL-1476 | chr19:41242989-41243016 |
| AXL-332 | chr19:41219345-41219372 | AXL-1477 | chr19:41242997-41243024 |
| AXL-333 | chr19:41219346-41219373 | AXL-1478 | chr19:41243006-41243033 |
| AXL-334 | chr19:41219349-41219376 | AXL-1479 | chr19:41243007-41243034 |
| AXL-335 | chr19:41219350-41219377 | AXL-1480 | chr19:41243008-41243035 |
| AXL-336 | chr19:41219351-41219378 | AXL-1481 | chr19:41243009-41243036 |
| AXL-337 | chr19:41219352-41219379 | AXL-1482 | chr19:41243012-41243039 |
| AXL-338 | chr19:41219360-41219387 | AXL-1483 | chr19:41243020-41243047 |
| AXL-339 | chr19:41219362-41219389 | AXL-1484 | chr19:41243025-41243052 |
| AXL-340 | chr19:41219363-41219390 | AXL-1485 | chr19:41243026-41243053 |
| AXL-341 | chr19:41219369-41219396 | AXL-1486 | chr19:41243027-41243054 |
| AXL-342 | chr19:41219374-41219401 | AXL-1487 | chr19:41243030-41243057 |
| AXL-343 | chr19:41219377-41219404 | AXL-1488 | chr19:41243035-41243062 |
| AXL-344 | chr19:41219386-41219413 | AXL-1489 | chr19:41243039-41243066 |
| AXL-345 | chr19:41219390-41219417 | AXL-1490 | chr19:41243042-41243069 |
| AXL-346 | chr19:41219391-41219418 | AXL-1491 | chr19:41243046-41243073 |
| AXL-347 | chr19:41219395-41219422 | AXL-1492 | chr19:41243047-41243074 |
| AXL-348 | chr19:41219396-41219423 | AXL-1493 | chr19:41243048-41243075 |
| AXL-349 | chr19:41219405-41219432 | AXL-1494 | chr19:41243050-41243077 |
| AXL-350 | chr19:41219407-41219434 | AXL-1495 | chr19:41243059-41243086 |
| AXL-351 | chr19:41219408-41219435 | AXL-1496 | chr19:41243062-41243089 |
| AXL-352 | chr19:41219409-41219436 | AXL-1497 | chr19:41243066-41243093 |
| AXL-353 | chr19:41219410-41219437 | AXL-1498 | chr19:41243072-41243099 |
| AXL-354 | chr19:41219417-41219444 | AXL-1499 | chr19:41243075-41243102 |
| AXL-355 | chr19:41219425-41219452 | AXL-1500 | chr19:41243078-41243105 |
| AXL-356 | chr19:41219426-41219453 | AXL-1501 | chr19:41243101-41243128 |
| AXL-357 | chr19:41219427-41219454 | AXL-1502 | chr19:41243102-41243129 |
| AXL-358 | chr19:41219431-41219458 | AXL-1503 | chr19:41243107-41243134 |
| AXL-359 | chr19:41219432-41219459 | AXL-1504 | chr19:41243108-41243135 |
| AXL-360 | chr19:41219433-41219460 | AXL-1505 | chr19:41243490-41243517 |
| AXL-361 | chr19:41219441-41219468 | AXL-1506 | chr19:41243494-41243521 |
| AXL-362 | chr19:41219449-41219476 | AXL-1507 | chr19:41243495-41243522 |
| AXL-363 | chr19:41219450-41219477 | AXL-1508 | chr19:41243502-41243529 |
| AXL-364 | chr19:41219451-41219478 | AXL-1509 | chr19:41243511-41243538 |
| AXL-365 | chr19:41219461-41219488 | AXL-1510 | chr19:41243529-41243556 |
| AXL-366 | chr19:41219462-41219489 | AXL-1511 | chr19:41243538-41243565 |
| AXL-367 | chr19:41219463-41219490 | AXL-1512 | chr19:41243539-41243566 |
| AXL-368 | chr19:41219464-41219491 | AXL-1513 | chr19:41243540-41243567 |
| AXL-369 | chr19:41219469-41219496 | AXL-1514 | chr19:41243541-41243568 |
| AXL-370 | chr19:41219470-41219497 | AXL-1515 | chr19:41243551-41243578 |
| AXL-371 | chr19:41219471-41219498 | AXL-1516 | chr19:41243571-41243598 |
| AXL-372 | chr19:41219472-41219499 | AXL-1517 | chr19:41243584-41243611 |
| AXL-373 | chr19:41219473-41219500 | AXL-1518 | chr19:41243585-41243612 |
| AXL-374 | chr19:41219474-41219501 | AXL-1519 | chr19:41243589-41243616 |
| AXL-375 | chr19:41219478-41219505 | AXL-1520 | chr19:41243590-41243617 |
| AXL-376 | chr19:41219479-41219506 | AXL-1521 | chr19:41243595-41243622 |
| AXL-377 | chr19:41219489-41219516 | AXL-1522 | chr19:41243601-41243628 |
| AXL-378 | chr19:41219492-41219519 | AXL-1523 | chr19:41243602-41243629 |
| AXL-379 | chr19:41219493-41219520 | AXL-1524 | chr19:41243603-41243630 |
| AXL-380 | chr19:41219494-41219521 | AXL-1525 | chr19:41243604-41243631 |
| AXL-381 | chr19:41219498-41219525 | AXL-1526 | chr19:41243607-41243634 |
| AXL-382 | chr19:41219499-41219526 | AXL-1527 | chr19:41243608-41243635 |
| AXL-383 | chr19:41219500-41219527 | AXL-1528 | chr19:41243609-41243636 |
| AXL-384 | chr19:41219504-41219531 | AXL-1529 | chr19:41243610-41243637 |
| AXL-385 | chr19:41219505-41219532 | AXL-1530 | chr19:41243611-41243638 |
| AXL-386 | chr19:41219506-41219533 | AXL-1531 | chr19:41243612-41243639 |
| AXL-387 | chr19:41219509-41219536 | AXL-1532 | chr19:41243618-41243645 |
| AXL-388 | chr19:41219510-41219537 | AXL-1533 | chr19:41243626-41243653 |
| AXL-389 | chr19:41219511-41219538 | AXL-1534 | chr19:41243629-41243656 |
| AXL-390 | chr19:41219513-41219540 | AXL-1535 | chr19:41243634-41243661 |
| AXL-391 | chr19:41219516-41219543 | AXL-1536 | chr19:41243664-41243691 |
| AXL-392 | chr19:41219517-41219544 | AXL-1537 | chr19:41243678-41243705 |
| AXL-393 | chr19:41219518-41219545 | AXL-1538 | chr19:41243693-41243720 |
| AXL-394 | chr19:41219519-41219546 | AXL-1539 | chr19:41243705-41243732 |
| AXL-395 | chr19:41219526-41219553 | AXL-1540 | chr19:41243710-41243737 |
| AXL-396 | chr19:41219530-41219557 | AXL-1541 | chr19:41243715-41243742 |
| AXL-397 | chr19:41219535-41219562 | AXL-1542 | chr19:41243717-41243744 |
| AXL-398 | chr19:41219536-41219563 | AXL-1543 | chr19:41243718-41243745 |
| AXL-399 | chr19:41219537-41219564 | AXL-1544 | chr19:41243724-41243751 |
| AXL-400 | chr19:41219538-41219565 | AXL-1545 | chr19:41243725-41243752 |
| AXL-401 | chr19:41219539-41219566 | AXL-1546 | chr19:41243726-41243753 |
| AXL-402 | chr19:41219544-41219571 | AXL-1547 | chr19:41243732-41243759 |
| AXL-403 | chr19:41219545-41219572 | AXL-1548 | chr19:41243733-41243760 |
| AXL-404 | chr19:41219549-41219576 | AXL-1549 | chr19:41243738-41243765 |
| AXL-405 | chr19:41219555-41219582 | AXL-1550 | chr19:41243739-41243766 |
| AXL-406 | chr19:41219556-41219583 | AXL-1551 | chr19:41243747-41243774 |
| AXL-407 | chr19:41219567-41219594 | AXL-1552 | chr19:41243748-41243775 |
| AXL-408 | chr19:41219568-41219595 | AXL-1553 | chr19:41243767-41243794 |
| AXL-409 | chr19:41220511-41220538 | AXL-1554 | chr19:41243783-41243810 |
| AXL-410 | chr19:41220512-41220539 | AXL-1555 | chr19:41243789-41243816 |
| AXL-411 | chr19:41220513-41220540 | AXL-1556 | chr19:41243790-41243817 |
| AXL-412 | chr19:41220519-41220546 | AXL-1557 | chr19:41243794-41243821 |
| AXL-413 | chr19:41220523-41220550 | AXL-1558 | chr19:41248406-41248433 |
| AXL-414 | chr19:41220526-41220553 | AXL-1559 | chr19:41248421-41248448 |
| AXL-415 | chr19:41220529-41220556 | AXL-1560 | chr19:41248424-41248451 |
| AXL-416 | chr19:41220530-41220557 | AXL-1561 | chr19:41248425-41248452 |
| AXL-417 | chr19:41220531-41220558 | AXL-1562 | chr19:41248440-41248467 |
| AXL-418 | chr19:41220534-41220561 | AXL-1563 | chr19:41248441-41248468 |
| AXL-419 | chr19:41220539-41220566 | AXL-1564 | chr19:41248462-41248489 |
| AXL-420 | chr19:41220542-41220569 | AXL-1565 | chr19:41248474-41248501 |
| AXL-421 | chr19:41220544-41220571 | AXL-1566 | chr19:41248475-41248502 |
| AXL-422 | chr19:41220545-41220572 | AXL-1567 | chr19:41248481-41248508 |
| AXL-423 | chr19:41220547-41220574 | AXL-1568 | chr19:41248493-41248520 |
| AXL-424 | chr19:41220548-41220575 | AXL-1569 | chr19:41248496-41248523 |
| AXL-425 | chr19:41220550-41220577 | AXL-1570 | chr19:41248497-41248524 |
| AXL-426 | chr19:41220556-41220583 | AXL-1571 | chr19:41248498-41248525 |
| AXL-427 | chr19:41220557-41220584 | AXL-1572 | chr19:41248499-41248526 |
| AXL-428 | chr19:41220561-41220588 | AXL-1573 | chr19:41248502-41248529 |
| AXL-429 | chr19:41220565-41220592 | AXL-1574 | chr19:41248519-41248546 |
| AXL-430 | chr19:41220571-41220598 | AXL-1575 | chr19:41248521-41248548 |
| AXL-431 | chr19:41220572-41220599 | AXL-1576 | chr19:41248530-41248557 |
| AXL-432 | chr19:41220573-41220600 | AXL-1577 | chr19:41248531-41248558 |
| AXL-433 | chr19:41220574-41220601 | AXL-1578 | chr19:41248540-41248567 |
| AXL-434 | chr19:41220575-41220602 | AXL-1579 | chr19:41248543-41248570 |
| AXL-435 | chr19:41220585-41220612 | AXL-1580 | chr19:41248548-41248575 |
| AXL-436 | chr19:41220605-41220632 | AXL-1581 | chr19:41248555-41248582 |
| AXL-437 | chr19:41220609-41220636 | AXL-1582 | chr19:41248558-41248585 |
| AXL-438 | chr19:41220617-41220644 | AXL-1583 | chr19:41248564-41248591 |
| AXL-439 | chr19:41220620-41220647 | AXL-1584 | chr19:41248565-41248592 |
| AXL-440 | chr19:41220621-41220648 | AXL-1585 | chr19:41248566-41248593 |
| AXL-441 | chr19:41220627-41220654 | AXL-1586 | chr19:41248572-41248599 |
| AXL-442 | chr19:41220628-41220655 | AXL-1587 | chr19:41248576-41248603 |
| AXL-443 | chr19:41220629-41220656 | AXL-1588 | chr19:41248577-41248604 |
| AXL-444 | chr19:41220632-41220659 | AXL-1589 | chr19:41248582-41248609 |
| AXL-445 | chr19:41220638-41220665 | AXL-1590 | chr19:41248583-41248610 |
| AXL-446 | chr19:41220639-41220666 | AXL-1591 | chr19:41248586-41248613 |
| AXL-447 | chr19:41220648-41220675 | AXL-1592 | chr19:41248587-41248614 |
| AXL-448 | chr19:41220649-41220676 | AXL-1593 | chr19:41248595-41248622 |
| AXL-449 | chr19:41220656-41220683 | AXL-1594 | chr19:41248616-41248643 |
| AXL-450 | chr19:41220657-41220684 | AXL-1595 | chr19:41248617-41248644 |
| AXL-451 | chr19:41220660-41220687 | AXL-1596 | chr19:41248618-41248645 |
| AXL-452 | chr19:41220667-41220694 | AXL-1597 | chr19:41248619-41248646 |
| AXL-453 | chr19:41220668-41220695 | AXL-1598 | chr19:41248637-41248664 |
| AXL-454 | chr19:41220669-41220696 | AXL-1599 | chr19:41248656-41248683 |
| AXL-455 | chr19:41220670-41220697 | AXL-1600 | chr19:41248660-41248687 |
| AXL-456 | chr19:41220671-41220698 | AXL-1601 | chr19:41248665-41248692 |
| AXL-457 | chr19:41220677-41220704 | AXL-1602 | chr19:41248672-41248699 |
| AXL-458 | chr19:41220678-41220705 | AXL-1603 | chr19:41248673-41248700 |
| AXL-459 | chr19:41220688-41220715 | AXL-1604 | chr19:41248676-41248703 |
| AXL-460 | chr19:41220690-41220717 | AXL-1605 | chr19:41248677-41248704 |
| AXL-461 | chr19:41220691-41220718 | AXL-1606 | chr19:41248678-41248705 |
| AXL-462 | chr19:41220701-41220728 | AXL-1607 | chr19:41248688-41248715 |
| AXL-463 | chr19:41220707-41220734 | AXL-1608 | chr19:41248697-41248724 |
| AXL-464 | chr19:41220708-41220735 | AXL-1609 | chr19:41248698-41248725 |
| AXL-465 | chr19:41220709-41220736 | AXL-1610 | chr19:41248699-41248726 |
| AXL-466 | chr19:41220722-41220749 | AXL-1611 | chr19:41248705-41248732 |
| AXL-467 | chr19:41220724-41220751 | AXL-1612 | chr19:41248706-41248733 |
| AXL-468 | chr19:41220730-41220757 | AXL-1613 | chr19:41248707-41248734 |
| AXL-469 | chr19:41220736-41220763 | AXL-1614 | chr19:41248716-41248743 |
| AXL-470 | chr19:41220737-41220764 | AXL-1615 | chr19:41248725-41248752 |
| AXL-471 | chr19:41220740-41220767 | AXL-1616 | chr19:41248726-41248753 |
| AXL-472 | chr19:41220741-41220768 | AXL-1617 | chr19:41248733-41248760 |
| AXL-473 | chr19:41220742-41220769 | AXL-1618 | chr19:41248734-41248761 |
| AXL-474 | chr19:41220743-41220770 | AXL-1619 | chr19:41248735-41248762 |
| AXL-475 | chr19:41220744-41220771 | AXL-1620 | chr19:41248736-41248763 |
| AXL-476 | chr19:41220752-41220779 | AXL-1621 | chr19:41248737-41248764 |
| AXL-477 | chr19:41220761-41220788 | AXL-1622 | chr19:41248740-41248767 |
| AXL-478 | chr19:41220775-41220802 | AXL-1623 | chr19:41248754-41248781 |
| AXL-479 | chr19:41220779-41220806 | AXL-1624 | chr19:41248768-41248795 |
| AXL-480 | chr19:41220788-41220815 | AXL-1625 | chr19:41248772-41248799 |
| AXL-481 | chr19:41220789-41220816 | AXL-1626 | chr19:41248775-41248802 |
| AXL-482 | chr19:41220794-41220821 | AXL-1627 | chr19:41248777-41248804 |
| AXL-483 | chr19:41220795-41220822 | AXL-1628 | chr19:41248788-41248815 |
| AXL-484 | chr19:41220796-41220823 | AXL-1629 | chr19:41248792-41248819 |
| AXL-485 | chr19:41220801-41220828 | AXL-1630 | chr19:41248817-41248844 |
| AXL-486 | chr19:41220802-41220829 | AXL-1631 | chr19:41248846-41248873 |
| AXL-487 | chr19:41220803-41220830 | AXL-1632 | chr19:41248847-41248874 |
| AXL-488 | chr19:41220809-41220836 | AXL-1633 | chr19:41248848-41248875 |
| AXL-489 | chr19:41220810-41220837 | AXL-1634 | chr19:41248849-41248876 |
| AXL-490 | chr19:41220811-41220838 | AXL-1635 | chr19:41248855-41248882 |
| AXL-491 | chr19:41220818-41220845 | AXL-1636 | chr19:41248859-41248886 |
| AXL-492 | chr19:41220832-41220859 | AXL-1637 | chr19:41248860-41248887 |
| AXL-493 | chr19:41220839-41220866 | AXL-1638 | chr19:41248866-41248893 |
| AXL-494 | chr19:41220850-41220877 | AXL-1639 | chr19:41248867-41248894 |
| AXL-495 | chr19:41220867-41220894 | AXL-1640 | chr19:41248868-41248895 |
| AXL-496 | chr19:41220873-41220900 | AXL-1641 | chr19:41248889-41248916 |
| AXL-497 | chr19:41220874-41220901 | AXL-1642 | chr19:41248897-41248924 |
| AXL-498 | chr19:41220875-41220902 | AXL-1643 | chr19:41248903-41248930 |
| AXL-499 | chr19:41220880-41220907 | AXL-1644 | chr19:41248909-41248936 |
| AXL-500 | chr19:41220881-41220908 | AXL-1645 | chr19:41252256-41252283 |
| AXL-501 | chr19:41220882-41220909 | AXL-1646 | chr19:41252261-41252288 |
| AXL-502 | chr19:41220887-41220914 | AXL-1647 | chr19:41252264-41252291 |
| AXL-503 | chr19:41220888-41220915 | AXL-1648 | chr19:41252267-41252294 |
| AXL-504 | chr19:41220891-41220918 | AXL-1649 | chr19:41252270-41252297 |
| AXL-505 | chr19:41220894-41220921 | AXL-1650 | chr19:41252271-41252298 |
| AXL-506 | chr19:41220897-41220924 | AXL-1651 | chr19:41252304-41252331 |
| AXL-507 | chr19:41220898-41220925 | AXL-1652 | chr19:41252307-41252334 |
| AXL-508 | chr19:41220901-41220928 | AXL-1653 | chr19:41252308-41252335 |
| AXL-509 | chr19:41220902-41220929 | AXL-1654 | chr19:41252313-41252340 |
| AXL-510 | chr19:41220916-41220943 | AXL-1655 | chr19:41252316-41252343 |
| AXL-511 | chr19:41220917-41220944 | AXL-1656 | chr19:41252317-41252344 |
| AXL-512 | chr19:41220920-41220947 | AXL-1657 | chr19:41252318-41252345 |
| AXL-513 | chr19:41220932-41220959 | AXL-1658 | chr19:41252321-41252348 |
| AXL-514 | chr19:41220933-41220960 | AXL-1659 | chr19:41252324-41252351 |
| AXL-515 | chr19:41220934-41220961 | AXL-1660 | chr19:41252332-41252359 |
| AXL-516 | chr19:41220943-41220970 | AXL-1661 | chr19:41252340-41252367 |
| AXL-517 | chr19:41220951-41220978 | AXL-1662 | chr19:41252343-41252370 |
| AXL-518 | chr19:41220954-41220981 | AXL-1663 | chr19:41252344-41252371 |
| AXL-519 | chr19:41221034-41221061 | AXL-1664 | chr19:41252350-41252377 |
| AXL-520 | chr19:41221053-41221080 | AXL-1665 | chr19:41252353-41252380 |
| AXL-521 | chr19:41221065-41221092 | AXL-1666 | chr19:41252354-41252381 |
| AXL-522 | chr19:41221066-41221093 | AXL-1667 | chr19:41252371-41252398 |
| AXL-523 | chr19:41221073-41221100 | AXL-1668 | chr19:41252383-41252410 |
| AXL-524 | chr19:41221086-41221113 | AXL-1669 | chr19:41252385-41252412 |
| AXL-525 | chr19:41221087-41221114 | AXL-1670 | chr19:41252409-41252436 |
| AXL-526 | chr19:41221088-41221115 | AXL-1671 | chr19:41252417-41252444 |
| AXL-527 | chr19:41221089-41221116 | AXL-1672 | chr19:41252418-41252445 |
| AXL-528 | chr19:41221092-41221119 | AXL-1673 | chr19:41252422-41252449 |
| AXL-529 | chr19:41221093-41221120 | AXL-1674 | chr19:41252423-41252450 |
| AXL-530 | chr19:41221100-41221127 | AXL-1675 | chr19:41252426-41252453 |
| AXL-531 | chr19:41221110-41221137 | AXL-1676 | chr19:41252427-41252454 |
| AXL-532 | chr19:41221111-41221138 | AXL-1677 | chr19:41252428-41252455 |
| AXL-533 | chr19:41221117-41221144 | AXL-1678 | chr19:41252439-41252466 |
| AXL-534 | chr19:41221130-41221157 | AXL-1679 | chr19:41252440-41252467 |
| AXL-535 | chr19:41221137-41221164 | AXL-1680 | chr19:41252441-41252468 |
| AXL-536 | chr19:41221138-41221165 | AXL-1681 | chr19:41252457-41252484 |
| AXL-537 | chr19:41221147-41221174 | AXL-1682 | chr19:41252458-41252485 |
| AXL-538 | chr19:41221148-41221175 | AXL-1683 | chr19:41252459-41252486 |
| AXL-539 | chr19:41221151-41221178 | AXL-1684 | chr19:41252460-41252487 |
| AXL-540 | chr19:41221154-41221181 | AXL-1685 | chr19:41252472-41252499 |
| AXL-541 | chr19:41221155-41221182 | AXL-1686 | chr19:41252473-41252500 |
| AXL-542 | chr19:41221165-41221192 | AXL-1687 | chr19:41252477-41252504 |
| AXL-543 | chr19:41221166-41221193 | AXL-1688 | chr19:41252478-41252505 |
| AXL-544 | chr19:41221174-41221201 | AXL-1689 | chr19:41252479-41252506 |
| AXL-545 | chr19:41221175-41221202 | AXL-1690 | chr19:41252488-41252515 |
| AXL-546 | chr19:41221182-41221209 | AXL-1691 | chr19:41252490-41252517 |
| AXL-547 | chr19:41221202-41221229 | AXL-1692 | chr19:41252496-41252523 |
| AXL-548 | chr19:41221211-41221238 | AXL-1693 | chr19:41252497-41252524 |
| AXL-549 | chr19:41221212-41221239 | AXL-1694 | chr19:41252506-41252533 |
| AXL-550 | chr19:41221216-41221243 | AXL-1695 | chr19:41252507-41252534 |
| AXL-551 | chr19:41221219-41221246 | AXL-1696 | chr19:41252514-41252541 |
| AXL-552 | chr19:41221220-41221247 | AXL-1697 | chr19:41252518-41252545 |
| AXL-553 | chr19:41221221-41221248 | AXL-1698 | chr19:41252519-41252546 |
| AXL-554 | chr19:41221225-41221252 | AXL-1699 | chr19:41252537-41252564 |
| AXL-555 | chr19:41221230-41221257 | AXL-1700 | chr19:41252538-41252565 |
| AXL-556 | chr19:41221231-41221258 | AXL-1701 | chr19:41252539-41252566 |
| AXL-557 | chr19:41221232-41221259 | AXL-1702 | chr19:41252722-41252749 |
| AXL-558 | chr19:41221237-41221264 | AXL-1703 | chr19:41252723-41252750 |
| AXL-559 | chr19:41221238-41221265 | AXL-1704 | chr19:41252724-41252751 |
| AXL-560 | chr19:41221239-41221266 | AXL-1705 | chr19:41252725-41252752 |
| AXL-561 | chr19:41221247-41221274 | AXL-1706 | chr19:41252726-41252753 |
| AXL-562 | chr19:41221248-41221275 | AXL-1707 | chr19:41252727-41252754 |
| AXL-563 | chr19:41221249-41221276 | AXL-1708 | chr19:41252738-41252765 |
| AXL-564 | chr19:41221256-41221283 | AXL-1709 | chr19:41252741-41252768 |
| AXL-565 | chr19:41221264-41221291 | AXL-1710 | chr19:41252743-41252770 |
| AXL-566 | chr19:41221268-41221295 | AXL-1711 | chr19:41252747-41252774 |
| AXL-567 | chr19:41221274-41221301 | AXL-1712 | chr19:41252750-41252777 |
| AXL-568 | chr19:41221280-41221307 | AXL-1713 | chr19:41252754-41252781 |
| AXL-569 | chr19:41221281-41221308 | AXL-1714 | chr19:41252758-41252785 |
| AXL-570 | chr19:41221289-41221316 | AXL-1715 | chr19:41252761-41252788 |
| AXL-571 | chr19:41221299-41221326 | AXL-1716 | chr19:41252762-41252789 |
| AXL-572 | chr19:41221300-41221327 | AXL-1717 | chr19:41252763-41252790 |
| AXL-573 | chr19:41221301-41221328 | AXL-1718 | chr19:41252764-41252791 |
| AXL-574 | chr19:41221307-41221334 | AXL-1719 | chr19:41252765-41252792 |
| AXL-575 | chr19:41221314-41221341 | AXL-1720 | chr19:41252770-41252797 |
| AXL-576 | chr19:41221315-41221342 | AXL-1721 | chr19:41252791-41252818 |
| AXL-577 | chr19:41221320-41221347 | AXL-1722 | chr19:41252800-41252827 |
| AXL-578 | chr19:41221321-41221348 | AXL-1723 | chr19:41252801-41252828 |
| AXL-579 | chr19:41221324-41221351 | AXL-1724 | chr19:41252802-41252829 |
| AXL-580 | chr19:41221331-41221358 | AXL-1725 | chr19:41252803-41252830 |
| AXL-581 | chr19:41221754-41221781 | AXL-1726 | chr19:41252809-41252836 |
| AXL-582 | chr19:41221755-41221782 | AXL-1727 | chr19:41252810-41252837 |
| AXL-583 | chr19:41221758-41221785 | AXL-1728 | chr19:41252811-41252838 |
| AXL-584 | chr19:41221759-41221786 | AXL-1729 | chr19:41252812-41252839 |
| AXL-585 | chr19:41221773-41221800 | AXL-1730 | chr19:41252813-41252840 |
| AXL-586 | chr19:41221774-41221801 | AXL-1731 | chr19:41252819-41252846 |
| AXL-587 | chr19:41221791-41221818 | AXL-1732 | chr19:41252832-41252859 |
| AXL-588 | chr19:41221794-41221821 | AXL-1733 | chr19:41252833-41252860 |
| AXL-589 | chr19:41221795-41221822 | AXL-1734 | chr19:41252834-41252861 |
| AXL-590 | chr19:41221800-41221827 | AXL-1735 | chr19:41252856-41252883 |
| AXL-591 | chr19:41221801-41221828 | AXL-1736 | chr19:41252866-41252893 |
| AXL-592 | chr19:41221808-41221835 | AXL-1737 | chr19:41252880-41252907 |
| AXL-593 | chr19:41221809-41221836 | AXL-1738 | chr19:41252881-41252908 |
| AXL-594 | chr19:41221810-41221837 | AXL-1739 | chr19:41252887-41252914 |
| AXL-595 | chr19:41221811-41221838 | AXL-1740 | chr19:41252891-41252918 |
| AXL-596 | chr19:41221819-41221846 | AXL-1741 | chr19:41252902-41252929 |
| AXL-597 | chr19:41221820-41221847 | AXL-1742 | chr19:41252922-41252949 |
| AXL-598 | chr19:41221838-41221865 | AXL-1743 | chr19:41252927-41252954 |
| AXL-599 | chr19:41221849-41221876 | AXL-1744 | chr19:41252928-41252955 |
| AXL-600 | chr19:41221850-41221877 | AXL-1745 | chr19:41252929-41252956 |
| AXL-601 | chr19:41221851-41221878 | AXL-1746 | chr19:41252934-41252961 |
| AXL-602 | chr19:41221853-41221880 | AXL-1747 | chr19:41252937-41252964 |
| AXL-603 | chr19:41221855-41221882 | AXL-1748 | chr19:41252941-41252968 |
| AXL-604 | chr19:41221861-41221888 | AXL-1749 | chr19:41252945-41252972 |
| AXL-605 | chr19:41221867-41221894 | AXL-1750 | chr19:41252946-41252973 |
| AXL-606 | chr19:41221868-41221895 | AXL-1751 | chr19:41252947-41252974 |
| AXL-607 | chr19:41221870-41221897 | AXL-1752 | chr19:41252948-41252975 |
| AXL-608 | chr19:41221871-41221898 | AXL-1753 | chr19:41252958-41252985 |
| AXL-609 | chr19:41221872-41221899 | AXL-1754 | chr19:41252962-41252989 |
| AXL-610 | chr19:41221873-41221900 | AXL-1755 | chr19:41252993-41253020 |
| AXL-611 | chr19:41221885-41221912 | AXL-1756 | chr19:41253017-41253044 |
| AXL-612 | chr19:41221887-41221914 | AXL-1757 | chr19:41253018-41253045 |
| AXL-613 | chr19:41221893-41221920 | AXL-1758 | chr19:41253023-41253050 |
| AXL-614 | chr19:41221894-41221921 | AXL-1759 | chr19:41253024-41253051 |
| AXL-615 | chr19:41221903-41221930 | AXL-1760 | chr19:41253028-41253055 |
| AXL-616 | chr19:41221904-41221931 | AXL-1761 | chr19:41253036-41253063 |
| AXL-617 | chr19:41221922-41221949 | AXL-1762 | chr19:41253039-41253066 |
| AXL-618 | chr19:41221925-41221952 | AXL-1763 | chr19:41253040-41253067 |
| AXL-619 | chr19:41221931-41221958 | AXL-1764 | chr19:41253050-41253077 |
| AXL-620 | chr19:41221932-41221959 | AXL-1765 | chr19:41253051-41253078 |
| AXL-621 | chr19:41221933-41221960 | AXL-1766 | chr19:41253059-41253086 |
| AXL-622 | chr19:41221934-41221961 | AXL-1767 | chr19:41253480-41253507 |
| AXL-623 | chr19:41221941-41221968 | AXL-1768 | chr19:41253481-41253508 |
| AXL-624 | chr19:41221950-41221977 | AXL-1769 | chr19:41253489-41253516 |
| AXL-625 | chr19:41221951-41221978 | AXL-1770 | chr19:41253491-41253518 |
| AXL-626 | chr19:41221962-41221989 | AXL-1771 | chr19:41253499-41253526 |
| AXL-627 | chr19:41221963-41221990 | AXL-1772 | chr19:41253503-41253530 |
| AXL-628 | chr19:41221964-41221991 | AXL-1773 | chr19:41253504-41253531 |
| AXL-629 | chr19:41221965-41221992 | AXL-1774 | chr19:41253508-41253535 |
| AXL-630 | chr19:41221966-41221993 | AXL-1775 | chr19:41253511-41253538 |
| AXL-631 | chr19:41221969-41221996 | AXL-1776 | chr19:41253512-41253539 |
| AXL-632 | chr19:41221972-41221999 | AXL-1777 | chr19:41253515-41253542 |
| AXL-633 | chr19:41221973-41222000 | AXL-1778 | chr19:41253518-41253545 |
| AXL-634 | chr19:41221980-41222007 | AXL-1779 | chr19:41253519-41253546 |
| AXL-635 | chr19:41221984-41222011 | AXL-1780 | chr19:41253531-41253558 |
| AXL-636 | chr19:41221990-41222017 | AXL-1781 | chr19:41253563-41253590 |
| AXL-637 | chr19:41221992-41222019 | AXL-1782 | chr19:41253569-41253596 |
| AXL-638 | chr19:41221997-41222024 | AXL-1783 | chr19:41253574-41253601 |
| AXL-639 | chr19:41222003-41222030 | AXL-1784 | chr19:41253577-41253604 |
| AXL-640 | chr19:41222004-41222031 | AXL-1785 | chr19:41253578-41253605 |
| AXL-641 | chr19:41222005-41222032 | AXL-1786 | chr19:41253581-41253608 |
| AXL-642 | chr19:41222006-41222033 | AXL-1787 | chr19:41253585-41253612 |
| AXL-643 | chr19:41222007-41222034 | AXL-1788 | chr19:41253593-41253620 |
| AXL-644 | chr19:41222012-41222039 | AXL-1789 | chr19:41253594-41253621 |
| AXL-645 | chr19:41222020-41222047 | AXL-1790 | chr19:41253595-41253622 |
| AXL-646 | chr19:41222030-41222057 | AXL-1791 | chr19:41253601-41253628 |
| AXL-647 | chr19:41222031-41222058 | AXL-1792 | chr19:41253605-41253632 |
| AXL-648 | chr19:41222032-41222059 | AXL-1793 | chr19:41253606-41253633 |
| AXL-649 | chr19:41222033-41222060 | AXL-1794 | chr19:41253621-41253648 |
| AXL-650 | chr19:41222034-41222061 | AXL-1795 | chr19:41253626-41253653 |
| AXL-651 | chr19:41222039-41222066 | AXL-1796 | chr19:41253642-41253669 |
| AXL-652 | chr19:41222040-41222067 | AXL-1797 | chr19:41253658-41253685 |
| AXL-653 | chr19:41222041-41222068 | AXL-1798 | chr19:41253659-41253686 |
| AXL-654 | chr19:41222043-41222070 | AXL-1799 | chr19:41253665-41253692 |
| AXL-655 | chr19:41222047-41222074 | AXL-1800 | chr19:41253666-41253693 |
| AXL-656 | chr19:41222048-41222075 | AXL-1801 | chr19:41253668-41253695 |
| AXL-657 | chr19:41222053-41222080 | AXL-1802 | chr19:41253673-41253700 |
| AXL-658 | chr19:41222063-41222090 | AXL-1803 | chr19:41253682-41253709 |
| AXL-659 | chr19:41222064-41222091 | AXL-1804 | chr19:41253688-41253715 |
| AXL-660 | chr19:41222065-41222092 | AXL-1805 | chr19:41253696-41253723 |
| AXL-661 | chr19:41222066-41222093 | AXL-1806 | chr19:41253697-41253724 |
| AXL-662 | chr19:41222070-41222097 | AXL-1807 | chr19:41253698-41253725 |
| AXL-663 | chr19:41222071-41222098 | AXL-1808 | chr19:41253716-41253743 |
| AXL-664 | chr19:41222075-41222102 | AXL-1809 | chr19:41253727-41253754 |
| AXL-665 | chr19:41222076-41222103 | AXL-1810 | chr19:41253728-41253755 |
| AXL-666 | chr19:41222082-41222109 | AXL-1811 | chr19:41253729-41253756 |
| AXL-667 | chr19:41222087-41222114 | AXL-1812 | chr19:41253730-41253757 |
| AXL-668 | chr19:41222094-41222121 | AXL-1813 | chr19:41253731-41253758 |
| AXL-669 | chr19:41222095-41222122 | AXL-1814 | chr19:41253732-41253759 |
| AXL-670 | chr19:41222133-41222160 | AXL-1815 | chr19:41253733-41253760 |
| AXL-671 | chr19:41222156-41222183 | AXL-1816 | chr19:41253734-41253761 |
| AXL-672 | chr19:41225230-41225257 | AXL-1817 | chr19:41253735-41253762 |
| AXL-673 | chr19:41225231-41225258 | AXL-1818 | chr19:41253736-41253763 |
| AXL-674 | chr19:41225235-41225262 | AXL-1819 | chr19:41253737-41253764 |
| AXL-675 | chr19:41225246-41225273 | AXL-1820 | chr19:41253746-41253773 |
| AXL-676 | chr19:41225247-41225274 | AXL-1821 | chr19:41253754-41253781 |
| AXL-677 | chr19:41225248-41225275 | AXL-1822 | chr19:41253755-41253782 |
| AXL-678 | chr19:41225249-41225276 | AXL-1823 | chr19:41253757-41253784 |
| AXL-679 | chr19:41225253-41225280 | AXL-1824 | chr19:41253761-41253788 |
| AXL-680 | chr19:41225256-41225283 | AXL-1825 | chr19:41253767-41253794 |
| AXL-681 | chr19:41225259-41225286 | AXL-1826 | chr19:41253768-41253795 |
| AXL-682 | chr19:41225262-41225289 | AXL-1827 | chr19:41253769-41253796 |
| AXL-683 | chr19:41225263-41225290 | AXL-1828 | chr19:41253771-41253798 |
| AXL-684 | chr19:41225290-41225317 | AXL-1829 | chr19:41253772-41253799 |
| AXL-685 | chr19:41225293-41225320 | AXL-1830 | chr19:41253775-41253802 |
| AXL-686 | chr19:41225298-41225325 | AXL-1831 | chr19:41253786-41253813 |
| AXL-687 | chr19:41225299-41225326 | AXL-1832 | chr19:41253787-41253814 |
| AXL-688 | chr19:41225308-41225335 | AXL-1833 | chr19:41253791-41253818 |
| AXL-689 | chr19:41225309-41225336 | AXL-1834 | chr19:41253803-41253830 |
| AXL-690 | chr19:41225325-41225352 | AXL-1835 | chr19:41253804-41253831 |
| AXL-691 | chr19:41225326-41225353 | AXL-1836 | chr19:41253805-41253832 |
| AXL-692 | chr19:41225348-41225375 | AXL-1837 | chr19:41256332-41256359 |
| AXL-693 | chr19:41225357-41225384 | AXL-1838 | chr19:41256333-41256360 |
| AXL-694 | chr19:41225359-41225386 | AXL-1839 | chr19:41256334-41256361 |
| AXL-695 | chr19:41225360-41225387 | AXL-1840 | chr19:41256335-41256362 |
| AXL-696 | chr19:41225365-41225392 | AXL-1841 | chr19:41256336-41256363 |
| AXL-697 | chr19:41225373-41225400 | AXL-1842 | chr19:41256337-41256364 |
| AXL-698 | chr19:41225385-41225412 | AXL-1843 | chr19:41256339-41256366 |
| AXL-699 | chr19:41225405-41225432 | AXL-1844 | chr19:41256340-41256367 |
| AXL-700 | chr19:41225432-41225459 | AXL-1845 | chr19:41256346-41256373 |
| AXL-701 | chr19:41225442-41225469 | AXL-1846 | chr19:41256363-41256390 |
| AXL-702 | chr19:41225443-41225470 | AXL-1847 | chr19:41256364-41256391 |
| AXL-703 | chr19:41225455-41225482 | AXL-1848 | chr19:41256368-41256395 |
| AXL-704 | chr19:41225475-41225502 | AXL-1849 | chr19:41256376-41256403 |
| AXL-705 | chr19:41225478-41225505 | AXL-1850 | chr19:41256379-41256406 |
| AXL-706 | chr19:41225486-41225513 | AXL-1851 | chr19:41256382-41256409 |
| AXL-707 | chr19:41225500-41225527 | AXL-1852 | chr19:41256386-41256413 |
| AXL-708 | chr19:41225501-41225528 | AXL-1853 | chr19:41256399-41256426 |
| AXL-709 | chr19:41225526-41225553 | AXL-1854 | chr19:41256419-41256446 |
| AXL-710 | chr19:41225557-41225584 | AXL-1855 | chr19:41256420-41256447 |
| AXL-711 | chr19:41225560-41225587 | AXL-1856 | chr19:41256425-41256452 |
| AXL-712 | chr19:41225615-41225642 | AXL-1857 | chr19:41256426-41256453 |
| AXL-713 | chr19:41225633-41225660 | AXL-1858 | chr19:41256432-41256459 |
| AXL-714 | chr19:41225634-41225661 | AXL-1859 | chr19:41256437-41256464 |
| AXL-715 | chr19:41225685-41225712 | AXL-1860 | chr19:41256438-41256465 |
| AXL-716 | chr19:41225687-41225714 | AXL-1861 | chr19:41256439-41256466 |
| AXL-717 | chr19:41225688-41225715 | AXL-1862 | chr19:41256444-41256471 |
| AXL-718 | chr19:41225744-41225771 | AXL-1863 | chr19:41256453-41256480 |
| AXL-719 | chr19:41225745-41225772 | AXL-1864 | chr19:41256456-41256483 |
| AXL-720 | chr19:41225762-41225789 | AXL-1865 | chr19:41256463-41256490 |
| AXL-721 | chr19:41225773-41225800 | AXL-1866 | chr19:41256464-41256491 |
| AXL-722 | chr19:41225804-41225831 | AXL-1867 | chr19:41256469-41256496 |
| AXL-723 | chr19:41225805-41225832 | AXL-1868 | chr19:41256487-41256514 |
| AXL-724 | chr19:41225806-41225833 | AXL-1869 | chr19:41256488-41256515 |
| AXL-725 | chr19:41225819-41225846 | AXL-1870 | chr19:41256489-41256516 |
| AXL-726 | chr19:41225820-41225847 | AXL-1871 | chr19:41256496-41256523 |
| AXL-727 | chr19:41225821-41225848 | AXL-1872 | chr19:41256504-41256531 |
| AXL-728 | chr19:41225822-41225849 | AXL-1873 | chr19:41256505-41256532 |
| AXL-729 | chr19:41225823-41225850 | AXL-1874 | chr19:41256524-41256551 |
| AXL-730 | chr19:41225824-41225851 | AXL-1875 | chr19:41256527-41256554 |
| AXL-731 | chr19:41225839-41225866 | AXL-1876 | chr19:41256533-41256560 |
| AXL-732 | chr19:41225875-41225902 | AXL-1877 | chr19:41256541-41256568 |
| AXL-733 | chr19:41225883-41225910 | AXL-1878 | chr19:41256549-41256576 |
| AXL-734 | chr19:41225884-41225911 | AXL-1879 | chr19:41256565-41256592 |
| AXL-735 | chr19:41225893-41225920 | AXL-1880 | chr19:41256584-41256611 |
| AXL-736 | chr19:41225894-41225921 | AXL-1881 | chr19:41256585-41256612 |
| AXL-737 | chr19:41225916-41225943 | AXL-1882 | chr19:41256589-41256616 |
| AXL-738 | chr19:41225917-41225944 | AXL-1883 | chr19:41256595-41256622 |
| AXL-739 | chr19:41225918-41225945 | AXL-1884 | chr19:41256609-41256636 |
| AXL-740 | chr19:41225920-41225947 | AXL-1885 | chr19:41256610-41256637 |
| AXL-741 | chr19:41225921-41225948 | AXL-1886 | chr19:41256611-41256638 |
| AXL-742 | chr19:41225924-41225951 | AXL-1887 | chr19:41256619-41256646 |
| AXL-743 | chr19:41225928-41225955 | AXL-1888 | chr19:41256620-41256647 |
| AXL-744 | chr19:41225929-41225956 | AXL-1889 | chr19:41256623-41256650 |
| AXL-745 | chr19:41225930-41225957 | AXL-1890 | chr19:41256624-41256651 |
| AXL-746 | chr19:41225931-41225958 | AXL-1891 | chr19:41256627-41256654 |
| AXL-747 | chr19:41225932-41225959 | AXL-1892 | chr19:41256629-41256656 |
| AXL-748 | chr19:41225933-41225960 | AXL-1893 | chr19:41256641-41256668 |
| AXL-749 | chr19:41225934-41225961 | AXL-1894 | chr19:41256654-41256681 |
| AXL-750 | chr19:41225942-41225969 | AXL-1895 | chr19:41256655-41256682 |
| AXL-751 | chr19:41225943-41225970 | AXL-1896 | chr19:41256657-41256684 |
| AXL-752 | chr19:41225944-41225971 | AXL-1897 | chr19:41256658-41256685 |
| AXL-753 | chr19:41225950-41225977 | AXL-1898 | chr19:41256662-41256689 |
| AXL-754 | chr19:41225951-41225978 | AXL-1899 | chr19:41256664-41256691 |
| AXL-755 | chr19:41225952-41225979 | AXL-1900 | chr19:41256672-41256699 |
| AXL-756 | chr19:41225953-41225980 | AXL-1901 | chr19:41256673-41256700 |
| AXL-757 | chr19:41225954-41225981 | AXL-1902 | chr19:41256677-41256704 |
| AXL-758 | chr19:41225958-41225985 | AXL-1903 | chr19:41256680-41256707 |
| AXL-759 | chr19:41225963-41225990 | AXL-1904 | chr19:41256681-41256708 |
| AXL-760 | chr19:41225971-41225998 | AXL-1905 | chr19:41256689-41256716 |
| AXL-761 | chr19:41225984-41226011 | AXL-1906 | chr19:41256690-41256717 |
| AXL-762 | chr19:41225990-41226017 | AXL-1907 | chr19:41256704-41256731 |
| AXL-763 | chr19:41226011-41226038 | AXL-1908 | chr19:41256705-41256732 |
| AXL-764 | chr19:41226018-41226045 | AXL-1909 | chr19:41256706-41256733 |
| AXL-765 | chr19:41226024-41226051 | AXL-1910 | chr19:41257378-41257405 |
| AXL-766 | chr19:41226030-41226057 | AXL-1911 | chr19:41257379-41257406 |
| AXL-767 | chr19:41226033-41226060 | AXL-1912 | chr19:41257388-41257415 |
| AXL-768 | chr19:41226034-41226061 | AXL-1913 | chr19:41257389-41257416 |
| AXL-769 | chr19:41226035-41226062 | AXL-1914 | chr19:41257406-41257433 |
| AXL-770 | chr19:41226039-41226066 | AXL-1915 | chr19:41257407-41257434 |
| AXL-771 | chr19:41226040-41226067 | AXL-1916 | chr19:41257413-41257440 |
| AXL-772 | chr19:41226041-41226068 | AXL-1917 | chr19:41257418-41257445 |
| AXL-773 | chr19:41226050-41226077 | AXL-1918 | chr19:41257419-41257446 |
| AXL-774 | chr19:41226070-41226097 | AXL-1919 | chr19:41257421-41257448 |
| AXL-775 | chr19:41226071-41226098 | AXL-1920 | chr19:41257422-41257449 |
| AXL-776 | chr19:41226072-41226099 | AXL-1921 | chr19:41257429-41257456 |
| AXL-777 | chr19:41226077-41226104 | AXL-1922 | chr19:41257433-41257460 |
| AXL-778 | chr19:41226080-41226107 | AXL-1923 | chr19:41257468-41257495 |
| AXL-779 | chr19:41226084-41226111 | AXL-1924 | chr19:41257476-41257503 |
| AXL-780 | chr19:41226085-41226112 | AXL-1925 | chr19:41257477-41257504 |
| AXL-781 | chr19:41226086-41226113 | AXL-1926 | chr19:41257478-41257505 |
| AXL-782 | chr19:41226087-41226114 | AXL-1927 | chr19:41257483-41257510 |
| AXL-783 | chr19:41226091-41226118 | AXL-1928 | chr19:41257484-41257511 |
| AXL-784 | chr19:41226092-41226119 | AXL-1929 | chr19:41257485-41257512 |
| AXL-785 | chr19:41226096-41226123 | AXL-1930 | chr19:41257489-41257516 |
| AXL-786 | chr19:41226097-41226124 | AXL-1931 | chr19:41257490-41257517 |
| AXL-787 | chr19:41226098-41226125 | AXL-1932 | chr19:41257497-41257524 |
| AXL-788 | chr19:41226099-41226126 | AXL-1933 | chr19:41257506-41257533 |
| AXL-789 | chr19:41226105-41226132 | AXL-1934 | chr19:41257523-41257550 |
| AXL-790 | chr19:41226106-41226133 | AXL-1935 | chr19:41257524-41257551 |
| AXL-791 | chr19:41226112-41226139 | AXL-1936 | chr19:41257529-41257556 |
| AXL-792 | chr19:41226114-41226141 | AXL-1937 | chr19:41257534-41257561 |
| AXL-793 | chr19:41226129-41226156 | AXL-1938 | chr19:41257539-41257566 |
| AXL-794 | chr19:41226130-41226157 | AXL-1939 | chr19:41257540-41257567 |
| AXL-795 | chr19:41226132-41226159 | AXL-1940 | chr19:41257541-41257568 |
| AXL-796 | chr19:41226136-41226163 | AXL-1941 | chr19:41257547-41257574 |
| AXL-797 | chr19:41226142-41226169 | AXL-1942 | chr19:41257562-41257589 |
| AXL-798 | chr19:41226145-41226172 | AXL-1943 | chr19:41257563-41257590 |
| AXL-799 | chr19:41226146-41226173 | AXL-1944 | chr19:41257585-41257612 |
| AXL-800 | chr19:41226147-41226174 | AXL-1945 | chr19:41257586-41257613 |
| AXL-801 | chr19:41226148-41226175 | AXL-1946 | chr19:41257595-41257622 |
| AXL-802 | chr19:41226149-41226176 | AXL-1947 | chr19:41257597-41257624 |
| AXL-803 | chr19:41226150-41226177 | AXL-1948 | chr19:41257599-41257626 |
| AXL-804 | chr19:41226151-41226178 | AXL-1949 | chr19:41257607-41257634 |
| AXL-805 | chr19:41226152-41226179 | AXL-1950 | chr19:41257608-41257635 |
| AXL-806 | chr19:41226155-41226182 | AXL-1951 | chr19:41257617-41257644 |
| AXL-807 | chr19:41226156-41226183 | AXL-1952 | chr19:41257637-41257664 |
| AXL-808 | chr19:41226160-41226187 | AXL-1953 | chr19:41257638-41257665 |
| AXL-809 | chr19:41226164-41226191 | AXL-1954 | chr19:41257648-41257675 |
| AXL-810 | chr19:41226176-41226203 | AXL-1955 | chr19:41257649-41257676 |
| AXL-811 | chr19:41226177-41226204 | AXL-1956 | chr19:41257650-41257677 |
| AXL-812 | chr19:41226178-41226205 | AXL-1957 | chr19:41257651-41257678 |
| AXL-813 | chr19:41226179-41226206 | AXL-1958 | chr19:41257655-41257682 |
| AXL-814 | chr19:41226183-41226210 | AXL-1959 | chr19:41257656-41257683 |
| AXL-815 | chr19:41226185-41226212 | AXL-1960 | chr19:41257666-41257693 |
| AXL-816 | chr19:41226186-41226213 | AXL-1961 | chr19:41257668-41257695 |
| AXL-817 | chr19:41226187-41226214 | AXL-1962 | chr19:41257673-41257700 |
| AXL-818 | chr19:41226188-41226215 | AXL-1963 | chr19:41257674-41257701 |
| AXL-819 | chr19:41226193-41226220 | AXL-1964 | chr19:41257675-41257702 |
| AXL-820 | chr19:41226197-41226224 | AXL-1965 | chr19:41257676-41257703 |
| AXL-821 | chr19:41226202-41226229 | AXL-1966 | chr19:41257682-41257709 |
| AXL-822 | chr19:41226203-41226230 | AXL-1967 | chr19:41257683-41257710 |
| AXL-823 | chr19:41226209-41226236 | AXL-1968 | chr19:41257684-41257711 |
| AXL-824 | chr19:41226232-41226259 | AXL-1969 | chr19:41257685-41257712 |
| AXL-825 | chr19:41226233-41226260 | AXL-1970 | chr19:41257686-41257713 |
| AXL-826 | chr19:41226235-41226262 | AXL-1971 | chr19:41257694-41257721 |
| AXL-827 | chr19:41226236-41226263 | AXL-1972 | chr19:41257695-41257722 |
| AXL-828 | chr19:41226239-41226266 | AXL-1973 | chr19:41257705-41257732 |
| AXL-829 | chr19:41226245-41226272 | AXL-1974 | chr19:41257721-41257748 |
| AXL-830 | chr19:41226246-41226273 | AXL-1975 | chr19:41257722-41257749 |
| AXL-831 | chr19:41226249-41226276 | AXL-1976 | chr19:41259434-41259461 |
| AXL-832 | chr19:41226252-41226279 | AXL-1977 | chr19:41259435-41259462 |
| AXL-833 | chr19:41226256-41226283 | AXL-1978 | chr19:41259442-41259469 |
| AXL-834 | chr19:41226257-41226284 | AXL-1979 | chr19:41259443-41259470 |
| AXL-835 | chr19:41226258-41226285 | AXL-1980 | chr19:41259454-41259481 |
| AXL-836 | chr19:41226269-41226296 | AXL-1981 | chr19:41259455-41259482 |
| AXL-837 | chr19:41226273-41226300 | AXL-1982 | chr19:41259456-41259483 |
| AXL-838 | chr19:41226278-41226305 | AXL-1983 | chr19:41259457-41259484 |
| AXL-839 | chr19:41226281-41226308 | AXL-1984 | chr19:41259462-41259489 |
| AXL-840 | chr19:41226282-41226309 | AXL-1985 | chr19:41259463-41259490 |
| AXL-841 | chr19:41226283-41226310 | AXL-1986 | chr19:41259464-41259491 |
| AXL-842 | chr19:41226288-41226315 | AXL-1987 | chr19:41259473-41259500 |
| AXL-843 | chr19:41226289-41226316 | AXL-1988 | chr19:41259483-41259510 |
| AXL-844 | chr19:41226297-41226324 | AXL-1989 | chr19:41259484-41259511 |
| AXL-845 | chr19:41226300-41226327 | AXL-1990 | chr19:41259485-41259512 |
| AXL-846 | chr19:41226301-41226328 | AXL-1991 | chr19:41259493-41259520 |
| AXL-847 | chr19:41226304-41226331 | AXL-1992 | chr19:41259503-41259530 |
| AXL-848 | chr19:41226311-41226338 | AXL-1993 | chr19:41259504-41259531 |
| AXL-849 | chr19:41226312-41226339 | AXL-1994 | chr19:41259505-41259532 |
| AXL-850 | chr19:41226313-41226340 | AXL-1995 | chr19:41259515-41259542 |
| AXL-851 | chr19:41226316-41226343 | AXL-1996 | chr19:41259516-41259543 |
| AXL-852 | chr19:41226320-41226347 | AXL-1997 | chr19:41259526-41259553 |
| AXL-853 | chr19:41226333-41226360 | AXL-1998 | chr19:41259528-41259555 |
| AXL-854 | chr19:41226334-41226361 | AXL-1999 | chr19:41259529-41259556 |
| AXL-855 | chr19:41226335-41226362 | AXL-2000 | chr19:41259532-41259559 |
| AXL-856 | chr19:41226338-41226365 | AXL-2001 | chr19:41259533-41259560 |
| AXL-857 | chr19:41226339-41226366 | AXL-2002 | chr19:41259534-41259561 |
| AXL-858 | chr19:41226346-41226373 | AXL-2003 | chr19:41259544-41259571 |
| AXL-859 | chr19:41226347-41226374 | AXL-2004 | chr19:41259547-41259574 |
| AXL-860 | chr19:41226348-41226375 | AXL-2005 | chr19:41259548-41259575 |
| AXL-861 | chr19:41226351-41226378 | AXL-2006 | chr19:41259550-41259577 |
| AXL-862 | chr19:41226352-41226379 | AXL-2007 | chr19:41259551-41259578 |
| AXL-863 | chr19:41226353-41226380 | AXL-2008 | chr19:41259558-41259585 |
| AXL-864 | chr19:41226354-41226381 | AXL-2009 | chr19:41259566-41259593 |
| AXL-865 | chr19:41226363-41226390 | AXL-2010 | chr19:41259571-41259598 |
| AXL-866 | chr19:41226366-41226393 | AXL-2011 | chr19:41259587-41259614 |
| AXL-867 | chr19:41226367-41226394 | AXL-2012 | chr19:41259588-41259615 |
| AXL-868 | chr19:41226369-41226396 | AXL-2013 | chr19:41259589-41259616 |
| AXL-869 | chr19:41226370-41226397 | AXL-2014 | chr19:41259592-41259619 |
| AXL-870 | chr19:41226371-41226398 | AXL-2015 | chr19:41259593-41259620 |
| AXL-871 | chr19:41226373-41226400 | AXL-2016 | chr19:41259595-41259622 |
| AXL-872 | chr19:41226378-41226405 | AXL-2017 | chr19:41259599-41259626 |
| AXL-873 | chr19:41226379-41226406 | AXL-2018 | chr19:41259602-41259629 |
| AXL-874 | chr19:41226380-41226407 | AXL-2019 | chr19:41259617-41259644 |
| AXL-875 | chr19:41226381-41226408 | AXL-2020 | chr19:41259635-41259662 |
| AXL-876 | chr19:41226387-41226414 | AXL-2021 | chr19:41259645-41259672 |
| AXL-877 | chr19:41226388-41226415 | AXL-2022 | chr19:41259650-41259677 |
| AXL-878 | chr19:41226390-41226417 | AXL-2023 | chr19:41259653-41259680 |
| AXL-879 | chr19:41226393-41226420 | AXL-2024 | chr19:41259657-41259684 |
| AXL-880 | chr19:41226394-41226421 | AXL-2025 | chr19:41259658-41259685 |
| AXL-881 | chr19:41226395-41226422 | AXL-2026 | chr19:41259665-41259692 |
| AXL-882 | chr19:41226403-41226430 | AXL-2027 | chr19:41259671-41259698 |
| AXL-883 | chr19:41226404-41226431 | AXL-2028 | chr19:41259672-41259699 |
| AXL-884 | chr19:41226409-41226436 | AXL-2029 | chr19:41259675-41259702 |
| AXL-885 | chr19:41226412-41226439 | AXL-2030 | chr19:41259676-41259703 |
| AXL-886 | chr19:41226418-41226445 | AXL-2031 | chr19:41259678-41259705 |
| AXL-887 | chr19:41226419-41226446 | AXL-2032 | chr19:41259696-41259723 |
| AXL-888 | chr19:41226423-41226450 | AXL-2033 | chr19:41259705-41259732 |
| AXL-889 | chr19:41226424-41226451 | AXL-2034 | chr19:41259708-41259735 |
| AXL-890 | chr19:41226425-41226452 | AXL-2035 | chr19:41259710-41259737 |
| AXL-891 | chr19:41226442-41226469 | AXL-2036 | chr19:41259716-41259743 |
| AXL-892 | chr19:41226443-41226470 | AXL-2037 | chr19:41259717-41259744 |
| AXL-893 | chr19:41226448-41226475 | AXL-2038 | chr19:41259718-41259745 |
| AXL-894 | chr19:41226455-41226482 | AXL-2039 | chr19:41259719-41259746 |
| AXL-895 | chr19:41226457-41226484 | AXL-2040 | chr19:41259740-41259767 |
| AXL-896 | chr19:41226460-41226487 | AXL-2041 | chr19:41259742-41259769 |
| AXL-897 | chr19:41226472-41226499 | AXL-2042 | chr19:41259743-41259770 |
| AXL-898 | chr19:41226476-41226503 | AXL-2043 | chr19:41259744-41259771 |
| AXL-899 | chr19:41226479-41226506 | AXL-2044 | chr19:41259745-41259772 |
| AXL-900 | chr19:41226488-41226515 | AXL-2045 | chr19:41259746-41259773 |
| AXL-901 | chr19:41226497-41226524 | AXL-2046 | chr19:41259750-41259777 |
| AXL-902 | chr19:41226504-41226531 | AXL-2047 | chr19:41259751-41259778 |
| AXL-903 | chr19:41226505-41226532 | AXL-2048 | chr19:41259755-41259782 |
| AXL-904 | chr19:41226506-41226533 | AXL-2049 | chr19:41259760-41259787 |
| AXL-905 | chr19:41226510-41226537 | AXL-2050 | chr19:41259761-41259788 |
| AXL-906 | chr19:41226511-41226538 | AXL-2051 | chr19:41259764-41259791 |
| AXL-907 | chr19:41226514-41226541 | AXL-2052 | chr19:41259770-41259797 |
| AXL-908 | chr19:41226515-41226542 | AXL-2053 | chr19:41259771-41259798 |
| AXL-909 | chr19:41226516-41226543 | AXL-2054 | chr19:41259781-41259808 |
| AXL-910 | chr19:41226521-41226548 | AXL-2055 | chr19:41259783-41259810 |
| AXL-911 | chr19:41226525-41226552 | AXL-2056 | chr19:41259799-41259826 |
| AXL-912 | chr19:41226538-41226565 | AXL-2057 | chr19:41259803-41259830 |
| AXL-913 | chr19:41226542-41226569 | AXL-2058 | chr19:41259820-41259847 |
| AXL-914 | chr19:41226546-41226573 | AXL-2059 | chr19:41259826-41259853 |
| AXL-915 | chr19:41226547-41226574 | AXL-2060 | chr19:41259827-41259854 |
| AXL-916 | chr19:41226552-41226579 | AXL-2061 | chr19:41259831-41259858 |
| AXL-917 | chr19:41226553-41226580 | AXL-2062 | chr19:41259837-41259864 |
| AXL-918 | chr19:41226556-41226583 | AXL-2063 | chr19:41259838-41259865 |
| AXL-919 | chr19:41226563-41226590 | AXL-2064 | chr19:41259839-41259866 |
| AXL-920 | chr19:41226567-41226594 | AXL-2065 | chr19:41259840-41259867 |
| AXL-921 | chr19:41226589-41226616 | AXL-2066 | chr19:41259844-41259871 |
| AXL-922 | chr19:41226592-41226619 | AXL-2067 | chr19:41259845-41259872 |
| AXL-923 | chr19:41226593-41226620 | AXL-2068 | chr19:41259846-41259873 |
| AXL-924 | chr19:41226599-41226626 | AXL-2069 | chr19:41259854-41259881 |
| AXL-925 | chr19:41226601-41226628 | AXL-2070 | chr19:41259858-41259885 |
| AXL-926 | chr19:41226602-41226629 | AXL-2071 | chr19:41259859-41259886 |
| AXL-927 | chr19:41226603-41226630 | AXL-2072 | chr19:41259863-41259890 |
| AXL-928 | chr19:41226608-41226635 | AXL-2073 | chr19:41259866-41259893 |
| AXL-929 | chr19:41226614-41226641 | AXL-2074 | chr19:41259870-41259897 |
| AXL-930 | chr19:41226685-41226712 | AXL-2075 | chr19:41259871-41259898 |
| AXL-931 | chr19:41226686-41226713 | AXL-2076 | chr19:41259872-41259899 |
| AXL-932 | chr19:41226692-41226719 | AXL-2077 | chr19:41259879-41259906 |
| AXL-933 | chr19:41226696-41226723 | AXL-2078 | chr19:41259880-41259907 |
| AXL-934 | chr19:41226725-41226752 | AXL-2079 | chr19:41259881-41259908 |
| AXL-935 | chr19:41226778-41226805 | AXL-2080 | chr19:41259894-41259921 |
| AXL-936 | chr19:41226785-41226812 | AXL-2081 | chr19:41259900-41259927 |
| AXL-937 | chr19:41226786-41226813 | AXL-2082 | chr19:41259908-41259935 |
| AXL-938 | chr19:41226790-41226817 | AXL-2083 | chr19:41259910-41259937 |
| AXL-939 | chr19:41226791-41226818 | AXL-2084 | chr19:41259911-41259938 |
| AXL-940 | chr19:41226801-41226828 | AXL-2085 | chr19:41259914-41259941 |
| AXL-941 | chr19:41226807-41226834 | AXL-2086 | chr19:41259917-41259944 |
| AXL-942 | chr19:41226808-41226835 | AXL-2087 | chr19:41259926-41259953 |
| AXL-943 | chr19:41226814-41226841 | AXL-2088 | chr19:41259927-41259954 |
| AXL-944 | chr19:41226821-41226848 | AXL-2089 | chr19:41259932-41259959 |
| AXL-945 | chr19:41226826-41226853 | AXL-2090 | chr19:41259933-41259960 |
| AXL-946 | chr19:41226827-41226854 | AXL-2091 | chr19:41259934-41259961 |
| AXL-947 | chr19:41226830-41226857 | AXL-2092 | chr19:41259938-41259965 |
| AXL-948 | chr19:41226834-41226861 | AXL-2093 | chr19:41259953-41259980 |
| AXL-949 | chr19:41226836-41226863 | AXL-2094 | chr19:41259968-41259995 |
| AXL-950 | chr19:41226839-41226866 | AXL-2095 | chr19:41259971-41259998 |
| AXL-951 | chr19:41226840-41226867 | AXL-2096 | chr19:41259975-41260002 |
| AXL-952 | chr19:41226842-41226869 | AXL-2097 | chr19:41259976-41260003 |
| AXL-953 | chr19:41226843-41226870 | AXL-2098 | chr19:41259983-41260010 |
| AXL-954 | chr19:41226852-41226879 | AXL-2099 | chr19:41259984-41260011 |
| AXL-955 | chr19:41226853-41226880 | AXL-2100 | chr19:41259987-41260014 |
| AXL-956 | chr19:41226854-41226881 | AXL-2101 | chr19:41259988-41260015 |
| AXL-957 | chr19:41226858-41226885 | AXL-2102 | chr19:41259989-41260016 |
| AXL-958 | chr19:41226877-41226904 | AXL-2103 | chr19:41259994-41260021 |
| AXL-959 | chr19:41226878-41226905 | AXL-2104 | chr19:41260000-41260027 |
| AXL-960 | chr19:41226882-41226909 | AXL-2105 | chr19:41260001-41260028 |
| AXL-961 | chr19:41226886-41226913 | AXL-2106 | chr19:41260002-41260029 |
| AXL-962 | chr19:41226900-41226927 | AXL-2107 | chr19:41260012-41260039 |
| AXL-963 | chr19:41226923-41226950 | AXL-2108 | chr19:41260013-41260040 |
| AXL-964 | chr19:41226938-41226965 | AXL-2109 | chr19:41260021-41260048 |
| AXL-965 | chr19:41226941-41226968 | AXL-2110 | chr19:41260023-41260050 |
| AXL-966 | chr19:41226956-41226983 | AXL-2111 | chr19:41260025-41260052 |
| AXL-967 | chr19:41226965-41226992 | AXL-2112 | chr19:41260030-41260057 |
| AXL-968 | chr19:41226966-41226993 | AXL-2113 | chr19:41260031-41260058 |
| AXL-969 | chr19:41226969-41226996 | AXL-2114 | chr19:41260034-41260061 |
| AXL-970 | chr19:41226972-41226999 | AXL-2115 | chr19:41260037-41260064 |
| AXL-971 | chr19:41226979-41227006 | AXL-2116 | chr19:41260041-41260068 |
| AXL-972 | chr19:41226980-41227007 | AXL-2117 | chr19:41260042-41260069 |
| AXL-973 | chr19:41226981-41227008 | AXL-2118 | chr19:41260052-41260079 |
| AXL-974 | chr19:41226982-41227009 | AXL-2119 | chr19:41260053-41260080 |
| AXL-975 | chr19:41226998-41227025 | AXL-2120 | chr19:41260056-41260083 |
| AXL-976 | chr19:41227001-41227028 | AXL-2121 | chr19:41260057-41260084 |
| AXL-977 | chr19:41227007-41227034 | AXL-2122 | chr19:41260058-41260085 |
| AXL-978 | chr19:41230846-41230873 | AXL-2123 | chr19:41260080-41260107 |
| AXL-979 | chr19:41230849-41230876 | AXL-2124 | chr19:41260086-41260113 |
| AXL-980 | chr19:41230851-41230878 | AXL-2125 | chr19:41260090-41260117 |
| AXL-981 | chr19:41230855-41230882 | AXL-2126 | chr19:41260091-41260118 |
| AXL-982 | chr19:41230861-41230888 | AXL-2127 | chr19:41260092-41260119 |
| AXL-983 | chr19:41230865-41230892 | AXL-2128 | chr19:41260096-41260123 |
| AXL-984 | chr19:41230867-41230894 | AXL-2129 | chr19:41260100-41260127 |
| AXL-985 | chr19:41230868-41230895 | AXL-2130 | chr19:41260122-41260149 |
| AXL-986 | chr19:41230869-41230896 | AXL-2131 | chr19:41260140-41260167 |
| AXL-987 | chr19:41230870-41230897 | AXL-2132 | chr19:41260141-41260168 |
| AXL-988 | chr19:41230875-41230902 | AXL-2133 | chr19:41260144-41260171 |
| AXL-989 | chr19:41230880-41230907 | AXL-2134 | chr19:41260145-41260172 |
| AXL-990 | chr19:41230883-41230910 | AXL-2135 | chr19:41260153-41260180 |
| AXL-991 | chr19:41230886-41230913 | AXL-2136 | chr19:41260160-41260187 |
| AXL-992 | chr19:41230898-41230925 | AXL-2137 | chr19:41260175-41260202 |
| AXL-993 | chr19:41230899-41230926 | AXL-2138 | chr19:41260176-41260203 |
| AXL-994 | chr19:41230908-41230935 | AXL-2139 | chr19:41260183-41260210 |
| AXL-995 | chr19:41230909-41230936 | AXL-2140 | chr19:41260208-41260235 |
| AXL-996 | chr19:41230910-41230937 | AXL-2141 | chr19:41260216-41260243 |
| AXL-997 | chr19:41230914-41230941 | AXL-2142 | chr19:41260245-41260272 |
| AXL-998 | chr19:41230916-41230943 | AXL-2143 | chr19:41260254-41260281 |
| AXL-999 | chr19:41230917-41230944 | AXL-2144 | chr19:41260258-41260285 |
| AXL-1000 | chr19:41230921-41230948 | AXL-2145 | chr19:41260266-41260293 |
| AXL-1001 | chr19:41230925-41230952 | AXL-2146 | chr19:41260294-41260321 |
| AXL-1002 | chr19:41230926-41230953 | AXL-2147 | chr19:41260330-41260357 |
| AXL-1003 | chr19:41230927-41230954 | AXL-2148 | chr19:41260334-41260361 |
| AXL-1004 | chr19:41230940-41230967 | AXL-2149 | chr19:41260344-41260371 |
| AXL-1005 | chr19:41230941-41230968 | AXL-2150 | chr19:41260352-41260379 |
| AXL-1006 | chr19:41230943-41230970 | AXL-2151 | chr19:41260353-41260380 |
| AXL-1007 | chr19:41230944-41230971 | AXL-2152 | chr19:41260382-41260409 |
| AXL-1008 | chr19:41230948-41230975 | AXL-2153 | chr19:41260393-41260420 |
| AXL-1009 | chr19:41230949-41230976 | AXL-2154 | chr19:41260399-41260426 |
| AXL-1010 | chr19:41230961-41230988 | AXL-2155 | chr19:41260403-41260430 |
| AXL-1011 | chr19:41230962-41230989 | AXL-2156 | chr19:41260419-41260446 |
| AXL-1012 | chr19:41230982-41231009 | AXL-2157 | chr19:41260420-41260447 |
| AXL-1013 | chr19:41230987-41231014 | AXL-2158 | chr19:41260421-41260448 |
| AXL-1014 | chr19:41230988-41231015 | AXL-2159 | chr19:41260425-41260452 |
| AXL-1015 | chr19:41230989-41231016 | AXL-2160 | chr19:41260428-41260455 |
| AXL-1016 | chr19:41230990-41231017 | AXL-2161 | chr19:41260431-41260458 |
| AXL-1017 | chr19:41230991-41231018 | AXL-2162 | chr19:41260434-41260461 |
| AXL-1018 | chr19:41231000-41231027 | AXL-2163 | chr19:41260435-41260462 |
| AXL-1019 | chr19:41231001-41231028 | AXL-2164 | chr19:41260447-41260474 |
| AXL-1020 | chr19:41231009-41231036 | AXL-2165 | chr19:41260462-41260489 |
| AXL-1021 | chr19:41231021-41231048 | AXL-2166 | chr19:41260465-41260492 |
| AXL-1022 | chr19:41231027-41231054 | AXL-2167 | chr19:41260470-41260497 |
| AXL-1023 | chr19:41231028-41231055 | AXL-2168 | chr19:41260471-41260498 |
| AXL-1024 | chr19:41231033-41231060 | AXL-2169 | chr19:41260476-41260503 |
| AXL-1025 | chr19:41231034-41231061 | AXL-2170 | chr19:41260477-41260504 |
| AXL-1026 | chr19:41231036-41231063 | AXL-2171 | chr19:41260491-41260518 |
| AXL-1027 | chr19:41231037-41231064 | AXL-2172 | chr19:41260496-41260523 |
| AXL-1028 | chr19:41231039-41231066 | AXL-2173 | chr19:41260500-41260527 |
| AXL-1029 | chr19:41231040-41231067 | AXL-2174 | chr19:41260510-41260537 |
| AXL-1030 | chr19:41231041-41231068 | AXL-2175 | chr19:41260519-41260546 |
| AXL-1031 | chr19:41231042-41231069 | AXL-2176 | chr19:41260537-41260564 |
| AXL-1032 | chr19:41231048-41231075 | AXL-2177 | chr19:41260542-41260569 |
| AXL-1033 | chr19:41231049-41231076 | AXL-2178 | chr19:41260556-41260583 |
| AXL-1034 | chr19:41231058-41231085 | AXL-2179 | chr19:41260557-41260584 |
| AXL-1035 | chr19:41231059-41231086 | AXL-2180 | chr19:41260560-41260587 |
| AXL-1036 | chr19:41231062-41231089 | AXL-2181 | chr19:41260563-41260590 |
| AXL-1037 | chr19:41231063-41231090 | AXL-2182 | chr19:41260566-41260593 |
| AXL-1038 | chr19:41231064-41231091 | AXL-2183 | chr19:41260569-41260596 |
| AXL-1039 | chr19:41231069-41231096 | AXL-2184 | chr19:41260570-41260597 |
| AXL-1040 | chr19:41231070-41231097 | AXL-2185 | chr19:41260597-41260624 |
| AXL-1041 | chr19:41231075-41231102 | AXL-2186 | chr19:41260610-41260637 |
| AXL-1042 | chr19:41231076-41231103 | AXL-2187 | chr19:41260618-41260645 |
| AXL-1043 | chr19:41231077-41231104 | AXL-2188 | chr19:41260621-41260648 |
| AXL-1044 | chr19:41231086-41231113 | AXL-2189 | chr19:41260636-41260663 |
| AXL-1045 | chr19:41231087-41231114 | AXL-2190 | chr19:41260664-41260691 |
| AXL-1046 | chr19:41231088-41231115 | AXL-2191 | chr19:41260671-41260698 |
| AXL-1047 | chr19:41231089-41231116 | AXL-2192 | chr19:41260684-41260711 |
| AXL-1048 | chr19:41231099-41231126 | AXL-2193 | chr19:41260700-41260727 |
| AXL-1049 | chr19:41231100-41231127 | AXL-2194 | chr19:41260708-41260735 |
| AXL-1050 | chr19:41231101-41231128 | AXL-2195 | chr19:41260776-41260803 |
| AXL-1051 | chr19:41231105-41231132 | AXL-2196 | chr19:41260799-41260826 |
| AXL-1052 | chr19:41231106-41231133 | AXL-2197 | chr19:41260802-41260829 |
| AXL-1053 | chr19:41231121-41231148 | AXL-2198 | chr19:41260810-41260837 |
| AXL-1054 | chr19:41231130-41231157 | AXL-2199 | chr19:41260816-41260843 |
| AXL-1055 | chr19:41231131-41231158 | AXL-2200 | chr19:41260838-41260865 |
| AXL-1056 | chr19:41231151-41231178 | AXL-2201 | chr19:41260840-41260867 |
| AXL-1057 | chr19:41231152-41231179 | AXL-2202 | chr19:41260863-41260890 |
| AXL-1058 | chr19:41231153-41231180 | AXL-2203 | chr19:41260871-41260898 |
| AXL-1059 | chr19:41231168-41231195 | AXL-2204 | chr19:41260886-41260913 |
| AXL-1060 | chr19:41231169-41231196 | AXL-2205 | chr19:41260948-41260975 |
| AXL-1061 | chr19:41231177-41231204 | AXL-2206 | chr19:41260993-41261020 |
| AXL-1062 | chr19:41231187-41231214 | AXL-2207 | chr19:41260994-41261021 |
| AXL-1063 | chr19:41231188-41231215 | AXL-2208 | chr19:41261007-41261034 |
| AXL-1064 | chr19:41231189-41231216 | AXL-2209 | chr19:41261014-41261041 |
| AXL-1065 | chr19:41231190-41231217 | AXL-2210 | chr19:41261044-41261071 |
| AXL-1066 | chr19:41231197-41231224 | AXL-2211 | chr19:41261054-41261081 |
| AXL-1067 | chr19:41231198-41231225 | AXL-2212 | chr19:41261055-41261082 |
| AXL-1068 | chr19:41231199-41231226 | AXL-2213 | chr19:41261060-41261087 |
| AXL-1069 | chr19:41231205-41231232 | AXL-2214 | chr19:41261084-41261111 |
| AXL-1070 | chr19:41231206-41231233 | AXL-2215 | chr19:41261164-41261191 |
| AXL-1071 | chr19:41231208-41231235 | AXL-2216 | chr19:41261171-41261198 |
| AXL-1072 | chr19:41231211-41231238 | AXL-2217 | chr19:41261188-41261215 |
| AXL-1073 | chr19:41231212-41231239 | AXL-2218 | chr19:41261206-41261233 |
| AXL-1074 | chr19:41231215-41231242 | AXL-2219 | chr19:41261219-41261246 |
| AXL-1075 | chr19:41231218-41231245 | AXL-2220 | chr19:41261227-41261254 |
| AXL-1076 | chr19:41231219-41231246 | AXL-2221 | chr19:41261235-41261262 |
| AXL-1077 | chr19:41231220-41231247 | AXL-2222 | chr19:41261243-41261270 |
| AXL-1078 | chr19:41231223-41231250 | AXL-2223 | chr19:41261249-41261276 |
| AXL-1079 | chr19:41231227-41231254 | AXL-2224 | chr19:41261301-41261328 |
| AXL-1080 | chr19:41231228-41231255 | AXL-2225 | chr19:41261312-41261339 |
| AXL-1081 | chr19:41231231-41231258 | AXL-2226 | chr19:41261332-41261359 |
| AXL-1082 | chr19:41231240-41231267 | AXL-2227 | chr19:41261339-41261366 |
| AXL-1083 | chr19:41231254-41231281 | AXL-2228 | chr19:41261346-41261373 |
| AXL-1084 | chr19:41231259-41231286 | AXL-2229 | chr19:41261357-41261384 |
| AXL-1085 | chr19:41231272-41231299 | AXL-2230 | chr19:41261368-41261395 |
| AXL-1086 | chr19:41231274-41231301 | AXL-2231 | chr19:41261382-41261409 |
| AXL-1087 | chr19:41231275-41231302 | AXL-2232 | chr19:41261390-41261417 |
| AXL-1088 | chr19:41231276-41231303 | AXL-2233 | chr19:41261391-41261418 |
| AXL-1089 | chr19:41231277-41231304 | AXL-2234 | chr19:41261440-41261467 |
| AXL-1090 | chr19:41231282-41231309 | AXL-2235 | chr19:41261448-41261475 |
| AXL-1091 | chr19:41231283-41231310 | AXL-2236 | chr19:41261462-41261489 |
| AXL-1092 | chr19:41231290-41231317 | AXL-2237 | chr19:41261469-41261496 |
| AXL-1093 | chr19:41231291-41231318 | AXL-2238 | chr19:41261475-41261502 |
| AXL-1094 | chr19:41231292-41231319 | AXL-2239 | chr19:41261504-41261531 |
| AXL-1095 | chr19:41231307-41231334 | AXL-2240 | chr19:41261505-41261532 |
| AXL-1096 | chr19:41231308-41231335 | AXL-2241 | chr19:41261506-41261533 |
| AXL-1097 | chr19:41231335-41231362 | AXL-2242 | chr19:41261509-41261536 |
| AXL-1098 | chr19:41231338-41231365 | AXL-2243 | chr19:41261510-41261537 |
| AXL-1099 | chr19:41231342-41231369 | AXL-2244 | chr19:41261536-41261563 |
| AXL-1100 | chr19:41231345-41231372 | AXL-2245 | chr19:41261537-41261564 |
| AXL-1101 | chr19:41231346-41231373 | AXL-2246 | chr19:41261538-41261565 |
| AXL-1102 | chr19:41231348-41231375 | AXL-2247 | chr19:41261543-41261570 |
| AXL-1103 | chr19:41231349-41231376 | AXL-2248 | chr19:41261554-41261581 |
| AXL-1104 | chr19:41231352-41231379 | AXL-2249 | chr19:41261555-41261582 |
| AXL-1105 | chr19:41231358-41231385 | AXL-2250 | chr19:41261556-41261583 |
| AXL-1106 | chr19:41231359-41231386 | AXL-2251 | chr19:41261557-41261584 |
| AXL-1107 | chr19:41231360-41231387 | AXL-2252 | chr19:41261564-41261591 |
| AXL-1108 | chr19:41231361-41231388 | AXL-2253 | chr19:41261567-41261594 |
| AXL-1109 | chr19:41231365-41231392 | AXL-2254 | chr19:41261570-41261597 |
| AXL-1110 | chr19:41231366-41231393 | AXL-2255 | chr19:41261571-41261598 |
| AXL-1111 | chr19:41231370-41231397 | AXL-2256 | chr19:41261572-41261599 |
| AXL-1112 | chr19:41231371-41231398 | AXL-2257 | chr19:41261589-41261616 |
| AXL-1113 | chr19:41231379-41231406 | AXL-2258 | chr19:41261590-41261617 |
| AXL-1114 | chr19:41231380-41231407 | AXL-2259 | chr19:41261591-41261618 |
| AXL-1115 | chr19:41231383-41231410 | AXL-2260 | chr19:41261592-41261619 |
| AXL-1116 | chr19:41231393-41231420 | AXL-2261 | chr19:41261600-41261627 |
| AXL-1117 | chr19:41231394-41231421 | AXL-2262 | chr19:41261610-41261637 |
| AXL-1118 | chr19:41231395-41231422 | AXL-2263 | chr19:41261611-41261638 |
| AXL-1119 | chr19:41231396-41231423 | AXL-2264 | chr19:41261612-41261639 |
| AXL-1120 | chr19:41231397-41231424 | AXL-2265 | chr19:41261616-41261643 |
| AXL-1121 | chr19:41237837-41237864 | AXL-2266 | chr19:41261622-41261649 |
| AXL-1122 | chr19:41237838-41237865 | AXL-2267 | chr19:41261625-41261652 |
| AXL-1123 | chr19:41237851-41237878 | AXL-2268 | chr19:41261630-41261657 |
| AXL-1124 | chr19:41237852-41237879 | AXL-2269 | chr19:41261631-41261658 |
| AXL-1125 | chr19:41237861-41237888 | AXL-2270 | chr19:41261635-41261662 |
| AXL-1126 | chr19:41237866-41237893 | AXL-2271 | chr19:41261636-41261663 |
| AXL-1127 | chr19:41237869-41237896 | AXL-2272 | chr19:41261651-41261678 |
| AXL-1128 | chr19:41237872-41237899 | AXL-2273 | chr19:41261652-41261679 |
| AXL-1129 | chr19:41237883-41237910 | AXL-2274 | chr19:41261655-41261682 |
| AXL-1130 | chr19:41237890-41237917 | AXL-2275 | chr19:41261664-41261691 |
| AXL-1131 | chr19:41237915-41237942 | AXL-2276 | chr19:41261672-41261699 |
| AXL-1132 | chr19:41237916-41237943 | AXL-2277 | chr19:41261673-41261700 |
| AXL-1133 | chr19:41237917-41237944 | AXL-2278 | chr19:41261674-41261701 |
| AXL-1134 | chr19:41237920-41237947 | AXL-2279 | chr19:41261675-41261702 |
| AXL-1135 | chr19:41237927-41237954 | AXL-2280 | chr19:41261682-41261709 |
| AXL-1136 | chr19:41237928-41237955 | AXL-2281 | chr19:41261686-41261713 |
| AXL-1137 | chr19:41237933-41237960 | AXL-2282 | chr19:41261687-41261714 |
| AXL-1138 | chr19:41237936-41237963 | AXL-2283 | chr19:41261688-41261715 |
| AXL-1139 | chr19:41237937-41237964 | AXL-2284 | chr19:41261693-41261720 |
| AXL-1140 | chr19:41237938-41237965 | AXL-2285 | chr19:41261705-41261732 |
| AXL-1141 | chr19:41237941-41237968 | AXL-2286 | chr19:41261706-41261733 |
| AXL-1142 | chr19:41237942-41237969 | AXL-2287 | chr19:41261707-41261734 |
| AXL-1143 | chr19:41237950-41237977 | AXL-2288 | chr19:41261713-41261740 |
| AXL-1144 | chr19:41237953-41237980 | AXL-2289 | chr19:41261723-41261750 |
| AXL-1145 | chr19:41237954-41237981 | AXL-2290 | chr19:41261726-41261753 |

**Table 2E Genomic coordinates of the human NFE2L1 gene**

| Target ID | Target Coordinate Range | Target ID | Target Coordinate Range |
|---|---|---|---|
| NFE2L1-1 | chr17:48046868-48046895 | NFE2L1-712 | chr17:48056670-48056697 |
| NFE2L1-2 | chr17:48046874-48046901 | NFE2L1-713 | chr17:48056671-48056698 |
| NFE2L1-3 | chr17:48046878-48046905 | NFE2L1-714 | chr17:48056673-48056700 |
| NFE2L1-4 | chr17:48046879-48046906 | NFE2L1-715 | chr17:48056680-48056707 |
| NFE2L1-5 | chr17:48046909-48046936 | NFE2L1-716 | chr17:48056681-48056708 |
| NFE2L1-6 | chr17:48046924-48046951 | NFE2L1-717 | chr17:48056682-48056709 |
| NFE2L1-7 | chr17:48046925-48046952 | NFE2L1-718 | chr17:48056685-48056712 |
| NFE2L1-8 | chr17:48046929-48046956 | NFE2L1-719 | chr17:48056906-48056933 |
| NFE2L1-9 | chr17:48046952-48046979 | NFE2L1-720 | chr17:48056907-48056934 |
| NFE2L1-10 | chr17:48046953-48046980 | NFE2L1-721 | chr17:48056910-48056937 |
| NFE2L1-11 | chr17:48046961-48046988 | NFE2L1-722 | chr17:48056911-48056938 |
| NFE2L1-12 | chr17:48046966-48046993 | NFE2L1-723 | chr17:48056912-48056939 |
| NFE2L1-13 | chr17:48046970-48046997 | NFE2L1-724 | chr17:48056916-48056943 |
| NFE2L1-14 | chr17:48046988-48047015 | NFE2L1-725 | chr17:48056921-48056948 |
| NFE2L1-15 | chr17:48047028-48047055 | NFE2L1-726 | chr17:48056922-48056949 |
| NFE2L1-16 | chr17:48047036-48047063 | NFE2L1-727 | chr17:48056923-48056950 |
| NFE2L1-17 | chr17:48047046-48047073 | NFE2L1-728 | chr17:48056937-48056964 |
| NFE2L1-18 | chr17:48047105-48047132 | NFE2L1-729 | chr17:48056938-48056965 |
| NFE2L1-19 | chr17:48047108-48047135 | NFE2L1-730 | chr17:48056956-48056983 |
| NFE2L1-20 | chr17:48047109-48047136 | NFE2L1-731 | chr17:48056958-48056985 |
| NFE2L1-21 | chr17:48047110-48047137 | NFE2L1-732 | chr17:48056967-48056994 |
| NFE2L1-22 | chr17:48047117-48047144 | NFE2L1-733 | chr17:48056973-48057000 |
| NFE2L1-23 | chr17:48047120-48047147 | NFE2L1-734 | chr17:48056979-48057006 |
| NFE2L1-24 | chr17:48047121-48047148 | NFE2L1-735 | chr17:48056980-48057007 |
| NFE2L1-25 | chr17:48047122-48047149 | NFE2L1-736 | chr17:48056981-48057008 |
| NFE2L1-26 | chr17:48047146-48047173 | NFE2L1-737 | chr17:48056989-48057016 |
| NFE2L1-27 | chr17:48047167-48047194 | NFE2L1-738 | chr17:48056995-48057022 |
| NFE2L1-28 | chr17:48047168-48047195 | NFE2L1-739 | chr17:48056996-48057023 |
| NFE2L1-29 | chr17:48047174-48047201 | NFE2L1-740 | chr17:48057005-48057032 |
| NFE2L1-30 | chr17:48047180-48047207 | NFE2L1-741 | chr17:48057015-48057042 |
| NFE2L1-31 | chr17:48047194-48047221 | NFE2L1-742 | chr17:48057016-48057043 |
| NFE2L1-32 | chr17:48047195-48047222 | NFE2L1-743 | chr17:48057017-48057044 |
| NFE2L1-33 | chr17:48047208-48047235 | NFE2L1-744 | chr17:48057019-48057046 |
| NFE2L1-34 | chr17:48047245-48047272 | NFE2L1-745 | chr17:48057020-48057047 |
| NFE2L1-35 | chr17:48047246-48047273 | NFE2L1-746 | chr17:48057026-48057053 |
| NFE2L1-36 | chr17:48047249-48047276 | NFE2L1-747 | chr17:48057029-48057056 |
| NFE2L1-37 | chr17:48047253-48047280 | NFE2L1-748 | chr17:48057035-48057062 |
| NFE2L1-38 | chr17:48047262-48047289 | NFE2L1-749 | chr17:48057041-48057068 |
| NFE2L1-39 | chr17:48047288-48047315 | NFE2L1-750 | chr17:48057049-48057076 |
| NFE2L1-40 | chr17:48047289-48047316 | NFE2L1-751 | chr17:48057055-48057082 |
| NFE2L1-41 | chr17:48047290-48047317 | NFE2L1-752 | chr17:48057057-48057084 |
| NFE2L1-42 | chr17:48047292-48047319 | NFE2L1-753 | chr17:48057058-48057085 |
| NFE2L1-43 | chr17:48047296-48047323 | NFE2L1-754 | chr17:48057062-48057089 |
| NFE2L1-44 | chr17:48047297-48047324 | NFE2L1-755 | chr17:48057063-48057090 |
| NFE2L1-45 | chr17:48047306-48047333 | NFE2L1-756 | chr17:48057064-48057091 |
| NFE2L1-46 | chr17:48047307-48047334 | NFE2L1-757 | chr17:48057076-48057103 |
| NFE2L1-47 | chr17:48047326-48047353 | NFE2L1-758 | chr17:48057081-48057108 |
| NFE2L1-48 | chr17:48047330-48047357 | NFE2L1-759 | chr17:48057082-48057109 |
| NFE2L1-49 | chr17:48047338-48047365 | NFE2L1-760 | chr17:48057083-48057110 |
| NFE2L1-50 | chr17:48047344-48047371 | NFE2L1-761 | chr17:48057093-48057120 |
| NFE2L1-51 | chr17:48047345-48047372 | NFE2L1-762 | chr17:48057095-48057122 |
| NFE2L1-52 | chr17:48047346-48047373 | NFE2L1-763 | chr17:48057096-48057123 |
| NFE2L1-53 | chr17:48047351-48047378 | NFE2L1-764 | chr17:48057100-48057127 |
| NFE2L1-54 | chr17:48047352-48047379 | NFE2L1-765 | chr17:48057101-48057128 |
| NFE2L1-55 | chr17:48047362-48047389 | NFE2L1-766 | chr17:48057102-48057129 |
| NFE2L1-56 | chr17:48047366-48047393 | NFE2L1-767 | chr17:48057103-48057130 |
| NFE2L1-57 | chr17:48047369-48047396 | NFE2L1-768 | chr17:48057133-48057160 |
| NFE2L1-58 | chr17:48047374-48047401 | NFE2L1-769 | chr17:48057134-48057161 |
| NFE2L1-59 | chr17:48047376-48047403 | NFE2L1-770 | chr17:48057145-48057172 |
| NFE2L1-60 | chr17:48047377-48047404 | NFE2L1-771 | chr17:48057148-48057175 |
| NFE2L1-61 | chr17:48047384-48047411 | NFE2L1-772 | chr17:48057163-48057190 |
| NFE2L1-62 | chr17:48047397-48047424 | NFE2L1-773 | chr17:48057165-48057192 |
| NFE2L1-63 | chr17:48047406-48047433 | NFE2L1-774 | chr17:48057169-48057196 |
| NFE2L1-64 | chr17:48047420-48047447 | NFE2L1-775 | chr17:48057170-48057197 |
| NFE2L1-65 | chr17:48047421-48047448 | NFE2L1-776 | chr17:48057171-48057198 |
| NFE2L1-66 | chr17:48047432-48047459 | NFE2L1-777 | chr17:48057181-48057208 |
| NFE2L1-67 | chr17:48047433-48047460 | NFE2L1-778 | chr17:48057182-48057209 |
| NFE2L1-68 | chr17:48047434-48047461 | NFE2L1-779 | chr17:48057185-48057212 |
| NFE2L1-69 | chr17:48047458-48047485 | NFE2L1-780 | chr17:48057188-48057215 |
| NFE2L1-70 | chr17:48047474-48047501 | NFE2L1-781 | chr17:48057199-48057226 |
| NFE2L1-71 | chr17:48047475-48047502 | NFE2L1-782 | chr17:48057206-48057233 |
| NFE2L1-72 | chr17:48047480-48047507 | NFE2L1-783 | chr17:48057207-48057234 |
| NFE2L1-73 | chr17:48047494-48047521 | NFE2L1-784 | chr17:48057208-48057235 |
| NFE2L1-74 | chr17:48047531-48047558 | NFE2L1-785 | chr17:48057209-48057236 |
| NFE2L1-75 | chr17:48047532-48047559 | NFE2L1-786 | chr17:48057216-48057243 |
| NFE2L1-76 | chr17:48047533-48047560 | NFE2L1-787 | chr17:48057221-48057248 |
| NFE2L1-77 | chr17:48047536-48047563 | NFE2L1-788 | chr17:48057225-48057252 |
| NFE2L1-78 | chr17:48047546-48047573 | NFE2L1-789 | chr17:48057240-48057267 |
| NFE2L1-79 | chr17:48047547-48047574 | NFE2L1-790 | chr17:48057246-48057273 |
| NFE2L1-80 | chr17:48047548-48047575 | NFE2L1-791 | chr17:48057249-48057276 |
| NFE2L1-81 | chr17:48047549-48047576 | NFE2L1-792 | chr17:48057259-48057286 |
| NFE2L1-82 | chr17:48047550-48047577 | NFE2L1-793 | chr17:48057260-48057287 |
| NFE2L1-83 | chr17:48047558-48047585 | NFE2L1-794 | chr17:48057263-48057290 |
| NFE2L1-84 | chr17:48047559-48047586 | NFE2L1-795 | chr17:48057269-48057296 |
| NFE2L1-85 | chr17:48047564-48047591 | NFE2L1-796 | chr17:48057274-48057301 |
| NFE2L1-86 | chr17:48047579-48047606 | NFE2L1-797 | chr17:48057307-48057334 |
| NFE2L1-87 | chr17:48047596-48047623 | NFE2L1-798 | chr17:48057311-48057338 |
| NFE2L1-88 | chr17:48047611-48047638 | NFE2L1-799 | chr17:48057312-48057339 |
| NFE2L1-89 | chr17:48047617-48047644 | NFE2L1-800 | chr17:48057313-48057340 |
| NFE2L1-90 | chr17:48047618-48047645 | NFE2L1-801 | chr17:48057314-48057341 |
| NFE2L1-91 | chr17:48047619-48047646 | NFE2L1-802 | chr17:48057325-48057352 |
| NFE2L1-92 | chr17:48047624-48047651 | NFE2L1-803 | chr17:48057338-48057365 |
| NFE2L1-93 | chr17:48047633-48047660 | NFE2L1-804 | chr17:48057339-48057366 |
| NFE2L1-94 | chr17:48047636-48047663 | NFE2L1-805 | chr17:48057347-48057374 |
| NFE2L1-95 | chr17:48047644-48047671 | NFE2L1-806 | chr17:48057360-48057387 |
| NFE2L1-96 | chr17:48047647-48047674 | NFE2L1-807 | chr17:48057380-48057407 |
| NFE2L1-97 | chr17:48047655-48047682 | NFE2L1-808 | chr17:48057395-48057422 |
| NFE2L1-98 | chr17:48047658-48047685 | NFE2L1-809 | chr17:48057396-48057423 |
| NFE2L1-99 | chr17:48047665-48047692 | NFE2L1-810 | chr17:48057397-48057424 |
| NFE2L1-100 | chr17:48047666-48047693 | NFE2L1-811 | chr17:48057398-48057425 |
| NFE2L1-101 | chr17:48047669-48047696 | NFE2L1-812 | chr17:48057411-48057438 |
| NFE2L1-102 | chr17:48047672-48047699 | NFE2L1-813 | chr17:48057412-48057439 |
| NFE2L1-103 | chr17:48047673-48047700 | NFE2L1-814 | chr17:48057415-48057442 |
| NFE2L1-104 | chr17:48047683-48047710 | NFE2L1-815 | chr17:48057425-48057452 |
| NFE2L1-105 | chr17:48047684-48047711 | NFE2L1-816 | chr17:48057434-48057461 |
| NFE2L1-106 | chr17:48047688-48047715 | NFE2L1-817 | chr17:48057435-48057462 |
| NFE2L1-107 | chr17:48047696-48047723 | NFE2L1-818 | chr17:48057445-48057472 |
| NFE2L1-108 | chr17:48047697-48047724 | NFE2L1-819 | chr17:48057449-48057476 |
| NFE2L1-109 | chr17:48047701-48047728 | NFE2L1-820 | chr17:48057467-48057494 |
| NFE2L1-110 | chr17:48047705-48047732 | NFE2L1-821 | chr17:48057476-48057503 |
| NFE2L1-111 | chr17:48047713-48047740 | NFE2L1-822 | chr17:48057480-48057507 |
| NFE2L1-112 | chr17:48047714-48047741 | NFE2L1-823 | chr17:48057486-48057513 |
| NFE2L1-113 | chr17:48047719-48047746 | NFE2L1-824 | chr17:48057488-48057515 |
| NFE2L1-114 | chr17:48047725-48047752 | NFE2L1-825 | chr17:48057494-48057521 |
| NFE2L1-115 | chr17:48047727-48047754 | NFE2L1-826 | chr17:48057495-48057522 |
| NFE2L1-116 | chr17:48047728-48047755 | NFE2L1-827 | chr17:48057509-48057536 |
| NFE2L1-117 | chr17:48047729-48047756 | NFE2L1-828 | chr17:48057518-48057545 |
| NFE2L1-118 | chr17:48047732-48047759 | NFE2L1-829 | chr17:48057529-48057556 |
| NFE2L1-119 | chr17:48047738-48047765 | NFE2L1-830 | chr17:48057557-48057584 |
| NFE2L1-120 | chr17:48047742-48047769 | NFE2L1-831 | chr17:48057573-48057600 |
| NFE2L1-121 | chr17:48047743-48047770 | NFE2L1-832 | chr17:48057579-48057606 |
| NFE2L1-122 | chr17:48047754-48047781 | NFE2L1-833 | chr17:48057597-48057624 |
| NFE2L1-123 | chr17:48047755-48047782 | NFE2L1-834 | chr17:48057598-48057625 |
| NFE2L1-124 | chr17:48047756-48047783 | NFE2L1-835 | chr17:48057599-48057626 |
| NFE2L1-125 | chr17:48047763-48047790 | NFE2L1-836 | chr17:48058190-48058217 |
| NFE2L1-126 | chr17:48047764-48047791 | NFE2L1-837 | chr17:48058194-48058221 |
| NFE2L1-127 | chr17:48047765-48047792 | NFE2L1-838 | chr17:48058195-48058222 |
| NFE2L1-128 | chr17:48047771-48047798 | NFE2L1-839 | chr17:48058198-48058225 |
| NFE2L1-129 | chr17:48047776-48047803 | NFE2L1-840 | chr17:48058199-48058226 |
| NFE2L1-130 | chr17:48047795-48047822 | NFE2L1-841 | chr17:48058212-48058239 |
| NFE2L1-131 | chr17:48047796-48047823 | NFE2L1-842 | chr17:48058219-48058246 |
| NFE2L1-132 | chr17:48047799-48047826 | NFE2L1-843 | chr17:48058220-48058247 |
| NFE2L1-133 | chr17:48047802-48047829 | NFE2L1-844 | chr17:48058221-48058248 |
| NFE2L1-134 | chr17:48047805-48047832 | NFE2L1-845 | chr17:48058224-48058251 |
| NFE2L1-135 | chr17:48047808-48047835 | NFE2L1-846 | chr17:48058225-48058252 |
| NFE2L1-136 | chr17:48047814-48047841 | NFE2L1-847 | chr17:48058240-48058267 |
| NFE2L1-137 | chr17:48047815-48047842 | NFE2L1-848 | chr17:48058241-48058268 |
| NFE2L1-138 | chr17:48047841-48047868 | NFE2L1-849 | chr17:48058242-48058269 |
| NFE2L1-139 | chr17:48047850-48047877 | NFE2L1-850 | chr17:48058248-48058275 |
| NFE2L1-140 | chr17:48047856-48047883 | NFE2L1-851 | chr17:48058261-48058288 |
| NFE2L1-141 | chr17:48047857-48047884 | NFE2L1-852 | chr17:48058268-48058295 |
| NFE2L1-142 | chr17:48047865-48047892 | NFE2L1-853 | chr17:48058274-48058301 |
| NFE2L1-143 | chr17:48047866-48047893 | NFE2L1-854 | chr17:48058285-48058312 |
| NFE2L1-144 | chr17:48047869-48047896 | NFE2L1-855 | chr17:48058286-48058313 |
| NFE2L1-145 | chr17:48047886-48047913 | NFE2L1-856 | chr17:48058287-48058314 |
| NFE2L1-146 | chr17:48047887-48047914 | NFE2L1-857 | chr17:48058290-48058317 |
| NFE2L1-147 | chr17:48047898-48047925 | NFE2L1-858 | chr17:48058291-48058318 |
| NFE2L1-148 | chr17:48047903-48047930 | NFE2L1-859 | chr17:48058296-48058323 |
| NFE2L1-149 | chr17:48047904-48047931 | NFE2L1-860 | chr17:48058320-48058347 |
| NFE2L1-150 | chr17:48047917-48047944 | NFE2L1-861 | chr17:48058327-48058354 |
| NFE2L1-151 | chr17:48047918-48047945 | NFE2L1-862 | chr17:48058338-48058365 |
| NFE2L1-152 | chr17:48047919-48047946 | NFE2L1-863 | chr17:48058339-48058366 |
| NFE2L1-153 | chr17:48047922-48047949 | NFE2L1-864 | chr17:48058340-48058367 |
| NFE2L1-154 | chr17:48047936-48047963 | NFE2L1-865 | chr17:48058343-48058370 |
| NFE2L1-155 | chr17:48047951-48047978 | NFE2L1-866 | chr17:48058351-48058378 |
| NFE2L1-156 | chr17:48047957-48047984 | NFE2L1-867 | chr17:48058352-48058379 |
| NFE2L1-157 | chr17:48047961-48047988 | NFE2L1-868 | chr17:48058362-48058389 |
| NFE2L1-158 | chr17:48047967-48047994 | NFE2L1-869 | chr17:48058372-48058399 |
| NFE2L1-159 | chr17:48047968-48047995 | NFE2L1-870 | chr17:48058377-48058404 |
| NFE2L1-160 | chr17:48047973-48048000 | NFE2L1-871 | chr17:48058378-48058405 |
| NFE2L1-161 | chr17:48047979-48048006 | NFE2L1-872 | chr17:48058379-48058406 |
| NFE2L1-162 | chr17:48047981-48048008 | NFE2L1-873 | chr17:48058380-48058407 |
| NFE2L1-163 | chr17:48047987-48048014 | NFE2L1-874 | chr17:48058388-48058415 |
| NFE2L1-164 | chr17:48047988-48048015 | NFE2L1-875 | chr17:48058389-48058416 |
| NFE2L1-165 | chr17:48047989-48048016 | NFE2L1-876 | chr17:48058391-48058418 |
| NFE2L1-166 | chr17:48047990-48048017 | NFE2L1-877 | chr17:48058400-48058427 |
| NFE2L1-167 | chr17:48047993-48048020 | NFE2L1-878 | chr17:48058401-48058428 |
| NFE2L1-168 | chr17:48047996-48048023 | NFE2L1-879 | chr17:48058402-48058429 |
| NFE2L1-169 | chr17:48048014-48048041 | NFE2L1-880 | chr17:48058403-48058430 |
| NFE2L1-170 | chr17:48048015-48048042 | NFE2L1-881 | chr17:48058404-48058431 |
| NFE2L1-171 | chr17:48048019-48048046 | NFE2L1-882 | chr17:48058408-48058435 |
| NFE2L1-172 | chr17:48048032-48048059 | NFE2L1-883 | chr17:48058415-48058442 |
| NFE2L1-173 | chr17:48048033-48048060 | NFE2L1-884 | chr17:48058422-48058449 |
| NFE2L1-174 | chr17:48048039-48048066 | NFE2L1-885 | chr17:48058423-48058450 |
| NFE2L1-175 | chr17:48048040-48048067 | NFE2L1-886 | chr17:48058438-48058465 |
| NFE2L1-176 | chr17:48048043-48048070 | NFE2L1-887 | chr17:48058439-48058466 |
| NFE2L1-177 | chr17:48048045-48048072 | NFE2L1-888 | chr17:48058442-48058469 |
| NFE2L1-178 | chr17:48048046-48048073 | NFE2L1-889 | chr17:48058457-48058484 |
| NFE2L1-179 | chr17:48048048-48048075 | NFE2L1-890 | chr17:48058459-48058486 |
| NFE2L1-180 | chr17:48048054-48048081 | NFE2L1-891 | chr17:48058460-48058487 |
| NFE2L1-181 | chr17:48048055-48048082 | NFE2L1-892 | chr17:48058461-48058488 |
| NFE2L1-182 | chr17:48048056-48048083 | NFE2L1-893 | chr17:48058474-48058501 |
| NFE2L1-183 | chr17:48048058-48048085 | NFE2L1-894 | chr17:48058478-48058505 |
| NFE2L1-184 | chr17:48048059-48048086 | NFE2L1-895 | chr17:48058484-48058511 |
| NFE2L1-185 | chr17:48048062-48048089 | NFE2L1-896 | chr17:48058485-48058512 |
| NFE2L1-186 | chr17:48048076-48048103 | NFE2L1-897 | chr17:48058494-48058521 |
| NFE2L1-187 | chr17:48048077-48048104 | NFE2L1-898 | chr17:48058504-48058531 |
| NFE2L1-188 | chr17:48048078-48048105 | NFE2L1-899 | chr17:48058505-48058532 |
| NFE2L1-189 | chr17:48048084-48048111 | NFE2L1-900 | chr17:48058512-48058539 |
| NFE2L1-190 | chr17:48048085-48048112 | NFE2L1-901 | chr17:48058513-48058540 |
| NFE2L1-191 | chr17:48048093-48048120 | NFE2L1-902 | chr17:48058514-48058541 |
| NFE2L1-192 | chr17:48048097-48048124 | NFE2L1-903 | chr17:48058515-48058542 |
| NFE2L1-193 | chr17:48048100-48048127 | NFE2L1-904 | chr17:48058521-48058548 |
| NFE2L1-194 | chr17:48048101-48048128 | NFE2L1-905 | chr17:48058527-48058554 |
| NFE2L1-195 | chr17:48048106-48048133 | NFE2L1-906 | chr17:48058531-48058558 |
| NFE2L1-196 | chr17:48048112-48048139 | NFE2L1-907 | chr17:48058539-48058566 |
| NFE2L1-197 | chr17:48048113-48048140 | NFE2L1-908 | chr17:48058540-48058567 |
| NFE2L1-198 | chr17:48048114-48048141 | NFE2L1-909 | chr17:48058542-48058569 |
| NFE2L1-199 | chr17:48048115-48048142 | NFE2L1-910 | chr17:48058551-48058578 |
| NFE2L1-200 | chr17:48048116-48048143 | NFE2L1-911 | chr17:48058554-48058581 |
| NFE2L1-201 | chr17:48048118-48048145 | NFE2L1-912 | chr17:48058558-48058585 |
| NFE2L1-202 | chr17:48048119-48048146 | NFE2L1-913 | chr17:48058566-48058593 |
| NFE2L1-203 | chr17:48048125-48048152 | NFE2L1-914 | chr17:48058577-48058604 |
| NFE2L1-204 | chr17:48048130-48048157 | NFE2L1-915 | chr17:48058580-48058607 |
| NFE2L1-205 | chr17:48048135-48048162 | NFE2L1-916 | chr17:48058581-48058608 |
| NFE2L1-206 | chr17:48048136-48048163 | NFE2L1-917 | chr17:48058582-48058609 |
| NFE2L1-207 | chr17:48048140-48048167 | NFE2L1-918 | chr17:48058587-48058614 |
| NFE2L1-208 | chr17:48048141-48048168 | NFE2L1-919 | chr17:48058599-48058626 |
| NFE2L1-209 | chr17:48048147-48048174 | NFE2L1-920 | chr17:48058610-48058637 |
| NFE2L1-210 | chr17:48048158-48048185 | NFE2L1-921 | chr17:48058611-48058638 |
| NFE2L1-211 | chr17:48048159-48048186 | NFE2L1-922 | chr17:48058612-48058639 |
| NFE2L1-212 | chr17:48048164-48048191 | NFE2L1-923 | chr17:48058613-48058640 |
| NFE2L1-213 | chr17:48048167-48048194 | NFE2L1-924 | chr17:48058614-48058641 |
| NFE2L1-214 | chr17:48048172-48048199 | NFE2L1-925 | chr17:48058615-48058642 |
| NFE2L1-215 | chr17:48048173-48048200 | NFE2L1-926 | chr17:48058616-48058643 |
| NFE2L1-216 | chr17:48048174-48048201 | NFE2L1-927 | chr17:48058625-48058652 |
| NFE2L1-217 | chr17:48048180-48048207 | NFE2L1-928 | chr17:48058630-48058657 |
| NFE2L1-218 | chr17:48048183-48048210 | NFE2L1-929 | chr17:48058631-48058658 |
| NFE2L1-219 | chr17:48048196-48048223 | NFE2L1-930 | chr17:48058637-48058664 |
| NFE2L1-220 | chr17:48048199-48048226 | NFE2L1-931 | chr17:48058655-48058682 |
| NFE2L1-221 | chr17:48048200-48048227 | NFE2L1-932 | chr17:48058661-48058688 |
| NFE2L1-222 | chr17:48048220-48048247 | NFE2L1-933 | chr17:48058674-48058701 |
| NFE2L1-223 | chr17:48048223-48048250 | NFE2L1-934 | chr17:48058675-48058702 |
| NFE2L1-224 | chr17:48048224-48048251 | NFE2L1-935 | chr17:48058684-48058711 |
| NFE2L1-225 | chr17:48048228-48048255 | NFE2L1-936 | chr17:48058689-48058716 |
| NFE2L1-226 | chr17:48048229-48048256 | NFE2L1-937 | chr17:48058691-48058718 |
| NFE2L1-227 | chr17:48048238-48048265 | NFE2L1-938 | chr17:48058703-48058730 |
| NFE2L1-228 | chr17:48048240-48048267 | NFE2L1-939 | chr17:48058706-48058733 |
| NFE2L1-229 | chr17:48048244-48048271 | NFE2L1-940 | chr17:48058708-48058735 |
| NFE2L1-230 | chr17:48048250-48048277 | NFE2L1-941 | chr17:48058709-48058736 |
| NFE2L1-231 | chr17:48048256-48048283 | NFE2L1-942 | chr17:48058719-48058746 |
| NFE2L1-232 | chr17:48048262-48048289 | NFE2L1-943 | chr17:48058722-48058749 |
| NFE2L1-233 | chr17:48048266-48048293 | NFE2L1-944 | chr17:48058723-48058750 |
| NFE2L1-234 | chr17:48048267-48048294 | NFE2L1-945 | chr17:48058728-48058755 |
| NFE2L1-235 | chr17:48048268-48048295 | NFE2L1-946 | chr17:48058756-48058783 |
| NFE2L1-236 | chr17:48048272-48048299 | NFE2L1-947 | chr17:48058761-48058788 |
| NFE2L1-237 | chr17:48048278-48048305 | NFE2L1-948 | chr17:48058774-48058801 |
| NFE2L1-238 | chr17:48048279-48048306 | NFE2L1-949 | chr17:48058779-48058806 |
| NFE2L1-239 | chr17:48048288-48048315 | NFE2L1-950 | chr17:48058780-48058807 |
| NFE2L1-240 | chr17:48048291-48048318 | NFE2L1-951 | chr17:48058781-48058808 |
| NFE2L1-241 | chr17:48048292-48048319 | NFE2L1-952 | chr17:48058788-48058815 |
| NFE2L1-242 | chr17:48048295-48048322 | NFE2L1-953 | chr17:48058789-48058816 |
| NFE2L1-243 | chr17:48048296-48048323 | NFE2L1-954 | chr17:48058815-48058842 |
| NFE2L1-244 | chr17:48048300-48048327 | NFE2L1-955 | chr17:48058824-48058851 |
| NFE2L1-245 | chr17:48048309-48048336 | NFE2L1-956 | chr17:48058827-48058854 |
| NFE2L1-246 | chr17:48048316-48048343 | NFE2L1-957 | chr17:48058830-48058857 |
| NFE2L1-247 | chr17:48048319-48048346 | NFE2L1-958 | chr17:48058836-48058863 |
| NFE2L1-248 | chr17:48048320-48048347 | NFE2L1-959 | chr17:48058845-48058872 |
| NFE2L1-249 | chr17:48048321-48048348 | NFE2L1-960 | chr17:48058856-48058883 |
| NFE2L1-250 | chr17:48048322-48048349 | NFE2L1-961 | chr17:48058857-48058884 |
| NFE2L1-251 | chr17:48048325-48048352 | NFE2L1-962 | chr17:48058860-48058887 |
| NFE2L1-252 | chr17:48048326-48048353 | NFE2L1-963 | chr17:48058863-48058890 |
| NFE2L1-253 | chr17:48048327-48048354 | NFE2L1-964 | chr17:48058865-48058892 |
| NFE2L1-254 | chr17:48048330-48048357 | NFE2L1-965 | chr17:48058869-48058896 |
| NFE2L1-255 | chr17:48048337-48048364 | NFE2L1-966 | chr17:48058872-48058899 |
| NFE2L1-256 | chr17:48048340-48048367 | NFE2L1-967 | chr17:48058895-48058922 |
| NFE2L1-257 | chr17:48048354-48048381 | NFE2L1-968 | chr17:48058901-48058928 |
| NFE2L1-258 | chr17:48048358-48048385 | NFE2L1-969 | chr17:48058907-48058934 |
| NFE2L1-259 | chr17:48048362-48048389 | NFE2L1-970 | chr17:48058913-48058940 |
| NFE2L1-260 | chr17:48048365-48048392 | NFE2L1-971 | chr17:48058914-48058941 |
| NFE2L1-261 | chr17:48048368-48048395 | NFE2L1-972 | chr17:48058917-48058944 |
| NFE2L1-262 | chr17:48048370-48048397 | NFE2L1-973 | chr17:48058918-48058945 |
| NFE2L1-263 | chr17:48048371-48048398 | NFE2L1-974 | chr17:48058922-48058949 |
| NFE2L1-264 | chr17:48048374-48048401 | NFE2L1-975 | chr17:48058923-48058950 |
| NFE2L1-265 | chr17:48048379-48048406 | NFE2L1-976 | chr17:48058935-48058962 |
| NFE2L1-266 | chr17:48048382-48048409 | NFE2L1-977 | chr17:48058954-48058981 |
| NFE2L1-267 | chr17:48048383-48048410 | NFE2L1-978 | chr17:48058958-48058985 |
| NFE2L1-268 | chr17:48048384-48048411 | NFE2L1-979 | chr17:48058967-48058994 |
| NFE2L1-269 | chr17:48048386-48048413 | NFE2L1-980 | chr17:48058968-48058995 |
| NFE2L1-270 | chr17:48048390-48048417 | NFE2L1-981 | chr17:48058978-48059005 |
| NFE2L1-271 | chr17:48048391-48048418 | NFE2L1-982 | chr17:48058990-48059017 |
| NFE2L1-272 | chr17:48048397-48048424 | NFE2L1-983 | chr17:48058997-48059024 |
| NFE2L1-273 | chr17:48048398-48048425 | NFE2L1-984 | chr17:48059000-48059027 |
| NFE2L1-274 | chr17:48048407-48048434 | NFE2L1-985 | chr17:48059009-48059036 |
| NFE2L1-275 | chr17:48048408-48048435 | NFE2L1-986 | chr17:48059010-48059037 |
| NFE2L1-276 | chr17:48048411-48048438 | NFE2L1-987 | chr17:48059014-48059041 |
| NFE2L1-277 | chr17:48048417-48048444 | NFE2L1-988 | chr17:48059018-48059045 |
| NFE2L1-278 | chr17:48048420-48048447 | NFE2L1-989 | chr17:48059019-48059046 |
| NFE2L1-279 | chr17:48048426-48048453 | NFE2L1-990 | chr17:48059023-48059050 |
| NFE2L1-280 | chr17:48048439-48048466 | NFE2L1-991 | chr17:48059033-48059060 |
| NFE2L1-281 | chr17:48048440-48048467 | NFE2L1-992 | chr17:48059038-48059065 |
| NFE2L1-282 | chr17:48048446-48048473 | NFE2L1-993 | chr17:48059044-48059071 |
| NFE2L1-283 | chr17:48048447-48048474 | NFE2L1-994 | chr17:48059048-48059075 |
| NFE2L1-284 | chr17:48048451-48048478 | NFE2L1-995 | chr17:48059063-48059090 |
| NFE2L1-285 | chr17:48048467-48048494 | NFE2L1-996 | chr17:48059064-48059091 |
| NFE2L1-286 | chr17:48048468-48048495 | NFE2L1-997 | chr17:48059070-48059097 |
| NFE2L1-287 | chr17:48048471-48048498 | NFE2L1-998 | chr17:48059071-48059098 |
| NFE2L1-288 | chr17:48048472-48048499 | NFE2L1-999 | chr17:48059082-48059109 |
| NFE2L1-289 | chr17:48048481-48048508 | NFE2L1-1000 | chr17:48059086-48059113 |
| NFE2L1-290 | chr17:48048486-48048513 | NFE2L1-1001 | chr17:48059088-48059115 |
| NFE2L1-291 | chr17:48048487-48048514 | NFE2L1-1002 | chr17:48059090-48059117 |
| NFE2L1-292 | chr17:48048488-48048515 | NFE2L1-1003 | chr17:48059093-48059120 |
| NFE2L1-293 | chr17:48048494-48048521 | NFE2L1-1004 | chr17:48059096-48059123 |
| NFE2L1-294 | chr17:48048501-48048528 | NFE2L1-1005 | chr17:48059097-48059124 |
| NFE2L1-295 | chr17:48048502-48048529 | NFE2L1-1006 | chr17:48059103-48059130 |
| NFE2L1-296 | chr17:48048503-48048530 | NFE2L1-1007 | chr17:48059104-48059131 |
| NFE2L1-297 | chr17:48048508-48048535 | NFE2L1-1008 | chr17:48059105-48059132 |
| NFE2L1-298 | chr17:48048509-48048536 | NFE2L1-1009 | chr17:48059117-48059144 |
| NFE2L1-299 | chr17:48048510-48048537 | NFE2L1-1010 | chr17:48059134-48059161 |
| NFE2L1-300 | chr17:48048514-48048541 | NFE2L1-1011 | chr17:48059135-48059162 |
| NFE2L1-301 | chr17:48048516-48048543 | NFE2L1-1012 | chr17:48059140-48059167 |
| NFE2L1-302 | chr17:48048522-48048549 | NFE2L1-1013 | chr17:48059158-48059185 |
| NFE2L1-303 | chr17:48048528-48048555 | NFE2L1-1014 | chr17:48059170-48059197 |
| NFE2L1-304 | chr17:48048531-48048558 | NFE2L1-1015 | chr17:48059175-48059202 |
| NFE2L1-305 | chr17:48048534-48048561 | NFE2L1-1016 | chr17:48059176-48059203 |
| NFE2L1-306 | chr17:48048535-48048562 | NFE2L1-1017 | chr17:48059181-48059208 |
| NFE2L1-307 | chr17:48048536-48048563 | NFE2L1-1018 | chr17:48059191-48059218 |
| NFE2L1-308 | chr17:48048537-48048564 | NFE2L1-1019 | chr17:48059192-48059219 |
| NFE2L1-309 | chr17:48048545-48048572 | NFE2L1-1020 | chr17:48059199-48059226 |
| NFE2L1-310 | chr17:48048546-48048573 | NFE2L1-1021 | chr17:48059201-48059228 |
| NFE2L1-311 | chr17:48048547-48048574 | NFE2L1-1022 | chr17:48059204-48059231 |
| NFE2L1-312 | chr17:48048564-48048591 | NFE2L1-1023 | chr17:48059218-48059245 |
| NFE2L1-313 | chr17:48048565-48048592 | NFE2L1-1024 | chr17:48059222-48059249 |
| NFE2L1-314 | chr17:48048570-48048597 | NFE2L1-1025 | chr17:48059250-48059277 |
| NFE2L1-315 | chr17:48048571-48048598 | NFE2L1-1026 | chr17:48059257-48059284 |
| NFE2L1-316 | chr17:48048577-48048604 | NFE2L1-1027 | chr17:48059271-48059298 |
| NFE2L1-317 | chr17:48048578-48048605 | NFE2L1-1028 | chr17:48059272-48059299 |
| NFE2L1-318 | chr17:48048580-48048607 | NFE2L1-1029 | chr17:48059278-48059305 |
| NFE2L1-319 | chr17:48048581-48048608 | NFE2L1-1030 | chr17:48059279-48059306 |
| NFE2L1-320 | chr17:48048584-48048611 | NFE2L1-1031 | chr17:48059280-48059307 |
| NFE2L1-321 | chr17:48048585-48048612 | NFE2L1-1032 | chr17:48059281-48059308 |
| NFE2L1-322 | chr17:48048586-48048613 | NFE2L1-1033 | chr17:48059287-48059314 |
| NFE2L1-323 | chr17:48048587-48048614 | NFE2L1-1034 | chr17:48059294-48059321 |
| NFE2L1-324 | chr17:48048588-48048615 | NFE2L1-1035 | chr17:48059296-48059323 |
| NFE2L1-325 | chr17:48048592-48048619 | NFE2L1-1036 | chr17:48059297-48059324 |
| NFE2L1-326 | chr17:48048593-48048620 | NFE2L1-1037 | chr17:48059302-48059329 |
| NFE2L1-327 | chr17:48048596-48048623 | NFE2L1-1038 | chr17:48059324-48059351 |
| NFE2L1-328 | chr17:48048597-48048624 | NFE2L1-1039 | chr17:48059335-48059362 |
| NFE2L1-329 | chr17:48048598-48048625 | NFE2L1-1040 | chr17:48059342-48059369 |
| NFE2L1-330 | chr17:48048599-48048626 | NFE2L1-1041 | chr17:48059354-48059381 |
| NFE2L1-331 | chr17:48048606-48048633 | NFE2L1-1042 | chr17:48059355-48059382 |
| NFE2L1-332 | chr17:48048614-48048641 | NFE2L1-1043 | chr17:48059357-48059384 |
| NFE2L1-333 | chr17:48048615-48048642 | NFE2L1-1044 | chr17:48059359-48059386 |
| NFE2L1-334 | chr17:48048616-48048643 | NFE2L1-1045 | chr17:48059380-48059407 |
| NFE2L1-335 | chr17:48048620-48048647 | NFE2L1-1046 | chr17:48059386-48059413 |
| NFE2L1-336 | chr17:48048621-48048648 | NFE2L1-1047 | chr17:48059389-48059416 |
| NFE2L1-337 | chr17:48048622-48048649 | NFE2L1-1048 | chr17:48059390-48059417 |
| NFE2L1-338 | chr17:48048625-48048652 | NFE2L1-1049 | chr17:48059399-48059426 |
| NFE2L1-339 | chr17:48048626-48048653 | NFE2L1-1050 | chr17:48059403-48059430 |
| NFE2L1-340 | chr17:48048630-48048657 | NFE2L1-1051 | chr17:48059404-48059431 |
| NFE2L1-341 | chr17:48048635-48048662 | NFE2L1-1052 | chr17:48059431-48059458 |
| NFE2L1-342 | chr17:48048636-48048663 | NFE2L1-1053 | chr17:48059435-48059462 |
| NFE2L1-343 | chr17:48048640-48048667 | NFE2L1-1054 | chr17:48059439-48059466 |
| NFE2L1-344 | chr17:48048641-48048668 | NFE2L1-1055 | chr17:48059440-48059467 |
| NFE2L1-345 | chr17:48048644-48048671 | NFE2L1-1056 | chr17:48059453-48059480 |
| NFE2L1-346 | chr17:48048647-48048674 | NFE2L1-1057 | chr17:48059456-48059483 |
| NFE2L1-347 | chr17:48048648-48048675 | NFE2L1-1058 | chr17:48059463-48059490 |
| NFE2L1-348 | chr17:48048658-48048685 | NFE2L1-1059 | chr17:48059464-48059491 |
| NFE2L1-349 | chr17:48048663-48048690 | NFE2L1-1060 | chr17:48059467-48059494 |
| NFE2L1-350 | chr17:48048670-48048697 | NFE2L1-1061 | chr17:48059470-48059497 |
| NFE2L1-351 | chr17:48048675-48048702 | NFE2L1-1062 | chr17:48059476-48059503 |
| NFE2L1-352 | chr17:48048681-48048708 | NFE2L1-1063 | chr17:48059484-48059511 |
| NFE2L1-353 | chr17:48048682-48048709 | NFE2L1-1064 | chr17:48059516-48059543 |
| NFE2L1-354 | chr17:48048687-48048714 | NFE2L1-1065 | chr17:48059517-48059544 |
| NFE2L1-355 | chr17:48048688-48048715 | NFE2L1-1066 | chr17:48059525-48059552 |
| NFE2L1-356 | chr17:48048689-48048716 | NFE2L1-1067 | chr17:48059526-48059553 |
| NFE2L1-357 | chr17:48048692-48048719 | NFE2L1-1068 | chr17:48059527-48059554 |
| NFE2L1-358 | chr17:48048693-48048720 | NFE2L1-1069 | chr17:48059528-48059555 |
| NFE2L1-359 | chr17:48048694-48048721 | NFE2L1-1070 | chr17:48059532-48059559 |
| NFE2L1-360 | chr17:48048698-48048725 | NFE2L1-1071 | chr17:48059542-48059569 |
| NFE2L1-361 | chr17:48048701-48048728 | NFE2L1-1072 | chr17:48059550-48059577 |
| NFE2L1-362 | chr17:48048702-48048729 | NFE2L1-1073 | chr17:48059561-48059588 |
| NFE2L1-363 | chr17:48048706-48048733 | NFE2L1-1074 | chr17:48059566-48059593 |
| NFE2L1-364 | chr17:48048709-48048736 | NFE2L1-1075 | chr17:48059569-48059596 |
| NFE2L1-365 | chr17:48048710-48048737 | NFE2L1-1076 | chr17:48059573-48059600 |
| NFE2L1-366 | chr17:48048713-48048740 | NFE2L1-1077 | chr17:48059575-48059602 |
| NFE2L1-367 | chr17:48048714-48048741 | NFE2L1-1078 | chr17:48059576-48059603 |
| NFE2L1-368 | chr17:48048715-48048742 | NFE2L1-1079 | chr17:48059577-48059604 |
| NFE2L1-369 | chr17:48048718-48048745 | NFE2L1-1080 | chr17:48059578-48059605 |
| NFE2L1-370 | chr17:48048719-48048746 | NFE2L1-1081 | chr17:48059581-48059608 |
| NFE2L1-371 | chr17:48048720-48048747 | NFE2L1-1082 | chr17:48059585-48059612 |
| NFE2L1-372 | chr17:48048722-48048749 | NFE2L1-1083 | chr17:48059589-48059616 |
| NFE2L1-373 | chr17:48048732-48048759 | NFE2L1-1084 | chr17:48059590-48059617 |
| NFE2L1-374 | chr17:48048733-48048760 | NFE2L1-1085 | chr17:48059593-48059620 |
| NFE2L1-375 | chr17:48048736-48048763 | NFE2L1-1086 | chr17:48059600-48059627 |
| NFE2L1-376 | chr17:48048737-48048764 | NFE2L1-1087 | chr17:48059601-48059628 |
| NFE2L1-377 | chr17:48048743-48048770 | NFE2L1-1088 | chr17:48059602-48059629 |
| NFE2L1-378 | chr17:48048748-48048775 | NFE2L1-1089 | chr17:48059605-48059632 |
| NFE2L1-379 | chr17:48048749-48048776 | NFE2L1-1090 | chr17:48059620-48059647 |
| NFE2L1-380 | chr17:48048769-48048796 | NFE2L1-1091 | chr17:48059621-48059648 |
| NFE2L1-381 | chr17:48048771-48048798 | NFE2L1-1092 | chr17:48059622-48059649 |
| NFE2L1-382 | chr17:48048779-48048806 | NFE2L1-1093 | chr17:48059629-48059656 |
| NFE2L1-383 | chr17:48048782-48048809 | NFE2L1-1094 | chr17:48059630-48059657 |
| NFE2L1-384 | chr17:48048795-48048822 | NFE2L1-1095 | chr17:48059631-48059658 |
| NFE2L1-385 | chr17:48048797-48048824 | NFE2L1-1096 | chr17:48059632-48059659 |
| NFE2L1-386 | chr17:48048800-48048827 | NFE2L1-1097 | chr17:48059643-48059670 |
| NFE2L1-387 | chr17:48048801-48048828 | NFE2L1-1098 | chr17:48059659-48059686 |
| NFE2L1-388 | chr17:48048802-48048829 | NFE2L1-1099 | chr17:48059665-48059692 |
| NFE2L1-389 | chr17:48048807-48048834 | NFE2L1-1100 | chr17:48059666-48059693 |
| NFE2L1-390 | chr17:48048808-48048835 | NFE2L1-1101 | chr17:48059667-48059694 |
| NFE2L1-391 | chr17:48048813-48048840 | NFE2L1-1102 | chr17:48059668-48059695 |
| NFE2L1-392 | chr17:48048819-48048846 | NFE2L1-1103 | chr17:48059670-48059697 |
| NFE2L1-393 | chr17:48048821-48048848 | NFE2L1-1104 | chr17:48059671-48059698 |
| NFE2L1-394 | chr17:48048825-48048852 | NFE2L1-1105 | chr17:48059676-48059703 |
| NFE2L1-395 | chr17:48048831-48048858 | NFE2L1-1106 | chr17:48059677-48059704 |
| NFE2L1-396 | chr17:48048837-48048864 | NFE2L1-1107 | chr17:48059682-48059709 |
| NFE2L1-397 | chr17:48048838-48048865 | NFE2L1-1108 | chr17:48059688-48059715 |
| NFE2L1-398 | chr17:48048845-48048872 | NFE2L1-1109 | chr17:48059699-48059726 |
| NFE2L1-399 | chr17:48048846-48048873 | NFE2L1-1110 | chr17:48059700-48059727 |
| NFE2L1-400 | chr17:48048850-48048877 | NFE2L1-1111 | chr17:48059701-48059728 |
| NFE2L1-401 | chr17:48048851-48048878 | NFE2L1-1112 | chr17:48059705-48059732 |
| NFE2L1-402 | chr17:48048854-48048881 | NFE2L1-1113 | chr17:48059711-48059738 |
| NFE2L1-403 | chr17:48048855-48048882 | NFE2L1-1114 | chr17:48059717-48059744 |
| NFE2L1-404 | chr17:48048867-48048894 | NFE2L1-1115 | chr17:48059738-48059765 |
| NFE2L1-405 | chr17:48048869-48048896 | NFE2L1-1116 | chr17:48059740-48059767 |
| NFE2L1-406 | chr17:48048873-48048900 | NFE2L1-1117 | chr17:48059741-48059768 |
| NFE2L1-407 | chr17:48048874-48048901 | NFE2L1-1118 | chr17:48059747-48059774 |
| NFE2L1-408 | chr17:48048884-48048911 | NFE2L1-1119 | chr17:48059751-48059778 |
| NFE2L1-409 | chr17:48048888-48048915 | NFE2L1-1120 | chr17:48059752-48059779 |
| NFE2L1-410 | chr17:48048899-48048926 | NFE2L1-1121 | chr17:48059764-48059791 |
| NFE2L1-411 | chr17:48048900-48048927 | NFE2L1-1122 | chr17:48059770-48059797 |
| NFE2L1-412 | chr17:48048902-48048929 | NFE2L1-1123 | chr17:48059774-48059801 |
| NFE2L1-413 | chr17:48048903-48048930 | NFE2L1-1124 | chr17:48059780-48059807 |
| NFE2L1-414 | chr17:48048909-48048936 | NFE2L1-1125 | chr17:48059784-48059811 |
| NFE2L1-415 | chr17:48048911-48048938 | NFE2L1-1126 | chr17:48059799-48059826 |
| NFE2L1-416 | chr17:48048929-48048956 | NFE2L1-1127 | chr17:48059800-48059827 |
| NFE2L1-417 | chr17:48048935-48048962 | NFE2L1-1128 | chr17:48059803-48059830 |
| NFE2L1-418 | chr17:48048936-48048963 | NFE2L1-1129 | chr17:48059808-48059835 |
| NFE2L1-419 | chr17:48048937-48048964 | NFE2L1-1130 | chr17:48059814-48059841 |
| NFE2L1-420 | chr17:48048940-48048967 | NFE2L1-1131 | chr17:48059819-48059846 |
| NFE2L1-421 | chr17:48048941-48048968 | NFE2L1-1132 | chr17:48059831-48059858 |
| NFE2L1-422 | chr17:48048959-48048986 | NFE2L1-1133 | chr17:48059836-48059863 |
| NFE2L1-423 | chr17:48050502-48050529 | NFE2L1-1134 | chr17:48059837-48059864 |
| NFE2L1-424 | chr17:48050509-48050536 | NFE2L1-1135 | chr17:48059842-48059869 |
| NFE2L1-425 | chr17:48050521-48050548 | NFE2L1-1136 | chr17:48059847-48059874 |
| NFE2L1-426 | chr17:48050541-48050568 | NFE2L1-1137 | chr17:48059864-48059891 |
| NFE2L1-427 | chr17:48050543-48050570 | NFE2L1-1138 | chr17:48059866-48059893 |
| NFE2L1-428 | chr17:48050545-48050572 | NFE2L1-1139 | chr17:48059868-48059895 |
| NFE2L1-429 | chr17:48050546-48050573 | NFE2L1-1140 | chr17:48059869-48059896 |
| NFE2L1-430 | chr17:48050579-48050606 | NFE2L1-1141 | chr17:48059870-48059897 |
| NFE2L1-431 | chr17:48050580-48050607 | NFE2L1-1142 | chr17:48059871-48059898 |
| NFE2L1-432 | chr17:48050589-48050616 | NFE2L1-1143 | chr17:48059872-48059899 |
| NFE2L1-433 | chr17:48050597-48050624 | NFE2L1-1144 | chr17:48059873-48059900 |
| NFE2L1-434 | chr17:48050598-48050625 | NFE2L1-1145 | chr17:48059876-48059903 |
| NFE2L1-435 | chr17:48050601-48050628 | NFE2L1-1146 | chr17:48059877-48059904 |
| NFE2L1-436 | chr17:48050610-48050637 | NFE2L1-1147 | chr17:48059878-48059905 |
| NFE2L1-437 | chr17:48050613-48050640 | NFE2L1-1148 | chr17:48059879-48059906 |
| NFE2L1-438 | chr17:48050616-48050643 | NFE2L1-1149 | chr17:48059883-48059910 |
| NFE2L1-439 | chr17:48050619-48050646 | NFE2L1-1150 | chr17:48059888-48059915 |
| NFE2L1-440 | chr17:48050620-48050647 | NFE2L1-1151 | chr17:48059891-48059918 |
| NFE2L1-441 | chr17:48050621-48050648 | NFE2L1-1152 | chr17:48059897-48059924 |
| NFE2L1-442 | chr17:48050622-48050649 | NFE2L1-1153 | chr17:48059907-48059934 |
| NFE2L1-443 | chr17:48050623-48050650 | NFE2L1-1154 | chr17:48059915-48059942 |
| NFE2L1-444 | chr17:48050624-48050651 | NFE2L1-1155 | chr17:48059925-48059952 |
| NFE2L1-445 | chr17:48050643-48050670 | NFE2L1-1156 | chr17:48059926-48059953 |
| NFE2L1-446 | chr17:48050657-48050684 | NFE2L1-1157 | chr17:48059927-48059954 |
| NFE2L1-447 | chr17:48050658-48050685 | NFE2L1-1158 | chr17:48059931-48059958 |
| NFE2L1-448 | chr17:48050671-48050698 | NFE2L1-1159 | chr17:48059936-48059963 |
| NFE2L1-449 | chr17:48050677-48050704 | NFE2L1-1160 | chr17:48059943-48059970 |
| NFE2L1-450 | chr17:48050680-48050707 | NFE2L1-1161 | chr17:48059948-48059975 |
| NFE2L1-451 | chr17:48050681-48050708 | NFE2L1-1162 | chr17:48059952-48059979 |
| NFE2L1-452 | chr17:48050684-48050711 | NFE2L1-1163 | chr17:48059953-48059980 |
| NFE2L1-453 | chr17:48050687-48050714 | NFE2L1-1164 | chr17:48059957-48059984 |
| NFE2L1-454 | chr17:48050698-48050725 | NFE2L1-1165 | chr17:48059960-48059987 |
| NFE2L1-455 | chr17:48050726-48050753 | NFE2L1-1166 | chr17:48059965-48059992 |
| NFE2L1-456 | chr17:48050731-48050758 | NFE2L1-1167 | chr17:48059979-48060006 |
| NFE2L1-457 | chr17:48050738-48050765 | NFE2L1-1168 | chr17:48059983-48060010 |
| NFE2L1-458 | chr17:48050745-48050772 | NFE2L1-1169 | chr17:48059986-48060013 |
| NFE2L1-459 | chr17:48050749-48050776 | NFE2L1-1170 | chr17:48059987-48060014 |
| NFE2L1-460 | chr17:48050764-48050791 | NFE2L1-1171 | chr17:48059995-48060022 |
| NFE2L1-461 | chr17:48050780-48050807 | NFE2L1-1172 | chr17:48059999-48060026 |
| NFE2L1-462 | chr17:48050787-48050814 | NFE2L1-1173 | chr17:48060003-48060030 |
| NFE2L1-463 | chr17:48050805-48050832 | NFE2L1-1174 | chr17:48060007-48060034 |
| NFE2L1-464 | chr17:48050806-48050833 | NFE2L1-1175 | chr17:48060037-48060064 |
| NFE2L1-465 | chr17:48050815-48050842 | NFE2L1-1176 | chr17:48060038-48060065 |
| NFE2L1-466 | chr17:48050821-48050848 | NFE2L1-1177 | chr17:48060039-48060066 |
| NFE2L1-467 | chr17:48050826-48050853 | NFE2L1-1178 | chr17:48060040-48060067 |
| NFE2L1-468 | chr17:48050827-48050854 | NFE2L1-1179 | chr17:48060041-48060068 |
| NFE2L1-469 | chr17:48050832-48050859 | NFE2L1-1180 | chr17:48060042-48060069 |
| NFE2L1-470 | chr17:48050834-48050861 | NFE2L1-1181 | chr17:48060043-48060070 |
| NFE2L1-471 | chr17:48050835-48050862 | NFE2L1-1182 | chr17:48060044-48060071 |
| NFE2L1-472 | chr17:48050844-48050871 | NFE2L1-1183 | chr17:48060045-48060072 |
| NFE2L1-473 | chr17:48050852-48050879 | NFE2L1-1184 | chr17:48060046-48060073 |
| NFE2L1-474 | chr17:48050857-48050884 | NFE2L1-1185 | chr17:48060047-48060074 |
| NFE2L1-475 | chr17:48050868-48050895 | NFE2L1-1186 | chr17:48060048-48060075 |
| NFE2L1-476 | chr17:48050869-48050896 | NFE2L1-1187 | chr17:48060056-48060083 |
| NFE2L1-477 | chr17:48050882-48050909 | NFE2L1-1188 | chr17:48060057-48060084 |
| NFE2L1-478 | chr17:48050887-48050914 | NFE2L1-1189 | chr17:48060061-48060088 |
| NFE2L1-479 | chr17:48050900-48050927 | NFE2L1-1190 | chr17:48060067-48060094 |
| NFE2L1-480 | chr17:48050906-48050933 | NFE2L1-1191 | chr17:48060071-48060098 |
| NFE2L1-481 | chr17:48050915-48050942 | NFE2L1-1192 | chr17:48060077-48060104 |
| NFE2L1-482 | chr17:48050916-48050943 | NFE2L1-1193 | chr17:48060082-48060109 |
| NFE2L1-483 | chr17:48050922-48050949 | NFE2L1-1194 | chr17:48060090-48060117 |
| NFE2L1-484 | chr17:48050928-48050955 | NFE2L1-1195 | chr17:48060097-48060124 |
| NFE2L1-485 | chr17:48050931-48050958 | NFE2L1-1196 | chr17:48060101-48060128 |
| NFE2L1-486 | chr17:48050932-48050959 | NFE2L1-1197 | chr17:48060105-48060132 |
| NFE2L1-487 | chr17:48050933-48050960 | NFE2L1-1198 | chr17:48060110-48060137 |
| NFE2L1-488 | chr17:48050936-48050963 | NFE2L1-1199 | chr17:48060113-48060140 |
| NFE2L1-489 | chr17:48050941-48050968 | NFE2L1-1200 | chr17:48060114-48060141 |
| NFE2L1-490 | chr17:48050942-48050969 | NFE2L1-1201 | chr17:48060127-48060154 |
| NFE2L1-491 | chr17:48050952-48050979 | NFE2L1-1202 | chr17:48060142-48060169 |
| NFE2L1-492 | chr17:48050961-48050988 | NFE2L1-1203 | chr17:48060154-48060181 |
| NFE2L1-493 | chr17:48050967-48050994 | NFE2L1-1204 | chr17:48060155-48060182 |
| NFE2L1-494 | chr17:48050968-48050995 | NFE2L1-1205 | chr17:48060156-48060183 |
| NFE2L1-495 | chr17:48050971-48050998 | NFE2L1-1206 | chr17:48060157-48060184 |
| NFE2L1-496 | chr17:48050972-48050999 | NFE2L1-1207 | chr17:48060158-48060185 |
| NFE2L1-497 | chr17:48050973-48051000 | NFE2L1-1208 | chr17:48060159-48060186 |
| NFE2L1-498 | chr17:48050977-48051004 | NFE2L1-1209 | chr17:48060160-48060187 |
| NFE2L1-499 | chr17:48050978-48051005 | NFE2L1-1210 | chr17:48060163-48060190 |
| NFE2L1-500 | chr17:48050979-48051006 | NFE2L1-1211 | chr17:48060164-48060191 |
| NFE2L1-501 | chr17:48050980-48051007 | NFE2L1-1212 | chr17:48060165-48060192 |
| NFE2L1-502 | chr17:48050990-48051017 | NFE2L1-1213 | chr17:48060166-48060193 |
| NFE2L1-503 | chr17:48050993-48051020 | NFE2L1-1214 | chr17:48060176-48060203 |
| NFE2L1-504 | chr17:48050996-48051023 | NFE2L1-1215 | chr17:48060177-48060204 |
| NFE2L1-505 | chr17:48050997-48051024 | NFE2L1-1216 | chr17:48060178-48060205 |
| NFE2L1-506 | chr17:48051008-48051035 | NFE2L1-1217 | chr17:48060179-48060206 |
| NFE2L1-507 | chr17:48051012-48051039 | NFE2L1-1218 | chr17:48060180-48060207 |
| NFE2L1-508 | chr17:48051017-48051044 | NFE2L1-1219 | chr17:48060183-48060210 |
| NFE2L1-509 | chr17:48051028-48051055 | NFE2L1-1220 | chr17:48060184-48060211 |
| NFE2L1-510 | chr17:48051046-48051073 | NFE2L1-1221 | chr17:48060185-48060212 |
| NFE2L1-511 | chr17:48051056-48051083 | NFE2L1-1222 | chr17:48060186-48060213 |
| NFE2L1-512 | chr17:48051081-48051108 | NFE2L1-1223 | chr17:48060189-48060216 |
| NFE2L1-513 | chr17:48051087-48051114 | NFE2L1-1224 | chr17:48060190-48060217 |
| NFE2L1-514 | chr17:48051110-48051137 | NFE2L1-1225 | chr17:48060191-48060218 |
| NFE2L1-515 | chr17:48051119-48051146 | NFE2L1-1226 | chr17:48060194-48060221 |
| NFE2L1-516 | chr17:48051123-48051150 | NFE2L1-1227 | chr17:48060195-48060222 |
| NFE2L1-517 | chr17:48051157-48051184 | NFE2L1-1228 | chr17:48060199-48060226 |
| NFE2L1-518 | chr17:48051159-48051186 | NFE2L1-1229 | chr17:48060241-48060268 |
| NFE2L1-519 | chr17:48051160-48051187 | NFE2L1-1230 | chr17:48060242-48060269 |
| NFE2L1-520 | chr17:48051161-48051188 | NFE2L1-1231 | chr17:48060243-48060270 |
| NFE2L1-521 | chr17:48051165-48051192 | NFE2L1-1232 | chr17:48060244-48060271 |
| NFE2L1-522 | chr17:48051166-48051193 | NFE2L1-1233 | chr17:48060252-48060279 |
| NFE2L1-523 | chr17:48051167-48051194 | NFE2L1-1234 | chr17:48060260-48060287 |
| NFE2L1-524 | chr17:48051170-48051197 | NFE2L1-1235 | chr17:48060261-48060288 |
| NFE2L1-525 | chr17:48051176-48051203 | NFE2L1-1236 | chr17:48060262-48060289 |
| NFE2L1-526 | chr17:48051211-48051238 | NFE2L1-1237 | chr17:48060281-48060308 |
| NFE2L1-527 | chr17:48051212-48051239 | NFE2L1-1238 | chr17:48060290-48060317 |
| NFE2L1-528 | chr17:48051216-48051243 | NFE2L1-1239 | chr17:48060291-48060318 |
| NFE2L1-529 | chr17:48051224-48051251 | NFE2L1-1240 | chr17:48060303-48060330 |
| NFE2L1-530 | chr17:48051225-48051252 | NFE2L1-1241 | chr17:48060311-48060338 |
| NFE2L1-531 | chr17:48051226-48051253 | NFE2L1-1242 | chr17:48060319-48060346 |
| NFE2L1-532 | chr17:48051227-48051254 | NFE2L1-1243 | chr17:48060320-48060347 |
| NFE2L1-533 | chr17:48051228-48051255 | NFE2L1-1244 | chr17:48060321-48060348 |
| NFE2L1-534 | chr17:48051229-48051256 | NFE2L1-1245 | chr17:48060325-48060352 |
| NFE2L1-535 | chr17:48051230-48051257 | NFE2L1-1246 | chr17:48060326-48060353 |
| NFE2L1-536 | chr17:48051235-48051262 | NFE2L1-1247 | chr17:48060339-48060366 |
| NFE2L1-537 | chr17:48051247-48051274 | NFE2L1-1248 | chr17:48060344-48060371 |
| NFE2L1-538 | chr17:48051254-48051281 | NFE2L1-1249 | chr17:48060345-48060372 |
| NFE2L1-539 | chr17:48051255-48051282 | NFE2L1-1250 | chr17:48060353-48060380 |
| NFE2L1-540 | chr17:48051264-48051291 | NFE2L1-1251 | chr17:48060382-48060409 |
| NFE2L1-541 | chr17:48051268-48051295 | NFE2L1-1252 | chr17:48060383-48060410 |
| NFE2L1-542 | chr17:48051276-48051303 | NFE2L1-1253 | chr17:48060397-48060424 |
| NFE2L1-543 | chr17:48051277-48051304 | NFE2L1-1254 | chr17:48060398-48060425 |
| NFE2L1-544 | chr17:48051278-48051305 | NFE2L1-1255 | chr17:48060399-48060426 |
| NFE2L1-545 | chr17:48051282-48051309 | NFE2L1-1256 | chr17:48060424-48060451 |
| NFE2L1-546 | chr17:48051283-48051310 | NFE2L1-1257 | chr17:48060429-48060456 |
| NFE2L1-547 | chr17:48051288-48051315 | NFE2L1-1258 | chr17:48060434-48060461 |
| NFE2L1-548 | chr17:48051289-48051316 | NFE2L1-1259 | chr17:48060440-48060467 |
| NFE2L1-549 | chr17:48051300-48051327 | NFE2L1-1260 | chr17:48060441-48060468 |
| NFE2L1-550 | chr17:48051314-48051341 | NFE2L1-1261 | chr17:48060444-48060471 |
| NFE2L1-551 | chr17:48051319-48051346 | NFE2L1-1262 | chr17:48060451-48060478 |
| NFE2L1-552 | chr17:48051322-48051349 | NFE2L1-1263 | chr17:48060455-48060482 |
| NFE2L1-553 | chr17:48051323-48051350 | NFE2L1-1264 | chr17:48060458-48060485 |
| NFE2L1-554 | chr17:48051324-48051351 | NFE2L1-1265 | chr17:48060459-48060486 |
| NFE2L1-555 | chr17:48051334-48051361 | NFE2L1-1266 | chr17:48060460-48060487 |
| NFE2L1-556 | chr17:48051337-48051364 | NFE2L1-1267 | chr17:48060474-48060501 |
| NFE2L1-557 | chr17:48051350-48051377 | NFE2L1-1268 | chr17:48060475-48060502 |
| NFE2L1-558 | chr17:48051355-48051382 | NFE2L1-1269 | chr17:48060484-48060511 |
| NFE2L1-559 | chr17:48051356-48051383 | NFE2L1-1270 | chr17:48060493-48060520 |
| NFE2L1-560 | chr17:48051357-48051384 | NFE2L1-1271 | chr17:48060498-48060525 |
| NFE2L1-561 | chr17:48051358-48051385 | NFE2L1-1272 | chr17:48060507-48060534 |
| NFE2L1-562 | chr17:48051362-48051389 | NFE2L1-1273 | chr17:48060511-48060538 |
| NFE2L1-563 | chr17:48051367-48051394 | NFE2L1-1274 | chr17:48060514-48060541 |
| NFE2L1-564 | chr17:48051374-48051401 | NFE2L1-1275 | chr17:48060515-48060542 |
| NFE2L1-565 | chr17:48051384-48051411 | NFE2L1-1276 | chr17:48060516-48060543 |
| NFE2L1-566 | chr17:48051385-48051412 | NFE2L1-1277 | chr17:48060522-48060549 |
| NFE2L1-567 | chr17:48051389-48051416 | NFE2L1-1278 | chr17:48060536-48060563 |
| NFE2L1-568 | chr17:48051397-48051424 | NFE2L1-1279 | chr17:48060537-48060564 |
| NFE2L1-569 | chr17:48051400-48051427 | NFE2L1-1280 | chr17:48060538-48060565 |
| NFE2L1-570 | chr17:48051404-48051431 | NFE2L1-1281 | chr17:48060541-48060568 |
| NFE2L1-571 | chr17:48051408-48051435 | NFE2L1-1282 | chr17:48060542-48060569 |
| NFE2L1-572 | chr17:48051422-48051449 | NFE2L1-1283 | chr17:48060543-48060570 |
| NFE2L1-573 | chr17:48051423-48051450 | NFE2L1-1284 | chr17:48060549-48060576 |
| NFE2L1-574 | chr17:48051424-48051451 | NFE2L1-1285 | chr17:48060550-48060577 |
| NFE2L1-575 | chr17:48051435-48051462 | NFE2L1-1286 | chr17:48060551-48060578 |
| NFE2L1-576 | chr17:48051436-48051463 | NFE2L1-1287 | chr17:48060555-48060582 |
| NFE2L1-577 | chr17:48051442-48051469 | NFE2L1-1288 | chr17:48060556-48060583 |
| NFE2L1-578 | chr17:48051443-48051470 | NFE2L1-1289 | chr17:48060562-48060589 |
| NFE2L1-579 | chr17:48051453-48051480 | NFE2L1-1290 | chr17:48060573-48060600 |
| NFE2L1-580 | chr17:48051470-48051497 | NFE2L1-1291 | chr17:48060574-48060601 |
| NFE2L1-581 | chr17:48051471-48051498 | NFE2L1-1292 | chr17:48060580-48060607 |
| NFE2L1-582 | chr17:48051477-48051504 | NFE2L1-1293 | chr17:48060581-48060608 |
| NFE2L1-583 | chr17:48051481-48051508 | NFE2L1-1294 | chr17:48060597-48060624 |
| NFE2L1-584 | chr17:48051486-48051513 | NFE2L1-1295 | chr17:48060602-48060629 |
| NFE2L1-585 | chr17:48051487-48051514 | NFE2L1-1296 | chr17:48060603-48060630 |
| NFE2L1-586 | chr17:48051495-48051522 | NFE2L1-1297 | chr17:48060609-48060636 |
| NFE2L1-587 | chr17:48051498-48051525 | NFE2L1-1298 | chr17:48060615-48060642 |
| NFE2L1-588 | chr17:48051505-48051532 | NFE2L1-1299 | chr17:48060616-48060643 |
| NFE2L1-589 | chr17:48051507-48051534 | NFE2L1-1300 | chr17:48060620-48060647 |
| NFE2L1-590 | chr17:48051508-48051535 | NFE2L1-1301 | chr17:48060621-48060648 |
| NFE2L1-591 | chr17:48051509-48051536 | NFE2L1-1302 | chr17:48060629-48060656 |
| NFE2L1-592 | chr17:48051523-48051550 | NFE2L1-1303 | chr17:48060654-48060681 |
| NFE2L1-593 | chr17:48051524-48051551 | NFE2L1-1304 | chr17:48060658-48060685 |
| NFE2L1-594 | chr17:48051527-48051554 | NFE2L1-1305 | chr17:48060674-48060701 |
| NFE2L1-595 | chr17:48051533-48051560 | NFE2L1-1306 | chr17:48060675-48060702 |
| NFE2L1-596 | chr17:48051534-48051561 | NFE2L1-1307 | chr17:48060676-48060703 |
| NFE2L1-597 | chr17:48051540-48051567 | NFE2L1-1308 | chr17:48060677-48060704 |
| NFE2L1-598 | chr17:48051551-48051578 | NFE2L1-1309 | chr17:48060694-48060721 |
| NFE2L1-599 | chr17:48051554-48051581 | NFE2L1-1310 | chr17:48060695-48060722 |
| NFE2L1-600 | chr17:48051560-48051587 | NFE2L1-1311 | chr17:48060710-48060737 |
| NFE2L1-601 | chr17:48051561-48051588 | NFE2L1-1312 | chr17:48060711-48060738 |
| NFE2L1-602 | chr17:48051562-48051589 | NFE2L1-1313 | chr17:48060715-48060742 |
| NFE2L1-603 | chr17:48051563-48051590 | NFE2L1-1314 | chr17:48060716-48060743 |
| NFE2L1-604 | chr17:48051585-48051612 | NFE2L1-1315 | chr17:48060717-48060744 |
| NFE2L1-605 | chr17:48051597-48051624 | NFE2L1-1316 | chr17:48060723-48060750 |
| NFE2L1-606 | chr17:48051598-48051625 | NFE2L1-1317 | chr17:48060728-48060755 |
| NFE2L1-607 | chr17:48051599-48051626 | NFE2L1-1318 | chr17:48060729-48060756 |
| NFE2L1-608 | chr17:48051600-48051627 | NFE2L1-1319 | chr17:48060733-48060760 |
| NFE2L1-609 | chr17:48051601-48051628 | NFE2L1-1320 | chr17:48060778-48060805 |
| NFE2L1-610 | chr17:48051602-48051629 | NFE2L1-1321 | chr17:48060779-48060806 |
| NFE2L1-611 | chr17:48051609-48051636 | NFE2L1-1322 | chr17:48060802-48060829 |
| NFE2L1-612 | chr17:48051617-48051644 | NFE2L1-1323 | chr17:48060809-48060836 |
| NFE2L1-613 | chr17:48051620-48051647 | NFE2L1-1324 | chr17:48060810-48060837 |
| NFE2L1-614 | chr17:48051621-48051648 | NFE2L1-1325 | chr17:48060834-48060861 |
| NFE2L1-615 | chr17:48051628-48051655 | NFE2L1-1326 | chr17:48060835-48060862 |
| NFE2L1-616 | chr17:48051629-48051656 | NFE2L1-1327 | chr17:48060844-48060871 |
| NFE2L1-617 | chr17:48051630-48051657 | NFE2L1-1328 | chr17:48060845-48060872 |
| NFE2L1-618 | chr17:48051635-48051662 | NFE2L1-1329 | chr17:48060863-48060890 |
| NFE2L1-619 | chr17:48051636-48051663 | NFE2L1-1330 | chr17:48060896-48060923 |
| NFE2L1-620 | chr17:48051637-48051664 | NFE2L1-1331 | chr17:48060897-48060924 |
| NFE2L1-621 | chr17:48051638-48051665 | NFE2L1-1332 | chr17:48060929-48060956 |
| NFE2L1-622 | chr17:48051642-48051669 | NFE2L1-1333 | chr17:48060936-48060963 |
| NFE2L1-623 | chr17:48051643-48051670 | NFE2L1-1334 | chr17:48060937-48060964 |
| NFE2L1-624 | chr17:48051644-48051671 | NFE2L1-1335 | chr17:48060940-48060967 |
| NFE2L1-625 | chr17:48051645-48051672 | NFE2L1-1336 | chr17:48060959-48060986 |
| NFE2L1-626 | chr17:48051648-48051675 | NFE2L1-1337 | chr17:48060973-48061000 |
| NFE2L1-627 | chr17:48051649-48051676 | NFE2L1-1338 | chr17:48060980-48061007 |
| NFE2L1-628 | chr17:48051653-48051680 | NFE2L1-1339 | chr17:48060981-48061008 |
| NFE2L1-629 | chr17:48051658-48051685 | NFE2L1-1340 | chr17:48060982-48061009 |
| NFE2L1-630 | chr17:48051662-48051689 | NFE2L1-1341 | chr17:48060983-48061010 |
| NFE2L1-631 | chr17:48051669-48051696 | NFE2L1-1342 | chr17:48061019-48061046 |
| NFE2L1-632 | chr17:48051682-48051709 | NFE2L1-1343 | chr17:48061056-48061083 |
| NFE2L1-633 | chr17:48051683-48051710 | NFE2L1-1344 | chr17:48061059-48061086 |
| NFE2L1-634 | chr17:48051703-48051730 | NFE2L1-1345 | chr17:48061060-48061087 |
| NFE2L1-635 | chr17:48051710-48051737 | NFE2L1-1346 | chr17:48061061-48061088 |
| NFE2L1-636 | chr17:48051711-48051738 | NFE2L1-1347 | chr17:48061081-48061108 |
| NFE2L1-637 | chr17:48051713-48051740 | NFE2L1-1348 | chr17:48061091-48061118 |
| NFE2L1-638 | chr17:48051714-48051741 | NFE2L1-1349 | chr17:48061096-48061123 |
| NFE2L1-639 | chr17:48051721-48051748 | NFE2L1-1350 | chr17:48061098-48061125 |
| NFE2L1-640 | chr17:48051722-48051749 | NFE2L1-1351 | chr17:48061101-48061128 |
| NFE2L1-641 | chr17:48056263-48056290 | NFE2L1-1352 | chr17:48061102-48061129 |
| NFE2L1-642 | chr17:48056272-48056299 | NFE2L1-1353 | chr17:48061103-48061130 |
| NFE2L1-643 | chr17:48056273-48056300 | NFE2L1-1354 | chr17:48061104-48061131 |
| NFE2L1-644 | chr17:48056274-48056301 | NFE2L1-1355 | chr17:48061110-48061137 |
| NFE2L1-645 | chr17:48056280-48056307 | NFE2L1-1356 | chr17:48061111-48061138 |
| NFE2L1-646 | chr17:48056281-48056308 | NFE2L1-1357 | chr17:48061112-48061139 |
| NFE2L1-647 | chr17:48056282-48056309 | NFE2L1-1358 | chr17:48061113-48061140 |
| NFE2L1-648 | chr17:48056284-48056311 | NFE2L1-1359 | chr17:48061116-48061143 |
| NFE2L1-649 | chr17:48056285-48056312 | NFE2L1-1360 | chr17:48061121-48061148 |
| NFE2L1-650 | chr17:48056286-48056313 | NFE2L1-1361 | chr17:48061125-48061152 |
| NFE2L1-651 | chr17:48056287-48056314 | NFE2L1-1362 | chr17:48061126-48061153 |
| NFE2L1-652 | chr17:48056288-48056315 | NFE2L1-1363 | chr17:48061133-48061160 |
| NFE2L1-653 | chr17:48056298-48056325 | NFE2L1-1364 | chr17:48061136-48061163 |
| NFE2L1-654 | chr17:48056299-48056326 | NFE2L1-1365 | chr17:48061139-48061166 |
| NFE2L1-655 | chr17:48056300-48056327 | NFE2L1-1366 | chr17:48061147-48061174 |
| NFE2L1-656 | chr17:48056304-48056331 | NFE2L1-1367 | chr17:48061154-48061181 |
| NFE2L1-657 | chr17:48056310-48056337 | NFE2L1-1368 | chr17:48061158-48061185 |
| NFE2L1-658 | chr17:48056314-48056341 | NFE2L1-1369 | chr17:48061167-48061194 |
| NFE2L1-659 | chr17:48056315-48056342 | NFE2L1-1370 | chr17:48061177-48061204 |
| NFE2L1-660 | chr17:48056322-48056349 | NFE2L1-1371 | chr17:48061209-48061236 |
| NFE2L1-661 | chr17:48056323-48056350 | NFE2L1-1372 | chr17:48061211-48061238 |
| NFE2L1-662 | chr17:48056344-48056371 | NFE2L1-1373 | chr17:48061215-48061242 |
| NFE2L1-663 | chr17:48056359-48056386 | NFE2L1-1374 | chr17:48061220-48061247 |
| NFE2L1-664 | chr17:48056385-48056412 | NFE2L1-1375 | chr17:48061221-48061248 |
| NFE2L1-665 | chr17:48056392-48056419 | NFE2L1-1376 | chr17:48061226-48061253 |
| NFE2L1-666 | chr17:48056404-48056431 | NFE2L1-1377 | chr17:48061227-48061254 |
| NFE2L1-667 | chr17:48056405-48056432 | NFE2L1-1378 | chr17:48061228-48061255 |
| NFE2L1-668 | chr17:48056406-48056433 | NFE2L1-1379 | chr17:48061232-48061259 |
| NFE2L1-669 | chr17:48056407-48056434 | NFE2L1-1380 | chr17:48061233-48061260 |
| NFE2L1-670 | chr17:48056411-48056438 | NFE2L1-1381 | chr17:48061240-48061267 |
| NFE2L1-671 | chr17:48056412-48056439 | NFE2L1-1382 | chr17:48061241-48061268 |
| NFE2L1-672 | chr17:48056419-48056446 | NFE2L1-1383 | chr17:48061247-48061274 |
| NFE2L1-673 | chr17:48056446-48056473 | NFE2L1-1384 | chr17:48061251-48061278 |
| NFE2L1-674 | chr17:48056452-48056479 | NFE2L1-1385 | chr17:48061252-48061279 |
| NFE2L1-675 | chr17:48056458-48056485 | NFE2L1-1386 | chr17:48061253-48061280 |
| NFE2L1-676 | chr17:48056463-48056490 | NFE2L1-1387 | chr17:48061254-48061281 |
| NFE2L1-677 | chr17:48056464-48056491 | NFE2L1-1388 | chr17:48061255-48061282 |
| NFE2L1-678 | chr17:48056466-48056493 | NFE2L1-1389 | chr17:48061256-48061283 |
| NFE2L1-679 | chr17:48056477-48056504 | NFE2L1-1390 | chr17:48061257-48061284 |
| NFE2L1-680 | chr17:48056480-48056507 | NFE2L1-1391 | chr17:48061258-48061285 |
| NFE2L1-681 | chr17:48056488-48056515 | NFE2L1-1392 | chr17:48061259-48061286 |
| NFE2L1-682 | chr17:48056496-48056523 | NFE2L1-1393 | chr17:48061260-48061287 |
| NFE2L1-683 | chr17:48056497-48056524 | NFE2L1-1394 | chr17:48061261-48061288 |
| NFE2L1-684 | chr17:48056501-48056528 | NFE2L1-1395 | chr17:48061282-48061309 |
| NFE2L1-685 | chr17:48056506-48056533 | NFE2L1-1396 | chr17:48061283-48061310 |
| NFE2L1-686 | chr17:48056507-48056534 | NFE2L1-1397 | chr17:48061284-48061311 |
| NFE2L1-687 | chr17:48056512-48056539 | NFE2L1-1398 | chr17:48061285-48061312 |
| NFE2L1-688 | chr17:48056519-48056546 | NFE2L1-1399 | chr17:48061300-48061327 |
| NFE2L1-689 | chr17:48056521-48056548 | NFE2L1-1400 | chr17:48061301-48061328 |
| NFE2L1-690 | chr17:48056526-48056553 | NFE2L1-1401 | chr17:48061304-48061331 |
| NFE2L1-691 | chr17:48056539-48056566 | NFE2L1-1402 | chr17:48061305-48061332 |
| NFE2L1-692 | chr17:48056543-48056570 | NFE2L1-1403 | chr17:48061313-48061340 |
| NFE2L1-693 | chr17:48056552-48056579 | NFE2L1-1404 | chr17:48061315-48061342 |
| NFE2L1-694 | chr17:48056553-48056580 | NFE2L1-1405 | chr17:48061324-48061351 |
| NFE2L1-695 | chr17:48056554-48056581 | NFE2L1-1406 | chr17:48061325-48061352 |
| NFE2L1-696 | chr17:48056556-48056583 | NFE2L1-1407 | chr17:48061347-48061374 |
| NFE2L1-697 | chr17:48056572-48056599 | NFE2L1-1408 | chr17:48061374-48061401 |
| NFE2L1-698 | chr17:48056589-48056616 | NFE2L1-1409 | chr17:48061389-48061416 |
| NFE2L1-699 | chr17:48056590-48056617 | NFE2L1-1410 | chr17:48061390-48061417 |
| NFE2L1-700 | chr17:48056594-48056621 | NFE2L1-1411 | chr17:48061395-48061422 |
| NFE2L1-701 | chr17:48056595-48056622 | NFE2L1-1412 | chr17:48061396-48061423 |
| NFE2L1-702 | chr17:48056602-48056629 | NFE2L1-1413 | chr17:48061397-48061424 |
| NFE2L1-703 | chr17:48056608-48056635 | NFE2L1-1414 | chr17:48061408-48061435 |
| NFE2L1-704 | chr17:48056609-48056636 | NFE2L1-1415 | chr17:48061412-48061439 |
| NFE2L1-705 | chr17:48056610-48056637 | NFE2L1-1416 | chr17:48061445-48061472 |
| NFE2L1-706 | chr17:48056632-48056659 | NFE2L1-1417 | chr17:48061499-48061526 |
| NFE2L1-707 | chr17:48056635-48056662 | NFE2L1-1418 | chr17:48061500-48061527 |
| NFE2L1-708 | chr17:48056636-48056663 | NFE2L1-1419 | chr17:48061501-48061528 |
| NFE2L1-709 | chr17:48056643-48056670 | NFE2L1-1420 | chr17:48061502-48061529 |
| NFE2L1-710 | chr17:48056644-48056671 | NFE2L1-1421 | chr17:48061508-48061535 |
| NFE2L1-711 | chr17:48056655-48056682 | NFE2L1-1422 | chr17:48061520-48061547 |

**Table 2F Genomic coordinates of the human RARG gene**

| Target ID | Target Coordinate Range | Target ID | Target Coordinate Range |
|---|---|---|---|
| RARG-1 | chr12:53210576-53210603 | RARG-909 | chr12:53220697-53220724 |
| RARG-2 | chr12:53210600-53210627 | RARG-910 | chr12:53220704-53220731 |
| RARG-3 | chr12:53210601-53210628 | RARG-911 | chr12:53220709-53220736 |
| RARG-4 | chr12:53210602-53210629 | RARG-912 | chr12:53220710-53220737 |
| RARG-5 | chr12:53210608-53210635 | RARG-913 | chr12:53220712-53220739 |
| RARG-6 | chr12:53210609-53210636 | RARG-914 | chr12:53220742-53220769 |
| RARG-7 | chr12:53210610-53210637 | RARG-915 | chr12:53220758-53220785 |
| RARG-8 | chr12:53210625-53210652 | RARG-916 | chr12:53220772-53220799 |
| RARG-9 | chr12:53210626-53210653 | RARG-917 | chr12:53220773-53220800 |
| RARG-10 | chr12:53210627-53210654 | RARG-918 | chr12:53220778-53220805 |
| RARG-11 | chr12:53210630-53210657 | RARG-919 | chr12:53220779-53220806 |
| RARG-12 | chr12:53210631-53210658 | RARG-920 | chr12:53220780-53220807 |
| RARG-13 | chr12:53210632-53210659 | RARG-921 | chr12:53220787-53220814 |
| RARG-14 | chr12:53210663-53210690 | RARG-922 | chr12:53220788-53220815 |
| RARG-15 | chr12:53210664-53210691 | RARG-923 | chr12:53220789-53220816 |
| RARG-16 | chr12:53210666-53210693 | RARG-924 | chr12:53220790-53220817 |
| RARG-17 | chr12:53210667-53210694 | RARG-925 | chr12:53220797-53220824 |
| RARG-18 | chr12:53210672-53210699 | RARG-926 | chr12:53220805-53220832 |
| RARG-19 | chr12:53210673-53210700 | RARG-927 | chr12:53220806-53220833 |
| RARG-20 | chr12:53210674-53210701 | RARG-928 | chr12:53220807-53220834 |
| RARG-21 | chr12:53210675-53210702 | RARG-929 | chr12:53220812-53220839 |
| RARG-22 | chr12:53210676-53210703 | RARG-930 | chr12:53220813-53220840 |
| RARG-23 | chr12:53210686-53210713 | RARG-931 | chr12:53220814-53220841 |
| RARG-24 | chr12:53210694-53210721 | RARG-932 | chr12:53220821-53220848 |
| RARG-25 | chr12:53210704-53210731 | RARG-933 | chr12:53220824-53220851 |
| RARG-26 | chr12:53210705-53210732 | RARG-934 | chr12:53220851-53220878 |
| RARG-27 | chr12:53210706-53210733 | RARG-935 | chr12:53220853-53220880 |
| RARG-28 | chr12:53210711-53210738 | RARG-936 | chr12:53220854-53220881 |
| RARG-29 | chr12:53210714-53210741 | RARG-937 | chr12:53220855-53220882 |
| RARG-30 | chr12:53210722-53210749 | RARG-938 | chr12:53220856-53220883 |
| RARG-31 | chr12:53210725-53210752 | RARG-939 | chr12:53220857-53220884 |
| RARG-32 | chr12:53210726-53210753 | RARG-940 | chr12:53220872-53220899 |
| RARG-33 | chr12:53210727-53210754 | RARG-941 | chr12:53220886-53220913 |
| RARG-34 | chr12:53210734-53210761 | RARG-942 | chr12:53220890-53220917 |
| RARG-35 | chr12:53210735-53210762 | RARG-943 | chr12:53220895-53220922 |
| RARG-36 | chr12:53210736-53210763 | RARG-944 | chr12:53220898-53220925 |
| RARG-37 | chr12:53210742-53210769 | RARG-945 | chr12:53220901-53220928 |
| RARG-38 | chr12:53210743-53210770 | RARG-946 | chr12:53220902-53220929 |
| RARG-39 | chr12:53210764-53210791 | RARG-947 | chr12:53220903-53220930 |
| RARG-40 | chr12:53210765-53210792 | RARG-948 | chr12:53220912-53220939 |
| RARG-41 | chr12:53210768-53210795 | RARG-949 | chr12:53220913-53220940 |
| RARG-42 | chr12:53210772-53210799 | RARG-950 | chr12:53220918-53220945 |
| RARG-43 | chr12:53210773-53210800 | RARG-951 | chr12:53220925-53220952 |
| RARG-44 | chr12:53210774-53210801 | RARG-952 | chr12:53220928-53220955 |
| RARG-45 | chr12:53210775-53210802 | RARG-953 | chr12:53220929-53220956 |
| RARG-46 | chr12:53210776-53210803 | RARG-954 | chr12:53220934-53220961 |
| RARG-47 | chr12:53210779-53210806 | RARG-955 | chr12:53220935-53220962 |
| RARG-48 | chr12:53210780-53210807 | RARG-956 | chr12:53220938-53220965 |
| RARG-49 | chr12:53210786-53210813 | RARG-957 | chr12:53220945-53220972 |
| RARG-50 | chr12:53210787-53210814 | RARG-958 | chr12:53220946-53220973 |
| RARG-51 | chr12:53210788-53210815 | RARG-959 | chr12:53220950-53220977 |
| RARG-52 | chr12:53210808-53210835 | RARG-960 | chr12:53220955-53220982 |
| RARG-53 | chr12:53210816-53210843 | RARG-961 | chr12:53220957-53220984 |
| RARG-54 | chr12:53210826-53210853 | RARG-962 | chr12:53220971-53220998 |
| RARG-55 | chr12:53210827-53210854 | RARG-963 | chr12:53220992-53221019 |
| RARG-56 | chr12:53210832-53210859 | RARG-964 | chr12:53220995-53221022 |
| RARG-57 | chr12:53210833-53210860 | RARG-965 | chr12:53220997-53221024 |
| RARG-58 | chr12:53210836-53210863 | RARG-966 | chr12:53220998-53221025 |
| RARG-59 | chr12:53210837-53210864 | RARG-967 | chr12:53221002-53221029 |
| RARG-60 | chr12:53210847-53210874 | RARG-968 | chr12:53221004-53221031 |
| RARG-61 | chr12:53210866-53210893 | RARG-969 | chr12:53221005-53221032 |
| RARG-62 | chr12:53210867-53210894 | RARG-970 | chr12:53221006-53221033 |
| RARG-63 | chr12:53210881-53210908 | RARG-971 | chr12:53221007-53221034 |
| RARG-64 | chr12:53210882-53210909 | RARG-972 | chr12:53221009-53221036 |
| RARG-65 | chr12:53210883-53210910 | RARG-973 | chr12:53221013-53221040 |
| RARG-66 | chr12:53210884-53210911 | RARG-974 | chr12:53221014-53221041 |
| RARG-67 | chr12:53210885-53210912 | RARG-975 | chr12:53221015-53221042 |
| RARG-68 | chr12:53210888-53210915 | RARG-976 | chr12:53221018-53221045 |
| RARG-69 | chr12:53210889-53210916 | RARG-977 | chr12:53221019-53221046 |
| RARG-70 | chr12:53210891-53210918 | RARG-978 | chr12:53221026-53221053 |
| RARG-71 | chr12:53210892-53210919 | RARG-979 | chr12:53221033-53221060 |
| RARG-72 | chr12:53210895-53210922 | RARG-980 | chr12:53221036-53221063 |
| RARG-73 | chr12:53210899-53210926 | RARG-981 | chr12:53221037-53221064 |
| RARG-74 | chr12:53210906-53210933 | RARG-982 | chr12:53221045-53221072 |
| RARG-75 | chr12:53210907-53210934 | RARG-983 | chr12:53221046-53221073 |
| RARG-76 | chr12:53210914-53210941 | RARG-984 | chr12:53221049-53221076 |
| RARG-77 | chr12:53210915-53210942 | RARG-985 | chr12:53221050-53221077 |
| RARG-78 | chr12:53210924-53210951 | RARG-986 | chr12:53221051-53221078 |
| RARG-79 | chr12:53210941-53210968 | RARG-987 | chr12:53221054-53221081 |
| RARG-80 | chr12:53210944-53210971 | RARG-988 | chr12:53221055-53221082 |
| RARG-81 | chr12:53210945-53210972 | RARG-989 | chr12:53221058-53221085 |
| RARG-82 | chr12:53210946-53210973 | RARG-990 | chr12:53221067-53221094 |
| RARG-83 | chr12:53210947-53210974 | RARG-991 | chr12:53221068-53221095 |
| RARG-84 | chr12:53210952-53210979 | RARG-992 | chr12:53221069-53221096 |
| RARG-85 | chr12:53210953-53210980 | RARG-993 | chr12:53221070-53221097 |
| RARG-86 | chr12:53210954-53210981 | RARG-994 | chr12:53221071-53221098 |
| RARG-87 | chr12:53210960-53210987 | RARG-995 | chr12:53221086-53221113 |
| RARG-88 | chr12:53210969-53210996 | RARG-996 | chr12:53221087-53221114 |
| RARG-89 | chr12:53210972-53210999 | RARG-997 | chr12:53221099-53221126 |
| RARG-90 | chr12:53210973-53211000 | RARG-998 | chr12:53221102-53221129 |
| RARG-91 | chr12:53210974-53211001 | RARG-999 | chr12:53221105-53221132 |
| RARG-92 | chr12:53210975-53211002 | RARG-1000 | chr12:53221108-53221135 |
| RARG-93 | chr12:53210977-53211004 | RARG-1001 | chr12:53221110-53221137 |
| RARG-94 | chr12:53210979-53211006 | RARG-1002 | chr12:53221111-53221138 |
| RARG-95 | chr12:53210980-53211007 | RARG-1003 | chr12:53221115-53221142 |
| RARG-96 | chr12:53210981-53211008 | RARG-1004 | chr12:53221116-53221143 |
| RARG-97 | chr12:53210989-53211016 | RARG-1005 | chr12:53221117-53221144 |
| RARG-98 | chr12:53211000-53211027 | RARG-1006 | chr12:53221120-53221147 |
| RARG-99 | chr12:53211001-53211028 | RARG-1007 | chr12:53221121-53221148 |
| RARG-100 | chr12:53211005-53211032 | RARG-1008 | chr12:53221122-53221149 |
| RARG-101 | chr12:53211006-53211033 | RARG-1009 | chr12:53221125-53221152 |
| RARG-102 | chr12:53211007-53211034 | RARG-1010 | chr12:53221126-53221153 |
| RARG-103 | chr12:53211008-53211035 | RARG-1011 | chr12:53221127-53221154 |
| RARG-104 | chr12:53211010-53211037 | RARG-1012 | chr12:53221129-53221156 |
| RARG-105 | chr12:53211011-53211038 | RARG-1013 | chr12:53221130-53221157 |
| RARG-106 | chr12:53211012-53211039 | RARG-1014 | chr12:53221131-53221158 |
| RARG-107 | chr12:53211023-53211050 | RARG-1015 | chr12:53221136-53221163 |
| RARG-108 | chr12:53211026-53211053 | RARG-1016 | chr12:53221151-53221178 |
| RARG-109 | chr12:53211027-53211054 | RARG-1017 | chr12:53221160-53221187 |
| RARG-110 | chr12:53211033-53211060 | RARG-1018 | chr12:53221161-53221188 |
| RARG-111 | chr12:53211034-53211061 | RARG-1019 | chr12:53221162-53221189 |
| RARG-112 | chr12:53211059-53211086 | RARG-1020 | chr12:53221163-53221190 |
| RARG-113 | chr12:53211069-53211096 | RARG-1021 | chr12:53221164-53221191 |
| RARG-114 | chr12:53211073-53211100 | RARG-1022 | chr12:53221167-53221194 |
| RARG-115 | chr12:53211074-53211101 | RARG-1023 | chr12:53221168-53221195 |
| RARG-116 | chr12:53211079-53211106 | RARG-1024 | chr12:53221169-53221196 |
| RARG-117 | chr12:53211098-53211125 | RARG-1025 | chr12:53221170-53221197 |
| RARG-118 | chr12:53211113-53211140 | RARG-1026 | chr12:53221173-53221200 |
| RARG-119 | chr12:53211114-53211141 | RARG-1027 | chr12:53221182-53221209 |
| RARG-120 | chr12:53211121-53211148 | RARG-1028 | chr12:53221192-53221219 |
| RARG-121 | chr12:53211122-53211149 | RARG-1029 | chr12:53221193-53221220 |
| RARG-122 | chr12:53211130-53211157 | RARG-1030 | chr12:53221194-53221221 |
| RARG-123 | chr12:53211134-53211161 | RARG-1031 | chr12:53221197-53221224 |
| RARG-124 | chr12:53211135-53211162 | RARG-1032 | chr12:53221200-53221227 |
| RARG-125 | chr12:53211136-53211163 | RARG-1033 | chr12:53221202-53221229 |
| RARG-126 | chr12:53211148-53211175 | RARG-1034 | chr12:53221203-53221230 |
| RARG-127 | chr12:53211150-53211177 | RARG-1035 | chr12:53221205-53221232 |
| RARG-128 | chr12:53211151-53211178 | RARG-1036 | chr12:53221206-53221233 |
| RARG-129 | chr12:53211155-53211182 | RARG-1037 | chr12:53221212-53221239 |
| RARG-130 | chr12:53211159-53211186 | RARG-1038 | chr12:53221215-53221242 |
| RARG-131 | chr12:53211160-53211187 | RARG-1039 | chr12:53221216-53221243 |
| RARG-132 | chr12:53211161-53211188 | RARG-1040 | chr12:53221219-53221246 |
| RARG-133 | chr12:53211162-53211189 | RARG-1041 | chr12:53221222-53221249 |
| RARG-134 | chr12:53211165-53211192 | RARG-1042 | chr12:53221228-53221255 |
| RARG-135 | chr12:53211169-53211196 | RARG-1043 | chr12:53221229-53221256 |
| RARG-136 | chr12:53211171-53211198 | RARG-1044 | chr12:53221234-53221261 |
| RARG-137 | chr12:53211188-53211215 | RARG-1045 | chr12:53221237-53221264 |
| RARG-138 | chr12:53211207-53211234 | RARG-1046 | chr12:53221240-53221267 |
| RARG-139 | chr12:53211208-53211235 | RARG-1047 | chr12:53221241-53221268 |
| RARG-140 | chr12:53211215-53211242 | RARG-1048 | chr12:53221244-53221271 |
| RARG-141 | chr12:53211216-53211243 | RARG-1049 | chr12:53221250-53221277 |
| RARG-142 | chr12:53211220-53211247 | RARG-1050 | chr12:53221251-53221278 |
| RARG-143 | chr12:53211230-53211257 | RARG-1051 | chr12:53221252-53221279 |
| RARG-144 | chr12:53211233-53211260 | RARG-1052 | chr12:53221258-53221285 |
| RARG-145 | chr12:53211234-53211261 | RARG-1053 | chr12:53221264-53221291 |
| RARG-146 | chr12:53211237-53211264 | RARG-1054 | chr12:53221265-53221292 |
| RARG-147 | chr12:53211264-53211291 | RARG-1055 | chr12:53221277-53221304 |
| RARG-148 | chr12:53211267-53211294 | RARG-1056 | chr12:53221278-53221305 |
| RARG-149 | chr12:53211268-53211295 | RARG-1057 | chr12:53221279-53221306 |
| RARG-150 | chr12:53211274-53211301 | RARG-1058 | chr12:53221280-53221307 |
| RARG-151 | chr12:53211281-53211308 | RARG-1059 | chr12:53221288-53221315 |
| RARG-152 | chr12:53211282-53211309 | RARG-1060 | chr12:53221294-53221321 |
| RARG-153 | chr12:53211283-53211310 | RARG-1061 | chr12:53221311-53221338 |
| RARG-154 | chr12:53211303-53211330 | RARG-1062 | chr12:53221351-53221378 |
| RARG-155 | chr12:53211306-53211333 | RARG-1063 | chr12:53221352-53221379 |
| RARG-156 | chr12:53211312-53211339 | RARG-1064 | chr12:53221353-53221380 |
| RARG-157 | chr12:53211316-53211343 | RARG-1065 | chr12:53221365-53221392 |
| RARG-158 | chr12:53211317-53211344 | RARG-1066 | chr12:53221369-53221396 |
| RARG-159 | chr12:53211318-53211345 | RARG-1067 | chr12:53221374-53221401 |
| RARG-160 | chr12:53211322-53211349 | RARG-1068 | chr12:53221379-53221406 |
| RARG-161 | chr12:53211324-53211351 | RARG-1069 | chr12:53221383-53221410 |
| RARG-162 | chr12:53211329-53211356 | RARG-1070 | chr12:53221397-53221424 |
| RARG-163 | chr12:53211333-53211360 | RARG-1071 | chr12:53221398-53221425 |
| RARG-164 | chr12:53211334-53211361 | RARG-1072 | chr12:53221399-53221426 |
| RARG-165 | chr12:53211351-53211378 | RARG-1073 | chr12:53221407-53221434 |
| RARG-166 | chr12:53211352-53211379 | RARG-1074 | chr12:53221413-53221440 |
| RARG-167 | chr12:53211361-53211388 | RARG-1075 | chr12:53221414-53221441 |
| RARG-168 | chr12:53211366-53211393 | RARG-1076 | chr12:53221415-53221442 |
| RARG-169 | chr12:53211369-53211396 | RARG-1077 | chr12:53221418-53221445 |
| RARG-170 | chr12:53211370-53211397 | RARG-1078 | chr12:53221421-53221448 |
| RARG-171 | chr12:53211371-53211398 | RARG-1079 | chr12:53221423-53221450 |
| RARG-172 | chr12:53211373-53211400 | RARG-1080 | chr12:53221430-53221457 |
| RARG-173 | chr12:53211374-53211401 | RARG-1081 | chr12:53221431-53221458 |
| RARG-174 | chr12:53211390-53211417 | RARG-1082 | chr12:53221432-53221459 |
| RARG-175 | chr12:53211391-53211418 | RARG-1083 | chr12:53221435-53221462 |
| RARG-176 | chr12:53211402-53211429 | RARG-1084 | chr12:53221441-53221468 |
| RARG-177 | chr12:53211419-53211446 | RARG-1085 | chr12:53221444-53221471 |
| RARG-178 | chr12:53211424-53211451 | RARG-1086 | chr12:53221445-53221472 |
| RARG-179 | chr12:53211425-53211452 | RARG-1087 | chr12:53221459-53221486 |
| RARG-180 | chr12:53211427-53211454 | RARG-1088 | chr12:53221460-53221487 |
| RARG-181 | chr12:53211430-53211457 | RARG-1089 | chr12:53221484-53221511 |
| RARG-182 | chr12:53211431-53211458 | RARG-1090 | chr12:53221498-53221525 |
| RARG-183 | chr12:53211435-53211462 | RARG-1091 | chr12:53221499-53221526 |
| RARG-184 | chr12:53211436-53211463 | RARG-1092 | chr12:53221504-53221531 |
| RARG-185 | chr12:53211437-53211464 | RARG-1093 | chr12:53221506-53221533 |
| RARG-186 | chr12:53211438-53211465 | RARG-1094 | chr12:53221520-53221547 |
| RARG-187 | chr12:53211442-53211469 | RARG-1095 | chr12:53221521-53221548 |
| RARG-188 | chr12:53211443-53211470 | RARG-1096 | chr12:53221524-53221551 |
| RARG-189 | chr12:53211449-53211476 | RARG-1097 | chr12:53221525-53221552 |
| RARG-190 | chr12:53211450-53211477 | RARG-1098 | chr12:53221528-53221555 |
| RARG-191 | chr12:53211455-53211482 | RARG-1099 | chr12:53221538-53221565 |
| RARG-192 | chr12:53211460-53211487 | RARG-1100 | chr12:53221539-53221566 |
| RARG-193 | chr12:53211461-53211488 | RARG-1101 | chr12:53221540-53221567 |
| RARG-194 | chr12:53211467-53211494 | RARG-1102 | chr12:53221547-53221574 |
| RARG-195 | chr12:53211470-53211497 | RARG-1103 | chr12:53221548-53221575 |
| RARG-196 | chr12:53211471-53211498 | RARG-1104 | chr12:53221550-53221577 |
| RARG-197 | chr12:53211472-53211499 | RARG-1105 | chr12:53221556-53221583 |
| RARG-198 | chr12:53211473-53211500 | RARG-1106 | chr12:53221561-53221588 |
| RARG-199 | chr12:53211477-53211504 | RARG-1107 | chr12:53221569-53221596 |
| RARG-200 | chr12:53211481-53211508 | RARG-1108 | chr12:53221570-53221597 |
| RARG-201 | chr12:53211482-53211509 | RARG-1109 | chr12:53221579-53221606 |
| RARG-202 | chr12:53211483-53211510 | RARG-1110 | chr12:53221580-53221607 |
| RARG-203 | chr12:53211491-53211518 | RARG-1111 | chr12:53221581-53221608 |
| RARG-204 | chr12:53211496-53211523 | RARG-1112 | chr12:53221584-53221611 |
| RARG-205 | chr12:53211514-53211541 | RARG-1113 | chr12:53221585-53221612 |
| RARG-206 | chr12:53211525-53211552 | RARG-1114 | chr12:53221590-53221617 |
| RARG-207 | chr12:53211526-53211553 | RARG-1115 | chr12:53221591-53221618 |
| RARG-208 | chr12:53211527-53211554 | RARG-1116 | chr12:53221592-53221619 |
| RARG-209 | chr12:53211528-53211555 | RARG-1117 | chr12:53221593-53221620 |
| RARG-210 | chr12:53211535-53211562 | RARG-1118 | chr12:53221598-53221625 |
| RARG-211 | chr12:53211539-53211566 | RARG-1119 | chr12:53221599-53221626 |
| RARG-212 | chr12:53211540-53211567 | RARG-1120 | chr12:53221601-53221628 |
| RARG-213 | chr12:53211541-53211568 | RARG-1121 | chr12:53221602-53221629 |
| RARG-214 | chr12:53211544-53211571 | RARG-1122 | chr12:53221604-53221631 |
| RARG-215 | chr12:53211545-53211572 | RARG-1123 | chr12:53221605-53221632 |
| RARG-216 | chr12:53211546-53211573 | RARG-1124 | chr12:53221606-53221633 |
| RARG-217 | chr12:53211551-53211578 | RARG-1125 | chr12:53221607-53221634 |
| RARG-218 | chr12:53211552-53211579 | RARG-1126 | chr12:53221608-53221635 |
| RARG-219 | chr12:53211561-53211588 | RARG-1127 | chr12:53221609-53221636 |
| RARG-220 | chr12:53211579-53211606 | RARG-1128 | chr12:53221613-53221640 |
| RARG-221 | chr12:53211582-53211609 | RARG-1129 | chr12:53221635-53221662 |
| RARG-222 | chr12:53211587-53211614 | RARG-1130 | chr12:53221640-53221667 |
| RARG-223 | chr12:53211588-53211615 | RARG-1131 | chr12:53221641-53221668 |
| RARG-224 | chr12:53211590-53211617 | RARG-1132 | chr12:53221644-53221671 |
| RARG-225 | chr12:53211593-53211620 | RARG-1133 | chr12:53221645-53221672 |
| RARG-226 | chr12:53211594-53211621 | RARG-1134 | chr12:53221653-53221680 |
| RARG-227 | chr12:53211595-53211622 | RARG-1135 | chr12:53221654-53221681 |
| RARG-228 | chr12:53211596-53211623 | RARG-1136 | chr12:53221655-53221682 |
| RARG-229 | chr12:53211630-53211657 | RARG-1137 | chr12:53221656-53221683 |
| RARG-230 | chr12:53211631-53211658 | RARG-1138 | chr12:53221657-53221684 |
| RARG-231 | chr12:53211632-53211659 | RARG-1139 | chr12:53221658-53221685 |
| RARG-232 | chr12:53211633-53211660 | RARG-1140 | chr12:53221663-53221690 |
| RARG-233 | chr12:53211635-53211662 | RARG-1141 | chr12:53221664-53221691 |
| RARG-234 | chr12:53211640-53211667 | RARG-1142 | chr12:53221670-53221697 |
| RARG-235 | chr12:53211641-53211668 | RARG-1143 | chr12:53221673-53221700 |
| RARG-236 | chr12:53211642-53211669 | RARG-1144 | chr12:53221686-53221713 |
| RARG-237 | chr12:53211646-53211673 | RARG-1145 | chr12:53221687-53221714 |
| RARG-238 | chr12:53211647-53211674 | RARG-1146 | chr12:53221692-53221719 |
| RARG-239 | chr12:53211648-53211675 | RARG-1147 | chr12:53221693-53221720 |
| RARG-240 | chr12:53211649-53211676 | RARG-1148 | chr12:53221694-53221721 |
| RARG-241 | chr12:53211653-53211680 | RARG-1149 | chr12:53221696-53221723 |
| RARG-242 | chr12:53211657-53211684 | RARG-1150 | chr12:53221704-53221731 |
| RARG-243 | chr12:53211658-53211685 | RARG-1151 | chr12:53221719-53221746 |
| RARG-244 | chr12:53211659-53211686 | RARG-1152 | chr12:53221720-53221747 |
| RARG-245 | chr12:53211667-53211694 | RARG-1153 | chr12:53221728-53221755 |
| RARG-246 | chr12:53211670-53211697 | RARG-1154 | chr12:53221729-53221756 |
| RARG-247 | chr12:53211671-53211698 | RARG-1155 | chr12:53221730-53221757 |
| RARG-248 | chr12:53211682-53211709 | RARG-1156 | chr12:53221731-53221758 |
| RARG-249 | chr12:53211690-53211717 | RARG-1157 | chr12:53221732-53221759 |
| RARG-250 | chr12:53211695-53211722 | RARG-1158 | chr12:53221733-53221760 |
| RARG-251 | chr12:53211696-53211723 | RARG-1159 | chr12:53221735-53221762 |
| RARG-252 | chr12:53211697-53211724 | RARG-1160 | chr12:53221736-53221763 |
| RARG-253 | chr12:53211698-53211725 | RARG-1161 | chr12:53221737-53221764 |
| RARG-254 | chr12:53211704-53211731 | RARG-1162 | chr12:53221738-53221765 |
| RARG-255 | chr12:53211705-53211732 | RARG-1163 | chr12:53221739-53221766 |
| RARG-256 | chr12:53211710-53211737 | RARG-1164 | chr12:53221744-53221771 |
| RARG-257 | chr12:53211711-53211738 | RARG-1165 | chr12:53221745-53221772 |
| RARG-258 | chr12:53211712-53211739 | RARG-1166 | chr12:53221746-53221773 |
| RARG-259 | chr12:53211713-53211740 | RARG-1167 | chr12:53221749-53221776 |
| RARG-260 | chr12:53211716-53211743 | RARG-1168 | chr12:53221750-53221777 |
| RARG-261 | chr12:53211717-53211744 | RARG-1169 | chr12:53221756-53221783 |
| RARG-262 | chr12:53211718-53211745 | RARG-1170 | chr12:53221759-53221786 |
| RARG-263 | chr12:53211720-53211747 | RARG-1171 | chr12:53221761-53221788 |
| RARG-264 | chr12:53211721-53211748 | RARG-1172 | chr12:53221764-53221791 |
| RARG-265 | chr12:53211722-53211749 | RARG-1173 | chr12:53221767-53221794 |
| RARG-266 | chr12:53211723-53211750 | RARG-1174 | chr12:53221768-53221795 |
| RARG-267 | chr12:53211726-53211753 | RARG-1175 | chr12:53221772-53221799 |
| RARG-268 | chr12:53211729-53211756 | RARG-1176 | chr12:53221776-53221803 |
| RARG-269 | chr12:53211737-53211764 | RARG-1177 | chr12:53221780-53221807 |
| RARG-270 | chr12:53211752-53211779 | RARG-1178 | chr12:53221781-53221808 |
| RARG-271 | chr12:53211759-53211786 | RARG-1179 | chr12:53221784-53221811 |
| RARG-272 | chr12:53211760-53211787 | RARG-1180 | chr12:53221785-53221812 |
| RARG-273 | chr12:53211771-53211798 | RARG-1181 | chr12:53221796-53221823 |
| RARG-274 | chr12:53211778-53211805 | RARG-1182 | chr12:53221800-53221827 |
| RARG-275 | chr12:53211785-53211812 | RARG-1183 | chr12:53221801-53221828 |
| RARG-276 | chr12:53211786-53211813 | RARG-1184 | chr12:53221802-53221829 |
| RARG-277 | chr12:53211789-53211816 | RARG-1185 | chr12:53221807-53221834 |
| RARG-278 | chr12:53211792-53211819 | RARG-1186 | chr12:53221814-53221841 |
| RARG-279 | chr12:53211795-53211822 | RARG-1187 | chr12:53221818-53221845 |
| RARG-280 | chr12:53211796-53211823 | RARG-1188 | chr12:53221819-53221846 |
| RARG-281 | chr12:53211801-53211828 | RARG-1189 | chr12:53221835-53221862 |
| RARG-282 | chr12:53211824-53211851 | RARG-1190 | chr12:53221837-53221864 |
| RARG-283 | chr12:53211834-53211861 | RARG-1191 | chr12:53221838-53221865 |
| RARG-284 | chr12:53211845-53211872 | RARG-1192 | chr12:53221842-53221869 |
| RARG-285 | chr12:53211852-53211879 | RARG-1193 | chr12:53221843-53221870 |
| RARG-286 | chr12:53211853-53211880 | RARG-1194 | chr12:53221844-53221871 |
| RARG-287 | chr12:53211862-53211889 | RARG-1195 | chr12:53221847-53221874 |
| RARG-288 | chr12:53211863-53211890 | RARG-1196 | chr12:53221848-53221875 |
| RARG-289 | chr12:53211868-53211895 | RARG-1197 | chr12:53221849-53221876 |
| RARG-290 | chr12:53211871-53211898 | RARG-1198 | chr12:53221852-53221879 |
| RARG-291 | chr12:53211878-53211905 | RARG-1199 | chr12:53221853-53221880 |
| RARG-292 | chr12:53211885-53211912 | RARG-1200 | chr12:53221854-53221881 |
| RARG-293 | chr12:53211894-53211921 | RARG-1201 | chr12:53221855-53221882 |
| RARG-294 | chr12:53211913-53211940 | RARG-1202 | chr12:53221858-53221885 |
| RARG-295 | chr12:53211914-53211941 | RARG-1203 | chr12:53221859-53221886 |
| RARG-296 | chr12:53211922-53211949 | RARG-1204 | chr12:53221860-53221887 |
| RARG-297 | chr12:53211928-53211955 | RARG-1205 | chr12:53227237-53227264 |
| RARG-298 | chr12:53211929-53211956 | RARG-1206 | chr12:53227245-53227272 |
| RARG-299 | chr12:53211934-53211961 | RARG-1207 | chr12:53227250-53227277 |
| RARG-300 | chr12:53211936-53211963 | RARG-1208 | chr12:53227251-53227278 |
| RARG-301 | chr12:53211937-53211964 | RARG-1209 | chr12:53227252-53227279 |
| RARG-302 | chr12:53211938-53211965 | RARG-1210 | chr12:53227253-53227280 |
| RARG-303 | chr12:53211939-53211966 | RARG-1211 | chr12:53227254-53227281 |
| RARG-304 | chr12:53211947-53211974 | RARG-1212 | chr12:53227257-53227284 |
| RARG-305 | chr12:53211948-53211975 | RARG-1213 | chr12:53227258-53227285 |
| RARG-306 | chr12:53211949-53211976 | RARG-1214 | chr12:53227259-53227286 |
| RARG-307 | chr12:53211952-53211979 | RARG-1215 | chr12:53227263-53227290 |
| RARG-308 | chr12:53211956-53211983 | RARG-1216 | chr12:53227267-53227294 |
| RARG-309 | chr12:53211957-53211984 | RARG-1217 | chr12:53227271-53227298 |
| RARG-310 | chr12:53211958-53211985 | RARG-1218 | chr12:53227284-53227311 |
| RARG-311 | chr12:53212967-53212994 | RARG-1219 | chr12:53227296-53227323 |
| RARG-312 | chr12:53212980-53213007 | RARG-1220 | chr12:53227300-53227327 |
| RARG-313 | chr12:53213006-53213033 | RARG-1221 | chr12:53227303-53227330 |
| RARG-314 | chr12:53213007-53213034 | RARG-1222 | chr12:53227304-53227331 |
| RARG-315 | chr12:53213017-53213044 | RARG-1223 | chr12:53227307-53227334 |
| RARG-316 | chr12:53213020-53213047 | RARG-1224 | chr12:53227314-53227341 |
| RARG-317 | chr12:53213023-53213050 | RARG-1225 | chr12:53227332-53227359 |
| RARG-318 | chr12:53213024-53213051 | RARG-1226 | chr12:53227340-53227367 |
| RARG-319 | chr12:53213025-53213052 | RARG-1227 | chr12:53227341-53227368 |
| RARG-320 | chr12:53213029-53213056 | RARG-1228 | chr12:53227342-53227369 |
| RARG-321 | chr12:53213036-53213063 | RARG-1229 | chr12:53227343-53227370 |
| RARG-322 | chr12:53213037-53213064 | RARG-1230 | chr12:53227349-53227376 |
| RARG-323 | chr12:53213038-53213065 | RARG-1231 | chr12:53227350-53227377 |
| RARG-324 | chr12:53213045-53213072 | RARG-1232 | chr12:53227355-53227382 |
| RARG-325 | chr12:53213046-53213073 | RARG-1233 | chr12:53227356-53227383 |
| RARG-326 | chr12:53213069-53213096 | RARG-1234 | chr12:53227357-53227384 |
| RARG-327 | chr12:53213070-53213097 | RARG-1235 | chr12:53227360-53227387 |
| RARG-328 | chr12:53213077-53213104 | RARG-1236 | chr12:53227365-53227392 |
| RARG-329 | chr12:53213080-53213107 | RARG-1237 | chr12:53227369-53227396 |
| RARG-330 | chr12:53213084-53213111 | RARG-1238 | chr12:53227371-53227398 |
| RARG-331 | chr12:53213085-53213112 | RARG-1239 | chr12:53227375-53227402 |
| RARG-332 | chr12:53213099-53213126 | RARG-1240 | chr12:53227377-53227404 |
| RARG-333 | chr12:53213100-53213127 | RARG-1241 | chr12:53227381-53227408 |
| RARG-334 | chr12:53213101-53213128 | RARG-1242 | chr12:53227389-53227416 |
| RARG-335 | chr12:53213106-53213133 | RARG-1243 | chr12:53227390-53227417 |
| RARG-336 | chr12:53213107-53213134 | RARG-1244 | chr12:53227397-53227424 |
| RARG-337 | chr12:53213117-53213144 | RARG-1245 | chr12:53227398-53227425 |
| RARG-338 | chr12:53213118-53213145 | RARG-1246 | chr12:53227403-53227430 |
| RARG-339 | chr12:53213122-53213149 | RARG-1247 | chr12:53227404-53227431 |
| RARG-340 | chr12:53213126-53213153 | RARG-1248 | chr12:53227405-53227432 |
| RARG-341 | chr12:53213127-53213154 | RARG-1249 | chr12:53227407-53227434 |
| RARG-342 | chr12:53213128-53213155 | RARG-1250 | chr12:53227410-53227437 |
| RARG-343 | chr12:53213134-53213161 | RARG-1251 | chr12:53227431-53227458 |
| RARG-344 | chr12:53213140-53213167 | RARG-1252 | chr12:53227432-53227459 |
| RARG-345 | chr12:53213141-53213168 | RARG-1253 | chr12:53227436-53227463 |
| RARG-346 | chr12:53213155-53213182 | RARG-1254 | chr12:53227441-53227468 |
| RARG-347 | chr12:53213156-53213183 | RARG-1255 | chr12:53227442-53227469 |
| RARG-348 | chr12:53213158-53213185 | RARG-1256 | chr12:53227443-53227470 |
| RARG-349 | chr12:53213164-53213191 | RARG-1257 | chr12:53227444-53227471 |
| RARG-350 | chr12:53213169-53213196 | RARG-1258 | chr12:53227452-53227479 |
| RARG-351 | chr12:53213176-53213203 | RARG-1259 | chr12:53227453-53227480 |
| RARG-352 | chr12:53213187-53213214 | RARG-1260 | chr12:53227454-53227481 |
| RARG-353 | chr12:53213195-53213222 | RARG-1261 | chr12:53227458-53227485 |
| RARG-354 | chr12:53213196-53213223 | RARG-1262 | chr12:53227459-53227486 |
| RARG-355 | chr12:53213199-53213226 | RARG-1263 | chr12:53227473-53227500 |
| RARG-356 | chr12:53213206-53213233 | RARG-1264 | chr12:53227475-53227502 |
| RARG-357 | chr12:53213211-53213238 | RARG-1265 | chr12:53227479-53227506 |
| RARG-358 | chr12:53213215-53213242 | RARG-1266 | chr12:53227480-53227507 |
| RARG-359 | chr12:53213222-53213249 | RARG-1267 | chr12:53227481-53227508 |
| RARG-360 | chr12:53213223-53213250 | RARG-1268 | chr12:53227490-53227517 |
| RARG-361 | chr12:53213224-53213251 | RARG-1269 | chr12:53227496-53227523 |
| RARG-362 | chr12:53213226-53213253 | RARG-1270 | chr12:53227501-53227528 |
| RARG-363 | chr12:53213229-53213256 | RARG-1271 | chr12:53227502-53227529 |
| RARG-364 | chr12:53213235-53213262 | RARG-1272 | chr12:53227503-53227530 |
| RARG-365 | chr12:53213237-53213264 | RARG-1273 | chr12:53227511-53227538 |
| RARG-366 | chr12:53213246-53213273 | RARG-1274 | chr12:53227514-53227541 |
| RARG-367 | chr12:53213247-53213274 | RARG-1275 | chr12:53227515-53227542 |
| RARG-368 | chr12:53213248-53213275 | RARG-1276 | chr12:53227520-53227547 |
| RARG-369 | chr12:53213252-53213279 | RARG-1277 | chr12:53227529-53227556 |
| RARG-370 | chr12:53213261-53213288 | RARG-1278 | chr12:53227530-53227557 |
| RARG-371 | chr12:53213262-53213289 | RARG-1279 | chr12:53227542-53227569 |
| RARG-372 | chr12:53213263-53213290 | RARG-1280 | chr12:53227545-53227572 |
| RARG-373 | chr12:53213282-53213309 | RARG-1281 | chr12:53227546-53227573 |
| RARG-374 | chr12:53213297-53213324 | RARG-1282 | chr12:53227547-53227574 |
| RARG-375 | chr12:53213305-53213332 | RARG-1283 | chr12:53227557-53227584 |
| RARG-376 | chr12:53213306-53213333 | RARG-1284 | chr12:53227558-53227585 |
| RARG-377 | chr12:53213317-53213344 | RARG-1285 | chr12:53227561-53227588 |
| RARG-378 | chr12:53213319-53213346 | RARG-1286 | chr12:53227562-53227589 |
| RARG-379 | chr12:53213320-53213347 | RARG-1287 | chr12:53227566-53227593 |
| RARG-380 | chr12:53213321-53213348 | RARG-1288 | chr12:53227567-53227594 |
| RARG-381 | chr12:53213336-53213363 | RARG-1289 | chr12:53227568-53227595 |
| RARG-382 | chr12:53213337-53213364 | RARG-1290 | chr12:53227602-53227629 |
| RARG-383 | chr12:53213340-53213367 | RARG-1291 | chr12:53227608-53227635 |
| RARG-384 | chr12:53213341-53213368 | RARG-1292 | chr12:53227609-53227636 |
| RARG-385 | chr12:53213342-53213369 | RARG-1293 | chr12:53227610-53227637 |
| RARG-386 | chr12:53213370-53213397 | RARG-1294 | chr12:53227611-53227638 |
| RARG-387 | chr12:53213378-53213405 | RARG-1295 | chr12:53227614-53227641 |
| RARG-388 | chr12:53213379-53213406 | RARG-1296 | chr12:53227615-53227642 |
| RARG-389 | chr12:53213386-53213413 | RARG-1297 | chr12:53227620-53227647 |
| RARG-390 | chr12:53213399-53213426 | RARG-1298 | chr12:53227621-53227648 |
| RARG-391 | chr12:53213408-53213435 | RARG-1299 | chr12:53227627-53227654 |
| RARG-392 | chr12:53213411-53213438 | RARG-1300 | chr12:53227628-53227655 |
| RARG-393 | chr12:53213414-53213441 | RARG-1301 | chr12:53227629-53227656 |
| RARG-394 | chr12:53213416-53213443 | RARG-1302 | chr12:53227633-53227660 |
| RARG-395 | chr12:53213417-53213444 | RARG-1303 | chr12:53227635-53227662 |
| RARG-396 | chr12:53213418-53213445 | RARG-1304 | chr12:53227644-53227671 |
| RARG-397 | chr12:53213419-53213446 | RARG-1305 | chr12:53227647-53227674 |
| RARG-398 | chr12:53213422-53213449 | RARG-1306 | chr12:53227648-53227675 |
| RARG-399 | chr12:53213423-53213450 | RARG-1307 | chr12:53227649-53227676 |
| RARG-400 | chr12:53213428-53213455 | RARG-1308 | chr12:53227650-53227677 |
| RARG-401 | chr12:53213434-53213461 | RARG-1309 | chr12:53227653-53227680 |
| RARG-402 | chr12:53213454-53213481 | RARG-1310 | chr12:53227654-53227681 |
| RARG-403 | chr12:53213455-53213482 | RARG-1311 | chr12:53227658-53227685 |
| RARG-404 | chr12:53213456-53213483 | RARG-1312 | chr12:53227663-53227690 |
| RARG-405 | chr12:53213457-53213484 | RARG-1313 | chr12:53227665-53227692 |
| RARG-406 | chr12:53213477-53213504 | RARG-1314 | chr12:53227666-53227693 |
| RARG-407 | chr12:53213482-53213509 | RARG-1315 | chr12:53227667-53227694 |
| RARG-408 | chr12:53213487-53213514 | RARG-1316 | chr12:53227668-53227695 |
| RARG-409 | chr12:53213488-53213515 | RARG-1317 | chr12:53227676-53227703 |
| RARG-410 | chr12:53213489-53213516 | RARG-1318 | chr12:53227679-53227706 |
| RARG-411 | chr12:53213490-53213517 | RARG-1319 | chr12:53227683-53227710 |
| RARG-412 | chr12:53213495-53213522 | RARG-1320 | chr12:53227687-53227714 |
| RARG-413 | chr12:53213498-53213525 | RARG-1321 | chr12:53227710-53227737 |
| RARG-414 | chr12:53213510-53213537 | RARG-1322 | chr12:53227727-53227754 |
| RARG-415 | chr12:53213527-53213554 | RARG-1323 | chr12:53227740-53227767 |
| RARG-416 | chr12:53213528-53213555 | RARG-1324 | chr12:53227745-53227772 |
| RARG-417 | chr12:53213529-53213556 | RARG-1325 | chr12:53227746-53227773 |
| RARG-418 | chr12:53213533-53213560 | RARG-1326 | chr12:53227753-53227780 |
| RARG-419 | chr12:53213544-53213571 | RARG-1327 | chr12:53227754-53227781 |
| RARG-420 | chr12:53213549-53213576 | RARG-1328 | chr12:53227776-53227803 |
| RARG-421 | chr12:53213550-53213577 | RARG-1329 | chr12:53227777-53227804 |
| RARG-422 | chr12:53213560-53213587 | RARG-1330 | chr12:53227781-53227808 |
| RARG-423 | chr12:53213566-53213593 | RARG-1331 | chr12:53227787-53227814 |
| RARG-424 | chr12:53213567-53213594 | RARG-1332 | chr12:53231052-53231079 |
| RARG-425 | chr12:53213569-53213596 | RARG-1333 | chr12:53231055-53231082 |
| RARG-426 | chr12:53213570-53213597 | RARG-1334 | chr12:53231056-53231083 |
| RARG-427 | chr12:53213571-53213598 | RARG-1335 | chr12:53231059-53231086 |
| RARG-428 | chr12:53213582-53213609 | RARG-1336 | chr12:53231060-53231087 |
| RARG-429 | chr12:53213596-53213623 | RARG-1337 | chr12:53231066-53231093 |
| RARG-430 | chr12:53213597-53213624 | RARG-1338 | chr12:53231072-53231099 |
| RARG-431 | chr12:53213599-53213626 | RARG-1339 | chr12:53231073-53231100 |
| RARG-432 | chr12:53213600-53213627 | RARG-1340 | chr12:53231098-53231125 |
| RARG-433 | chr12:53213602-53213629 | RARG-1341 | chr12:53231104-53231131 |
| RARG-434 | chr12:53213606-53213633 | RARG-1342 | chr12:53231105-53231132 |
| RARG-435 | chr12:53213608-53213635 | RARG-1343 | chr12:53231113-53231140 |
| RARG-436 | chr12:53213609-53213636 | RARG-1344 | chr12:53231116-53231143 |
| RARG-437 | chr12:53213621-53213648 | RARG-1345 | chr12:53231128-53231155 |
| RARG-438 | chr12:53213626-53213653 | RARG-1346 | chr12:53231129-53231156 |
| RARG-439 | chr12:53213627-53213654 | RARG-1347 | chr12:53231130-53231157 |
| RARG-440 | chr12:53213633-53213660 | RARG-1348 | chr12:53231134-53231161 |
| RARG-441 | chr12:53213641-53213668 | RARG-1349 | chr12:53231135-53231162 |
| RARG-442 | chr12:53213642-53213669 | RARG-1350 | chr12:53231136-53231163 |
| RARG-443 | chr12:53213646-53213673 | RARG-1351 | chr12:53231137-53231164 |
| RARG-444 | chr12:53213647-53213674 | RARG-1352 | chr12:53231140-53231167 |
| RARG-445 | chr12:53213648-53213675 | RARG-1353 | chr12:53231143-53231170 |
| RARG-446 | chr12:53213664-53213691 | RARG-1354 | chr12:53231148-53231175 |
| RARG-447 | chr12:53213677-53213704 | RARG-1355 | chr12:53231153-53231180 |
| RARG-448 | chr12:53213680-53213707 | RARG-1356 | chr12:53231165-53231192 |
| RARG-449 | chr12:53213683-53213710 | RARG-1357 | chr12:53231168-53231195 |
| RARG-450 | chr12:53213684-53213711 | RARG-1358 | chr12:53231169-53231196 |
| RARG-451 | chr12:53213685-53213712 | RARG-1359 | chr12:53231176-53231203 |
| RARG-452 | chr12:53213686-53213713 | RARG-1360 | chr12:53231179-53231206 |
| RARG-453 | chr12:53213687-53213714 | RARG-1361 | chr12:53231180-53231207 |
| RARG-454 | chr12:53213690-53213717 | RARG-1362 | chr12:53231185-53231212 |
| RARG-455 | chr12:53213693-53213720 | RARG-1363 | chr12:53231191-53231218 |
| RARG-456 | chr12:53213696-53213723 | RARG-1364 | chr12:53231192-53231219 |
| RARG-457 | chr12:53213697-53213724 | RARG-1365 | chr12:53231198-53231225 |
| RARG-458 | chr12:53213713-53213740 | RARG-1366 | chr12:53231199-53231226 |
| RARG-459 | chr12:53213714-53213741 | RARG-1367 | chr12:53231223-53231250 |
| RARG-460 | chr12:53213715-53213742 | RARG-1368 | chr12:53231227-53231254 |
| RARG-461 | chr12:53213716-53213743 | RARG-1369 | chr12:53231235-53231262 |
| RARG-462 | chr12:53213720-53213747 | RARG-1370 | chr12:53231243-53231270 |
| RARG-463 | chr12:53213721-53213748 | RARG-1371 | chr12:53231244-53231271 |
| RARG-464 | chr12:53213722-53213749 | RARG-1372 | chr12:53231258-53231285 |
| RARG-465 | chr12:53213730-53213757 | RARG-1373 | chr12:53231259-53231286 |
| RARG-466 | chr12:53213731-53213758 | RARG-1374 | chr12:53231271-53231298 |
| RARG-467 | chr12:53213740-53213767 | RARG-1375 | chr12:53231273-53231300 |
| RARG-468 | chr12:53213749-53213776 | RARG-1376 | chr12:53231281-53231308 |
| RARG-469 | chr12:53213752-53213779 | RARG-1377 | chr12:53231288-53231315 |
| RARG-470 | chr12:53213757-53213784 | RARG-1378 | chr12:53231289-53231316 |
| RARG-471 | chr12:53213784-53213811 | RARG-1379 | chr12:53231294-53231321 |
| RARG-472 | chr12:53213786-53213813 | RARG-1380 | chr12:53231295-53231322 |
| RARG-473 | chr12:53213787-53213814 | RARG-1381 | chr12:53231300-53231327 |
| RARG-474 | chr12:53213788-53213815 | RARG-1382 | chr12:53231301-53231328 |
| RARG-475 | chr12:53213789-53213816 | RARG-1383 | chr12:53231303-53231330 |
| RARG-476 | chr12:53213795-53213822 | RARG-1384 | chr12:53231320-53231347 |
| RARG-477 | chr12:53213935-53213962 | RARG-1385 | chr12:53231325-53231352 |
| RARG-478 | chr12:53213936-53213963 | RARG-1386 | chr12:53231332-53231359 |
| RARG-479 | chr12:53213937-53213964 | RARG-1387 | chr12:53231333-53231360 |
| RARG-480 | chr12:53213938-53213965 | RARG-1388 | chr12:53231847-53231874 |
| RARG-481 | chr12:53213944-53213971 | RARG-1389 | chr12:53231848-53231875 |
| RARG-482 | chr12:53213945-53213972 | RARG-1390 | chr12:53231852-53231879 |
| RARG-483 | chr12:53213946-53213973 | RARG-1391 | chr12:53231856-53231883 |
| RARG-484 | chr12:53213947-53213974 | RARG-1392 | chr12:53231861-53231888 |
| RARG-485 | chr12:53213950-53213977 | RARG-1393 | chr12:53231865-53231892 |
| RARG-486 | chr12:53213964-53213991 | RARG-1394 | chr12:53231882-53231909 |
| RARG-487 | chr12:53213971-53213998 | RARG-1395 | chr12:53231883-53231910 |
| RARG-488 | chr12:53213982-53214009 | RARG-1396 | chr12:53231893-53231920 |
| RARG-489 | chr12:53213983-53214010 | RARG-1397 | chr12:53231896-53231923 |
| RARG-490 | chr12:53213995-53214022 | RARG-1398 | chr12:53231898-53231925 |
| RARG-491 | chr12:53213996-53214023 | RARG-1399 | chr12:53231901-53231928 |
| RARG-492 | chr12:53214000-53214027 | RARG-1400 | chr12:53231902-53231929 |
| RARG-493 | chr12:53214001-53214028 | RARG-1401 | chr12:53231903-53231930 |
| RARG-494 | chr12:53214007-53214034 | RARG-1402 | chr12:53231904-53231931 |
| RARG-495 | chr12:53214011-53214038 | RARG-1403 | chr12:53231907-53231934 |
| RARG-496 | chr12:53214012-53214039 | RARG-1404 | chr12:53231908-53231935 |
| RARG-497 | chr12:53214013-53214040 | RARG-1405 | chr12:53231909-53231936 |
| RARG-498 | chr12:53214020-53214047 | RARG-1406 | chr12:53231910-53231937 |
| RARG-499 | chr12:53214021-53214048 | RARG-1407 | chr12:53231914-53231941 |
| RARG-500 | chr12:53214035-53214062 | RARG-1408 | chr12:53231915-53231942 |
| RARG-501 | chr12:53214040-53214067 | RARG-1409 | chr12:53231916-53231943 |
| RARG-502 | chr12:53214041-53214068 | RARG-1410 | chr12:53231917-53231944 |
| RARG-503 | chr12:53214044-53214071 | RARG-1411 | chr12:53231919-53231946 |
| RARG-504 | chr12:53214048-53214075 | RARG-1412 | chr12:53231920-53231947 |
| RARG-505 | chr12:53214049-53214076 | RARG-1413 | chr12:53231922-53231949 |
| RARG-506 | chr12:53214058-53214085 | RARG-1414 | chr12:53231930-53231957 |
| RARG-507 | chr12:53214071-53214098 | RARG-1415 | chr12:53231931-53231958 |
| RARG-508 | chr12:53214100-53214127 | RARG-1416 | chr12:53231950-53231977 |
| RARG-509 | chr12:53214111-53214138 | RARG-1417 | chr12:53231952-53231979 |
| RARG-510 | chr12:53214113-53214140 | RARG-1418 | chr12:53231955-53231982 |
| RARG-511 | chr12:53214114-53214141 | RARG-1419 | chr12:53231956-53231983 |
| RARG-512 | chr12:53214123-53214150 | RARG-1420 | chr12:53231959-53231986 |
| RARG-513 | chr12:53214131-53214158 | RARG-1421 | chr12:53231960-53231987 |
| RARG-514 | chr12:53214141-53214168 | RARG-1422 | chr12:53231961-53231988 |
| RARG-515 | chr12:53214145-53214172 | RARG-1423 | chr12:53231962-53231989 |
| RARG-516 | chr12:53214172-53214199 | RARG-1424 | chr12:53231964-53231991 |
| RARG-517 | chr12:53214190-53214217 | RARG-1425 | chr12:53231966-53231993 |
| RARG-518 | chr12:53214191-53214218 | RARG-1426 | chr12:53231967-53231994 |
| RARG-519 | chr12:53214194-53214221 | RARG-1427 | chr12:53231970-53231997 |
| RARG-520 | chr12:53214197-53214224 | RARG-1428 | chr12:53231973-53232000 |
| RARG-521 | chr12:53214198-53214225 | RARG-1429 | chr12:53231976-53232003 |
| RARG-522 | chr12:53214199-53214226 | RARG-1430 | chr12:53231986-53232013 |
| RARG-523 | chr12:53214204-53214231 | RARG-1431 | chr12:53231992-53232019 |
| RARG-524 | chr12:53214205-53214232 | RARG-1432 | chr12:53231998-53232025 |
| RARG-525 | chr12:53214217-53214244 | RARG-1433 | chr12:53231999-53232026 |
| RARG-526 | chr12:53214218-53214245 | RARG-1434 | chr12:53232006-53232033 |
| RARG-527 | chr12:53214219-53214246 | RARG-1435 | chr12:53232007-53232034 |
| RARG-528 | chr12:53214222-53214249 | RARG-1436 | chr12:53232008-53232035 |
| RARG-529 | chr12:53214223-53214250 | RARG-1437 | chr12:53232010-53232037 |
| RARG-530 | chr12:53214224-53214251 | RARG-1438 | chr12:53232014-53232041 |
| RARG-531 | chr12:53214231-53214258 | RARG-1439 | chr12:53232015-53232042 |
| RARG-532 | chr12:53214235-53214262 | RARG-1440 | chr12:53232016-53232043 |
| RARG-533 | chr12:53214239-53214266 | RARG-1441 | chr12:53232031-53232058 |
| RARG-534 | chr12:53214249-53214276 | RARG-1442 | chr12:53232032-53232059 |
| RARG-535 | chr12:53214260-53214287 | RARG-1443 | chr12:53232033-53232060 |
| RARG-536 | chr12:53214261-53214288 | RARG-1444 | chr12:53232046-53232073 |
| RARG-537 | chr12:53214262-53214289 | RARG-1445 | chr12:53232047-53232074 |
| RARG-538 | chr12:53214263-53214290 | RARG-1446 | chr12:53232048-53232075 |
| RARG-539 | chr12:53214277-53214304 | RARG-1447 | chr12:53232049-53232076 |
| RARG-540 | chr12:53214278-53214305 | RARG-1448 | chr12:53232050-53232077 |
| RARG-541 | chr12:53214281-53214308 | RARG-1449 | chr12:53232055-53232082 |
| RARG-542 | chr12:53214305-53214332 | RARG-1450 | chr12:53232057-53232084 |
| RARG-543 | chr12:53214312-53214339 | RARG-1451 | chr12:53232058-53232085 |
| RARG-544 | chr12:53214313-53214340 | RARG-1452 | chr12:53232059-53232086 |
| RARG-545 | chr12:53214324-53214351 | RARG-1453 | chr12:53232060-53232087 |
| RARG-546 | chr12:53214340-53214367 | RARG-1454 | chr12:53232061-53232088 |
| RARG-547 | chr12:53214341-53214368 | RARG-1455 | chr12:53232062-53232089 |
| RARG-548 | chr12:53214344-53214371 | RARG-1456 | chr12:53232065-53232092 |
| RARG-549 | chr12:53214350-53214377 | RARG-1457 | chr12:53232067-53232094 |
| RARG-550 | chr12:53214358-53214385 | RARG-1458 | chr12:53232074-53232101 |
| RARG-551 | chr12:53214372-53214399 | RARG-1459 | chr12:53232078-53232105 |
| RARG-552 | chr12:53214375-53214402 | RARG-1460 | chr12:53232079-53232106 |
| RARG-553 | chr12:53214384-53214411 | RARG-1461 | chr12:53232080-53232107 |
| RARG-554 | chr12:53214385-53214412 | RARG-1462 | chr12:53232089-53232116 |
| RARG-555 | chr12:53214389-53214416 | RARG-1463 | chr12:53232090-53232117 |
| RARG-556 | chr12:53214390-53214417 | RARG-1464 | chr12:53232091-53232118 |
| RARG-557 | chr12:53214391-53214418 | RARG-1465 | chr12:53232099-53232126 |
| RARG-558 | chr12:53214396-53214423 | RARG-1466 | chr12:53232112-53232139 |
| RARG-559 | chr12:53214397-53214424 | RARG-1467 | chr12:53232113-53232140 |
| RARG-560 | chr12:53214398-53214425 | RARG-1468 | chr12:53232119-53232146 |
| RARG-561 | chr12:53214400-53214427 | RARG-1469 | chr12:53232129-53232156 |
| RARG-562 | chr12:53214401-53214428 | RARG-1470 | chr12:53232130-53232157 |
| RARG-563 | chr12:53214413-53214440 | RARG-1471 | chr12:53232133-53232160 |
| RARG-564 | chr12:53214414-53214441 | RARG-1472 | chr12:53232134-53232161 |
| RARG-565 | chr12:53214418-53214445 | RARG-1473 | chr12:53232137-53232164 |
| RARG-566 | chr12:53214419-53214446 | RARG-1474 | chr12:53232138-53232165 |
| RARG-567 | chr12:53214423-53214450 | RARG-1475 | chr12:53232146-53232173 |
| RARG-568 | chr12:53214434-53214461 | RARG-1476 | chr12:53232149-53232176 |
| RARG-569 | chr12:53214435-53214462 | RARG-1477 | chr12:53232150-53232177 |
| RARG-570 | chr12:53214440-53214467 | RARG-1478 | chr12:53232151-53232178 |
| RARG-571 | chr12:53214445-53214472 | RARG-1479 | chr12:53232160-53232187 |
| RARG-572 | chr12:53214450-53214477 | RARG-1480 | chr12:53232172-53232199 |
| RARG-573 | chr12:53214451-53214478 | RARG-1481 | chr12:53232174-53232201 |
| RARG-574 | chr12:53214452-53214479 | RARG-1482 | chr12:53232175-53232202 |
| RARG-575 | chr12:53214459-53214486 | RARG-1483 | chr12:53232176-53232203 |
| RARG-576 | chr12:53214460-53214487 | RARG-1484 | chr12:53232177-53232204 |
| RARG-577 | chr12:53214467-53214494 | RARG-1485 | chr12:53232180-53232207 |
| RARG-578 | chr12:53214468-53214495 | RARG-1486 | chr12:53232183-53232210 |
| RARG-579 | chr12:53214483-53214510 | RARG-1487 | chr12:53232233-53232260 |
| RARG-580 | chr12:53214487-53214514 | RARG-1488 | chr12:53232234-53232261 |
| RARG-581 | chr12:53214505-53214532 | RARG-1489 | chr12:53232237-53232264 |
| RARG-582 | chr12:53214506-53214533 | RARG-1490 | chr12:53232238-53232265 |
| RARG-583 | chr12:53214526-53214553 | RARG-1491 | chr12:53232239-53232266 |
| RARG-584 | chr12:53214557-53214584 | RARG-1492 | chr12:53232242-53232269 |
| RARG-585 | chr12:53214558-53214585 | RARG-1493 | chr12:53232243-53232270 |
| RARG-586 | chr12:53214565-53214592 | RARG-1494 | chr12:53232244-53232271 |
| RARG-587 | chr12:53214566-53214593 | RARG-1495 | chr12:53232245-53232272 |
| RARG-588 | chr12:53214571-53214598 | RARG-1496 | chr12:53232246-53232273 |
| RARG-589 | chr12:53214586-53214613 | RARG-1497 | chr12:53232247-53232274 |
| RARG-590 | chr12:53214587-53214614 | RARG-1498 | chr12:53232248-53232275 |
| RARG-591 | chr12:53214590-53214617 | RARG-1499 | chr12:53232249-53232276 |
| RARG-592 | chr12:53214593-53214620 | RARG-1500 | chr12:53232250-53232277 |
| RARG-593 | chr12:53214600-53214627 | RARG-1501 | chr12:53232254-53232281 |
| RARG-594 | chr12:53214605-53214632 | RARG-1502 | chr12:53232257-53232284 |
| RARG-595 | chr12:53214606-53214633 | RARG-1503 | chr12:53232258-53232285 |
| RARG-596 | chr12:53214611-53214638 | RARG-1504 | chr12:53232261-53232288 |
| RARG-597 | chr12:53214632-53214659 | RARG-1505 | chr12:53232262-53232289 |
| RARG-598 | chr12:53214633-53214660 | RARG-1506 | chr12:53232266-53232293 |
| RARG-599 | chr12:53214640-53214667 | RARG-1507 | chr12:53232267-53232294 |
| RARG-600 | chr12:53214641-53214668 | RARG-1508 | chr12:53232273-53232300 |
| RARG-601 | chr12:53214649-53214676 | RARG-1509 | chr12:53232274-53232301 |
| RARG-602 | chr12:53214652-53214679 | RARG-1510 | chr12:53232277-53232304 |
| RARG-603 | chr12:53214653-53214680 | RARG-1511 | chr12:53232280-53232307 |
| RARG-604 | chr12:53214664-53214691 | RARG-1512 | chr12:53232281-53232308 |
| RARG-605 | chr12:53214673-53214700 | RARG-1513 | chr12:53232282-53232309 |
| RARG-606 | chr12:53214674-53214701 | RARG-1514 | chr12:53232283-53232310 |
| RARG-607 | chr12:53214692-53214719 | RARG-1515 | chr12:53232286-53232313 |
| RARG-608 | chr12:53214694-53214721 | RARG-1516 | chr12:53232287-53232314 |
| RARG-609 | chr12:53215175-53215202 | RARG-1517 | chr12:53232290-53232317 |
| RARG-610 | chr12:53215177-53215204 | RARG-1518 | chr12:53232291-53232318 |
| RARG-611 | chr12:53215178-53215205 | RARG-1519 | chr12:53232298-53232325 |
| RARG-612 | chr12:53215179-53215206 | RARG-1520 | chr12:53232301-53232328 |
| RARG-613 | chr12:53215180-53215207 | RARG-1521 | chr12:53232302-53232329 |
| RARG-614 | chr12:53215181-53215208 | RARG-1522 | chr12:53232305-53232332 |
| RARG-615 | chr12:53215187-53215214 | RARG-1523 | chr12:53232306-53232333 |
| RARG-616 | chr12:53215188-53215215 | RARG-1524 | chr12:53232307-53232334 |
| RARG-617 | chr12:53215189-53215216 | RARG-1525 | chr12:53232311-53232338 |
| RARG-618 | chr12:53215193-53215220 | RARG-1526 | chr12:53232315-53232342 |
| RARG-619 | chr12:53215200-53215227 | RARG-1527 | chr12:53232316-53232343 |
| RARG-620 | chr12:53215207-53215234 | RARG-1528 | chr12:53232317-53232344 |
| RARG-621 | chr12:53215208-53215235 | RARG-1529 | chr12:53232318-53232345 |
| RARG-622 | chr12:53215221-53215248 | RARG-1530 | chr12:53232319-53232346 |
| RARG-623 | chr12:53215222-53215249 | RARG-1531 | chr12:53232320-53232347 |
| RARG-624 | chr12:53215226-53215253 | RARG-1532 | chr12:53232321-53232348 |
| RARG-625 | chr12:53215236-53215263 | RARG-1533 | chr12:53232322-53232349 |
| RARG-626 | chr12:53215242-53215269 | RARG-1534 | chr12:53232324-53232351 |
| RARG-627 | chr12:53215253-53215280 | RARG-1535 | chr12:53232325-53232352 |
| RARG-628 | chr12:53215255-53215282 | RARG-1536 | chr12:53232326-53232353 |
| RARG-629 | chr12:53215257-53215284 | RARG-1537 | chr12:53232327-53232354 |
| RARG-630 | chr12:53215265-53215292 | RARG-1538 | chr12:53232328-53232355 |
| RARG-631 | chr12:53215269-53215296 | RARG-1539 | chr12:53232329-53232356 |
| RARG-632 | chr12:53215270-53215297 | RARG-1540 | chr12:53232331-53232358 |
| RARG-633 | chr12:53215274-53215301 | RARG-1541 | chr12:53232332-53232359 |
| RARG-634 | chr12:53215275-53215302 | RARG-1542 | chr12:53232334-53232361 |
| RARG-635 | chr12:53215288-53215315 | RARG-1543 | chr12:53232335-53232362 |
| RARG-636 | chr12:53215292-53215319 | RARG-1544 | chr12:53232339-53232366 |
| RARG-637 | chr12:53215296-53215323 | RARG-1545 | chr12:53232340-53232367 |
| RARG-638 | chr12:53215306-53215333 | RARG-1546 | chr12:53232341-53232368 |
| RARG-639 | chr12:53215307-53215334 | RARG-1547 | chr12:53232343-53232370 |
| RARG-640 | chr12:53215308-53215335 | RARG-1548 | chr12:53232344-53232371 |
| RARG-641 | chr12:53215315-53215342 | RARG-1549 | chr12:53232347-53232374 |
| RARG-642 | chr12:53215328-53215355 | RARG-1550 | chr12:53232348-53232375 |
| RARG-643 | chr12:53215330-53215357 | RARG-1551 | chr12:53232351-53232378 |
| RARG-644 | chr12:53215351-53215378 | RARG-1552 | chr12:53232352-53232379 |
| RARG-645 | chr12:53215359-53215386 | RARG-1553 | chr12:53232353-53232380 |
| RARG-646 | chr12:53215360-53215387 | RARG-1554 | chr12:53232354-53232381 |
| RARG-647 | chr12:53215387-53215414 | RARG-1555 | chr12:53232359-53232386 |
| RARG-648 | chr12:53215396-53215423 | RARG-1556 | chr12:53232360-53232387 |
| RARG-649 | chr12:53215401-53215428 | RARG-1557 | chr12:53232363-53232390 |
| RARG-650 | chr12:53215411-53215438 | RARG-1558 | chr12:53232364-53232391 |
| RARG-651 | chr12:53215419-53215446 | RARG-1559 | chr12:53232365-53232392 |
| RARG-652 | chr12:53215420-53215447 | RARG-1560 | chr12:53232368-53232395 |
| RARG-653 | chr12:53215431-53215458 | RARG-1561 | chr12:53232373-53232400 |
| RARG-654 | chr12:53215432-53215459 | RARG-1562 | chr12:53232374-53232401 |
| RARG-655 | chr12:53215433-53215460 | RARG-1563 | chr12:53232382-53232409 |
| RARG-656 | chr12:53215434-53215461 | RARG-1564 | chr12:53232383-53232410 |
| RARG-657 | chr12:53215452-53215479 | RARG-1565 | chr12:53232396-53232423 |
| RARG-658 | chr12:53215453-53215480 | RARG-1566 | chr12:53232397-53232424 |
| RARG-659 | chr12:53215460-53215487 | RARG-1567 | chr12:53232405-53232432 |
| RARG-660 | chr12:53215472-53215499 | RARG-1568 | chr12:53232407-53232434 |
| RARG-661 | chr12:53215481-53215508 | RARG-1569 | chr12:53232417-53232444 |
| RARG-662 | chr12:53215485-53215512 | RARG-1570 | chr12:53232418-53232445 |
| RARG-663 | chr12:53215489-53215516 | RARG-1571 | chr12:53232422-53232449 |
| RARG-664 | chr12:53215500-53215527 | RARG-1572 | chr12:53232423-53232450 |
| RARG-665 | chr12:53215512-53215539 | RARG-1573 | chr12:53232429-53232456 |
| RARG-666 | chr12:53215513-53215540 | RARG-1574 | chr12:53232430-53232457 |
| RARG-667 | chr12:53215514-53215541 | RARG-1575 | chr12:53232433-53232460 |
| RARG-668 | chr12:53215519-53215546 | RARG-1576 | chr12:53232437-53232464 |
| RARG-669 | chr12:53215524-53215551 | RARG-1577 | chr12:53232438-53232465 |
| RARG-670 | chr12:53215537-53215564 | RARG-1578 | chr12:53232443-53232470 |
| RARG-671 | chr12:53215540-53215567 | RARG-1579 | chr12:53232446-53232473 |
| RARG-672 | chr12:53215559-53215586 | RARG-1580 | chr12:53232447-53232474 |
| RARG-673 | chr12:53215576-53215603 | RARG-1581 | chr12:53232450-53232477 |
| RARG-674 | chr12:53215577-53215604 | RARG-1582 | chr12:53232453-53232480 |
| RARG-675 | chr12:53215582-53215609 | RARG-1583 | chr12:53232456-53232483 |
| RARG-676 | chr12:53215584-53215611 | RARG-1584 | chr12:53232461-53232488 |
| RARG-677 | chr12:53215614-53215641 | RARG-1585 | chr12:53232468-53232495 |
| RARG-678 | chr12:53215615-53215642 | RARG-1586 | chr12:53232491-53232518 |
| RARG-679 | chr12:53215616-53215643 | RARG-1587 | chr12:53232496-53232523 |
| RARG-680 | chr12:53215617-53215644 | RARG-1588 | chr12:53232497-53232524 |
| RARG-681 | chr12:53215624-53215651 | RARG-1589 | chr12:53232498-53232525 |
| RARG-682 | chr12:53215625-53215652 | RARG-1590 | chr12:53232506-53232533 |
| RARG-683 | chr12:53215630-53215657 | RARG-1591 | chr12:53232510-53232537 |
| RARG-684 | chr12:53215631-53215658 | RARG-1592 | chr12:53232521-53232548 |
| RARG-685 | chr12:53215634-53215661 | RARG-1593 | chr12:53232522-53232549 |
| RARG-686 | chr12:53215645-53215672 | RARG-1594 | chr12:53232523-53232550 |
| RARG-687 | chr12:53215652-53215679 | RARG-1595 | chr12:53232524-53232551 |
| RARG-688 | chr12:53215653-53215680 | RARG-1596 | chr12:53232525-53232552 |
| RARG-689 | chr12:53215662-53215689 | RARG-1597 | chr12:53232533-53232560 |
| RARG-690 | chr12:53215669-53215696 | RARG-1598 | chr12:53232541-53232568 |
| RARG-691 | chr12:53215670-53215697 | RARG-1599 | chr12:53232545-53232572 |
| RARG-692 | chr12:53215671-53215698 | RARG-1600 | chr12:53232550-53232577 |
| RARG-693 | chr12:53215683-53215710 | RARG-1601 | chr12:53232567-53232594 |
| RARG-694 | chr12:53215687-53215714 | RARG-1602 | chr12:53232573-53232600 |
| RARG-695 | chr12:53215702-53215729 | RARG-1603 | chr12:53232578-53232605 |
| RARG-696 | chr12:53215705-53215732 | RARG-1604 | chr12:53232590-53232617 |
| RARG-697 | chr12:53215708-53215735 | RARG-1605 | chr12:53232591-53232618 |
| RARG-698 | chr12:53215711-53215738 | RARG-1606 | chr12:53232595-53232622 |
| RARG-699 | chr12:53215712-53215739 | RARG-1607 | chr12:53232601-53232628 |
| RARG-700 | chr12:53215713-53215740 | RARG-1608 | chr12:53232602-53232629 |
| RARG-701 | chr12:53215714-53215741 | RARG-1609 | chr12:53232603-53232630 |
| RARG-702 | chr12:53215719-53215746 | RARG-1610 | chr12:53232606-53232633 |
| RARG-703 | chr12:53215720-53215747 | RARG-1611 | chr12:53232608-53232635 |
| RARG-704 | chr12:53215723-53215750 | RARG-1612 | chr12:53232611-53232638 |
| RARG-705 | chr12:53215724-53215751 | RARG-1613 | chr12:53232612-53232639 |
| RARG-706 | chr12:53215728-53215755 | RARG-1614 | chr12:53232613-53232640 |
| RARG-707 | chr12:53215729-53215756 | RARG-1615 | chr12:53232629-53232656 |
| RARG-708 | chr12:53215749-53215776 | RARG-1616 | chr12:53232634-53232661 |
| RARG-709 | chr12:53215759-53215786 | RARG-1617 | chr12:53232636-53232663 |
| RARG-710 | chr12:53215765-53215792 | RARG-1618 | chr12:53232637-53232664 |
| RARG-711 | chr12:53215771-53215798 | RARG-1619 | chr12:53232638-53232665 |
| RARG-712 | chr12:53215772-53215799 | RARG-1620 | chr12:53232639-53232666 |
| RARG-713 | chr12:53215775-53215802 | RARG-1621 | chr12:53232642-53232669 |
| RARG-714 | chr12:53215776-53215803 | RARG-1622 | chr12:53232643-53232670 |
| RARG-715 | chr12:53215789-53215816 | RARG-1623 | chr12:53232646-53232673 |
| RARG-716 | chr12:53215792-53215819 | RARG-1624 | chr12:53232647-53232674 |
| RARG-717 | chr12:53215793-53215820 | RARG-1625 | chr12:53232662-53232689 |
| RARG-718 | chr12:53215798-53215825 | RARG-1626 | chr12:53232677-53232704 |
| RARG-719 | chr12:53215799-53215826 | RARG-1627 | chr12:53232697-53232724 |
| RARG-720 | chr12:53215800-53215827 | RARG-1628 | chr12:53232701-53232728 |
| RARG-721 | chr12:53215806-53215833 | RARG-1629 | chr12:53232702-53232729 |
| RARG-722 | chr12:53215824-53215851 | RARG-1630 | chr12:53232719-53232746 |
| RARG-723 | chr12:53215835-53215862 | RARG-1631 | chr12:53232725-53232752 |
| RARG-724 | chr12:53215836-53215863 | RARG-1632 | chr12:53232734-53232761 |
| RARG-725 | chr12:53215837-53215864 | RARG-1633 | chr12:53232742-53232769 |
| RARG-726 | chr12:53215844-53215871 | RARG-1634 | chr12:53232747-53232774 |
| RARG-727 | chr12:53215847-53215874 | RARG-1635 | chr12:53232748-53232775 |
| RARG-728 | chr12:53215855-53215882 | RARG-1636 | chr12:53232749-53232776 |
| RARG-729 | chr12:53215864-53215891 | RARG-1637 | chr12:53232752-53232779 |
| RARG-730 | chr12:53215869-53215896 | RARG-1638 | chr12:53232753-53232780 |
| RARG-731 | chr12:53215870-53215897 | RARG-1639 | chr12:53232754-53232781 |
| RARG-732 | chr12:53215871-53215898 | RARG-1640 | chr12:53232756-53232783 |
| RARG-733 | chr12:53215872-53215899 | RARG-1641 | chr12:53232759-53232786 |
| RARG-734 | chr12:53215886-53215913 | RARG-1642 | chr12:53232760-53232787 |
| RARG-735 | chr12:53215892-53215919 | RARG-1643 | chr12:53232763-53232790 |
| RARG-736 | chr12:53215893-53215920 | RARG-1644 | chr12:53232766-53232793 |
| RARG-737 | chr12:53219824-53219851 | RARG-1645 | chr12:53232770-53232797 |
| RARG-738 | chr12:53219830-53219857 | RARG-1646 | chr12:53232777-53232804 |
| RARG-739 | chr12:53219846-53219873 | RARG-1647 | chr12:53232778-53232805 |
| RARG-740 | chr12:53219855-53219882 | RARG-1648 | chr12:53232779-53232806 |
| RARG-741 | chr12:53219858-53219885 | RARG-1649 | chr12:53232787-53232814 |
| RARG-742 | chr12:53219862-53219889 | RARG-1650 | chr12:53232788-53232815 |
| RARG-743 | chr12:53219865-53219892 | RARG-1651 | chr12:53232789-53232816 |
| RARG-744 | chr12:53219873-53219900 | RARG-1652 | chr12:53232790-53232817 |
| RARG-745 | chr12:53219901-53219928 | RARG-1653 | chr12:53232798-53232825 |
| RARG-746 | chr12:53219902-53219929 | RARG-1654 | chr12:53232803-53232830 |
| RARG-747 | chr12:53219905-53219932 | RARG-1655 | chr12:53232804-53232831 |
| RARG-748 | chr12:53219906-53219933 | RARG-1656 | chr12:53232826-53232853 |
| RARG-749 | chr12:53219908-53219935 | RARG-1657 | chr12:53232836-53232863 |
| RARG-750 | chr12:53219911-53219938 | RARG-1658 | chr12:53232846-53232873 |
| RARG-751 | chr12:53219915-53219942 | RARG-1659 | chr12:53232847-53232874 |
| RARG-752 | chr12:53219917-53219944 | RARG-1660 | chr12:53232849-53232876 |
| RARG-753 | chr12:53219918-53219945 | RARG-1661 | chr12:53232856-53232883 |
| RARG-754 | chr12:53219924-53219951 | RARG-1662 | chr12:53232857-53232884 |
| RARG-755 | chr12:53219925-53219952 | RARG-1663 | chr12:53232859-53232886 |
| RARG-756 | chr12:53219926-53219953 | RARG-1664 | chr12:53232864-53232891 |
| RARG-757 | chr12:53219930-53219957 | RARG-1665 | chr12:53232865-53232892 |
| RARG-758 | chr12:53219931-53219958 | RARG-1666 | chr12:53232872-53232899 |
| RARG-759 | chr12:53219932-53219959 | RARG-1667 | chr12:53232887-53232914 |
| RARG-760 | chr12:53219934-53219961 | RARG-1668 | chr12:53232894-53232921 |
| RARG-761 | chr12:53219937-53219964 | RARG-1669 | chr12:53232895-53232922 |
| RARG-762 | chr12:53219938-53219965 | RARG-1670 | chr12:53232897-53232924 |
| RARG-763 | chr12:53219939-53219966 | RARG-1671 | chr12:53232902-53232929 |
| RARG-764 | chr12:53219944-53219971 | RARG-1672 | chr12:53232903-53232930 |
| RARG-765 | chr12:53219945-53219972 | RARG-1673 | chr12:53232908-53232935 |
| RARG-766 | chr12:53219949-53219976 | RARG-1674 | chr12:53232911-53232938 |
| RARG-767 | chr12:53219967-53219994 | RARG-1675 | chr12:53232912-53232939 |
| RARG-768 | chr12:53219972-53219999 | RARG-1676 | chr12:53232913-53232940 |
| RARG-769 | chr12:53219976-53220003 | RARG-1677 | chr12:53232914-53232941 |
| RARG-770 | chr12:53219985-53220012 | RARG-1678 | chr12:53232917-53232944 |
| RARG-771 | chr12:53219988-53220015 | RARG-1679 | chr12:53232919-53232946 |
| RARG-772 | chr12:53219991-53220018 | RARG-1680 | chr12:53232923-53232950 |
| RARG-773 | chr12:53220006-53220033 | RARG-1681 | chr12:53232925-53232952 |
| RARG-774 | chr12:53220009-53220036 | RARG-1682 | chr12:53232928-53232955 |
| RARG-775 | chr12:53220012-53220039 | RARG-1683 | chr12:53232931-53232958 |
| RARG-776 | chr12:53220013-53220040 | RARG-1684 | chr12:53232932-53232959 |
| RARG-777 | chr12:53220018-53220045 | RARG-1685 | chr12:53232936-53232963 |
| RARG-778 | chr12:53220030-53220057 | RARG-1686 | chr12:53232939-53232966 |
| RARG-779 | chr12:53220034-53220061 | RARG-1687 | chr12:53232947-53232974 |
| RARG-780 | chr12:53220035-53220062 | RARG-1688 | chr12:53232951-53232978 |
| RARG-781 | chr12:53220036-53220063 | RARG-1689 | chr12:53232952-53232979 |
| RARG-782 | chr12:53220040-53220067 | RARG-1690 | chr12:53232957-53232984 |
| RARG-783 | chr12:53220041-53220068 | RARG-1691 | chr12:53232958-53232985 |
| RARG-784 | chr12:53220042-53220069 | RARG-1692 | chr12:53232961-53232988 |
| RARG-785 | chr12:53220046-53220073 | RARG-1693 | chr12:53232969-53232996 |
| RARG-786 | chr12:53220047-53220074 | RARG-1694 | chr12:53232970-53232997 |
| RARG-787 | chr12:53220048-53220075 | RARG-1695 | chr12:53232974-53233001 |
| RARG-788 | chr12:53220049-53220076 | RARG-1696 | chr12:53232975-53233002 |
| RARG-789 | chr12:53220050-53220077 | RARG-1697 | chr12:53232991-53233018 |
| RARG-790 | chr12:53220051-53220078 | RARG-1698 | chr12:53232995-53233022 |
| RARG-791 | chr12:53220059-53220086 | RARG-1699 | chr12:53233001-53233028 |
| RARG-792 | chr12:53220069-53220096 | RARG-1700 | chr12:53233002-53233029 |
| RARG-793 | chr12:53220070-53220097 | RARG-1701 | chr12:53233017-53233044 |
| RARG-794 | chr12:53220083-53220110 | RARG-1702 | chr12:53233018-53233045 |
| RARG-795 | chr12:53220085-53220112 | RARG-1703 | chr12:53233028-53233055 |
| RARG-796 | chr12:53220091-53220118 | RARG-1704 | chr12:53233040-53233067 |
| RARG-797 | chr12:53220092-53220119 | RARG-1705 | chr12:53233041-53233068 |
| RARG-798 | chr12:53220099-53220126 | RARG-1706 | chr12:53233046-53233073 |
| RARG-799 | chr12:53220100-53220127 | RARG-1707 | chr12:53233049-53233076 |
| RARG-800 | chr12:53220106-53220133 | RARG-1708 | chr12:53233052-53233079 |
| RARG-801 | chr12:53220107-53220134 | RARG-1709 | chr12:53233056-53233083 |
| RARG-802 | chr12:53220117-53220144 | RARG-1710 | chr12:53233060-53233087 |
| RARG-803 | chr12:53220139-53220166 | RARG-1711 | chr12:53233064-53233091 |
| RARG-804 | chr12:53220146-53220173 | RARG-1712 | chr12:53233068-53233095 |
| RARG-805 | chr12:53220147-53220174 | RARG-1713 | chr12:53233072-53233099 |
| RARG-806 | chr12:53220150-53220177 | RARG-1714 | chr12:53233079-53233106 |
| RARG-807 | chr12:53220153-53220180 | RARG-1715 | chr12:53233080-53233107 |
| RARG-808 | chr12:53220159-53220186 | RARG-1716 | chr12:53233085-53233112 |
| RARG-809 | chr12:53220160-53220187 | RARG-1717 | chr12:53233103-53233130 |
| RARG-810 | chr12:53220161-53220188 | RARG-1718 | chr12:53233162-53233189 |
| RARG-811 | chr12:53220162-53220189 | RARG-1719 | chr12:53233167-53233194 |
| RARG-812 | chr12:53220165-53220192 | RARG-1720 | chr12:53233168-53233195 |
| RARG-813 | chr12:53220167-53220194 | RARG-1721 | chr12:53233169-53233196 |
| RARG-814 | chr12:53220168-53220195 | RARG-1722 | chr12:53233170-53233197 |
| RARG-815 | chr12:53220169-53220196 | RARG-1723 | chr12:53233226-53233253 |
| RARG-816 | chr12:53220170-53220197 | RARG-1724 | chr12:53233230-53233257 |
| RARG-817 | chr12:53220171-53220198 | RARG-1725 | chr12:53233231-53233258 |
| RARG-818 | chr12:53220172-53220199 | RARG-1726 | chr12:53233246-53233273 |
| RARG-819 | chr12:53220175-53220202 | RARG-1727 | chr12:53233247-53233274 |
| RARG-820 | chr12:53220176-53220203 | RARG-1728 | chr12:53233249-53233276 |
| RARG-821 | chr12:53220177-53220204 | RARG-1729 | chr12:53233256-53233283 |
| RARG-822 | chr12:53220178-53220205 | RARG-1730 | chr12:53233257-53233284 |
| RARG-823 | chr12:53220180-53220207 | RARG-1731 | chr12:53233271-53233298 |
| RARG-824 | chr12:53220181-53220208 | RARG-1732 | chr12:53233272-53233299 |
| RARG-825 | chr12:53220182-53220209 | RARG-1733 | chr12:53233297-53233324 |
| RARG-826 | chr12:53220187-53220214 | RARG-1734 | chr12:53233308-53233335 |
| RARG-827 | chr12:53220192-53220219 | RARG-1735 | chr12:53233316-53233343 |
| RARG-828 | chr12:53220193-53220220 | RARG-1736 | chr12:53233317-53233344 |
| RARG-829 | chr12:53220199-53220226 | RARG-1737 | chr12:53233318-53233345 |
| RARG-830 | chr12:53220200-53220227 | RARG-1738 | chr12:53233323-53233350 |
| RARG-831 | chr12:53220210-53220237 | RARG-1739 | chr12:53233327-53233354 |
| RARG-832 | chr12:53220211-53220238 | RARG-1740 | chr12:53233328-53233355 |
| RARG-833 | chr12:53220216-53220243 | RARG-1741 | chr12:53233329-53233356 |
| RARG-834 | chr12:53220217-53220244 | RARG-1742 | chr12:53233330-53233357 |
| RARG-835 | chr12:53220218-53220245 | RARG-1743 | chr12:53233333-53233360 |
| RARG-836 | chr12:53220221-53220248 | RARG-1744 | chr12:53233334-53233361 |
| RARG-837 | chr12:53220222-53220249 | RARG-1745 | chr12:53233337-53233364 |
| RARG-838 | chr12:53220225-53220252 | RARG-1746 | chr12:53233338-53233365 |
| RARG-839 | chr12:53220226-53220253 | RARG-1747 | chr12:53233343-53233370 |
| RARG-840 | chr12:53220227-53220254 | RARG-1748 | chr12:53233349-53233376 |
| RARG-841 | chr12:53220233-53220260 | RARG-1749 | chr12:53233350-53233377 |
| RARG-842 | chr12:53220234-53220261 | RARG-1750 | chr12:53233351-53233378 |
| RARG-843 | chr12:53220235-53220262 | RARG-1751 | chr12:53233366-53233393 |
| RARG-844 | chr12:53220236-53220263 | RARG-1752 | chr12:53233374-53233401 |
| RARG-845 | chr12:53220237-53220264 | RARG-1753 | chr12:53233378-53233405 |
| RARG-846 | chr12:53220249-53220276 | RARG-1754 | chr12:53233415-53233442 |
| RARG-847 | chr12:53220250-53220277 | RARG-1755 | chr12:53233416-53233443 |
| RARG-848 | chr12:53220251-53220278 | RARG-1756 | chr12:53233419-53233446 |
| RARG-849 | chr12:53220256-53220283 | RARG-1757 | chr12:53233420-53233447 |
| RARG-850 | chr12:53220257-53220284 | RARG-1758 | chr12:53233424-53233451 |
| RARG-851 | chr12:53220262-53220289 | RARG-1759 | chr12:53233428-53233455 |
| RARG-852 | chr12:53220263-53220290 | RARG-1760 | chr12:53233429-53233456 |
| RARG-853 | chr12:53220266-53220293 | RARG-1761 | chr12:53233430-53233457 |
| RARG-854 | chr12:53220267-53220294 | RARG-1762 | chr12:53233434-53233461 |
| RARG-855 | chr12:53220268-53220295 | RARG-1763 | chr12:53233435-53233462 |
| RARG-856 | chr12:53220269-53220296 | RARG-1764 | chr12:53233449-53233476 |
| RARG-857 | chr12:53220279-53220306 | RARG-1765 | chr12:53233450-53233477 |
| RARG-858 | chr12:53220280-53220307 | RARG-1766 | chr12:53233451-53233478 |
| RARG-859 | chr12:53220281-53220308 | RARG-1767 | chr12:53233457-53233484 |
| RARG-860 | chr12:53220312-53220339 | RARG-1768 | chr12:53233472-53233499 |
| RARG-861 | chr12:53220315-53220342 | RARG-1769 | chr12:53233473-53233500 |
| RARG-862 | chr12:53220316-53220343 | RARG-1770 | chr12:53233483-53233510 |
| RARG-863 | chr12:53220321-53220348 | RARG-1771 | chr12:53233492-53233519 |
| RARG-864 | chr12:53220324-53220351 | RARG-1772 | chr12:53233505-53233532 |
| RARG-865 | chr12:53220328-53220355 | RARG-1773 | chr12:53233506-53233533 |
| RARG-866 | chr12:53220337-53220364 | RARG-1774 | chr12:53233507-53233534 |
| RARG-867 | chr12:53220338-53220365 | RARG-1775 | chr12:53233508-53233535 |
| RARG-868 | chr12:53220339-53220366 | RARG-1776 | chr12:53233513-53233540 |
| RARG-869 | chr12:53220365-53220392 | RARG-1777 | chr12:53233517-53233544 |
| RARG-870 | chr12:53220368-53220395 | RARG-1778 | chr12:53233524-53233551 |
| RARG-871 | chr12:53220369-53220396 | RARG-1779 | chr12:53233525-53233552 |
| RARG-872 | chr12:53220370-53220397 | RARG-1780 | chr12:53233526-53233553 |
| RARG-873 | chr12:53220373-53220400 | RARG-1781 | chr12:53233527-53233554 |
| RARG-874 | chr12:53220374-53220401 | RARG-1782 | chr12:53233528-53233555 |
| RARG-875 | chr12:53220375-53220402 | RARG-1783 | chr12:53233529-53233556 |
| RARG-876 | chr12:53220376-53220403 | RARG-1784 | chr12:53233534-53233561 |
| RARG-877 | chr12:53220377-53220404 | RARG-1785 | chr12:53233536-53233563 |
| RARG-878 | chr12:53220380-53220407 | RARG-1786 | chr12:53233544-53233571 |
| RARG-879 | chr12:53220381-53220408 | RARG-1787 | chr12:53233547-53233574 |
| RARG-880 | chr12:53220382-53220409 | RARG-1788 | chr12:53233548-53233575 |
| RARG-881 | chr12:53220387-53220414 | RARG-1789 | chr12:53233551-53233578 |
| RARG-882 | chr12:53220388-53220415 | RARG-1790 | chr12:53233558-53233585 |
| RARG-883 | chr12:53220397-53220424 | RARG-1791 | chr12:53233564-53233591 |
| RARG-884 | chr12:53220398-53220425 | RARG-1792 | chr12:53233570-53233597 |
| RARG-885 | chr12:53220399-53220426 | RARG-1793 | chr12:53233571-53233598 |
| RARG-886 | chr12:53220400-53220427 | RARG-1794 | chr12:53233572-53233599 |
| RARG-887 | chr12:53220404-53220431 | RARG-1795 | chr12:53233573-53233600 |
| RARG-888 | chr12:53220405-53220432 | RARG-1796 | chr12:53233575-53233602 |
| RARG-889 | chr12:53220428-53220455 | RARG-1797 | chr12:53233583-53233610 |
| RARG-890 | chr12:53220432-53220459 | RARG-1798 | chr12:53233593-53233620 |
| RARG-891 | chr12:53220435-53220462 | RARG-1799 | chr12:53233603-53233630 |
| RARG-892 | chr12:53220468-53220495 | RARG-1800 | chr12:53233604-53233631 |
| RARG-893 | chr12:53220490-53220517 | RARG-1801 | chr12:53233609-53233636 |
| RARG-894 | chr12:53220518-53220545 | RARG-1802 | chr12:53233610-53233637 |
| RARG-895 | chr12:53220521-53220548 | RARG-1803 | chr12:53233616-53233643 |
| RARG-896 | chr12:53220548-53220575 | RARG-1804 | chr12:53233622-53233649 |
| RARG-897 | chr12:53220581-53220608 | RARG-1805 | chr12:53233623-53233650 |
| RARG-898 | chr12:53220615-53220642 | RARG-1806 | chr12:53233624-53233651 |
| RARG-899 | chr12:53220616-53220643 | RARG-1807 | chr12:53233625-53233652 |
| RARG-900 | chr12:53220630-53220657 | RARG-1808 | chr12:53233654-53233681 |
| RARG-901 | chr12:53220633-53220660 | RARG-1809 | chr12:53233655-53233682 |
| RARG-902 | chr12:53220652-53220679 | RARG-1810 | chr12:53233660-53233687 |
| RARG-903 | chr12:53220666-53220693 | RARG-1811 | chr12:53233663-53233690 |
| RARG-904 | chr12:53220675-53220702 | RARG-1812 | chr12:53233664-53233691 |
| RARG-905 | chr12:53220687-53220714 | RARG-1813 | chr12:53233670-53233697 |
| RARG-906 | chr12:53220691-53220718 | RARG-1814 | chr12:53233677-53233704 |
| RARG-907 | chr12:53220692-53220719 | RARG-1815 | chr12:53233708-53233735 |
| RARG-908 | chr12:53220693-53220720 | | |

**Table 2G Genomic coordinates of the human UBFD1 gene**

| Target ID | Target Coordinate Range | Target ID | Target Coordinate Range |
|---|---|---|---|
| UBFD1-1 | chr16:23556209-23556236 | UBFD1-747 | chr16:23565360-23565387 |
| UBFD1-2 | chr16:23556229-23556256 | UBFD1-748 | chr16:23 5653 65-235653 92 |
| UBFD1-3 | chr16:23556231-23556258 | UBFD1-749 | chr16:23565379-23565406 |
| UBFD1-4 | chr16:23556237-23556264 | UBFD1-750 | chr16:23565380-23565407 |
| UBFD1-5 | chr16:23556238-23556265 | UBFD1-751 | chr16:23565381-23565408 |
| UBFD1-6 | chr16:23556242-23556269 | UBFD1-752 | chr16:23565386-23565413 |
| UBFD1-7 | chr16:23556243-23556270 | UBFD1-753 | chr16:23565404-23565431 |
| UBFD1-8 | chr16:23556251-23556278 | UBFD1-754 | chr16:23565411-23565438 |
| UBFD1-9 | chr16:23556271-23556298 | UBFD1-755 | chr16:23565412-23565439 |
| UBFD1-10 | chr16:23556280-23556307 | UBFD1-756 | chr16:23565434-23565461 |
| UBFD1-11 | chr16:23556281-23556308 | UBFD1-757 | chr16:23565441-23565468 |
| UBFD1-12 | chr16:23556298-23556325 | UBFD1-758 | chr16:23565450-23565477 |
| UBFD1-13 | chr16:23556299-23556326 | UBFD1-759 | chr16:23565451-23565478 |
| UBFD1-14 | chr16:23556300-23556327 | UBFD1-760 | chr16:23565477-23565504 |
| UBFD1-15 | chr16:23556320-23556347 | UBFD1-761 | chr16:23565495-23565522 |
| UBFD1-16 | chr16:23556325-23556352 | UBFD1-762 | chr16:23565503-23565530 |
| UBFD1-17 | chr16:23556328-23556355 | UBFD1-763 | chr16:23565509-23565536 |
| UBFD1-18 | chr16:23556338-23556365 | UBFD1-764 | chr16:23565544-23565571 |
| UBFD1-19 | chr16:23556344-23556371 | UBFD1-765 | chr16:23565545-23565572 |
| UBFD1-20 | chr16:23556345-23556372 | UBFD1-766 | chr16:23565556-23565583 |
| UBFD1-21 | chr16:23556355-23556382 | UBFD1-767 | chr16:23565557-23565584 |
| UBFD1-22 | chr16:23556373-23556400 | UBFD1-768 | chr16:23565566-23565593 |
| UBFD1-23 | chr16:23556377-23556404 | UBFD1-769 | chr16:23565567-23565594 |
| UBFD1-24 | chr16:23556387-23556414 | UBFD1-770 | chr16:23565573-23565600 |
| UBFD1-25 | chr16:23556395-23556422 | UBFD1-771 | chr16:23565585-23565612 |
| UBFD1-26 | chr16:23556396-23556423 | UBFD1-772 | chr16:23565600-23565627 |
| UBFD1-27 | chr16:23556398-23556425 | UBFD1-773 | chr16:23565608-23565635 |
| UBFD1-28 | chr16:23556430-23556457 | UBFD1-774 | chr16:23565611-23565638 |
| UBFD1-29 | chr16:23556439-23556466 | UBFD1-775 | chr16:23565616-23565643 |
| UBFD1-30 | chr16:23556442-23556469 | UBFD1-776 | chr16:23565637-23565664 |
| UBFD1-31 | chr16:23556445-23556472 | UBFD1-777 | chr16:23565638-23565665 |
| UBFD1-32 | chr16:23556462-23556489 | UBFD1-778 | chr16:23565644-23565671 |
| UBFD1-33 | chr16:23556463-23556490 | UBFD1-779 | chr16:23565645-23565672 |
| UBFD1-34 | chr16:23556464-23556491 | UBFD1-780 | chr16:23565646-23565673 |
| UBFD1-35 | chr16:23556468-23556495 | UBFD1-781 | chr16:23565650-23565677 |
| UBFD1-36 | chr16:23556471-23556498 | UBFD1-782 | chr16:23565651-23565678 |
| UBFD1-37 | chr16:23556474-23556501 | UBFD1-783 | chr16:23565652-23565679 |
| UBFD1-38 | chr16:23556477-23556504 | UBFD1-784 | chr16:23565653-23565680 |
| UBFD1-39 | chr16:23556478-23556505 | UBFD1-785 | chr16:23565673-23565700 |
| UBFD1-40 | chr16:23556505-23556532 | UBFD1-786 | chr16:23565674-23565701 |
| UBFD1-41 | chr16:23556508-23556535 | UBFD1-787 | chr16:23565698-23565725 |
| UBFD1-42 | chr16:23556513-23556540 | UBFD1-788 | chr16:23565703-23565730 |
| UBFD1-43 | chr16:23556514-23556541 | UBFD1-789 | chr16:23565704-23565731 |
| UBFD1-44 | chr16:23556518-23556545 | UBFD1-790 | chr16:23565705-23565732 |
| UBFD1-45 | chr16:23556519-23556546 | UBFD1-791 | chr16:23565714-23565741 |
| UBFD1-46 | chr16:23556533-23556560 | UBFD1-792 | chr16:23565722-23565749 |
| UBFD1-47 | chr16:23556541-23556568 | UBFD1-793 | chr16:23565736-23565763 |
| UBFD1-48 | chr16:23556552-23556579 | UBFD1-794 | chr16:23565750-23565777 |
| UBFD1-49 | chr16:23556581-23556608 | UBFD1-795 | chr16:23565775-23565802 |
| UBFD1-50 | chr16:23556589-23556616 | UBFD1-796 | chr16:23565780-23565807 |
| UBFD1-51 | chr16:23556601-23556628 | UBFD1-797 | chr16:23565797-23565824 |
| UBFD1-52 | chr16:23556602-23556629 | UBFD1-798 | chr16:23565817-23565844 |
| UBFD1-53 | chr16:23556603-23556630 | UBFD1-799 | chr16:23565842-23565869 |
| UBFD1-54 | chr16:23556604-23556631 | UBFD1-800 | chr16:23565845-23565872 |
| UBFD1-55 | chr16:23556607-23556634 | UBFD1-801 | chr16:23565846-23565873 |
| UBFD1-56 | chr16:23556613-23556640 | UBFD1-802 | chr16:23565850-23565877 |
| UBFD1-57 | chr16:23556616-23556643 | UBFD1-803 | chr16:23565851-23565878 |
| UBFD1-58 | chr16:23556617-23556644 | UBFD1-804 | chr16:23565852-23565879 |
| UBFD1-59 | chr16:23556629-23556656 | UBFD1-805 | chr16:23565853-23565880 |
| UBFD1-60 | chr16:23556640-23556667 | UBFD1-806 | chr16:23565854-23565881 |
| UBFD1-61 | chr16:23556644-23556671 | UBFD1-807 | chr16:23565857-23565884 |
| UBFD1-62 | chr16:23556647-23556674 | UBFD1-808 | chr16:23565858-23565885 |
| UBFD1-63 | chr16:23556652-23556679 | UBFD1-809 | chr16:23565859-23565886 |
| UBFD1-64 | chr16:23556653-23556680 | UBFD1-810 | chr16:23565860-23565887 |
| UBFD1-65 | chr16:23556657-23556684 | UBFD1-811 | chr16:23565866-23565893 |
| UBFD1-66 | chr16:23556660-23556687 | UBFD1-812 | chr16:23565867-23565894 |
| UBFD1-67 | chr16:23556670-23556697 | UBFD1-813 | chr16:23565868-23565895 |
| UBFD1-68 | chr16:23556685-23556712 | UBFD1-814 | chr16:23565869-23565896 |
| UBFD1-69 | chr16:23556697-23556724 | UBFD1-815 | chr16:23565873-23565900 |
| UBFD1-70 | chr16:23556725-23556752 | UBFD1-816 | chr16:23565874-23565901 |
| UBFD1-71 | chr16:23556731-23556758 | UBFD1-817 | chr16:23565892-23565919 |
| UBFD1-72 | chr16:23556745-23556772 | UBFD1-818 | chr16:23565901-23565928 |
| UBFD1-73 | chr16:23556758-23556785 | UBFD1-819 | chr16:23565907-23565934 |
| UBFD1-74 | chr16:23556768-23556795 | UBFD1-820 | chr16:23565908-23565935 |
| UBFD1-75 | chr16:23556769-23556796 | UBFD1-821 | chr16:23565909-23565936 |
| UBFD1-76 | chr16:23556770-23556797 | UBFD1-822 | chr16:23565924-23565951 |
| UBFD1-77 | chr16:23556775-23556802 | UBFD1-823 | chr16:23565929-23565956 |
| UBFD1-78 | chr16:23556777-23556804 | UBFD1-824 | chr16:23565932-23565959 |
| UBFD1-79 | chr16:23556782-23556809 | UBFD1-825 | chr16:23565933-23565960 |
| UBFD1-80 | chr16:23556794-23556821 | UBFD1-826 | chr16:23565934-23565961 |
| UBFD1-81 | chr16:23556795-23556822 | UBFD1-827 | chr16:23565936-23565963 |
| UBFD1-82 | chr16:23556821-23556848 | UBFD1-828 | chr16:23565939-23565966 |
| UBFD1-83 | chr16:23556852-23556879 | UBFD1-829 | chr16:23565944-23565971 |
| UBFD1-84 | chr16:23556855-23556882 | UBFD1-830 | chr16:23565945-23565972 |
| UBFD1-85 | chr16:23556870-23556897 | UBFD1-831 | chr16:23565947-23565974 |
| UBFD1-86 | chr16:23556883-23556910 | UBFD1-832 | chr16:23565950-23565977 |
| UBFD1-87 | chr16:23556884-23556911 | UBFD1-833 | chr16:23565951-23565978 |
| UBFD1-88 | chr16:23556885-23556912 | UBFD1-834 | chr16:23565967-23565994 |
| UBFD1-89 | chr16:23556889-23556916 | UBFD1-835 | chr16:23565970-23565997 |
| UBFD1-90 | chr16:23556911-23556938 | UBFD1-836 | chr16:23565971-23565998 |
| UBFD1-91 | chr16:23556912-23556939 | UBFD1-837 | chr16:23565990-23566017 |
| UBFD1-92 | chr16:23556927-23556954 | UBFD1-838 | chr16:23565998-23566025 |
| UBFD1-93 | chr16:23556935-23556962 | UBFD1-839 | chr16:23565999-23566026 |
| UBFD1-94 | chr16:23556965-23556992 | UBFD1-840 | chr16:23566007-23566034 |
| UBFD1-95 | chr16:23556966-23556993 | UBFD1-841 | chr16:23566008-23566035 |
| UBFD1-96 | chr16:23556970-23556997 | UBFD1-842 | chr16:23566009-23566036 |
| UBFD1-97 | chr16:23556995-23557022 | UBFD1-843 | chr16:23566010-23566037 |
| UBFD1-98 | chr16:23557018-23557045 | UBFD1-844 | chr16:23566020-23566047 |
| UBFD1-99 | chr16:23557038-23557065 | UBFD1-845 | chr16:23566024-23566051 |
| UBFD1-100 | chr16:23557043-23557070 | UBFD1-846 | chr16:23566028-23566055 |
| UBFD1-101 | chr16:23557047-23557074 | UBFD1-847 | chr16:23566031-23566058 |
| UBFD1-102 | chr16:23557048-23557075 | UBFD1-848 | chr16:23566042-23566069 |
| UBFD1-103 | chr16:23557049-23557076 | UBFD1-849 | chr16:23566047-23566074 |
| UBFD1-104 | chr16:23557058-23557085 | UBFD1-850 | chr16:23566058-23566085 |
| UBFD1-105 | chr16:23557061-23557088 | UBFD1-851 | chr16:23566059-23566086 |
| UBFD1-106 | chr16:23557085-23557112 | UBFD1-852 | chr16:23566060-23566087 |
| UBFD1-107 | chr16:23557091-23557118 | UBFD1-853 | chr16:23566072-23566099 |
| UBFD1-108 | chr16:23557092-23557119 | UBFD1-854 | chr16:23566123-23566150 |
| UBFD1-109 | chr16:23557095-23557122 | UBFD1-855 | chr16:23566126-23566153 |
| UBFD1-110 | chr16:23557096-23557123 | UBFD1-856 | chr16:23566134-23566161 |
| UBFD1-111 | chr16:23557098-23557125 | UBFD1-857 | chr16:23566137-23566164 |
| UBFD1-112 | chr16:23557101-23557128 | UBFD1-858 | chr16:23566138-23566165 |
| UBFD1-113 | chr16:23557102-23557129 | UBFD1-859 | chr16:23566141-23566168 |
| UBFD1-114 | chr16:23557105-23557132 | UBFD1-860 | chr16:23566145-23566172 |
| UBFD1-115 | chr16:23557106-23557133 | UBFD1-861 | chr16:23566146-23566173 |
| UBFD1-116 | chr16:23557111-23557138 | UBFD1-862 | chr16:23566147-23566174 |
| UBFD1-117 | chr16:23557112-23557139 | UBFD1-863 | chr16:23566151-23566178 |
| UBFD1-118 | chr16:23557117-23557144 | UBFD1-864 | chr16:23566152-23566179 |
| UBFD1-119 | chr16:23557120-23557147 | UBFD1-865 | chr16:23566158-23566185 |
| UBFD1-120 | chr16:23557123-23557150 | UBFD1-866 | chr16:23566159-23566186 |
| UBFD1-121 | chr16:23557124-23557151 | UBFD1-867 | chr16:23566165-23566192 |
| UBFD1-122 | chr16:23557125-23557152 | UBFD1-868 | chr16:23566169-23566196 |
| UBFD1-123 | chr16:23557128-23557155 | UBFD1-869 | chr16:23566191-23566218 |
| UBFD1-124 | chr16:23557134-23557161 | UBFD1-870 | chr16:23566205-23566232 |
| UBFD1-125 | chr16:23557135-23557162 | UBFD1-871 | chr16:23566206-23566233 |
| UBFD1-126 | chr16:23557140-23557167 | UBFD1-872 | chr16:23566214-23566241 |
| UBFD1-127 | chr16:23557141-23557168 | UBFD1-873 | chr16:23566220-23566247 |
| UBFD1-128 | chr16:23557142-23557169 | UBFD1-874 | chr16:23566221-23566248 |
| UBFD1-129 | chr16:23557143-23557170 | UBFD1-875 | chr16:23566222-23566249 |
| UBFD1-130 | chr16:23557149-23557176 | UBFD1-876 | chr16:23566223-23566250 |
| UBFD1-131 | chr16:23557171-23557198 | UBFD1-877 | chr16:23566224-23566251 |
| UBFD1-132 | chr16:23557172-23557199 | UBFD1-878 | chr16:23566225-23566252 |
| UBFD1-133 | chr16:23557173-23557200 | UBFD1-879 | chr16:23566229-23566256 |
| UBFD1-134 | chr16:23557177-23557204 | UBFD1-880 | chr16:23566230-23566257 |
| UBFD1-135 | chr16:23557183-23557210 | UBFD1-881 | chr16:23566241-23566268 |
| UBFD1-136 | chr16:23557184-23557211 | UBFD1-882 | chr16:23566247-23566274 |
| UBFD1-137 | chr16:23557185-23557212 | UBFD1-883 | chr16:23566259-23566286 |
| UBFD1-138 | chr16:23557189-23557216 | UBFD1-884 | chr16:23566275-23566302 |
| UBFD1-139 | chr16:23557190-23557217 | UBFD1-885 | chr16:23566287-23566314 |
| UBFD1-140 | chr16:23557192-23557219 | UBFD1-886 | chr16:23566288-23566315 |
| UBFD1-141 | chr16:23557195-23557222 | UBFD1-887 | chr16:23566290-23566317 |
| UBFD1-142 | chr16:23557204-23557231 | UBFD1-888 | chr16:23566291-23566318 |
| UBFD1-143 | chr16:23557219-23557246 | UBFD1-889 | chr16:23566295-23566322 |
| UBFD1-144 | chr16:23557224-23557251 | UBFD1-890 | chr16:23566296-23566323 |
| UBFD1-145 | chr16:23557233-23557260 | UBFD1-891 | chr16:23566302-23566329 |
| UBFD1-146 | chr16:23557235-23557262 | UBFD1-892 | chr16:23566311-23566338 |
| UBFD1-147 | chr16:23557237-23557264 | UBFD1-893 | chr16:23566321-23566348 |
| UBFD1-148 | chr16:23557241-23557268 | UBFD1-894 | chr16:23566322-23566349 |
| UBFD1-149 | chr16:23557242-23557269 | UBFD1-895 | chr16:23566358-23566385 |
| UBFD1-150 | chr16:23557243-23557270 | UBFD1-896 | chr16:23566373-23566400 |
| UBFD1-151 | chr16:23557255-23557282 | UBFD1-897 | chr16:23566379-23566406 |
| UBFD1-152 | chr16:23557256-23557283 | UBFD1-898 | chr16:23566393-23566420 |
| UBFD1-153 | chr16:23557257-23557284 | UBFD1-899 | chr16:23566401-23566428 |
| UBFD1-154 | chr16:23557260-23557287 | UBFD1-900 | chr16:23566402-23566429 |
| UBFD1-155 | chr16:23557265-23557292 | UBFD1-901 | chr16:23566404-23566431 |
| UBFD1-156 | chr16:23557267-23557294 | UBFD1-902 | chr16:23566428-23566455 |
| UBFD1-157 | chr16:23557272-23557299 | UBFD1-903 | chr16:23566429-23566456 |
| UBFD1-158 | chr16:23557276-23557303 | UBFD1-904 | chr16:23566448-23566475 |
| UBFD1-159 | chr16:23557277-23557304 | UBFD1-905 | chr16:23566449-23566476 |
| UBFD1-160 | chr16:23557288-23557315 | UBFD1-906 | chr16:23566450-23566477 |
| UBFD1-161 | chr16:23557295-23557322 | UBFD1-907 | chr16:23566451-23566478 |
| UBFD1-162 | chr16:23557298-23557325 | UBFD1-908 | chr16:23566452-23566479 |
| UBFD1-163 | chr16:23557305-23557332 | UBFD1-909 | chr16:23566468-23566495 |
| UBFD1-164 | chr16:23557320-23557347 | UBFD1-910 | chr16:23566469-23566496 |
| UBFD1-165 | chr16:23557321-23557348 | UBFD1-911 | chr16:23566477-23566504 |
| UBFD1-166 | chr16:23557325-23557352 | UBFD1-912 | chr16:23566480-23566507 |
| UBFD1-167 | chr16:23557326-23557353 | UBFD1-913 | chr16:23566483-23566510 |
| UBFD1-168 | chr16:23557337-23557364 | UBFD1-914 | chr16:23566496-23566523 |
| UBFD1-169 | chr16:23557339-23557366 | UBFD1-915 | chr16:23566507-23566534 |
| UBFD1-170 | chr16:23557340-23557367 | UBFD1-916 | chr16:23566508-23566535 |
| UBFD1-171 | chr16:23557362-23557389 | UBFD1-917 | chr16:23566511-23566538 |
| UBFD1-172 | chr16:23557363-23557390 | UBFD1-918 | chr16:23566514-23566541 |
| UBFD1-173 | chr16:23557364-23557391 | UBFD1-919 | chr16:23566519-23566546 |
| UBFD1-174 | chr16:23557373-23557400 | UBFD1-920 | chr16:23566525-23566552 |
| UBFD1-175 | chr16:23557374-23557401 | UBFD1-921 | chr16:23566526-23566553 |
| UBFD1-176 | chr16:23557383-23557410 | UBFD1-922 | chr16:23566527-23566554 |
| UBFD1-177 | chr16:23557386-23557413 | UBFD1-923 | chr16:23566541-23566568 |
| UBFD1-178 | chr16:23557399-23557426 | UBFD1-924 | chr16:23566546-23566573 |
| UBFD1-179 | chr16:23557403-23557430 | UBFD1-925 | chr16:23566550-23566577 |
| UBFD1-180 | chr16:23557411-23557438 | UBFD1-926 | chr16:23566560-23566587 |
| UBFD1-181 | chr16:23557414-23557441 | UBFD1-927 | chr16:23566569-23566596 |
| UBFD1-182 | chr16:23557417-23557444 | UBFD1-928 | chr16:23566571-23566598 |
| UBFD1-183 | chr16:23557423-23557450 | UBFD1-929 | chr16:23566587-23566614 |
| UBFD1-184 | chr16:23557426-23557453 | UBFD1-930 | chr16:23566588-23566615 |
| UBFD1-185 | chr16:23557432-23557459 | UBFD1-931 | chr16:23566603-23566630 |
| UBFD1-186 | chr16:23557437-23557464 | UBFD1-932 | chr16:23566606-23566633 |
| UBFD1-187 | chr16:23557438-23557465 | UBFD1-933 | chr16:23566607-23566634 |
| UBFD1-188 | chr16:23557439-23557466 | UBFD1-934 | chr16:23566610-23566637 |
| UBFD1-189 | chr16:23557454-23557481 | UBFD1-935 | chr16:23566615-23566642 |
| UBFD1-190 | chr16:23557455-23557482 | UBFD1-936 | chr16:23566616-23566643 |
| UBFD1-191 | chr16:23557456-23557483 | UBFD1-937 | chr16:23566622-23566649 |
| UBFD1-192 | chr16:23557457-23557484 | UBFD1-938 | chr16:23566623-23566650 |
| UBFD1-193 | chr16:23557461-23557488 | UBFD1-939 | chr16:23566642-23566669 |
| UBFD1-194 | chr16:23557467-23557494 | UBFD1-940 | chr16:23566652-23566679 |
| UBFD1-195 | chr16:23557470-23557497 | UBFD1-941 | chr16:23566657-23566684 |
| UBFD1-196 | chr16:23557471-23557498 | UBFD1-942 | chr16:23566660-23566687 |
| UBFD1-197 | chr16:23557476-23557503 | UBFD1-943 | chr16:23566665-23566692 |
| UBFD1-198 | chr16:23557477-23557504 | UBFD1-944 | chr16:23566670-23566697 |
| UBFD1-199 | chr16:23557478-23557505 | UBFD1-945 | chr16:23566691-23566718 |
| UBFD1-200 | chr16:23557479-23557506 | UBFD1-946 | chr16:23566692-23566719 |
| UBFD1-201 | chr16:23557480-23557507 | UBFD1-947 | chr16:23566693-23566720 |
| UBFD1-202 | chr16:23557484-23557511 | UBFD1-948 | chr16:23566714-23566741 |
| UBFD1-203 | chr16:23557485-23557512 | UBFD1-949 | chr16:23566715-23566742 |
| UBFD1-204 | chr16:23557486-23557513 | UBFD1-950 | chr16:23566735-23566762 |
| UBFD1-205 | chr16:23557487-23557514 | UBFD1-951 | chr16:23566736-23566763 |
| UBFD1-206 | chr16:23557493-23557520 | UBFD1-952 | chr16:23566745-23566772 |
| UBFD1-207 | chr16:23557495-23557522 | UBFD1-953 | chr16:23566756-23566783 |
| UBFD1-208 | chr16:23557504-23557531 | UBFD1-954 | chr16:23566762-23566789 |
| UBFD1-209 | chr16:23557505-23557532 | UBFD1-955 | chr16:23566764-23566791 |
| UBFD1-210 | chr16:23557507-23557534 | UBFD1-956 | chr16:23566767-23566794 |
| UBFD1-211 | chr16:23557516-23557543 | UBFD1-957 | chr16:23566768-23566795 |
| UBFD1-212 | chr16:23557521-23557548 | UBFD1-958 | chr16:23566775-23566802 |
| UBFD1-213 | chr16:23557536-23557563 | UBFD1-959 | chr16:23566776-23566803 |
| UBFD1-214 | chr16:23557537-23557564 | UBFD1-960 | chr16:23566785-23566812 |
| UBFD1-215 | chr16:23557539-23557566 | UBFD1-961 | chr16:23566796-23566823 |
| UBFD1-216 | chr16:23557540-23557567 | UBFD1-962 | chr16:23566797-23566824 |
| UBFD1-217 | chr16:23557545-23557572 | UBFD1-963 | chr16:23566801-23566828 |
| UBFD1-218 | chr16:23557546-23557573 | UBFD1-964 | chr16:23566803-23566830 |
| UBFD1-219 | chr16:23557549-23557576 | UBFD1-965 | chr16:23566804-23566831 |
| UBFD1-220 | chr16:23557550-23557577 | UBFD1-966 | chr16:23566814-23566841 |
| UBFD1-221 | chr16:23557551-23557578 | UBFD1-967 | chr16:23566815-23566842 |
| UBFD1-222 | chr16:23557552-23557579 | UBFD1-968 | chr16:23566832-23566859 |
| UBFD1-223 | chr16:23557556-23557583 | UBFD1-969 | chr16:23566833-23566860 |
| UBFD1-224 | chr16:23557557-23557584 | UBFD1-970 | chr16:23566837-23566864 |
| UBFD1-225 | chr16:23557558-23557585 | UBFD1-971 | chr16:23566838-23566865 |
| UBFD1-226 | chr16:23557562-23557589 | UBFD1-972 | chr16:23566851-23566878 |
| UBFD1-227 | chr16:23557568-23557595 | UBFD1-973 | chr16:23566852-23566879 |
| UBFD1-228 | chr16:23557569-23557596 | UBFD1-974 | chr16:23566853-23566880 |
| UBFD1-229 | chr16:23557574-23557601 | UBFD1-975 | chr16:23566868-23566895 |
| UBFD1-230 | chr16:23557577-23557604 | UBFD1-976 | chr16:23566890-23566917 |
| UBFD1-231 | chr16:23557580-23557607 | UBFD1-977 | chr16:23566891-23566918 |
| UBFD1-232 | chr16:23557583-23557610 | UBFD1-978 | chr16:23566893-23566920 |
| UBFD1-233 | chr16:23557584-23557611 | UBFD1-979 | chr16:23566894-23566921 |
| UBFD1-234 | chr16:23557586-23557613 | UBFD1-980 | chr16:23566900-23566927 |
| UBFD1-235 | chr16:23557591-23557618 | UBFD1-981 | chr16:23566903-23566930 |
| UBFD1-236 | chr16:23557613-23557640 | UBFD1-982 | chr16:23566907-23566934 |
| UBFD1-237 | chr16:23557615-23557642 | UBFD1-983 | chr16:23566908-23566935 |
| UBFD1-238 | chr16:23557617-23557644 | UBFD1-984 | chr16:23566919-23566946 |
| UBFD1-239 | chr16:23557618-23557645 | UBFD1-985 | chr16:23566922-23566949 |
| UBFD1-240 | chr16:23557624-23557651 | UBFD1-986 | chr16:23566923-23566950 |
| UBFD1-241 | chr16:23557627-23557654 | UBFD1-987 | chr16:23566951-23566978 |
| UBFD1-242 | chr16:23557630-23557657 | UBFD1-988 | chr16:23566952-23566979 |
| UBFD1-243 | chr16:23557633-23557660 | UBFD1-989 | chr16:23566964-23566991 |
| UBFD1-244 | chr16:23557634-23557661 | UBFD1-990 | chr16:23566973-23567000 |
| UBFD1-245 | chr16:23557635-23557662 | UBFD1-991 | chr16:23566974-23567001 |
| UBFD1-246 | chr16:23557637-23557664 | UBFD1-992 | chr16:23566983-23567010 |
| UBFD1-247 | chr16:23557641-23557668 | UBFD1-993 | chr16:23566984-23567011 |
| UBFD1-248 | chr16:23557644-23557671 | UBFD1-994 | chr16:23567001-23567028 |
| UBFD1-249 | chr16:23557645-23557672 | UBFD1-995 | chr16:23567004-23567031 |
| UBFD1-250 | chr16:23557646-23557673 | UBFD1-996 | chr16:23567005-23567032 |
| UBFD1-251 | chr16:23557647-23557674 | UBFD1-997 | chr16:23567014-23567041 |
| UBFD1-252 | chr16:23557675-23557702 | UBFD1-998 | chr16:23567020-23567047 |
| UBFD1-253 | chr16:23557679-23557706 | UBFD1-999 | chr16:23567037-23567064 |
| UBFD1-254 | chr16:23557685-23557712 | UBFD1-1000 | chr16:23567043-23567070 |
| UBFD1-255 | chr16:23557688-23557715 | UBFD1-1001 | chr16:23567056-23567083 |
| UBFD1-256 | chr16:23557689-23557716 | UBFD1-1002 | chr16:23567060-23567087 |
| UBFD1-257 | chr16:23557693-23557720 | UBFD1-1003 | chr16:23567094-23567121 |
| UBFD1-258 | chr16:23557694-23557721 | UBFD1-1004 | chr16:23567095-23567122 |
| UBFD1-259 | chr16:23557697-23557724 | UBFD1-1005 | chr16:23567096-23567123 |
| UBFD1-260 | chr16:23557698-23557725 | UBFD1-1006 | chr16:23567100-23567127 |
| UBFD1-261 | chr16:23557699-23557726 | UBFD1-1007 | chr16:23567101-23567128 |
| UBFD1-262 | chr16:23557700-23557727 | UBFD1-1008 | chr16:23567110-23567137 |
| UBFD1-263 | chr16:23557707-23557734 | UBFD1-1009 | chr16:23567111-23567138 |
| UBFD1-264 | chr16:23557719-23557746 | UBFD1-1010 | chr16:23567119-23567146 |
| UBFD1-265 | chr16:23557722-23557749 | UBFD1-1011 | chr16:23567129-23567156 |
| UBFD1-266 | chr16:23557725-23557752 | UBFD1-1012 | chr16:23570377-23570404 |
| UBFD1-267 | chr16:23557729-23557756 | UBFD1-1013 | chr16:23570378-23570405 |
| UBFD1-268 | chr16:23557730-23557757 | UBFD1-1014 | chr16:23570398-23570425 |
| UBFD1-269 | chr16:23557731-23557758 | UBFD1-1015 | chr16:23570399-23570426 |
| UBFD1-270 | chr16:23557737-23557764 | UBFD1-1016 | chr16:23570403-23570430 |
| UBFD1-271 | chr16:23557741-23557768 | UBFD1-1017 | chr16:23570404-23570431 |
| UBFD1-272 | chr16:23557750-23557777 | UBFD1-1018 | chr16:23570405-23570432 |
| UBFD1-273 | chr16:23557753-23557780 | UBFD1-1019 | chr16:23570409-23570436 |
| UBFD1-274 | chr16:23557754-23557781 | UBFD1-1020 | chr16:23570410-23570437 |
| UBFD1-275 | chr16:23557755-23557782 | UBFD1-1021 | chr16:23570420-23570447 |
| UBFD1-276 | chr16:23557756-23557783 | UBFD1-1022 | chr16:23570424-23570451 |
| UBFD1-277 | chr16:23557759-23557786 | UBFD1-1023 | chr16:23570432-23570459 |
| UBFD1-278 | chr16:23557760-23557787 | UBFD1-1024 | chr16:23570436-23570463 |
| UBFD1-279 | chr16:23557761-23557788 | UBFD1-1025 | chr16:23570437-23570464 |
| UBFD1-280 | chr16:23557762-23557789 | UBFD1-1026 | chr16:23570441-23570468 |
| UBFD1-281 | chr16:23557763-23557790 | UBFD1-1027 | chr16:23570452-23570479 |
| UBFD1-282 | chr16:23557769-23557796 | UBFD1-1028 | chr16:23570453-23570480 |
| UBFD1-283 | chr16:23557770-23557797 | UBFD1-1029 | chr16:23570464-23570491 |
| UBFD1-284 | chr16:23557774-23557801 | UBFD1-1030 | chr16:23570465-23570492 |
| UBFD1-285 | chr16:23557775-23557802 | UBFD1-1031 | chr16:23570466-23570493 |
| UBFD1-286 | chr16:23557776-23557803 | UBFD1-1032 | chr16:23570471-23570498 |
| UBFD1-287 | chr16:23557782-23557809 | UBFD1-1033 | chr16:23570472-23570499 |
| UBFD1-288 | chr16:23557788-23557815 | UBFD1-1034 | chr16:23570474-23570501 |
| UBFD1-289 | chr16:23557793-23557820 | UBFD1-1035 | chr16:23570476-23570503 |
| UBFD1-290 | chr16:23557798-23557825 | UBFD1-1036 | chr16:23570491-23570518 |
| UBFD1-291 | chr16:23557800-23557827 | UBFD1-1037 | chr16:23570492-23570519 |
| UBFD1-292 | chr16:23557801-23557828 | UBFD1-1038 | chr16:23570494-23570521 |
| UBFD1-293 | chr16:23557802-23557829 | UBFD1-1039 | chr16:23570495-23570522 |
| UBFD1-294 | chr16:23557803-23557830 | UBFD1-1040 | chr16:23570496-23570523 |
| UBFD1-295 | chr16:23557809-23557836 | UBFD1-1041 | chr16:23570500-23570527 |
| UBFD1-296 | chr16:23557814-23557841 | UBFD1-1042 | chr16:23570501-23570528 |
| UBFD1-297 | chr16:23557819-23557846 | UBFD1-1043 | chr16:23570505-23570532 |
| UBFD1-298 | chr16:23557820-23557847 | UBFD1-1044 | chr16:23570519-23570546 |
| UBFD1-299 | chr16:23557821-23557848 | UBFD1-1045 | chr16:23570531-23570558 |
| UBFD1-300 | chr16:23557823-23557850 | UBFD1-1046 | chr16:23570543-23570570 |
| UBFD1-301 | chr16:23557824-23557851 | UBFD1-1047 | chr16:23570544-23570571 |
| UBFD1-302 | chr16:23557834-23557861 | UBFD1-1048 | chr16:23570545-23570572 |
| UBFD1-303 | chr16:23557837-23557864 | UBFD1-1049 | chr16:23570551-23570578 |
| UBFD1-304 | chr16:23557838-23557865 | UBFD1-1050 | chr16:23570581-23570608 |
| UBFD1-305 | chr16:23557839-23557866 | UBFD1-1051 | chr16:23570587-23570614 |
| UBFD1-306 | chr16:23557840-23557867 | UBFD1-1052 | chr16:23570602-23570629 |
| UBFD1-307 | chr16:23557842-23557869 | UBFD1-1053 | chr16:23570607-23570634 |
| UBFD1-308 | chr16:23557843-23557870 | UBFD1-1054 | chr16:23570609-23570636 |
| UBFD1-309 | chr16:23557845-23557872 | UBFD1-1055 | chr16:23570610-23570637 |
| UBFD1-310 | chr16:23557848-23557875 | UBFD1-1056 | chr16:23570617-23570644 |
| UBFD1-311 | chr16:23557853-23557880 | UBFD1-1057 | chr16:23570618-23570645 |
| UBFD1-312 | chr16:23557856-23557883 | UBFD1-1058 | chr16:23570622-23570649 |
| UBFD1-313 | chr16:23557863-23557890 | UBFD1-1059 | chr16:23570623-23570650 |
| UBFD1-314 | chr16:23557877-23557904 | UBFD1-1060 | chr16:23570639-23570666 |
| UBFD1-315 | chr16:23557886-23557913 | UBFD1-1061 | chr16:23570641-23570668 |
| UBFD1-316 | chr16:23557887-23557914 | UBFD1-1062 | chr16:23570651-23570678 |
| UBFD1-317 | chr16:23557893-23557920 | UBFD1-1063 | chr16:23570654-23570681 |
| UBFD1-318 | chr16:23557898-23557925 | UBFD1-1064 | chr16:23570661-23570688 |
| UBFD1-319 | chr16:23557903-23557930 | UBFD1-1065 | chr16:23570662-23570689 |
| UBFD1-320 | chr16:23557904-23557931 | UBFD1-1066 | chr16:23570690-23570717 |
| UBFD1-321 | chr16:23557909-23557936 | UBFD1-1067 | chr16:23570698-23570725 |
| UBFD1-322 | chr16:23557910-23557937 | UBFD1-1068 | chr16:23570714-23570741 |
| UBFD1-323 | chr16:23557914-23557941 | UBFD1-1069 | chr16:23570735-23570762 |
| UBFD1-324 | chr16:23557915-23557942 | UBFD1-1070 | chr16:23570755-23570782 |
| UBFD1-325 | chr16:23557923-23557950 | UBFD1-1071 | chr16:23570783-23570810 |
| UBFD1-326 | chr16:23557927-23557954 | UBFD1-1072 | chr16:23570789-23570816 |
| UBFD1-327 | chr16:23557928-23557955 | UBFD1-1073 | chr16:23570790-23570817 |
| UBFD1-328 | chr16:23557931-23557958 | UBFD1-1074 | chr16:23570809-23570836 |
| UBFD1-329 | chr16:23557932-23557959 | UBFD1-1075 | chr16:23570813-23570840 |
| UBFD1-330 | chr16:23557933-23557960 | UBFD1-1076 | chr16:23570845-23570872 |
| UBFD1-331 | chr16:23557934-23557961 | UBFD1-1077 | chr16:23570856-23570883 |
| UBFD1-332 | chr16:23557938-23557965 | UBFD1-1078 | chr16:23570895-23570922 |
| UBFD1-333 | chr16:23557939-23557966 | UBFD1-1079 | chr16:23570896-23570923 |
| UBFD1-334 | chr16:23557940-23557967 | UBFD1-1080 | chr16:23570905-23570932 |
| UBFD1-335 | chr16:23557941-23557968 | UBFD1-1081 | chr16:23570969-23570996 |
| UBFD1-336 | chr16:23557942-23557969 | UBFD1-1082 | chr16:23570983-23571010 |
| UBFD1-337 | chr16:23557943-23557970 | UBFD1-1083 | chr16:23571003-23571030 |
| UBFD1-338 | chr16:23557949-23557976 | UBFD1-1084 | chr16:23571004-23571031 |
| UBFD1-339 | chr16:23557952-23557979 | UBFD1-1085 | chr16:23571005-23571032 |
| UBFD1-340 | chr16:23557961-23557988 | UBFD1-1086 | chr16:23571045-23571072 |
| UBFD1-341 | chr16:23557964-23557991 | UBFD1-1087 | chr16:23571048-23571075 |
| UBFD1-342 | chr16:23557973-23558000 | UBFD1-1088 | chr16:23571049-23571076 |
| UBFD1-343 | chr16:23557980-23558007 | UBFD1-1089 | chr16:23571050-23571077 |
| UBFD1-344 | chr16:23557984-23558011 | UBFD1-1090 | chr16:23571051-23571078 |
| UBFD1-345 | chr16:23558000-23558027 | UBFD1-1091 | chr16:23571054-23571081 |
| UBFD1-346 | chr16:23558003-23558030 | UBFD1-1092 | chr16:23571055-23571082 |
| UBFD1-347 | chr16:23558004-23558031 | UBFD1-1093 | chr16:23571091-23571118 |
| UBFD1-348 | chr16:23558012-23558039 | UBFD1-1094 | chr16:23571105-23571132 |
| UBFD1-349 | chr16:23558013-23558040 | UBFD1-1095 | chr16:23571129-23571156 |
| UBFD1-350 | chr16:23558015-23558042 | UBFD1-1096 | chr16:23571130-23571157 |
| UBFD1-351 | chr16:23558018-23558045 | UBFD1-1097 | chr16:23571150-23571177 |
| UBFD1-352 | chr16:23558019-23558046 | UBFD1-1098 | chr16:23571175-23571202 |
| UBFD1-353 | chr16:23558020-23558047 | UBFD1-1099 | chr16:23571177-23571204 |
| UBFD1-354 | chr16:23558021-23558048 | UBFD1-1100 | chr16:23571178-23571205 |
| UBFD1-355 | chr16:23558023-23558050 | UBFD1-1101 | chr16:23571201-23571228 |
| UBFD1-356 | chr16:23558024-23558051 | UBFD1-1102 | chr16:23571202-23571229 |
| UBFD1-357 | chr16:23558027-23558054 | UBFD1-1103 | chr16:23571205-23571232 |
| UBFD1-358 | chr16:23558033-23558060 | UBFD1-1104 | chr16:23571219-23571246 |
| UBFD1-359 | chr16:23558037-23558064 | UBFD1-1105 | chr16:23571224-23571251 |
| UBFD1-360 | chr16:23558040-23558067 | UBFD1-1106 | chr16:23571232-23571259 |
| UBFD1-361 | chr16:23558052-23558079 | UBFD1-1107 | chr16:23571233-23571260 |
| UBFD1-362 | chr16:23558055-23558082 | UBFD1-1108 | chr16:23571236-23571263 |
| UBFD1-363 | chr16:23558064-23558091 | UBFD1-1109 | chr16:23571237-23571264 |
| UBFD1-364 | chr16:23558066-23558093 | UBFD1-1110 | chr16:23571240-23571267 |
| UBFD1-365 | chr16:23558070-23558097 | UBFD1-1111 | chr16:23571241-23571268 |
| UBFD1-366 | chr16:23558072-23558099 | UBFD1-1112 | chr16:23571244-23571271 |
| UBFD1-367 | chr16:23558083-23558110 | UBFD1-1113 | chr16:23571245-23571272 |
| UBFD1-368 | chr16:23558085-23558112 | UBFD1-1114 | chr16:23571250-23571277 |
| UBFD1-369 | chr16:23558086-23558113 | UBFD1-1115 | chr16:23571254-23571281 |
| UBFD1-370 | chr16:23558087-23558114 | UBFD1-1116 | chr16:23571269-23571296 |
| UBFD1-371 | chr16:23558090-23558117 | UBFD1-1117 | chr16:23571270-23571297 |
| UBFD1-372 | chr16:23558094-23558121 | UBFD1-1118 | chr16:23571275-23571302 |
| UBFD1-373 | chr16:23558095-23558122 | UBFD1-1119 | chr16:23571295-23571322 |
| UBFD1-374 | chr16:23558096-23558123 | UBFD1-1120 | chr16:23571347-23571374 |
| UBFD1-375 | chr16:23558100-23558127 | UBFD1-1121 | chr16:23571348-23571375 |
| UBFD1-376 | chr16:23558101-23558128 | UBFD1-1122 | chr16:23571354-23571381 |
| UBFD1-377 | chr16:23558102-23558129 | UBFD1-1123 | chr16:23571355-23571382 |
| UBFD1-378 | chr16:23558105-23558132 | UBFD1-1124 | chr16:23571357-23571384 |
| UBFD1-379 | chr16:23558106-23558133 | UBFD1-1125 | chr16:23571370-23571397 |
| UBFD1-380 | chr16:23558107-23558134 | UBFD1-1126 | chr16:23571374-23571401 |
| UBFD1-381 | chr16:23558108-23558135 | UBFD1-1127 | chr16:23571375-23571402 |
| UBFD1-382 | chr16:23558116-23558143 | UBFD1-1128 | chr16:23571383-23571410 |
| UBFD1-383 | chr16:23558117-23558144 | UBFD1-1129 | chr16:23571384-23571411 |
| UBFD1-384 | chr16:23558118-23558145 | UBFD1-1130 | chr16:23571385-23571412 |
| UBFD1-385 | chr16:23558124-23558151 | UBFD1-1131 | chr16:23571386-23571413 |
| UBFD1-386 | chr16:23558125-23558152 | UBFD1-1132 | chr16:23571388-23571415 |
| UBFD1-387 | chr16:23558129-23558156 | UBFD1-1133 | chr16:23571392-23571419 |
| UBFD1-388 | chr16:23558130-23558157 | UBFD1-1134 | chr16:23571402-23571429 |
| UBFD1-389 | chr16:23558133-23558160 | UBFD1-1135 | chr16:23571418-23571445 |
| UBFD1-390 | chr16:23558136-23558163 | UBFD1-1136 | chr16:23571426-23571453 |
| UBFD1-391 | chr16:23558141-23558168 | UBFD1-1137 | chr16:23571429-23571456 |
| UBFD1-392 | chr16:23558142-23558169 | UBFD1-1138 | chr16:23571430-23571457 |
| UBFD1-393 | chr16:23558146-23558173 | UBFD1-1139 | chr16:23571431-23571458 |
| UBFD1-394 | chr16:23558147-23558174 | UBFD1-1140 | chr16:23571436-23571463 |
| UBFD1-395 | chr16:23558153-23558180 | UBFD1-1141 | chr16:23571441-23571468 |
| UBFD1-396 | chr16:23558156-23558183 | UBFD1-1142 | chr16:23571446-23571473 |
| UBFD1-397 | chr16:23558173-23558200 | UBFD1-1143 | chr16:23571451-23571478 |
| UBFD1-398 | chr16:23558207-23558234 | UBFD1-1144 | chr16:23571452-23571479 |
| UBFD1-399 | chr16:23558208-23558235 | UBFD1-1145 | chr16:23571454-23571481 |
| UBFD1-400 | chr16:23558217-23558244 | UBFD1-1146 | chr16:23571455-23571482 |
| UBFD1-401 | chr16:23558227-23558254 | UBFD1-1147 | chr16:23571459-23571486 |
| UBFD1-402 | chr16:23558228-23558255 | UBFD1-1148 | chr16:23571474-23571501 |
| UBFD1-403 | chr16:23558229-23558256 | UBFD1-1149 | chr16:23571485-23571512 |
| UBFD1-404 | chr16:23558230-23558257 | UBFD1-1150 | chr16:23571496-23571523 |
| UBFD1-405 | chr16:23558247-23558274 | UBFD1-1151 | chr16:23571497-23571524 |
| UBFD1-406 | chr16:23558253-23558280 | UBFD1-1152 | chr16:23571498-23571525 |
| UBFD1-407 | chr16:23558265-23558292 | UBFD1-1153 | chr16:23571503-23571530 |
| UBFD1-408 | chr16:23558266-23558293 | UBFD1-1154 | chr16:23571504-23571531 |
| UBFD1-409 | chr16:23558286-23558313 | UBFD1-1155 | chr16:23571505-23571532 |
| UBFD1-410 | chr16:23558302-23558329 | UBFD1-1156 | chr16:23571506-23571533 |
| UBFD1-411 | chr16:23558303-23558330 | UBFD1-1157 | chr16:23571513-23571540 |
| UBFD1-412 | chr16:23558310-23558337 | UBFD1-1158 | chr16:23571520-23571547 |
| UBFD1-413 | chr16:23558311-23558338 | UBFD1-1159 | chr16:23571526-23571553 |
| UBFD1-414 | chr16:23558312-23558339 | UBFD1-1160 | chr16:23571536-23571563 |
| UBFD1-415 | chr16:23558315-23558342 | UBFD1-1161 | chr16:23571559-23571586 |
| UBFD1-416 | chr16:23558316-23558343 | UBFD1-1162 | chr16:23571569-23571596 |
| UBFD1-417 | chr16:23558317-23558344 | UBFD1-1163 | chr16:23571575-23571602 |
| UBFD1-418 | chr16:23558320-23558347 | UBFD1-1164 | chr16:23571583-23571610 |
| UBFD1-419 | chr16:23558321-23558348 | UBFD1-1165 | chr16:23571601-23571628 |
| UBFD1-420 | chr16:23558323-23558350 | UBFD1-1166 | chr16:23571623-23571650 |
| UBFD1-421 | chr16:23558328-23558355 | UBFD1-1167 | chr16:23571628-23571655 |
| UBFD1-422 | chr16:23558331-23558358 | UBFD1-1168 | chr16:23571629-23571656 |
| UBFD1-423 | chr16:23558332-23558359 | UBFD1-1169 | chr16:23571649-23571676 |
| UBFD1-424 | chr16:23558339-23558366 | UBFD1-1170 | chr16:23571658-23571685 |
| UBFD1-425 | chr16:23558340-23558367 | UBFD1-1171 | chr16:23571672-23571699 |
| UBFD1-426 | chr16:23558347-23558374 | UBFD1-1172 | chr16:23571687-23571714 |
| UBFD1-427 | chr16:23558351-23558378 | UBFD1-1173 | chr16:23571694-23571721 |
| UBFD1-428 | chr16:23558363-23558390 | UBFD1-1174 | chr16:23571695-23571722 |
| UBFD1-429 | chr16:23558376-23558403 | UBFD1-1175 | chr16:23571713-23571740 |
| UBFD1-430 | chr16:23558377-23558404 | UBFD1-1176 | chr16:23571717-23571744 |
| UBFD1-431 | chr16:23558378-23558405 | UBFD1-1177 | chr16:23571721-23571748 |
| UBFD1-432 | chr16:23558379-23558406 | UBFD1-1178 | chr16:23571735-23571762 |
| UBFD1-433 | chr16:23559357-23559384 | UBFD1-1179 | chr16:23571736-23571763 |
| UBFD1-434 | chr16:23559392-23559419 | UBFD1-1180 | chr16:23571747-23571774 |
| UBFD1-435 | chr16:23559411-23559438 | UBFD1-1181 | chr16:23571748-23571775 |
| UBFD1-436 | chr16:23559427-23559454 | UBFD1-1182 | chr16:23571749-23571776 |
| UBFD1-437 | chr16:23559431-23559458 | UBFD1-1183 | chr16:23571784-23571811 |
| UBFD1-438 | chr16:23559435-23559462 | UBFD1-1184 | chr16:23571801-23571828 |
| UBFD1-439 | chr16:23559441-23559468 | UBFD1-1185 | chr16:23571818-23571845 |
| UBFD1-440 | chr16:23559446-23559473 | UBFD1-1186 | chr16:23571819-23571846 |
| UBFD1-441 | chr16:23559449-23559476 | UBFD1-1187 | chr16:23571835-23571862 |
| UBFD1-442 | chr16:23559456-23559483 | UBFD1-1188 | chr16:23571840-23571867 |
| UBFD1-443 | chr16:23559462-23559489 | UBFD1-1189 | chr16:23571854-23571881 |
| UBFD1-444 | chr16:23559463-23559490 | UBFD1-1190 | chr16:23571855-23571882 |
| UBFD1-445 | chr16:23559472-23559499 | UBFD1-1191 | chr16:23571857-23571884 |
| UBFD1-446 | chr16:23559473-23559500 | UBFD1-1192 | chr16:23571858-23571885 |
| UBFD1-447 | chr16:23559476-23559503 | UBFD1-1193 | chr16:23571870-23571897 |
| UBFD1-448 | chr16:23559477-23559504 | UBFD1-1194 | chr16:23571871-23571898 |
| UBFD1-449 | chr16:23559480-23559507 | UBFD1-1195 | chr16:23571936-23571963 |
| UBFD1-450 | chr16:23559491-23559518 | UBFD1-1196 | chr16:23571937-23571964 |
| UBFD1-451 | chr16:23559511-23559538 | UBFD1-1197 | chr16:23571943-23571970 |
| UBFD1-452 | chr16:23559512-23559539 | UBFD1-1198 | chr16:23571953-23571980 |
| UBFD1-453 | chr16:23559513-23559540 | UBFD1-1199 | chr16:23571957-23571984 |
| UBFD1-454 | chr16:23559525-23559552 | UBFD1-1200 | chr16:23571962-23571989 |
| UBFD1-455 | chr16:23559526-23559553 | UBFD1-1201 | chr16:23571991-23572018 |
| UBFD1-456 | chr16:23559527-23559554 | UBFD1-1202 | chr16:23571999-23572026 |
| UBFD1-457 | chr16:23559539-23559566 | UBFD1-1203 | chr16:23572001-23572028 |
| UBFD1-458 | chr16:23559542-23559569 | UBFD1-1204 | chr16:23572033-23572060 |
| UBFD1-459 | chr16:23559546-23559573 | UBFD1-1205 | chr16:23572034-23572061 |
| UBFD1-460 | chr16:23559555-23559582 | UBFD1-1206 | chr16:23572035-23572062 |
| UBFD1-461 | chr16:23559576-23559603 | UBFD1-1207 | chr16:23572068-23572095 |
| UBFD1-462 | chr16:23559579-23559606 | UBFD1-1208 | chr16:23572069-23572096 |
| UBFD1-463 | chr16:23559602-23559629 | UBFD1-1209 | chr16:23572083-23572110 |
| UBFD1-464 | chr16:23559608-23559635 | UBFD1-1210 | chr16:23572092-23572119 |
| UBFD1-465 | chr16:23559609-23559636 | UBFD1-1211 | chr16:23572114-23572141 |
| UBFD1-466 | chr16:23559611-23559638 | UBFD1-1212 | chr16:23572115-23572142 |
| UBFD1-467 | chr16:23559629-23559656 | UBFD1-1213 | chr16:23572116-23572143 |
| UBFD1-468 | chr16:23559639-23559666 | UBFD1-1214 | chr16:23572120-23572147 |
| UBFD1-469 | chr16:23559643-23559670 | UBFD1-1215 | chr16:23572121-23572148 |
| UBFD1-470 | chr16:23559659-23559686 | UBFD1-1216 | chr16:23572145-23572172 |
| UBFD1-471 | chr16:23559673-23559700 | UBFD1-1217 | chr16:23572149-23572176 |
| UBFD1-472 | chr16:23559683-23559710 | UBFD1-1218 | chr16:23572150-23572177 |
| UBFD1-473 | chr16:23559690-23559717 | UBFD1-1219 | chr16:23572151-23572178 |
| UBFD1-474 | chr16:23559711-23559738 | UBFD1-1220 | chr16:23572173-23572200 |
| UBFD1-475 | chr16:23559715-23559742 | UBFD1-1221 | chr16:23572180-23572207 |
| UBFD1-476 | chr16:23559724-23559751 | UBFD1-1222 | chr16:23572184-23572211 |
| UBFD1-477 | chr16:23559733-23559760 | UBFD1-1223 | chr16:23572190-23572217 |
| UBFD1-478 | chr16:23559747-23559774 | UBFD1-1224 | chr16:23572208-23572235 |
| UBFD1-479 | chr16:23559752-23559779 | UBFD1-1225 | chr16:23572209-23572236 |
| UBFD1-480 | chr16:23559753-23559780 | UBFD1-1226 | chr16:23572210-23572237 |
| UBFD1-481 | chr16:23559763-23559790 | UBFD1-1227 | chr16:23572211-23572238 |
| UBFD1-482 | chr16:23559766-23559793 | UBFD1-1228 | chr16:23572267-23572294 |
| UBFD1-483 | chr16:23559773-23559800 | UBFD1-1229 | chr16:23572276-23572303 |
| UBFD1-484 | chr16:23559781-23559808 | UBFD1-1230 | chr16:23572278-23572305 |
| UBFD1-485 | chr16:23559782-23559809 | UBFD1-1231 | chr16:23572291-23572318 |
| UBFD1-486 | chr16:23559783-23559810 | UBFD1-1232 | chr16:23572292-23572319 |
| UBFD1-487 | chr16:23559784-23559811 | UBFD1-1233 | chr16:23572308-23572335 |
| UBFD1-488 | chr16:23559797-23559824 | UBFD1-1234 | chr16:23572322-23572349 |
| UBFD1-489 | chr16:23559800-23559827 | UBFD1-1235 | chr16:23572323-23572350 |
| UBFD1-490 | chr16:23559814-23559841 | UBFD1-1236 | chr16:23572324-23572351 |
| UBFD1-491 | chr16:23559837-23559864 | UBFD1-1237 | chr16:23572327-23572354 |
| UBFD1-492 | chr16:23559838-23559865 | UBFD1-1238 | chr16:23572336-23572363 |
| UBFD1-493 | chr16:23559841-23559868 | UBFD1-1239 | chr16:23572345-23572372 |
| UBFD1-494 | chr16:23559842-23559869 | UBFD1-1240 | chr16:23572368-23572395 |
| UBFD1-495 | chr16:23559872-23559899 | UBFD1-1241 | chr16:23572375-23572402 |
| UBFD1-496 | chr16:23559884-23559911 | UBFD1-1242 | chr16:23572377-23572404 |
| UBFD1-497 | chr16:23559889-23559916 | UBFD1-1243 | chr16:23572381-23572408 |
| UBFD1-498 | chr16:23559890-23559917 | UBFD1-1244 | chr16:23572382-23572409 |
| UBFD1-499 | chr16:23559891-23559918 | UBFD1-1245 | chr16:23572383-23572410 |
| UBFD1-500 | chr16:23559894-23559921 | UBFD1-1246 | chr16:23572384-23572411 |
| UBFD1-501 | chr16:23559906-23559933 | UBFD1-1247 | chr16:23572417-23572444 |
| UBFD1-502 | chr16:23559916-23559943 | UBFD1-1248 | chr16:23572418-23572445 |
| UBFD1-503 | chr16:23559928-23559955 | UBFD1-1249 | chr16:23572456-23572483 |
| UBFD1-504 | chr16:23559929-23559956 | UBFD1-1250 | chr16:23572457-23572484 |
| UBFD1-505 | chr16:23562093-23562120 | UBFD1-1251 | chr16:23572458-23572485 |
| UBFD1-506 | chr16:23562107-23562134 | UBFD1-1252 | chr16:23572469-23572496 |
| UBFD1-507 | chr16:23562117-23562144 | UBFD1-1253 | chr16:23572475-23572502 |
| UBFD1-508 | chr16:23562118-23562145 | UBFD1-1254 | chr16:23572476-23572503 |
| UBFD1-509 | chr16:23562131-23562158 | UBFD1-1255 | chr16:23572477-23572504 |
| UBFD1-510 | chr16:23562187-23562214 | UBFD1-1256 | chr16:23572482-23572509 |
| UBFD1-511 | chr16:23562197-23562224 | UBFD1-1257 | chr16:23572495-23572522 |
| UBFD1-512 | chr16:23562204-23562231 | UBFD1-1258 | chr16:23572505-23572532 |
| UBFD1-513 | chr16:23562236-23562263 | UBFD1-1259 | chr16:23572509-23572536 |
| UBFD1-514 | chr16:23562237-23562264 | UBFD1-1260 | chr16:23572512-23572539 |
| UBFD1-515 | chr16:23562238-23562265 | UBFD1-1261 | chr16:23572534-23572561 |
| UBFD1-516 | chr16:23562239-23562266 | UBFD1-1262 | chr16:23572542-23572569 |
| UBFD1-517 | chr16:23562245-23562272 | UBFD1-1263 | chr16:23572546-23572573 |
| UBFD1-518 | chr16:23562250-23562277 | UBFD1-1264 | chr16:23572547-23572574 |
| UBFD1-519 | chr16:23562251-23562278 | UBFD1-1265 | chr16:23572548-23572575 |
| UBFD1-520 | chr16:23562253-23562280 | UBFD1-1266 | chr16:23572557-23572584 |
| UBFD1-521 | chr16:23562267-23562294 | UBFD1-1267 | chr16:23572569-23572596 |
| UBFD1-522 | chr16:23562268-23562295 | UBFD1-1268 | chr16:23572575-23572602 |
| UBFD1-523 | chr16:23562282-23562309 | UBFD1-1269 | chr16:23572576-23572603 |
| UBFD1-524 | chr16:23562305-23562332 | UBFD1-1270 | chr16:23572577-23572604 |
| UBFD1-525 | chr16:23562313-23562340 | UBFD1-1271 | chr16:23572580-23572607 |
| UBFD1-526 | chr16:23562314-23562341 | UBFD1-1272 | chr16:23572583-23572610 |
| UBFD1-527 | chr16:23562315-23562342 | UBFD1-1273 | chr16:23572585-23572612 |
| UBFD1-528 | chr16:23562318-23562345 | UBFD1-1274 | chr16:23572589-23572616 |
| UBFD1-529 | chr16:23562335-23562362 | UBFD1-1275 | chr16:23572590-23572617 |
| UBFD1-530 | chr16:23562338-23562365 | UBFD1-1276 | chr16:23572591-23572618 |
| UBFD1-531 | chr16:23562343-23562370 | UBFD1-1277 | chr16:23572593-23572620 |
| UBFD1-532 | chr16:23562348-23562375 | UBFD1-1278 | chr16:23572594-23572621 |
| UBFD1-533 | chr16:23562349-23562376 | UBFD1-1279 | chr16:23572599-23572626 |
| UBFD1-534 | chr16:23562362-23562389 | UBFD1-1280 | chr16:23572600-23572627 |
| UBFD1-535 | chr16:23562363-23562390 | UBFD1-1281 | chr16:23572601-23572628 |
| UBFD1-536 | chr16:23562498-23562525 | UBFD1-1282 | chr16:23572602-23572629 |
| UBFD1-537 | chr16:23562510-23562537 | UBFD1-1283 | chr16:23572617-23572644 |
| UBFD1-538 | chr16:23562511-23562538 | UBFD1-1284 | chr16:23572618-23572645 |
| UBFD1-539 | chr16:23562513-23562540 | UBFD1-1285 | chr16:23572622-23572649 |
| UBFD1-540 | chr16:23562519-23562546 | UBFD1-1286 | chr16:23572630-23572657 |
| UBFD1-541 | chr16:23562522-23562549 | UBFD1-1287 | chr16:23572633-23572660 |
| UBFD1-542 | chr16:23562525-23562552 | UBFD1-1288 | chr16:23572634-23572661 |
| UBFD1-543 | chr16:23562526-23562553 | UBFD1-1289 | chr16:23572642-23572669 |
| UBFD1-544 | chr16:23562538-23562565 | UBFD1-1290 | chr16:23572652-23572679 |
| UBFD1-545 | chr16:23562553-23562580 | UBFD1-1291 | chr16:23572653-23572680 |
| UBFD1-546 | chr16:23562556-23562583 | UBFD1-1292 | chr16:23572656-23572683 |
| UBFD1-547 | chr16:23562566-23562593 | UBFD1-1293 | chr16:23572665-23572692 |
| UBFD1-548 | chr16:23562567-23562594 | UBFD1-1294 | chr16:23572666-23572693 |
| UBFD1-549 | chr16:23562568-23562595 | UBFD1-1295 | chr16:23572667-23572694 |
| UBFD1-550 | chr16:23562569-23562596 | UBFD1-1296 | chr16:23572672-23572699 |
| UBFD1-551 | chr16:23562591-23562618 | UBFD1-1297 | chr16:23572673-23572700 |
| UBFD1-552 | chr16:23562592-23562619 | UBFD1-1298 | chr16:23572674-23572701 |
| UBFD1-553 | chr16:23562595-23562622 | UBFD1-1299 | chr16:23572677-23572704 |
| UBFD1-554 | chr16:23562598-23562625 | UBFD1-1300 | chr16:23572681-23572708 |
| UBFD1-555 | chr16:23562605-23562632 | UBFD1-1301 | chr16:23572689-23572716 |
| UBFD1-556 | chr16:23562613-23562640 | UBFD1-1302 | chr16:23572692-23572719 |
| UBFD1-557 | chr16:23562618-23562645 | UBFD1-1303 | chr16:23572721-23572748 |
| UBFD1-558 | chr16:23562626-23562653 | UBFD1-1304 | chr16:23572727-23572754 |
| UBFD1-559 | chr16:23562630-23562657 | UBFD1-1305 | chr16:23572732-23572759 |
| UBFD1-560 | chr16:23562634-23562661 | UBFD1-1306 | chr16:23572737-23572764 |
| UBFD1-561 | chr16:23562643-23562670 | UBFD1-1307 | chr16:23572769-23572796 |
| UBFD1-562 | chr16:23562644-23562671 | UBFD1-1308 | chr16:23572797-23572824 |
| UBFD1-563 | chr16:23562647-23562674 | UBFD1-1309 | chr16:23572812-23572839 |
| UBFD1-564 | chr16:23562650-23562677 | UBFD1-1310 | chr16:23572860-23572887 |
| UBFD1-565 | chr16:23562689-23562716 | UBFD1-1311 | chr16:23572881-23572908 |
| UBFD1-566 | chr16:23562690-23562717 | UBFD1-1312 | chr16:23572890-23572917 |
| UBFD1-567 | chr16:23562695-23562722 | UBFD1-1313 | chr16:23572894-23572921 |
| UBFD1-568 | chr16:23562704-23562731 | UBFD1-1314 | chr16:23572895-23572922 |
| UBFD1-569 | chr16:23562708-23562735 | UBFD1-1315 | chr16:23572896-23572923 |
| UBFD1-570 | chr16:23562730-23562757 | UBFD1-1316 | chr16:23572904-23572931 |
| UBFD1-571 | chr16:23562741-23562768 | UBFD1-1317 | chr16:23572905-23572932 |
| UBFD1-572 | chr16:23562746-23562773 | UBFD1-1318 | chr16:23572913-23572940 |
| UBFD1-573 | chr16:23562748-23562775 | UBFD1-1319 | chr16:23572934-23572961 |
| UBFD1-574 | chr16:23562749-23562776 | UBFD1-1320 | chr16:23572935-23572962 |
| UBFD1-575 | chr16:23562758-23562785 | UBFD1-1321 | chr16:23572948-23572975 |
| UBFD1-576 | chr16:23562759-23562786 | UBFD1-1322 | chr16:23572955-23572982 |
| UBFD1-577 | chr16:23562765-23562792 | UBFD1-1323 | chr16:23572960-23572987 |
| UBFD1-578 | chr16:23562769-23562796 | UBFD1-1324 | chr16:23572961-23572988 |
| UBFD1-579 | chr16:23562778-23562805 | UBFD1-1325 | chr16:23572978-23573005 |
| UBFD1-580 | chr16:23562779-23562806 | UBFD1-1326 | chr16:23572989-23573016 |
| UBFD1-581 | chr16:23562812-23562839 | UBFD1-1327 | chr16:23573022-23573049 |
| UBFD1-582 | chr16:23562813-23562840 | UBFD1-1328 | chr16:23573031-23573058 |
| UBFD1-583 | chr16:23562814-23562841 | UBFD1-1329 | chr16:23573082-23573109 |
| UBFD1-584 | chr16:23562817-23562844 | UBFD1-1330 | chr16:23573096-23573123 |
| UBFD1-585 | chr16:23562827-23562854 | UBFD1-1331 | chr16:23573113-23573140 |
| UBFD1-586 | chr16:23563922-23563949 | UBFD1-1332 | chr16:23573134-23573161 |
| UBFD1-587 | chr16:23563926-23563953 | UBFD1-1333 | chr16:23573146-23573173 |
| UBFD1-588 | chr16:23563936-23563963 | UBFD1-1334 | chr16:23573147-23573174 |
| UBFD1-589 | chr16:23563937-23563964 | UBFD1-1335 | chr16:23573159-23573186 |
| UBFD1-590 | chr16:23563938-23563965 | UBFD1-1336 | chr16:23573170-23573197 |
| UBFD1-591 | chr16:23563939-23563966 | UBFD1-1337 | chr16:23573174-23573201 |
| UBFD1-592 | chr16:23563940-23563967 | UBFD1-1338 | chr16:23573175-23573202 |
| UBFD1-593 | chr16:23563943-23563970 | UBFD1-1339 | chr16:23573176-23573203 |
| UBFD1-594 | chr16:23563944-23563971 | UBFD1-1340 | chr16:23573185-23573212 |
| UBFD1-595 | chr16:23563947-23563974 | UBFD1-1341 | chr16:23573186-23573213 |
| UBFD1-596 | chr16:23563962-23563989 | UBFD1-1342 | chr16:23573187-23573214 |
| UBFD1-597 | chr16:23563994-23564021 | UBFD1-1343 | chr16:23573188-23573215 |
| UBFD1-598 | chr16:23563995-23564022 | UBFD1-1344 | chr16:23573195-23573222 |
| UBFD1-599 | chr16:23563996-23564023 | UBFD1-1345 | chr16:23573204-23573231 |
| UBFD1-600 | chr16:23564019-23564046 | UBFD1-1346 | chr16:23573211-23573238 |
| UBFD1-601 | chr16:23564022-23564049 | UBFD1-1347 | chr16:23573235-23573262 |
| UBFD1-602 | chr16:23564023-23564050 | UBFD1-1348 | chr16:23573250-23573277 |
| UBFD1-603 | chr16:23564028-23564055 | UBFD1-1349 | chr16:23573253-23573280 |
| UBFD1-604 | chr16:23564029-23564056 | UBFD1-1350 | chr16:23573254-23573281 |
| UBFD1-605 | chr16:23564030-23564057 | UBFD1-1351 | chr16:23573255-23573282 |
| UBFD1-606 | chr16:23564031-23564058 | UBFD1-1352 | chr16:23573256-23573283 |
| UBFD1-607 | chr16:23564042-23564069 | UBFD1-1353 | chr16:23573259-23573286 |
| UBFD1-608 | chr16:23564053-23564080 | UBFD1-1354 | chr16:23573277-23573304 |
| UBFD1-609 | chr16:23564062-23564089 | UBFD1-1355 | chr16:23573287-23573314 |
| UBFD1-610 | chr16:23564067-23564094 | UBFD1-1356 | chr16:23573300-23573327 |
| UBFD1-611 | chr16:23564071-23564098 | UBFD1-1357 | chr16:23573301-23573328 |
| UBFD1-612 | chr16:23564082-23564109 | UBFD1-1358 | chr16:23573308-23573335 |
| UBFD1-613 | chr16:23564083-23564110 | UBFD1-1359 | chr16:23573318-23573345 |
| UBFD1-614 | chr16:23564106-23564133 | UBFD1-1360 | chr16:23573321-23573348 |
| UBFD1-615 | chr16:23564107-23564134 | UBFD1-1361 | chr16:23573329-23573356 |
| UBFD1-616 | chr16:23564108-23564135 | UBFD1-1362 | chr16:23573332-23573359 |
| UBFD1-617 | chr16:23564109-23564136 | UBFD1-1363 | chr16:23573344-23573371 |
| UBFD1-618 | chr16:23564132-23564159 | UBFD1-1364 | chr16:23573350-23573377 |
| UBFD1-619 | chr16:23564138-23564165 | UBFD1-1365 | chr16:23573353-23573380 |
| UBFD1-620 | chr16:23564139-23564166 | UBFD1-1366 | chr16:23573361-23573388 |
| UBFD1-621 | chr16:23564140-23564167 | UBFD1-1367 | chr16:23573364-23573391 |
| UBFD1-622 | chr16:23564173-23564200 | UBFD1-1368 | chr16:23573389-23573416 |
| UBFD1-623 | chr16:23564174-23564201 | UBFD1-1369 | chr16:23573396-23573423 |
| UBFD1-624 | chr16:23564188-23564215 | UBFD1-1370 | chr16:23573397-23573424 |
| UBFD1-625 | chr16:23564199-23564226 | UBFD1-1371 | chr16:23573405-23573432 |
| UBFD1-626 | chr16:23564200-23564227 | UBFD1-1372 | chr16:23573406-23573433 |
| UBFD1-627 | chr16:23564201-23564228 | UBFD1-1373 | chr16:23573409-23573436 |
| UBFD1-628 | chr16:23564203-23564230 | UBFD1-1374 | chr16:23573410-23573437 |
| UBFD1-629 | chr16:23564205-23564232 | UBFD1-1375 | chr16:23573419-23573446 |
| UBFD1-630 | chr16:23564206-23564233 | UBFD1-1376 | chr16:23573424-23573451 |
| UBFD1-631 | chr16:23564207-23564234 | UBFD1-1377 | chr16:23573438-23573465 |
| UBFD1-632 | chr16:23564427-23564454 | UBFD1-1378 | chr16:23573439-23573466 |
| UBFD1-633 | chr16:23564428-23564455 | UBFD1-1379 | chr16:23573447-23573474 |
| UBFD1-634 | chr16:23564436-23564463 | UBFD1-1380 | chr16:23573452-23573479 |
| UBFD1-635 | chr16:23564437-23564464 | UBFD1-1381 | chr16:23573453-23573480 |
| UBFD1-636 | chr16:23564438-23564465 | UBFD1-1382 | chr16:23573454-23573481 |
| UBFD1-637 | chr16:23564439-23564466 | UBFD1-1383 | chr16:23573455-23573482 |
| UBFD1-638 | chr16:23564440-23564467 | UBFD1-1384 | chr16:23573456-23573483 |
| UBFD1-639 | chr16:23564456-23564483 | UBFD1-1385 | chr16:23573479-23573506 |
| UBFD1-640 | chr16:23564464-23564491 | UBFD1-1386 | chr16:23573489-23573516 |
| UBFD1-641 | chr16:23564465-23564492 | UBFD1-1387 | chr16:23573508-23573535 |
| UBFD1-642 | chr16:23564494-23564521 | UBFD1-1388 | chr16:23573511-23573538 |
| UBFD1-643 | chr16:23564499-23564526 | UBFD1-1389 | chr16:23573514-23573541 |
| UBFD1-644 | chr16:23564506-23564533 | UBFD1-1390 | chr16:23573515-23573542 |
| UBFD1-645 | chr16:23564507-23564534 | UBFD1-1391 | chr16:23573533-23573560 |
| UBFD1-646 | chr16:23564523-23564550 | UBFD1-1392 | chr16:23573543-23573570 |
| UBFD1-647 | chr16:23564527-23564554 | UBFD1-1393 | chr16:23573564-23573591 |
| UBFD1-648 | chr16:23564528-23564555 | UBFD1-1394 | chr16:23573580-23573607 |
| UBFD1-649 | chr16:23564532-23564559 | UBFD1-1395 | chr16:23573590-23573617 |
| UBFD1-650 | chr16:23564591-23564618 | UBFD1-1396 | chr16:23573591-23573618 |
| UBFD1-651 | chr16:23564604-23564631 | UBFD1-1397 | chr16:23573592-23573619 |
| UBFD1-652 | chr16:23564608-23564635 | UBFD1-1398 | chr16:23573603-23573630 |
| UBFD1-653 | chr16:23564611-23564638 | UBFD1-1399 | chr16:23573614-23573641 |
| UBFD1-654 | chr16:23564612-23564639 | UBFD1-1400 | chr16:23573620-23573647 |
| UBFD1-655 | chr16:23564620-23564647 | UBFD1-1401 | chr16:23573621-23573648 |
| UBFD1-656 | chr16:23564627-23564654 | UBFD1-1402 | chr16:23573631-23573658 |
| UBFD1-657 | chr16:23564639-23564666 | UBFD1-1403 | chr16:23573632-23573659 |
| UBFD1-658 | chr16:23564643-23564670 | UBFD1-1404 | chr16:23573643-23573670 |
| UBFD1-659 | chr16:23564648-23564675 | UBFD1-1405 | chr16:23573644-23573671 |
| UBFD1-660 | chr16:23564671-23564698 | UBFD1-1406 | chr16:23573661-23573688 |
| UBFD1-661 | chr16:23564677-23564704 | UBFD1-1407 | chr16:23573672-23573699 |
| UBFD1-662 | chr16:23564681-23564708 | UBFD1-1408 | chr16:23573690-23573717 |
| UBFD1-663 | chr16:23564682-23564709 | UBFD1-1409 | chr16:23573698-23573725 |
| UBFD1-664 | chr16:23564686-23564713 | UBFD1-1410 | chr16:23573708-23573735 |
| UBFD1-665 | chr16:23564690-23564717 | UBFD1-1411 | chr16:23573709-23573736 |
| UBFD1-666 | chr16:23564694-23564721 | UBFD1-1412 | chr16:23573710-23573737 |
| UBFD1-667 | chr16:23564695-23564722 | UBFD1-1413 | chr16:23573717-23573744 |
| UBFD1-668 | chr16:23564720-23564747 | UBFD1-1414 | chr16:23573727-23573754 |
| UBFD1-669 | chr16:23564728-23564755 | UBFD1-1415 | chr16:23573728-23573755 |
| UBFD1-670 | chr16:23564729-23564756 | UBFD1-1416 | chr16:23573746-23573773 |
| UBFD1-671 | chr16:23564730-23564757 | UBFD1-1417 | chr16:23573749-23573776 |
| UBFD1-672 | chr16:23564747-23564774 | UBFD1-1418 | chr16:23573752-23573779 |
| UBFD1-673 | chr16:23564750-23564777 | UBFD1-1419 | chr16:23573763-23573790 |
| UBFD1-674 | chr16:23564759-23564786 | UBFD1-1420 | chr16:23573764-23573791 |
| UBFD1-675 | chr16:23564760-23564787 | UBFD1-1421 | chr16:23573765-23573792 |
| UBFD1-676 | chr16:23564779-23564806 | UBFD1-1422 | chr16:23573772-23573799 |
| UBFD1-677 | chr16:23564815-23564842 | UBFD1-1423 | chr16:23573782-23573809 |
| UBFD1-678 | chr16:23564827-23564854 | UBFD1-1424 | chr16:23573796-23573823 |
| UBFD1-679 | chr16:23564832-23564859 | UBFD1-1425 | chr16:23573797-23573824 |
| UBFD1-680 | chr16:23564833-23564860 | UBFD1-1426 | chr16:23573798-23573825 |
| UBFD1-681 | chr16:23564837-23564864 | UBFD1-1427 | chr16:23573804-23573831 |
| UBFD1-682 | chr16:23564859-23564886 | UBFD1-1428 | chr16:23573805-23573832 |
| UBFD1-683 | chr16:23564867-23564894 | UBFD1-1429 | chr16:23573807-23573834 |
| UBFD1-684 | chr16:23564868-23564895 | UBFD1-1430 | chr16:23573808-23573835 |
| UBFD1-685 | chr16:23564870-23564897 | UBFD1-1431 | chr16:23573809-23573836 |
| UBFD1-686 | chr16:23564873-23564900 | UBFD1-1432 | chr16:23573813-23573840 |
| UBFD1-687 | chr16:23564877-23564904 | UBFD1-1433 | chr16:23573814-23573841 |
| UBFD1-688 | chr16:23564887-23564914 | UBFD1-1434 | chr16:23573815-23573842 |
| UBFD1-689 | chr16:23564897-23564924 | UBFD1-1435 | chr16:23573816-23573843 |
| UBFD1-690 | chr16:23564898-23564925 | UBFD1-1436 | chr16:23573819-23573846 |
| UBFD1-691 | chr16:23564899-23564926 | UBFD1-1437 | chr16:23573835-23573862 |
| UBFD1-692 | chr16:23564935-23564962 | UBFD1-1438 | chr16:23573836-23573863 |
| UBFD1-693 | chr16:23564936-23564963 | UBFD1-1439 | chr16:23573846-23573873 |
| UBFD1-694 | chr16:23564944-23564971 | UBFD1-1440 | chr16:23573847-23573874 |
| UBFD1-695 | chr16:23564945-23564972 | UBFD1-1441 | chr16:23573851-23573878 |
| UBFD1-696 | chr16:23564948-23564975 | UBFD1-1442 | chr16:23573857-23573884 |
| UBFD1-697 | chr16:23564951-23564978 | UBFD1-1443 | chr16:23573864-23573891 |
| UBFD1-698 | chr16:23564957-23564984 | UBFD1-1444 | chr16:23573876-23573903 |
| UBFD1-699 | chr16:23564976-23565003 | UBFD1-1445 | chr16:23573877-23573904 |
| UBFD1-700 | chr16:23564977-23565004 | UBFD1-1446 | chr16:23573887-23573914 |
| UBFD1-701 | chr16:23564979-23565006 | UBFD1-1447 | chr16:23573890-23573917 |
| UBFD1-702 | chr16:23564987-23565014 | UBFD1-1448 | chr16:23573891-23573918 |
| UBFD1-703 | chr16:23565026-23565053 | UBFD1-1449 | chr16:23573907-23573934 |
| UBFD1-704 | chr16:23565029-23565056 | UBFD1-1450 | chr16:23573908-23573935 |
| UBFD1-705 | chr16:23565030-23565057 | UBFD1-1451 | chr16:23573913-23573940 |
| UBFD1-706 | chr16:23565033-23565060 | UBFD1-1452 | chr16:23573922-23573949 |
| UBFD1-707 | chr16:23565034-23565061 | UBFD1-1453 | chr16:23573924-23573951 |
| UBFD1-708 | chr16:23565035-23565062 | UBFD1-1454 | chr16:23573927-23573954 |
| UBFD1-709 | chr16:23565036-23565063 | UBFD1-1455 | chr16:23573930-23573957 |
| UBFD1-710 | chr16:23565037-23565064 | UBFD1-1456 | chr16:23573934-23573961 |
| UBFD1-711 | chr16:23565041-23565068 | UBFD1-1457 | chr16:23573939-23573966 |
| UBFD1-712 | chr16:23565047-23565074 | UBFD1-1458 | chr16:23573950-23573977 |
| UBFD1-713 | chr16:23565056-23565083 | UBFD1-1459 | chr16:23573963-23573990 |
| UBFD1-714 | chr16:23565057-23565084 | UBFD1-1460 | chr16:23573964-23573991 |
| UBFD1-715 | chr16:23565084-23565111 | UBFD1-1461 | chr16:23573978-23574005 |
| UBFD1-716 | chr16:23565085-23565112 | UBFD1-1462 | chr16:23573997-23574024 |
| UBFD1-717 | chr16:23565090-23565117 | UBFD1-1463 | chr16:23574011-23574038 |
| UBFD1-718 | chr16:23565097-23565124 | UBFD1-1464 | chr16:23574013-23574040 |
| UBFD1-719 | chr16:23565111-23565138 | UBFD1-1465 | chr16:23574014-23574041 |
| UBFD1-720 | chr16:23565112-23565139 | UBFD1-1466 | chr16:23574020-23574047 |
| UBFD1-721 | chr16:23565118-23565145 | UBFD1-1467 | chr16:23574032-23574059 |
| UBFD1-722 | chr16:23565132-23565159 | UBFD1-1468 | chr16:23574034-23574061 |
| UBFD1-723 | chr16:23565149-23565176 | UBFD1-1469 | chr16:23574035-23574062 |
| UBFD1-724 | chr16:23565153-23565180 | UBFD1-1470 | chr16:23574039-23574066 |
| UBFD1-725 | chr16:23565162-23565189 | UBFD1-1471 | chr16:23574040-23574067 |
| UBFD1-726 | chr16:23565168-23565195 | UBFD1-1472 | chr16:23574042-23574069 |
| UBFD1-727 | chr16:23565193-23565220 | UBFD1-1473 | chr16:23574066-23574093 |
| UBFD1-728 | chr16:23565201-23565228 | UBFD1-1474 | chr16:23574070-23574097 |
| UBFD1-729 | chr16:23565204-23565231 | UBFD1-1475 | chr16:23574071-23574098 |
| UBFD1-730 | chr16:23565205-23565232 | UBFD1-1476 | chr16:23574083-23574110 |
| UBFD1-731 | chr16:23565206-23565233 | UBFD1-1477 | chr16:23574087-23574114 |
| UBFD1-732 | chr16:23565221-23565248 | UBFD1-1478 | chr16:23574088-23574115 |
| UBFD1-733 | chr16:23565234-23565261 | UBFD1-1479 | chr16:23574091-23574118 |
| UBFD1-734 | chr16:23565235-23565262 | UBFD1-1480 | chr16:23574106-23574133 |
| UBFD1-735 | chr16:23565253-23565280 | UBFD1-1481 | chr16:23574146-23574173 |
| UBFD1-736 | chr16:23565254-23565281 | UBFD1-1482 | chr16:23574188-23574215 |
| UBFD1-737 | chr16:23565272-23565299 | UBFD1-1483 | chr16:23574200-23574227 |
| UBFD1-738 | chr16:23565275-23565302 | UBFD1-1484 | chr16:23574205-23574232 |
| UBFD1-739 | chr16:23565278-23565305 | UBFD1-1485 | chr16:23574208-23574235 |
| UBFD1-740 | chr16:23565318-23565345 | UBFD1-1486 | chr16:23574235-23574262 |
| UBFD1-741 | chr16:23565322-23565349 | UBFD1-1487 | chr16:23574236-23574263 |
| UBFD1-742 | chr16:23565329-23565356 | UBFD1-1488 | chr16:23574239-23574266 |
| UBFD1-743 | chr16:23565339-23565366 | UBFD1-1489 | chr16:23574254-23574281 |
| UBFD1-744 | chr16:23565341-23565368 | UBFD1-1490 | chr16:23574277-23574304 |
| UBFD1-745 | chr16:23565342-23565369 | UBFD1-1491 | chr16:23574325-23574352 |
| UBFD1-746 | chr16:23565353-23565380 | UBFD1-1492 | chr16:23574351-23574378 |

**Table 2H Genomic coordinates of the human CRP gene**

| Target ID | Target Coordinate Range | Target ID | Target Coordinate Range |
|---|---|---|---|
| CRP-1 | chr1:159712274-159712301 | CRP-238 | chr1:159713845-159713872 |
| CRP-2 | chr1:159712275-159712302 | CRP-239 | chr1:159713846-159713873 |
| CRP-3 | chr1:159712276-159712303 | CRP-240 | chr1:159713860-159713887 |
| CRP-4 | chr1:159712285-159712312 | CRP-241 | chr1:159713890-159713917 |
| CRP-5 | chr1:159712298-159712325 | CRP-242 | chr1:159713909-159713936 |
| CRP-6 | chr1:159712299-159712326 | CRP-243 | chr1:159713910-159713937 |
| CRP-7 | chr1:159712308-159712335 | CRP-244 | chr1:159713913-159713940 |
| CRP-8 | chr1:159712319-159712346 | CRP-245 | chr1:159713914-159713941 |
| CRP-9 | chr1:159712321-159712348 | CRP-246 | chr1:159713931-159713958 |
| CRP-10 | chr1:159712331-159712358 | CRP-247 | chr1:159713938-159713965 |
| CRP-11 | chr1:159712349-159712376 | CRP-248 | chr1:159713945-159713972 |
| CRP-12 | chr1:159712353-159712380 | CRP-249 | chr1:159713952-159713979 |
| CRP-13 | chr1:159712359-159712386 | CRP-250 | chr1:159713955-159713982 |
| CRP-14 | chr1:159712373-159712400 | CRP-251 | chr1:159713981-159714008 |
| CRP-15 | chr1:159712383-159712410 | CRP-252 | chr1:159713982-159714009 |
| CRP-16 | chr1:159712403-159712430 | CRP-253 | chr1:159713988-159714015 |
| CRP-17 | chr1:159712404-159712431 | CRP-254 | chr1:159714002-159714029 |
| CRP-18 | chr1:159712405-159712432 | CRP-255 | chr1:159714003-159714030 |
| CRP-19 | chr1:159712413-159712440 | CRP-256 | chr1:159714008-159714035 |
| CRP-20 | chr1:159712416-159712443 | CRP-257 | chr1:159714011-159714038 |
| CRP-21 | chr1:159712422-159712449 | CRP-258 | chr1:159714022-159714049 |
| CRP-22 | chr1:159712433-159712460 | CRP-259 | chr1:159714024-159714051 |
| CRP-23 | chr1:159712438-159712465 | CRP-260 | chr1:159714034-159714061 |
| CRP-24 | chr1:159712445-159712472 | CRP-261 | chr1:159714046-159714073 |
| CRP-25 | chr1:159712452-159712479 | CRP-262 | chr1:159714056-159714083 |
| CRP-26 | chr1:159712453-159712480 | CRP-263 | chr1:159714057-159714084 |
| CRP-27 | chr1:159712455-159712482 | CRP-264 | chr1:159714066-159714093 |
| CRP-28 | chr1:159712462-159712489 | CRP-265 | chr1:159714084-159714111 |
| CRP-29 | chr1:159712482-159712509 | CRP-266 | chr1:159714085-159714112 |
| CRP-30 | chr1:159712484-159712511 | CRP-267 | chr1:159714100-159714127 |
| CRP-31 | chr1:159712485-159712512 | CRP-268 | chr1:159714124-159714151 |
| CRP-32 | chr1:159712497-159712524 | CRP-269 | chr1:159714128-159714155 |
| CRP-33 | chr1:159712514-159712541 | CRP-270 | chr1:159714144-159714171 |
| CRP-34 | chr1:159712536-159712563 | CRP-271 | chr1:159714146-159714173 |
| CRP-35 | chr1:159712537-159712564 | CRP-272 | chr1:159714195-159714222 |
| CRP-36 | chr1:159712538-159712565 | CRP-273 | chr1:159714196-159714223 |
| CRP-37 | chr1:159712539-159712566 | CRP-274 | chr1:159714197-159714224 |
| CRP-38 | chr1:159712546-159712573 | CRP-275 | chr1:159714238-159714265 |
| CRP-39 | chr1:159712551-159712578 | CRP-276 | chr1:159714310-159714337 |
| CRP-40 | chr1:159712558-159712585 | CRP-277 | chr1:159714371-159714398 |
| CRP-41 | chr1:159712561-159712588 | CRP-278 | chr1:159714372-159714399 |
| CRP-42 | chr1:159712580-159712607 | CRP-279 | chr1:159714373-159714400 |
| CRP-43 | chr1:159712594-159712621 | CRP-280 | chr1:159714376-159714403 |
| CRP-44 | chr1:159712595-159712622 | CRP-281 | chr1:159714377-159714404 |
| CRP-45 | chr1:159712601-159712628 | CRP-282 | chr1:159714378-159714405 |
| CRP-46 | chr1:159712602-159712629 | CRP-283 | chr1:159714379-159714406 |
| CRP-47 | chr1:159712603-159712630 | CRP-284 | chr1:159714384-159714411 |
| CRP-48 | chr1:159712609-159712636 | CRP-285 | chr1:159714385-159714412 |
| CRP-49 | chr1:159712610-159712637 | CRP-286 | chr1:159714386-159714413 |
| CRP-50 | chr1:159712613-159712640 | CRP-287 | chr1:159714387-159714414 |
| CRP-51 | chr1:159712616-159712643 | CRP-288 | chr1:159714390-159714417 |
| CRP-52 | chr1:159712617-159712644 | CRP-289 | chr1:159714401-159714428 |
| CRP-53 | chr1:159712630-159712657 | CRP-290 | chr1:159714402-159714429 |
| CRP-54 | chr1:159712641-159712668 | CRP-291 | chr1:159714405-159714432 |
| CRP-55 | chr1:159712647-159712674 | CRP-292 | chr1:159714408-159714435 |
| CRP-56 | chr1:159712660-159712687 | CRP-293 | chr1:159714418-159714445 |
| CRP-57 | chr1:159712661-159712688 | CRP-294 | chr1:159714419-159714446 |
| CRP-58 | chr1:159712662-159712689 | CRP-295 | chr1:159714423-159714450 |
| CRP-59 | chr1:159712665-159712692 | CRP-296 | chr1:159714424-159714451 |
| CRP-60 | chr1:159712666-159712693 | CRP-297 | chr1:159714427-159714454 |
| CRP-61 | chr1:159712674-159712701 | CRP-298 | chr1:159714433-159714460 |
| CRP-62 | chr1:159712675-159712702 | CRP-299 | chr1:159714460-159714487 |
| CRP-63 | chr1:159712676-159712703 | CRP-300 | chr1:159714461-159714488 |
| CRP-64 | chr1:159712682-159712709 | CRP-301 | chr1:159714482-159714509 |
| CRP-65 | chr1:159712683-159712710 | CRP-302 | chr1:159714487-159714514 |
| CRP-66 | chr1:159712684-159712711 | CRP-303 | chr1:159714488-159714515 |
| CRP-67 | chr1:159712688-159712715 | CRP-304 | chr1:159714494-159714521 |
| CRP-68 | chr1:159712713-159712740 | CRP-305 | chr1:159714501-159714528 |
| CRP-69 | chr1:159712722-159712749 | CRP-306 | chr1:159714507-159714534 |
| CRP-70 | chr1:159712728-159712755 | CRP-307 | chr1:159714516-159714543 |
| CRP-71 | chr1:159712729-159712756 | CRP-308 | chr1:159714522-159714549 |
| CRP-72 | chr1:159712730-159712757 | CRP-309 | chr1:159714528-159714555 |
| CRP-73 | chr1:159712731-159712758 | CRP-310 | chr1:159714535-159714562 |
| CRP-74 | chr1:159712732-159712759 | CRP-311 | chr1:159714536-159714563 |
| CRP-75 | chr1:159712733-159712760 | CRP-312 | chr1:159714537-159714564 |
| CRP-76 | chr1:159712766-159712793 | CRP-313 | chr1:159714573-159714600 |
| CRP-77 | chr1:159712767-159712794 | CRP-314 | chr1:159714584-159714611 |
| CRP-78 | chr1:159712776-159712803 | CRP-315 | chr1:159714586-159714613 |
| CRP-79 | chr1:159712804-159712831 | CRP-316 | chr1:159714597-159714624 |
| CRP-80 | chr1:159712812-159712839 | CRP-317 | chr1:159714600-159714627 |
| CRP-81 | chr1:159712846-159712873 | CRP-318 | chr1:159714603-159714630 |
| CRP-82 | chr1:159712851-159712878 | CRP-319 | chr1:159714606-159714633 |
| CRP-83 | chr1:159712871-159712898 | CRP-320 | chr1:159714607-159714634 |
| CRP-84 | chr1:159712872-159712899 | CRP-321 | chr1:159714620-159714647 |
| CRP-85 | chr1:159712873-159712900 | CRP-322 | chr1:159714643-159714670 |
| CRP-86 | chr1:159712885-159712912 | CRP-323 | chr1:159714651-159714678 |
| CRP-87 | chr1:159712912-159712939 | CRP-324 | chr1:159714652-159714679 |
| CRP-88 | chr1:159712913-159712940 | CRP-325 | chr1:159714653-159714680 |
| CRP-89 | chr1:159712915-159712942 | CRP-326 | chr1:159714657-159714684 |
| CRP-90 | chr1:159712916-159712943 | CRP-327 | chr1:159714662-159714689 |
| CRP-91 | chr1:159712918-159712945 | CRP-328 | chr1:159714671-159714698 |
| CRP-92 | chr1:159712925-159712952 | CRP-329 | chr1:159714672-159714699 |
| CRP-93 | chr1:159712934-159712961 | CRP-330 | chr1:159714678-159714705 |
| CRP-94 | chr1:159712935-159712962 | CRP-331 | chr1:159714679-159714706 |
| CRP-95 | chr1:159712949-159712976 | CRP-332 | chr1:159714680-159714707 |
| CRP-96 | chr1:159712957-159712984 | CRP-333 | chr1:159714761-159714788 |
| CRP-97 | chr1:159712958-159712985 | CRP-334 | chr1:159714769-159714796 |
| CRP-98 | chr1:159712966-159712993 | CRP-335 | chr1:159714779-159714806 |
| CRP-99 | chr1:159712967-159712994 | CRP-336 | chr1:159714791-159714818 |
| CRP-100 | chr1:159712980-159713007 | CRP-337 | chr1:159714792-159714819 |
| CRP-101 | chr1:159712986-159713013 | CRP-338 | chr1:159714803-159714830 |
| CRP-102 | chr1:159712987-159713014 | CRP-339 | chr1:159714822-159714849 |
| CRP-103 | chr1:159712994-159713021 | CRP-340 | chr1:159714827-159714854 |
| CRP-104 | chr1:159713013-159713040 | CRP-341 | chr1:159714828-159714855 |
| CRP-105 | chr1:159713023-159713050 | CRP-342 | chr1:159714834-159714861 |
| CRP-106 | chr1:159713024-159713051 | CRP-343 | chr1:159714839-159714866 |
| CRP-107 | chr1:159713029-159713056 | CRP-344 | chr1:159714853-159714880 |
| CRP-108 | chr1:159713036-159713063 | CRP-345 | chr1:159714861-159714888 |
| CRP-109 | chr1:159713041-159713068 | CRP-346 | chr1:159714862-159714889 |
| CRP-110 | chr1:159713048-159713075 | CRP-347 | chr1:159714871-159714898 |
| CRP-111 | chr1:159713055-159713082 | CRP-348 | chr1:159714881-159714908 |
| CRP-112 | chr1:159713056-159713083 | CRP-349 | chr1:159714905-159714932 |
| CRP-113 | chr1:159713069-159713096 | CRP-350 | chr1:159714910-159714937 |
| CRP-114 | chr1:159713070-159713097 | CRP-351 | chr1:159714916-159714943 |
| CRP-115 | chr1:159713077-159713104 | CRP-352 | chr1:159714923-159714950 |
| CRP-116 | chr1:159713078-159713105 | CRP-353 | chr1:159714926-159714953 |
| CRP-117 | chr1:159713079-159713106 | CRP-354 | chr1:159714927-159714954 |
| CRP-118 | chr1:159713080-159713107 | CRP-355 | chr1:159714941-159714968 |
| CRP-119 | chr1:159713086-159713113 | CRP-356 | chr1:159714947-159714974 |
| CRP-120 | chr1:159713087-159713114 | CRP-357 | chr1:159714988-159715015 |
| CRP-121 | chr1:159713089-159713116 | CRP-358 | chr1:159714989-159715016 |
| CRP-122 | chr1:159713090-159713117 | CRP-359 | chr1:159714990-159715017 |
| CRP-123 | chr1:159713091-159713118 | CRP-360 | chr1:159714991-159715018 |
| CRP-124 | chr1:159713092-159713119 | CRP-361 | chr1:159714999-159715026 |
| CRP-125 | chr1:159713093-159713120 | CRP-362 | chr1:159715029-159715056 |
| CRP-126 | chr1:159713143-159713170 | CRP-363 | chr1:159715041-159715068 |
| CRP-127 | chr1:159713158-159713185 | CRP-364 | chr1:159715044-159715071 |
| CRP-128 | chr1:159713168-159713195 | CRP-365 | chr1:159715045-159715072 |
| CRP-129 | chr1:159713169-159713196 | CRP-366 | chr1:159715051-159715078 |
| CRP-130 | chr1:159713187-159713214 | CRP-367 | chr1:159715067-159715094 |
| CRP-131 | chr1:159713194-159713221 | CRP-368 | chr1:159715068-159715095 |
| CRP-132 | chr1:159713235-159713262 | CRP-369 | chr1:159715089-159715116 |
| CRP-133 | chr1:159713246-159713273 | CRP-370 | chr1:159715121-159715148 |
| CRP-134 | chr1:159713270-159713297 | CRP-371 | chr1:159715140-159715167 |
| CRP-135 | chr1:159713276-159713303 | CRP-372 | chr1:159715157-159715184 |
| CRP-136 | chr1:159713299-159713326 | CRP-373 | chr1:159715158-159715185 |
| CRP-137 | chr1:159713305-159713332 | CRP-374 | chr1:159715159-159715186 |
| CRP-138 | chr1:159713306-159713333 | CRP-375 | chr1:159715203-159715230 |
| CRP-139 | chr1:159713325-159713352 | CRP-376 | chr1:159715204-159715231 |
| CRP-140 | chr1:159713326-159713353 | CRP-377 | chr1:159715207-159715234 |
| CRP-141 | chr1:159713329-159713356 | CRP-378 | chr1:159715208-159715235 |
| CRP-142 | chr1:159713331-159713358 | CRP-379 | chr1:159715209-159715236 |
| CRP-143 | chr1:159713337-159713364 | CRP-380 | chr1:159715210-159715237 |
| CRP-144 | chr1:159713340-159713367 | CRP-381 | chr1:159715211-159715238 |
| CRP-145 | chr1:159713341-159713368 | CRP-382 | chr1:159715214-159715241 |
| CRP-146 | chr1:159713343-159713370 | CRP-383 | chr1:159715215-159715242 |
| CRP-147 | chr1:159713344-159713371 | CRP-384 | chr1:159715216-159715243 |
| CRP-148 | chr1:159713346-159713373 | CRP-385 | chr1:159715217-159715244 |
| CRP-149 | chr1:159713350-159713377 | CRP-386 | chr1:159715218-159715245 |
| CRP-150 | chr1:159713367-159713394 | CRP-387 | chr1:159715221-159715248 |
| CRP-151 | chr1:159713368-159713395 | CRP-388 | chr1:159715222-159715249 |
| CRP-152 | chr1:159713369-159713396 | CRP-389 | chr1:159715223-159715250 |
| CRP-153 | chr1:159713377-159713404 | CRP-390 | chr1:159715233-159715260 |
| CRP-154 | chr1:159713378-159713405 | CRP-391 | chr1:159715234-159715261 |
| CRP-155 | chr1:159713396-159713423 | CRP-392 | chr1:159715235-159715262 |
| CRP-156 | chr1:159713397-159713424 | CRP-393 | chr1:159715250-159715277 |
| CRP-157 | chr1:159713405-159713432 | CRP-394 | chr1:159715251-159715278 |
| CRP-158 | chr1:159713406-159713433 | CRP-395 | chr1:159715252-159715279 |
| CRP-159 | chr1:159713420-159713447 | CRP-396 | chr1:159715256-159715283 |
| CRP-160 | chr1:159713421-159713448 | CRP-397 | chr1:159715263-159715290 |
| CRP-161 | chr1:159713424-159713451 | CRP-398 | chr1:159715283-159715310 |
| CRP-162 | chr1:159713427-159713454 | CRP-399 | chr1:159715286-159715313 |
| CRP-163 | chr1:159713444-159713471 | CRP-400 | chr1:159715287-159715314 |
| CRP-164 | chr1:159713445-159713472 | CRP-401 | chr1:159715290-159715317 |
| CRP-165 | chr1:159713446-159713473 | CRP-402 | chr1:159715302-159715329 |
| CRP-166 | chr1:159713454-159713481 | CRP-403 | chr1:159715344-159715371 |
| CRP-167 | chr1:159713455-159713482 | CRP-404 | chr1:159715357-159715384 |
| CRP-168 | chr1:159713468-159713495 | CRP-405 | chr1:159715370-159715397 |
| CRP-169 | chr1:159713469-159713496 | CRP-406 | chr1:159715398-159715425 |
| CRP-170 | chr1:159713472-159713499 | CRP-407 | chr1:159715399-159715426 |
| CRP-171 | chr1:159713474-159713501 | CRP-408 | chr1:159715414-159715441 |
| CRP-172 | chr1:159713475-159713502 | CRP-409 | chr1:159715429-159715456 |
| CRP-173 | chr1:159713482-159713509 | CRP-410 | chr1:159715465-159715492 |
| CRP-174 | chr1:159713492-159713519 | CRP-411 | chr1:159715471-159715498 |
| CRP-175 | chr1:159713494-159713521 | CRP-412 | chr1:159715475-159715502 |
| CRP-176 | chr1:159713495-159713522 | CRP-413 | chr1:159715482-159715509 |
| CRP-177 | chr1:159713502-159713529 | CRP-414 | chr1:159715530-159715557 |
| CRP-178 | chr1:159713503-159713530 | CRP-415 | chr1:159715555-159715582 |
| CRP-179 | chr1:159713511-159713538 | CRP-416 | chr1:159715556-159715583 |
| CRP-180 | chr1:159713512-159713539 | CRP-417 | chr1:159715559-159715586 |
| CRP-181 | chr1:159713513-159713540 | CRP-418 | chr1:159715588-159715615 |
| CRP-182 | chr1:159713514-159713541 | CRP-419 | chr1:159715589-159715616 |
| CRP-183 | chr1:159713515-159713542 | CRP-420 | chr1:159715625-159715652 |
| CRP-184 | chr1:159713520-159713547 | CRP-421 | chr1:159715631-159715658 |
| CRP-185 | chr1:159713523-159713550 | CRP-422 | chr1:159715638-159715665 |
| CRP-186 | chr1:159713531-159713558 | CRP-423 | chr1:159715646-159715673 |
| CRP-187 | chr1:159713559-159713586 | CRP-424 | chr1:159715647-159715674 |
| CRP-188 | chr1:159713567-159713594 | CRP-425 | chr1:159715648-159715675 |
| CRP-189 | chr1:159713575-159713602 | CRP-426 | chr1:159715688-159715715 |
| CRP-190 | chr1:159713581-159713608 | CRP-427 | chr1:159715724-159715751 |
| CRP-191 | chr1:159713582-159713609 | CRP-428 | chr1:159715727-159715754 |
| CRP-192 | chr1:159713583-159713610 | CRP-429 | chr1:159715733-159715760 |
| CRP-193 | chr1:159713584-159713611 | CRP-430 | chr1:159715755-159715782 |
| CRP-194 | chr1:159713585-159713612 | CRP-431 | chr1:159715760-159715787 |
| CRP-195 | chr1:159713601-159713628 | CRP-432 | chr1:159715761-159715788 |
| CRP-196 | chr1:159713612-159713639 | CRP-433 | chr1:159715769-159715796 |
| CRP-197 | chr1:159713613-159713640 | CRP-434 | chr1:159715785-159715812 |
| CRP-198 | chr1:159713616-159713643 | CRP-435 | chr1:159715786-159715813 |
| CRP-199 | chr1:159713617-159713644 | CRP-436 | chr1:159715793-159715820 |
| CRP-200 | chr1:159713659-159713686 | CRP-437 | chr1:159715825-159715852 |
| CRP-201 | chr1:159713660-159713687 | CRP-438 | chr1:159715826-159715853 |
| CRP-202 | chr1:159713666-159713693 | CRP-439 | chr1:159715827-159715854 |
| CRP-203 | chr1:159713667-159713694 | CRP-440 | chr1:159715836-159715863 |
| CRP-204 | chr1:159713672-159713699 | CRP-441 | chr1:159715844-159715871 |
| CRP-205 | chr1:159713676-159713703 | CRP-442 | chr1:159715845-159715872 |
| CRP-206 | chr1:159713685-159713712 | CRP-443 | chr1:159715849-159715876 |
| CRP-207 | chr1:159713686-159713713 | CRP-444 | chr1:159715850-159715877 |
| CRP-208 | chr1:159713689-159713716 | CRP-445 | chr1:159715851-159715878 |
| CRP-209 | chr1:159713697-159713724 | CRP-446 | chr1:159715852-159715879 |
| CRP-210 | chr1:159713703-159713730 | CRP-447 | chr1:159715862-159715889 |
| CRP-211 | chr1:159713714-159713741 | CRP-448 | chr1:159715886-159715913 |
| CRP-212 | chr1:159713715-159713742 | CRP-449 | chr1:159715887-159715914 |
| CRP-213 | chr1:159713718-159713745 | CRP-450 | chr1:159715890-159715917 |
| CRP-214 | chr1:159713728-159713755 | CRP-451 | chr1:159715899-159715926 |
| CRP-215 | chr1:159713734-159713761 | CRP-452 | chr1:159715916-159715943 |
| CRP-216 | chr1:159713738-159713765 | CRP-453 | chr1:159715918-159715945 |
| CRP-217 | chr1:159713739-159713766 | CRP-454 | chr1:159715956-159715983 |
| CRP-218 | chr1:159713740-159713767 | CRP-455 | chr1:159715957-159715984 |
| CRP-219 | chr1:159713761-159713788 | CRP-456 | chr1:159715958-159715985 |
| CRP-220 | chr1:159713762-159713789 | CRP-457 | chr1:159715961-159715988 |
| CRP-221 | chr1:159713763-159713790 | CRP-458 | chr1:159715962-159715989 |
| CRP-222 | chr1:159713764-159713791 | CRP-459 | chr1:159715968-159715995 |
| CRP-223 | chr1:159713775-159713802 | CRP-460 | chr1:159715969-159715996 |
| CRP-224 | chr1:159713776-159713803 | CRP-461 | chr1:159715970-159715997 |
| CRP-225 | chr1:159713792-159713819 | CRP-462 | chr1:159715971-159715998 |
| CRP-226 | chr1:159713797-159713824 | CRP-463 | chr1:159715978-159716005 |
| CRP-227 | chr1:159713798-159713825 | CRP-464 | chr1:159715979-159716006 |
| CRP-228 | chr1:159713807-159713834 | CRP-465 | chr1:159715982-159716009 |
| CRP-229 | chr1:159713808-159713835 | CRP-466 | chr1:159715988-159716015 |
| CRP-230 | chr1:159713811-159713838 | CRP-467 | chr1:159716005-159716032 |
| CRP-231 | chr1:159713814-159713841 | CRP-468 | chr1:159716032-159716059 |
| CRP-232 | chr1:159713815-159713842 | CRP-469 | chr1:159716033-159716060 |
| CRP-233 | chr1:159713816-159713843 | CRP-470 | chr1:159716036-159716063 |
| CRP-234 | chr1:159713824-159713851 | CRP-471 | chr1:159716041-159716068 |
| CRP-235 | chr1:159713831-159713858 | CRP-472 | chr1:159716056-159716083 |
| CRP-236 | chr1:159713832-159713859 | CRP-473 | chr1:159716063-159716090 |
| CRP-237 | chr1:159713842-159713869 | CRP-474 | chr1:159716086-159716113 |

**Table 2I Genomic coordinates of the human CYLD gene**

| Target ID | Target Coordinate Range | Target ID | Target Coordinate Range |
|---|---|---|---|
| CYLD-1 | chr16:50740584-50740611 | CYLD-749 | chr16:50781243-50781270 |
| CYLD-2 | chr16:50740585-50740612 | CYLD-750 | chr16:50781247-50781274 |
| CYLD-3 | chr16:50740590-50740617 | CYLD-751 | chr16:50781259-50781286 |
| CYLD-4 | chr16:50740592-50740619 | CYLD-752 | chr16:50781266-50781293 |
| CYLD-5 | chr16:50740596-50740623 | CYLD-753 | chr16:50781277-50781304 |
| CYLD-6 | chr16:50740599-50740626 | CYLD-754 | chr16:50781291-50781318 |
| CYLD-7 | chr16:50740631-50740658 | CYLD-755 | chr16:50781301-50781328 |
| CYLD-8 | chr16:50740647-50740674 | CYLD-756 | chr16:50781307-50781334 |
| CYLD-9 | chr16:50740664-50740691 | CYLD-757 | chr16:50781308-50781335 |
| CYLD-10 | chr16:50740666-50740693 | CYLD-758 | chr16:50781320-50781347 |
| CYLD-11 | chr16:50740675-50740702 | CYLD-759 | chr16:50781332-50781359 |
| CYLD-12 | chr16:50740679-50740706 | CYLD-760 | chr16:50781348-50781375 |
| CYLD-13 | chr16:50740689-50740716 | CYLD-761 | chr16:50781351-50781378 |
| CYLD-14 | chr16:50740697-50740724 | CYLD-762 | chr16:50781375-50781402 |
| CYLD-15 | chr16:50740701-50740728 | CYLD-763 | chr16:50781382-50781409 |
| CYLD-16 | chr16:50740718-50740745 | CYLD-764 | chr16:50781385-50781412 |
| CYLD-17 | chr16:50740722-50740749 | CYLD-765 | chr16:50781392-50781419 |
| CYLD-18 | chr16:50740729-50740756 | CYLD-766 | chr16:50781419-50781446 |
| CYLD-19 | chr16:50740740-50740767 | CYLD-767 | chr16:50781442-50781469 |
| CYLD-20 | chr16:50740741-50740768 | CYLD-768 | chr16:50781458-50781485 |
| CYLD-21 | chr16:50740746-50740773 | CYLD-769 | chr16:50781464-50781491 |
| CYLD-22 | chr16:50740747-50740774 | CYLD-770 | chr16:50782232-50782259 |
| CYLD-23 | chr16:50740757-50740784 | CYLD-771 | chr16:50782233-50782260 |
| CYLD-24 | chr16:50740758-50740785 | CYLD-772 | chr16:50782304-50782331 |
| CYLD-25 | chr16:50740771-50740798 | CYLD-773 | chr16:50782307-50782334 |
| CYLD-26 | chr16:50740779-50740806 | CYLD-774 | chr16:50782351-50782378 |
| CYLD-27 | chr16:50740787-50740814 | CYLD-775 | chr16:50782361-50782388 |
| CYLD-28 | chr16:50740794-50740821 | CYLD-776 | chr16:50782366-50782393 |
| CYLD-29 | chr16:50740801-50740828 | CYLD-777 | chr16:50782369-50782396 |
| CYLD-30 | chr16:50740802-50740829 | CYLD-778 | chr16:50782379-50782406 |
| CYLD-31 | chr16:50740807-50740834 | CYLD-779 | chr16:50782380-50782407 |
| CYLD-32 | chr16:50740819-50740846 | CYLD-780 | chr16:50782391-50782418 |
| CYLD-33 | chr16:50740825-50740852 | CYLD-781 | chr16:50782399-50782426 |
| CYLD-34 | chr16:50740853-50740880 | CYLD-782 | chr16:50782400-50782427 |
| CYLD-35 | chr16:50740863-50740890 | CYLD-783 | chr16:50782422-50782449 |
| CYLD-36 | chr16:50740868-50740895 | CYLD-784 | chr16:50782440-50782467 |
| CYLD-37 | chr16:50740876-50740903 | CYLD-785 | chr16:50782457-50782484 |
| CYLD-38 | chr16:50740879-50740906 | CYLD-786 | chr16:50782464-50782491 |
| CYLD-39 | chr16:50740907-50740934 | CYLD-787 | chr16:50782483-50782510 |
| CYLD-40 | chr16:50740936-50740963 | CYLD-788 | chr16:50782487-50782514 |
| CYLD-41 | chr16:50740950-50740977 | CYLD-789 | chr16:50782488-50782515 |
| CYLD-42 | chr16:50740972-50740999 | CYLD-790 | chr16:50782499-50782526 |
| CYLD-43 | chr16:50740973-50741000 | CYLD-791 | chr16:50782503-50782530 |
| CYLD-44 | chr16:50740979-50741006 | CYLD-792 | chr16:50782523-50782550 |
| CYLD-45 | chr16:50740980-50741007 | CYLD-793 | chr16:50784231-50784258 |
| CYLD-46 | chr16:50740981-50741008 | CYLD-794 | chr16:50784249-50784276 |
| CYLD-47 | chr16:50740982-50741009 | CYLD-795 | chr16:50784313-50784340 |
| CYLD-48 | chr16:50741000-50741027 | CYLD-796 | chr16:50784327-50784354 |
| CYLD-49 | chr16:50741064-50741091 | CYLD-797 | chr16:50784372-50784399 |
| CYLD-50 | chr16:50741065-50741092 | CYLD-798 | chr16:50784373-50784400 |
| CYLD-51 | chr16:50741066-50741093 | CYLD-799 | chr16:50784398-50784425 |
| CYLD-52 | chr16:50741069-50741096 | CYLD-800 | chr16:50784409-50784436 |
| CYLD-53 | chr16:50741072-50741099 | CYLD-801 | chr16:50784410-50784437 |
| CYLD-54 | chr16:50741091-50741118 | CYLD-802 | chr16:50784441-50784468 |
| CYLD-55 | chr16:50741097-50741124 | CYLD-803 | chr16:50784462-50784489 |
| CYLD-56 | chr16:50741098-50741125 | CYLD-804 | chr16:50784479-50784506 |
| CYLD-57 | chr16:50741118-50741145 | CYLD-805 | chr16:50784484-50784511 |
| CYLD-58 | chr16:50741137-50741164 | CYLD-806 | chr16:50784528-50784555 |
| CYLD-59 | chr16:50741144-50741171 | CYLD-807 | chr16:50784529-50784556 |
| CYLD-60 | chr16:50741151-50741178 | CYLD-808 | chr16:50784530-50784557 |
| CYLD-61 | chr16:50741189-50741216 | CYLD-809 | chr16:50784548-50784575 |
| CYLD-62 | chr16:50741190-50741217 | CYLD-810 | chr16:50786718-50786745 |
| CYLD-63 | chr16:50741192-50741219 | CYLD-811 | chr16:50786730-50786757 |
| CYLD-64 | chr16:50741227-50741254 | CYLD-812 | chr16:50786734-50786761 |
| CYLD-65 | chr16:50741230-50741257 | CYLD-813 | chr16:50786822-50786849 |
| CYLD-66 | chr16:50741231-50741258 | CYLD-814 | chr16:50786833-50786860 |
| CYLD-67 | chr16:50741244-50741271 | CYLD-815 | chr16:50786867-50786894 |
| CYLD-68 | chr16:50741248-50741275 | CYLD-816 | chr16:50786872-50786899 |
| CYLD-69 | chr16:50741251-50741278 | CYLD-817 | chr16:50786883-50786910 |
| CYLD-70 | chr16:50741256-50741283 | CYLD-818 | chr16:50786886-50786913 |
| CYLD-71 | chr16:50741257-50741284 | CYLD-819 | chr16:50786889-50786916 |
| CYLD-72 | chr16:50741258-50741285 | CYLD-820 | chr16:50786899-50786926 |
| CYLD-73 | chr16:50741259-50741286 | CYLD-821 | chr16:50786920-50786947 |
| CYLD-74 | chr16:50741260-50741287 | CYLD-822 | chr16:50786932-50786959 |
| CYLD-75 | chr16:50741263-50741290 | CYLD-823 | chr16:50786951-50786978 |
| CYLD-76 | chr16:50741264-50741291 | CYLD-824 | chr16:50787009-50787036 |
| CYLD-77 | chr16:50741265-50741292 | CYLD-825 | chr16:50787010-50787037 |
| CYLD-78 | chr16:50741267-50741294 | CYLD-826 | chr16:50787011-50787038 |
| CYLD-79 | chr16:50741275-50741302 | CYLD-827 | chr16:50787012-50787039 |
| CYLD-80 | chr16:50741278-50741305 | CYLD-828 | chr16:50787013-50787040 |
| CYLD-81 | chr16:50741279-50741306 | CYLD-829 | chr16:50787751-50787778 |
| CYLD-82 | chr16:50741285-50741312 | CYLD-830 | chr16:50787767-50787794 |
| CYLD-83 | chr16:50741309-50741336 | CYLD-831 | chr16:50787781-50787808 |
| CYLD-84 | chr16:50741310-50741337 | CYLD-832 | chr16:50787788-50787815 |
| CYLD-85 | chr16:50741317-50741344 | CYLD-833 | chr16:50787802-50787829 |
| CYLD-86 | chr16:50741318-50741345 | CYLD-834 | chr16:50787803-50787830 |
| CYLD-87 | chr16:50741327-50741354 | CYLD-835 | chr16:50787826-50787853 |
| CYLD-88 | chr16:50741329-50741356 | CYLD-836 | chr16:50787836-50787863 |
| CYLD-89 | chr16:50741330-50741357 | CYLD-837 | chr16:50787855-50787882 |
| CYLD-90 | chr16:50741331-50741358 | CYLD-838 | chr16:50787857-50787884 |
| CYLD-91 | chr16:50741335-50741362 | CYLD-839 | chr16:50787910-50787937 |
| CYLD-92 | chr16:50741338-50741365 | CYLD-840 | chr16:50787933-50787960 |
| CYLD-93 | chr16:50741344-50741371 | CYLD-841 | chr16:50787939-50787966 |
| CYLD-94 | chr16:50741345-50741372 | CYLD-842 | chr16:50787940-50787967 |
| CYLD-95 | chr16:50741347-50741374 | CYLD-843 | chr16:50787951-50787978 |
| CYLD-96 | chr16:50741348-50741375 | CYLD-844 | chr16:50791434-50791461 |
| CYLD-97 | chr16:50741352-50741379 | CYLD-845 | chr16:50791436-50791463 |
| CYLD-98 | chr16:50741371-50741398 | CYLD-846 | chr16:50791447-50791474 |
| CYLD-99 | chr16:50741383-50741410 | CYLD-847 | chr16:50791459-50791486 |
| CYLD-100 | chr16:50741386-50741413 | CYLD-848 | chr16:50791460-50791487 |
| CYLD-101 | chr16:50741389-50741416 | CYLD-849 | chr16:50791498-50791525 |
| CYLD-102 | chr16:50741393-50741420 | CYLD-850 | chr16:50791539-50791566 |
| CYLD-103 | chr16:50741394-50741421 | CYLD-851 | chr16:50791547-50791574 |
| CYLD-104 | chr16:50741414-50741441 | CYLD-852 | chr16:50791580-50791607 |
| CYLD-105 | chr16:50741427-50741454 | CYLD-853 | chr16:50791599-50791626 |
| CYLD-106 | chr16:50741428-50741455 | CYLD-854 | chr16:50791630-50791657 |
| CYLD-107 | chr16:50741432-50741459 | CYLD-855 | chr16:50791631-50791658 |
| CYLD-108 | chr16:50741441-50741468 | CYLD-856 | chr16:50791632-50791659 |
| CYLD-109 | chr16:50741451-50741478 | CYLD-857 | chr16:50791664-50791691 |
| CYLD-110 | chr16:50741476-50741503 | CYLD-858 | chr16:50791675-50791702 |
| CYLD-111 | chr16:50741483-50741510 | CYLD-859 | chr16:50791684-50791711 |
| CYLD-112 | chr16:50741495-50741522 | CYLD-860 | chr16:50791705-50791732 |
| CYLD-113 | chr16:50741499-50741526 | CYLD-861 | chr16:50791706-50791733 |
| CYLD-114 | chr16:50741508-50741535 | CYLD-862 | chr16:50791707-50791734 |
| CYLD-115 | chr16:50741510-50741537 | CYLD-863 | chr16:50791724-50791751 |
| CYLD-116 | chr16:50741518-50741545 | CYLD-864 | chr16:50792471-50792498 |
| CYLD-117 | chr16:50741534-50741561 | CYLD-865 | chr16:50792487-50792514 |
| CYLD-118 | chr16:50741555-50741582 | CYLD-866 | chr16:50792507-50792534 |
| CYLD-119 | chr16:50741570-50741597 | CYLD-867 | chr16:50792516-50792543 |
| CYLD-120 | chr16:50741577-50741604 | CYLD-868 | chr16:50792517-50792544 |
| CYLD-121 | chr16:50741586-50741613 | CYLD-869 | chr16:50792520-50792547 |
| CYLD-122 | chr16:50741606-50741633 | CYLD-870 | chr16:50792570-50792597 |
| CYLD-123 | chr16:50741614-50741641 | CYLD-871 | chr16:50792600-50792627 |
| CYLD-124 | chr16:50741637-50741664 | CYLD-872 | chr16:50792607-50792634 |
| CYLD-125 | chr16:50741638-50741665 | CYLD-873 | chr16:50792624-50792651 |
| CYLD-126 | chr16:50741645-50741672 | CYLD-874 | chr16:50792648-50792675 |
| CYLD-127 | chr16:50741655-50741682 | CYLD-875 | chr16:50792666-50792693 |
| CYLD-128 | chr16:50741674-50741701 | CYLD-876 | chr16:50792712-50792739 |
| CYLD-129 | chr16:50741675-50741702 | CYLD-877 | chr16:50792720-50792747 |
| CYLD-130 | chr16:50741679-50741706 | CYLD-878 | chr16:50792750-50792777 |
| CYLD-131 | chr16:50741680-50741707 | CYLD-879 | chr16:50792751-50792778 |
| CYLD-132 | chr16:50741685-50741712 | CYLD-880 | chr16:50792766-50792793 |
| CYLD-133 | chr16:50741687-50741714 | CYLD-881 | chr16:50792784-50792811 |
| CYLD-134 | chr16:50741690-50741717 | CYLD-882 | chr16:50792805-50792832 |
| CYLD-135 | chr16:50741691-50741718 | CYLD-883 | chr16:50793437-50793464 |
| CYLD-136 | chr16:50741698-50741725 | CYLD-884 | chr16:50793450-50793477 |
| CYLD-137 | chr16:50741699-50741726 | CYLD-885 | chr16:50793461-50793488 |
| CYLD-138 | chr16:50741702-50741729 | CYLD-886 | chr16:50793512-50793539 |
| CYLD-139 | chr16:50741705-50741732 | CYLD-887 | chr16:50793523-50793550 |
| CYLD-140 | chr16:50741711-50741738 | CYLD-888 | chr16:50793524-50793551 |
| CYLD-141 | chr16:50741714-50741741 | CYLD-889 | chr16:50793528-50793555 |
| CYLD-142 | chr16:50741735-50741762 | CYLD-890 | chr16:50793529-50793556 |
| CYLD-143 | chr16:50741752-50741779 | CYLD-891 | chr16:50793530-50793557 |
| CYLD-144 | chr16:50741753-50741780 | CYLD-892 | chr16:50793535-50793562 |
| CYLD-145 | chr16:50741754-50741781 | CYLD-893 | chr16:50793543-50793570 |
| CYLD-146 | chr16:50741757-50741784 | CYLD-894 | chr16:50793546-50793573 |
| CYLD-147 | chr16:50741758-50741785 | CYLD-895 | chr16:50793547-50793574 |
| CYLD-148 | chr16:50741759-50741786 | CYLD-896 | chr16:50793548-50793575 |
| CYLD-149 | chr16:50741762-50741789 | CYLD-897 | chr16:50793549-50793576 |
| CYLD-150 | chr16:50741765-50741792 | CYLD-898 | chr16:50793559-50793586 |
| CYLD-151 | chr16:50741775-50741802 | CYLD-899 | chr16:50793587-50793614 |
| CYLD-152 | chr16:50741792-50741819 | CYLD-900 | chr16:50793600-50793627 |
| CYLD-153 | chr16:50741793-50741820 | CYLD-901 | chr16:50793611-50793638 |
| CYLD-154 | chr16:50741799-50741826 | CYLD-902 | chr16:50793651-50793678 |
| CYLD-155 | chr16:50741806-50741833 | CYLD-903 | chr16:50793660-50793687 |
| CYLD-156 | chr16:50741807-50741834 | CYLD-904 | chr16:50793673-50793700 |
| CYLD-157 | chr16:50741824-50741851 | CYLD-905 | chr16:50793674-50793701 |
| CYLD-158 | chr16:50741825-50741852 | CYLD-906 | chr16:50793717-50793744 |
| CYLD-159 | chr16:50741828-50741855 | CYLD-907 | chr16:50793744-50793771 |
| CYLD-160 | chr16:50741831-50741858 | CYLD-908 | chr16:50794086-50794113 |
| CYLD-161 | chr16:50741835-50741862 | CYLD-909 | chr16:50794087-50794114 |
| CYLD-162 | chr16:50741838-50741865 | CYLD-910 | chr16:50794092-50794119 |
| CYLD-163 | chr16:50741848-50741875 | CYLD-911 | chr16:50794093-50794120 |
| CYLD-164 | chr16:50741851-50741878 | CYLD-912 | chr16:50794112-50794139 |
| CYLD-165 | chr16:50741852-50741879 | CYLD-913 | chr16:50794116-50794143 |
| CYLD-166 | chr16:50741853-50741880 | CYLD-914 | chr16:50794126-50794153 |
| CYLD-167 | chr16:50741856-50741883 | CYLD-915 | chr16:50794152-50794179 |
| CYLD-168 | chr16:50741857-50741884 | CYLD-916 | chr16:50794153-50794180 |
| CYLD-169 | chr16:50741858-50741885 | CYLD-917 | chr16:50794158-50794185 |
| CYLD-170 | chr16:50741868-50741895 | CYLD-918 | chr16:50794167-50794194 |
| CYLD-171 | chr16:50741869-50741896 | CYLD-919 | chr16:50794170-50794197 |
| CYLD-172 | chr16:50741871-50741898 | CYLD-920 | chr16:50794174-50794201 |
| CYLD-173 | chr16:50741878-50741905 | CYLD-921 | chr16:50794180-50794207 |
| CYLD-174 | chr16:50741881-50741908 | CYLD-922 | chr16:50794185-50794212 |
| CYLD-175 | chr16:50741884-50741911 | CYLD-923 | chr16:50794203-50794230 |
| CYLD-176 | chr16:50741888-50741915 | CYLD-924 | chr16:50794205-50794232 |
| CYLD-177 | chr16:50741894-50741921 | CYLD-925 | chr16:50794214-50794241 |
| CYLD-178 | chr16:50741895-50741922 | CYLD-926 | chr16:50794217-50794244 |
| CYLD-179 | chr16:50741899-50741926 | CYLD-927 | chr16:50794224-50794251 |
| CYLD-180 | chr16:50741904-50741931 | CYLD-928 | chr16:50794250-50794277 |
| CYLD-181 | chr16:50741905-50741932 | CYLD-929 | chr16:50794251-50794278 |
| CYLD-182 | chr16:50741909-50741936 | CYLD-930 | chr16:50794256-50794283 |
| CYLD-183 | chr16:50741912-50741939 | CYLD-931 | chr16:50794257-50794284 |
| CYLD-184 | chr16:50741916-50741943 | CYLD-932 | chr16:50794262-50794289 |
| CYLD-185 | chr16:50741918-50741945 | CYLD-933 | chr16:50794263-50794290 |
| CYLD-186 | chr16:50741927-50741954 | CYLD-934 | chr16:50794264-50794291 |
| CYLD-187 | chr16:50741928-50741955 | CYLD-935 | chr16:50794270-50794297 |
| CYLD-188 | chr16:50741930-50741957 | CYLD-936 | chr16:50794275-50794302 |
| CYLD-189 | chr16:50741931-50741958 | CYLD-937 | chr16:50794276-50794303 |
| CYLD-190 | chr16:50741942-50741969 | CYLD-938 | chr16:50794293-50794320 |
| CYLD-191 | chr16:50741952-50741979 | CYLD-939 | chr16:50794304-50794331 |
| CYLD-192 | chr16:50741959-50741986 | CYLD-940 | chr16:50794305-50794332 |
| CYLD-193 | chr16:50741960-50741987 | CYLD-941 | chr16:50794310-50794337 |
| CYLD-194 | chr16:50741964-50741991 | CYLD-942 | chr16:50794351-50794378 |
| CYLD-195 | chr16:50741970-50741997 | CYLD-943 | chr16:50794352-50794379 |
| CYLD-196 | chr16:50741971-50741998 | CYLD-944 | chr16:50794356-50794383 |
| CYLD-197 | chr16:50741975-50742002 | CYLD-945 | chr16:50794370-50794397 |
| CYLD-198 | chr16:50741984-50742011 | CYLD-946 | chr16:50794389-50794416 |
| CYLD-199 | chr16:50741987-50742014 | CYLD-947 | chr16:50794393-50794420 |
| CYLD-200 | chr16:50741992-50742019 | CYLD-948 | chr16:50794397-50794424 |
| CYLD-201 | chr16:50742018-50742045 | CYLD-949 | chr16:50794398-50794425 |
| CYLD-202 | chr16:50742019-50742046 | CYLD-950 | chr16:50794402-50794429 |
| CYLD-203 | chr16:50742020-50742047 | CYLD-951 | chr16:50794417-50794444 |
| CYLD-204 | chr16:50742021-50742048 | CYLD-952 | chr16:50794431-50794458 |
| CYLD-205 | chr16:50742024-50742051 | CYLD-953 | chr16:50794437-50794464 |
| CYLD-206 | chr16:50742027-50742054 | CYLD-954 | chr16:50794438-50794465 |
| CYLD-207 | chr16:50742028-50742055 | CYLD-955 | chr16:50794441-50794468 |
| CYLD-208 | chr16:50742029-50742056 | CYLD-956 | chr16:50794444-50794471 |
| CYLD-209 | chr16:50742030-50742057 | CYLD-957 | chr16:50794454-50794481 |
| CYLD-210 | chr16:50742033-50742060 | CYLD-958 | chr16:50794455-50794482 |
| CYLD-211 | chr16:50742035-50742062 | CYLD-959 | chr16:50794469-50794496 |
| CYLD-212 | chr16:50742036-50742063 | CYLD-960 | chr16:50794470-50794497 |
| CYLD-213 | chr16:50742039-50742066 | CYLD-961 | chr16:50794502-50794529 |
| CYLD-214 | chr16:50742040-50742067 | CYLD-962 | chr16:50794513-50794540 |
| CYLD-215 | chr16:50742041-50742068 | CYLD-963 | chr16:50794523-50794550 |
| CYLD-216 | chr16:50742045-50742072 | CYLD-964 | chr16:50796216-50796243 |
| CYLD-217 | chr16:50742046-50742073 | CYLD-965 | chr16:50796225-50796252 |
| CYLD-218 | chr16:50742047-50742074 | CYLD-966 | chr16:50796226-50796253 |
| CYLD-219 | chr16:50742048-50742075 | CYLD-967 | chr16:50796242-50796269 |
| CYLD-220 | chr16:50742049-50742076 | CYLD-968 | chr16:50796249-50796276 |
| CYLD-221 | chr16:50742050-50742077 | CYLD-969 | chr16:50796250-50796277 |
| CYLD-222 | chr16:50742051-50742078 | CYLD-970 | chr16:50796293-50796320 |
| CYLD-223 | chr16:50742054-50742081 | CYLD-971 | chr16:50796294-50796321 |
| CYLD-224 | chr16:50742055-50742082 | CYLD-972 | chr16:50796297-50796324 |
| CYLD-225 | chr16:50742061-50742088 | CYLD-973 | chr16:50796300-50796327 |
| CYLD-226 | chr16:50742068-50742095 | CYLD-974 | chr16:50796309-50796336 |
| CYLD-227 | chr16:50742073-50742100 | CYLD-975 | chr16:50796310-50796337 |
| CYLD-228 | chr16:50742081-50742108 | CYLD-976 | chr16:50796318-50796345 |
| CYLD-229 | chr16:50742082-50742109 | CYLD-977 | chr16:50796347-50796374 |
| CYLD-230 | chr16:50742083-50742110 | CYLD-978 | chr16:50796348-50796375 |
| CYLD-231 | chr16:50742084-50742111 | CYLD-979 | chr16:50796357-50796384 |
| CYLD-232 | chr16:50742095-50742122 | CYLD-980 | chr16:50796358-50796385 |
| CYLD-233 | chr16:50742098-50742125 | CYLD-981 | chr16:50796359-50796386 |
| CYLD-234 | chr16:50742109-50742136 | CYLD-982 | chr16:50796364-50796391 |
| CYLD-235 | chr16:50742110-50742137 | CYLD-983 | chr16:50796365-50796392 |
| CYLD-236 | chr16:50742112-50742139 | CYLD-984 | chr16:50796371-50796398 |
| CYLD-237 | chr16:50742113-50742140 | CYLD-985 | chr16:50796389-50796416 |
| CYLD-238 | chr16:50742116-50742143 | CYLD-986 | chr16:50796397-50796424 |
| CYLD-239 | chr16:50742117-50742144 | CYLD-987 | chr16:50796403-50796430 |
| CYLD-240 | chr16:50742122-50742149 | CYLD-988 | chr16:50796408-50796435 |
| CYLD-241 | chr16:50742123-50742150 | CYLD-989 | chr16:50796411-50796438 |
| CYLD-242 | chr16:50742124-50742151 | CYLD-990 | chr16:50796420-50796447 |
| CYLD-243 | chr16:50742130-50742157 | CYLD-991 | chr16:50796430-50796457 |
| CYLD-244 | chr16:50742131-50742158 | CYLD-992 | chr16:50796478-50796505 |
| CYLD-245 | chr16:50742132-50742159 | CYLD-993 | chr16:50796485-50796512 |
| CYLD-246 | chr16:50742136-50742163 | CYLD-994 | chr16:50796486-50796513 |
| CYLD-247 | chr16:50742141-50742168 | CYLD-995 | chr16:50796487-50796514 |
| CYLD-248 | chr16:50742142-50742169 | CYLD-996 | chr16:50796494-50796521 |
| CYLD-249 | chr16:50742148-50742175 | CYLD-997 | chr16:50796495-50796522 |
| CYLD-250 | chr16:50742149-50742176 | CYLD-998 | chr16:50796500-50796527 |
| CYLD-251 | chr16:50742153-50742180 | CYLD-999 | chr16:50796515-50796542 |
| CYLD-252 | chr16:50742154-50742181 | CYLD-1000 | chr16:50796548-50796575 |
| CYLD-253 | chr16:50742155-50742182 | CYLD-1001 | chr16:50796549-50796576 |
| CYLD-254 | chr16:50742160-50742187 | CYLD-1002 | chr16:50796573-50796600 |
| CYLD-255 | chr16:50742161-50742188 | CYLD-1003 | chr16:50796579-50796606 |
| CYLD-256 | chr16:50742162-50742189 | CYLD-1004 | chr16:50796591-50796618 |
| CYLD-257 | chr16:50742163-50742190 | CYLD-1005 | chr16:50796597-50796624 |
| CYLD-258 | chr16:50742164-50742191 | CYLD-1006 | chr16:50796611-50796638 |
| CYLD-259 | chr16:50742165-50742192 | CYLD-1007 | chr16:50796626-50796653 |
| CYLD-260 | chr16:50742168-50742195 | CYLD-1008 | chr16:50796642-50796669 |
| CYLD-261 | chr16:50742169-50742196 | CYLD-1009 | chr16:50796672-50796699 |
| CYLD-262 | chr16:50742170-50742197 | CYLD-1010 | chr16:50796673-50796700 |
| CYLD-263 | chr16:50742185-50742212 | CYLD-1011 | chr16:50796699-50796726 |
| CYLD-264 | chr16:50742190-50742217 | CYLD-1012 | chr16:50796759-50796786 |
| CYLD-265 | chr16:50742203-50742230 | CYLD-1013 | chr16:50796775-50796802 |
| CYLD-266 | chr16:50742204-50742231 | CYLD-1014 | chr16:50796797-50796824 |
| CYLD-267 | chr16:50742205-50742232 | CYLD-1015 | chr16:50796835-50796862 |
| CYLD-268 | chr16:50742206-50742233 | CYLD-1016 | chr16:50796846-50796873 |
| CYLD-269 | chr16:50742208-50742235 | CYLD-1017 | chr16:50796854-50796881 |
| CYLD-270 | chr16:50742209-50742236 | CYLD-1018 | chr16:50796861-50796888 |
| CYLD-271 | chr16:50742211-50742238 | CYLD-1019 | chr16:50796883-50796910 |
| CYLD-272 | chr16:50742212-50742239 | CYLD-1020 | chr16:50796893-50796920 |
| CYLD-273 | chr16:50742213-50742240 | CYLD-1021 | chr16:50796904-50796931 |
| CYLD-274 | chr16:50742216-50742243 | CYLD-1022 | chr16:50796918-50796945 |
| CYLD-275 | chr16:50742220-50742247 | CYLD-1023 | chr16:50796924-50796951 |
| CYLD-276 | chr16:50742223-50742250 | CYLD-1024 | chr16:50796970-50796997 |
| CYLD-277 | chr16:50742224-50742251 | CYLD-1025 | chr16:50797007-50797034 |
| CYLD-278 | chr16:50742227-50742254 | CYLD-1026 | chr16:50797048-50797075 |
| CYLD-279 | chr16:50742231-50742258 | CYLD-1027 | chr16:50797077-50797104 |
| CYLD-280 | chr16:50742241-50742268 | CYLD-1028 | chr16:50797095-50797122 |
| CYLD-281 | chr16:50742244-50742271 | CYLD-1029 | chr16:50797096-50797123 |
| CYLD-282 | chr16:50742254-50742281 | CYLD-1030 | chr16:50797099-50797126 |
| CYLD-283 | chr16:50742255-50742282 | CYLD-1031 | chr16:50797152-50797179 |
| CYLD-284 | chr16:50742261-50742288 | CYLD-1032 | chr16:50797153-50797180 |
| CYLD-285 | chr16:50742262-50742289 | CYLD-1033 | chr16:50797154-50797181 |
| CYLD-286 | chr16:50742263-50742290 | CYLD-1034 | chr16:50797158-50797185 |
| CYLD-287 | chr16:50742269-50742296 | CYLD-1035 | chr16:50797161-50797188 |
| CYLD-288 | chr16:50742274-50742301 | CYLD-1036 | chr16:50797162-50797189 |
| CYLD-289 | chr16:50742277-50742304 | CYLD-1037 | chr16:50797167-50797194 |
| CYLD-290 | chr16:50742280-50742307 | CYLD-1038 | chr16:50797168-50797195 |
| CYLD-291 | chr16:50742282-50742309 | CYLD-1039 | chr16:50797181-50797208 |
| CYLD-292 | chr16:50742290-50742317 | CYLD-1040 | chr16:50797219-50797246 |
| CYLD-293 | chr16:50742291-50742318 | CYLD-1041 | chr16:50797229-50797256 |
| CYLD-294 | chr16:50742293-50742320 | CYLD-1042 | chr16:50797253-50797280 |
| CYLD-295 | chr16:50742298-50742325 | CYLD-1043 | chr16:50797258-50797285 |
| CYLD-296 | chr16:50742299-50742326 | CYLD-1044 | chr16:50797259-50797286 |
| CYLD-297 | chr16:50742302-50742329 | CYLD-1045 | chr16:50797260-50797287 |
| CYLD-298 | chr16:50742304-50742331 | CYLD-1046 | chr16:50797285-50797312 |
| CYLD-299 | chr16:50742311-50742338 | CYLD-1047 | chr16:50797325-50797352 |
| CYLD-300 | chr16:50742319-50742346 | CYLD-1048 | chr16:50797335-50797362 |
| CYLD-301 | chr16:50742320-50742347 | CYLD-1049 | chr16:50797343-50797370 |
| CYLD-302 | chr16:50742321-50742348 | CYLD-1050 | chr16:50797361-50797388 |
| CYLD-303 | chr16:50742328-50742355 | CYLD-1051 | chr16:50797362-50797389 |
| CYLD-304 | chr16:50742331-50742358 | CYLD-1052 | chr16:50797370-50797397 |
| CYLD-305 | chr16:50742332-50742359 | CYLD-1053 | chr16:50797384-50797411 |
| CYLD-306 | chr16:50742333-50742360 | CYLD-1054 | chr16:50797407-50797434 |
| CYLD-307 | chr16:50742335-50742362 | CYLD-1055 | chr16:50797457-50797484 |
| CYLD-308 | chr16:50742336-50742363 | CYLD-1056 | chr16:50797465-50797492 |
| CYLD-309 | chr16:50742343-50742370 | CYLD-1057 | chr16:50797466-50797493 |
| CYLD-310 | chr16:50742344-50742371 | CYLD-1058 | chr16:50797535-50797562 |
| CYLD-311 | chr16:50742349-50742376 | CYLD-1059 | chr16:50797537-50797564 |
| CYLD-312 | chr16:50742350-50742377 | CYLD-1060 | chr16:50797538-50797565 |
| CYLD-313 | chr16:50742355-50742382 | CYLD-1061 | chr16:50797584-50797611 |
| CYLD-314 | chr16:50742359-50742386 | CYLD-1062 | chr16:50797585-50797612 |
| CYLD-315 | chr16:50742364-50742391 | CYLD-1063 | chr16:50797599-50797626 |
| CYLD-316 | chr16:50742365-50742392 | CYLD-1064 | chr16:50797603-50797630 |
| CYLD-317 | chr16:50742371-50742398 | CYLD-1065 | chr16:50797622-50797649 |
| CYLD-318 | chr16:50742372-50742399 | CYLD-1066 | chr16:50797628-50797655 |
| CYLD-319 | chr16:50742375-50742402 | CYLD-1067 | chr16:50797658-50797685 |
| CYLD-320 | chr16:50742380-50742407 | CYLD-1068 | chr16:50797699-50797726 |
| CYLD-321 | chr16:50742381-50742408 | CYLD-1069 | chr16:50797701-50797728 |
| CYLD-322 | chr16:50742389-50742416 | CYLD-1070 | chr16:50797765-50797792 |
| CYLD-323 | chr16:50742390-50742417 | CYLD-1071 | chr16:50797804-50797831 |
| CYLD-324 | chr16:50742391-50742418 | CYLD-1072 | chr16:50797809-50797836 |
| CYLD-325 | chr16:50742394-50742421 | CYLD-1073 | chr16:50797810-50797837 |
| CYLD-326 | chr16:50742395-50742422 | CYLD-1074 | chr16:50797820-50797847 |
| CYLD-327 | chr16:50742403-50742430 | CYLD-1075 | chr16:50797858-50797885 |
| CYLD-328 | chr16:50742404-50742431 | CYLD-1076 | chr16:50797872-50797899 |
| CYLD-329 | chr16:50742405-50742432 | CYLD-1077 | chr16:50797937-50797964 |
| CYLD-330 | chr16:50742406-50742433 | CYLD-1078 | chr16:50797959-50797986 |
| CYLD-331 | chr16:50742407-50742434 | CYLD-1079 | chr16:50797963-50797990 |
| CYLD-332 | chr16:50742408-50742435 | CYLD-1080 | chr16:50797964-50797991 |
| CYLD-333 | chr16:50742409-50742436 | CYLD-1081 | chr16:50797965-50797992 |
| CYLD-334 | chr16:50742410-50742437 | CYLD-1082 | chr16:50797968-50797995 |
| CYLD-335 | chr16:50742413-50742440 | CYLD-1083 | chr16:50797989-50798016 |
| CYLD-336 | chr16:50742417-50742444 | CYLD-1084 | chr16:50797990-50798017 |
| CYLD-337 | chr16:50742418-50742445 | CYLD-1085 | chr16:50797991-50798018 |
| CYLD-338 | chr16:50742421-50742448 | CYLD-1086 | chr16:50797996-50798023 |
| CYLD-339 | chr16:50742422-50742449 | CYLD-1087 | chr16:50798010-50798037 |
| CYLD-340 | chr16:50742423-50742450 | CYLD-1088 | chr16:50798011-50798038 |
| CYLD-341 | chr16:50742424-50742451 | CYLD-1089 | chr16:50798017-50798044 |
| CYLD-342 | chr16:50742428-50742455 | CYLD-1090 | chr16:50798018-50798045 |
| CYLD-343 | chr16:50742429-50742456 | CYLD-1091 | chr16:50798043-50798070 |
| CYLD-344 | chr16:50742430-50742457 | CYLD-1092 | chr16:50798044-50798071 |
| CYLD-345 | chr16:50742431-50742458 | CYLD-1093 | chr16:50798050-50798077 |
| CYLD-346 | chr16:50742434-50742461 | CYLD-1094 | chr16:50798051-50798078 |
| CYLD-347 | chr16:50742435-50742462 | CYLD-1095 | chr16:50798055-50798082 |
| CYLD-348 | chr16:50742436-50742463 | CYLD-1096 | chr16:50798056-50798083 |
| CYLD-349 | chr16:50742440-50742467 | CYLD-1097 | chr16:50798058-50798085 |
| CYLD-350 | chr16:50742441-50742468 | CYLD-1098 | chr16:50798061-50798088 |
| CYLD-351 | chr16:50742445-50742472 | CYLD-1099 | chr16:50798062-50798089 |
| CYLD-352 | chr16:50742446-50742473 | CYLD-1100 | chr16:50798063-50798090 |
| CYLD-353 | chr16:50742447-50742474 | CYLD-1101 | chr16:50798064-50798091 |
| CYLD-354 | chr16:50742458-50742485 | CYLD-1102 | chr16:50798065-50798092 |
| CYLD-355 | chr16:50742460-50742487 | CYLD-1103 | chr16:50798079-50798106 |
| CYLD-356 | chr16:50742461-50742488 | CYLD-1104 | chr16:50798107-50798134 |
| CYLD-357 | chr16:50742472-50742499 | CYLD-1105 | chr16:50798126-50798153 |
| CYLD-358 | chr16:50742478-50742505 | CYLD-1106 | chr16:50798135-50798162 |
| CYLD-359 | chr16:50742479-50742506 | CYLD-1107 | chr16:50798142-50798169 |
| CYLD-360 | chr16:50742481-50742508 | CYLD-1108 | chr16:50798143-50798170 |
| CYLD-361 | chr16:50742485-50742512 | CYLD-1109 | chr16:50798148-50798175 |
| CYLD-362 | chr16:50742486-50742513 | CYLD-1110 | chr16:50798149-50798176 |
| CYLD-363 | chr16:50742487-50742514 | CYLD-1111 | chr16:50798192-50798219 |
| CYLD-364 | chr16:50742490-50742517 | CYLD-1112 | chr16:50798195-50798222 |
| CYLD-365 | chr16:50742491-50742518 | CYLD-1113 | chr16:50798205-50798232 |
| CYLD-366 | chr16:50742492-50742519 | CYLD-1114 | chr16:50798215-50798242 |
| CYLD-367 | chr16:50742493-50742520 | CYLD-1115 | chr16:50798229-50798256 |
| CYLD-368 | chr16:50742501-50742528 | CYLD-1116 | chr16:50798239-50798266 |
| CYLD-369 | chr16:50742502-50742529 | CYLD-1117 | chr16:50798246-50798273 |
| CYLD-370 | chr16:50742521-50742548 | CYLD-1118 | chr16:50798250-50798277 |
| CYLD-371 | chr16:50742537-50742564 | CYLD-1119 | chr16:50798251-50798278 |
| CYLD-372 | chr16:50742538-50742565 | CYLD-1120 | chr16:50798253-50798280 |
| CYLD-373 | chr16:50742539-50742566 | CYLD-1121 | chr16:50798273-50798300 |
| CYLD-374 | chr16:50742540-50742567 | CYLD-1122 | chr16:50798275-50798302 |
| CYLD-375 | chr16:50742543-50742570 | CYLD-1123 | chr16:50798287-50798314 |
| CYLD-376 | chr16:50742544-50742571 | CYLD-1124 | chr16:50798299-50798326 |
| CYLD-377 | chr16:50742547-50742574 | CYLD-1125 | chr16:50798303-50798330 |
| CYLD-378 | chr16:50742548-50742575 | CYLD-1126 | chr16:50798337-50798364 |
| CYLD-379 | chr16:50742549-50742576 | CYLD-1127 | chr16:50798395-50798422 |
| CYLD-380 | chr16:50742550-50742577 | CYLD-1128 | chr16:50798437-50798464 |
| CYLD-381 | chr16:50742551-50742578 | CYLD-1129 | chr16:50798440-50798467 |
| CYLD-382 | chr16:50742553-50742580 | CYLD-1130 | chr16:50798484-50798511 |
| CYLD-383 | chr16:50742554-50742581 | CYLD-1131 | chr16:50798485-50798512 |
| CYLD-384 | chr16:50742555-50742582 | CYLD-1132 | chr16:50798497-50798524 |
| CYLD-385 | chr16:50742556-50742583 | CYLD-1133 | chr16:50798508-50798535 |
| CYLD-386 | chr16:50742559-50742586 | CYLD-1134 | chr16:50798518-50798545 |
| CYLD-387 | chr16:50742566-50742593 | CYLD-1135 | chr16:50798519-50798546 |
| CYLD-388 | chr16:50742569-50742596 | CYLD-1136 | chr16:50798526-50798553 |
| CYLD-389 | chr16:50742570-50742597 | CYLD-1137 | chr16:50798549-50798576 |
| CYLD-390 | chr16:50742579-50742606 | CYLD-1138 | chr16:50798551-50798578 |
| CYLD-391 | chr16:50742585-50742612 | CYLD-1139 | chr16:50798552-50798579 |
| CYLD-392 | chr16:50742587-50742614 | CYLD-1140 | chr16:50798556-50798583 |
| CYLD-393 | chr16:50742589-50742616 | CYLD-1141 | chr16:50798561-50798588 |
| CYLD-394 | chr16:50742596-50742623 | CYLD-1142 | chr16:50798562-50798589 |
| CYLD-395 | chr16:50742598-50742625 | CYLD-1143 | chr16:50798563-50798590 |
| CYLD-396 | chr16:50742599-50742626 | CYLD-1144 | chr16:50798565-50798592 |
| CYLD-397 | chr16:50742600-50742627 | CYLD-1145 | chr16:50798574-50798601 |
| CYLD-398 | chr16:50742603-50742630 | CYLD-1146 | chr16:50798587-50798614 |
| CYLD-399 | chr16:50742604-50742631 | CYLD-1147 | chr16:50798605-50798632 |
| CYLD-400 | chr16:50742605-50742632 | CYLD-1148 | chr16:50798648-50798675 |
| CYLD-401 | chr16:50742607-50742634 | CYLD-1149 | chr16:50798653-50798680 |
| CYLD-402 | chr16:50742617-50742644 | CYLD-1150 | chr16:50798658-50798685 |
| CYLD-403 | chr16:50742626-50742653 | CYLD-1151 | chr16:50798675-50798702 |
| CYLD-404 | chr16:50742627-50742654 | CYLD-1152 | chr16:50798684-50798711 |
| CYLD-405 | chr16:50742632-50742659 | CYLD-1153 | chr16:50798697-50798724 |
| CYLD-406 | chr16:50742633-50742660 | CYLD-1154 | chr16:50798698-50798725 |
| CYLD-407 | chr16:50742634-50742661 | CYLD-1155 | chr16:50798711-50798738 |
| CYLD-408 | chr16:50742641-50742668 | CYLD-1156 | chr16:50798715-50798742 |
| CYLD-409 | chr16:50742645-50742672 | CYLD-1157 | chr16:50798723-50798750 |
| CYLD-410 | chr16:50742649-50742676 | CYLD-1158 | chr16:50798730-50798757 |
| CYLD-411 | chr16:50742654-50742681 | CYLD-1159 | chr16:50798737-50798764 |
| CYLD-412 | chr16:50742658-50742685 | CYLD-1160 | chr16:50798742-50798769 |
| CYLD-413 | chr16:50742659-50742686 | CYLD-1161 | chr16:50798746-50798773 |
| CYLD-414 | chr16:50742665-50742692 | CYLD-1162 | chr16:50798756-50798783 |
| CYLD-415 | chr16:50742668-50742695 | CYLD-1163 | chr16:50798757-50798784 |
| CYLD-416 | chr16:50742670-50742697 | CYLD-1164 | chr16:50798760-50798787 |
| CYLD-417 | chr16:50742680-50742707 | CYLD-1165 | chr16:50798761-50798788 |
| CYLD-418 | chr16:50742685-50742712 | CYLD-1166 | chr16:50798784-50798811 |
| CYLD-419 | chr16:50742692-50742719 | CYLD-1167 | chr16:50798789-50798816 |
| CYLD-420 | chr16:50742693-50742720 | CYLD-1168 | chr16:50798795-50798822 |
| CYLD-421 | chr16:50742694-50742721 | CYLD-1169 | chr16:50798800-50798827 |
| CYLD-422 | chr16:50742698-50742725 | CYLD-1170 | chr16:50798803-50798830 |
| CYLD-423 | chr16:50742699-50742726 | CYLD-1171 | chr16:50798831-50798858 |
| CYLD-424 | chr16:50742700-50742727 | CYLD-1172 | chr16:50798832-50798859 |
| CYLD-425 | chr16:50742703-50742730 | CYLD-1173 | chr16:50798833-50798860 |
| CYLD-426 | chr16:50742712-50742739 | CYLD-1174 | chr16:50798836-50798863 |
| CYLD-427 | chr16:50742728-50742755 | CYLD-1175 | chr16:50798837-50798864 |
| CYLD-428 | chr16:50742750-50742777 | CYLD-1176 | chr16:50798851-50798878 |
| CYLD-429 | chr16:50742751-50742778 | CYLD-1177 | chr16:50798852-50798879 |
| CYLD-430 | chr16:50742756-50742783 | CYLD-1178 | chr16:50798860-50798887 |
| CYLD-431 | chr16:50742760-50742787 | CYLD-1179 | chr16:50798861-50798888 |
| CYLD-432 | chr16:50742765-50742792 | CYLD-1180 | chr16:50798871-50798898 |
| CYLD-433 | chr16:50742766-50742793 | CYLD-1181 | chr16:50798874-50798901 |
| CYLD-434 | chr16:50742767-50742794 | CYLD-1182 | chr16:50798881-50798908 |
| CYLD-435 | chr16:50742768-50742795 | CYLD-1183 | chr16:50798882-50798909 |
| CYLD-436 | chr16:50742778-50742805 | CYLD-1184 | chr16:50798883-50798910 |
| CYLD-437 | chr16:50742796-50742823 | CYLD-1185 | chr16:50798886-50798913 |
| CYLD-438 | chr16:50742798-50742825 | CYLD-1186 | chr16:50798889-50798916 |
| CYLD-439 | chr16:50742801-50742828 | CYLD-1187 | chr16:50798904-50798931 |
| CYLD-440 | chr16:50742802-50742829 | CYLD-1188 | chr16:50798919-50798946 |
| CYLD-441 | chr16:50742810-50742837 | CYLD-1189 | chr16:50798920-50798947 |
| CYLD-442 | chr16:50742815-50742842 | CYLD-1190 | chr16:50798924-50798951 |
| CYLD-443 | chr16:50742828-50742855 | CYLD-1191 | chr16:50798925-50798952 |
| CYLD-444 | chr16:50742829-50742856 | CYLD-1192 | chr16:50798926-50798953 |
| CYLD-445 | chr16:50742856-50742883 | CYLD-1193 | chr16:50798930-50798957 |
| CYLD-446 | chr16:50742857-50742884 | CYLD-1194 | chr16:50798955-50798982 |
| CYLD-447 | chr16:50742858-50742885 | CYLD-1195 | chr16:50798961-50798988 |
| CYLD-448 | chr16:50742859-50742886 | CYLD-1196 | chr16:50798962-50798989 |
| CYLD-449 | chr16:50742860-50742887 | CYLD-1197 | chr16:50798963-50798990 |
| CYLD-450 | chr16:50742863-50742890 | CYLD-1198 | chr16:50798965-50798992 |
| CYLD-451 | chr16:50742864-50742891 | CYLD-1199 | chr16:50798966-50798993 |
| CYLD-452 | chr16:50742865-50742892 | CYLD-1200 | chr16:50798969-50798996 |
| CYLD-453 | chr16:50742866-50742893 | CYLD-1201 | chr16:50798978-50799005 |
| CYLD-454 | chr16:50742867-50742894 | CYLD-1202 | chr16:50798985-50799012 |
| CYLD-455 | chr16:50742874-50742901 | CYLD-1203 | chr16:50798986-50799013 |
| CYLD-456 | chr16:50742875-50742902 | CYLD-1204 | chr16:50798997-50799024 |
| CYLD-457 | chr16:50742876-50742903 | CYLD-1205 | chr16:50798998-50799025 |
| CYLD-458 | chr16:50742877-50742904 | CYLD-1206 | chr16:50798999-50799026 |
| CYLD-459 | chr16:50742878-50742905 | CYLD-1207 | chr16:50799000-50799027 |
| CYLD-460 | chr16:50742881-50742908 | CYLD-1208 | chr16:50799002-50799029 |
| CYLD-461 | chr16:50742882-50742909 | CYLD-1209 | chr16:50799014-50799041 |
| CYLD-462 | chr16:50742883-50742910 | CYLD-1210 | chr16:50799025-50799052 |
| CYLD-463 | chr16:50742884-50742911 | CYLD-1211 | chr16:50799026-50799053 |
| CYLD-464 | chr16:50742905-50742932 | CYLD-1212 | chr16:50799027-50799054 |
| CYLD-465 | chr16:50742911-50742938 | CYLD-1213 | chr16:50799031-50799058 |
| CYLD-466 | chr16:50742923-50742950 | CYLD-1214 | chr16:50799032-50799059 |
| CYLD-467 | chr16:50742934-50742961 | CYLD-1215 | chr16:50799040-50799067 |
| CYLD-468 | chr16:50742935-50742962 | CYLD-1216 | chr16:50799046-50799073 |
| CYLD-469 | chr16:50744607-50744634 | CYLD-1217 | chr16:50799047-50799074 |
| CYLD-470 | chr16:50744624-50744651 | CYLD-1218 | chr16:50799048-50799075 |
| CYLD-471 | chr16:50744636-50744663 | CYLD-1219 | chr16:50799064-50799091 |
| CYLD-472 | chr16:50744673-50744700 | CYLD-1220 | chr16:50799084-50799111 |
| CYLD-473 | chr16:50744709-50744736 | CYLD-1221 | chr16:50799107-50799134 |
| CYLD-474 | chr16:50744722-50744749 | CYLD-1222 | chr16:50799110-50799137 |
| CYLD-475 | chr16:50744775-50744802 | CYLD-1223 | chr16:50799121-50799148 |
| CYLD-476 | chr16:50744788-50744815 | CYLD-1224 | chr16:50799127-50799154 |
| CYLD-477 | chr16:50744815-50744842 | CYLD-1225 | chr16:50799130-50799157 |
| CYLD-478 | chr16:50744823-50744850 | CYLD-1226 | chr16:50799167-50799194 |
| CYLD-479 | chr16:50744827-50744854 | CYLD-1227 | chr16:50799193-50799220 |
| CYLD-480 | chr16:50744874-50744901 | CYLD-1228 | chr16:50799267-50799294 |
| CYLD-481 | chr16:50744875-50744902 | CYLD-1229 | chr16:50799272-50799299 |
| CYLD-482 | chr16:50744876-50744903 | CYLD-1230 | chr16:50799275-50799302 |
| CYLD-483 | chr16:50744890-50744917 | CYLD-1231 | chr16:50799293-50799320 |
| CYLD-484 | chr16:50744895-50744922 | CYLD-1232 | chr16:50799296-50799323 |
| CYLD-485 | chr16:50744915-50744942 | CYLD-1233 | chr16:50799301-50799328 |
| CYLD-486 | chr16:50744942-50744969 | CYLD-1234 | chr16:50799302-50799329 |
| CYLD-487 | chr16:50744949-50744976 | CYLD-1235 | chr16:50799311-50799338 |
| CYLD-488 | chr16:50744954-50744981 | CYLD-1236 | chr16:50799312-50799339 |
| CYLD-489 | chr16:50744955-50744982 | CYLD-1237 | chr16:50799313-50799340 |
| CYLD-490 | chr16:50749437-50749464 | CYLD-1238 | chr16:50799317-50799344 |
| CYLD-491 | chr16:50749522-50749549 | CYLD-1239 | chr16:50799344-50799371 |
| CYLD-492 | chr16:50749553-50749580 | CYLD-1240 | chr16:50799382-50799409 |
| CYLD-493 | chr16:50749561-50749588 | CYLD-1241 | chr16:50799383-50799410 |
| CYLD-494 | chr16:50749581-50749608 | CYLD-1242 | chr16:50799387-50799414 |
| CYLD-495 | chr16:50749582-50749609 | CYLD-1243 | chr16:50799388-50799415 |
| CYLD-496 | chr16:50749584-50749611 | CYLD-1244 | chr16:50799420-50799447 |
| CYLD-497 | chr16:50749608-50749635 | CYLD-1245 | chr16:50799424-50799451 |
| CYLD-498 | chr16:50749614-50749641 | CYLD-1246 | chr16:50799444-50799471 |
| CYLD-499 | chr16:50749662-50749689 | CYLD-1247 | chr16:50799492-50799519 |
| CYLD-500 | chr16:50749663-50749690 | CYLD-1248 | chr16:50799516-50799543 |
| CYLD-501 | chr16:50749682-50749709 | CYLD-1249 | chr16:50799527-50799554 |
| CYLD-502 | chr16:50749689-50749716 | CYLD-1250 | chr16:50799558-50799585 |
| CYLD-503 | chr16:50749719-50749746 | CYLD-1251 | chr16:50799565-50799592 |
| CYLD-504 | chr16:50749720-50749747 | CYLD-1252 | chr16:50799580-50799607 |
| CYLD-505 | chr16:50749728-50749755 | CYLD-1253 | chr16:50799586-50799613 |
| CYLD-506 | chr16:50749738-50749765 | CYLD-1254 | chr16:50799596-50799623 |
| CYLD-507 | chr16:50749739-50749766 | CYLD-1255 | chr16:50799601-50799628 |
| CYLD-508 | chr16:50749801-50749828 | CYLD-1256 | chr16:50799602-50799629 |
| CYLD-509 | chr16:50749802-50749829 | CYLD-1257 | chr16:50799611-50799638 |
| CYLD-510 | chr16:50749811-50749838 | CYLD-1258 | chr16:50799620-50799647 |
| CYLD-511 | chr16:50749812-50749839 | CYLD-1259 | chr16:50799621-50799648 |
| CYLD-512 | chr16:50749822-50749849 | CYLD-1260 | chr16:50799622-50799649 |
| CYLD-513 | chr16:50749837-50749864 | CYLD-1261 | chr16:50799623-50799650 |
| CYLD-514 | chr16:50749838-50749865 | CYLD-1262 | chr16:50799636-50799663 |
| CYLD-515 | chr16:50749839-50749866 | CYLD-1263 | chr16:50799676-50799703 |
| CYLD-516 | chr16:50749848-50749875 | CYLD-1264 | chr16:50799703-50799730 |
| CYLD-517 | chr16:50749865-50749892 | CYLD-1265 | chr16:50799704-50799731 |
| CYLD-518 | chr16:50749879-50749906 | CYLD-1266 | chr16:50799705-50799732 |
| CYLD-519 | chr16:50749883-50749910 | CYLD-1267 | chr16:50799712-50799739 |
| CYLD-520 | chr16:50749930-50749957 | CYLD-1268 | chr16:50799721-50799748 |
| CYLD-521 | chr16:50749933-50749960 | CYLD-1269 | chr16:50799743-50799770 |
| CYLD-522 | chr16:50749975-50750002 | CYLD-1270 | chr16:50799757-50799784 |
| CYLD-523 | chr16:50749993-50750020 | CYLD-1271 | chr16:50799758-50799785 |
| CYLD-524 | chr16:50749998-50750025 | CYLD-1272 | chr16:50799761-50799788 |
| CYLD-525 | chr16:50750009-50750036 | CYLD-1273 | chr16:50799785-50799812 |
| CYLD-526 | chr16:50750031-50750058 | CYLD-1274 | chr16:50799789-50799816 |
| CYLD-527 | chr16:50750036-50750063 | CYLD-1275 | chr16:50799811-50799838 |
| CYLD-528 | chr16:50750053-50750080 | CYLD-1276 | chr16:50799814-50799841 |
| CYLD-529 | chr16:50750054-50750081 | CYLD-1277 | chr16:50799866-50799893 |
| CYLD-530 | chr16:50750064-50750091 | CYLD-1278 | chr16:50799872-50799899 |
| CYLD-531 | chr16:50750067-50750094 | CYLD-1279 | chr16:50799876-50799903 |
| CYLD-532 | chr16:50750084-50750111 | CYLD-1280 | chr16:50799878-50799905 |
| CYLD-533 | chr16:50750085-50750112 | CYLD-1281 | chr16:50799886-50799913 |
| CYLD-534 | chr16:50750086-50750113 | CYLD-1282 | chr16:50799899-50799926 |
| CYLD-535 | chr16:50750102-50750129 | CYLD-1283 | chr16:50799900-50799927 |
| CYLD-536 | chr16:50750120-50750147 | CYLD-1284 | chr16:50799908-50799935 |
| CYLD-537 | chr16:50750125-50750152 | CYLD-1285 | chr16:50799942-50799969 |
| CYLD-538 | chr16:50750139-50750166 | CYLD-1286 | chr16:50799943-50799970 |
| CYLD-539 | chr16:50750149-50750176 | CYLD-1287 | chr16:50799947-50799974 |
| CYLD-540 | chr16:50750150-50750177 | CYLD-1288 | chr16:50799948-50799975 |
| CYLD-541 | chr16:50750151-50750178 | CYLD-1289 | chr16:50799952-50799979 |
| CYLD-542 | chr16:50750162-50750189 | CYLD-1290 | chr16:50799956-50799983 |
| CYLD-543 | chr16:50750174-50750201 | CYLD-1291 | chr16:50799971-50799998 |
| CYLD-544 | chr16:50750176-50750203 | CYLD-1292 | chr16:50799972-50799999 |
| CYLD-545 | chr16:50750217-50750244 | CYLD-1293 | chr16:50799977-50800004 |
| CYLD-546 | chr16:50750286-50750313 | CYLD-1294 | chr16:50799978-50800005 |
| CYLD-547 | chr16:50751510-50751537 | CYLD-1295 | chr16:50799985-50800012 |
| CYLD-548 | chr16:50751523-50751550 | CYLD-1296 | chr16:50799991-50800018 |
| CYLD-549 | chr16:50751530-50751557 | CYLD-1297 | chr16:50799992-50800019 |
| CYLD-550 | chr16:50751531-50751558 | CYLD-1298 | chr16:50799993-50800020 |
| CYLD-551 | chr16:50751532-50751559 | CYLD-1299 | chr16:50800002-50800029 |
| CYLD-552 | chr16:50751543-50751570 | CYLD-1300 | chr16:50800003-50800030 |
| CYLD-553 | chr16:50751544-50751571 | CYLD-1301 | chr16:50800006-50800033 |
| CYLD-554 | chr16:50751545-50751572 | CYLD-1302 | chr16:50800009-50800036 |
| CYLD-555 | chr16:50751551-50751578 | CYLD-1303 | chr16:50800031-50800058 |
| CYLD-556 | chr16:50751577-50751604 | CYLD-1304 | chr16:50800040-50800067 |
| CYLD-557 | chr16:50751583-50751610 | CYLD-1305 | chr16:50800041-50800068 |
| CYLD-558 | chr16:50751584-50751611 | CYLD-1306 | chr16:50800077-50800104 |
| CYLD-559 | chr16:50751590-50751617 | CYLD-1307 | chr16:50800089-50800116 |
| CYLD-560 | chr16:50751596-50751623 | CYLD-1308 | chr16:50800090-50800117 |
| CYLD-561 | chr16:50751608-50751635 | CYLD-1309 | chr16:50800133-50800160 |
| CYLD-562 | chr16:50751609-50751636 | CYLD-1310 | chr16:50800144-50800171 |
| CYLD-563 | chr16:50751610-50751637 | CYLD-1311 | chr16:50800145-50800172 |
| CYLD-564 | chr16:50751620-50751647 | CYLD-1312 | chr16:50800151-50800178 |
| CYLD-565 | chr16:50751621-50751648 | CYLD-1313 | chr16:50800154-50800181 |
| CYLD-566 | chr16:50751642-50751669 | CYLD-1314 | chr16:50800166-50800193 |
| CYLD-567 | chr16:50751653-50751680 | CYLD-1315 | chr16:50800167-50800194 |
| CYLD-568 | chr16:50751670-50751697 | CYLD-1316 | chr16:50800187-50800214 |
| CYLD-569 | chr16:50751709-50751736 | CYLD-1317 | chr16:50800188-50800215 |
| CYLD-570 | chr16:50751725-50751752 | CYLD-1318 | chr16:50800195-50800222 |
| CYLD-571 | chr16:50751731-50751758 | CYLD-1319 | chr16:50800210-50800237 |
| CYLD-572 | chr16:50751732-50751759 | CYLD-1320 | chr16:50800217-50800244 |
| CYLD-573 | chr16:50751733-50751760 | CYLD-1321 | chr16:50800223-50800250 |
| CYLD-574 | chr16:50751745-50751772 | CYLD-1322 | chr16:50800228-50800255 |
| CYLD-575 | chr16:50751754-50751781 | CYLD-1323 | chr16:50800229-50800256 |
| CYLD-576 | chr16:50751763-50751790 | CYLD-1324 | chr16:50800235-50800262 |
| CYLD-577 | chr16:50751781-50751808 | CYLD-1325 | chr16:50800238-50800265 |
| CYLD-578 | chr16:50751784-50751811 | CYLD-1326 | chr16:50800247-50800274 |
| CYLD-579 | chr16:50751790-50751817 | CYLD-1327 | chr16:50800248-50800275 |
| CYLD-580 | chr16:50751794-50751821 | CYLD-1328 | chr16:50800257-50800284 |
| CYLD-581 | chr16:50751800-50751827 | CYLD-1329 | chr16:50800258-50800285 |
| CYLD-582 | chr16:50751812-50751839 | CYLD-1330 | chr16:50800259-50800286 |
| CYLD-583 | chr16:50751842-50751869 | CYLD-1331 | chr16:50800260-50800287 |
| CYLD-584 | chr16:50751857-50751884 | CYLD-1332 | chr16:50800279-50800306 |
| CYLD-585 | chr16:50751865-50751892 | CYLD-1333 | chr16:50800280-50800307 |
| CYLD-586 | chr16:50751869-50751896 | CYLD-1334 | chr16:50800281-50800308 |
| CYLD-587 | chr16:50751874-50751901 | CYLD-1335 | chr16:50800298-50800325 |
| CYLD-588 | chr16:50751880-50751907 | CYLD-1336 | chr16:50800302-50800329 |
| CYLD-589 | chr16:50751894-50751921 | CYLD-1337 | chr16:50800337-50800364 |
| CYLD-590 | chr16:50754211-50754238 | CYLD-1338 | chr16:50800341-50800368 |
| CYLD-591 | chr16:50754214-50754241 | CYLD-1339 | chr16:50800374-50800401 |
| CYLD-592 | chr16:50754220-50754247 | CYLD-1340 | chr16:50800375-50800402 |
| CYLD-593 | chr16:50754225-50754252 | CYLD-1341 | chr16:50800382-50800409 |
| CYLD-594 | chr16:50754260-50754287 | CYLD-1342 | chr16:50800478-50800505 |
| CYLD-595 | chr16:50754263-50754290 | CYLD-1343 | chr16:50800484-50800511 |
| CYLD-596 | chr16:50754266-50754293 | CYLD-1344 | chr16:50800498-50800525 |
| CYLD-597 | chr16:50754292-50754319 | CYLD-1345 | chr16:50800515-50800542 |
| CYLD-598 | chr16:50754305-50754332 | CYLD-1346 | chr16:50800537-50800564 |
| CYLD-599 | chr16:50754312-50754339 | CYLD-1347 | chr16:50800560-50800587 |
| CYLD-600 | chr16:50754313-50754340 | CYLD-1348 | chr16:50800568-50800595 |
| CYLD-601 | chr16:50754317-50754344 | CYLD-1349 | chr16:50800575-50800602 |
| CYLD-602 | chr16:50754324-50754351 | CYLD-1350 | chr16:50800576-50800603 |
| CYLD-603 | chr16:50754325-50754352 | CYLD-1351 | chr16:50800577-50800604 |
| CYLD-604 | chr16:50754329-50754356 | CYLD-1352 | chr16:50800584-50800611 |
| CYLD-605 | chr16:50754398-50754425 | CYLD-1353 | chr16:50800614-50800641 |
| CYLD-606 | chr16:50754420-50754447 | CYLD-1354 | chr16:50800675-50800702 |
| CYLD-607 | chr16:50754421-50754448 | CYLD-1355 | chr16:50800687-50800714 |
| CYLD-608 | chr16:50754429-50754456 | CYLD-1356 | chr16:50800688-50800715 |
| CYLD-609 | chr16:50754477-50754504 | CYLD-1357 | chr16:50800703-50800730 |
| CYLD-610 | chr16:50754478-50754505 | CYLD-1358 | chr16:50800704-50800731 |
| CYLD-611 | chr16:50754479-50754506 | CYLD-1359 | chr16:50800733-50800760 |
| CYLD-612 | chr16:50754480-50754507 | CYLD-1360 | chr16:50800741-50800768 |
| CYLD-613 | chr16:50754486-50754513 | CYLD-1361 | chr16:50800748-50800775 |
| CYLD-614 | chr16:50754489-50754516 | CYLD-1362 | chr16:50800752-50800779 |
| CYLD-615 | chr16:50754496-50754523 | CYLD-1363 | chr16:50800760-50800787 |
| CYLD-616 | chr16:50754504-50754531 | CYLD-1364 | chr16:50800788-50800815 |
| CYLD-617 | chr16:50754520-50754547 | CYLD-1365 | chr16:50800793-50800820 |
| CYLD-618 | chr16:50775053-50775080 | CYLD-1366 | chr16:50800794-50800821 |
| CYLD-619 | chr16:50775078-50775105 | CYLD-1367 | chr16:50800799-50800826 |
| CYLD-620 | chr16:50775104-50775131 | CYLD-1368 | chr16:50800809-50800836 |
| CYLD-621 | chr16:50775112-50775139 | CYLD-1369 | chr16:50800836-50800863 |
| CYLD-622 | chr16:50775113-50775140 | CYLD-1370 | chr16:50800846-50800873 |
| CYLD-623 | chr16:50775123-50775150 | CYLD-1371 | chr16:50800851-50800878 |
| CYLD-624 | chr16:50775131-50775158 | CYLD-1372 | chr16:50800857-50800884 |
| CYLD-625 | chr16:50775148-50775175 | CYLD-1373 | chr16:50800858-50800885 |
| CYLD-626 | chr16:50775172-50775199 | CYLD-1374 | chr16:50800859-50800886 |
| CYLD-627 | chr16:50775181-50775208 | CYLD-1375 | chr16:50800862-50800889 |
| CYLD-628 | chr16:50775183-50775210 | CYLD-1376 | chr16:50800863-50800890 |
| CYLD-629 | chr16:50775203-50775230 | CYLD-1377 | chr16:50800864-50800891 |
| CYLD-630 | chr16:50775205-50775232 | CYLD-1378 | chr16:50800901-50800928 |
| CYLD-631 | chr16:50775250-50775277 | CYLD-1379 | chr16:50800904-50800931 |
| CYLD-632 | chr16:50775254-50775281 | CYLD-1380 | chr16:50800910-50800937 |
| CYLD-633 | chr16:50775263-50775290 | CYLD-1381 | chr16:50800917-50800944 |
| CYLD-634 | chr16:50775264-50775291 | CYLD-1382 | chr16:50800948-50800975 |
| CYLD-635 | chr16:50776059-50776086 | CYLD-1383 | chr16:50800949-50800976 |
| CYLD-636 | chr16:50776066-50776093 | CYLD-1384 | chr16:50800950-50800977 |
| CYLD-637 | chr16:50776067-50776094 | CYLD-1385 | chr16:50800953-50800980 |
| CYLD-638 | chr16:50776076-50776103 | CYLD-1386 | chr16:50800954-50800981 |
| CYLD-639 | chr16:50776119-50776146 | CYLD-1387 | chr16:50800955-50800982 |
| CYLD-640 | chr16:50776145-50776172 | CYLD-1388 | chr16:50800966-50800993 |
| CYLD-641 | chr16:50776166-50776193 | CYLD-1389 | chr16:50800975-50801002 |
| CYLD-642 | chr16:50776171-50776198 | CYLD-1390 | chr16:50800976-50801003 |
| CYLD-643 | chr16:50776174-50776201 | CYLD-1391 | chr16:50800978-50801005 |
| CYLD-644 | chr16:50776202-50776229 | CYLD-1392 | chr16:50800983-50801010 |
| CYLD-645 | chr16:50776203-50776230 | CYLD-1393 | chr16:50800984-50801011 |
| CYLD-646 | chr16:50776205-50776232 | CYLD-1394 | chr16:50800985-50801012 |
| CYLD-647 | chr16:50776206-50776233 | CYLD-1395 | chr16:50800989-50801016 |
| CYLD-648 | chr16:50776209-50776236 | CYLD-1396 | chr16:50801010-50801037 |
| CYLD-649 | chr16:50776210-50776237 | CYLD-1397 | chr16:50801011-50801038 |
| CYLD-650 | chr16:50776211-50776238 | CYLD-1398 | chr16:50801017-50801044 |
| CYLD-651 | chr16:50776215-50776242 | CYLD-1399 | chr16:50801018-50801045 |
| CYLD-652 | chr16:50776218-50776245 | CYLD-1400 | chr16:50801019-50801046 |
| CYLD-653 | chr16:50776244-50776271 | CYLD-1401 | chr16:50801024-50801051 |
| CYLD-654 | chr16:50776251-50776278 | CYLD-1402 | chr16:50801035-50801062 |
| CYLD-655 | chr16:50776256-50776283 | CYLD-1403 | chr16:50801036-50801063 |
| CYLD-656 | chr16:50776265-50776292 | CYLD-1404 | chr16:50801055-50801082 |
| CYLD-657 | chr16:50776299-50776326 | CYLD-1405 | chr16:50801075-50801102 |
| CYLD-658 | chr16:50776320-50776347 | CYLD-1406 | chr16:50801076-50801103 |
| CYLD-659 | chr16:50776329-50776356 | CYLD-1407 | chr16:50801078-50801105 |
| CYLD-660 | chr16:50777798-50777825 | CYLD-1408 | chr16:50801079-50801106 |
| CYLD-661 | chr16:50777816-50777843 | CYLD-1409 | chr16:50801080-50801107 |
| CYLD-662 | chr16:50777818-50777845 | CYLD-1410 | chr16:50801082-50801109 |
| CYLD-663 | chr16:50777831-50777858 | CYLD-1411 | chr16:50801087-50801114 |
| CYLD-664 | chr16:50777838-50777865 | CYLD-1412 | chr16:50801088-50801115 |
| CYLD-665 | chr16:50777839-50777866 | CYLD-1413 | chr16:50801089-50801116 |
| CYLD-666 | chr16:50777855-50777882 | CYLD-1414 | chr16:50801091-50801118 |
| CYLD-667 | chr16:50777856-50777883 | CYLD-1415 | chr16:50801092-50801119 |
| CYLD-668 | chr16:50777873-50777900 | CYLD-1416 | chr16:50801095-50801122 |
| CYLD-669 | chr16:50777901-50777928 | CYLD-1417 | chr16:50801097-50801124 |
| CYLD-670 | chr16:50777902-50777929 | CYLD-1418 | chr16:50801098-50801125 |
| CYLD-671 | chr16:50777909-50777936 | CYLD-1419 | chr16:50801099-50801126 |
| CYLD-672 | chr16:50777915-50777942 | CYLD-1420 | chr16:50801100-50801127 |
| CYLD-673 | chr16:50777975-50778002 | CYLD-1421 | chr16:50801132-50801159 |
| CYLD-674 | chr16:50777978-50778005 | CYLD-1422 | chr16:50801144-50801171 |
| CYLD-675 | chr16:50778007-50778034 | CYLD-1423 | chr16:50801155-50801182 |
| CYLD-676 | chr16:50779541-50779568 | CYLD-1424 | chr16:50801161-50801188 |
| CYLD-677 | chr16:50779544-50779571 | CYLD-1425 | chr16:50801170-50801197 |
| CYLD-678 | chr16:50779545-50779572 | CYLD-1426 | chr16:50801194-50801221 |
| CYLD-679 | chr16:50779552-50779579 | CYLD-1427 | chr16:50801200-50801227 |
| CYLD-680 | chr16:50779624-50779651 | CYLD-1428 | chr16:50801209-50801236 |
| CYLD-681 | chr16:50779627-50779654 | CYLD-1429 | chr16:50801215-50801242 |
| CYLD-682 | chr16:50779675-50779702 | CYLD-1430 | chr16:50801216-50801243 |
| CYLD-683 | chr16:50779676-50779703 | CYLD-1431 | chr16:50801221-50801248 |
| CYLD-684 | chr16:50779714-50779741 | CYLD-1432 | chr16:50801222-50801249 |
| CYLD-685 | chr16:50779724-50779751 | CYLD-1433 | chr16:50801225-50801252 |
| CYLD-686 | chr16:50779727-50779754 | CYLD-1434 | chr16:50801254-50801281 |
| CYLD-687 | chr16:50779732-50779759 | CYLD-1435 | chr16:50801257-50801284 |
| CYLD-688 | chr16:50779736-50779763 | CYLD-1436 | chr16:50801260-50801287 |
| CYLD-689 | chr16:50779739-50779766 | CYLD-1437 | chr16:50801263-50801290 |
| CYLD-690 | chr16:50779742-50779769 | CYLD-1438 | chr16:50801264-50801291 |
| CYLD-691 | chr16:50779758-50779785 | CYLD-1439 | chr16:50801271-50801298 |
| CYLD-692 | chr16:50779761-50779788 | CYLD-1440 | chr16:50801278-50801305 |
| CYLD-693 | chr16:50779774-50779801 | CYLD-1441 | chr16:50801294-50801321 |
| CYLD-694 | chr16:50779784-50779811 | CYLD-1442 | chr16:50801300-50801327 |
| CYLD-695 | chr16:50779791-50779818 | CYLD-1443 | chr16:50801310-50801337 |
| CYLD-696 | chr16:50779797-50779824 | CYLD-1444 | chr16:50801313-50801340 |
| CYLD-697 | chr16:50779800-50779827 | CYLD-1445 | chr16:50801322-50801349 |
| CYLD-698 | chr16:50779805-50779832 | CYLD-1446 | chr16:50801323-50801350 |
| CYLD-699 | chr16:50779806-50779833 | CYLD-1447 | chr16:50801328-50801355 |
| CYLD-700 | chr16:50779812-50779839 | CYLD-1448 | chr16:50801329-50801356 |
| CYLD-701 | chr16:50779828-50779855 | CYLD-1449 | chr16:50801335-50801362 |
| CYLD-702 | chr16:50779835-50779862 | CYLD-1450 | chr16:50801372-50801399 |
| CYLD-703 | chr16:50779838-50779865 | CYLD-1451 | chr16:50801373-50801400 |
| CYLD-704 | chr16:50779851-50779878 | CYLD-1452 | chr16:50801378-50801405 |
| CYLD-705 | chr16:50779852-50779879 | CYLD-1453 | chr16:50801379-50801406 |
| CYLD-706 | chr16:50779880-50779907 | CYLD-1454 | chr16:50801385-50801412 |
| CYLD-707 | chr16:50779883-50779910 | CYLD-1455 | chr16:50801386-50801413 |
| CYLD-708 | chr16:50779884-50779911 | CYLD-1456 | chr16:50801396-50801423 |
| CYLD-709 | chr16:50779888-50779915 | CYLD-1457 | chr16:50801399-50801426 |
| CYLD-710 | chr16:50779893-50779920 | CYLD-1458 | chr16:50801400-50801427 |
| CYLD-711 | chr16:50779894-50779921 | CYLD-1459 | chr16:50801414-50801441 |
| CYLD-712 | chr16:50779898-50779925 | CYLD-1460 | chr16:50801433-50801460 |
| CYLD-713 | chr16:50779901-50779928 | CYLD-1461 | chr16:50801444-50801471 |
| CYLD-714 | chr16:50779902-50779929 | CYLD-1462 | chr16:50801481-50801508 |
| CYLD-715 | chr16:50779905-50779932 | CYLD-1463 | chr16:50801482-50801509 |
| CYLD-716 | chr16:50779908-50779935 | CYLD-1464 | chr16:50801492-50801519 |
| CYLD-717 | chr16:50779913-50779940 | CYLD-1465 | chr16:50801510-50801537 |
| CYLD-718 | chr16:50779916-50779943 | CYLD-1466 | chr16:50801528-50801555 |
| CYLD-719 | chr16:50779917-50779944 | CYLD-1467 | chr16:50801531-50801558 |
| CYLD-720 | chr16:50779925-50779952 | CYLD-1468 | chr16:50801532-50801559 |
| CYLD-721 | chr16:50779937-50779964 | CYLD-1469 | chr16:50801541-50801568 |
| CYLD-722 | chr16:50779956-50779983 | CYLD-1470 | chr16:50801556-50801583 |
| CYLD-723 | chr16:50779957-50779984 | CYLD-1471 | chr16:50801621-50801648 |
| CYLD-724 | chr16:50779958-50779985 | CYLD-1472 | chr16:50801622-50801649 |
| CYLD-725 | chr16:50779969-50779996 | CYLD-1473 | chr16:50801623-50801650 |
| CYLD-726 | chr16:50779970-50779997 | CYLD-1474 | chr16:50801629-50801656 |
| CYLD-727 | chr16:50779973-50780000 | CYLD-1475 | chr16:50801630-50801657 |
| CYLD-728 | chr16:50779974-50780001 | CYLD-1476 | chr16:50801643-50801670 |
| CYLD-729 | chr16:50779986-50780013 | CYLD-1477 | chr16:50801644-50801671 |
| CYLD-730 | chr16:50779990-50780017 | CYLD-1478 | chr16:50801646-50801673 |
| CYLD-731 | chr16:50780006-50780033 | CYLD-1479 | chr16:50801654-50801681 |
| CYLD-732 | chr16:50780007-50780034 | CYLD-1480 | chr16:50801655-50801682 |
| CYLD-733 | chr16:50780009-50780036 | CYLD-1481 | chr16:50801656-50801683 |
| CYLD-734 | chr16:50780012-50780039 | CYLD-1482 | chr16:50801686-50801713 |
| CYLD-735 | chr16:50780018-50780045 | CYLD-1483 | chr16:50801703-50801730 |
| CYLD-736 | chr16:50780059-50780086 | CYLD-1484 | chr16:50801704-50801731 |
| CYLD-737 | chr16:50780060-50780087 | CYLD-1485 | chr16:50801760-50801787 |
| CYLD-738 | chr16:50780062-50780089 | CYLD-1486 | chr16:50801791-50801818 |
| CYLD-739 | chr16:50780106-50780133 | CYLD-1487 | chr16:50801794-50801821 |
| CYLD-740 | chr16:50780107-50780134 | CYLD-1488 | chr16:50801806-50801833 |
| CYLD-741 | chr16:50781126-50781153 | CYLD-1489 | chr16:50801807-50801834 |
| CYLD-742 | chr16:50781127-50781154 | CYLD-1490 | chr16:50801808-50801835 |
| CYLD-743 | chr16:50781142-50781169 | CYLD-1491 | chr16:50801812-50801839 |
| CYLD-744 | chr16:50781179-50781206 | CYLD-1492 | chr16:50801835-50801862 |
| CYLD-745 | chr16:50781182-50781209 | CYLD-1493 | chr16:50801854-50801881 |
| CYLD-746 | chr16:50781187-50781214 | CYLD-1494 | chr16:50801905-50801932 |
| CYLD-747 | chr16:50781219-50781246 | CYLD-1495 | chr16:50801935-50801962 |
| CYLD-748 | chr16:50781235-50781262 | | |

**Table 2J Genomic coordinates of the human CBLIF gene**

| Target ID | Target Coordinate Range | Target ID | Target Coordinate Range |
|---|---|---|---|
| CBLIF-1 | chr11:59829320-59829347 | CBLIF-276 | chr11:59843087-59843114 |
| CBLIF-2 | chr11:59829326-59829353 | CBLIF-277 | chr11:59843092-59843119 |
| CBLIF-3 | chr11:59829346-59829373 | CBLIF-278 | chr11:59843106-59843133 |
| CBLIF-4 | chr11:59829353-59829380 | CBLIF-279 | chr11:59843108-59843135 |
| CBLIF-5 | chr11:59829358-59829385 | CBLIF-280 | chr11:59843120-59843147 |
| CBLIF-6 | chr11:59829365-59829392 | CBLIF-281 | chr11:59843128-59843155 |
| CBLIF-7 | chr11:59829369-59829396 | CBLIF-282 | chr11:59843129-59843156 |
| CBLIF-8 | chr11:59829400-59829427 | CBLIF-283 | chr11:59843137-59843164 |
| CBLIF-9 | chr11:59829423-59829450 | CBLIF-284 | chr11:59843158-59843185 |
| CBLIF-10 | chr11:59829439-59829466 | CBLIF-285 | chr11:59843159-59843186 |
| CBLIF-11 | chr11:59829440-59829467 | CBLIF-286 | chr11:59843160-59843187 |
| CBLIF-12 | chr11:59829444-59829471 | CBLIF-287 | chr11:59843173-59843200 |
| CBLIF-13 | chr11:59829472-59829499 | CBLIF-288 | chr11:59843179-59843206 |
| CBLIF-14 | chr11:59829473-59829500 | CBLIF-289 | chr11:59843196-59843223 |
| CBLIF-15 | chr11:59829476-59829503 | CBLIF-290 | chr11:59843203-59843230 |
| CBLIF-16 | chr11:59829493-59829520 | CBLIF-291 | chr11:59843757-59843784 |
| CBLIF-17 | chr11:59829500-59829527 | CBLIF-292 | chr11:59843766-59843793 |
| CBLIF-18 | chr11:59829501-59829528 | CBLIF-293 | chr11:59843767-59843794 |
| CBLIF-19 | chr11:59829522-59829549 | CBLIF-294 | chr11:59843768-59843795 |
| CBLIF-20 | chr11:59829543-59829570 | CBLIF-295 | chr11:59843771-59843798 |
| CBLIF-21 | chr11:59829544-59829571 | CBLIF-296 | chr11:59843772-59843799 |
| CBLIF-22 | chr11:59829545-59829572 | CBLIF-297 | chr11:59843776-59843803 |
| CBLIF-23 | chr11:59829546-59829573 | CBLIF-298 | chr11:59843788-59843815 |
| CBLIF-24 | chr11:59829555-59829582 | CBLIF-299 | chr11:59843795-59843822 |
| CBLIF-25 | chr11:59829583-59829610 | CBLIF-300 | chr11:59843798-59843825 |
| CBLIF-26 | chr11:59829584-59829611 | CBLIF-301 | chr11:59843801-59843828 |
| CBLIF-27 | chr11:59829592-59829619 | CBLIF-302 | chr11:59843823-59843850 |
| CBLIF-28 | chr11:59829595-59829622 | CBLIF-303 | chr11:59843824-59843851 |
| CBLIF-29 | chr11:59829598-59829625 | CBLIF-304 | chr11:59843837-59843864 |
| CBLIF-30 | chr11:59829602-59829629 | CBLIF-305 | chr11:59843866-59843893 |
| CBLIF-31 | chr11:59829603-59829630 | CBLIF-306 | chr11:59843877-59843904 |
| CBLIF-32 | chr11:59829604-59829631 | CBLIF-307 | chr11:59843878-59843905 |
| CBLIF-33 | chr11:59829605-59829632 | CBLIF-308 | chr11:59843886-59843913 |
| CBLIF-34 | chr11:59829629-59829656 | CBLIF-309 | chr11:59843895-59843922 |
| CBLIF-35 | chr11:59829636-59829663 | CBLIF-310 | chr11:59843896-59843923 |
| CBLIF-36 | chr11:59829637-59829664 | CBLIF-311 | chr11:59843911-59843938 |
| CBLIF-37 | chr11:59831619-59831646 | CBLIF-312 | chr11:59843917-59843944 |
| CBLIF-38 | chr11:59831633-59831660 | CBLIF-313 | chr11:59843924-59843951 |
| CBLIF-39 | chr11:59831656-59831683 | CBLIF-314 | chr11:59843929-59843956 |
| CBLIF-40 | chr11:59831663-59831690 | CBLIF-315 | chr11:59843937-59843964 |
| CBLIF-41 | chr11:59831671-59831698 | CBLIF-316 | chr11:59843941-59843968 |
| CBLIF-42 | chr11:59831672-59831699 | CBLIF-317 | chr11:59843944-59843971 |
| CBLIF-43 | chr11:59831677-59831704 | CBLIF-318 | chr11:59843945-59843972 |
| CBLIF-44 | chr11:59831698-59831725 | CBLIF-319 | chr11:59843946-59843973 |
| CBLIF-45 | chr11:59831712-59831739 | CBLIF-320 | chr11:59843951-59843978 |
| CBLIF-46 | chr11:59831738-59831765 | CBLIF-321 | chr11:59843952-59843979 |
| CBLIF-47 | chr11:59831742-59831769 | CBLIF-322 | chr11:59843956-59843983 |
| CBLIF-48 | chr11:59831761-59831788 | CBLIF-323 | chr11:59843996-59844023 |
| CBLIF-49 | chr11:59831770-59831797 | CBLIF-324 | chr11:59844005-59844032 |
| CBLIF-50 | chr11:59831771-59831798 | CBLIF-325 | chr11:59844007-59844034 |
| CBLIF-51 | chr11:59831772-59831799 | CBLIF-326 | chr11:59844008-59844035 |
| CBLIF-52 | chr11:59831809-59831836 | CBLIF-327 | chr11:59844022-59844049 |
| CBLIF-53 | chr11:59831814-59831841 | CBLIF-328 | chr11:59844023-59844050 |
| CBLIF-54 | chr11:59831815-59831842 | CBLIF-329 | chr11:59844028-59844055 |
| CBLIF-55 | chr11:59831824-59831851 | CBLIF-330 | chr11:59844029-59844056 |
| CBLIF-56 | chr11:59831835-59831862 | CBLIF-331 | chr11:59844038-59844065 |
| CBLIF-57 | chr11:59831836-59831863 | CBLIF-332 | chr11:59844039-59844066 |
| CBLIF-58 | chr11:59831886-59831913 | CBLIF-333 | chr11:59844071-59844098 |
| CBLIF-59 | chr11:59835694-59835721 | CBLIF-334 | chr11:59844082-59844109 |
| CBLIF-60 | chr11:59835706-59835733 | CBLIF-335 | chr11:59844090-59844117 |
| CBLIF-61 | chr11:59835714-59835741 | CBLIF-336 | chr11:59844091-59844118 |
| CBLIF-62 | chr11:59835718-59835745 | CBLIF-337 | chr11:59844098-59844125 |
| CBLIF-63 | chr11:59835729-59835756 | CBLIF-338 | chr11:59844116-59844143 |
| CBLIF-64 | chr11:59835730-59835757 | CBLIF-339 | chr11:59844117-59844144 |
| CBLIF-65 | chr11:59835743-59835770 | CBLIF-340 | chr11:59844118-59844145 |
| CBLIF-66 | chr11:59835771-59835798 | CBLIF-341 | chr11:59845260-59845287 |
| CBLIF-67 | chr11:59835791-59835818 | CBLIF-342 | chr11:59845261-59845288 |
| CBLIF-68 | chr11:59835816-59835843 | CBLIF-343 | chr11:59845274-59845301 |
| CBLIF-69 | chr11:59835836-59835863 | CBLIF-344 | chr11:59845286-59845313 |
| CBLIF-70 | chr11:59835859-59835886 | CBLIF-345 | chr11:59845297-59845324 |
| CBLIF-71 | chr11:59835861-59835888 | CBLIF-346 | chr11:59845305-59845332 |
| CBLIF-72 | chr11:59835862-59835889 | CBLIF-347 | chr11:59845306-59845333 |
| CBLIF-73 | chr11:59835894-59835921 | CBLIF-348 | chr11:59845320-59845347 |
| CBLIF-74 | chr11:59835904-59835931 | CBLIF-349 | chr11:59845321-59845348 |
| CBLIF-75 | chr11:59835908-59835935 | CBLIF-350 | chr11:59845322-59845349 |
| CBLIF-76 | chr11:59835909-59835936 | CBLIF-351 | chr11:59845326-59845353 |
| CBLIF-77 | chr11:59835910-59835937 | CBLIF-352 | chr11:59845327-59845354 |
| CBLIF-78 | chr11:59835911-59835938 | CBLIF-353 | chr11:59845328-59845355 |
| CBLIF-79 | chr11:59835912-59835939 | CBLIF-354 | chr11:59845330-59845357 |
| CBLIF-80 | chr11:59835934-59835961 | CBLIF-355 | chr11:59845343-59845370 |
| CBLIF-81 | chr11:59835937-59835964 | CBLIF-356 | chr11:59845344-59845371 |
| CBLIF-82 | chr11:59835938-59835965 | CBLIF-357 | chr11:59845349-59845376 |
| CBLIF-83 | chr11:59835939-59835966 | CBLIF-358 | chr11:59845352-59845379 |
| CBLIF-84 | chr11:59835944-59835971 | CBLIF-359 | chr11:59845361-59845388 |
| CBLIF-85 | chr11:59835945-59835972 | CBLIF-360 | chr11:59845362-59845389 |
| CBLIF-86 | chr11:59835950-59835977 | CBLIF-361 | chr11:59845367-59845394 |
| CBLIF-87 | chr11:59835951-59835978 | CBLIF-362 | chr11:59845368-59845395 |
| CBLIF-88 | chr11:59835958-59835985 | CBLIF-363 | chr11:59845397-59845424 |
| CBLIF-89 | chr11:59835959-59835986 | CBLIF-364 | chr11:59845403-59845430 |
| CBLIF-90 | chr11:59835967-59835994 | CBLIF-365 | chr11:59845404-59845431 |
| CBLIF-91 | chr11:59835968-59835995 | CBLIF-366 | chr11:59845406-59845433 |
| CBLIF-92 | chr11:59835973-59836000 | CBLIF-367 | chr11:59845412-59845439 |
| CBLIF-93 | chr11:59835991-59836018 | CBLIF-368 | chr11:59845413-59845440 |
| CBLIF-94 | chr11:59835992-59836019 | CBLIF-369 | chr11:59845414-59845441 |
| CBLIF-95 | chr11:59836000-59836027 | CBLIF-370 | chr11:59845415-59845442 |
| CBLIF-96 | chr11:59836001-59836028 | CBLIF-371 | chr11:59845422-59845449 |
| CBLIF-97 | chr11:59836022-59836049 | CBLIF-372 | chr11:59845445-59845472 |
| CBLIF-98 | chr11:59836023-59836050 | CBLIF-373 | chr11:59845450-59845477 |
| CBLIF-99 | chr11:59836024-59836051 | CBLIF-374 | chr11:59845459-59845486 |
| CBLIF-100 | chr11:59836028-59836055 | CBLIF-375 | chr11:59845478-59845505 |
| CBLIF-101 | chr11:59836037-59836064 | CBLIF-376 | chr11:59845480-59845507 |
| CBLIF-102 | chr11:59836057-59836084 | CBLIF-377 | chr11:59845489-59845516 |
| CBLIF-103 | chr11:59836092-59836119 | CBLIF-378 | chr11:59845518-59845545 |
| CBLIF-104 | chr11:59836093-59836120 | CBLIF-379 | chr11:59845537-59845564 |
| CBLIF-105 | chr11:59836094-59836121 | CBLIF-380 | chr11:59845538-59845565 |
| CBLIF-106 | chr11:59837052-59837079 | CBLIF-381 | chr11:59845557-59845584 |
| CBLIF-107 | chr11:59837062-59837089 | CBLIF-382 | chr11:59845558-59845585 |
| CBLIF-108 | chr11:59837063-59837090 | CBLIF-383 | chr11:59845559-59845586 |
| CBLIF-109 | chr11:59837090-59837117 | CBLIF-384 | chr11:59845563-59845590 |
| CBLIF-110 | chr11:59837098-59837125 | CBLIF-385 | chr11:59845564-59845591 |
| CBLIF-111 | chr11:59837102-59837129 | CBLIF-386 | chr11:59845568-59845595 |
| CBLIF-112 | chr11:59837113-59837140 | CBLIF-387 | chr11:59845573-59845600 |
| CBLIF-113 | chr11:59837129-59837156 | CBLIF-388 | chr11:59845581-59845608 |
| CBLIF-114 | chr11:59837134-59837161 | CBLIF-389 | chr11:59845582-59845609 |
| CBLIF-115 | chr11:59837137-59837164 | CBLIF-390 | chr11:59845583-59845610 |
| CBLIF-116 | chr11:59837150-59837177 | CBLIF-391 | chr11:59845593-59845620 |
| CBLIF-117 | chr11:59837166-59837193 | CBLIF-392 | chr11:59845598-59845625 |
| CBLIF-118 | chr11:59837167-59837194 | CBLIF-393 | chr11:59845603-59845630 |
| CBLIF-119 | chr11:59837168-59837195 | CBLIF-394 | chr11:59845639-59845666 |
| CBLIF-120 | chr11:59837173-59837200 | CBLIF-395 | chr11:59845646-59845673 |
| CBLIF-121 | chr11:59837178-59837205 | CBLIF-396 | chr11:59845649-59845676 |
| CBLIF-122 | chr11:59837189-59837216 | CBLIF-397 | chr11:59845657-59845684 |
| CBLIF-123 | chr11:59837196-59837223 | CBLIF-398 | chr11:59845658-59845685 |
| CBLIF-124 | chr11:59837197-59837224 | CBLIF-399 | chr11:59845660-59845687 |
| CBLIF-125 | chr11:59837201-59837228 | CBLIF-400 | chr11:59845661-59845688 |
| CBLIF-126 | chr11:59837202-59837229 | CBLIF-401 | chr11:59845685-59845712 |
| CBLIF-127 | chr11:59837205-59837232 | CBLIF-402 | chr11:59845686-59845713 |
| CBLIF-128 | chr11:59837215-59837242 | CBLIF-403 | chr11:59845687-59845714 |
| CBLIF-129 | chr11:59837218-59837245 | CBLIF-404 | chr11:59845691-59845718 |
| CBLIF-130 | chr11:59837224-59837251 | CBLIF-405 | chr11:59845692-59845719 |
| CBLIF-131 | chr11:59837225-59837252 | CBLIF-406 | chr11:59845705-59845732 |
| CBLIF-132 | chr11:59837226-59837253 | CBLIF-407 | chr11:59845708-59845735 |
| CBLIF-133 | chr11:59837232-59837259 | CBLIF-408 | chr11:59845735-59845762 |
| CBLIF-134 | chr11:59837265-59837292 | CBLIF-409 | chr11:59845740-59845767 |
| CBLIF-135 | chr11:59837266-59837293 | CBLIF-410 | chr11:59845741-59845768 |
| CBLIF-136 | chr11:59837267-59837294 | CBLIF-411 | chr11:59845750-59845777 |
| CBLIF-137 | chr11:59837294-59837321 | CBLIF-412 | chr11:59845753-59845780 |
| CBLIF-138 | chr11:59837303-59837330 | CBLIF-413 | chr11:59845756-59845783 |
| CBLIF-139 | chr11:59837309-59837336 | CBLIF-414 | chr11:59845761-59845788 |
| CBLIF-140 | chr11:59837313-59837340 | CBLIF-415 | chr11:59845763-59845790 |
| CBLIF-141 | chr11:59837318-59837345 | CBLIF-416 | chr11:59845812-59845839 |
| CBLIF-142 | chr11:59837328-59837355 | CBLIF-417 | chr11:59845822-59845849 |
| CBLIF-143 | chr11:59837329-59837356 | CBLIF-418 | chr11:59845823-59845850 |
| CBLIF-144 | chr11:59837333-59837360 | CBLIF-419 | chr11:59845834-59845861 |
| CBLIF-145 | chr11:59837351-59837378 | CBLIF-420 | chr11:59845845-59845872 |
| CBLIF-146 | chr11:59837364-59837391 | CBLIF-421 | chr11:59845848-59845875 |
| CBLIF-147 | chr11:59837380-59837407 | CBLIF-422 | chr11:59845849-59845876 |
| CBLIF-148 | chr11:59837422-59837449 | CBLIF-423 | chr11:59845856-59845883 |
| CBLIF-149 | chr11:59841019-59841046 | CBLIF-424 | chr11:59845867-59845894 |
| CBLIF-150 | chr11:59841049-59841076 | CBLIF-425 | chr11:59845884-59845911 |
| CBLIF-151 | chr11:59841071-59841098 | CBLIF-426 | chr11:59845914-59845941 |
| CBLIF-152 | chr11:59841072-59841099 | CBLIF-427 | chr11:59845920-59845947 |
| CBLIF-153 | chr11:59841077-59841104 | CBLIF-428 | chr11:59845922-59845949 |
| CBLIF-154 | chr11:59841079-59841106 | CBLIF-429 | chr11:59845928-59845955 |
| CBLIF-155 | chr11:59841083-59841110 | CBLIF-430 | chr11:59845932-59845959 |
| CBLIF-156 | chr11:59841094-59841121 | CBLIF-431 | chr11:59845933-59845960 |
| CBLIF-157 | chr11:59841113-59841140 | CBLIF-432 | chr11:59845939-59845966 |
| CBLIF-158 | chr11:59841114-59841141 | CBLIF-433 | chr11:59845943-59845970 |
| CBLIF-159 | chr11:59841118-59841145 | CBLIF-434 | chr11:59845953-59845980 |
| CBLIF-160 | chr11:59841123-59841150 | CBLIF-435 | chr11:59845966-59845993 |
| CBLIF-161 | chr11:59841128-59841155 | CBLIF-436 | chr11:59845981-59846008 |
| CBLIF-162 | chr11:59841142-59841169 | CBLIF-437 | chr11:59845987-59846014 |
| CBLIF-163 | chr11:59841156-59841183 | CBLIF-438 | chr11:59846004-59846031 |
| CBLIF-164 | chr11:59841174-59841201 | CBLIF-439 | chr11:59846010-59846037 |
| CBLIF-165 | chr11:59841183-59841210 | CBLIF-440 | chr11:59846022-59846049 |
| CBLIF-166 | chr11:59841214-59841241 | CBLIF-441 | chr11:59846025-59846052 |
| CBLIF-167 | chr11:59841218-59841245 | CBLIF-442 | chr11:59846035-59846062 |
| CBLIF-168 | chr11:59841219-59841246 | CBLIF-443 | chr11:59846042-59846069 |
| CBLIF-169 | chr11:59841223-59841250 | CBLIF-444 | chr11:59846056-59846083 |
| CBLIF-170 | chr11:59841232-59841259 | CBLIF-445 | chr11:59846064-59846091 |
| CBLIF-171 | chr11:59841237-59841264 | CBLIF-446 | chr11:59846073-59846100 |
| CBLIF-172 | chr11:59841247-59841274 | CBLIF-447 | chr11:59846074-59846101 |
| CBLIF-173 | chr11:59841248-59841275 | CBLIF-448 | chr11:59846085-59846112 |
| CBLIF-174 | chr11:59841255-59841282 | CBLIF-449 | chr11:59846103-59846130 |
| CBLIF-175 | chr11:59841259-59841286 | CBLIF-450 | chr11:59846122-59846149 |
| CBLIF-176 | chr11:59841267-59841294 | CBLIF-451 | chr11:59846134-59846161 |
| CBLIF-177 | chr11:59841279-59841306 | CBLIF-452 | chr11:59846135-59846162 |
| CBLIF-178 | chr11:59841301-59841328 | CBLIF-453 | chr11:59846187-59846214 |
| CBLIF-179 | chr11:59841310-59841337 | CBLIF-454 | chr11:59846224-59846251 |
| CBLIF-180 | chr11:59841313-59841340 | CBLIF-455 | chr11:59846225-59846252 |
| CBLIF-181 | chr11:59841314-59841341 | CBLIF-456 | chr11:59846239-59846266 |
| CBLIF-182 | chr11:59841318-59841345 | CBLIF-457 | chr11:59846262-59846289 |
| CBLIF-183 | chr11:59841352-59841379 | CBLIF-458 | chr11:59846308-59846335 |
| CBLIF-184 | chr11:59841361-59841388 | CBLIF-459 | chr11:59846309-59846336 |
| CBLIF-185 | chr11:59841386-59841413 | CBLIF-460 | chr11:59846310-59846337 |
| CBLIF-186 | chr11:59841416-59841443 | CBLIF-461 | chr11:59846324-59846351 |
| CBLIF-187 | chr11:59842323-59842350 | CBLIF-462 | chr11:59846345-59846372 |
| CBLIF-188 | chr11:59842324-59842351 | CBLIF-463 | chr11:59846354-59846381 |
| CBLIF-189 | chr11:59842331-59842358 | CBLIF-464 | chr11:59846355-59846382 |
| CBLIF-190 | chr11:59842339-59842366 | CBLIF-465 | chr11:59846378-59846405 |
| CBLIF-191 | chr11:59842342-59842369 | CBLIF-466 | chr11:59846379-59846406 |
| CBLIF-192 | chr11:59842345-59842372 | CBLIF-467 | chr11:59846386-59846413 |
| CBLIF-193 | chr11:59842359-59842386 | CBLIF-468 | chr11:59846390-59846417 |
| CBLIF-194 | chr11:59842371-59842398 | CBLIF-469 | chr11:59846393-59846420 |
| CBLIF-195 | chr11:59842372-59842399 | CBLIF-470 | chr11:59846399-59846426 |
| CBLIF-196 | chr11:59842376-59842403 | CBLIF-471 | chr11:59846416-59846443 |
| CBLIF-197 | chr11:59842382-59842409 | CBLIF-472 | chr11:59846418-59846445 |
| CBLIF-198 | chr11:59842385-59842412 | CBLIF-473 | chr11:59846424-59846451 |
| CBLIF-199 | chr11:59842395-59842422 | CBLIF-474 | chr11:59846433-59846460 |
| CBLIF-200 | chr11:59842396-59842423 | CBLIF-475 | chr11:59846441-59846468 |
| CBLIF-201 | chr11:59842397-59842424 | CBLIF-476 | chr11:59846452-59846479 |
| CBLIF-202 | chr11:59842402-59842429 | CBLIF-477 | chr11:59846453-59846480 |
| CBLIF-203 | chr11:59842403-59842430 | CBLIF-478 | chr11:59846454-59846481 |
| CBLIF-204 | chr11:59842413-59842440 | CBLIF-479 | chr11:59846466-59846493 |
| CBLIF-205 | chr11:59842414-59842441 | CBLIF-480 | chr11:59846471-59846498 |
| CBLIF-206 | chr11:59842427-59842454 | CBLIF-481 | chr11:59846472-59846499 |
| CBLIF-207 | chr11:59842428-59842455 | CBLIF-482 | chr11:59846494-59846521 |
| CBLIF-208 | chr11:59842433-59842460 | CBLIF-483 | chr11:59846495-59846522 |
| CBLIF-209 | chr11:59842434-59842461 | CBLIF-484 | chr11:59846496-59846523 |
| CBLIF-210 | chr11:59842439-59842466 | CBLIF-485 | chr11:59846514-59846541 |
| CBLIF-211 | chr11:59842442-59842469 | CBLIF-486 | chr11:59846517-59846544 |
| CBLIF-212 | chr11:59842447-59842474 | CBLIF-487 | chr11:59846530-59846557 |
| CBLIF-213 | chr11:59842448-59842475 | CBLIF-488 | chr11:59846531-59846558 |
| CBLIF-214 | chr11:59842454-59842481 | CBLIF-489 | chr11:59846532-59846559 |
| CBLIF-215 | chr11:59842462-59842489 | CBLIF-490 | chr11:59846533-59846560 |
| CBLIF-216 | chr11:59842466-59842493 | CBLIF-491 | chr11:59846539-59846566 |
| CBLIF-217 | chr11:59842467-59842494 | CBLIF-492 | chr11:59846540-59846567 |
| CBLIF-218 | chr11:59842473-59842500 | CBLIF-493 | chr11:59846544-59846571 |
| CBLIF-219 | chr11:59842478-59842505 | CBLIF-494 | chr11:59846546-59846573 |
| CBLIF-220 | chr11:59842498-59842525 | CBLIF-495 | chr11:59846547-59846574 |
| CBLIF-221 | chr11:59842509-59842536 | CBLIF-496 | chr11:59846564-59846591 |
| CBLIF-222 | chr11:59842523-59842550 | CBLIF-497 | chr11:59846578-59846605 |
| CBLIF-223 | chr11:59842524-59842551 | CBLIF-498 | chr11:59846580-59846607 |
| CBLIF-224 | chr11:59842533-59842560 | CBLIF-499 | chr11:59846586-59846613 |
| CBLIF-225 | chr11:59842535-59842562 | CBLIF-500 | chr11:59846606-59846633 |
| CBLIF-226 | chr11:59842552-59842579 | CBLIF-501 | chr11:59846624-59846651 |
| CBLIF-227 | chr11:59842553-59842580 | CBLIF-502 | chr11:59846628-59846655 |
| CBLIF-228 | chr11:59842554-59842581 | CBLIF-503 | chr11:59846629-59846656 |
| CBLIF-229 | chr11:59842555-59842582 | CBLIF-504 | chr11:59846634-59846661 |
| CBLIF-230 | chr11:59842565-59842592 | CBLIF-505 | chr11:59846637-59846664 |
| CBLIF-231 | chr11:59842566-59842593 | CBLIF-506 | chr11:59846638-59846665 |
| CBLIF-232 | chr11:59842567-59842594 | CBLIF-507 | chr11:59846650-59846677 |
| CBLIF-233 | chr11:59842583-59842610 | CBLIF-508 | chr11:59846657-59846684 |
| CBLIF-234 | chr11:59842586-59842613 | CBLIF-509 | chr11:59846662-59846689 |
| CBLIF-235 | chr11:59842603-59842630 | CBLIF-510 | chr11:59846665-59846692 |
| CBLIF-236 | chr11:59842607-59842634 | CBLIF-511 | chr11:59846674-59846701 |
| CBLIF-237 | chr11:59842623-59842650 | CBLIF-512 | chr11:59846675-59846702 |
| CBLIF-238 | chr11:59842624-59842651 | CBLIF-513 | chr11:59846676-59846703 |
| CBLIF-239 | chr11:59842645-59842672 | CBLIF-514 | chr11:59846683-59846710 |
| CBLIF-240 | chr11:59842656-59842683 | CBLIF-515 | chr11:59846684-59846711 |
| CBLIF-241 | chr11:59842661-59842688 | CBLIF-516 | chr11:59846685-59846712 |
| CBLIF-242 | chr11:59842922-59842949 | CBLIF-517 | chr11:59846690-59846717 |
| CBLIF-243 | chr11:59842923-59842950 | CBLIF-518 | chr11:59846703-59846730 |
| CBLIF-244 | chr11:59842932-59842959 | CBLIF-519 | chr11:59846707-59846734 |
| CBLIF-245 | chr11:59842938-59842965 | CBLIF-520 | chr11:59846708-59846735 |
| CBLIF-246 | chr11:59842939-59842966 | CBLIF-521 | chr11:59846751-59846778 |
| CBLIF-247 | chr11:59842941-59842968 | CBLIF-522 | chr11:59846752-59846779 |
| CBLIF-248 | chr11:59842944-59842971 | CBLIF-523 | chr11:59846753-59846780 |
| CBLIF-249 | chr11:59842945-59842972 | CBLIF-524 | chr11:59846755-59846782 |
| CBLIF-250 | chr11:59842946-59842973 | CBLIF-525 | chr11:59846756-59846783 |
| CBLIF-251 | chr11:59842947-59842974 | CBLIF-526 | chr11:59846757-59846784 |
| CBLIF-252 | chr11:59842950-59842977 | CBLIF-527 | chr11:59846771-59846798 |
| CBLIF-253 | chr11:59842951-59842978 | CBLIF-528 | chr11:59846812-59846839 |
| CBLIF-254 | chr11:59842954-59842981 | CBLIF-529 | chr11:59846818-59846845 |
| CBLIF-255 | chr11:59842957-59842984 | CBLIF-530 | chr11:59846819-59846846 |
| CBLIF-256 | chr11:59842987-59843014 | CBLIF-531 | chr11:59846832-59846859 |
| CBLIF-257 | chr11:59842994-59843021 | CBLIF-532 | chr11:59846870-59846897 |
| CBLIF-258 | chr11:59843003-59843030 | CBLIF-533 | chr11:59846871-59846898 |
| CBLIF-259 | chr11:59843007-59843034 | CBLIF-534 | chr11:59846896-59846923 |
| CBLIF-260 | chr11:59843008-59843035 | CBLIF-535 | chr11:59846897-59846924 |
| CBLIF-261 | chr11:59843009-59843036 | CBLIF-536 | chr11:59846905-59846932 |
| CBLIF-262 | chr11:59843013-59843040 | CBLIF-537 | chr11:59846906-59846933 |
| CBLIF-263 | chr11:59843037-59843064 | CBLIF-538 | chr11:59846915-59846942 |
| CBLIF-264 | chr11:59843038-59843065 | CBLIF-539 | chr11:59846916-59846943 |
| CBLIF-265 | chr11:59843039-59843066 | CBLIF-540 | chr11:59846917-59846944 |
| CBLIF-266 | chr11:59843046-59843073 | CBLIF-541 | chr11:59846918-59846945 |
| CBLIF-267 | chr11:59843055-59843082 | CBLIF-542 | chr11:59846932-59846959 |
| CBLIF-268 | chr11:59843056-59843083 | CBLIF-543 | chr11:59846933-59846960 |
| CBLIF-269 | chr11:59843062-59843089 | CBLIF-544 | chr11:59846943-59846970 |
| CBLIF-270 | chr11:59843066-59843093 | CBLIF-545 | chr11:59846944-59846971 |
| CBLIF-271 | chr11:59843069-59843096 | CBLIF-546 | chr11:59846945-59846972 |
| CBLIF-272 | chr11:59843072-59843099 | CBLIF-547 | chr11:59846953-59846980 |
| CBLIF-273 | chr11:59843076-59843103 | CBLIF-548 | chr11:59846969-59846996 |
| CBLIF-274 | chr11:59843077-59843104 | CBLIF-549 | chr11:59846997-59847024 |
| CBLIF-275 | chr11:59843078-59843105 | CBLIF-550 | chr11:59846998-59847025 |

**Table 2K Genomic coordinates of the human KLF4 gene**

| Target ID | Target Coordinate Range | Target ID | Target Coordinate Range |
|---|---|---|---|
| KLF4-1 | chr9:107484844-107484871 | KLF4-462 | chr9:107488872-107488899 |
| KLF4-2 | chr9:107484894-107484921 | KLF4-463 | chr9:107488873-107488900 |
| KLF4-3 | chr9:107484918-107484945 | KLF4-464 | chr9:107488874-107488901 |
| KLF4-4 | chr9:107484973-107485000 | KLF4-465 | chr9:107488877-107488904 |
| KLF4-5 | chr9:107484978-107485005 | KLF4-466 | chr9:107488882-107488909 |
| KLF4-6 | chr9:107485013-107485040 | KLF4-467 | chr9:107488883-107488910 |
| KLF4-7 | chr9:107485022-107485049 | KLF4-468 | chr9:107488885-107488912 |
| KLF4-8 | chr9:107485023-107485050 | KLF4-469 | chr9:107488890-107488917 |
| KLF4-9 | chr9:107485057-107485084 | KLF4-470 | chr9:107488892-107488919 |
| KLF4-10 | chr9:107485086-107485113 | KLF4-471 | chr9:107488893-107488920 |
| KLF4-11 | chr9:107485110-107485137 | KLF4-472 | chr9:107488896-107488923 |
| KLF4-12 | chr9:107485128-107485155 | KLF4-473 | chr9:107488905-107488932 |
| KLF4-13 | chr9:107485133-107485160 | KLF4-474 | chr9:107488906-107488933 |
| KLF4-14 | chr9:107485145-107485172 | KLF4-475 | chr9:107488911-107488938 |
| KLF4-15 | chr9:107485191-107485218 | KLF4-476 | chr9:107488912-107488939 |
| KLF4-16 | chr9:107485198-107485225 | KLF4-477 | chr9:107488913-107488940 |
| KLF4-17 | chr9:107485211-107485238 | KLF4-478 | chr9:107488914-107488941 |
| KLF4-18 | chr9:107485229-107485256 | KLF4-479 | chr9:107488943-107488970 |
| KLF4-19 | chr9:107485241-107485268 | KLF4-480 | chr9:107488950-107488977 |
| KLF4-20 | chr9:107485250-107485277 | KLF4-481 | chr9:107488951-107488978 |
| KLF4-21 | chr9:107485251-107485278 | KLF4-482 | chr9:107488965-107488992 |
| KLF4-22 | chr9:107485260-107485287 | KLF4-483 | chr9:107488966-107488993 |
| KLF4-23 | chr9:107485267-107485294 | KLF4-484 | chr9:107488967-107488994 |
| KLF4-24 | chr9:107485301-107485328 | KLF4-485 | chr9:107488970-107488997 |
| KLF4-25 | chr9:107485312-107485339 | KLF4-486 | chr9:107488971-107488998 |
| KLF4-26 | chr9:107485313-107485340 | KLF4-487 | chr9:107488974-107489001 |
| KLF4-27 | chr9:107485323-107485350 | KLF4-488 | chr9:107488977-107489004 |
| KLF4-28 | chr9:107485339-107485366 | KLF4-489 | chr9:107488978-107489005 |
| KLF4-29 | chr9:107485380-107485407 | KLF4-490 | chr9:107488979-107489006 |
| KLF4-30 | chr9:107485381-107485408 | KLF4-491 | chr9:107489016-107489043 |
| KLF4-31 | chr9:107485389-107485416 | KLF4-492 | chr9:107489019-107489046 |
| KLF4-32 | chr9:107485411-107485438 | KLF4-493 | chr9:107489025-107489052 |
| KLF4-33 | chr9:107485421-107485448 | KLF4-494 | chr9:107489035-107489062 |
| KLF4-34 | chr9:107485422-107485449 | KLF4-495 | chr9:107489039-107489066 |
| KLF4-35 | chr9:107485425-107485452 | KLF4-496 | chr9:107489040-107489067 |
| KLF4-36 | chr9:107485426-107485453 | KLF4-497 | chr9:107489045-107489072 |
| KLF4-37 | chr9:107485427-107485454 | KLF4-498 | chr9:107489050-107489077 |
| KLF4-38 | chr9:107485428-107485455 | KLF4-499 | chr9:107489052-107489079 |
| KLF4-39 | chr9:107485451-107485478 | KLF4-500 | chr9:107489061-107489088 |
| KLF4-40 | chr9:107485452-107485479 | KLF4-501 | chr9:107489062-107489089 |
| KLF4-41 | chr9:107485457-107485484 | KLF4-502 | chr9:107489063-107489090 |
| KLF4-42 | chr9:107485460-107485487 | KLF4-503 | chr9:107489066-107489093 |
| KLF4-43 | chr9:107485469-107485496 | KLF4-504 | chr9:107489073-107489100 |
| KLF4-44 | chr9:107485474-107485501 | KLF4-505 | chr9:107489085-107489112 |
| KLF4-45 | chr9:107485476-107485503 | KLF4-506 | chr9:107489098-107489125 |
| KLF4-46 | chr9:107485477-107485504 | KLF4-507 | chr9:107489101-107489128 |
| KLF4-47 | chr9:107485478-107485505 | KLF4-508 | chr9:107489109-107489136 |
| KLF4-48 | chr9:107485479-107485506 | KLF4-509 | chr9:107489118-107489145 |
| KLF4-49 | chr9:107485488-107485515 | KLF4-510 | chr9:107489119-107489146 |
| KLF4-50 | chr9:107485497-107485524 | KLF4-511 | chr9:107489122-107489149 |
| KLF4-51 | chr9:107485500-107485527 | KLF4-512 | chr9:107489127-107489154 |
| KLF4-52 | chr9:107485541-107485568 | KLF4-513 | chr9:107489132-107489159 |
| KLF4-53 | chr9:107485552-107485579 | KLF4-514 | chr9:107489133-107489160 |
| KLF4-54 | chr9:107485553-107485580 | KLF4-515 | chr9:107489134-107489161 |
| KLF4-55 | chr9:107485554-107485581 | KLF4-516 | chr9:107489137-107489164 |
| KLF4-56 | chr9:107485555-107485582 | KLF4-517 | chr9:107489138-107489165 |
| KLF4-57 | chr9:107485586-107485613 | KLF4-518 | chr9:107489144-107489171 |
| KLF4-58 | chr9:107485596-107485623 | KLF4-519 | chr9:107489145-107489172 |
| KLF4-59 | chr9:107485601-107485628 | KLF4-520 | chr9:107489151-107489178 |
| KLF4-60 | chr9:107485602-107485629 | KLF4-521 | chr9:107489153-107489180 |
| KLF4-61 | chr9:107485605-107485632 | KLF4-522 | chr9:107489154-107489181 |
| KLF4-62 | chr9:107485631-107485658 | KLF4-523 | chr9:107489155-107489182 |
| KLF4-63 | chr9:107485635-107485662 | KLF4-524 | chr9:107489156-107489183 |
| KLF4-64 | chr9:107485636-107485663 | KLF4-525 | chr9:107489158-107489185 |
| KLF4-65 | chr9:107485637-107485664 | KLF4-526 | chr9:107489163-107489190 |
| KLF4-66 | chr9:107485652-107485679 | KLF4-527 | chr9:107489165-107489192 |
| KLF4-67 | chr9:107485653-107485680 | KLF4-528 | chr9:107489167-107489194 |
| KLF4-68 | chr9:107485666-107485693 | KLF4-529 | chr9:107489172-107489199 |
| KLF4-69 | chr9:107485670-107485697 | KLF4-530 | chr9:107489184-107489211 |
| KLF4-70 | chr9:107485671-107485698 | KLF4-531 | chr9:107489185-107489212 |
| KLF4-71 | chr9:107485694-107485721 | KLF4-532 | chr9:107489189-107489216 |
| KLF4-72 | chr9:107485702-107485729 | KLF4-533 | chr9:107489190-107489217 |
| KLF4-73 | chr9:107485703-107485730 | KLF4-534 | chr9:107489191-107489218 |
| KLF4-74 | chr9:107485720-107485747 | KLF4-535 | chr9:107489194-107489221 |
| KLF4-75 | chr9:107485721-107485748 | KLF4-536 | chr9:107489203-107489230 |
| KLF4-76 | chr9:107485737-107485764 | KLF4-537 | chr9:107489204-107489231 |
| KLF4-77 | chr9:107485749-107485776 | KLF4-538 | chr9:107489221-107489248 |
| KLF4-78 | chr9:107485754-107485781 | KLF4-539 | chr9:107489227-107489254 |
| KLF4-79 | chr9:107485757-107485784 | KLF4-540 | chr9:107489228-107489255 |
| KLF4-80 | chr9:107485760-107485787 | KLF4-541 | chr9:107489229-107489256 |
| KLF4-81 | chr9:107485767-107485794 | KLF4-542 | chr9:107489253-107489280 |
| KLF4-82 | chr9:107485778-107485805 | KLF4-543 | chr9:107489257-107489284 |
| KLF4-83 | chr9:107485786-107485813 | KLF4-544 | chr9:107489263-107489290 |
| KLF4-84 | chr9:107485790-107485817 | KLF4-545 | chr9:107489264-107489291 |
| KLF4-85 | chr9:107485796-107485823 | KLF4-546 | chr9:107489270-107489297 |
| KLF4-86 | chr9:107485801-107485828 | KLF4-547 | chr9:107489271-107489298 |
| KLF4-87 | chr9:107485802-107485829 | KLF4-548 | chr9:107489272-107489299 |
| KLF4-88 | chr9:107485805-107485832 | KLF4-549 | chr9:107489273-107489300 |
| KLF4-89 | chr9:107485823-107485850 | KLF4-550 | chr9:107489281-107489308 |
| KLF4-90 | chr9:107485833-107485860 | KLF4-551 | chr9:107489288-107489315 |
| KLF4-91 | chr9:107485835-107485862 | KLF4-552 | chr9:107489289-107489316 |
| KLF4-92 | chr9:107485836-107485863 | KLF4-553 | chr9:107489338-107489365 |
| KLF4-93 | chr9:107485837-107485864 | KLF4-554 | chr9:107489369-107489396 |
| KLF4-94 | chr9:107485850-107485877 | KLF4-555 | chr9:107489381-107489408 |
| KLF4-95 | chr9:107485851-107485878 | KLF4-556 | chr9:107489386-107489413 |
| KLF4-96 | chr9:107485856-107485883 | KLF4-557 | chr9:107489389-107489416 |
| KLF4-97 | chr9:107485886-107485913 | KLF4-558 | chr9:107489393-107489420 |
| KLF4-98 | chr9:107485890-107485917 | KLF4-559 | chr9:107489394-107489421 |
| KLF4-99 | chr9:107485891-107485918 | KLF4-560 | chr9:107489410-107489437 |
| KLF4-100 | chr9:107485896-107485923 | KLF4-561 | chr9:107489417-107489444 |
| KLF4-101 | chr9:107485900-107485927 | KLF4-562 | chr9:107489434-107489461 |
| KLF4-102 | chr9:107485901-107485928 | KLF4-563 | chr9:107489438-107489465 |
| KLF4-103 | chr9:107485908-107485935 | KLF4-564 | chr9:107489448-107489475 |
| KLF4-104 | chr9:107485926-107485953 | KLF4-565 | chr9:107489449-107489476 |
| KLF4-105 | chr9:107485972-107485999 | KLF4-566 | chr9:107489450-107489477 |
| KLF4-106 | chr9:107485980-107486007 | KLF4-567 | chr9:107489451-107489478 |
| KLF4-107 | chr9:107485981-107486008 | KLF4-568 | chr9:107489454-107489481 |
| KLF4-108 | chr9:107485982-107486009 | KLF4-569 | chr9:107489459-107489486 |
| KLF4-109 | chr9:107486000-107486027 | KLF4-570 | chr9:107489460-107489487 |
| KLF4-110 | chr9:107486012-107486039 | KLF4-571 | chr9:107489466-107489493 |
| KLF4-111 | chr9:107486016-107486043 | KLF4-572 | chr9:107489467-107489494 |
| KLF4-112 | chr9:107486022-107486049 | KLF4-573 | chr9:107489468-107489495 |
| KLF4-113 | chr9:107486023-107486050 | KLF4-574 | chr9:107489469-107489496 |
| KLF4-114 | chr9:107486901-107486928 | KLF4-575 | chr9:107489474-107489501 |
| KLF4-115 | chr9:107486906-107486933 | KLF4-576 | chr9:107489478-107489505 |
| KLF4-116 | chr9:107486913-107486940 | KLF4-577 | chr9:107489479-107489506 |
| KLF4-117 | chr9:107486914-107486941 | KLF4-578 | chr9:107489480-107489507 |
| KLF4-118 | chr9:107486916-107486943 | KLF4-579 | chr9:107489481-107489508 |
| KLF4-119 | chr9:107486923-107486950 | KLF4-580 | chr9:107489483-107489510 |
| KLF4-120 | chr9:107486926-107486953 | KLF4-581 | chr9:107489486-107489513 |
| KLF4-121 | chr9:107486934-107486961 | KLF4-582 | chr9:107489487-107489514 |
| KLF4-122 | chr9:107486936-107486963 | KLF4-583 | chr9:107489490-107489517 |
| KLF4-123 | chr9:107486946-107486973 | KLF4-584 | chr9:107489491-107489518 |
| KLF4-124 | chr9:107486956-107486983 | KLF4-585 | chr9:107489494-107489521 |
| KLF4-125 | chr9:107486957-107486984 | KLF4-586 | chr9:107489502-107489529 |
| KLF4-126 | chr9:107486958-107486985 | KLF4-587 | chr9:107489504-107489531 |
| KLF4-127 | chr9:107486962-107486989 | KLF4-588 | chr9:107489513-107489540 |
| KLF4-128 | chr9:107486967-107486994 | KLF4-589 | chr9:107489522-107489549 |
| KLF4-129 | chr9:107486977-107487004 | KLF4-590 | chr9:107489524-107489551 |
| KLF4-130 | chr9:107486997-107487024 | KLF4-591 | chr9:107489527-107489554 |
| KLF4-131 | chr9:107486998-107487025 | KLF4-592 | chr9:107489540-107489567 |
| KLF4-132 | chr9:107486999-107487026 | KLF4-593 | chr9:107489544-107489571 |
| KLF4-133 | chr9:107487000-107487027 | KLF4-594 | chr9:107489547-107489574 |
| KLF4-134 | chr9:107487001-107487028 | KLF4-595 | chr9:107489554-107489581 |
| KLF4-135 | chr9:107487004-107487031 | KLF4-596 | chr9:107489559-107489586 |
| KLF4-136 | chr9:107487005-107487032 | KLF4-597 | chr9:107489566-107489593 |
| KLF4-137 | chr9:107487008-107487035 | KLF4-598 | chr9:107489578-107489605 |
| KLF4-138 | chr9:107487009-107487036 | KLF4-599 | chr9:107489589-107489616 |
| KLF4-139 | chr9:107487017-107487044 | KLF4-600 | chr9:107489590-107489617 |
| KLF4-140 | chr9:107487020-107487047 | KLF4-601 | chr9:107489595-107489622 |
| KLF4-141 | chr9:107487021-107487048 | KLF4-602 | chr9:107489596-107489623 |
| KLF4-142 | chr9:107487022-107487049 | KLF4-603 | chr9:107489601-107489628 |
| KLF4-143 | chr9:107487023-107487050 | KLF4-604 | chr9:107489602-107489629 |
| KLF4-144 | chr9:107487027-107487054 | KLF4-605 | chr9:107489608-107489635 |
| KLF4-145 | chr9:107487032-107487059 | KLF4-606 | chr9:107489609-107489636 |
| KLF4-146 | chr9:107487033-107487060 | KLF4-607 | chr9:107489612-107489639 |
| KLF4-147 | chr9:107487048-107487075 | KLF4-608 | chr9:107489613-107489640 |
| KLF4-148 | chr9:107487049-107487076 | KLF4-609 | chr9:107489616-107489643 |
| KLF4-149 | chr9:107487080-107487107 | KLF4-610 | chr9:107489617-107489644 |
| KLF4-150 | chr9:107487081-107487108 | KLF4-611 | chr9:107489620-107489647 |
| KLF4-151 | chr9:107487084-107487111 | KLF4-612 | chr9:107489621-107489648 |
| KLF4-152 | chr9:107487087-107487114 | KLF4-613 | chr9:107489624-107489651 |
| KLF4-153 | chr9:107487088-107487115 | KLF4-614 | chr9:107489625-107489652 |
| KLF4-154 | chr9:107487098-107487125 | KLF4-615 | chr9:107489626-107489653 |
| KLF4-155 | chr9:107487099-107487126 | KLF4-616 | chr9:107489627-107489654 |
| KLF4-156 | chr9:107487100-107487127 | KLF4-617 | chr9:107489628-107489655 |
| KLF4-157 | chr9:107487116-107487143 | KLF4-618 | chr9:107489630-107489657 |
| KLF4-158 | chr9:107487122-107487149 | KLF4-619 | chr9:107489635-107489662 |
| KLF4-159 | chr9:107487124-107487151 | KLF4-620 | chr9:107489636-107489663 |
| KLF4-160 | chr9:107487128-107487155 | KLF4-621 | chr9:107489637-107489664 |
| KLF4-161 | chr9:107487129-107487156 | KLF4-622 | chr9:107489640-107489667 |
| KLF4-162 | chr9:107487130-107487157 | KLF4-623 | chr9:107489641-107489668 |
| KLF4-163 | chr9:107487139-107487166 | KLF4-624 | chr9:107489642-107489669 |
| KLF4-164 | chr9:107487141-107487168 | KLF4-625 | chr9:107489643-107489670 |
| KLF4-165 | chr9:107487142-107487169 | KLF4-626 | chr9:107489646-107489673 |
| KLF4-166 | chr9:107487143-107487170 | KLF4-627 | chr9:107489647-107489674 |
| KLF4-167 | chr9:107487147-107487174 | KLF4-628 | chr9:107489655-107489682 |
| KLF4-168 | chr9:107487153-107487180 | KLF4-629 | chr9:107489656-107489683 |
| KLF4-169 | chr9:107487154-107487181 | KLF4-630 | chr9:107489658-107489685 |
| KLF4-170 | chr9:107487157-107487184 | KLF4-631 | chr9:107489665-107489692 |
| KLF4-171 | chr9:107487160-107487187 | KLF4-632 | chr9:107489688-107489715 |
| KLF4-172 | chr9:107487170-107487197 | KLF4-633 | chr9:107489689-107489716 |
| KLF4-173 | chr9:107487171-107487198 | KLF4-634 | chr9:107489695-107489722 |
| KLF4-174 | chr9:107487172-107487199 | KLF4-635 | chr9:107489698-107489725 |
| KLF4-175 | chr9:107487177-107487204 | KLF4-636 | chr9:107489701-107489728 |
| KLF4-176 | chr9:107487181-107487208 | KLF4-637 | chr9:107489704-107489731 |
| KLF4-177 | chr9:107487184-107487211 | KLF4-638 | chr9:107489705-107489732 |
| KLF4-178 | chr9:107487185-107487212 | KLF4-639 | chr9:107489708-107489735 |
| KLF4-179 | chr9:107487186-107487213 | KLF4-640 | chr9:107489711-107489738 |
| KLF4-180 | chr9:107487215-107487242 | KLF4-641 | chr9:107489714-107489741 |
| KLF4-181 | chr9:107487216-107487243 | KLF4-642 | chr9:107489722-107489749 |
| KLF4-182 | chr9:107487217-107487244 | KLF4-643 | chr9:107489723-107489750 |
| KLF4-183 | chr9:107487224-107487251 | KLF4-644 | chr9:107489724-107489751 |
| KLF4-184 | chr9:107487225-107487252 | KLF4-645 | chr9:107489729-107489756 |
| KLF4-185 | chr9:107487231-107487258 | KLF4-646 | chr9:107489731-107489758 |
| KLF4-186 | chr9:107487232-107487259 | KLF4-647 | chr9:107489732-107489759 |
| KLF4-187 | chr9:107487233-107487260 | KLF4-648 | chr9:107489733-107489760 |
| KLF4-188 | chr9:107487234-107487261 | KLF4-649 | chr9:107489734-107489761 |
| KLF4-189 | chr9:107487245-107487272 | KLF4-650 | chr9:107489744-107489771 |
| KLF4-190 | chr9:107487260-107487287 | KLF4-651 | chr9:107489751-107489778 |
| KLF4-191 | chr9:107487261-107487288 | KLF4-652 | chr9:107489768-107489795 |
| KLF4-192 | chr9:107487262-107487289 | KLF4-653 | chr9:107489772-107489799 |
| KLF4-193 | chr9:107487263-107487290 | KLF4-654 | chr9:107489775-107489802 |
| KLF4-194 | chr9:107487264-107487291 | KLF4-655 | chr9:107489787-107489814 |
| KLF4-195 | chr9:107487268-107487295 | KLF4-656 | chr9:107489802-107489829 |
| KLF4-196 | chr9:107487269-107487296 | KLF4-657 | chr9:107489814-107489841 |
| KLF4-197 | chr9:107487272-107487299 | KLF4-658 | chr9:107489820-107489847 |
| KLF4-198 | chr9:107487278-107487305 | KLF4-659 | chr9:107489823-107489850 |
| KLF4-199 | chr9:107487282-107487309 | KLF4-660 | chr9:107489826-107489853 |
| KLF4-200 | chr9:107487283-107487310 | KLF4-661 | chr9:107489832-107489859 |
| KLF4-201 | chr9:107487284-107487311 | KLF4-662 | chr9:107489833-107489860 |
| KLF4-202 | chr9:107487286-107487313 | KLF4-663 | chr9:107489838-107489865 |
| KLF4-203 | chr9:107487287-107487314 | KLF4-664 | chr9:107489839-107489866 |
| KLF4-204 | chr9:107487294-107487321 | KLF4-665 | chr9:107489840-107489867 |
| KLF4-205 | chr9:107487295-107487322 | KLF4-666 | chr9:107489843-107489870 |
| KLF4-206 | chr9:107487309-107487336 | KLF4-667 | chr9:107489844-107489871 |
| KLF4-207 | chr9:107487310-107487337 | KLF4-668 | chr9:107489845-107489872 |
| KLF4-208 | chr9:107487314-107487341 | KLF4-669 | chr9:107489848-107489875 |
| KLF4-209 | chr9:107487318-107487345 | KLF4-670 | chr9:107489849-107489876 |
| KLF4-210 | chr9:107487322-107487349 | KLF4-671 | chr9:107489861-107489888 |
| KLF4-211 | chr9:107487323-107487350 | KLF4-672 | chr9:107489864-107489891 |
| KLF4-212 | chr9:107487330-107487357 | KLF4-673 | chr9:107489865-107489892 |
| KLF4-213 | chr9:107487331-107487358 | KLF4-674 | chr9:107489866-107489893 |
| KLF4-214 | chr9:107487337-107487364 | KLF4-675 | chr9:107489867-107489894 |
| KLF4-215 | chr9:107487338-107487365 | KLF4-676 | chr9:107489868-107489895 |
| KLF4-216 | chr9:107487341-107487368 | KLF4-677 | chr9:107489869-107489896 |
| KLF4-217 | chr9:107487342-107487369 | KLF4-678 | chr9:107489870-107489897 |
| KLF4-218 | chr9:107487343-107487370 | KLF4-679 | chr9:107489878-107489905 |
| KLF4-219 | chr9:107487347-107487374 | KLF4-680 | chr9:107489879-107489906 |
| KLF4-220 | chr9:107487354-107487381 | KLF4-681 | chr9:107489883-107489910 |
| KLF4-221 | chr9:107487355-107487382 | KLF4-682 | chr9:107489891-107489918 |
| KLF4-222 | chr9:107487358-107487385 | KLF4-683 | chr9:107489900-107489927 |
| KLF4-223 | chr9:107487359-107487386 | KLF4-684 | chr9:107489901-107489928 |
| KLF4-224 | chr9:107487360-107487387 | KLF4-685 | chr9:107489902-107489929 |
| KLF4-225 | chr9:107487361-107487388 | KLF4-686 | chr9:107489903-107489930 |
| KLF4-226 | chr9:107487364-107487391 | KLF4-687 | chr9:107489906-107489933 |
| KLF4-227 | chr9:107487368-107487395 | KLF4-688 | chr9:107489907-107489934 |
| KLF4-228 | chr9:107487369-107487396 | KLF4-689 | chr9:107489908-107489935 |
| KLF4-229 | chr9:107487373-107487400 | KLF4-690 | chr9:107489909-107489936 |
| KLF4-230 | chr9:107487374-107487401 | KLF4-691 | chr9:107489912-107489939 |
| KLF4-231 | chr9:107487378-107487405 | KLF4-692 | chr9:107489913-107489940 |
| KLF4-232 | chr9:107487409-107487436 | KLF4-693 | chr9:107489914-107489941 |
| KLF4-233 | chr9:107487410-107487437 | KLF4-694 | chr9:107489918-107489945 |
| KLF4-234 | chr9:107487413-107487440 | KLF4-695 | chr9:107489921-107489948 |
| KLF4-235 | chr9:107487414-107487441 | KLF4-696 | chr9:107489922-107489949 |
| KLF4-236 | chr9:107487418-107487445 | KLF4-697 | chr9:107489923-107489950 |
| KLF4-237 | chr9:107487419-107487446 | KLF4-698 | chr9:107489928-107489955 |
| KLF4-238 | chr9:107487420-107487447 | KLF4-699 | chr9:107489929-107489956 |
| KLF4-239 | chr9:107487427-107487454 | KLF4-700 | chr9:107489932-107489959 |
| KLF4-240 | chr9:107487432-107487459 | KLF4-701 | chr9:107489933-107489960 |
| KLF4-241 | chr9:107487433-107487460 | KLF4-702 | chr9:107489934-107489961 |
| KLF4-242 | chr9:107487434-107487461 | KLF4-703 | chr9:107489938-107489965 |
| KLF4-243 | chr9:107487435-107487462 | KLF4-704 | chr9:107489939-107489966 |
| KLF4-244 | chr9:107487436-107487463 | KLF4-705 | chr9:107489943-107489970 |
| KLF4-245 | chr9:107487449-107487476 | KLF4-706 | chr9:107489944-107489971 |
| KLF4-246 | chr9:107487450-107487477 | KLF4-707 | chr9:107489961-107489988 |
| KLF4-247 | chr9:107487451-107487478 | KLF4-708 | chr9:107489967-107489994 |
| KLF4-248 | chr9:107487452-107487479 | KLF4-709 | chr9:107489968-107489995 |
| KLF4-249 | chr9:107487467-107487494 | KLF4-710 | chr9:107489971-107489998 |
| KLF4-250 | chr9:107487469-107487496 | KLF4-711 | chr9:107489972-107489999 |
| KLF4-251 | chr9:107487470-107487497 | KLF4-712 | chr9:107489976-107490003 |
| KLF4-252 | chr9:107487471-107487498 | KLF4-713 | chr9:107489980-107490007 |
| KLF4-253 | chr9:107487472-107487499 | KLF4-714 | chr9:107489984-107490011 |
| KLF4-254 | chr9:107487473-107487500 | KLF4-715 | chr9:107489993-107490020 |
| KLF4-255 | chr9:107487476-107487503 | KLF4-716 | chr9:107489996-107490023 |
| KLF4-256 | chr9:107487490-107487517 | KLF4-717 | chr9:107490002-107490029 |
| KLF4-257 | chr9:107487491-107487518 | KLF4-718 | chr9:107490006-107490033 |
| KLF4-258 | chr9:107487492-107487519 | KLF4-719 | chr9:107490008-107490035 |
| KLF4-259 | chr9:107487493-107487520 | KLF4-720 | chr9:107490011-107490038 |
| KLF4-260 | chr9:107487497-107487524 | KLF4-721 | chr9:107490014-107490041 |
| KLF4-261 | chr9:107487504-107487531 | KLF4-722 | chr9:107490017-107490044 |
| KLF4-262 | chr9:107487509-107487536 | KLF4-723 | chr9:107490020-107490047 |
| KLF4-263 | chr9:107487510-107487537 | KLF4-724 | chr9:107490037-107490064 |
| KLF4-264 | chr9:107487522-107487549 | KLF4-725 | chr9:107490038-107490065 |
| KLF4-265 | chr9:107487528-107487555 | KLF4-726 | chr9:107490039-107490066 |
| KLF4-266 | chr9:107487529-107487556 | KLF4-727 | chr9:107490046-107490073 |
| KLF4-267 | chr9:107487543-107487570 | KLF4-728 | chr9:107490051-107490078 |
| KLF4-268 | chr9:107487544-107487571 | KLF4-729 | chr9:107490052-107490079 |
| KLF4-269 | chr9:107487545-107487572 | KLF4-730 | chr9:107490053-107490080 |
| KLF4-270 | chr9:107487550-107487577 | KLF4-731 | chr9:107490054-107490081 |
| KLF4-271 | chr9:107487553-107487580 | KLF4-732 | chr9:107490055-107490082 |
| KLF4-272 | chr9:107487556-107487583 | KLF4-733 | chr9:107490056-107490083 |
| KLF4-273 | chr9:107487559-107487586 | KLF4-734 | chr9:107490059-107490086 |
| KLF4-274 | chr9:107487573-107487600 | KLF4-735 | chr9:107490064-107490091 |
| KLF4-275 | chr9:107487574-107487601 | KLF4-736 | chr9:107490076-107490103 |
| KLF4-276 | chr9:107487587-107487614 | KLF4-737 | chr9:107490080-107490107 |
| KLF4-277 | chr9:107487588-107487615 | KLF4-738 | chr9:107490081-107490108 |
| KLF4-278 | chr9:107487589-107487616 | KLF4-739 | chr9:107490103-107490130 |
| KLF4-279 | chr9:107487590-107487617 | KLF4-740 | chr9:107490109-107490136 |
| KLF4-280 | chr9:107487591-107487618 | KLF4-741 | chr9:107490111-107490138 |
| KLF4-281 | chr9:107487593-107487620 | KLF4-742 | chr9:107490112-107490139 |
| KLF4-282 | chr9:107487604-107487631 | KLF4-743 | chr9:107490118-107490145 |
| KLF4-283 | chr9:107487605-107487632 | KLF4-744 | chr9:107490121-107490148 |
| KLF4-284 | chr9:107487607-107487634 | KLF4-745 | chr9:107490133-107490160 |
| KLF4-285 | chr9:107487610-107487637 | KLF4-746 | chr9:107490134-107490161 |
| KLF4-286 | chr9:107487613-107487640 | KLF4-747 | chr9:107490136-107490163 |
| KLF4-287 | chr9:107487624-107487651 | KLF4-748 | chr9:107490137-107490164 |
| KLF4-288 | chr9:107487634-107487661 | KLF4-749 | chr9:107490138-107490165 |
| KLF4-289 | chr9:107487635-107487662 | KLF4-750 | chr9:107490145-107490172 |
| KLF4-290 | chr9:107487636-107487663 | KLF4-751 | chr9:107490146-107490173 |
| KLF4-291 | chr9:107487655-107487682 | KLF4-752 | chr9:107490147-107490174 |
| KLF4-292 | chr9:107487656-107487683 | KLF4-753 | chr9:107490148-107490175 |
| KLF4-293 | chr9:107487657-107487684 | KLF4-754 | chr9:107490149-107490176 |
| KLF4-294 | chr9:107487666-107487693 | KLF4-755 | chr9:107490154-107490181 |
| KLF4-295 | chr9:107487678-107487705 | KLF4-756 | chr9:107490190-107490217 |
| KLF4-296 | chr9:107487697-107487724 | KLF4-757 | chr9:107490195-107490222 |
| KLF4-297 | chr9:107487706-107487733 | KLF4-758 | chr9:107490196-107490223 |
| KLF4-298 | chr9:107487709-107487736 | KLF4-759 | chr9:107490197-107490224 |
| KLF4-299 | chr9:107487714-107487741 | KLF4-760 | chr9:107490209-107490236 |
| KLF4-300 | chr9:107487715-107487742 | KLF4-761 | chr9:107490217-107490244 |
| KLF4-301 | chr9:107487722-107487749 | KLF4-762 | chr9:107490253-107490280 |
| KLF4-302 | chr9:107487723-107487750 | KLF4-763 | chr9:107490266-107490293 |
| KLF4-303 | chr9:107487724-107487751 | KLF4-764 | chr9:107490270-107490297 |
| KLF4-304 | chr9:107487726-107487753 | KLF4-765 | chr9:107490271-107490298 |
| KLF4-305 | chr9:107487727-107487754 | KLF4-766 | chr9:107490274-107490301 |
| KLF4-306 | chr9:107487729-107487756 | KLF4-767 | chr9:107490277-107490304 |
| KLF4-307 | chr9:107487739-107487766 | KLF4-768 | chr9:107490280-107490307 |
| KLF4-308 | chr9:107487740-107487767 | KLF4-769 | chr9:107490281-107490308 |
| KLF4-309 | chr9:107487751-107487778 | KLF4-770 | chr9:107490282-107490309 |
| KLF4-310 | chr9:107487758-107487785 | KLF4-771 | chr9:107490283-107490310 |
| KLF4-311 | chr9:107487763-107487790 | KLF4-772 | chr9:107490286-107490313 |
| KLF4-312 | chr9:107487765-107487792 | KLF4-773 | chr9:107490287-107490314 |
| KLF4-313 | chr9:107487769-107487796 | KLF4-774 | chr9:107490308-107490335 |
| KLF4-314 | chr9:107487779-107487806 | KLF4-775 | chr9:107490309-107490336 |
| KLF4-315 | chr9:107487783-107487810 | KLF4-776 | chr9:107490312-107490339 |
| KLF4-316 | chr9:107487784-107487811 | KLF4-777 | chr9:107490315-107490342 |
| KLF4-317 | chr9:107487785-107487812 | KLF4-778 | chr9:107490321-107490348 |
| KLF4-318 | chr9:107487793-107487820 | KLF4-779 | chr9:107490327-107490354 |
| KLF4-319 | chr9:107487801-107487828 | KLF4-780 | chr9:107490328-107490355 |
| KLF4-320 | chr9:107487804-107487831 | KLF4-781 | chr9:107490333-107490360 |
| KLF4-321 | chr9:107487805-107487832 | KLF4-782 | chr9:107490343-107490370 |
| KLF4-322 | chr9:107487809-107487836 | KLF4-783 | chr9:107490345-107490372 |
| KLF4-323 | chr9:107487816-107487843 | KLF4-784 | chr9:107490353-107490380 |
| KLF4-324 | chr9:107487817-107487844 | KLF4-785 | chr9:107490354-107490381 |
| KLF4-325 | chr9:107487826-107487853 | KLF4-786 | chr9:107490356-107490383 |
| KLF4-326 | chr9:107487829-107487856 | KLF4-787 | chr9:107490361-107490388 |
| KLF4-327 | chr9:107487830-107487857 | KLF4-788 | chr9:107490368-107490395 |
| KLF4-328 | chr9:107487831-107487858 | KLF4-789 | chr9:107490369-107490396 |
| KLF4-329 | chr9:107487832-107487859 | KLF4-790 | chr9:107490370-107490397 |
| KLF4-330 | chr9:107487837-107487864 | KLF4-791 | chr9:107490373-107490400 |
| KLF4-331 | chr9:107487847-107487874 | KLF4-792 | chr9:107490374-107490401 |
| KLF4-332 | chr9:107487854-107487881 | KLF4-793 | chr9:107490375-107490402 |
| KLF4-333 | chr9:107487857-107487884 | KLF4-794 | chr9:107490376-107490403 |
| KLF4-334 | chr9:107487868-107487895 | KLF4-795 | chr9:107490377-107490404 |
| KLF4-335 | chr9:107487869-107487896 | KLF4-796 | chr9:107490382-107490409 |
| KLF4-336 | chr9:107487873-107487900 | KLF4-797 | chr9:107490383-107490410 |
| KLF4-337 | chr9:107487877-107487904 | KLF4-798 | chr9:107490384-107490411 |
| KLF4-338 | chr9:107487885-107487912 | KLF4-799 | chr9:107490385-107490412 |
| KLF4-339 | chr9:107487888-107487915 | KLF4-800 | chr9:107490386-107490413 |
| KLF4-340 | chr9:107487889-107487916 | KLF4-801 | chr9:107490391-107490418 |
| KLF4-341 | chr9:107487890-107487917 | KLF4-802 | chr9:107490396-107490423 |
| KLF4-342 | chr9:107487891-107487918 | KLF4-803 | chr9:107490399-107490426 |
| KLF4-343 | chr9:107487892-107487919 | KLF4-804 | chr9:107490429-107490456 |
| KLF4-344 | chr9:107487897-107487924 | KLF4-805 | chr9:107490430-107490457 |
| KLF4-345 | chr9:107487900-107487927 | KLF4-806 | chr9:107490432-107490459 |
| KLF4-346 | chr9:107487901-107487928 | KLF4-807 | chr9:107490433-107490460 |
| KLF4-347 | chr9:107487905-107487932 | KLF4-808 | chr9:107490445-107490472 |
| KLF4-348 | chr9:107487907-107487934 | KLF4-809 | chr9:107490452-107490479 |
| KLF4-349 | chr9:107487910-107487937 | KLF4-810 | chr9:107490460-107490487 |
| KLF4-350 | chr9:107487912-107487939 | KLF4-811 | chr9:107490465-107490492 |
| KLF4-351 | chr9:107487913-107487940 | KLF4-812 | chr9:107490466-107490493 |
| KLF4-352 | chr9:107487915-107487942 | KLF4-813 | chr9:107490472-107490499 |
| KLF4-353 | chr9:107487923-107487950 | KLF4-814 | chr9:107490473-107490500 |
| KLF4-354 | chr9:107487924-107487951 | KLF4-815 | chr9:107490475-107490502 |
| KLF4-355 | chr9:107487927-107487954 | KLF4-816 | chr9:107490482-107490509 |
| KLF4-356 | chr9:107487928-107487955 | KLF4-817 | chr9:107490486-107490513 |
| KLF4-357 | chr9:107487929-107487956 | KLF4-818 | chr9:107490489-107490516 |
| KLF4-358 | chr9:107487930-107487957 | KLF4-819 | chr9:107490491-107490518 |
| KLF4-359 | chr9:107487933-107487960 | KLF4-820 | chr9:107490492-107490519 |
| KLF4-360 | chr9:107487934-107487961 | KLF4-821 | chr9:107490493-107490520 |
| KLF4-361 | chr9:107487942-107487969 | KLF4-822 | chr9:107490501-107490528 |
| KLF4-362 | chr9:107487946-107487973 | KLF4-823 | chr9:107490528-107490555 |
| KLF4-363 | chr9:107487947-107487974 | KLF4-824 | chr9:107490529-107490556 |
| KLF4-364 | chr9:107487961-107487988 | KLF4-825 | chr9:107490538-107490565 |
| KLF4-365 | chr9:107487975-107488002 | KLF4-826 | chr9:107490539-107490566 |
| KLF4-366 | chr9:107487978-107488005 | KLF4-827 | chr9:107490558-107490585 |
| KLF4-367 | chr9:107487993-107488020 | KLF4-828 | chr9:107490565-107490592 |
| KLF4-368 | chr9:107487999-107488026 | KLF4-829 | chr9:107490566-107490593 |
| KLF4-369 | chr9:107488002-107488029 | KLF4-830 | chr9:107490576-107490603 |
| KLF4-370 | chr9:107488005-107488032 | KLF4-831 | chr9:107490577-107490604 |
| KLF4-371 | chr9:107488018-107488045 | KLF4-832 | chr9:107490586-107490613 |
| KLF4-372 | chr9:107488021-107488048 | KLF4-833 | chr9:107490591-107490618 |
| KLF4-373 | chr9:107488025-107488052 | KLF4-834 | chr9:107490632-107490659 |
| KLF4-374 | chr9:107488026-107488053 | KLF4-835 | chr9:107490636-107490663 |
| KLF4-375 | chr9:107488033-107488060 | KLF4-836 | chr9:107490647-107490674 |
| KLF4-376 | chr9:107488038-107488065 | KLF4-837 | chr9:107490648-107490675 |
| KLF4-377 | chr9:107488042-107488069 | KLF4-838 | chr9:107490653-107490680 |
| KLF4-378 | chr9:107488055-107488082 | KLF4-839 | chr9:107490689-107490716 |
| KLF4-379 | chr9:107488061-107488088 | KLF4-840 | chr9:107490699-107490726 |
| KLF4-380 | chr9:107488078-107488105 | KLF4-841 | chr9:107490707-107490734 |
| KLF4-381 | chr9:107488080-107488107 | KLF4-842 | chr9:107490708-107490735 |
| KLF4-382 | chr9:107488084-107488111 | KLF4-843 | chr9:107490748-107490775 |
| KLF4-383 | chr9:107488093-107488120 | KLF4-844 | chr9:107490751-107490778 |
| KLF4-384 | chr9:107488097-107488124 | KLF4-845 | chr9:107490754-107490781 |
| KLF4-385 | chr9:107488098-107488125 | KLF4-846 | chr9:107490757-107490784 |
| KLF4-386 | chr9:107488099-107488126 | KLF4-847 | chr9:107490764-107490791 |
| KLF4-387 | chr9:107488102-107488129 | KLF4-848 | chr9:107490773-107490800 |
| KLF4-388 | chr9:107488106-107488133 | KLF4-849 | chr9:107490774-107490801 |
| KLF4-389 | chr9:107488115-107488142 | KLF4-850 | chr9:107490775-107490802 |
| KLF4-390 | chr9:107488129-107488156 | KLF4-851 | chr9:107490776-107490803 |
| KLF4-391 | chr9:107488131-107488158 | KLF4-852 | chr9:107490783-107490810 |
| KLF4-392 | chr9:107488140-107488167 | KLF4-853 | chr9:107490786-107490813 |
| KLF4-393 | chr9:107488143-107488170 | KLF4-854 | chr9:107490789-107490816 |
| KLF4-394 | chr9:107488148-107488175 | KLF4-855 | chr9:107490790-107490817 |
| KLF4-395 | chr9:107488150-107488177 | KLF4-856 | chr9:107490801-107490828 |
| KLF4-396 | chr9:107488155-107488182 | KLF4-857 | chr9:107490817-107490844 |
| KLF4-397 | chr9:107488161-107488188 | KLF4-858 | chr9:107490823-107490850 |
| KLF4-398 | chr9:107488164-107488191 | KLF4-859 | chr9:107490825-107490852 |
| KLF4-399 | chr9:107488171-107488198 | KLF4-860 | chr9:107490829-107490856 |
| KLF4-400 | chr9:107488175-107488202 | KLF4-861 | chr9:107490830-107490857 |
| KLF4-401 | chr9:107488182-107488209 | KLF4-862 | chr9:107490831-107490858 |
| KLF4-402 | chr9:107488183-107488210 | KLF4-863 | chr9:107490845-107490872 |
| KLF4-403 | chr9:107488184-107488211 | KLF4-864 | chr9:107490850-107490877 |
| KLF4-404 | chr9:107488187-107488214 | KLF4-865 | chr9:107490854-107490881 |
| KLF4-405 | chr9:107488191-107488218 | KLF4-866 | chr9:107490864-107490891 |
| KLF4-406 | chr9:107488192-107488219 | KLF4-867 | chr9:107490873-107490900 |
| KLF4-407 | chr9:107488195-107488222 | KLF4-868 | chr9:107490891-107490918 |
| KLF4-408 | chr9:107488198-107488225 | KLF4-869 | chr9:107490892-107490919 |
| KLF4-409 | chr9:107488201-107488228 | KLF4-870 | chr9:107490896-107490923 |
| KLF4-410 | chr9:107488202-107488229 | KLF4-871 | chr9:107490908-107490935 |
| KLF4-411 | chr9:107488203-107488230 | KLF4-872 | chr9:107490909-107490936 |
| KLF4-412 | chr9:107488204-107488231 | KLF4-873 | chr9:107490910-107490937 |
| KLF4-413 | chr9:107488215-107488242 | KLF4-874 | chr9:107490914-107490941 |
| KLF4-414 | chr9:107488220-107488247 | KLF4-875 | chr9:107490917-107490944 |
| KLF4-415 | chr9:107488221-107488248 | KLF4-876 | chr9:107490920-107490947 |
| KLF4-416 | chr9:107488246-107488273 | KLF4-877 | chr9:107490923-107490950 |
| KLF4-417 | chr9:107488247-107488274 | KLF4-878 | chr9:107490924-107490951 |
| KLF4-418 | chr9:107488248-107488275 | KLF4-879 | chr9:107490951-107490978 |
| KLF4-419 | chr9:107488253-107488280 | KLF4-880 | chr9:107490957-107490984 |
| KLF4-420 | chr9:107488254-107488281 | KLF4-881 | chr9:107491013-107491040 |
| KLF4-421 | chr9:107488255-107488282 | KLF4-882 | chr9:107491014-107491041 |
| KLF4-422 | chr9:107488257-107488284 | KLF4-883 | chr9:107491054-107491081 |
| KLF4-423 | chr9:107488258-107488285 | KLF4-884 | chr9:107491055-107491082 |
| KLF4-424 | chr9:107488259-107488286 | KLF4-885 | chr9:107491066-107491093 |
| KLF4-425 | chr9:107488262-107488289 | KLF4-886 | chr9:107491071-107491098 |
| KLF4-426 | chr9:107488274-107488301 | KLF4-887 | chr9:107491078-107491105 |
| KLF4-427 | chr9:107488277-107488304 | KLF4-888 | chr9:107491086-107491113 |
| KLF4-428 | chr9:107488291-107488318 | KLF4-889 | chr9:107491096-107491123 |
| KLF4-429 | chr9:107488306-107488333 | KLF4-890 | chr9:107491103-107491130 |
| KLF4-430 | chr9:107488307-107488334 | KLF4-891 | chr9:107491114-107491141 |
| KLF4-431 | chr9:107488308-107488335 | KLF4-892 | chr9:107491120-107491147 |
| KLF4-432 | chr9:107488311-107488338 | KLF4-893 | chr9:107491126-107491153 |
| KLF4-433 | chr9:107488314-107488341 | KLF4-894 | chr9:107491132-107491159 |
| KLF4-434 | chr9:107488315-107488342 | KLF4-895 | chr9:107491133-107491160 |
| KLF4-435 | chr9:107488318-107488345 | KLF4-896 | chr9:107491137-107491164 |
| KLF4-436 | chr9:107488319-107488346 | KLF4-897 | chr9:107491141-107491168 |
| KLF4-437 | chr9:107488343-107488370 | KLF4-898 | chr9:107491142-107491169 |
| KLF4-438 | chr9:107488344-107488371 | KLF4-899 | chr9:107491154-107491181 |
| KLF4-439 | chr9:107488345-107488372 | KLF4-900 | chr9:107491155-107491182 |
| KLF4-440 | chr9:107488346-107488373 | KLF4-901 | chr9:107491156-107491183 |
| KLF4-441 | chr9:107488351-107488378 | KLF4-902 | chr9:107491165-107491192 |
| KLF4-442 | chr9:107488356-107488383 | KLF4-903 | chr9:107491166-107491193 |
| KLF4-443 | chr9:107488358-107488385 | KLF4-904 | chr9:107491170-107491197 |
| KLF4-444 | chr9:107488360-107488387 | KLF4-905 | chr9:107491171-107491198 |
| KLF4-445 | chr9:107488361-107488388 | KLF4-906 | chr9:107491172-107491199 |
| KLF4-446 | chr9:107488362-107488389 | KLF4-907 | chr9:107491173-107491200 |
| KLF4-447 | chr9:107488809-107488836 | KLF4-908 | chr9:107491174-107491201 |
| KLF4-448 | chr9:107488816-107488843 | KLF4-909 | chr9:107491184-107491211 |
| KLF4-449 | chr9:107488820-107488847 | KLF4-910 | chr9:107491187-107491214 |
| KLF4-450 | chr9:107488823-107488850 | KLF4-911 | chr9:107491188-107491215 |
| KLF4-451 | chr9:107488825-107488852 | KLF4-912 | chr9:107491190-107491217 |
| KLF4-452 | chr9:107488830-107488857 | KLF4-913 | chr9:107491200-107491227 |
| KLF4-453 | chr9:107488831-107488858 | KLF4-914 | chr9:107491203-107491230 |
| KLF4-454 | chr9:107488834-107488861 | KLF4-915 | chr9:107491204-107491231 |
| KLF4-455 | chr9:107488854-107488881 | KLF4-916 | chr9:107491207-107491234 |
| KLF4-456 | chr9:107488855-107488882 | KLF4-917 | chr9:107491234-107491261 |
| KLF4-457 | chr9:107488856-107488883 | KLF4-918 | chr9:107491244-107491271 |
| KLF4-458 | chr9:107488864-107488891 | KLF4-919 | chr9:107491256-107491283 |
| KLF4-459 | chr9:107488867-107488894 | KLF4-920 | chr9:107491261-107491288 |
| KLF4-460 | chr9:107488868-107488895 | KLF4-921 | chr9:107491262-107491289 |
| KLF4-461 | chr9:107488871-107488898 | KLF4-922 | chr9:107491265-107491292 |

**Table 2L Genomic coordinates of the human NDST1 gene**

| Target ID | Target Coordinate Range | Target ID | Target Coordinate Range |
|---|---|---|---|
| NDST1-1 | chr5:150496256-150496283 | NDST1-1347 | chr5:150540200-150540227 |
| NDST1-2 | chr5:150496260-150496287 | NDST1-1348 | chr5:150540201-150540228 |
| NDST1-3 | chr5:150496263-150496290 | NDST1-1349 | chr5:150540202-150540229 |
| NDST1-4 | chr5:150496264-150496291 | NDST1-1350 | chr5:150540217-150540244 |
| NDST1-5 | chr5:150496279-150496306 | NDST1-1351 | chr5:150540223-150540250 |
| NDST1-6 | chr5:150496297-150496324 | NDST1-1352 | chr5:150540228-150540255 |
| NDST1-7 | chr5:150496301-150496328 | NDST1-1353 | chr5:150540234-150540261 |
| NDST1-8 | chr5:150496302-150496329 | NDST1-1354 | chr5:150540238-150540265 |
| NDST1-9 | chr5:150496316-150496343 | NDST1-1355 | chr5:150540239-150540266 |
| NDST1-10 | chr5:150496320-150496347 | NDST1-1356 | chr5:150540241-150540268 |
| NDST1-11 | chr5:150496339-150496366 | NDST1-1357 | chr5:150540242-150540269 |
| NDST1-12 | chr5:150496340-150496367 | NDST1-1358 | chr5:150540243-150540270 |
| NDST1-13 | chr5:150496349-150496376 | NDST1-1359 | chr5:150540244-150540271 |
| NDST1-14 | chr5:150496362-150496389 | NDST1-1360 | chr5:150540245-150540272 |
| NDST1-15 | chr5:150496363-150496390 | NDST1-1361 | chr5:150540249-150540276 |
| NDST1-16 | chr5:150496364-150496391 | NDST1-1362 | chr5:150540250-150540277 |
| NDST1-17 | chr5:150496370-150496397 | NDST1-1363 | chr5:150540252-150540279 |
| NDST1-18 | chr5:150496413-150496440 | NDST1-1364 | chr5:150540253-150540280 |
| NDST1-19 | chr5:150496414-150496441 | NDST1-1365 | chr5:150540258-150540285 |
| NDST1-20 | chr5:150496420-150496447 | NDST1-1366 | chr5:150540259-150540286 |
| NDST1-21 | chr5:150496421-150496448 | NDST1-1367 | chr5:150540263-150540290 |
| NDST1-22 | chr5:150496442-150496469 | NDST1-1368 | chr5:150540273-150540300 |
| NDST1-23 | chr5:150496451-150496478 | NDST1-1369 | chr5:150540274-150540301 |
| NDST1-24 | chr5:150496452-150496479 | NDST1-1370 | chr5:150540278-150540305 |
| NDST1-25 | chr5:150496453-150496480 | NDST1-1371 | chr5:150540281-150540308 |
| NDST1-26 | chr5:150496455-150496482 | NDST1-1372 | chr5:150540296-150540323 |
| NDST1-27 | chr5:150496456-150496483 | NDST1-1373 | chr5:150540310-150540337 |
| NDST1-28 | chr5:150496464-150496491 | NDST1-1374 | chr5:150540313-150540340 |
| NDST1-29 | chr5:150496465-150496492 | NDST1-1375 | chr5:150540314-150540341 |
| NDST1-30 | chr5:150496472-150496499 | NDST1-1376 | chr5:150540322-150540349 |
| NDST1-31 | chr5:150496473-150496500 | NDST1-1377 | chr5:150540332-150540359 |
| NDST1-32 | chr5:150496476-150496503 | NDST1-1378 | chr5:150540355-150540382 |
| NDST1-33 | chr5:150496499-150496526 | NDST1-1379 | chr5:150540363-150540390 |
| NDST1-34 | chr5:150496500-150496527 | NDST1-1380 | chr5:150541455-150541482 |
| NDST1-35 | chr5:150496501-150496528 | NDST1-1381 | chr5:150541456-150541483 |
| NDST1-36 | chr5:150496506-150496533 | NDST1-1382 | chr5:150541464-150541491 |
| NDST1-37 | chr5:150496507-150496534 | NDST1-1383 | chr5:150541465-150541492 |
| NDST1-38 | chr5:150496508-150496535 | NDST1-1384 | chr5:150541475-150541502 |
| NDST1-39 | chr5:150496516-150496543 | NDST1-1385 | chr5:150541476-150541503 |
| NDST1-40 | chr5:150496522-150496549 | NDST1-1386 | chr5:150541486-150541513 |
| NDST1-41 | chr5:150496523-150496550 | NDST1-1387 | chr5:150541487-150541514 |
| NDST1-42 | chr5:150496535-150496562 | NDST1-1388 | chr5:150541493-150541520 |
| NDST1-43 | chr5:150496536-150496563 | NDST1-1389 | chr5:150541501-150541528 |
| NDST1-44 | chr5:150496544-150496571 | NDST1-1390 | chr5:150541502-150541529 |
| NDST1-45 | chr5:150496545-150496572 | NDST1-1391 | chr5:150541508-150541535 |
| NDST1-46 | chr5:150496546-150496573 | NDST1-1392 | chr5:150541509-150541536 |
| NDST1-47 | chr5:150496547-150496574 | NDST1-1393 | chr5:150541510-150541537 |
| NDST1-48 | chr5:150496556-150496583 | NDST1-1394 | chr5:150541518-150541545 |
| NDST1-49 | chr5:150496557-150496584 | NDST1-1395 | chr5:150541540-150541567 |
| NDST1-50 | chr5:150496563-150496590 | NDST1-1396 | chr5:150541541-150541568 |
| NDST1-51 | chr5:150496565-150496592 | NDST1-1397 | chr5:150541542-150541569 |
| NDST1-52 | chr5:150496593-150496620 | NDST1-1398 | chr5:150541543-150541570 |
| NDST1-53 | chr5:150496598-150496625 | NDST1-1399 | chr5:150541550-150541577 |
| NDST1-54 | chr5:150496600-150496627 | NDST1-1400 | chr5:150541551-150541578 |
| NDST1-55 | chr5:150496602-150496629 | NDST1-1401 | chr5:150541555-150541582 |
| NDST1-56 | chr5:150496603-150496630 | NDST1-1402 | chr5:150541572-150541599 |
| NDST1-57 | chr5:150496604-150496631 | NDST1-1403 | chr5:150541573-150541600 |
| NDST1-58 | chr5:150496617-150496644 | NDST1-1404 | chr5:150541574-150541601 |
| NDST1-59 | chr5:150496621-150496648 | NDST1-1405 | chr5:150541578-150541605 |
| NDST1-60 | chr5:150496639-150496666 | NDST1-1406 | chr5:150541585-150541612 |
| NDST1-61 | chr5:150496640-150496667 | NDST1-1407 | chr5:150541607-150541634 |
| NDST1-62 | chr5:150496645-150496672 | NDST1-1408 | chr5:150541625-150541652 |
| NDST1-63 | chr5:150496646-150496673 | NDST1-1409 | chr5:150541626-150541653 |
| NDST1-64 | chr5:150496649-150496676 | NDST1-1410 | chr5:150541632-150541659 |
| NDST1-65 | chr5:150496651-150496678 | NDST1-1411 | chr5:150541633-150541660 |
| NDST1-66 | chr5:150496652-150496679 | NDST1-1412 | chr5:150541640-150541667 |
| NDST1-67 | chr5:150496656-150496683 | NDST1-1413 | chr5:150541641-150541668 |
| NDST1-68 | chr5:150496657-150496684 | NDST1-1414 | chr5:150541644-150541671 |
| NDST1-69 | chr5:150496658-150496685 | NDST1-1415 | chr5:150541653-150541680 |
| NDST1-70 | chr5:150496661-150496688 | NDST1-1416 | chr5:150541654-150541681 |
| NDST1-71 | chr5:150496662-150496689 | NDST1-1417 | chr5:150541655-150541682 |
| NDST1-72 | chr5:150496663-150496690 | NDST1-1418 | chr5:150541656-150541683 |
| NDST1-73 | chr5:150496671-150496698 | NDST1-1419 | chr5:150541687-150541714 |
| NDST1-74 | chr5:150496672-150496699 | NDST1-1420 | chr5:150541695-150541722 |
| NDST1-75 | chr5:150496675-150496702 | NDST1-1421 | chr5:150541696-150541723 |
| NDST1-76 | chr5:150496691-150496718 | NDST1-1422 | chr5:150541697-150541724 |
| NDST1-77 | chr5:150496692-150496719 | NDST1-1423 | chr5:150541704-150541731 |
| NDST1-78 | chr5:150496693-150496720 | NDST1-1424 | chr5:150541705-150541732 |
| NDST1-79 | chr5:150496694-150496721 | NDST1-1425 | chr5:150541717-150541744 |
| NDST1-80 | chr5:150496703-150496730 | NDST1-1426 | chr5:150541720-150541747 |
| NDST1-81 | chr5:150496704-150496731 | NDST1-1427 | chr5:150541732-150541759 |
| NDST1-82 | chr5:150496706-150496733 | NDST1-1428 | chr5:150541737-150541764 |
| NDST1-83 | chr5:150496733-150496760 | NDST1-1429 | chr5:150541742-150541769 |
| NDST1-84 | chr5:150496734-150496761 | NDST1-1430 | chr5:150541743-150541770 |
| NDST1-85 | chr5:150496735-150496762 | NDST1-1431 | chr5:150541748-150541775 |
| NDST1-86 | chr5:150496736-150496763 | NDST1-1432 | chr5:150541749-150541776 |
| NDST1-87 | chr5:150496738-150496765 | NDST1-1433 | chr5:150541750-150541777 |
| NDST1-88 | chr5:150496739-150496766 | NDST1-1434 | chr5:150541755-150541782 |
| NDST1-89 | chr5:150496740-150496767 | NDST1-1435 | chr5:150541756-150541783 |
| NDST1-90 | chr5:150496752-150496779 | NDST1-1436 | chr5:150541763-150541790 |
| NDST1-91 | chr5:150496754-150496781 | NDST1-1437 | chr5:150542722-150542749 |
| NDST1-92 | chr5:150496774-150496801 | NDST1-1438 | chr5:150542723-150542750 |
| NDST1-93 | chr5:150496785-150496812 | NDST1-1439 | chr5:150542734-150542761 |
| NDST1-94 | chr5:150496789-150496816 | NDST1-1440 | chr5:150542735-150542762 |
| NDST1-95 | chr5:150496797-150496824 | NDST1-1441 | chr5:150542740-150542767 |
| NDST1-96 | chr5:150496798-150496825 | NDST1-1442 | chr5:150542741-150542768 |
| NDST1-97 | chr5:150496803-150496830 | NDST1-1443 | chr5:150542742-150542769 |
| NDST1-98 | chr5:150496804-150496831 | NDST1-1444 | chr5:150542743-150542770 |
| NDST1-99 | chr5:150496805-150496832 | NDST1-1445 | chr5:150542744-150542771 |
| NDST1-100 | chr5:150496806-150496833 | NDST1-1446 | chr5:150542745-150542772 |
| NDST1-101 | chr5:150496816-150496843 | NDST1-1447 | chr5:150542750-150542777 |
| NDST1-102 | chr5:150496824-150496851 | NDST1-1448 | chr5:150542752-150542779 |
| NDST1-103 | chr5:150496832-150496859 | NDST1-1449 | chr5:150542753-150542780 |
| NDST1-104 | chr5:150496834-150496861 | NDST1-1450 | chr5:150542754-150542781 |
| NDST1-105 | chr5:150496835-150496862 | NDST1-1451 | chr5:150542755-150542782 |
| NDST1-106 | chr5:150496838-150496865 | NDST1-1452 | chr5:150542776-150542803 |
| NDST1-107 | chr5:150496839-150496866 | NDST1-1453 | chr5:150542778-150542805 |
| NDST1-108 | chr5:150496845-150496872 | NDST1-1454 | chr5:150542779-150542806 |
| NDST1-109 | chr5:150496846-150496873 | NDST1-1455 | chr5:150542780-150542807 |
| NDST1-110 | chr5:150496858-150496885 | NDST1-1456 | chr5:150542781-150542808 |
| NDST1-111 | chr5:150496859-150496886 | NDST1-1457 | chr5:150542782-150542809 |
| NDST1-112 | chr5:150496892-150496919 | NDST1-1458 | chr5:150542783-150542810 |
| NDST1-113 | chr5:150496898-150496925 | NDST1-1459 | chr5:150542785-150542812 |
| NDST1-114 | chr5:150496922-150496949 | NDST1-1460 | chr5:150542786-150542813 |
| NDST1-115 | chr5:150496945-150496972 | NDST1-1461 | chr5:150542787-150542814 |
| NDST1-116 | chr5:150496949-150496976 | NDST1-1462 | chr5:150542788-150542815 |
| NDST1-117 | chr5:150496979-150497006 | NDST1-1463 | chr5:150542794-150542821 |
| NDST1-118 | chr5:150496980-150497007 | NDST1-1464 | chr5:150542795-150542822 |
| NDST1-119 | chr5:150497042-150497069 | NDST1-1465 | chr5:150542802-150542829 |
| NDST1-120 | chr5:150497046-150497073 | NDST1-1466 | chr5:150542816-150542843 |
| NDST1-121 | chr5:150497056-150497083 | NDST1-1467 | chr5:150542817-150542844 |
| NDST1-122 | chr5:150497064-150497091 | NDST1-1468 | chr5:150542821-150542848 |
| NDST1-123 | chr5:150497065-150497092 | NDST1-1469 | chr5:150542836-150542863 |
| NDST1-124 | chr5:150497067-150497094 | NDST1-1470 | chr5:150542837-150542864 |
| NDST1-125 | chr5:150497091-150497118 | NDST1-1471 | chr5:150542840-150542867 |
| NDST1-126 | chr5:150497092-150497119 | NDST1-1472 | chr5:150542841-150542868 |
| NDST1-127 | chr5:150497105-150497132 | NDST1-1473 | chr5:150542842-150542869 |
| NDST1-128 | chr5:150497108-150497135 | NDST1-1474 | chr5:150542847-150542874 |
| NDST1-129 | chr5:150497114-150497141 | NDST1-1475 | chr5:150542848-150542875 |
| NDST1-130 | chr5:150497115-150497142 | NDST1-1476 | chr5:150542851-150542878 |
| NDST1-131 | chr5:150497131-150497158 | NDST1-1477 | chr5:150542857-150542884 |
| NDST1-132 | chr5:150497132-150497159 | NDST1-1478 | chr5:150542858-150542885 |
| NDST1-133 | chr5:150497133-150497160 | NDST1-1479 | chr5:150542864-150542891 |
| NDST1-134 | chr5:150497137-150497164 | NDST1-1480 | chr5:150542870-150542897 |
| NDST1-135 | chr5:150497140-150497167 | NDST1-1481 | chr5:150542882-150542909 |
| NDST1-136 | chr5:150497143-150497170 | NDST1-1482 | chr5:150542883-150542910 |
| NDST1-137 | chr5:150497146-150497173 | NDST1-1483 | chr5:150542888-150542915 |
| NDST1-138 | chr5:150497147-150497174 | NDST1-1484 | chr5:150542898-150542925 |
| NDST1-139 | chr5:150497174-150497201 | NDST1-1485 | chr5:150542903-150542930 |
| NDST1-140 | chr5:150497177-150497204 | NDST1-1486 | chr5:150542904-150542931 |
| NDST1-141 | chr5:150497182-150497209 | NDST1-1487 | chr5:150542905-150542932 |
| NDST1-142 | chr5:150497183-150497210 | NDST1-1488 | chr5:150542917-150542944 |
| NDST1-143 | chr5:150497191-150497218 | NDST1-1489 | chr5:150542930-150542957 |
| NDST1-144 | chr5:150497192-150497219 | NDST1-1490 | chr5:150542935-150542962 |
| NDST1-145 | chr5:150497193-150497220 | NDST1-1491 | chr5:150542945-150542972 |
| NDST1-146 | chr5:150497212-150497239 | NDST1-1492 | chr5:150542953-150542980 |
| NDST1-147 | chr5:150497216-150497243 | NDST1-1493 | chr5:150542971-150542998 |
| NDST1-148 | chr5:150497226-150497253 | NDST1-1494 | chr5:150542972-150542999 |
| NDST1-149 | chr5:150497235-150497262 | NDST1-1495 | chr5:150542976-150543003 |
| NDST1-150 | chr5:150497237-150497264 | NDST1-1496 | chr5:150542977-150543004 |
| NDST1-151 | chr5:150497253-150497280 | NDST1-1497 | chr5:150542981-150543008 |
| NDST1-152 | chr5:150497254-150497281 | NDST1-1498 | chr5:150542990-150543017 |
| NDST1-153 | chr5:150497258-150497285 | NDST1-1499 | chr5:150542991-150543018 |
| NDST1-154 | chr5:150497270-150497297 | NDST1-1500 | chr5:150542992-150543019 |
| NDST1-155 | chr5:150497271-150497298 | NDST1-1501 | chr5:150543001-150543028 |
| NDST1-156 | chr5:150497272-150497299 | NDST1-1502 | chr5:150543009-150543036 |
| NDST1-157 | chr5:150497279-150497306 | NDST1-1503 | chr5:150543013-150543040 |
| NDST1-158 | chr5:150497282-150497309 | NDST1-1504 | chr5:150543020-150543047 |
| NDST1-159 | chr5:150497285-150497312 | NDST1-1505 | chr5:150543023-150543050 |
| NDST1-160 | chr5:150497286-150497313 | NDST1-1506 | chr5:150543024-150543051 |
| NDST1-161 | chr5:150497298-150497325 | NDST1-1507 | chr5:150543033-150543060 |
| NDST1-162 | chr5:150497306-150497333 | NDST1-1508 | chr5:150543041-150543068 |
| NDST1-163 | chr5:150497316-150497343 | NDST1-1509 | chr5:150543070-150543097 |
| NDST1-164 | chr5:150497321-150497348 | NDST1-1510 | chr5:150543071-150543098 |
| NDST1-165 | chr5:150497326-150497353 | NDST1-1511 | chr5:150545190-150545217 |
| NDST1-166 | chr5:150497327-150497354 | NDST1-1512 | chr5:150545200-150545227 |
| NDST1-167 | chr5:150497328-150497355 | NDST1-1513 | chr5:150545201-150545228 |
| NDST1-168 | chr5:150497334-150497361 | NDST1-1514 | chr5:150545226-150545253 |
| NDST1-169 | chr5:150497340-150497367 | NDST1-1515 | chr5:150545227-150545254 |
| NDST1-170 | chr5:150497341-150497368 | NDST1-1516 | chr5:150545228-150545255 |
| NDST1-171 | chr5:150497345-150497372 | NDST1-1517 | chr5:150545234-150545261 |
| NDST1-172 | chr5:150497379-150497406 | NDST1-1518 | chr5:150545239-150545266 |
| NDST1-173 | chr5:150497387-150497414 | NDST1-1519 | chr5:150545240-150545267 |
| NDST1-174 | chr5:150497390-150497417 | NDST1-1520 | chr5:150545254-150545281 |
| NDST1-175 | chr5:150497398-150497425 | NDST1-1521 | chr5:150545255-150545282 |
| NDST1-176 | chr5:150497401-150497428 | NDST1-1522 | chr5:150545261-150545288 |
| NDST1-177 | chr5:150497402-150497429 | NDST1-1523 | chr5:150545262-150545289 |
| NDST1-178 | chr5:150497403-150497430 | NDST1-1524 | chr5:150545263-150545290 |
| NDST1-179 | chr5:150497404-150497431 | NDST1-1525 | chr5:150545264-150545291 |
| NDST1-180 | chr5:150497405-150497432 | NDST1-1526 | chr5:150545267-150545294 |
| NDST1-181 | chr5:150497416-150497443 | NDST1-1527 | chr5:150545273-150545300 |
| NDST1-182 | chr5:150497429-150497456 | NDST1-1528 | chr5:150545274-150545301 |
| NDST1-183 | chr5:150497433-150497460 | NDST1-1529 | chr5:150545285-150545312 |
| NDST1-184 | chr5:150497438-150497465 | NDST1-1530 | chr5:150545289-150545316 |
| NDST1-185 | chr5:150497444-150497471 | NDST1-1531 | chr5:150545292-150545319 |
| NDST1-186 | chr5:150497460-150497487 | NDST1-1532 | chr5:150545305-150545332 |
| NDST1-187 | chr5:150497467-150497494 | NDST1-1533 | chr5:150545327-150545354 |
| NDST1-188 | chr5:150497477-150497504 | NDST1-1534 | chr5:150545328-150545355 |
| NDST1-189 | chr5:150497495-150497522 | NDST1-1535 | chr5:150545329-150545356 |
| NDST1-190 | chr5:150497496-150497523 | NDST1-1536 | chr5:150545333-150545360 |
| NDST1-191 | chr5:150497499-150497526 | NDST1-1537 | chr5:150545334-150545361 |
| NDST1-192 | chr5:150497502-150497529 | NDST1-1538 | chr5:150545338-150545365 |
| NDST1-193 | chr5:150497506-150497533 | NDST1-1539 | chr5:150545344-150545371 |
| NDST1-194 | chr5:150497511-150497538 | NDST1-1540 | chr5:150545347-150545374 |
| NDST1-195 | chr5:150497512-150497539 | NDST1-1541 | chr5:150545350-150545377 |
| NDST1-196 | chr5:150497513-150497540 | NDST1-1542 | chr5:150545370-150545397 |
| NDST1-197 | chr5:150497514-150497541 | NDST1-1543 | chr5:150545374-150545401 |
| NDST1-198 | chr5:150497515-150497542 | NDST1-1544 | chr5:150545378-150545405 |
| NDST1-199 | chr5:150497518-150497545 | NDST1-1545 | chr5:150545381-150545408 |
| NDST1-200 | chr5:150497519-150497546 | NDST1-1546 | chr5:150545382-150545409 |
| NDST1-201 | chr5:150497522-150497549 | NDST1-1547 | chr5:150545400-150545427 |
| NDST1-202 | chr5:150497525-150497552 | NDST1-1548 | chr5:150545401-150545428 |
| NDST1-203 | chr5:150497534-150497561 | NDST1-1549 | chr5:150545402-150545429 |
| NDST1-204 | chr5:150497535-150497562 | NDST1-1550 | chr5:150545409-150545436 |
| NDST1-205 | chr5:150497536-150497563 | NDST1-1551 | chr5:150545416-150545443 |
| NDST1-206 | chr5:150497564-150497591 | NDST1-1552 | chr5:150545417-150545444 |
| NDST1-207 | chr5:150497565-150497592 | NDST1-1553 | chr5:150545424-150545451 |
| NDST1-208 | chr5:150497566-150497593 | NDST1-1554 | chr5:150545425-150545452 |
| NDST1-209 | chr5:150497567-150497594 | NDST1-1555 | chr5:150545431-150545458 |
| NDST1-210 | chr5:150497571-150497598 | NDST1-1556 | chr5:150545436-150545463 |
| NDST1-211 | chr5:150497577-150497604 | NDST1-1557 | chr5:150545438-150545465 |
| NDST1-212 | chr5:150497582-150497609 | NDST1-1558 | chr5:150545441-150545468 |
| NDST1-213 | chr5:150497587-150497614 | NDST1-1559 | chr5:150545442-150545469 |
| NDST1-214 | chr5:150497593-150497620 | NDST1-1560 | chr5:150545443-150545470 |
| NDST1-215 | chr5:150497594-150497621 | NDST1-1561 | chr5:150545447-150545474 |
| NDST1-216 | chr5:150497605-150497632 | NDST1-1562 | chr5:150545453-150545480 |
| NDST1-217 | chr5:150497611-150497638 | NDST1-1563 | chr5:150545456-150545483 |
| NDST1-218 | chr5:150497614-150497641 | NDST1-1564 | chr5:150545457-150545484 |
| NDST1-219 | chr5:150497615-150497642 | NDST1-1565 | chr5:150545458-150545485 |
| NDST1-220 | chr5:150497616-150497643 | NDST1-1566 | chr5:150545460-150545487 |
| NDST1-221 | chr5:150497622-150497649 | NDST1-1567 | chr5:150545465-150545492 |
| NDST1-222 | chr5:150497631-150497658 | NDST1-1568 | chr5:150545467-150545494 |
| NDST1-223 | chr5:150497632-150497659 | NDST1-1569 | chr5:150545468-150545495 |
| NDST1-224 | chr5:150497640-150497667 | NDST1-1570 | chr5:150545469-150545496 |
| NDST1-225 | chr5:150497663-150497690 | NDST1-1571 | chr5:150545470-150545497 |
| NDST1-226 | chr5:150497667-150497694 | NDST1-1572 | chr5:150545473-150545500 |
| NDST1-227 | chr5:150497673-150497700 | NDST1-1573 | chr5:150545474-150545501 |
| NDST1-228 | chr5:150497676-150497703 | NDST1-1574 | chr5:150545475-150545502 |
| NDST1-229 | chr5:150497677-150497704 | NDST1-1575 | chr5:150545476-150545503 |
| NDST1-230 | chr5:150497683-150497710 | NDST1-1576 | chr5:150545478-150545505 |
| NDST1-231 | chr5:150497690-150497717 | NDST1-1577 | chr5:150545483-150545510 |
| NDST1-232 | chr5:150497695-150497722 | NDST1-1578 | chr5:150545492-150545519 |
| NDST1-233 | chr5:150497696-150497723 | NDST1-1579 | chr5:150545493-150545520 |
| NDST1-234 | chr5:150497698-150497725 | NDST1-1580 | chr5:150545501-150545528 |
| NDST1-235 | chr5:150497699-150497726 | NDST1-1581 | chr5:150545502-150545529 |
| NDST1-236 | chr5:150497707-150497734 | NDST1-1582 | chr5:150545509-150545536 |
| NDST1-237 | chr5:150497714-150497741 | NDST1-1583 | chr5:150545510-150545537 |
| NDST1-238 | chr5:150497715-150497742 | NDST1-1584 | chr5:150545532-150545559 |
| NDST1-239 | chr5:150497721-150497748 | NDST1-1585 | chr5:150545581-150545608 |
| NDST1-240 | chr5:150497730-150497757 | NDST1-1586 | chr5:150545582-150545609 |
| NDST1-241 | chr5:150497736-150497763 | NDST1-1587 | chr5:150548098-150548125 |
| NDST1-242 | chr5:150497741-150497768 | NDST1-1588 | chr5:150548100-150548127 |
| NDST1-243 | chr5:150497742-150497769 | NDST1-1589 | chr5:150548101-150548128 |
| NDST1-244 | chr5:150497764-150497791 | NDST1-1590 | chr5:150548107-150548134 |
| NDST1-245 | chr5:150497770-150497797 | NDST1-1591 | chr5:150548109-150548136 |
| NDST1-246 | chr5:150497774-150497801 | NDST1-1592 | chr5:150548116-150548143 |
| NDST1-247 | chr5:150497779-150497806 | NDST1-1593 | chr5:150548118-150548145 |
| NDST1-248 | chr5:150497780-150497807 | NDST1-1594 | chr5:150548119-150548146 |
| NDST1-249 | chr5:150497789-150497816 | NDST1-1595 | chr5:150548135-150548162 |
| NDST1-250 | chr5:150497793-150497820 | NDST1-1596 | chr5:150548136-150548163 |
| NDST1-251 | chr5:150497794-150497821 | NDST1-1597 | chr5:150548138-150548165 |
| NDST1-252 | chr5:150497798-150497825 | NDST1-1598 | chr5:150548154-150548181 |
| NDST1-253 | chr5:150497799-150497826 | NDST1-1599 | chr5:150548155-150548182 |
| NDST1-254 | chr5:150497800-150497827 | NDST1-1600 | chr5:150548162-150548189 |
| NDST1-255 | chr5:150497817-150497844 | NDST1-1601 | chr5:150548164-150548191 |
| NDST1-256 | chr5:150497818-150497845 | NDST1-1602 | chr5:150548170-150548197 |
| NDST1-257 | chr5:150497821-150497848 | NDST1-1603 | chr5:150548179-150548206 |
| NDST1-258 | chr5:150497826-150497853 | NDST1-1604 | chr5:150548182-150548209 |
| NDST1-259 | chr5:150497831-150497858 | NDST1-1605 | chr5:150548198-150548225 |
| NDST1-260 | chr5:150497836-150497863 | NDST1-1606 | chr5:150548199-150548226 |
| NDST1-261 | chr5:150497837-150497864 | NDST1-1607 | chr5:150548206-150548233 |
| NDST1-262 | chr5:150497839-150497866 | NDST1-1608 | chr5:150548211-150548238 |
| NDST1-263 | chr5:150497842-150497869 | NDST1-1609 | chr5:150548218-150548245 |
| NDST1-264 | chr5:150497848-150497875 | NDST1-1610 | chr5:150548220-150548247 |
| NDST1-265 | chr5:150497849-150497876 | NDST1-1611 | chr5:150548228-150548255 |
| NDST1-266 | chr5:150497850-150497877 | NDST1-1612 | chr5:150548229-150548256 |
| NDST1-267 | chr5:150497858-150497885 | NDST1-1613 | chr5:150548238-150548265 |
| NDST1-268 | chr5:150497859-150497886 | NDST1-1614 | chr5:150548239-150548266 |
| NDST1-269 | chr5:150497865-150497892 | NDST1-1615 | chr5:150548242-150548269 |
| NDST1-270 | chr5:150497870-150497897 | NDST1-1616 | chr5:150548246-150548273 |
| NDST1-271 | chr5:150497871-150497898 | NDST1-1617 | chr5:150548247-150548274 |
| NDST1-272 | chr5:150497879-150497906 | NDST1-1618 | chr5:150548255-150548282 |
| NDST1-273 | chr5:150497895-150497922 | NDST1-1619 | chr5:150548258-150548285 |
| NDST1-274 | chr5:150497896-150497923 | NDST1-1620 | chr5:150548262-150548289 |
| NDST1-275 | chr5:150497905-150497932 | NDST1-1621 | chr5:150548276-150548303 |
| NDST1-276 | chr5:150497908-150497935 | NDST1-1622 | chr5:150548279-150548306 |
| NDST1-277 | chr5:150497924-150497951 | NDST1-1623 | chr5:150548294-150548321 |
| NDST1-278 | chr5:150497933-150497960 | NDST1-1624 | chr5:150548302-150548329 |
| NDST1-279 | chr5:150497934-150497961 | NDST1-1625 | chr5:150548309-150548336 |
| NDST1-280 | chr5:150497935-150497962 | NDST1-1626 | chr5:150548310-150548337 |
| NDST1-281 | chr5:150497937-150497964 | NDST1-1627 | chr5:150548313-150548340 |
| NDST1-282 | chr5:150497942-150497969 | NDST1-1628 | chr5:150548319-150548346 |
| NDST1-283 | chr5:150497947-150497974 | NDST1-1629 | chr5:150548325-150548352 |
| NDST1-284 | chr5:150497948-150497975 | NDST1-1630 | chr5:150548331-150548358 |
| NDST1-285 | chr5:150497951-150497978 | NDST1-1631 | chr5:150548332-150548359 |
| NDST1-286 | chr5:150497953-150497980 | NDST1-1632 | chr5:150548337-150548364 |
| NDST1-287 | chr5:150497964-150497991 | NDST1-1633 | chr5:150548345-150548372 |
| NDST1-288 | chr5:150497969-150497996 | NDST1-1634 | chr5:150548348-150548375 |
| NDST1-289 | chr5:150497971-150497998 | NDST1-1635 | chr5:150548349-150548376 |
| NDST1-290 | chr5:150497972-150497999 | NDST1-1636 | chr5:150548362-150548389 |
| NDST1-291 | chr5:150497973-150498000 | NDST1-1637 | chr5:150548372-150548399 |
| NDST1-292 | chr5:150497974-150498001 | NDST1-1638 | chr5:150548381-150548408 |
| NDST1-293 | chr5:150498009-150498036 | NDST1-1639 | chr5:150548385-150548412 |
| NDST1-294 | chr5:150498030-150498057 | NDST1-1640 | chr5:150548389-150548416 |
| NDST1-295 | chr5:150498034-150498061 | NDST1-1641 | chr5:150548390-150548417 |
| NDST1-296 | chr5:150498035-150498062 | NDST1-1642 | chr5:150548400-150548427 |
| NDST1-297 | chr5:150498046-150498073 | NDST1-1643 | chr5:150548401-150548428 |
| NDST1-298 | chr5:150498057-150498084 | NDST1-1644 | chr5:150548405-150548432 |
| NDST1-299 | chr5:150498058-150498085 | NDST1-1645 | chr5:150548406-150548433 |
| NDST1-300 | chr5:150498059-150498086 | NDST1-1646 | chr5:150548407-150548434 |
| NDST1-301 | chr5:150498066-150498093 | NDST1-1647 | chr5:150548415-150548442 |
| NDST1-302 | chr5:150498067-150498094 | NDST1-1648 | chr5:150548422-150548449 |
| NDST1-303 | chr5:150498074-150498101 | NDST1-1649 | chr5:150548453-150548480 |
| NDST1-304 | chr5:150498075-150498102 | NDST1-1650 | chr5:150548454-150548481 |
| NDST1-305 | chr5:150506604-150506631 | NDST1-1651 | chr5:150548455-150548482 |
| NDST1-306 | chr5:150506605-150506632 | NDST1-1652 | chr5:150548457-150548484 |
| NDST1-307 | chr5:150506620-150506647 | NDST1-1653 | chr5:150548471-150548498 |
| NDST1-308 | chr5:150506639-150506666 | NDST1-1654 | chr5:150548474-150548501 |
| NDST1-309 | chr5:150506640-150506667 | NDST1-1655 | chr5:150548475-150548502 |
| NDST1-310 | chr5:150506652-150506679 | NDST1-1656 | chr5:150549551-150549578 |
| NDST1-311 | chr5:150506663-150506690 | NDST1-1657 | chr5:150549558-150549585 |
| NDST1-312 | chr5:150506668-150506695 | NDST1-1658 | chr5:150549570-150549597 |
| NDST1-313 | chr5:150506669-150506696 | NDST1-1659 | chr5:150549580-150549607 |
| NDST1-314 | chr5:150506676-150506703 | NDST1-1660 | chr5:150549581-150549608 |
| NDST1-315 | chr5:150506677-150506704 | NDST1-1661 | chr5:150549589-150549616 |
| NDST1-316 | chr5:150506678-150506705 | NDST1-1662 | chr5:150549598-150549625 |
| NDST1-317 | chr5:150506679-150506706 | NDST1-1663 | chr5:150549599-150549626 |
| NDST1-318 | chr5:150506682-150506709 | NDST1-1664 | chr5:150549604-150549631 |
| NDST1-319 | chr5:150506687-150506714 | NDST1-1665 | chr5:150549611-150549638 |
| NDST1-320 | chr5:150506688-150506715 | NDST1-1666 | chr5:150549623-150549650 |
| NDST1-321 | chr5:150506689-150506716 | NDST1-1667 | chr5:150549624-150549651 |
| NDST1-322 | chr5:150506692-150506719 | NDST1-1668 | chr5:150549633-150549660 |
| NDST1-323 | chr5:150506699-150506726 | NDST1-1669 | chr5:150549636-150549663 |
| NDST1-324 | chr5:150506700-150506727 | NDST1-1670 | chr5:150549653-150549680 |
| NDST1-325 | chr5:150506701-150506728 | NDST1-1671 | chr5:150549654-150549681 |
| NDST1-326 | chr5:150506702-150506729 | NDST1-1672 | chr5:150549657-150549684 |
| NDST1-327 | chr5:150506705-150506732 | NDST1-1673 | chr5:150549659-150549686 |
| NDST1-328 | chr5:150506706-150506733 | NDST1-1674 | chr5:150549660-150549687 |
| NDST1-329 | chr5:150506707-150506734 | NDST1-1675 | chr5:150549661-150549688 |
| NDST1-330 | chr5:150506708-150506735 | NDST1-1676 | chr5:150549663-150549690 |
| NDST1-331 | chr5:150506712-150506739 | NDST1-1677 | chr5:150549667-150549694 |
| NDST1-332 | chr5:150506721-150506748 | NDST1-1678 | chr5:150549668-150549695 |
| NDST1-333 | chr5:150506724-150506751 | NDST1-1679 | chr5:150549669-150549696 |
| NDST1-334 | chr5:150506732-150506759 | NDST1-1680 | chr5:150549674-150549701 |
| NDST1-335 | chr5:150506740-150506767 | NDST1-1681 | chr5:150549702-150549729 |
| NDST1-336 | chr5:150506743-150506770 | NDST1-1682 | chr5:150549708-150549735 |
| NDST1-337 | chr5:150506744-150506771 | NDST1-1683 | chr5:150549714-150549741 |
| NDST1-338 | chr5:150506752-150506779 | NDST1-1684 | chr5:150549718-150549745 |
| NDST1-339 | chr5:150506753-150506780 | NDST1-1685 | chr5:150549724-150549751 |
| NDST1-340 | chr5:150506754-150506781 | NDST1-1686 | chr5:150549725-150549752 |
| NDST1-341 | chr5:150506771-150506798 | NDST1-1687 | chr5:150549726-150549753 |
| NDST1-342 | chr5:150506784-150506811 | NDST1-1688 | chr5:150549746-150549773 |
| NDST1-343 | chr5:150506785-150506812 | NDST1-1689 | chr5:150549757-150549784 |
| NDST1-344 | chr5:150506790-150506817 | NDST1-1690 | chr5:150549759-150549786 |
| NDST1-345 | chr5:150506794-150506821 | NDST1-1691 | chr5:150549763-150549790 |
| NDST1-346 | chr5:150506795-150506822 | NDST1-1692 | chr5:150549773-150549800 |
| NDST1-347 | chr5:150506796-150506823 | NDST1-1693 | chr5:150549781-150549808 |
| NDST1-348 | chr5:150506804-150506831 | NDST1-1694 | chr5:150549788-150549815 |
| NDST1-349 | chr5:150506805-150506832 | NDST1-1695 | chr5:150549798-150549825 |
| NDST1-350 | chr5:150506812-150506839 | NDST1-1696 | chr5:150549804-150549831 |
| NDST1-351 | chr5:150506826-150506853 | NDST1-1697 | chr5:150549805-150549832 |
| NDST1-352 | chr5:150506833-150506860 | NDST1-1698 | chr5:150549812-150549839 |
| NDST1-353 | chr5:150506834-150506861 | NDST1-1699 | chr5:150549813-150549840 |
| NDST1-354 | chr5:150506836-150506863 | NDST1-1700 | chr5:150549822-150549849 |
| NDST1-355 | chr5:150506845-150506872 | NDST1-1701 | chr5:150549823-150549850 |
| NDST1-356 | chr5:150506848-150506875 | NDST1-1702 | chr5:150549835-150549862 |
| NDST1-357 | chr5:150506857-150506884 | NDST1-1703 | chr5:150549841-150549868 |
| NDST1-358 | chr5:150506864-150506891 | NDST1-1704 | chr5:150549850-150549877 |
| NDST1-359 | chr5:150506865-150506892 | NDST1-1705 | chr5:150549877-150549904 |
| NDST1-360 | chr5:150506873-150506900 | NDST1-1706 | chr5:150551628-150551655 |
| NDST1-361 | chr5:150506874-150506901 | NDST1-1707 | chr5:150551632-150551659 |
| NDST1-362 | chr5:150506875-150506902 | NDST1-1708 | chr5:150551648-150551675 |
| NDST1-363 | chr5:150506876-150506903 | NDST1-1709 | chr5:150551649-150551676 |
| NDST1-364 | chr5:150506877-150506904 | NDST1-1710 | chr5:150551650-150551677 |
| NDST1-365 | chr5:150506880-150506907 | NDST1-1711 | chr5:150551654-150551681 |
| NDST1-366 | chr5:150506885-150506912 | NDST1-1712 | chr5:150551655-150551682 |
| NDST1-367 | chr5:150506895-150506922 | NDST1-1713 | chr5:150551663-150551690 |
| NDST1-368 | chr5:150506915-150506942 | NDST1-1714 | chr5:150551666-150551693 |
| NDST1-369 | chr5:150506935-150506962 | NDST1-1715 | chr5:150551671-150551698 |
| NDST1-370 | chr5:150506936-150506963 | NDST1-1716 | chr5:150551675-150551702 |
| NDST1-371 | chr5:150506939-150506966 | NDST1-1717 | chr5:150551679-150551706 |
| NDST1-372 | chr5:150506940-150506967 | NDST1-1718 | chr5:150551681-150551708 |
| NDST1-373 | chr5:150506952-150506979 | NDST1-1719 | chr5:150551682-150551709 |
| NDST1-374 | chr5:150506953-150506980 | NDST1-1720 | chr5:150551685-150551712 |
| NDST1-375 | chr5:150506955-150506982 | NDST1-1721 | chr5:150551718-150551745 |
| NDST1-376 | chr5:150506958-150506985 | NDST1-1722 | chr5:150551724-150551751 |
| NDST1-377 | chr5:150506959-150506986 | NDST1-1723 | chr5:150551725-150551752 |
| NDST1-378 | chr5:150506961-150506988 | NDST1-1724 | chr5:150551726-150551753 |
| NDST1-379 | chr5:150506962-150506989 | NDST1-1725 | chr5:150551729-150551756 |
| NDST1-380 | chr5:150506963-150506990 | NDST1-1726 | chr5:150551740-150551767 |
| NDST1-381 | chr5:150506968-150506995 | NDST1-1727 | chr5:150551741-150551768 |
| NDST1-382 | chr5:150506969-150506996 | NDST1-1728 | chr5:150551743-150551770 |
| NDST1-383 | chr5:150506970-150506997 | NDST1-1729 | chr5:150551744-150551771 |
| NDST1-384 | chr5:150506971-150506998 | NDST1-1730 | chr5:150551747-150551774 |
| NDST1-385 | chr5:150506974-150507001 | NDST1-1731 | chr5:150551750-150551777 |
| NDST1-386 | chr5:150506983-150507010 | NDST1-1732 | chr5:150551760-150551787 |
| NDST1-387 | chr5:150506990-150507017 | NDST1-1733 | chr5:150551767-150551794 |
| NDST1-388 | chr5:150506996-150507023 | NDST1-1734 | chr5:150551770-150551797 |
| NDST1-389 | chr5:150507011-150507038 | NDST1-1735 | chr5:150551780-150551807 |
| NDST1-390 | chr5:150507012-150507039 | NDST1-1736 | chr5:150551787-150551814 |
| NDST1-391 | chr5:150507022-150507049 | NDST1-1737 | chr5:150551788-150551815 |
| NDST1-392 | chr5:150507030-150507057 | NDST1-1738 | chr5:150551799-150551826 |
| NDST1-393 | chr5:150507055-150507082 | NDST1-1739 | chr5:150551800-150551827 |
| NDST1-394 | chr5:150507061-150507088 | NDST1-1740 | chr5:150551803-150551830 |
| NDST1-395 | chr5:150507062-150507089 | NDST1-1741 | chr5:150551804-150551831 |
| NDST1-396 | chr5:150507064-150507091 | NDST1-1742 | chr5:150551820-150551847 |
| NDST1-397 | chr5:150507074-150507101 | NDST1-1743 | chr5:150551826-150551853 |
| NDST1-398 | chr5:150507075-150507102 | NDST1-1744 | chr5:150551828-150551855 |
| NDST1-399 | chr5:150507079-150507106 | NDST1-1745 | chr5:150551836-150551863 |
| NDST1-400 | chr5:150507082-150507109 | NDST1-1746 | chr5:150551838-150551865 |
| NDST1-401 | chr5:150507090-150507117 | NDST1-1747 | chr5:150551839-150551866 |
| NDST1-402 | chr5:150507094-150507121 | NDST1-1748 | chr5:150551867-150551894 |
| NDST1-403 | chr5:150507097-150507124 | NDST1-1749 | chr5:150551868-150551895 |
| NDST1-404 | chr5:150507100-150507127 | NDST1-1750 | chr5:150551873-150551900 |
| NDST1-405 | chr5:150507103-150507130 | NDST1-1751 | chr5:150551874-150551901 |
| NDST1-406 | chr5:150507120-150507147 | NDST1-1752 | chr5:150551875-150551902 |
| NDST1-407 | chr5:150507124-150507151 | NDST1-1753 | chr5:150551884-150551911 |
| NDST1-408 | chr5:150507125-150507152 | NDST1-1754 | chr5:150551886-150551913 |
| NDST1-409 | chr5:150507126-150507153 | NDST1-1755 | chr5:150551894-150551921 |
| NDST1-410 | chr5:150507128-150507155 | NDST1-1756 | chr5:150551895-150551922 |
| NDST1-411 | chr5:150507134-150507161 | NDST1-1757 | chr5:150551896-150551923 |
| NDST1-412 | chr5:150507142-150507169 | NDST1-1758 | chr5:150551897-150551924 |
| NDST1-413 | chr5:150507143-150507170 | NDST1-1759 | chr5:150551904-150551931 |
| NDST1-414 | chr5:150507146-150507173 | NDST1-1760 | chr5:150551905-150551932 |
| NDST1-415 | chr5:150507150-150507177 | NDST1-1761 | chr5:150551934-150551961 |
| NDST1-416 | chr5:150507154-150507181 | NDST1-1762 | chr5:150551935-150551962 |
| NDST1-417 | chr5:150507156-150507183 | NDST1-1763 | chr5:150551940-150551967 |
| NDST1-418 | chr5:150507162-150507189 | NDST1-1764 | chr5:150551951-150551978 |
| NDST1-419 | chr5:150507166-150507193 | NDST1-1765 | chr5:150553089-150553116 |
| NDST1-420 | chr5:150507167-150507194 | NDST1-1766 | chr5:150553092-150553119 |
| NDST1-421 | chr5:150507174-150507201 | NDST1-1767 | chr5:150553095-150553122 |
| NDST1-422 | chr5:150507183-150507210 | NDST1-1768 | chr5:150553098-150553125 |
| NDST1-423 | chr5:150507184-150507211 | NDST1-1769 | chr5:150553099-150553126 |
| NDST1-424 | chr5:150507186-150507213 | NDST1-1770 | chr5:150553111-150553138 |
| NDST1-425 | chr5:150507190-150507217 | NDST1-1771 | chr5:150553121-150553148 |
| NDST1-426 | chr5:150507193-150507220 | NDST1-1772 | chr5:150553126-150553153 |
| NDST1-427 | chr5:150507194-150507221 | NDST1-1773 | chr5:150553129-150553156 |
| NDST1-428 | chr5:150507216-150507243 | NDST1-1774 | chr5:150553134-150553161 |
| NDST1-429 | chr5:150507225-150507252 | NDST1-1775 | chr5:150553135-150553162 |
| NDST1-430 | chr5:150507229-150507256 | NDST1-1776 | chr5:150553137-150553164 |
| NDST1-431 | chr5:150507230-150507257 | NDST1-1777 | chr5:150553139-150553166 |
| NDST1-432 | chr5:150507234-150507261 | NDST1-1778 | chr5:150553161-150553188 |
| NDST1-433 | chr5:150507237-150507264 | NDST1-1779 | chr5:150553199-150553226 |
| NDST1-434 | chr5:150507241-150507268 | NDST1-1780 | chr5:150553203-150553230 |
| NDST1-435 | chr5:150507248-150507275 | NDST1-1781 | chr5:150553204-150553231 |
| NDST1-436 | chr5:150507249-150507276 | NDST1-1782 | chr5:150553214-150553241 |
| NDST1-437 | chr5:150507250-150507277 | NDST1-1783 | chr5:150553215-150553242 |
| NDST1-438 | chr5:150507265-150507292 | NDST1-1784 | chr5:150553216-150553243 |
| NDST1-439 | chr5:150507293-150507320 | NDST1-1785 | chr5:150553220-150553247 |
| NDST1-440 | chr5:150507298-150507325 | NDST1-1786 | chr5:150553224-150553251 |
| NDST1-441 | chr5:150507313-150507340 | NDST1-1787 | chr5:150553239-150553266 |
| NDST1-442 | chr5:150507324-150507351 | NDST1-1788 | chr5:150553245-150553272 |
| NDST1-443 | chr5:150507328-150507355 | NDST1-1789 | chr5:150553255-150553282 |
| NDST1-444 | chr5:150507335-150507362 | NDST1-1790 | chr5:150553256-150553283 |
| NDST1-445 | chr5:150507344-150507371 | NDST1-1791 | chr5:150553262-150553289 |
| NDST1-446 | chr5:150507347-150507374 | NDST1-1792 | chr5:150553275-150553302 |
| NDST1-447 | chr5:150507348-150507375 | NDST1-1793 | chr5:150553284-150553311 |
| NDST1-448 | chr5:150507362-150507389 | NDST1-1794 | chr5:150553285-150553312 |
| NDST1-449 | chr5:150507385-150507412 | NDST1-1795 | chr5:150553294-150553321 |
| NDST1-450 | chr5:150507394-150507421 | NDST1-1796 | chr5:150553295-150553322 |
| NDST1-451 | chr5:150507407-150507434 | NDST1-1797 | chr5:150553302-150553329 |
| NDST1-452 | chr5:150507408-150507435 | NDST1-1798 | chr5:150553311-150553338 |
| NDST1-453 | chr5:150507413-150507440 | NDST1-1799 | chr5:150553314-150553341 |
| NDST1-454 | chr5:150507417-150507444 | NDST1-1800 | chr5:150553317-150553344 |
| NDST1-455 | chr5:150507422-150507449 | NDST1-1801 | chr5:150553325-150553352 |
| NDST1-456 | chr5:150507423-150507450 | NDST1-1802 | chr5:150553333-150553360 |
| NDST1-457 | chr5:150507429-150507456 | NDST1-1803 | chr5:150553339-150553366 |
| NDST1-458 | chr5:150507445-150507472 | NDST1-1804 | chr5:150553342-150553369 |
| NDST1-459 | chr5:150507446-150507473 | NDST1-1805 | chr5:150553346-150553373 |
| NDST1-460 | chr5:150507451-150507478 | NDST1-1806 | chr5:150553347-150553374 |
| NDST1-461 | chr5:150507488-150507515 | NDST1-1807 | chr5:150553348-150553375 |
| NDST1-462 | chr5:150507490-150507517 | NDST1-1808 | chr5:150553379-150553406 |
| NDST1-463 | chr5:150507491-150507518 | NDST1-1809 | chr5:150553380-150553407 |
| NDST1-464 | chr5:150507499-150507526 | NDST1-1810 | chr5:150553383-150553410 |
| NDST1-465 | chr5:150507518-150507545 | NDST1-1811 | chr5:150553384-150553411 |
| NDST1-466 | chr5:150507527-150507554 | NDST1-1812 | chr5:150553385-150553412 |
| NDST1-467 | chr5:150507536-150507563 | NDST1-1813 | chr5:150553392-150553419 |
| NDST1-468 | chr5:150507537-150507564 | NDST1-1814 | chr5:150553395-150553422 |
| NDST1-469 | chr5:150507540-150507567 | NDST1-1815 | chr5:150553398-150553425 |
| NDST1-470 | chr5:150507545-150507572 | NDST1-1816 | chr5:150553399-150553426 |
| NDST1-471 | chr5:150507551-150507578 | NDST1-1817 | chr5:150553400-150553427 |
| NDST1-472 | chr5:150507552-150507579 | NDST1-1818 | chr5:150553421-150553448 |
| NDST1-473 | chr5:150507555-150507582 | NDST1-1819 | chr5:150553430-150553457 |
| NDST1-474 | chr5:150507556-150507583 | NDST1-1820 | chr5:150553433-150553460 |
| NDST1-475 | chr5:150507557-150507584 | NDST1-1821 | chr5:150553438-150553465 |
| NDST1-476 | chr5:150507558-150507585 | NDST1-1822 | chr5:150553441-150553468 |
| NDST1-477 | chr5:150507563-150507590 | NDST1-1823 | chr5:150553442-150553469 |
| NDST1-478 | chr5:150507564-150507591 | NDST1-1824 | chr5:150553443-150553470 |
| NDST1-479 | chr5:150507566-150507593 | NDST1-1825 | chr5:150553447-150553474 |
| NDST1-480 | chr5:150507568-150507595 | NDST1-1826 | chr5:150553448-150553475 |
| NDST1-481 | chr5:150507569-150507596 | NDST1-1827 | chr5:150553449-150553476 |
| NDST1-482 | chr5:150507574-150507601 | NDST1-1828 | chr5:150553450-150553477 |
| NDST1-483 | chr5:150507586-150507613 | NDST1-1829 | chr5:150553460-150553487 |
| NDST1-484 | chr5:150507587-150507614 | NDST1-1830 | chr5:150553463-150553490 |
| NDST1-485 | chr5:150507590-150507617 | NDST1-1831 | chr5:150553480-150553507 |
| NDST1-486 | chr5:150507591-150507618 | NDST1-1832 | chr5:150553482-150553509 |
| NDST1-487 | chr5:150507592-150507619 | NDST1-1833 | chr5:150553483-150553510 |
| NDST1-488 | chr5:150507597-150507624 | NDST1-1834 | chr5:150553495-150553522 |
| NDST1-489 | chr5:150507598-150507625 | NDST1-1835 | chr5:150553496-150553523 |
| NDST1-490 | chr5:150507599-150507626 | NDST1-1836 | chr5:150553502-150553529 |
| NDST1-491 | chr5:150507620-150507647 | NDST1-1837 | chr5:150553503-150553530 |
| NDST1-492 | chr5:150507627-150507654 | NDST1-1838 | chr5:150553510-150553537 |
| NDST1-493 | chr5:150507634-150507661 | NDST1-1839 | chr5:150553511-150553538 |
| NDST1-494 | chr5:150507638-150507665 | NDST1-1840 | chr5:150553512-150553539 |
| NDST1-495 | chr5:150507639-150507666 | NDST1-1841 | chr5:150553513-150553540 |
| NDST1-496 | chr5:150507654-150507681 | NDST1-1842 | chr5:150553516-150553543 |
| NDST1-497 | chr5:150507655-150507682 | NDST1-1843 | chr5:150553531-150553558 |
| NDST1-498 | chr5:150507658-150507685 | NDST1-1844 | chr5:150553540-150553567 |
| NDST1-499 | chr5:150507667-150507694 | NDST1-1845 | chr5:150553543-150553570 |
| NDST1-500 | chr5:150507671-150507698 | NDST1-1846 | chr5:150553544-150553571 |
| NDST1-501 | chr5:150507672-150507699 | NDST1-1847 | chr5:150553549-150553576 |
| NDST1-502 | chr5:150507675-150507702 | NDST1-1848 | chr5:150553557-150553584 |
| NDST1-503 | chr5:150507676-150507703 | NDST1-1849 | chr5:150553558-150553585 |
| NDST1-504 | chr5:150507677-150507704 | NDST1-1850 | chr5:150553559-150553586 |
| NDST1-505 | chr5:150507680-150507707 | NDST1-1851 | chr5:150553560-150553587 |
| NDST1-506 | chr5:150507686-150507713 | NDST1-1852 | chr5:150553562-150553589 |
| NDST1-507 | chr5:150507691-150507718 | NDST1-1853 | chr5:150553565-150553592 |
| NDST1-508 | chr5:150507694-150507721 | NDST1-1854 | chr5:150553570-150553597 |
| NDST1-509 | chr5:150507695-150507722 | NDST1-1855 | chr5:150553571-150553598 |
| NDST1-510 | chr5:150507696-150507723 | NDST1-1856 | chr5:150553573-150553600 |
| NDST1-511 | chr5:150507697-150507724 | NDST1-1857 | chr5:150553577-150553604 |
| NDST1-512 | chr5:150507701-150507728 | NDST1-1858 | chr5:150553580-150553607 |
| NDST1-513 | chr5:150507702-150507729 | NDST1-1859 | chr5:150553581-150553608 |
| NDST1-514 | chr5:150507707-150507734 | NDST1-1860 | chr5:150553590-150553617 |
| NDST1-515 | chr5:150507708-150507735 | NDST1-1861 | chr5:150553596-150553623 |
| NDST1-516 | chr5:150507712-150507739 | NDST1-1862 | chr5:150553612-150553639 |
| NDST1-517 | chr5:150507719-150507746 | NDST1-1863 | chr5:150553631-150553658 |
| NDST1-518 | chr5:150507730-150507757 | NDST1-1864 | chr5:150553632-150553659 |
| NDST1-519 | chr5:150507742-150507769 | NDST1-1865 | chr5:150553644-150553671 |
| NDST1-520 | chr5:150507748-150507775 | NDST1-1866 | chr5:150553647-150553674 |
| NDST1-521 | chr5:150507749-150507776 | NDST1-1867 | chr5:150553655-150553682 |
| NDST1-522 | chr5:150507757-150507784 | NDST1-1868 | chr5:150553656-150553683 |
| NDST1-523 | chr5:150507763-150507790 | NDST1-1869 | chr5:150553657-150553684 |
| NDST1-524 | chr5:150507765-150507792 | NDST1-1870 | chr5:150553663-150553690 |
| NDST1-525 | chr5:150507777-150507804 | NDST1-1871 | chr5:150553664-150553691 |
| NDST1-526 | chr5:150507780-150507807 | NDST1-1872 | chr5:150553667-150553694 |
| NDST1-527 | chr5:150507783-150507810 | NDST1-1873 | chr5:150553668-150553695 |
| NDST1-528 | chr5:150507784-150507811 | NDST1-1874 | chr5:150553676-150553703 |
| NDST1-529 | chr5:150507797-150507824 | NDST1-1875 | chr5:150553677-150553704 |
| NDST1-530 | chr5:150507807-150507834 | NDST1-1876 | chr5:150553684-150553711 |
| NDST1-531 | chr5:150507808-150507835 | NDST1-1877 | chr5:150553694-150553721 |
| NDST1-532 | chr5:150507813-150507840 | NDST1-1878 | chr5:150553695-150553722 |
| NDST1-533 | chr5:150507814-150507841 | NDST1-1879 | chr5:150553696-150553723 |
| NDST1-534 | chr5:150507820-150507847 | NDST1-1880 | chr5:150553705-150553732 |
| NDST1-535 | chr5:150507821-150507848 | NDST1-1881 | chr5:150553711-150553738 |
| NDST1-536 | chr5:150507837-150507864 | NDST1-1882 | chr5:150553712-150553739 |
| NDST1-537 | chr5:150507841-150507868 | NDST1-1883 | chr5:150553713-150553740 |
| NDST1-538 | chr5:150507851-150507878 | NDST1-1884 | chr5:150553714-150553741 |
| NDST1-539 | chr5:150507853-150507880 | NDST1-1885 | chr5:150553717-150553744 |
| NDST1-540 | chr5:150507855-150507882 | NDST1-1886 | chr5:150553719-150553746 |
| NDST1-541 | chr5:150507856-150507883 | NDST1-1887 | chr5:150553720-150553747 |
| NDST1-542 | chr5:150507858-150507885 | NDST1-1888 | chr5:150553725-150553752 |
| NDST1-543 | chr5:150507859-150507886 | NDST1-1889 | chr5:150553726-150553753 |
| NDST1-544 | chr5:150507861-150507888 | NDST1-1890 | chr5:150553728-150553755 |
| NDST1-545 | chr5:150507862-150507889 | NDST1-1891 | chr5:150553730-150553757 |
| NDST1-546 | chr5:150507867-150507894 | NDST1-1892 | chr5:150553731-150553758 |
| NDST1-547 | chr5:150507871-150507898 | NDST1-1893 | chr5:150553732-150553759 |
| NDST1-548 | chr5:150507874-150507901 | NDST1-1894 | chr5:150553733-150553760 |
| NDST1-549 | chr5:150507876-150507903 | NDST1-1895 | chr5:150553744-150553771 |
| NDST1-550 | chr5:150507885-150507912 | NDST1-1896 | chr5:150553749-150553776 |
| NDST1-551 | chr5:150507893-150507920 | NDST1-1897 | chr5:150553754-150553781 |
| NDST1-552 | chr5:150507894-150507921 | NDST1-1898 | chr5:150553767-150553794 |
| NDST1-553 | chr5:150507899-150507926 | NDST1-1899 | chr5:150553778-150553805 |
| NDST1-554 | chr5:150507900-150507927 | NDST1-1900 | chr5:150553779-150553806 |
| NDST1-555 | chr5:150507901-150507928 | NDST1-1901 | chr5:150553780-150553807 |
| NDST1-556 | chr5:150507906-150507933 | NDST1-1902 | chr5:150553788-150553815 |
| NDST1-557 | chr5:150507910-150507937 | NDST1-1903 | chr5:150553789-150553816 |
| NDST1-558 | chr5:150507913-150507940 | NDST1-1904 | chr5:150553790-150553817 |
| NDST1-559 | chr5:150507915-150507942 | NDST1-1905 | chr5:150553796-150553823 |
| NDST1-560 | chr5:150507916-150507943 | NDST1-1906 | chr5:150553800-150553827 |
| NDST1-561 | chr5:150507933-150507960 | NDST1-1907 | chr5:150553808-150553835 |
| NDST1-562 | chr5:150507939-150507966 | NDST1-1908 | chr5:150553809-150553836 |
| NDST1-563 | chr5:150507942-150507969 | NDST1-1909 | chr5:150553813-150553840 |
| NDST1-564 | chr5:150507946-150507973 | NDST1-1910 | chr5:150553817-150553844 |
| NDST1-565 | chr5:150507947-150507974 | NDST1-1911 | chr5:150553818-150553845 |
| NDST1-566 | chr5:150507948-150507975 | NDST1-1912 | chr5:150553819-150553846 |
| NDST1-567 | chr5:150507952-150507979 | NDST1-1913 | chr5:150553820-150553847 |
| NDST1-568 | chr5:150507955-150507982 | NDST1-1914 | chr5:150553821-150553848 |
| NDST1-569 | chr5:150507966-150507993 | NDST1-1915 | chr5:150553822-150553849 |
| NDST1-570 | chr5:150507967-150507994 | NDST1-1916 | chr5:150553825-150553852 |
| NDST1-571 | chr5:150507970-150507997 | NDST1-1917 | chr5:150553831-150553858 |
| NDST1-572 | chr5:150507973-150508000 | NDST1-1918 | chr5:150553838-150553865 |
| NDST1-573 | chr5:150507981-150508008 | NDST1-1919 | chr5:150553853-150553880 |
| NDST1-574 | chr5:150507982-150508009 | NDST1-1920 | chr5:150553854-150553881 |
| NDST1-575 | chr5:150507983-150508010 | NDST1-1921 | chr5:150553855-150553882 |
| NDST1-576 | chr5:150507988-150508015 | NDST1-1922 | chr5:150553856-150553883 |
| NDST1-577 | chr5:150507991-150508018 | NDST1-1923 | chr5:150553859-150553886 |
| NDST1-578 | chr5:150507999-150508026 | NDST1-1924 | chr5:150553869-150553896 |
| NDST1-579 | chr5:150508006-150508033 | NDST1-1925 | chr5:150553870-150553897 |
| NDST1-580 | chr5:150508010-150508037 | NDST1-1926 | chr5:150553874-150553901 |
| NDST1-581 | chr5:150508013-150508040 | NDST1-1927 | chr5:150553877-150553904 |
| NDST1-582 | chr5:150508019-150508046 | NDST1-1928 | chr5:150553889-150553916 |
| NDST1-583 | chr5:150508020-150508047 | NDST1-1929 | chr5:150553892-150553919 |
| NDST1-584 | chr5:150508029-150508056 | NDST1-1930 | chr5:150553893-150553920 |
| NDST1-585 | chr5:150508030-150508057 | NDST1-1931 | chr5:150553894-150553921 |
| NDST1-586 | chr5:150508058-150508085 | NDST1-1932 | chr5:150553906-150553933 |
| NDST1-587 | chr5:150508061-150508088 | NDST1-1933 | chr5:150553907-150553934 |
| NDST1-588 | chr5:150508064-150508091 | NDST1-1934 | chr5:150553910-150553937 |
| NDST1-589 | chr5:150508065-150508092 | NDST1-1935 | chr5:150553911-150553938 |
| NDST1-590 | chr5:150508068-150508095 | NDST1-1936 | chr5:150553935-150553962 |
| NDST1-591 | chr5:150508069-150508096 | NDST1-1937 | chr5:150553936-150553963 |
| NDST1-592 | chr5:150508070-150508097 | NDST1-1938 | chr5:150553946-150553973 |
| NDST1-593 | chr5:150508071-150508098 | NDST1-1939 | chr5:150553947-150553974 |
| NDST1-594 | chr5:150508074-150508101 | NDST1-1940 | chr5:150553951-150553978 |
| NDST1-595 | chr5:150508075-150508102 | NDST1-1941 | chr5:150553953-150553980 |
| NDST1-596 | chr5:150508076-150508103 | NDST1-1942 | chr5:150553954-150553981 |
| NDST1-597 | chr5:150508080-150508107 | NDST1-1943 | chr5:150553955-150553982 |
| NDST1-598 | chr5:150508081-150508108 | NDST1-1944 | chr5:150553966-150553993 |
| NDST1-599 | chr5:150508082-150508109 | NDST1-1945 | chr5:150553967-150553994 |
| NDST1-600 | chr5:150508084-150508111 | NDST1-1946 | chr5:150553972-150553999 |
| NDST1-601 | chr5:150508085-150508112 | NDST1-1947 | chr5:150553973-150554000 |
| NDST1-602 | chr5:150508092-150508119 | NDST1-1948 | chr5:150553975-150554002 |
| NDST1-603 | chr5:150508107-150508134 | NDST1-1949 | chr5:150553986-150554013 |
| NDST1-604 | chr5:150508110-150508137 | NDST1-1950 | chr5:150553987-150554014 |
| NDST1-605 | chr5:150508113-150508140 | NDST1-1951 | chr5:150553988-150554015 |
| NDST1-606 | chr5:150508114-150508141 | NDST1-1952 | chr5:150553993-150554020 |
| NDST1-607 | chr5:150508120-150508147 | NDST1-1953 | chr5:150554001-150554028 |
| NDST1-608 | chr5:150508122-150508149 | NDST1-1954 | chr5:150554002-150554029 |
| NDST1-609 | chr5:150508130-150508157 | NDST1-1955 | chr5:150554010-150554037 |
| NDST1-610 | chr5:150508135-150508162 | NDST1-1956 | chr5:150554015-150554042 |
| NDST1-611 | chr5:150508136-150508163 | NDST1-1957 | chr5:150554016-150554043 |
| NDST1-612 | chr5:150508141-150508168 | NDST1-1958 | chr5:150554017-150554044 |
| NDST1-613 | chr5:150508153-150508180 | NDST1-1959 | chr5:150554020-150554047 |
| NDST1-614 | chr5:150508154-150508181 | NDST1-1960 | chr5:150554026-150554053 |
| NDST1-615 | chr5:150508159-150508186 | NDST1-1961 | chr5:150554027-150554054 |
| NDST1-616 | chr5:150508172-150508199 | NDST1-1962 | chr5:150554028-150554055 |
| NDST1-617 | chr5:150508192-150508219 | NDST1-1963 | chr5:150554030-150554057 |
| NDST1-618 | chr5:150508200-150508227 | NDST1-1964 | chr5:150554036-150554063 |
| NDST1-619 | chr5:150508202-150508229 | NDST1-1965 | chr5:150554037-150554064 |
| NDST1-620 | chr5:150508203-150508230 | NDST1-1966 | chr5:150554038-150554065 |
| NDST1-621 | chr5:150508204-150508231 | NDST1-1967 | chr5:150554039-150554066 |
| NDST1-622 | chr5:150508209-150508236 | NDST1-1968 | chr5:150554042-150554069 |
| NDST1-623 | chr5:150508210-150508237 | NDST1-1969 | chr5:150554055-150554082 |
| NDST1-624 | chr5:150508215-150508242 | NDST1-1970 | chr5:150554059-150554086 |
| NDST1-625 | chr5:150508224-150508251 | NDST1-1971 | chr5:150554063-150554090 |
| NDST1-626 | chr5:150508230-150508257 | NDST1-1972 | chr5:150554064-150554091 |
| NDST1-627 | chr5:150508259-150508286 | NDST1-1973 | chr5:150554065-150554092 |
| NDST1-628 | chr5:150508260-150508287 | NDST1-1974 | chr5:150554066-150554093 |
| NDST1-629 | chr5:150508265-150508292 | NDST1-1975 | chr5:150554087-150554114 |
| NDST1-630 | chr5:150508274-150508301 | NDST1-1976 | chr5:150554098-150554125 |
| NDST1-631 | chr5:150508281-150508308 | NDST1-1977 | chr5:150554100-150554127 |
| NDST1-632 | chr5:150508282-150508309 | NDST1-1978 | chr5:150554111-150554138 |
| NDST1-633 | chr5:150508286-150508313 | NDST1-1979 | chr5:150554112-150554139 |
| NDST1-634 | chr5:150508287-150508314 | NDST1-1980 | chr5:150554124-150554151 |
| NDST1-635 | chr5:150508288-150508315 | NDST1-1981 | chr5:150554129-150554156 |
| NDST1-636 | chr5:150508289-150508316 | NDST1-1982 | chr5:150554130-150554157 |
| NDST1-637 | chr5:150508290-150508317 | NDST1-1983 | chr5:150554131-150554158 |
| NDST1-638 | chr5:150508294-150508321 | NDST1-1984 | chr5:150554136-150554163 |
| NDST1-639 | chr5:150508295-150508322 | NDST1-1985 | chr5:150554137-150554164 |
| NDST1-640 | chr5:150508300-150508327 | NDST1-1986 | chr5:150554139-150554166 |
| NDST1-641 | chr5:150508305-150508332 | NDST1-1987 | chr5:150554140-150554167 |
| NDST1-642 | chr5:150508309-150508336 | NDST1-1988 | chr5:150554141-150554168 |
| NDST1-643 | chr5:150508315-150508342 | NDST1-1989 | chr5:150554145-150554172 |
| NDST1-644 | chr5:150520750-150520777 | NDST1-1990 | chr5:150554148-150554175 |
| NDST1-645 | chr5:150520752-150520779 | NDST1-1991 | chr5:150554152-150554179 |
| NDST1-646 | chr5:150520753-150520780 | NDST1-1992 | chr5:150554153-150554180 |
| NDST1-647 | chr5:150520763-150520790 | NDST1-1993 | chr5:150554166-150554193 |
| NDST1-648 | chr5:150520764-150520791 | NDST1-1994 | chr5:150554184-150554211 |
| NDST1-649 | chr5:150520785-150520812 | NDST1-1995 | chr5:150554186-150554213 |
| NDST1-650 | chr5:150520797-150520824 | NDST1-1996 | chr5:150554188-150554215 |
| NDST1-651 | chr5:150520806-150520833 | NDST1-1997 | chr5:150554192-150554219 |
| NDST1-652 | chr5:150520821-150520848 | NDST1-1998 | chr5:150554193-150554220 |
| NDST1-653 | chr5:150520822-150520849 | NDST1-1999 | chr5:150554204-150554231 |
| NDST1-654 | chr5:150520829-150520856 | NDST1-2000 | chr5:150554207-150554234 |
| NDST1-655 | chr5:150520830-150520857 | NDST1-2001 | chr5:150554214-150554241 |
| NDST1-656 | chr5:150520845-150520872 | NDST1-2002 | chr5:150554231-150554258 |
| NDST1-657 | chr5:150520854-150520881 | NDST1-2003 | chr5:150554232-150554259 |
| NDST1-658 | chr5:150520855-150520882 | NDST1-2004 | chr5:150554233-150554260 |
| NDST1-659 | chr5:150520862-150520889 | NDST1-2005 | chr5:150554234-150554261 |
| NDST1-660 | chr5:150520863-150520890 | NDST1-2006 | chr5:150554240-150554267 |
| NDST1-661 | chr5:150520868-150520895 | NDST1-2007 | chr5:150554243-150554270 |
| NDST1-662 | chr5:150520872-150520899 | NDST1-2008 | chr5:150554244-150554271 |
| NDST1-663 | chr5:150520874-150520901 | NDST1-2009 | chr5:150554268-150554295 |
| NDST1-664 | chr5:150520875-150520902 | NDST1-2010 | chr5:150554288-150554315 |
| NDST1-665 | chr5:150520876-150520903 | NDST1-2011 | chr5:150554289-150554316 |
| NDST1-666 | chr5:150520883-150520910 | NDST1-2012 | chr5:150554295-150554322 |
| NDST1-667 | chr5:150520884-150520911 | NDST1-2013 | chr5:150554296-150554323 |
| NDST1-668 | chr5:150520885-150520912 | NDST1-2014 | chr5:150554297-150554324 |
| NDST1-669 | chr5:150520888-150520915 | NDST1-2015 | chr5:150554298-150554325 |
| NDST1-670 | chr5:150520889-150520916 | NDST1-2016 | chr5:150554310-150554337 |
| NDST1-671 | chr5:150520908-150520935 | NDST1-2017 | chr5:150554311-150554338 |
| NDST1-672 | chr5:150520909-150520936 | NDST1-2018 | chr5:150554364-150554391 |
| NDST1-673 | chr5:150520910-150520937 | NDST1-2019 | chr5:150554368-150554395 |
| NDST1-674 | chr5:150520911-150520938 | NDST1-2020 | chr5:150554369-150554396 |
| NDST1-675 | chr5:150520912-150520939 | NDST1-2021 | chr5:150554398-150554425 |
| NDST1-676 | chr5:150520917-150520944 | NDST1-2022 | chr5:150554401-150554428 |
| NDST1-677 | chr5:150520922-150520949 | NDST1-2023 | chr5:150554414-150554441 |
| NDST1-678 | chr5:150520929-150520956 | NDST1-2024 | chr5:150554418-150554445 |
| NDST1-679 | chr5:150520933-150520960 | NDST1-2025 | chr5:150554419-150554446 |
| NDST1-680 | chr5:150520936-150520963 | NDST1-2026 | chr5:150554420-150554447 |
| NDST1-681 | chr5:150520943-150520970 | NDST1-2027 | chr5:150554433-150554460 |
| NDST1-682 | chr5:150520944-150520971 | NDST1-2028 | chr5:150554434-150554461 |
| NDST1-683 | chr5:150520947-150520974 | NDST1-2029 | chr5:150554435-150554462 |
| NDST1-684 | chr5:150520948-150520975 | NDST1-2030 | chr5:150554448-150554475 |
| NDST1-685 | chr5:150520949-150520976 | NDST1-2031 | chr5:150554451-150554478 |
| NDST1-686 | chr5:150520950-150520977 | NDST1-2032 | chr5:150554452-150554479 |
| NDST1-687 | chr5:150520951-150520978 | NDST1-2033 | chr5:150554455-150554482 |
| NDST1-688 | chr5:150520952-150520979 | NDST1-2034 | chr5:150554456-150554483 |
| NDST1-689 | chr5:150520953-150520980 | NDST1-2035 | chr5:150554459-150554486 |
| NDST1-690 | chr5:150520961-150520988 | NDST1-2036 | chr5:150554460-150554487 |
| NDST1-691 | chr5:150520962-150520989 | NDST1-2037 | chr5:150554463-150554490 |
| NDST1-692 | chr5:150520968-150520995 | NDST1-2038 | chr5:150554478-150554505 |
| NDST1-693 | chr5:150520969-150520996 | NDST1-2039 | chr5:150554483-150554510 |
| NDST1-694 | chr5:150520970-150520997 | NDST1-2040 | chr5:150554484-150554511 |
| NDST1-695 | chr5:150520977-150521004 | NDST1-2041 | chr5:150554485-150554512 |
| NDST1-696 | chr5:150520978-150521005 | NDST1-2042 | chr5:150554497-150554524 |
| NDST1-697 | chr5:150520979-150521006 | NDST1-2043 | chr5:150554501-150554528 |
| NDST1-698 | chr5:150520980-150521007 | NDST1-2044 | chr5:150554505-150554532 |
| NDST1-699 | chr5:150520990-150521017 | NDST1-2045 | chr5:150554506-150554533 |
| NDST1-700 | chr5:150520997-150521024 | NDST1-2046 | chr5:150554507-150554534 |
| NDST1-701 | chr5:150521001-150521028 | NDST1-2047 | chr5:150554522-150554549 |
| NDST1-702 | chr5:150521002-150521029 | NDST1-2048 | chr5:150554525-150554552 |
| NDST1-703 | chr5:150521003-150521030 | NDST1-2049 | chr5:150554534-150554561 |
| NDST1-704 | chr5:150521008-150521035 | NDST1-2050 | chr5:150554535-150554562 |
| NDST1-705 | chr5:150521011-150521038 | NDST1-2051 | chr5:150554536-150554563 |
| NDST1-706 | chr5:150521012-150521039 | NDST1-2052 | chr5:150554539-150554566 |
| NDST1-707 | chr5:150521015-150521042 | NDST1-2053 | chr5:150554540-150554567 |
| NDST1-708 | chr5:150521019-150521046 | NDST1-2054 | chr5:150554541-150554568 |
| NDST1-709 | chr5:150521037-150521064 | NDST1-2055 | chr5:150554546-150554573 |
| NDST1-710 | chr5:150521050-150521077 | NDST1-2056 | chr5:150554547-150554574 |
| NDST1-711 | chr5:150521054-150521081 | NDST1-2057 | chr5:150554556-150554583 |
| NDST1-712 | chr5:150521055-150521082 | NDST1-2058 | chr5:150554559-150554586 |
| NDST1-713 | chr5:150521058-150521085 | NDST1-2059 | chr5:150554567-150554594 |
| NDST1-714 | chr5:150521062-150521089 | NDST1-2060 | chr5:150554568-150554595 |
| NDST1-715 | chr5:150521067-150521094 | NDST1-2061 | chr5:150554569-150554596 |
| NDST1-716 | chr5:150521068-150521095 | NDST1-2062 | chr5:150554572-150554599 |
| NDST1-717 | chr5:150521069-150521096 | NDST1-2063 | chr5:150554573-150554600 |
| NDST1-718 | chr5:150521070-150521097 | NDST1-2064 | chr5:150554592-150554619 |
| NDST1-719 | chr5:150521071-150521098 | NDST1-2065 | chr5:150554599-150554626 |
| NDST1-720 | chr5:150521072-150521099 | NDST1-2066 | chr5:150554600-150554627 |
| NDST1-721 | chr5:150521073-150521100 | NDST1-2067 | chr5:150554605-150554632 |
| NDST1-722 | chr5:150521098-150521125 | NDST1-2068 | chr5:150554615-150554642 |
| NDST1-723 | chr5:150521099-150521126 | NDST1-2069 | chr5:150554626-150554653 |
| NDST1-724 | chr5:150521102-150521129 | NDST1-2070 | chr5:150554627-150554654 |
| NDST1-725 | chr5:150521103-150521130 | NDST1-2071 | chr5:150554628-150554655 |
| NDST1-726 | chr5:150521105-150521132 | NDST1-2072 | chr5:150554635-150554662 |
| NDST1-727 | chr5:150521108-150521135 | NDST1-2073 | chr5:150554639-150554666 |
| NDST1-728 | chr5:150521113-150521140 | NDST1-2074 | chr5:150554642-150554669 |
| NDST1-729 | chr5:150521114-150521141 | NDST1-2075 | chr5:150554646-150554673 |
| NDST1-730 | chr5:150521120-150521147 | NDST1-2076 | chr5:150554647-150554674 |
| NDST1-731 | chr5:150521121-150521148 | NDST1-2077 | chr5:150554653-150554680 |
| NDST1-732 | chr5:150521122-150521149 | NDST1-2078 | chr5:150554654-150554681 |
| NDST1-733 | chr5:150521135-150521162 | NDST1-2079 | chr5:150554658-150554685 |
| NDST1-734 | chr5:150521139-150521166 | NDST1-2080 | chr5:150554659-150554686 |
| NDST1-735 | chr5:150521142-150521169 | NDST1-2081 | chr5:150554663-150554690 |
| NDST1-736 | chr5:150521169-150521196 | NDST1-2082 | chr5:150554664-150554691 |
| NDST1-737 | chr5:150521170-150521197 | NDST1-2083 | chr5:150554672-150554699 |
| NDST1-738 | chr5:150521171-150521198 | NDST1-2084 | chr5:150554673-150554700 |
| NDST1-739 | chr5:150521177-150521204 | NDST1-2085 | chr5:150554674-150554701 |
| NDST1-740 | chr5:150521178-150521205 | NDST1-2086 | chr5:150554676-150554703 |
| NDST1-741 | chr5:150521179-150521206 | NDST1-2087 | chr5:150554708-150554735 |
| NDST1-742 | chr5:150521185-150521212 | NDST1-2088 | chr5:150554712-150554739 |
| NDST1-743 | chr5:150521187-150521214 | NDST1-2089 | chr5:150554713-150554740 |
| NDST1-744 | chr5:150521188-150521215 | NDST1-2090 | chr5:150554714-150554741 |
| NDST1-745 | chr5:150521192-150521219 | NDST1-2091 | chr5:150554715-150554742 |
| NDST1-746 | chr5:150521196-150521223 | NDST1-2092 | chr5:150554717-150554744 |
| NDST1-747 | chr5:150521197-150521224 | NDST1-2093 | chr5:150554718-150554745 |
| NDST1-748 | chr5:150521199-150521226 | NDST1-2094 | chr5:150554719-150554746 |
| NDST1-749 | chr5:150521204-150521231 | NDST1-2095 | chr5:150554720-150554747 |
| NDST1-750 | chr5:150521207-150521234 | NDST1-2096 | chr5:150554725-150554752 |
| NDST1-751 | chr5:150521210-150521237 | NDST1-2097 | chr5:150554726-150554753 |
| NDST1-752 | chr5:150521213-150521240 | NDST1-2098 | chr5:150554738-150554765 |
| NDST1-753 | chr5:150521219-150521246 | NDST1-2099 | chr5:150554739-150554766 |
| NDST1-754 | chr5:150521220-150521247 | NDST1-2100 | chr5:150554752-150554779 |
| NDST1-755 | chr5:150521222-150521249 | NDST1-2101 | chr5:150554753-150554780 |
| NDST1-756 | chr5:150521225-150521252 | NDST1-2102 | chr5:150554754-150554781 |
| NDST1-757 | chr5:150521227-150521254 | NDST1-2103 | chr5:150554770-150554797 |
| NDST1-758 | chr5:150521228-150521255 | NDST1-2104 | chr5:150554771-150554798 |
| NDST1-759 | chr5:150521235-150521262 | NDST1-2105 | chr5:150554772-150554799 |
| NDST1-760 | chr5:150521240-150521267 | NDST1-2106 | chr5:150554774-150554801 |
| NDST1-761 | chr5:150521250-150521277 | NDST1-2107 | chr5:150554780-150554807 |
| NDST1-762 | chr5:150521251-150521278 | NDST1-2108 | chr5:150554781-150554808 |
| NDST1-763 | chr5:150521254-150521281 | NDST1-2109 | chr5:150554788-150554815 |
| NDST1-764 | chr5:150521258-150521285 | NDST1-2110 | chr5:150554789-150554816 |
| NDST1-765 | chr5:150521262-150521289 | NDST1-2111 | chr5:150554795-150554822 |
| NDST1-766 | chr5:150521263-150521290 | NDST1-2112 | chr5:150554797-150554824 |
| NDST1-767 | chr5:150521272-150521299 | NDST1-2113 | chr5:150554798-150554825 |
| NDST1-768 | chr5:150521275-150521302 | NDST1-2114 | chr5:150554800-150554827 |
| NDST1-769 | chr5:150521279-150521306 | NDST1-2115 | chr5:150554801-150554828 |
| NDST1-770 | chr5:150521298-150521325 | NDST1-2116 | chr5:150554809-150554836 |
| NDST1-771 | chr5:150521299-150521326 | NDST1-2117 | chr5:150554813-150554840 |
| NDST1-772 | chr5:150521300-150521327 | NDST1-2118 | chr5:150554814-150554841 |
| NDST1-773 | chr5:150521311-150521338 | NDST1-2119 | chr5:150554815-150554842 |
| NDST1-774 | chr5:150521317-150521344 | NDST1-2120 | chr5:150554849-150554876 |
| NDST1-775 | chr5:150521330-150521357 | NDST1-2121 | chr5:150554853-150554880 |
| NDST1-776 | chr5:150521335-150521362 | NDST1-2122 | chr5:150554854-150554881 |
| NDST1-777 | chr5:150521346-150521373 | NDST1-2123 | chr5:150554855-150554882 |
| NDST1-778 | chr5:150521350-150521377 | NDST1-2124 | chr5:150554858-150554885 |
| NDST1-779 | chr5:150521358-150521385 | NDST1-2125 | chr5:150554859-150554886 |
| NDST1-780 | chr5:150521363-150521390 | NDST1-2126 | chr5:150554860-150554887 |
| NDST1-781 | chr5:150521365-150521392 | NDST1-2127 | chr5:150554863-150554890 |
| NDST1-782 | chr5:150521372-150521399 | NDST1-2128 | chr5:150554864-150554891 |
| NDST1-783 | chr5:150521375-150521402 | NDST1-2129 | chr5:150554865-150554892 |
| NDST1-784 | chr5:150521386-150521413 | NDST1-2130 | chr5:150554878-150554905 |
| NDST1-785 | chr5:150521393-150521420 | NDST1-2131 | chr5:150554895-150554922 |
| NDST1-786 | chr5:150521394-150521421 | NDST1-2132 | chr5:150554900-150554927 |
| NDST1-787 | chr5:150521397-150521424 | NDST1-2133 | chr5:150554901-150554928 |
| NDST1-788 | chr5:150521398-150521425 | NDST1-2134 | chr5:150554906-150554933 |
| NDST1-789 | chr5:150521399-150521426 | NDST1-2135 | chr5:150554907-150554934 |
| NDST1-790 | chr5:150521406-150521433 | NDST1-2136 | chr5:150554908-150554935 |
| NDST1-791 | chr5:150521407-150521434 | NDST1-2137 | chr5:150554909-150554936 |
| NDST1-792 | chr5:150521408-150521435 | NDST1-2138 | chr5:150554912-150554939 |
| NDST1-793 | chr5:150521409-150521436 | NDST1-2139 | chr5:150554919-150554946 |
| NDST1-794 | chr5:150521414-150521441 | NDST1-2140 | chr5:150554920-150554947 |
| NDST1-795 | chr5:150521428-150521455 | NDST1-2141 | chr5:150554932-150554959 |
| NDST1-796 | chr5:150521429-150521456 | NDST1-2142 | chr5:150554933-150554960 |
| NDST1-797 | chr5:150521432-150521459 | NDST1-2143 | chr5:150554938-150554965 |
| NDST1-798 | chr5:150521435-150521462 | NDST1-2144 | chr5:150554941-150554968 |
| NDST1-799 | chr5:150521442-150521469 | NDST1-2145 | chr5:150554942-150554969 |
| NDST1-800 | chr5:150521443-150521470 | NDST1-2146 | chr5:150554943-150554970 |
| NDST1-801 | chr5:150521444-150521471 | NDST1-2147 | chr5:150554970-150554997 |
| NDST1-802 | chr5:150521445-150521472 | NDST1-2148 | chr5:150554971-150554998 |
| NDST1-803 | chr5:150521450-150521477 | NDST1-2149 | chr5:150554972-150554999 |
| NDST1-804 | chr5:150521452-150521479 | NDST1-2150 | chr5:150554976-150555003 |
| NDST1-805 | chr5:150521459-150521486 | NDST1-2151 | chr5:150554980-150555007 |
| NDST1-806 | chr5:150521468-150521495 | NDST1-2152 | chr5:150554991-150555018 |
| NDST1-807 | chr5:150521480-150521507 | NDST1-2153 | chr5:150554992-150555019 |
| NDST1-808 | chr5:150521481-150521508 | NDST1-2154 | chr5:150555003-150555030 |
| NDST1-809 | chr5:150521484-150521511 | NDST1-2155 | chr5:150555009-150555036 |
| NDST1-810 | chr5:150521485-150521512 | NDST1-2156 | chr5:150555013-150555040 |
| NDST1-811 | chr5:150521486-150521513 | NDST1-2157 | chr5:150555014-150555041 |
| NDST1-812 | chr5:150521490-150521517 | NDST1-2158 | chr5:150555015-150555042 |
| NDST1-813 | chr5:150521491-150521518 | NDST1-2159 | chr5:150555018-150555045 |
| NDST1-814 | chr5:150521495-150521522 | NDST1-2160 | chr5:150555021-150555048 |
| NDST1-815 | chr5:150521500-150521527 | NDST1-2161 | chr5:150555030-150555057 |
| NDST1-816 | chr5:150521501-150521528 | NDST1-2162 | chr5:150555032-150555059 |
| NDST1-817 | chr5:150521516-150521543 | NDST1-2163 | chr5:150555042-150555069 |
| NDST1-818 | chr5:150521517-150521544 | NDST1-2164 | chr5:150555049-150555076 |
| NDST1-819 | chr5:150521522-150521549 | NDST1-2165 | chr5:150555052-150555079 |
| NDST1-820 | chr5:150521525-150521552 | NDST1-2166 | chr5:150555053-150555080 |
| NDST1-821 | chr5:150521527-150521554 | NDST1-2167 | chr5:150555058-150555085 |
| NDST1-822 | chr5:150521528-150521555 | NDST1-2168 | chr5:150555059-150555086 |
| NDST1-823 | chr5:150521531-150521558 | NDST1-2169 | chr5:150555060-150555087 |
| NDST1-824 | chr5:150521540-150521567 | NDST1-2170 | chr5:150555072-150555099 |
| NDST1-825 | chr5:150521545-150521572 | NDST1-2171 | chr5:150555079-150555106 |
| NDST1-826 | chr5:150521559-150521586 | NDST1-2172 | chr5:150555086-150555113 |
| NDST1-827 | chr5:150521563-150521590 | NDST1-2173 | chr5:150555100-150555127 |
| NDST1-828 | chr5:150521571-150521598 | NDST1-2174 | chr5:150555101-150555128 |
| NDST1-829 | chr5:150521575-150521602 | NDST1-2175 | chr5:150555109-150555136 |
| NDST1-830 | chr5:150521579-150521606 | NDST1-2176 | chr5:150555110-150555137 |
| NDST1-831 | chr5:150521580-150521607 | NDST1-2177 | chr5:150555116-150555143 |
| NDST1-832 | chr5:150521585-150521612 | NDST1-2178 | chr5:150555122-150555149 |
| NDST1-833 | chr5:150521586-150521613 | NDST1-2179 | chr5:150555123-150555150 |
| NDST1-834 | chr5:150521587-150521614 | NDST1-2180 | chr5:150555133-150555160 |
| NDST1-835 | chr5:150521603-150521630 | NDST1-2181 | chr5:150555139-150555166 |
| NDST1-836 | chr5:150521612-150521639 | NDST1-2182 | chr5:150555144-150555171 |
| NDST1-837 | chr5:150521613-150521640 | NDST1-2183 | chr5:150555145-150555172 |
| NDST1-838 | chr5:150521619-150521646 | NDST1-2184 | chr5:150555153-150555180 |
| NDST1-839 | chr5:150521621-150521648 | NDST1-2185 | chr5:150555161-150555188 |
| NDST1-840 | chr5:150521622-150521649 | NDST1-2186 | chr5:150555162-150555189 |
| NDST1-841 | chr5:150521641-150521668 | NDST1-2187 | chr5:150555163-150555190 |
| NDST1-842 | chr5:150521647-150521674 | NDST1-2188 | chr5:150555166-150555193 |
| NDST1-843 | chr5:150521655-150521682 | NDST1-2189 | chr5:150555174-150555201 |
| NDST1-844 | chr5:150521656-150521683 | NDST1-2190 | chr5:150555175-150555202 |
| NDST1-845 | chr5:150521669-150521696 | NDST1-2191 | chr5:150555186-150555213 |
| NDST1-846 | chr5:150521677-150521704 | NDST1-2192 | chr5:150555192-150555219 |
| NDST1-847 | chr5:150521683-150521710 | NDST1-2193 | chr5:150555193-150555220 |
| NDST1-848 | chr5:150521684-150521711 | NDST1-2194 | chr5:150555198-150555225 |
| NDST1-849 | chr5:150521692-150521719 | NDST1-2195 | chr5:150555199-150555226 |
| NDST1-850 | chr5:150521693-150521720 | NDST1-2196 | chr5:150555202-150555229 |
| NDST1-851 | chr5:150521700-150521727 | NDST1-2197 | chr5:150555203-150555230 |
| NDST1-852 | chr5:150521707-150521734 | NDST1-2198 | chr5:150555206-150555233 |
| NDST1-853 | chr5:150521708-150521735 | NDST1-2199 | chr5:150555208-150555235 |
| NDST1-854 | chr5:150521715-150521742 | NDST1-2200 | chr5:150555209-150555236 |
| NDST1-855 | chr5:150521720-150521747 | NDST1-2201 | chr5:150555210-150555237 |
| NDST1-856 | chr5:150521721-150521748 | NDST1-2202 | chr5:150555225-150555252 |
| NDST1-857 | chr5:150521730-150521757 | NDST1-2203 | chr5:150555234-150555261 |
| NDST1-858 | chr5:150521736-150521763 | NDST1-2204 | chr5:150555235-150555262 |
| NDST1-859 | chr5:150521741-150521768 | NDST1-2205 | chr5:150555243-150555270 |
| NDST1-860 | chr5:150521756-150521783 | NDST1-2206 | chr5:150555248-150555275 |
| NDST1-861 | chr5:150521757-150521784 | NDST1-2207 | chr5:150555252-150555279 |
| NDST1-862 | chr5:150521786-150521813 | NDST1-2208 | chr5:150555253-150555280 |
| NDST1-863 | chr5:150521787-150521814 | NDST1-2209 | chr5:150555257-150555284 |
| NDST1-864 | chr5:150521788-150521815 | NDST1-2210 | chr5:150555261-150555288 |
| NDST1-865 | chr5:150521803-150521830 | NDST1-2211 | chr5:150555265-150555292 |
| NDST1-866 | chr5:150521804-150521831 | NDST1-2212 | chr5:150555273-150555300 |
| NDST1-867 | chr5:150521805-150521832 | NDST1-2213 | chr5:150555283-150555310 |
| NDST1-868 | chr5:150521806-150521833 | NDST1-2214 | chr5:150555297-150555324 |
| NDST1-869 | chr5:150521819-150521846 | NDST1-2215 | chr5:150555298-150555325 |
| NDST1-870 | chr5:150521822-150521849 | NDST1-2216 | chr5:150555299-150555326 |
| NDST1-871 | chr5:150521827-150521854 | NDST1-2217 | chr5:150555302-150555329 |
| NDST1-872 | chr5:150521844-150521871 | NDST1-2218 | chr5:150555303-150555330 |
| NDST1-873 | chr5:150521845-150521872 | NDST1-2219 | chr5:150555316-150555343 |
| NDST1-874 | chr5:150521846-150521873 | NDST1-2220 | chr5:150555317-150555344 |
| NDST1-875 | chr5:150521853-150521880 | NDST1-2221 | chr5:150555318-150555345 |
| NDST1-876 | chr5:150521854-150521881 | NDST1-2222 | chr5:150555319-150555346 |
| NDST1-877 | chr5:150521857-150521884 | NDST1-2223 | chr5:150555320-150555347 |
| NDST1-878 | chr5:150521858-150521885 | NDST1-2224 | chr5:150555325-150555352 |
| NDST1-879 | chr5:150521860-150521887 | NDST1-2225 | chr5:150555326-150555353 |
| NDST1-880 | chr5:150521861-150521888 | NDST1-2226 | chr5:150555328-150555355 |
| NDST1-881 | chr5:150521864-150521891 | NDST1-2227 | chr5:150555332-150555359 |
| NDST1-882 | chr5:150521867-150521894 | NDST1-2228 | chr5:150555333-150555360 |
| NDST1-883 | chr5:150527684-150527711 | NDST1-2229 | chr5:150555334-150555361 |
| NDST1-884 | chr5:150527685-150527712 | NDST1-2230 | chr5:150555338-150555365 |
| NDST1-885 | chr5:150527688-150527715 | NDST1-2231 | chr5:150555348-150555375 |
| NDST1-886 | chr5:150527697-150527724 | NDST1-2232 | chr5:150555367-150555394 |
| NDST1-887 | chr5:150527713-150527740 | NDST1-2233 | chr5:150555368-150555395 |
| NDST1-888 | chr5:150527725-150527752 | NDST1-2234 | chr5:150555371-150555398 |
| NDST1-889 | chr5:150527726-150527753 | NDST1-2235 | chr5:150555372-150555399 |
| NDST1-890 | chr5:150527727-150527754 | NDST1-2236 | chr5:150555380-150555407 |
| NDST1-891 | chr5:150527733-150527760 | NDST1-2237 | chr5:150555382-150555409 |
| NDST1-892 | chr5:150527746-150527773 | NDST1-2238 | chr5:150555393-150555420 |
| NDST1-893 | chr5:150527777-150527804 | NDST1-2239 | chr5:150555394-150555421 |
| NDST1-894 | chr5:150527778-150527805 | NDST1-2240 | chr5:150555406-150555433 |
| NDST1-895 | chr5:150527779-150527806 | NDST1-2241 | chr5:150555409-150555436 |
| NDST1-896 | chr5:150527783-150527810 | NDST1-2242 | chr5:150555423-150555450 |
| NDST1-897 | chr5:150527787-150527814 | NDST1-2243 | chr5:150555453-150555480 |
| NDST1-898 | chr5:150527788-150527815 | NDST1-2244 | chr5:150555454-150555481 |
| NDST1-899 | chr5:150527791-150527818 | NDST1-2245 | chr5:150555455-150555482 |
| NDST1-900 | chr5:150527805-150527832 | NDST1-2246 | chr5:150555460-150555487 |
| NDST1-901 | chr5:150527813-150527840 | NDST1-2247 | chr5:150555461-150555488 |
| NDST1-902 | chr5:150527814-150527841 | NDST1-2248 | chr5:150555462-150555489 |
| NDST1-903 | chr5:150527818-150527845 | NDST1-2249 | chr5:150555468-150555495 |
| NDST1-904 | chr5:150527843-150527870 | NDST1-2250 | chr5:150555469-150555496 |
| NDST1-905 | chr5:150527844-150527871 | NDST1-2251 | chr5:150555470-150555497 |
| NDST1-906 | chr5:150527845-150527872 | NDST1-2252 | chr5:150555476-150555503 |
| NDST1-907 | chr5:150527846-150527873 | NDST1-2253 | chr5:150555477-150555504 |
| NDST1-908 | chr5:150527847-150527874 | NDST1-2254 | chr5:150555483-150555510 |
| NDST1-909 | chr5:150527848-150527875 | NDST1-2255 | chr5:150555486-150555513 |
| NDST1-910 | chr5:150527852-150527879 | NDST1-2256 | chr5:150555487-150555514 |
| NDST1-911 | chr5:150527854-150527881 | NDST1-2257 | chr5:150555491-150555518 |
| NDST1-912 | chr5:150527866-150527893 | NDST1-2258 | chr5:150555495-150555522 |
| NDST1-913 | chr5:150527872-150527899 | NDST1-2259 | chr5:150555509-150555536 |
| NDST1-914 | chr5:150527893-150527920 | NDST1-2260 | chr5:150555510-150555537 |
| NDST1-915 | chr5:150527894-150527921 | NDST1-2261 | chr5:150555515-150555542 |
| NDST1-916 | chr5:150527895-150527922 | NDST1-2262 | chr5:150555517-150555544 |
| NDST1-917 | chr5:150527901-150527928 | NDST1-2263 | chr5:150555522-150555549 |
| NDST1-918 | chr5:150527902-150527929 | NDST1-2264 | chr5:150555523-150555550 |
| NDST1-919 | chr5:150527903-150527930 | NDST1-2265 | chr5:150555526-150555553 |
| NDST1-920 | chr5:150527906-150527933 | NDST1-2266 | chr5:150555568-150555595 |
| NDST1-921 | chr5:150527909-150527936 | NDST1-2267 | chr5:150555575-150555602 |
| NDST1-922 | chr5:150527916-150527943 | NDST1-2268 | chr5:150555576-150555603 |
| NDST1-923 | chr5:150527924-150527951 | NDST1-2269 | chr5:150555577-150555604 |
| NDST1-924 | chr5:150527928-150527955 | NDST1-2270 | chr5:150555584-150555611 |
| NDST1-925 | chr5:150527933-150527960 | NDST1-2271 | chr5:150555585-150555612 |
| NDST1-926 | chr5:150527938-150527965 | NDST1-2272 | chr5:150555589-150555616 |
| NDST1-927 | chr5:150527942-150527969 | NDST1-2273 | chr5:150555590-150555617 |
| NDST1-928 | chr5:150527950-150527977 | NDST1-2274 | chr5:150555591-150555618 |
| NDST1-929 | chr5:150527951-150527978 | NDST1-2275 | chr5:150555598-150555625 |
| NDST1-930 | chr5:150527952-150527979 | NDST1-2276 | chr5:150555624-150555651 |
| NDST1-931 | chr5:150527974-150528001 | NDST1-2277 | chr5:150555625-150555652 |
| NDST1-932 | chr5:150527984-150528011 | NDST1-2278 | chr5:150555626-150555653 |
| NDST1-933 | chr5:150527988-150528015 | NDST1-2279 | chr5:150555628-150555655 |
| NDST1-934 | chr5:150527991-150528018 | NDST1-2280 | chr5:150555631-150555658 |
| NDST1-935 | chr5:150527999-150528026 | NDST1-2281 | chr5:150555632-150555659 |
| NDST1-936 | chr5:150528012-150528039 | NDST1-2282 | chr5:150555635-150555662 |
| NDST1-937 | chr5:150528020-150528047 | NDST1-2283 | chr5:150555636-150555663 |
| NDST1-938 | chr5:150528021-150528048 | NDST1-2284 | chr5:150555638-150555665 |
| NDST1-939 | chr5:150528033-150528060 | NDST1-2285 | chr5:150555639-150555666 |
| NDST1-940 | chr5:150528037-150528064 | NDST1-2286 | chr5:150555640-150555667 |
| NDST1-941 | chr5:150528038-150528065 | NDST1-2287 | chr5:150555643-150555670 |
| NDST1-942 | chr5:150528043-150528070 | NDST1-2288 | chr5:150555644-150555671 |
| NDST1-943 | chr5:150528057-150528084 | NDST1-2289 | chr5:150555649-150555676 |
| NDST1-944 | chr5:150528061-150528088 | NDST1-2290 | chr5:150555666-150555693 |
| NDST1-945 | chr5:150528062-150528089 | NDST1-2291 | chr5:150555671-150555698 |
| NDST1-946 | chr5:150528068-150528095 | NDST1-2292 | chr5:150555687-150555714 |
| NDST1-947 | chr5:150528074-150528101 | NDST1-2293 | chr5:150555688-150555715 |
| NDST1-948 | chr5:150528075-150528102 | NDST1-2294 | chr5:150555689-150555716 |
| NDST1-949 | chr5:150528081-150528108 | NDST1-2295 | chr5:150555692-150555719 |
| NDST1-950 | chr5:150528087-150528114 | NDST1-2296 | chr5:150555695-150555722 |
| NDST1-951 | chr5:150528091-150528118 | NDST1-2297 | chr5:150555706-150555733 |
| NDST1-952 | chr5:150528097-150528124 | NDST1-2298 | chr5:150555707-150555734 |
| NDST1-953 | chr5:150528103-150528130 | NDST1-2299 | chr5:150555714-150555741 |
| NDST1-954 | chr5:150528108-150528135 | NDST1-2300 | chr5:150555715-150555742 |
| NDST1-955 | chr5:150528118-150528145 | NDST1-2301 | chr5:150555718-150555745 |
| NDST1-956 | chr5:150528127-150528154 | NDST1-2302 | chr5:150555722-150555749 |
| NDST1-957 | chr5:150528142-150528169 | NDST1-2303 | chr5:150555723-150555750 |
| NDST1-958 | chr5:150528149-150528176 | NDST1-2304 | chr5:150555727-150555754 |
| NDST1-959 | chr5:150528158-150528185 | NDST1-2305 | chr5:150555730-150555757 |
| NDST1-960 | chr5:150528170-150528197 | NDST1-2306 | chr5:150555731-150555758 |
| NDST1-961 | chr5:150528171-150528198 | NDST1-2307 | chr5:150555735-150555762 |
| NDST1-962 | chr5:150528172-150528199 | NDST1-2308 | chr5:150555741-150555768 |
| NDST1-963 | chr5:150528180-150528207 | NDST1-2309 | chr5:150555742-150555769 |
| NDST1-964 | chr5:150528186-150528213 | NDST1-2310 | chr5:150555746-150555773 |
| NDST1-965 | chr5:150528190-150528217 | NDST1-2311 | chr5:150555763-150555790 |
| NDST1-966 | chr5:150528194-150528221 | NDST1-2312 | chr5:150555770-150555797 |
| NDST1-967 | chr5:150528205-150528232 | NDST1-2313 | chr5:150555776-150555803 |
| NDST1-968 | chr5:150528209-150528236 | NDST1-2314 | chr5:150555777-150555804 |
| NDST1-969 | chr5:150528210-150528237 | NDST1-2315 | chr5:150555778-150555805 |
| NDST1-970 | chr5:150528211-150528238 | NDST1-2316 | chr5:150555781-150555808 |
| NDST1-971 | chr5:150528212-150528239 | NDST1-2317 | chr5:150555787-150555814 |
| NDST1-972 | chr5:150528215-150528242 | NDST1-2318 | chr5:150555790-150555817 |
| NDST1-973 | chr5:150528223-150528250 | NDST1-2319 | chr5:150555791-150555818 |
| NDST1-974 | chr5:150528232-150528259 | NDST1-2320 | chr5:150555794-150555821 |
| NDST1-975 | chr5:150528233-150528260 | NDST1-2321 | chr5:150555822-150555849 |
| NDST1-976 | chr5:150528234-150528261 | NDST1-2322 | chr5:150555826-150555853 |
| NDST1-977 | chr5:150528239-150528266 | NDST1-2323 | chr5:150555834-150555861 |
| NDST1-978 | chr5:150528242-150528269 | NDST1-2324 | chr5:150555852-150555879 |
| NDST1-979 | chr5:150528243-150528270 | NDST1-2325 | chr5:150555857-150555884 |
| NDST1-980 | chr5:150528257-150528284 | NDST1-2326 | chr5:150555859-150555886 |
| NDST1-981 | chr5:150528260-150528287 | NDST1-2327 | chr5:150555863-150555890 |
| NDST1-982 | chr5:150528263-150528290 | NDST1-2328 | chr5:150555864-150555891 |
| NDST1-983 | chr5:150528272-150528299 | NDST1-2329 | chr5:150555875-150555902 |
| NDST1-984 | chr5:150528277-150528304 | NDST1-2330 | chr5:150555888-150555915 |
| NDST1-985 | chr5:150528281-150528308 | NDST1-2331 | chr5:150555892-150555919 |
| NDST1-986 | chr5:150528282-150528309 | NDST1-2332 | chr5:150555901-150555928 |
| NDST1-987 | chr5:150528283-150528310 | NDST1-2333 | chr5:150555914-150555941 |
| NDST1-988 | chr5:150528284-150528311 | NDST1-2334 | chr5:150555929-150555956 |
| NDST1-989 | chr5:150528295-150528322 | NDST1-2335 | chr5:150555937-150555964 |
| NDST1-990 | chr5:150528296-150528323 | NDST1-2336 | chr5:150555940-150555967 |
| NDST1-991 | chr5:150528301-150528328 | NDST1-2337 | chr5:150555941-150555968 |
| NDST1-992 | chr5:150528305-150528332 | NDST1-2338 | chr5:150555946-150555973 |
| NDST1-993 | chr5:150528308-150528335 | NDST1-2339 | chr5:150555952-150555979 |
| NDST1-994 | chr5:150528309-150528336 | NDST1-2340 | chr5:150555961-150555988 |
| NDST1-995 | chr5:150528310-150528337 | NDST1-2341 | chr5:150555962-150555989 |
| NDST1-996 | chr5:150528315-150528342 | NDST1-2342 | chr5:150555965-150555992 |
| NDST1-997 | chr5:150528319-150528346 | NDST1-2343 | chr5:150555966-150555993 |
| NDST1-998 | chr5:150528333-150528360 | NDST1-2344 | chr5:150555971-150555998 |
| NDST1-999 | chr5:150528338-150528365 | NDST1-2345 | chr5:150555975-150556002 |
| NDST1-1000 | chr5:150528346-150528373 | NDST1-2346 | chr5:150555976-150556003 |
| NDST1-1001 | chr5:150528347-150528374 | NDST1-2347 | chr5:150555977-150556004 |
| NDST1-1002 | chr5:150528353-150528380 | NDST1-2348 | chr5:150555978-150556005 |
| NDST1-1003 | chr5:150528363-150528390 | NDST1-2349 | chr5:150555979-150556006 |
| NDST1-1004 | chr5:150528364-150528391 | NDST1-2350 | chr5:150555980-150556007 |
| NDST1-1005 | chr5:150528365-150528392 | NDST1-2351 | chr5:150555985-150556012 |
| NDST1-1006 | chr5:150528377-150528404 | NDST1-2352 | chr5:150555987-150556014 |
| NDST1-1007 | chr5:150528378-150528405 | NDST1-2353 | chr5:150555992-150556019 |
| NDST1-1008 | chr5:150528379-150528406 | NDST1-2354 | chr5:150556008-150556035 |
| NDST1-1009 | chr5:150528380-150528407 | NDST1-2355 | chr5:150556009-150556036 |
| NDST1-1010 | chr5:150528393-150528420 | NDST1-2356 | chr5:150556010-150556037 |
| NDST1-1011 | chr5:150528394-150528421 | NDST1-2357 | chr5:150556011-150556038 |
| NDST1-1012 | chr5:150528395-150528422 | NDST1-2358 | chr5:150556012-150556039 |
| NDST1-1013 | chr5:150532820-150532847 | NDST1-2359 | chr5:150556026-150556053 |
| NDST1-1014 | chr5:150532824-150532851 | NDST1-2360 | chr5:150556027-150556054 |
| NDST1-1015 | chr5:150532827-150532854 | NDST1-2361 | chr5:150556031-150556058 |
| NDST1-1016 | chr5:150532828-150532855 | NDST1-2362 | chr5:150556034-150556061 |
| NDST1-1017 | chr5:150532858-150532885 | NDST1-2363 | chr5:150556065-150556092 |
| NDST1-1018 | chr5:150532859-150532886 | NDST1-2364 | chr5:150556066-150556093 |
| NDST1-1019 | chr5:150532860-150532887 | NDST1-2365 | chr5:150556067-150556094 |
| NDST1-1020 | chr5:150532868-150532895 | NDST1-2366 | chr5:150556068-150556095 |
| NDST1-1021 | chr5:150532869-150532896 | NDST1-2367 | chr5:150556071-150556098 |
| NDST1-1022 | chr5:150532876-150532903 | NDST1-2368 | chr5:150556072-150556099 |
| NDST1-1023 | chr5:150532877-150532904 | NDST1-2369 | chr5:150556081-150556108 |
| NDST1-1024 | chr5:150532878-150532905 | NDST1-2370 | chr5:150556085-150556112 |
| NDST1-1025 | chr5:150532901-150532928 | NDST1-2371 | chr5:150556100-150556127 |
| NDST1-1026 | chr5:150532910-150532937 | NDST1-2372 | chr5:150556109-150556136 |
| NDST1-1027 | chr5:150532911-150532938 | NDST1-2373 | chr5:150556120-150556147 |
| NDST1-1028 | chr5:150532918-150532945 | NDST1-2374 | chr5:150556123-150556150 |
| NDST1-1029 | chr5:150532922-150532949 | NDST1-2375 | chr5:150556131-150556158 |
| NDST1-1030 | chr5:150532929-150532956 | NDST1-2376 | chr5:150556148-150556175 |
| NDST1-1031 | chr5:150532930-150532957 | NDST1-2377 | chr5:150556158-150556185 |
| NDST1-1032 | chr5:150532931-150532958 | NDST1-2378 | chr5:150556161-150556188 |
| NDST1-1033 | chr5:150532935-150532962 | NDST1-2379 | chr5:150556181-150556208 |
| NDST1-1034 | chr5:150532940-150532967 | NDST1-2380 | chr5:150556191-150556218 |
| NDST1-1035 | chr5:150532946-150532973 | NDST1-2381 | chr5:150556207-150556234 |
| NDST1-1036 | chr5:150532947-150532974 | NDST1-2382 | chr5:150556208-150556235 |
| NDST1-1037 | chr5:150532978-150533005 | NDST1-2383 | chr5:150556210-150556237 |
| NDST1-1038 | chr5:150532979-150533006 | NDST1-2384 | chr5:150556211-150556238 |
| NDST1-1039 | chr5:150532983-150533010 | NDST1-2385 | chr5:150556212-150556239 |
| NDST1-1040 | chr5:150532984-150533011 | NDST1-2386 | chr5:150556215-150556242 |
| NDST1-1041 | chr5:150532988-150533015 | NDST1-2387 | chr5:150556218-150556245 |
| NDST1-1042 | chr5:150532989-150533016 | NDST1-2388 | chr5:150556222-150556249 |
| NDST1-1043 | chr5:150532994-150533021 | NDST1-2389 | chr5:150556228-150556255 |
| NDST1-1044 | chr5:150533001-150533028 | NDST1-2390 | chr5:150556229-150556256 |
| NDST1-1045 | chr5:150533006-150533033 | NDST1-2391 | chr5:150556230-150556257 |
| NDST1-1046 | chr5:150533013-150533040 | NDST1-2392 | chr5:150556257-150556284 |
| NDST1-1047 | chr5:150533014-150533041 | NDST1-2393 | chr5:150556267-150556294 |
| NDST1-1048 | chr5:150533025-150533052 | NDST1-2394 | chr5:150556273-150556300 |
| NDST1-1049 | chr5:150533042-150533069 | NDST1-2395 | chr5:150556276-150556303 |
| NDST1-1050 | chr5:150533045-150533072 | NDST1-2396 | chr5:150556285-150556312 |
| NDST1-1051 | chr5:150533046-150533073 | NDST1-2397 | chr5:150556291-150556318 |
| NDST1-1052 | chr5:150533047-150533074 | NDST1-2398 | chr5:150556293-150556320 |
| NDST1-1053 | chr5:150533048-150533075 | NDST1-2399 | chr5:150556296-150556323 |
| NDST1-1054 | chr5:150533052-150533079 | NDST1-2400 | chr5:150556309-150556336 |
| NDST1-1055 | chr5:150533053-150533080 | NDST1-2401 | chr5:150556319-150556346 |
| NDST1-1056 | chr5:150533068-150533095 | NDST1-2402 | chr5:150556320-150556347 |
| NDST1-1057 | chr5:150533076-150533103 | NDST1-2403 | chr5:150556348-150556375 |
| NDST1-1058 | chr5:150533084-150533111 | NDST1-2404 | chr5:150556359-150556386 |
| NDST1-1059 | chr5:150533085-150533112 | NDST1-2405 | chr5:150556360-150556387 |
| NDST1-1060 | chr5:150533086-150533113 | NDST1-2406 | chr5:150556361-150556388 |
| NDST1-1061 | chr5:150533087-150533114 | NDST1-2407 | chr5:150556362-150556389 |
| NDST1-1062 | chr5:150533090-150533117 | NDST1-2408 | chr5:150556363-150556390 |
| NDST1-1063 | chr5:150533091-150533118 | NDST1-2409 | chr5:150556372-150556399 |
| NDST1-1064 | chr5:150533093-150533120 | NDST1-2410 | chr5:150556389-150556416 |
| NDST1-1065 | chr5:150533094-150533121 | NDST1-2411 | chr5:150556390-150556417 |
| NDST1-1066 | chr5:150533097-150533124 | NDST1-2412 | chr5:150556395-150556422 |
| NDST1-1067 | chr5:150533098-150533125 | NDST1-2413 | chr5:150556403-150556430 |
| NDST1-1068 | chr5:150533101-150533128 | NDST1-2414 | chr5:150556412-150556439 |
| NDST1-1069 | chr5:150533102-150533129 | NDST1-2415 | chr5:150556413-150556440 |
| NDST1-1070 | chr5:150533106-150533133 | NDST1-2416 | chr5:150556421-150556448 |
| NDST1-1071 | chr5:150533119-150533146 | NDST1-2417 | chr5:150556425-150556452 |
| NDST1-1072 | chr5:150533125-150533152 | NDST1-2418 | chr5:150556428-150556455 |
| NDST1-1073 | chr5:150534741-150534768 | NDST1-2419 | chr5:150556439-150556466 |
| NDST1-1074 | chr5:150534742-150534769 | NDST1-2420 | chr5:150556453-150556480 |
| NDST1-1075 | chr5:150534743-150534770 | NDST1-2421 | chr5:150556454-150556481 |
| NDST1-1076 | chr5:150534746-150534773 | NDST1-2422 | chr5:150556457-150556484 |
| NDST1-1077 | chr5:150534747-150534774 | NDST1-2423 | chr5:150556458-150556485 |
| NDST1-1078 | chr5:150534753-150534780 | NDST1-2424 | chr5:150556465-150556492 |
| NDST1-1079 | chr5:150534755-150534782 | NDST1-2425 | chr5:150556466-150556493 |
| NDST1-1080 | chr5:150534765-150534792 | NDST1-2426 | chr5:150556474-150556501 |
| NDST1-1081 | chr5:150534774-150534801 | NDST1-2427 | chr5:150556475-150556502 |
| NDST1-1082 | chr5:150534780-150534807 | NDST1-2428 | chr5:150556518-150556545 |
| NDST1-1083 | chr5:150534781-150534808 | NDST1-2429 | chr5:150556539-150556566 |
| NDST1-1084 | chr5:150534786-150534813 | NDST1-2430 | chr5:150556540-150556567 |
| NDST1-1085 | chr5:150534790-150534817 | NDST1-2431 | chr5:150556545-150556572 |
| NDST1-1086 | chr5:150534791-150534818 | NDST1-2432 | chr5:150556546-150556573 |
| NDST1-1087 | chr5:150534793-150534820 | NDST1-2433 | chr5:150556547-150556574 |
| NDST1-1088 | chr5:150534794-150534821 | NDST1-2434 | chr5:150556580-150556607 |
| NDST1-1089 | chr5:150534795-150534822 | NDST1-2435 | chr5:150556624-150556651 |
| NDST1-1090 | chr5:150534796-150534823 | NDST1-2436 | chr5:150556664-150556691 |
| NDST1-1091 | chr5:150534797-150534824 | NDST1-2437 | chr5:150556665-150556692 |
| NDST1-1092 | chr5:150534802-150534829 | NDST1-2438 | chr5:150556673-150556700 |
| NDST1-1093 | chr5:150534808-150534835 | NDST1-2439 | chr5:150556674-150556701 |
| NDST1-1094 | chr5:150534809-150534836 | NDST1-2440 | chr5:150556679-150556706 |
| NDST1-1095 | chr5:150534810-150534837 | NDST1-2441 | chr5:150556688-150556715 |
| NDST1-1096 | chr5:150534811-150534838 | NDST1-2442 | chr5:150556692-150556719 |
| NDST1-1097 | chr5:150534814-150534841 | NDST1-2443 | chr5:150556708-150556735 |
| NDST1-1098 | chr5:150534822-150534849 | NDST1-2444 | chr5:150556720-150556747 |
| NDST1-1099 | chr5:150534830-150534857 | NDST1-2445 | chr5:150556721-150556748 |
| NDST1-1100 | chr5:150534831-150534858 | NDST1-2446 | chr5:150556733-150556760 |
| NDST1-1101 | chr5:150534840-150534867 | NDST1-2447 | chr5:150556752-150556779 |
| NDST1-1102 | chr5:150534852-150534879 | NDST1-2448 | chr5:150556753-150556780 |
| NDST1-1103 | chr5:150534854-150534881 | NDST1-2449 | chr5:150556779-150556806 |
| NDST1-1104 | chr5:150534861-150534888 | NDST1-2450 | chr5:150556787-150556814 |
| NDST1-1105 | chr5:150534862-150534889 | NDST1-2451 | chr5:150556788-150556815 |
| NDST1-1106 | chr5:150534863-150534890 | NDST1-2452 | chr5:150556789-150556816 |
| NDST1-1107 | chr5:150534870-150534897 | NDST1-2453 | chr5:150556790-150556817 |
| NDST1-1108 | chr5:150534890-150534917 | NDST1-2454 | chr5:150556791-150556818 |
| NDST1-1109 | chr5:150534898-150534925 | NDST1-2455 | chr5:150556795-150556822 |
| NDST1-1110 | chr5:150534901-150534928 | NDST1-2456 | chr5:150556801-150556828 |
| NDST1-1111 | chr5:150534916-150534943 | NDST1-2457 | chr5:150556802-150556829 |
| NDST1-1112 | chr5:150534931-150534958 | NDST1-2458 | chr5:150556820-150556847 |
| NDST1-1113 | chr5:150534932-150534959 | NDST1-2459 | chr5:150556838-150556865 |
| NDST1-1114 | chr5:150534933-150534960 | NDST1-2460 | chr5:150556875-150556902 |
| NDST1-1115 | chr5:150534934-150534961 | NDST1-2461 | chr5:150556883-150556910 |
| NDST1-1116 | chr5:150534946-150534973 | NDST1-2462 | chr5:150556907-150556934 |
| NDST1-1117 | chr5:150534956-150534983 | NDST1-2463 | chr5:150556908-150556935 |
| NDST1-1118 | chr5:150534957-150534984 | NDST1-2464 | chr5:150556922-150556949 |
| NDST1-1119 | chr5:150534958-150534985 | NDST1-2465 | chr5:150556929-150556956 |
| NDST1-1120 | chr5:150534959-150534986 | NDST1-2466 | chr5:150556950-150556977 |
| NDST1-1121 | chr5:150534961-150534988 | NDST1-2467 | chr5:150556951-150556978 |
| NDST1-1122 | chr5:150534967-150534994 | NDST1-2468 | chr5:150556957-150556984 |
| NDST1-1123 | chr5:150534971-150534998 | NDST1-2469 | chr5:150556975-150557002 |
| NDST1-1124 | chr5:150534973-150535000 | NDST1-2470 | chr5:150556976-150557003 |
| NDST1-1125 | chr5:150534978-150535005 | NDST1-2471 | chr5:150556977-150557004 |
| NDST1-1126 | chr5:150534987-150535014 | NDST1-2472 | chr5:150556984-150557011 |
| NDST1-1127 | chr5:150534999-150535026 | NDST1-2473 | chr5:150557022-150557049 |
| NDST1-1128 | chr5:150535015-150535042 | NDST1-2474 | chr5:150557024-150557051 |
| NDST1-1129 | chr5:150535016-150535043 | NDST1-2475 | chr5:150557034-150557061 |
| NDST1-1130 | chr5:150535024-150535051 | NDST1-2476 | chr5:150557035-150557062 |
| NDST1-1131 | chr5:150535025-150535052 | NDST1-2477 | chr5:150557036-150557063 |
| NDST1-1132 | chr5:150535026-150535053 | NDST1-2478 | chr5:150557037-150557064 |
| NDST1-1133 | chr5:150535029-150535056 | NDST1-2479 | chr5:150557077-150557104 |
| NDST1-1134 | chr5:150535030-150535057 | NDST1-2480 | chr5:150557109-150557136 |
| NDST1-1135 | chr5:150535034-150535061 | NDST1-2481 | chr5:150557120-150557147 |
| NDST1-1136 | chr5:150535036-150535063 | NDST1-2482 | chr5:150557123-150557150 |
| NDST1-1137 | chr5:150535037-150535064 | NDST1-2483 | chr5:150557132-150557159 |
| NDST1-1138 | chr5:150535041-150535068 | NDST1-2484 | chr5:150557133-150557160 |
| NDST1-1139 | chr5:150535042-150535069 | NDST1-2485 | chr5:150557134-150557161 |
| NDST1-1140 | chr5:150535046-150535073 | NDST1-2486 | chr5:150557138-150557165 |
| NDST1-1141 | chr5:150535049-150535076 | NDST1-2487 | chr5:150557145-150557172 |
| NDST1-1142 | chr5:150535062-150535089 | NDST1-2488 | chr5:150557156-150557183 |
| NDST1-1143 | chr5:150535078-150535105 | NDST1-2489 | chr5:150557157-150557184 |
| NDST1-1144 | chr5:150535090-150535117 | NDST1-2490 | chr5:150557158-150557185 |
| NDST1-1145 | chr5:150535094-150535121 | NDST1-2491 | chr5:150557159-150557186 |
| NDST1-1146 | chr5:150535114-150535141 | NDST1-2492 | chr5:150557160-150557187 |
| NDST1-1147 | chr5:150535115-150535142 | NDST1-2493 | chr5:150557161-150557188 |
| NDST1-1148 | chr5:150535120-150535147 | NDST1-2494 | chr5:150557162-150557189 |
| NDST1-1149 | chr5:150535121-150535148 | NDST1-2495 | chr5:150557165-150557192 |
| NDST1-1150 | chr5:150535585-150535612 | NDST1-2496 | chr5:150557178-150557205 |
| NDST1-1151 | chr5:150535592-150535619 | NDST1-2497 | chr5:150557184-150557211 |
| NDST1-1152 | chr5:150535597-150535624 | NDST1-2498 | chr5:150557189-150557216 |
| NDST1-1153 | chr5:150535601-150535628 | NDST1-2499 | chr5:150557197-150557224 |
| NDST1-1154 | chr5:150535606-150535633 | NDST1-2500 | chr5:150557198-150557225 |
| NDST1-1155 | chr5:150535609-150535636 | NDST1-2501 | chr5:150557200-150557227 |
| NDST1-1156 | chr5:150535610-150535637 | NDST1-2502 | chr5:150557201-150557228 |
| NDST1-1157 | chr5:150535611-150535638 | NDST1-2503 | chr5:150557204-150557231 |
| NDST1-1158 | chr5:150535612-150535639 | NDST1-2504 | chr5:150557208-150557235 |
| NDST1-1159 | chr5:150535613-150535640 | NDST1-2505 | chr5:150557212-150557239 |
| NDST1-1160 | chr5:150535619-150535646 | NDST1-2506 | chr5:150557219-150557246 |
| NDST1-1161 | chr5:150535622-150535649 | NDST1-2507 | chr5:150557220-150557247 |
| NDST1-1162 | chr5:150535623-150535650 | NDST1-2508 | chr5:150557226-150557253 |
| NDST1-1163 | chr5:150535627-150535654 | NDST1-2509 | chr5:150557230-150557257 |
| NDST1-1164 | chr5:150535628-150535655 | NDST1-2510 | chr5:150557233-150557260 |
| NDST1-1165 | chr5:150535629-150535656 | NDST1-2511 | chr5:150557236-150557263 |
| NDST1-1166 | chr5:150535633-150535660 | NDST1-2512 | chr5:150557251-150557278 |
| NDST1-1167 | chr5:150535647-150535674 | NDST1-2513 | chr5:150557255-150557282 |
| NDST1-1168 | chr5:150535648-150535675 | NDST1-2514 | chr5:150557256-150557283 |
| NDST1-1169 | chr5:150535652-150535679 | NDST1-2515 | chr5:150557269-150557296 |
| NDST1-1170 | chr5:150535657-150535684 | NDST1-2516 | chr5:150557270-150557297 |
| NDST1-1171 | chr5:150535662-150535689 | NDST1-2517 | chr5:150557271-150557298 |
| NDST1-1172 | chr5:150535663-150535690 | NDST1-2518 | chr5:150557275-150557302 |
| NDST1-1173 | chr5:150535664-150535691 | NDST1-2519 | chr5:150557276-150557303 |
| NDST1-1174 | chr5:150535673-150535700 | NDST1-2520 | chr5:150557283-150557310 |
| NDST1-1175 | chr5:150535674-150535701 | NDST1-2521 | chr5:150557284-150557311 |
| NDST1-1176 | chr5:150535675-150535702 | NDST1-2522 | chr5:150557291-150557318 |
| NDST1-1177 | chr5:150535680-150535707 | NDST1-2523 | chr5:150557292-150557319 |
| NDST1-1178 | chr5:150535689-150535716 | NDST1-2524 | chr5:150557304-150557331 |
| NDST1-1179 | chr5:150535694-150535721 | NDST1-2525 | chr5:150557314-150557341 |
| NDST1-1180 | chr5:150535695-150535722 | NDST1-2526 | chr5:150557322-150557349 |
| NDST1-1181 | chr5:150535697-150535724 | NDST1-2527 | chr5:150557323-150557350 |
| NDST1-1182 | chr5:150535698-150535725 | NDST1-2528 | chr5:150557324-150557351 |
| NDST1-1183 | chr5:150535699-150535726 | NDST1-2529 | chr5:150557331-150557358 |
| NDST1-1184 | chr5:150535709-150535736 | NDST1-2530 | chr5:150557332-150557359 |
| NDST1-1185 | chr5:150535712-150535739 | NDST1-2531 | chr5:150557346-150557373 |
| NDST1-1186 | chr5:150535713-150535740 | NDST1-2532 | chr5:150557353-150557380 |
| NDST1-1187 | chr5:150535724-150535751 | NDST1-2533 | chr5:150557354-150557381 |
| NDST1-1188 | chr5:150535725-150535752 | NDST1-2534 | chr5:150557356-150557383 |
| NDST1-1189 | chr5:150535739-150535766 | NDST1-2535 | chr5:150557369-150557396 |
| NDST1-1190 | chr5:150535740-150535767 | NDST1-2536 | chr5:150557370-150557397 |
| NDST1-1191 | chr5:150535741-150535768 | NDST1-2537 | chr5:150557377-150557404 |
| NDST1-1192 | chr5:150535744-150535771 | NDST1-2538 | chr5:150557378-150557405 |
| NDST1-1193 | chr5:150535757-150535784 | NDST1-2539 | chr5:150557382-150557409 |
| NDST1-1194 | chr5:150535759-150535786 | NDST1-2540 | chr5:150557399-150557426 |
| NDST1-1195 | chr5:150535760-150535787 | NDST1-2541 | chr5:150557400-150557427 |
| NDST1-1196 | chr5:150535761-150535788 | NDST1-2542 | chr5:150557405-150557432 |
| NDST1-1197 | chr5:150535762-150535789 | NDST1-2543 | chr5:150557414-150557441 |
| NDST1-1198 | chr5:150535769-150535796 | NDST1-2544 | chr5:150557415-150557442 |
| NDST1-1199 | chr5:150535774-150535801 | NDST1-2545 | chr5:150557416-150557443 |
| NDST1-1200 | chr5:150535796-150535823 | NDST1-2546 | chr5:150557433-150557460 |
| NDST1-1201 | chr5:150535799-150535826 | NDST1-2547 | chr5:150557438-150557465 |
| NDST1-1202 | chr5:150535807-150535834 | NDST1-2548 | chr5:150557449-150557476 |
| NDST1-1203 | chr5:150535815-150535842 | NDST1-2549 | chr5:150557452-150557479 |
| NDST1-1204 | chr5:150535831-150535858 | NDST1-2550 | chr5:150557453-150557480 |
| NDST1-1205 | chr5:150535832-150535859 | NDST1-2551 | chr5:150557455-150557482 |
| NDST1-1206 | chr5:150535833-150535860 | NDST1-2552 | chr5:150557459-150557486 |
| NDST1-1207 | chr5:150535834-150535861 | NDST1-2553 | chr5:150557466-150557493 |
| NDST1-1208 | chr5:150535836-150535863 | NDST1-2554 | chr5:150557477-150557504 |
| NDST1-1209 | chr5:150535837-150535864 | NDST1-2555 | chr5:150557478-150557505 |
| NDST1-1210 | chr5:150535844-150535871 | NDST1-2556 | chr5:150557491-150557518 |
| NDST1-1211 | chr5:150535848-150535875 | NDST1-2557 | chr5:150557492-150557519 |
| NDST1-1212 | chr5:150535849-150535876 | NDST1-2558 | chr5:150557510-150557537 |
| NDST1-1213 | chr5:150535851-150535878 | NDST1-2559 | chr5:150557511-150557538 |
| NDST1-1214 | chr5:150535855-150535882 | NDST1-2560 | chr5:150557515-150557542 |
| NDST1-1215 | chr5:150535858-150535885 | NDST1-2561 | chr5:150557528-150557555 |
| NDST1-1216 | chr5:150535859-150535886 | NDST1-2562 | chr5:150557535-150557562 |
| NDST1-1217 | chr5:150535866-150535893 | NDST1-2563 | chr5:150557540-150557567 |
| NDST1-1218 | chr5:150535867-150535894 | NDST1-2564 | chr5:150557547-150557574 |
| NDST1-1219 | chr5:150535870-150535897 | NDST1-2565 | chr5:150557550-150557577 |
| NDST1-1220 | chr5:150535874-150535901 | NDST1-2566 | chr5:150557551-150557578 |
| NDST1-1221 | chr5:150535883-150535910 | NDST1-2567 | chr5:150557556-150557583 |
| NDST1-1222 | chr5:150535886-150535913 | NDST1-2568 | chr5:150557557-150557584 |
| NDST1-1223 | chr5:150535889-150535916 | NDST1-2569 | chr5:150557564-150557591 |
| NDST1-1224 | chr5:150535890-150535917 | NDST1-2570 | chr5:150557565-150557592 |
| NDST1-1225 | chr5:150535895-150535922 | NDST1-2571 | chr5:150557566-150557593 |
| NDST1-1226 | chr5:150535896-150535923 | NDST1-2572 | chr5:150557576-150557603 |
| NDST1-1227 | chr5:150535897-150535924 | NDST1-2573 | chr5:150557577-150557604 |
| NDST1-1228 | chr5:150535905-150535932 | NDST1-2574 | chr5:150557594-150557621 |
| NDST1-1229 | chr5:150535906-150535933 | NDST1-2575 | chr5:150557595-150557622 |
| NDST1-1230 | chr5:150535936-150535963 | NDST1-2576 | chr5:150557598-150557625 |
| NDST1-1231 | chr5:150535945-150535972 | NDST1-2577 | chr5:150557624-150557651 |
| NDST1-1232 | chr5:150535951-150535978 | NDST1-2578 | chr5:150557629-150557656 |
| NDST1-1233 | chr5:150535959-150535986 | NDST1-2579 | chr5:150557630-150557657 |
| NDST1-1234 | chr5:150535965-150535992 | NDST1-2580 | chr5:150557631-150557658 |
| NDST1-1235 | chr5:150535966-150535993 | NDST1-2581 | chr5:150557632-150557659 |
| NDST1-1236 | chr5:150535967-150535994 | NDST1-2582 | chr5:150557633-150557660 |
| NDST1-1237 | chr5:150539109-150539136 | NDST1-2583 | chr5:150557636-150557663 |
| NDST1-1238 | chr5:150539112-150539139 | NDST1-2584 | chr5:150557658-150557685 |
| NDST1-1239 | chr5:150539118-150539145 | NDST1-2585 | chr5:150557664-150557691 |
| NDST1-1240 | chr5:150539119-150539146 | NDST1-2586 | chr5:150557667-150557694 |
| NDST1-1241 | chr5:150539126-150539153 | NDST1-2587 | chr5:150557670-150557697 |
| NDST1-1242 | chr5:150539135-150539162 | NDST1-2588 | chr5:150557671-150557698 |
| NDST1-1243 | chr5:150539147-150539174 | NDST1-2589 | chr5:150557672-150557699 |
| NDST1-1244 | chr5:150539154-150539181 | NDST1-2590 | chr5:150557673-150557700 |
| NDST1-1245 | chr5:150539167-150539194 | NDST1-2591 | chr5:150557676-150557703 |
| NDST1-1246 | chr5:150539169-150539196 | NDST1-2592 | chr5:150557677-150557704 |
| NDST1-1247 | chr5:150539172-150539199 | NDST1-2593 | chr5:150557680-150557707 |
| NDST1-1248 | chr5:150539173-150539200 | NDST1-2594 | chr5:150557681-150557708 |
| NDST1-1249 | chr5:150539176-150539203 | NDST1-2595 | chr5:150557684-150557711 |
| NDST1-1250 | chr5:150539179-150539206 | NDST1-2596 | chr5:150557686-150557713 |
| NDST1-1251 | chr5:150539180-150539207 | NDST1-2597 | chr5:150557698-150557725 |
| NDST1-1252 | chr5:150539187-150539214 | NDST1-2598 | chr5:150557699-150557726 |
| NDST1-1253 | chr5:150539197-150539224 | NDST1-2599 | chr5:150557703-150557730 |
| NDST1-1254 | chr5:150539201-150539228 | NDST1-2600 | chr5:150557707-150557734 |
| NDST1-1255 | chr5:150539205-150539232 | NDST1-2601 | chr5:150557725-150557752 |
| NDST1-1256 | chr5:150539210-150539237 | NDST1-2602 | chr5:150557729-150557756 |
| NDST1-1257 | chr5:150539211-150539238 | NDST1-2603 | chr5:150557734-150557761 |
| NDST1-1258 | chr5:150539212-150539239 | NDST1-2604 | chr5:150557735-150557762 |
| NDST1-1259 | chr5:150539213-150539240 | NDST1-2605 | chr5:150557740-150557767 |
| NDST1-1260 | chr5:150539216-150539243 | NDST1-2606 | chr5:150557751-150557778 |
| NDST1-1261 | chr5:150539217-150539244 | NDST1-2607 | chr5:150557752-150557779 |
| NDST1-1262 | chr5:150539218-150539245 | NDST1-2608 | chr5:150557757-150557784 |
| NDST1-1263 | chr5:150539219-150539246 | NDST1-2609 | chr5:150557763-150557790 |
| NDST1-1264 | chr5:150539222-150539249 | NDST1-2610 | chr5:150557764-150557791 |
| NDST1-1265 | chr5:150539223-150539250 | NDST1-2611 | chr5:150557771-150557798 |
| NDST1-1266 | chr5:150539233-150539260 | NDST1-2612 | chr5:150557772-150557799 |
| NDST1-1267 | chr5:150539234-150539261 | NDST1-2613 | chr5:150557773-150557800 |
| NDST1-1268 | chr5:150539244-150539271 | NDST1-2614 | chr5:150557774-150557801 |
| NDST1-1269 | chr5:150539251-150539278 | NDST1-2615 | chr5:150557785-150557812 |
| NDST1-1270 | chr5:150539262-150539289 | NDST1-2616 | chr5:150557792-150557819 |
| NDST1-1271 | chr5:150539265-150539292 | NDST1-2617 | chr5:150557793-150557820 |
| NDST1-1272 | chr5:150539279-150539306 | NDST1-2618 | chr5:150557794-150557821 |
| NDST1-1273 | chr5:150539284-150539311 | NDST1-2619 | chr5:150557795-150557822 |
| NDST1-1274 | chr5:150539285-150539312 | NDST1-2620 | chr5:150557802-150557829 |
| NDST1-1275 | chr5:150539295-150539322 | NDST1-2621 | chr5:150557808-150557835 |
| NDST1-1276 | chr5:150539296-150539323 | NDST1-2622 | chr5:150557815-150557842 |
| NDST1-1277 | chr5:150539297-150539324 | NDST1-2623 | chr5:150557841-150557868 |
| NDST1-1278 | chr5:150539298-150539325 | NDST1-2624 | chr5:150557851-150557878 |
| NDST1-1279 | chr5:150539335-150539362 | NDST1-2625 | chr5:150557855-150557882 |
| NDST1-1280 | chr5:150539340-150539367 | NDST1-2626 | chr5:150557856-150557883 |
| NDST1-1281 | chr5:150539347-150539374 | NDST1-2627 | chr5:150557857-150557884 |
| NDST1-1282 | chr5:150539351-150539378 | NDST1-2628 | chr5:150557859-150557886 |
| NDST1-1283 | chr5:150539354-150539381 | NDST1-2629 | chr5:150557860-150557887 |
| NDST1-1284 | chr5:150539358-150539385 | NDST1-2630 | chr5:150557869-150557896 |
| NDST1-1285 | chr5:150539366-150539393 | NDST1-2631 | chr5:150557870-150557897 |
| NDST1-1286 | chr5:150539367-150539394 | NDST1-2632 | chr5:150557873-150557900 |
| NDST1-1287 | chr5:150539368-150539395 | NDST1-2633 | chr5:150557875-150557902 |
| NDST1-1288 | chr5:150539372-150539399 | NDST1-2634 | chr5:150557878-150557905 |
| NDST1-1289 | chr5:150539373-150539400 | NDST1-2635 | chr5:150557879-150557906 |
| NDST1-1290 | chr5:150539400-150539427 | NDST1-2636 | chr5:150557880-150557907 |
| NDST1-1291 | chr5:150539407-150539434 | NDST1-2637 | chr5:150557918-150557945 |
| NDST1-1292 | chr5:150539408-150539435 | NDST1-2638 | chr5:150557919-150557946 |
| NDST1-1293 | chr5:150539415-150539442 | NDST1-2639 | chr5:150557949-150557976 |
| NDST1-1294 | chr5:150539420-150539447 | NDST1-2640 | chr5:150557961-150557988 |
| NDST1-1295 | chr5:150539421-150539448 | NDST1-2641 | chr5:150557962-150557989 |
| NDST1-1296 | chr5:150539424-150539451 | NDST1-2642 | chr5:150557967-150557994 |
| NDST1-1297 | chr5:150539429-150539456 | NDST1-2643 | chr5:150557972-150557999 |
| NDST1-1298 | chr5:150539431-150539458 | NDST1-2644 | chr5:150557973-150558000 |
| NDST1-1299 | chr5:150539438-150539465 | NDST1-2645 | chr5:150557981-150558008 |
| NDST1-1300 | chr5:150539446-150539473 | NDST1-2646 | chr5:150557984-150558011 |
| NDST1-1301 | chr5:150539453-150539480 | NDST1-2647 | chr5:150557986-150558013 |
| NDST1-1302 | chr5:150539454-150539481 | NDST1-2648 | chr5:150557987-150558014 |
| NDST1-1303 | chr5:150539966-150539993 | NDST1-2649 | chr5:150557990-150558017 |
| NDST1-1304 | chr5:150539970-150539997 | NDST1-2650 | chr5:150557991-150558018 |
| NDST1-1305 | chr5:150539974-150540001 | NDST1-2651 | chr5:150557992-150558019 |
| NDST1-1306 | chr5:150539975-150540002 | NDST1-2652 | chr5:150557993-150558020 |
| NDST1-1307 | chr5:150539983-150540010 | NDST1-2653 | chr5:150557994-150558021 |
| NDST1-1308 | chr5:150539984-150540011 | NDST1-2654 | chr5:150558001-150558028 |
| NDST1-1309 | chr5:150539994-150540021 | NDST1-2655 | chr5:150558002-150558029 |
| NDST1-1310 | chr5:150539997-150540024 | NDST1-2656 | chr5:150558003-150558030 |
| NDST1-1311 | chr5:150540002-150540029 | NDST1-2657 | chr5:150558004-150558031 |
| NDST1-1312 | chr5:150540003-150540030 | NDST1-2658 | chr5:150558011-150558038 |
| NDST1-1313 | chr5:150540006-150540033 | NDST1-2659 | chr5:150558017-150558044 |
| NDST1-1314 | chr5:150540021-150540048 | NDST1-2660 | chr5:150558034-150558061 |
| NDST1-1315 | chr5:150540022-150540049 | NDST1-2661 | chr5:150558035-150558062 |
| NDST1-1316 | chr5:150540049-150540076 | NDST1-2662 | chr5:150558036-150558063 |
| NDST1-1317 | chr5:150540050-150540077 | NDST1-2663 | chr5:150558037-150558064 |
| NDST1-1318 | chr5:150540051-150540078 | NDST1-2664 | chr5:150558038-150558065 |
| NDST1-1319 | chr5:150540055-150540082 | NDST1-2665 | chr5:150558041-150558068 |
| NDST1-1320 | chr5:150540062-150540089 | NDST1-2666 | chr5:150558042-150558069 |
| NDST1-1321 | chr5:150540065-150540092 | NDST1-2667 | chr5:150558043-150558070 |
| NDST1-1322 | chr5:150540066-150540093 | NDST1-2668 | chr5:150558048-150558075 |
| NDST1-1323 | chr5:150540067-150540094 | NDST1-2669 | chr5:150558050-150558077 |
| NDST1-1324 | chr5:150540070-150540097 | NDST1-2670 | chr5:150558051-150558078 |
| NDST1-1325 | chr5:150540071-150540098 | NDST1-2671 | chr5:150558052-150558079 |
| NDST1-1326 | chr5:150540072-150540099 | NDST1-2672 | chr5:150558053-150558080 |
| NDST1-1327 | chr5:150540089-150540116 | NDST1-2673 | chr5:150558057-150558084 |
| NDST1-1328 | chr5:150540090-150540117 | NDST1-2674 | chr5:150558058-150558085 |
| NDST1-1329 | chr5:150540102-150540129 | NDST1-2675 | chr5:150558059-150558086 |
| NDST1-1330 | chr5:150540103-150540130 | NDST1-2676 | chr5:150558060-150558087 |
| NDST1-1331 | chr5:150540104-150540131 | NDST1-2677 | chr5:150558084-150558111 |
| NDST1-1332 | chr5:150540107-150540134 | NDST1-2678 | chr5:150558085-150558112 |
| NDST1-1333 | chr5:150540123-150540150 | NDST1-2679 | chr5:150558094-150558121 |
| NDST1-1334 | chr5:150540126-150540153 | NDST1-2680 | chr5:150558102-150558129 |
| NDST1-1335 | chr5:150540127-150540154 | NDST1-2681 | chr5:150558106-150558133 |
| NDST1-1336 | chr5:150540132-150540159 | NDST1-2682 | chr5:150558107-150558134 |
| NDST1-1337 | chr5:150540140-150540167 | NDST1-2683 | chr5:150558119-150558146 |
| NDST1-1338 | chr5:150540147-150540174 | NDST1-2684 | chr5:150558121-150558148 |
| NDST1-1339 | chr5:150540150-150540177 | NDST1-2685 | chr5:150558125-150558152 |
| NDST1-1340 | chr5:150540159-150540186 | NDST1-2686 | chr5:150558152-150558179 |
| NDST1-1341 | chr5:150540162-150540189 | NDST1-2687 | chr5:150558156-150558183 |
| NDST1-1342 | chr5:150540170-150540197 | NDST1-2688 | chr5:150558159-150558186 |
| NDST1-1343 | chr5:150540180-150540207 | NDST1-2689 | chr5:150558171-150558198 |
| NDST1-1344 | chr5:150540183-150540210 | NDST1-2690 | chr5:150558172-150558199 |
| NDST1-1345 | chr5:150540187-150540214 | NDST1-2691 | chr5:150558173-150558200 |
| NDST1-1346 | chr5:150540199-150540226 | | |

**Table 2M Genomic coordinates of the human NLRP1 gene**

| Target ID | Target Coordinate Range | Target ID | Target Coordinate Range |
|---|---|---|---|
| NLRP1-1 | chr17:5501371-5501398 | NLRP1-977 | chr17:5553352-5553379 |
| NLRP1-2 | chr17:5501455-5501482 | NLRP1-978 | chr17:5553353-5553380 |
| NLRP1-3 | chr17:5501495-5501522 | NLRP1-979 | chr17:5553364-5553391 |
| NLRP1-4 | chr17:5501505-5501532 | NLRP1-980 | chr17:5553365-5553392 |
| NLRP1-5 | chr17:5501508-5501535 | NLRP1-981 | chr17:5553370-5553397 |
| NLRP1-6 | chr17:5501509-5501536 | NLRP1-982 | chr17:5553371-5553398 |
| NLRP1-7 | chr17:5501516-5501543 | NLRP1-983 | chr17:5553373-5553400 |
| NLRP1-8 | chr17:5501517-5501544 | NLRP1-984 | chr17:5553376-5553403 |
| NLRP1-9 | chr17:5501523-5501550 | NLRP1-985 | chr17:5553384-5553411 |
| NLRP1-10 | chr17:5501524-5501551 | NLRP1-986 | chr17:5553385-5553412 |
| NLRP1-11 | chr17:5501563-5501590 | NLRP1-987 | chr17:5553394-5553421 |
| NLRP1-12 | chr17:5501570-5501597 | NLRP1-988 | chr17:5553395-5553422 |
| NLRP1-13 | chr17:5501590-5501617 | NLRP1-989 | chrl7:5553396-5553423 |
| NLRP1-14 | chr17:5501614-5501641 | NLRP1-990 | chr17:5553424-5553451 |
| NLRP 1-15 | chr17:5501624-5501651 | NLRP1-991 | chr17:5553445-5553472 |
| NLRP1-16 | chr17:5501638-5501665 | NLRP1-992 | chr17:5553460-5553487 |
| NLRP1-17 | chr17:5501639-5501666 | NLRP1-993 | chr17:5553464-5553491 |
| NLRP1-18 | chr17:5501655-5501682 | NLRP1-994 | chr17:5553467-5553494 |
| NLRP1-19 | chr17:5501659-5501686 | NLRP1-995 | chr17:5553474-5553501 |
| NLRP1-20 | chr17:5501694-5501721 | NLRP1-996 | chr17:5553478-5553505 |
| NLRP1-21 | chr17:5501704-5501731 | NLRP1-997 | chr17:5553480-5553507 |
| NLRP1-22 | chr17:5501708-5501735 | NLRP1-998 | chr17:5553482-5553509 |
| NLRP1-23 | chr17:5501713-5501740 | NLRP1-999 | chr17:5553498-5553525 |
| NLRP1-24 | chr17:5501715-5501742 | NLRP1-1000 | chr17:5553503-5553530 |
| NLRP1-25 | chr17:5501719-5501746 | NLRP1-1001 | chr17:5553516-5553543 |
| NLRP1-26 | chr17:5501720-5501747 | NLRP1-1002 | chr17:5553517-5553544 |
| NLRP1-27 | chr17:5501725-5501752 | NLRP1-1003 | chr17:5553523-5553550 |
| NLRP1-28 | chr17:5501732-5501759 | NLRP1-1004 | chr17:5553535-5553562 |
| NLRP1-29 | chr17:5501733-5501760 | NLRP1-1005 | chr17:5553536-5553563 |
| NLRP1-30 | chr17:5501736-5501763 | NLRP1-1006 | chr17:5553537-5553564 |
| NLRP1-31 | chr17:5501753-5501780 | NLRP1-1007 | chr17:5553538-5553565 |
| NLRP1-32 | chr17:5501755-5501782 | NLRP1-1008 | chr17:5553546-5553573 |
| NLRP1-33 | chr17:5501756-5501783 | NLRP1-1009 | chr17:5553547-5553574 |
| NLRP1-34 | chr17:5501764-5501791 | NLRP1-1010 | chr17:5553548-5553575 |
| NLRP1-35 | chr17:5501765-5501792 | NLRP1-1011 | chr17:5553550-5553577 |
| NLRP1-36 | chr17:5501768-5501795 | NLRP1-1012 | chr17:5553553-5553580 |
| NLRP1-37 | chr17:5501773-5501800 | NLRP1-1013 | chr17:5553556-5553583 |
| NLRP1-38 | chr17:5501774-5501801 | NLRP1-1014 | chr17:5553572-5553599 |
| NLRP1-39 | chr17:5501776-5501803 | NLRP1-1015 | chr17:5553576-5553603 |
| NLRP1-40 | chr17:5501783-5501810 | NLRP1-1016 | chr17:5553577-5553604 |
| NLRP1-41 | chr17:5501784-5501811 | NLRP1-1017 | chr17:5553578-5553605 |
| NLRP1-42 | chr17:5501797-5501824 | NLRP1-1018 | chr17:5553585-5553612 |
| NLRP1-43 | chr17:5501800-5501827 | NLRP1-1019 | chr17:5553611-5553638 |
| NLRP1-44 | chr17:5501820-5501847 | NLRP1-1020 | chr17:5553612-5553639 |
| NLRP1-45 | chr17:5501823-5501850 | NLRP1-1021 | chr17:5553615-5553642 |
| NLRP1-46 | chr17:5501828-5501855 | NLRP1-1022 | chr17:5553616-5553643 |
| NLRP1-47 | chr17:5501832-5501859 | NLRP1-1023 | chr17:5553620-5553647 |
| NLRP1-48 | chr17:5501836-5501863 | NLRP1-1024 | chr17:5553621-5553648 |
| NLRP1-49 | chr17:5501841-5501868 | NLRP1-1025 | chr17:5553634-5553661 |
| NLRP1-50 | chr17:5501849-5501876 | NLRP1-1026 | chr17:5553643-5553670 |
| NLRP1-51 | chr17:5501850-5501877 | NLRP1-1027 | chr17:5553644-5553671 |
| NLRP1-52 | chr17:5501854-5501881 | NLRP1-1028 | chr17:5553646-5553673 |
| NLRP1-53 | chr17:5501857-5501884 | NLRP1-1029 | chr17:5553647-5553674 |
| NLRP1-54 | chr17:5501858-5501885 | NLRP1-1030 | chr17:5553648-5553675 |
| NLRP1-55 | chr17:5501868-5501895 | NLRP1-1031 | chr17:5553649-5553676 |
| NLRP1-56 | chr17:5501872-5501899 | NLRP1-1032 | chr17:5558218-5558245 |
| NLRP1-57 | chr17:5501873-5501900 | NLRP1-1033 | chr17:5558219-5558246 |
| NLRP1-58 | chr17:5501898-5501925 | NLRP1-1034 | chr17:5558220-5558247 |
| NLRP1-59 | chr17:5501903-5501930 | NLRP1-1035 | chr17:5558225-5558252 |
| NLRP1-60 | chr17:5501911-5501938 | NLRP1-1036 | chr17:5558232-5558259 |
| NLRP1-61 | chr17:5501913-5501940 | NLRP1-1037 | chr17:5558235-5558262 |
| NLRP1-62 | chr17:5501914-5501941 | NLRP1-1038 | chr17:5558236-5558263 |
| NLRP1-63 | chr17:5501915-5501942 | NLRP1-1039 | chr17:5558237-5558264 |
| NLRP1-64 | chr17:5501932-5501959 | NLRP1-1040 | chr17:5558238-5558265 |
| NLRP1-65 | chr17:5501939-5501966 | NLRP1-1041 | chr17:5558241-5558268 |
| NLRP1-66 | chr17:5501963-5501990 | NLRP1-1042 | chr17:5558242-5558269 |
| NLRP1-67 | chr17:5514093-5514120 | NLRP1-1043 | chr17:5558243-5558270 |
| NLRP1-68 | chr17:5514096-5514123 | NLRP1-1044 | chr17:5558244-5558271 |
| NLRP1-69 | chr17:5514099-5514126 | NLRP1-1045 | chr17:5558245-5558272 |
| NLRP1-70 | chr17:5514114-5514141 | NLRP1-1046 | chr17:5558248-5558275 |
| NLRP1-71 | chr17:5514139-5514166 | NLRP1-1047 | chr17:5558253-5558280 |
| NLRP1-72 | chr17:5514184-5514211 | NLRP1-1048 | chr17:5558261-5558288 |
| NLRP1-73 | chr17:5514185-5514212 | NLRP1-1049 | chr17:5558262-5558289 |
| NLRP1-74 | chr17:5514186-5514213 | NLRP1-1050 | chr17:5558266-5558293 |
| NLRP1-75 | chr17:5514194-5514221 | NLRP1-1051 | chr17:5558269-5558296 |
| NLRP1-76 | chr17:5514204-5514231 | NLRP1-1052 | chr17:5558280-5558307 |
| NLRP1-77 | chr17:5514236-5514263 | NLRP1-1053 | chr17:5558294-5558321 |
| NLRP1-78 | chr17:5514270-5514297 | NLRP1-1054 | chr17:5558295-5558322 |
| NLRP1-79 | chr17:5514285-5514312 | NLRP1-1055 | chr17:5558298-5558325 |
| NLRP1-80 | chr17:5514292-5514319 | NLRP1-1056 | chr17:5558303-5558330 |
| NLRP1-81 | chr17:5514296-5514323 | NLRP1-1057 | chr17:5558304-5558331 |
| NLRP1-82 | chr17:5514330-5514357 | NLRP1-1058 | chr17:5558314-5558341 |
| NLRP1-83 | chr17:5514331-5514358 | NLRP1-1059 | chr17:5558324-5558351 |
| NLRP1-84 | chr17:5514342-5514369 | NLRP1-1060 | chr17:5558327-5558354 |
| NLRP1-85 | chr17:5514350-5514377 | NLRP1-1061 | chr17:5558328-5558355 |
| NLRP1-86 | chr17:5514356-5514383 | NLRP1-1062 | chr17:5558329-5558356 |
| NLRP1-87 | chr17:5514357-5514384 | NLRP1-1063 | chr17:5558346-5558373 |
| NLRP1-88 | chr17:5514358-5514385 | NLRP1-1064 | chr17:5558359-5558386 |
| NLRP1-89 | chr17:5514361-5514388 | NLRP1-1065 | chr17:5558377-5558404 |
| NLRP1-90 | chr17:5514362-5514389 | NLRP1-1066 | chr17:5558381-5558408 |
| NLRP1-91 | chr17:5514372-5514399 | NLRP1-1067 | chr17:5558382-5558409 |
| NLRP1-92 | chr17:5514373-5514400 | NLRP1-1068 | chr17:5558383-5558410 |
| NLRP1-93 | chr17:5514374-5514401 | NLRP1-1069 | chr17:5558386-5558413 |
| NLRP1-94 | chr17:5514382-5514409 | NLRP1-1070 | chr17:5558448-5558475 |
| NLRP1-95 | chr17:5514389-5514416 | NLRP1-1071 | chr17:5558461-5558488 |
| NLRP1-96 | chr17:5514390-5514417 | NLRP1-1072 | chr17:5558462-5558489 |
| NLRP1-97 | chr17:5514391-5514418 | NLRP1-1073 | chr17:5558471-5558498 |
| NLRP1-98 | chr17:5514403-5514430 | NLRP1-1074 | chr17:5558472-5558499 |
| NLRP1-99 | chr17:5514419-5514446 | NLRP1-1075 | chr17:5558479-5558506 |
| NLRP1-100 | chr17:5514422-5514449 | NLRP1-1076 | chr17:5558490-5558517 |
| NLRP1-101 | chr17:5514425-5514452 | NLRP1-1077 | chr17:5558512-5558539 |
| NLRP1-102 | chr17:5514426-5514453 | NLRP1-1078 | chr17:5558515-5558542 |
| NLRP1-103 | chr17:5514427-5514454 | NLRP1-1079 | chr17:5558516-5558543 |
| NLRP1-104 | chr17:5514429-5514456 | NLRP1-1080 | chr17:5558518-5558545 |
| NLRP1-105 | chr17:5514433-5514460 | NLRP1-1081 | chr17:5558532-5558559 |
| NLRP1-106 | chr17:5514435-5514462 | NLRP1-1082 | chr17:5558547-5558574 |
| NLRP1-107 | chr17:5514436-5514463 | NLRP1-1083 | chr17:5558551-5558578 |
| NLRP1-108 | chr17:5514437-5514464 | NLRP1-1084 | chr17:5558552-5558579 |
| NLRP1-109 | chr17:5514444-5514471 | NLRP1-1085 | chr17:5558556-5558583 |
| NLRP1-110 | chr17:5514445-5514472 | NLRP1-1086 | chr17:5558557-5558584 |
| NLRP1-111 | chr17:5514449-5514476 | NLRP1-1087 | chr17:5558572-5558599 |
| NLRP1-112 | chr17:5514457-5514484 | NLRP1-1088 | chr17:5558586-5558613 |
| NLRP1-113 | chr17:5514458-5514485 | NLRP1-1089 | chr17:5558587-5558614 |
| NLRP1-114 | chr17:5514471-5514498 | NLRP1-1090 | chr17:5558593-5558620 |
| NLRP1-115 | chr17:5514481-5514508 | NLRP1-1091 | chr17:5558602-5558629 |
| NLRP1-116 | chr17:5514486-5514513 | NLRP1-1092 | chr17:5558605-5558632 |
| NLRP1-117 | chr17:5514489-5514516 | NLRP1-1093 | chr17:5558606-5558633 |
| NLRP1-118 | chr17:5514490-5514517 | NLRP1-1094 | chr17:5558607-5558634 |
| NLRP1-119 | chr17:5514491-5514518 | NLRP1-1095 | chr17:5558617-5558644 |
| NLRP1-120 | chr17:5514510-5514537 | NLRP1-1096 | chr17:5558637-5558664 |
| NLRP1-121 | chr17:5514524-5514551 | NLRP1-1097 | chr17:5558641-5558668 |
| NLRP1-122 | chr17:5514530-5514557 | NLRP1-1098 | chr17:5558649-5558676 |
| NLRP1-123 | chr17:5514533-5514560 | NLRP1-1099 | chr17:5558650-5558677 |
| NLRP1-124 | chr17:5514541-5514568 | NLRP1-1100 | chr17:5558651-5558678 |
| NLRP1-125 | chr17:5514543-5514570 | NLRP1-1101 | chr17:5558652-5558679 |
| NLRP1-126 | chr17:5514544-5514571 | NLRP1-1102 | chr17:5558660-5558687 |
| NLRP1-127 | chr17:5514546-5514573 | NLRP1-1103 | chr17:5558669-5558696 |
| NLRP1-128 | chr17:5514547-5514574 | NLRP1-1104 | chr17:5558670-5558697 |
| NLRP1-129 | chr17:5514548-5514575 | NLRP1-1105 | chr17:5558677-5558704 |
| NLRP1-130 | chr17:5514558-5514585 | NLRP1-1106 | chr17:5558694-5558721 |
| NLRP1-131 | chr17:5514561-5514588 | NLRP1-1107 | chr17:5558695-5558722 |
| NLRP1-132 | chr17:5514564-5514591 | NLRP1-1108 | chr17:5558696-5558723 |
| NLRP1-133 | chr17:5514567-5514594 | NLRP1-1109 | chr17:5558705-5558732 |
| NLRP1-134 | chr17:5514572-5514599 | NLRP1-1110 | chr17:5558714-5558741 |
| NLRP1-135 | chr17:5514576-5514603 | NLRP1-1111 | chr17:5558736-5558763 |
| NLRP1-136 | chr17:5514577-5514604 | NLRP1-1112 | chr17:5558765-5558792 |
| NLRP1-137 | chr17:5514578-5514605 | NLRP1-1113 | chr17:5558768-5558795 |
| NLRP1-138 | chr17:5514579-5514606 | NLRP1-1114 | chr17:5558770-5558797 |
| NLRP1-139 | chr17:5514589-5514616 | NLRP1-1115 | chr17:5558771-5558798 |
| NLRP1-140 | chr17:5514590-5514617 | NLRP1-1116 | chr17:5558772-5558799 |
| NLRP1-141 | chr17:5514593-5514620 | NLRP1-1117 | chr17:5558781-5558808 |
| NLRP1-142 | chr17:5514610-5514637 | NLRP1-1118 | chr17:5558784-5558811 |
| NLRP1-143 | chr17:5514611-5514638 | NLRP1-1119 | chr17:5558791-5558818 |
| NLRP1-144 | chr17:5514624-5514651 | NLRP1-1120 | chr17:5558800-5558827 |
| NLRP1-145 | chr17:5514628-5514655 | NLRP1-1121 | chr17:5558823-5558850 |
| NLRP1-146 | chr17:5514634-5514661 | NLRP1-1122 | chr17:5558824-5558851 |
| NLRP1-147 | chr17:5514640-5514667 | NLRP1-1123 | chr17:5558828-5558855 |
| NLRP1-148 | chr17:5514660-5514687 | NLRP1-1124 | chr17:5558829-5558856 |
| NLRP1-149 | chr17:5514680-5514707 | NLRP1-1125 | chr17:5558830-5558857 |
| NLRP1-150 | chr17:5514681-5514708 | NLRP1-1126 | chr17:5558861-5558888 |
| NLRP1-151 | chr17:5514696-5514723 | NLRP1-1127 | chr17:5558864-5558891 |
| NLRP1-152 | chr17:5514697-5514724 | NLRP1-1128 | chr17:5558873-5558900 |
| NLRP1-153 | chr17:5514698-5514725 | NLRP1-1129 | chr17:5558874-5558901 |
| NLRP1-154 | chr17:5514704-5514731 | NLRP1-1130 | chr17:5558899-5558926 |
| NLRP1-155 | chr17:5514705-5514732 | NLRP1-1131 | chr17:5558901-5558928 |
| NLRP1-156 | chr17:5514711-5514738 | NLRP1-1132 | chr17:5558902-5558929 |
| NLRP1-157 | chr17:5514712-5514739 | NLRP1-1133 | chr17:5558903-5558930 |
| NLRP1-158 | chr17:5514713-5514740 | NLRP1-1134 | chr17:5558907-5558934 |
| NLRP1-159 | chr17:5514716-5514743 | NLRP1-1135 | chr17:5558911-5558938 |
| NLRP1-160 | chr17:5514719-5514746 | NLRP1-1136 | chr17:5558912-5558939 |
| NLRP1-161 | chr17:5514741-5514768 | NLRP1-1137 | chr17:5558917-5558944 |
| NLRP1-162 | chr17:5514745-5514772 | NLRP1-1138 | chr17:5558918-5558945 |
| NLRP1-163 | chr17:5514776-5514803 | NLRP1-1139 | chr17:5558920-5558947 |
| NLRP1-164 | chr17:5514777-5514804 | NLRP1-1140 | chr17:5558956-5558983 |
| NLRP1-165 | chr17:5514787-5514814 | NLRP1-1141 | chr17:5558959-5558986 |
| NLRP1-166 | chr17:5514788-5514815 | NLRP1-1142 | chr17:5558963-5558990 |
| NLRP1-167 | chr17:5514789-5514816 | NLRP1-1143 | chr17:5558964-5558991 |
| NLRP1-168 | chr17:5514792-5514819 | NLRP1-1144 | chr17:5558971-5558998 |
| NLRP1-169 | chr17:5514795-5514822 | NLRP1-1145 | chr17:5558972-5558999 |
| NLRP1-170 | chr17:5514796-5514823 | NLRP1-1146 | chr17:5558973-5559000 |
| NLRP1-171 | chr17:5514797-5514824 | NLRP1-1147 | chr17:5558982-5559009 |
| NLRP1-172 | chr17:5514804-5514831 | NLRP1-1148 | chr17:5558992-5559019 |
| NLRP1-173 | chr17:5514808-5514835 | NLRP1-1149 | chr17:5559001-5559028 |
| NLRP1-174 | chr17:5514809-5514836 | NLRP1-1150 | chr17:5559002-5559029 |
| NLRP1-175 | chr17:5514814-5514841 | NLRP1-1151 | chr17:5559003-5559030 |
| NLRP1-176 | chr17:5514828-5514855 | NLRP1-1152 | chr17:5559007-5559034 |
| NLRP1-177 | chr17:5514838-5514865 | NLRP1-1153 | chr17:5559022-5559049 |
| NLRP1-178 | chr17:5514845-5514872 | NLRP1-1154 | chr17:5559023-5559050 |
| NLRP1-179 | chr17:5514848-5514875 | NLRP1-1155 | chr17:5559024-5559051 |
| NLRP1-180 | chr17:5514850-5514877 | NLRP1-1156 | chr17:5559029-5559056 |
| NLRP1-181 | chr17:5514862-5514889 | NLRP1-1157 | chr17:5559032-5559059 |
| NLRP1-182 | chr17:5514867-5514894 | NLRP1-1158 | chr17:5559038-5559065 |
| NLRP1-183 | chr17:5514868-5514895 | NLRP1-1159 | chr17:5559082-5559109 |
| NLRP1-184 | chr17:5514877-5514904 | NLRP1-1160 | chr17:5559084-5559111 |
| NLRP1-185 | chr17:5514881-5514908 | NLRP1-1161 | chr17:5559088-5559115 |
| NLRP1-186 | chr17:5514882-5514909 | NLRP1-1162 | chr17:5559092-5559119 |
| NLRP1-187 | chr17:5514895-5514922 | NLRP1-1163 | chr17:5559101-5559128 |
| NLRP1-188 | chr17:5514910-5514937 | NLRP1-1164 | chr17:5559110-5559137 |
| NLRP1-189 | chr17:5514922-5514949 | NLRP1-1165 | chr17:5559111-5559138 |
| NLRP1-190 | chr17:5514924-5514951 | NLRP1-1166 | chr17:5559120-5559147 |
| NLRP1-191 | chr17:5514930-5514957 | NLRP1-1167 | chr17:5559124-5559151 |
| NLRP1-192 | chr17:5514931-5514958 | NLRP1-1168 | chr17:5559125-5559152 |
| NLRP1-193 | chr17:5514945-5514972 | NLRP1-1169 | chr17:5559132-5559159 |
| NLRP1-194 | chr17:5514957-5514984 | NLRP1-1170 | chr17:5559133-5559160 |
| NLRP1-195 | chr17:5514962-5514989 | NLRP1-1171 | chr17:5559153-5559180 |
| NLRP1-196 | chr17:5514975-5515002 | NLRP1-1172 | chr17:5559154-5559181 |
| NLRP1-197 | chr17:5514976-5515003 | NLRP1-1173 | chr17:5559161-5559188 |
| NLRP1-198 | chr17:5514977-5515004 | NLRP1-1174 | chr17:5559177-5559204 |
| NLRP1-199 | chr17:5514984-5515011 | NLRP1-1175 | chr17:5559181-5559208 |
| NLRP1-200 | chr17:5514987-5515014 | NLRP1-1176 | chr17:5559202-5559229 |
| NLRP1-201 | chr17:5514990-5515017 | NLRP1-1177 | chr17:5559218-5559245 |
| NLRP1-202 | chr17:5515000-5515027 | NLRP1-1178 | chr17:5559234-5559261 |
| NLRP1-203 | chr17:5515023-5515050 | NLRP1-1179 | chr17:5559235-5559262 |
| NLRP1-204 | chr17:5515024-5515051 | NLRP1-1180 | chr17:5559238-5559265 |
| NLRP1-205 | chr17:5515025-5515052 | NLRP1-1181 | chr17:5559256-5559283 |
| NLRP1-206 | chr17:5515029-5515056 | NLRP1-1182 | chr17:5559257-5559284 |
| NLRP1-207 | chr17:5515035-5515062 | NLRP1-1183 | chr17:5559258-5559285 |
| NLRP1-208 | chr17:5515041-5515068 | NLRP1-1184 | chr17:5559264-5559291 |
| NLRP1-209 | chr17:5515046-5515073 | NLRP1-1185 | chr17:5559267-5559294 |
| NLRP1-210 | chr17:5515050-5515077 | NLRP1-1186 | chr17:5559270-5559297 |
| NLRP1-211 | chr17:5515056-5515083 | NLRP1-1187 | chr17:5559271-5559298 |
| NLRP1-212 | chr17:5515066-5515093 | NLRP1-1188 | chr17:5559274-5559301 |
| NLRP1-213 | chr17:5515067-5515094 | NLRP1-1189 | chr17:5559279-5559306 |
| NLRP1-214 | chr17:5515097-5515124 | NLRP1-1190 | chr17:5559285-5559312 |
| NLRP1-215 | chr17:5515101-5515128 | NLRP1-1191 | chr17:5559290-5559317 |
| NLRP1-216 | chr17:5515107-5515134 | NLRP1-1192 | chr17:5559307-5559334 |
| NLRP1-217 | chr17:5515110-5515137 | NLRP1-1193 | chr17:5559311-5559338 |
| NLRP1-218 | chr17:5515113-5515140 | NLRP1-1194 | chr17:5559319-5559346 |
| NLRP1-219 | chr17:5515116-5515143 | NLRP1-1195 | chr17:5559320-5559347 |
| NLRP1-220 | chr17:5515121-5515148 | NLRP1-1196 | chr17:5559321-5559348 |
| NLRP1-221 | chr17:5515122-5515149 | NLRP1-1197 | chr17:5559324-5559351 |
| NLRP1-222 | chr17:5515123-5515150 | NLRP1-1198 | chr17:5559342-5559369 |
| NLRP1-223 | chr17:5515126-5515153 | NLRP1-1199 | chr17:5559361-5559388 |
| NLRP1-224 | chr17:5515139-5515166 | NLRP1-1200 | chr17:5559362-5559389 |
| NLRP1-225 | chr17:5515157-5515184 | NLRP1-1201 | chr17:5559363-5559390 |
| NLRP1-226 | chr17:5515158-5515185 | NLRP1-1202 | chr17:5559366-5559393 |
| NLRP1-227 | chr17:5515166-5515193 | NLRP1-1203 | chr17:5559372-5559399 |
| NLRP1-228 | chr17:5515173-5515200 | NLRP1-1204 | chr17:5559374-5559401 |
| NLRP1-229 | chr17:5515346-5515373 | NLRP1-1205 | chr17:5559375-5559402 |
| NLRP1-230 | chr17:5515352-5515379 | NLRP1-1206 | chr17:5559376-5559403 |
| NLRP1-231 | chr17:5515384-5515411 | NLRP1-1207 | chr17:5559387-5559414 |
| NLRP1-232 | chr17:5515385-5515412 | NLRP1-1208 | chr17:5559390-5559417 |
| NLRP1-233 | chr17:5515389-5515416 | NLRP1-1209 | chr17:5559405-5559432 |
| NLRP1-234 | chr17:5515401-5515428 | NLRP1-1210 | chr17:5559406-5559433 |
| NLRP1-235 | chr17:5515402-5515429 | NLRP1-1211 | chr17:5559426-5559453 |
| NLRP1-236 | chr17:5515406-5515433 | NLRP1-1212 | chr17:5559435-5559462 |
| NLRP1-237 | chr17:5515407-5515434 | NLRP1-1213 | chr17:5559444-5559471 |
| NLRP1-238 | chr17:5515408-5515435 | NLRP1-1214 | chr17:5559445-5559472 |
| NLRP1-239 | chr17:5515409-5515436 | NLRP1-1215 | chr17:5559449-5559476 |
| NLRP1-240 | chr17:5515415-5515442 | NLRP1-1216 | chr17:5559464-5559491 |
| NLRP1-241 | chr17:5515424-5515451 | NLRP1-1217 | chr17:5559465-5559492 |
| NLRP1-242 | chr17:5515425-5515452 | NLRP1-1218 | chr17:5559475-5559502 |
| NLRP1-243 | chr17:5515426-5515453 | NLRP1-1219 | chr17:5559484-5559511 |
| NLRP1-244 | chr17:5515427-5515454 | NLRP1-1220 | chr17:5559488-5559515 |
| NLRP1-245 | chr17:5515436-5515463 | NLRP1-1221 | chr17:5559489-5559516 |
| NLRP1-246 | chr17:5515439-5515466 | NLRP1-1222 | chr17:5559493-5559520 |
| NLRP1-247 | chr17:5515442-5515469 | NLRP1-1223 | chr17:5559495-5559522 |
| NLRP1-248 | chr17:5515445-5515472 | NLRP1-1224 | chr17:5559500-5559527 |
| NLRP1-249 | chr17:5515453-5515480 | NLRP1-1225 | chr17:5559519-5559546 |
| NLRP1-250 | chr17:5515454-5515481 | NLRP1-1226 | chr17:5559532-5559559 |
| NLRP1-251 | chr17:5515458-5515485 | NLRP1-1227 | chr17:5559533-5559560 |
| NLRP1-252 | chr17:5515461-5515488 | NLRP1-1228 | chr17:5559542-5559569 |
| NLRP1-253 | chr17:5515462-5515489 | NLRP1-1229 | chr17:5559545-5559572 |
| NLRP1-254 | chr17:5515472-5515499 | NLRP1-1230 | chr17:5559550-5559577 |
| NLRP1-255 | chr17:5515479-5515506 | NLRP1-1231 | chr17:5559551-5559578 |
| NLRP1-256 | chr17:5515494-5515521 | NLRP1-1232 | chr17:5559564-5559591 |
| NLRP1-257 | chr17:5515497-5515524 | NLRP1-1233 | chr17:5559567-5559594 |
| NLRP1-258 | chr17:5515517-5515544 | NLRP1-1234 | chr17:5559579-5559606 |
| NLRP1-259 | chr17:5515550-5515577 | NLRP1-1235 | chr17:5559586-5559613 |
| NLRP1-260 | chr17:5515582-5515609 | NLRP1-1236 | chr17:5559592-5559619 |
| NLRP1-261 | chr17:5515585-5515612 | NLRP1-1237 | chr17:5559607-5559634 |
| NLRP1-262 | chr17:5515592-5515619 | NLRP1-1238 | chr17:5559620-5559647 |
| NLRP1-263 | chr17:5515593-5515620 | NLRP1-1239 | chr17:5559621-5559648 |
| NLRP1-264 | chr17:5517628-5517655 | NLRP1-1240 | chr17:5559622-5559649 |
| NLRP1-265 | chr17:5517629-5517656 | NLRP1-1241 | chr17:5559623-5559650 |
| NLRP1-266 | chr17:5517630-5517657 | NLRP1-1242 | chr17:5559635-5559662 |
| NLRP1-267 | chr17:5517634-5517661 | NLRP1-1243 | chr17:5559638-5559665 |
| NLRP1-268 | chr17:5517637-5517664 | NLRP1-1244 | chr17:5559640-5559667 |
| NLRP1-269 | chr17:5517640-5517667 | NLRP1-1245 | chr17:5559641-5559668 |
| NLRP1-270 | chr17:5517643-5517670 | NLRP1-1246 | chr17:5559642-5559669 |
| NLRP1-271 | chr17:5517644-5517671 | NLRP1-1247 | chr17:5559643-5559670 |
| NLRP1-272 | chr17:5517671-5517698 | NLRP1-1248 | chr17:5559651-5559678 |
| NLRP1-273 | chr17:5517679-5517706 | NLRP1-1249 | chr17:5559657-5559684 |
| NLRP1-274 | chr17:5517680-5517707 | NLRP1-1250 | chr17:5559661-5559688 |
| NLRP1-275 | chr17:5517686-5517713 | NLRP1-1251 | chr17:5559666-5559693 |
| NLRP1-276 | chr17:5517701-5517728 | NLRP1-1252 | chr17:5559679-5559706 |
| NLRP1-277 | chr17:5517706-5517733 | NLRP1-1253 | chr17:5559680-5559707 |
| NLRP1-278 | chr17:5517709-5517736 | NLRP1-1254 | chr17:5559686-5559713 |
| NLRP1-279 | chr17:5517710-5517737 | NLRP1-1255 | chr17:5559690-5559717 |
| NLRP1-280 | chr17:5517712-5517739 | NLRP1-1256 | chr17:5559693-5559720 |
| NLRP1-281 | chr17:5517713-5517740 | NLRP1-1257 | chr17:5559694-5559721 |
| NLRP1-282 | chr17:5517722-5517749 | NLRP1-1258 | chr17:5559695-5559722 |
| NLRP1-283 | chr17:5517733-5517760 | NLRP1-1259 | chr17:5559696-5559723 |
| NLRP1-284 | chr17:5517735-5517762 | NLRP1-1260 | chr17:5559697-5559724 |
| NLRP1-285 | chr17:5517745-5517772 | NLRP1-1261 | chr17:5559698-5559725 |
| NLRP1-286 | chr17:5517755-5517782 | NLRP1-1262 | chr17:5559706-5559733 |
| NLRP1-287 | chr17:5517761-5517788 | NLRP1-1263 | chr17:5559711-5559738 |
| NLRP1-288 | chr17:5517764-5517791 | NLRP1-1264 | chr17:5559712-5559739 |
| NLRP1-289 | chr17:5517765-5517792 | NLRP1-1265 | chr17:5559729-5559756 |
| NLRP1-290 | chr17:5517770-5517797 | NLRP1-1266 | chr17:5559734-5559761 |
| NLRP1-291 | chr17:5517798-5517825 | NLRP1-1267 | chr17:5559740-5559767 |
| NLRP1-292 | chr17:5517804-5517831 | NLRP1-1268 | chr17:5559768-5559795 |
| NLRP1-293 | chr17:5517810-5517837 | NLRP1-1269 | chr17:5559770-5559797 |
| NLRP1-294 | chr17:5517811-5517838 | NLRP1-1270 | chr17:5559780-5559807 |
| NLRP1-295 | chr17:5517817-5517844 | NLRP1-1271 | chr17:5559783-5559810 |
| NLRP1-296 | chr17:5517818-5517845 | NLRP1-1272 | chr17:5559787-5559814 |
| NLRP1-297 | chr17:5517825-5517852 | NLRP1-1273 | chr17:5559788-5559815 |
| NLRP1-298 | chr17:5517826-5517853 | NLRP1-1274 | chr17:5559789-5559816 |
| NLRP1-299 | chr17:5517836-5517863 | NLRP1-1275 | chr17:5559796-5559823 |
| NLRP1-300 | chr17:5517837-5517864 | NLRP1-1276 | chr17:5559803-5559830 |
| NLRP1-301 | chr17:5517839-5517866 | NLRP1-1277 | chr17:5559804-5559831 |
| NLRP1-302 | chr17:5517859-5517886 | NLRP1-1278 | chr17:5559805-5559832 |
| NLRP1-303 | chr17:5517869-5517896 | NLRP1-1279 | chr17:5559810-5559837 |
| NLRP1-304 | chr17:5517881-5517908 | NLRP1-1280 | chr17:5559844-5559871 |
| NLRP1-305 | chr17:5517887-5517914 | NLRP1-1281 | chr17:5559866-5559893 |
| NLRP1-306 | chr17:5517906-5517933 | NLRP1-1282 | chr17:5559867-5559894 |
| NLRP1-307 | chr17:5517910-5517937 | NLRP1-1283 | chr17:5559875-5559902 |
| NLRP1-308 | chr17:5517912-5517939 | NLRP1-1284 | chr17:5559879-5559906 |
| NLRP1-309 | chr17:5517915-5517942 | NLRP1-1285 | chr17:5559883-5559910 |
| NLRP1-310 | chr17:5517916-5517943 | NLRP1-1286 | chr17:5559889-5559916 |
| NLRP1-311 | chr17:5517936-5517963 | NLRP1-1287 | chr17:5559895-5559922 |
| NLRP1-312 | chr17:5517943-5517970 | NLRP1-1288 | chr17:5559900-5559927 |
| NLRP1-313 | chr17:5517944-5517971 | NLRP1-1289 | chr17:5559909-5559936 |
| NLRP1-314 | chr17:5517945-5517972 | NLRP1-1290 | chr17:5559910-5559937 |
| NLRP1-315 | chr17:5517946-5517973 | NLRP1-1291 | chr17:5559913-5559940 |
| NLRP1-316 | chr17:5517948-5517975 | NLRP1-1292 | chr17:5559916-5559943 |
| NLRP1-317 | chr17:5517955-5517982 | NLRP1-1293 | chr17:5559919-5559946 |
| NLRP1-318 | chr17:5517956-5517983 | NLRP1-1294 | chr17:5559920-5559947 |
| NLRP1-319 | chr17:5517964-5517991 | NLRP1-1295 | chr17:5559921-5559948 |
| NLRP1-320 | chr17:5517965-5517992 | NLRP1-1296 | chr17:5559928-5559955 |
| NLRP1-321 | chr17:5517976-5518003 | NLRP1-1297 | chr17:5559930-5559957 |
| NLRP1-322 | chr17:5520754-5520781 | NLRP1-1298 | chr17:5559931-5559958 |
| NLRP1-323 | chr17:5520755-5520782 | NLRP1-1299 | chr17:5559932-5559959 |
| NLRP1-324 | chr17:5520774-5520801 | NLRP1-1300 | chr17:5559945-5559972 |
| NLRP1-325 | chr17:5520785-5520812 | NLRP1-1301 | chr17:5559946-5559973 |
| NLRP1-326 | chr17:5520790-5520817 | NLRP1-1302 | chr17:5559947-5559974 |
| NLRP1-327 | chr17:5520791-5520818 | NLRP1-1303 | chr17:5559948-5559975 |
| NLRP1-328 | chr17:5520794-5520821 | NLRP1-1304 | chr17:5559966-5559993 |
| NLRP1-329 | chr17:5520801-5520828 | NLRP1-1305 | chr17:5559972-5559999 |
| NLRP1-330 | chr17:5520802-5520829 | NLRP1-1306 | chr17:5559973-5560000 |
| NLRP1-331 | chr17:5520808-5520835 | NLRP1-1307 | chr17:5559974-5560001 |
| NLRP1-332 | chr17:5520809-5520836 | NLRP1-1308 | chr17:5559975-5560002 |
| NLRP1-333 | chr17:5520812-5520839 | NLRP1-1309 | chr17:5559980-5560007 |
| NLRP1-334 | chr17:5520813-5520840 | NLRP1-1310 | chr17:5559984-5560011 |
| NLRP1-335 | chr17:5520814-5520841 | NLRP1-1311 | chr17:5559987-5560014 |
| NLRP1-336 | chr17:5520815-5520842 | NLRP1-1312 | chr17:5559993-5560020 |
| NLRP1-337 | chr17:5520830-5520857 | NLRP1-1313 | chr17:5559994-5560021 |
| NLRP1-338 | chr17:5520831-5520858 | NLRP1-1314 | chr17:5560003-5560030 |
| NLRP1-339 | chr17:5520834-5520861 | NLRP1-1315 | chr17:5560007-5560034 |
| NLRP1-340 | chr17:5520844-5520871 | NLRP1-1316 | chr17:5560026-5560053 |
| NLRP1-341 | chr17:5520845-5520872 | NLRP1-1317 | chr17:5560027-5560054 |
| NLRP1-342 | chr17:5520858-5520885 | NLRP1-1318 | chr17:5560030-5560057 |
| NLRP1-343 | chr17:5520859-5520886 | NLRP1-1319 | chr17:5560031-5560058 |
| NLRP1-344 | chr17:5520860-5520887 | NLRP1-1320 | chr17:5560039-5560066 |
| NLRP1-345 | chr17:5520880-5520907 | NLRP1-1321 | chr17:5560049-5560076 |
| NLRP1-346 | chr17:5520895-5520922 | NLRP1-1322 | chr17:5560094-5560121 |
| NLRP1-347 | chr17:5520902-5520929 | NLRP1-1323 | chr17:5560124-5560151 |
| NLRP1-348 | chr17:5520903-5520930 | NLRP1-1324 | chr17:5560130-5560157 |
| NLRP1-349 | chr17:5520909-5520936 | NLRP1-1325 | chr17:5560135-5560162 |
| NLRP1-350 | chr17:5520914-5520941 | NLRP1-1326 | chr17:5560136-5560163 |
| NLRP1-351 | chr17:5520915-5520942 | NLRP1-1327 | chr17:5581754-5581781 |
| NLRP1-352 | chr17:5520922-5520949 | NLRP1-1328 | chr17:5581756-5581783 |
| NLRP1-353 | chr17:5520923-5520950 | NLRP1-1329 | chr17:5581760-5581787 |
| NLRP1-354 | chr17:5520924-5520951 | NLRP1-1330 | chr17:5581764-5581791 |
| NLRP1-355 | chr17:5520931-5520958 | NLRP1-1331 | chr17:5581765-5581792 |
| NLRP1-356 | chr17:5520932-5520959 | NLRP1-1332 | chr17:5581766-5581793 |
| NLRP1-357 | chr17:5520933-5520960 | NLRP1-1333 | chr17:5581771-5581798 |
| NLRP1-358 | chr17:5520934-5520961 | NLRP1-1334 | chr17:5581775-5581802 |
| NLRP1-359 | chr17:5520936-5520963 | NLRP1-1335 | chr17:5581779-5581806 |
| NLRP1-360 | chr17:5520937-5520964 | NLRP1-1336 | chr17:5581785-5581812 |
| NLRP1-361 | chr17:5520944-5520971 | NLRP1-1337 | chr17:5581786-5581813 |
| NLRP1-362 | chr17:5520959-5520986 | NLRP1-1338 | chr17:5581787-5581814 |
| NLRP1-363 | chr17:5520984-5521011 | NLRP1-1339 | chr17:5581803-5581830 |
| NLRP1-364 | chr17:5520999-5521026 | NLRP1-1340 | chr17:5581813-5581840 |
| NLRP1-365 | chr17:5521000-5521027 | NLRP1-1341 | chr17:5581814-5581841 |
| NLRP1-366 | chr17:5521012-5521039 | NLRP1-1342 | chr17:5581819-5581846 |
| NLRP1-367 | chr17:5521014-5521041 | NLRP1-1343 | chr17:5581820-5581847 |
| NLRP1-368 | chr17:5521015-5521042 | NLRP1-1344 | chr17:5581823-5581850 |
| NLRP1-369 | chr17:5521018-5521045 | NLRP1-1345 | chr17:5581824-5581851 |
| NLRP1-370 | chr17:5521032-5521059 | NLRP1-1346 | chr17:5581825-5581852 |
| NLRP1-371 | chr17:5521039-5521066 | NLRP1-1347 | chr17:5581828-5581855 |
| NLRP1-372 | chr17:5521049-5521076 | NLRP1-1348 | chr17:5581831-5581858 |
| NLRP1-373 | chr17:5521050-5521077 | NLRP1-1349 | chr17:5581832-5581859 |
| NLRP1-374 | chr17:5521051-5521078 | NLRP1-1350 | chr17:5581833-5581860 |
| NLRP1-375 | chr17:5521052-5521079 | NLRP1-1351 | chr17:5581836-5581863 |
| NLRP1-376 | chr17:5521053-5521080 | NLRP1-1352 | chr17:5581858-5581885 |
| NLRP1-377 | chr17:5521054-5521081 | NLRP1-1353 | chr17:5581865-5581892 |
| NLRP1-378 | chr17:5521057-5521084 | NLRP1-1354 | chr17:5581872-5581899 |
| NLRP1-379 | chr17:5521078-5521105 | NLRP1-1355 | chr17:5581873-5581900 |
| NLRP1-380 | chr17:5521087-5521114 | NLRP1-1356 | chr17:5581880-5581907 |
| NLRP1-381 | chr17:5521088-5521115 | NLRP1-1357 | chr17:5581886-5581913 |
| NLRP1-382 | chr17:5521089-5521116 | NLRP1-1358 | chr17:5581893-5581920 |
| NLRP1-383 | chr17:5521403-5521430 | NLRP1-1359 | chr17:5581897-5581924 |
| NLRP1-384 | chr17:5521407-5521434 | NLRP1-1360 | chr17:5581898-5581925 |
| NLRP1-385 | chr17:5521420-5521447 | NLRP1-1361 | chr17:5581902-5581929 |
| NLRP1-386 | chr17:5521421-5521448 | NLRP1-1362 | chr17:5581903-5581930 |
| NLRP1-387 | chr17:5521425-5521452 | NLRP1-1363 | chr17:5581905-5581932 |
| NLRP1-388 | chr17:5521426-5521453 | NLRP1-1364 | chr17:5581909-5581936 |
| NLRP1-389 | chr17:5521427-5521454 | NLRP1-1365 | chr17:5581910-5581937 |
| NLRP1-390 | chr17:5521428-5521455 | NLRP1-1366 | chr17:5581913-5581940 |
| NLRP1-391 | chr17:5521432-5521459 | NLRP1-1367 | chr17:5581916-5581943 |
| NLRP1-392 | chr17:5521433-5521460 | NLRP1-1368 | chr17:5581919-5581946 |
| NLRP1-393 | chr17:5521436-5521463 | NLRP1-1369 | chr17:5581920-5581947 |
| NLRP1-394 | chr17:5521444-5521471 | NLRP1-1370 | chr17:5581921-5581948 |
| NLRP1-395 | chr17:5521445-5521472 | NLRP1-1371 | chr17:5581948-5581975 |
| NLRP1-396 | chr17:5521469-5521496 | NLRP1-1372 | chr17:5581951-5581978 |
| NLRP1-397 | chr17:5521480-5521507 | NLRP1-1373 | chr17:5581952-5581979 |
| NLRP1-398 | chr17:5521483-5521510 | NLRP1-1374 | chr17:5581953-5581980 |
| NLRP1-399 | chr17:5521487-5521514 | NLRP1-1375 | chr17:5581954-5581981 |
| NLRP1-400 | chr17:5521488-5521515 | NLRP1-1376 | chr17:5581955-5581982 |
| NLRP1-401 | chr17:5521491-5521518 | NLRP1-1377 | chr17:5581956-5581983 |
| NLRP1-402 | chr17:5521497-5521524 | NLRP1-1378 | chr17:5581957-5581984 |
| NLRP1-403 | chr17:5521498-5521525 | NLRP1-1379 | chr17:5581959-5581986 |
| NLRP1-404 | chr17:5521502-5521529 | NLRP1-1380 | chr17:5581960-5581987 |
| NLRP1-405 | chr17:5521507-5521534 | NLRP1-1381 | chr17:5581961-5581988 |
| NLRP1-406 | chr17:5521520-5521547 | NLRP1-1382 | chr17:5581963-5581990 |
| NLRP1-407 | chr17:5521521-5521548 | NLRP1-1383 | chr17:5581964-5581991 |
| NLRP1-408 | chr17:5521523-5521550 | NLRP1-1384 | chr17:5581974-5582001 |
| NLRP1-409 | chr17:5521527-5521554 | NLRP1-1385 | chr17:5581979-5582006 |
| NLRP1-410 | chr17:5521533-5521560 | NLRP1-1386 | chr17:5581989-5582016 |
| NLRP1-411 | chr17:5521536-5521563 | NLRP1-1387 | chr17:5581994-5582021 |
| NLRP1-412 | chr17:5521540-5521567 | NLRP1-1388 | chr17:5581995-5582022 |
| NLRP1-413 | chr17:5521553-5521580 | NLRP1-1389 | chr17:5581996-5582023 |
| NLRP1-414 | chr17:5521557-5521584 | NLRP1-1390 | chr17:5581997-5582024 |
| NLRP1-415 | chr17:5521563-5521590 | NLRP1-1391 | chr17:5582003-5582030 |
| NLRP1-416 | chr17:5521566-5521593 | NLRP1-1392 | chr17:5582013-5582040 |
| NLRP1-417 | chr17:5521574-5521601 | NLRP1-1393 | chr17:5582019-5582046 |
| NLRP1-418 | chr17:5521585-5521612 | NLRP1-1394 | chr17:5582026-5582053 |
| NLRP1-419 | chr17:5521591-5521618 | NLRP1-1395 | chr17:5582039-5582066 |
| NLRP1-420 | chr17:5521592-5521619 | NLRP1-1396 | chr17:5582040-5582067 |
| NLRP1-421 | chr17:5521604-5521631 | NLRP1-1397 | chr17:5582041-5582068 |
| NLRP1-422 | chr17:5521605-5521632 | NLRP1-1398 | chr17:5582042-5582069 |
| NLRP1-423 | chr17:5521606-5521633 | NLRP1-1399 | chr17:5582043-5582070 |
| NLRP1-424 | chr17:5521614-5521641 | NLRP1-1400 | chr17:5582088-5582115 |
| NLRP1-425 | chr17:5521626-5521653 | NLRP1-1401 | chr17:5582090-5582117 |
| NLRP1-426 | chr17:5521629-5521656 | NLRP1-1402 | chr17:5582130-5582157 |
| NLRP1-427 | chr17:5521639-5521666 | NLRP1-1403 | chr17:5582131-5582158 |
| NLRP1-428 | chr17:5521640-5521667 | NLRP1-1404 | chr17:5582146-5582173 |
| NLRP1-429 | chr17:5521641-5521668 | NLRP1-1405 | chr17:5582545-5582572 |
| NLRP1-430 | chr17:5521642-5521669 | NLRP1-1406 | chr17:5582546-5582573 |
| NLRP1-431 | chr17:5521651-5521678 | NLRP1-1407 | chr17:5582547-5582574 |
| NLRP1-432 | chr17:5521652-5521679 | NLRP1-1408 | chr17:5582551-5582578 |
| NLRP1-433 | chr17:5521653-5521680 | NLRP1-1409 | chr17:5582552-5582579 |
| NLRP1-434 | chr17:5521660-5521687 | NLRP1-1410 | chr17:5582556-5582583 |
| NLRP1-435 | chr17:5521661-5521688 | NLRP1-1411 | chr17:5582566-5582593 |
| NLRP1-436 | chr17:5521685-5521712 | NLRP1-1412 | chr17:5582567-5582594 |
| NLRP1-437 | chr17:5521687-5521714 | NLRP1-1413 | chr17:5582568-5582595 |
| NLRP1-438 | chr17:5521691-5521718 | NLRP1-1414 | chr17:5582569-5582596 |
| NLRP1-439 | chr17:5521701-5521728 | NLRP1-1415 | chr17:5582578-5582605 |
| NLRP1-440 | chr17:5521706-5521733 | NLRP1-1416 | chr17:5582579-5582606 |
| NLRP1-441 | chr17:5521709-5521736 | NLRP1-1417 | chr17:5582580-5582607 |
| NLRP1-442 | chr17:5521710-5521737 | NLRP1-1418 | chr17:5582581-5582608 |
| NLRP1-443 | chr17:5521724-5521751 | NLRP1-1419 | chr17:5582582-5582609 |
| NLRP1-444 | chr17:5521725-5521752 | NLRP1-1420 | chr17:5582583-5582610 |
| NLRP1-445 | chr17:5521726-5521753 | NLRP1-1421 | chr17:5582584-5582611 |
| NLRP1-446 | chr17:5521734-5521761 | NLRP1-1422 | chr17:5582599-5582626 |
| NLRP1-447 | chr17:5521735-5521762 | NLRP1-1423 | chr17:5582612-5582639 |
| NLRP1-448 | chr17:5521736-5521763 | NLRP1-1424 | chr17:5582613-5582640 |
| NLRP1-449 | chr17:5521739-5521766 | NLRP1-1425 | chr17:5582614-5582641 |
| NLRP1-450 | chr17:5521740-5521767 | NLRP1-1426 | chr17:5582615-5582642 |
| NLRP1-451 | chr17:5521741-5521768 | NLRP1-1427 | chr17:5582616-5582643 |
| NLRP1-452 | chr17:5521754-5521781 | NLRP1-1428 | chr17:5582633-5582660 |
| NLRP1-453 | chr17:5521755-5521782 | NLRP1-1429 | chr17:5582636-5582663 |
| NLRP1-454 | chr17:5521773-5521800 | NLRP1-1430 | chr17:5582637-5582664 |
| NLRP1-455 | chr17:5521775-5521802 | NLRP1-1431 | chr17:5582650-5582677 |
| NLRP1-456 | chr17:5521783-5521810 | NLRP1-1432 | chr17:5582653-5582680 |
| NLRP1-457 | chr17:5521784-5521811 | NLRP1-1433 | chr17:5582654-5582681 |
| NLRP1-458 | chr17:5521785-5521812 | NLRP1-1434 | chr17:5582664-5582691 |
| NLRP1-459 | chr17:5521786-5521813 | NLRP1-1435 | chr17:5582669-5582696 |
| NLRP1-460 | chr17:5521789-5521816 | NLRP1-1436 | chr17:5582670-5582697 |
| NLRP1-461 | chr17:5521843-5521870 | NLRP1-1437 | chr17:5582674-5582701 |
| NLRP1-462 | chr17:5521847-5521874 | NLRP1-1438 | chr17:5582684-5582711 |
| NLRP1-463 | chr17:5521857-5521884 | NLRP1-1439 | chr17:5582704-5582731 |
| NLRP1-464 | chr17:5521865-5521892 | NLRP1-1440 | chr17:5582705-5582732 |
| NLRP1-465 | chr17:5521866-5521893 | NLRP1-1441 | chr17:5582712-5582739 |
| NLRP1-466 | chr17:5521868-5521895 | NLRP1-1442 | chr17:5582713-5582740 |
| NLRP1-467 | chr17:5530356-5530383 | NLRP1-1443 | chr17:5582718-5582745 |
| NLRP1-468 | chr17:5530362-5530389 | NLRP1-1444 | chr17:5582726-5582753 |
| NLRP1-469 | chr17:5530363-5530390 | NLRP1-1445 | chr17:5582727-5582754 |
| NLRP1-470 | chr17:5530364-5530391 | NLRP1-1446 | chr17:5582728-5582755 |
| NLRP1-471 | chr17:5530365-5530392 | NLRP1-1447 | chr17:5582735-5582762 |
| NLRP1-472 | chr17:5530366-5530393 | NLRP1-1448 | chr17:5582736-5582763 |
| NLRP1-473 | chr17:5530367-5530394 | NLRP1-1449 | chr17:5582737-5582764 |
| NLRP1-474 | chr17:5530373-5530400 | NLRP1-1450 | chr17:5582738-5582765 |
| NLRP1-475 | chr17:5530374-5530401 | NLRP1-1451 | chr17:5582739-5582766 |
| NLRP1-476 | chr17:5530375-5530402 | NLRP1-1452 | chr17:5582742-5582769 |
| NLRP1-477 | chr17:5530379-5530406 | NLRP1-1453 | chr17:5582750-5582777 |
| NLRP1-478 | chr17:5530387-5530414 | NLRP1-1454 | chr17:5582753-5582780 |
| NLRP1-479 | chr17:5530395-5530422 | NLRP1-1455 | chr17:5582756-5582783 |
| NLRP1-480 | chr17:5530396-5530423 | NLRP1-1456 | chr17:5582758-5582785 |
| NLRP1-481 | chr17:5530401-5530428 | NLRP1-1457 | chr17:5582759-5582786 |
| NLRP1-482 | chr17:5530402-5530429 | NLRP1-1458 | chr17:5582760-5582787 |
| NLRP1-483 | chr17:5530407-5530434 | NLRP1-1459 | chr17:5582764-5582791 |
| NLRP1-484 | chr17:5530425-5530452 | NLRP1-1460 | chr17:5582768-5582795 |
| NLRP1-485 | chr17:5530426-5530453 | NLRP1-1461 | chr17:5582769-5582796 |
| NLRP1-486 | chr17:5530435-5530462 | NLRP1-1462 | chr17:5582779-5582806 |
| NLRP1-487 | chr17:5530436-5530463 | NLRP1-1463 | chr17:5582782-5582809 |
| NLRP1-488 | chr17:5530445-5530472 | NLRP1-1464 | chr17:5582783-5582810 |
| NLRP1-489 | chr17:5530448-5530475 | NLRP1-1465 | chr17:5582784-5582811 |
| NLRP1-490 | chr17:5530451-5530478 | NLRP1-1466 | chr17:5582793-5582820 |
| NLRP1-491 | chr17:5530455-5530482 | NLRP1-1467 | chr17:5582794-5582821 |
| NLRP1-492 | chr17:5530458-5530485 | NLRP1-1468 | chr17:5582800-5582827 |
| NLRP1-493 | chr17:5530459-5530486 | NLRP1-1469 | chr17:5582801-5582828 |
| NLRP1-494 | chr17:5530464-5530491 | NLRP1-1470 | chr17:5582802-5582829 |
| NLRP1-495 | chr17:5530468-5530495 | NLRP1-1471 | chr17:5582803-5582830 |
| NLRP1-496 | chr17:5530469-5530496 | NLRP1-1472 | chr17:5582810-5582837 |
| NLRP1-497 | chr17:5530475-5530502 | NLRP1-1473 | chr17:5582811-5582838 |
| NLRP1-498 | chr17:5530476-5530503 | NLRP1-1474 | chr17:5582818-5582845 |
| NLRP1-499 | chr17:5530479-5530506 | NLRP1-1475 | chr17:5582826-5582853 |
| NLRP1-500 | chr17:5530480-5530507 | NLRP1-1476 | chr17:5582841-5582868 |
| NLRP1-501 | chr17:5530490-5530517 | NLRP1-1477 | chr17:5582845-5582872 |
| NLRP1-502 | chr17:5530502-5530529 | NLRP1-1478 | chr17:5582846-5582873 |
| NLRP1-503 | chr17:5530517-5530544 | NLRP1-1479 | chr17:5582859-5582886 |
| NLRP1-504 | chr17:5530525-5530552 | NLRP1-1480 | chr17:5582860-5582887 |
| NLRP1-505 | chr17:5530526-5530553 | NLRP1-1481 | chr17:5582861-5582888 |
| NLRP1-506 | chr17:5530535-5530562 | NLRP1-1482 | chr17:5582865-5582892 |
| NLRP1-507 | chr17:5530544-5530571 | NLRP1-1483 | chr17:5582866-5582893 |
| NLRP1-508 | chr17:5530553-5530580 | NLRP1-1484 | chr17:5582867-5582894 |
| NLRP1-509 | chr17:5530554-5530581 | NLRP1-1485 | chr17:5582872-5582899 |
| NLRP1-510 | chr17:5530555-5530582 | NLRP1-1486 | chr17:5582879-5582906 |
| NLRP1-511 | chr17:5530559-5530586 | NLRP1-1487 | chr17:5582880-5582907 |
| NLRP1-512 | chr17:5530562-5530589 | NLRP1-1488 | chr17:5582891-5582918 |
| NLRP1-513 | chr17:5530566-5530593 | NLRP1-1489 | chr17:5582902-5582929 |
| NLRP1-514 | chr17:5530569-5530596 | NLRP1-1490 | chr17:5582914-5582941 |
| NLRP1-515 | chr17:5530575-5530602 | NLRP1-1491 | chr17:5582919-5582946 |
| NLRP1-516 | chr17:5530591-5530618 | NLRP1-1492 | chr17:5582922-5582949 |
| NLRP1-517 | chr17:5530592-5530619 | NLRP1-1493 | chr17:5582932-5582959 |
| NLRP1-518 | chr17:5530603-5530630 | NLRP1-1494 | chr17:5583561-5583588 |
| NLRP1-519 | chr17:5530604-5530631 | NLRP1-1495 | chr17:5583566-5583593 |
| NLRP1-520 | chr17:5530605-5530632 | NLRP1-1496 | chr17:5583570-5583597 |
| NLRP1-521 | chr17:5530634-5530661 | NLRP1-1497 | chr17:5583571-5583598 |
| NLRP1-522 | chr17:5530642-5530669 | NLRP1-1498 | chr17:5583574-5583601 |
| NLRP1-523 | chr17:5530643-5530670 | NLRP1-1499 | chr17:5583575-5583602 |
| NLRP1-524 | chr17:5530650-5530677 | NLRP1-1500 | chr17:5583583-5583610 |
| NLRP1-525 | chr17:5530654-5530681 | NLRP1-1501 | chr17:5583586-5583613 |
| NLRP1-526 | chr17:5530660-5530687 | NLRP1-1502 | chr17:5583587-5583614 |
| NLRP1-527 | chr17:5530661-5530688 | NLRP1-1503 | chr17:5583588-5583615 |
| NLRP1-528 | chr17:5530667-5530694 | NLRP1-1504 | chr17:5583589-5583616 |
| NLRP1-529 | chr17:5530668-5530695 | NLRP1-1505 | chr17:5583598-5583625 |
| NLRP1-530 | chr17:5530671-5530698 | NLRP1-1506 | chr17:5583599-5583626 |
| NLRP1-531 | chr17:5530681-5530708 | NLRP1-1507 | chr17:5583603-5583630 |
| NLRP1-532 | chr17:5530682-5530709 | NLRP1-1508 | chr17:5583604-5583631 |
| NLRP1-533 | chr17:5530684-5530711 | NLRP1-1509 | chr17:5583608-5583635 |
| NLRP1-534 | chr17:5530691-5530718 | NLRP1-1510 | chr17:5583615-5583642 |
| NLRP1-535 | chr17:5530692-5530719 | NLRP1-1511 | chr17:5583616-5583643 |
| NLRP1-536 | chr17:5530697-5530724 | NLRP1-1512 | chr17:5583617-5583644 |
| NLRP1-537 | chr17:5530735-5530762 | NLRP1-1513 | chr17:5583618-5583645 |
| NLRP1-538 | chr17:5530754-5530781 | NLRP1-1514 | chr17:5583621-5583648 |
| NLRP1-539 | chr17:5530755-5530782 | NLRP1-1515 | chr17:5583622-5583649 |
| NLRP1-540 | chr17:5530756-5530783 | NLRP1-1516 | chr17:5583623-5583650 |
| NLRP1-541 | chr17:5530763-5530790 | NLRP1-1517 | chr17:5583634-5583661 |
| NLRP1-542 | chr17:5530766-5530793 | NLRP1-1518 | chr17:5583635-5583662 |
| NLRP1-543 | chr17:5530769-5530796 | NLRP1-1519 | chr17:5583641-5583668 |
| NLRP1-544 | chr17:5530771-5530798 | NLRP1-1520 | chr17:5583642-5583669 |
| NLRP1-545 | chr17:5530772-5530799 | NLRP1-1521 | chr17:5583643-5583670 |
| NLRP1-546 | chr17:5530773-5530800 | NLRP1-1522 | chr17:5583644-5583671 |
| NLRP1-547 | chr17:5530777-5530804 | NLRP1-1523 | chr17:5583655-5583682 |
| NLRP1-548 | chr17:5530796-5530823 | NLRP1-1524 | chr17:5583656-5583683 |
| NLRP1-549 | chr17:5532701-5532728 | NLRP1-1525 | chr17:5583672-5583699 |
| NLRP1-550 | chr17:5532702-5532729 | NLRP1-1526 | chr17:5583673-5583700 |
| NLRP1-551 | chr17:5532703-5532730 | NLRP1-1527 | chr17:5583674-5583701 |
| NLRP1-552 | chr17:5532704-5532731 | NLRP1-1528 | chr17:5583679-5583706 |
| NLRP1-553 | chr17:5532707-5532734 | NLRP1-1529 | chr17:5583680-5583707 |
| NLRP1-554 | chr17:5532708-5532735 | NLRP1-1530 | chr17:5583686-5583713 |
| NLRP1-555 | chr17:5532709-5532736 | NLRP1-1531 | chr17:5583690-5583717 |
| NLRP1-556 | chr17:5532710-5532737 | NLRP1-1532 | chr17:5583691-5583718 |
| NLRP1-557 | chr17:5532711-5532738 | NLRP1-1533 | chr17:5583692-5583719 |
| NLRP1-558 | chr17:5532714-5532741 | NLRP1-1534 | chr17:5583696-5583723 |
| NLRP1-559 | chr17:5532730-5532757 | NLRP1-1535 | chr17:5583713-5583740 |
| NLRP1-560 | chr17:5532731-5532758 | NLRP1-1536 | chr17:5583718-5583745 |
| NLRP1-561 | chr17:5532732-5532759 | NLRP1-1537 | chr17:5583721-5583748 |
| NLRP1-562 | chr17:5532739-5532766 | NLRP1-1538 | chr17:5583722-5583749 |
| NLRP1-563 | chr17:5532743-5532770 | NLRP1-1539 | chr17:5583724-5583751 |
| NLRP1-564 | chr17:5532744-5532771 | NLRP1-1540 | chr17:5583725-5583752 |
| NLRP1-565 | chr17:5532750-5532777 | NLRP1-1541 | chr17:5583726-5583753 |
| NLRP1-566 | chr17:5532751-5532778 | NLRP1-1542 | chr17:5583727-5583754 |
| NLRP1-567 | chr17:5532752-5532779 | NLRP1-1543 | chr17:5583734-5583761 |
| NLRP1-568 | chr17:5532757-5532784 | NLRP1-1544 | chr17:5583735-5583762 |
| NLRP1-569 | chr17:5532758-5532785 | NLRP1-1545 | chr17:5583736-5583763 |
| NLRP1-570 | chr17:5532761-5532788 | NLRP1-1546 | chr17:5583756-5583783 |
| NLRP1-571 | chr17:5532762-5532789 | NLRP1-1547 | chr17:5583757-5583784 |
| NLRP1-572 | chr17:5532770-5532797 | NLRP1-1548 | chr17:5583762-5583789 |
| NLRP1-573 | chr17:5532771-5532798 | NLRP1-1549 | chr17:5583763-5583790 |
| NLRP1-574 | chr17:5532789-5532816 | NLRP1-1550 | chr17:5583770-5583797 |
| NLRP1-575 | chr17:5532793-5532820 | NLRP1-1551 | chr17:5583771-5583798 |
| NLRP1-576 | chr17:5532797-5532824 | NLRP1-1552 | chr17:5583772-5583799 |
| NLRP1-577 | chr17:5532800-5532827 | NLRP1-1553 | chr17:5583773-5583800 |
| NLRP1-578 | chr17:5532809-5532836 | NLRP1-1554 | chr17:5583783-5583810 |
| NLRP1-579 | chr17:5532810-5532837 | NLRP1-1555 | chr17:5583786-5583813 |
| NLRP1-580 | chr17:5532811-5532838 | NLRP1-1556 | chr17:5583798-5583825 |
| NLRP1-581 | chr17:5532812-5532839 | NLRP1-1557 | chr17:5583801-5583828 |
| NLRP1-582 | chr17:5532821-5532848 | NLRP1-1558 | chr17:5583804-5583831 |
| NLRP1-583 | chr17:5532838-5532865 | NLRP1-1559 | chr17:5583806-5583833 |
| NLRP1-584 | chr17:5532846-5532873 | NLRP1-1560 | chr17:5583807-5583834 |
| NLRP1-585 | chr17:5532847-5532874 | NLRP1-1561 | chr17:5583809-5583836 |
| NLRP1-586 | chr17:5532848-5532875 | NLRP1-1562 | chr17:5583830-5583857 |
| NLRP1-587 | chr17:5532850-5532877 | NLRP1-1563 | chr17:5583842-5583869 |
| NLRP1-588 | chr17:5532859-5532886 | NLRP1-1564 | chr17:5583843-5583870 |
| NLRP1-589 | chr17:5532866-5532893 | NLRP1-1565 | chr17:5583845-5583872 |
| NLRP1-590 | chr17:5532867-5532894 | NLRP1-1566 | chr17:5583853-5583880 |
| NLRP1-591 | chr17:5532868-5532895 | NLRP1-1567 | chr17:5583859-5583886 |
| NLRP1-592 | chr17:5532876-5532903 | NLRP1-1568 | chr17:5583886-5583913 |
| NLRP1-593 | chr17:5532877-5532904 | NLRP1-1569 | chr17:5583891-5583918 |
| NLRP1-594 | chr17:5532878-5532905 | NLRP1-1570 | chr17:5583900-5583927 |
| NLRP1-595 | chr17:5532890-5532917 | NLRP1-1571 | chr17:5583902-5583929 |
| NLRP1-596 | chr17:5532896-5532923 | NLRP1-1572 | chr17:5583903-5583930 |
| NLRP1-597 | chr17:5532897-5532924 | NLRP1-1573 | chr17:5583907-5583934 |
| NLRP1-598 | chr17:5532898-5532925 | NLRP1-1574 | chr17:5583910-5583937 |
| NLRP1-599 | chr17:5532903-5532930 | NLRP1-1575 | chr17:5583917-5583944 |
| NLRP1-600 | chr17:5532907-5532934 | NLRP1-1576 | chr17:5583918-5583945 |
| NLRP1-601 | chr17:5532913-5532940 | NLRP1-1577 | chr17:5583930-5583957 |
| NLRP1-602 | chr17:5532919-5532946 | NLRP1-1578 | chr17:5583938-5583965 |
| NLRP1-603 | chr17:5532920-5532947 | NLRP1-1579 | chr17:5583939-5583966 |
| NLRP1-604 | chr17:5532921-5532948 | NLRP1-1580 | chr17:5583940-5583967 |
| NLRP1-605 | chr17:5532934-5532961 | NLRP1-1581 | chr17:5583941-5583968 |
| NLRP1-606 | chr17:5532946-5532973 | NLRP1-1582 | chr17:5583947-5583974 |
| NLRP1-607 | chr17:5532947-5532974 | NLRP1-1583 | chr17:5583950-5583977 |
| NLRP1-608 | chr17:5532948-5532975 | NLRP1-1584 | chr17:5583951-5583978 |
| NLRP1-609 | chr17:5532955-5532982 | NLRP1-1585 | chr17:5583952-5583979 |
| NLRP1-610 | chr17:5532958-5532985 | NLRP1-1586 | chr17:5583954-5583981 |
| NLRP1-611 | chr17:5532967-5532994 | NLRP1-1587 | chr17:5583959-5583986 |
| NLRP1-612 | chr17:5532971-5532998 | NLRP1-1588 | chr17:5583960-5583987 |
| NLRP1-613 | chr17:5532977-5533004 | NLRP1-1589 | chr17:5583961-5583988 |
| NLRP1-614 | chr17:5532982-5533009 | NLRP1-1590 | chr17:5583962-5583989 |
| NLRP1-615 | chr17:5533001-5533028 | NLRP1-1591 | chr17:5583964-5583991 |
| NLRP1-616 | chr17:5533011-5533038 | NLRP1-1592 | chr17:5583965-5583992 |
| NLRP1-617 | chr17:5533020-5533047 | NLRP1-1593 | chr17:5583973-5584000 |
| NLRP1-618 | chr17:5533021-5533048 | NLRP1-1594 | chr17:5583976-5584003 |
| NLRP1-619 | chr17:5533024-5533051 | NLRP1-1595 | chr17:5583981-5584008 |
| NLRP1-620 | chr17:5533029-5533056 | NLRP1-1596 | chr17:5583982-5584009 |
| NLRP1-621 | chr17:5533032-5533059 | NLRP1-1597 | chr17:5583989-5584016 |
| NLRP1-622 | chr17:5533033-5533060 | NLRP1-1598 | chr17:5583993-5584020 |
| NLRP1-623 | chr17:5533034-5533061 | NLRP1-1599 | chr17:5583995-5584022 |
| NLRP1-624 | chr17:5533035-5533062 | NLRP1-1600 | chr17:5583996-5584023 |
| NLRP1-625 | chr17:5533039-5533066 | NLRP1-1601 | chr17:5584021-5584048 |
| NLRP1-626 | chr17:5533040-5533067 | NLRP1-1602 | chr17:5584037-5584064 |
| NLRP1-627 | chr17:5533041-5533068 | NLRP1-1603 | chr17:5584043-5584070 |
| NLRP1-628 | chr17:5533047-5533074 | NLRP1-1604 | chr17:5584049-5584076 |
| NLRP1-629 | chr17:5533051-5533078 | NLRP1-1605 | chr17:5584055-5584082 |
| NLRP1-630 | chr17:5533060-5533087 | NLRP1-1606 | chr17:5584072-5584099 |
| NLRP1-631 | chr17:5533063-5533090 | NLRP1-1607 | chr17:5584073-5584100 |
| NLRP1-632 | chr17:5533064-5533091 | NLRP1-1608 | chr17:5584079-5584106 |
| NLRP1-633 | chr17:5533067-5533094 | NLRP1-1609 | chr17:5584080-5584107 |
| NLRP1-634 | chr17:5533068-5533095 | NLRP1-1610 | chr17:5584094-5584121 |
| NLRP1-635 | chr17:5533069-5533096 | NLRP1-1611 | chr17:5584095-5584122 |
| NLRP1-636 | chr17:5533071-5533098 | NLRP1-1612 | chr17:5584099-5584126 |
| NLRP1-637 | chr17:5533072-5533099 | NLRP1-1613 | chr17:5584100-5584127 |
| NLRP1-638 | chr17:5533079-5533106 | NLRP1-1614 | chr17:5584101-5584128 |
| NLRP1-639 | chr17:5533083-5533110 | NLRP1-1615 | chr17:5584102-5584129 |
| NLRP1-640 | chr17:5533169-5533196 | NLRP1-1616 | chr17:5584103-5584130 |
| NLRP1-641 | chr17:5533172-5533199 | NLRP1-1617 | chr17:5584107-5584134 |
| NLRP1-642 | chr17:5533176-5533203 | NLRP1-1618 | chr17:5584111-5584138 |
| NLRP1-643 | chr17:5533177-5533204 | NLRP1-1619 | chr17:5584117-5584144 |
| NLRP1-644 | chr17:5533181-5533208 | NLRP1-1620 | chr17:5584121-5584148 |
| NLRP1-645 | chr17:5533184-5533211 | NLRP1-1621 | chr17:5584122-5584149 |
| NLRP1-646 | chr17:5533187-5533214 | NLRP1-1622 | chr17:5584123-5584150 |
| NLRP1-647 | chr17:5533188-5533215 | NLRP1-1623 | chr17:5584126-5584153 |
| NLRP1-648 | chr17:5533194-5533221 | NLRP1-1624 | chr17:5584127-5584154 |
| NLRP1-649 | chr17:5533195-5533222 | NLRP1-1625 | chr17:5584130-5584157 |
| NLRP1-650 | chr17:5533196-5533223 | NLRP1-1626 | chr17:5584140-5584167 |
| NLRP1-651 | chr17:5533200-5533227 | NLRP1-1627 | chr17:5584141-5584168 |
| NLRP1-652 | chr17:5533201-5533228 | NLRP1-1628 | chr17:5584142-5584169 |
| NLRP1-653 | chr17:5533202-5533229 | NLRP1-1629 | chr17:5584144-5584171 |
| NLRP1-654 | chr17:5533214-5533241 | NLRP1-1630 | chr17:5584157-5584184 |
| NLRP1-655 | chr17:5533217-5533244 | NLRP1-1631 | chr17:5584160-5584187 |
| NLRP1-656 | chr17:5533218-5533245 | NLRP1-1632 | chr17:5584161-5584188 |
| NLRP1-657 | chr17:5533219-5533246 | NLRP1-1633 | chr17:5584170-5584197 |
| NLRP1-658 | chr17:5533222-5533249 | NLRP1-1634 | chr17:5584175-5584202 |
| NLRP1-659 | chr17:5533226-5533253 | NLRP1-1635 | chr17:5584176-5584203 |
| NLRP1-660 | chr17:5533227-5533254 | NLRP1-1636 | chr17:5584179-5584206 |
| NLRP1-661 | chr17:5533228-5533255 | NLRP1-1637 | chr17:5584185-5584212 |
| NLRP1-662 | chr17:5533236-5533263 | NLRP1-1638 | chr17:5584186-5584213 |
| NLRP1-663 | chr17:5533237-5533264 | NLRP1-1639 | chr17:5584190-5584217 |
| NLRP1-664 | chr17:5533238-5533265 | NLRP1-1640 | chr17:5584191-5584218 |
| NLRP1-665 | chr17:5533254-5533281 | NLRP1-1641 | chr17:5584192-5584219 |
| NLRP1-666 | chr17:5533255-5533282 | NLRP1-1642 | chr17:5584198-5584225 |
| NLRP1-667 | chr17:5533256-5533283 | NLRP1-1643 | chr17:5584199-5584226 |
| NLRP1-668 | chr17:5533261-5533288 | NLRP1-1644 | chr17:5584204-5584231 |
| NLRP1-669 | chr17:5533269-5533296 | NLRP1-1645 | chr17:5584208-5584235 |
| NLRP1-670 | chr17:5533284-5533311 | NLRP1-1646 | chr17:5584209-5584236 |
| NLRP1-671 | chr17:5533291-5533318 | NLRP1-1647 | chr17:5584212-5584239 |
| NLRP1-672 | chr17:5533295-5533322 | NLRP1-1648 | chr17:5584213-5584240 |
| NLRP1-673 | chr17:5533296-5533323 | NLRP1-1649 | chr17:5584218-5584245 |
| NLRP1-674 | chr17:5533303-5533330 | NLRP1-1650 | chr17:5584219-5584246 |
| NLRP1-675 | chr17:5533317-5533344 | NLRP1-1651 | chr17:5584221-5584248 |
| NLRP1-676 | chr17:5533340-5533367 | NLRP1-1652 | chr17:5584224-5584251 |
| NLRP1-677 | chr17:5533344-5533371 | NLRP1-1653 | chr17:5584225-5584252 |
| NLRP1-678 | chr17:5533345-5533372 | NLRP1-1654 | chr17:5584226-5584253 |
| NLRP1-679 | chr17:5533358-5533385 | NLRP1-1655 | chr17:5584227-5584254 |
| NLRP1-680 | chr17:5533376-5533403 | NLRP1-1656 | chr17:5584234-5584261 |
| NLRP1-681 | chr17:5533379-5533406 | NLRP1-1657 | chr17:5584240-5584267 |
| NLRP1-682 | chr17:5533380-5533407 | NLRP1-1658 | chr17:5584241-5584268 |
| NLRP1-683 | chr17:5533386-5533413 | NLRP1-1659 | chr17:5584247-5584274 |
| NLRP1-684 | chr17:5533391-5533418 | NLRP1-1660 | chr17:5584248-5584275 |
| NLRP1-685 | chr17:5533394-5533421 | NLRP1-1661 | chr17:5584249-5584276 |
| NLRP1-686 | chr17:5533409-5533436 | NLRP1-1662 | chr17:5584254-5584281 |
| NLRP1-687 | chr17:5533424-5533451 | NLRP1-1663 | chr17:5584255-5584282 |
| NLRP1-688 | chr17:5533427-5533454 | NLRP1-1664 | chr17:5584299-5584326 |
| NLRP1-689 | chr17:5533430-5533457 | NLRP1-1665 | chr17:5584300-5584327 |
| NLRP1-690 | chr17:5533433-5533460 | NLRP1-1666 | chr17:5584304-5584331 |
| NLRP1-691 | chr17:5533436-5533463 | NLRP1-1667 | chr17:5584306-5584333 |
| NLRP1-692 | chr17:5533437-5533464 | NLRP1-1668 | chr17:5584316-5584343 |
| NLRP1-693 | chr17:5533440-5533467 | NLRP1-1669 | chr17:5584317-5584344 |
| NLRP1-694 | chr17:5533459-5533486 | NLRP1-1670 | chr17:5584322-5584349 |
| NLRP1-695 | chr17:5533465-5533492 | NLRP1-1671 | chr17:5584329-5584356 |
| NLRP1-696 | chr17:5533773-5533800 | NLRP1-1672 | chr17:5584336-5584363 |
| NLRP1-697 | chr17:5533774-5533801 | NLRP1-1673 | chr17:5584337-5584364 |
| NLRP1-698 | chr17:5533783-5533810 | NLRP1-1674 | chr17:5584347-5584374 |
| NLRP1-699 | chr17:5533784-5533811 | NLRP1-1675 | chr17:5584348-5584375 |
| NLRP1-700 | chr17:5533785-5533812 | NLRP1-1676 | chr17:5584349-5584376 |
| NLRP1-701 | chr17:5533790-5533817 | NLRP1-1677 | chr17:5584350-5584377 |
| NLRP1-702 | chr17:5533791-5533818 | NLRP1-1678 | chr17:5584351-5584378 |
| NLRP1-703 | chr17:5533792-5533819 | NLRP1-1679 | chr17:5584352-5584379 |
| NLRP1-704 | chr17:5533794-5533821 | NLRP1-1680 | chr17:5584355-5584382 |
| NLRP1-705 | chr17:5533795-5533822 | NLRP1-1681 | chr17:5584356-5584383 |
| NLRP1-706 | chr17:5533796-5533823 | NLRP1-1682 | chr17:5584359-5584386 |
| NLRP1-707 | chr17:5533799-5533826 | NLRP1-1683 | chr17:5584373-5584400 |
| NLRP1-708 | chr17:5533800-5533827 | NLRP1-1684 | chr17:5584374-5584401 |
| NLRP1-709 | chr17:5533801-5533828 | NLRP1-1685 | chr17:5584379-5584406 |
| NLRP1-710 | chr17:5533833-5533860 | NLRP1-1686 | chr17:5584380-5584407 |
| NLRP1-711 | chr17:5533836-5533863 | NLRP1-1687 | chr17:5584389-5584416 |
| NLRP1-712 | chr17:5533837-5533864 | NLRP1-1688 | chr17:5584399-5584426 |
| NLRP1-713 | chr17:5533847-5533874 | NLRP1-1689 | chr17:5584407-5584434 |
| NLRP1-714 | chr17:5533850-5533877 | NLRP1-1690 | chr17:5584408-5584435 |
| NLRP1-715 | chr17:5533851-5533878 | NLRP1-1691 | chr17:5584415-5584442 |
| NLRP1-716 | chr17:5533852-5533879 | NLRP1-1692 | chr17:5584417-5584444 |
| NLRP1-717 | chr17:5533853-5533880 | NLRP1-1693 | chr17:5584418-5584445 |
| NLRP1-718 | chr17:5533857-5533884 | NLRP1-1694 | chr17:5584423-5584450 |
| NLRP1-719 | chr17:5533858-5533885 | NLRP1-1695 | chr17:5584429-5584456 |
| NLRP1-720 | chr17:5533859-5533886 | NLRP1-1696 | chr17:5584430-5584457 |
| NLRP1-721 | chr17:5533873-5533900 | NLRP1-1697 | chr17:5584431-5584458 |
| NLRP1-722 | chr17:5533874-5533901 | NLRP1-1698 | chr17:5584443-5584470 |
| NLRP1-723 | chr17:5533878-5533905 | NLRP1-1699 | chr17:5584445-5584472 |
| NLRP1-724 | chr17:5533883-5533910 | NLRP1-1700 | chr17:5584446-5584473 |
| NLRP1-725 | chr17:5533884-5533911 | NLRP1-1701 | chr17:5584449-5584476 |
| NLRP1-726 | chr17:5533885-5533912 | NLRP1-1702 | chr17:5584450-5584477 |
| NLRP1-727 | chr17:5533896-5533923 | NLRP1-1703 | chr17:5584451-5584478 |
| NLRP1-728 | chr17:5533899-5533926 | NLRP1-1704 | chr17:5584461-5584488 |
| NLRP1-729 | chr17:5533902-5533929 | NLRP1-1705 | chr17:5584466-5584493 |
| NLRP1-730 | chr17:5533913-5533940 | NLRP1-1706 | chr17:5584471-5584498 |
| NLRP1-731 | chr17:5533931-5533958 | NLRP1-1707 | chr17:5584472-5584499 |
| NLRP1-732 | chr17:5533942-5533969 | NLRP1-1708 | chr17:5584481-5584508 |
| NLRP1-733 | chr17:5533943-5533970 | NLRP1-1709 | chr17:5584485-5584512 |
| NLRP1-734 | chr17:5533944-5533971 | NLRP1-1710 | chr17:5584491-5584518 |
| NLRP1-735 | chr17:5533947-5533974 | NLRP1-1711 | chr17:5584495-5584522 |
| NLRP1-736 | chr17:5533948-5533975 | NLRP1-1712 | chr17:5584500-5584527 |
| NLRP1-737 | chr17:5533954-5533981 | NLRP1-1713 | chr17:5584511-5584538 |
| NLRP1-738 | chr17:5533957-5533984 | NLRP1-1714 | chr17:5584523-5584550 |
| NLRP1-739 | chr17:5533959-5533986 | NLRP1-1715 | chr17:5584524-5584551 |
| NLRP1-740 | chr17:5533960-5533987 | NLRP1-1716 | chr17:5584525-5584552 |
| NLRP1-741 | chr17:5533965-5533992 | NLRP1-1717 | chr17:5584530-5584557 |
| NLRP1-742 | chr17:5533988-5534015 | NLRP1-1718 | chr17:5584531-5584558 |
| NLRP1-743 | chr17:5533998-5534025 | NLRP1-1719 | chr17:5584532-5584559 |
| NLRP1-744 | chr17:5534001-5534028 | NLRP1-1720 | chr17:5584535-5584562 |
| NLRP1-745 | chr17:5534009-5534036 | NLRP1-1721 | chr17:5584536-5584563 |
| NLRP1-746 | chr17:5534010-5534037 | NLRP1-1722 | chr17:5584537-5584564 |
| NLRP1-747 | chr17:5534013-5534040 | NLRP1-1723 | chr17:5584538-5584565 |
| NLRP1-748 | chr17:5534042-5534069 | NLRP1-1724 | chr17:5584542-5584569 |
| NLRP1-749 | chr17:5534053-5534080 | NLRP1-1725 | chr17:5584543-5584570 |
| NLRP1-750 | chr17:5534054-5534081 | NLRP1-1726 | chr17:5584553-5584580 |
| NLRP1-751 | chr17:5534055-5534082 | NLRP1-1727 | chr17:5584558-5584585 |
| NLRP1-752 | chr17:5534058-5534085 | NLRP1-1728 | chr17:5584559-5584586 |
| NLRP1-753 | chr17:5534059-5534086 | NLRP1-1729 | chr17:5584563-5584590 |
| NLRP1-754 | chr17:5534067-5534094 | NLRP1-1730 | chr17:5584576-5584603 |
| NLRP1-755 | chr17:5534072-5534099 | NLRP1-1731 | chr17:5584581-5584608 |
| NLRP1-756 | chr17:5534075-5534102 | NLRP1-1732 | chr17:5584591-5584618 |
| NLRP1-757 | chr17:5536731-5536758 | NLRP1-1733 | chr17:5584592-5584619 |
| NLRP1-758 | chr17:5536753-5536780 | NLRP1-1734 | chr17:5584600-5584627 |
| NLRP1-759 | chr17:5536754-5536781 | NLRP1-1735 | chr17:5584610-5584637 |
| NLRP1-760 | chr17:5536755-5536782 | NLRP1-1736 | chr17:5584613-5584640 |
| NLRP1-761 | chr17:5536775-5536802 | NLRP1-1737 | chr17:5584619-5584646 |
| NLRP1-762 | chr17:5536776-5536803 | NLRP1-1738 | chr17:5584620-5584647 |
| NLRP1-763 | chr17:5536777-5536804 | NLRP1-1739 | chr17:5584627-5584654 |
| NLRP1-764 | chr17:5536784-5536811 | NLRP1-1740 | chr17:5584642-5584669 |
| NLRP1-765 | chr17:5536785-5536812 | NLRP1-1741 | chr17:5584653-5584680 |
| NLRP1-766 | chr17:5536789-5536816 | NLRP1-1742 | chr17:5584655-5584682 |
| NLRP1-767 | chr17:5536790-5536817 | NLRP1-1743 | chr17:5584656-5584683 |
| NLRP1-768 | chr17:5536791-5536818 | NLRP1-1744 | chr17:5584659-5584686 |
| NLRP1-769 | chr17:5536797-5536824 | NLRP1-1745 | chr17:5584661-5584688 |
| NLRP1-770 | chr17:5536802-5536829 | NLRP1-1746 | chr17:5584675-5584702 |
| NLRP1-771 | chr17:5536803-5536830 | NLRP1-1747 | chr17:5584687-5584714 |
| NLRP1-772 | chr17:5536806-5536833 | NLRP1-1748 | chr17:5584688-5584715 |
| NLRP1-773 | chr17:5536807-5536834 | NLRP1-1749 | chr17:5584694-5584721 |
| NLRP1-774 | chr17:5536811-5536838 | NLRP1-1750 | chr17:5584695-5584722 |
| NLRP1-775 | chr17:5536812-5536839 | NLRP1-1751 | chr17:5584711-5584738 |
| NLRP1-776 | chr17:5536813-5536840 | NLRP1-1752 | chr17:5584714-5584741 |
| NLRP1-777 | chr17:5536823-5536850 | NLRP1-1753 | chr17:5584727-5584754 |
| NLRP1-778 | chr17:5536841-5536868 | NLRP1-1754 | chr17:5584730-5584757 |
| NLRP1-779 | chr17:5536842-5536869 | NLRP1-1755 | chr17:5584734-5584761 |
| NLRP1-780 | chr17:5536843-5536870 | NLRP1-1756 | chr17:5584735-5584762 |
| NLRP1-781 | chr17:5536844-5536871 | NLRP1-1757 | chr17:5584738-5584765 |
| NLRP1-782 | chr17:5536845-5536872 | NLRP1-1758 | chr17:5584748-5584775 |
| NLRP1-783 | chr17:5536849-5536876 | NLRP1-1759 | chr17:5584761-5584788 |
| NLRP1-784 | chr17:5536850-5536877 | NLRP1-1760 | chr17:5584775-5584802 |
| NLRP1-785 | chr17:5536865-5536892 | NLRP1-1761 | chr17:5584777-5584804 |
| NLRP1-786 | chr17:5536866-5536893 | NLRP1-1762 | chr17:5584789-5584816 |
| NLRP1-787 | chr17:5536882-5536909 | NLRP1-1763 | chr17:5584793-5584820 |
| NLRP1-788 | chr17:5536888-5536915 | NLRP1-1764 | chr17:5584794-5584821 |
| NLRP1-789 | chr17:5536894-5536921 | NLRP1-1765 | chr17:5584797-5584824 |
| NLRP1-790 | chr17:5536895-5536922 | NLRP1-1766 | chr17:5584798-5584825 |
| NLRP1-791 | chr17:5536903-5536930 | NLRP1-1767 | chr17:5584799-5584826 |
| NLRP1-792 | chr17:5536907-5536934 | NLRP1-1768 | chr17:5584803-5584830 |
| NLRP1-793 | chr17:5536928-5536955 | NLRP1-1769 | chr17:5584804-5584831 |
| NLRP1-794 | chr17:5536929-5536956 | NLRP1-1770 | chr17:5584808-5584835 |
| NLRP1-795 | chr17:5536935-5536962 | NLRP1-1771 | chr17:5584817-5584844 |
| NLRP1-796 | chr17:5536936-5536963 | NLRP1-1772 | chr17:5584818-5584845 |
| NLRP1-797 | chr17:5536940-5536967 | NLRP1-1773 | chr17:5584827-5584854 |
| NLRP1-798 | chr17:5536945-5536972 | NLRP1-1774 | chr17:5584828-5584855 |
| NLRP1-799 | chr17:5536946-5536973 | NLRP1-1775 | chr17:5584829-5584856 |
| NLRP1-800 | chr17:5536959-5536986 | NLRP1-1776 | chr17:5584830-5584857 |
| NLRP1-801 | chr17:5536965-5536992 | NLRP1-1777 | chr17:5584832-5584859 |
| NLRP1-802 | chr17:5536967-5536994 | NLRP1-1778 | chr17:5584833-5584860 |
| NLRP1-803 | chr17:5536968-5536995 | NLRP1-1779 | chr17:5584840-5584867 |
| NLRP1-804 | chr17:5536975-5537002 | NLRP1-1780 | chr17:5584841-5584868 |
| NLRP1-805 | chr17:5536976-5537003 | NLRP1-1781 | chr17:5584848-5584875 |
| NLRP1-806 | chr17:5536977-5537004 | NLRP1-1782 | chr17:5584849-5584876 |
| NLRP1-807 | chr17:5536978-5537005 | NLRP1-1783 | chr17:5584851-5584878 |
| NLRP1-808 | chr17:5536979-5537006 | NLRP1-1784 | chr17:5584854-5584881 |
| NLRP1-809 | chr17:5536988-5537015 | NLRP1-1785 | chr17:5584855-5584882 |
| NLRP1-810 | chr17:5536989-5537016 | NLRP1-1786 | chr17:5584877-5584904 |
| NLRP1-811 | chr17:5536990-5537017 | NLRP1-1787 | chr17:5584879-5584906 |
| NLRP1-812 | chr17:5536996-5537023 | NLRP1-1788 | chr17:5584885-5584912 |
| NLRP1-813 | chr17:5536997-5537024 | NLRP1-1789 | chr17:5584886-5584913 |
| NLRP1-814 | chr17:5536998-5537025 | NLRP1-1790 | chr17:5584892-5584919 |
| NLRP1-815 | chr17:5537004-5537031 | NLRP1-1791 | chr17:5584893-5584920 |
| NLRP1-816 | chr17:5537005-5537032 | NLRP1-1792 | chr17:5584897-5584924 |
| NLRP1-817 | chr17:5537008-5537035 | NLRP1-1793 | chr17:5584927-5584954 |
| NLRP1-818 | chr17:5537012-5537039 | NLRP1-1794 | chr17:5584928-5584955 |
| NLRP1-819 | chr17:5537014-5537041 | NLRP1-1795 | chr17:5584929-5584956 |
| NLRP1-820 | chr17:5537019-5537046 | NLRP1-1796 | chr17:5584938-5584965 |
| NLRP1-821 | chr17:5537021-5537048 | NLRP1-1797 | chr17:5584942-5584969 |
| NLRP1-822 | chr17:5537022-5537049 | NLRP1-1798 | chr17:5584950-5584977 |
| NLRP1-823 | chr17:5537025-5537052 | NLRP1-1799 | chr17:5584956-5584983 |
| NLRP1-824 | chr17:5537026-5537053 | NLRP1-1800 | chr17:5584957-5584984 |
| NLRP1-825 | chr17:5537034-5537061 | NLRP1-1801 | chr17:5584958-5584985 |
| NLRP1-826 | chr17:5539293-5539320 | NLRP1-1802 | chr17:5584959-5584986 |
| NLRP1-827 | chr17:5539294-5539321 | NLRP1-1803 | chr17:5584972-5584999 |
| NLRP1-828 | chr17:5539298-5539325 | NLRP1-1804 | chr17:5584973-5585000 |
| NLRP1-829 | chr17:5539342-5539369 | NLRP1-1805 | chr17:5584974-5585001 |
| NLRP1-830 | chr17:5539349-5539376 | NLRP1-1806 | chr17:5584975-5585002 |
| NLRP1-831 | chr17:5539353-5539380 | NLRP1-1807 | chr17:5584978-5585005 |
| NLRP1-832 | chr17:5539354-5539381 | NLRP1-1808 | chr17:5584979-5585006 |
| NLRP1-833 | chr17:5539355-5539382 | NLRP1-1809 | chr17:5584994-5585021 |
| NLRP1-834 | chr17:5539360-5539387 | NLRP1-1810 | chr17:5585006-5585033 |
| NLRP1-835 | chr17:5539366-5539393 | NLRP1-1811 | chr17:5585007-5585034 |
| NLRP1-836 | chr17:5539367-5539394 | NLRP1-1812 | chr17:5585031-5585058 |
| NLRP1-837 | chr17:5539368-5539395 | NLRP1-1813 | chr17:5585034-5585061 |
| NLRP1-838 | chr17:5539369-5539396 | NLRP1-1814 | chr17:5585047-5585074 |
| NLRP1-839 | chr17:5539370-5539397 | NLRP1-1815 | chr17:5585050-5585077 |
| NLRP1-840 | chr17:5539371-5539398 | NLRP1-1816 | chr17:5585051-5585078 |
| NLRP1-841 | chr17:5539375-5539402 | NLRP1-1817 | chr17:5585052-5585079 |
| NLRP1-842 | chr17:5539376-5539403 | NLRP1-1818 | chr17:5585053-5585080 |
| NLRP1-843 | chr17:5539380-5539407 | NLRP1-1819 | chr17:5585054-5585081 |
| NLRP1-844 | chr17:5539381-5539408 | NLRP1-1820 | chr17:5585055-5585082 |
| NLRP1-845 | chr17:5539382-5539409 | NLRP1-1821 | chr17:5585056-5585083 |
| NLRP1-846 | chr17:5539383-5539410 | NLRP1-1822 | chr17:5585062-5585089 |
| NLRP1-847 | chr17:5539389-5539416 | NLRP1-1823 | chr17:5585063-5585090 |
| NLRP1-848 | chr17:5539390-5539417 | NLRP1-1824 | chr17:5585064-5585091 |
| NLRP1-849 | chr17:5539393-5539420 | NLRP1-1825 | chr17:5585075-5585102 |
| NLRP1-850 | chr17:5539400-5539427 | NLRP1-1826 | chr17:5585083-5585110 |
| NLRP1-851 | chr17:5539401-5539428 | NLRP1-1827 | chr17:5585085-5585112 |
| NLRP1-852 | chr17:5539409-5539436 | NLRP1-1828 | chr17:5585099-5585126 |
| NLRP1-853 | chr17:5539413-5539440 | NLRP1-1829 | chr17:5585110-5585137 |
| NLRP1-854 | chr17:5539414-5539441 | NLRP1-1830 | chr17:5585116-5585143 |
| NLRP1-855 | chr17:5539415-5539442 | NLRP1-1831 | chr17:5585124-5585151 |
| NLRP1-856 | chr17:5539416-5539443 | NLRP1-1832 | chr17:5585154-5585181 |
| NLRP1-857 | chr17:5539444-5539471 | NLRP1-1833 | chr17:5585159-5585186 |
| NLRP1-858 | chr17:5539450-5539477 | NLRP1-1834 | chr17:5585168-5585195 |
| NLRP1-859 | chr17:5539451-5539478 | NLRP1-1835 | chr17:5585179-5585206 |
| NLRP1-860 | chr17:5539452-5539479 | NLRP1-1836 | chr17:5585182-5585209 |
| NLRP1-861 | chr17:5539459-5539486 | NLRP1-1837 | chr17:5585189-5585216 |
| NLRP1-862 | chr17:5539472-5539499 | NLRP1-1838 | chr17:5585194-5585221 |
| NLRP1-863 | chr17:5539474-5539501 | NLRP1-1839 | chr17:5585205-5585232 |
| NLRP1-864 | chr17:5539482-5539509 | NLRP1-1840 | chr17:5585218-5585245 |
| NLRP1-865 | chr17:5539489-5539516 | NLRP1-1841 | chr17:5585227-5585254 |
| NLRP1-866 | chr17:5539493-5539520 | NLRP1-1842 | chr17:5585228-5585255 |
| NLRP1-867 | chr17:5539494-5539521 | NLRP1-1843 | chr17:5585233-5585260 |
| NLRP1-868 | chr17:5539495-5539522 | NLRP1-1844 | chr17:5585239-5585266 |
| NLRP1-869 | chr17:5539499-5539526 | NLRP1-1845 | chr17:5585240-5585267 |
| NLRP1-870 | chr17:5539509-5539536 | NLRP1-1846 | chr17:5585264-5585291 |
| NLRP1-871 | chr17:5539514-5539541 | NLRP1-1847 | chr17:5585273-5585300 |
| NLRP1-872 | chr17:5539519-5539546 | NLRP1-1848 | chr17:5585290-5585317 |
| NLRP1-873 | chr17:5539525-5539552 | NLRP1-1849 | chr17:5585291-5585318 |
| NLRP1-874 | chr17:5539542-5539569 | NLRP1-1850 | chr17:5585292-5585319 |
| NLRP1-875 | chr17:5539543-5539570 | NLRP1-1851 | chr17:5585301-5585328 |
| NLRP1-876 | chr17:5539548-5539575 | NLRP1-1852 | chr17:5585302-5585329 |
| NLRP1-877 | chr17:5539565-5539592 | NLRP1-1853 | chr17:5585303-5585330 |
| NLRP1-878 | chr17:5539571-5539598 | NLRP1-1854 | chr17:5585313-5585340 |
| NLRP1-879 | chr17:5539581-5539608 | NLRP1-1855 | chr17:5585331-5585358 |
| NLRP1-880 | chr17:5539585-5539612 | NLRP1-1856 | chr17:5585333-5585360 |
| NLRP1-881 | chr17:5539586-5539613 | NLRP1-1857 | chr17:5585358-5585385 |
| NLRP1-882 | chr17:5539600-5539627 | NLRP1-1858 | chr17:5585362-5585389 |
| NLRP1-883 | chr17:5539601-5539628 | NLRP1-1859 | chr17:5585372-5585399 |
| NLRP1-884 | chr17:5539604-5539631 | NLRP1-1860 | chr17:5585380-5585407 |
| NLRP1-885 | chr17:5539608-5539635 | NLRP1-1861 | chr17:5585381-5585408 |
| NLRP1-886 | chr17:5539609-5539636 | NLRP1-1862 | chr17:5585383-5585410 |
| NLRP1-887 | chr17:5539614-5539641 | NLRP1-1863 | chr17:5585411-5585438 |
| NLRP1-888 | chr17:5539615-5539642 | NLRP1-1864 | chr17:5585412-5585439 |
| NLRP1-889 | chr17:5539621-5539648 | NLRP1-1865 | chr17:5585424-5585451 |
| NLRP1-890 | chr17:5539623-5539650 | NLRP1-1866 | chr17:5585425-5585452 |
| NLRP1-891 | chr17:5539624-5539651 | NLRP1-1867 | chr17:5585428-5585455 |
| NLRP1-892 | chr17:5539651-5539678 | NLRP1-1868 | chr17:5585431-5585458 |
| NLRP1-893 | chr17:5539661-5539688 | NLRP1-1869 | chr17:5585434-5585461 |
| NLRP1-894 | chr17:5539662-5539689 | NLRP1-1870 | chr17:5585442-5585469 |
| NLRP1-895 | chr17:5539667-5539694 | NLRP1-1871 | chr17:5585448-5585475 |
| NLRP1-896 | chr17:5539668-5539695 | NLRP1-1872 | chr17:5585449-5585476 |
| NLRP1-897 | chr17:5539679-5539706 | NLRP1-1873 | chr17:5585453-5585480 |
| NLRP1-898 | chr17:5541737-5541764 | NLRP1-1874 | chr17:5585456-5585483 |
| NLRP1-899 | chr17:5541741-5541768 | NLRP1-1875 | chr17:5585457-5585484 |
| NLRP1-900 | chr17:5541747-5541774 | NLRP1-1876 | chr17:5585463-5585490 |
| NLRP1-901 | chr17:5541750-5541777 | NLRP1-1877 | chr17:5585464-5585491 |
| NLRP1-902 | chr17:5541751-5541778 | NLRP1-1878 | chr17:5585471-5585498 |
| NLRP1-903 | chr17:5541756-5541783 | NLRP1-1879 | chr17:5585484-5585511 |
| NLRP1-904 | chr17:5541757-5541784 | NLRP1-1880 | chr17:5585499-5585526 |
| NLRP1-905 | chr17:5541774-5541801 | NLRP1-1881 | chr17:5585502-5585529 |
| NLRP1-906 | chr17:5541782-5541809 | NLRP1-1882 | chr17:5585507-5585534 |
| NLRP1-907 | chr17:5541785-5541812 | NLRP1-1883 | chr17:5585514-5585541 |
| NLRP1-908 | chr17:5541789-5541816 | NLRP1-1884 | chr17:5585515-5585542 |
| NLRP1-909 | chr17:5541791-5541818 | NLRP1-1885 | chr17:5585516-5585543 |
| NLRP1-910 | chr17:5541792-5541819 | NLRP1-1886 | chr17:5585546-5585573 |
| NLRP1-911 | chr17:5541798-5541825 | NLRP1-1887 | chr17:5585549-5585576 |
| NLRP1-912 | chr17:5541802-5541829 | NLRP1-1888 | chr17:5585558-5585585 |
| NLRP1-913 | chr17:5541822-5541849 | NLRP1-1889 | chr17:5585563-5585590 |
| NLRP1-914 | chr17:5541823-5541850 | NLRP1-1890 | chr17:5585568-5585595 |
| NLRP1-915 | chr17:5541826-5541853 | NLRP1-1891 | chr17:5585578-5585605 |
| NLRP1-916 | chr17:5541829-5541856 | NLRP1-1892 | chr17:5585579-5585606 |
| NLRP1-917 | chr17:5541832-5541859 | NLRP1-1893 | chr17:5585580-5585607 |
| NLRP1-918 | chr17:5541833-5541860 | NLRP1-1894 | chr17:5585581-5585608 |
| NLRP1-919 | chr17:5541834-5541861 | NLRP1-1895 | chr17:5585582-5585609 |
| NLRP1-920 | chr17:5541835-5541862 | NLRP1-1896 | chr17:5585588-5585615 |
| NLRP1-921 | chr17:5541839-5541866 | NLRP1-1897 | chr17:5585591-5585618 |
| NLRP1-922 | chr17:5541840-5541867 | NLRP1-1898 | chr17:5585594-5585621 |
| NLRP1-923 | chr17:5541843-5541870 | NLRP1-1899 | chr17:5585595-5585622 |
| NLRP1-924 | chr17:5541855-5541882 | NLRP1-1900 | chr17:5585607-5585634 |
| NLRP1-925 | chr17:5541871-5541898 | NLRP1-1901 | chr17:5585622-5585649 |
| NLRP1-926 | chr17:5541877-5541904 | NLRP1-1902 | chr17:5585625-5585652 |
| NLRP1-927 | chr17:5541884-5541911 | NLRP1-1903 | chr17:5585630-5585657 |
| NLRP1-928 | chr17:5541914-5541941 | NLRP1-1904 | chr17:5585635-5585662 |
| NLRP1-929 | chr17:5541916-5541943 | NLRP1-1905 | chr17:5585645-5585672 |
| NLRP1-930 | chr17:5541921-5541948 | NLRP1-1906 | chr17:5585664-5585691 |
| NLRP1-931 | chr17:5541926-5541953 | NLRP1-1907 | chr17:5585694-5585721 |
| NLRP1-932 | chr17:5541931-5541958 | NLRP1-1908 | chr17:5585712-5585739 |
| NLRP1-933 | chr17:5541932-5541959 | NLRP1-1909 | chr17:5585716-5585743 |
| NLRP1-934 | chr17:5541937-5541964 | NLRP1-1910 | chr17:5585717-5585744 |
| NLRP1-935 | chr17:5541941-5541968 | NLRP1-1911 | chr17:5585725-5585752 |
| NLRP1-936 | chr17:5541945-5541972 | NLRP1-1912 | chr17:5585726-5585753 |
| NLRP1-937 | chr17:5541956-5541983 | NLRP1-1913 | chr17:5585729-5585756 |
| NLRP1-938 | chr17:5541961-5541988 | NLRP1-1914 | chr17:5585749-5585776 |
| NLRP1-939 | chr17:5541976-5542003 | NLRP1-1915 | chr17:5585768-5585795 |
| NLRP1-940 | chr17:5541977-5542004 | NLRP1-1916 | chr17:5585771-5585798 |
| NLRP1-941 | chr17:5541985-5542012 | NLRP1-1917 | chr17:5585772-5585799 |
| NLRP1-942 | chr17:5541990-5542017 | NLRP1-1918 | chr17:5585775-5585802 |
| NLRP1-943 | chr17:5541999-5542026 | NLRP1-1919 | chr17:5585778-5585805 |
| NLRP1-944 | chr17:5542006-5542033 | NLRP1-1920 | chr17:5585780-5585807 |
| NLRP1-945 | chr17:5542013-5542040 | NLRP1-1921 | chr17:5585785-5585812 |
| NLRP1-946 | chr17:5542019-5542046 | NLRP1-1922 | chr17:5585788-5585815 |
| NLRP1-947 | chr17:5542023-5542050 | NLRP1-1923 | chr17:5585791-5585818 |
| NLRP1-948 | chr17:5542027-5542054 | NLRP1-1924 | chr17:5585792-5585819 |
| NLRP1-949 | chr17:5542031-5542058 | NLRP1-1925 | chr17:5585800-5585827 |
| NLRP1-950 | chr17:5542044-5542071 | NLRP1-1926 | chr17:5585801-5585828 |
| NLRP1-951 | chr17:5542045-5542072 | NLRP1-1927 | chr17:5585808-5585835 |
| NLRP1-952 | chr17:5542066-5542093 | NLRP1-1928 | chr17:5585833-5585860 |
| NLRP1-953 | chr17:5542085-5542112 | NLRP1-1929 | chr17:5585837-5585864 |
| NLRP1-954 | chr17:5542086-5542113 | NLRP1-1930 | chr17:5585840-5585867 |
| NLRP1-955 | chr17:5542094-5542121 | NLRP1-1931 | chr17:5585847-5585874 |
| NLRP1-956 | chr17:5542096-5542123 | NLRP1-1932 | chr17:5585851-5585878 |
| NLRP1-957 | chr17:5542101-5542128 | NLRP1-1933 | chr17:5585858-5585885 |
| NLRP1-958 | chr17:5542102-5542129 | NLRP1-1934 | chr17:5585863-5585890 |
| NLRP1-959 | chr17:5542106-5542133 | NLRP1-1935 | chr17:5585878-5585905 |
| NLRP1-960 | chr17:5542107-5542134 | NLRP1-1936 | chr17:5585889-5585916 |
| NLRP1-961 | chr17:5542113-5542140 | NLRP1-1937 | chr17:5585893-5585920 |
| NLRP1-962 | chr17:5542117-5542144 | NLRP1-1938 | chr17:5585899-5585926 |
| NLRP1-963 | chr17:5542122-5542149 | NLRP1-1939 | chr17:5585915-5585942 |
| NLRP1-964 | chr17:5553260-5553287 | NLRP1-1940 | chr17:5585916-5585943 |
| NLRP1-965 | chr17:5553261-5553288 | NLRP1-1941 | chr17:5585921-5585948 |
| NLRP1-966 | chr17:5553265-5553292 | NLRP1-1942 | chr17:5585922-5585949 |
| NLRP1-967 | chr17:5553275-5553302 | NLRP1-1943 | chr17:5585934-5585961 |
| NLRP1-968 | chr17:5553309-5553336 | NLRP1-1944 | chr17:5585939-5585966 |
| NLRP1-969 | chr17:5553310-5553337 | NLRP1-1945 | chr17:5585967-5585994 |
| NLRP1-970 | chr17:5553311-5553338 | NLRP1-1946 | chr17:5585968-5585995 |
| NLRP1-971 | chr17:5553316-5553343 | NLRP1-1947 | chr17:5585969-5585996 |
| NLRP1-972 | chr17:5553317-5553344 | NLRP1-1948 | chr17:5585981-5586008 |
| NLRP1-973 | chr17:5553333-5553360 | NLRP1-1949 | chr17:5585984-5586011 |
| NLRP1-974 | chr17:5553346-5553373 | NLRP1-1950 | chr17:5585999-5586026 |
| NLRP1-975 | chr17:5553347-5553374 | NLRP1-1951 | chr17:5586000-5586027 |
| NLRP1-976 | chr17:5553348-5553375 | NLRP1-1952 | chr17:5586005-5586032 |

**Table 2N Genomic coordinates of the human SCGB1A1 gene**

| Target ID | Target Coordinate Range | Target ID | Target Coordinate Range |
|---|---|---|---|
| SCGB1A1-1 | chr11:62417509-62417536 | SCGB1A1-188 | chr11:62418746-62418773 |
| SCGB1A1-2 | chr11:62417510-62417537 | SCGB1A1-189 | chr11:62418747-62418774 |
| SCGB1A1-3 | chr11:62417518-62417545 | SCGB1A1-190 | chr11:62418749-62418776 |
| SCGB1A1-4 | chr11:62417537-62417564 | SCGB1A1-191 | chr11:62418750-62418777 |
| SCGB1A1-5 | chr11:62417552-62417579 | SCGB1A1-192 | chr11:62418771-62418798 |
| SCGB1A1-6 | chr11:62417555-62417582 | SCGB1A1-193 | chr11:62418772-62418799 |
| SCGB1A1-7 | chr11:62417556-62417583 | SCGB1A1-194 | chr11:62418781-62418808 |
| SCGB1A1-8 | chr11:62417565-62417592 | SCGB1A1-195 | chr11:62418787-62418814 |
| SCGB1A1-9 | chr11:62417583-62417610 | SCGB1A1-196 | chr11:62418792-62418819 |
| SCGB1A1-10 | chr11:62417624-62417651 | SCGB1A1-197 | chr11:62418796-62418823 |
| SCGB1A1-11 | chr11:62417634-62417661 | SCGB1A1-198 | chr11:62418797-62418824 |
| SCGB1A1-12 | chr11:62417639-62417666 | SCGB1A1-199 | chr11:62418802-62418829 |
| SCGB1A1-13 | chr11:62417652-62417679 | SCGB1A1-200 | chr11:62418803-62418830 |
| SCGB1A1-14 | chr11:62417653-62417680 | SCGB1A1-201 | chr11:62418812-62418839 |
| SCGB1A1-15 | chr11:62417654-62417681 | SCGB1A1-202 | chr11:62418815-62418842 |
| SCGB1A1-16 | chr11:62417656-62417683 | SCGB1A1-203 | chr11:62418820-62418847 |
| SCGB1A1-17 | chr11:62417660-62417687 | SCGB1A1-204 | chr11:62418830-62418857 |
| SCGB1A1-18 | chr11:62417661-62417688 | SCGB1A1-205 | chr11:62418831-62418858 |
| SCGB1A1-19 | chr11:62417667-62417694 | SCGB1A1-206 | chr11:62418834-62418861 |
| SCGB1A1-20 | chr11:62417670-62417697 | SCGB1A1-207 | chr11:62418837-62418864 |
| SCGB1A1-21 | chr11:62417674-62417701 | SCGB1A1-208 | chr11:62418838-62418865 |
| SCGB1A1-22 | chr11:62417684-62417711 | SCGB1A1-209 | chr11:62418839-62418866 |
| SCGB1A1-23 | chr11:62417696-62417723 | SCGB1A1-210 | chr11:62418840-62418867 |
| SCGB1A1-24 | chr11:62417737-62417764 | SCGB1A1-211 | chr11:62418847-62418874 |
| SCGB1A1-25 | chr11:62417745-62417772 | SCGB1A1-212 | chr11:62418848-62418875 |
| SCGB1A1-26 | chr11:62417751-62417778 | SCGB1A1-213 | chr11:62418849-62418876 |
| SCGB1A1-27 | chr11:62417755-62417782 | SCGB1A1-214 | chr11:62418860-62418887 |
| SCGB1A1-28 | chr11:62417770-62417797 | SCGB1A1-215 | chr11:62418861-62418888 |
| SCGB1A1-29 | chr11:62417777-62417804 | SCGB1A1-216 | chr11:62418872-62418899 |
| SCGB1A1-30 | chr11:62417794-62417821 | SCGB1A1-217 | chr11:62418873-62418900 |
| SCGB1A1-31 | chr11:62417802-62417829 | SCGB1A1-218 | chr11:62418909-62418936 |
| SCGB1A1-32 | chr11:62417817-62417844 | SCGB1A1-219 | chr11:62418914-62418941 |
| SCGB1A1-33 | chr11:62417849-62417876 | SCGB1A1-220 | chr11:62418915-62418942 |
| SCGB1A1-34 | chr11:62417850-62417877 | SCGB1A1-221 | chr11:62418918-62418945 |
| SCGB1A1-35 | chr11:62417858-62417885 | SCGB1A1-222 | chr11:62418923-62418950 |
| SCGB1A1-36 | chr11:62417866-62417893 | SCGB1A1-223 | chr11:62418929-62418956 |
| SCGB1A1-37 | chr11:62417867-62417894 | SCGB1A1-224 | chr11:62418958-62418985 |
| SCGB1A1-38 | chr11:62417868-62417895 | SCGB1A1-225 | chr11:62418959-62418986 |
| SCGB1A1-39 | chr11:62417869-62417896 | SCGB1A1-226 | chr11:62418960-62418987 |
| SCGB1A1-40 | chr11:62417874-62417901 | SCGB1A1-227 | chr11:62418961-62418988 |
| SCGB1A1-41 | chr11:62417893-62417920 | SCGB1A1-228 | chr11:62418966-62418993 |
| SCGB1A1-42 | chr11:62417897-62417924 | SCGB1A1-229 | chr11:62418967-62418994 |
| SCGB1A1-43 | chr11:62417903-62417930 | SCGB1A1-230 | chr11:62418971-62418998 |
| SCGB1A1-44 | chr11:62417910-62417937 | SCGB1A1-231 | chr11:62418972-62418999 |
| SCGB1A1-45 | chr11:62417911-62417938 | SCGB1A1-232 | chr11:62418976-62419003 |
| SCGB1A1-46 | chr11:62417916-62417943 | SCGB1A1-233 | chr11:62418977-62419004 |
| SCGB1A1-47 | chr11:62417917-62417944 | SCGB1A1-234 | chr11:62418978-62419005 |
| SCGB1A1-48 | chr11:62417945-62417972 | SCGB1A1-235 | chr11:62418985-62419012 |
| SCGB1A1-49 | chr11:62417951-62417978 | SCGB1A1-236 | chr11:62418995-62419022 |
| SCGB1A1-50 | chr11:62417958-62417985 | SCGB1A1-237 | chr11:62418996-62419023 |
| SCGB1A1-51 | chr11:62417970-62417997 | SCGB1A1-238 | chr11:62418997-62419024 |
| SCGB1A1-52 | chr11:62417971-62417998 | SCGB1A1-239 | chr11:62419015-62419042 |
| SCGB1A1-53 | chr11:62417985-62418012 | SCGB1A1-240 | chr11:62419032-62419059 |
| SCGB1A1-54 | chr11:62417998-62418025 | SCGB1A1-241 | chr11:62419040-62419067 |
| SCGB1A1-55 | chr11:62417999-62418026 | SCGB1A1-242 | chr11:62419041-62419068 |
| SCGB1A1-56 | chr11:62418006-62418033 | SCGB1A1-243 | chr11:62419044-62419071 |
| SCGB1A1-57 | chr11:62418019-62418046 | SCGB1A1-244 | chr11:62419045-62419072 |
| SCGB1A1-58 | chr11:62418022-62418049 | SCGB1A1-245 | chr11:62419062-62419089 |
| SCGB1A1-59 | chr11:62418033-62418060 | SCGB1A1-246 | chr11:62419073-62419100 |
| SCGB1A1-60 | chr11:62418037-62418064 | SCGB1A1-247 | chr11:62419082-62419109 |
| SCGB1A1-61 | chr11:62418043-62418070 | SCGB1A1-248 | chr11:62419083-62419110 |
| SCGB1A1-62 | chr11:62418050-62418077 | SCGB1A1-249 | chr11:62419084-62419111 |
| SCGB1A1-63 | chr11:62418055-62418082 | SCGB1A1-250 | chr11:62419085-62419112 |
| SCGB1A1-64 | chr11:62418056-62418083 | SCGB1A1-251 | chr11:62419088-62419115 |
| SCGB1A1-65 | chr11:62418059-62418086 | SCGB1A1-252 | chr11:62419091-62419118 |
| SCGB1A1-66 | chr11:62418063-62418090 | SCGB1A1-253 | chr11:62419094-62419121 |
| SCGB1A1-67 | chr11:62418064-62418091 | SCGB1A1-254 | chr11:62419096-62419123 |
| SCGB1A1-68 | chr11:62418067-62418094 | SCGB1A1-255 | chr11:62419105-62419132 |
| SCGB1A1-69 | chr11:62418077-62418104 | SCGB1A1-256 | chr11:62419112-62419139 |
| SCGB1A1-70 | chr11:62418078-62418105 | SCGB1A1-257 | chr11:62419113-62419140 |
| SCGB1A1-71 | chr11:62418079-62418106 | SCGB1A1-258 | chr11:62419118-62419145 |
| SCGB1A1-72 | chr11:62418091-62418118 | SCGB1A1-259 | chr11:62419119-62419146 |
| SCGB1A1-73 | chr11:62418096-62418123 | SCGB1A1-260 | chr11:62419124-62419151 |
| SCGB1A1-74 | chr11:62418101-62418128 | SCGB1A1-261 | chr11:62419148-62419175 |
| SCGB1A1-75 | chr11:62418111-62418138 | SCGB1A1-262 | chr11:62419156-62419183 |
| SCGB1A1-76 | chr11:62418114-62418141 | SCGB1A1-263 | chr11:62419163-62419190 |
| SCGB1A1-77 | chr11:62418116-62418143 | SCGB1A1-264 | chr11:62419166-62419193 |
| SCGB1A1-78 | chr11:62418117-62418144 | SCGB1A1-265 | chr11:62419167-62419194 |
| SCGB1A1-79 | chr11:62418119-62418146 | SCGB1A1-266 | chr11:62419171-62419198 |
| SCGB1A1-80 | chr11:62418123-62418150 | SCGB1A1-267 | chr11:62419172-62419199 |
| SCGB1A1-81 | chr11:62418135-62418162 | SCGB1A1-268 | chr11:62419173-62419200 |
| SCGB1A1-82 | chr11:62418139-62418166 | SCGB1A1-269 | chr11:62419175-62419202 |
| SCGB1A1-83 | chr11:62418140-62418167 | SCGB1A1-270 | chr11:62419176-62419203 |
| SCGB1A1-84 | chr11:62418141-62418168 | SCGB1A1-271 | chr11:62419179-62419206 |
| SCGB1A1-85 | chr11:62418148-62418175 | SCGB1A1-272 | chr11:62419199-62419226 |
| SCGB1A1-86 | chr11:62418151-62418178 | SCGB1A1-273 | chr11:62419202-62419229 |
| SCGB1A1-87 | chr11:62418158-62418185 | SCGB1A1-274 | chr11:62419203-62419230 |
| SCGB1A1-88 | chr11:62418160-62418187 | SCGB1A1-275 | chr11:62419204-62419231 |
| SCGB1A1-89 | chr11:62418161-62418188 | SCGB1A1-276 | chr11:62419205-62419232 |
| SCGB1A1-90 | chr11:62418162-62418189 | SCGB1A1-277 | chr11:62419206-62419233 |
| SCGB1A1-91 | chr11:62418168-62418195 | SCGB1A1-278 | chr11:62419224-62419251 |
| SCGB1A1-92 | chr11:62418179-62418206 | SCGB1A1-279 | chr11:62419233-62419260 |
| SCGB1A1-93 | chr11:62418180-62418207 | SCGB1A1-280 | chr11:62419234-62419261 |
| SCGB1A1-94 | chr11:62418196-62418223 | SCGB1A1-281 | chr11:62419236-62419263 |
| SCGB1A1-95 | chr11:62418202-62418229 | SCGB1A1-282 | chr11:62419237-62419264 |
| SCGB1A1-96 | chr11:62418210-62418237 | SCGB1A1-283 | chr11:62419242-62419269 |
| SCGB1A1-97 | chr11:62418234-62418261 | SCGB1A1-284 | chr11:62419243-62419270 |
| SCGB1A1-98 | chr11:62418239-62418266 | SCGB1A1-285 | chr11:62419244-62419271 |
| SCGB1A1-99 | chr11:62418250-62418277 | SCGB1A1-286 | chr11:62422099-62422126 |
| SCGB1A1-100 | chr11:62418258-62418285 | SCGB1A1-287 | chr11:62422100-62422127 |
| SCGB1A1-101 | chr11:62418267-62418294 | SCGB1A1-288 | chr11:62422105-62422132 |
| SCGB1A1-102 | chr11:62418270-62418297 | SCGB1A1-289 | chr11:62422119-62422146 |
| SCGB1A1-103 | chr11:62418275-62418302 | SCGB1A1-290 | chr11:62422120-62422147 |
| SCGB1A1-104 | chr11:62418276-62418303 | SCGB1A1-291 | chr11:62422128-62422155 |
| SCGB1A1-105 | chr11:62418295-62418322 | SCGB1A1-292 | chr11:62422136-62422163 |
| SCGB1A1-106 | chr11:62418305-62418332 | SCGB1A1-293 | chr11:62422137-62422164 |
| SCGB1A1-107 | chr11:62418318-62418345 | SCGB1A1-294 | chr11:62422150-62422177 |
| SCGB1A1-108 | chr11:62418326-62418353 | SCGB1A1-295 | chr11:62422152-62422179 |
| SCGB1A1-109 | chr11:62418333-62418360 | SCGB1A1-296 | chr11:62422153-62422180 |
| SCGB1A1-110 | chr11:62418334-62418361 | SCGB1A1-297 | chr11:62422155-62422182 |
| SCGB1A1-111 | chr11:62418337-62418364 | SCGB1A1-298 | chr11:62422156-62422183 |
| SCGB1A1-112 | chr11:62418352-62418379 | SCGB1A1-299 | chr11:62422157-62422184 |
| SCGB1A1-113 | chr11:62418354-62418381 | SCGB1A1-300 | chr11:62422162-62422189 |
| SCGB1A1-114 | chr11:62418360-62418387 | SCGB1A1-301 | chr11:62422169-62422196 |
| SCGB1A1-115 | chr11:62418373-62418400 | SCGB1A1-302 | chr11:62422185-62422212 |
| SCGB1A1-116 | chr11:62418374-62418401 | SCGB1A1-303 | chr11:62422199-62422226 |
| SCGB1A1-117 | chr11:62418382-62418409 | SCGB1A1-304 | chr11:62422207-62422234 |
| SCGB1A1-118 | chr11:62418389-62418416 | SCGB1A1-305 | chr11:62422237-62422264 |
| SCGB1A1-119 | chr11:62418390-62418417 | SCGB1A1-306 | chr11:62422238-62422265 |
| SCGB1A1-120 | chr11:62418393-62418420 | SCGB1A1-307 | chr11:62422247-62422274 |
| SCGB1A1-121 | chr11:62418402-62418429 | SCGB1A1-308 | chr11:62422263-62422290 |
| SCGB1A1-122 | chr11:62418408-62418435 | SCGB1A1-309 | chr11:62422264-62422291 |
| SCGB1A1-123 | chr11:62418409-62418436 | SCGB1A1-310 | chr11:62422267-62422294 |
| SCGB1A1-124 | chr11:62418412-62418439 | SCGB1A1-311 | chr11:62422268-62422295 |
| SCGB1A1-125 | chr11:62418413-62418440 | SCGB1A1-312 | chr11:62422277-62422304 |
| SCGB1A1-126 | chr11:62418429-62418456 | SCGB1A1-313 | chr11:62422280-62422307 |
| SCGB1A1-127 | chr11:62418430-62418457 | SCGB1A1-314 | chr11:62422281-62422308 |
| SCGB1A1-128 | chr11:62418431-62418458 | SCGB1A1-315 | chr11:62422284-62422311 |
| SCGB1A1-129 | chr11:62418435-62418462 | SCGB1A1-316 | chr11:62422299-62422326 |
| SCGB1A1-130 | chr11:62418436-62418463 | SCGB1A1-317 | chr11:62422306-62422333 |
| SCGB1A1-131 | chr11:62418442-62418469 | SCGB1A1-318 | chr11:62422307-62422334 |
| SCGB1A1-132 | chr11:62418447-62418474 | SCGB1A1-319 | chr11:62422310-62422337 |
| SCGB1A1-133 | chr11:62418448-62418475 | SCGB1A1-320 | chr11:62422314-62422341 |
| SCGB1A1-134 | chr11:62418452-62418479 | SCGB1A1-321 | chr11:62422315-62422342 |
| SCGB1A1-135 | chr11:62418453-62418480 | SCGB1A1-322 | chr11:62422316-62422343 |
| SCGB1A1-136 | chr11:62418461-62418488 | SCGB1A1-323 | chr11:62422334-62422361 |
| SCGB1A1-137 | chr11:62418465-62418492 | SCGB1A1-324 | chr11:62422337-62422364 |
| SCGB1A1-138 | chr11:62418466-62418493 | SCGB1A1-325 | chr11:62422368-62422395 |
| SCGB1A1-139 | chr11:62418495-62418522 | SCGB1A1-326 | chr11:62422369-62422396 |
| SCGB1A1-140 | chr11:62418496-62418523 | SCGB1A1-327 | chr11:62422372-62422399 |
| SCGB1A1-141 | chr11:62418497-62418524 | SCGB1A1-328 | chr11:62422373-62422400 |
| SCGB1A1-142 | chr11:62418498-62418525 | SCGB1A1-329 | chr11:62422374-62422401 |
| SCGB1A1-143 | chr11:62418519-62418546 | SCGB1A1-330 | chr11:62422375-62422402 |
| SCGB1A1-144 | chr11:62418526-62418553 | SCGB1A1-331 | chr11:62422382-62422409 |
| SCGB1A1-145 | chr11:62418527-62418554 | SCGB1A1-332 | chr11:62422383-62422410 |
| SCGB1A1-146 | chr11:62418528-62418555 | SCGB1A1-333 | chr11:62422409-62422436 |
| SCGB1A1-147 | chr11:62418529-62418556 | SCGB1A1-334 | chr11:62422413-62422440 |
| SCGB1A1-148 | chr11:62418540-62418567 | SCGB1A1-335 | chr11:62422419-62422446 |
| SCGB1A1-149 | chr11:62418547-62418574 | SCGB1A1-336 | chr11:62422432-62422459 |
| SCGB1A1-150 | chr11:62418553-62418580 | SCGB1A1-337 | chr11:62422433-62422460 |
| SCGB1A1-151 | chr11:62418554-62418581 | SCGB1A1-338 | chr11:62422434-62422461 |
| SCGB1A1-152 | chr11:62418558-62418585 | SCGB1A1-339 | chr11:62422441-62422468 |
| SCGB1A1-153 | chr11:62418570-62418597 | SCGB1A1-340 | chr11:62422450-62422477 |
| SCGB1A1-154 | chr11:62418575-62418602 | SCGB1A1-341 | chr11:62422451-62422478 |
| SCGB1A1-155 | chr11:62418576-62418603 | SCGB1A1-342 | chr11:62422452-62422479 |
| SCGB1A1-156 | chr11:62418577-62418604 | SCGB1A1-343 | chr11:62422473-62422500 |
| SCGB1A1-157 | chr11:62418590-62418617 | SCGB1A1-344 | chr11:62422474-62422501 |
| SCGB1A1-158 | chr11:62418591-62418618 | SCGB1A1-345 | chr11:62422475-62422502 |
| SCGB1A1-159 | chr11:62418594-62418621 | SCGB1A1-346 | chr11:62422480-62422507 |
| SCGB1A1-160 | chr11:62418599-62418626 | SCGB1A1-347 | chr11:62422487-62422514 |
| SCGB1A1-161 | chr11:62418607-62418634 | SCGB1A1-348 | chr11:62422499-62422526 |
| SCGB1A1-162 | chr11:62418608-62418635 | SCGB1A1-349 | chr11:62422506-62422533 |
| SCGB1A1-163 | chr11:62418609-62418636 | SCGB1A1-350 | chr11:62422933-62422960 |
| SCGB1A1-164 | chr11:62418619-62418646 | SCGB1A1-351 | chr11:62422934-62422961 |
| SCGB1A1-165 | chr11:62418620-62418647 | SCGB1A1-352 | chr11:62422937-62422964 |
| SCGB1A1-166 | chr11:62418638-62418665 | SCGB1A1-353 | chr11:62422953-62422980 |
| SCGB1A1-167 | chr11:62418645-62418672 | SCGB1A1-354 | chr11:62422954-62422981 |
| SCGB1A1-168 | chr11:62418651-62418678 | SCGB1A1-355 | chr11:62422995-62423022 |
| SCGB1A1-169 | chr11:62418652-62418679 | SCGB1A1-356 | chr11:62423000-62423027 |
| SCGB1A1-170 | chr11:62418657-62418684 | SCGB1A1-357 | chr11:62423001-62423028 |
| SCGB1A1-171 | chr11:62418659-62418686 | SCGB1A1-358 | chr11:62423032-62423059 |
| SCGB1A1-172 | chr11:62418660-62418687 | SCGB1A1-359 | chr11:62423069-62423096 |
| SCGB1A1-173 | chr11:62418662-62418689 | SCGB1A1-360 | chr11:62423070-62423097 |
| SCGB1A1-174 | chr11:62418663-62418690 | SCGB1A1-361 | chr11:62423110-62423137 |
| SCGB1A1-175 | chr11:62418667-62418694 | SCGB1A1-362 | chr11:62423111-62423138 |
| SCGB1A1-176 | chr11:62418676-62418703 | SCGB1A1-363 | chr11:62423112-62423139 |
| SCGB1A1-177 | chr11:62418679-62418706 | SCGB1A1-364 | chr11:62423121-62423148 |
| SCGB1A1-178 | chr11:62418680-62418707 | SCGB1A1-365 | chr11:62423125-62423152 |
| SCGB1A1-179 | chr11:62418688-62418715 | SCGB1A1-366 | chr11:62423131-62423158 |
| SCGB1A1-180 | chr11:62418699-62418726 | SCGB1A1-367 | chr11:62423137-62423164 |
| SCGB1A1-181 | chr11:62418706-62418733 | SCGB1A1-368 | chr11:62423150-62423177 |
| SCGB1A1-182 | chr11:62418710-62418737 | SCGB1A1-369 | chr11:62423155-62423182 |
| SCGB1A1-183 | chr11:62418724-62418751 | SCGB1A1-370 | chr11:62423156-62423183 |
| SCGB1A1-184 | chr11:62418738-62418765 | SCGB1A1-371 | chr11:62423159-62423186 |
| SCGB1A1-185 | chr11:62418739-62418766 | SCGB1A1-372 | chr11:62423163-62423190 |
| SCGB1A1-186 | chr11:62418740-62418767 | SCGB1A1-373 | chr11:62423182-62423209 |
| SCGB1A1-187 | chr11:62418742-62418769 | | |

## Claims

1. A method for culturing a cell is provided, wherein the method comprises causing a family member selected from the Bcl-2 family or the protein tyrosine phosphatase family and/or a functionally active fragment thereof in the cell to be reduced and/or attenuated.

2. The method according to claim 1, wherein the cell comprises an immune cell.

3. The method according to claim 2, wherein the immune cell comprises a phagocyte, a lymphocyte, a neutrophil, an eosinophil, and/or a basophil.

4. The method according to any one of claims 2 to 3, wherein the immune cell comprises a monocyte, a macrophage, and/or a dendritic cell.

5. The method according to any one of claims 2-4, wherein the immune cell is derived from a stem cell-differentiated immune cell.

6. The method according to claim 5, wherein the stem cell comprises an induced pluripotent stem cell (iPSC), an embryonic stem cell, a bone marrow stem cell, a cord blood stem cell, and/or a peripheral blood stem cell.

7. The method according to any one of claims 2-6, wherein the immune cell comprises a B cell, a T cell, a natural killer cell, and/or a natural killer-like T cell (NKT).

8. The method according to any one of claims 2-7, wherein the immune cell comprises an αβ T cell and/or a γδ T cell.

9. The method according to any one of claims 2-8, wherein the immune cell comprises a tumor-infiltrating lymphocyte (TIL).

10. The method according to claim 9, wherein the TIL is a TIL derived from tumor tissue, tumor-draining lymph nodes with or without tumor metastasis, metastatic tumor lesions, fragments of para-carcinoma tissue, pleural effusion and/or ascites, and/or a TIL derived from cryopreserved and subsequently thawed TILs.

11. The method according to claim 10, wherein the size of fragment is about 1 cubic millimeter to about 27 cubic millimeters.

12. The method according to any one of claims 2-11, wherein the immune cell comprises an engineered immune receptor displayed on a cell surface.

13. The method according to claim 12, wherein the engineered immune receptor specifically binds to an antigen expressed on a target cell.

14. The method according to any one of claims 2-13, wherein the immune cell comprises a chimeric antigen receptor and/or a T cell receptor.

15. The method according to any one of claims 1-14, wherein causing the family member selected from the Bcl-2 family or the protein tyrosine phosphatase family and/or the functionally active fragment thereof in the cell to be reduced and/or attenuated comprises an effect selected from the group consisting of inhibiting an apoptosis-inducing function or inhibiting a tyrosine phosphatase function.

16. The method according to any one of claims 1-15, wherein, as compared with a cell in which the family member selected from the Bcl-2 family or the protein tyrosine phosphatase family and/or the functionally active fragment thereof is altered and/or unaltered, a cell obtained by causing the family member selected from the Bcl-2 family or the protein tyrosine phosphatase family and/or the functionally active fragment thereof to be reduced and/or attenuated exhibits improved cellular characteristics.

17. The method according to claim 16, wherein the improved cellular characteristics comprise one or more selected from the group consisting of improved cell proliferation capacity, an increased proportion of viable cells, an improved cell subpopulation ratio, enhanced cytokine secretion capacity, enhanced in vitro tumor cell killing capacity, and enhanced in vivo tumor killing capacity.

18. The method according to claim 17, wherein the improved cell subpopulation ratio comprises one or more selected from the group consisting of an increased proportion of activated cells, a reduced proportion of regulatory cells, a reduced proportion of exhausted cells, an increased proportion of central memory cells and/or naïve cells, a reduced proportion of apoptotic cells, and an increased proportion of stem cell-like cells.

19. The method according to any one of claims 1-18, wherein the family members of the Bcl-2 family and the protein tyrosine phosphatase family comprise a Bcl-2 homology domain 3 domain and a tyrosine phosphatase domain, respectively.

20. The method according to any one of claims 1-19, wherein the family members of the Bcl-2 family and the protein tyrosine phosphatase family comprise BCL2L11 and PTPN2, respectively.

21. The method according to any one of claims 1-20, wherein causing the family member of the Bcl-2 family or the protein tyrosine phosphatase family in the cell to be reduced and/or attenuated comprises introducing into the cell.

22. The method according to claim 21, wherein the gene regulation system disrupts a family member of the Bcl-2 family or the protein tyrosine phosphatase family at the DNA level; and optionally, expression of a gene selected from BRD4, FAS, TNFAIP3, ZC3H12A, SOCS1, CBLB, FIBP, IKZF1, LAG3, MED12, PD1, RASA2, TIGIT, TIM3, ADNP, NFKBIA, PTPN6, AFF3, AXL, NFE2L1, RARG, UBFD1, CRP, CYLD, CBLIF, KLF4, NDST1, NLRP1, SCGB1A1, or TNIP1 in the cell is reduced and/or activity thereof is attenuated.

23. The method according to any one of claims 21 to 22, wherein the gene regulation system comprises a guide nucleic acid molecule and an enzyme protein.

24. The method according to claim 23, wherein causing expression of the family member of the Bcl-2 family or the protein tyrosine phosphatase family to be reduced and/or activity to be attenuated comprises introducing into the cell a complex comprising the guide nucleic acid molecule and the enzyme protein, or a complex comprising the guide nucleic acid molecule and a nucleic acid encoding the enzyme protein.

25. The method according to any one of claims 23-24, wherein the enzyme protein comprises a Cas protein homolog, or a functionally active fragment thereof, preferably selected from Cas 9 and Cas 12.

26. The method according to any one of claims 23-25, wherein the guide nucleic acid molecule comprises (gRNA).

27. The method according to any one of claims 23-26, wherein the guide nucleic acid molecule binds to a sequence of the family member selected from the Bcl-2 family or the protein tyrosine phosphatase family.

28. The method according to any one of claims 23-27, wherein the guide nucleic acid molecule binds to a sequence consisting of about 15 to about 25 nucleotides upstream of the 5' end of a protospacer adjacent motif (PAM) selected from the group consisting of AGG, TGG, CGG, and GGG, or binds to a sequence consisting of about 15 to about 25 nucleotides downstream of the 3' end of a protospacer adjacent motif (PAM) selected from the group consisting of NTTN, TTYN, VTTV, TRTV, TTTV, TATV, TYCV, TNN, and NTN, wherein N is A, T, C, or G, Y is T or C, V is A, C, or G, and R is A or G, or binds to a sequence consisting of about 15 to about 25 nucleotides downstream of the 3' end of a protospacer adjacent motif (PAM) selected from the group consisting of TTTN, ATTN, GTTN, CTTN, TTC, TTG, TTA, TTT, TAN, TGN, and TCN, wherein N is A, T, C, or G.

29. The method according to any one of claims 23-28, wherein the guide nucleic acid molecule binds to a region defined by genomic coordinates shown in Table 2A and Table 2B, or a fragment thereof.

30. The method according to any one of claims 23-29, wherein the guide nucleic acid molecule binds to a region selected from the group consisting of SEQ ID NO: 24789-26713 and SEQ ID NO: 26714-28115, or a fragment thereof.

31. The method according to any one of claims 23-30, wherein the guide nucleic acid molecule comprises a sequence as shown in SEQ ID NO: 1-1925, SEQ ID NO: 1926-3327, SEQ ID NO: 49607-49627, and SEQ ID NO: 49628-49637.

32. The method according to any one of claims 1-31, wherein, as compared with a cell in which expression and/or activity of a family member of the Bcl-2 family or the protein tyrosine phosphatase family is unaltered, in a cell obtained by causing expression of the family member of the Bcl-2 family or the protein tyrosine phosphatase family to be reduced and/or activity to be attenuated, a proportion of cells expressing the target gene is reduced and/or an expression level of the target gene in a single cell is decreased.

33. The method according to any one of claims 1-32, wherein, in the cell obtained by causing expression of the family member of the Bcl-2 family or the protein tyrosine phosphatase family to be reduced and/or activity to be attenuated, the proportion of cells expressing the target gene is about 95% or less.

34. A cell, wherein the cell is obtained by the method according to any one of claims 1-33.

35. A phmarceutical composition comprising the cell according to claim 34, and optionally a pharmaceutically acceptable carrier.

36. A method for affecting cell growth, wherein the method comprises the cell according to claim 34 and/or the pharmaceutical composition according to claim 35.

37. Use of the cell according to claim 34 and/or the pharmaceutical composition according to claim 35 in the manufacture of a medicament, wherein the medicament is used for preventing and/or treating a disease and/or symptom.

38. A medicament for preventing and/or treating a disease and/or symptom, wherein the medicament comprises the cell according to claim 34 and/or the pharmaceutical composition according to claim 35 as an active ingredient.

39. A method for preventing and/or treating a disease and/or symptom, wherein the method comprises administering, to a subject in need thereof, the cell according to claim 34 and/or the pharmaceutical composition according to claim 35.

40. The cell according to claim 34 and/or the pharmaceutical composition according to claim 35, wherein the use is preventing and/or treating a disease and/or symptom.

41. The use according to claim 37, the medicament according to claim 38, the method according to claim 39, and/or the use of the cell and/or the pharmaceutical composition according to claim 40, wherein the disease and/or symptom comprises a tumor.

42. The use according to claim 37, the medicament according to claim 38, the method according to claim 39, and/or the use of the cell and/or the pharmaceutical composition according to claim 40, wherein the disease and/or symptom comprises a solid tumor.

43. The use according to claim 37, the medicament according to claim 38, the method according to claim 39, and/or the use of the cell and/or the pharmaceutical composition according to claim 40, wherein the disease and/or symptom comprises one or more selected from the group consisting of melanoma, ovarian cancer, cervical cancer, lung cancer, bladder cancer, breast cancer, head and neck cancer, pancreatic cancer, liver cancer, gastric cancer, colorectal cancer, and renal cancer.
